# EUROPEAN PATENT SPECIFICATION

(11) **EP 3 081 552 B1**
(45) Date of publication and mention of the grant of the patent: **03.03.2021**
(21) Application number: 16166952.8
(22) Date of filing: 30.06.2009
(51) Int. Cl.: C07C 237/42, A01N 37/46, A01P 7/02, A01P 7/04, C07C 255/60, C07D 213/82

(54) **PEST CONTROL AGENT CONTAINING AN AMIDE DERIVATIVE AND USE OF THE PEST CONTROL AGENT**
SCHÄDLINGSBEKÄMPFUNGSMITTEL ENTHALTEND EINEN AMIDDERIVAT UND VERWENDUNG DES SCHÄDLINGSBEKÄMPFUNGSMITTELS
AGENT ANTIPARASITAIRE CONTENANT UN DÉRIVÉ AMIDE ET UTILISATION DE L'AGENT ANTIPARASITAIRE

(30) Priority: 13.08.2008 JP 2008208714
(43) Date of publication of application: 19.10.2016
(62) Divisional of application: 09806610.3
(73) Proprietor: Mitsui Chemicals Agro, Inc., Tokyo 103-0027 (JP)
(72) Inventor: KOBAYASHI, Yumi, Mobara-shi, Chiba 297-0017 (JP); DAIDO, Hidenori, Mobara-shi, Chiba 297-0017 (JP); KATSUTA, Hiroyuki, Mobara-shi, Chiba 297-0017 (JP); NOMURA, Michikazu, Mobara-shi, Chiba 297-0017 (JP); TSUKADA, Hidetaka, Omuta-shi, Fukuoka 836-8610 (JP); HIRABAYASHI, Atsushi, Omuta-shi, Fukuoka 836-8610 (JP); TAKAHASHI, Yusuke, Omuta-shi, Fukuoka 836-8610 (JP); AOKI, Yoji, Mobara-shi, Chiba 297-0017 (JP); KAWAHARA, Atsuko, Mobara-shi, Chiba 297-0017 (JP); FUKAZAWA, Yasuaki, Mobara-shi, Chiba 297-0017 (JP); HIROSE, Mai, Mobara-shi, Chiba 297-0017 (JP)
(74) Representative: Mewburn Ellis LLP

(56) References cited:
- EP-A1- 1 661 886
- EP-A1- 1 714 958
- EP-A1- 1 911 751
- EP-A1- 1 916 236
- WO-A1-2005/019196

## Description

### Technical Field with

The present invention relates to a pest control agent, with an amide derivative contained in the pest control agent.

### Background Art

Various amide derivatives are described in the pamphlets of International Publication WO 2005/21488, International Publication WO 2005/73165, International Publication WO 2006/137376, and International Publication WO 2006/137395.

### Disclosure of Invention

### Problems to be Solved by the Invention

In the production of, for example, agricultural and horticultural crops, due to causes such as currently-occurring large scale damage due to pests or the like and the propagation of pests having resistance to existing chemicals, there is a demand for a novel agricultural/horticultural pest control agent.

It is an object of the present invention to provide a pest control agent containing an amide derivative as an active ingredien showing a pesticidal effect against a wide range of agricultural/horticultural pests and a pest controlling method. Means for Solving the Problems novel

The present inventors have conducted intensive studies to develop a novel pest control agent, and as a result, they have found that the aromatic carboxamide derivative represented by Formula (1) of the present invention is a novel compound unknown in the literature, and it is also a pest control agent, particularly an agricultural/horticultural pest control agent, showing a particularly high efficiency, thereby completing the present invention.

Further, they have also discovered a novel preparation method and a useful intermediate for the preparation of the compound of the present invention. As a result, they have completed the present invention.

That is, the present invention is as follows.
1. A pest control agent comprising at least one amide derivative selected from the group consisting of the following Formula (1), Formula (7), Formula (8) and Formula (9) as an active ingredient: 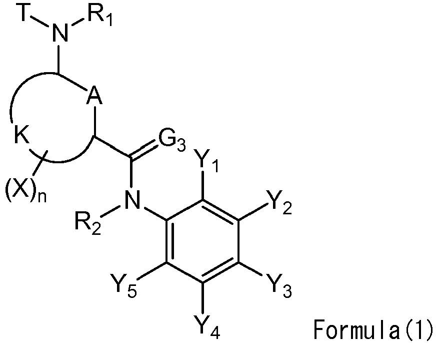 wherein, in Formula (1), A represents a carbon atom, an oxygen atom, a nitrogen atom, an oxidized nitrogen atom, or a sulfur atom; K represents a non-metal atom group necessary for forming a cyclic linking group derived from pyridine, pyridine-N-oxide, pyrrole, thiazole, furan, or thiophene in combination with A and two carbon atoms to which A bonds;
   X represents a hydrogen atom, a halogen atom, a C1-C6 alkyl group, a C1-C6 haloalkyl group, a C3-C9 cycloalkyl group, a C3-C9 halocycloalkyl group, a C2-C6 alkenyl group, a C2-C6 haloalkenyl group, a C2-C6 alkynyl group, a C2-C6 haloalkynyl group, a C1-C6 alkoxy group, a C1-C6 haloalkoxy group, a C2-C6 alkenyloxy group, a C2-C6 haloalkenyloxy group, a C2-C6 alkynyloxy group, a C2-C6 haloalkynyloxy group, a C3-C9 cycloalkoxy group, a C3-C9 halocycloalkoxy group, a C1-C6 alkylthio group, a C1-C6 haloalkylthio group, a C1-C6 alkylsulfinyl group, a C1-C6 haloalkylsulfinyl group, a C1-C6 alkylsulfonyl group, a C1-C6 haloalkylsulfonyl group, a C1-C6 alkylsulfonyloxy group, a C1-C6 haloalkylsulfonyloxy group, a C2-C7 alkylcarbonyl group, a C2-C7 haloalkylcarbonyl group, a C3-C7 alkenylcarbonyl group, a C3-C7 haloalkenylcarbonyl group, a C3-C7 alkynylcarbonyl group, a C3-C7 haloalkynylcarbonyl group, a C4-C10 cycloalkylcarbonyl group, a C4-C10 halocycloalkylcarbonyl group, a C2-C7 alkylcarbonyloxy group, a C2-C7 haloalkylcarbonyloxy group, arylcarbonyloxy group, a C2-C7 alkoxycarbonyl group, a C2-C7 haloalkoxycarbonyl group, a C3-C7 alkenyloxycarbonyl group, a C3-C7 haloalkenyloxycarbonyl group, a C3-C7 alkynyloxycarbonyl group, a C3-C7 haloalkynyloxycarbonyl group, a C4-C10 cycloalkyloxycarbonyl group, a C4-C10 halocycloalkyloxycarbonyl group, a C2-C7 alkylcarbonylamino group, a C2-C7 haloalkylcarbonylamino group, a C2-C7 alkoxycarbonylamino group, a C2-C7 haloalkoxycarbonylamino group, a C2-C7 alkoxycarbonyloxy group, a C2-C7 haloalkoxycarbonyloxy group, an arylcarbonylamino group, an amino group, a carbamoyl group, a cyano group, a hydroxy group, pentafluorosulfanyl group, a C1-C6 alkylamino group, a C1-C6 haloalkylamino group, a C2-C6 alkenylamino group, a C2-C6 haloalkenylamino group, a C2-C6 alkynylamino group, a C2-C6 haloalkynylamino group, a C3-C9 cycloalkylamino group, a C3-C9 halocycloalkylamino group, a C2-C7 alkylaminocarbonyl group, a C2-C7 haloalkylaminocarbonyl group, a C3-C7 alkenylaminocarbonyl group, a C3-C7 haloalkenylaminocarbonyl group, a C3-C7 alkynylaminocarbonyl group, a C3-C7 haloalkynylaminocarbonyl group, a C4-C10 cycloalkylaminocarbonyl group, a C4-C10 halocycloalkylaminocarbonyl group, a phenyl group, or a heterocyclic group, and when there are plural X's, each X may be the same as or different from each other;
   n represents an integer of from 0 to 4;
   T represents -C(=G₁)-Q₁ or -C(=G₁)-G₂Q₂;
   G₁ and G₂ each independently represent an oxygen atom or a sulfur atom;
   Q₁ and Q₂ each represent a hydrogen atom, a C1-C6 alkyl group, a C1-C6 haloalkyl group, a C2-C6 alkenyl group, a C2-C6 haloalkenyl group, a C2-C6 alkynyl group, a C2-C6 haloalkynyl group, a C3-C9 cycloalkyl group, a C3-C9 halocycloalkyl group, a benzyl group, a phenyl group which may have a substituent, a naphthyl group, or a heterocyclic group which may have a substituent;
   Y₁ and Y₅ represent a halogen atom or a C1-C3 haloalkyl group, and either Y₁ or Y₅ represents a C1-C3 haloalkyl group; Y₂ and Y₄ represent a hydrogen atom; Y₃ represents a C2-C4 perfluoroalkyl group;
   wherein, in Qi and Q₂, the substituent of a phenyl group which may have a substituent and a heterocyclic group which may have a substituent represents one or more substituents selected from a group consisting of a halogen atom, a C1-C6 alkyl group, a C1-C6 haloalkyl group, a C3-C9 cycloalkyl group, a C3-C9 halocycloalkyl group, a C2-C6 alkenyl group, a C2-C6 haloalkenyl group, a C2-C6 alkynyl group, a C2-C6 haloalkynyl group, a C1-C6 alkoxy group, a C1-C6 haloalkoxy group, a C2-C6 alkenyloxy group, a C2-C6 haloalkenyloxy group, a C2-C6 alkynyloxy group, a C2-C6 haloalkynyloxy group, a C3-C9 cycloalkoxy group, a C3-C9 halocycloalkoxy group, a C1-C6 alkylthio group, a C1-C6 haloalkylthio group, a C1-C6 alkylsulfinyl group, a C1-C6 haloalkylsulfinyl group, a C1-C6 alkylsulfonyl group, a C1-C6 haloalkylsulfonyl group, a C2-C7 alkylcarbonyl group, a C2-C7 haloalkylcarbonyl group, a C3-C7 alkenylcarbonyl group, a C3-C7 haloalkenylcarbonyl group, a C3-C7 alkynylcarbonyl group, a C3-C7 haloalkynylcarbonyl group, a C4-C10 cycloalkylcarbonyl group, a C4-C10 halocycloalkylcarbonyl group, a C2-C7 alkylcarbonyloxy group, a C2-C7 haloalkylcarbonyloxy group, a C1-C6 alkylsulfonyloxy group, a C1-C6 haloalkylsulfonyloxy group, a C2-C7 alkoxycarbonyl group, a C2-C7 haloalkoxycarbonyl group, a C3-C7 alkenyloxycarbonyl group, a C3-C7 haloalkenyloxycarbonyl group, a C3-C7 alkynyloxycarbonyl group, a C3-C7 haloalkynyloxycarbonyl group, a C4-C10 cycloalkyloxycarbonyl group, a C4-C10 halocycloalkyloxycarbonyl group, a C2-C7 alkylcarbonylamino group, a C2-C7 haloalkylcarbonylamino group, a C2-C7 alkoxycarbonylamino group, a C2-C7 haloalkoxycarbonylamino group, a C1-C6 alkylamino group, a C1-C6 haloalkylamino group, a C2-C6 alkenylamino group, a C2-C6 haloalkenylamino group, a C2-C6 alkynylamino group, a C2-C6 haloalkynylamino group, a C3-C9 cycloalkylamino group, a C3-C9 halocycloalkylamino group, a C2-C7 alkylaminocarbonyl group, a C2-C7 haloalkylaminocarbonyl group, a C3-C7 alkenylaminocarbonyl group, a C3-C7 haloalkenylaminocarbonyl group, a C3-C7 alkynylaminocarbonyl group, a C3-C7 haloalkynylaminocarbonyl group, a C4-C10 cycloalkylaminocarbonyl group, a C4-C10 halocycloalkylaminocarbonyl group, an amino group, a carbamoyl group, a cyano group, a nitro group, a hydroxy group, a pentafluorosulfanyl group, a phenyl group which may have a substituent, and a heterocyclic group which may have a substituent, and when there are two or more substituents, the substituents may be the same as or different from each other;
   wherein, the heterocyclic group in X, Q₁, and Q₂ represents a pyridyl group, a pyridine-N-oxide group, a pyrimidinyl group, a pyrazinyl group, a pyridazyl group, a furyl group, a thienyl group, an oxazolyl group, an isoxazolyl group, an oxadiazolyl group, a thiazolyl group, an isothiazolyl group, a thiadiazolyl group, a pyrrolyl group, an imidazolyl group, a triazolyl group, a pyrazolyl group, or a tetrazolyl group;
   G₃ represents an oxygen atom or a sulfur atom; and
   R₁ and R₂ each independently represents a hydrogen atom, an oxygen atom, a halogen atom, a hydroxy group, a nitro group, a nitroso group, a trimethylsilyl group, a t-butyldimethylsilyl group, a cyano group, an amino group, a C1-C6 alkyl group which may have a substituent, a C1-C6 haloalkyl group which may have a substituent, a C2-C7 alkylcarbonyl group, a C2-C7 haloalkylcarbonyl group, a C2-C6 alkenyl group which may have a substituent, a C2-C6 haloalkenyl group which may have a substituent, a C2-C6 alkynyl group which may have a substituent, a C2-C6 haloalkynyl group which may have a substituent, a C2-C7 alkoxycarbonyl group, a C2-C7 haloalkoxycarbonyl group, a C3-C7 alkenyloxycarbonyl group, a C3-C7 haloalkenyloxycarbonyl group, a C3-C7 alkynyloxycarbonyl group, a C3-C7 haloalkynyloxycarbonyl group, a phenoxycarbonyl group, a C2-C7 alkylaminocarbonyl group, a C2-C7 haloalkylaminocarbonyl group, a C2-C7 alkylcarbonyloxy group, a C2-C7 haloalkylcarbonyloxy group, a benzoyl group, a C1-C6 alkoxy group, a C1-C6 haloalkoxy group, a C1-C6 alkylthio group, a C1-C6 haloalkylthio group, a C1-C6 alkylsulfinyl group, a C1-C6 haloalkylsulfinyl group, a C1-C6 alkylsulfonyl group, a C1-C6 haloalkylsulfonyl group, a benzenesulfonyl group, a benzylsulfonyl group, a benzyl group, or -C(=O)C(=O)R₇, wherein R₇ represents a C1-C6 alkyl group, a C1-C6 haloalkyl group, a C1-C6 alkoxy group, or a C1-C6 haloalkoxy group; 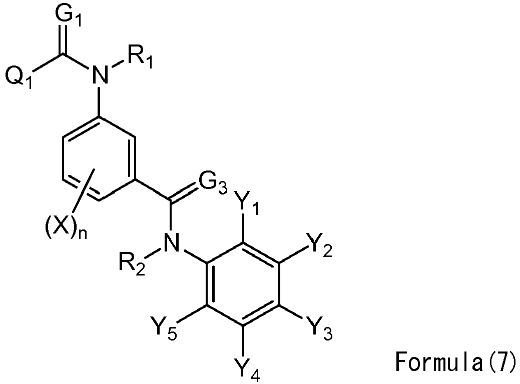
   wherein in Formula (7), n represents 4;
   X's each independently represent a hydrogen atom, a halogen atom, or cyano group;
   Y₃ represents a C2-C4 perfluoroalkyl group; Y₂ and Y₄ represent a hydrogen atom;
   Y₁ and Y₅ represent a halogen atom or a C1-C3 haloalkyl group, and either Y₁ or Y₅ represents a C1-C3 haloalkyl group;
   G₁ and G₃ each independently represent an oxygen atom or a sulfur atom;
   Q₁ represents a hydrogen atom, a C1-C6 alkyl group, a C1-C6 haloalkyl group, a C2-C6 alkenyl group, a C2-C6 haloalkenyl group, a C2-C6 alkynyl group, a C2-C6 haloalkynyl group, a C3-C9 cycloalkyl group, a C3-C9 halocycloalkyl group, a benzyl group, a phenyl group which may have a substituent, a naphthyl group, or a heterocyclic group which may have a substituent;
   wherein, in Q₁ the substituent of a phenyl group which may have a substituent and a substituents heterocyclic group which may have a substituent represents one or more substituents selected from a group consisting of a halogen atom, a C1-C6 alkyl group, a C1-C6 haloalkyl group, a C3-C9 cycloalkyl group, a C3-C9 halocycloalkyl group, a C2-C6 alkenyl group, a C2-C6 haloalkenyl group, a C2-C6 alkynyl group, a C2-C6 haloalkynyl group, a C1-C6 alkoxy group, a C1-C6 haloalkoxy group, a C2-C6 alkenyloxy group, a C2-C6 haloalkenyloxy group, a C2-C6 alkynyloxy group, a C2-C6 haloalkynyloxy group, a C3-C9 cycloalkoxy group, a C3-C9 halocycloalkoxy group, a C1-C6 alkylthio group, a C1-C6 haloalkylthio group, a C1-C6 alkylsulfinyl group, a C1-C6 haloalkylsulfinyl group, a C1-C6 alkylsulfonyl group, a C1-C6 haloalkylsulfonyl group, a C2-C7 alkylcarbonyl group, a C2-C7 haloalkylcarbonyl group, a C3-C7 alkenylcarbonyl group, a C3-C7 haloalkenylcarbonyl group, a C3-C7 alkynylcarbonyl group, a C3-C7 haloalkynylcarbonyl group, a C4-C10 cycloalkylcarbonyl group, a C4-C10 halocycloalkylcarbonyl group, a C2-C7 alkylcarbonyloxy group, a C2-C7 haloalkylcarbonyloxy group, a C1-C6 alkylsulfonyloxy group, a C1-C6 haloalkylsulfonyloxy group, a C2-C7 alkoxycarbonyl group, a C2-C7 haloalkoxycarbonyl group, a C3-C7 alkenyloxycarbonyl group, a C3-C7 haloalkenyloxycarbonyl group, a C3-C7 alkynyloxycarbonyl group, a C3-C7 haloalkynyloxycarbonyl group, a C4-C10 cycloalkyloxycarbonyl group, a C4-C10 halocycloalkyloxycarbonyl group, a C2-C7 alkylcarbonylamino group, a C2-C7 haloalkylcarbonylamino group, a C2-C7 alkoxycarbonylamino group, a C2-C7 haloalkoxycarbonylamino group, a C1-C6 alkylamino group, a C1-C6 haloalkylamino group, a C2-C6 alkenylamino group, a C2-C6 haloalkenylamino group, a C2-C6 alkynylamino group, a C2-C6 haloalkynylamino group, a C3-C9 cycloalkylamino group, a C3-C9 halocycloalkylamino group, a C2-C7 alkylaminocarbonyl group, a C2-C7 haloalkylaminocarbonyl group, a C3-C7 alkenylaminocarbonyl group, a C3-C7 haloalkenylaminocarbonyl group, a C3-C7 alkynylaminocarbonyl group, a C3-C7 haloalkynylaminocarbonyl group, a C4-C10 cycloalkylaminocarbonyl group, a C4-C10 halocycloalkylaminocarbonyl group, an amino group, a carbamoyl group, a cyano group, a nitro group, a hydroxy group, a pentafluorosulfanyl group, a phenyl group which may have a substituent, and a heterocyclic group which may have a substituent, and when there are two or more substituents, the substituents may be the same as or different from each other;
   wherein, the heterocyclic group in Qi represents a pyridyl group, a pyridine-N-oxide group, a pyrimidinyl group, a pyrazinyl group, a pyridazyl group, a furyl group, a thienyl group, an oxazolyl group, an isoxazolyl group, an oxadiazolyl group, a thiazolyl group, an isothiazolyl group, a thiadiazolyl group, a pyrrolyl group, an imidazolyl group, a triazolyl group, a pyrazolyl group, or a tetrazolyl group;
   R₁ and R₂ each independently represents a hydrogen atom, an oxygen atom, a halogen atom, a hydroxy group, a nitro group, a nitroso group, a trimethylsilyl group, a t-butyldimethylsilyl group, a cyano group, an amino group, a C1-C6 alkyl group which may have a substituent, a C1-C6 haloalkyl group which may have a substituent, a C2-C7 alkylcarbonyl group, a C2-C7 haloalkylcarbonyl group, a C2-C6 alkenyl group which may have a substituent, a C2-C6 haloalkenyl group which may have a substituent, a C2-C6 alkynyl group which may have a substituent, a C2-C6 haloalkynyl group which may have a substituent, a C2-C7 alkoxycarbonyl group, a C2-C7 haloalkoxycarbonyl group, a C3-C7 alkenyloxycarbonyl group, a C3-C7 haloalkenyloxycarbonyl group, a C3-C7 alkynyloxycarbonyl group, a C3-C7 haloalkynyloxycarbonyl group, a phenoxycarbonyl group, a C2-C7 alkylaminocarbonyl group, a C2-C7 haloalkylaminocarbonyl group, a C2-C7 alkylcarbonyloxy group, a C2-C7 haloalkylcarbonyloxy group, a benzoyl group, a C1-C6 alkoxy group, a C1-C6 haloalkoxy group, a C1-C6 alkylthio group, a C1-C6 haloalkylthio group, a C1-C6 alkylsulfinyl group, a C1-C6 haloalkylsulfinyl group, a C1-C6 alkylsulfonyl group, a C1-C6 haloalkylsulfonyl group, a benzenesulfonyl group, a benzylsulfonyl group, a benzyl group, or -C(=O)C(=O)R₇, wherein R₇ represents a C1-C6 alkyl group, a C1-C6 haloalkyl group, a C1-C6 alkoxy group, or a C1-C6 haloalkoxy group; 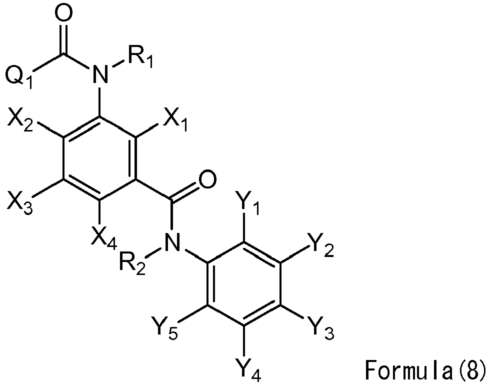
   wherein in Formula (8), Q₁ represents a phenyl group or a pyridyl group which may have a substituent selected from a group consisting of: a halogen atom, C1 haloalkyl group, substituent nitro group, and a cyano group, in a case where Q₁ has the substituent the number of the substituents is 1 or 2;
   X₁ and X₂ each independently represent a hydrogen atom or a fluorine atom, and X₃ and X₄ represent a hydrogen atom;
   R₁ and R₂ each independently represent a hydrogen atom or a methyl group;
   Y₁ and Y₅ each independently represent a chlorine atom, a bromine atom, an iodine atom, a trifluoromethoxy group, a trifluoromethyl group, or a pentafluoroethyl group;
   Y₂ and Y₄ represent a hydrogen atom; and Y₃ represents a C3-C4 perfluoroalkyl group; in a case where Y₁ and Y₅ represent halogen atoms simultaneously, at least one of X₁ or X₂ represents a fluorine atom; and in a case where Y₁ or Y₅ represents a trifluoromethoxy group, X₂ represents a fluorine atom; 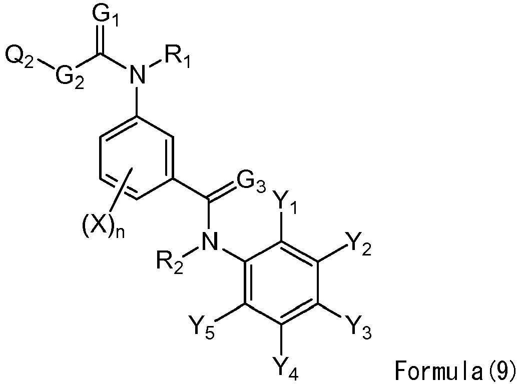
   wherein in Formula (9), n represents 4;
   four X's each independently represent a hydrogen atom, a halogen atom, or cyano group;
   Y₃ represents a C2-C4 perfluoroalkyl group; Y₂ and Y₄ represent a hydrogen atom; Y₁ and Y₅ each independently represent a halogen atom or a C1-C3 haloalkyl group, and either Y₁ or Y₅ represents a C1-C3 haloalkyl group;
   G₁, G₂ and G₃ each independently represent an oxygen atom or a sulfur atom;
   Q₂ represents a hydrogen atom, a C1-C6 alkyl group, a C1-C6 haloalkyl group, a C2-C6 alkenyl group, a C2-C6 haloalkenyl group, a C2-C6 alkynyl group, a C2-C6 haloalkynyl group, a C3-C9 cycloalkyl group, a C3-C9 halocycloalkyl group, a benzyl group, a phenyl group which may have a substituent, a naphthyl group, or a heterocyclic group which may have a substituent;
   wherein, in Q₂ the substituent of a phenyl group which may have a substituent and a heterocyclic group which may have a substituent represents one or more substituents selected from a group consisting of a halogen atom, a C1-C6 alkyl group, a C1-C6 haloalkyl group, a C3-C9 cycloalkyl group, a C3-C9 halocycloalkyl group, a C2-C6 alkenyl group, a C2-C6 haloalkenyl group, a C2-C6 alkynyl group, a C2-C6 haloalkynyl group, a C1-C6 alkoxy group, a C1-C6 haloalkoxy group, a C2-C6 alkenyloxy group, a C2-C6 haloalkenyloxy group, a C2-C6 alkynyloxy group, a C2-C6 haloalkynyloxy group, a C3-C9 cycloalkoxy group, a C3-C9 halocycloalkoxy group, a C1-C6 alkylthio group, a C1-C6 haloalkylthio group, a C1-C6 alkylsulfinyl group, a C1-C6 haloalkylsulfinyl group, a C1-C6 alkylsulfonyl group, a C1-C6 haloalkylsulfonyl group, a C2-C7 alkylcarbonyl group, a C2-C7 haloalkylcarbonyl group, a C3-C7 alkenylcarbonyl group, a C3-C7 haloalkenylcarbonyl group, a C3-C7 alkynylcarbonyl group, a C3-C7 haloalkynylcarbonyl group, a C4-C10 cycloalkylcarbonyl group, a C4-C10 halocycloalkylcarbonyl group, a C2-C7 alkylcarbonyloxy group, a C2-C7 haloalkylcarbonyloxy group, a C1-C6 alkylsulfonyloxy group, a C1-C6 haloalkylsulfonyloxy group, a C2-C7 alkoxycarbonyl group, a C2-C7 haloalkoxycarbonyl group, a C3-C7 alkenyloxycarbonyl group, a C3-C7 haloalkenyloxycarbonyl group, a C3-C7 alkynyloxycarbonyl group, a C3-C7 haloalkynyloxycarbonyl group, a C4-C10 cycloalkyloxycarbonyl group, a C4-C10 halocycloalkyloxycarbonyl group, a C2-C7 alkylcarbonylamino group, a C2-C7 haloalkylcarbonylamino group, a C2-C7 alkoxycarbonylamino group, a C2-C7 haloalkoxycarbonylamino group, a C1-C6 alkylamino group, a C1-C6 haloalkylamino group, a C2-C6 alkenylamino group, a C2-C6 haloalkenylamino group, a C2-C6 alkynylamino group, a C2-C6 haloalkynylamino group, a C3-C9 cycloalkylamino group, a C3-C9 halocycloalkylamino group, a C2-C7 alkylaminocarbonyl group, a C2-C7 haloalkylaminocarbonyl group, a C3-C7 alkenylaminocarbonyl group, a C3-C7 haloalkenylaminocarbonyl group, a C3-C7 alkynylaminocarbonyl group, a C3-C7 haloalkynylaminocarbonyl group, a C4-C10 cycloalkylaminocarbonyl group, a C4-C10 halocycloalkylaminocarbonyl group, an amino group, a carbamoyl group, a cyano group, a nitro group, a hydroxy group, a pentafluorosulfanyl group, a phenyl group which may have a substituent, and a heterocyclic group which may have a substituent, and when there are two or more substituents, the substituents may be the same as or different from each other;
   wherein, the heterocyclic group in Q₂ represents a pyridyl group, a pyridine-N-oxide group, a pyrimidinyl group, a pyrazinyl group, a pyridazyl group, a furyl group, a thienyl group, an oxazolyl group, an isoxazolyl group, an oxadiazolyl group, a thiazolyl group, an isothiazolyl group, a thiadiazolyl group, a pyrrolyl group, an imidazolyl group, a triazolyl group, a pyrazolyl group, or a tetrazolyl group;
   R₁ and R₂ each independently represent a hydrogen atom, an oxygen atom, a halogen atom, a hydroxy group, a nitro group, a nitroso group, a trimethylsilyl group, a t-butyldimethylsilyl group, a cyano group, an amino group, a C1-C6 alkyl group which may have a substituent, a C1-C6 haloalkyl group which may have a substituent, a C2-C7 alkylcarbonyl group, a C2-C7 haloalkylcarbonyl group, a C2-C6 alkenyl group which may have a substituent, a C2-C6 haloalkenyl group which may have a substituent, a C2-C6 alkynyl group which may have a substituent, a C2-C6 haloalkynyl group which may have a substituent, a C2-C7 alkoxycarbonyl group, a C2-C7 haloalkoxycarbonyl group, a C3-C7 alkenyloxycarbonyl group, a C3-C7 haloalkenyloxycarbonyl group, a C3-C7 alkynyloxycarbonyl group, a C3-C7 haloalkynyloxycarbonyl group, a phenoxycarbonyl group, a C2-C7 alkylaminocarbonyl group, a C2-C7 haloalkylaminocarbonyl group, a C2-C7 alkylcarbonyloxy group, a C2-C7 haloalkylcarbonyloxy group, a benzoyl group, a C1-C6 alkoxy group, a C1-C6 haloalkoxy group, a C1-C6 alkylthio group, a C1-C6 haloalkylthio group, a C1-C6 alkylsulfinyl group, a C1-C6 haloalkylsulfinyl group, a C1-C6 alkylsulfonyl group, a C1-C6 haloalkylsulfonyl group, a benzenesulfonyl group, a benzylsulfonyl group, a benzyl group, or -C(=O)C(=O)R₇, wherein R₇ represents a C1-C6 alkyl group, a C1-C6 haloalkyl group, a C1-C6 alkoxy group, or a C1-C6 haloalkoxy group,
   wherein the pest is selected from the group consisting of:
      Siphonaptera insects such as *Ctenocephalides felis, Ctenocephalides canis, Echidnophaga gallinacea, Pulex irritans, and Xenopsylla cheopis;*
      mallophaga insects such as *Menacanthus stramineus, and Bovicola bovis;*
      anoplura insects such as *Haematopinus eurysternus, Haematopinus suis, Linognathus vituli, and Solenopotes capillatus*;
      ixodidae such as *Boophilus microplus, Rhipicephalus sanguineus, Haemaphysalis longicornis, Haemaphysalis flava, Haemaphysalis campanulata, Ixodes ovatus, Ixodes persulcatus, Amblyomma spp., and Dermacentor spp.;*
      cheyletidae such as *Cheyletiella yasguri, and Cheyletiella blakei*;
      demodicidae such as *Demodex canis, Demodex cati;*
      psoroptidae such as *Psoroptes ovis;* and
      sarcoptidae such as *Sarcoptes scabiei, Notoedres cati, and Knemidocoptes spp.*
2. A composition comprising a pest control agent comprising at least one amide derivative according to Formula (1), Formula (7), Formula (8) or Formula (9), as defined in 1.
3. A pest controlling method comprising applying the pest control agent according to 1, or the composition as defined in 2 to a pest as defined in 1, wherein the method is not a method of treatment.
4. A pest control agent comprising at least one amide derivative according to Formula (1), Formula (7), Formula (8) and Formula (9), as defined in 1, or a composition as defined in 2, for use in a method of treatment of the human or animal body.
5. A pest control agent comprising at least one amide derivative according to Formula (1), Formula (7), Formula (8) and Formula (9), as defined in 1, or a composition as defined in 2, for use in a method of treatment of the human or animal body,
   wherein the method is for exterminating a pest selected from the group consisting of:
   Siphonaptera insects such as *Ctenocephalides felis, Ctenocephalides canis, Echidnophaga gallinacea, Pulex irritans, and Xenopsylla cheopis*;
   mallophaga insects such as *Menacanthus stramineus, and Bovicola bovis;*
   anoplura insects such as *Haematopinus eurysternus, Haematopinus suis, Linognathus vituli, and Solenopotes capillatus*;
   ixodidae such as *Boophilus microplus, Rhipicephalus sanguineus, Haemaphysalis longicornis, Haemaphysalis flava, Haemaphysalis campanulata, Ixodes ovatus, Ixodes persulcatus, Amblyomma spp., and Dermacentor spp.;*
   cheyletidae such as *Cheyletiella yasguri, and Cheyletiella blakei*;
   demodicidae such as *Demodex canis, Demodex cati;*
   psoroptidae such as *Psoroptes ovis;* and
   sarcoptidae such as *Sarcoptes scabiei, Notoedres cati, and Knemidocoptes spp.*
6. Use of a pest control agent comprising at least one amide derivative according to Formula (1), Formula (7), Formula (8) and Formula (9), as defined in 1, or a composition as defined in 2, as a pest control agent, wherein the use is not a method of treatment of the human or animal body.
7. The use according to 6, wherein the use is for the control of a pest selected from the group consisting of:
   Siphonaptera insects such as *Ctenocephalides felis, Ctenocephalides canis, Echidnophaga gallinacea, Pulex irritans, and Xenopsylla cheopis;*
   mallophaga insects such as *Menacanthus stramineus, and Bovicola bovis;*
   anoplura insects such as *Haematopinus eurysternus, Haematopinus suis, Linognathus vituli, and Solenopotes capillatus*;
   ixodidae such as *Boophilus microplus, Rhipicephalus sanguineus, Haemaphysalis longicornis, Haemaphysalis flava, Haemaphysalis campanulata, Ixodes ovatus, Ixodes persulcatus, Amblyomma spp., and Dermacentor spp.;*
   cheyletidae such as *Cheyletiella yasguri, and Cheyletiella blakei*;
   demodicidae such as *Demodex canis, Demodex cati;*
   psoroptidae such as *Psoroptes ovis;* and
   sarcoptidae such as *Sarcoptes scabiei, Notoedres cati, and Knemidocoptes spp.*

In Formula (8), Q₁ may represent a phenyl group, a 2-fluorophenyl group, a 3-fluorophenyl group, a 4-fluorophenyl group, a 2-chlorophenyl group, a 3-chlorophenyl group, a 4-chlorophenyl group, a 2-bromophenyl group, a 3-bromophenyl group, a 4-bromophenyl group, a 2-iodophenyl group, a 3-iodophenyl group, a 4-iodophenyl group, a (2-trifluoromethyl)phenyl group, a (3-trifluoromethyl)phenyl group, a (4-trifluoromethyl)phenyl group, a 2-nitrophenyl group, a 3-nitrophenyl group, a 4-nitrophenyl group, a 2-cyanophenyl group, a 3-cyanophenyl group, a 4-cyanophenyl group, a 2,6-difluorophenyl group, a 3,4-dichlorophenyl group, a 2,4-dichlorophenyl group, a 2-chloro-4-fluorophenyl group, a 4-bromo-2-chlorophenyl group, a 2-bromo-4-chlorophenyl group, a 2-bromo-4-fluorophenyl group, a 2-chloro-4-nitrophenyl group, a 3, 5-dicyanophenyl group, a 4-cyano-2-fluorophenyl group, a 2-chloro-4-cyanophenyl group, a pyridin-3-yl group, a 2-fluoropyridin-3-yl group, a 2-chloropyridin-3-yl group, a 2-bromopyridin-3-yl group, a 2-iodopyridin-3-yl group, a 2-(trifluoromethyl)pyridin-3-yl group, a 2-nitropyridin-3yl group, a 2-cyanopyridin-3-yl group, a 6-fluoropyridin-3-yl group, a 6-chloropyridin-3-yl group, a 6-bromopyridin-3-yl group, a 6-iodopyridin-3-yl group, a 6-(trifluoromethyl)pyridin-3-yl group, a 6-nitropyridin-3-yl group, a 6-cyanopyridin-3-yl group, a 5-fluoropyridin-3-yl group, a 5-chloropyridin-3-yl group, a 5-bromopyridin-3-yl group, a 5-iodopyridin-3-yl group, a 5-(trifluoromethyl)pyridin-3-yl group, a 5-nitropyridin-3-yl group, a 5-cyanopyridin-3-yl group, a 4-fluoropyridin-3-yl group, a 4-chloropyridin-3-yl group, a 4-bromopyridin-3-yl group, a 4-iodopyridin-3-yl group, a 4-(trifluoromethyl)pyridin-3-yl group, a 4-nitropyridin-3-yl group, a 4-cyanopyridin-3-yl group, a 2,6-dichloropyridin-3-yl group, a pyridin-4-yl group, a 2-chloropyridin-4-yl group, a 3-bromopyridin-4-yl group, a 3,5-dichloropyridin-4-yl group, a 3-(trifluoromethyl)pyridin-4-yl group, a 2,6-dicyanopyridin-4-yl group, a pyridin-2-yl group, a 3-chloropyridin-2-yl group, a 4-bromopyridin-2-yl group, a 5-iodopyridin-2-yl group, a 6-chloropyridin-2-yl group, or a 4-cyanopyridin-2-yl group,
The compound represented by Formula (8) may be 3-benzamido-N-(2-bromo-6-chloro-4-(perfluoropropan-2-yl)phenyl)-2-fluorobenzamide, 2-chloro-N-(3-(2,6-dibromo-4-(perfluoropropan-2-yl)phenylcarbamoyl)-2-fluorophenyl)nicotinamide,3-(4-cyano-N-methylbenzamido)-N-(2,6-dibromo-4-(perfluoropropan-2-yl)phenyl)-2-fluorobenzamide, 2-chloro-N-(3-(2,6-dibromo-4-(perfluoropropan-2-yl)phenylcarbamoyl)-2-fluorophenyl)-N-methylnicotinamide, 3-benzamido-N-(2,6-diiodo-4-(perfluoropropan-2-yl)phenyl)-₂-fluorobenzamide, 3-benzamido-N-(2,6-dibromo-4-(perfluoropropan-2-yl)phenyl)-2-fluorobenzamide, N-(2,6-dibromo-4-(perfluoropropan-2-yl)phenyl)-2-fluoro-3-(N-methylbenzamido)benzamide, 6-chloro-N-(3-(2,6-dibromo-4-(perfluoropropan-2-yl)phenylcarbamoyl)-2-fluorophenyl)-N-methylnicotinamide, 3-(3-cyano-N-methylbenzamido)-N-(2,6-dibromo-4-(perfluoropropan-2-yl)phenyl)-2-fluorobenzamide, N-(2,6-dichloro-4-(perfluoropropan-2-yl)phenyl)-2-fluoro-3-(N-methylbenzamido)benzamide, 3-(4-cyano-N-methylbenzamido)-N-(2,6-dichloro-4-(perfluoropropan-2-yl)phenyl)-2-fluorobenzamide, 2-chloro-N-(3-(2,6-dichloro-4-(perfluoropropan-2-yl)phenylcarbamoyl)-2-fluorophenyl)-N-methylnicotinamide, N-(2,6-diiodo-4-(perfluoropropan-2-yl)phenyl)-2-fluoro-3-(N-methylbenzamido)benzamide, 3-(4-cyano-N-methylbenzamido)-N-(2,6-diiodo-4-(perfluoropropan-2-yl)phenyl)-2-fluorobenzamide, 3-benzamido-N-(2,6-dibromo-4-(perfluorobutan-2-yl)phenyl)-2-fluorobenzamide, N-(2,6-diiodo-4-(perfluoropropan-2-yl)phenyl)-2-fluoro-3-(2-fluorobenzamido)benzamide, N-(2,6-diiodo-4-(perfluoropropan-2-yl)phenyl)-2-fluoro-3-(4-fluorobenzamido)benzamide, 3-(2,6-difluorobenzamido)-N-(2,6-diiodo-4-(perfluoropropan-2-yl)phenyl)-2-fluorobenzamide, N-(2-bromo-6-iodo-4-(perfluoropropan-2-yl)phenyl)-2-fluoro-3-(N-methylbenzamido)benzamide, N-(3-(2-bromo-6-iodo-4-(perfluoropropan-2-yl)phenylcarbamoyl)-2-fluorophenyl)-2-chloro-N-methylnicotinamide,
3-(4-cyanobenzamido)-N-(2,6-dibromo-4-(perfluorobutan-2-yl)phenyl)-2-fluorobenzamide, 3-(3-cyanobenzamido)-N-(2,6-dibromo-4-(perfluorobutan-2-yl)phenyl)-2-fluorobenzamide, N-(2,6-dibromo-4-(perfluorobutan-2-yl)phenyl)-2-fluoro-3-(N-methylbenzamido)benzamide, 3-(4-cyano-N-methylbenzamido)-N-(2,6-dibromo-4-(perfluorobutan-2-yl)phenyl)-2-fluorobenzamide, 3-(3-cyano-N-methylbenzamido)-N-(2,6-dibromo-4-(perfluorobutan-2-yl)phenyl)-2-fluorobenzamide, 6-chloro-N-(3-(2,6-dibromo-4-(perfluorobutan-2-yl)phenylcarbamoyl)-2-fluorophenyl)-N-methylnicotinamide, 3-benzamido-N-(2,6-diiodo-4-(perfluorobutan-2-yl)phenyl)-2-fluorobenzamide, 3-(3-cyanobenzamido)-N-(2,6-diiodo-4-(perfluorobutan-2-yl)phenyl)-2-fluorobenzamide, 3-(4-cyanobenzamido)-N-(2,6-diiodo-4-(perfluorobutan-2-yl)phenyl)-2-fluorobenzamide, N-(2,6-diiodo-4-(perfluorobutan-2-yl)phenyl)-2-fluoro-3-(N-methylbenzamido)benzamide, 3-(3-cyano-N-methylbenzamido)-N-(2,6-diiodo-4-(perfluorobutan-2-yl)phenyl)-2-fluorobenzamide, 3-(4-cyano-N-methylbenzamido)-N-(2,6-diiodo-4-(perfluorobutan-2-yl)phenyl)-2-fluorobenzamide, 3-benzamido-N-(2,6-diiodo-4-(perfluorobutan-2-yl)phenyl)benzamide, 2-chloro-N-(3-(2,6-diiodo-4-(perfluorobutan-2-yl)phenylcarbamoyl)phenyl)nicotinamide, 6-chloro-N-(3-(2,6-diiodo-4-(perfluorobutan-2-yl)phenylcarbamoyl)phenyl)nicotinamide, 3-cyano-N-(3-(2,6-diiodo-4-(perfluorobutan-2-yl)phenylcarbamoyl)phenyl)benzamide, 3-(4-cyanobenzamido)-N-(2,6-diiodo-4-(perfluorobutan-2-yl)phenyl)benzamide, N-(3-(2,6-diiodo-4-(perfluorobutan-2-yl)phenylcarbamoyl)phenyl)-2-fluorobenzamide, N-(2,6-diiodo-4-(perfluorobutan-2-yl)phenyl)-3-(3-fluorobenzamido)benzamide, N-(2,6-diiodo-4-(perfluorobutan-2-yl)phenyl)-3-(4-fluorobenzamido)benzamide,
N-(3-(2,6-diiodo-4-(perfluorobutan-2-yl)phenylcarbamoyl)phenyl)-2,6-difluorobenzamide, N-(3-(2,6-diiodo-4-(perfluorobutan-2-yl)phenylcarbamoyl)phenyl)-N-methylbenzamide, 2-chloro-N-(3-(2,6-diiodo-4-(perfluorobutan-2-yl)phenylcarbamoyl)phenyl)-N-methylnicotinamide, 6-chloro-N-(3-(2,6-diiodo-4-(perfluorobutan-2-yl)phenylcarbamoyl)phenyl)-N-methylnicotinamide, 3-cyano-N-(3-(2,6-diiodo-4-(perfluorobutan-2-yl)phenylcarbamoyl)phenyl)-N-methylbenzamide, 4-cyano-N-(3-(2,6-diiodo-4-(perfluorobutan-2-yl)phenylcarbamoyl)phenyl)-N-methylbenzamide, N-(3-(2,6-diiodo-4-(perfluorobutan-2-yl)phenylcarbamoyl)phenyl)-2-fluoro-N-methylbenzamide, N-(3-(2,6-diiodo-4-(perfluorobutan-2-yl)phenylcarbamoyl)phenyl)-3-fluoro-N-methylbenzamide, N-(3-(2,6-diiodo-4-(perfluorobutan-2-yl)phenylcarbamoyl)phenyl)-4-fluoro-N-methylbenzamide, N-(3-(2,6-diiodo-4-(perfluorobutan-2-yl)phenylcarbamoyl)phenyl)-2,6-difluoro-N-methylbenzamide, 2-chloro-N-(3-(2,6-diiodo-4-(perfluorobutan-2-yl)phenylcarbamoyl)-2-fluorophenyl)nicotinamide, 6-chloro-N-(3-(2,6-diiodo-4-(perfluorobutan-2-yl)phenylcarbamoyl)-2-fluorophenyl)nicotinamide, N-(2,6-diiodo-4-(perfluorobutan-2-yl)phenyl)-2-fluoro-3-(2-fluorobenzamido)benzamide, N-(2,6-diiodo-4-(perfluorobutan-2-yl)phenyl)-2-fluoro-3-(3-fluorobenzamido)benzamide, N-(2,6-diiodo-4-(perfluorobutan-2-yl)phenyl)-2-fluoro-3-(4-fluorobenzamido)benzamide, 3-(2,6-difluorobenzamido)-N-(2,6-diiodo-4-(perfluorobutan-2-yl)phenyl)-2-fluorobenzamide, 3-benzamido-N-(2,6-dibromo-4-(perfluorobutan-2-yl)phenyl)benzamide, 6-chloro-N-(3-(2,6-dibromo-4-(perfluorobutan-2-yl)phenylcarbamoyl)phenyl)nicotinamide, N-(3-(2,6-dibromo-4-(perfluorobutan-2-yl)phenylcarbamoyl)phenyl)-2-fluorobenzamide, 2-chloro-N-(3-(2,6-dibromo-4-(perfluorobutan-2-yl)phenylcarbamoyl)phenyl)nicotinamide,
N-(2,6-dibromo-4-(perfluorobutan-2-yl)phenyl)-3-(3-fluorobenzamido)benzamide, N-(2,6-dibromo-4-(perfluorobutan-2-yl)phenyl)-3-(4-fluorobenzamido)benzamide, N-(3-(2,6-dibromo-4-(perfluorobutan-2-yl)phenylcarbamoyl)phenyl)-2,6-difluorobenzamide, 3-cyano-N-(3-(2,6-dibromo-4-(perfluorobutan-2-yl)phenylcarbamoyl)phenyl)benzamide, 3-(4-cyanobenzamido)-N-(2,6-dibromo-4-(perfluorobutan-2-yl)phenyl)benzamide, 2-chloro-N-(3-(2,6-diiodo-4-(perfluorobutan-2-yl)phenylcarbamoyl)-2-fluorophenyl)-N-methylnicotinamide, 6-chloro-N-(3-(2,6-diiodo-4-(perfluorobutan-2-yl)phenylcarbamoyl)-2-fluorophenyl)-N-methylnicotinamide, N-(3-(2,6-diiodo-4-(perfluorobutan-2-yl)phenylcarbamoyl)-2-fluorophenyl)-2-fluoro-N-methylbenzamide, N-(2,6-diiodo-4-(perfluorobutan-2-yl)phenyl)-2-fluoro-3-(3-fluoro-N-methylbenzamido)benzamide, N-(2,6-diiodo-4-(perfluorobutan-2-yl)phenyl)-2-fluoro-3-(4-fluoro-N-methylbenzamido)benzamide, N-(3-(2,6-diiodo-4-(perfluorobutan-2-yl)phenylcarbamoyl)-2-fluorophenyl)-2,6-difluoro-N-methylbenzamide, N-(3-(2,6-dibromo-4-(perfluorobutan-2-yl)phenylcarbamoyl)phenyl)-N-methylbenzamide, 2-chloro-N-(3-(2,6-dibromo-4-(perfluorobutan-2-yl)phenylcarbamoyl)phenyl)-N-methylnicotinamide, 6-chloro-N-(3-(2,6-dibromo-4-(perfluorobutan-2-yl)phenylcarbamoyl)phenyl)-N-methylnicotinamide, 3-cyano-N-(3-(2,6-dibromo-4-(perfluorobutan-2-yl)phenylcarbamoyl)phenyl)-N-methylbenzamide, 4-cyano-N-(3-(2,6-dibromo-4-(perfluorobutan-2-yl)phenylcarbamoyl)phenyl)-N-methylbenzamide, N-(3-(2,6-dibromo-4-(perfluorobutan-2-yl)phenylcarbamoyl)phenyl)-2-fluoro-N-methylbenzamide, N-(3-(2,6-dibromo-4-(perfluorobutan-2-yl)phenylcarbamoyl)phenyl)-3-fluoro-N-methylbenzamide, N-(3-(2,6-dibromo-4-(perfluorobutan-2-yl)phenylcarbamoyl)phenyl)-4-fluoro-N-methylbenzamide, N-(3-(2,6-dibromo-4-(perfluorobutan-2-yl)phenylcarbamoyl)phenyl)-2,6-difluoro-N-methylbenzamide,
2-chloro-N-(3-(2,6-dibromo-4-(perfluorobutan-2-yl)phenylcarbamoyl)-2-fluorophenyl)nicotinamide, 6-chloro-N-(3-(2,6-dibromo-4-(perfluorobutan-2-yl)phenylcarbamoyl)-2-fluorophenyl)nicotinamide, N-(2,6-dibromo-4-(perfluorobutan-2-yl)phenyl)-2-fluoro-3-(2-fluorobenzamido)benzamide, N-(2,6-dibromo-4-(perfluorobutan-2-yl)phenyl)-2-fluoro-3-(3-fluorobenzamido)benzamide, N-(2,6-dibromo-4-(perfluorobutan-2-yl)phenyl)-2-fluoro-3-(4-fluorobenzamido)benzamide, N-(2,6-dibromo-4-(perfluorobutan-2-yl)phenyl)-3-(2,6-difluorobenzamido)-2-fluorobenzamide, 2-chloro-N-(3-(2,6-diiodo-4-(perfluoropropan-2-yl)phenylcarbamoyl)-2-fluorophenyl)nicotinamide, 6-chloro-N-(3-(2,6-diiodo-4-(perfluoropropan-2-yl)phenylcarbamoyl)-2-fluorophenyl)nicotinamide, 3-(3-cyanobenzamido)-N-(2,6-diiodo-4-(perfluoropropan-2-yl)phenyl)-2-fluorobenzamide, 3-(4-cyanobenzamido)-N-(2,6-diiodo-4-(perfluoropropan-2-yl)phenyl)-2-fluorobenzamide, N-(2,6-diiodo-4-(perfluoropropan-2-yl)phenyl)-2-fluoro-3-(3-fluorobenzamido)benzamide, 2-chloro-N-(3-(2,6-dibromo-4-(perfluorobutan-2-yl)phenylcarbamoyl)-2-fluorophenyl)-N-methylnicotinamide, N-(3-(2,6-dibromo-4-(perfluorobutan-2-yl)phenylcarbamoyl)-2-fluorophenyl)-2-fluoro-N-methylbenzamide, N-(2,6-dibromo-4-(perfluorobutan-2-yl)phenyl)-2-fluoro-3-(3-fluoro-N-methylbenzamido)benzamide, N-(2,6-dibromo-4-(perfluorobutan-2-yl)phenyl)-2-fluoro-3-(4-fluoro-N-methylbenzamido)benzamide, N-(3-(2,6-dibromo-4-(perfluorobutan-2-yl)phenylcarbamoyl)-2-fluorophenyl)-2,6-difluoro-N-methylbenzamide, 3-(3-cyano-N-methylbenzamido)-N-(2,6-diiodo-4-(perfluoropropan-2-yl)p henyl)-2-fluorobenzamide, N-(3-(2,6-diiodo-4-(perfluoropropan-2-yl)phenylcarbamoyl)-2-flu orophenyl)-2-fluoro-N-methylbenzamide, N-(2,6-diiodo-4-(perfluoropropan-2-yl)phenyl)-2-f luoro-3-(3-fluoro-N-methylbenzamido)benzamide, 6-chloro-N-(3-(2,6-diiodo-4-(perfluoropr opan-2-yl)phenylcarbamoyl)-2-fluorophenyl)-N-methylnicotinamide,
N-(2,6-diiodo-4-(perfluoropropan-2-yl)phenyl)-2-fluoro-3-(4-fluoro-N-methylbenzamido)benzamide, N-(3-(2,6-diiodo-4-(perfluoropropan-2-yl)phenylcarbamoyl)-2-fluorophenyl)-2,6-difluoro-N-methylbenzamide, N-(2,6-dibromo-4-(perfluoropropan-2-yl)phenyl)-2-fluoro-3-(2-fluorobenzamido)benzamide, N-(2,6-dibromo-4-(perfluoropropan-2-yl)phenyl)-2-fluoro-3-(3-fluorobenzamido)benzamide, N-(2,6-dibromo-4-(perfluoropropan-2-yl)phenyl)-2-fluoro-3-(4-fluorobenzamido)benzamide, N-(2,6-dibromo-4-(perfluoropropan-2-yl)phenyl)-3-(2,6-difluorobenzamido)-2-fluorobenzamide, 6-chloro-N-(3-(2,6-dibromo-4-(perfluoropropan-2-yl)phenylcarbamoyl)-2-fluorophenyl)nicotinamide, 3-(3-cyanobenzamido)-N-(2,6-dibromo-4-(perfluoropropan-2-yl)phenyl)-2-fluorobenzamide, 3-(4-cyanobenzamido)-N-(2,6-dibromo-4-(perfluoropropan-2-yl)phenyl)-2-fluorobenzamide, 2-chloro-N-(3-(2,6-diiodo-4-(perfluoropropan-2-yl)phenylcarbamoyl)-2-fluorophenyl)-N-methylnicotinamide, N-(3-(2,6-dibromo-4-(perfluoropropan-2-yl)phenylcarbamoyl)-2-fluorophenyl)-2-fluoro-N-methylbenzamide, N-(2,6-dibromo-4-(perfluoropropan-2-yl)phenyl)-2-fluoro-3-(3-fluoro-N-methylbenzamido)benzamide, N-(2,6-dibromo-4-(perfluoropropan-2-yl)phenyl)-2-fluoro-3-(4-fluoro-N-methylbenzamido)benzamide, N-(3-(2,6-dibromo-4-(perfluoropropan-2-yl)phenylcarbamoyl)-2-fluorophenyl)-2,6-difluoro-N-methylbenzamide, 3-benzamido-N-(2-bromo-4-(perfluoropropan-2-yl)-6-(trifluoromethoxy)phenyl)-2-fluorobenzamide, N-(3-(2-bromo-4-(perfluoropropan-2-yl)-6-(trifluoromethoxy)phenylcarbamoyl)-2-fluorophenyl)-2-chloronicotinamide, N-(2-bromo-4-(perfluoropropan-2-yl)-6-(trifluoromethoxy)phenyl)-3-(4-cyanobenzamido)-2-fluorobenzamide, 3-benzamido-N-(2-bromo-4-(perfluoropropan-2-yl)-6-(trifluoromethyl)phenyl)benzamide, N-(3-(2-bromo-4-(perfluoropropan-2-yl)-6-(trifluoromethyl)phenylcarbamoyl)phenyl)-2-chloronicotinamide, N-(3-(2-bromo-4-(perfluoropropan-2-yl)-6-(trifluoromethyl)phenylcarbamoyl)phenyl)-N-methylbenzamide, N-(3-(2-bromo-4-(perfluoropropan-2-yl)-6-(trifluoromethyl)phenylcarbamoyl)phenyl)-2-chloro-N-methylnicotinamide, 3-benzamido-N-(2-chloro-4-(perfluoropropan-2-yl)-6-(trifluoromethyl)phenyl)benzamide, 2-chloro-N-(3-(2-chloro-4-(perfluoropropan-2-yl)-6-(trifluoromethyl)phenylcarbamoyl)phenyl)nicotinamide, N-(2-bromo-4-(perfluoropropan-2-yl)-6-(trifluoromethyl)phenyl)-3-(3-cyanobenzamido)benzamide,
N-(2-bromo-4-(perfluoropropan-2-yl)-6-(trifluoromethyl)phenyl)-3-(4-cyanobenzamido)benzamide, N-(3-(2-bromo-4-(perfluoropropan-2-yl)-6-(trifluoromethyl)phenylcarbamoyl)phenyl)-3-cyano-N-methylbenzamide, N-(3-(2-bromo-4-(perfluoropropan-2-yl)-6-(trifluoromethyl)phenylcarbamoyl)phenyl)-4-cyano-N-methylbenzamide, N-(3-(2-bromo-4-(perfluoropropan-2-yl)-6-(trifluoromethyl)phenylcarbamoyl)phenyl)-2-fluoro-N-methylnicotinamide, N-(3-(2-bromo-4-(perfluoropropan-2-yl)-6-(trifluoromethyl)phenylcarbamoyl)phenyl)-N-methyl-4-nitrobenzamide, N-(3-(2-bromo-4-(perfluoropropan-2-yl)-6-(trifluoromethyl)phenylcarbamoyl)phenyl)-6-chloro-N-methylnicotinamide, N-(3-(2-bromo-4-(perfluoropropan-2-yl)-6-(trifluoromethyl)phenylcarbamoyl)phenyl)-6-cyano-N-methylnicotinamide, N-(3-(2-bromo-4-(perfluoropropan-2-yl)-6-(trifluoromethyl)phenylcarbamoyl)phenyl)-N-methyl-3-nitrobenzamide, N-(2-bromo-4-(perfluoropropan-2-yl)-6-(trifluoromethyl)phenyl)-N-methyl-3-(N-methylbenzamido)benzamide, N-(3-((2-bromo-4-(perfluoropropan-2-yl)-6-(trifluoromethyl)phenyl)(methyl)carbamoyl)phenyl)-2-chloro-N-methylnicotinamide, 2-bromo-N-(3-(2-bromo-4-(perfluoropropan-2-yl)-6-(trifluoromethyl)phenylcarbamoyl)phenyl)nicotinamide, 2-chloro-N-(3-(2-chloro-4-(perfluoropropan-2-yl)-6-(trifluoromethyl)phenylcarbamoyl)phenyl)-N-methylnicotinamide, N-(3-(2-chloro-4-(perfluoropropan-2-yl)-6-(trifluoromethyl)phenylcarbamoyl)phenyl)-4-cyano-N-methylbenzamide, 3-benzamido-N-(2-bromo-4-(perfluoropropan-2-yl)-6-(trifluoromethyl)phenyl)-4-fluorobenzamide, N-(5-(2-bromo-4-(perfluoropropan-2-yl)-6-(trifluoromethyl)phenylcarbamoyl)-2-fluorophenyl)-2-chloronicotinamide, N-(3-((2-bromo-4-(perfluoropropan-2-yl)-6-(trifluoromethyl)phenyl)(methyl)carbamoyl)phenyl)-2-chloronicotinamide, N-(2-bromo-4-(perfluoropropan-2-yl)-6-(trifluoromethyl)phenyl)-4-fluoro-3-(N-methylbenzamido)benzamide, N-(2-bromo-4-(perfluoropropan-2-yl)-6-(trifluoromethyl)phenyl)-3-(4-cyano-N-methylbenzamido)-4-fluorobenzamide, N-(5-(2-bromo-4-(perfluoropropan-2-yl)-6-(trifluoromethyl)phenylcarbamoyl)-2-fluorophenyl)-2-chloro-N-methylnicotinamide, 3-benzamido-N-(4-(perfluoropropan-2-yl)-2,6-bis(trifluoromethyl)phenyl)benzamide,
2-chloro-N-(3-(4-(perfluoropropan-2-yl)-2,6-bis(trifluoromethyl)phenylcarbamoyl)phenyl)nicotinamide, N-methyl-N-(3-(4-(perfluoropropan-2-yl)-2,6-bis(trifluoromethyl)phenylcarbamoyl)phenyl)benzamide, 2-chloro-N-methyl-N-(3-(4-(perfluoropropan-2-yl)-2,6-bis(trifluoromethyl)phenylcarbamoyl)phenyl)nicotinamide, 3-benzamido-N-(2-bromo-4-(perfluoropropan-2-yl)-6-(trifluoromethyl)phenyl)-N-methylbenzamide, N-(2-bromo-4-(perfluoropropan-2-yl)-6-(trifluoromethyl)phenyl)-3-(3-cyanobenzamido)-N-methylbenzamide, N-(2-bromo-4-(perfluoropropan-2-yl)-6-(trifluoromethyl)phenyl)-3-(4-cyanobenzamido)-N-methylbenzamide, N-(2-bromo-4-(perfluoropropan-2-yl)-6-(trifluoromethyl)phenyl)-3-(3-cyano-N-methylbenzamido)-N-methylbenzamide, N-(2-bromo-4-(perfluoropropan-2-yl)-6-(trifluoromethyl)phenyl)-3-(4-cyano-N-methylbenzamido)-N-methylbenzamide, 2-bromo-N-(3-(2-bromo-4-(perfluoropropan-2-yl)-6-(trifluoromethyl)phenylcarbamoyl)phenyl)-N-methylnicotinamide, 3-benzamido-N-(2-bromo-4-(perfluoropropan-2-yl)-6-(trifluoromethyl)phenyl)-2-fluorobenzamide, 2-bromo-N-(3-((2-bromo-4-(perfluoropropan-2-yl)-6-(trifluoromethyl)phenyl)(methyl)carbamoyl)phenyl)nicotinamide, N-(3-(2-bromo-4-(perfluoropropan-2-yl)-6-(trifluoromethyl)phenylcarbamoyl)-2-fluorophenyl)-2-chloronicotinamide, 3-benzamido-N-(2-iodo-4-(perfluoropropan-2-yl)-6-(trifluoromethyl)phenyl)benzamide, 2-chloro-N-(3-(2-iodo-4-(perfluoropropan-2-yl)-6-(trifluoromethyl)phenylcarbamoyl)phenyl)nicotinamide, N-(3-(2-bromo-4-(perfluoropropan-2-yl)-6-(trifluoromethyl)phenylcarbamoyl)phenyl)-4-cyano-2-fluoro-N-methylbenzamide, N-(3-(2-bromo-4-(perfluoropropan-2-yl)-6-(trifluoromethyl)phenylcarbamoyl)phenyl)-4-cyano-2-fluorobenzamide, 3-cyano-N-(3-(4-(perfluoropropan-2-yl)-2,6-bis(trifluoromethyl)phenylcarbamoyl)phenyl)benzamide, 3-(4-cyanobenzamido)-N-(4-(perfluoropropan-2-yl)-2,6-bis(trifluoromethyl)phenyl)benzamide, 3-cyano-N-methyl-N-(3-(4-(perfluoropropan-2-yl)-2,6-bis(trifluoromethyl)phenylcarbamoyl)phenyl)benzamide, 4-cyano-N-methyl-N-(3-(4-(perfluoropropan-2-yl)-2,6-bis(trifluoromethyl)phenylcarbamoyl)phenyl)benzamide,
N-(2-bromo-4-(perfluoropropan-2-yl)-6-(trifluoromethyl)phenyl)-3-(4-cyanobenzamido)-2-fluorobenzamide, N-(3-(2-bromo-4-(perfluoropropan-2-yl)-6-(trifluoromethyl)phenylcarbamoyl)-2-fluorophenyl)-2-chloro-N-methylnicotinamide, N-(2-bromo-4-(perfluoropropan-2-yl)-6-(trifluoromethyl)phenyl)-3-(4-cyano-N-methylbenzamido)-2-fluorobenzamide, N-(2-bromo-4-(perfluoropropan-2-yl)-6-(trifluoromethyl)phenyl)-2-fluoro-3-(N-methylbenzamido)benzamide, N-(2-bromo-4-(perfluoropropan-2-yl)-6-(trifluoromethyl)phenyl)-3-(3-cyano-N-methylbenzamido)-2-fluorobenzamide, N-(3-(2-bromo-4-(perfluoropropan-2-yl)-6-(trifluoromethyl)phenylcarbamoyl)phenyl)-2-chloro-4-cyano-N-methylbenzamide, 3-benzamido-N-methyl-N-(4-(perfluoropropan-2-yl)-2,6-bis(trifluoromethyl)phenyl)benzamide, 2-chloro-N-(3-(methyl(4-(perfluoropropan-2-yl)-2,6-bis(trifluoromethyl)phenyl)carbamoyl)phenyl)nicotinamide, 3-(3-cyanobenzamido)-N-methyl-N-(4-(perfluoropropan-2-yl)-2,6-bis(trifluoromethyl)phenyl)benzamide, 2-bromo-N-(3-(methyl(4-(perfluoropropan-2-yl)-2,6-bis(trifluoromethyl)phenyl)carbamoyl)phenyl)nicotinamide, 2-bromo-N-(3-(4-(perfluoropropan-2-yl)-2,6-bis(trifluoromethyl)phenylcarbamoyl)phenyl)nicotinamide, 2-bromo-N-methyl-N-(3-(4-(perfluoropropan-2-yl)-2,6-bis(trifluoromethyl)phenylcarbamoyl)phenyl)nicotinamide, 3-(4-cyanobenzamido)-N-(2-iodo-4-(perfluoropropan-2-yl)-6-(trifluoromethyl)phenyl)benzamide, 3-cyano-N-(3-(2-iodo-4-(perfluoropropan-2-yl)-6-(trifluoromethyl)phenylcarbamoyl)phenyl)benzamide, 2-bromo-N-(3-(2-iodo-4-(perfluoropropan-2-yl)-6-(trifluoromethyl)phenylcarbamoyl)phenyl)nicotinamide, 4-cyano-N-(3-(2-iodo-4-(perfluoropropan-2-yl)-6-(trifluoromethyl)phenylcarbamoyl)phenyl)-N-methylbenzamide, 2-chloro-N-(3-(2-iodo-4-(perfluoropropan-2-yl)-6-(trifluoromethyl)phenylcarbamoyl)phenyl)-N-methylnicotinamide, N-(3-(2-bromo-4-(perfluoropropan-2-yl)-6-(trifluoromethyl)phenylcarbamoyl)phenyl)-2-iodonicotinamide, N-(3-(2-bromo-4-(perfluoropropan-2-yl)-6-(trifluoromethyl)phenylcarbamoyl)-2-fluorophenyl)-N-methylnicotinamide, N-(3-(2-bromo-4-(perfluoropropan-2-yl)-6-(trifluoromethyl)phenylcarbamoyl)-2-fluorophenyl)-6-fluoro-N-methylnicotinamide, N-(3-(2-bromo-4-(perfluoropropan-2-yl)-6-(trifluoromethyl)phenylcarbamoyl)-2-fluorophenyl)-4-cyano-2-fluoro-N-methylbenzamide,
N-(3-(2-bromo-4-(perfluoropropan-2-yl)-6-(trifluoromethyl)phenylcarbamoyl)-2-fluorophenyl)-6-chloro-N-methylnicotinamide, N-(3-(2-bromo-4-(perfluoropropan-2-yl)-6-(trifluoromethyl)phenylcarbamoyl)-2-fluorophenyl)-3,5-dicyano-N-methylbenzamide, 3-benzamido-2-fluoro-N-(4-(perfluoropropan-2-yl)-2,6-bis(trifluoromethyl)phenyl)benzamide, N-(3-(2-bromo-4-(perfluoropropan-2-yl)-6-(trifluoromethyl)phenylcarbamoyl)phenyl)-3,5-dicyano-N-methylbenzamide, 3-benzamido-2-fluoro-N-(2-iodo-4-(perfluoropropan-2-yl)-6-(trifluoromethyl)phenyl)benzamide, 2-chloro-N-(2-fluoro-3-(2-iodo-4-(perfluoropropan-2-yl)-6-(trifluoromethyl)phenylcarbamoyl)phenyl)nicotinamide, 3-benzamido-N-(2-bromo-6-(perfluoroethyl)-4-(perfluoropropan-2-yl)phenyl)-2-fluorobenzamide, 3-benzamido-N-(2-bromo-4-(perfluorobutan-2-yl)-6-(trifluoromethyl)phenyl)-2-fluorobenzamide, N-(3-(2-bromo-4-(perfluorobutan-2-yl)-6-(trifluoromethyl)phenylcarbamoyl)-2-fluorophenyl)-2-chloronicotinamide, N-(2-bromo-6-(perfluoroethyl)-4-(perfluoropropan-2-yl)phenyl)-3-(4-cyanobenzamido)-2-fluorobenzamide, N-(2-bromo-4-(perfluorobutan-2-yl)-6-(trifluoromethyl)phenyl)-2-fluoro-3-(N-methylbenzamido)benzamide, N-(2-bromo-4-(perfluorobutan-2-yl)-6-(trifluoromethyl)phenyl)-3-(3-cyano-N-methylbenzamido)-2-fluorobenzamide, N-(2-bromo-4-(perfluorobutan-2-yl)-6-(trifluoromethyl)phenyl)-3-(4-cyano-N-methylbenzamido)-2-fluorobenzamide, 2-fluoro-N-(2-iodo-4-(perfluoropropan-2-yl)-6-(trifluoromethyl)phenyl)-3-(N-methylbenzamido)benzamide, 3-(4-cyano-N-methylbenzamido)-2-fluoro-N-(2-iodo-4-(perfluoropropan-2-yl)-6-(trifluoromethyl)phenyl)benzamide, N-(3-(2-bromo-4-(perfluorobutan-2-yl)-6-(trifluoromethyl)phenylcarbamoyl)phenyl)-N-methylbenzamide, 3-benzamido-N-(2-bromo-4-(perfluorobutan-2-yl)-6-(trifluoromethyl)phenyl)benzamide, 3-benzamido-2-fluoro-N-(2-iodo-4-(perfluorobutan-2-yl)-6-(trifluoromethyl)phenyl)benzamide, 2-fluoro-N-(2-iodo-4-(perfluorobutan-2-yl)-6-(trifluoromethyl)phenyl)-3-(N-methylbenzamido)benzamide, 3-(4-cyano-N-methylbenzamido)-2-fluoro-N-(2-iodo-4-(perfluorobutan-2-yl)-6-(trifluoromethyl)phenyl)benzamide,
3-(3-cyano-N-methylbenzamido)-2-fluoro-N-(2-iodo-4-(perfluoropropan-2-yl)-6-(trifluoromethyl)phenyl)benzamide, 2-fluoro-N-(2-fluoro-3-(2-iodo-4-(perfluoropropan-2-yl)-6-(trifluoromethyl)phenylcarbamoyl)phenyl)-N-methylbenzamide, 2-fluoro-3-(3-fluoro-N-methylbenzamido)-N-(2-iodo-4-(perfluoropropan-2-yl)-6-(trifluoromethyl)phenyl)benzamide, 2-fluoro-3-(4-fluoro-N-methylbenzamido)-N-(2-iodo-4-(perfluoropropan-2-yl)-6-(trifluoromethyl)phenyl)benzamide, 2,6-difluoro-N-(2-fluoro-3-(2-iodo-4-(perfluoropropan-2-yl)-6-(trifluoromethyl)phenylcarbamoyl)phenyl)-N-methylbenzamide, 6-chloro-N-(2-fluoro-3-(2-iodo-4-(perfluoropropan-2-yl)-6-(trifluoromethyl)phenylcarbamoyl)phenyl)nicotinamide, 3-(3-cyanobenzamido)-2-fluoro-N-(2-iodo-4-(perfluoropropan-2-yl)-6-(trifluoromethyl)phenyl)benzamide, 3-(4-cyanobenzamido)-2-fluoro-N-(2-iodo-4-(perfluoropropan-2-yl)-6-(trifluoromethyl)phenyl)benzamide, 2-fluoro-3-(2-fluorobenzamido)-N-(2-iodo-4-(perfluoropropan-2-yl)-6-(trifluoromethyl)phenyl)benzamide, 2-fluoro-3-(3-fluorobenzamido)-N-(2-iodo-4-(perfluoropropan-2-yl)-6-(trifluoromethyl)phenyl)benzamide, N-(3-(2-bromo-4-(perfluoropropan-2-yl)-6-(trifluoromethyl)phenylcarbamoyl)-2-fluorophenyl)-2-fluoro-N-methylbenzamide, N-(3-(2-bromo-4-(perfluoropropan-2-yl)-6-(trifluoromethyl)phenylcarbamoyl)-2-fluorophenyl)-2,6-difluoro-N-methylbenzamide, N-(2-bromo-4-(perfluoropropan-2-yl)-6-(trifluoromethyl)phenyl)-2-fluoro-3-(3-fluoro-N-methylbenzamido)benzamide, N-(2-bromo-4-(perfluoropropan-2-yl)-6-(trifluoromethyl)phenyl)-2-fluoro-3-(4-fluoro-N-methylbenzamido)benzamide, N-(3-(2-bromo-4-(perfluoropropan-2-yl)-6-(trifluoromethyl)phenylcarbamoyl)-2-fluorophenyl)-2-chloronicotinamide, N-(3-(2-bromo-4-(perfluoropropan-2-yl)-6-(trifluoromethyl)phenylcarbamoyl)-2-fluorophenyl)-6-chloronicotinamide, N-(2-bromo-4-(perfluoropropan-2-yl)-6-(trifluoromethyl)phenyl)-3-(3-cyanobenzamido)-2-fluorobenzamide, N-(2-bromo-4-(perfluoropropan-2-yl)-6-(trifluoromethyl)phenyl)-2-fluoro-3-(2-fluorobenzamido)benzamide, N-(2-bromo-4-(perfluoropropan-2-yl)-6-(trifluoromethyl)phenyl)-2-fluoro-3-(3-fluorobenzamido)benzamide, N-(2-bromo-4-(perfluoropropan-2-yl)-6-(trifluoromethyl)phenyl)-2-fluoro-3-(4-fluorobenzamido)benzamide, N-(3-(2-bromo-4-(perfluoropropan-2-yl)-6-(trifluoromethyl)phenylcarbamoyl)-2-fluorophenyl)-N-methylnicotinamide, 2-chloro-N-(2-fluoro-3-(2-iodo-4-(perfluoropropan-2-yl)-6-(trifluoromethyl)phenylcarbamoyl)phenyl)-N-methylnicotinamide,
2-fluoro-3-(4-fluorobenzamido)-N-(2-iodo-4-(perfluoropropan-2-yl)-6-(trifluoromethyl)phenyl)benzamide, 3-(2,6-difluorobenzamido)-2-fluoro-N-(2-iodo-4-(perfluoropropan-2-yl)-6-(trifluoromethyl)phenyl)benzamide, 6-chloro-N-(2-fluoro-3-(2-iodo-4-(perfluoropropan-2-yl)-6-(trifluoromethyl)phenylcarbamoyl)phenyl)-N-methylnicotinamide, N-(3-(2-bromo-4-(perfluoropropan-2-yl)-6-(trifluoromethyl)phenylcarbamoyl)phenyl)-6-chloronicotinamide, N-(3-(2-bromo-4-(perfluoropropan-2-yl)-6-(trifluoromethyl)phenylcarbamoyl)phenyl)-2-fluorobenzamide, N-(2-bromo-4-(perfluoropropan-2-yl)-6-(trifluoromethyl)phenyl)-3-(3-fluorobenzamido)benzamide, N-(2-bromo-4-(perfluoropropan-2-yl)-6-(trifluoromethyl)phenyl)-3-(4-fluorobenzamido)benzamide, N-(3-(2-bromo-4-(perfluoropropan-2-yl)-6-(trifluoromethyl)phenylcarbamoyl)phenyl)-2,6-difluorobenzamide, N-(2-bromo-4-(perfluoropropan-2-yl)-6-(trifluoromethyl)phenyl)-3-(2,6-difluorobenzamido)-2-fluorobenzamide, 3-fluoro-N-(3-(2-iodo-4-(perfluoropropan-2-yl)-6-(trifluoromethyl)phenylcarbamoyl)phenyl)benzamide, 3-(4-fluorobenzamido)-N-(2-iodo-4-(perfluoropropan-2-yl)-6-(trifluoromethyl)phenyl)benzamide, 2-fluoro-N-(3-(2-iodo-4-(perfluoropropan-2-yl)-6-(trifluoromethyl)phenylcarbamoyl)phenyl)benzamide, 2,6-difluoro-N-(3-(2-iodo-4-(perfluoropropan-2-yl)-6-(trifluoromethyl)phenylcarbamoyl)phenyl)benzamide, 6-chloro-N-(3-(2-iodo-4-(perfluoropropan-2-yl)-6-(trifluoromethyl)phenylcarbamoyl)phenyl)nicotinamide, 6-chloro-N-(3-(2-iodo-4-(perfluoropropan-2-yl)-6-(trifluoromethyl)phenylcarbamoyl)phenyl)-N-methylnicotinamide, 3-cyano-N-(3-(2-iodo-4-(perfluoropropan-2-yl)-6-(trifluoromethyl)phenylcarbamoyl)phenyl)-N-methylbenzamide, 2-fluoro-N-(3-(2-iodo-4-(perfluoropropan-2-yl)-6-(trifluoromethyl)phenylcarbamoyl)phenyl)-N-methylbenzamide, 3-fluoro-N-(3-(2-iodo-4-(perfluoropropan-2-yl)-6-(trifluoromethyl)phenylcarbamoyl)phenyl)-N-methylbenzamide, 4-fluoro-N-(3-(2-iodo-4-(perfluoropropan-2-yl)-6-(trifluoromethyl)phenylcarbamoyl)phenyl)-N-methylbenzamide, N-(3-(2-bromo-4-(perfluoropropan-2-yl)-6-(trifluoromethyl)phenylcarbamoyl)phenyl)-2-fluor o-N-methylbenzamide,
N-(3-(2-bromo-4-(perfluoropropan-2-yl)-6-(trifluoromethyl)phenylcarbamoyl)phenyl)-3-fluoro-N-methylbenzamide, N-(3-(2-bromo-4-(perfluoropropan-2-yl)-6-(trifluoromethyl)phenylcarbamoyl)phenyl)-4-fluoro-N-methylbenzamide, N-(3-(2-bromo-4-(perfluoropropan-2-yl)-6-(trifluoromethyl)phenylcarbamoyl)phenyl)-2,6-difluoro-N-methylbenzamide, 2,4-dichloro-N-(3-(2-iodo-4-(perfluoropropan-2-yl)-6-(trifluoromethyl)phenylcarbamoyl)phenyl)benzamide, 3-(2,4-dichlorobenzamido)-2-fluoro-N-(2-iodo-4-(perfluoropropan-2-yl)-6-(trifluoromethyl)phenyl)benzamide, 2,6-difluoro-N-(3-(2-iodo-4-(perfluoropropan-2-yl)-6-(trifluoromethyl)phenylcarbamoyl)phenyl)-N-methylbenzamide, 3-(2-chloro-4-fluorobenzamido)-2-fluoro-N-(2-iodo-4-(perfluoropropan-2-yl)-6-(trifluoromethyl)phenyl)benzamide, N-(3-(2-iodo-4-(perfluoropropan-2-yl)-6-(trifluoromethyl)phenylcarbamoyl)phenyl)-N-methylbenzamide, 3-benzamido-N-(2-iodo-4-(perfluorobutan-2-yl)-6-(trifluoromethyl)phenyl)benzamide, 2-chloro-N-(3-(2-iodo-4-(perfluorobutan-2-yl)-6-(trifluoromethyl)phenylcarbamoyl)phenyl)nicotinamide, 6-chloro-N-(3-(2-iodo-4-(perfluorobutan-2-yl)-6-(trifluoromethyl)phenylcarbamoyl)phenyl)nicotinamide, 3-cyano-N-(3-(2-iodo-4-(perfluorobutan-2-yl)-6-(trifluoromethyl)phenylcarbamoyl)phenyl)benzamide, 3-(4-cyanobenzamido)-N-(2-iodo-4-(perfluorobutan-2-yl)-6-(trifluoromethyl)phenyl)benzamide, 2-fluoro-N-(3-(2-iodo-4-(perfluorobutan-2-yl)-6-(trifluoromethyl)phenylcarbamoyl)phenyl)benzamide, 3-fluoro-N-(3-(2-iodo-4-(perfluorobutan-2-yl)-6-(trifluoromethyl)phenylcarbamoyl)phenyl)benzamide, 3-(4-fluorobenzamido)-N-(2-iodo-4-(perfluorobutan-2-yl)-6-(trifluoromethyl)phenyl)benzamide, 2,6-difluoro-N-(3-(2-iodo-4-(perfluorobutan-2-yl)-6-(trifluoromethyl)phenylcarbamoyl)phenyl)benzamide, N-(3-(2-iodo-4-(perfluorobutan-2-yl)-6-(trifluoromethyl)phenylcarbamoyl)phenyl)-N-methylbenzamide, 2-chloro-N-(3-(2-iodo-4-(perfluorobutan-2-yl)-6-(trifluoromethyl)phenylcarbamoyl)phenyl)-N-methylnicotinamide, 3-cyano-N-(3-(2-iodo-4-(perfluorobutan-2-yl)-6-(trifluoromethyl)phenylcarbamoyl)phenyl)-N-methylbenzamide, 4-cyano-N-(3-(2-iodo-4-(perfluorobutan-2-yl)-6-(trifluoromethyl)phenylcarbamoyl)phenyl)-N-methylbenzamide,
3-fluoro-N-(3-(2-iodo-4-(perfluorobutan-2-yl)-6-(trifluoromethyl)phenylcarbamoyl)phenyl)-N-methylbenzamide, 4-fluoro-N-(3-(2-iodo-4-(perfluorobutan-2-yl)-6-(trifluoromethyl)phenylcarbamoyl)phenyl)-N-methylbenzamide, N-(3-(2-bromo-4-(perfluoropropan-2-yl)-6-(trifluoromethyl)phenylcarbamoyl)-2-fluorophenyl)-N-methylisonicotinamide, 3-((3-(2-bromo-4-(perfluoropropan-2-yl)-6-(trifluoromethyl)phenylcarbamoyl)-2-fluorophenyl)(methyl)carbamoyl)pyridine 1-oxide, 4-((3-(2-bromo-4-(perfluoropropan-2-yl)-6-(trifluoromethyl)phenylcarbamoyl)-2-fluorophenyl)(methyl)carbamoyl)pyridine 1-oxide, 2-chloro-4-fluoro-N-(3-(2-iodo-4-(perfluoropropan-2-yl)-6-(trifluoromethyl)phenylcarbamoyl)phenyl)benzamide, N-(3-(2-bromo-4-(perfluorobutan-2-yl)-6-(trifluoromethyl)phenylcarbamoyl)phenyl)-2-chloronicotinamide, N-(2-bromo-4-(perfluorobutan-2-yl)-6-(trifluoromethyl)phenyl)-3 -(3 - cyanobenzamido)benzamide, N-(3-(2-bromo-4-(perfluorobutan-2-yl)-6-(trifluoromethyl)phenylcarbamoyl)phenyl)-2-fluorobenzamide, N-(2-bromo-4-(perfluorobutan-2-yl)-6-(trifluoromethyl)phenyl)-3-(3-fluorobenzamido)benzamide, N-(3-(2-bromo-4-(perfluorobutan-2-yl)-6-(trifluoromethyl)phenylcarbamoyl)phenyl)-6-chloronicotinamide, N-(2-bromo-4-(perfluorobutan-2-yl)-6-(trifluoromethyl)phenyl)-3-(4-cyanobenzamido)benzamide, N-(2-bromo-4-(perfluorobutan-2-yl)-6-(trifluoromethyl)phenyl)-3-(4-fluorobenzamido)benzamide, N-(3-(2-bromo-4-(perfluorobutan-2-yl)-6-(trifluoromethyl)phenylcarbamoyl)phenyl)-2,6-difluorobenzamide, N-(3-(2-bromo-4-(perfluorobutan-2-yl)-6-(trifluoromethyl)phenylcarbamoyl)phenyl)-2-chloro-N-methylnicotinamide, N-(3-(2-bromo-4-(perfluorobutan-2-yl)-6-(trifluoromethyl)phenylcarbamoyl)phenyl)-3-cyano-N-methylbenzamide, N-(3-(2-bromo-4-(perfluorobutan-2-yl)-6-(trifluoromethyl)phenylcarbamoyl)phenyl)-2-fluoro-N-methylbenzamide, N-(3-(2-bromo-4-(perfluorobutan-2-yl)-6-(trifluoromethyl)phenylcarbamoyl)phenyl)-3-fluoro-N-methylbenzamide, N-(3-(2-bromo-4-(perfluorobutan-2-yl)-6-(trifluoromethyl)phenylcarbamoyl)phenyl)-6-chloro-N-methylnicotinamide, N-(3-(2-bromo-4-(perfluorobutan-2-yl)-6-(trifluoromethyl)phenylcarbamoyl)phenyl)-4-cyano-N-methylbenzamide,
N-(3-(2-bromo-4-(perfluorobutan-2-yl)-6-(trifluoromethyl)phenylcarbamoyl)phenyl)-4-fluoro-N-methylbenzamide, N-(3-(2-bromo-4-(perfluorobutan-2-yl)-6-(trifluoromethyl)phenylcarbamoyl)phenyl)-2,6-difluoro-N-methylbenzamide, 6-chloro-N-(2-fluoro-3-(2-iodo-4-(perfluorobutan-2-yl)-6-(trifluoromethyl)phenylcarbamoyl)phenyl)nicotinamide, 3-(3-cyanobenzamido)-2-fluoro-N-(2-iodo-4-(perfluorobutan-2-yl)-6-(trifluoromethyl)phenyl)benzamide, 3-(4-cyanobenzamido)-2-fluoro-N-(2-iodo-4-(perfluorobutan-2-yl)-6-(trifluoromethyl)phenyl)benzamide, 2-fluoro-3-(2-fluorobenzamido)-N-(2-iodo-4-(perfluorobutan-2-yl)-6-(trifluoromethyl)phenyl)benzamide, 2-fluoro-3-(3-fluorobenzamido)-N-(2-iodo-4-(perfluorobutan-2-yl)-6-(trifluoromethyl)phenyl)benzamide, 2-fluoro-3-(4-fluorobenzamido)-N-(2-iodo-4-(perfluorobutan-2-yl)-6-(trifluoromethyl)phenyl)benzamide, 3-(2,6-difluorobenzamido)-2-fluoro-N-(2-iodo-4-(perfluorobutan-2-yl)-6-(trifluoromethyl)phenyl)benzamide, N-(3-(2-bromo-4-(perfluorobutan-2-yl)-6-(trifluoromethyl)phenylcarbamoyl)-2-fluorophenyl)-6-chloronicotinamide, N-(2-bromo-4-(perfluorobutan-2-yl)-6-(trifluoromethyl)phenyl)-3-(3-cyanobenzamido)-2-fluorobenzamide, N-(2-bromo-4-(perfluorobutan-2-yl)-6-(trifluoromethyl)phenyl)-3-(4-cyanobenzamido)-2-fluorobenzamide, N-(2-bromo-4-(perfluorobutan-2-yl)-6-(trifluoromethyl)phenyl)-2-fluoro-3-(2-fluorobenzamido)benzamide, N-(2-bromo-4-(perfluorobutan-2-yl)-6-(trifluoromethyl)phenyl)-2-fluoro-3-(3-fluorobenzamido)benzamide, N-(2-bromo-4-(perfluorobutan-2-yl)-6-(trifluoromethyl)phenyl)-2-fluoro-3-(4-fluorobenzamido)benzamide, N-(2-bromo-4-(perfluorobutan-2-yl)-6-(trifluoromethyl)phenyl)-3-(2,6-difluorobenzamido)-2-fluorobenzamide, 2-chloro-N-(2-fluoro-3-(2-iodo-4-(perfluorobutan-2-yl)-6-(trifluoromethyl)phenylcarbamoyl)phenyl)-N-methylnicotinamide, 6-chloro-N-(2-fluoro-3-(2-iodo-4-(perfluorobutan-2-yl)-6-(trifluoromethyl)phenylcarbamoyl)phenyl)-N-methylnicotinamide, 3-(3-cyano-N-methylbenzamido)-2-fluoro-N-(2-iodo-4-(perfluorobutan-2-yl)-6-(trifluoromethyl)phenyl)benzamide, 2-fluoro-N-(2-fluoro-3-(2-iodo-4-(perfluorobutan-2-yl)-6-(trifluoromethyl)phenylcarbamoyl)phenyl)-N-methylbenzamide,
2-fluoro-3-(3-fluoro-N-methylbenzamido)-N-(2-iodo-4-(perfluorobutan-2-yl)-6-(trifl uoromethyl)phenyl)benzamide, 2-fluoro-3-(4-fluoro-N-methylbenzamido)-N-(2-iodo-4-(perfl uorobutan-2-yl)-6-(trifluoromethyl)phenyl)benzamide, 2-chloro-4,5-difluoro-N-(3-(2-iodo-4-(perfluoropropan-2-yl)-6-(trifluoromethyl)phenylcarbamoyl)phenyl)benzamide, 2-chloro-4-fl uoro-N-(3-(2-iodo-4-(perfluoropropan-2-yl)-6-(trifluoromethyl)phenylcarbamoyl)phenyl)-N-methylbenzamide, N-(3-(2-bromo-4-(perfluoropropan-2-yl)-6-(trifluoromethyl)phenylcarbam oyl)phenyl)-2-chloro-4-fluorobenzamide, 2-chloro-N-(3-(2-iodo-4-(perfluoropropan-2-yl)-6-(trifluoromethyl)phenylcarbamoyl)phenyl)benzamide, 3-(2-chlorobenzamido)-2-fluoro-N-(2-iodo-4-(perfluoropropan-2-yl)-6-(trifluoromethyl)phenyl)benzamide, 2-chloro-N-(3-(2-iodo-4-(perfluoropropan-2-yl)-6-(trifluoromethyl)phenylcarbamoyl)phenyl)-N-methylbenzamide, N-(3-(2-bromo-4-(perfluoropropan-2-yl)-6-(trifluoromethyl)phenylcarbamoyl)phenyl)-2-chlor obenzamide, 2-chloro-N-(3-(2-iodo-4-(perfluoropropan-2-yl)-6-(trifluoromethyl)phenylcarba moyl)phenyl)-4-nitrobenzamide, N-(2-fluoro-3-(2-iodo-4-(perfluoropropan-2-yl)-6-(trifluoro methyl)phenylcarbamoyl)phenyl)-N-methylnicotinamide, 3-((2-fluoro-3-(2-iodo-4-(perfluoro propan-2-yl)-6-(trifluoromethyl)phenylcarbamoyl)phenyl)(methyl)carbamoyl)pyridine 1-oxid e, 4-bromo-2-chloro-N-(3-(2-iodo-4-(perfluoropropan-2-yl)-6-(trifluoromethyl)phenylcarbam oyl)phenyl)benzamide, 2-bromo-4-chloro-N-(3-(2-iodo-4-(perfluoropropan-2-yl)-6-(trifluoro methyl)phenylcarbamoyl)phenyl)benzamide, 2-bromo-4-fluoro-N-(3-(2-iodo-4-(perfluoropro pan-2-yl)-6-(trifluoromethyl)phenylcarbamoyl)phenyl)benzamide, 2-fluoro-3-(3-fluoro-N-me thylbenzamido)-N-(2-iodo-4-(perfluoropropan-2-yl)-6-(trifluoromethyl)phenyl)-N-methylben zamide, N-(2-bromo-4-(perfluoropropan-2-yl)-6-(trifluoromethyl)phenyl)-2-fluoro-N-methyl -3-(N-methylbenzamido)benzamide, or N-(2-chloro-4-(perfluoropropan-2-yl)-6-(trifluorome thyl)phenyl)-2-fluoro-3-(N-methylbenzamido)benzamide.

In Formula (1), K may represent Ia non-metal atom group necessary for forming a cyclic linking group derived from pyridine, in combination with A and two carbon atoms to which A bonds

In Formula (1), K may represent a non-metal atom group necessary for forming a cyclic linking group derived from pyridine, pyridine-N-oxide, or thiazole, in combination with A and two carbon atoms to which A bonds.

In formula (7), G₁ and G₃ may represent oxygen atoms. <33> In Formula (9), G₁, G₂, and G₃ may represent oxygen atoms. <34> In Formula (1), G₁, G₂, and G₃ may represent oxygen atoms.

### Effects of the Invention

According to the present invention, a pest control agent containing an amide derivative as an active ingredient showing a pesticidal effect against a wide range of agricultural/horticultural pests and a pest controlling method are provided. Carrying Best Mode for Carrying out the Invention represented

An amide compound for use in the pest control agent according to the present invention is represented by the following Formula (1). 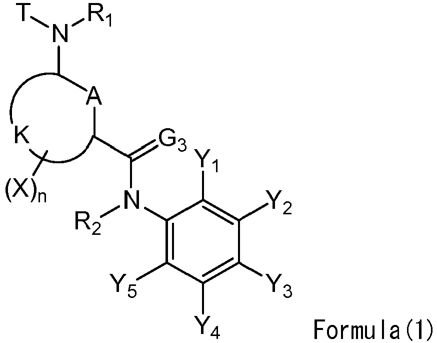 <The definitions for the groups X, n, K, A Y1 to Y5, T, G3, R1 and R2 in the compound of formula (1) are given above.>

A further amide compound for use in the pest control agent is represented by Formula (7):

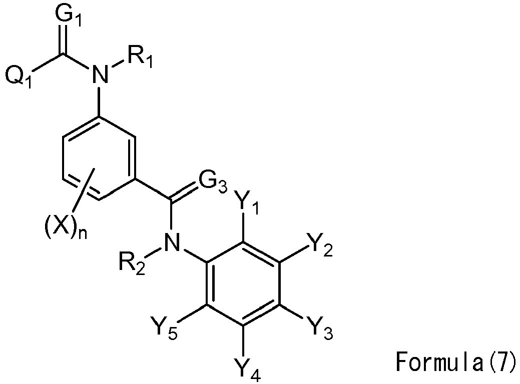

The definitions for the groups X, n, Y₁ to Y₅, Q₁, G₁, G₃, R₁ and R₂ in the compound of Formula (7) are given above.

A further amide compound for use in the pest control agent is represented by Formula (8):

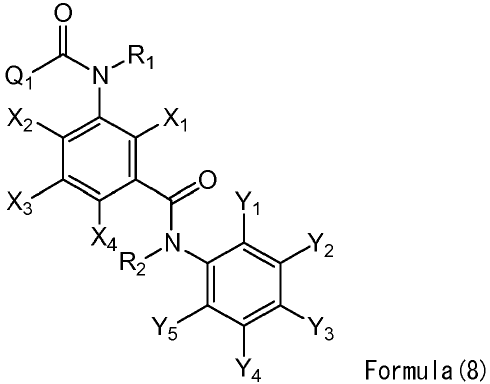

The definitions for the groups X₁ to X₄, Y₁ to Y₅, Q₁, R₁ and R₂ in the compound of Formula (8) are given above.

A further amide compound for use in the pest control agent is represented by Formula (9):

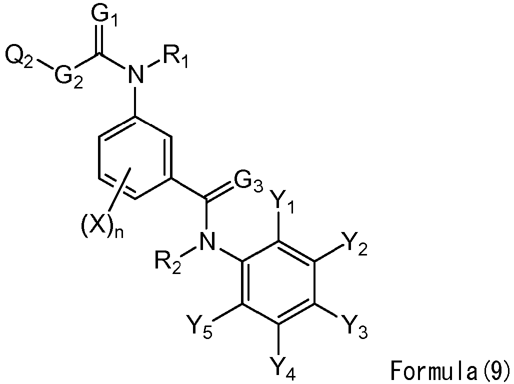

The definitions for the groups X, n, Y₁ to Y₅, Q₂, G₁, G₂, G₃, R₁ and R₂ in the compound of Formula (9) are given above.

The terms used in the formulae including the Formula (1) and the like according to the present invention, have the same meanings as described below in the definitions.

The "halogen atom" represents a fluorine atom, a chlorine atom, a bromine atom, or an iodine atom.

The expression "Ca-Cb (wherein a and b represent an integer of 1 or more)", for example, "C1-C3" means the number of carbon atoms of from 1 to 3, the "C2-C6" means the number of carbon atoms of from 2 to 6, and the "C1-C4" means the number of carbon atoms of from 1 to 4.

"n-" means normal, "i-" means iso, "s-" means secondary, and "t-" means tertiary.

The "C1-C3 alkyl group" in the present invention represents, for example, a linear or branched alkyl group having from 1 to 3 carbon atoms such as methyl, ethyl, n-propyl, i-propyl, and the like.

The "C1-C3 haloalkyl group" represents, for example, a linear or branched alkyl group having from 1 to 3 carbon atoms, that is substituted with one or more halogen atoms which may be the same as or different from each other, such as trifluoromethyl, pentafluoroethyl, heptafluoro-n-propyl, heptafluoro-i-propyl, 2,2-difluoroethyl, 2,2-dichloroethyl, 2,2,2-trifluoroethyl, 2-fluoroethyl, 2-chloroethyl, 2-bromoethyl, 2-iodoethyl, 2,2,2-trichloroethyl, 2,2,2-tribromoethyl, 1,3-difluoro-2-propyl, 1,3-dichloro-2-propyl, 1-chloro-3-fluoro-2-propyl, 1,1,1-trifluoro-2-propyl, 2,3,3,3-trifluoro-n-propyl, 1,1,1,3,3,3-hexafluoro-2-propyl, 1,1,1,3,3,3-hexafluoro-2-chloro-2-propyl, 1,1,1,3,3,3-hexafluoro-2-bromo-2-propyl, 1,1,2,3,3,3-hexafluoro-2-chloro-n-propyl, 1,1,2,3,3,3-hexafluoro-2-bromo-n-propyl, 1,1,2,3,3,3-hexafluoro-1-bromo-2-propyl, 2,2,3,3,3-pentafluoro-n-propyl, 3-fluoro-n-propyl, 3-chloro-n-propyl, 3-bromo-n-propyl, and the like.

The "C1-C4 alkyl group" represents, for example, a linear or branched alkyl group having from 1 to 4 carbon atoms such as methyl, ethyl, n-propyl, i-propyl, n-butyl, s-butyl, t-butyl, and the like.

The "C1-C3 haloalkoxy group" represents, for example, a linear or branched alkoxy group having from 1 to 3 carbon atoms, that is substituted with one or more halogen atoms which may be the same as or different from each other, such as trifluoromethoxy, pentafluoroethoxy, heptafluoro-n-propyloxy, heptafluoro-i-propyloxy, 2,2-difluoroethoxy, 2,2-dichloroethoxy, 2,2,2-trifluoroethoxy, 2-fluoroethoxy, 2-chloroethoxy, 2-bromoethoxy, 2-iodoethoxy, 2,2,2-trichloroethoxy, 2,2,2-tribromoethoxy, 1,3-difluoro-2-propyloxy, 1,3-dichloro-2-propyloxy, 1-chloro-3-fluoro-2-propyloxy, 1,1,1-trifluoro-2-propyloxy, 2,3,3,3-trifluoro-n-propyloxy, 1,1,1,3,3,3-hexafluoro-2-propyloxy, 1,1,1,3,3,3-hexafluoro-2-chloro-2-propyloxy, 1,1,1,3,3,3-hexafluoro-2-bromo-2-propyloxy, 1,1,2,3,3,3-hexafluoro-2-chloro-n-propyloxy, 1,1,2,3,3,3-hexafluoro-2-bromo-n-propyloxy, 1,1,2,3,3,3-hexafluoro-1-bromo-2-propyloxy, 2,2,3,3,3-pentafluoro-n-propyloxy, 3-fluoro-n-propyloxy, 3-chloro-n-propyloxy, 3-bromo-n-propyloxy, and the like.

The "C3-C4 perfluoroalkyl group" represents, for example, a linear or branched alkyl group having from 3 to 4 carbon atoms, that all hydrogen atoms is substituted with fluorine atoms such as perfluoro-n-propyl, perfluoro-i-propyl, perfluoro-n-butyl, perfluoro-i-butyl, perfluoro-s-butyl, perfluoro-t-butyl, and the like.

The "C1 haloalkyl group" represents, for example, a methyl group that is substituted with one or more halogen atoms which may be the same as or different from each other, such as trifluoromethyl, 1,1-difluoro-1-bromomethyl, and the like.

The "C1-C6 alkyl group" in the present invention represents, for example, a linear or branched alkyl group having from 1 to 6 carbon atoms such as methyl, ethyl, n-propyl, i-propyl, n-butyl, s-butyl, t-butyl, n-pentyl, 2-pentyl, neopentyl, 4-methyl-2-pentyl, n-hexyl, 3-methyl-n-pentyl, and the like.

The "C1-C6 haloalkyl group" represents, for example, a linear or branched alkyl group having from 1 to 6 carbon atoms, that is substituted with one or more halogen atoms which may be the same as or different from each other, such as trifluoromethyl, pentafluoroethyl, heptafluoro-n-propyl, heptafluoro-i-propyl, 2,2-difluoroethyl, 2,2-dichloroethyl, 2,2,2-trifluoroethyl, 2-fluoroethyl, 2-chloroethyl, 2-bromoethyl, 2-iodoethyl, 2,2,2-trichloroethyl, 2,2,2-tribromoethyl, 1,3-difluoro-2-propyl, 1,3-dichloro-2-propyl, 1-chloro-3-fluoro-2-propyl, 1,1,1-trifluoro-2-propyl, 2,3,3,3-trifluoro-n-propyl, 4,4,4-trifluoro-n-butyl, 1,1,1,3,3,3-hexafluoro-2-propyl, 1,1,1,3,3,3-hexafluoro-2-chloro-2-propyl, 1,1,1,3,3,3-hexafluoro-2-bromo-2-propyl, 1,1,2,3,3,3-hexafluoro-2-chloro-n-propyl, 1,1,2,3,3,3-hexafluoro-2-bromo-n-propyl, 1,1,2,3,3,3-hexafluoro-1-bromo-2-propyl, 2,2,3,3,3-pentafluoro-n-propyl, 3-fluoro-n-propyl, 3-chloro-n-propyl, 3-bromo-n-propyl, 3,3,4,4,4-pentafluoro-2-butyl, nonafluoro-n-butyl, nonafluoro-2-butyl, 5,5,5-trifluoro-n-pentyl, 4,4,5,5,5-pentafluoro-2-pentyl, 3-chloro-n-pentyl, 4-bromo-2-pentyl, and the like.

The "C3-C9 cycloalkyl group" represents, for example, a cycloalkyl group having from 3 to 9 carbon atoms, that has a cyclic structure, such as cyclopropyl, cyclobutyl, cyclopentyl, 2-methylcyclopentyl, 3-methylcyclopentyl, cyclohexyl, 2-methylcyclohexyl, 3-methylcyclohexyl, 4-methylcyclohexyl, and the like.

The "C3-C9 halocycloalkyl group" represents, for example, a cycloalkyl group having 3 to 9 carbon atoms, that is substituted with one or more halogen atoms which may be the same as or different from each other and has a cyclic structure, such as, 2,2,3,3-tetrafluorocyclobutyl, 2-chlorocyclohexyl, 4-chlorocyclohexyl, and the like.

The "C2-C6 alkenyl group" represents, for example, an alkenyl group having from 2 to 6 carbon atoms, that has a double bond in the carbon chain, such as vinyl, allyl, 2-butenyl, 3-butenyl, and the like.

The "C2-C6 haloalkenyl group" represents, for example, a linear or branched alkenyl group having from 2 to 6 carbon atoms, that is substituted with one or more halogen atoms which may be the same as or different from each other and has a double bond in the carbon chain, such as 3,3-difluoro-2-propenyl, 3,3-dichloro-2-propenyl, 3,3-dibromo-2-propenyl, 2,3-dibromo-2-propenyl, 4,4-difluoro-3-butenyl, 3,4,4-tribromo-3-butenyl, and the like.

The "C2-C6 alkynyl group" represents, for example, an alkynyl group having from 2 to 6 carbon atoms, that has a triple bond in the carbon chain, such as propargyl, 1-butyn-3-yl, 1-butyn-3-methyl-3-yl, and the like.

The "C2-C6 haloalkynyl group" represents, for example, a linear or branched alkynyl group having from 2 to 6 carbon atoms, that is substituted with one or more halogen atoms which may be the same as or different from each other and has a triple bond in the carbon chain, such as fluoroethynyl, chloroethynyl, bromoethynyl, 3,3,3-trifluoro-1-propynyl, 3,3,3-trichloro-1-propynyl, 3,3,3-tribromo-1-propynyl, 4,4,4-trifluoro-1-butynyl, 4,4,4-trichloro-1-butynyl, 4,4,4-tribromo-1-butynyl, and the like.

The "C1-C6 alkoxy group" represents, for example, a linear, branched, or cyclic alkoxy group having from 1 to 6 carbon atoms, such as methoxy, ethoxy, n-propyloxy, i-propyloxy, cyclopropoxy, n-butoxy, s-butoxy, i-butoxy, t-butoxy, n-pentyloxy, i-pentyloxy, n-hexyloxy, cyclohexyloxy, and the like.

The "C1-C6 haloalkoxy group" represents, for example, a linear, branched, or cyclic alkoxy group having from 1 to 6 carbon atoms, that is substituted with one or more halogen atoms which may be the same as or different from each other, such as trifluoromethoxy, pentafluoroethoxy, 2-chloroethoxy, 2,2,2-trifluoroethoxy, heptafluoro-n-propoxy, heptafluoro-i-propoxy, 1,1,1,3,3,3-hexafluoro-2-propoxy, 3-fluoro-n-propoxy, 1-chlorocyclopropoxy, 2-bromocyclopropoxy, 3,3,4,4,4-pentafluoro-2-butoxy, nonafluoro-n-butoxy, nonafluoro-2-butoxy, 5,5,5-trifluoro-n-pentyloxy, 4,4,5,5,5-pentafluoro-2-pentyloxy, 3-chloro-n-pentyloxy, 4-bromo-2-pentyloxy, 4-chlorobutyloxy, 2-iodo-n-propyloxy, and the like.

The "C1-C6 alkylthio group" represents, for example, a linear, branched, or cyclic alkylthio group having from 1 to 6 carbon atoms, such as methylthio, ethylthio, n-propylthio, i-propylthio, cyclopropylthio, n-butylthio, s-butylthio, i-butylthio, t-butylthio, n-pentylthio, i-pentylthio, n-hexylthio, cyclohexylthio, and the like.

The "C1-C6 haloalkylthio group" represents, for example, a linear, branched, or cyclic alkylthio group having from 1 to 6 carbon atoms, that is substituted with one or more halogen atoms which may be the same as or different from each other, such as trifluoromethylthio, pentafluoroethylthio, 2-chloroethylthio, 2,2,2-trifluoroethylthio, heptafluoro-n-propylthio, heptafluoro-i-propylthio, 1,1,1,3,3,3-hexafluoro-2-propylthio, 3-fluoro-n-propylthio, 1-chlorocyclopropylthio, 2-bromocyclopropylthio, 3,3,4,4,4-pentafluoro-2-butylthio, nonafluoro-n-butylthio, nonafluoro-2-butylthio, 5,5,5-trifluoro-n-pentylthio, 4,4,5,5,5-pentafluoro-2-pentylthio, 3-chloro-n-pentylthio, 4-bromo-2-pentylthio, 4-chlorobutylthio, 2-iodo-n-propylthio, and the like.

The "C1-C6 alkylsulfinyl group" represents, for example, a linear, branched, or cyclic alkylsulfinyl group having from 1 to 6 carbon atoms, such as methylsulfinyl, ethylsulfinyl, n-propylsulfinyl, i-propylsulfinyl, cyclopropylsulfinyl, n-butylsulfinyl, s-butylsulfinyl, i-butylsulfinyl, t-butylsulfinyl, n-pentylsulfinyl, i-pentylsulfinyl, n-hexylsulfinyl, cyclohexylsulfinyl, and the like.

The "C1-C6 haloalkylsulfinyl group" represents, for example, a linear, branched, or cyclic alkylsulfinyl group having from 1 to 6 carbon atoms, that is substituted with one or more halogen atoms which may be the same as or different from each other, such as trifluoromethylsulfinyl, pentafluoroethylsulfinyl, 2-chloroethylsulfinyl, 2,2,2-trifluoroethylsulfinyl, heptafluoro-n-propylsulfinyl, heptafluoro-i-propylsulfinyl, 1,1,1,3,3,3-hexafluoro-2-propylsulfinyl, 3-fluoro-n-propylsulfinyl, 1-chlorocyclopropylsulfinyl, 2-bromocyclopropylsulfinyl, 3,3,4,4,4-pentafluoro-2-butylsulfinyl, nonafluoro-n-butylsulfinyl, nonafluoro-2-butylsulfinyl, 5,5,5-trifluoro-n-pentylsulfinyl, 4,4,5,5,5-pentafluoro-2-pentylsulfinyl, 3-chloro-n-pentylsulfinyl, 4-bromo-2-pentylsulfinyl, 4-chlorobutylsulfinyl, 2-iodo-n-propylsulfinyl, and the like.

The "C1-C6 alkylsulfonyl group" represents, for example, a linear, branched, or cyclic alkylsulfonyl group having from 1 to 6 carbon atoms, such as methylsulfonyl, ethylsulfonyl, n-propylsulfonyl, i-propylsulfonyl, cyclopropylsulfonyl, n-butylsulfonyl, s-butylsulfonyl, i-butylsulfonyl, t-butylsulfonyl, n-pentylsulfonyl, i-pentylsulfonyl, n-hexylsulfonyl, cyclohexylsulfonyl, and the like.

The "C1-C6 haloalkylsulfonyl group" represents, for example, a linear, branched, or cyclic alkylsulfonyl group having from 1 to 6 carbon atoms, that is substituted with one or more halogen atoms which may be the same as or different from each other, such as trifluoromethylsulfonyl, pentafluoroethylsulfonyl, 2-chloroethylsulfonyl, 2,2,2-trifluoroethylsulfonyl, heptafluoro-n-propylsulfonyl, heptafluoro-i-propylsulfonyl, 1,1,1,3,3,3-hexafluoro-2-propylsulfonyl, 3-fluoro-n-propylsulfonyl, 1-chlorocyclopropylsulfonyl, 2-bromocyclopropylsulfonyl, 3,3,4,4,4-pentafluoro-2-butylsulfonyl, nonafluoro-n-butylsulfonyl, nonafluoro-2-butylsulfonyl, 5,5,5-trifluoro-n-pentylsulfonyl, 4,4,5,5,5-pentafluoro-2-pentylsulfonyl, 3-chloro-n-pentylsulfonyl, 4-bromo-2-pentylsulfonyl, 4-chlorobutylsulfonyl, 2-iodo-n-propylsulfonyl, and the like.

The "C1-C6 alkylsulfonyloxy group" represents, for example, a linear, branched, or cyclic alkylsulfonyloxy group having from 1 to 6 carbon atoms, such as methanesulfonyloxy, ethanesulfonyloxy, n-propanesulfonyloxy, i-propanesulfonyloxy, cyclopropanesulfonyloxy, n-butanesulfonyloxy, s-butanesulfonyloxy, i-butanesulfonyloxy, t-butanesulfonyloxy, n-pentanesulfonyloxy, i-pentanesulfonyloxy, n-hexanesulfonyloxy, cyclohexanesulfonyloxy, and the like.

The "C1-C6 haloalkylsulfonyloxy group" represents, for example, a linear, branched, or cyclic alkylsulfonyloxy group having from 1 to 6 carbon atoms, that is substituted with one or more halogen atoms which may be the same as or different from each other, such as trifluoromethanesulfonyloxy, pentafluoropropanesulfonyloxy, 2-chloropropanesulfonyloxy, 2,2,2-trifluoropropanesulfonyloxy, heptafluoro-n-propanesulfonyloxy, heptafluoro-i-propanesulfonyloxy, 1,1,1,3,3,3-hexafluoro-2-propanesulfonyloxy, 3 -fluoro-n-propanesulfonyloxy, 1-chlorocyclopropanesulfonyloxy, 2-bromocyclopropanesulfonyloxy, 3,3,4,4,4-pentafluoro-2-butanesulfonyloxy, nonafluoro-n-butanesulfonyloxy, nonafluoro-2-butanesulfonyloxy, 5,5,5-trifluoro-n-pentanesulfonyloxy, 4,4,5,5,5-pentafluoro-2-pentanesulfonyloxy, 3-chloro-n-pentanesulfonyloxy, 4-bromo-2-pentanesulfonyloxy, 4-chlorobutanesulfonyloxy, 2-iodo-n-propanesulfonyloxy, and the like.

The "C2-C7 alkylcarbonyl group" represents, for example, a linear, branched, or cyclic alkylcarbonyl group having from 2 to 7 carbon atoms, such as acetyl, propionyl, i-propylcarbonyl, cyclopropylcarbonyl, n-butylcarbonyl, s-butylcarbonyl, t-butylcarbonyl, n-pentylcarbonyl, 2-pentylcarbonyl, neopentylcarbonyl, cyclopentylcarbonyl, and the like.

The "C2-C7 haloalkylcarbonyl group" represents, for example, a linear, branched, or cyclic alkylcarbonyl group having from 2 to 7 carbon atoms, that is substituted with one or more halogen atoms which may be the same as or different from each other, such as trifluoroacetyl, pentafluoropropionyl, 2-chloropropionyl, 2,2,2-trifluoropropionyl, heptafluoro-n-propylcarbonyl, heptafluoro-i-propylcarbonyl, 1,1,1,3,3,3-hexafluoro-2-propylcarbonyl, 3-fluoro-n-propylcarbonyl, 1-chlorocyclopropylcarbonyl, 2-bromocyclopropylcarbonyl, 3,3,4,4,4-pentafluoro-2-butylcarbonyl, nonafluoro-n-butylcarbonyl, nonafluoro-2-butylcarbonyl, 5,5,5-trifluoro-n-pentylcarbonyl, 4,4,5,5,5-pentafluoro-2-pentylcarbonyl, 3-chloro-n-pentylcarbonyl, 4-bromo-2-pentylcarbonyl, 4-chlorobutylcarbonyl, 2-iodo-n-propylcarbonyl, and the like.

The "C2-C7 alkylcarbonyloxy group" represents, for example, a linear, branched, or cyclic alkylcarbonyloxy group having from 2 to 7 carbon atoms, such as acetyloxy, propionyloxy, i-propylcarbonyloxy, cyclopropylcarbonyloxy, n-butylcarbonyloxy, s-butylcarbonyloxy, t-butylcarbonyloxy, n-pentylcarbonyloxy, 2-pentylcarbonyloxy, neopentylcarbonyloxy, cyclopentylcarbonyloxy, and the like.

The "C2-C7 haloalkylcarbonyloxy group" represents, for example, a linear, branched, or cyclic alkylcarbonyloxy group having from 2 to 7 carbon atoms, that is substituted with one or more halogen atoms which may be the same as or different from each other, such as trifluoroacetyloxy, pentafluoropropionyloxy, 2-chloropropionyloxy, 2,2,2-trifluoropropionyloxy, heptafluoro-n-propylcarbonyloxy, heptafluoro-i-propylcarbonyloxy, 1,1,1,3,3,3-hexafluoro-2-propylcarbonyloxy, 3-fluoro-n-propylcarbonyloxy, 1-chlorocyclopropylcarbonyloxy, 2-bromocyclopropylcarbonyloxy, 3,3,4,4,4-pentafluoro-2-butylcarbonyloxy, nonafluoro-n-butylcarbonyloxy, nonafluoro-2-butylcarbonyloxy, 5,5,5-trifluoro-n-pentylcarbonyloxy, 4,4,5,5,5-pentafluoro-2-pentylcarbonyloxy, 3-chloro-n-pentylcarbonyloxy, 4-bromo-2-pentylcarbonyloxy, 4-chlorobutylcarbonyloxy, 2-iodo-n-propylcarbonyloxy, and the like.

The "C2-C7 alkoxycarbonyl group" represents, for example, a linear, branched, or cyclic alkoxycarbonyl group having from 2 to 7 carbon atoms, such as methoxycarbonyl, ethoxycarbonyl, isopropoxycarbonyl, cyclopropoxycarbonyl, n-butoxycarbonyl, s-butoxycarbonyl, t-butoxycarbonyl, n-pentyloxycarbonyl, 2-pentyloxycarbonyl, neopentyloxycarbonyl, cyclopentyloxycarbonyl, and the like.

The "C2-C7 haloalkoxycarbonyl group" represents, for example, a linear, branched, or cyclic alkoxycarbonyl group having from 2 to 7 carbon atoms, that is substituted with one or more halogen atoms which may be the same as or different from each other, such as trifluoromethoxycarbonyl, pentafluoroethoxycarbonyl, 2-chloroethoxycarbonyl, 2,2,2-trifluoroethoxycarbonyl, heptafluoro-n-propoxycarbonyl, heptafluoro-i-propoxycarbonyl, 1,1,1,3,3,3-hexafluoro-2-propoxycarbonyl, 3-fluoro-n-propoxycarbonyl, 1-chlorocyclopropoxycarbonyl, 2-bromocyclopropoxycarbonyl, 3,3,4,4,4-pentafluoro-2-butoxycarbonyl, nonafluoro-n-butoxycarbonyl, nonafluoro-2-butoxycarbonyl, 5,5,5-trifluoro-n-pentyloxycarbonyl, 4,4,5,5,5-pentafluoro-2-pentyloxycarbonyl, 3-chloro-n-pentyloxycarbonyl, 4-bromo-2-pentyloxycarbonyl, 4-chlorobutyloxycarbonyl, 2-iodo-n-propyloxycarbonyl, and the like.

The "C2-C7 alkylcarbonylamino group" represents, for example, a linear, branched, or cyclic alkylcarbonylamino group having from 2 to 7 carbon atoms, such as acetylamino, propionylamino, n-propylcarbonylamino, i-propylcarbonylamino, cyclopropylcarbonylamino, n-butylcarbonylamino, s-butylcarbonylamino, i-butylcarbonylamino, t-butylcarbonylamino, n-pentylcarbonylamino, i-pentylcarbonylamino, n-hexylcarbonylamino, cyclohexylcarbonylamino, and the like.

The "C2-C7 haloalkylcarbonylamino group" represents, for example, a linear, branched, or cyclic alkylcarbonylamino group having from 2 to 7 carbon atoms, that is substituted with one or more halogen atoms which may be the same as or different from each other, such as trifluoroacetylamino, pentafluoropropionylamino, 2-chloropropionylamino, 2,2,2-trifluoropropionylamino, heptafluoro-n-propylcarbonylamino, heptafluoro-i-propylcarbonylamino, 1,1,1,3,3,3-hexafluoro-2-propylcarbonylamino, 3-fluoro-n-propylcarbonylamino, 1-chlorocyclopropylcarbonylamino, 2-bromocyclopropylcarbonylamino, 3,3,4,4,4-pentafluoro-2-butylcarbonylamino, nonafluoro-n-butylcarbonylamino, nonafluoro-2-butylcarbonylamino, 5,5,5-trifluoro-n-pentylcarbonylamino, 4,4,5,5,5-pentafluoro-2-pentylcarbonylamino, 3-chloro-n-pentylcarbonylamino, 4-bromo-2-pentylcarbonylamino, 4-chlorobutylcarbonylamino, 2-iodo-n-propylcarbonylamino, and the like.

The "C2-C7 alkoxycarbonylamino group" represents, for example, a linear, branched, or cyclic alkoxycarbonylamino group having from 2 to 7 carbon atoms, such as methoxycarbonylamino, ethoxycarbonylamino, n-propyloxycarbonylamino, i-propyloxycarbonylamino, cyclopropoxycarbonylamino, n-butoxycarbonylamino, s-butoxycarbonylamino, i-butoxycarbonylamino, t-butoxycarbonylamino, n-pentyloxycarbonylamino, i-pentyloxycarbonylamino, n-hexyloxycarbonylamino, cyclohexyloxycarbonylamino, and the like.

The "C2-C7 haloalkoxycarbonylamino group" represents, for example, a linear, branched, or cyclic alkoxycarbonylamino group having from 2 to 7 carbon atoms, that is substituted with one or more halogen atoms which may be the same as or different from each other, such as trifluoromethoxycarbonylamino, pentafluoroethoxycarbonylamino, 2-chloroethoxycarbonylamino, 2,2,2-trifluoroethoxycarbonylamino, heptafluoro-n-propoxycarbonylamino, heptafluoro-i-propoxycarbonylamino, 1,1,1,3,3,3-hexafluoro-2-propoxycarbonylamino, 3-fluoro-n-propoxycarbonylamino, 1-chlorocyclopropoxycarbonylamino, 2-bromocyclopropoxycarbonylamino, 3,3,4,4,4-pentafluoro-2-butoxycarbonylamino, nonafluoro-n-butoxycarbonylamino, nonafluoro-2-butoxycarbonylamino, 5,5,5-trifluoro-n-pentyloxycarbonylamino, 4,4,5,5,5-pentafluoro-2-pentyloxycarbonylamino, 3-chloro-n-pentyloxycarbonylamino, 4-bromo-2-pentyloxycarbonylamino, 4-chlorobutyloxycarbonylamino, 2-iodo-n-propyloxycarbonylamino, and the like.

The "C2-C7 alkoxycarbonyloxy group" represents, for example, a linear, branched, or cyclic alkoxycarbonyloxy group having from 2 to 7 carbon atoms, such as methoxycarbonyloxy, ethoxycarbonyloxy, n-propyloxycarbonyloxy, i-propyloxycarbonyloxy, cyclopropoxycarbonyloxy, n-butoxycarbonyloxy, s-butoxycarbonyloxy, i-butoxycarbonyloxy, t-butoxycarbonyloxy, n-pentyloxycarbonyloxy, i-pentyloxycarbonyloxy, n-hexyloxycarbonyloxy, cyclohexyloxycarbonyloxy, and the like.

The "C2-C7 haloalkoxycarbonyloxy group" represents, for example, a linear, branched, or cyclic alkoxycarbonyloxy group having from 2 to 7 carbon atoms, that is substituted with one or more halogen atoms which may be the same as or different from each other, such as trifluoromethoxycarbonyloxy, pentafluoroethoxycarbonyloxy, 2-chloroethoxycarbonyloxy, 2,2,2-trifluoroethoxycarbonyloxy, heptafluoro-n-propoxycarbonyloxy, heptafluoro-i-propoxycarbonyloxy, 1,1,1,3,3,3-hexafluoro-2-propoxycarbonyloxy, 3-fluoro-n-propoxycarbonyloxy, 1-chlorocyclopropoxycarbonyloxy, 2-bromocyclopropoxycarbonyloxy, 3,3,4,4,4-pentafluoro-2-butoxycarbonyloxy, nonafluoro-n-butoxycarbonyloxy, nonafluoro-2-butoxycarbonyloxy, 5,5,5-trifluoro-n-pentyloxycarbonyloxy, 4,4,5,5,5-pentafluoro-2-pentyloxycarbonyloxy, 3-chloro-n-pentyloxycarbonyloxy, 4-bromo-2-pentyloxycarbonyloxy, 4-chlorobutyloxycarbonyloxy, 2-iodo-n-propyloxycarbonyloxy, and the like.

The "C1-C6 alkylamino group" represents, for example, a linear, branched, or cyclic alkylamino group having from 1 to 6 carbon atoms, such as methylamino, dimethylamino, ethylamino, diethylamino, n-propylamino, i-propylamino, cyclopropylamino, n-butylamino, s-butylamino, i-butylamino, t-butylamino, n-pentylamino, i-pentylamino, n-hexylamino, cyclohexylamino, and the like.

The "C1-C6 haloalkylamino group" represents, for example, a linear, branched, or cyclic alkylamino group having from 1 to 6 carbon atoms substituted with one or more halogen atoms which may be the same as or different from each other, such as trifluoromethylamino, ditrifluoromethylamino, pentafluoroethylamino, dipentafluoroethylamino, 2-chloroethylamino, 2,2,2-trifluoroethylamino, heptafluoro-n-propylamino, heptafluoro-i-propylamino, 1,1,1,3,3,3-hexafluoro-2-propylamino, 3-fluoro-n-propylamino, 1-chlorocyclopropylamino, 2-bromocyclopropylamino, 3,3,4,4,4-pentafluoro-2-butylamino, nonafluoro-n-butylamino, nonafluoro-2-butylamino, 5,5,5-trifluoro-n-pentylamino, 4,4,5,5,5-pentafluoro-2-pentylamino, 3-chloro-n-pentylamino, 4-bromo-2-pentylamino, 4-chlorobutylamino, 2-iodo-n-propylamino, and the like.

The "C2-C6 alkenyloxy group" represents, for example, an alkenyloxy group having from 2 to 6 carbon atoms, that has a double bond in the carbon chain, such as vinyloxy, allyloxy, 2-butenyloxy, 3-butenyloxy, and the like.

The "C2-C6 haloalkenyloxy group" represents, for example, a linear or branched alkenyloxy group having from 2 to 6 carbon atoms, that is substituted with one or more halogen atoms which may be the same as or different from each other and has a double bond in the carbon chain, such as 3,3-difluoro-2-propenyloxy, 3,3-dichloro-2-propenyloxy, 3,3-dibromo-2-propenyloxy, 2,3-dibromo-2-propenyloxy, 4,4-difluoro-3-butenyloxy, 3,4,4-tribromo-3-butenyloxy, and the like.

The "C2-C6 alkynyloxy group" represents, for example, an alkynyloxy group having from 2 to 6 carbon atoms, that has a triple bond in the carbon chain, such as propargyloxy, 1-butyn-3-yloxy, 1-butyn-3-methyl-3-yloxy, and the like.

The "C2-C6 haloalkynyloxy group" represents, for example, a linear or branched alkynyloxy group having from 2 to 6 carbon atoms, that is substituted with one or more halogen atoms which may be the same as or different from each other and has a triple bond in the carbon chain, such as fluoroethynyloxy, chloroethynyloxy, bromoethynyloxy, 3,3,3-trifluoro-1-propynyloxy, 3,3,3-trichloro-1-propynyloxy, 3,3,3-tribromo-1-propynyloxy, 4,4,4-trifluoro-1-butynyloxy, 4,4,4-trichloro-1-butynyloxy, 4,4,4-tribromo-1-butynyloxy, and the like.

The "C3-C9 cycloalkoxy group" represents, for example, a cycloalkyloxy group having from 3 to 9 carbon atoms, that has a cyclic structure, such as cyclopropyloxy, cyclobutyloxy, cyclopentyloxy, 2-methylcyclopentyloxy, 3-methylcyclopentyloxy, cyclohexyloxy, 2-methylcyclohexyloxy, 3-methylcyclohexyloxy, 4-methylcyclohexyloxy, and the like.

The "C3-C9 halocycloalkoxy group" represents, for example, a cycloalkyloxy group having from 3 to 9 carbon atoms, that is substituted with one or more halogen atoms which may be the same as or different from each other and has a cyclic structure, such as 2,2,3,3-tetrafluorocyclobutyloxy, 2-chlorocyclohexyloxy, 4-chlorocyclohexyloxy, and the like.

The "C3-C7 alkenylcarbonyl group" represents, for example, an alkenylcarbonyl group having from 3 to 7 carbon atoms, that has a double bond in the carbon chain, such as vinylcarbonyl, allylcarbonyl, 2-butenylcarbonyl, 3-butenylcarbonyl, and the like.

The "C3-C7 haloalkenylcarbonyl group" represents an alkenylcarbonyl group having from 3 to 7 carbon atoms, that is substituted with one or more halogen atoms which may be the same as or different from each other and has a double bond in the carbon chain, such as 3,3-difluoro-2-propenylcarbonyl, 3,3-dichloro-2-propenylcarbonyl, 3,3-dibromo-2-propenylcarbonyl, 2,3-dibromo-2-propenylcarbonyl, 4,4-difluoro-3-butenylcarbonyl, 3,4,4-tribromo-3-butenylcarbonyl, and the like.

The "C3-C7 alkynylcarbonyl group" represents an alkynylcarbonyl group having from 3 to 7 carbon atoms and has a triple bond in the carbon chain, such as propargylcarbonyl, 1-butyn-3-ylcarbonyl, 1-butyn-3-methyl-3-ylcarbonyl, and the like.

The "C3-C7 haloalkynylcarbonyl group" represents, for example, a linear or branched alkynylcarbonyl group having from 3 to 7 carbon atoms, that is substituted with one or more halogen atoms which may be the same as or different from each other and has a triple bond in the carbon chain, such as fluoroethynylcarbonyl, chloroethynylcarbonyl, bromoethynylcarbonyl, 3,3,3-trifluoro-1-propynylcarbonyl, 3,3,3-trichloro-1-propynylcarbonyl, 3,3,3-tribromo-1-propynylcarbonyl, 4,4,4-trifluoro-1-butynylcarbonyl, 4,4,4-trichloro-1-butynylcarbonyl, 4,4,4-tribromo-1-butynylcarbonyl, and the like.

The "C4-C10 cycloalkylcarbonyl group" represents, for example, a cycloalkylcarbonyl group having from 4 to 10 carbon atoms, that has a cyclic structure, such as cyclopropylcarbonyl, cyclobutylcarbonyl, cyclopentylcarbonyl, 2-methylcyclopentylcarbonyl, 3-methylcyclopentylcarbonyl, cyclohexylcarbonyl, 2-methylcyclohexylcarbonyl, 3-methylcyclohexylcarbonyl, 4-methylcyclohexylcarbonyl, and the like.

The "C4-C10 halocycloalkylcarbonyl group" represents, for example, a cycloalkylcarbonyl group having from 4 to 10 carbon atoms, that is substituted with one or more halogen atoms which may be the same as or different from each other and has a cyclic structure, such as 2,2,3,3-tetrafluorocyclobutylcarbonyl, 2-chlorocyclohexylcarbonyl, 4-chlorocyclohexylcarbonyl, and the like.

The "C3-C7 alkenyloxycarbonyl group" represents an alkenyloxycarbonyl group having 3 to 7 carbon atoms, that has a double bond in the carbon chain, such as vinyloxycarbonyl, allyloxycarbonyl, 2-butenyloxycarbonyl, 3-butenyloxycarbonyl, and the like.

The "C3-C7 haloalkenyloxycarbonyl group" represents, for example, a linear or branched alkenyloxycarbonyl group having from 3 to 7 carbon atoms, that is substituted with one or more halogen atoms which may be the same as or different from each other and has a double bond in the carbon chain, such as 3,3-difluoro-2-propenyloxycarbonyl, 3,3-dichloro-2-propenyloxycarbonyl, 3,3-dibromo-2-propenyloxycarbonyl, 2,3-dibromo-2-propenyloxycarbonyl, 4,4-difluoro-3-butenyloxycarbonyl, 3,4,4-tribromo-3-butenyloxycarbonyl, and the like.

The "C3-C7 alkynyloxycarbonyl group" represents, for example, an alkynyloxycarbonyl group having from 3 to 7 carbon atoms, that has a triple bond in the carbon chain, such as propargyloxycarbonyl, 1-butyn-3-yloxycarbonyl, 1-butyn-3-methyl-3-yloxycarbonyl, and the like.

The "C3-C7 haloalkynyloxycarbonyl group" represents, for example, a linear or branched alkynyloxycarbonyl group having from 3 to 7 carbon atoms, that is substituted with one or more halogen atoms which may be the same as or different from each other and has a triple bond in the carbon chain, such as fluoroethynyloxycarbonyl, chloroethynyloxycarbonyl, bromoethynyloxycarbonyl, 3,3,3-trifluoro-1-propynyloxycarbonyl, 3,3,3-trichloro-1-propynyloxycarbonyl, 3,3,3-tribromo-1-propynyloxycarbonyl, 4,4,4-trifluoro-1-butynyloxycarbonyl, 4,4,4-trichloro-1-butynyloxycarbonyl, 4,4,4-tribromo-1-butynyloxycarbonyl, and the like.

The "C4-C10 cycloalkyloxycarbonyl group" represents, for example, a cycloalkyloxycarbonyl group having from 4 to 10 carbon atoms, that has a cyclic structure, such as cyclopropyloxycarbonyl, cyclobutyloxycarbonyl, cyclopentyloxycarbonyl, 2-methylcyclopentyloxycarbonyl, 3-methylcyclopentyloxycarbonyl, cyclohexyloxycarbonyl, 2-methylcyclohexyloxycarbonyl, 3-methylcyclohexyloxycarbonyl, 4-methylcyclohexyloxycarbonyl, and the like.

The "C4-C10 halocycloalkyloxycarbonyl group" represents, for example, a cycloalkyloxycarbonyl group having from 4 to 10 carbon atoms, that is substituted with one or more halogen atoms which may be the same as or different from each other and has a cyclic structure, such as 2,2,3,3-tetrafluorocyclobutyloxycarbonyl, 2-chlorocyclohexyloxycarbonyl, 4-chlorocyclohexyloxycarbonyl, and the like.

The "C2-C6 alkenylamino group" represents, for example, an alkenylamino group having 2 to 6 carbon atoms, that has a double bond in the carbon chain, such as vinylamino, allylamino, 2-butenylamino, 3-butenylamino, and the like.

The "C2-C6 haloalkenylamino group" represents a linear or branched alkenylamino group having from 2 to 6 carbon atoms, that is substituted with one or more halogen atoms which may be the same as or different from each other and has a double bond in the carbon chain, such as 3,3-difluoro-2-propenylamino, 3,3-dichloro-2-propenylamino, 3,3-dibromo-2-propenylamino, 2,3-dibromo-2-propenylamino, 4,4-difluoro-3-butenylamino, 3,4,4-tribromo-3-butenylamino, and the like.

The "C2-C6 alkynylamino group" represents, for example, an alkynylamino group having from 2 to 6 carbon atoms, that has a triple bond in the carbon chain, such as propargylamino, 1-butyn-3-ylamino, 1-butyn-3-methyl-3-ylamino, and the like.

The "C2-C6 haloalkynylamino group" represents, for example, a linear or branched alkynylamino group having from 2 to 6 carbon atoms, that is substituted with one or more halogen atoms which may be the same as or different from each other and has a triple bond in the carbon chain, such as fluoroethynylamino, chloroethynylamino, bromoethynylamino, 3,3,3-trifluoro-1-propynylamino, 3,3,3-trichloro-1-propynylamino, 3,3,3-tribromo-1-propynylamino, 4,4,4-trifluoro-1-butynylamino, 4,4,4-trichloro-1-butynylamino, 4,4,4-tribromo-1-butynylamino, and the like.

The "C3-C9 cycloalkylamino group" represents, for example, a cycloalkyl group amino having from 3 to 9 carbon atoms, that has a cyclic structure, such as cyclopropylamino, cyclobutylamino, cyclopentylamino, 2-methylcyclopentylamino, 3-methylcyclopentylamino, cyclohexylamino, 2-methylcyclohexylamino, 3-methylcyclohexylamino, 4-methylcyclohexylamino, and the like.

The "C3-C9 halocycloalkylamino group" represents, for example, a cycloalkylamino group having from 3 to 9 carbon atoms, that is substituted with one or more halogen atoms which may be the same as or different from each other and has a cyclic structure, such as 2,2,3,3-tetrafluorocyclobutylamino, 2-chlorocyclohexylamino, 4-chlorocyclohexylamino, and the like.

The "C2-C7 alkylaminocarbonyl group" represents, for example, a linear or branched alkylaminocarbonyl group having from 2 to 7 carbon atoms, such as methylaminocarbonyl, ethylaminocarbonyl, n-propylaminocarbonyl, i-propylaminocarbonyl, n-butylaminocarbonyl, s-butylaminocarbonyl, t-butylaminocarbonyl, n-pentylaminocarbonyl, 2-pentylaminocarbonyl, neopentylaminocarbonyl, 4-methyl-2-pentylaminocarbonyl, n-hexylaminocarbonyl, 3-methyl-n-pentylaminocarbonyl, and the like.

The "C2-C7 haloalkylaminocarbonyl group" represents, for example, a linear or branched alkylaminocarbonyl group having from 2 to 7 carbon atoms, that is substituted with one or more halogen atoms which may be the same as or different from each other, such as trifluoromethylaminocarbonyl, pentafluoroethylaminocarbonyl, heptafluoro-n-propylaminocarbonyl, heptafluoro-i-propylaminocarbonyl, 2,2-difluoroethylaminocarbonyl, 2,2-dichloroethylaminocarbonyl, 2,2,2-trifluoroethylaminocarbonyl, 2-fluoroethylaminocarbonyl, 2-chloroethylaminocarbonyl, 2-bromoethylaminocarbonyl, 2-iodoethylaminocarbonyl, 2,2,2-trichloroethylaminocarbonyl, 2,2,2-tribromoethylaminocarbonyl, 1,3-difluoro-2-propylaminocarbonyl, 1,3-dichloro-2-propylaminocarbonyl, 1-chloro-3-fluoro-2-propylaminocarbonyl, 1,1,1-trifluoro-2-propylaminocarbonyl, 2,3,3,3-trifluoro-n-propylaminocarbonyl, 4,4,4-trifluoro-n-butylaminocarbonyl, 1,1,1,3,3,3-hexafluoro-2-propylaminocarbonyl, 1,1,1,3,3,3-hexafluoro-2-chloro-2-propylaminocarbonyl, 1,1,1,3,3,3-hexafluoro-2-bromo-2-propylaminocarbonyl, 1,1,2,3,3,3-hexafluoro-2-chloro-n-propylaminocarbonyl, 1,1,2,3,3,3-hexafluoro-2-bromo-n-propylaminocarbonyl, 1,1,2,3,3,3-hexafluoro-1-bromo-2-propylaminocarbonyl, 2,2,3,3,3-pentafluoro-n-propylaminocarbonyl, 3 -fluoro-n-propylaminocarbonyl, 3 -chloro-n-propylaminocarbonyl, 3-bromo-n-propylaminocarbonyl, 3,3,4,4,4-pentafluoro-2-butylaminocarbonyl, nonafluoro-n-butylaminocarbonyl, nonafluoro-2-butylaminocarbonyl, 5,5,5-trifluoro-n-pentylaminocarbonyl, 4,4,5,5,5-pentafluoro-2-pentylaminocarbonyl, 3-chloro-n-pentylaminocarbonyl, 4-bromo-2-pentylaminocarbonyl, and the like.

The "C3-C7 alkenylaminocarbonyl group" represents, for example, an alkenylaminocarbonyl group having from 3 to 7 carbon atoms, that has a double bond in the carbon chain, such as vinylaminocarbonyl, allylaminocarbonyl, 2-butenylaminocarbonyl, 3-butenylaminocarbonyl, and the like.

The "C3-C7 haloalkenylaminocarbonyl group" represents, for example, a linear or branched alkenylaminocarbonyl group having from 3 to 7 carbon atoms, that is substituted with one or more halogen atoms which may be the same as or different from each other and has a double bond in the carbon chain, such as 3,3-difluoro-2-propenylaminocarbonyl, 3,3-dichloro-2-propenylaminocarbonyl, 3,3-dibromo-2-propenylaminocarbonyl, 2,3-dibromo-2-propenylaminocarbonyl, 4,4-difluoro-3-butenylaminocarbonyl, 3,4,4-tribromo-3-butenylaminocarbonyl, and the like.

The "C3-C7 alkynylaminocarbonyl group" represents, for example, an alkynylaminocarbonyl group having from 3 to 7 carbon atoms, that has a triple bond in the carbon chain, such as propargylaminocarbonyl, 1-butyn-3-ylaminocarbonyl, 1-butyn-3-methyl-3-ylaminocarbonyl, and the like.

The "C3-C7 haloalkynylaminocarbonyl group" represents, for example, a linear or branched alkynylaminocarbonyl group having from 3 to 7 carbon atoms, that is substituted with one or more halogen atoms which may be the same as or different from each other and has a triple bond in the carbon chain, such as fluoroethynylaminocarbonyl, chloroethynylaminocarbonyl, bromoethynylaminocarbonyl, 3,3,3-trifluoro-1-propynylaminocarbonyl, 3,3,3-trichloro-1-propynylaminocarbonyl, 3,3,3-tribromo-1. propynylaminocarbonyl, 4,4,4-trifluoro-1-butynylaminocarbonyl, 4,4,4-trichloro-1-butynylaminocarbonyl, 4,4,4-tribromo-1-butynylaminocarbonyl, and the like.

The "C4-C10 cycloalkylaminocarbonyl group" represents, for example, a cycloalkylaminocarbonyl group having from 4 to 10 carbon atoms, that has a cyclic structure, such as cyclopropylaminocarbonyl, cyclobutylaminocarbonyl, cyclopentylaminocarbonyl, 2-methylcyclopentylaminocarbonyl, 3-methylcyclopentylaminocarbonyl, cyclohexylaminocarbonyl, 2-methylcyclohexylaminocarbonyl, 3-methylcyclohexylaminocarbonyl, 4-methylcyclohexylaminocarbonyl, and the like.

The "C4-C10 halocycloalkylaminocarbonyl group" represents, for example, a cycloalkylaminocarbonyl group having from 4 to 10 carbon atoms that is substituted with one or more halogen atoms which may be the same as or different from each other and has a cyclic structure, such as 2,3,3-tetrafluorocyclobutylaminocarbonyl, 2-chlorocyclohexylaminocarbonyl, 4-chlorocyclohexylaminocarbonyl, and the like.

The "aryl group" represents, for example, a monocyclic and polycyclic aromatic hydrocarbon, such as phenyl group, naphthyl group, and the like.

The "arylcarbonyloxy group" represents, for example, a arylcarbonyloxy group, such as phenylcarbonyloxy group, naphthylcarbonyloxy group, and the like.

The "arylcarbonylamino group" represents, for example, a arylcarbonylamino group, such as phenylcarbonylamino group, naphthylcarbonylamino group, and the like.

The "C1-C6 alkyl group which may have a substituent" in R₁ and R₂ represents an unsubstituted linear, branched, or cyclic alkyl group having 1 to 6 carbon atoms, or a linear, branched, or cyclic alkyl group having from 1 to 6 carbon atoms, which is substituted only with at least one of a nitro group, a phenyl group which may have a substituent, and an unsaturated heterocyclic group which may have a substituent. Further, when there are two or more substituents, these substituents may be the same as or different from each other.

The "C1-C6 haloalkyl group which may have a substituent" in R₁ and R₂ represents a linear, branched, or cyclic alkyl group having from 1 to 6 carbon atoms, which is substituted with one or more halogen atoms, or a linear, branched, or cyclic alkyl group having 1 to 6 carbon atoms, which is substituted with at least one of a nitro group, a phenyl group which may have a substituent, and an unsaturated heterocyclic group which may have a substituent, in addition to one or more halogen atoms.

The "C2-C6 alkenyl group which may have a substituent" in R₁ and R₂ represents an unsubstituted linear, branched, or cyclic alkenyl group having from 2 to 6 carbon atoms, or a linear, branched, or cyclic alkenyl group having 2 to 6 carbon atoms, which is substituted with at least one of a nitro group, a phenyl group which may have a substituent, and an unsaturated heterocyclic group which may have a substituent. Further, when there are two or more substituents, these substituents may be the same as or different from each other.

The "C2-C6 haloalkenyl group which may have a substituent" in R₁ and R₂ represents a linear, branched, or cyclic alkenyl group having from 2 to 6 carbon atoms, which is substituted only with one or more halogen atoms, or a linear, branched, or cyclic alkenyl group having 2 to 6 carbon atoms, which is substituted with at least one of a nitro group, a phenyl group which may have a substituent, and an unsaturated heterocyclic group which may have a substituent, in addition to one or more halogen atoms.

The "C2-C6 alkynyl group which may have a substituent" in R₁ and R₂ represents an unsubstituted linear, branched, or cyclic alkynyl group having from 2 to 6 carbon atoms, or a linear, branched, or cyclic alkynyl group having 2 to 6 carbon atoms, which is substituted with at least one of a nitro group, a phenyl group which may have a substituent, and an unsaturated heterocyclic group which may have a substituent. Further, when there are two or more substituents, these substituents may be the same as or different from each other.

The "C2-C6 haloalkynyl group which may have a substituent" in R₁ and R₂ represents a linear, branched, or cyclic alkynyl group having from 2 to 6 carbon atoms, which is substituted only with one or more halogen atoms, or a linear, branched, or cyclic alkynyl group having 2 to 6 carbon atoms, which is substituted with at least one of a nitro group, a phenyl group which may have a substituent, and an unsaturated heterocyclic group which may have a substituent, in addition to one or more halogen atoms.

Further, each of the substituents in the present invention may further have a substituent, and examples of the substituent include the following:
one or more substituents selected from a group consisting of a halogen atom, a C1-C6 alkyl group, a C1-C6 haloalkyl group, a C3-C9 cycloalkyl group, a C3-C9 halocycloalkyl group, a C2-C6 alkenyl group, a C2-C6 haloalkenyl group, a C2-C6 alkynyl group, a C2-C6 haloalkynyl group, a C1-C6 alkoxy group, a C1-C6 haloalkoxy group, a C1-C6 alkylthio group, a C1-C6 haloalkylthio group, a C1-C6 alkylsulfinyl group, a C1-C6 haloalkylsulfinyl group, a C1-C6 alkylsulfonyl group, a C1-C6 haloalkylsulfonyl group, a C2-C7 alkylcarbonyl group, a C2-C7 haloalkylcarbonyl group, a C2-C7 alkylcarbonyloxy group, a C2-C7 haloalkylcarbonyloxy group, a C1-C6 alkylsulfonyloxy group, a C1-C6 haloalkylsulfonyloxy group, a C2-C7 alkoxycarbonyl group, a C2-C7 haloalkoxycarbonyl group, a C2-C7 alkylcarbonylamino group, a C2-C7 haloalkylcarbonylamino group, a C2-C7 alkoxycarbonylamino group, a C2-C7 haloalkoxycarbonylamino group, a C2-C7 alkylaminocarbonyl group, a C2-C7 haloalkylaminocarbonyl group, a C1-C6 alkylamino group, a C1-C6 haloalkylamino group, a C2-C6 alkenylamino group, a C2-C6 haloalkenylamino group, a C2-C6 alkynylamino group, a C2-C6 haloalkynylamino group, a C3-C9 cycloalkylamino group, a C3-C9 halocycloalkylamino group, a C3-C7 alkenylaminocarbonyl group, a C3-C7 haloalkenylaminocarbonyl group, a C3-C7 alkynylaminocarbonyl group, a C3-C7 haloalkynylaminocarbonyl group, a C4-C10 cycloalkylaminocarbonyl group, a C4-C10 halocycloalkylaminocarbonyl group, an amino group, a carbamoyl group, a sulfamoyl group, a cyano group, a nitro group, a hydroxyl group, a carboxylic group, a pentafluorosulfanyl group, a phenyl group which may have a substituent, a heterocyclic group which may have a substituent, a benzyl group which may have a substituent, a phenylcarbonyl group which may have a substituent, and a phenylamino group which may have a substituent, and when there are two or more substituents, each substituent may be the same as or different from each other. Formula (9)

The compounds represented by Formula (1), Formula (7), Formula (8), and for use in the pest control agent of the present invention may include one or plural chiral carbon atoms or chiral centers in their structural Formulae, and thus two or more optical isomers may exist. However, the present invention includes each of the optical isomers and a mixture thereof at any proportions. Further, the compounds represented by Formula (1), Formula (7), Formula (8), and Formula (9) for use in the pest control agent of the present invention may include two or more kinds of geometrical isomers derived from carbon-carbon double bonds in the structural Formulae. However, the present invention includes each of the geometrical isomers and a mixture thereof at any proportions.

The combinations of the substituents or atoms preferred as the substituents or the like for the amide derivative represented by Formula (1) for use in the pest control agent of the present invention are as follows. T is preferably -C(=G₁)-Q₁, G₁ is preferably an oxygen atom, Qi is preferably a phenyl group which may have a substituent, or a pyridyl group which may have a substituent, and Q₁ is more preferably a phenyl group or a pyridyl group which has one or more substituents selected from a group consisting of a halogen atom, a C1 haloalkyl group, a nitro group, and a cyano group, and when there are two or more substituents, each substituent may be the same as or different from each other.
G₃ is preferably an oxygen atom.
R₁ is preferably a hydrogen atom or a C1-C3 alkyl group.
R₂ is preferably a hydrogen atom.
X is preferably a hydrogen atom, a halogen atom, or a cyano group, and more preferably a hydrogen atom, a fluorine atom, or a cyano group.
When X is a substituent other than a hydrogen atom, n is preferably 0, 1, or 2.
Y₅ is preferably a C1-C3 haloalkyl group.

Furthermore, the combinations of the substituent or atom preferred as the substituent and the like for the amide derivative represented by Formula (8) are as follows.

The representative methods for producing the compound of for use in the pest control agent the present invention are shown below, and the method for producing the amide derivative of the present invention is not limited to the preparation methods below.

In Formula shown in the following preparation method, A, K, X, n, R₁, R₂, T, G₃, Y₁, Y₂, Y₃, Y₄, and Y₅ represent the same definitions as A, K, X, n, R₁, R₂, T, G₃, Y₁, Y₂, Y₃, Y₄, and Y₅, respectively, in Formula (1). LG represents a functional group having a leaving ability, such as a halogen atom, a hydroxy group, or the like, Hal represents a chlorine atom or a bromine atom, and Xa, Xb, and Xc represent chlorine atoms, bromine atoms, or iodine atoms. The methods are also suitable for the preparation of compounds of Formula (7), (8) and (9).

### <Preparation Method 1>

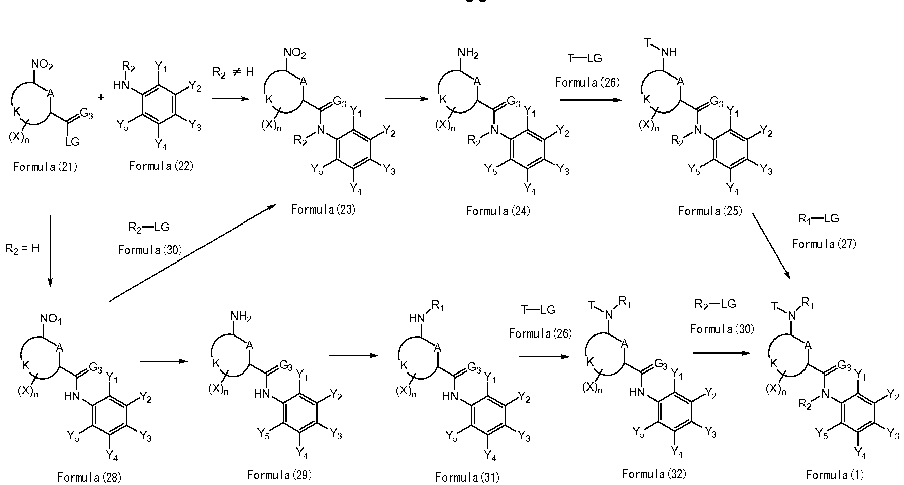

### 1-(i): Formula (21) + Formula (22) → Formula (23)

### Formula (21) + Formula (22) → Formula (28)

A nitro aromatic carboxamide derivative represented by Formula (23) or Formula (28) can be prepared by reacting a nitro aromatic carboxylic acid derivative having a leaving group represented by Formula (21) with an aromatic amine derivative represented by Formula (22) in a suitable solvent or without a solvent. In the present step, a suitable base can be used.

The solvent may be any of those which do not inhibit the present reaction significantly. Examples thereof may include water and aromatic hydrocarbons such as benzene, toluene, xylene, and the like, halogenated hydrocarbons such as dichloromethane, chloroform, carbon tetrachloride, and the like, chained or cyclic ethers such as diethyl ether, dioxane, tetrahydrofuran, 1,2-dimethoxy ethane, and the like, esters such as ethyl acetate, butyl acetate, and the like, alcohols such as methanol, ethanol, and the like, ketones such as acetone, methyl isobutyl ketone, cyclohexanone, and the like, amides such as dimethyl formamide, dimethylacetamide, and the like, nitriles such as acetonitrile and the like, and inert solvents such as 1,3-dimethyl-2-imidazolidinone and the like. These solvents may be used alone or as a mixture of two or more kinds thereof.

Furthermore, examples of the base may include organic bases such as triethylamine, tri-n-butyl amine, pyridine, 4-dimethylamino pyridine, and the like, alkali metal hydroxides such as sodium hydroxide, potassium hydroxide, and the like, carbonates such as sodium hydrogen carbonate, potassium carbonate, and the like, phosphates such as dipotassium monohydrogen phosphate, trisodium phosphate, and the like, alkali metal hydride salts such as sodium hydride and the like, alkali metal alkoxides such as sodium methoxide, sodium ethoxide, and the like, and lithium amides such as lithium diisopropyl amide, and the like.

These bases may be appropriately used in an amount in the range from 0.01-fold molar equivalent to 5-fold molar equivalents with respect to the compound represented by Formula (21).

The reaction temperature may be appropriately selected from -20° to the reflux temperature of the solvent used. Further, the reaction time may be appropriately selected within the range from several minutes to 96 hours.

Among the compounds represented by Formula (21), the aromatic carbonyl halide derivative can be prepared easily by a general method using a halogenating agent from an aromatic carboxylic acid. Examples of the halogenating agent include thionyl chloride, thionyl bromide, phosphorus oxychloride, oxalyl chloride, phosphorus trichloride, and the like.

Meanwhile, it is possible to prepare the compound represented by Formula (23) or Formula (28) from the nitro aromatic carboxylic acid derivative and the compound represented by Formula (22) without using a halogenating agent. Examples of the method may include a method described in, for example, Chem. Ber. p. 788 (1970), in which a condensing agent such as N,N'-dicyclohexylcarbodiimide and the like is appropriately used, suitably with the use of an additive such as 1-hydroxybenzotriazole and the like. Other condensing agents that can be used in this case may include 1-ethyl-3-(3-dimethylaminopropyl)carbodiimide, 1,1'-carbonylbis-1H-imidazole, and the like.

Furthermore, examples of other methods for producing the compound represented by Formula (23) or Formula (28) may include a mixed anhydride method using chloroformic acid esters, and examples thereof include a method described in J. Am. Chem. Soc., p. 5012 (1967), whereby the compound represented by Formula (23) or Formula (28) can be prepared. Examples of the chloroformic acid esters used in this case may include isobutyl chloroformate, isopropyl chloroformate and the like. In addition to chloroformic acid esters, diethylacetyl chloride, trimethylacetyl chloride and the like may also be included.

Both the method using a condensing agent and the mixed anhydride method are not limited by the solvent, the reaction temperature, and the reaction time according to the literature above. An inert solvent may be used which does not inhibit the progress of the appropriate reaction significantly, and the reaction temperature and the reaction time may also be selected appropriately according to the progress of the reaction.

### 1-(ii): Formula (23) → Formula (24)

### Formula (28) → Formula (29)

An aromatic carboxamide derivative having an amino group represented by Formula (24) or Formula (29) can be derived from the aromatic carboxamide derivative having a nitro group represented by Formula (23) or Formula (28) by means of a reduction reaction. Examples of such reduction include a method using a hydrogenation reaction and a method using a metal compound (for example, stannous chloride (anhydride), iron powder, zinc powder, and the like).

The reaction of the former method can be carried out in a suitable solvent in the presence of a catalyst at normal pressure or a higher pressure under a hydrogen atmosphere. Examples of the catalyst may include palladium catalysts such as palladium-carbon and the like, nickel catalysts such as Raney-nickel and the like, cobalt catalysts, ruthenium catalysts, rhodium catalysts, platinum catalysts, and the like, and examples of the solvent may include water; alcohols such as methanol, ethanol, and the like; aromatic hydrocarbons such as benzene, toluene, and the like; chained or cyclic ethers such as ether, dioxane, tetrahydrofuran, and the like; and esters such as ethyl acetate and the like. The pressure may be appropriately selected within a range of 0.1 MPa to 10 MPa, the reaction temperature may be appropriately selected within a range of -20°C to the reflux temperature of the solvent used, and the reaction time may be appropriately selected within a range of several minutes to 96 hours, whereby the compound of Formula (24) or Formula (29) can be efficiently prepared.

Examples of the latter method include a method using stannous chloride (anhydride) as a metal compound under the conditions described in "Organic Syntheses" Coll. Vol. III, P. 453.

### 1-(iii): Formula (24) + Formula (26) → Formula (25)

An aromatic carboxamide or carbamate derivative represented by Formula (25) can be prepared by reacting the aromatic amine derivative represented by Formula (24) with the carboxylic acid derivative or the carbonate ester derivative having a leaving group represented by Formula (26) in a suitable solvent. In the present step, a suitable base or solvent can be used, and as the base or solvent, those exemplified in 1-(i) can be used. Examples of the reaction temperature, the reaction time, and the like may include those exemplified in 1-(i).

In Formula (26), the carbonyl chloride derivative can be prepared easily from a carboxylic acid derivative by a general method using a halogenating agent. The halogenating agent may include those exemplified in 1-(i).

There may be a method for producing a compound represented by Formula (25) from the carboxylic acid derivative (26) and the compound represented by Formula (24) without the use of a halogenating agent, and the preparation can be conducted according to the method exemplified in 1-(i).

### 1-(iv): Formula (25) + Formula (27) → Formula (1)

The compound represented by Formula (1) of the present invention can be prepared by reacting the amide compound represented by Formula (25) with the compound having a leaving group such as halogen and the like, represented by Formula (27) in a solvent or without a solvent. Compounds of Formula (7), (8) and (9) may also be prepared in this way. In the present step, a suitable base or solvent can be used, and as the base or solvent, those exemplified in 1-(i) can be used. Examples of the reaction temperature, the reaction time, and the like may include those exemplified in 1-(i).

### 1-(v): Formula (28) + Formula (30) → Formula (23)

A compound represented by Formula (23) can be prepared by reacting the amide compound represented by Formula (28) with the compound having a leaving group such as halogen and the like, represented by Formula (30) in a solvent or without a solvent. In the present step, a suitable base or solvent can be used, and as the base or solvent, those exemplified in 1-(i) can be used. Examples of the reaction temperature, the reaction time, and the like may include those exemplified in 1-(i).

### 1-(vi): Formula (29) → Formula (31)

### (Method A)

A compound represented by Formula (31) can be prepared by reacting the compound represented by Formula (29) with an aldehyde or a ketone in a solvent, and reacting them under a hydrogen atmosphere with the addition of a catalyst.

The solvent may be any of those which do not inhibit the progress of the reaction significantly, and examples thereof may include aliphatic hydrocarbons such as hexane, cyclohexane, methylcyclohexane, and the like, aromatic hydrocarbons such as benzene, xylene, toluene, and the like, halogenated hydrocarbons such as dichloromethane, chloroform, carbon tetrachloride, 1,2-dichloroethane, and the like, ethers such as diethyl ether, dioxane, tetrahydrofuran, 1,2-dimethoxy ethane, and the like, amides such as dimethyl formamide, dimethylacetamide, and the like, nitriles such as acetonitrile, propionitrile, and the like, ketones such as acetone, methyl isobutyl ketone, cyclohexanone, methyl ethyl ketone, and the like, esters such as ethyl acetate, butyl acetate, and the like, alcohols such as 1,3-dimethyl-2-imidazolidinone, sulfolane, dimethylsulfoxide, methanol, ethanol, and the like, and water. These solvents may be used alone or as a mixture of two or more kinds thereof.

Examples of the catalyst may include palladium catalysts such as palladium-carbon, palladium hydroxide-carbon, and the like, nickel catalysts such as Raney-nickel and the like, cobalt catalysts, platinum catalysts, ruthenium catalysts, rhodium catalysts, and the like.

Examples of the aldehydes may include formaldehyde, acetaldehyde, propionaldehyde, trifluoroacetaldehyde, difluoroacetaldehyde, fluoroacetaldehyde, chloroacetaldehyde, dichloroacetaldehyde, trichloroacetaldehyde, bromoacetaldehyde, and the like.

Examples of the ketones may include acetone, perfluoroacetone, methyl ethyl ketone, and the like.

The reaction pressure may be appropriately selected within the range of 1 atm to 100 atm. The reaction temperature may be appropriately selected within the range from -20°C to the reflux temperature of the solvent used. Further, the reaction time may be appropriately selected within the range from several minutes to 96 hours.

### (Method B)

A compound represented by Formula (31) can be prepared by reacting the compound represented by Formula (29) with an aldehyde or a ketone in a solvent, and treating the product with a reducing agent.

The solvent may be any of those which do not inhibit the progress of the reaction significantly, and examples thereof may include aliphatic hydrocarbons such as hexane, cyclohexane, methylcyclohexane, and the like, aromatic hydrocarbons such as benzene, xylene, toluene, and the like, halogenated hydrocarbons such as dichloromethane, chloroform, carbon tetrachloride, 1,2-dichloroethane, and the like, ethers such as diethyl ether, dioxane, tetrahydrofuran, 1,2-dimethoxy ethane, and the like, amides such as dimethyl formamide, dimethylacetamide, and the like, nitriles such as acetonitrile, propionitrile, and the like, ketones such as acetone, methyl isobutyl ketone, cyclohexanone, methyl ethyl ketone, and the like, esters such as ethyl acetate, butyl acetate, and the like, alcohols such as 1,3-dimethyl-2-imidazolidinone, sulfolane, dimethylsulfoxide, methanol, ethanol, and the like, water, and the like. These solvents may be used alone or as a mixture of two or more kinds thereof.

Examples of the reducing agent may include, for example, borohydrides such as sodium borohydride, sodium cyanoborohydride, sodium triacetate borohydride, and the like.

Examples of the aldehydes may include formaldehyde, acetaldehyde, propionaldehyde, trifluoroacetaldehyde, difluoroacetaldehyde, fluoroacetaldehyde, chloroacetaldehyde, dichloroacetaldehyde, trichloroacetaldehyde, bromoacetaldehyde, and the like.

Examples of the ketones may include acetone, perfluoroacetone, methyl ethyl ketone, and the like.

The reaction temperature may be appropriately selected within the range from -20°C to the reflux temperature of the solvent used. Further, the reaction time may be appropriately selected within the range from several minutes to 96 hours.

### (Method C)

A compound of Formula (31) can be prepared by reacting the compound represented by Formula (29) with an aldehyde in a solvent or without a solvent.

The solvent may be any of those which do not inhibit the progress of the reaction significantly, and examples thereof may include aliphatic hydrocarbons such as hexane, cyclohexane, methylcyclohexane, and the like, aromatic hydrocarbons such as benzene, xylene, toluene, and the like, halogenated hydrocarbons such as dichloromethane, chloroform, carbon tetrachloride, 1,2-dichloroethane, and the like, ethers such as diethyl ether, dioxane, tetrahydrofuran, 1,2-dimethoxy ethane, and the like, amides such as dimethyl formamide, dimethylacetamide, and the like, nitriles such as acetonitrile, propionitrile, and the like, ketones such as acetone, methyl isobutyl ketone, cyclohexanone, methyl ethyl ketone, and the like, esters such as ethyl acetate, butyl acetate, and the like, alcohols such as 1,3-dimethyl-2-imidazolidinone, sulfolane, dimethylsulfoxide, methanol, ethanol, and the like, inorganic acids such as sulfuric acid, hydrochloric acid, and the like, organic acids such as formic acid, acetic acid, and the like, water, and the like. These solvents may be used alone or as a mixture of two or more kinds thereof.

Examples of the aldehydes may include formaldehyde, acetaldehyde, propionaldehyde, and the like.

The reaction temperature may be appropriately selected within the range from -20°C to the reflux temperature of the solvent used, and the reaction time may be appropriately selected within the range from several minutes to 96 hours.

### 1-(vii): Formula (31) + Formula (26) → General Formula (32)

An aromatic carboxamide or carbamate derivative represented by Formula (32) can be prepared by reacting the aromatic amine derivative represented by Formula (31) with the carboxylic acid derivative or the carbonate ester derivative having a leaving group represented by Formula (26) in a suitable solvent. In the present step, a suitable base or solvent can be used, and as the base or solvent, those exemplified in 1-(i) can be used. Examples of the reaction temperature, the reaction time, and the like may include those exemplified in 1-(i).

In Formula (26), the carbonyl chloride derivative can be prepared easily from a carboxylic acid derivative by a general method using a halogenating agent. The halogenating agent may include those exemplified in 1-(i).

There may be exemplified a method for producing a compound represented by Formula (32) from the carboxylic acid derivative (26) and the compound represented by Formula (31) without the use of a halogenating agent, and the preparation can be conducted according to the method exemplified in 1-(i).

### 1-(viii): Formula (32) + Formula (30) → Formula (1)

The compound represented by Formula (1) of the present invention can be prepared by reacting the amide compound represented by Formula (32) with the compound having a leaving group such as a halogen and the like, represented by Formula (30) in a solvent or without a solvent. Compounds of Formula (7), (8) and (9) may also be prepared in this way. In the present step, a suitable base or solvent can be used, and as the base or solvent, those exemplified in 1-(i) can be used. Examples of the reaction temperature, the reaction time, and the like may include those exemplified in 1-(i).

### <Preparation Method 2>

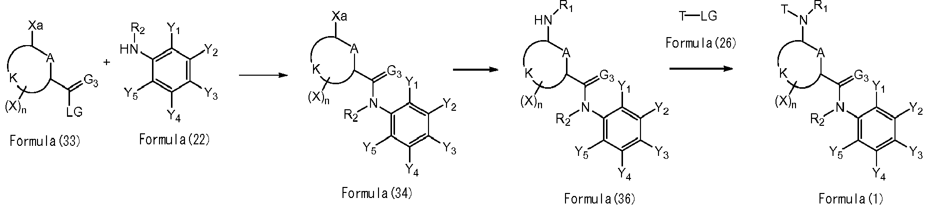

### 2-(i): Formula (33) + Formula (22) → Formula (34)

A compound represented by Formula (34) can be prepared by reacting the compound represented by Formula (33) with a compound represented by Formula (22) under the condition described in 1-(i).

### 2-(ii): Formula (34) → Formula (36)

A compound represented by Formula (36) can be prepared by carrying out an amination reaction using an amination agent such as ammonia and the like according to the conditions described, for example, in J. Org. Chem. p. 280 (1958). However, the conditions such as a reaction solvent and the like are not restricted to those described in the literature, and an inert solvent which does not inhibit the proper progress of the reaction significantly may be used. The reaction temperature and reaction time may be suitably selected as the reaction proceeds. Further, examples of the amination agent include methylamine, ethylamine or the like, in addition to ammonia.

### 2-(iii): Formula (36) + Formula (26) → Formula (1)

The compound represented by Formula (1) can be prepared by reacting the compound represented by Formula (36) with a compound represented by Formula (26) according to the conditions described in 1-(i).

### <Preparation Method 3>

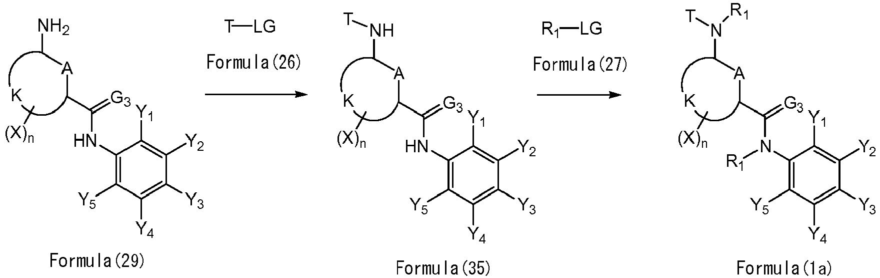

### 3-(i): Formula (29) + Formula (26) → Formula (35)

An aromatic carboxamide or carbamate derivative represented by Formula (35) can be prepared by reacting the aromatic amine derivative represented by Formula (29) with the carboxylic acid derivative or the carbonate ester derivative having a leaving group represented by Formula (26) in a suitable solvent. In the present step, a suitable base or solvent can be used, and as the base or solvent, those exemplified in 1-(i) can be used. Examples of the reaction temperature, the reaction time, and the like may include those exemplified in 1-(i).

In Formula (26), the carbonyl chloride derivative can be prepared easily from a carboxylic acid derivative by a general method using a halogenating agent. The halogenating agent may include those exemplified in 1-(i).

There may be exemplified a method for producing a compound represented by Formula (35) from the carboxylic acid derivative (26) and the compound represented by Formula (29) without the use of a halogenating agent, and the preparation can be conducted according to the method exemplified in 1-(i).

### 3-(ii): Formula (35) + Formula (27) → Formula (1a)

The compound represented by Formula (1a) of the present invention can be prepared by reacting the amide compound represented by Formula (35) with the compound having a leaving group such as halogen and the like, represented by Formula (27) in a solvent or without a solvent. In the present step, a suitable base or solvent can be used, and as the base or solvent, those exemplified in 1-(i) can be used. Examples of the reaction temperature, the reaction time, and the like may include those exemplified in 1-(i).

### <Preparation Method 4>

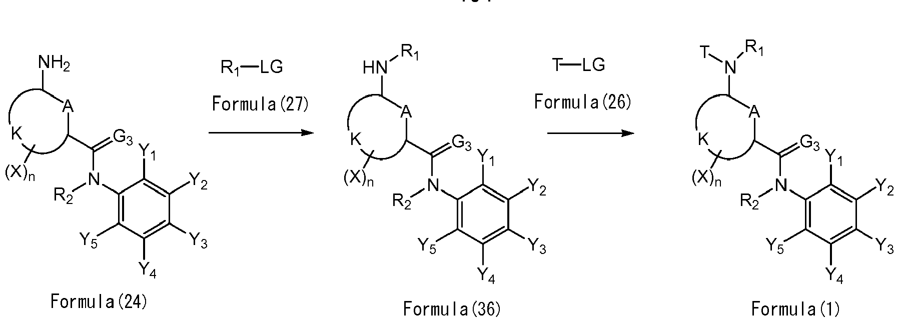

### 4-(i): Formula (24) → Formula (36)

A compound represented by Formula (36) can be prepared by reacting the compound represented by Formula (24) as a starting material according to the conditions of (Method A), (Method B), or (Method C) described in 1-(vi).

### 4-(i'): Formula (24) + Formula (27) → Formula (36)

An aromatic carboxamide represented by Formula (36) can be prepared by reacting the aromatic amine derivative represented by Formula (24) with the carboxylic acid derivative or the carbonate ester derivative having a leaving group represented by Formula (27) in a suitable solvent. In the present step, a suitable base or solvent can be used, and as the base or solvent, those exemplified in 1-(i) can be used. Examples of the reaction temperature, the reaction time, and the like may include those exemplified in 1-(i).

In Formula (27), the carbonyl chloride derivative can be prepared easily from a carboxylic acid derivative by a general method using a halogenating agent. The halogenating agent may include those exemplified in 1-(i).

There may be exemplified a method for producing a compound represented by Formula (36) from the carboxylic acid derivative (27) and the compound represented by Formula (24) without the use of a halogenating agent, and the preparation can be conducted according to the method exemplified in 1-(i).

### 4-(ii): Formula (36) + Formula (26) → Formula (1)

A compound represented by Formula (1) can be prepared by reacting the compound represented by Formula (36) and the compound represented by Formula (26) as starting materials according to the conditions described in 1-(i).

### <Preparation Method 5>

Compounds of Formula (7), (8) and (9) may also be prepared in this way

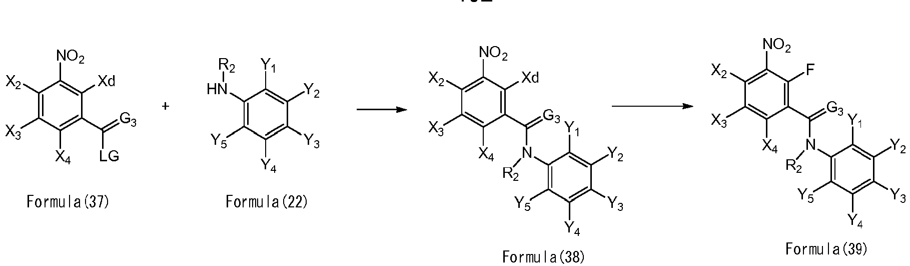

### 5-(i): Formula (37) + Formula (22) → Formula (38)

A compound represented by Formula (38) can be prepared by reacting the compound represented by Formula (37) and the compound represented by Formula (22) according to the conditions described in 1-(i).

### 5-(ii): Formula (38) → Formula (39)

A compound represented by Formula (39) can be prepared by reacting the nitro aromatic carboxamide derivative represented by Formula (38) with a suitable fluorinating agent in a suitable solvent or without a solvent.

The solvent may be any of those which do not inhibit the progress of the reaction significantly, and examples thereof may include aliphatic hydrocarbons such as hexane, cyclohexane, methylcyclohexane, and the like, aromatic hydrocarbons such as benzene, toluene, xylene, and the like, halogenated hydrocarbons such as dichloromethane, chloroform, carbon tetrachloride, 1,2-dichloroethane, and the like, chained or cyclic ethers such as diethyl ether, dioxane, tetrahydrofuran, 1,2-dimethoxy ethane, and the like, esters such as ethyl acetate, butyl acetate, and the like, ketones such as acetone, methyl isobutyl ketone, cyclohexanone, methyl ethyl ketone, and the like, nitriles such as acetonitrile, propionitrile, and the like, and aprotic polar solvents such as 1,3-dimethyl-2-imidazolidinone, sulfolane, dimethylsulfoxide, N,N-dimethyl formamide, N-methyl pyrrolidone, N,N-dimethylacetamide, and the like. These solvents may be used alone or as a mixture of two or more kinds thereof.

Examples of the fluorinating agent may include 1,1,2,2-tetrafluoroethyl diethylamine, 2-chloro-1,1,2-trifluoroethyl diethylamine, trifluorodiphenylphospholane,
difluorotriphenylphospholane, fluoroformic acid esters, sulfur tetrafluoride, potassium fluoride, potassium hydrogen fluoride, cesium fluoride, rubidium fluoride, sodium fluoride, lithium fluoride, antimony (III) fluoride, antimony (V) fluoride, zinc fluoride, cobalt fluoride, lead fluoride, copper fluoride, mercury (II) fluoride, silver fluoride, silver fluoroborate, thallium (I) fluoride, molybdenum (VI) fluoride, arsenic (III) fluoride, bromine fluoride, selenium tetrafluoride, tris(dimethylamino)sulfonium difluorotrimethylsilicate, sodium hexafluorosilicate, quaternary ammonium fluorides, (2-chloroethyl) diethylamine,
diethylaminosulfur trifluoride, morpholinosulfur trifluoride, silicon tetrafluoride, hydrogen fluoride, hydrofluoric acid, hydrogen fluoride-pyridine complex, hydrogen fluoride-triethylamine complex, hydrogen fluoride salts, bis(2-methoxyethyl)amino sulfurtrifluoride, 2,2-difluoro-1,3-dimethyl-2-imidazolidinone, iodine pentafluoride,
tris(diethylamino)phosphonium 2,2,3,3,4,4-hexafluorocyclobutanilide, triethylammonium hexafluorocylcobutanilide, hexafluoropropene, and the like. These fluorinating agents may be used alone or as a mixture of two or more kinds thereof.

The fluorinating agent may be appropriately selected and used as a solvent, in the range of 1-fold molar equivalent to 10-fold molar equivalents with respect to the nitro aromatic carboxamide derivative represented by Formula (38).

Additives may be used, and examples thereof may include crown ethers such as 18-crown-6 and the like, phase transfer catalysts such as a tetraphenylphosphonium salt and the like, inorganic salts such as calcium fluoride, calcium chloride, and the like, metal oxides such as mercury oxide and the like, ion exchange resins, and the like. These additives may not only be added to the reaction system but also used as a pretreating agent for the fluorinating agent.

The reaction temperature may be appropriately selected within the range from -80°C to the reflux temperature of the solvent used, and the reaction time may be appropriately selected within the range from several minutes to 96 hours.

### <Preparation Method 6>

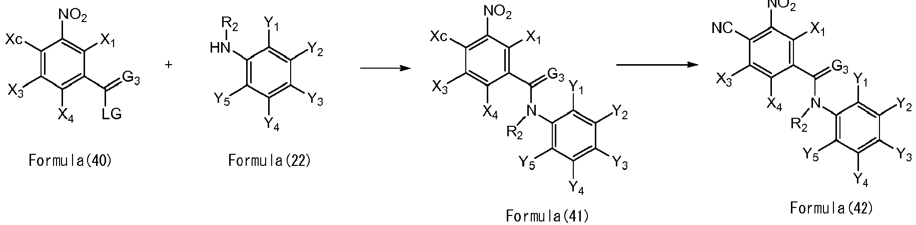

### 6-(i): Formula (40) + Formula (22) → Formula (41)

A compound represented by Formula (41) can be prepared by reacting the compound represented by Formula (40) with a compound represented by Formula (22) according to the conditions described in 1-(i).

### 6-(ii): Formula (41) → Formula (42)

A compound represented by Formula (42) can be prepared by reacting the halogen aromatic carboxamide derivative represented by Formula (41) with a suitable cyanating agent in a suitable solvent or without a solvent.

The solvent may be any of those which do not inhibit the progress of the present reaction significantly. Examples thereof may include aliphatic hydrocarbons such as hexane, cyclohexane, methylcyclohexane, and the like, aromatic hydrocarbons such as benzene, toluene, xylene, and the like, halogenated hydrocarbons such as dichloromethane, chloroform, carbon tetrachloride, 1,2-dichloroethane, and the like, chained or cyclic ethers such as diethyl ether, dioxane, tetrahydrofuran, 1,2-dimethoxy ethane, and the like, esters such as ethyl acetate, butyl acetate, and the like, ketones such as acetone, methyl isobutyl ketone, cyclohexanone, methyl ethyl ketone, and the like, nitriles such as acetonitrile, propionitrile, and the like, and aprotic polar solvents such as 1,3-dimethyl-2-imidazolidinone, sulfolane, dimethylsulfoxide, N,N-dimethyl formamide, N-methyl pyrrolidone, N,N-dimethylacetamide, and the like. These solvents may be used alone or as a mixture of two or more kinds thereof.

Examples of the cyanating agent include cyanide salts such as sodium cyanide, potassium cyanide, sodium cyanoborohydride, and the like, metal cyanides such as copper cyanide, silver cyanide, lithium cyanide, and the like, hydrogen cyanide, tetraethylammonium cyanide, and the like.

These cyanating agents may be appropriately selected and used in the range of 1-fold molar equivalent to 10-fold molar equivalents with respect to the halogen aromatic carboxamide derivative represented by Formula (41).

Additives may be used, and examples thereof may include crown ethers such as 18-crown-6 and the like, phase transfer catalysts such as a tetraphenylphosphonium salt and the like, inorganic salts such as sodium iodide and the like.

The reaction temperature may be appropriately selected within the range from -20°C to the reflux temperature of the solvent used. Further, the reaction time may be appropriately selected within the range from several minutes to 96 hours.

### <Preparation Method 7>

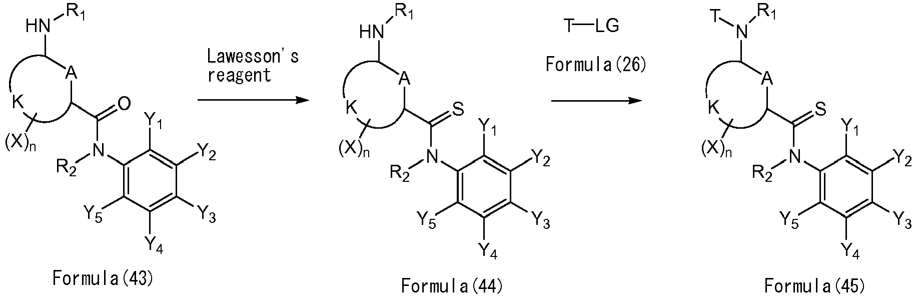

### 7-(i): Formula (43) → Formula (44)

A compound represented by Formula (44) can be prepared by reacting the compound represented by Formula (43) with a Lawesson's reagent according to the known conditions described in Synthesis p. 463 (1993), Synthesis p. 829 (1984) and the like. The conditions such as a solvent, reaction temperature and the like are not restricted to those described in the literature.

### 2-(ii): Formula (44) + Formula (26) → Formula (45)

A compound represented by Formula (45) can be prepared by reacting the compound represented by Formula (44) with a compound represented by Formula (26) according to the conditions described in 1-(i).

### <Preparation Method 8>

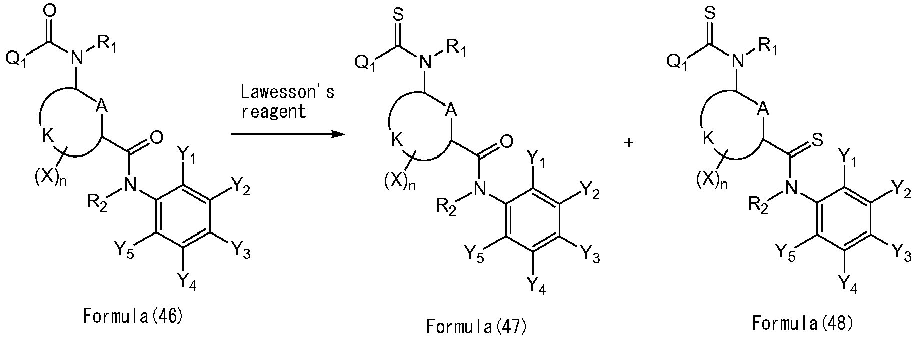

### 8: Formula (46) → Formula (47) + Formula (48)

The compounds represented by Formula (47) and Formula (48) can be prepared from a compound represented by Formula (46) according to the conditions described in 7-(i). The conditions such as a solvent, a reaction temperature, and the like are not restricted to those described in the literature. These two compounds can be easily separated and purified by a known separation and purification technique such as silica gel column chromatography and the like.

### <Preparation Method 9>

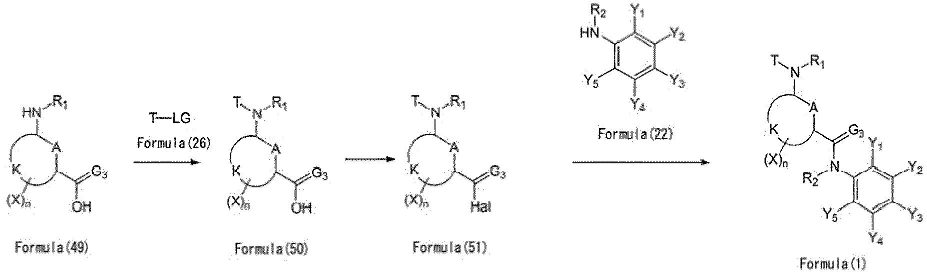

### 9-(i): Formula (49) + Formula (26) → Formula (50)

Carboxylic acids having an acylamino group represented by Formula (50) can be prepared by reacting a carboxylic acid having an amino group represented by Formula (49) as a starting material with a compound represented by Formula (26) according to the conditions described in 1-(i).

### 9-(ii): Formula (50) → Formula (51)

A compound represented by Formula (51) can be prepared by a known general method including reacting the compound represented by Formula (50) with thionyl chloride, oxalyl chloride, phosgene, phosphorus oxychloride, phosphorus pentachloride, phosphorus trichloride, thionyl bromide, phosphorus tribromide, diethylaminosulfur trifluoride, or the like.

### 9-(iii): Formula (51) + Formula (22) → Formula (1)

The compound represented by Formula (1) can be prepared by reacting a compound represented by Formula (51) with a compound represented by Formula (22) according to the conditions described in 1-(i). Compounds of Formula (7), (8) and (9) may also be prepared in this way.

### 9-(iv): Formula (50) + Formula (22) → Formula (1)

The compound represented by Formula (1) can be prepared by reacting the compound represented by Formula (50) with a compound represented by Formula (22) according to the condition using a condensing agent or the condition using a mixed anhydride method according to 1-(i). Compounds of Formula (7), (8) and (9) may also be prepared in this way.

In all of the preparation methods as described above, a desired product may be isolated from the reaction system after the reaction is completed according to a general method, but if required, purification can be carried out by operations such as recrystallization, column chromatography, distillation, and the like. In addition, the desired product can be also provided to the subsequent reaction process without being separated from the reaction system.

Hereinbelow, examples of the representative compounds of the amide derivative represented by Formula (1), Formula (7), Formula (8) or Formula (9) as an active ingredient for the pest control agent of the present invention will be given in Table 1 to Table 51 below, but the present invention is not limited thereto.

In addition, in the tables, "n-" represents normal, "i-" represents iso, "s-" represents secondary, "t-" represents tertiary, "Me" represents a methyl group, "Et" represents an ethyl group, "n-Pr" represents a normal propyl group, "i-Pr" represents an isopropyl group, "n-Bu" represents a normal butyl group, "i-Bu" represents an isobutyl group, "s-Bu" represents a secondary butyl group, "t-Bu" represents a tertiary butyl group, "CF3" represents a trifluoromethyl group, "C2F5" represents a pentafluoroethyl group, "n-C3F7" represents a heptafluoronormal propyl group, "i-C3F7" represents a heptafluoroisopropyl group, "OCF3" represents a trifluoromethoxy group, "OC2F5" represents a pentafluoroethoxy group, "H" represents a hydrogen atom, "F" represents a fluorine atom, "Cl" represents a chlorine atom, "Br" represents a bromine atom, "I" represents an iodine atom, "O" represents an oxygen atom, "C(O)" represents a carbonyl group, "CN" represents a cyano group, "Py" represents a pyridyl group, "Ph" represents a phenyl group, and "S(O)2" represents a sulfonyl group, respectively.

The Tables contain compounds for use in the pest control agents within the scope of the claims, and compounds that are reference examples. These latter compounds are provided for the useful understanding of the invention. The compounds for use in the invention are compounds of Formula (1), (7), (8) and (9) as defined in claim 1.

The compounds of Formula (1) contain a pyridine, pyridine-N-oxide, pyrrole, thiazole, furan, or thiophene group, formed from A and K, and two carbon atoms to which A bonds. Y1 and Y5 represent a halogen atom or a C1-C3 haloalkyl group, and either Y1 or Y5 represents a C1-C3 haloalkyl group; Y2 and Y4 represent a hydrogen atom; Y3 represents a C2-C4 perfluoroalkyl group.

In the compounds of Formula (7) each X independently represents a hydrogen atom, a halogen atom, or cyano group; and Y3 represents a C2-C4 perfluoroalkyl group; Y2 and Y4 represent a hydrogen atom; Y1 and Y5 represent a halogen atom or a C1-C3 haloalkyl group, and either Y1 or Y5 represents a C1-C3 haloalkyl group.

In the compounds of Formula (8) Q1 represents a phenyl group or a pyridyl group which may have a substituent selected from a group consisting of: a halogen atom, C1 haloalkyl group, nitro group, and a cyano group, in a case where Q1 has the substituents the number of the substituents is 1 or 2; X1 and X2 each independently represent a hydrogen atom or a fluorine atom, and X3 and X4 represent a hydrogen atom; and Y1 and Y5 each independently represent a chlorine atom, a bromine atom, an iodine atom, a trifluoromethoxy group, a trifluoromethyl group, or a pentafluoroethyl group; Y2 and Y4 represent a hydrogen atom; and Y3 represents a C3-C4 perfluoroalkyl group; in a case where Y1 and Y5 represent halogen atoms simultaneously, at least one of X1 or X2 represents a fluorine atom; and in a case where Y1 or Y5 represents a trifluoromethoxy group, X2 represents a fluorine atom.

In the compounds of Formula (9) each X independently represents a hydrogen atom, a halogen atom, or cyano group; Y3 represents a C2-C4 perfluoroalkyl group; Y2 and Y4 represent a hydrogen atom; Y1 and Y5 each independently represent a halogen atom or a C1-C3 haloalkyl group, and either Y1 or Y5 represents a C1-C3 haloalkyl group.

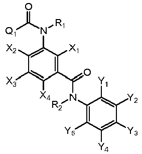

**Table 1(1)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 1-1 | phenyl | H | H | H | CN | H | H | Cl | H | heptafluoroisopropyl | H | OCF3 |
| 1-13 | 4-iodophenyl | H | H | H | CN | H | H | Cl | H | pentafluoroethyl | H | OCF3 |
| 1-16 | (4-trifluoromethyl)phenyl | H | H | H | CN | H | H | Cl | H | heptafluoroisopropyl | H | OC2F5 |
| 1-17 | 2-nitrophenyl | H | H | H | CN | H | H | F | H | heptafluoroisopropyl | H | OCF3 |
| 1-20 | 2-cyanophenyl | H | H | H | CN | H | H | Cl | H | pentafluoroethyl | H | OC2F5 |
| 1-21 | 3-cyanophenyl | H | H | H | CN | H | H | Cl | H | heptafluoroisopropyl | H | OCF3 |
| 1-22 | 4-cyanophenyl | H | H | H | CN | H | H | Cl | H | heptafluoroisopropyl | H | OCF3 |
| 1-28 | 4-bromo-2-chlorophenyl | H | H | H | CN | H | H | Cl | H | heptafluoroisopropyl | H | OC2F5 |
| 1-36 | 2-fluoropyridin-3-yl | H | H | H | CN | H | H | Cl | H | pentafluoroethyl | H | OCF3 |
| 1-37 | 2-chloropyridin-3-yl | H | H | H | CN | H | H | Cl | H | heptafluoroisopropyl | H | OCF3 |
| 1-44 | 6-chloropyridin-3-yl | H | H | H | CN | H | H | Cl | H | heptafluoroisopropyl | H | OC2F5 |
| 1-50 | 5-fluoropyridin-3-yl | H | H | H | CN | H | H | Cl | H | heptafluoroisopropyl | H | OC2F5 |
| 1-55 | 5-nitropyridin-3-yl | H | H | H | CN | H | H | Cl | H | pentafluoroethyl | H | OCF3 |
| 1-57 | 4-fluoropyridin-3-yl | H | H | H | CN | H | H | Cl | H | pentafluoroethyl | H | OCF3 |
| 1-59 | 4-bromopyridin-3-yl | H | H | H | CN | H | H | Cl | H | heptafluoroisopropyl | H | OC2F5 |
| 1-60 | 4-iodopyridin-3-yl | H | H | H | CN | H | H | Cl | H | heptafluoroisopropyl | H | OC2F5 |
| 1-79 | phenyl | H | H | H | CN | H | H | Cl | H | nonafluoro-2-butyl | H | OCF3 |
| 1-95 | 2-nitrophenyl | H | H | H | CN | H | H | F | H | nonafluoro-2-butyl | H | OCF3 |
| 1-99 | 3-cyanophenyl | H | H | H | CN | H | H | Cl | H | nonafluoro-2-butyl | H | OCF3 |
| 1-100 | 4-cyanophenyl | H | H | H | CN | H | H | Cl | H | nonafluoro-2-butyl | H | OCF3 |
| 1-105 | 2-chloro-4,5-difluorophenyl | H | H | H | CN | H | H | Cl | H | nonafluoro-2-butyl | H | OC2F5 |
| 1-115 | 2-chloropyridin-3-yl | H | H | H | CN | H | H | Cl | H | nonafluoro-2-butyl | H | OCF3 |
| 1-121 | 6-fluoropyridin-3-yl | H | H | H | CN | H | H | Cl | H | nonafluoro-2-butyl | H | OC2F5 |
| 1-130 | 5-bromopyridin-3-yl | H | H | H | CN | H | H | Cl | H | nonafluoro-2-butyl | H | OC2F5 |
| 1-131 | 5-iodopyridin-3-yl | H | H | H | CN | H | H | Cl | H | nonafluoro-2-butyl | H | OC2F5 |
| 1-139 | 4-(trifluoromethyl)pyridin-3-yl | H | H | H | CN | H | H | Cl | H | nonafluoro-2-butyl | H | OC2F5 |
| 1-140 | 4-nitropyridin-3-yl | H | H | H | CN | H | H | Cl | H | nonafluoro-2-butyl | H | OC2F5 |
| 1-157 | phenyl | H | H | H | CN | H | H | Br | H | heptafluoroisopropyl | H | OCF3 |
| 1-177 | 3-cyanophenyl | H | H | H | CN | H | H | Br | H | heptafluoroisopropyl | H | OCF3 |
| 1-178 | 4-cyanophenyl | H | H | H | CN | H | H | Br | H | heptafluoroisopropyl | H | OCF3 |
| 1-193 | 2-chloropyridin-3-yl | H | H | H | CN | H | H | Br | H | heptafluoroisopropyl | H | OCF3 |
| 1-200 | 6-chloropyridin-3-yl | H | H | H | CN | H | H | Br | H | heptafluoroisopropyl | H | OCF3 |
| 1-222 | pyridin-4-yl | H | H | H | CN | H | H | Br | H | heptafluoroisopropyl | H | OCF3 |
| 1-235 | phenyl | H | H | H | CN | H | H | Br | H | nonafluoro-2-butyl | H | OCF3 |
| 1-255 | 3-cyanophenyl | H | H | H | CN | H | H | Br | H | nonafluoro-2-butyl | H | OCF3 |
| 1-256 | 4-cyanophenyl | H | H | H | CN | H | H | Br | H | nonafluoro-2-butyl | H | OCF3 |
| 1-271 | 2-chloropyridin-3-yl | H | H | H | CN | H | H | Br | H | nonafluoro-2-butyl | H | OCF3 |
| 1-313 | phenyl | H | H | H | CN | H | H | I | H | heptafluoroisopropyl | H | OCF3 |
| 1-314 | 2-fluorophenyl | H | H | H | CN | H | H | I | H | heptafluoroisopropyl | H | OCF3 |
| 1-333 | 3-cyanophenyl | H | H | H | CN | H | H | I | H | heptafluoroisopropyl | H | OCF3 |
| 1-334 | 4-cyanophenyl | H | H | H | CN | H | H | I | H | heptafluoroisopropyl | H | OCF3 |
| 1-349 | 2-chloropyridin-3-yl | H | H | H | CN | H | H | I | H | heptafluoroisopropyl | H | OCF3 |
| 1-356 | 6-chloropyridin-3-yl | H | H | H | CN | H | H | I | H | heptafluoroisopropyl | H | OCF3 |
| 1-391 | phenyl | H | H | H | CN | H | H | I | H | nonafluoro-2-butyl | H | OCF3 |
| 1-411 | 3-cyanophenyl | H | H | H | CN | H | H | I | H | nonafluoro-2-butyl | H | OCF3 |
| 1-412 | 4-cyanophenyl | H | H | H | CN | H | H | I | H | nonafluoro-2-butyl | H | OCF3 |
| 1-427 | 2-chloropyridin-3-yl | H | H | H | CN | H | H | I | H | nonafluoro-2-butyl | H | OCF3 |
| 1-434 | 6-chloropyridin-3-yl | H | H | H | CN | H | H | I | H | nonafluoro-2-butyl | H | OCF3 |
| 1-469 | phenyl | H | H | H | CN | H | H | F | H | heptafluoroisopropyl | H | CF3 |
| 1-470 | phenyl | H | H | H | CN | H | H | Cl | H | heptafluoroisopropyl | H | CF3 |
| 1-471 | 2-fluorophenyl | H | H | H | CN | H | H | Cl | H | heptafluoroisopropyl | H | CF3 |
| 1-472 | 3-fluorophenyl | H | H | H | CN | H | H | Cl | H | heptafluoroisopropyl | H | CF3 |
| 1-473 | 4-fluorophenyl | H | H | H | CN | H | H | Cl | H | heptafluoroisopropyl | H | CF3 |
| 1-474 | 2-chlorophenyl | H | H | H | CN | H | H | Cl | H | heptafluoroisopropyl | H | CF3 |
| 1-475 | 3-chlorophenyl | H | H | H | CN | H | H | Cl | H | heptafluoroisopropyl | H | CF3 |
| 1-476 | 4-chlorophenyl | H | H | H | CN | H | H | Cl | H | heptafluoroisopropyl | H | CF3 |
| 1-477 | 2-bromophenyl | H | H | H | CN | H | H | Cl | H | heptafluoroisopropyl | H | CF3 |

**Table 1 (2)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 1-478 | 3-bromophenyl | H | H | H | CN | H | H | Cl | H | heptafluoroisopropyl | H | CF3 |
| 1-479 | 4-bromophenyl | H | H | H | CN | H | H | Cl | H | heptafluoroisopropyl | H | CF3 |
| 1-480 | 2-iodophenyl | H | H | H | CN | H | H | Cl | H | heptafluoroisopropyl | H | CF3 |
| 1-481 | 3-iodophenyl | H | H | H | CN | H | H | Cl | H | pentafluoroethyl | H | CF3 |
| 1-482 | 4-iodophenyl | H | H | H | CN | H | H | Cl | H | pentafluoroethyl | H | CF3 |
| 1-483 | (2-trifluoromethyl)phenyl | H | H | H | CN | H | H | Cl | H | heptafluoroisopropyl | H | CF3 |
| 1-484 | (3-trifluoromethyl)phenyl | H | H | H | CN | H | H | Cl | H | heptafluoroisopropyl | H | CF3 |
| 1-485 | (4-trifluoromethyl)phenyl | H | H | H | CN | H | H | Cl | H | heptafluoroisopropyl | H | CF3 |
| 1-486 | 2-nitrophenyl | H | H | H | CN | H | H | Cl | H | heptafluoroisopropyl | H | CF3 |
| 1-487 | 3-nitrophenyl | H | H | H | CN | H | H | Cl | H | heptafluoroisopropyl | H | CF3 |
| 1-488 | 4-nitrophenyl | H | H | H | CN | H | H | Cl | H | heptafluoroisopropyl | H | CF3 |
| 1-489 | 2-cyanophenyl | H | H | H | CN | H | H | Cl | H | pentafluoroethyl | H | C2F5 |
| 1-490 | 3-cyanophenyl | H | H | H | CN | H | H | Cl | H | heptafluoroisopropyl | H | CF3 |
| 1-491 | 4-cyanophenyl | H | H | H | CN | H | H | Cl | H | heptafluoroisopropyl | H | CF3 |
| 1-492 | 2,6-difluorophenyl | H | H | H | CN | H | H | Cl | H | heptafluoroisopropyl | H | CF3 |
| 1-493 | 3,4-dichlorophenyl | H | H | H | CN | H | H | Cl | H | heptafluoroisopropyl | H | CF3 |
| 1-494 | 2,4-dichlorophenyl | H | H | H | CN | H | H | Cl | H | heptafluoroisopropyl | H | C2F5 |
| 1-495 | 2-chloro-4-fluorophenyl | H | H | H | CN | H | H | Cl | H | heptafluoroisopropyl | H | CF3 |
| 1-496 | 2-chloro-4,5-difluorophenyl | H | H | H | CN | H | H | Cl | H | heptafluoroisopropyl | H | CF3 |
| 1-497 | 4-bromo-2-chlorophenyl | H | H | H | CN | H | H | Cl | H | heptafluoroisopropyl | H | CF3 |
| 1-498 | 2-bromo-4-chlorophenyl | H | H | H | CN | H | H | Cl | H | heptafluoroisopropyl | H | n-C3F7 |
| 1-499 | 2-bromo-4-fluorophenyl | H | H | H | CN | H | H | Cl | H | heptafluoroisopropyl | H | CF3 |
| 1-500 | 2-chloro-4-nitrophenyl | H | H | H | CN | H | H | Cl | H | heptafluoroisopropyl | H | CF3 |
| 1-501 | 3,5-dicyanophenyl | H | H | H | CN | H | H | Cl | H | heptafluoroisopropyl | H | CF3 |
| 1-502 | 4-cyano-2-fluorophenyl | H | H | H | CN | H | H | Cl | H | heptafluoroisopropyl | H | CF3 |
| 1-503 | 2-chloro-4-cyanophenyl | H | H | H | CN | H | H | Cl | H | heptafluoroisopropyl | H | CF3 |
| 1-504 | pyridin-3-yl | H | H | H | CN | H | H | Cl | H | heptafluoroisopropyl | H | CF3 |
| 1-505 | 2-fluoropyridin-3-yl | H | H | H | CN | H | H | Cl | H | pentafluoroethyl | H | CF3 |
| 1-506 | 2-chloropyridin-3-yl | H | H | H | CN | H | H | Cl | H | heptafluoroisopropyl | H | CF3 |
| 1-507 | 2-bromopyridin-3-yl | H | H | H | CN | H | H | Cl | H | heptafluoroisopropyl | H | CF3 |
| 1-508 | 2-iodopyridin-3-yl | H | H | H | CN | H | H | Cl | H | heptafluoroisopropyl | H | CF3 |
| 1-509 | 2-(trifluoromethyl)pyridin-3-yl | H | H | H | CN | H | H | Cl | H | heptafluoroisopropyl | H | CF3 |
| 1-510 | 2-nitropyridin-3-yl | H | H | H | CN | H | H | Cl | H | heptafluoroisopropyl | H | CF3 |
| 1-511 | 2-cyanopyridin-3-yl | H | H | H | CN | H | H | Cl | H | heptafluoroisopropyl | H | CF3 |
| 1-512 | 6-fluoropyridin-3-yl | H | H | H | CN | H | H | Cl | H | heptafluoroisopropyl | H | CF3 |
| 1-513 | 6-chloropyridin-3-yl | H | H | H | CN | H | H | Cl | H | heptafluoroisopropyl | H | CF3 |
| 1-514 | 6-bromopyridin-3-yl | H | H | H | CN | H | H | Cl | H | heptafluoroisopropyl | H | CF3 |
| 1-515 | 6-iodopyridin-3-yl | H | H | H | CN | H | H | Cl | H | heptafluoroisopropyl | H | CF3 |
| 1-516 | 6-(trifluoromethyl)pyridin-3-yl | H | H | H | CN | H | H | Cl | H | heptafluoroisopropyl | H | CF3 |
| 1-517 | 6-nitropyridin-3-yl | H | H | H | CN | H | H | Cl | H | heptafluoroisopropyl | H | CF3 |
| 1-518 | 6-cyanopyridin-3-yl | H | H | H | CN | H | H | Cl | H | heptafluoroisopropyl | H | CF3 |
| 1-519 | 5-fluoropyridin-3-yl | H | H | H | CN | H | H | Cl | H | heptafluoroisopropyl | H | C2F5 |
| 1-520 | 5-chloropyridin-3-yl | H | H | H | CN | H | H | Cl | H | heptafluoroisopropyl | H | CF3 |
| 1-521 | 5-bromopyridin-3-yl | H | H | H | CN | H | H | Cl | H | heptafluoroisopropyl | H | n-C3F7 |
| 1-522 | 5-iodopyridin-3-yl | H | H | H | CN | H | H | Cl | H | heptafluoroisopropyl | H | i-C3F7 |
| 1-523 | 5-(trifluoromethyl)pyridin-3-yl | H | H | H | CN | H | H | Cl | H | heptafluoroisopropyl | H | CF3 |
| 1-524 | 5-nitropyridin-3-yl | H | H | H | CN | H | H | Cl | H | pentafluoroethyl | H | CF3 |
| 1-525 | 5-cyanopyridin-3-yl | H | H | H | CN | H | H | Cl | H | heptafluoroisopropyl | H | CF3 |
| 1-526 | 4-fluoropyridin-3-yl | H | H | H | CN | H | H | Cl | H | pentafluoroethyl | H | CF3 |
| 1-527 | 4-chloropyridin-3-yl | H | H | H | CN | H | H | Cl | H | heptafluoroisopropyl | H | CF3 |
| 1-528 | 4-bromopyridin-3-yl | H | H | H | CN | H | H | Cl | H | heptafluoroisopropyl | H | n-C3F7 |
| 1-529 | 4-iodopyridin-3-yl | H | H | H | CN | H | H | Cl | H | heptafluoroisopropyl | H | i-C3F7 |
| 1-530 | 4-(trifluoromethyl)pyridin-3-yl | H | H | H | CN | H | H | Cl | H | heptafluoroisopropyl | H | CF3 |
| 1-531 | 4-nitropyridin-3-yl | H | H | H | CN | H | H | Cl | H | heptafluoroisopropyl | H | CF3 |
| 1-532 | 4-cyanopyridin-3-yl | H | H | H | CN | H | H | Cl | H | heptafluoroisopropyl | H | CF3 |
| 1-533 | 2,6-dichloropyridin-3-yl | H | H | H | CN | H | H | Cl | H | heptafluoroisopropyl | H | CF3 |
| 1-534 | pyridin-3-yl N-oxide | H | H | H | CN | H | H | Cl | H | heptafluoroisopropyl | H | CF3 |
| 1-535 | pyridin-4-yl | H | H | H | CN | H | H | Cl | H | heptafluoroisopropyl | H | CF3 |
| 1-536 | 2-chloropyridin-4-yl | H | H | H | CN | H | H | Cl | H | heptafluoroisopropyl | H | CF3 |
| 1-537 | 3-bromopyridin-4-yl | H | H | H | CN | H | H | Cl | H | heptafluoroisopropyl | H | CF3 |
| 1-538 | 3,5-dichloropyridin-4-yl | H | H | H | CN | H | H | Cl | H | heptafluoroisopropyl | H | CF3 |
| 1-539 | 3-(trifluoromethyl)pyridin-4-yl | H | H | H | CN | H | H | Cl | H | heptafluoroisopropyl | H | CF3 |
| 1-540 | 2,6-dicyanopyridin-4-yl | H | H | H | CN | H | H | Cl | H | heptafluoroisopropyl | H | CF3 |
| 1-541 | pyridin-4-yl N-oxide | H | H | H | CN | H | H | Cl | H | heptafluoroisopropyl | H | CF3 |
| 1-542 | pyridin-2-yl | H | H | H | CN | H | H | Cl | H | heptafluoroisopropyl | H | CF3 |

**Table 1 (3)**

| compound number | Q₁ | R₁ | R₂ | X₁ | X₂ | X₃ | ×₄ | Y₁ | Y₂ | Y₂ | Y₄ | Y₅ |
|---|---|---|---|---|---|---|---|---|---|---|---|---|
| 1-543 | 3-chloropyridin-2-yl | H | H | H | CN | H | H | Cl | H | heptafluoroisopropyl | H | CF3 |
| 1-544 | 4-bromopyridin-2-yl | H | H | H | CN | H | H | Cl | H | heptafluoroisopropyl | H | CF3 |
| 1-545 | 5-iodopyridin-2-yl | H | H | H | CN | H | H | Cl | H | heptafluoroisopropyl | H | CF3 |
| 1-546 | 6-chloropyridin-2-yl | H | H | H | CN | H | H | Cl | H | heptafluoroisopropyl | H | CF3 |
| 1-547 | 4-cyanopyridin-2-yl | H | H | H | CN | H | H | Cl | H | pentafluoroethyl | H | CF3 |
| 1-548 | phenyl | H | H | H | CN | H | H | F | H | nonafluoro-2-butyl | H | CF3 |
| 1-549 | phenyl | H | H | H | CN | H | H | Cl | H | nonafluoro-2-butyl | H | CF3 |
| 1-550 | 2-fluorophenyl | H | H | H | CN | H | H | Cl | H | nonafluoro-2-butyl | H | CF3 |
| 1-551 | 3-fluorophenyl | H | H | H | CN | H | H | Cl | H | nonafluoro-2-butyl | H | CF3 |
| 1-552 | 4-fluorophenyl | H | H | H | CN | H | H | Cl | H | nonafluoro-2-butyl | H | CF3 |
| 1-553 | 2-chlorophenyl | H | H | H | CN | H | H | Cl | H | nonafluoro-2-butyl | H | CF3 |
| 1-554 | 3-chlorophenyl | H | H | H | CN | H | H | Cl | H | nonafluoro-2-butyl | H | CF3 |
| 1-555 | 4-chlorophenyl | H | H | H | CN | H | H | Cl | H | nonafluoro-2-butyl | H | CF3 |
| 1-556 | 2-bromophenyl | H | H | H | CN | H | H | Cl | H | nonafluoro-2-butyl | H | CF3 |
| 1-557 | 3-bromophenyl | H | H | H | CN | H | H | Cl | H | nonafluoro-2-butyl | H | CF3 |
| 1-558 | 4-bromophenyl | H | H | H | CN | H | H | Cl | H | nonafluoro-2-butyl | H | CF3 |
| 1-559 | 2-iodophenyl | H | H | H | CN | H | H | Cl | H | nonafluoro-2-butyl | H | CF3 |
| 1-560 | 3-iodophenyl | H | H | H | CN | H | H | Cl | H | nonafluoro-2-butyl | H | CF3 |
| 1-561 | 4-iodophenyl | H | H | H | CN | H | H | Cl | H | nonafluoro-2-butyl | H | CF3 |
| 1-562 | (2-trifluoromethyl)phenyl | H | H | H | CN | H | H | Cl | H | nonafluoro-2-butyl | H | C2F5 |
| 1-563 | (3-trifluoromethyl)phenyl | H | H | H | CN | H | H | Cl | H | nonafluoro-2-butyl | H | CF3 |
| 1-564 | (4-trifluoromethyl)phenyl | H | H | H | CN | H | H | Cl | H | nonafluoro-2-butyl | H | CF3 |
| 1-565 | 2-nitrophenyl | H | H | H | CN | H | H | Cl | H | nonafluoro-2-butyl | H | CF3 |
| 1-566 | 3-nitrophenyl | H | H | H | CN | H | H | Cl | H | nonafluoro-2-butyl | H | CF3 |
| 1-567 | 4-nitrophenyl | H | H | H | CN | H | H | Cl | H | nonafluoro-2-butyl | H | CF3 |
| 1-568 | 2-cyanophenyl | H | H | H | CN | H | H | Cl | H | nonafluoro-2-butyl | H | CF3 |
| 1-569 | 3-cyanophenyl | H | H | H | CN | H | H | Cl | H | nonafluoro-2-butyl | H | CF3 |
| 1-570 | 4-cyanophenyl | H | H | H | CN | H | H | Cl | H | nonafluoro-2-butyl | H | CF3 |
| 1-571 | 2,6-difluorophenyl | H | H | H | CN | H | H | Cl | H | nonafluoro-2-butyl | H | CF3 |
| 1-572 | 3,4-dichlorophenyl | H | H | H | CN | H | H | Cl | H | nonafluoro-2-butyl | H | CF3 |
| 1-573 | 2,4-dichlorophenyl | H | H | H | CN | H | H | Cl | H | nonafluoro-2-butyl | H | CF3 |
| 1-574 | 2-chloro-4-fluorophenyl | H | H | H | CN | H | H | Cl | H | nonafluoro-2-butyl | H | CF3 |
| 1-575 | 2-chloro-4,5-difluorophenyl | H | H | H | CN | H | H | Cl | H | nonafluoro-2-butyl | H | CF3 |
| 1-576 | 4-bromo-2-chlorophenyl | H | H | H | CN | H | H | Cl | H | nonafluoro-2-butyl | H | CF3 |
| 1-577 | 2-bromo-4-chlorophenyl | H | H | H | CN | H | H | Cl | H | nonafluoro-2-butyl | H | CF3 |
| 1-578 | 2-bromo-4-fluorophenyl | H | H | H | CN | H | H | Cl | H | nonafluoro-2-butyl | H | CF3 |
| 1-579 | 2-chloro-4-nitrophenyl | H | H | H | CN | H | H | Cl | H | nonafluoro-2-butyl | H | i-C3F7 |
| 1-580 | 3,5-dicyanophenyl | H | H | H | CN | H | H | Cl | H | nonafluoro-2-butyl | H | CF3 |
| 1-581 | 4-cyano-2-fluorophenyl | H | H | H | CN | H | H | Cl | H | nonafluoro-2-butyl | H | CF3 |
| 1-582 | 2-chloro-4-cyanophenyl | H | H | H | CN | H | H | Cl | H | nonafluoro-2-butyl | H | CF3 |
| 1-583 | pyridin-3-yl | H | H | H | CN | H | H | Cl | H | nonafluoro-2-butyl | H | CF3 |
| 1-584 | 2-fluoropyridin-3-yl | H | H | H | CN | H | H | Cl | H | nonafluoro-2-butyl | H | CF3 |
| 1-585 | 2-chloropyridin-3-yl | H | H | H | CN | H | H | Cl | H | nonafluoro-2-butyl | H | CF3 |
| 1-586 | 2-bromopyridin-3-yl | H | H | H | CN | H | H | Cl | H | nonafluoro-2-butyl | H | CF3 |
| 1-587 | 2-iodopyridin-3-yl | H | H | H | CN | H | H | Cl | H | nonafluoro-2-butyl | H | CF3 |
| 1-588 | 2-(trifluoromethyl)pyridin-3-yl | H | H | H | CN | H | H | Cl | H | nonafluoro-2-butyl | H | CF3 |
| 1-589 | 2-nitropyridin-3-yl | H | H | H | CN | H | H | Cl | H | nonafluoro-2-butyl | H | CF3 |
| 1-590 | 2-cyanopyridin-3-yl | H | H | H | CN | H | H | Cl | H | nonafluoro-2-butyl | H | CF3 |
| 1-591 | 6-fluoropyridin-3-yl | H | H | H | CN | H | H | Cl | H | nonafluoro-2-butyl | H | CF3 |
| 1-592 | 6-chloropyridin-3-yl | H | H | H | CN | H | H | Cl | H | nonafluoro-2-butyl | H | CF3 |
| 1-593 | 6-bromopyridin-3-yl | H | H | H | CN | H | H | Cl | H | nonafluoro-2-butyl | H | CF3 |
| 1-594 | 6-iodopyridin-3-yl | H | H | H | CN | H | H | Cl | H | nonafluoro-2-butyl | H | CF3 |
| 1-595 | 6-(trifluoromethyl)pyridin-3-yl | H | H | H | CN | H | H | Cl | H | nonafluoro-2-butyl | H | CF3 |
| 1-596 | 6-nitropyridin-3-yl | H | H | H | CN | H | H | Cl | H | nonafluoro-2-butyl | H | CF3 |
| 1-597 | 6-cyanopyridin-3-yl | H | H | H | CN | H | H | Cl | H | nonafluoro-2-butyl | H | CF3 |
| 1-598 | 5-fluoropyridin-3-yl | H | H | H | CN | H | H | Cl | H | nonafluoro-2-butyl | H | C2F5 |
| 1-599 | 5-chloropyridin-3-yl | H | H | H | CN | H | H | Cl | H | nonafluoro-2-butyl | H | CF3 |
| 1-600 | 5-bromopyridin-3-yl | H | H | H | CN | H | H | Cl | H | nonafluoro-2-butyl | H | n-C3F7 |
| 1-601 | 5-iodopyridin-3-yl | H | H | H | CN | H | H | Cl | H | nonafluoro-2-butyl | H | i-C3F7 |
| 1-602 | 5-(trifluoromethyl)pyridin-3-yl | H | H | H | CN | H | H | Cl | H | nonafluoro-2-butyl | H | CF3 |
| 1-603 | 5-nitropyridin-3-yl | H | H | H | CN | H | H | Cl | H | nonafluoro-2-butyl | H | CF3 |
| 1-604 | 5-cyanopyridin-3-yl | H | H | H | CN | H | H | Cl | H | nonafluoro-2-butyl | H | CF3 |
| 1-605 | 4-fluoropyridin-3-yl | H | H | H | CN | H | H | Cl | H | nonafluoro-2-butyl | H | C2F5 |
| 1-606 | 4-chloropyridin-3-yl | H | H | H | CN | H | H | Cl | H | nonafluoro-2-butyl | H | CF3 |
| 1-607 | 4-bromopyridin-3-yl | H | H | H | CN | H | H | Cl | H | nonafluoro-2-butyl | H | CF3 |

**Table 1 (4)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 1-608 | 4-iodopyridin-3-yl | H | H | H | CN | H | H | Cl | H | nonafluoro-2-butyl | H | CF3 |
| 1-609 | 4-(trifluoromethyl)pyridin-3-yl | H | H | H | CN | H | H | Cl | H | nonafluoro-2-butyl | H | n-C3F7 |
| 1-610 | 4-nitropyridin-3-yl | H | H | H | CN | H | H | Cl | H | nonafluoro-2-butyl | H | i-C3F7 |
| 1-611 | 4-cyanopyridin-3-yl | H | H | H | CN | H | H | Cl | H | nonafluoro-2-butyl | H | CF3 |
| 1-612 | 2,6-dichloropyridin-3-yl | H | H | H | CN | H | H | Cl | H | nonafluoro-2-butyl | H | CF3 |
| 1-613 | pyridin-3-yl N-oxide | H | H | H | CN | H | H | Cl | H | nonafluoro-2-butyl | H | CF3 |
| 1-614 | pyridin-4-yl | H | H | H | CN | H | H | Cl | H | nonafluoro-2-butyl | H | CF3 |
| 1-615 | 2-chloropyridin-4-yl | H | H | H | CN | H | H | Cl | H | nonafluoro-2-butyl | H | CF3 |
| 1-616 | 3-bromopyridin-4-yl | H | H | H | CN | H | H | Cl | H | nonafluoro-2-butyl | H | CF3 |
| 1-617 | 3,5-dichloropyridin-4-yl | H | H | H | CN | H | H | Cl | H | nonafluoro-2-butyl | H | CF3 |
| 1-618 | 3-(trifluoromethyl)pyridin-4-yl | H | H | H | CN | H | H | Cl | H | nonafluoro-2-butyl | H | CF3 |
| 1-619 | 2,6-dicyanopyridin-4-yl | H | H | H | CN | H | H | Cl | H | nonafluoro-2-butyl | H | CF3 |
| 1-620 | pyridin-4-yl N-oxide | H | H | H | CN | H | H | Cl | H | nonafluoro-2-butyl | H | CF3 |
| 1-621 | pyridin-2-yl | H | H | H | CN | H | H | Cl | H | nonafluoro-2-butyl | H | CF3 |
| 1-622 | 3-chloropyridin-2-yl | H | H | H | CN | H | H | Cl | H | nonafluoro-2-butyl | H | CF3 |
| 1-623 | 4-bromopyridin-2-yl | H | H | H | CN | H | H | Cl | H | nonafluoro-2-butyl | H | CF3 |
| 1-624 | 5-iodopyridin-2-yl | H | H | H | C | H | H | Cl | H | nonafluoro-2-butyl | H | CF3 |
| 1-625 | 6-chloropyridin-2-yl | H | H | H | CN | H | H | Cl | H | nonafluoro-2-butyl | H | CF3 |
| 1-626 | 4-cyanopyridin-2-yl | H | H | H | CN | H | H | Cl | H | nonafluoro-2-butyl | H | C2F5 |
| 1-627 | phenyl | H | H | H | CN | H | H | Br | H | heptafluoroisopropyl | H | CF3 |
| 1-628 | 2-fluorophenyl | H | H | H | CN | H | H | Br | H | heptafluoroisopropyl | H | CF3 |
| 1-629 | 3-fluorophenyl | H | H | H | CN | H | H | Br | H | heptafluoroisopropyl | H | CF3 |
| 1-630 | 4-fluorophenyl | H | H | H | CN | H | H | Br | H | heptafluoroisopropyl | H | CF3 |
| 1-631 | 2-chlorophenyl | H | H | H | CN | H | H | Br | H | heptafluoroisopropyl | H | CF3 |
| 1-632 | 3-chlorophenyl | H | H | H | CN | H | H | Br | H | heptafluoroisopropyl | H | CF3 |
| 1-633 | 4-chlorophenyl | H | H | H | CN | H | H | Br | H | heptafluoroisopropyl | H | CF3 |
| 1-634 | 2-bromophenyl | H | H | H | CN | H | H | Br | H | heptafluoroisopropyl | H | CF3 |
| 1-635 | 3-bromophenyl | H | H | H | CN | H | H | Br | H | heptafluoroisopropyl | H | CF3 |
| 1-636 | 4-bromophenyl | H | H | H | CN | H | H | Br | H | heptafluoroisopropyl | H | CF3 |
| 1-637 | 2-iodophenyl | H | H | H | CN | H | H | Br | H | heptafluoroisopropyl | H | CF3 |
| 1-638 | 3-iodophenyl | H | H | H | CN | H | H | Br | H | heptafluoroisopropyl | H | CF3 |
| 1-639 | 4-iodophenyl | H | H | H | CN | H | H | Br | H | heptafluoroisopropyl | H | CF3 |
| 1-640 | (2-trifluoromethyl)phenyl | H | H | H | CN | H | H | Br | H | heptafluoroisopropyl | H | CF3 |
| 1-641 | (3-trifluoromethyl)phenyl | H | H | H | CN | H | H | Br | H | heptafluoroisopropyl | H | CF3 |
| 1-642 | (4-trifluoromethyl)phenyl | H | H | H | CN | H | H | Br | H | heptafluoroisopropyl | H | CF3 |
| 1-643 | 2-nitrophenyl | H | H | H | CN | H | H | Br | H | heptafluoroisopropyl | H | CF3 |
| 1-644 | 3-nitrophenyl | H | H | H | CN | H | H | Br | H | heptafluoroisopropyl | H | CF3 |
| 1-645 | 4-nitrophenyl | H | H | H | CN | H | H | Br | H | heptafluoroisopropyl | H | CF3 |
| 1-646 | 2-cyanophenyl | H | H | H | CN | H | H | Br | H | heptafluoroisopropyl | H | CF3 |
| 1-647 | 3-cyanophenyl | H | H | H | CN | H | H | Br | H | heptafluoroisopropyl | H | CF3 |
| 1-648 | 4-cyanophenyl | H | H | H | CN | H | H | Br | H | heptafluoroisopropyl | H | CF3 |
| 1-649 | 2,6-difluorophenyl | H | H | H | CN | H | H | Br | H | heptafluoroisopropyl | H | CF3 |
| 1-650 | 3,4-dichlorophenyl | H | H | H | CN | H | H | Br | H | heptafluoroisopropyl | H | CF3 |
| 1-651 | 2,4-dichlorophenyl | H | H | H | CN | H | H | Br | H | heptafluoroisopropyl | H | CF3 |
| 1-652 | 2-chloro-4-fluorophenyl | H | H | H | CN | H | H | Br | H | heptafluoroisopropyl | H | CF3 |
| 1-653 | 2-chloro-4,5-difluorophenyl | H | H | H | CN | H | H | Br | H | heptafluoroisopropyl | H | CF3 |
| 1-654 | 4-bromo-2-chlorophenyl | H | H | H | CN | H | H | Br | H | heptafluoroisopropyl | H | CF3 |
| 1-655 | 2-bromo-4-chlorophenyl | H | H | H | CN | H | H | Br | H | heptafluoroisopropyl | H | CF3 |
| 1-656 | 2-bromo-4-fluorophenyl | H | H | H | CN | H | H | Br | H | heptafluoroisopropyl | H | CF3 |
| 1-657 | 2-chloro-4-nitrophenyl | H | H | H | CN | H | H | Br | H | heptafluoroisopropyl | H | CF3 |
| 1-658 | 3,5-dicyanophenyl | H | H | H | CN | H | H | Br | H | heptafluoroisopropyl | H | CF3 |
| 1-659 | 4-cyano-2-fluorophenyl | H | H | H | CN | H | H | Br | H | heptafluoroisopropyl | H | CF3 |
| 1-660 | 2-chloro-4-cyanophenyl | H | H | H | CN | H | H | Br | H | heptafluoroisopropyl | H | CF3 |
| 1-661 | pyridin-3-yl | H | H | H | CN | H | H | Br | H | heptafluoroisopropyl | H | CF3 |
| 1-662 | 2-fluoropyridin-3-yl | H | H | H | CN | H | H | Br | H | heptafluoroisopropyl | H | CF3 |
| 1-663 | 2-chloropyridin-3-yl | H | H | H | CN | H | H | Br | H | heptafluoroisopropyl | H | CF3 |
| 1-664 | 2-bromopyridin-3-yl | H | H | H | CN | H | H | Br | H | heptafluoroisopropyl | H | CF3 |
| 1-665 | 2-iodopyridin-3-yl | H | H | H | CN | H | H | Br | H | heptafluoroisopropyl | H | CF3 |
| 1-666 | 2-(trifluoromethyl)pyridin-3-yl | H | H | H | CN | H | H | Br | H | heptafluoroisopropyl | H | CF3 |
| 1-667 | 2-nitropyridin-3-yl | H | H | H | CN | H | H | Br | H | heptafluoroisopropyl | H | CF3 |
| 1-668 | 2-cyanopyridin-3-yl | H | H | H | CN | H | H | Br | H | heptafluoroisopropyl | H | CF3 |
| 1-669 | 6-fluoropyridin-3-yl | H | H | H | CN | H | H | Br | H | heptafluoroisopropyl | H | CF3 |
| 1-670 | 6-chloropyridin-3-yl | H | H | H | CN | H | H | Br | H | heptafluoroisopropyl | H | CF3 |
| 1-671 | 6-bromopyridin-3-yl | H | H | H | CN | H | H | Br | H | heptafluoroisopropyl | H | CF3 |

**Table 1 (5)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 1-672 | 6-iodopyridin-3-yl | H | H | H | CN | H | H | Br | H | heptafluoroisopropyl | H | CF3 |
| 1-673 | 6-(trifluoromethyl)pyridin-3-yl | H | H | H | CN | H | H | Br | H | heptafluoroisopropyl | H | CF3 |
| 1-674 | 6-nitropyridin-3-yl | H | H | H | CN | H | H | Br | H | heptafluoroisopropyl | H | CF3 |
| 1-675 | 6-cyanopyridin-3-yl | H | H | H | CN | H | H | Br | H | heptafluoroisopropyl | H | CF3 |
| 1-676 | 5-fluoropyridin-3-yl | H | H | H | CN | H | H | Br | H | heptafluoroisopropyl | H | CF3 |
| 1-677 | 5-chloropyridin-3-yl | H | H | H | CN | H | H | Br | H | heptafluoroisopropyl | H | CF3 |
| 1-678 | 5-bromopyridin-3-yl | H | H | H | CN | H | H | Br | H | heptafluoroisopropyl | H | CF3 |
| 1-679 | 5-iodopyridin-3-yl | H | H | H | CN | H | H | Br | H | heptafluoroisopropyl | H | CF3 |
| 1-680 | 5-(trifluoromethyl)pyridin-3-yl | H | H | H | CN | H | H | Br | H | heptafluoroisopropyl | H | CF3 |
| 1-681 | 5-nitropyridin-3-yl | H | H | H | CN | H | H | Br | H | heptafluoroisopropyl | H | CF3 |
| 1-682 | 5-cyanopyridin-3-yl | H | H | H | CN | H | H | Br | H | heptafluoroisopropyl | H | CF3 |
| 1-683 | 4-fluoropyridin-3-yl | H | H | H | CN | H | H | Br | H | heptafluoroisopropyl | H | CF3 |
| 1-684 | 4-chloropyridin-3-yl | H | H | H | CN | H | H | Br | H | heptafluoroisopropyl | H | CF3 |
| 1-685 | 4-bromopyridin-3-yl | H | H | H | CN | H | H | Br | H | heptafluoroisopropyl | H | CF3 |
| 1-686 | 4-iodopyridin-3-yl | H | H | H | CN | H | H | Br | H | heptafluoroisopropyl | H | CF3 |
| 1-687 | 4-(trifluoromethyl)pyridin-3-yl | H | H | H | CN | H | H | Br | H | heptafluoroisopropyl | H | CF3 |
| 1-688 | 4-nitropyridin-3-yl | H | H | H | CN | H | H | Br | H | heptafluoroisopropyl | H | CF3 |
| 1-689 | 4-cyanopyridin-3-yl | H | H | H | CN | H | H | Br | H | heptafluoroisopropyl | H | CF3 |
| 1-690 | 2,6-dichloropyridin-3-yl | H | H | H | CN | H | H | Br | H | heptafluoroisopropyl | H | CF3 |
| 1-691 | pyridin-3-yl N-oxide | H | H | H | CN | H | H | Br | H | heptafluoroisopropyl | H | CF3 |
| 1-692 | pyridin-4-yl | H | H | H | CN | H | H | Br | H | heptafluoroisopropyl | H | CF3 |
| 1-693 | 2-chloropyridin-4-yl | H | H | H | CN | H | H | Br | H | heptafluoroisopropyl | H | CF3 |
| 1-694 | 3-bromopyridin-4-yl | H | H | H | CN | H | H | Br | H | heptafluoroisopropyl | H | CF3 |
| 1-695 | 3,5-dichloropyridin-4-yl | H | H | H | CN | H | H | Br | H | heptafluoroisopropyl | H | CF3 |
| 1-696 | 3-(trifluoromethyl)pyridin-4-yl | H | H | H | CN | H | H | Br | H | heptafluoroisopropyl | H | CF3 |
| 1-697 | 2,6-dicyanopyridin-4-yl | H | H | H | CN | H | H | Br | H | heptafluoroisopropyl | H | CF3 |
| 1-698 | pyridin-4-yl N-oxide | H | H | H | CN | H | H | Br | H | heptafluoroisopropyl | H | CF3 |
| 1-699 | pyridin-2-yl | H | H | H | CN | H | H | Br | H | heptafluoroisopropyl | H | CF3 |
| 1-700 | 3-chloropyridin-2-yl | H | H | H | CN | H | H | Br | H | heptafluoroisopropyl | H | CF3 |
| 1-701 | 4-bromopyridin-2-yl | H | H | H | CN | H | H | Br | H | heptafluoroisopropyl | H | CF3 |
| 1-702 | 5-iodopyridin-2-yl | H | H | H | CN | H | H | Br | H | heptafluoroisopropyl | H | CF3 |
| 1-703 | 6-chloropyridin-2-yl | H | H | H | CN | H | H | Br | H | heptafluoroisopropyl | H | CF3 |
| 1-704 | 4-cyanopyridin-2-yl | H | H | H | CN | H | H | Br | H | heptafluoroisopropyl | H | CF3 |
| 1-705 | phenyl | H | H | H | CN | H | H | Br | H | nonafluoro-2-butyl | H | CF3 |
| 1-706 | 2-fluorophenyl | H | H | H | CN | H | H | Br | H | nonafluoro-2-butyl | H | CF3 |
| 1-707 | 3-fluorophenyl | H | H | H | CN | H | H | Br | H | nonafluoro-2-butyl | H | CF3 |
| 1-708 | 4-fluorophenyl | H | H | H | CN | H | H | Br | H | nonafluoro-2-butyl | H | CF3 |
| 1-709 | 2-chlorophenyl | H | H | H | CN | H | H | Br | H | nonafluoro-2-butyl | H | CF3 |
| 1-710 | 3-chlorophenyl | H | H | H | CN | H | H | Br | H | nonafluoro-2-butyl | H | CF3 |
| 1-711 | 4-chlorophenyl | H | H | H | CN | H | H | Br | H | nonafluoro-2-butyl | H | CF3 |
| 1-712 | 2-bromophenyl | H | H | H | CN | H | H | Br | H | nonafluoro-2-butyl | H | CF3 |
| 1-713 | 3-bromophenyl | H | H | H | CN | H | H | Br | H | nonafluoro-2-butyl | H | CF3 |
| 1-714 | 4-bromophenyl | H | H | H | CN | H | H | Br | H | nonafluoro-2-butyl | H | CF3 |
| 1-715 | 2-iodophenyl | H | H | H | CN | H | H | Br | H | nonafluoro-2-butyl | H | CF3 |
| 1-716 | 3-iodophenyl | H | H | H | CN | H | H | Br | H | nonafluoro-2-butyl | H | CF3 |
| 1-717 | 4-iodophenyl | H | H | H | CN | H | H | Br | H | nonafluoro-2-butyl | H | CF3 |
| 1-718 | (2-trifluoromethyl)phenyl | H | H | H | CN | H | H | Br | H | nonafluoro-2-butyl | H | CF3 |
| 1-719 | (3-trifluoromethyl)phenyl | H | H | H | CN | H | H | Br | H | nonafluoro-2-butyl | H | CF3 |
| 1-720 | (4-trifluoromethyl)phenyl | H | H | H | CN | H | H | Br | H | nonafluoro-2-butyl | H | CF3 |
| 1-721 | 2-nitrophenyl | H | H | H | CN | H | H | Br | H | nonafluoro-2-butyl | H | CF3 |
| 1-722 | 3-nitrophenyl | H | H | H | CN | H | H | Br | H | nonafluoro-2-butyl | H | CF3 |
| 1-723 | 4-nitrophenyl | H | H | H | CN | H | H | Br | H | nonafluoro-2-butyl | H | CF3 |
| 1-724 | 2-cyanophenyl | H | H | H | CN | H | H | Br | H | nonafluoro-2-butyl | H | CF3 |
| 1-725 | 3-cyanophenyl | H | H | H | CN | H | H | Br | H | nonafluoro-2-butyl | H | CF3 |
| 1-726 | 4-cyanophenyl | H | H | H | CN | H | H | Br | H | nonafluoro-2-butyl | H | CF3 |
| 1-727 | 2,6-difluorophenyl | H | H | H | CN | H | H | Br | H | nonafluoro-2-butyl | H | CF3 |
| 1-728 | 3,4-dichlorophenyl | H | H | H | CN | H | H | Br | H | nonafluoro-2-butyl | H | CF3 |
| 1-729 | 2,4-dichlorophenyl | H | H | H | CN | H | H | Br | H | nonafluoro-2-butyl | H | CF3 |
| 1-730 | 2-chloro-4-fluorophenyl | H | H | H | CN | H | H | Br | H | nonafluoro-2-butyl | H | CF3 |
| 1-731 | 2-chloro-4,5-difluorophenyl | H | H | H | CN | H | H | Br | H | nonafluoro-2-butyl | H | CF3 |
| 1-732 | 4-bromo-2-chlorophenyl | H | H | H | CN | H | H | Br | H | nonafluoro-2-butyl | H | CF3 |
| 1-733 | 2-bromo-4-chlorophenyl | H | H | H | CN | H | H | Br | H | nonafluoro-2-butyl | H | CF3 |
| 1-734 | 2-bromo-4-fluorophenyl | H | H | H | CN | H | H | Br | H | nonafluoro-2-butyl | H | CF3 |
| 1-735 | 2-chloro-4-nitrophenyl | H | H | H | CN | H | H | Br | H | nonafluoro-2-butyl | H | CF3 |

**Table 1 (6)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 1-736 | 3,5-dicyanophenyl | H | H | H | CN | H | H | Br | H | nonafluoro-2-butyl | H | CF3 |
| 1-737 | 4-cyano-2-fluorophenyl | H | H | H | CN | H | H | Br | H | nonafluoro-2-butyl | H | CF3 |
| 1-738 | 2-chloro-4-cyanophenyl | H | H | H | CN | H | H | Br | H | nonafluoro-2-butyl | H | CF3 |
| 1-739 | pyridin-3-yl | H | H | H | CN | H | H | Br | H | nonafluoro-2-butyl | H | CF3 |
| 1-740 | 2-fluoropyridin-3-yl | H | H | H | CN | H | H | Br | H | nonafluoro-2-butyl | H | CF3 |
| 1-741 | 2-chloropyridin-3-yl | H | H | H | CN | H | H | Br | H | nonafluoro-2-butyl | H | CF3 |
| 1-742 | 2-bromopyridin-3-yl | H | H | H | CN | H | H | Br | H | nonafluoro-2-butyl | H | CF3 |
| 1-743 | 2-iodopyridin-3-yl | H | H | H | CN | H | H | Br | H | nonafluoro-2-butyl | H | CF3 |
| 1-744 | 2-(trifluoromethyl)pyridin-3-yl | H | H | H | CN | H | H | Br | H | nonafluoro-2-butyl | H | CF3 |
| 1-745 | 2-nitropyridin-3yl | H | H | H | CN | H | H | Br | H | nonafluoro-2-butyl | H | CF3 |
| 1-746 | 2-cyanopyridin-3-yl | H | H | H | CN | H | H | Br | H | nonafluoro-2-butyl | H | CF3 |
| 1-747 | 6-fluoropyridin-3-yl | H | H | H | CN | H | H | Br | H | nonafluoro-2-butyl | H | CF3 |
| 1-748 | 6-chloropyridin-3-yl | H | H | H | CN | H | H | Br | H | nonafluoro-2-butyl | H | CF3 |
| 1-749 | 6-bromopyridin-3-yl | H | H | H | CN | H | H | Br | H | nonafluoro-2-butyl | H | CF3 |
| 1-750 | 6-iodopyridin-3yl | H | H | H | CN | H | H | Br | H | nonafluoro-2-butyl | H | CF3 |
| 1-751 | 6-(trifluoromethyl)pyridin-3-yl | H | H | H | CN | H | H | Br | H | nonafluoro-2-butyl | H | CF3 |
| 1-752 | 6-nitropyridin-3yl | H | H | H | CN | H | H | Br | H | nonafluoro-2-butyl | H | CF3 |
| 1-753 | 6-cyanopyridin-3-yl | H | H | H | CN | H | H | Br | H | nonafluoro-2-butyl | H | CF3 |
| 1-754 | 5-fluoropyridin-3-yl | H | H | H | CN | H | H | Br | H | nonafluoro-2-butyl | H | CF3 |
| 1-755 | 5-chloropyridin-3-yl | H | H | H | CN | H | H | Br | H | nonafluoro-2-butyl | H | CF3 |
| 1-756 | 5-bromopyridin-3-yl | H | H | H | CN | H | H | Br | H | nonafluoro-2-butyl | H | CF3 |
| 1-757 | 5-iodopyridin-3-yl | H | H | H | CN | H | H | Br | H | nonafluoro-2-butyl | H | CF3 |
| 1-758 | 5-(trifluoromethyl)pyridin-3-yl | H | H | H | CN | H | H | Br | H | nonafluoro-2-butyl | H | CF3 |
| 1-759 | 5-nitropyridin-3-yl | H | H | H | CN | H | H | Br | H | nonafluoro-2-butyl | H | CF3 |
| 1-760 | 5-cyanopyridin-3-yl | H | H | H | CN | H | H | Br | H | nonafluoro-2-butyl | H | CF3 |
| 1-761 | 4-fluoropyridin-3-yl | H | H | H | CN | H | H | Br | H | nonafluoro-2-butyl | H | CF3 |
| 1-762 | 4-chloropyridin-3-yl | H | H | H | CN | H | H | Br | H | nonafluoro-2-butyl | H | CF3 |
| 1-763 | 4-bromopyridin-3-yl | H | H | H | CN | H | H | Br | H | nonafluoro-2-butyl | H | CF3 |
| 1-764 | 4-iodopyridin-3-yl | H | H | H | CN | H | H | Br | H | nonafluoro-2-butyl | H | CF3 |
| 1-765 | 4-(trifluoromethyl)pyridin-3-yl | H | H | H | CN | H | H | Br | H | nonafluoro-2-butyl | H | CF3 |
| 1-766 | 4-nitropyridin-3-yl | H | H | H | CN | H | H | Br | H | nonafluoro-2-butyl | H | CF3 |
| 1-767 | 4-cyanopyridin-3-yl | H | H | H | CN | H | H | Br | H | nonafluoro-2-butyl | H | CF3 |
| 1-768 | 2,6-dichloropyridin-3-yl | H | H | H | CN | H | H | Br | H | nonafluoro-2-butyl | H | CF3 |
| 1-769 | pyridin-3-yl N-oxide | H | H | H | CN | H | H | Br | H | nonafluoro-2-butyl | H | CF3 |
| 1-770 | pyridin-4-yl | H | H | H | CN | H | H | Br | H | nonafluoro-2-butyl | H | CF3 |
| 1-771 | 2-chloropyridin-4-yl | H | H | H | CN | H | H | Br | H | nonafluoro-2-butyl | H | CF3 |
| 1-772 | 3-bromopyridin-4-yl | H | H | H | CN | H | H | Br | H | nonafluoro-2-butyl | H | CF3 |
| 1-773 | 3,5-dichloropyridin-4-yl | H | H | H | CN | H | H | Br | H | nonafluoro-2-butyl | H | CF3 |
| 1-774 | 3-(trifluoromethyl)pyridin-4-yl | H | H | H | CN | H | H | Br | H | nonafluoro-2-butyl | H | CF3 |
| 1-775 | 2,6-dicyanopyridin-4-yl | H | H | H | CN | H | H | Br | H | nonafluoro-2-butyl | H | CF3 |
| 1-776 | pyridin-4-yl N-oxide | H | H | H | CN | H | H | Br | H | nonafluoro-2-butyl | H | CF3 |
| 1-777 | pyridin-2-yl | H | H | H | CN | H | H | Br | H | nonafluoro-2-butyl | H | CF3 |
| 1-778 | 3-chloropyridin-2-yl | H | H | H | CN | H | H | Br | H | nonafluoro-2-butyl | H | CF3 |
| 1-779 | 4-bromopyridin-2-yl | H | H | H | CN | H | H | Br | H | nonafluoro-2-butyl | H | CF3 |
| 1-780 | 5-iodopyridin-2-yl | H | H | H | CN | H | H | Br | H | nonafluoro-2-butyl | H | CF3 |
| 1-781 | 6-chloropyridin-2-yl | H | H | H | CN | H | H | Br | H | nonafluoro-2-butyl | H | CF3 |
| 1-782 | 4-cyanopyridin-2-yl | H | H | H | CN | H | H | Br | H | nonafluoro-2-butyl | H | CF3 |
| 1-783 | phenyl | H | H | H | CN | H | H | I | H | heptafluoroisopropyl | H | CF3 |
| 1-784 | 2-fluorophenyl | H | H | H | CN | H | H | I | H | heptafluoroisopropyl | H | CF3 |
| 1-785 | 3-fluorophenyl | H | H | H | CN | H | H | I | H | heptafluoroisopropyl | H | CF3 |
| 1-786 | 4-fluorophenyl | H | H | H | CN | H | H | I | H | heptafluoroisopropyl | H | CF3 |
| 1-787 | 2-chlorophenyl | H | H | H | CN | H | H | I | H | heptafluoroisopropyl | H | CF3 |
| 1-788 | 3-chlorophenyl | H | H | H | CN | H | H | I | H | heptafluoroisopropyl | H | CF3 |
| 1-789 | 4-chlorophenyl | H | H | H | CN | H | H | I | H | heptafluoroisopropyl | H | CF3 |
| 1-790 | 2-bromophenyl | H | H | H | CN | H | H | I | H | heptafluoroisopropyl | H | CF3 |
| 1-791 | 3-bromophenyl | H | H | H | CN | H | H | I | H | heptafluoroisopropyl | H | CF3 |
| 1-792 | 4-bromophenyl | H | H | H | CN | H | H | I | H | heptafluoroisopropyl | H | CF3 |
| 1-793 | 2-iodophenyl | H | H | H | CN | H | H | I | H | heptafluoroisopropyl | H | CF3 |
| 1-794 | 3-iodophenyl | H | H | H | CN | H | H | I | H | heptafluoroisopropyl | H | CF3 |
| 1-795 | 4-iodophenyl | H | H | H | CN | H | H | I | H | heptafluoroisopropyl | H | CF3 |
| 1-796 | (2-trifluoromethyl)phenyl | H | H | H | CN | H | H | I | H | heptafluoroisopropyl | H | CF3 |
| 1-797 | (3-trifluoromethyl)phenyl | H | H | H | CN | H | H | I | H | heptafluoroisopropyl | H | CF3 |
| 1-798 | (4-trifluoromethyl)phenyl | H | H | H | CN | H | H | I | H | heptafluoroisopropyl | H | CF3 |
| 1-799 | 2-nitrophenyl | H | H | H | CN | H | H | I | H | heptafluoroisopropyl | H | CF3 |

**Table 1 (7)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 1-800 | 3-nitrophenyl | H | H | H | CN | H | H | I | H | heptafluoroisopropyl | H | CF3 |
| 1-801 | 4-nitrophenyl | H | H | H | CN | H | H | I | H | heptafluoroisopropyl | H | CF3 |
| 1-802 | 2-cyanophenyl | H | H | H | CN | H | H | I | H | heptafluoroisopropyl | H | CF3 |
| 1-803 | 3-cyanophenyl | H | H | H | CN | H | H | I | H | heptafluoroisopropyl | H | CF3 |
| 1-804 | 4-cyanophenyl | H | H | H | CN | H | H | I | H | heptafluoroisopropyl | H | CF3 |
| 1-805 | 2,6-difluorophenyl | H | H | H | CN | H | H | I | H | heptafluoroisopropyl | H | CF3 |
| 1-806 | 3,4-dichlorophenyl | H | H | H | CN | H | H | I | H | heptafluoroisopropyl | H | CF3 |
| 1-807 | 2,4-dichlorophenyl | H | H | H | CN | H | H | I | H | heptafluoroisopropyl | H | CF3 |
| 1-808 | 2-chloro-4-fluorophenyl | H | H | H | CN | H | H | I | H | heptafluoroisopropyl | H | CF3 |
| 1-809 | 2-chloro-4,5-difluorophenyl | H | H | H | CN | H | H | I | H | heptafluoroisopropyl | H | CF3 |
| 1-810 | 4-bromo-2-chlorophenyl | H | H | H | CN | H | H | I | H | heptafluoroisopropyl | H | CF3 |
| 1-811 | 2-bromo-4-chlorophenyl | H | H | H | CN | H | H | I | H | heptafluoroisopropyl | H | CF3 |
| 1-812 | 2-bromo-4-fluorophenyl | H | H | H | CN | H | H | I | H | heptafluoroisopropyl | H | CF3 |
| 1-813 | 2-chloro-4-nitrophenyl | H | H | H | CN | H | H | I | H | heptafluoroisopropyl | H | CF3 |
| 1-814 | 3,5-dicyanophenyl | H | H | H | CN | H | H | I | H | heptafluoroisopropyl | H | CF3 |
| 1-815 | 4-cyano-2-fluorophenyl | H | H | H | CN | H | H | I | H | heptafluoroisopropyl | H | CF3 |
| 1-816 | 2-chloro-4-cyanophenyl | H | H | H | CN | H | H | I | H | heptafluoroisopropyl | H | CF3 |
| 1-817 | pyridin-3-yl | H | H | H | CN | H | H | I | H | heptafluoroisopropyl | H | CF3 |
| 1-818 | 2-fluoropyridin-3-yl | H | H | H | CN | H | H | I | H | heptafluoroisopropyl | H | CF3 |
| 1-819 | 2-chloropyridin-3-yl | H | H | H | CN | H | H | I | H | heptafluoroisopropyl | H | CF3 |
| 1-820 | 2-bromopyridin-3-yl | H | H | H | CN | H | H | I | H | heptafluoroisopropyl | H | CF3 |
| 1-821 | 2-iodopyridin-3-yl | H | H | H | CN | H | H | I | H | heptafluoroisopropyl | H | CF3 |
| 1-822 | 2-(trifluoromethyl)pyridin-3-yl | H | H | H | CN | H | H | I | H | heptafluoroisopropyl | H | CF3 |
| 1-823 | 2-nitropyridin-3-yl | H | H | H | CN | H | H | I | H | heptafluoroisopropyl | H | CF3 |
| 1-824 | 2-cyanopyridin-3-yl | H | H | H | CN | H | H | I | H | heptafluoroisopropyl | H | CF3 |
| 1-825 | 6-fluoropyridin-3-yl | H | H | H | CN | H | H | I | H | heptafluoroisopropyl | H | CF3 |
| 1-826 | 6-chloropyridin-3-yl | H | H | H | CN | H | H | I | H | heptafluoroisopropyl | H | CF3 |
| 1-827 | 6-bromopyridin-3-yl | H | H | H | CN | H | H | I | H | heptafluoroisopropyl | H | CF3 |
| 1-828 | 6-iodopyridin-3-yl | H | H | H | CN | H | H | I | H | heptafluoroisopropyl | H | CF3 |
| 1-829 | 6-(trifluoromethyl)pyridin-3-yl | H | H | H | CN | H | H | I | H | heptafluoroisopropyl | H | CF3 |
| 1-830 | 6-nitropyridin-3-yl | H | H | H | CN | H | H | I | H | heptafluoroisopropyl | H | CF3 |
| 1-831 | 6-cyanopyridin-3-yl | H | H | H | CN | H | H | I | H | heptafluoroisopropyl | H | CF3 |
| 1-832 | 5-fluoropyridin-3-yl | H | H | H | CN | H | H | I | H | heptafluoroisopropyl | H | CF3 |
| 1-833 | 5-chloropyridin-3-yl | H | H | H | CN | H | H | I | H | heptafluoroisopropyl | H | CF3 |
| 1-834 | 5-bromopyridin-3-yl | H | H | H | CN | H | H | I | H | heptafluoroisopropyl | H | CF3 |
| 1-835 | 5-iodopyridin-3-yl | H | H | H | CN | H | H | I | H | heptafluoroisopropyl | H | CF3 |
| 1-836 | 5-(trifluoromethyl)pyridin-3-yl | H | H | H | CN | H | H | I | H | heptafluoroisopropyl | H | CF3 |
| 1-837 | 5-nitropyridin-3-yl | H | H | H | CN | H | H | I | H | heptafluoroisopropyl | H | CF3 |
| 1-838 | 5-cyanopyridin-3-yl | H | H | H | CN | H | H | I | H | heptafluoroisopropyl | H | CF3 |
| 1-839 | 4-fluoropyridin-3-yl | H | H | H | CN | H | H | I | H | heptafluoroisopropyl | H | CF3 |
| 1-840 | 4-chloropyridin-3-yl | H | H | H | CN | H | H | I | H | heptafluoroisopropyl | H | CF3 |
| 1-841 | 4-bromopyridin-3-yl | H | H | H | CN | H | H | I | H | heptafluoroisopropyl | H | CF3 |
| 1-842 | 4-iodopyridin-3-yl | H | H | H | CN | H | H | I | H | heptafluoroisopropyl | H | CF3 |
| 1-843 | 4-(trifluoromethyl)pyridin-3-yl | H | H | H | CN | H | H | I | H | heptafluoroisopropyl | H | CF3 |
| 1-844 | 4-nitropyridin-3-yl | H | H | H | CN | H | H | I | H | heptafluoroisopropyl | H | CF3 |
| 1-845 | 4-cyanopyridin-3-yl | H | H | H | CN | H | H | I | H | heptafluoroisopropyl | H | CF3 |
| 1-846 | 2,6-dichloropyridin-3-yl | H | H | H | CN | H | H | I | H | heptafluoroisopropyl | H | CF3 |
| 1-847 | pyridin-3-yl N-oxide | H | H | H | CN | H | H | I | H | heptafluoroisopropyl | H | CF3 |
| 1-848 | pyridin-4-yl | H | H | H | CN | H | H | I | H | heptafluoroisopropyl | H | CF3 |
| 1-849 | 2-chloropyridin-4-yl | H | H | H | CN | H | H | I | H | heptafluoroisopropyl | H | CF3 |
| 1-850 | 3-bromopyridin-4-yl | H | H | H | CN | H | H | I | H | heptafluoroisopropyl | H | CF3 |
| 1-851 | 3,5-dichloropyridin-4-yl | H | H | H | CN | H | H | I | H | heptafluoroisopropyl | H | CF3 |
| 1-852 | 3-(trifluoromethyl)pyridin-4-yl | H | H | H | CN | H | H | I | H | heptafluoroisopropyl | H | CF3 |
| 1-853 | 2,6-dicyanopyridin-4-yl | H | H | H | CN | H | H | I | H | heptafluoroisopropyl | H | CF3 |
| 1-854 | pyridin-4-yl N-oxide | H | H | H | CN | H | H | I | H | heptafluoroisopropyl | H | CF3 |
| 1-855 | pyridin-2-yl | H | H | H | CN | H | H | I | H | heptafluoroisopropyl | H | CF3 |
| 1-856 | 3-chloropyridin-2-yl | H | H | H | CN | H | H | I | H | heptafluoroisopropyl | H | CF3 |
| 1-857 | 4-bromopyridin-2-yl | H | H | H | CN | H | H | I | H | heptafluoroisopropyl | H | CF3 |
| 1-858 | 5-iodopyridin-2-yl | H | H | H | CN | H | H | I | H | heptafluoroisopropyl | H | CF3 |
| 1-859 | 6-chloropyridin-2-yl | H | H | H | CN | H | H | I | H | heptafluoroisopropyl | H | CF3 |
| 1-860 | 4-cyanopyridin-2-yl | H | H | H | CN | H | H | I | H | heptafluoroisopropyl | H | CF3 |
| 1-861 | phenyl | H | H | H | CN | H | H | I | H | nonafluoro-2-butyl | H | CF3 |
| 1-862 | 2-fluorophenyl | H | H | H | CN | H | H | I | H | nonafluoro-2-butyl | H | CF3 |

**Table 1 (8)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 1-863 | 3-fluorophenyl | H | H | H | CN | H | H | I | H | nonafluoro-2-butyl | H | CF3 |
| 1-864 | 4-fluorophenyl | H | H | H | CN | H | H | I | H | nonafluoro-2-butyl | H | CF3 |
| 1-865 | 2-chlorophenyl | H | H | H | CN | H | H | I | H | nonafluoro-2-butyl | H | CF3 |
| 1-866 | 3-chlorophenyl | H | H | H | CN | H | H | I | H | nonafluoro-2-butyl | H | CF3 |
| 1-867 | 4-chlorophenyl | H | H | H | CN | H | H | I | H | nonafluoro-2-butyl | H | CF3 |
| 1-868 | 2-bromophenyl | H | H | H | CN | H | H | I | H | nonafluoro-2-butyl | H | CF3 |
| 1-869 | 3-bromophenyl | H | H | H | CN | H | H | I | H | nonafluoro-2-butyl | H | CF3 |
| 1-870 | 4-bromophenyl | H | H | H | CN | H | H | I | H | nonafluoro-2-butyl | H | CF3 |
| 1-871 | 2-iodophenyl | H | H | H | CN | H | H | I | H | nonafluoro-2-butyl | H | CF3 |
| 1-872 | 3-iodophenyl | H | H | H | CN | H | H | I | H | nonafluoro-2-butyl | H | CF3 |
| 1-873 | 4-iodophenyl | H | H | H | CN | H | H | I | H | nonafluoro-2-butyl | H | CF3 |
| 1-874 | (2-trifluoromethyl)phenyl | H | H | H | CN | H | H | I | H | nonafluoro-2-butyl | H | CF3 |
| 1-875 | (3-trifluoromethyl)phenyl | H | H | H | CN | H | H | I | H | nonafluoro-2-butyl | H | CF3 |
| 1-876 | (4-trifluoromethyl)phenyl | H | H | H | CN | H | H | I | H | nonafluoro-2-butyl | H | CF3 |
| 1-877 | 2-nitrophenyl | H | H | H | CN | H | H | I | H | nonafluoro-2-butyl | H | CF3 |
| 1-878 | 3-nitrophenyl | H | H | H | CN | H | H | I | H | nonafluoro-2-butyl | H | CF3 |
| 1-879 | 4-nitrophenyl | H | H | H | CN | H | H | I | H | nonafluoro-2-butyl | H | CF3 |
| 1-880 | 2-cyanophenyl | H | H | H | CN | H | H | I | H | nonafluoro-2-butyl | H | CF3 |
| 1-881 | 3-cyanophenyl | H | H | H | CN | H | H | I | H | nonafluoro-2-butyl | H | CF3 |
| 1-882 | 4-cyanophenyl | H | H | H | CN | H | H | I | H | nonafluoro-2-butyl | H | CF3 |
| 1-883 | 2,6-difluorophenyl | H | H | H | CN | H | H | I | H | nonafluoro-2-butyl | H | CF3 |
| 1-884 | 3,4-dichlorophenyl | H | H | H | CN | H | H | I | H | nonafluoro-2-butyl | H | CF3 |
| 1-885 | 2,4-dichlorophenyl | H | H | H | CN | H | H | I | H | nonafluoro-2-butyl | H | CF3 |
| 1-886 | 2-chloro-4-fluorophenyl | H | H | H | CN | H | H | I | H | nonafluoro-2-butyl | H | CF3 |
| 1-887 | 2-chloro-4,5-difluorophenyl | H | H | H | CN | H | H | I | H | nonafluoro-2-butyl | H | CF3 |
| 1-888 | 4-bromo-2-chlorophenyl | H | H | H | CN | H | H | I | H | nonafluoro-2-butyl | H | CF3 |
| 1-889 | 2-bromo-4-chlorophenyl | H | H | H | CN | H | H | I | H | nonafluoro-2-butyl | H | CF3 |
| 1-890 | 2-bromo-4-fluorophenyl | H | H | H | CN | H | H | I | H | nonafluoro-2-butyl | H | CF3 |
| 1-891 | 2-chloro-4-nitrophenyl | H | H | H | CN | H | H | I | H | nonafluoro-2-butyl | H | CF3 |
| 1-892 | 3,5-dicyanophenyl | H | H | H | CN | H | H | I | H | nonafluoro-2-butyl | H | CF3 |
| 1-893 | 4-cyano-2-fluorophenyl | H | H | H | CN | H | H | I | H | nonafluoro-2-butyl | H | CF3 |
| 1-894 | 2-chloro-4-cyanophenyl | H | H | H | CN | H | H | I | H | nonafluoro-2-butyl | H | CF3 |
| 1-895 | pyridin-3-yl | H | H | H | CN | H | H | I | H | nonafluoro-2-butyl | H | CF3 |
| 1-896 | 2-fluoropyridin-3-yl | H | H | H | CN | H | H | I | H | nonafluoro-2-butyl | H | CF3 |
| 1-897 | 2-chloropyridin-3-yl | H | H | H | CN | H | H | I | H | nonafluoro-2-butyl | H | CF3 |
| 1-898 | 2-bromopyridin-3-yl | H | H | H | CN | H | H | I | H | nonafluoro-2-butyl | H | CF3 |
| 1-899 | 2-iodopyridin-3-yl | H | H | H | CN | H | H | I | H | nonafluoro-2-butyl | H | CF3 |
| 1-900 | 2-(trifluoromethyl)pyridin-3-yl | H | H | H | CN | H | H | I | H | nonafluoro-2-butyl | H | CF3 |
| 1-901 | 2-nitropyridin-3-yl | H | H | H | CN | H | H | I | H | nonafluoro-2-butyl | H | CF3 |
| 1-902 | 2-cyanopyridin-3-yl | H | H | H | CN | H | H | I | H | nonafluoro-2-butyl | H | CF3 |
| 1-903 | 6-fluoropyridin-3-yl | H | H | H | CN | H | H | I | H | nonafluoro-2-butyl | H | CF3 |
| 1-904 | 6-chloropyridin-3-yl | H | H | H | CN | H | H | I | H | nonafluoro-2-butyl | H | CF3 |
| 1-905 | 6-bromopyridin-3-yl | H | H | H | CN | H | H | I | H | nonafluoro-2-butyl | H | CF3 |
| 1-906 | 6-iodopyridin-3-yl | H | H | H | CN | H | H | I | H | nonafluoro-2-butyl | H | CF3 |
| 1-907 | 6-(trifluoromethyl)pyridin-3-yl | H | H | H | CN | H | H | I | H | nonafluoro-2-butyl | H | CF3 |
| 1-908 | 6-nitropyridin-3-yl | H | H | H | CN | H | H | I | H | nonafluoro-2-butyl | H | CF3 |
| 1-909 | 6-cyanopyridin-3-yl | H | H | H | CN | H | H | I | H | nonafluoro-2-butyl | H | CF3 |
| 1-910 | 5-fluoropyridin-3-yl | H | H | H | CN | H | H | I | H | nonafluoro-2-butyl | H | CF3 |
| 1-911 | 5-chloropyridin-3-yl | H | H | H | CN | H | H | I | H | nonafluoro-2-butyl | H | CF3 |
| 1-912 | 5-bromopyridin-3-yl | H | H | H | CN | H | H | I | H | nonafluoro-2-butyl | H | CF3 |
| 1-913 | 5-iodopyridin-3-yl | H | H | H | CN | H | H | I | H | nonafluoro-2-butyl | H | CF3 |
| 1-914 | 5-(trifluoromethyl)pyridin-3-yl | H | H | H | CN | H | H | I | H | nonafluoro-2-butyl | H | CF3 |
| 1-915 | 5-nitropyridin-3-yl | H | H | H | CN | H | H | I | H | nonafluoro-2-butyl | H | CF3 |
| 1-916 | 5-cyanopyridin-3-yl | H | H | H | CN | H | H | I | H | nonafluoro-2-butyl | H | CF3 |
| 1-917 | 4-fluoropyridin-3-yl | H | H | H | CN | H | H | I | H | nonafluoro-2-butyl | H | CF3 |
| 1-918 | 4-chloropyridin-3-yl | H | H | H | CN | H | H | I | H | nonafluoro-2-butyl | H | CF3 |
| 1-919 | 4-bromopyridin-3-yl | H | H | H | CN | H | H | I | H | nonafluoro-2-butyl | H | CF3 |
| 1-920 | 4-iodopyridin-3-yl | H | H | H | CN | H | H | I | H | nonafluoro-2-butyl | H | CF3 |
| 1-921 | 4-(trifluoromethyl)pyridin-3-yl | H | H | H | CN | H | H | I | H | nonafluoro-2-butyl | H | CF3 |
| 1-922 | 4-nitropyridin-3-yl | H | H | H | CN | H | H | I | H | nonafluoro-2-butyl | H | CF3 |
| 1-923 | 4-cyanopyridin-3-yl | H | H | H | CN | H | H | I | H | nonafluoro-2-butyl | H | CF3 |
| 1-924 | 2,6-dichloropyridin-3-yl | H | H | H | CN | H | H | I | H | nonafluoro-2-butyl | H | CF3 |
| 1-925 | pyridin-3-yl N-oxide | H | H | H | CN | H | H | I | H | nonafluoro-2-butyl | H | CF3 |
| 1-926 | pyridin-4-yl | H | H | H | CN | H | H | I | H | nonafluoro-2-butyl | H | CF3 |

**Table 1 (9)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 1-927 | 2-chloropyridin-4-yl | H | H | H | CN | H | H | | H | nonafluoro-2-butyl | H | CF3 |
| 1-928 | 3-bromopyridin-4-yl | H | H | H | CN | H | H | I | H | nonafluoro-2-butyl | H | CF3 |
| 1-929 | 3,5-dichloropyridin-4-yl | H | H | H | CN | H | H | I | H | nonafluoro-2-butyl | H | CF3 |
| 1-930 | 3-(trifluoromethyl)pyridin-4-yl | H | H | H | CN | H | H | I | H | nonafluoro-2-butyl | H | CF3 |
| 1-931 | 2,6-dicyanopyridin-4-yl | H | H | H | CN | H | H | I | H | nonafluoro-2-butyl | H | CF3 |
| 1-932 | pyridin-4-yl N-oxide | H | H | H | CN | H | H | I | H | nonafluoro-2-butyl | H | CF3 |
| 1-933 | pyridin-2-yl | H | H | H | CN | H | H | I | H | nonafluoro-2-butyl | H | CF3 |
| 1-934 | 3-chloropyridin-2-yl | H | H | H | CN | H | H | I | H | nonafluoro-2-butyl | H | CF3 |
| 1-935 | 4-bromopyridin-2-yl | H | H | H | CN | H | H | I | H | nonafluoro-2-butyl | H | CF3 |
| 1-936 | 5-iodopyridin-2-yl | H | H | H | CN | H | H | I | H | nonafluoro-2-butyl | H | CF3 |
| 1-937 | 6-chloropyridin-2-yl | H | H | H | CN | H | H | I | H | nonafluoro-2-butyl | H | CF3 |
| 1-938 | 4-cyanopyridin-2-yl | H | H | H | CN | H | H | I | H | nonafluoro-2-butyl | H | CF3 |
| 1-939 | phenyl | H | H | H | CN | H | H | OCF3 | H | heptafluoroisopropyl | H | OCF3 |
| 1-954 | 3-cyanophenyl | H | H | H | CN | H | H | OCF3 | H | heptafluoroisopropyl | H | OCF3 |
| 1-955 | 4-cyanophenyl | H | H | H | CN | H | H | OCF3 | H | heptafluoroisopropyl | H | OCF3 |
| 1-970 | 2-chloropyridin-3-yl | H | H | H | CN | H | H | OCF3 | H | heptafluoroisopropyl | H | OCF3 |
| 1-995 | phenyl | H | H | H | CN | H | H | OCF3 | H | nonafluoro-2-butyl | H | OCF3 |
| 1-1010 | 3-cyanophenyl | H | H | H | CN | H | H | OCF3 | H | nonafluoro-2-butyl | H | OCF3 |
| 1-1011 | 4-cyanophenyl | H | H | H | CN | H | H | OCF3 | H | nonafluoro-2-butyl | H | OCF3 |
| 1-1026 | 2-chloropyridin-3-yl | H | H | H | CN | H | H | OCF3 | H | nonafluoro-2-butyl | H | OCF3 |
| 1-1051 | phenyl | H | H | H | CN | H | H | CF3 | H | heptafluoroisopropyl | H | OCF3 |
| 1-1066 | 3-cyanophenyl | H | H | H | CN | H | H | CF3 | H | heptafluoroisopropyl | H | OCF3 |
| 1-1067 | 4-cyanophenyl | H | H | H | CN | H | H | CF3 | H | heptafluoroisopropyl | H | OCF3 |
| 1-1082 | 2-chloropyridin-3-yl | H | H | H | CN | H | H | CF3 | H | heptafluoroisopropyl | H | OCF3 |
| 1-1107 | phenyl | H | H | H | CN | H | H | CF3 | H | nonafluoro-2-butyl | H | OCF3 |
| 1-1122 | 3-cyanophenyl | H | H | H | CN | H | H | CF3 | H | nonafluoro-2-butyl | H | OCF3 |
| 1-1123 | 4-cyanophenyl | H | H | H | CN | H | H | CF3 | H | nonafluoro-2-butyl | H | OCF3 |
| 1-1138 | 2-chloropyridin-3-yl | H | H | H | CN | H | H | CF3 | H | nonafluoro-2-butyl | H | OCF3 |
| 1-1163 | phenyl | H | H | H | CN | H | H | CF3 | H | heptafluoroisopropyl | H | CF3 |
| 1-1175 | 4-iodophenyl | H | H | H | CN | H | H | CF3 | H | pentafluoroethyl | H | CF3 |
| 1-1177 | (3-trifluoromethyl)phenyl | H | H | H | CN | H | H | C2F5 | H | heptafluoroisopropyl | H | CF3 |
| 1-1183 | 3-cyanophenyl | H | H | H | CN | H | H | CF3 | H | heptafluoroisopropyl | H | CF3 |
| 1-1184 | 4-cyanophenyl | H | H | H | CN | H | H | CF3 | H | heptafluoroisopropyl | H | CF3 |
| 1-1190 | 4-bromo-2-chlorophenyl | H | H | H | CN | H | H | n-C3F7 | H | heptafluoroisopropyl | H | CF3 |
| 1-1195 | 4-cyano-2-fluorophenyl | H | H | H | CN | H | H | i-C3F7 | H | heptafluoroisopropyl | H | CF3 |
| 1-1199 | 2-chloropyridin-3-yl | H | H | H | CN | H | H | CF3 | H | heptafluoroisopropyl | H | CF3 |
| 1-1200 | 2-bromopyridin-3-yl | H | H | H | CN | H | H | C2F5 | H | heptafluoroisopropyl | H | C2F5 |
| 1-1211 | 6-cyanopyridin-3-yl | H | H | H | CN | H | H | C2F5 | H | heptafluoroisopropyl | H | n-C3F7 |
| 1-1215 | 4-cyanopyridin-3-yl | H | H | H | CN | H | H | C2F5 | H | heptafluoroisopropyl | H | i-C3F7 |
| 1-1231 | phenyl | H | H | H | CN | H | H | CF3 | H | nonafluoro-2-butyl | H | CF3 |
| 1-1243 | 4-iodophenyl | H | H | H | CN | H | H | C2F5 | H | nonafluoro-2-butyl | H | CF3 |
| 1-1251 | 3-cyanophenyl | H | H | H | CN | H | H | CF3 | H | nonafluoro-2-butyl | H | CF3 |
| 1-1252 | 4-cyanophenyl | H | H | H | CN | H | H | CF3 | H | nonafluoro-2-butyl | H | CF3 |
| 1-1258 | 4-bromo-2-chlorophenyl | H | H | H | CN | H | H | n-C3F7 | H | nonafluoro-2-butyl | H | CF3 |
| 1-1263 | 4-cyano-2-fluorophenyl | H | H | H | CN | H | H | i-C3F7 | H | nonafluoro-2-butyl | H | CF3 |
| 1-1267 | 2-chloropyridin-3-yl | H | H | H | CN | H | H | CF3 | H | nonafluoro-2-butyl | H | CF3 |
| 1-1268 | 2-bromopyridin-3-yl | H | H | H | CN | H | H | C2F5 | H | nonafluoro-2-butyl | H | C2F5 |
| 1-1279 | 6-cyanopyridin-3-yl | H | H | H | CN | H | H | C2F5 | H | nonafluoro-2-butyl | H | n-C3F7 |
| 1-1283 | 4-cyanopyridin-3-yl | H | H | H | CN | H | H | C2F5 | H | nonafluoro-2-butyl | H | i-C3F7 |
| 1-1299 | phenyl | H | H | F | CN | H | H | Cl | H | heptafluoroisopropyl | H | OCF3 |
| 1-1319 | 3-cyanophenyl | H | H | F | CN | H | H | Cl | H | heptafluoroisopropyl | H | OCF3 |
| 1-1320 | 4-cyanophenyl | H | H | F | CN | H | H | Cl | H | heptafluoroisopropyl | H | OCF3 |
| 1-1335 | 2-chloropyridin-3-yl | H | H | F | CN | H | H | Cl | H | heptafluoroisopropyl | H | OCF3 |
| 1-1377 | phenyl | H | H | F | CN | H | H | Cl | H | nonafluoro-2-butyl | H | OCF3 |
| 1-1384 | 2-bromophenyl | H | H | F | CN | H | H | F | H | nonafluoro-2-butyl | H | OCF3 |
| 1-1397 | 3-cyanophenyl | H | H | F | CN | H | H | Cl | H | nonafluoro-2-butyl | H | OCF3 |
| 1-1398 | 4-cyanophenyl | H | H | F | CN | H | H | Cl | H | nonafluoro-2-butyl | H | OCF3 |
| 1-1413 | 2-chloropyridin-3-yl | H | H | F | CN | H | H | Cl | H | nonafluoro-2-butyl | H | OCF3 |
| 1-1455 | phenyl | H | H | F | CN | H | H | Br | H | heptafluoroisopropyl | H | OCF3 |
| 1-1475 | 3-cyanophenyl | H | H | F | CN | H | H | Br | H | heptafluoroisopropyl | H | OCF3 |
| 1-1476 | 4-cyanophenyl | H | H | F | CN | H | H | Br | H | heptafluoroisopropyl | H | OCF3 |
| 1-1491 | 2-chloropyridin-3-yl | H | H | F | CN | H | H | Br | H | heptafluoroisopropyl | H | OCF3 |
| 1-1533 | phenyl | H | H | F | CN | H | H | Br | H | nonafluoro-2-butyl | H | OCF3 |
| 1-1553 | 3-cyanophenyl | H | H | F | CN | H | H | Br | H | nonafluoro-2-butyl | H | OCF3 |

**Table 1 (10)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 1-1554 | 4-cyanophenyl | H | H | F | CN | H | H | Br | H | nonafluoro-2-butyl | H | OCF3 |
| 1-1569 | 2-chloropyridin-3-yl | H | H | F | CN | H | H | Br | H | nonafluoro-2-butyl | H | OCF3 |
| 1-1611 | phenyl | H | H | F | CN | H | H | I | H | heptafluoroisopropyl | H | OCF3 |
| 1-1631 | 3-cyanophenyl | H | H | F | CN | H | H | I | H | heptafluoroisopropyl | H | OCF3 |
| 1-1632 | 4-cyanophenyl | H | H | F | CN | H | H | I | H | heptafluoroisopropyl | H | OCF3 |
| 1-1647 | 2-chloropyridin-3-yl | H | H | F | CN | H | H | I | H | heptafluoroisopropyl | H | OCF3 |
| 1-1689 | phenyl | H | H | F | CN | H | H | I | H | nonafluoro-2-butyl | H | OCF3 |
| 1-1709 | 3-cyanophenyl | H | H | F | CN | H | H | I | H | nonafluoro-2-butyl | H | OCF3 |
| 1-1710 | 4-cyanophenyl | H | H | F | CN | H | H | I | H | nonafluoro-2-butyl | H | OCF3 |
| 1-1725 | 2-chloropyridin-3-yl | H | H | F | CN | H | H | I | H | nonafluoro-2-butyl | H | OCF3 |
| 1-1767 | phenyl | H | H | F | CN | H | H | F | H | heptafluoroisopropyl | H | CF3 |
| 1-1768 | phenyl | H | H | F | CN | H | H | Cl | H | heptafluoroisopropyl | H | CF3 |
| 1-1788 | 3-cyanophenyl | H | H | F | CN | H | H | Cl | H | heptafluoroisopropyl | H | CF3 |
| 1-1789 | 4-cyanophenyl | H | H | F | CN | H | H | Cl | H | heptafluoroisopropyl | H | CF3 |
| 1-1804 | 2-chloropyridin-3-yl | H | H | F | CN | H | H | Cl | H | heptafluoroisopropyl | H | CF3 |
| 1-1846 | phenyl | H | H | F | CN | H | H | F | H | nonafluoro-2-butyl | H | CF3 |
| 1-1847 | phenyl | H | H | F | CN | H | H | Cl | H | nonafluoro-2-butyl | H | CF3 |
| 1-1867 | 3-cyanophenyl | H | H | F | CN | H | H | Cl | H | nonafluoro-2-butyl | H | CF3 |
| 1-1868 | 4-cyanophenyl | H | H | F | CN | H | H | Cl | H | nonafluoro-2-butyl | H | CF3 |
| 1-1883 | 2-chloropyridin-3-yl | H | H | F | CN | H | H | Cl | H | nonafluoro-2-butyl | H | CF3 |
| 1-1925 | phenyl | H | H | F | CN | H | H | Br | H | heptafluoroisopropyl | H | CF3 |
| 1-1945 | 3-cyanophenyl | H | H | F | CN | H | H | Br | H | heptafluoroisopropyl | H | CF3 |
| 1-1946 | 4-cyanophenyl | H | H | F | CN | H | H | Br | H | heptafluoroisopropyl | H | CF3 |
| 1-1961 | 2-chloropyridin-3-yl | H | H | F | CN | H | H | Br | H | heptafluoroisopropyl | H | CF3 |
| 1-1968 | 6-chloropyridin-3-yl | H | H | F | CN | H | H | Br | H | heptafluoroisopropyl | H | CF3 |
| 1-2003 | phenyl | H | H | F | CN | H | H | Br | H | nonafluoro-2-butyl | H | CF3 |
| 1-2023 | 3-cyanophenyl | H | H | F | CN | H | H | Br | H | nonafluoro-2-butyl | H | CF3 |
| 1-2024 | 4-cyanophenyl | H | H | F | CN | H | H | Br | H | nonafluoro-2-butyl | H | CF3 |
| 1-2039 | 2-chloropyridin-3-yl | H | H | F | CN | H | H | Br | H | nonafluoro-2-butyl | H | CF3 |
| 1-2081 | phenyl | H | H | F | CN | H | H | I | H | heptafluoroisopropyl | H | CF3 |
| 1-2101 | 3-cyanophenyl | H | H | F | CN | H | H | I | H | heptafluoroisopropyl | H | CF3 |
| 1-2102 | 4-cyanophenyl | H | H | F | CN | H | H | I | H | heptafluoroisopropyl | H | CF3 |
| 1-2117 | 2-chloropyridin-3-yl | H | H | F | CN | H | H | I | H | heptafluoroisopropyl | H | CF3 |
| 1-2159 | phenyl | H | H | F | CN | H | H | I | H | nonafluoro-2-butyl | H | CF3 |
| 1-2179 | 3-cyanophenyl | H | H | F | CN | H | H | I | H | nonafluoro-2-butyl | H | CF3 |
| 1-2180 | 4-cyanophenyl | H | H | F | CN | H | H | I | H | nonafluoro-2-butyl | H | CF3 |
| 1-2195 | 2-chloropyridin-3-yl | H | H | F | CN | H | H | I | H | nonafluoro-2-butyl | H | CF3 |
| 1-2237 | phenyl | H | H | F | CN | H | H | OCF3 | H | heptafluoroisopropyl | H | OCF3 |
| 1-2252 | 3-cyanophenyl | H | H | F | CN | H | H | OCF3 | H | heptafluoroisopropyl | H | OCF3 |
| 1-2253 | 4-cyanophenyl | H | H | F | CN | H | H | OCF3 | H | heptafluoroisopropyl | H | OCF3 |
| 1-2268 | 2-chloropyridin-3-yl | H | H | F | CN | H | H | OCF3 | H | heptafluoroisopropyl | H | OCF3 |
| 1-2293 | phenyl | H | H | F | CN | H | H | OCF3 | H | nonafluoro-2-butyl | H | OCF3 |
| 1-2308 | 3-cyanophenyl | H | H | F | CN | H | H | OCF3 | H | nonafluoro-2-butyl | H | OCF3 |
| 1-2309 | 4-cyanophenyl | H | H | F | CN | H | H | OCF3 | H | nonafluoro-2-butyl | H | OCF3 |
| 1-2324 | 2-chloropyridin-3-yl | H | H | F | CN | H | H | OCF3 | H | nonafluoro-2-butyl | H | OCF3 |
| 1-2325 | 2-bromopyridin-3-yl | H | H | F | CN | H | H | OCF3 | H | nonafluoro-2-butyl | H | OCF3 |
| 1-2349 | phenyl | H | H | F | CN | H | H | CF3 | H | heptafluoroisopropyl | H | OCF3 |
| 1-2364 | 3-cyanophenyl | H | H | F | CN | H | H | CF3 | H | heptafluoroisopropyl | H | OCF3 |
| 1-2365 | 4-cyanophenyl | H | H | F | CN | H | H | CF3 | H | heptafluoroisopropyl | H | OCF3 |
| 1-2380 | 2-chloropyridin-3-yl | H | H | F | CN | H | H | CF3 | H | heptafluoroisopropyl | H | OCF3 |
| 1-2405 | phenyl | H | H | F | CN | H | H | CF3 | H | nonafluoro-2-butyl | H | OCF3 |
| 1-2420 | 3-cyanophenyl | H | H | F | CN | H | H | CF3 | H | nonafluoro-2-butyl | H | OCF3 |
| 1-2421 | 4-cyanophenyl | H | H | F | CN | H | H | CF3 | H | nonafluoro-2-butyl | H | OCF3 |
| 1-2436 | 2-chloropyridin-3-yl | H | H | F | CN | H | H | CF3 | H | nonafluoro-2-butyl | H | OCF3 |
| 1-2461 | phenyl | H | H | F | CN | H | H | CF3 | H | heptafluoroisopropyl | H | CF3 |
| 1-2481 | 3-cyanophenyl | H | H | F | CN | H | H | CF3 | H | heptafluoroisopropyl | H | CF3 |
| 1-2482 | 4-cyanophenyl | H | H | F | CN | H | H | CF3 | H | heptafluoroisopropyl | H | CF3 |
| 1-2497 | 2-chloropyridin-3-yl | H | H | F | CN | H | H | CF3 | H | heptafluoroisopropyl | H | CF3 |
| 1-2529 | phenyl | H | H | F | CN | H | H | CF3 | H | nonafluoro-2-butyl | H | CF3 |
| 1-2549 | 3-cyanophenyl | H | H | F | CN | H | H | CF3 | H | nonafluoro-2-butyl | H | CF3 |
| 1-2550 | 4-cyanophenyl | H | H | F | CN | H | H | CF3 | H | nonafluoro-2-butyl | H | CF3 |
| 1-2565 | 2-chloropyridin-3-yl | H | H | F | CN | H | H | CF3 | H | nonafluoro-2-butyl | H | CF3 |
| 1-2597 | phenyl | H | H | F | CN | F | H | Cl | H | heptafluoroisopropyl | H | OCF3 |
| 1-2617 | 3-cyanophenyl | H | H | F | CN | F | H | Cl | H | heptafluoroisopropyl | H | OCF3 |

**Table 1 (11)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 1-2618 | 4-cyanophenyl | H | H | F | CN | F | H | Cl | H | heptafluoroisopropyl | H | OCF3 |
| 1-2633 | 2-chloropyridin-3-yl | H | H | F | CN | F | H | Cl | H | heptafluoroisopropyl | H | OCF3 |
| 1-2675 | phenyl | H | H | F | CN | F | H | Cl | H | nonafluoro-2-butyl | H | OCF3 |
| 1-2695 | 3-cyanophenyl | H | H | F | CN | F | H | Cl | H | nonafluoro-2-butyl | H | OCF3 |
| 1-2696 | 4-cyanophenyl | H | H | F | CN | F | H | Cl | H | nonafluoro-2-butyl | H | OCF3 |
| 1-2711 | 2-chloropyridin-3-yl | H | H | F | CN | F | H | Cl | H | nonafluoro-2-butyl | H | OCF3 |
| 1-2753 | phenyl | H | H | F | CN | F | H | Br | H | heptafluoroisopropyl | H | OCF3 |
| 1-2773 | 3-cyanophenyl | H | H | F | CN | F | H | Br | H | heptafluoroisopropyl | H | OCF3 |
| 1-2774 | 4-cyanophenyl | H | H | F | CN | F | H | Br | H | heptafluoroisopropyl | H | OCF3 |
| 1-2789 | 2-chloropyridin-3-yl | H | H | F | CN | F | H | Br | H | heptafluoroisopropyl | H | OCF3 |
| 1-2831 | phenyl | H | H | F | CN | F | H | Br | H | nonafluoro-2-butyl | H | OCF3 |
| 1-2851 | 3-cyanophenyl | H | H | F | CN | F | H | Br | H | nonafluoro-2-butyl | H | OCF3 |
| 1-2852 | 4-cyanophenyl | H | H | F | CN | F | H | Br | H | nonafluoro-2-butyl | H | OCF3 |
| 1-2867 | 2-chloropyridin-3-yl | H | H | F | CN | F | H | Br | H | nonafluoro-2-butyl | H | OCF3 |
| 1-2909 | phenyl | H | H | F | CN | F | H | I | H | heptafluoroisopropyl | H | OCF3 |
| 1-2929 | 3-cyanophenyl | H | H | F | CN | F | H | I | H | heptafluoroisopropyl | H | OCF3 |
| 1-2930 | 4-cyanophenyl | H | H | F | CN | F | H | I | H | heptafluoroisopropyl | H | OCF3 |
| 1-2945 | 2-chloropyridin-3-yl | H | H | F | CN | F | H | I | H | heptafluoroisopropyl | H | OCF3 |
| 1-2987 | phenyl | H | H | F | CN | F | H | I | H | nonafluoro-2-butyl | H | OCF3 |
| 1-3007 | 3-cyanophenyl | H | H | F | CN | F | H | I | H | nonafluoro-2-butyl | H | OCF3 |
| 1-3008 | 4-cyanophenyl | H | H | F | CN | F | H | I | H | nonafluoro-2-butyl | H | OCF3 |
| 1-3023 | 2-chloropyridin-3-yl | H | H | F | CN | F | H | I | H | nonafluoro-2-butyl | H | OCF3 |
| 1-3065 | phenyl | H | H | F | CN | F | H | F | H | heptafluoroisopropyl | H | CF3 |
| 1-3066 | phenyl | H | H | F | CN | F | H | Cl | H | heptafluoroisopropyl | H | CF3 |
| 1-3086 | 3-cyanophenyl | H | H | F | CN | F | H | Cl | H | heptafluoroisopropyl | H | CF3 |
| 1-3087 | 4-cyanophenyl | H | H | F | CN | F | H | Cl | H | heptafluoroisopropyl | H | CF3 |
| 1-3102 | 2-chloropyridin-3-yl | H | H | F | CN | F | H | Cl | H | heptafluoroisopropyl | H | CF3 |
| 1-3144 | phenyl | H | H | F | CN | F | H | F | H | nonafluoro-2-butyl | H | CF3 |
| 1-3145 | phenyl | H | H | F | CN | F | H | Cl | H | nonafluoro-2-butyl | H | CF3 |
| 1-3165 | 3-cyanophenyl | H | H | F | CN | F | H | Cl | H | nonafluoro-2-butyl | H | CF3 |
| 1-3166 | 4-cyanophenyl | H | H | F | CN | F | H | Cl | H | nonafluoro-2-butyl | H | CF3 |
| 1-3181 | 2-chloropyridin-3-yl | H | H | F | CN | F | H | Cl | H | nonafluoro-2-butyl | H | CF3 |
| 1-3223 | phenyl | H | H | F | CN | F | H | Br | H | heptafluoroisopropyl | H | CF3 |
| 1-3243 | 3-cyanophenyl | H | H | F | CN | F | H | Br | H | heptafluoroisopropyl | H | CF3 |
| 1-3244 | 4-cyanophenyl | H | H | F | CN | F | H | Br | H | heptafluoroisopropyl | H | CF3 |
| 1-3259 | 2-chloropyridin-3-yl | H | H | F | CN | F | H | Br | H | heptafluoroisopropyl | H | CF3 |
| 1-3301 | phenyl | H | H | F | CN | F | H | Br | H | nonafluoro-2-butyl | H | CF3 |
| 1-3321 | 3-cyanophenyl | H | H | F | CN | F | H | Br | H | nonafluoro-2-butyl | H | CF3 |
| 1-3322 | 4-cyanophenyl | H | H | F | CN | F | H | Br | H | nonafluoro-2-butyl | H | CF3 |
| 1-3337 | 2-chloropyridin-3-yl | H | H | F | CN | F | H | Br | H | nonafluoro-2-butyl | H | CF3 |
| 1-3379 | phenyl | H | H | F | CN | F | H | I | H | heptafluoroisopropyl | H | CF3 |
| 1-3399 | 3-cyanophenyl | H | H | F | CN | F | H | I | H | heptafluoroisopropyl | H | CF3 |
| 1-3400 | 4-cyanophenyl | H | H | F | CN | F | H | I | H | heptafluoroisopropyl | H | CF3 |
| 1-3415 | 2-chloropyridin-3-yl | H | H | F | CN | F | H | I | H | heptafluoroisopropyl | H | CF3 |
| 1-3457 | phenyl | H | H | F | CN | F | H | I | H | nonafluoro-2-butyl | H | CF3 |
| 1-3477 | 3-cyanophenyl | H | H | F | CN | F | H | I | H | nonafluoro-2-butyl | H | CF3 |
| 1-3478 | 4-cyanophenyl | H | H | F | CN | F | H | I | H | nonafluoro-2-butyl | H | CF3 |
| 1-3493 | 2-chloropyridin-3-yl | H | H | F | CN | F | H | I | H | nonafluoro-2-butyl | H | CF3 |
| 1-3535 | phenyl | H | H | F | CN | F | H | OCF3 | H | heptafluoroisopropyl | H | OCF3 |
| 1-3550 | 3-cyanophenyl | H | H | F | CN | F | H | OCF3 | H | heptafluoroisopropyl | H | OCF3 |
| 1-3551 | 4-cyanophenyl | H | H | F | CN | F | H | OCF3 | H | heptafluoroisopropyl | H | OCF3 |
| 1-3566 | 2-chloropyridin-3-yl | H | H | F | CN | F | H | OCF3 | H | heptafluoroisopropyl | H | OCF3 |
| 1-3591 | phenyl | H | H | F | CN | F | H | OCF3 | H | nonafluoro-2-butyl | H | OCF3 |
| 1-3606 | 3-cyanophenyl | H | H | F | CN | F | H | OCF3 | H | nonafluoro-2-butyl | H | OCF3 |
| 1-3607 | 4-cyanophenyl | H | H | F | CN | F | H | OCF3 | H | nonafluoro-2-butyl | H | OCF3 |
| 1-3622 | 2-chloropyridin-3-yl | H | H | F | CN | F | H | OCF3 | H | nonafluoro-2-butyl | H | OCF3 |
| 1-3647 | phenyl | H | H | F | CN | F | H | CF3 | H | heptafluoroisopropyl | H | OCF3 |
| 1-3662 | 3-cyanophenyl | H | H | F | CN | F | H | CF3 | H | heptafluoroisopropyl | H | OCF3 |
| 1-3663 | 4-cyanophenyl | H | H | F | CN | F | H | CF3 | H | heptafluoroisopropyl | H | OCF3 |
| 1-3678 | 2-chloropyridin-3-yl | H | H | F | CN | F | H | CF3 | H | heptafluoroisopropyl | H | OCF3 |
| 1-3703 | phenyl | H | H | F | CN | F | H | CF3 | H | nonafluoro-2-butyl | H | OCF3 |
| 1-3718 | 3-cyanophenyl | H | H | F | CN | F | H | CF3 | H | nonafluoro-2-butyl | H | OCF3 |
| 1-3719 | 4-cyanophenyl | H | H | F | CN | F | H | CF3 | H | nonafluoro-2-butyl | H | OCF3 |
| 1-3734 | 2-chloropyridin-3-yl | H | H | F | CN | F | H | CF3 | H | nonafluoro-2-butyl | H | OCF3 |

**Table 1 (12)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 1-3759 | phenyl | H | H | F | CN | F | H | CF3 | H | heptafluoroisopropyl | H | CF3 |
| 1-3779 | 3-cyanophenyl | H | H | F | CN | F | H | CF3 | H | heptafluoroisopropyl | H | CF3 |
| 1-3780 | 4-cyanophenyl | H | H | F | CN | F | H | CF3 | H | heptafluoroisopropyl | H | CF3 |
| 1-3795 | 2-chloropyridin-3-yl | H | H | F | CN | F | H | CF3 | H | heptafluoroisopropyl | H | CF3 |
| 1-3827 | phenyl | H | H | F | CN | F | H | CF3 | H | nonafluoro-2-butyl | H | CF3 |
| 1-3847 | 3-cyanophenyl | H | H | F | CN | F | H | CF3 | H | nonafluoro-2-butyl | H | CF3 |
| 1-3848 | 4-cyanophenyl | H | H | F | CN | F | H | CF3 | H | nonafluoro-2-butyl | H | CF3 |
| 1-3863 | 2-chloropyridin-3-yl | H | H | F | CN | F | H | CF3 | H | nonafluoro-2-butyl | H | CF3 |

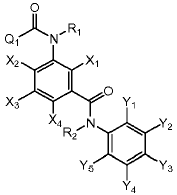

**Table 2(1)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 2-1 | phenyl | Me | H | H | CN | H | H | Cl | H | heptafluoroisopropyl | H | OCF3 |
| 2-21 | 3-cyanophenyl | Me | H | H | CN | H | H | Cl | H | heptafluoroisopropyl | H | OCF3 |
| 2-22 | 4-cyanophenyl | Me | H | H | CN | H | H | Cl | H | heptafluoroisopropyl | H | OCF3 |
| 2-37 | 2-chloropyridin-3-yl | Me | H | H | CN | H | H | Cl | H | heptafluoroisopropyl | H | OCF3 |
| 2-79 | phenyl | Me | H | H | CN | H | H | Cl | H | nonafluoro-2-butyl | H | OCF3 |
| 2-99 | 3-cyanophenyl | Me | H | H | CN | H | H | Cl | H | nonafluoro-2-butyl | H | OCF3 |
| 2-100 | 4-cyanophenyl | Me | H | H | CN | H | H | Cl | H | nonafluoro-2-butyl | H | OCF3 |
| 2-115 | 2-chloropyridin-3-yl | Me | H | H | CN | H | H | Cl | H | nonafluoro-2-butyl | H | OCF3 |
| 2-157 | phenyl | Me | H | H | CN | H | H | Br | H | heptafluoroisopropyl | H | OCF3 |
| 2-177 | 3-cyanophenyl | Me | H | H | CN | H | H | Br | H | heptafluoroisopropyl | H | OCF3 |
| 2-178 | 4-cyanophenyl | Me | H | H | CN | H | H | Br | H | heptafluoroisopropyl | H | OCF3 |
| 2-193 | 2-chloropyridin-3-yl | Me | H | H | CN | H | H | Br | H | heptafluoroisopropyl | H | OCF3 |
| 2-235 | phenyl | Me | H | H | CN | H | H | Br | H | nonafluoro-2-butyl | H | OCF3 |
| 2-255 | 3-cyanophenyl | Me | H | H | CN | H | H | Br | H | nonafluoro-2-butyl | H | OCF3 |
| 2-256 | 4-cyanophenyl | Me | H | H | CN | H | H | Br | H | nonafluoro-2-butyl | H | OCF3 |
| 2-271 | 2-chloropyridin-3-yl | Me | H | H | CN | H | H | Br | H | nonafluoro-2-butyl | H | OCF3 |
| 2-313 | phenyl | Me | H | H | CN | H | H | I | H | heptafluoroisopropyl | H | OCF3 |
| 2-333 | 3-cyanophenyl | Me | H | H | CN | H | H | I | H | heptafluoroisopropyl | H | OCF3 |
| 2-334 | 4-cyanophenyl | Me | H | H | CN | H | H | I | H | heptafluoroisopropyl | H | OCF3 |
| 2-349 | 2-chloropyridin-3-yl | Me | H | H | CN | H | H | I | H | heptafluoroisopropyl | H | OCF3 |
| 2-391 | phenyl | Me | H | H | CN | H | H | I | H | nonafluoro-2-butyl | H | OCF3 |
| 2-411 | 3-cyanophenyl | Me | H | H | CN | H | H | I | H | nonafluoro-2-butyl | H | OCF3 |
| 2-412 | 4-cyanophenyl | Me | H | H | CN | H | H | I | H | nonafluoro-2-butyl | H | OCF3 |
| 2-427 | 2-chloropyridin-3-yl | Me | H | H | CN | H | H | I | H | nonafluoro-2-butyl | H | OCF3 |
| 2-469 | phenyl | Me | H | H | CN | H | H | F | H | heptafluoroisopropyl | H | CF3 |
| 2-470 | phenyl | Me | H | H | CN | H | H | Cl | H | heptafluoroisopropyl | H | CF3 |
| 2-481 | 3-iodophenyl | Me | H | H | CN | H | H | Cl | H | pentafluoroethyl | H | CF3 |
| 2-482 | 4-iodophenyl | Me | H | H | CN | H | H | Cl | H | pentafluoroethyl | H | CF3 |
| 2-484 | (3-trifluoromethyl) phenyl | Et | H | H | CN | H | H | Cl | H | heptafluoroisopropyl | H | CF3 |
| 2-489 | 2-cyanophenyl | Me | H | H | CN | H | H | Cl | H | pentafluoroethyl | H | C2F5 |
| 2-490 | 3-cyanophenyl | Me | H | H | CN | H | H | Cl | H | heptafluoroisopropyl | H | CF3 |
| 2-491 | 4-cyanophenyl | Me | H | H | CN | H | H | Cl | H | heptafluoroisopropyl | H | CF3 |
| 2-494 | 2,4-dichlorophenyl | Me | H | H | CN | H | H | Cl | H | heptafluoroisopropyl | H | C2F5 |
| 2-495 | 2-chloro-4-fluorophenyl | n-Pr | H | H | CN | H | H | Cl | H | heptafluoroisopropyl | H | CF3 |
| 2-498 | 2-bromo-4-chlorophenyl | Me | H | H | CN | H | H | Cl | H | heptafluoroisopropyl | H | n-C3F7 |
| 2-505 | 2-fluoropyridin-3-yl | Me | H | H | CN | H | H | Cl | H | pentafluoroethyl | H | CF3 |
| 2-506 | 2-chloropyridin-3-yl | Me | H | H | CN | H | H | Cl | H | heptafluoroisopropyl | H | CF3 |
| 2-512 | 6-fluoropyridin-3-yl | i-Pr | H | H | CN | H | H | Cl | H | heptafluoroisopropyl | H | CF3 |
| 2-513 | 6-chloropyridin-3-yl | Me | H | H | CN | H | H | Cl | H | heptafluoroisopropyl | H | CF3 |
| 2-518 | 6-cyanopyridin-3-yl | Me | H | H | CN | H | H | Cl | H | heptafluoroisopropyl | H | CF3 |
| 2-519 | 5-fluoropyridin-3-yl | Me | H | H | CN | H | H | Cl | H | heptafluoroisopropyl | H | C2F5 |
| 2-521 | 5-bromopyridin-3-yl | Me | H | H | CN | H | H | Cl | H | heptafluoroisopropyl | H | n-C3F7 |
| 2-522 | 5-iodopyridin-3-yl | Me | H | H | CN | H | H | Cl | H | heptafluoroisopropyl | H | i-C3F7 |
| 2-524 | 5-nitropyridin-3-yl | Me | H | H | CN | H | H | Cl | H | pentafluoroethyl | H | CF3 |
| 2-526 | 4-fluoropyridin-3-yl | Me | H | H | CN | H | H | Cl | H | pentafluoroethyl | H | CF3 |
| 2-528 | 4-bromopyridin-3-yl | CH2CH=CH2 | H | H | CN | H | H | Cl | H | heptafluoroisopropyl | H | n-C3F7 |
| 2-529 | 4-iodopyridin-3-yl | Me | H | H | CN | H | H | Cl | H | heptafluoroisopropyl | H | i-C3F7 |
| 2-535 | pyridin-4-yl | CN | H | H | CN | H | H | Cl | H | heptafluoroisopropyl | H | CF3 |
| 2-547 | 4-cyanopyridin-2-yl | Me | H | H | CN | H | H | Cl | H | pentafluoroethyl | H | CF3 |
| 2-548 | phenyl | Me | H | H | CN | H | H | F | H | nonafluoro-2-butyl | H | CF3 |
| 2-549 | phenyl | Me | H | H | CN | H | H | Cl | H | nonafluoro-2-butyl | H | CF3 |
| 2-556 | 2-bromophenyl | CH2C≡CH | H | H | CN | H | H | Cl | H | nonafluoro-2-butyl | H | CF3 |
| 2-562 | (2-trifluoromethyl) phenyl | Me | H | H | CN | H | H | Cl | H | nonafluoro-2-butyl | H | C2F5 |
| 2-563 | (3-trifluoromethyl) phenyl | NH2 | H | H | CN | H | H | Cl | H | nonafluoro-2-butyl | H | CF3 |
| 2-569 | 3-cyanophenyl | Me | H | H | CN | H | H | Cl | H | nonafluoro-2-butyl | H | CF3 |
| 2-570 | 4-cyanophenyl | Me | H | H | CN | H | H | Cl | H | nonafluoro-2-butyl | H | CF3 |

**Table 2(2)**

| compound number | Q₁ | R₁ | R₂ | X₁ | X₄ | X₃ | X₄ | Y₁ | Y₂ | Y₃ | Y₄ | Y₅ |
|---|---|---|---|---|---|---|---|---|---|---|---|---|
| 2-576 | 4-bromo-2-chlorophenyl | C(O)OMe | H | H | CN | H | H | Cl | H | nonafluoro-2-butyl | H | CF3 |
| 2-579 | 2-chloro-4-nitrophenyl | Me | H | H | CN | H | H | Cl | H | nonafluoro-2-butyl | H | i-C3F7 |
| 2-585 | 2-chloropyridin-3-yl | Me | H | H | CN | H | H | Cl | H | nonafluoro-2-butyl | H | CF3 |
| 2-596 | 6-nitropyridin-3-yl | C(O)OEt | H | H | CN | H | H | Cl | H | nonafluoro-2-butyl | H | CF3 |
| 2-598 | 5-fluoropyridin-3-yl | Me | H | H | CN | H | H | Cl | H | nonafluoro-2-butyl | H | C2F5 |
| 2-600 | 5-bromopyridin-3-yl | Me | H | H | CN | H | H | Cl | H | nonafluoro-2-butyl | H | n-C3F7 |
| 2-601 | 5-iodopyridin-3-yl | Me | H | H | CN | H | H | Cl | H | nonafluoro-2-butyl | H | i-C3F7 |
| 2-605 | 4-fluoropyridin-3-yl | Me | H | H | CN | H | H | Cl | H | nonafluoro-2-butyl | H | C2F5 |
| 2-607 | 4-bromopyridin-3-yl | C(O)C(O)Me | H | H | CN | H | H | Cl | H | nonafluoro-2-butyl | H | CF3 |
| 2-609 | 4-(trifluoromethyl)pyridin-3-yl | Me | H | H | CN | H | H | Cl | H | nonafluoro-2-butyl | H | n-C3F7 |
| 2-610 | 4-nitropyridin-3-yl | C(O)C(O)Et | H | H | CN | H | H | Cl | H | nonafluoro-2-butyl | H | i-C3F7 |
| 2-623 | 4-bromopyridin-2-yl | C(O)Me | H | H | CN | H | H | Cl | H | nonafluoro-2-butyl | H | CF3 |
| 2-625 | 6-chloropyridin-2-yl | C(O)Et | H | H | CN | H | H | Cl | H | nonafluoro-2-butyl | H | CF3 |
| 2-626 | 4-cyanopyridin-2-yl | Me | H | H | CN | H | H | Cl | H | nonafluoro-2-butyl | H | C2F5 |
| 2-627 | phenyl | Me | H | H | CN | H | H | Br | H | heptafluoroisopropyl | H | CF3 |
| 2-628 | 2-fluorophenyl | Me | H | H | CN | H | H | Br | H | heptafluoroisopropyl | H | CF3 |
| 2-629 | 3-fluorophenyl | Me | H | H | CN | H | H | Br | H | heptafluoroisopropyl | H | CF3 |
| 2-630 | 4-fluorophenyl | Me | H | H | CN | H | H | Br | H | heptafluoroisopropyl | H | CF3 |
| 2-631 | 2-chlorophenyl | Me | H | H | CN | H | H | Br | H | heptafluoroisopropyl | H | CF3 |
| 2-632 | 3-chlorophenyl | Me | H | H | CN | H | H | Br | H | heptafluoroisopropyl | H | CF3 |
| 2-633 | 4-chlorophenyl | Me | H | H | CN | H | H | Br | H | heptafluoroisopropyl | H | CF3 |
| 2-634 | 2-bromophenyl | Me | H | H | CN | H | H | Br | H | heptafluoroisopropyl | H | CF3 |
| 2-635 | 3-bromophenyl | Me | H | H | CN | H | H | Br | H | heptafluoroisopropyl | H | CF3 |
| 2-636 | 4-bromophenyl | Me | H | H | CN | H | H | Br | H | heptafluoroisopropyl | H | CF3 |
| 2-637 | 2-iodophenyl | Me | H | H | CN | H | H | Br | H | heptafluoroisopropyl | H | CF3 |
| 2-638 | 3-iodophenyl | Me | H | H | CN | H | H | Br | H | heptafluoroisopropyl | H | CF3 |
| 2-639 | 4-iodophenyl | Me | H | H | CN | H | H | Br | H | heptafluoroisopropyl | H | CF3 |
| 2-640 | (2-trifluoromethyl)phenyl | Me | H | H | CN | H | H | Br | H | heptafluoroisopropyl | H | CF3 |
| 2-641 | (3-trifluoromethyl) phenyl | S(O)2Me | H | H | CN | H | H | Br | H | heptafluoroisopropyl | H | CF3 |
| 2-642 | (4⁻trifluoromethyl)phenyl | Me | H | H | CN | H | H | Br | H | heptafluoroisopropyl | H | CF3 |
| 2-643 | 2-nitrophenyl | Me | H | H | CN | H | H | Br | H | heptafluoroisopropyl | H | CF3 |
| 2-644 | 3-nitrophenyl | Me | H | H | CN | H | H | Br | H | heptafluoroisopropyl | H | CF3 |
| 2-645 | 4-nitrophenyl | Me | H | H | CN | H | H | Br | H | heptafluoroisopropyl | H | CF3 |
| 2-646 | 2-cyanophenyl | Me | H | H | CN | H | H | Br | H | heptafluoroisopropyl | H | CF3 |
| 2-647 | 3-cyanophenyl | Me | H | H | CN | H | H | Br | H | heptafluoroisopropyl | H | CF3 |
| 2-648 | 4-cyanophenyl | Me | H | H | CN | H | H | Br | H | heptafluoroisopropyl | H | CF3 |
| 2-649 | 2,6-difluorophenyl | Me | H | H | CN | H | H | Br | H | heptafluoroisopropyl | H | CF3 |
| 2-650 | 3,4-dichlorophenyl | Me | H | H | CN | H | H | Br | H | heptafluoroisopropyl | H | CF3 |
| 2-651 | 2,4-dichlorophenyl | Me | H | H | CN | H | H | Br | H | heptafluoroisopropyl | H | CF3 |
| 2-652 | 2-chloro-4-fluorophenyl | Me | H | H | CN | H | H | Br | H | heptafluoroisopropyl | H | CF3 |
| 2-653 | 2-chloro-4,5-difluorophenyl | Me | H | H | CN | H | H | Br | H | heptafluoroisopropyl | H | CF3 |
| 2-654 | 4-bromo-2-chlorophenyl | Me | H | H | CN | H | H | Br | H | heptafluoroisopropyl | H | CF3 |
| 2-655 | 2-bromo-4-chlorophenyl | Me | H | H | CN | H | H | Br | H | heptafluoroisopropyl | H | CF3 |
| 2-656 | 2-bromo-4-fluorophenyl | Me | H | H | CN | H | H | Br | H | heptafluoroisopropyl | H | CF3 |
| 2-657 | 2-chloro-4-nitrophenyl | Me | H | H | CN | H | H | Br | H | heptafluoroisopropyl | H | CF3 |
| 2-658 | 3,5-dicyanophenyl | Me | H | H | CN | H | H | Br | H | heptafluoroisopropyl | H | CF3 |
| 2-659 | 4-cyano-2-fluorophenyl | S(O)2Et | H | H | CN | H | H | Br | H | heptafluoroisopropyl | H | CF3 |
| 2-660 | 2-chloro-4-cyanophenyl | Me | H | H | CN | H | H | Br | H | heptafluoroisopropyl | H | CF3 |
| 2-661 | pyridin-3-yl | Me | H | H | CN | H | H | Br | H | heptafluoroisopropyl | H | CF3 |
| 2-662 | 2-fluoropyridin-3-yl | Me | H | H | CN | H | H | Br | H | heptafluoroisopropyl | H | CF3 |
| 2-663 | 2-chloropyridin-3-yl | Me | H | H | CN | H | H | Br | H | heptafluoroisopropyl | H | CF3 |
| 2-664 | 2-bromopyridin-3-yl | Me | H | H | CN | H | H | Br | H | heptafluoroisopropyl | H | CF3 |
| 2-665 | 2-iodopyridin-3-yl | Me | H | H | CN | H | H | Br | H | heptafluoroisopropyl | H | CF3 |
| 2-666 | 2- (trifluoromethyl) pyridin-3-yl | Me | H | H | CN | H | H | Br | H | heptafluoroisopropyl | H | CF3 |
| 2-667 | 2-nitropyridin-3-yl | Me | H | H | CN | H | H | Br | H | heptafluoroisopropyl | H | CF3 |
| 2-668 | 2-cyanopyridin-3-yl | Me | H | H | CN | H | H | Br | H | heptafluoroisopropyl | H | CF3 |
| 2-669 | 6-fluoropyridin-3-yl | Me | H | H | CN | H | H | Br | H | heptafluoroisopropyl | H | CF3 |
| 2-670 | 6-chloropyridin-3-yl | Me | H | H | CN | H | H | Br | H | heptafluoroisopropyl | H | CF3 |
| 2-671 | 6-bromopyridin-3-yl | Me | H | H | CN | H | H | Br | H | heptafluoroisopropyl | H | CF3 |
| 2-672 | 6-iodopyridin-3-yl | Me | H | H | CN | H | H | Br | H | heptafluoroisopropyl | H | CF3 |
| 2-673 | 6- (trifluoromethyl) pyridin-3-yl | Me | H | H | CN | H | H | Br | H | heptafluoroisopropyl | H | CF3 |
| 2-674 | 6-nitropyridin-3-yl | CH2Ph | H | H | CN | H | H | Br | H | heptafluoroisopropyl | H | CF3 |
| 2-675 | 6-cyanopyridin-3-yl | Me | H | H | CN | H | H | Br | H | heptafluoroisopropyl | H | CF3 |
| 2-676 | 5-fluoropyridin-3-yl | Me | H | H | CN | H | H | Br | H | heptafluoroisopropyl | H | CF3 |

**Table 2(3)**

| compound number | Q₁ | R₁ | R₂ | X₄ | X₂ | X₃ | X₄ | Y₁ | Y₂ | Y₃ | Y₄ | Y₅ |
|---|---|---|---|---|---|---|---|---|---|---|---|---|
| 2-677 | 5-chloropyridin-3-yl | Me | H | H | CN | H | H | Br | H | heptafluoroisopropyl | H | CF3 |
| 2-678 | 5-bromopyridin-3-yl | Me | H | H | CN | H | H | Br | H | heptafluoroisopropyl | H | CF3 |
| 2-679 | 5-iodopyridin-3-yl | Me | H | H | CN | H | H | Br | H | heptafluoroisopropyl | H | CF3 |
| 2-680 | 5- (trifluoromethyl) pyridin-3-yl | Me | H | H | CN | H | H | Br | H | heptafluoroisopropyl | H | CF3 |
| 2-681 | 5-nitropyridin-3-yl | Me | H | H | CN | H | H | Br | H | heptafluoroisopropyl | H | CF3 |
| 2-682 | 5-cyanopyridin-3-yl | Me | H | H | CN | H | H | Br | H | heptafluoroisopropyl | H | CF3 |
| 2-683 | 4-fluoropyridin-3-yl | Me | H | H | CN | H | H | Br | H | heptafluoroisopropyl | H | CF3 |
| 2-684 | 4-chloropyridin-3-yl | Me | H | H | CN | H | H | Br | H | heptafluoroisopropyl | H | CF3 |
| 2-685 | 4-bromopyridin-3-yl | Me | H | H | CN | H | H | Br | H | heptafluoroisopropyl | H | CF3 |
| 2-686 | 4-iodopyridin-3-yl | Me | H | H | CN | H | H | Br | H | heptafluoroisopropyl | H | CF3 |
| 2-687 | 4- (trifluoromethyl) pyridin-3-yl | Me | H | H | CN | H | H | Br | H | heptafluoroisopropyl | H | CF3 |
| 2-688 | 4-nitropyridin-3-yl | CH2(3-Py) | H | H | CN | H | H | Br | H | heptafluoroisopropyl | H | CF3 |
| 2-689 | 4-cyanopyridin-3-yl | Me | H | H | CN | H | H | Br | H | heptafluoroisopropyl | H | CF3 |
| 2-690 | 2,6-diohloropyridin-3-yl | Me | H | H | CN | H | H | Br | H | heptafluoroisopropyl | H | CF3 |
| 2-691 | pyridin-3-yl N-oxide | Me | H | H | CN | H | H | Br | H | heptafluoroisopropyl | H | CF3 |
| 2-692 | pyridin-4-yl | Me | H | H | CN | H | H | Br | H | heptafluoroisopropyl | H | CF3 |
| 2-693 | 2-chloropyridin-4-yl | Me | H | H | CN | H | H | Br | H | heptafluoroisopropyl | H | CF3 |
| 2-694 | 3-bromopyridin-4-yl | Me | H | H | CN | H | H | Br | H | heptafluoroisopropyl | H | CF3 |
| 2-695 | 3,5-dichloropyridin-4-yl | Me | H | H | CN | H | H | Br | H | heptafluoroisopropyl | H | CF3 |
| 2-696 | 3-(trifluoromethyl)pyridin-4-yl | Me | H | H | CN | H | H | Br | H | heptafluoroisopropyl | H | CF3 |
| 2-697 | 2,6-dicyanopyridin-4-yl | Me | H | H | CN | H | H | Br | H | heptafluoroisopropyl | H | CF3 |
| 2-698 | pyridin-4-yl N-oxide | Me | H | H | CN | H | H | Br | H | heptafluoroisopropyl | H | CF3 |
| 2-699 | pyridin-2-yl | Me | H | H | CN | H | H | Br | H | heptafluoroisopropyl | H | CF3 |
| 2-700 | 3-chloropyridin-2-yl | Me | H | H | CN | H | H | Br | H | heptafluoroisopropyl | H | CF3 |
| 2-701 | 4-bromopyridin-2-yl | Me | H | H | CN | H | H | Br | H | heptafluoroisopropyl | H | CF3 |
| 2-702 | 5-iodopyridin-2-yl | CH2(3-Py-N-oxide) | H | H | CN | H | H | Br | H | heptafluoroisopropyl | H | CF3 |
| 2-703 | 6-chloropyridin-2-yl | Me | H | H | CN | H | H | Br | H | heptafluoroisopropyl | H | CF3 |
| 2-704 | 4-cyanopyridin-2-yl | Me | H | H | CN | H | H | Br | H | heptafluoroisopropyl | H | CF3 |
| 2-705 | phenyl | Me | H | H | CN | H | H | Br | H | nonafluoro-2-butyl | H | CF3 |
| 2-706 | 2-fluorophenyl | Me | H | H | CN | H | H | Br | H | nonafluoro-2-butyl | H | CF3 |
| 2-707 | 3-fluorophenyl | Me | H | H | CN | H | H | Br | H | nonafluoro-2-butyl | H | CF3 |
| 2-708 | 4-fluorophenyl | Me | H | H | CN | H | H | Br | H | nonafluoro-2-butyl | H | CF3 |
| 2-709 | 2-chlorophenyl | Me | H | H | CN | H | H | Br | H | nonafluoro-2-butyl | H | CF3 |
| 2-710 | 3-chlorophenyl | Me | H | H | CN | H | H | Br | H | nonafluoro-2-butyl | H | CF3 |
| 2-711 | 4-chlorophenyl | Me | H | H | CN | H | H | Br | H | nonafluoro-2-butyl | H | CF3 |
| 2-712 | 2-bromophenyl | Me | H | H | CN | H | H | Br | H | nonafluoro-2-butyl | H | CF3 |
| 2-713 | 3-bromophenyl | Me | H | H | CN | H | H | Br | H | nonafluoro-2-butyl | H | CF3 |
| 2-714 | 4-bromophenyl | Me | H | H | CN | H | H | Br | H | nonafluoro-2-butyl | H | CF3 |
| 2-715 | 2-iodophenyl | Me | H | H | CN | H | H | Br | H | nonafluoro-2-butyl | H | CF3 |
| 2-716 | 3-iodophenyl | Me | H | H | CN | H | H | Br | H | nonafluoro-2-butyl | H | CF3 |
| 2-717 | 4-iodophenyl | Me | H | H | CN | H | H | Br | H | nonafluoro-2-butyl | H | CF3 |
| 2-718 | (2-trifluoromethyl)phenyl | Me | H | H | CN | H | H | Br | H | nonafluoro-2-butyl | H | CF3 |
| 2-719 | (3-trifluoromethyl) phenyl | Me | H | H | CN | H | H | Br | H | nonafluoro-2-butyl | H | CF3 |
| 2-720 | (4-trifluoromethyl)phenyl | Me | H | H | CN | H | H | Br | H | nonafluoro-2-butyl | H | CF3 |
| 2-721 | 2-nitrophenyl | Me | H | H | CN | H | H | Br | H | nonafluoro-2-butyl | H | CF3 |
| 2-722 | 3-nitrophenyl | Me | H | H | CN | H | H | Br | H | nonafluoro-2-butyl | H | CF3 |
| 2-723 | 4-nitrophenyl | Me | H | H | CN | H | H | Br | H | nonafluoro-2-butyl | H | CF3 |
| 2-724 | 2-cyanophenyl | Me | H | H | CN | H | H | Br | H | nonafluoro-2-butyl | H | CF3 |
| 2-725 | 3-cyanophenyl | Me | H | H | CN | H | H | Br | H | nonafluoro-2-butyl | H | CF3 |
| 2-726 | 4-cyanophenyl | Me | H | H | CN | H | H | Br | H | nonafluoro-2-butyl | H | CF3 |
| 2-727 | 2,6-difluorophenyl | Me | H | H | CN | H | H | Br | H | nonafluoro-2-butyl | H | CF3 |
| 2-728 | 3,4-dichlorophenyl | Me | H | H | CN | H | H | Br | H | nonafluoro-2-butyl | H | CF3 |
| 2-729 | 2,4-dichlorophenyl | Me | H | H | CN | H | H | Br | H | nonafluoro-2-butyl | H | CF3 |
| 2-730 | 2-chloro-4-fluorophenyl | Me | H | H | CN | H | H | Br | H | nonafluoro-2-butyl | H | CF3 |
| 2-731 | 2-chloro-4,5-difluorophenyl | Me | H | H | CN | H | H | Br | H | nonafluoro-2-butyl | H | CF3 |
| 2-732 | 4-bromo-2-chlorophenyl | Et | H | H | CN | H | H | Br | H | nonafluoro-2-butyl | H | CF3 |
| 2-733 | 2-bromo-4-chlorophenyl | Me | H | H | CN | H | H | Br | H | nonafluoro-2-butyl | H | CF3 |
| 2-734 | 2-bromo-4-fluorophenyl | Me | H | H | CN | H | H | Br | H | nonafluoro-2-butyl | H | CF3 |
| 2-735 | 2-chloro-4-nitrophenyl | Me | H | H | CN | H | H | Br | H | nonafluoro-2-butyl | H | CF3 |
| 2-736 | 3,5-dicyanophenyl | Me | H | H | CN | H | H | Br | H | nonafluoro-2-butyl | H | CF3 |
| 2-737 | 4-cyano-2-fluorophenyl | Me | H | H | CN | H | H | Br | H | nonafluoro-2-butyl | H | CF3 |
| 2-738 | 2-chloro-4-cyanophenyl | Me | H | H | CN | H | H | Br | H | nonafluoro-2-butyl | H | CF3 |

**Table 2(4)**

| compound | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 2-739 | pyridin-3-yl | Me | H | H | CN | H | H | Br | H | nonafluoro-2-butyl | H | CF3 |
| 2-740 | 2-fluoropyridin-3-yl | Me | H | H | CN | H | H | Br | H | nonafluoro-2-butyl | H | CF3 |
| 2-741 | 2-chloropyridin-3-yl | Me | H | H | CN | H | H | Br | H | nonafluoro-2-butyl | H | CF3 |
| 2-742 | 2-bromopyridin-3-yl | Me | H | H | CN | H | H | Br | H | nonafluoro-2-butyl | H | CF3 |
| 2-743 | 2-iodopyridin-3-yl | Me | H | H | CN | H | H | Br | H | nonafluoro-2-butyl | H | CF3 |
| 2-744 | 2- (trifluoromethyl) pyridin-3-yl | Me | H | H | CN | H | H | Br | H | nonafluoro-2-butyl | H | CF3 |
| 2-745 | 2-nitropyridin-3-yl | Me | H | H | CN | H | H | Br | H | nonafluoro-2-butyl | H | CF3 |
| 2-746 | 2-cyanopyridin-3-yl | Me | H | H | CN | H | H | Br | H | nonafluoro-2-butyl | H | CF3 |
| 2-747 | 6-fluoropyridin-3-yl | Me | H | H | CN | H | H | Br | H | nonafluoro-2-butyl | H | CF3 |
| 2-748 | 6-chloropyridin-3-yl | Me | H | H | CN | H | H | Br | H | nonafluoro-2-butyl | H | CF3 |
| 2-749 | 6-bromopyridin-3-yl | Me | H | H | CN | H | H | Br | H | nonafluoro-2-butyl | H | CF3 |
| 2-750 | 6-iodopyridin-3-yl | Me | H | H | CN | H | H | Br | H | nonafluoro-2-butyl | H | CF3 |
| 2-751 | 6- (trifluoromethyl) pyridin-3-yl | Me | H | H | CN | H | H | Br | H | nonafluoro-2-butyl | H | CF3 |
| 2-752 | 6-nitropyridin-3-yl | Me | H | H | CN | H | H | Br | H | nonafluoro-2-butyl | H | CF3 |
| 2-753 | 6-cyanopyridin-3-yl | Me | H | H | CN | H | H | Br | H | nonafluoro-2-butyl | H | CF3 |
| 2-754 | 5-fluoropyridin-3-yl | Me | H | H | CN | H | H | Br | H | nonafluoro-2-butyl | H | CF3 |
| 2-755 | 5-chloropyridin-3-yl | Me | H | H | CN | H | H | Br | H | nonafluoro-2-butyl | H | CF3 |
| 2-756 | 5-bromopyridin-3-yl | Me | H | H | CN | H | H | Br | H | nonafluoro-2-butyl | H | CF3 |
| 2-757 | 5-iodopyridin-3-yl | Me | H | H | CN | H | H | Br | H | nonafluoro-2-butyl | H | CF3 |
| 2-758 | 5- (trifluoromethyl) pyridin-3-yl | CN | H | H | CN | H | H | Br | H | nonafluoro-2-butyl | H | CF3 |
| 2-759 | 5-nitropyridin-3-yl | Me | H | H | CN | H | H | Br | H | nonafluoro-2-butyl | H | CF3 |
| 2-760 | 5-cyanopyridin-3-yl | Me | H | H | CN | H | H | Br | H | nonafluoro-2-butyl | H | CF3 |
| 2-761 | 4-fluoropyridin-3-yl | Me | H | H | CN | H | H | Br | H | nonafluoro-2-butyl | H | CF3 |
| 2-762 | 4-chloropyridin-3-yl | Me | H | H | CN | H | H | Br | H | nonafluoro-2-butyl | H | CF3 |
| 2-763 | 4-bromopyridin-3-yl | Me | H | H | CN | H | H | Br | H | nonafluoro-2-butyl | H | CF3 |
| 2-764 | 4-iodopyridin-3-yl | Me | H | H | CN | H | H | Br | H | nonafluoro-2-butyl | H | CF3 |
| 2-765 | 4- (trifluoromethyl) pyridin-3-yl | Me | H | H | CN | H | H | Br | H | nonafluoro-2-butyl | H | CF3 |
| 2-766 | 4-nitropyridin-3-yl | Me | H | H | CN | H | H | Br | H | nonafluoro-2-butyl | H | CF3 |
| 2-767 | 4-cyanopyridin-3-yl | Me | H | H | CN | H | H | Br | H | nonafluoro-2-butyl | H | CF3 |
| 2-768 | 2,6-dichloropyridin-3-yl | Me | H | H | CN | H | H | Br | H | nonafluoro-2-butyl | H | CF3 |
| 2-769 | pyridin-3-yl N-oxide | Me | H | H | CN | H | H | Br | H | nonafluoro-2-butyl | H | CF3 |
| 2-770 | pyridin-4-yl | Me | H | H | CN | H | H | Br | H | nonafluoro-2-butyl | H | CF3 |
| 2-771 | 2-chloropyridin-4-yl | Me | H | H | CN | H | H | Br | H | nonafluoro-2-butyl | H | CF3 |
| 2-772 | 3-bromopyridin-4-yl | Me | H | H | CN | H | H | Br | H | nonafluoro-2-butyl | H | CF3 |
| 2-773 | 3,5-dichloropyridin-4-yl | Me | H | H | CN | H | H | Br | H | nonafluoro-2-butyl | H | CF3 |
| 2-774 | 3-(trifluoromethyl) pyridin-4-yl | Me | H | H | CN | H | H | Br | H | nonafluoro-2-butyl | H | CF3 |
| 2-775 | 26-dicyanopyridin-4-yl | Me | H | H | CN | H | H | Br | H | nonafluoro-2-butyl | H | CF3 |
| 2-776 | pyridin-4-yl N-oxide | Me | H | H | CN | H | H | Br | H | nonafluoro-2-butyl | H | CF3 |
| 2-777 | pyridin-2-yl | Me | H | H | CN | H | H | Br | H | nonafluoro-2-butyl | H | CF3 |
| 2-778 | 3-chloropyridin-2-yl | Me | H | H | CN | H | H | Br | H | nonafluoro-2-butyl | H | CF3 |
| 2-779 | 4-bromopyridin-2-yl | Me | H | H | CN | H | H | Br | H | nonafluoro-2-butyl | H | CF3 |
| 2-780 | 5-iodopyridin-2-yl | Me | H | H | CN | H | H | Br | H | nonafluoro-2-butyl | H | CF3 |
| 2-781 | 6-chloropyridin-2-yl | Me | H | H | CN | H | H | Br | H | nonafluoro-2-butyl | H | CF3 |
| 2-782 | 4-cyanopyridin-2-yl | Me | H | H | CN | H | H | Br | H | nonafluoro-2-butyl | H | CF3 |
| 2-783 | phenyl | Me | H | H | CN | H | H | I | H | heptafluoroisopropyl | H | CF3 |
| 2-784 | 2-fluorophenyl | Me | H | H | CN | H | H | I | H | heptafluoroisopropyl | H | CF3 |
| 2-785 | 3-fluorophenyl | Me | H | H | CN | H | H | I | H | heptafluoroisopropyl | H | CF3 |
| 2-786 | 4-fluorophenyl | Me | H | H | CN | H | H | I | H | heptafluoroisopropyl | H | CF3 |
| 2-787 | 2-chlorophenyl | Me | H | H | CN | H | H | I | H | heptafluoroisopropyl | H | CF3 |
| 2-788 | 3-chlorophenyl | Me | H | H | CN | H | H | I | H | heptafluoroisopropyl | H | CF3 |
| 2-789 | 4-chlorophenyl | Me | H | H | CN | H | H | I | H | heptafluoroisopropyl | H | CF3 |
| 2-790 | 2-bromophenyl | Me | H | H | CN | H | H | I | H | heptafluoroisopropyl | H | CF3 |
| 2-791 | 3-bromophenyl | Me | H | H | CN | H | H | I | H | heptafluoroisopropyl | H | CF3 |
| 2-792 | 4-bromophenyl | Me | H | H | CN | H | H | I | H | heptafluoroisopropyl | H | CF3 |
| 2-793 | 2-iodophenyl | Me | H | H | CN | H | H | I | H | heptafluoroisopropyl | H | CF3 |
| 2-794 | 3-iodophenyl | Me | H | H | CN | H | H | I | H | heptafluoroisopropyl | H | CF3 |
| 2-795 | 4-iodophenyl | Me | H | H | CN | H | H | I | H | heptafluoroisopropyl | H | CF3 |
| 2-796 | (2-trifluoromethyl) phenyl | Me | H | H | CN | H | H | I | H | heptafluoroisopropyl | H | CF3 |
| 2-797 | (3-trifluoromethyl) phenyl | Me | H | H | CN | H | H | I | H | heptafluoroisopropyl | H | CF3 |
| 2-798 | (4-trifluoromethyl) phenyl | Me | H | H | CN | H | H | I | H | heptafluoroisopropyl | H | CF3 |
| 2-799 | 2-nitrophenyl | Me | H | H | CN | H | H | I | H | heptafluoroisopropyl | H | CF3 |
| 2-800 | 3-nitrophenyl | Me | H | H | CN | H | H | I | H | heptafluoroisopropyl | H | CF3 |
| 2-801 | 4-nitrophenyl | Me | H | H | CN | H | H | I | H | heptafluoroisopropyl | H | CF3 |
| 2-802 | 2-cyanophenyl | Me | H | H | CN | H | H | I | H | heptafluoroisopropyl | H | CF3 |

**Table 2(5)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 2-803 | 3-cyanophenyl | Me | H | H | CN | H | H | I | H | heptafluoroisopropyl | H | CF3 |
| 2-804 | 4-cyanophenyl | Me | H | H | CN | H | H | I | H | heptafluoroisopropyl | H | CF3 |
| 2-805 | 2,6-difluorophenyl | Me | H | H | CN | H | H | I | H | heptafluoroisopropyl | H | CF3 |
| 2-806 | 3,4-dichlorophenyl | Me | H | H | CN | H | H | I | H | heptafluoroisopropyl | H | CF3 |
| 2-807 | 2,4-dichlorophenyl | Me | H | H | CN | H | H | I | H | heptafluoroisopropyl | H | CF3 |
| 2-808 | 2-chloro-4-fluorophenyl | Me | H | H | CN | H | H | I | H | heptafluoroisopropyl | H | CF3 |
| 2-809 | 2-chloro-4,5-difluorophenyl | Me | H | H | CN | H | H | I | H | heptafluoroisopropyl | H | CF3 |
| 2-810 | 4-bromo-2-chlorophenyl | Me | H | H | CN | H | H | I | H | heptafluoroisopropyl | H | CF3 |
| 2-811 | 2-bromo-4-chlorophenyl | Me | H | H | CN | H | H | I | H | heptafluoroisopropyl | H | CF3 |
| 2-812 | 2-bromo-4-fluorophenyl | Me | H | H | CN | H | H | I | H | heptafluoroisopropyl | H | CF3 |
| 2-813 | 2-chloro-4-nitrophenyl | Me | H | H | CN | H | H | I | H | heptafluoroisopropyl | H | CF3 |
| 2-814 | 3,5-dicyanophenyl | Me | H | H | CN | H | H | I | H | heptafluoroisopropyl | H | CF3 |
| 2-815 | 4-cyano-2-fluorophenyl | Me | H | H | CN | H | H | I | H | heptafluoroisopropyl | H | CF3 |
| 2-816 | 2-chloro-4-cyanophenyl | Me | H | H | CN | H | H | I | H | heptafluoroisopropyl | H | CF3 |
| 2-817 | pyridin-3-yl | Me | H | H | CN | H | H | I | H | heptafluoroisopropyl | H | CF3 |
| 2-818 | 2-fluoropyridin-3-yl | Me | H | H | CN | H | H | I | H | heptafluoroisopropyl | H | CF3 |
| 2-819 | 2-chloropyridin-3-yl | Me | H | H | CN | H | H | I | H | heptafluoroisopropyl | H | CF3 |
| 2-820 | 2-bromopyridin-3-yl | Me | H | H | CN | H | H | I | H | heptafluoroisopropyl | H | CF3 |
| 2-821 | 2-iodopyridin-3-yl | Me | H | H | CN | H | H | I | H | heptafluoroisopropyl | H | CF3 |
| 2-822 | 2-(trifluoromethyl)pyridin-3-yl | Me | H | H | CN | H | H | I | H | heptafluoroisopropyl | H | CF3 |
| 2-823 | 2-nitropyridin-3-yl | Me | H | H | CN | H | H | I | H | heptafluoroisopropyl | H | CF3 |
| 2-824 | 2-cyanopyridin-3-yl | Me | H | H | CN | H | H | I | H | heptafluoroisopropyl | H | CF3 |
| 2-825 | 6-fluoropyridin-3-yl | Me | H | H | CN | H | H | I | H | heptafluoroisopropyl | H | CF3 |
| 2-826 | 6-chloropyridin-3-yl | Me | H | H | CN | H | H | I | H | heptafluoroisopropyl | H | CF3 |
| 2-827 | 6-bromopyridin-3-yl | Me | H | H | CN | H | H | I | H | heptafluoroisopropyl | H | CF3 |
| 2-828 | 6-iodopyridin-3-yl | Me | H | H | CN | H | H | I | H | heptafluoroisopropyl | H | CF3 |
| 2-829 | 6-(trifluoromethyl)pyridin-3-yl | Me | H | H | CN | H | H | I | H | heptafluoroisopropyl | H | CF3 |
| 2-830 | 6-nitropyridin-3-yl | Me | H | H | CN | H | H | I | H | heptafluoroisopropyl | H | CF3 |
| 2-831 | 6-cyanopyridin-3-yl | Me | H | H | CN | H | H | I | H | heptafluoroisopropyl | H | CF3 |
| 2-832 | 5-fluoropyridin-3-yl | Me | H | H | CN | H | H | I | H | heptafluoroisopropyl | H | CF3 |
| 2-833 | 5-chloropyridin-3-yl | Me | H | H | CN | H | H | I | H | heptafluoroisopropyl | H | CF3 |
| 2-834 | 5-bromopyridin-3-yl | Me | H | H | CN | H | H | I | H | heptafluoroisopropyl | H | CF3 |
| 2-835 | 5-iodopyridin-3-yl | Me | H | H | CN | H | H | I | H | heptafluoroisopropyl | H | CF3 |
| 2-836 | 5-(trifluoromethyl)pyridin-3-yl | Me | H | H | CN | H | H | I | H | heptafluoroisopropyl | H | CF3 |
| 2-837 | 5-nitropyridin-3-yl | Me | H | H | CN | H | H | I | H | heptafluoroisopropyl | H | CF3 |
| 2-838 | 5-cyanopyridin-3-yl | Me | H | H | CN | H | H | I | H | heptafluoroisopropyl | H | CF3 |
| 2-839 | 4-fluoropyridin-3-yl | Me | H | H | CN | H | H | I | H | heptafluoroisopropyl | H | CF3 |
| 2-840 | 4-chloropyridin-3-yl | Me | H | H | CN | H | H | I | H | heptafluoroisopropyl | H | CF3 |
| 2-841 | 4-bromopyridin-3-yl | C(O)Me | H | H | CN | H | H | I | H | heptafluoroisopropyl | H | CF3 |
| 2-842 | 4-iodopyridin-3-yl | Me | H | H | CN | H | H | I | H | heptafluoroisopropyl | H | CF3 |
| 2-843 | 4-(trifluoromethyl)pyridin-3-yl | Me | H | H | CN | H | H | I | H | heptafluoroisopropyl | H | CF3 |
| 2-844 | 4-nitropyridin-3-yl | Me | H | H | CN | H | H | I | H | heptafluoroisopropyl | H | CF3 |
| 2-845 | 4-cyanopyridin-3-yl | Me | H | H | CN | H | H | I | H | heptafluoroisopropyl | H | CF3 |
| 2-846 | 2,6-dichloropyridin-3-yl | Me | H | H | CN | H | H | I | H | heptafluoroisopropyl | H | CF3 |
| 2-847 | pyridin-3-yl N-oxide | Me | H | H | CN | H | H | I | H | heptafluoroisopropyl | H | CF3 |
| 2-848 | pyridin-4-yl | Me | H | H | CN | H | H | I | H | heptafluoroisopropyl | H | CF3 |
| 2-849 | 2-chloropyridin-4-yl | Me | H | H | CN | H | H | I | H | heptafluoroisopropyl | H | CF3 |
| 2-850 | 3-bromopyridin-4-yl | Me | H | H | CN | H | H | I | H | heptafluoroisopropyl | H | CF3 |
| 2-851 | 3,5-dichloropyridin-4-yl | Me | H | H | CN | H | H | I | H | heptafluoroisopropyl | H | CF3 |
| 2-852 | 3- (trifluoromethyl)pyridin-4-yl | Me | H | H | CN | H | H | I | H | heptafluoroisopropyl | H | CF3 |
| 2-853 | 2,6-dicyanopyridin-4-yl | Me | H | H | CN | H | H | I | H | heptafluoroisopropyl | H | CF3 |
| 2-854 | pyridin-4-yl N-oxide | Me | H | H | CN | H | H | I | H | heptafluoroisopropyl | H | CF3 |
| 2-855 | pyridin-2-yl | Me | H | H | CN | H | H | I | H | heptafluoroisopropyl | H | CF3 |
| 2-856 | 3-chloropyridin-2-yl | Me | H | H | CN | H | H | I | H | heptafluoroisopropyl | H | CF3 |
| 2-857 | 4-bromopyridin-2-yl | Me | H | H | CN | H | H | I | H | heptafluoroisopropyl | H | CF3 |
| 2-858 | 5-iodopyridin-2-yl | Me | H | H | CN | H | H | I | H | heptafluoroisopropyl | H | CF3 |
| 2-859 | 6-chloropyridin-2-yl | Me | H | H | CN | H | H | I | H | heptafluoroisopropyl | H | CF3 |
| 2-860 | 4-cyanopyridin-2-yl | Me | H | H | CN | H | H | I | H | heptafluoroisopropyl | H | CF3 |
| 2-861 | phenyl | Me | H | H | CN | H | H | I | H | nonafluoro-2-butyl | H | CF3 |
| 2-862 | 2-fluorophenyl | Me | H | H | CN | H | H | I | H | nonafluoro-2-butyl | H | CF3 |
| 2-863 | 3-fluorophenyl | Me | H | H | CN | H | H | I | H | nonafluoro-2-butyl | H | CF3 |
| 2-864 | 4-fluorophenyl | Me | H | H | CN | H | H | I | H | nonafluoro-2-butyl | H | CF3 |
| 2-865 | 2-chlorophenyl | Me | H | H | CN | H | H | I | H | nonafluoro-2-butyl | H | CF3 |
| 2-866 | 3-chlorophenyl | Me | H | H | CN | H | H | I | H | nonafluoro-2-butyl | H | CF3 |

**Table 2(6)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 2-867 | 4-chlorophenyl | Me | H | H | CN | H | H | I | H | nonafluoro-2-butyl | H | CF3 |
| 2-868 | 2-bromophenyl | Me | H | H | CN | H | H | I | H | nonafluoro-2-butyl | H | CF3 |
| 2-869 | 3-bromophenyl | Me | H | H | CN | H | H | I | H | nonafluoro-2-butyl | H | CF3 |
| 2-870 | 4-bromophenyl | Me | H | H | CN | H | H | I | H | nonafluoro-2-butyl | H | CF3 |
| 2-871 | 2-iodophenyl | Me | H | H | CN | H | H | I | H | nonafluoro-2-butyl | H | CF3 |
| 2-872 | 3-iodophenyl | Me | H | H | CN | H | H | I | H | nonafluoro-2-butyl | H | CF3 |
| 2-873 | 4-iodophenyl | Me | H | H | CN | H | H | I | H | nonafluoro-2-butyl | H | CF3 |
| 2-874 | (2-trifluoromethyl) phenyl | Me | H | H | CN | H | H | I | H | nonafluoro-2-butyl | H | CF3 |
| 2-875 | (3-trifluoromethyl) phenyl | Me | H | H | CN | H | H | I | H | nonafluoro-2-butyl | H | CF3 |
| 2-876 | (4-trifluoromethyl) phenyl | Me | H | H | CN | H | H | I | H | nonafluoro-2-butyl | H | CF3 |
| 2-877 | 2-nitrophenyl | Me | H | H | CN | H | H | I | H | nonafluoro-2-butyl | H | CF3 |
| 2-878 | 3-nitrophenyl | Me | H | H | CN | H | H | I | H | nonafluoro-2-butyl | H | CF3 |
| 2-879 | 4-nitrophenyl | Me | H | H | CN | H | H | I | H | nonafluoro-2-butyl | H | CF3 |
| 2-880 | 2-cyanophenyl | Me | H | H | CN | H | H | I | H | nonafluoro-2-butyl | H | CF3 |
| 2-881 | 3-cyanophenyl | Me | H | H | CN | H | H | I | H | nonafluoro-2-butyl | H | CF3 |
| 2-882 | 4-cyanophenyl | Me | H | H | CN | H | H | I | H | nonafluoro-2-butyl | H | CF3 |
| 2-883 | 2,6 -difluorophenyl | Me | H | H | CN | H | H | I | H | nonafluoro-2-butyl | H | CF3 |
| 2-884 | 3,4-dichlorophenyl | Me | H | H | CN | H | H | I | H | nonafluoro-2-butyl | H | CF3 |
| 2-885 | 2,4-dichlorophenyl | Me | H | H | CN | H | H | I | H | nonafluoro-2-butyl | H | CF3 |
| 2-886 | 2-chloro-4-fluorophenyl | Me | H | H | CN | H | H | I | H | nonafluoro-2-butyl | H | CF3 |
| 2-887 | 2-chloro-4,5-difluorophenyl | Me | H | H | CN | H | H | I | H | nonafluoro-2-butyl | H | CF3 |
| 2-888 | 4-bromo-2-chlorophenyl | Me | H | H | CN | H | H | I | H | nonafluoro-2-butyl | H | CF3 |
| 2-889 | 2-bromo-4-chlorophenyl | Me | H | H | CN | H | H | I | H | nonafluoro-2-butyl | H | CF3 |
| 2-890 | 2-bromo-4-fluorophenyl | Me | H | H | CN | H | H | I | H | nonafluoro-2-butyl | H | CF3 |
| 2-891 | 2-chloro-4-nitrophenyl | Me | H | H | CN | H | H | I | H | nonafluoro-2-butyl | H | CF3 |
| 2-892 | 3,5-dicyanophenyl | Me | H | H | CN | H | H | I | H | nonafluoro-2-butyl | H | CF3 |
| 2-893 | 4-cyano-2-fluorophenyl | Me | H | H | CN | H | H | I | H | nonafluoro-2-butyl | H | CF3 |
| 2-894 | 2-chloro-4-cyanophenyl | Me | H | H | CN | H | H | I | H | nonafluoro-2-butyl | H | CF3 |
| 2-895 | pyridin-3-yl | Me | H | H | CN | H | H | I | H | nonafluoro-2-butyl | H | CF3 |
| 2-896 | 2-fluoropyridin-3-yl | Me | H | H | CN | H | H | I | H | nonafluoro-2-butyl | H | CF3 |
| 2-897 | 2-chloropyridin-3-yl | Me | H | H | CN | H | H | I | H | nonafluoro-2-butyl | H | CF3 |
| 2-898 | 2-bromopyridin-3-yl | Me | H | H | CN | H | H | I | H | nonafluoro-2-butyl | H | CF3 |
| 2-899 | 2-iodopyridin-3-yl | Me | H | H | CN | H | H | I | H | nonafluoro-2-butyl | H | CF3 |
| 2-900 | 2- (trifluoromethyl) pyridin-3-yl | Me | H | H | CN | H | H | I | H | nonafluoro-2-butyl | H | CF3 |
| 2-901 | 2-nitropyridin-3-yl | Me | H | H | CN | H | H | I | H | nonafluoro-2-butyl | H | CF3 |
| 2-902 | 2-cyanopyridin-3-yl | Me | H | H | CN | H | H | I | H | nonafluoro-2-butyl | H | CF3 |
| 2-903 | 6-fluoropyridin-3-yl | Me | H | H | CN | H | H | I | H | nonafluoro-2-butyl | H | CF3 |
| 2-904 | 6-chloropyridin-3-yl | Me | H | H | CN | H | H | I | H | nonafluoro-2-butyl | H | CF3 |
| 2-905 | 6-bromopyridin-3-yl | Me | H | H | CN | H | H | I | H | nonafluoro-2-butyl | H | CF3 |
| 2-906 | 6-iodopyridin-3-yl | Me | H | H | CN | H | H | I | H | nonafluoro-2-butyl | H | CF3 |
| 2-907 | 6- (trifluoromethyl) pyridin-3-yl | Me | H | H | CN | H | H | I | H | nonafluoro-2-butyl | H | CF3 |
| 2-908 | 6-nitropyridin-3-yl | Me | H | H | CN | H | H | I | H | nonafluoro-2-butyl | H | CF3 |
| 2-909 | 6-cyanopyridin-3-yl | Me | H | H | CN | H | H | I | H | nonafluoro-2-butyl | H | CF3 |
| 2-910 | 5-fluoropyridin-3-yl | Me | H | H | CN | H | H | I | H | nonafluoro-2-butyl | H | CF3 |
| 2-911 | 5-chloropyridin-3-yl | Me | H | H | CN | H | H | I | H | nonafluoro-2-butyl | H | CF3 |
| 2-912 | 5-bromopyridin-3-yl | Me | H | H | CN | H | H | I | H | nonafluoro-2-butyl | H | CF3 |
| 2-913 | 5-iodopyridin-3-yl | Me | H | H | CN | H | H | I | H | nonafluoro-2-butyl | H | CF3 |
| 2-914 | 5- (trifluoromethyl) pyridin-3-yl | Me | H | H | CN | H | H | I | H | nonafluoro-2-butyl | H | CF3 |
| 2-915 | 5-nitropyridin-3-yl | Me | H | H | CN | H | H | I | H | nonafluoro-2-butyl | H | CF3 |
| 2-916 | 5-cyanopyridin-3-yl | Me | H | H | CN | H | H | I | H | nonafluoro-2-butyl | H | CF3 |
| 2-917 | 4-fluoropyridin-3-yl | Me | H | H | CN | H | H | I | H | nonafluoro-2-butyl | H | CF3 |
| 2-918 | 4-chloropyridin-3-yl | Me | H | H | CN | H | H | I | H | nonafluoro-2-butyl | H | CF3 |
| 2-919 | 4-bromopyridin-3-yl | Me | H | H | CN | H | H | I | H | nonafluoro-2-butyl | H | CF3 |
| 2-920 | 4-iodopyridin-3-yl | Me | H | H | CN | H | H | I | H | nonafluoro-2-butyl | H | CF3 |
| 2-921 | 4- (trifluoromethyl) pyridin-3-yl | Me | H | H | CN | H | H | I | H | nonafluoro-2-butyl | H | CF3 |
| 2-922 | 4-nitropyridin-3-yl | Me | H | H | CN | H | H | I | H | nonafluoro-2-butyl | H | CF3 |
| 2-923 | 4-cyanopyridin-3-yl | Me | H | H | CN | H | H | I | H | nonafluoro-2-butyl | H | CF3 |
| 2-924 | 2,6-dichloropyridin-3-yl | Me | H | H | CN | H | H | I | H | nonafluoro-2-butyl | H | CF3 |
| 2-925 | pyridin-3-yl N-oxide | Me | H | H | CN | H | H | I | H | nonafluoro-2-butyl | H | CF3 |
| 2-926 | pyridin-4-yl | Me | H | H | CN | H | H | I | H | nonafluoro-2-butyl | H | CF3 |
| 2-927 | 2-chloropyridin-4-yl | Me | H | H | CN | H | H | I | H | nonafluoro-2-butyl | H | CF3 |
| 2-928 | 3-bromopyridin-4-yl | Me | H | H | CN | H | H | I | H | nonafluoro-2-butyl | H | CF3 |
| 2-929 | 3,5-dichloropyridin-4-yl | Me | H | H | CN | H | H | I | H | nonafluoro-2-butyl | H | CF3 |
| 2-930 | 3- (trifluoromethyl) pyridin-4-yl | Me | H | H | CN | H | H | I | H | nonafluoro-2-butyl | H | CF3 |

**Table 2(7)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 2-931 | 2,6-dicyanopyridin-4-yl | Me | H | H | CN | H | H | I | H | nonafluoro-2-butyl | H | CF3 |
| 2-932 | pyridin-4-yl N-oxide | Me | H | H | CN | H | H | I | H | nonafluoro-2-butyl | H | CF3 |
| 2-933 | pyridin-2-yl | Me | H | H | CN | H | H | I | H | nonafluoro-2-butyl | H | CF3 |
| 2-934 | 3-chloropyridin-2-yl | Me | H | H | CN | H | H | I | H | nonafluoro-2-butyl | H | CF3 |
| 2-935 | 4-bromopyridin-2-yl | Me | H | H | CN | H | H | I | H | nonafluoro-2-butyl | H | CF3 |
| 2-936 | 5-iodopyridin-2-yl | Me | H | H | CN | H | H | I | H | nonafluoro-2-butyl | H | CF3 |
| 2-937 | 6-chloropyridin-2-yl | Me | H | H | CN | H | H | I | H | nonafluoro-2-butyl | H | CF3 |
| 2-938 | 4-cyanopyridin-2-yl | Me | H | H | CN | H | H | I | H | nonafluoro-2-butyl | H | CF3 |
| 2-939 | phenyl | Me | H | H | CN | H | H | OCF3 | H | heptafluoroisopropyl | H | OCF3 |
| 2-970 | 2-chloropyridin-3-yl | Me | H | H | CN | H | H | OCF3 | H | heptafluoroisopropyl | H | OCF3 |
| 2-995 | phenyl | Me | H | H | CN | H | H | OCF3 | H | nonafluoro-2-butyl | H | OCF3 |
| 2-1026 | 2-chloropyridin-3-yl | Me | H | H | CN | H | H | OCF3 | H | nonafluoro-2-butyl | H | OCF3 |
| 2-1051 | phenyl | Me | H | H | CN | H | H | CF3 | H | heptafluoroisopropyl | H | OCF3 |
| 2-1082 | 2-chloropyridin-3-yl | Me | H | H | CN | H | H | CF3 | H | heptafluoroisopropyl | H | OCF3 |
| 2-1107 | phenyl | Me | H | H | CN | H | H | CF3 | H | nonafluoro-2-butyl | H | OCF3 |
| 2-1138 | 2-chloropyridin-3-yl | Me | H | H | CN | H | H | CF3 | H | nonafluoro-2-butyl | H | OCF3 |
| 2-1163 | phenyl | Me | H | H | CN | H | H | CF3 | H | heptafluoroisopropyl | H | CF3 |
| 2-1183 | 3-cyanophenyl | Me | H | H | CN | H | H | CF3 | H | heptafluoroisopropyl | H | CF3 |
| 2-1184 | 4-cyanophenyl | Me | H | H | CN | H | H | CF3 | H | heptafluoroisopropyl | H | CF3 |
| 2-1199 | 2-chloropyridin-3-yl | Me | H | H | CN | H | H | CF3 | H | heptafluoroisopropyl | H | CF3 |
| 2-1231 | phenyl | Me | H | H | CN | H | H | CF3 | H | nonafluoro-2-butyl | H | CF3 |
| 2-1251 | 3-cyanophenyl | Me | H | H | CN | H | H | CF3 | H | nonafluoro-2-butyl | H | CF3 |
| 2-1252 | 4-cyanophenyl | Me | H | H | CN | H | H | CF3 | H | nonafluoro-2-butyl | H | CF3 |
| 2-1267 | 2-chloropyridin-3-yl | Me | H | H | CN | H | H | CF3 | H | nonafluoro-2-butyl | H | CF3 |
| 2-1299 | phenyl | Me | H | F | CN | H | H | Cl | H | heptafluoroisopropyl | H | OCF3 |
| 2-1319 | 3-cyanophenyl | Me | H | F | CN | H | H | Cl | H | heptafluoroisopropyl | H | OCF3 |
| 2-1320 | 4-cyanophenyl | Me | H | F | CN | H | H | Cl | H | heptafluoroisopropyl | H | OCF3 |
| 2-1335 | 2-chloropyridin-3-yl | Me | H | F | CN | H | H | Cl | H | heptafluoroisopropyl | H | OCF3 |
| 2-1377 | phenyl | Me | H | F | CN | H | H | Cl | H | nonafluoro-2-butyl | H | OCF3 |
| 2-1397 | 3-cyanophenyl | Me | H | F | CN | H | H | Cl | H | nonafluoro-2-butyl | H | OCF3 |
| 2-1398 | 4-cyanophenyl | Me | H | F | CN | H | H | Cl | H | nonafluoro-2-butyl | H | OCF3 |
| 2-1413 | 2-chloropyridin-3-yl | Me | H | F | CN | H | H | Cl | H | nonafluoro-2-butyl | H | OCF3 |
| 2-1455 | phenyl | Me | H | F | CN | H | H | Br | H | heptafluoroisopropyl | H | OCF3 |
| 2-1475 | 3-cyanophenyl | Me | H | F | CN | H | H | Br | H | heptafluoroisopropyl | H | OCF3 |
| 2-1476 | 4-cyanophenyl | Me | H | F | CN | H | H | Br | H | heptafluoroisopropyl | H | OCF3 |
| 2-1491 | 2-chloropyridin-3-yl | Me | H | F | CN | H | H | Br | H | heptafluoroisopropyl | H | OCF3 |
| 2-1533 | phenyl | Me | H | F | CN | H | H | Br | H | nonafluoro-2-butyl | H | OCF3 |
| 2-1553 | 3-cyanophenyl | Me | H | F | CN | H | H | Br | H | nonafluoro-2-butyl | H | OCF3 |
| 2-1554 | 4-cyanophenyl | Me | H | F | CN | H | H | Br | H | nonafluoro-2-butyl | H | OCF3 |
| 2-1569 | 2-chloropyridin-3-yl | Me | H | F | CN | H | H | Br | H | nonafluoro-2-butyl | H | OCF3 |
| 2-1611 | phenyl | Me | H | F | CN | H | H | I | H | heptafluoroisopropyl | H | OCF3 |
| 2-1631 | 3-cyanophenyl | Me | H | F | CN | H | H | I | H | heptafluoroisopropyl | H | OCF3 |
| 2-1632 | 4-cyanophenyl | Me | H | F | CN | H | H | I | H | heptafluoroisopropyl | H | OCF3 |
| 2-1647 | 2-chloropyridin-3-yl | Me | H | F | CN | H | H | I | H | heptafluoroisopropyl | H | OCF3 |
| 2-1689 | phenyl | Me | H | F | CN | H | H | I | H | nonafluoro-2-butyl | H | OCF3 |
| 2-1709 | 3-cyanophenyl | Me | H | F | CN | H | H | I | H | nonafluoro-2-butyl | H | OCF3 |
| 2-1710 | 4-cyanophenyl | Me | H | F | CN | H | H | I | H | nonafluoro-2-butyl | H | OCF3 |
| 2-1725 | 2-chloropyridin-3-yl | Me | H | F | CN | H | H | I | H | nonafluoro-2-butyl | H | OCF3 |
| 2-1767 | phenyl | Me | H | F | CN | H | H | F | H | heptafluoroisopropyl | H | CF3 |
| 2-1768 | phenyl | Me | H | F | CN | H | H | Cl | H | heptafluoroisopropyl | H | CF3 |
| 2-1788 | 3-cyanophenyl | Me | H | F | CN | H | H | Cl | H | heptafluoroisopropyl | H | CF3 |
| 2-1789 | 4-cyanophenyl | Me | H | F | CN | H | H | Cl | H | heptafluoroisopropyl | H | CF3 |
| 2-1804 | 2-chloropyridin-3-yl | Me | H | F | CN | H | H | Cl | H | heptafluoroisopropyl | H | CF3 |
| 2-1846 | phenyl | Me | H | F | CN | H | H | F | H | nonafluoro-2-butyl | H | CF3 |
| 2-1847 | phenyl | Me | H | F | CN | H | H | Cl | H | nonafluoro-2-butyl | H | CF3 |
| 2-1867 | 3-cyanophenyl | Me | H | F | CN | H | H | Cl | H | nonafluoro-2-butyl | H | CF3 |
| 2-1868 | 4-cyanophenyl | Me | H | F | CN | H | H | Cl | H | nonafluoro-2-butyl | H | CF3 |
| 2-1883 | 2-chloropyridin-3-yl | Me | H | F | CN | H | H | Cl | H | nonafluoro-2-butyl | H | CF3 |
| 2-1925 | phenyl | Me | H | F | CN | H | H | Br | H | heptafluoroisopropyl | H | CF3 |
| 2-1945 | 3-cyanophenyl | Me | H | F | CN | H | H | Br | H | heptafluoroisopropyl | H | CF3 |
| 2-1946 | 4-cyanophenyl | Me | H | F | CN | H | H | Br | H | heptafluoroisopropyl | H | CF3 |
| 2-1961 | 2-chloropyridin-3-yl | Me | H | F | CN | H | H | Br | H | heptafluoroisopropyl | H | CF3 |
| 2-2003 | phenyl | Me | H | F | CN | H | H | Br | H | nonafluoro-2-butyl | H | CF3 |
| 2-2023 | 3-cyanophenyl | Me | H | F | CN | H | H | Br | H | nonafluoro-2-butyl | H | CF3 |

**Table 2(8)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 2-2024 | 4-cyanophenyl | Me | H | F | CN | H | H | Br | H | nonafluoro-2-butyl | H | CF3 |
| 2-2039 | 2-chloropyridin-3-yl | Me | H | F | CN | H | H | Br | H | nonafluoro-2-butyl | H | CF3 |
| 2-2081 | phenyl | Me | H | F | CN | H | H | I | H | heptafluoroisopropyl | H | CF3 |
| 2-2101 | 3-cyanophenyl | Me | H | F | CN | H | H | I | H | heptafluoroisopropyl | H | CF3 |
| 2-2102 | 4-cyanophenyl | Me | H | F | CN | H | H | I | H | heDtafluoroisoDroDyl | H | CF3 |
| 2-2117 | 2-chloropyridin-3-yl | Me | H | F | CN | H | H | I | H | heptafluoroisopropyl | H | CF3 |
| 2-2159 | phenyl | Me | H | F | CN | H | H | I | H | nonafluoro-2-butyl | H | CF3 |
| 2-2179 | 3-cyanophenyl | Me | H | F | CN | H | H | I | H | nonafluoro-2-butyl | H | CF3 |
| 2-2180 | 4-cyanophenyl | Me | H | F | CN | H | H | I | H | nonafluoro-2-butyl | H | CF3 |
| 2-2195 | 2-chloropyridin-3-yl | Me | H | F | CN | H | H | I | H | nonafluoro-2-butyl | H | CF3 |
| 2-2237 | phenyl | Me | H | F | CN | H | H | OCF3 | H | heptafluoroisopropyl | H | OCF3 |
| 2-2268 | 2-chloropyridin-3-yl | Me | H | F | CN | H | H | OCF3 | H | heptafluoroisopropyl | H | OCF3 |
| 2-2293 | phenyl | Me | H | F | CN | H | H | OCF3 | H | nonafluoro-2-butyl | H | OCF3 |
| 2-2324 | 2-chloropyridin-3-yl | Me | H | F | CN | H | H | OCF3 | H | nonafluoro-2-butyl | H | OCF3 |
| 2-2349 | phenyl | Me | H | F | CN | H | H | CF3 | H | heptafluoroisopropyl | H | OCF3 |
| 2-2380 | 2-chloropyridin-3-yl | Me | H | F | CN | H | H | CF3 | H | heptafluoroisopropyl | H | OCF3 |
| 2-2405 | phenyl | Me | H | F | CN | H | H | CF3 | H | nonafluoro-2-butyl | H | OCF3 |
| 2-2436 | 2-chloropyridin-3-yl | Me | H | F | CN | H | H | CF3 | H | nonafluoro-2-butyl | H | OCF3 |
| 2-2461 | phenyl | Me | H | F | CN | H | H | CF3 | H | heptafluoroisopropyl | H | CF3 |
| 2-2481 | 3-cyanophenyl | Me | H | F | CN | H | H | CF3 | H | heptafluoroisopropyl | H | CF3 |
| 2-2482 | 4-cyanophenyl | Me | H | F | CN | H | H | CF3 | H | heptafluoroisopropyl | H | CF3 |
| 2-2497 | 2-chloropyridin-3-yl | Me | H | F | CN | H | H | CF3 | H | heptafluoroisopropyl | H | CF3 |
| 2-2529 | phenyl | Me | H | F | CN | H | H | CF3 | H | nonafluoro-2-butyl | H | CF3 |
| 2-2549 | 3-cyanophenyl | Me | H | F | CN | H | H | CF3 | H | nonafluoro-2-butyl | H | CF3 |
| 2-2550 | 4-cyanophenyl | Me | H | F | CN | H | H | CF3 | H | nonafluoro-2-butyl | H | CF3 |
| 2-2565 | 2-chloropyridin-3-yl | Me | H | F | CN | H | H | CF3 | H | nonafluoro-2-butyl | H | CF3 |
| 2-2597 | phenyl | Me | H | F | CN | F | H | Cl | H | heptafluoroisopropyl | H | OCF3 |
| 2-2617 | 3-cyanophenyl | Me | H | F | CN | F | H | Cl | H | heptafluoroisopropyl | H | OCF3 |
| 2-2618 | 4-cyanophenyl | Me | H | F | CN | F | H | Cl | H | heptafluoroisopropyl | H | OCF3 |
| 2-2633 | 2-chloropyridin-3-yl | Me | H | F | CN | F | H | Cl | H | heptafluoroisopropyl | H | OCF3 |
| 2-2675 | phenyl | Me | H | F | CN | F | H | Cl | H | nonafluoro-2-butyl | H | OCF3 |
| 2-2695 | 3-cyanophenyl | Me | H | F | CN | F | H | Cl | H | nonafluoro-2-butyl | H | OCF3 |
| 2-2696 | 4-cyanophenyl | Me | H | F | CN | F | H | Cl | H | nonafluoro-2-butyl | H | OCF3 |
| 2-2711 | 2-chloropyridin-3-yl | Me | H | F | CN | F | H | Cl | H | nonafluoro-2-butyl | H | OCF3 |
| 2-2753 | phenyl | Me | H | F | CN | F | H | Br | H | heptafluoroisopropyl | H | OCF3 |
| 2-2773 | 3-cyanophenyl | Me | H | F | CN | F | H | Br | H | heptafluoroisopropyl | H | OCF3 |
| 2-2774 | 4-cyanophenyl | Me | H | F | CN | F | H | Br | H | heptafluoroisopropyl | H | OCF3 |
| 2-2789 | 2-chloropyridin-3-yl | Me | H | F | CN | F | H | Br | H | heptafluoroisopropyl | H | OCF3 |
| 2-2831 | phenyl | Me | H | F | CN | F | H | Br | H | nonafluoro-2-butyl | H | OCF3 |
| 2-2851 | 3-cyanophenyl | Me | H | F | CN | F | H | Br | H | nonafluoro-2-butyl | H | OCF3 |
| 2-2852 | 4-cyanophenyl | Me | H | F | CN | F | H | Br | H | nonafluoro-2-butyl | H | OCF3 |
| 2-2867 | 2-chloropyridin-3-yl | Me | H | F | CN | F | H | Br | H | nonafluoro-2-butyl | H | OCF3 |
| 2-2909 | phenyl | Me | H | F | CN | F | H | I | H | heptafluoroisopropyl | H | OCF3 |
| 2-2929 | 3-cyanophenyl | Me | H | F | CN | F | H | I | H | heptafluoroisopropyl | H | OCF3 |
| 2-2930 | 4-cyanophenyl | Me | H | F | CN | F | H | I | H | heptafluoroisopropyl | H | OCF3 |
| 2-2945 | 2-chloropyridin-3-yl | Me | H | F | CN | F | H | I | H | heptafluoroisopropyl | H | OCF3 |
| 2-2987 | phenyl | Me | H | F | CN | F | H | I | H | nonafluoro-2-butyl | H | OCF3 |
| 2-3007 | 3-cyanophenyl | Me | H | F | CN | F | H | I | H | nonafluoro-2-butyl | H | OCF3 |
| 2-3008 | 4-cyanophenyl | Me | H | F | CN | F | H | I | H | nonafluoro-2-butyl | H | OCF3 |
| 2-3023 | 2-chloropyridin-3-yl | Me | H | F | CN | F | H | I | H | nonafluoro-2-butyl | H | OCF3 |
| 2-3065 | phenyl | Me | H | F | CN | F | H | F | H | heptafluoroisopropyl | H | CF3 |
| 2-3066 | phenyl | Me | H | F | CN | F | H | Cl | H | heptafluoroisopropyl | H | CF3 |
| 2-3086 | 3-cyanophenyl | Me | H | F | CN | F | H | Cl | H | heptafluoroisopropyl | H | CF3 |
| 2-3087 | 4-cyanophenyl | Me | H | F | CN | F | H | Cl | H | heptafluoroisopropyl | H | CF3 |
| 2-3102 | 2-chloropyridin-3-yl | Me | H | F | CN | F | H | Cl | H | heptafluoroisopropyl | H | CF3 |
| 2-3144 | phenyl | Me | H | F | CN | F | H | F | H | nonafluoro-2-butyl | H | CF3 |
| 2-3165 | 3-cyanophenyl | Me | H | F | CN | F | H | Cl | H | nonafluoro-2-butyl | H | CF3 |
| 2-3166 | 4-cyanophenyl | Me | H | F | CN | F | H | Cl | H | nonafluoro-2-butyl | H | CF3 |
| 2-3181 | 2-chloropyridin-3-yl | Me | H | F | CN | F | H | Cl | H | nonafluoro-2-butyl | H | CF3 |
| 2-3223 | phenyl | Me | H | F | CN | F | H | Br | H | heptafluoroisopropyl | H | CF3 |
| 2-3243 | 3-cyanophenyl | Me | H | F | CN | F | H | Br | H | heptafluoroisopropyl | H | CF3 |
| 2-3244 | 4-cyanophenyl | Me | H | F | CN | F | H | Br | H | heptafluoroisopropyl | H | CF3 |
| 2-3259 | 2-chloropyridin-3-yl | Me | H | F | CN | F | H | Br | H | heptafluoroisopropyl | H | CF3 |
| 2-3301 | phenyl | Me | H | F | CN | F | H | Br | H | nonafluoro-2-butyl | H | CF3 |

**Table 2(9)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 2-3321 | 3-cyanophenyl | Me | H | F | CN | F | H | Br | H | nonafluoro-2-butyl | H | CF3 |
| 2-3322 | 4-cyanophenyl | Me | H | F | CN | F | H | Br | H | nonafluoro-2-butyl | H | CF3 |
| 2-3337 | 2-chloropyridin-3-yl | Me | H | F | CN | F | H | Br | H | nonafluoro-2-butyl | H | CF3 |
| 2-3379 | phenyl | Me | H | F | CN | F | H | I | H | heptafluoroisopropyl | H | CF3 |
| 2-3399 | 3-cyanophenyl | Me | H | F | CN | F | H | I | H | heptafluoroisopropyl | H | CF3 |
| 2-3400 | 4-cyanophenyl | Me | H | F | CN | F | H | I | H | heptafluoroisopropyl | H | CF3 |
| 2-3415 | 2-chloropyridin-3-yl | Me | H | F | CN | F | H | I | H | heptafluoroisopropyl | H | CF3 |
| 2-3457 | phenyl | Me | H | F | CN | F | H | I | H | nonafluoro-2-butyl | H | CF3 |
| 2-3477 | 3-cyanophenyl | Me | H | F | CN | F | H | I | H | nonafluoro-2-butyl | H | CF3 |
| 2-3478 | 4-cyanophenyl | Me | H | F | CN | F | H | I | H | nonafluoro-2-butyl | H | CF3 |
| 2-3493 | 2-chloropyridin-3-yl | Me | H | F | CN | F | H | I | H | nonafluoro-2-butyl | H | CF3 |
| 2-3494 | 2-bromopyridin-3-yl | Me | H | F | CN | F | H | I | H | nonafluoro-2-butyl | H | CF3 |
| 2-3535 | phenyl | Me | H | F | CN | F | H | OCF3 | H | heptafluoroisopropyl | H | OCF3 |
| 2-3566 | 2-chloropyridin-3-yl | Me | H | F | CN | F | H | OCF3 | H | heptafluoroisopropyl | H | OCF3 |
| 2-3591 | phenyl | Me | H | F | CN | F | H | OCF3 | H | nonafluoro-2-butyl | H | OCF3 |
| 2-3622 | 2-chloropyridin-3-yl | Me | H | F | CN | F | H | OCF3 | H | nonafluoro-2-butyl | H | OCF3 |
| 2-3647 | phenyl | Me | H | F | CN | F | H | CF3 | H | heptafluoroisopropyl | H | OCF3 |
| 2-3678 | 2-chloropyridin-3-yl | Me | H | F | CN | F | H | CF3 | H | heptafluoroisopropyl | H | OCF3 |
| 2-3703 | phenyl | Me | H | F | CN | F | H | CF3 | H | nonafluoro-2-butyl | H | OCF3 |
| 2-3734 | 2-chloropyridin-3-yl | Me | H | F | CN | F | H | CF3 | H | nonafluoro-2-butyl | H | OCF3 |
| 2-3759 | phenyl | Me | H | F | CN | F | H | CF3 | H | heptafluoroisopropyl | H | CF3 |
| 2-3779 | 3-cyanophenyl | Me | H | F | CN | F | H | CF3 | H | heptafluoroisopropyl | H | CF3 |
| 2-3780 | 4-cyanophenyl | Me | H | F | CN | F | H | CF3 | H | heptafluoroisopropyl | H | CF3 |
| 2-3795 | 2-chloropyridin-3-yl | Me | H | F | CN | F | H | CF3 | H | heptafluoroisopropyl | H | CF3 |
| 2-3827 | phenyl | Me | H | F | CN | F | H | CF3 | H | nonafluoro-2-butyl | H | CF3 |
| 2-3847 | 3-cyanophenyl | Me | H | F | CN | F | H | CF3 | H | nonafluoro-2-butyl | H | CF3 |
| 2-3848 | 4-cyanophenyl | Me | H | F | CN | F | H | CF3 | H | nonafluoro-2-butyl | H | CF3 |
| 2-3863 | 2-chloropyridin-3-yl | Me | H | F | CN | F | H | CF3 | H | nonafluoro-2-butyl | H | CF3 |

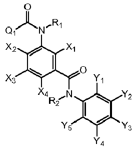

**Table 3(1)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 3-1 | phenyl | H | Me | H | CN | H | H | Cl | H | heptafluoroisopropyl | H | OCF3 |
| 3-21 | 3-cyanophenyl | H | Me | H | CN | H | H | Cl | H | heptafluoroisopropyl | H | OCF3 |
| 3-22 | 4-cyanophenyl | H | Me | H | CN | H | H | Cl | H | heptafluoroisopropyl | H | OCF3 |
| 3-37 | 2-chloropyridin-3-yl | H | Me | H | CN | H | H | Cl | H | heptafluoroisopropyl | H | OCF3 |
| 3-79 | phenyl | H | Me | H | CN | H | H | Cl | H | nonafluoro-2-butyl | H | OCF3 |
| 3-99 | 3-cyanophenyl | H | Me | H | CN | H | H | Cl | H | nonafluoro-2-butyl | H | OCF3 |
| 3-100 | 4-cyanophenyl | H | Me | H | CN | H | H | Cl | H | nonafluoro-2-butyl | H | OCF3 |
| 3-115 | 2-chloropyridin-3-yl | H | Me | H | CN | H | H | Cl | H | nonafluoro-2-butyl | H | OCF3 |
| 3-157 | phenyl | H | Me | H | CN | H | H | Br | H | heptafluoroisopropyl | H | OCF3 |
| 3-177 | 3-cyanophenyl | H | Me | H | CN | H | H | Br | H | heptafluoroisopropyl | H | OCF3 |
| 3-178 | 4-cyanophenyl | H | Me | H | CN | H | H | Br | H | heptafluoroisopropyl | H | OCF3 |
| 3-193 | 2-chloropyridin-3-yl | H | Me | H | CN | H | H | Br | H | heptafluoroisopropyl | H | OCF3 |
| 3-235 | phenyl | H | Me | H | CN | H | H | Br | H | nonafluoro-2-butyl | H | OCF3 |
| 3-255 | 3-cyanophenyl | H | Me | H | CN | H | H | Br | H | nonafluoro-2-butyl | H | OCF3 |
| 3-256 | 4-cyanophenyl | H | Me | H | CN | H | H | Br | H | nonafluoro-2-butyl | H | OCF3 |
| 3-271 | 2-chloropyridin-3-yl | H | Me | H | CN | H | H | Br | H | nonafluoro-2-butyl | H | OCF3 |
| 3-313 | phenyl | H | Me | H | CN | H | H | I | H | heptafluoroisopropyl | H | OCF3 |
| 3-333 | 3-cyanophenyl | H | Me | H | CN | H | H | I | H | heptafluoroisopropyl | H | OCF3 |
| 3-334 | 4-cyanophenyl | H | Me | H | CN | H | H | I | H | heptafluoroisopropyl | H | OC2F5 |
| 3-349 | 2-chloropyridin-3-yl | H | Me | H | CN | H | H | I | H | heptafluoroisopropyl | H | OCF3 |
| 3-391 | phenyl | H | Me | H | CN | H | H | I | H | nonafluoro-2-butyl | H | OCF3 |
| 3-411 | 3-cyanophenyl | H | Me | H | CN | H | H | I | H | nonafluoro-2-butyl | H | OCF3 |
| 3-412 | 4-cyanophenyl | H | Me | H | CN | H | H | I | H | nonafluoro-2-butyl | H | OCF3 |
| 3-427 | 2-chloropyridin-3-yl | H | Me | H | CN | H | H | I | H | nonafluoro-2-butyl | H | OCF3 |
| 3-469 | phenyl | H | Me | H | CN | H | H | F | H | heptafluoroisopropyl | H | CF3 |
| 3-470 | phenyl | H | Me | H | CN | H | H | Cl | H | heptafluoroisopropyl | H | CF3 |
| 3-484 | (3-trifluoromethyl) phenyl | H | Et | H | CN | H | H | Cl | H | pentafluoroethyl | H | CF3 |
| 3-490 | 3-cyanophenyl | H | Me | H | CN | H | H | Cl | H | heptafluoroisopropyl | H | CF3 |
| 3-491 | 4-cyanophenyl | H | Me | H | CN | H | H | Cl | H | heptafluoroisopropyl | H | CF3 |
| 3-495 | 2-chloro-4-fluorophenyl | H | n-Pr | H | CN | H | H | Cl | H | heptafluoroisopropyl | H | C2F5 |
| 3-506 | 2-chloropyridin-3-yl | H | Me | H | CN | H | H | Cl | H | heptafluoroisopropyl | H | CF3 |
| 3-512 | 6-fluoropyridin-3-yl | H | i-Pr | H | CN | H | H | Cl | H | heptafluoroisopropyl | H | CF3 |
| 3-528 | 4-bromopyridin-3-yl | H | CH2CH=CH2 | H | CN | H | H | Cl | H | heptafluoroisopropyl | H | n-C3F7 |
| 3-535 | pyridin-4-yl | H | CN | H | CN | H | H | Cl | H | heptafluoroisopropyl | H | CF3 |
| 3-548 | phenyl | H | Me | H | CN | H | H | F | H | nonafluoro-2-butyl | H | CF3 |
| 3-549 | phenyl | H | Me | H | CN | H | H | Cl | H | nonafluoro-2-butyl | H | CF3 |
| 3-556 | 2-bromophenyl | H | CH2C≡CH | H | CN | H | H | Cl | H | nonafluoro-2-butyl | H | CF3 |
| 3-563 | (3-trifluoromethyl) phenyl | H | NH2 | H | CN | H | H | Cl | H | nonafluoro-2-butyl | H | CF3 |
| 3-569 | 3-cyanophenyl | H | Me | H | CN | H | H | Cl | H | nonafluoro-2-butyl | H | CF3 |
| 3-570 | 4-cyanophenyl | H | Me | H | CN | H | H | Cl | H | nonafluoro-2-butyl | H | CF3 |
| 3-576 | 4-bromo-2-chlorophenyl | H | C(O)OMe | H | CN | H | H | Cl | H | nonafluoro-2-butyl | H | CF3 |
| 3-585 | 2-chloropyridin-3-yl | H | Me | H | CN | H | H | Cl | H | nonafluoro-2-butyl | H | CF3 |
| 3-596 | 6-nitropyridin-3-yl | H | C(O)OEt | H | CN | H | H | Cl | H | nonafluoro-2-butyl | H | CF3 |
| 3-607 | 4-bromopyridin-3-yl | H | C(O)C(O)Me | H | CN | H | H | Cl | H | nonafluoro-2-butyl | H | CF3 |
| 3-610 | 4-nitropyridin-3-yl | H | C(O)C(O)Et | H | CN | H | H | Cl | H | nonafluoro-2-butyl | H | i-C3F7 |
| 3-623 | 4-bromopyridin-2-yl | H | C(O)Me | H | CN | H | H | Cl | H | nonafluoro-2-butyl | H | CF3 |
| 3-625 | 6-chloropyridin-2-yl | H | C(O)Et | H | CN | H | H | Cl | H | nonafluoro-2-butyl | H | CF3 |
| 3-627 | phenyl | H | Me | H | CN | H | H | Br | H | heptafluoroisopropyl | H | CF3 |
| 3-641 | (3-trifluoromethyl) phenyl | H | S(O)2Me | H | CN | H | H | Br | H | heptafluoroisopropyl | H | CF3 |
| 3-647 | 3-cyanophenyl | H | Me | H | CN | H | H | Br | H | heptafluoroisopropyl | H | CF3 |
| 3-648 | 4-cyanophenyl | H | Me | H | CN | H | H | Br | H | heptafluoroisopropyl | H | CF3 |
| 3-659 | 4-cyano-2-fluorophenyl | H | S(O)2Et | H | CN | H | H | Br | H | heptafluoroisopropyl | H | CF3 |
| 3-663 | 2-chloropyridin-3-yl | H | Me | H | CN | H | H | Br | H | heptafluoroisopropyl | H | CF3 |
| 3-674 | 6-nitropyridin-3-yl | H | CH2Ph | H | CN | H | H | Br | H | heptafluoroisopropyl | H | CF3 |
| 3-688 | 4-nitropyridin-3-yl | H | CH2(3-Py) | H | CN | H | H | Br | H | heptafluoroisopropyl | H | CF3 |

**Table 3(2)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 3-702 | 5-iodopyridin-2-yl | H | CH2(3-Py-N-oxide) | H | CN | H | H | Br | H | heptafluoroisopropyl | H | CF3 |
| 3-705 | phenyl | H | Me | H | CN | H | H | Br | H | nonafluoro-2-butyl | H | CF3 |
| 3-725 | 3-cyanophenyl | H | Me | H | CN | H | H | Br | H | nonafluoro-2-butyl | H | CF3 |
| 3-726 | 4-cyanophenyl | H | Me | H | CN | H | H | Br | H | nonafluoro-2-butyl | H | CF3 |
| 3-732 | 4-bromo-2-chlorophenyl | H | Et | H | CN | H | H | Br | H | nonafluoro-2-butyl | H | CF3 |
| 3-741 | 2-chloropyridin-3-yl | H | Me | H | CN | H | H | Br | H | nonafluoro-2-butyl | H | CF3 |
| 3-758 | 5- (trifluoromethyl) pyridin-3-yl | H | CN | H | CN | H | H | Br | H | nonafluoro-2-butyl | H | CF3 |
| 3-783 | phenyl | H | Me | H | CN | H | H | I | H | heptafluoroisopropyl | H | CF3 |
| 3-803 | 3-cyanophenyl | H | Me | H | CN | H | H | I | H | heptafluoroisopropyl | H | CF3 |
| 3-804 | 4-cyanophenyl | H | Me | H | CN | H | H | I | H | heptafluoroisopropyl | H | CF3 |
| 3-819 | 2-chloropyridin-3-yl | H | Me | H | CN | H | H | I | H | heptafluoroisopropyl | H | CF3 |
| 3-841 | 4-bromopyridin-3-yl | H | C(O)Me | H | CN | H | H | I | H | heptafluoroisopropyl | H | CF3 |
| 3-861 | phenyl | H | Me | H | CN | H | H | I | H | nonafluoro-2-butyl | H | CF3 |
| 3-881 | 3-cyanophenyl | H | Me | H | CN | H | H | I | H | nonafluoro-2-butyl | H | CF3 |
| 3-882 | 4-cyanophenyl | H | Me | H | CN | H | H | I | H | nonafluoro-2-butyl | H | CF3 |
| 3-897 | 2-chloropyridin-3-yl | H | Me | H | CN | H | H | I | H | nonafluoro-2-butyl | H | CF3 |
| 3-939 | phenyl | H | Me | H | CN | H | H | OCF3 | H | heptafluoroisopropyl | H | OCF3 |
| 3-970 | 2-chloropyridin-3-yl | H | Me | H | CN | H | H | OCF3 | H | heptafluoroisopropyl | H | OCF3 |
| 3-995 | phenyl | H | Me | H | CN | H | H | OCF3 | H | nonafluoro-2-butyl | H | OCF3 |
| 3-1026 | 2-chloropyridin-3-yl | H | Me | H | CN | H | H | OCF3 | H | nonafluoro-2-butyl | H | OCF3 |
| 3-1051 | phenyl | H | Me | H | CN | H | H | CF3 | H | heptafluoroisopropyl | H | OCF3 |
| 3-1082 | 2-chloropyridin-3-yl | H | Me | H | CN | H | H | CF3 | H | heptafluoroisopropyl | H | OCF3 |
| 3-1107 | phenyl | H | Me | H | CN | H | H | CF3 | H | nonafluoro-2-butyl | H | OCF3 |
| 3-1138 | 2-chloropyridin-3-yl | H | Me | H | CN | H | H | CF3 | H | nonafluoro-2-butyl | H | OCF3 |
| 3-1163 | phenyl | H | Me | H | CN | H | H | CF3 | H | heptafluoroisopropyl | H | CF3 |
| 3-1183 | 3-cyanophenyl | H | Me | H | CN | H | H | CF3 | H | heptafluoroisopropyl | H | CF3 |
| 3-1184 | 4-cyanophenyl | H | Me | H | CN | H | H | CF3 | H | heptafluoroisopropyl | H | CF3 |
| 3-1199 | 2-chloropyridin-3-yl | H | Me | H | CN | H | H | CF3 | H | heptafluoroisopropyl | H | CF3 |
| 3-1231 | phenyl | H | Me | H | CN | H | H | CF3 | H | nonafluoro-2-butyl | H | CF3 |
| 3-1251 | 3-cyanophenyl | H | Me | H | CN | H | H | CF3 | H | nonafluoro-2-butyl | H | C2F5 |
| 3-1252 | 4-cyanophenyl | H | Me | H | CN | H | H | CF3 | H | nonafluoro-2-butyl | H | CF3 |
| 3-1267 | 2-chloropyridin-3-yl | H | Me | H | CN | H | H | CF3 | H | nonafluoro-2-butyl | H | CF3 |
| 3-1299 | phenyl | H | Me | F | CN | H | H | Cl | H | heptafluoroisopropyl | H | OCF3 |
| 3-1319 | 3-cyanophenyl | H | Me | F | CN | H | H | Cl | H | heptafluoroisopropyl | H | OCF3 |
| 3-1320 | 4-cyanophenyl | H | Me | F | CN | H | H | Cl | H | heptafluoroisopropyl | H | OCF3 |
| 3-1335 | 2-chloropyridin-3-yl | H | Me | F | CN | H | H | Cl | H | heptafluoroisopropyl | H | OCF3 |
| 3-1377 | phenyl | H | Me | F | CN | H | H | Cl | H | nonafluoro-2-butyl | H | OCF3 |
| 3-1397 | 3-cyanophenyl | H | Me | F | CN | H | H | Cl | H | nonafluoro-2-butyl | H | OCF3 |
| 3-1398 | 4-cyanophenyl | H | Me | F | CN | H | H | Cl | H | nonafluoro-2-butyl | H | OCF3 |
| 3-1413 | 2-chloropyridin-3-yl | H | Me | F | CN | H | H | Cl | H | nonafluoro-2-butyl | H | OCF3 |
| 3-1455 | phenyl | H | Me | F | CN | H | H | Br | H | heptafluoroisopropyl | H | OCF3 |
| 3-1491 | 2-chloropyridin-3-yl | H | Me | F | CN | H | H | Br | H | heptafluoroisopropyl | H | OCF3 |
| 3-1533 | phenyl | H | Me | F | CN | H | H | Br | H | nonafluoro-2-butyl | H | OCF3 |
| 3-1553 | 3-cyanophenyl | H | Me | F | CN | H | H | Br | H | nonafluoro-2-butyl | H | OCF3 |
| 3-1554 | 4-cyanophenyl | H | Me | F | CN | H | H | Br | H | nonafluoro-2-butyl | H | OCF3 |
| 3-1569 | 2-chloropyridin-3-yl | H | Me | F | CN | H | H | Br | H | nonafluoro-2-butyl | H | OCF3 |
| 3-1611 | phenyl | H | Me | F | CN | H | H | I | H | heptafluoroisopropyl | H | OCF3 |
| 3-1631 | 3-cyanophenyl | H | Me | F | CN | H | H | I | H | heptafluoroisopropyl | H | OCF3 |
| 3-1632 | 4-cyanophenyl | H | Me | F | CN | H | H | I | H | heptafluoroisopropyl | H | OCF3 |
| 3-1647 | 2-chloropyridin-3-yl | H | Me | F | CN | H | H | I | H | heptafluoroisopropyl | H | OCF3 |
| 3-1689 | phenyl | H | Me | F | CN | H | H | I | H | nonafluoro-2-butyl | H | OCF3 |
| 3-1709 | 3-cyanophenyl | H | Me | F | CN | H | H | I | H | nonafluoro-2-butyl | H | OCF3 |
| 3-1710 | 4-cyanophenyl | H | Me | F | CN | H | H | I | H | nonafluoro-2-butyl | H | OCF3 |
| 3-1725 | 2-chloropyridin-3-yl | H | Me | F | CN | H | H | I | H | nonafluoro-2-butyl | H | OCF3 |
| 3-1767 | phenyl | H | Me | F | CN | H | H | F | H | heptafluoroisopropyl | H | CF3 |
| 3-1768 | phenyl | H | Me | F | CN | H | H | Cl | H | heptafluoroisopropyl | H | CF3 |
| 3-1788 | 3-cyanophenyl | H | Me | F | CN | H | H | Cl | H | heptafluoroisopropyl | H | CF3 |
| 3-1789 | 4-cyanophenyl | H | Me | F | CN | H | H | Cl | H | heptafluoroisopropyl | H | CF3 |
| 3-1804 | 2-chloropyridin-3-yl | H | Me | F | CN | H | H | Cl | H | heptafluoroisopropyl | H | CF3 |
| 3-1846 | phenyl | H | Me | F | CN | H | H | F | H | nonafluoro-2-butyl | H | CF3 |
| 3-1847 | phenyl | H | Me | F | CN | H | H | Cl | H | nonafluoro-2-butyl | H | CF3 |
| 3-1867 | 3-cyanophenyl | H | Me | F | CN | H | H | Cl | H | nonafluoro-2-butyl | H | CF3 |
| 3-1868 | 4-cyanophenyl | H | Me | F | CN | H | H | Cl | H | nonafluoro-2-butyl | H | CF3 |
| 3-1883 | 2-chloropyridin-3-yl | H | Me | F | CN | H | H | Cl | H | nonafluoro-2-butyl | H | CF3 |

**Table 3(3)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 3-1925 | phenyl | H | Me | F | CN | H | H | Br | H | heptafluoroisopropyl | H | CF3 |
| 3-1945 | 3-cyanophenyl | H | Me | F | CN | H | H | Br | H | heptafluoroisopropyl | H | CF3 |
| 3-1946 | 4-cyanophenyl | H | Me | F | CN | H | H | Br | H | heptafluoroisopropyl | H | CF3 |
| 3-1961 | 2-chloropyridin-3-yl | H | Me | F | CN | H | H | Br | H | heptafluoroisopropyl | H | CF3 |
| 3-2003 | phenyl | H | Me | F | CN | H | H | Br | H | nonafluoro-2-butyl | H | CF3 |
| 3-2023 | 3-cyanophenyl | H | Me | F | CN | H | H | Br | H | nonafluoro-2-butyl | H | CF3 |
| 3-2024 | 4-cyanophenyl | H | Me | F | CN | H | H | Br | H | nonafluoro-2-butyl | H | CF3 |
| 3-2039 | 2-chloropyridin-3-yl | H | Me | F | CN | H | H | Br | H | nonafluoro-2-butyl | H | CF3 |
| 3-2081 | phenyl | H | Me | F | CN | H | H | I | H | heptafluoroisopropyl | H | CF3 |
| 3-2101 | 3-cyanophenyl | H | Me | F | CN | H | H | I | H | heptafluoroisopropyl | H | CF3 |
| 3-2102 | 4-cyanophenyl | H | Me | F | CN | H | H | I | H | heptafluoroisopropyl | H | CF3 |
| 3-2117 | 2-chloropyridin-3-yl | H | Me | F | CN | H | H | I | H | heptafluoroisopropyl | H | CF3 |
| 3-2159 | phenyl | H | Me | F | CN | H | H | I | H | nonafluoro-2-butyl | H | CF3 |
| 3-2179 | 3-cyanophenyl | H | Me | F | CN | H | H | I | H | nonafluoro-2-butyl | H | CF3 |
| 3-2180 | 4-cyanophenyl | H | Me | F | CN | H | H | I | H | nonafluoro-2-butyl | H | CF3 |
| 3-2195 | 2-chloropyridin-3-yl | H | Me | F | CN | H | H | I | H | nonafluoro-2-butyl | H | CF3 |
| 3-2237 | phenyl | H | Me | F | CN | H | H | OCF3 | H | heptafluoroisopropyl | H | OCF3 |
| 3-2268 | 2-chloropyridin-3-yl | H | Me | F | CN | H | H | OCF3 | H | heptafluoroisopropyl | H | OCF3 |
| 3-2293 | phenyl | H | Me | F | CN | H | H | OCF3 | H | nonafluoro-2-butyl | H | OCF3 |
| 3-2324 | 2-chloropyridin-3-yl | H | Me | F | CN | H | H | OCF3 | H | nonafluoro-2-butyl | H | OCF3 |
| 3-2349 | phenyl | H | Me | F | CN | H | H | CF3 | H | heptafluoroisopropyl | H | OCF3 |
| 3-2380 | 2-chloropyridin-3-yl | H | Me | F | CN | H | H | CF3 | H | heptafluoroisopropyl | H | OCF3 |
| 3-2405 | phenyl | H | Me | F | CN | H | H | CF3 | H | nonafluoro-2-butyl | H | OCF3 |
| 3-2436 | 2-chloropyridin-3-yl | H | Me | F | CN | H | H | CF3 | H | nonafluoro-2-butyl | H | OCF3 |
| 3-2461 | phenyl | H | Me | F | CN | H | H | CF3 | H | heptafluoroisopropyl | H | CF3 |
| 3-2481 | 3-cyanophenyl | H | Me | F | CN | H | H | CF3 | H | heptafluoroisopropyl | H | CF3 |
| 3-2482 | 4-cyanophenyl | H | Me | F | CN | H | H | CF3 | H | heptafluoroisopropyl | H | CF3 |
| 3-2497 | 2-chloropyridin-3-yl | H | Me | F | CN | H | H | CF3 | H | heptafluoroisopropyl | H | CF3 |
| 3-2529 | phenyl | H | Me | F | CN | H | H | CF3 | H | nonafluoro-2-butyl | H | CF3 |
| 3-2549 | 3-cyanophenyl | H | Me | F | CN | H | H | CF3 | H | nonafluoro-2-butyl | H | CF3 |
| 3-2550 | 4-cyanophenyl | H | Me | F | CN | H | H | CF3 | H | nonafluoro-2-butyl | H | CF3 |
| 3-2565 | 2-chloropyridin-3-yl | H | Me | F | CN | H | H | CF3 | H | nonafluoro-2-butyl | H | CF3 |
| 3-2597 | phenyl | H | Me | F | CN | F | H | Cl | H | heptafluoroisopropyl | H | OCF3 |
| 3-2617 | 3-cyanophenyl | H | Me | F | CN | F | H | Cl | H | heptafluoroisopropyl | H | OCF3 |
| 3-2618 | 4-cyanophenyl | H | Me | F | CN | F | H | Cl | H | heptafluoroisopropyl | H | OCF3 |
| 3-2633 | 2-chloropyridin-3-yl | H | Me | F | CN | F | H | Cl | H | heptafluoroisopropyl | H | OCF3 |
| 3-2675 | phenyl | H | Me | F | CN | F | H | Cl | H | nonafluoro-2-butyl | H | OCF3 |
| 3-2695 | 3-cyanophenyl | H | Me | F | CN | F | H | Cl | H | nonafluoro-2-butyl | H | OCF3 |
| 3-2696 | 4-cyanophenyl | H | Me | F | CN | F | H | Cl | H | nonafluoro-2-butyl | H | OCF3 |
| 3-2711 | 2-chloropyridin-3-yl | H | Me | F | CN | F | H | Cl | H | nonafluoro-2-butyl | H | OCF3 |
| 3-2753 | phenyl | H | Me | F | CN | F | H | Br | H | heptafluoroisopropyl | H | OCF3 |
| 3-2773 | 3-cyanophenyl | H | Me | F | CN | F | H | Br | H | heptafluoroisopropyl | H | OCF3 |
| 3-2774 | 4-cyanophenyl | H | Me | F | CN | F | H | Br | H | heptafluoroisopropyl | H | OCF3 |
| 3-2789 | 2-chloropyridin-3-yl | H | Me | F | CN | F | H | Br | H | heptafluoroisopropyl | H | OCF3 |
| 3-2831 | phenyl | H | Me | F | CN | F | H | Br | H | nonafluoro-2-butyl | H | OCF3 |
| 3-2851 | 3-cyanophenyl | H | Me | F | CN | F | H | Br | H | nonafluoro-2-butyl | H | OCF3 |
| 3-2852 | 4-cyanophenyl | H | Me | F | CN | F | H | Br | H | nonafluoro-2-butyl | H | OCF3 |
| 3-2867 | 2-chloropyridin-3-yl | H | Me | F | CN | F | H | Br | H | nonafluoro-2-butyl | H | OCF3 |
| 3-2909 | phenyl | H | Me | F | CN | F | H | I | H | heptafluoroisopropyl | H | OCF3 |
| 3-2929 | 3-cyanophenyl | H | Me | F | CN | F | H | I | H | heptafluoroisopropyl | H | OCF3 |
| 3-2930 | 4-cyanophenyl | H | Me | F | CN | F | H | I | H | heptafluoroisopropyl | H | OCF3 |
| 3-2945 | 2-chloropyridin-3-yl | H | Me | F | CN | F | H | I | H | heptafluoroisopropyl | H | OCF3 |
| 3-2987 | phenyl | H | Me | F | CN | F | H | I | H | nonafluoro-2-butyl | H | OCF3 |
| 3-2988 | 2-fluorophenyl | H | Me | F | CN | F | H | I | H | nonafluoro-2-butyl | H | OCF3 |
| 3-3007 | 3-cyanophenyl | H | Me | F | CN | F | H | I | H | nonafluoro-2-butyl | H | OCF3 |
| 3-3008 | 4-cyanophenyl | H | Me | F | CN | F | H | I | H | nonafluoro-2-butyl | H | OCF3 |
| 3-3023 | 2-chloropyridin-3-yl | H | Me | F | CN | F | H | I | H | nonafluoro-2-butyl | H | OCF3 |
| 3-3065 | phenyl | H | Me | F | CN | F | H | F | H | heptafluoroisopropyl | H | CF3 |
| 3-3066 | phenyl | H | Me | F | CN | F | H | Cl | H | heptafluoroisopropyl | H | CF3 |
| 3-3086 | 3-cyanophenyl | H | Me | F | CN | F | H | Cl | H | heptafluoroisopropyl | H | CF3 |
| 3-3087 | 4-cyanophenyl | H | Me | F | CN | F | H | Cl | H | heptafluoroisopropyl | H | CF3 |

**Table 3(4)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 3-3102 | 2-chloropyridin-3-yl | H | Me | F | CN | F | H | Cl | H | heptafluoroisopropyl | H | CF3 |
| 3-3144 | phenyl | H | Me | F | CN | F | H | F | H | nonafluoro-2-butyl | H | CF3 |
| 3-3145 | phenyl | H | Me | F | CN | F | H | Cl | H | nonafluoro-2-butvl | H | CF3 |
| 3-3165 | 3-cyanophenyl | H | Me | F | CN | F | H | Cl | H | nonafluoro-2-butyl | H | CF3 |
| 3-3166 | 4-cyanophenyl | H | Me | F | CN | F | H | Cl | H | nonafluoro-2-butyl | H | CF3 |
| 3-3181 | 2-chloropyridin-3-yl | H | Me | F | CN | F | H | Cl | H | nonafluoro-2-butyl | H | CF3 |
| 3-3223 | phenyl | H | Me | F | CN | F | H | Br | H | heptafluoroisopropyl | H | CF3 |
| 3-3243 | 3-cyanophenyl | H | Me | F | CN | F | H | Br | H | heptafluoroisopropyl | H | CF3 |
| 3-3244 | 4-cyanophenyl | H | Me | F | CN | F | H | Br | H | heptafluoroisopropyl | H | CF3 |
| 3-3259 | 2-chloropyridin-3-yl | H | Me | F | CN | F | H | Br | H | heptafluoroisopropyl | H | CF3 |
| 3-3301 | phenyl | H | Me | F | CN | F | H | Br | H | nonafluoro-2-butyl | H | CF3 |
| 3-3321 | 3-cyanophenyl | H | Me | F | CN | F | H | Br | H | nonafluoro-2-butyl | H | CF3 |
| 3-3322 | 4-cyanophenyl | H | Me | F | CN | F | H | Br | H | nonafluoro-2-butyl | H | CF3 |
| 3-3337 | 2-chloropyridin-3-yl | H | Me | F | CN | F | H | Br | H | nonafluoro-2-butyl | H | CF3 |
| 3-3379 | phenyl | H | Me | F | CN | F | H | I | H | heptafluoroisopropyl | H | CF3 |
| 3-3399 | 3-cyanophenyl | H | Me | F | CN | F | H | I | H | heptafluoroisopropyl | H | CF3 |
| 3-3400 | 4-cyanophenyl | H | Me | F | CN | F | H | I | H | heptafluoroisopropyl | H | CF3 |
| 3-3415 | 2-chloropyridin-3-yl | H | Me | F | CN | F | H | I | H | heptafluoroisopropyl | H | CF3 |
| 3-3457 | phenyl | H | Me | F | CN | F | H | I | H | nonafluoro-2-butyl | H | CF3 |
| 3-3477 | 3-cyanophenyl | H | Me | F | CN | F | H | I | H | nonafluoro-2-butyl | H | CF3 |
| 3-3478 | 4-cyanophenyl | H | Me | F | CN | F | H | I | H | nonafluoro-2-butyl | H | CF3 |
| 3-3493 | 2-chloropyridin-3-yl | H | Me | F | CN | F | H | I | H | nonafluoro-2-butyl | H | CF3 |
| 3-3535 | phenyl | H | Me | F | CN | F | H | OCF3 | H | heptafluoroisopropyl | H | OCF3 |
| 3-3566 | 2-chloropyridin-3-yl | H | Me | F | CN | F | H | OCF3 | H | heptafluoroisopropyl | H | OCF3 |
| 3-3591 | phenyl | H | Me | F | CN | F | H | OCF3 | H | nonafluoro-2-butyl | H | OCF3 |
| 3-3622 | 2-chloropyridin-3-yl | H | Me | F | CN | F | H | OCF3 | H | nonafluoro-2-butyl | H | OCF3 |
| 3-3647 | phenyl | H | Me | F | CN | F | H | CF3 | H | heptafluoroisopropyl | H | OCF3 |
| 3-3678 | 2-chloropyridin-3-yl | H | Me | F | CN | F | H | CF3 | H | heptafluoroisopropyl | H | OCF3 |
| 3-3703 | phenyl | H | Me | F | CN | F | H | CF3 | H | nonafluoro-2-butyl | H | OCF3 |
| 3-3734 | 2-chloropyridin-3-yl | H | Me | F | CN | F | H | CF3 | H | nonafluoro-2-butyl | H | OCF3 |
| 3-3759 | phenyl | H | Me | F | CN | F | H | CF3 | H | heptafluoroisopropyl | H | CF3 |
| 3-3779 | 3-cyanophenyl | H | Me | F | CN | F | H | CF3 | H | heptafluoroisopropyl | H | CF3 |
| 3-3780 | 4-cyanophenyl | H | Me | F | CN | F | H | CF3 | H | heptafluoroisopropyl | H | CF3 |
| 3-3795 | 2-chloropyridin-3-yl | H | Me | F | CN | F | H | CF3 | H | heptafluoroisopropyl | H | CF3 |

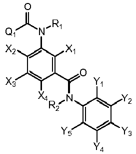

**Table 4(1)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 4-1 | phenyl | Me | Me | H | CN | H | H | Cl | H | heptafluoroisopropyl | H | OCF3 |
| 4-21 | 3-cyanophenyl | Me | Me | H | CN | H | H | Cl | H | heptafluoroisopropyl | H | OCF3 |
| 4-22 | 4-cyanophenyl | Me | Me | H | CN | H | H | Cl | H | heptafluoroisopropyl | H | OCF3 |
| 4-37 | 2-chloropyridin-3-yl | Me | Me | H | CN | H | H | Cl | H | heptafluoroisopropyl | H | OCF3 |
| 4-79 | phenyl | Me | Me | H | CN | H | H | Cl | H | nonafluoro-2-butyl | H | OCF3 |
| 4-99 | 3-cyanophenyl | Me | Me | H | CN | H | H | Cl | H | nonafluoro-2-butyl | H | OCF3 |
| 4-100 | 4-cyanophenyl | Me | Me | H | CN | H | H | Cl | H | nonafluoro-2-butyl | H | OCF3 |
| 4-115 | 2-chloropyridin-3-yl | Me | Me | H | CN | H | H | Cl | H | nonafluoro-2-butyl | H | OCF3 |
| 4-157 | phenyl | Me | Me | H | CN | H | H | Br | H | heptafluoroisopropyl | H | OCF3 |
| 4-177 | 3-cyanophenyl | Me | Me | H | CN | H | H | Br | H | heptafluoroisopropyl | H | OCF3 |
| 4-178 | 4-cyanophenyl | Me | Me | H | CN | H | H | Br | H | heptafluoroisopropyl | H | OCF3 |
| 4-193 | 2-chloropyridin-3-yl | Me | Me | H | CN | H | H | Br | H | heptafluoroisopropyl | H | OCF3 |
| 4-235 | phenyl | Me | Me | H | CN | H | H | Br | H | nonafluoro-2-butyl | H | OCF3 |
| 4-255 | 3-cyanophenyl | Me | Me | H | CN | H | H | Br | H | nonafluoro-2-butyl | H | OCF3 |
| 4-256 | 4-cyanophenyl | Me | Me | H | CN | H | H | Br | H | nonafluoro-2-butyl | H | OCF3 |
| 4-271 | 2-chloropyridin-3-yl | Me | Me | H | CN | H | H | Br | H | nonafluoro-2-butyl | H | OCF3 |
| 4-313 | phenyl | Me | Me | H | CN | H | H | I | H | heptafluoroisopropyl | H | OCF3 |
| 4-314 | 2-fluorophenyl | Me | Me | H | CN | H | H | I | H | heptafluoroisopropyl | H | OCF3 |
| 4-333 | 3-cyanophenyl | Me | Me | H | CN | H | H | I | H | heptafluoroisopropyl | H | OCF3 |
| 4-334 | 4-cyanophenyl | Me | Me | H | CN | H | H | I | H | heptafluoroisopropyl | H | OCF3 |
| 4-349 | 2-chloropyridin-3-yl | Me | Me | H | CN | H | H | I | H | heptafluoroisopropyl | H | OCF3 |
| 4-391 | phenyl | Me | Me | H | CN | H | H | I | H | nonafluoro-2-butyl | H | OCF3 |
| 4-392 | 2-fluorophenyl | Me | Me | H | CN | H | H | I | H | nonafluoro-2-butyl | H | OCF3 |
| 4-411 | 3-cyanophenyl | Me | Me | H | CN | H | H | I | H | nonafluoro-2-butvl | H | OCF3 |
| 4-412 | 4-cyanophenyl | Me | Me | H | CN | H | H | I | H | nonafluoro-2-butyl | H | OCF3 |
| 4-427 | 2-chloropyridin-3-yl | Me | Me | H | CN | H | H | I | H | nonafluoro-2-butyl | H | OCF3 |
| 4-665 | phenyl | Me | Me | H | CN | H | H | Cl | H | heptafluoroisopropyl | H | CF3 |
| 4-666 | (3-trifluoromethyl) phenyl | Et | Me | H | CN | H | H | Cl | H | heptafluoroisopropyl | H | CF3 |
| 4-667 | (4-trifluoromethyl) phenyl | Me | Et | H | CN | H | H | Cl | H | heptafluoroisopropyl | H | CF3 |
| 4-671 | 2-cyanophenyl | Me | Me | H | CN | H | H | Cl | H | pentafluoroethyl | H | C2F5 |
| 4-672 | 3-cyanophenyl | Me | Me | H | CN | H | H | Cl | H | heptafluoroisopropyl | H | CF3 |
| 4-673 | 4-cyanophenyl | Me | Me | H | CN | H | H | Cl | H | heptafluoroisopropyl | H | CF3 |
| 4-676 | 2,4-dichlorophenyl | Me | Me | H | CN | H | H | Cl | H | heptafluoroisopropyl | H | C2F5 |
| 4-677 | 2-chloro-4-fluorophenyl | n-Pr | Me | H | CN | H | H | Cl | H | heptafluoroisopropyl | H | CF3 |
| 4-680 | 2-bromo-4-chlorophenyl | Me | Me | H | CN | H | H | Cl | H | heptafluoroisopropyl | H | n-C3F7 |
| 4-681 | 2-bromo-4-fluorophenyl | Me | n-Pr | H | CN | H | H | Cl | H | heptafluoroisopropyl | H | CF3 |
| 4-687 | 2-fluoropyridin-3-yl | Me | Me | H | CN | H | H | Cl | H | pentafluoroethyl | H | CF3 |
| 4-688 | 2-chloropyridin-3-yl | Me | Me | H | CN | H | H | Cl | H | heptafluoroisopropyl | H | CF3 |
| 4-692 | 2-nitropyridin-3-yl | Me | i-Pr | H | CN | H | H | Cl | H | heptafluoroisopropyl | H | CF3 |
| 4-694 | 6-fluoropyridin-3-yl | i-Pr | Me | H | CN | H | H | Cl | H | heptafluoroisopropyl | H | CF3 |
| 4-701 | 5-fluoropyridin-3-yl | Me | Me | H | CN | H | H | Cl | H | heptafluoroisopropyl | H | C2F5 |
| 4-703 | 5-bromopyridin-3-yl | Me | Me | H | CN | H | H | Cl | H | heptafluoroisopropyl | H | n-C3F7 |
| 4-704 | 5-iodopyridin-3-yl | Me | Me | H | CN | H | H | Cl | H | heptafluoroisopropyl | H | i-C3F7 |
| 4-706 | 5-nitropyridin-3-yl | Me | CH2CH=CH2 | H | CN | H | H | Cl | H | pentafluoroethyl | H | CF3 |
| 4-708 | 4-fluoropyridin-3-yl | Me | Me | H | CN | H | H | Cl | H | pentafluoroethyl | H | CF3 |
| 4-710 | 4-bromopyridin-3-yl | CH2CH=CH2 | Me | H | CN | H | H | Cl | H | heptafluoroisopropyl | H | n-C3F7 |
| 4-711 | 4-iodopyridin-3-yl | Me | Me | H | CN | H | H | Cl | H | heptafluoroisopropyl | H | i-C3F7 |
| 4-717 | pyridin-4-yl | CN | Me | H | CN | H | H | Cl | H | heptafluoroisopropyl | H | CF3 |
| 4-724 | pyridin-2-yl | Me | CN | H | CN | H | H | Cl | H | heptafluoroisopropyl | H | CF3 |
| 4-729 | 4-cyanopyridin-2-yl | Me | Me | H | CN | H | H | Cl | H | pentafluoroethyl | H | CF3 |
| 4-730 | phenyl | Me | Me | H | CN | H | H | F | H | nonafluoro-2-butyl | H | CF3 |
| 4-731 | phenyl | Me | Me | H | CN | H | H | Cl | H | nonafluoro-2-butyl | H | CF3 |
| 4-732 | 2-fluorophenyl | Me | Me | H | CN | H | H | Cl | H | nonafluoro-2-butyl | H | CF3 |
| 4-739 | 3-bromophenyl | CH2C≡CH | Me | H | CN | H | H | Cl | H | nonafluoro-2-butyl | H | CF3 |
| 4-744 | (2-trifluoromethyl) phenyl | Me | Me | H | CN | H | H | Cl | H | nonafluoro-2-butyl | H | C2F5 |

**Table 4(2)**

| compound | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 4-745 | (3-trifluoromethyl) phenyl | Me | CH2C≡CH | H | CN | H | H | Cl | H | nonafluoro-2-butyl | H | CF3 |
| 4-746 | (4-trifluoromethyl) phenyl | NH2 | Me | H | CN | H | H | Cl | H | nonafluoro-2-butyl | H | CF3 |
| 4-750 | 2-cyanophenyl | Me | NH2 | H | CN | H | H | Cl | H | nonafluoro-2-butyl | H | CF3 |
| 4-751 | 3-cyanophenyl | Me | Me | H | CN | H | H | Cl | H | nonafluoro-2-butyl | H | CF3 |
| 4-752 | 4-cyanophenyl | Me | Me | H | CN | H | H | Cl | H | nonafluoro-2-butyl | H | CF3 |
| 4-759 | 2-bromo-4-chlorophenyl | e(O)OMe | Me | H | CN | H | H | Cl | H | nonafluoro-2-butyl | H | CF3 |
| 4-761 | 2-chloro-4-nitrophenyl | Me | C(O)OMe | H | CN | H | H | Cl | H | nonafluoro-2-butyl | H | i-C3F7 |
| 4-767 | 2-chloropyridin-3-yl | Me | Me | H | CN | H | H | Cl | H | nonafluoro-2-butyl | H | CF3 |
| 4-773 | 6-fluoropyridin-3-yl | Me | C(O)OEt | H | CN | H | H | Cl | H | nonafluoro-2-butyl | H | CF3 |
| 4-780 | 5-fluoropyridin-3-yl | C(O)OEt | Me | H | CN | H | H | Cl | H | nonafluoro-2-butyl | H | C2F5 |
| 4-781 | 5-chloropyridin-3-yl | Me | Me | H | CN | H | H | Cl | H | nonafluoro-2-butyl | H | CF3 |
| 4-782 | 5-bromopyridin-3-yl | Me | Me | H | CN | H | H | Cl | H | nonafluoro-2-butyl | H | n-C3F7 |
| 4-783 | 5 iodopyridin-3-yl | Me | Me | H | CN | H | H | Cl | H | nonafluoro-2-butyl | H | i-C3F7 |
| 4-785 | 5-nitropyridin-3-yl | Me | C(O)C(O)Me | H | CN | H | H | Cl | H | nonafluoro-2-butyl | H | CF3 |
| 4-787 | 4-fluoropyridin-3-y! | Me | Me | H | CN | H | H | Cl | H | nonafluoro-2-butyl | H | C2F5 |
| 4-789 | 4-bromopyridin-3-yl | C(O)OMe | Me | H | CN | H | H | Cl | H | nonafluoro-2-butyl | H | CF3 |
| 4-790 | 4-iodopyridin-3-yl | Me | C(O)C(O)Et | H | CN | H | H | Cl | H | nonafluoro-2-butyl | H | CF3 |
| 4-791 | 4-(trifluoromethyl) pyridin-3-yl | Me | Me | H | CN | H | H | Cl | H | nonafluoro-2-butyl | H | n-C3F7 |
| 4-792 | 4-nitropyridin-3-yl | C(O)C(O)Et | Me | H | CN | H | H | Cl | H | nonafluoro-2-butyl | H | i-C3F7 |
| 4-803 | pyridin-2-yl | Me | C(O)Me | H | CN | H | H | Cl | H | nonafluoro-2-butyl | H | CF3 |
| 4-805 | 4-bromopyridin-2-yl | Me | C(O)Et | H | CN | H | H | Cl | H | nonafluoro-2-butyl | H | CF3 |
| 4-806 | 5 iodopyridin-2-yl | C(O)Me | Me | H | CN | H | H | Cl | H | nonafluoro-2-butyl | H | CF3 |
| 4-808 | 4-cyanopyridin-2-yl | C(O)Et | Me | H | CN | H | H | Cl | H | nonafluoro-2-butyl | H | C2F5 |
| 4-809 | phenyl | Me | Me | H | CN | H | H | Br | H | heptafluoroisopropyl | H | CF3 |
| 4-823 | (3-tᵣifluoromethyl) phenyl | S(O)2Me | Me | H | CN | H | H | Br | H | heptafluoroisopropyl | H | CF3 |
| 4-828 | 2-cyanophenyl | Me | S(O)2Me | H | CN | H | H | Br | H | heptafluoroisopropyl | H | CF3 |
| 4-829 | 3-cyanophenyl | Me | Me | H | CN | H | H | Br | H | heptafluoroisopropyl | H | CF3 |
| 4-830 | 4-cyanophenyl | Me | Me | H | CN | H | H | Br | H | heptafluoroisopropyl | H | CF3 |
| 4-839 | 2-chloro-4-nitrophenyl | Me | S(O)2Et | H | CN | H | H | Br | H | heptafluoroisopropyl | H | CF3 |
| 4-841 | 4-cyano-2-fluorophenyl | S(O)2Et | Me | H | CN | H | H | Br | H | heptafluoroisopropyl | H | CF3 |
| 4-845 | 2-chloropyridin-3-yl | Me | Me | H | CN | H | H | Br | H | heptafluoroisopropyl | H | CF3 |
| 4-853 | 8-bromopyridin-3-yl | Me | CH2Ph | H | CN | H | H | Br | H | heptafluoroisopropyl | H | CF3 |
| 4-855 | 6-(trifluoromethyl) pyridin-3-yl | Me | Me | H | CN | H | H | Br | H | heptafluoroisopropyl | H | CF3 |
| 4-856 | 6-nitropyridin-3-yl | CH2Ph | Me | H | CN | H | H | Br | H | heptafluoroisopropyl | H | CF3 |
| 4-863 | 5-nitropyridin-3-yl | Me | CH2(3-Py) | H | CN | H | H | Br | H | heptafluoroisopropyl | H | CF3 |
| 4-865 | 4-fluoropyridin-3-yl | CH2(3-Py) | Me | H | CN | H | H | Br | H | heptafluoroisopropyl | H | CF3 |
| 4-883 | 4-bromopyridin-2-yl | Me | CH2(3-Py-N-oxide) | H | CN | H | H | Br | H | heptafluoroisopropyl | H | CF3 |
| 4-886 | 4-cyanopyridin-2-yl | CH2(3-Py-N-oxide) | Me | H | CN | H | H | Br | H | heptafluoroisopropyl | H | CF3 |
| 4-887 | phenyl | Me | Me | H | CN | H | H | Br | H | nonafluoro-2-butyl | H | CF3 |
| 4-907 | 3-cyanophenyl | Me | Me | H | CN | H | H | Br | H | nonafluoro-2-butyl | H | CF3 |
| 4-908 | 4-cyanophenyl | Me | Me | H | CN | H | H | Br | H | nonafluoro-2-butyl | H | CF3 |
| 4-923 | 2-chloropyridin-3-yl | Me | Me | H | CN | H | H | Br | H | nonafluoro-2-butyl | H | CF3 |
| 4-965 | phenyl | Me | Me | H | CN | H | H | I | H | heptafluoroisopropyl | H | CF3 |
| 4-985 | 3-cyanophenyl | Me | Me | H | CN | H | H | I | H | heptafluoroisopropyl | H | CF3 |
| 4-986 | 4-cyanophenyl | Me | Me | H | CN | H | H | I | H | heptafluoroisopropyl | H | CF3 |
| 4-1001 | 2-chloropyridin-3-yl | Me | Me | H | CN | H | H | I | H | heptafluoroisopropyl | H | CF3 |
| 4-1043 | phenyl | Me | Me | H | CN | H | H | I | H | nonafluoro-2-butyl | H | CF3 |
| 4-1063 | 3-cyanophenyl | Me | Me | H | CN | H | H | I | H | nonafluoro-2-butyl | H | CF3 |
| 4-1064 | 4-cyanophenyl | Me | Me | H | CN | H | H | I | H | nonafluoro-2-butyl | H | CF3 |
| 4-1079 | 2-chloropyridin-3-yl | Me | Me | H | CN | H | H | I | H | nonafluoro-2-butyl | H | CF3 |
| 4-1121 | phenyl | Et | Et | H | CN | H | H | I | H | nonafluoro-2-butyl | H | CF3 |
| 4-1122 | phenyl | Et | n-Pr | H | CN | H | H | I | H | nonafluoro-2-butyl | H | CF3 |
| 4-1123 | phenyl | Et | i-Pr | H | CN | H | H | I | H | nonafluoro-2-butyl | H | CF3 |
| 4-1124 | phenyl | Et | CH2CH=CH2 | H | CN | H | H | I | H | nonafluoro-2-butyl | H | CF3 |
| 4-1125 | phenyl | Et | CN | H | CN | H | H | I | H | nonafluoro-2-butyl | H | CF3 |
| 4-1126 | phenyl | Et | CH2C≡CH | H | CN | H | H | I | H | nonafluoro-2-butyl | H | CF3 |
| 4-1127 | phenyl | Et | NH2 | H | CN | H | H | I | H | nonafluoro-2-butyl | H | CF3 |
| 4-1128 | phenyl | Et | C(O)OMe | H | CN | H | H | I | H | nonafluoro-2-butyl | H | CF3 |
| 4-1129 | phenyl | Et | C(O)C(O)OMe | H | CN | H | H | I | H | nonafluoro-2-butyl | H | CF3 |
| 4-1130 | phenyl | Et | C(O)Me | H | CN | H | H | I | H | nonafluoro-2-butyl | H | CF3 |
| 4-1131 | phenyl | Et | S(O)2Me | H | CN | H | H | I | H | nonafluoro-2-butyl | H | CF3 |
| 4-1132 | phenyl | Et | CH2Ph | H | CN | H | H | I | H | nonafluoro-2-butyl | H | CF3 |
| 4-1133 | phenyl | Et | CH2(3-Py) | H | CN | H | H | I | H | nonafluoro-2-butyl | H | CF3 |
| 4-1134 | phenyl | Et | CH2(3-Py-N-oxide) | H | CN | H | H | I | H | nonafluoro-2-butyl | H | CF3 |
| 4-1135 | phenyl | n-Pr | Et | H | CN | H | H | I | H | nonafluoro-2-butyl | H | CF3 |
| 4-1136 | phenyl | n-Pr | n-Pr | H | CN | H | H | I | H | nonafluoro-2-butyl | H | CF3 |

**Table 4(3)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 4-1137 | phenyl | n-Pr | i-Pr | H | CN | H | H | I | H | nonafluoro-2-butyl | H | CF3 |
| 4-1138 | phenyl | n-Pr | CH2CH=CH2 | H | CN | H | H | I | H | nonafluoro-2-butyl | H | CF3 |
| 4-1139 | phenyl | n-Pr | CN | H | CN | H | H | I | H | nonafluoro-2-butyl | H | CF3 |
| 4-1140 | phenyl | n-Pr | CH2C≡CH | H | CN | H | H | I | H | nonafluoro-2-butyl | H | CF3 |
| 4-1141 | phenyl | n-Pr | NH2 | H | CN | H | H | I | H | nonafluoro-2-butyl | H | CF3 |
| 4-1142 | phenyl | n-Pr | O(O)OMe | H | CN | H | H | I | H | nonafluoro-2-butyl | H | CF3 |
| 4-1143 | phenyl | n-Pr | C(O)C(O)OMe | H | CN | H | H | I | H | nonafluoro-2-butyl | H | CF3 |
| 4-1144 | phenyl | n-Pr | C(O)Me | H | CN | H | H | I | H | nonafluoro-2-butyl | H | CF3 |
| 4-1145 | phenyl | n-Pr | S(O)2Me | H | CN | H | H | I | H | nonafluoro-2-butyl | H | CF3 |
| 4-1146 | phenyl | n-Pr | CH2Ph | H | CN | H | H | I | H | nonafluoro-2-butyl | H | CF3 |
| 4-1147 | phenyl | n-Pr | CH2(3-Py) | H | CN | H | H | I | H | nonafluoro-2-butyl | H | CF3 |
| 4-1148 | phenyl | n-Pr | CH2(3-Py-N-oxide) | H | CN | H | H | I | H | nonafluoro-2-butyl | H | CF3 |
| 4-1149 | phenyl | i-Pr | Et | H | CN | H | H | I | H | nonafluoro-2-butyl | H | CF3 |
| 4-1150 | phenyl | i-Pr | n-Pr | H | CN | H | H | I | H | nonafluoro-2-butyl | H | CF3 |
| 4-1151 | phenyl | i-Pr | i-Pr | H | CN | H | H | I | H | nonafluoro-2-butyl | H | CF3 |
| 4-1152 | phenyl | i-Pr | CH2CH=CH2 | H | CN | H | H | I | H | nonafluoro-2-butyl | H | CF3 |
| 4-1153 | phenyl | i-Pr | CN | H | CN | H | H | I | H | nonafluoro-2-butyl | H | CF3 |
| 4-1154 | phenyl | i-Pr | CH2C≡CH | H | CN | H | H | I | H | nonafluoro-2-butyl | H | CF3 |
| 4-1155 | phenyl | i-Pr | NH2 | H | CN | H | H | I | H | nonafluoro-2-butyl | H | CF3 |
| 4-1156 | phenyl | i-Pr | C(O)OMe | H | CN | H | H | I | H | nonafluoro-2-butyl | H | CF3 |
| 4-1157 | phenyl | i-Pr | C(O)C(O)OMe | H | CN | H | H | I | H | nonafluoro-2-butyl | H | CF3 |
| 4-1158 | phenyl | i-Pr | C(O)Me | H | CN | H | H | I | H | nonafluoro-2-butyl | H | CF3 |
| 4-1159 | phenyl | i-Pr | S(O)2Me | H | CN | H | H | I | H | nonafluoro-2-butyl | H | CF3 |
| 4-1160 | phenyl | i-Pr | CH2Ph | H | CN | H | H | I | H | nonafluoro-2-butyl | H | CF3 |
| 4-1161 | phenyl | i-Pr | CH2(3-Py) | H | CN | H | H | I | H | nonafluoro-2-butyl | H | CF3 |
| 4-1162 | phenyl | i-Pr | CH2(3-Py-N-oxide) | H | CN | H | H | I | H | nonafluoro-2-butyl | H | CF3 |
| 4-1163 | phenyl | CH2CH=CH2 | Et | H | CN | H | H | I | H | nonafluoro-2-butyl | H | CF3 |
| 4-1164 | phenyl | CH2CH=CH2 | n-Pr | H | CN | H | H | I | H | nonafluoro-2-butyl | H | CF3 |
| 4-1165 | phenyl | CH2CH=CH2 | i-Pr | H | CN | H | H | I | H | nonafluoro-2-butyl | H | CF3 |
| 4-1166 | phenyl | CH2CH=CH2 | CH2CH=CH2 | H | CN | H | H | I | H | nonafluoro-2-butyl | H | CF3 |
| 4-1167 | phenyl | CH2CH=CH2 | CN | H | CN | H | H | I | H | nonafluoro-2-butyl | H | CF3 |
| 4-1168 | phenyl | CH2CH=CH2 | CH2C≡CH | H | CN | H | H | I | H | nonafluoro-2-butyl | H | CF3 |
| 4-1169 | phenyl | CH2CH=CH2 | NH2 | H | CN | H | H | I | H | nonafluoro-2-butyl | H | CF3 |
| 4-1170 | phenyl | CH2CH=CH2 | C(O)OMe | H | CN | H | H | I | H | nonafluoro-2-butyl | H | CF3 |
| 4-1171 | phenyl | CH2CH=CH2 | C(O)C(O)OMe | H | CN | H | H | I | H | nonafluoro-2-butyl | H | CF3 |
| 4-1172 | phenyl | CH2CH=CH2 | C(O)Me | H | CN | H | H | I | H | nonafluoro-2-butyl | H | CF3 |
| 4-1173 | phenyl | CH2CH=CH2 | S(O)2Me | H | CN | H | H | I | H | nonafluoro-2-butyl | H | CF3 |
| 4-1174 | phenyl | CH2CH=CH2 | CH2Ph | H | CN | H | H | I | H | nonafluoro-2-butyl | H | CF3 |
| 4-1175 | phenyl | CH2CH=CH2 | CH2(3-Py) | H | CN | H | H | I | H | nonafluoro-2-butyl | H | CF3 |
| 4-1176 | phenyl | CH2CH=CH2 | CH2(3-Py-N-oxide) | H | CN | H | H | I | H | nonafluoro-2-butyl | H | CF3 |
| 4-1177 | phenyl | CN | Et | H | CN | H | H | I | H | nonafluoro-2-butyl | H | CF3 |
| 4-1178 | phenyl | CN | n-Pr | H | CN | H | H | I | H | nonafluoro-2-butyl | H | CF3 |
| 4-1179 | phenyl | CN | i-Pr | H | CN | H | H | I | H | nonafluoro-2-butyl | H | CF3 |
| 4-1180 | phenyl | CN | CH2CH=CH2 | H | CN | H | H | I | H | nonafluoro-2-butyl | H | CF3 |
| 4-1181 | phenyl | CN | CN | H | CN | H | H | I | H | nonafluoro-2-butyl | H | CF3 |
| 4-1182 | phenyl | CN | CH2C≡CH | H | CN | H | H | I | H | nonafluoro-2-butyl | H | CF3 |
| 4-1183 | phenyl | CN | NH2 | H | CN | H | H | I | H | nonafluoro-2-butyl | H | CF3 |
| 4-1184 | phenyl | CN | C(O)OMe | H | CN | H | H | I | H | nonafluoro-2-butyl | H | CF3 |
| 4-1185 | phenyl | CN | C(O)C(O)OMe | H | CN | H | H | I | H | nonafluoro-2-butyl | H | CF3 |
| 4-1186 | phenyl | CN | C(O)Me | H | CN | H | H | I | H | nonafluoro-2-butyl | H | CF3 |
| 4-1187 | phenyl | CN | S(O)2Me | H | CN | H | H | I | H | nonafluoro-2-butyl | H | CF3 |
| 4-1188 | phenyl | CN | CH2Ph | H | CN | H | H | I | H | nonafluoro-2-butyl | H | CF3 |
| 4-1189 | phenyl | CN | CH2(3-Py) | H | CN | H | H | I | H | nonafluoro-2-butyl | H | CF3 |
| 4-1190 | phenyl | CN | CH2(3-Py-N-oxide) | H | CN | H | H | I | H | nonafluoro-2-butyl | H | CF3 |
| 4-1191 | phenyl | CH2C≡CH | Et | H | CN | H | H | I | H | nonafluoro-2-butyl | H | CF3 |
| 4-1192 | phenyl | CH2C≡CH | n-Pr | H | CN | H | H | I | H | nonafluoro-2-butyl | H | CF3 |
| 4-1193 | phenyl | CH2C≡CH | i-Pr | H | CN | H | H | I | H | nonafluoro-2-butyl | H | CF3 |
| 4-1194 | phenyl | CH2C≡CH | CH2CH=CH2 | H | CN | H | H | I | H | nonafluoro-2-butvl | H | CF3 |
| 4-1195 | phenyl | CH2C≡CH | CN | H | CN | H | H | I | H | nonafluoro-2-butyl | H | CF3 |
| 4-1196 | phenyl | CH2C≡CH | CH2C≡CH | H | CN | H | H | I | H | nonafluoro-2-butyl | H | CF3 |
| 4-1197 | phenyl | CH2C≡CH | NH2 | H | CN | H | H | I | H | nonafluoro-2-butyl | H | CF3 |
| 4-1198 | phenyl | CH2C≡CH | C(O)OMe | H | CN | H | H | I | H | nonafluoro-2-butyl | H | CF3 |
| 4-1199 | phenyl | CH2C≡CH | C(O)C(O)OMe | H | CN | H | H | I | H | nonafluoro-2-butyl | H | CF3 |
| 4-1200 | phenyl | CH2C≡CH | C(O)Me | H | CN | H | H | I | H | nonafluoro-2-butyl | H | CF3 |
| 4-1201 | phenyl | CH2C≡CH | S(O)2Me | H | CN | H | H | I | H | nonafluoro-2-butyl | H | CF3 |
| 4-1202 | phenyl | CH2C≡CH | CH2Ph | H | CN | H | H | I | H | nonafluoro-2-butyl | H | CF3 |

**Table 4(4)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 4-1203 | phenyl | CH2C≡CH | CH2(3-Py) | H | CN | H | H | I | H | nonafluoro-2-butyl | H | CF3 |
| 4-1204 | phenyl | CH2C≡CH | CH2(3-Py-N-oxide) | H | CN | H | H | I | H | nonafluoro-2-butyl | H | CF3 |
| 4-1205 | phenyl | NH2 | Et | H | CN | H | H | I | H | nonafluoro-2-butyl | H | CF3 |
| 4-1206 | phenyl | NH2 | n-Pr | H | CN | H | H | I | H | nonafluoro-2-butyl | H | CF3 |
| 4-1207 | phenyl | NH2 | i-Pr | H | CN | H | H | I | H | nonafluoro-2-butyl | H | CF3 |
| 4-1208 | phenyl | NH2 | CH2CH=CH2 | H | CN | H | H | I | H | nonafluoro-2-butyl | H | CF3 |
| 4-1209 | phenyl | NH2 | CN | H | CN | H | H | I | H | nonafluoro-2-butyl | H | CF3 |
| 4-1210 | phenyl | NH2 | CH2C≡CH | H | CN | H | H | I | H | nonafluoro-2-butyl | H | CF3 |
| 4-1211 | phenyl | NH2 | NH2 | H | CN | H | H | I | H | nonafluoro-2-butyl | H | CF3 |
| 4-1212 | phenyl | NH2 | C(O)OMe | H | CN | H | H | I | H | nonafluoro-2-butyl | H | CF3 |
| 4-1213 | phenyl | NH2 | C(O)C(O)OMe | H | CN | H | H | I | H | nonafluoro-2-butyl | H | CF3 |
| 4-1214 | phenyl | NH2 | C(O)Me | H | CN | H | H | I | H | nonafluoro-2-butyl | H | CF3 |
| 4-1215 | phenyl | NH2 | S(O)2Me | H | CN | H | H | I | H | nonafluoro-2-butyl | H | CF3 |
| 4-1216 | phenyl | NH2 | CH2Ph | H | CN | H | H | I | H | nonafluoro-2-butyl | H | CF3 |
| 4-1217 | phenyl | NH2 | CH2(3-Py) | H | CN | H | H | I | H | nonafluoro-2-butyl | H | CF3 |
| 4-1218 | phenyl | NH2 | CH2(3-Py-N-oxide) | H | CN | H | H | I | H | nonafluoro-2-butyl | H | CF3 |
| 4-1219 | phenyl | C(O)OMe | Et | H | CN | H | H | I | H | nonafluoro-2-butyl | H | CF3 |
| 4-1220 | phenyl | C(O)OMe | n-Pr | H | CN | H | H | I | H | nonafluoro-2-butyl | H | CF3 |
| 4-1221 | phenyl | C(O)OMe | i-Pr | H | CN | H | H | I | H | nonafluoro-2-butyl | H | CF3 |
| 4-1222 | phenyl | C(O)OMe | CH2CH=CH2 | H | CN | H | H | I | H | nonafluoro-2-butyl | H | CF3 |
| 4-1223 | phenyl | C(O)OMe | CN | H | CN | H | H | I | H | nonafluoro-2-butyl | H | CF3 |
| 4-1224 | phenyl | C(O)OMe | CH2C≡CH | H | CN | H | H | I | H | nonafluoro-2-butyl | H | CF3 |
| 4-1225 | phenyl | C(O)OMe | NH2 | H | CN | H | H | I | H | nonafluoro-2-butyl | H | CF3 |
| 4-1226 | phenyl | C(O)OMe | C(O)OMe | H | CN | H | H | I | H | nonafluoro-2-butyl | H | CF3 |
| 4-1227 | phenyl | C(O)OMe | C(O)C(O)OMe | H | CN | H | H | I | H | nonafluoro-2-butyl | H | CF3 |
| 4-1228 | phenyl | C(O)OMe | C(O)Me | H | CN | H | H | I | H | nonafluoro-2-butyl | H | CF3 |
| 4-1229 | phenyl | C(O)OMe | S(O)2Me | H | CN | H | H | I | H | nonafluoro-2-butyl | H | CF3 |
| 4-1230 | phenyl | C(O)OMe | CH2Ph | H | CN | H | H | I | H | nonafluoro-2-butyl | H | CF3 |
| 4-1231 | phenyl | C(O)OMe | CH2(3-Py) | H | CN | H | H | I | H | nonafluoro-2-butyl | H | CF3 |
| 4-1232 | phenyl | C(O)OMe | CH2(3-Py-N-oxide) | H | CN | H | H | I | H | nonafluoro-2-butyl | H | CF3 |
| 4-1233 | phenyl | C(O)C(O)OMe | Et | H | CN | H | H | I | H | nonafluoro-2-butyl | H | CF3 |
| 4-1234 | phenyl | C(O)C(O)OMe | n-Pr | H | CN | H | H | I | H | nonafluoro-2-butyl | H | CF3 |
| 4-1235 | phenyl | C(O)C(O)OMe | i-Pr | H | CN | H | H | I | H | nonafluoro-2-butyl | H | CF3 |
| 4-1236 | phenyl | C(O)C(O)OMe | CH2CH=CH2 | H | CN | H | H | I | H | nonafluoro-2-butyl | H | CF3 |
| 4-1237 | phenyl | C(O)C(O)OMe | CN | H | CN | H | H | I | H | nonafluoro-2-butyl | H | CF3 |
| 4-1238 | phenyl | C(O)C(O)OMe | CH2C≡CH | H | CN | H | H | I | H | nonafluoro-2-butyl | H | CF3 |
| 4-1239 | phenyl | C(O)C(O)OMe | NH2 | H | CN | H | H | I | H | nonafluoro-2-butyl | H | CF3 |
| 4-1240 | phenyl | C(O)C(O)OMe | C(O)OMe | H | CN | H | H | I | H | nonafluoro-2-butyl | H | CF3 |
| 4-1241 | phenyl | C(O)C(O)OMe | C(O)C(O)OMe | H | CN | H | H | I | H | nonafluoro-2-butyl | H | CF3 |
| 4-1242 | phenyl | C(O)C(O)OMe | C(O)Me | H | CN | H | H | I | H | nonafluoro-2-butyl | H | CF3 |
| 4-1243 | phenyl | C(O)C(O)OMe | S(O)2Me | H | CN | H | H | I | H | nonafluoro-2-butyl | H | CF3 |
| 4-1244 | phenyl | C(O)C(O)OMe | CH2Ph | H | CN | H | H | I | H | nonafluoro-2-butyl | H | CF3 |
| 4-1245 | phenyl | C(O)C(O)OMe | CH2(3-Py) | H | CN | H | H | I | H | nonafluoro-2-butyl | H | CF3 |
| 4-1246 | phenyl | C(O)C(O)OMe | CH2(3-Py-N-oxide) | H | CN | H | H | I | H | nonafluoro-2-butyl | H | CF3 |
| 4-1247 | phenyl | C(O)Me | Et | H | CN | H | H | I | H | nonafluoro-2-butyl | H | CF3 |
| 4-1248 | phenyl | C(O)Me | n-Pr | H | CN | H | H | I | H | nonafluoro-2-butyl | H | CF3 |
| 4-1249 | phenyl | C(O)Me | i-Pr | H | CN | H | H | I | H | nonafluoro-2-butyl | H | CF3 |
| 4-1250 | phenyl | C(O)Me | CH2CH=CH2 | H | CN | H | H | I | H | nonafluoro-2-butyl | H | CF3 |
| 4-1251 | phenyl | C(O)Me | CN | H | CN | H | H | I | H | nonafluoro-2-butyl | H | CF3 |
| 4-1252 | phenyl | C(O)Me | CH2C≡CH | H | CN | H | H | I | H | nonafluoro-2-butyl | H | CF3 |
| 4-1253 | phenyl | C(O)Me | NH2 | H | CN | H | H | I | H | nonafluoro-2-butyl | H | CF3 |
| 4-1254 | phenyl | C(O)Me | C(O)OMe | H | CN | H | H | I | H | nonafluoro-2-butyl | H | CF3 |
| 4-1255 | phenyl | C(O)Me | C(O)C(O)OMe | H | CN | H | H | I | H | nonafluoro-2-butyl | H | CF3 |
| 4-1256 | phenyl | C(O)Me | C(O)Me | H | CN | H | H | I | H | nonafluoro-2-butyl | H | CF3 |
| 4-1257 | phenyl | C(O)Me | S(O)2Me | H | CN | H | H | I | H | nonafluoro-2-butyl | H | CF3 |
| 4-1258 | phenyl | C(O)Me | CH2Ph | H | CN | H | H | I | H | nonafluoro-2-butyl | H | CF3 |
| 4-1259 | phenyl | C(O)Me | CH2(3-Py) | H | CN | H | H | I | H | nonafluoro-2-butyl | H | CF3 |
| 4-1260 | phenyl | C(O)Me | CH2(3-Py-N-oxide) | H | CN | H | H | I | H | nonafluoro-2-butyl | H | CF3 |
| 4-1261 | phenyl | S(O)2Me | Et | H | CN | H | H | I | H | nonafluoro-2-butyl | H | CF3 |
| 4-1262 | phenyl | S(O)2Me | n-Pr | H | CN | H | H | I | H | nonafluoro-2-butyl | H | CF3 |
| 4-1263 | phenyl | S(O)2Me | i-Pr | H | CN | H | H | I | H | nonafluoro-2-butyl | H | CF3 |
| 4-1264 | phenyl | S(O)2Me | CH2CH=CH2 | H | CN | H | H | I | H | nonafluoro-2-butyl | H | CF3 |
| 4-1265 | phenyl | S(O)2Me | CN | H | CN | H | H | I | H | nonafluoro-2-butyl | H | CF3 |
| 4-1266 | phenyl | S(O)2Me | CH2C≡CH | H | CN | H | H | I | H | nonafluoro-2-butyl | H | CF3 |
| 4-1267 | phenyl | S(O)2Me | NH2 | H | CN | H | H | I | H | nonafluoro-2-butyl | H | CF3 |
| 4-1268 | phenyl | S(O)2Me | C(O)OMe | H | CN | H | H | I | H | nonafluoro-2-butyl | H | CF3 |

**Table 4(5)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 4-1269 | phenyl | S(O)2Me | C(O)C(O)OMe | H | CN | H | H | I | H | nonafluoro-2-butyl | H | CF3 |
| 4-1270 | phenyl | S(O)2Me | C(O)Me | H | CN | H | H | I | H | nonafluoro-2-butyl | H | CF3 |
| 4-1271 | phenyl | S(O)2Me | S(O)2Me | H | CN | H | H | I | H | nonafluoro-2-butyl | H | CF3 |
| 4-1272 | phenyl | S(O)2Me | CH2Ph | H | CN | H | H | I | H | nonafluoro-2-butyl | H | CF3 |
| 4-1273 | phenyl | S(O)2Me | CH2(3-Py) | H | CN | H | H | I | H | nonafluoro-2-butyl | H | CF3 |
| 4-1274 | phenyl | S(O)2Me | CH2(3-Py-N-oxide) | H | CN | H | H | I | H | nonafluoro-2-butyl | H | CF3 |
| 4-1275 | phenyl | CH2Ph | Et | H | CN | H | H | I | H | nonafluoro-2-butyl | H | CF3 |
| 4-1276 | phenyl | CH2Ph | n-Pr | H | CN | H | H | I | H | nonafluoro-2-butyl | H | CF3 |
| 4-1277 | phenyl | CH2Ph | i-Pr | H | CN | H | H | I | H | nonafluoro-2-butyl | H | CF3 |
| 4-1278 | phenyl | CH2Ph | CH2CH=CH2 | H | CN | H | H | I | H | nonafluoro-2-butyl | H | CF3 |
| 4-1279 | phenyl | CH2Ph | CN | H | CN | H | H | I | H | nonafluoro-2-butyl | H | CF3 |
| 4-1280 | phenyl | CH2Ph | CH2C≡CH | H | CN | H | H | I | H | nonafluoro-2-butyl | H | CF3 |
| 4-1281 | phenyl | CH2Ph | NH2 | H | CN | H | H | I | H | nonafluoro-2-butyl | H | CF3 |
| 4-1282 | phenyl | CH2Ph | C(O)OMe | H | CN | H | H | I | H | nonafluoro-2-butyl | H | CF3 |
| 4-1283 | phenyl | CH2Ph | C(O)C(O)OMe | H | CN | H | H | I | H | nonafluoro-2-butyl | H | CF3 |
| 4-1284 | phenyl | CH2Ph | C(O)Me | H | CN | H | H | I | H | nonafluoro-2-butyl | H | CF3 |
| 4-1285 | phenyl | CH2Ph | S(O)2Me | H | CN | H | H | I | H | nonafluoro-2-butyl | H | CF3 |
| 4-1286 | phenyl | CH2Ph | CH2Ph | H | CN | H | H | I | H | nonafluoro-2-butyl | H | CF3 |
| 4-1287 | phenyl | CH2Ph | CH2(3-Py) | H | CN | H | H | I | H | nonafluoro-2-butyl | H | CF3 |
| 4-1288 | phenyl | CH2Ph | CH2(3-Py-N-oxide) | H | CN | H | H | I | H | nonafluoro-2-butyl | H | CF3 |
| 4-1289 | phenyl | CH2(3-Py) | Et | H | CN | H | H | I | H | nonafluoro-2-butyl | H | CF3 |
| 4-1290 | phenyl | CH2(3-Py) | n-Pr | H | CN | H | H | I | H | nonafluoro-2-butyl | H | CF3 |
| 4-1291 | phenyl | CH2(3-Py) | i-Pr | H | CN | H | H | I | H | nonafluoro-2-butyl | H | CF3 |
| 4-1292 | phenyl | CH2(3-Py) | CH2CH=CH2 | H | CN | H | H | I | H | nonafluoro-2-butyl | H | CF3 |
| 4-1293 | phenyl | CH2(3-Py) | CN | H | CN | H | H | I | H | nonafluoro-2-butyl | H | CF3 |
| 4-1294 | phenyl | CH2(3-Py) | CH2C≡CH | H | CN | H | H | I | H | nonafluoro-2-butyl | H | CF3 |
| 4-1295 | phenyl | CH2(3-Py) | NH2 | H | CN | H | H | I | H | nonafluoro-2-butyl | H | CF3 |
| 4-1296 | phenyl | CH2(3-Py) | C(O)OMe | H | CN | H | H | I | H | nonafluoro-2-butyl | H | CF3 |
| 4-1297 | phenyl | CH2(3-Py) | C(O)C(O)OMe | H | CN | H | H | I | H | nonafluoro-2-butyl | H | CF3 |
| 4-1298 | phenyl | CH2(3-Py) | C(O)Me | H | CN | H | H | I | H | nonafluoro-2-butyl | H | CF3 |
| 4-1299 | phenyl | CH2(3-Py) | S(O)2Me | H | CN | H | H | I | H | nonafluoro-2-butyl | H | CF3 |
| 4-1300 | phenyl | CH2(3-Py) | CH2Ph | H | CN | H | H | I | H | nonafluoro-2-butyl | H | CF3 |
| 4-1301 | phenyl | CH2(3-Py) | CH2(3-Py) | H | CN | H | H | I | H | nonafluoro-2-butyl | H | CF3 |
| 4-1302 | phenyl | CH2(3-Py) | CH2(3-Py-N-oxide) | H | CN | H | H | I | H | nonafluoro-2-butyl | H | CF3 |
| 4-1303 | phenyl | CH2(3-Py-N-oxide) | Et | H | CN | H | H | I | H | nonafluoro-2-butyl | H | CF3 |
| 4-1304 | phenyl | CH2(3-Py-N-oxide) | n-Pr | H | CN | H | H | I | H | nonafluoro-2-butyl | H | CF3 |
| 4-1305 | phenyl | CH2(3-Py-N-oxide) | i-Pr | H | CN | H | H | I | H | nonafluoro-2-butyl | H | CF3 |
| 4-1306 | phenyl | CH2(3-Py-N-oxide) | CH2CH=CH2 | H | CN | H | H | I | H | nonafluoro-2-butyl | H | CF3 |
| 4-1307 | phenyl | CH2(3-Py-N-oxide) | CN | H | CN | H | H | I | H | nonafluoro-2-butyl | H | CF3 |
| 4-1308 | phenyl | CH2(3-Py-N-oxide) | CH2C≡CH | H | CN | H | H | I | H | nonafluoro-2-butyl | H | CF3 |
| 4-1309 | phenyl | CH2(3-Py-N-oxide) | NH2 | H | CN | H | H | I | H | nonafluoro-2-butyl | H | CF3 |
| 4-1310 | phenyl | CH2(3-Py-N-oxide) | C(O)OMe | H | CN | H | H | I | H | nonafluoro-2-butyl | H | CF3 |
| 4-1311 | phenyl | CH2(3-Py-N-oxide) | C(O)C(O)OMe | H | CN | H | H | I | H | nonafluoro-2-butyl | H | CF3 |
| 4-1312 | phenyl | CH2(3-Py-N-oxide) | C(O)Me | H | CN | H | H | I | H | nonafluoro-2-butyl | H | CF3 |
| 4-1313 | phenyl | CH2(3-Py-N-oxide) | S(O)2Me | H | CN | H | H | I | H | nonafluoro-2-butyl | H | CF3 |
| 4-1314 | phenyl | CH2(3-Py-N-oxide) | CH2Ph | H | CN | H | H | I | H | nonafluoro-2-butyl | H | CF3 |
| 4-1315 | phenyl | CH2(3-Py-N-oxide) | CH2(3-Py) | H | CN | H | H | I | H | nonafluoro-2-butyl | H | CF3 |
| 4-1316 | phenyl | CH2(3-Py-N-oxide) | CH2(3-Py-N-oxide) | H | CN | H | H | I | H | nonafluoro-2-butyl | H | CF3 |
| 4-1317 | phenyl | Me | Me | H | CN | H | H | OCF3 | H | heptafluoroisopropyl | H | OCF3 |
| 4-1348 | 2-chloropyridin-3-yl | Me | Me | H | CN | H | H | OCF3 | H | heptafluoroisopropyl | H | OCF3 |
| 4-1373 | phenyl | Me | Me | H | CN | H | H | OCF3 | H | nonafluoro-2-butyl | H | OCF3 |
| 4-1404 | 2-chloropyridin-3-yl | Me | Me | H | CN | H | H | OCF3 | H | nonafluoro-2-butyl | H | OCF3 |
| 4-1429 | phenyl | Me | Me | H | CN | H | H | CF3 | H | heptafluoroisopropyl | H | OCF3 |
| 4-1460 | 2-chloropyridin-3-yl | Me | Me | H | CN | H | H | CF3 | H | heptafluoroisopropyl | H | OCF3 |
| 4-1485 | phenyl | Me | Me | H | CN | H | H | CF3 | H | nonafluoro-2-butyl | H | OCF3 |
| 4-1516 | 2-chloropyridin-3-yl | Me | Me | H | CN | H | H | CF3 | H | nonafluoro-2-butyl | H | OCF3 |
| 4-1541 | phenyl | Me | Me | H | CN | H | H | CF3 | H | heptafluoroisopropyl | H | CF3 |
| 4-1561 | 3-cyanophenyl | Me | Me | H | CN | H | H | CF3 | H | heptafluoroisopropyl | H | CF3 |

**Table 4(6)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 4-1562 | 4-cyanophenyl | Me | Me | H | CN | H | H | CF3 | H | heptafluoroisopropyl | H | CF3 |
| 4-1577 | 2-chloropyridin-3-yl | Me | Me | H | CN | H | H | CF3 | H | heptafluoroisopropyl | H | CF3 |
| 4-1609 | phenyl | Me | Me | H | CN | H | H | CF3 | H | nonafluoro-2-butyl | H | CF3 |
| 4-1629 | 3-cyanophenyl | Me | Me | H | CN | H | H | CF3 | H | nonafluoro-2-butyl | H | CF3 |
| 4-1630 | 4-cyanophenyl | Me | Me | H | CN | H | H | CF3 | H | nonafluoro-2-butyl | H | CF3 |
| 4-1645 | 2-chloropyridin-3-yl | Me | Me | H | CN | H | H | CF3 | H | nonafluoro-2-butyl | H | CF3 |
| 4-1873 | phenyl | Me | Me | F | CN | H | H | Cl | H | heptafluoroisopropyl | H | OCF3 |
| 4-1893 | 3-cyanophenyl | Me | Me | F | CN | H | H | Cl | H | heptafluoroisopropyl | H | OCF3 |
| 4-1909 | 2-chloropyridin-3-yl | Me | Me | F | CN | H | H | Cl | H | heptafluoroisopropyl | H | OCF3 |
| 4-1951 | phenyl | Me | Me | F | CN | H | H | Cl | H | nonafluoro-2-butyl | H | OCF3 |
| 4-1971 | 3-cyanophenyl | Me | Me | F | CN | H | H | Cl | H | nonafluoro-2-butyl | H | OCF3 |
| 4-1972 | 4-cyanophenyl | Me | Me | F | CN | H | H | Cl | H | nonafluoro-2-butyl | H | OCF3 |
| 4-1987 | 2-chloropyridin-3-yl | Me | Me | F | CN | H | H | Cl | H | nonafluoro-2-butyl | H | OCF3 |
| 4-2029 | phenyl | Me | Me | F | CN | H | H | Br | H | heptafluoroisopropyl | H | OCF3 |
| 4-2049 | 3-cyanophenyl | Me | Me | F | CN | H | H | Br | H | heptafluoroisopropyl | H | OCF3 |
| 4-2050 | 4-cyanophenyl | Me | Me | F | CN | H | H | Br | H | heptafluoroisopropyl | H | OCF3 |
| 4-2065 | 2-chloropyridin-3-yl | Me | Me | F | CN | H | H | Br | H | heptafluoroisopropyl | H | OCF3 |
| 4-2107 | phenyl | Me | Me | F | CN | H | H | Br | H | nonafluoro-2-butyl | H | OCF3 |
| 4-2127 | 3-cyanophenyl | Me | Me | F | CN | H | H | Br | H | nonafluoro-2-butyl | H | OCF3 |
| 4-2128 | 4-cyanophenyl | Me | Me | F | CN | H | H | Br | H | nonafluoro-2-butyl | H | OCF3 |
| 4-2143 | 2-chloropyridin-3-yl | Me | Me | F | CN | H | H | Br | H | nonafluoro-2-butyl | H | OCF3 |
| 4-2185 | phenyl | Me | Me | F | CN | H | H | I | H | heptafluoroisopropyl | H | OCF3 |
| 4-2205 | 3-cyanophenyl | Me | Me | F | CN | H | H | I | H | heptafluoroisopropyl | H | OCF3 |
| 4-2206 | 4-cyanophenyl | Me | Me | F | CN | H | H | I | H | heptafluoroisopropyl | H | OCF3 |
| 4-2221 | 2-chloropyridin-3-yl | Me | Me | F | CN | H | H | I | H | heptafluoroisopropyl | H | OCF3 |
| 4-2263 | phenyl | Me | Me | F | CN | H | H | I | H | nonafluoro-2-butyl | H | OCF3 |
| 4-2283 | 3-cyanophenyl | Me | Me | F | CN | H | H | I | H | nonafluoro-2-butyl | H | OCF3 |
| 4-2284 | 4-cyanophenyl | Me | Me | F | CN | H | H | I | H | nonafluoro-2-butyl | H | OCF3 |
| 4-2299 | 2-chloropyridin-3-yl | Me | Me | F | CN | H | H | I | H | nonafluoro-2-butyl | H | OCF3 |
| 4-2537 | phenyl | Me | Me | F | CN | H | H | Cl | H | heptafluoroisopropyl | H | CF3 |
| 4-2538 | phenyl | Et | Me | F | CN | H | H | F | H | heptafluoroisopropyl | H | CF3 |
| 4-2544 | 3-cyanophenyl | Me | Me | F | CN | H | H | Cl | H | heptafluoroisopropyl | H | CF3 |
| 4-2545 | 4-cyanophenyl | Me | Me | F | CN | H | H | Cl | H | heptafluoroisopropyl | H | CF3 |
| 4-2560 | 2-chloropyridin-3-yl | Me | Me | F | CN | H | H | Cl | H | heptafluoroisopropyl | H | CF3 |
| 4-2602 | phenyl | Me | Me | F | CN | H | H | F | H | nonafluoro-2-butyl | H | CF3 |
| 4-2603 | phenyl | Me | Me | F | CN | H | H | Cl | H | nonafluoro-2-butyl | H | CF3 |
| 4-2623 | 3-cyanophenyl | Me | Me | F | CN | H | H | Cl | H | nonafluoro-2-butyl | H | CF3 |
| 4-2624 | 4-cyanophenyl | Me | Me | F | CN | H | H | Cl | H | nonafluoro-2-butyl | H | CF3 |
| 4-2639 | 2-chloropyridin-3-yl | Me | Me | F | CN | H | H | Cl | H | nonafluoro-2-butyl | H | CF3 |
| 4-2681 | phenyl | Me | Me | F | CN | H | H | Br | H | heptafluoroisopropyl | H | CF3 |
| 4-2701 | 3-cyanophenyl | Me | Me | F | CN | H | H | Br | H | heptafluoroisopropyl | H | CF3 |
| 4-2702 | 4-cyanophenyl | Me | Me | F | CN | H | H | Br | H | heptafluoroisopropyl | H | CF3 |
| 4-2717 | 2-chloropyridin-3-yl | Me | Me | F | CN | H | H | Br | H | heptafluoroisopropyl | H | CF3 |
| 4-2759 | phenyl | Me | Me | F | CN | H | H | Br | H | nonafluoro-2-butyl | H | CF3 |
| 4-2779 | 3-cyanophenyl | Me | Me | F | CN | H | H | Br | H | nonafluoro-2-butyl | H | CF3 |
| 4-2780 | 4-cyanophenyl | Me | Me | F | CN | H | H | Br | H | nonafluoro-2-butyl | H | CF3 |
| 4-2795 | 2-chloropyridin-3-yl | Me | Me | F | CN | H | H | Br | H | nonafluoro-2-butyl | H | CF3 |
| 4-2837 | phenyl | Me | Me | F | CN | H | H | I | H | heptafluoroisopropyl | H | CF3 |
| 4-2857 | 3-cyanophenyl | Me | Me | F | CN | H | H | I | H | heptafluoroisopropyl | H | CF3 |
| 4-2858 | 4-cyanophenyl | Me | Me | F | CN | H | H | I | H | heptafluoroisopropyl | H | CF3 |
| 4-2873 | 2-chloropyridin-3-yl | Me | Me | F | CN | H | H | I | H | heptafluoroisopropyl | H | CF3 |
| 4-2915 | phenyl | Me | Me | F | CN | H | H | I | H | nonafluoro-2-butyl | H | CF3 |
| 4-2935 | 3-cyanophenyl | Me | Me | F | CN | H | H | I | H | nonafluoro-2-butyl | H | CF3 |
| 4-2936 | 4-cyanophenyl | Me | Me | F | CN | H | H | I | H | nonafluoro-2-butyl | H | CF3 |
| 4-2951 | 2-chloropyridin-3-yl | Me | Me | F | CN | H | H | I | H | nonafluoro-2-butyl | H | CF3 |
| 4-3189 | phenyl | Me | Me | F | CN | H | H | OCF3 | H | heptafluoroisopropyl | H | OCF3 |
| 4-3220 | 2-chloropyridin-3-yl | Me | Me | F | CN | H | H | OCF3 | H | heptafluoroisopropyl | H | OCF3 |
| 4-3245 | phenyl | Me | Me | F | CN | H | H | OCF3 | H | nonafluoro-2-butyl | H | OCF3 |
| 4-3276 | 2-chloropyridin-3-yl | Me | Me | F | CN | H | H | OCF3 | H | nonafluoro-2-butyl | H | OCF3 |
| 4-3301 | phenyl | Me | Me | F | CN | H | H | CF3 | H | heptafluoroisopropyl | H | OCF3 |
| 4-3332 | 2-chloropyridin-3-yl | Me | Me | F | CN | H | H | CF3 | H | heptafluoroisopropyl | H | OCF3 |
| 4-3357 | phenyl | Me | Me | F | CN | H | H | CF3 | H | nonafluoro-2-butvl | H | OCF3 |
| 4-3388 | 2-chloropyridin-3-yl | Me | Me | F | CN | H | H | CF3 | H | nonafluoro-2-butyl | H | OCF3 |
| 4-3413 | phenyl | Me | Me | F | CN | H | H | CF3 | H | heptafluoroisopropyl | H | CF3 |
| 4-3433 | 3-cyanophenyl | Me | Me | F | CN | H | H | CF3 | H | heptafluoroisopropyl | H | CF3 |
| 4-3434 | 4-cyanophenyl | Me | Me | F | CN | H | H | CF3 | H | heptafluoroisopropyl | H | CF3 |
| 4-3449 | 2-chloropyridin-3-yl | Me | Me | F | CN | H | H | CF3 | H | heptafluoroisopropyl | H | CF3 |
| 4-3481 | phenyl | Me | Me | F | CN | H | H | CF3 | H | nonafluoro-2-butyl | H | CF3 |
| 4-3493 | 4-iodophenyl | Me | Me | F | CN | H | H | C2F5 | H | nonafluoro-2-butyl | H | CF3 |
| 4-3501 | 3-cyanophenyl | Me | Me | F | CN | H | H | CF3 | H | nonafluoro-2-butyl | H | CF3 |
| 4-3502 | 4-cyanophenyl | Me | Me | F | CN | H | H | CF3 | H | nonafluoro-2-butyl | H | CF3 |
| 4-3517 | 2-chloropyridin-3-yl | Me | Me | F | CN | H | H | CF3 | H | nonafluoro-2-butyl | H | CF3 |

**Table 4(7)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 4-3745 | phenyl | Me | Me | F | CN | F | H | Cl | H | heptafluoroisopropyl | H | OCF3 |
| 4-3765 | 3-cyanophenyl | Me | Me | F | CN | F | H | Cl | H | heptafluoroisopropyl | H | OCF3 |
| 4-3766 | 4-cyanophenyl | Me | Me | F | CN | F | H | Cl | H | heptafluoroisopropyl | H | OCF3 |
| 4-3781 | 2-chloropyridin-3-yl | Me | Me | F | CN | F | H | Cl | H | heptafluoroisopropyl | H | OCF3 |
| 4-3823 | phenyl | Me | Me | F | CN | F | H | Cl | H | nonafluoro-2-butyl | H | OCF3 |
| 4-3843 | 3-cyanophenyl | Me | Me | F | CN | F | H | Cl | H | nonafluoro-2-butyl | H | OCF3 |
| 4-3844 | 4-cyanophenyl | Me | Me | F | CN | F | H | Cl | H | nonafluoro-2-butyl | H | OCF3 |
| 4-3859 | 2-chloropyridin-3-yl | Me | Me | F | CN | F | H | Cl | H | nonafluoro-2-butyl | H | OCF3 |
| 4-3901 | phenyl | Me | Me | F | CN | F | H | Br | H | heptafluoroisopropyl | H | OCF3 |
| 4-3921 | 3-cyanophenyl | Me | Me | F | CN | F | H | Br | H | heptafluoroisopropyl | H | OCF3 |
| 4-3922 | 4-cyanophenyl | Me | Me | F | CN | F | H | Br | H | heptafluoroisopropyl | H | OCF3 |
| 4-3937 | 2-chloropyridin-3-yl | Me | Me | F | CN | F | H | Br | H | heptafluoroisopropyl | H | OCF3 |
| 4-3979 | phenyl | Me | Me | F | CN | F | H | Br | H | nonafluoro-2-butyl | H | OCF3 |
| 4-3999 | 3-cyanophenyl | Me | Me | F | CN | F | H | Br | H | nonafluoro-2-butyl | H | OCF3 |
| 4-4000 | 4-cyanophenyl | Me | Me | F | CN | F | H | Br | H | nonafluoro-2-butyl | H | OCF3 |
| 4-4015 | 2-chloropyridin-3-yl | Me | Me | F | CN | F | H | Br | H | nonafluoro-2-butyl | H | OCF3 |
| 4-4057 | phenyl | Me | Me | F | CN | F | H | I | H | heptafluoroisopropyl | H | OCF3 |
| 4-4077 | 3-cyanophenyl | Me | Me | F | CN | F | H | I | H | heptafluoroisopropyl | H | OCF3 |
| 4-4078 | 4-cyanophenyl | Me | Me | F | CN | F | H | I | H | heptafluoroisopropyl | H | OCF3 |
| 4-4093 | 2-chloropyridin-3-yl | Me | Me | F | CN | F | H | I | H | heptafluoroisopropyl | H | OCF3 |
| 4-4135 | phenyl | Me | Me | F | CN | F | H | I | H | nonafluoro-2-butyl | H | OCF3 |
| 4-4155 | 3-cyanophenyl | Me | Me | F | CN | F | H | I | H | nonafluoro-2-butyl | H | OCF3 |
| 4-4156 | 4-cyanophenyl | Me | Me | F | CN | F | H | I | H | nonafluoro-2-butyl | H | OCF3 |
| 4-4171 | 2-chloropyridin-3-yl | Me | Me | F | CN | F | H | I | H | nonafluoro-2-butyl | H | OCF3 |
| 4-4409 | phenyl | Me | Me | F | CN | F | H | Cl | H | heptafluoroisopropyl | H | CF3 |
| 4-4410 | phenyl | Et | Me | F | CN | F | H | F | H | heptafluoroisopropyl | H | CF3 |
| 4-4416 | 3-cyanophenyl | Me | Me | F | CN | F | H | Cl | H | heptafluoroisopropyl | H | CF3 |
| 4-4417 | 4-cyanophenyl | Me | Me | F | CN | F | H | Cl | H | heptafluoroisopropyl | H | CF3 |
| 4-4432 | 2-chloropyridin-3-yl | Me | Me | F | CN | F | H | Cl | H | heptafluoroisopropyl | H | CF3 |
| 4-4474 | phenyl | Me | Me | F | CN | F | H | F | H | nonafluoro-2-butyl | H | CF3 |
| 4-4475 | phenyl | Me | Me | F | CN | F | H | Cl | H | nonafluoro-2-butyl | H | CF3 |
| 4-4495 | 3-cyanophenyl | Me | Me | F | CN | F | H | Cl | H | nonafluoro-2-butyl | H | CF3 |
| 4-4496 | 4-cyanophenyl | Me | Me | F | CN | F | H | Cl | H | nonafluoro-2-butyl | H | CF3 |
| 4-4511 | 2-chloropyridin-3-yl | Me | Me | F | CN | F | H | Cl | H | nonafluoro-2-butyl | H | CF3 |
| 4-4553 | phenyl | Me | Me | F | CN | F | H | Br | H | heptafluoroisopropyl | H | CF3 |
| 4-4573 | 3-cyanophenyl | Me | Me | F | CN | F | H | Br | H | heptafluoroisopropyl | H | CF3 |
| 4-4574 | 4-cyanophenyl | Me | Me | F | CN | F | H | Br | H | heptafluoroisopropyl | H | CF3 |
| 4-4589 | 2-chloropyridin-3-yl | Me | Me | F | CN | F | H | Br | H | heptafluoroisopropyl | H | CF3 |
| 4-4631 | phenyl | Me | Me | F | CN | F | H | Br | H | nonafluoro-2-butyl | H | CF3 |
| 4-4651 | 3-cyanophenyl | Me | Me | F | CN | F | H | Br | H | nonafluoro-2-butyl | H | CF3 |
| 4-4652 | 4-cyanophenyl | Me | Me | F | CN | F | H | Br | H | nonafluoro-2-butyl | H | CF3 |
| 4-4667 | 2-chloropyridin-3-yl | Me | Me | F | CN | F | H | Br | H | nonafluoro-2-butyl | H | CF3 |
| 4-4709 | phenyl | Me | Me | F | CN | F | H | I | H | heptafluoroisopropyl | H | CF3 |
| 4-4729 | 3-cyanophenyl | Me | Me | F | CN | F | H | I | H | heptafluoroisopropyl | H | CF3 |
| 4-4730 | 4-cyanophenyl | Me | Me | F | CN | F | H | I | H | heptafluoroisopropyl | H | CF3 |
| 4-4745 | 2-chloropyridin-3-yl | Me | Me | F | CN | F | H | I | H | heptafluoroisopropyl | H | CF3 |
| 4-4787 | phenyl | Me | Me | F | CN | F | H | I | H | nonafluoro-2-butyl | H | CF3 |
| 4-4807 | 3-cyanophenyl | Me | Me | F | CN | F | H | I | H | nonafluoro-2-butyl | H | CF3 |
| 4-4808 | 4-cyanophenyl | Me | Me | F | CN | F | H | I | H | nonafluoro-2-butyl | H | CF3 |
| 4-4823 | 2-chloropyridin-3-yl | Me | Me | F | CN | F | H | I | H | nonafluoro-2-butyl | H | CF3 |
| 4-5061 | phenyl | Me | Me | F | CN | F | H | OCF3 | H | heptafluoroisopropyl | H | OCF3 |
| 4-5092 | 2-chloropyridin-3-yl | Me | Me | F | CN | F | H | OCF3 | H | heptafluoroisopropyl | H | OCF3 |
| 4-5117 | phenyl | Me | Me | F | CN | F | H | OCF3 | H | nonafluoro-2-butyl | H | OCF3 |
| 4-5148 | 2-chloropyridin-3-yl | Me | Me | F | CN | F | H | OCF3 | H | nonafluoro-2-butyl | H | OCF3 |
| 4-5173 | phenyl | Me | Me | F | CN | F | H | CF3 | H | heptafluoroisopropyl | H | OCF3 |
| 4-5204 | 2-chloropyridin-3-yl | Me | Me | F | CN | F | H | CF3 | H | heptafluoroisopropyl | H | OCF3 |
| 4-5229 | phenyl | Me | Me | F | CN | F | H | CF3 | H | nonafluoro-2-butyl | H | OCF3 |
| 4-5260 | 2-chloropyridin-3-yl | Me | Me | F | CN | F | H | CF3 | H | nonafluoro-2-butyl | H | OCF3 |
| 4-5285 | phenyl | Me | Me | F | CN | F | H | CF3 | H | heptafluoroisopropyl | H | CF3 |
| 4-5305 | 3-cyanophenyl | Me | Me | F | CN | F | H | CF3 | H | heptafluoroisopropyl | H | CF3 |
| 4-5306 | 4-cyanophenyl | Me | Me | F | CN | F | H | CF3 | H | heptafluoroisopropyl | H | CF3 |
| 4-5321 | 2-chloropyridin-3-yl | Me | Me | F | CN | F | H | CF3 | H | heptafluoroisopropyl | H | CF3 |
| 4-5353 | phenyl | Me | Me | F | CN | F | H | CF3 | H | nonafluoro-2-butyl | H | CF3 |
| 4-5373 | 3-cyanophenyl | Me | Me | F | CN | F | H | CF3 | H | nonafluoro-2-butyl | H | CF3 |
| 4-5374 | 4-cyanophenyl | Me | Me | F | CN | F | H | CF3 | H | nonafluoro-2-butyl | H | CF3 |
| 4-5389 | 2-chloropyridin-3-yl | Me | Me | F | CN | F | H | CF3 | H | nonafluoro-2-butyl | H | CF3 |

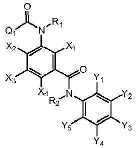

**Table 5(1)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 5-9 | 3-bromophenyl | H | Et | H | CN | H | H | Cl | F | heptafluoroisopropyl | F | OCF3 |
| 5-39 | 2-iodopyridin-3-yl | H | H | H | CN | H | H | Cl | Me | nonafluoro-2-butyl | Me | OCF3 |
| 5-144 | phenyl | H | H | H | CN | H | H | Cl | F | heptafluoroisopropyl | H | CF3 |
| 5-145 | 2-fluorophenyl | Me | H | H | CN | H | H | Cl | H | heptafluoroisopropyl | F | CF3 |
| 5-146 | 3-fluorophenyl | H | Me | H | CN | H | H | Cl | Cl | heptafluoroisopropyl | H | CF3 |
| 5-147 | 4-fluorophenyl | Me | Me | H | CN | H | H | Cl | H | heptafluoroisopropyl | Cl | CF3 |
| 5-148 | 2-chlorophenyl | H | H | H | CN | H | H | F | Br | heptafluoroisopropyl | H | CF3 |
| 5-149 | 3-chlorophenyl | H | H | H | CN | H | H | Cl | H | heptafluoroisopropyl | Br | CF3 |
| 5-150 | 4-chlorophenyl | Et | H | H | CN | H | H | Cl | I | heptafluoroisopropyl | H | C2F5 |
| 5-151 | 2-bromophenyl | H | H | H | CN | H | H | Cl | H | heptafluoroisopropyl | I | CF3 |
| 5-152 | 3-bromophenyl | H | Et | H | CN | H | H | Cl | F | heptafluoroisopropyl | F | CF3 |
| 5-153 | 4-bromophenyl | H | H | H | CN | H | H | Cl | F | heptafluoroisopropyl | Cl | CF3 |
| 5-154 | 2-iodophenyl | H | H | H | CN | H | H | Cl | F | heptafluoroisopropyl | Br | CF3 |
| 5-155 | 3-iodophenyl | Et | Et | H | CN | H | H | Cl | F | pentafluoroethyl | I | C2F5 |
| 5-156 | 4-iodophenyl | H | H | H | CN | H | H | Cl | Cl | pentafluoroethyl | F | CF3 |
| 5-157 | (2-trifluoromethyl)phenyl | H | H | H | CN | H | H | Cl | Cl | heptafluoroisopropyl | Cl | CF3 |
| 5-158 | (3-trifluoromethyl)phenyl | n-Pr | H | H | CN | H | H | Cl | Cl | heptafluoroisopropyl | Br | CF3 |
| 5-159 | (4-trifluoromethyl)phenyl | H | H | H | CN | H | H | Cl | Cl | heptafluoroisopropyl | I | n-C3F7 |
| 5-160 | 2-nitrophenyl | H | n-Pr | H | CN | H | H | Cl | Br | heptafluoroisopropyl | F | CF3 |
| 5-161 | 3-nitrophenyl | H | H | H | CN | H | H | Cl | Br | heptafluoroisopropyl | Cl | CF3 |
| 5-162 | 4-nitrophenyl | H | H | H | CN | H | H | Cl | Br | heptafluoroisopropyl | Br | CF3 |
| 5-163 | 2-cyanophenyl | n-Pr | n-Pr | H | CN | H | H | Cl | Br | pentafluoroethyl | I | CF3 |
| 5-164 | 3-cyanophenyl | H | H | H | CN | H | H | F | I | nonafluoro-2-butyl | F | CF3 |
| 5-165 | 4-cyanophenyl | H | H | H | CN | H | H | Cl | I | nonafluoro-2-butyl | Cl | CF3 |
| 5-166 | 2,6-difluorophenyl | H | H | H | CN | H | H | Cl | I | nonafluoro-2-butyl | Br | CF3 |
| 5-167 | 3,4-dichlorophenyl | H | H | H | CN | H | H | Cl | I | nonafluoro-2-butyl | I | CF3 |
| 5-168 | 2,4-dichlorophenyl | H | H | H | CN | H | H | Cl | Me | nonafluoro-2-butyl | H | CF3 |
| 5-169 | 2-chloro-4-fluorophenyl | H | H | H | CN | H | H | Cl | H | nonafluoro-2-butyl | Me | CF3 |
| 5-170 | 2-chloro-4,5-difluorophenyl | i-Pr | H | H | CN | H | H | Cl | Et | nonafluoro-2-butyl | H | CF3 |
| 5-171 | 4-bromo-2-chlorophenyl | H | H | H | CN | H | H | Cl | H | nonafluoro-2-butyl | Et | CF3 |
| 5-172 | 2-bromo-4-chlorophenyl | H | H | H | CN | H | H | Cl | n-Pr | nonafluoro-2-butyl | H | CF3 |
| 5-173 | 2-bromo-4-fluorophenyl | H | i-Pr | H | CN | H | H | Cl | H | nonafluoro-2-butyl | n-Pr | CF3 |
| 5-174 | 2-chloro-4-nitrophenyl | i-Pr | i-Pr | H | CN | H | H | Cl | i-Pr | nonafluoro-2-butyl | H | CF3 |
| 5-175 | 3,5-dicyanophenyl | H | H | H | CN | H | H | Cl | H | nonafluoro-2-butyl | i-Pr | CF3 |
| 5-176 | 4-cyano-2-fluorophenyl | H | H | H | CN | H | H | Cl | n-Bu | nonafluoro-2-butyl | H | CF3 |
| 5-177 | 2-chloro-4-cyanophenyl | H | H | H | CN | H | H | Cl | H | nonafluoro-2-butyl | n-Bu | CF3 |
| 5-178 | pyridin-3-yl | H | H | H | CN | H | H | Cl | i-Bu | nonafluoro-2-butyl | H | CF3 |
| 5-179 | 2-fluoropyridin-3-yl | H | H | H | CN | H | H | Cl | H | nonafluoro-2-butyl | i-Bu | CF3 |
| 5-180 | 2-chloropyridin-3-yl | H | H | H | CN | H | H | Cl | s-Bu | nonafluoro-2-butyl | H | C2F5 |
| 5-181 | 2-bromopyridin-3-yl | H | H | H | CN | H | H | Cl | H | nonafluoro-2-butyl | s-Bu | CF3 |
| 5-182 | 2-iodopyridin-3-yl | H | H | H | CN | H | H | Cl | Me | nonafluoro-2-butyl | Me | n-C3F7 |
| 5-183 | 2-(trifluoromethyl)pyridin-3-yl | H | H | H | CN | H | H | Cl | Me | nonafluoro-2-butyl | Et | i-C3F7 |
| 5-184 | 2-nitropyridin-3-yl | C(O)Me | H | H | CN | H | H | Cl | Me | nonafluoro-2-butyl | n-Pr | CF3 |
| 5-185 | 2-cyanopyridin-3-yl | H | H | H | CN | H | H | Cl | Me | nonafluoro-2-butyl | i-Pr | CF3 |
| 5-186 | 6-fluoropyridin-3-yl | H | H | H | CN | H | H | Cl | Me | nonafluoro-2-butyl | n-Bu | CF3 |
| 5-187 | 6-chloropyridin-3-yl | H | H | H | CN | H | H | Cl | Me | nonafluoro-2-butyl | i-Bu | CF3 |
| 5-188 | 6-bromopyridin-3-yl | H | C(O)Me | H | CN | H | H | Cl | Me | nonafluoro-2-butyl | s-Bu | CF3 |
| 5-189 | 6-iodopyridin-3-yl | H | H | H | CN | H | H | Cl | Et | nonafluoro-2-butyl | Me | n-C3F7 |
| 5-190 | 6-(trifluoromethyl)pyridin-3-yl | H | H | H | CN | H | H | Cl | Et | nonafluoro-2-butyl | Et | i-C3F7 |
| 5-191 | 6-nitropyridin-3-yl | C(O)Me | C(O)Me | H | CN | H | H | Cl | Et | nonafluoro-2-butyl | n-Pr | CF3 |
| 5-192 | 6-cyanopyridin-3-yl | H | H | H | CN | H | H | Cl | Et | nonafluoro-2-butyl | i-Pr | CF3 |
| 5-193 | phenyl | H | H | H | CN | H | H | Br | Et | heptafluoroisopropyl | n-Bu | CF3 |
| 5-194 | 5-fluoropyridin-3-yl | H | H | H | CN | H | H | Br | Et | heptafluoroisopropyl | i-Bu | CF3 |
| 5-195 | 5-chloropyridin-3-yl | H | H | H | CN | H | H | Br | Et | heptafluoroisopropyl | s-Bu | CF3 |
| 5-196 | 5-bromopyridin-3-yl | H | H | H | CN | H | H | Br | n-Pr | heptafluoroisopropyl | Me | CF3 |

**Table 5(2)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 5-197 | 5-iodopyridin-3-yl | H | H | H | CN | H | H | Br | n-Pr | heptafluoroisopropyl | Et | CF3 |
| 5-198 | 5-(trifluoromethyl)pyridin-3-yl | Me | Et | H | CN | H | H | Br | n-Pr | pentafluoroethyl | n-Pr | CF3 |
| 5-199 | 5-nitropyridin-3-yl | H | H | H | CN | H | H | Br | n-Pr | heptafluoroisopropyl | i-Pr | CF3 |
| 5-200 | 5-cyanopyridin-3-yl | H | H | H | CN | H | H | Br | n-Pr | pentafluoroethyl | n-Bu | CF3 |
| 5-201 | 4-fluoropyridin-3-yl | H | H | H | CN | H | H | Br | n-Pr | hectafluoroisopropyl | i-Bu | CF3 |
| 5-202 | 4-chloropyridin-3-yl | H | H | H | CN | H | H | Br | n-Pr | heptafluoroisopropyl | s-Bu | CF3 |
| 5-203 | 4-bromopyridin-3-yl | Me | n-Pr | H | CN | H | H | Br | i-Pr | heptafluoroisopropyl | Me | CF3 |
| 5-204 | 4-iodopyridin-3-yl | H | H | H | CN | H | H | Br | i-Pr | heptafluoroisopropyl | Et | CF3 |
| 5-205 | 4-(trifluoromethyl)pyridin-3-yl | H | H | H | CN | H | H | Br | i-Pr | hectafluoroisopropyl | n-Pr | CF3 |
| 5-206 | 4-nitropyridin-3-yl | H | H | H | CN | H | H | Br | i-Pr | hectafluoroisopropyl | i-Pr | CF3 |
| 5-207 | 4-cyanopyridin-3-yl | H | H | H | CN | H | H | Br | i-Pr | hectafluoroisopropyl | n-Bu | CF3 |
| 5-208 | 2,6-dichloropyridin-3-yl | H | H | H | CN | H | H | Br | i-Pr | heptafluoroisopropyl | i-Bu | CF3 |
| 5-209 | pyridin-3-yl N-oxide | Me | i-Pr | H | CN | H | H | Br | i-Pr | heptafluoroisopropyl | s-Bu | CF3 |
| 5-210 | pyridin-4-yl | H | H | H | CN | H | H | Br | n-Bu | heptafluoroisopropyl | Me | CF3 |
| 5-211 | 2-chloropyridin-4-yl | H | H | H | CN | H | H | Br | n-Bu | heptafluoroisopropyl | Et | CF3 |
| 5-212 | 3-bromopyridin-4-yl | H | H | H | CN | H | H | Br | n-Bu | heptafluoroisopropyl | n-Pr | CF3 |
| 5-213 | 3,5-dichloropyridin-4-yl | H | H | H | CN | H | H | Br | n-Bu | nonafluoro-2-butyl | i-Pr | CF3 |
| 5-214 | 3-(trifluommothyl)pyridin-4-yl | H | H | H | CN | H | H | Br | n-Bu | nonafluoro-2-butyl | n-Bu | CF3 |
| 5-215 | 2,6-dicyanopyridin-4-yl | H | H | H | CN | H | H | Br | n-Bu | nonafluoro-2-butyl | i-Bu | CF3 |
| 5-216 | pyridin-4-yl N-oxide | Me | C(O)Me | H | CN | H | H | Br | n-Bu | nonafluoro-2-butyl | s-Bu | CF3 |
| 5-217 | pyridin-2-yl | H | H | H | CN | H | H | Br | i-Bu | nonafluoro-2-butyl | Me | CF3 |
| 5-218 | 3-chloropyridin-2-yl | H | H | H | CN | H | H | Br | i-Bu | nonafluoro-2-butyl | Et | CF3 |
| 5-219 | 4-bromopyridin-2-yl | H | H | H | CN | H | H | Br | i-Bu | nonafluoro-2-butyl | n-Pr | CF3 |
| 5-220 | 5-iodopyridin-2-yl | H | H | H | CN | H | H | Br | i-Bu | nonafluoro-2-butyl | i-Pr | CF3 |
| 5-221 | 6-chloropyridin-2-yl | H | H | H | CN | H | H | Br | i-Bu | nonafluoro-2-butyl | n-Bu | CF3 |
| 5-222 | 4-cyanopyridin-2-yl | H | H | H | CN | H | H | Br | i-Bu | nonafluoro-2-butyl | i-Bu | CF3 |
| 5-223 | phenyl | H | H | H | CN | H | H | Br | i-Bu | nonafluoro-2-butyl | s-Bu | C2F5 |
| 5-224 | 2-fluorophenyl | H | H | H | CN | H | H | Br | s-Bu | nonafluoro-2-butyl | Me | CF3 |
| 5-225 | 3-fluorophenyl | H | H | H | CN | H | H | Br | s-Bu | nonafluoro-2-butyl | Et | CF3 |
| 5-226 | 4-fluorophenyl | H | H | H | CN | H | H | Br | s-Bu | nonafluoro-2-butyl | n-Pr | CF3 |
| 5-227 | 2-chlorophenyl | H | H | H | CN | H | H | Br | s-Bu | nonafluoro-2-butyl | i-Pr | CF3 |
| 5-228 | 3-chlorophenyl | H | H | H | CN | H | H | Br | s-Bu | nonafluoro-2-butyl | n-Bu | CF3 |
| 5-229 | 4-chlorophenyl | H | H | H | CN | H | H | Br | s-Bu | nonafluoro-2-butyl | i-Bu | CF3 |
| 5-230 | 2-bromophenyl | Et | Me | H | CN | H | H | Br | s-Bu | nonafluoro-2-butyl | s-Bu | CF3 |
| 5-231 | 3-bromophenyl | H | H | H | CN | H | H | Br | F | nonafluoro-2-butyl | Me | CF3 |
| 5-232 | 4-bromophenyl | H | H | H | CN | H | H | Br | F | nonafluoro-2-butyl | Et | CF3 |
| 5-233 | 2-iodophenyl | H | H | H | CN | H | H | Br | F | nonafluoro-2-butyl | n-Pr | CF3 |
| 5-234 | 3-iodophenyl | H | H | H | CN | H | H | Br | F | nonafluoro-2-butyl | i-Pr | CF3 |
| 5-235 | 4-iodophenyl | H | H | H | CN | H | H | Br | F | nonafluoro-2-butyl | n-Bu | CF3 |
| 5-236 | (2-trifluoromethyl)phenyl | H | H | H | CN | H | H | Br | F | nonafluoro-2-butyl | i-Bu | CF3 |
| 5-237 | (3-trifluoromethyl)phenyl | Et | n-Pr | H | CN | H | H | Br | F | nonafluoro-2-butyl | s-Bu | CF3 |
| 5-238 | (4-trifluoromethyl)phenyl | Et | i-Pr | H | CN | H | H | Br | Cl | nonafluoro-2-butyl | Me | CF3 |
| 5-239 | 2-nitrophenyl | H | H | H | CN | H | H | Br | Cl | nonafluoro-2-butyl | Et | CF3 |
| 5-240 | phenyl | H | H | H | CN | H | H | I | Cl | pentafluoroethyl | n-Pr | i-C3F7 |
| 5-241 | 3-nitrophenyl | H | H | H | CN | H | H | I | Cl | heptafluoroisopropyl | i-Pr | CF3 |
| 5-242 | 4-nitrophenyl | H | H | H | CN | H | H | I | Cl | heptafluoroisopropyl | n-Bu | CF3 |
| 5-243 | 2-cyanophenyl | Et | C(O)Me | H | CN | H | H | I | Cl | heptafluoroisopropyl | i-Bu | CF3 |
| 5-244 | 3-cyanophenyl | H | H | H | CN | H | H | I | Cl | heptafluoroisopropyl | s-Bu | CF3 |
| 5-245 | 4-cyanophenyl | H | H | H | CN | H | H | I | Br | heptafluoroisopropyl | Me | CF3 |
| 5-246 | 2,6-difluorophanyl | H | H | H | CN | H | H | I | Br | heptafluoroisopropyl | Et | CF3 |
| 5-247 | 3,4-dichlorophenyl | H | H | H | CN | H | H | I | Br | pentafluoroethyl | n-Pr | CF3 |
| 5-248 | 2,4-dichlorophanyl | H | H | H | CN | H | H | I | Br | heptafluoroisopropyl | i-Pr | CF3 |
| 5-249 | 2-chloro-4-fluorophenyl | H | H | H | CN | H | H | I | Br | heptafluoroisopropyl | n-Bu | CF3 |
| 5-250 | 2-chloro-4,5-difluorophenyl | H | H | H | CN | H | H | I | Br | heptafluoroisopropyl | i-Bu | CF3 |
| 5-251 | 4-bromo-2-chlorophenyl | H | H | H | CN | H | H | I | Br | heptafluoroisopropyl | s-Bu | CF3 |
| 5-252 | 2-bromo-4-chlorophenyl | n-Pr | Me | H | CN | H | H | I | I | heptafluoroisopropyl | Me | CF3 |
| 5-253 | 2-bromo-4-fluorophenyl | H | H | H | CN | H | H | I | I | heptafluoroisopropyl | Et | CF3 |
| 5-254 | 2-chloro-4-nitrophenyl | n-Pr | Et | H | CN | H | H | I | I | heptafluoroisopropyl | n-Pr | CF3 |
| 5-255 | 3,5-dicyanophenyl | H | H | H | CN | H | H | I | I | heptafluoroisopropyl | i-Pr | CF3 |
| 5-256 | 4-cyano-2-fluorophenyl | H | H | H | CN | H | H | I | I | heptafluoroisopropyl | n-Bu | CF3 |
| 5-257 | 2-chloro-4-cyanophenyl | H | H | H | CN | H | H | I | I | heptafluoroisopropyl | i-Bu | CF3 |
| 5-258 | pyridin-3-yl | H | H | H | CN | H | H | I | I | heptafluoroisopropyl | s-Bu | CF3 |
| 5-259 | 2-fluoropyridin-3-yl | H | H | H | CN | H | H | I | Me | heptafluoroisopropyl | F | C2F5 |
| 5-260 | 2-chloropyridin-3-yl | H | H | H | CN | H | H | I | Me | nonafluoro-2-butyl | Cl | CF3 |

**Table 5(3)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 5-261 | 2-bromopyridin-3-yl | H | H | H | CN | H | H | I | Me | nonafluoro-2-butyl | Br | n-C3F7 |
| 5-262 | 2-iodopyridin-3-yl | H | H | H | CN | H | H | I | Me | nonafluoro-2-butyl | I | i-C3F7 |
| 5-263 | 2-(trifluoromethyl)pyridin-3-yl | H | H | H | CN | H | H | I | Et | nonafluoro-2-butyl | F | CF3 |
| 5-264 | 2-nitropyridin-3-yl | H | H | H | CN | H | H | I | Et | nonafluoro-2-butyl | Cl | CF3 |
| 5-265 | 2-cyanopyridin-3-yl | H | H | H | CN | H | H | I | Et | nonafluoro-2-butyl | Br | CF3 |
| 5-266 | 6-fluoropyridin-3-yl | n-Pr | i-Pr | H | CN | H | H | I | Et | nonafluoro-2-butyl | I | C2F5 |
| 5-267 | 6-chloropyridin-3-yl | H | H | H | CN | H | H | I | n-Pr | nonafluoro-2-butyl | F | CF3 |
| 5-268 | 6-bromopyridin-3-yl | H | H | H | CN | H | H | I | n-Pr | nonafluoro-2-butyl | Cl | CF3 |
| 5-269 | 6-iodopyridin-3-yl | H | H | H | CN | H | H | I | n-Pr | nonafluoro-2-butyl | Br | CF3 |
| 5-270 | 6-(trifluoromethyl)pyridin-3-yl | H | H | H | CN | H | H | I | n-Pr | nonafluoro-2-butyl | I | n-C3F7 |
| 5-271 | 6-nitropyridin-3-yl | H | H | H | CN | H | H | I | i-Pr | nonafluoro-2-butyl | F | i-C3F7 |
| 5-272 | 6-cyanopyridin-3-yl | H | H | H | CN | H | H | I | i-Pr | nonafluoro-2-butyl | Cl | CF3 |
| 5-273 | 5-fluoropyridin-3-yl | n-Pr | C(O)Me | H | CN | H | H | I | i-Pr | nonafluoro-2-butyl | Br | CF3 |
| 5-274 | 5-chloropyridin-3-yl | H | H | H | CN | H | H | I | i-Pr | nonafluoro-2-butyl | I | CF3 |
| 5-275 | 5-bromopyridin-3-yl | H | H | H | CN | H | H | I | n-Bu | nonafluoro-2-butyl | F | CF3 |
| 5-276 | 5-iodopyridin-3-yl | H | H | H | CN | H | H | I | n-Bu | nonafluoro-2-butyl | Cl | CF3 |
| 5-277 | 5-(trifluoromethyl)pyridin-3-yl | H | H | H | CN | H | H | I | n-Bu | nonafluoro-2-butyl | Br | CF3 |
| 5-278 | 5-nitropyridin-3-yl | i-Pr | Me | H | CN | H | H | I | n-Bu | nonafluoro-2-butyl | I | CF3 |
| 5-279 | 5-cyanopyridin-3-yl | H | H | H | CN | H | H | I | i-Bu | nonafluoro-2-butyl | F | CF3 |
| 5-280 | 4-fluoropyridin-3-yl | H | H | H | CN | H | H | I | i-Bu | nonafluoro-2-butyl | Cl | CF3 |
| 5-281 | 4-chloropyridin-3-yl | H | H | H | CN | H | H | I | i-Bu | nonafluoro-2-butyl | Br | CF3 |
| 5-282 | 4-bromopyridin-3-yl | i-Pr | Et | H | CN | H | H | I | i-Bu | nonafluoro-2-butyl | I | CF3 |
| 5-283 | 4-iodopyridin-3-yl | i-Pr | n-Pr | H | CN | H | H | I | s-Bu | nonafluoro-2-butyl | F | CF3 |
| 5-284 | 4-(trifluoromethyl)pyridin-3-yl | H | H | H | CN | H | H | I | s-Bu | nonafluoro-2-butyl | Cl | CF3 |
| 5-285 | 4-nitropyridin-3-yl | i-Pr | C(O)Me | H | CN | H | H | I | s-Bu | nonafluoro-2-butyl | Br | CF3 |
| 5-286 | 4-cyanopyridin-3-yl | H | H | H | CN | H | H | I | s-Bu | nonafluoro-2-butyl | I | CF3 |
| 5-295 | 3-bromophenyl | H | Et | H | CN | H | H | OCF3 | F | heptafluoroisopropyl | F | OCF3 |
| 5-325 | 2-iodopyridin-3-yl | H | H | H | CN | H | H | OCF3 | Me | nonafluoro-2-butyl | Me | OCF3 |
| 5-438 | 3-bromophenyl | H | Et | H | CN | H | H | OCF3 | F | heptafluoroisopropyl | F | CF3 |
| 5-468 | 2-iodopyridin-3-yl | H | H | H | CN | H | H | OCF3 | Me | nonafluoro-2-butyl | Me | CF3 |
| 5-581 | 3-bromophenyl | H | Et | H | CN | H | H | CF3 | F | heptafluoroisopropyl | F | CF3 |
| 5-611 | 2-iodopyridin-3-yl | H | H | H | CN | H | H | CF3 | Me | nonafluoro-2-butyl | Me | CF3 |
| 5-724 | 3-bromophenyl | H | Et | F | CN | H | H | Cl | F | heptafluoroisopropyl | F | OCF3 |
| 5-754 | 2-iodopyridin-3-yl | H | H | F | CN | H | H | Cl | Me | nonafluoro-2-butyl | Me | OCF3 |
| 5-863 | 2-chlorophenyl | H | H | F | CN | H | H | F | Br | heptafluoroisopropyl | H | CF3 |
| 5-869 | 2-iodophenyl | H | H | F | CN | H | H | Cl | F | heptafluoroisopropyl | Br | CF3 |
| 5-876 | 3-nitrophenyl | H | H | F | CN | H | H | Cl | Br | heptafluoroisopropyl | Cl | CF3 |
| 5-883 | 2,4-dichlorophanyl | H | H | F | CN | H | H | Cl | Me | nonafluoro-2-butyl | H | CF3 |
| 5-884 | 2-chloro-4-fluorophenyl | H | H | F | CN | H | H | Cl | H | nonafluoro-2-butyl | Me | CF3 |
| 5-885 | 2-chloro-4,5-difluorophenyl | i-Pr | H | F | CN | H | H | Cl | Et | nonafluoro-2-butyl | H | CF3 |
| 5-886 | 4-bromo-2-chlorophenyl | H | H | F | CN | H | H | Cl | H | nonafluoro-2-butyl | Et | CF3 |
| 5-897 | 2-iodopyridin-3-yl | H | H | F | CN | H | H | Cl | Me | nonafluoro-2-butyl | Me | n-C3F7 |
| 5-898 | 2-(trifluoromethyl)pyridin-3-yl | H | H | F | CN | H | H | Cl | Me | nonafluoro-2-butyl | Et | i-C3F7 |
| 5-904 | 6-iodopyridin-3-yl | H | H | F | CN | H | H | Cl | Et | nonafluoro-2-butyl | Me | n-C3F7 |
| 5-905 | 6-(trifluoromethyl)pyridin-3-yl | H | H | F | CN | H | H | Cl | Et | nonafluoro-2-butyl | Et | i-C3F7 |
| 5-911 | 5-bromopyridin-3-yl | H | H | F | CN | H | H | Br | n-Pr | heptafluoroisopropyl | Me | CF3 |
| 5-912 | 5-iodopyridin-3-yl | H | H | F | CN | H | H | Br | n-Pr | heptafluoroisopropyl | Et | CF3 |
| 5-918 | 4-bromopyridin-3-yl | Me | n-Pr | F | CN | H | H | Br | i-Pr | heptafluoroisopropyl | Me | CF3 |
| 5-919 | 4-iodopyridin-3-yl | H | H | F | CN | H | H | Br | i-Pr | heptafluoroisopropyl | Et | CF3 |
| 5-925 | pyridin-4-yl | H | H | F | CN | H | H | Br | n-Bu | heptafluoroisopropyl | Me | CF3 |
| 5-926 | 2-chloropyridin-4-yl | H | H | F | CN | H | H | Br | n-Bu | heptafluoroisopropyl | Et | CF3 |
| 5-932 | pyridin-2-yl | H | H | F | CN | H | H | Br | i-Bu | nonafluoro-2-butyl | Me | CF3 |
| 5-933 | 3-chloropyridin-2-yl | H | H | F | CN | H | H | Br | i-Bu | nonafluoro-2-butyl | Et | CF3 |
| 5-939 | 2-fluorophenyl | H | H | F | CN | H | H | Br | s-Bu | nonafluoro-2-butyl | Me | CF3 |
| 5-940 | 3-fluorophenyl | H | H | F | CN | H | H | Br | s-Bu | nonafluoro-2-butyl | Et | CF3 |
| 5-946 | 3-bromophenyl | H | H | F | CN | H | H | Br | F | nonafluoro-2-butyl | Me | CF3 |
| 5-947 | 4-bromophenyl | H | H | F | CN | H | H | Br | F | nonafluoro-2-butyl | Et | CF3 |
| 5-953 | (4-trifluoromethyl)phenyl | Et | i-Pr | F | CN | H | H | Br | Cl | nonafluoro-2-butyl | Me | CF3 |
| 5-954 | 2-nitrophenyl | H | H | F | CN | H | H | Br | Cl | nonafluoro-2-butyl | Et | CF3 |
| 5-960 | 4-cyanophenyl | H | H | F | CN | H | H | I | Br | heptafluoroisopropyl | Me | CF3 |
| 5-961 | 2,6-difluorophanyl | H | H | F | CN | H | H | I | Br | heptafluoroisopropyl | Et | CF3 |
| 5-967 | 2-bromo-4-chlorophenyl | n-Pr | Me | F | CN | H | H | I | I | heptafluoroisopropyl | Me | CF3 |
| 5-968 | 2-bromo-4-fluorophenyl | H | H | F | CN | H | H | I | I | heptafluoroisopropyl | Et | CF3 |
| 5-974 | 2-fluoropyridin-3-yl | H | H | F | CN | H | H | I | Me | heptafluoroisopropyl | F | C2F5 |

**Table 5(4)**

| compound number | Q₁ | R₁ | R₂ | X₁ | X₂ | X₃ | X₄ | Y₁ | Y, | Y₃ | Y₄ | Y₅ |
|---|---|---|---|---|---|---|---|---|---|---|---|---|
| 5-975 | 2-chloropyridin-3-yl | H | H | F | CN | H | H | I | Me | nonafluoro-2-butyl | Cl | CF3 |
| 5-978 | 2-(trifluoromethyl)pyridin-3-yl | H | H | F | CN | H | H | I | Et | nonafluoro-2-butyl | F | CF3 |
| 5-979 | 2-nitropyridin-3-yl | H | H | F | CN | H | H | I | Et | nonafluoro-2-butyl | Cl | CF3 |
| 5-982 | 6-chloropyridin-3-yl | H | H | F | CN | H | H | I | n-Pr | nonafluoro-2-butyl | F | CF3 |
| 5-983 | 6-bromopyridin-3-yl | H | H | F | CN | H | H | I | n-Pr | nonafluoro-2-butyl | Cl | CF3 |
| 5-986 | 6-nitropyridin-3-yl | H | H | F | CN | H | H | I | i-Pr | nonafluoro-2-butyl | F | i-C3F7 |
| 5-987 | 6-cyanopyridin-3-yl | H | H | F | CN | H | H | I | i-Pr | nonafluoro-2-butyl | Cl | CF3 |
| 5-990 | 5-bromopyridin-3-yl | H | H | F | CN | H | H | I | n-Bu | nonafluoro-2-butyl | F | CF3 |
| 5-991 | 5-iodopyridin-3-yl | H | H | F | CN | H | H | I | n-Bu | nonafluoro-2-butyl | Cl | CF3 |
| 5-994 | 5-cyanopyridin-3-yl | H | H | F | CN | H | H | I | i-Bu | nonafluoro-2-butyl | F | CF3 |
| 5-995 | 4-fluoropyridin-3-yl | H | H | F | CN | H | H | I | i-Bu | nonafluoro-2-butyl | Cl | CF3 |
| 5-998 | 4-iodopyridin-3-yl | i-Pr | n-Pr | F | CN | H | H | I | s-Bu | nonafluoro-2-butyl | F | CF3 |
| 5-999 | 4-(trifluoromethyl)pyridin-3-yl | H | H | F | CN | H | H | I | s-Bu | nonafluoro-2-butyl | Cl | CF3 |
| 5-1010 | 3-bromophenyl | H | Et | F | CN | H | H | OCF3 | F | heptafluoroisopropyl | F | OCF3 |
| 5-1040 | 2-iodopyridin-3-yl | H | H | F | CN | H | H | OCF3 | Me | nonafluoro-2-butyl | Me | OCF3 |
| 5-1153 | 3-bromophenyl | H | Et | F | CN | H | H | OCF3 | F | heotafluoroisoorooyl | F | CF3 |
| 5-1183 | 2-iodopyridin-3-yl | H | H | F | CN | H | H | OCF3 | Me | nonafluoro-2-butyl | Me | CF3 |
| 5-1296 | 3-bromophenyl | H | Et | F | CN | H | H | CF3 | F | heptafluoroisopropyl | F | CF3 |
| 5-1326 | 2-iodopyridin-3-yl | H | H | F | CN | H | H | CF3 | Me | nonafluoro-2-butyl | Me | CF3 |
| 5-1439 | 3-bromophenyl | H | Et | F | CN | F | H | Cl | F | heptafluoroisopropyl | F | OCF3 |
| 5-1469 | 2-iodopyridin-3-yl | H | H | F | CN | F | H | Cl | Me | nonafluoro-2-butyl | Me | OCF3 |
| 5-1576 | 3-fluorophenyl | H | Me | F | CN | F | H | Cl | Cl | heptafluoroisopropyl | H | CF3 |
| 5-1583 | 4-bromophenyl | H | H | F | CN | F | H | Cl | F | heptafluoroisopropyl | Cl | CF3 |
| 5-1591 | 3-nitrophenyl | H | H | F | CN | F | H | Cl | Br | heptafluoroisopropyl | Cl | CF3 |
| 5-1598 | 2,4-dichlorophenyl | H | H | F | CN | F | H | Cl | Me | nonafluoro-2-butyl | H | CF3 |
| 5-1599 | 2-chloro-4-fluorophenyl | H | H | F | CN | F | H | Cl | H | nonafluoro-2-butyl | Me | CF3 |
| 5-1600 | 2-chloro-4,5-difluorophenyl | i-Pr | H | F | CN | F | H | Cl | Et | nonafluoro-2-butyl | H | CF3 |
| 5-1601 | 4-bromo-2-chlorophenyl | H | H | F | CN | F | H | Cl | H | nonafluoro-2-butyl | Et | CF3 |
| 5-1612 | 2-iodopyridin-3-yl | H | H | F | CN | F | H | Cl | Me | nonafluoro-2-butyl | Me | n-C3F7 |
| 5-1613 | 2-(trifluoromethyl)pyridin-3-yl | H | H | F | CN | F | H | Cl | Me | nonafluoro-2-butyl | Et | i-C3F7 |
| 5-1619 | 6-iodopyridin-3-yl | H | H | F | CN | F | H | Cl | Et | nonafluoro-2-butyl | Me | n-C3F7 |
| 5-1620 | 6- (trifluoromethyl) pyridin-3-yl | H | H | F | CN | F | H | Cl | Et | nonafluoro-2-butyl | Et | i-C3F7 |
| 5-1626 | 5-bromopyridin-3-yl | H | H | F | CN | F | H | Br | n-Pr | heptafluoroisopropyl | Me | CF3 |
| 5-1627 | 5-iodopyridin-3-yl | H | H | F | CN | F | H | Br | n-Pr | heptafluoroisopropyl | Et | CF3 |
| 5-1633 | 4-bromopyridin-3-yl | Me | n-Pr | F | CN | F | H | Br | i-Pr | heptafluoroisopropyl | Me | CF3 |
| 5-1634 | 4-iodopyridin-3-yl | H | H | F | CN | F | H | Br | i-Pr | heptafluoroisopropyl | Et | CF3 |
| 5-1640 | pyridin-4-yl | H | H | F | CN | F | H | Br | n-Bu | heptafluoroisopropyl | Me | CF3 |
| 5-1641 | 2-chloropyridin-4-yl | H | H | F | CN | F | H | Br | n-Bu | heptafluoroisopropyl | Et | CF3 |
| 5-1647 | pyridin-2-yl | H | H | F | CN | F | H | Br | i-Bu | nonafluoro-2-butyl | Me | CF3 |
| 5-1648 | 3-chloropyridin-2-yl | H | H | F | CN | F | H | Br | i-Bu | nonafluoro-2-butyl | Et | CF3 |
| 5-1654 | 2-fluorophenyl | H | H | F | CN | F | H | Br | s-Bu | nonafluoro-2-butyl | Me | CF3 |
| 5-1655 | 3-fluorophanyl | H | H | F | CN | F | H | Br | s-Bu | nonafluoro-2-butyl | Et | CF3 |
| 5-1661 | 3-bromophenyl | H | H | F | CN | F | H | Br | F | nonafluoro-2-butyl | Me | CF3 |
| 5-1662 | 4-bromophenyl | H | H | F | CN | F | H | Br | F | nonafluoro-2-butyl | Et | CF3 |
| 5-1668 | (4-trifluoromethyl)phenyl | Et | i-Pr | F | CN | F | H | Br | Cl | nonafluoro-2-butyl | Me | CF3 |
| 5-1669 | 2-nitrophenyl | H | H | F | CN | F | H | Br | Cl | nonafluoro-2-butyl | Et | CF3 |
| 5-1675 | 4-cyanophenyl | H | H | F | CN | F | H | I | Br | heptafluoroisopropyl | Me | CF3 |
| 5-1676 | 2,6-difluorophanyl | H | H | F | CN | F | H | I | Br | heptafluoroisopropyl | Et | CF3 |
| 5-1682 | 2-bromo-4-chlorophenyl | n-Pr | Me | F | CN | F | H | I | I | heptafluoroisopropyl | Me | CF3 |
| 5-1683 | 2-bromo-4-fluorophenyl | H | H | F | CN | F | H | I | I | heptafluoroisopropyl | Et | CF3 |
| 5-1689 | 2-fluoropyridin-3-yl | H | H | F | CN | F | H | I | Me | heptafluoroisopropyl | F | C2F5 |
| 5-1690 | 2-chloropyridin-3-yl | H | H | F | CN | F | H | I | Me | nonafluoro-2-butyl | Cl | CF3 |
| 5-1693 | 2- (trifluoromethyl) pyridin-3-yl | H | H | F | CN | F | H | I | Et | nonafluoro-2-butyl | F | CF3 |
| 5-1694 | 2-nitropyridin-3-yl | H | H | F | CN | F | H | I | Et | nonafluoro-2-butyl | Cl | CF3 |
| 5-1697 | 6-chloropyridin-3-yl | H | H | F | CN | F | H | I | n-Pr | nonafluoro-2-butyl | F | CF3 |
| 5-1698 | 6-bromopyridin-3-yl | H | H | F | CN | F | H | I | n-Pr | nonafluoro-2-butyl | Cl | CF3 |
| 5-1701 | 6-nitropyridin-3-yl | H | H | F | CN | F | H | I | i-Pr | nonafluoro-2-butyl | F | i-C3F7 |
| 5-1702 | 6-cyanopyridin-3-yl | H | H | F | CN | F | H | I | i-Pr | nonafluoro-2-butyl | Cl | CF3 |
| 5-1705 | 5-bromopyridin-3-yl | H | H | F | CN | F | H | I | n-Bu | nonafluoro-2-butyl | F | CF3 |
| 5-1706 | 5-iodopyridin-3-yl | H | H | F | CN | F | H | I | n-Bu | nonafluoro-2-butyl | Cl | CF3 |
| 5-1709 | 5-cyanopyridin-3-yl | H | H | F | CN | F | H | I | i-Bu | nonafluoro-2-butyl | F | CF3 |
| 5-1710 | 4-fluoropyridin-3-yl | H | H | F | CN | F | H | I | i-Bu | nonafluoro-2-butyl | Cl | CF3 |
| 5-1713 | 4-iodopyridin-3-yl | i-Pr | n-Pr | F | CN | F | H | I | s-Bu | nonafluoro-2-butyl | F | CF3 |
| 5-1714 | 4-(thfluoromethyl)pyridin-3-yl | H | H | F | CN | F | H | I | s-Bu | nonafluoro-2-butyl | Cl | CF3 |
| 5-1725 | 3-bromophenyl | H | Et | F | CN | F | H | OCF3 | F | heptafluoroisopropyl | F | OCF3 |
| 5-1755 | 2-iodopyridin-3-yl | H | H | F | CN | F | H | OCF3 | Me | nonafluoro-2-butyl | Me | OCF3 |
| 5-1868 | 3-bromophenyl | H | Et | F | CN | F | H | OCF3 | F | heptafluoroisopropyl | F | CF3 |
| 5-1898 | 2-iodopyridin-3-yl | H | H | F | CN | F | H | OCF3 | Me | nonafluoro-2-butyl | Me | CF3 |
| 5-2011 | 3-bromophenyl | H | Et | F | CN | F | H | CF3 | F | heptafluoroisopropyl | F | CF3 |
| 5-2041 | 2-iodopyridin-3-yl | H | H | F | CN | F | H | CF3 | Me | nonafluoro-2-butyl | Me | CF3 |

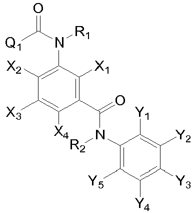

**Table 6(1)**

| compound number | Q₁ | R₁ | R₂ | X₃ | X₂ | X₃ | X₄ | Y₁ | Y₂ | Y₃ | Y₄ | Y₅ |
|---|---|---|---|---|---|---|---|---|---|---|---|---|
| 6-1 | phenyl | H | H | H | H | H | H | F | H | nonafluoro-2-butyl | H | F |
| 6-2 | 3-cyanophenyl | H | H | H | H | H | H | F | H | nonafluoro-2-butyl | H | F |
| 6-3 | 4-cyanophenyl | H | H | H | H | H | H | F | H | nonafluoro-2-butyl | H | F |
| 6-4 | 2-chloropyridin-3-yl | H | H | H | H | H | H | F | H | nonafluoro-2-butyl | H | F |
| 6-5 | phenyl | H | H | H | H | H | H | Cl | H | nonafluoro-2-butyl | H | Cl |
| 6-12 | 3-cyanophenyl | H | H | H | H | H | H | Cl | H | nonafluoro-2-butyl | H | Cl |
| 6-13 | 4-cyanophenyl | H | H | H | H | H | H | Cl | H | nonafluoro-2-butyl | H | Cl |
| 6-24 | 2-chloropyridin-3-yl | H | H | H | H | H | H | Cl | H | nonafluoro-2-butyl | H | Cl |
| 6-38 | phenyl | H | H | H | H | H | H | Br | H | nonafluoro-2-butyl | H | Br |
| 6-39 | 2-fluorophenyl | H | H | H | H | H | H | Br | H | nonafluoro-2-butyl | H | Br |
| 6-45 | 3-cyanophenyl | H | H | H | H | H | H | Br | H | nonafluoro-2-butyl | H | Br |
| 6-46 | 4-cyanophenyl | H | H | H | H | H | H | Br | H | nonafluoro-2-butyl | H | Br |
| 6-47 | 2,6- difluorophenyl | H | H | H | H | H | H | Br | H | nonafluoro-2-butyl | H | Br |
| 6-57 | 2-chloropyridin-3-yl | H | H | H | H | H | H | Br | H | nonafluoro-2-butyl | H | Br |
| 6-60 | 6-chloropyridin-3-yl | H | H | H | H | H | H | Br | H | nonafluoro-2-butyl | H | Br |
| 6-71 | phenyl | H | H | H | H | H | H | I | H | nonafluoro-2-butyl | H | I |
| 6-72 | 2-fluorophenyl | H | H | H | H | H | H | I | H | nonafluoro-2-butyl | H | I |
| 6-78 | 3-cyanophenyl | H | H | H | H | H | H | I | H | nonafluoro-2-butyl | H | I |
| 6-79 | 4-cyanophenyl | H | H | H | H | H | H | I | H | nonafluoro-2-butyl | H | I |
| 6-80 | 2,6- difluorophenyl | H | H | H | H | H | H | I | H | nonafluoro-2-butyl | H | I |
| 6-90 | 2-chloropyridin-3-yl | H | H | H | H | H | H | I | H | nonafluoro-2-butyl | H | I |
| 6-93 | 6-chloropyridin-3-yl | H | H | H | H | H | H | I | H | nonafluoro-2-butyl | H | I |
| 6-104 | phenyl | H | H | H | H | H | H | F | H | nonafluoro-2-butyl | H | Cl |
| 6-105 | phenyl | H | H | H | H | H | H | F | H | nonafluoro-2-butyl | H | Br |
| 6-106 | phenyl | H | H | H | H | H | H | F | H | nonafluoro-2-butyl | H | I |
| 6-107 | phenyl | H | H | H | H | H | H | Cl | H | nonafluoro-2-butyl | H | Br |
| 6-108 | phenyl | H | H | H | H | H | H | Cl | H | nonafluoro-2-butyl | H | I |
| 6-109 | phenyl | H | H | H | H | H | H | Br | H | nonafluoro-2-butyl | H | I |
| 6-110 | phenyl | H | H | H | H | H | H | F | H | heptafluoroisopropyl | H | CF3 |
| 6-111 | phenyl | H | H | H | H | H | H | Cl | H | heptafluoroisopropyl | H | CF3 |
| 6-131 | 3-cyanophenyl | H | H | H | H | H | H | Cl | H | heptafluoroisopropyl | H | CF3 |
| 6-132 | 4-cyanophenyl | H | H | H | H | H | H | Cl | H | heptafluoroisopropyl | H | CF3 |
| 6-147 | 2-chloropyridin-3-yl | H | H | H | H | H | H | Cl | H | heptafluoroisopropyl | H | CF3 |
| 6-189 | phenyl | H | H | H | H | H | H | F | H | nonafluoro-2-butyl | H | CF3 |
| 6-190 | phenyl | H | H | H | H | H | H | Cl | H | nonafluoro-2-butyl | H | CF3 |
| 6-210 | 3-cyanophenyl | H | H | H | H | H | H | Cl | H | nonafluoro-2-butyl | H | CF3 |
| 6-211 | 4-cyanophenyl | H | H | H | H | H | H | Cl | H | nonafluoro-2-butyl | H | CF3 |
| 6-226 | 2-chloropyridin-3-yl | H | H | H | H | H | H | Cl | H | nonafluoro-2-butyl | H | CF3 |
| 6-268 | phenyl | H | H | H | H | H | H | Br | H | heptafluoroisopropyl | H | CF3 |
| 6-269 | 2-fluorophenyl | H | H | H | H | H | H | Br | H | heptafluoroisopropyl | H | CF3 |
| 6-270 | 3-fluorophenyl | H | H | H | H | H | H | Br | H | heptafluoroisopropyl | H | CF3 |
| 6-271 | 4-fluorophenyl | H | H | H | H | H | H | Br | H | heptafluoroisopropyl | H | CF3 |
| 6-272 | 2-chlorophenyl | H | H | H | H | H | H | Br | H | heptafluoroisopropyl | H | CF3 |
| 6-288 | 3-cyanophenyl | H | H | H | H | H | H | Br | H | heptafluoroisopropyl | H | CF3 |
| 6-289 | 4-cyanophenyl | H | H | H | H | H | H | Br | H | heptafluoroisopropyl | H | CF3 |
| 6-290 | 2,6- difluorophenyl | H | H | H | H | H | H | Br | H | heptafluoroisopropyl | H | CF3 |
| 6-293 | 2-chloro-4-fluorophenyl | H | H | H | H | H | H | Br | H | heptafluoroisopropyl | H | CF3 |
| 6-300 | 4-cyano-2-fluorop he nyl | H | H | H | H | H | H | Br | H | heptafluoroisopropyl | H | CF3 |
| 6-304 | 2-chloropyridin-3-yl | H | H | H | H | H | H | Br | H | heptafluoroisopropyl | H | CF3 |
| 6-305 | 2-bromopyridin-3-yl | H | H | H | H | H | H | Br | H | heptafluoroisopropyl | H | CF3 |
| 6-306 | 2-iodopyridin-3-yl | H | H | H | H | H | H | Br | H | heptafluoroisopropyl | H | CF3 |
| 6-311 | 6-chloropyridin-3-yl | H | H | H | H | H | H | Br | H | heptafluoroisopropyl | H | CF3 |
| 6-346 | phenyl | H | H | H | H | H | H | Br | H | nonafluoro-2-butyl | H | CF3 |
| 6-347 | 2-fluorophenyl | H | H | H | H | H | H | Br | H | nonafluoro-2-butyl | H | CF3 |
| 6-348 | 3-fluorophenyl | H | H | H | H | H | H | Br | H | nonafluoro-2-butyl | H | CF3 |

**Table 6(2)**

| compound number | Q₁ | R₁ | R₂ | X₃ | X₂ | X₃ | X₄ | Y₁ | Y₂ | Y₃ | Y₄ | Y₅ |
|---|---|---|---|---|---|---|---|---|---|---|---|---|
| 6-349 | 4-fluorophenyl | H | H | H | H | H | H | Br | H | nonafluoro-2-butyl | H | CF3 |
| 6-366 | 3-cyanophenyl | H | H | H | H | H | H | Br | H | nonafluoro-2-butyl | H | CF3 |
| 6-367 | 4-cyanophenyl | H | H | H | H | H | H | Br | H | nonafluoro-2-butyl | H | CF3 |
| 6-368 | 2,6-difluorophenyl | H | H | H | H | H | H | Br | H | nonafluoro-2-butyl | H | CF3 |
| 6-382 | 2-chloropyridin-3-yl | H | H | H | H | H | H | Br | H | nonafluoro-2-butyl | H | CF3 |
| 6-389 | 6-chloropyridin-3-yl | H | H | H | H | H | H | Br | H | nonafluoro-2-butyl | H | CF3 |
| 6-424 | phenyl | H | H | H | H | H | H | I | H | heptafluoroisopropyl | H | CF3 |
| 6-425 | 2-fluorophenyl | H | H | H | H | H | H | I | H | heptafluoroisopropyl | H | CF3 |
| 6-426 | 3-fluorophenyl | H | H | H | H | H | H | I | H | heptafluoroisopropyl | H | CF3 |
| 6-427 | 4-fluorophenyl | H | H | H | H | H | H | I | H | heptafluoroisopropyl | H | CF3 |
| 6-428 | 2-chlorophenyl | H | H | H | H | H | H | I | H | heptafluoroisopropyl | H | CF3 |
| 6-444 | 3-cyanophenyl | H | H | H | H | H | H | I | H | heptafluoroisopropyl | H | CF3 |
| 6-445 | 4-cyanophenyl | H | H | H | H | H | H | I | H | heptafluoroisopropyl | H | CF3 |
| 6-446 | 2,6-difluorophenyl | H | H | H | H | H | H | I | H | heptafluoroisopropyl | H | CF3 |
| 6-448 | 2,4-dichlorophenyl | H | H | H | H | H | H | I | H | heptafluoroisopropyl | H | CF3 |
| 6-449 | 2-chloro-4-fluorophenyl | H | H | H | H | H | H | I | H | heptafluoroisopropyl | H | CF3 |
| 6-450 | 2-chloro-4,5-difluorophenyl | H | H | H | H | H | H | I | H | heptafluoroisopropyl | H | CF3 |
| 6-451 | 4-bromo-2-chlorophenyl | H | H | H | H | H | H | I | H | heptafluoroisopropyl | H | CF3 |
| 6-452 | 2-bromo-4-chlorophenyl | H | H | H | H | H | H | I | H | heptafluoroisopropyl | H | CF3 |
| 6-453 | 2-bromo-4-fluorophenyl | H | H | H | H | H | H | I | H | heptafluoroisopropyl | H | CF3 |
| 6-454 | 2-chloro-4-nitrophenyl | H | H | H | H | H | H | I | H | heptafluoroisopropyl | H | CF3 |
| 6-460 | 2-chloropyridin-3-yl | H | H | H | H | H | H | I | H | heptafluoroisopropyl | H | CF3 |
| 6-461 | 2-bromopyridin-3-yl | H | H | H | H | H | H | I | H | heptafluoroisopropyl | H | CF3 |
| 6-467 | 6-chloropyridin-3-yl | H | H | H | H | H | H | I | H | heptafluoroisopropyl | H | CF3 |
| 6-502 | phenyl | H | H | H | H | H | H | I | H | nonafluoro-2-butyl | H | CF3 |
| 6-503 | 2-fluorophenyl | H | H | H | H | H | H | I | H | nonafluoro-2-butyl | H | CF3 |
| 6-504 | 3-fluorophenyl | H | H | H | H | H | H | I | H | nonafluoro-2-butyl | H | CF3 |
| 6-505 | 4-fluorophenyl | H | H | H | H | H | H | I | H | nonafluoro-2-butyl | H | CF3 |
| 6-522 | 3-cyanophenyl | H | H | H | H | H | H | I | H | nonafluoro-2-butyl | H | CF3 |
| 6-523 | 4-cyanophenyl | H | H | H | H | H | H | I | H | nonafluoro-2-butyl | H | CF3 |
| 6-524 | 2,6-difluorophenyl | H | H | H | H | H | H | I | H | nonafluoro-2-butyl | H | CF3 |
| 6-538 | 2-chloropyridin-3-yl | H | H | H | H | H | H | I | H | nonafluoro-2-butyl | H | CF3 |
| 6-545 | 6-chloropyridin-3-yl | H | H | H | H | H | H | I | H | nonafluoro-2-butyl | H | CF3 |
| 6-580 | phenyl | H | H | H | H | H | H | OCF3 | H | heptafluoroisopropyl | H | OCF3 |
| 6-595 | 3-cyanophenyl | H | H | H | H | H | H | OCF3 | H | heptafluoroisopropyl | H | OCF3 |
| 6-596 | 4-cyanophenyl | H | H | H | H | H | H | OCF3 | H | heptafluoroisopropyl | H | OCF3 |
| 6-611 | 2-chloropyridin-3-yl | H | H | H | H | H | H | OCF3 | H | heptafluoroisopropyl | H | OCF3 |
| 6-636 | phenyl | H | H | H | H | H | H | OCF3 | H | nonafluoro-2-butyl | H | OCF3 |
| 6-651 | 3-cyanophenyl | H | H | H | H | H | H | OCF3 | H | nonafluoro-2-butyl | H | OCF3 |
| 6-652 | 4-cyanophenyl | H | H | H | H | H | H | OCF3 | H | nonafluoro-2-butyl | H | OCF3 |
| 6-667 | 2-chloropyridin-3-yl | H | H | H | H | H | H | OCF3 | H | nonafluoro-2-butyl | H | OCF3 |
| 6-692 | phenyl | H | H | H | H | H | H | CF3 | H | heptafluoroisopropyl | H | OCF3 |
| 6-707 | 3-cyanophenyl | H | H | H | H | H | H | CF3 | H | heptafluoroisopropyl | H | OCF3 |
| 6-708 | 4-cyanophenyl | H | H | H | H | H | H | CF3 | H | heptafluoroisopropyl | H | OCF3 |
| 6-723 | 2-chloropyridin-3-yl | H | H | H | H | H | H | CF3 | H | heptafluoroisopropyl | H | OCF3 |
| 6-748 | phenyl | H | H | H | H | H | H | CF3 | H | nonafluoro-2-butyl | H | OCF3 |
| 6-763 | 3-cyanophenyl | H | H | H | H | H | H | CF3 | H | nonafluoro-2-butyl | H | OCF3 |
| 6-764 | 4-cyanophenyl | H | H | H | H | H | H | CF3 | H | nonafluoro-2-butyl | H | OCF3 |
| 6-779 | 2-chloropyridin-3-yl | H | H | H | H | H | H | CF3 | H | nonafluoro-2-butyl | H | OCF3 |
| 6-804 | phenyl | H | H | H | H | H | H | CF3 | H | heptafluoroisopropyl | H | CF3 |
| 6-824 | 3-cyanophenyl | H | H | H | H | H | H | CF3 | H | heptafluoroisopropyl | H | CF3 |
| 6-825 | 4-cyanophenyl | H | H | H | H | H | H | CF3 | H | heptafluoroisopropyl | H | CF3 |
| 6-840 | 2-chloropyridin-3-yl | H | H | H | H | H | H | CF3 | H | heptafluoroisopropyl | H | CF3 |
| 6-841 | 2-bromopyridin-3-yl | H | H | H | H | H | H | CF3 | H | heptafluoroisopropyl | H | CF3 |
| 6-872 | phenyl | H | H | H | H | H | H | CF3 | H | nonafluoro-2-butyl | H | CF3 |
| 6-892 | 3-cyanophenyl | H | H | H | H | H | H | CF3 | H | nonafluoro-2-butyl | H | CF3 |
| 6-893 | 4-cyanophenyl | H | H | H | H | H | H | CF3 | H | nonafluoro-2-butyl | H | CF3 |
| 6-908 | 2-chloropyridin-3-yl | H | H | H | H | H | H | CF3 | H | nonafluoro-2-butyl | H | CF3 |
| 6-940 | phenyl | H | H | F | H | H | H | F | H | heptafluoroisopropyl | H | F |
| 6-941 | 3-cyanophenyl | H | H | F | H | H | H | F | H | heptafluoroisopropyl | H | F |
| 6-942 | 4-cyanophenyl | H | H | F | H | H | H | F | H | heptafluoroisopropyl | H | F |
| 6-943 | 2-chloropyridin-3-yl | H | H | F | H | H | H | F | H | heptafluoroisopropyl | H | F |
| 6-944 | phenyl | H | H | F | H | H | H | F | H | nonafluoro-2-butyl | H | F |
| 6-945 | 3-cyanophenyl | H | H | F | H | H | H | F | H | nonafluoro-2-butyl | H | F |

**Table 6(3)**

| compound number | Q₁ | R₁ | R₂ | X₃ | X₂ | X₃ | X₄ | Y₁ | Y₂ | Y₃ | Y₄ | Y₅ |
|---|---|---|---|---|---|---|---|---|---|---|---|---|
| 6-946 | 4-cyanophenyl | H | H | F | H | H | H | F | H | nonafluoro-2-butyl | H | F |
| 6-947 | 2-chloropyridin-3-yl | H | H | F | H | H | H | F | H | nonafluoro-2-butyl | H | F |
| 6-948 | phenyl | H | H | F | H | H | H | Cl | H | heptafluoroisopropyl | H | Cl |
| 6-952 | 2-chlorophenyl | H | H | F | H | H | H | F | H | heptafluoroisopropyl | H | Cl |
| 6-956 | 3-bromophenyl | H | H | F | H | H | H | F | H | heptafluoroisopropyl | H | Br |
| 6-961 | (2-trifluoromethyl) phenyl | H | H | F | H | H | H | F | H | heptafluoroisopropyl | H | I |
| 6-964 | 2-nitrophenyl | H | H | F | H | H | H | Cl | H | heptafluoroisopropyl | H | Br |
| 6-968 | 3-cyanophenyl | H | H | F | H | H | H | Cl | H | heptafluoroisopropyl | H | Cl |
| 6-969 | 4-cyanophenyl | H | H | F | H | H | H | Cl | H | heptafluoroisopropyl | H | Cl |
| 6-970 | 2,6-difluorophenyl | H | H | F | H | H | H | Cl | H | heptafluoroisopropyl | H | I |
| 6-982 | pyridin-3-yl | H | H | F | H | H | H | Br | H | heptafluoroisopropyl | H | I |
| 6-984 | 2-chloropyridin-3-yl | H | H | F | H | H | H | Cl | H | heptafluoroisopropyl | H | Cl |
| 6-1026 | phenyl | H | H | F | H | H | H | Cl | H | nonafluoro-2-butyl | H | Cl |
| 6-1046 | 3-cyanophenyl | H | H | F | H | H | H | Cl | H | nonafluoro-2-butyl | H | Cl |
| 6-1047 | 4-cyanophenyl | H | H | F | H | H | H | Cl | H | nonafluoro-2-butyl | H | Cl |
| 6-1062 | 2-chloropyridin-3-yl | H | H | F | H | H | H | Cl | H | nonafluoro-2-butyl | H | Cl |
| 6-1104 | phenyl | H | H | F | H | H | H | Br | H | heptafluoroisopropyl | H | Br |
| 6-1105 | 2-fluorophenyl | H | H | F | H | H | H | Br | H | heptafluoroisopropyl | H | Br |
| 6-1106 | 3-fluorophenyl | H | H | F | H | H | H | Br | H | heptafluoroisopropyl | H | Br |
| 6-1107 | 4-fluorophenyl | H | H | F | H | H | H | Br | H | heptafluoroisopropyl | H | Br |
| 6-1124 | 3-cyanophenyl | H | H | F | H | H | H | Br | H | heptafluoroisopropyl | H | Br |
| 6-1125 | 4-cyanophenyl | H | H | F | H | H | H | Br | H | heptafluoroisopropyl | H | Br |
| 6-1126 | 2,6-difluorophenyl | H | H | F | H | H | H | Br | H | heptafluoroisopropyl | H | Br |
| 6-1140 | 2-chloropyridin-3-yl | H | H | F | H | H | H | Br | H | heptafluoroisopropyl | H | Br |
| 6-1147 | 6-chloropyridin-3-yl | H | H | F | H | H | H | Br | H | heptafluoroisopropyl | H | Br |
| 6-1182 | phenyl | H | H | F | H | H | H | Br | H | nonafluoro-2-butyl | H | Br |
| 6-1183 | 2-fluorophenyl | H | H | F | H | H | H | Br | H | nonafluoro-2-butyl | H | Br |
| 6-1184 | 3-fluorophenyl | H | H | F | H | H | H | Br | H | nonafluoro-2-butyl | H | Br |
| 6-1185 | 4-fluorophenyl | H | H | F | H | H | H | Br | H | nonafluoro-2-butyl | H | Br |
| 6-1202 | 3-cyanophenyl | H | H | F | H | H | H | Br | H | nonafluoro-2-butyl | H | Br |
| 6-1203 | 4-cyanophenyl | H | H | F | H | H | H | Br | H | nonafluoro-2-butyl | H | Br |
| 6-1204 | 2,6-difluorophenyl | H | H | F | H | H | H | Br | H | nonafluoro-2-butyl | H | Br |
| 6-1218 | 2-chloropyridin-3-yl | H | H | F | H | H | H | Br | H | nonafluoro-2-butyl | H | Br |
| 6-1225 | 6-chloropyridin-3-yl | H | H | F | H | H | H | Br | H | nonafluoro-2-butyl | H | Br |
| 6-1260 | phenyl | H | H | F | H | H | H | I | H | heptafluoroisopropyl | H | I |
| 6-1261 | 2-fluorophenyl | H | H | F | H | H | H | I | H | heptafluoroisopropyl | H | I |
| 6-1262 | 3-fluorophenyl | H | H | F | H | H | H | I | H | heptafluoroisopropyl | H | I |
| 6-1263 | 4-fluorophenyl | H | H | F | H | H | H | I | H | heptafluoroisopropyl | H | I |
| 6-1280 | 3-cyanophenyl | H | H | F | H | H | H | I | H | heptafluoroisopropyl | H | I |
| 6-1281 | 4-cyanophenyl | H | H | F | H | H | H | I | H | heptafluoroisopropyl | H | I |
| 6-1282 | 2,6-difluorophenyl | H | H | F | H | H | H | I | H | heptafluoroisopropyl | H | I |
| 6-1296 | 2-chloropyridin-3-yl | H | H | F | H | H | H | I | H | heptafluoroisopropyl | H | I |
| 6-1303 | 6-chloropyridin-3-yl | H | H | F | H | H | H | I | H | heptafluoroisopropyl | H | I |
| 6-1338 | phenyl | H | H | F | H | H | H | I | H | nonafluoro-2-butyl | H | I |
| 6-1339 | 2-fluorophenyl | H | H | F | H | H | H | I | H | nonafluoro-2-butyl | H | I |
| 6-1340 | 3-fluorophenyl | H | H | F | H | H | H | I | H | nonafluoro-2-butyl | H | I |
| 6-1341 | 4-fluorophenyl | H | H | F | H | H | H | I | H | nonafluoro-2-butyl | H | I |
| 6-1358 | 3-cyanophenyl | H | H | F | H | H | H | I | H | nonafluoro-2-butyl | H | I |
| 6-1359 | 4-cyanophenyl | H | H | F | H | H | H | I | H | nonafluoro-2-butyl | H | I |
| 6-1360 | 2,6-difluorophenyl | H | H | F | H | H | H | I | H | nonafluoro-2-butyl | H | I |
| 6-1374 | 2-chloropyridin-3-yl | H | H | F | H | H | H | I | H | nonafluoro-2-butyl | H | I |
| 6-1381 | 6-chloropyridin-3-yl | H | H | F | H | H | H | I | H | nonafluoro-2-butyl | H | I |
| 6-1416 | phenyl | H | H | F | H | H | H | F | H | heptafluoroisopropyl | H | CF3 |
| 6-1417 | phenyl | H | H | F | H | H | H | Cl | H | heptafluoroisopropyl | H | CF3 |
| 6-1418 | 2-fluorophenyl | H | H | F | H | H | H | Cl | H | heptafluoroisopropyl | H | CF3 |
| 6-1422 | 3-chlorophenyl | H | H | F | H | H | H | Cl | H | heptafluoroisopropyl | H | CF3 |
| 6-1426 | 4-bromophenyl | H | H | F | H | H | H | Cl | H | heptafluoroisopropyl | H | CF3 |
| 6-1427 | 2-iodophenyl | H | H | F | H | H | H | Cl | H | pentafluoroethyl | H | CF3 |
| 6-1431 | (3-trifluoromethyl) phenyl | H | H | F | H | H | H | Cl | H | heptafluoroisopropyl | H | CF3 |
| 6-1435 | 4-nitrophenyl | H | H | F | H | H | H | Cl | H | heptafluoroisopropyl | H | CF3 |
| 6-1436 | 2-cyanophenyl | H | H | F | H | H | H | Cl | H | pentafluoroethyl | H | C2F5 |
| 6-1437 | 3-cyanophenyl | H | H | F | H | H | H | Cl | H | heptafluoroisopropyl | H | CF3 |
| 6-1438 | 4-cyanophenyl | H | H | F | H | H | H | Cl | H | heptafluoroisopropyl | H | CF3 |
| 6-1439 | 2,6-difluorophenyl | H | H | F | H | H | H | Cl | H | heptafluoroisopropyl | H | CF3 |

**Table 6(4)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 6-1440 | 3,4- dichlorophenyl | H | H | F | H | H | H | Cl | H | heptafluoroisopropyl | H | CF3 |
| 6-1441 | 2,4- dichlorophenyl | H | H | F | H | H | H | Cl | H | heptafluoroisopropyl | H | C2F5 |
| 6-1442 | 2-chloro-4-fluorophenyl | H | H | F | H | H | H | Cl | H | heptafluoroisopropyl | H | CF3 |
| 6-1445 | 2-bromo-4-chlorophenyl | H | H | F | H | H | H | Cl | H | heptafluoroisopropyl | H | n-C3F7 |
| 6-1446 | 2-bromo-4-fluorophenyl | H | H | F | H | H | H | Cl | H | heptafluoroisopropyl | H | CF3 |
| 6-1447 | 2-chloro-4-nitrophenyl | H | H | F | H | H | H | Cl | H | heptafluoroisopropyl | H | CF3 |
| 6-1448 | 3,5-dicyanophenyl | H | H | F | H | H | H | Cl | H | heptafluoroisopropyl | H | CF3 |
| 6-1449 | 4-cyano-2-fluorophenyl | H | H | F | H | H | H | Cl | H | heptafluoroisopropyl | H | CF3 |
| 6-1451 | pyridin-3-yl | H | H | F | H | H | H | Cl | H | heptafluoroisopropyl | H | CF3 |
| 6-1452 | 2-fluoropyridin-3-yl | H | H | F | H | H | H | Cl | H | pentafluoroethyl | H | CF3 |
| 6-1453 | 2-chloropyridin-3-yl | H | H | F | H | H | H | Cl | H | heptafluoroisopropyl | H | CF3 |
| 6-1456 | 2-(trifluoromethyl)pyridin-3-yl | H | H | F | H | H | H | Cl | H | heptafluoroisopropyl | H | CF3 |
| 6-1457 | 2-nitropyridin-3-yl | H | H | F | H | H | H | Cl | H | heptafluoroisopropyl | H | n-C3F7 |
| 6-1460 | 6-chloropyridin-3-yl | H | H | F | H | H | H | Cl | H | heptafluoroisopropyl | H | i-C3F7 |
| 6-1467 | 5-chloropyridin-3-yl | H | H | F | H | H | H | Cl | H | heptafluoroisopropyl | H | CF3 |
| 6-1474 | 4-chloropyridin-3-yl | H | H | F | H | H | H | Cl | H | heptafluoroisopropyl | H | CF3 |
| 6-1480 | 2,6-dichloropyridin-3-yl | H | H | F | H | H | H | Cl | H | heptafluoroisopropyl | H | CF3 |
| 6-1481 | pyridin-3-yl N-oxide | H | H | F | H | H | H | Cl | H | heptafluoroisopropyl | H | CF3 |
| 6-1482 | pyridin-4-yl | H | H | F | H | H | H | Cl | H | heptafluoroisopropyl | H | CF3 |
| 6-1483 | 2-chloropyridin-4-yl | H | H | F | H | H | H | Cl | H | heptafluoroisopropyl | H | CF3 |
| 6-1485 | 3,5-dichloropyridin-4-yl | H | H | F | H | H | H | Cl | H | heptafluoroisopropyl | H | CF3 |
| 6-1488 | pyridin-4-yl N-oxide | H | H | F | H | H | H | Cl | H | heptafluoroisopropyl | H | CF3 |
| 6-1489 | pyridin-2-yl | H | H | F | H | H | H | Cl | H | heptafluoroisopropyl | H | CF3 |
| 6-1490 | 3-chloropyridin-2-yl | H | H | F | H | H | H | Cl | H | heptafluoroisopropyl | H | CF3 |
| 6-1493 | 6-chloropyridin-2-yl | H | H | F | H | H | H | Cl | H | heptafluoroisopropyl | H | CF3 |
| 6-1495 | phenyl | H | H | F | H | H | H | F | H | nonafluoro-2-butyl | H | CF3 |
| 6-1496 | phenyl | H | H | F | H | H | H | Cl | H | nonafluoro-2-butyl | H | CF3 |
| 6-1498 | 3-fluorophenyl | H | H | F | H | H | H | Cl | H | nonafluoro-2-butyl | H | CF3 |
| 6-1502 | 4-chlorophenyl | H | H | F | H | H | H | Cl | H | nonafluoro-2-butyl | H | CF3 |
| 6-1503 | 2-bromophenyl | H | H | F | H | H | H | Cl | H | nonafluoro-2-butyl | H | CF3 |
| 6-1507 | 3-iodophenyl | H | H | F | H | H | H | Cl | H | nonafluoro-2-butyl | H | CF3 |
| 6-1511 | (4-trifluoromethyl)phenyl | H | H | F | H | H | H | Cl | H | nonafluoro-2-butyl | H | CF3 |
| 6-1512 | 2-nitrophenyl | H | H | F | H | H | H | Cl | H | nonafluoro-2-butyl | H | CF3 |
| 6-1516 | 3-cyanophenyl | H | H | F | H | H | H | Cl | H | nonafluoro-2-butyl | H | CF3 |
| 6-1517 | 4-cyanophenyl | H | H | F | H | H | H | Cl | H | nonafluoro-2-butyl | H | CF3 |
| 6-1518 | 2,6-difluorophenyl | H | H | F | H | H | H | Cl | H | nonafluoro-2-butyl | H | CF3 |
| 6-1520 | 2,4-dichlorophenyl | H | H | F | H | H | H | Cl | H | nonafluoro-2-butyl | H | CF3 |
| 6-1521 | 2-chloro-4-fluorophenyl | H | H | F | H | H | H | Cl | H | nonafluoro-2-butyl | H | CF3 |
| 6-1524 | 2-bromo-4-chlorophenyl | H | H | F | H | H | H | Cl | H | nonafluoro-2-butyl | H | CF3 |
| 6-1525 | 2-bromo-4-fluorophenyl | H | H | F | H | H | H | Cl | H | nonafluoro-2-butyl | H | CF3 |
| 6-1526 | 2-chloro-4-nitrophenyl | H | H | F | H | H | H | Cl | H | nonafluoro-2-butyl | H | i-C3F7 |
| 6-1528 | 4-cyano-2-fluorophenyl | H | H | F | H | H | H | Cl | H | nonafluoro-2-butyl | H | CF3 |
| 6-1529 | 2-chloro-4-cyanophenyl | H | H | F | H | H | H | Cl | H | nonafluoro-2-butyl | H | CF3 |
| 6-1530 | pyridin-3-yl | H | H | F | H | H | H | Cl | H | nonafluoro-2-butyl | H | CF3 |
| 6-1532 | 2-chloropyridin-3-yl | H | H | F | H | H | H | Cl | H | nonafluoro-2-butyl | H | CF3 |
| 6-1539 | 6-chloropyridin-3-yl | H | H | F | H | H | H | Cl | H | nonafluoro-2-butyl | H | CF3 |
| 6-1545 | 5-fluoropyridin-3-yl | H | H | F | H | H | H | Cl | H | nonafluoro-2-butyl | H | C2F5 |
| 6-1552 | 4-fluoropyridin-3-yl | H | H | F | H | H | H | Cl | H | nonafluoro-2-butyl | H | C2F5 |
| 6-1559 | 2,6-dichloropyridn-3-yl | H | H | F | H | H | H | Cl | H | nonafluoro-2-butyl | H | CF3 |
| 6-1560 | pyridin-3-yl N-oxide | H | H | F | H | H | H | Cl | H | nonafluoro-2-butyl | H | CF3 |
| 6-1561 | pyridin-4-yl | H | H | F | H | H | H | Cl | H | nonafluoro-2-butyl | H | CF3 |
| 6-1562 | 2-chloropyridin-4-yl | H | H | F | H | H | H | Cl | H | nonafluoro-2-butyl | H | CF3 |
| 6-1567 | pyridin-4-yl N-oxide | H | H | F | H | H | H | Cl | H | nonafluoro-2-butyl | H | CF3 |
| 6-1568 | pyridin-2-yl | H | H | F | H | H | H | Cl | H | nonafluoro-2-butyl | H | CF3 |
| 6-1569 | 3-chloropyridin-2-yl | H | H | F | H | H | H | Cl | H | nonafluoro-2-butyl | H | CF3 |
| 6-1572 | 6-chloropyridin-2-yl | H | H | F | H | H | H | Cl | H | nonafluoro-2-butyl | H | CF3 |
| 6-1574 | phenyl | H | H | F | H | H | H | Br | H | heptafluoroisopropyl | H | CF3 |
| 6-1575 | 2-fluorophenyl | H | H | F | H | H | H | Br | H | heptafluoroisopropyl | H | CF3 |
| 6-1576 | 3-fluorophenyl | H | H | F | H | H | H | Br | H | heptafluoroisopropyl | H | CF3 |
| 6-1577 | 4-fluorophenyl | H | H | F | H | H | H | Br | H | heptafluoroisopropyl | H | CF3 |
| 6-1578 | 2-chlorophenyl | H | H | F | H | H | H | Br | H | heptafluoroisopropyl | H | CF3 |
| 6-1579 | 3-chlorophenyl | H | H | F | H | H | H | Br | H | heptafluoroisopropyl | H | CF3 |
| 6-1580 | 4-chlorophenyl | H | H | F | H | H | H | Br | H | heptafluoroisopropyl | H | CF3 |
| 6-1581 | 2-bromophenyl | H | H | F | H | H | H | Br | H | heptafluoroisopropyl | H | CF3 |

**Table 6(5)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 6-1582 | 3-bromophenyl | H | H | F | H | H | H | Br | H | heptafluoroisopropyl | H | CF3 |
| 6-1583 | 4-bromophenyl | H | H | F | H | H | H | Br | H | heptafluoroisopropyl | H | CF3 |
| 6-1584 | 2-iodophenyl | H | H | F | H | H | H | Br | H | heptafluoroisopropyl | H | CF3 |
| 6-1585 | 3-iodophenyl | H | H | F | H | H | H | Br | H | heptafluoroisopropyl | H | CF3 |
| 6-1586 | 4-iodophenyl | H | H | F | H | H | H | Br | H | heptafluoroisopropyl | H | CF3 |
| 6-1587 | (2-trifluoromethyl) phenyl | H | H | F | H | H | H | Br | H | heptafluoroisopropyl | H | CF3 |
| 6-1588 | (3-trifluoromethyl)phenyl | H | H | F | H | H | H | Br | H | heptafluoroisopropyl | H | CF3 |
| 6-1589 | (4-trifluoromethyl)phenyl | H | H | F | H | H | H | Br | H | heptafluoroisopropyl | H | CF3 |
| 6-1590 | 2-nitrophenyl | H | H | F | H | H | H | Br | H | heptafluoroisopropyl | H | CF3 |
| 6-1591 | 3-nitrophenyl | H | H | F | H | H | H | Br | H | heptafluoroisopropyl | H | CF3 |
| 6-1592 | 4-nitrophenyl | H | H | F | H | H | H | Br | H | heptafluoroisopropyl | H | CF3 |
| 6-1593 | 2-cyanophenyl | H | H | F | H | H | H | Br | H | heptafluoroisopropyl | H | CF3 |
| 6-1594 | 3-cyanophenyl | H | H | F | H | H | H | Br | H | heptafluoroisopropyl | H | CF3 |
| 6-1595 | 4-cyanophenyl | H | H | F | H | H | H | Br | H | heptafluoroisopropyl | H | CF3 |
| 6-1596 | 2,6-difluorophenyl | H | H | F | H | H | H | Br | H | heptafluoroisopropyl | H | CF3 |
| 6-1597 | 3,4-dichlorophenyl | H | H | F | H | H | H | Br | H | heptafluoroisopropyl | H | CF3 |
| 6-1598 | 2,4-dichlorophenyl | H | H | F | H | H | H | Br | H | heptafluoroisopropyl | H | CF3 |
| 6-1599 | 2-chloro-4-fluorophenyl | H | H | F | H | H | H | Br | H | heptafluoroisopropyl | H | CF3 |
| 6-1600 | 2-chloro-4,5-difluorophenyl | H | H | F | H | H | H | Br | H | heptafluoroisopropyl | H | CF3 |
| 6-1601 | 4-bromo-2-chlorophenyl | H | H | F | H | H | H | Br | H | heptafluoroisopropyl | H | CF3 |
| 6-1602 | 2-bromo-4-chlorophenyl | H | H | F | H | H | H | Br | H | heptafluoroisopropyl | H | CF3 |
| 6-1603 | 2-bromo-4-fluorophenyl | H | H | F | H | H | H | Br | H | heptafluoroisopropyl | H | CF3 |
| 6-1604 | 2-chloro-4-nitrophenyl | H | H | F | H | H | H | Br | H | heptafluoroisopropyl | H | CF3 |
| 6-1605 | 3,5-dicyanophenyl | H | H | F | H | H | H | Br | H | heptafluoroisopropyl | H | CF3 |
| 6-1606 | 4-cyano-2-fluorophenyl | H | H | F | H | H | H | Br | H | heptafluoroisopropyl | H | CF3 |
| 6-1607 | 2-chloro-4-cyanophenyl | H | H | F | H | H | H | Br | H | heptafluoroisopropyl | H | CF3 |
| 6-1608 | pyridin-3-yl | H | H | F | H | H | H | Br | H | heptafluoroisopropyl | H | CF3 |
| 6-1609 | 2-fluoropyridin-3-yl | H | H | F | H | H | H | Br | H | heptafluoroisopropyl | H | CF3 |
| 6-1610 | 2-chloropyridin-3-yl | H | H | F | H | H | H | Br | H | heptafluoroisopropyl | H | CF3 |
| 6-1611 | 2-bromopyridin-3-yl | H | H | F | H | H | H | Br | H | heptafluoroisopropyl | H | CF3 |
| 6-1612 | 2-iodopyridin-3-yl | H | H | F | H | H | H | Br | H | heptafluoroisopropyl | H | CF3 |
| 6-1613 | 2-(trifluoromethyl)pyridin-3-yl | H | H | F | H | H | H | Br | H | heptafluoroisopropyl | H | CF3 |
| 6-1614 | 2-nitropyridin-3-yl | H | H | F | H | H | H | Br | H | heptafluoroisopropyl | H | CF3 |
| 6-1615 | 2-cyanopyridin-3-yl | H | H | F | H | H | H | Br | H | heptafluoroisopropyl | H | CF3 |
| 6-1616 | 6-fluoropyridin-3-yl | H | H | F | H | H | H | Br | H | heptafluoroisopropyl | H | CF3 |
| 6-1617 | 6-chloropyridin-3-yl | H | H | F | H | H | H | Br | H | heptafluoroisopropyl | H | CF3 |
| 6-1618 | 6-bromopyridin-3-yl | H | H | F | H | H | H | Br | H | heptafluoroisopropyl | H | CF3 |
| 6-1619 | 6-iodopyridin-3-yl | H | H | F | H | H | H | Br | H | heptafluoroisopropyl | H | CF3 |
| 6-1620 | 6-(trifluoromethyl)pyridin-3-yl | H | H | F | H | H | H | Br | H | heptafluoroisopropyl | H | CF3 |
| 6-1621 | 6-nitropyridin-3-yl | H | H | F | H | H | H | Br | H | heptafluoroisopropyl | H | CF3 |
| 6-1622 | 6-cyanopyridin-3-yl | H | H | F | H | H | H | Br | H | heptafluoroisopropyl | H | CF3 |
| 6-1623 | 5-fluoropyridin-3-yl | H | H | F | H | H | H | Br | H | heptafluoroisopropyl | H | CF3 |
| 6-1624 | 5-chloropyridin-3-yl | H | H | F | H | H | H | Br | H | heptafluoroisopropyl | H | CF3 |
| 6-1625 | 5-bromopyridin-3-yl | H | H | F | H | H | H | Br | H | heptafluoroisopropyl | H | CF3 |
| 6-1626 | 5-iodopyridin-3-yl | H | H | F | H | H | H | Br | H | heptafluoroisopropyl | H | CF3 |
| 6-1627 | 5-(trifluoromethyl)pyridin-3-yl | H | H | F | H | H | H | Br | H | heptafluoroisopropyl | H | CF3 |
| 6-1628 | 5-nitropyridin-3-yl | H | H | F | H | H | H | Br | H | heptafluoroisopropyl | H | CF3 |
| 6-1629 | 5-cyanopyridin-3-yl | H | H | F | H | H | H | Br | H | heptafluoroisopropyl | H | CF3 |
| 6-1630 | 4-fluoropyridin-3-yl | H | H | F | H | H | H | Br | H | heptafluoroisopropyl | H | CF3 |
| 6-1631 | 4-chloropyridin-3-yl | H | H | F | H | H | H | Br | H | heptafluoroisopropyl | H | CF3 |
| 6-1632 | 4-bromopyridin-3-yl | H | H | F | H | H | H | Br | H | heptafluoroisopropyl | H | CF3 |
| 6-1633 | 4-iodopyridin-3-yl | H | H | F | H | H | H | Br | H | heptafluoroisopropyl | H | CF3 |
| 6-1634 | 4-(trifluoromethyl)pyridin-3-yl | H | H | F | H | H | H | Br | H | heptafluoroisopropyl | H | CF3 |
| 6-1635 | 4-nitropyridin-3-yl | H | H | F | H | H | H | Br | H | heptafluoroisopropyl | H | CF3 |
| 6-1636 | 4-cyanopyridin-3-yl | H | H | F | H | H | H | Br | H | heptafluoroisopropyl | H | CF3 |
| 6-1637 | 2,6-dichloropyridin-3-yl | H | H | F | H | H | H | Br | H | heptafluoroisopropyl | H | CF3 |
| 6-1638 | pyridin-3-yl N-oxide | H | H | F | H | H | H | Br | H | heptafluoroisopropyl | H | CF3 |
| 6-1639 | pyridin-4-yl | H | H | F | H | H | H | Br | H | heptafluoroisopropyl | H | CF3 |
| 6-1640 | 2-chloropyridin-4-yl | H | H | F | H | H | H | Br | H | heptafluoroisopropyl | H | CF3 |
| 6-1641 | 3-bromopyridin-4-yl | H | H | F | H | H | H | Br | H | heptafluoroisopropyl | H | CF3 |
| 6-1642 | 3,5-dichloropyridin-4-yl | H | H | F | H | H | H | Br | H | heptafluoroisopropyl | H | CF3 |
| 6-1643 | 3-(trifluoromethyl)pyridin-4-yl | H | H | F | H | H | H | Br | H | heptafluoroisopropyl | H | CF3 |
| 6-1644 | 2,6-dicyanopyridin-4-yl | H | H | F | H | H | H | Br | H | heptafluoroisopropyl | H | CF3 |
| 6-1645 | pyridin-4-yl N-oxide | H | H | F | H | H | H | Br | H | heptafluoroisopropyl | H | CF3 |
| 6-1646 | pyridin-2-yl | H | H | F | H | H | H | Br | H | heptafluoroisopropyl | H | CF3 |

**Table 6(6)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 6-1647 | 3-chloropyridin-2-yl | H | H | F | H | H | H | Br | H | heptafluoroisopropyl | H | CF3 |
| 6-1648 | 4-bromopyridin-2-yl | H | H | F | H | H | H | Br | H | heptafluoroisopropyl | H | CF3 |
| 6-1649 | 5-iodopyridin-2-yl | H | H | F | H | H | H | Br | H | heptafluoroisopropyl | H | CF3 |
| 6-1650 | 6-chloropyridin-2-yl | H | H | F | H | H | H | Br | H | heptafluoroisopropyl | H | CF3 |
| 6-1651 | 4-cyanopyridin-2-yl | H | H | F | H | H | H | Br | H | heptafluoroisopropyl | H | CF3 |
| 6-1652 | phenyl | H | H | F | H | H | H | Br | H | nonafluoro-2-butyl | H | CF3 |
| 6-1653 | 2-fluorophenyl | H | H | F | H | H | H | Br | H | nonafluoro-2-butyl | H | CF3 |
| 6-1654 | 3-fluorophenyl | H | H | F | H | H | H | Br | H | nonafluoro-2-butyl | H | CF3 |
| 6-1655 | 4-fluorophenyl | H | H | F | H | H | H | Br | H | nonafluoro-2-butyl | H | CF3 |
| 6-1656 | 2-chlorophenyl | H | H | F | H | H | H | Br | H | nonafluoro-2-butyl | H | CF3 |
| 6-1657 | 3-chlorophenyl | H | H | F | H | H | H | Br | H | nonafluoro-2-butyl | H | CF3 |
| 6-1658 | 4-chlorophenyl | H | H | F | H | H | H | Br | H | nonafluoro-2-butyl | H | CF3 |
| 6-1659 | 2-bromophenyl | H | H | F | H | H | H | Br | H | nonafluoro-2-butyl | H | CF3 |
| 6-1660 | 3-bromophenyl | H | H | F | H | H | H | Br | H | nonafluoro-2-butyl | H | CF3 |
| 6-1661 | 4-bromophenyl | H | H | F | H | H | H | Br | H | nonafluoro-2-butyl | H | CF3 |
| 6-1662 | 2-iodophenyl | H | H | F | H | H | H | Br | H | nonafluoro-2-butyl | H | CF3 |
| 6-1663 | 3-iodophenyl | H | H | F | H | H | H | Br | H | nonafluoro-2-butyl | H | CF3 |
| 6-1664 | 4-iodophenyl | H | H | F | H | H | H | Br | H | nonafluoro-2-butyl | H | CF3 |
| 6-1665 | (2-trifluoromethyl)phenyl | H | H | F | H | H | H | Br | H | nonafluoro-2-butyl | H | CF3 |
| 6-1666 | (3-trifluoromethyl)phenyl | H | H | F | H | H | H | Br | H | nonafluoro-2-butyl | H | CF3 |
| 6-1667 | (4-trifluoromethyl)phenyl | H | H | F | H | H | H | Br | H | nonafluoro-2-butyl | H | CF3 |
| 6-1668 | 2-nitrophenyl | H | H | F | H | H | H | Br | H | nonafluoro-2-butyl | H | CF3 |
| 6-1669 | 3-nitrophenyl | H | H | F | H | H | H | Br | H | nonafluoro-2-butyl | H | CF3 |
| 6-1670 | 4-nitrophenyl | H | H | F | H | H | H | Br | H | nonafluoro-2-butyl | H | CF3 |
| 6-1671 | 2-cyanophenyl | H | H | F | H | H | H | Br | H | nonafluoro-2-butyl | H | CF3 |
| 6-1672 | 3-cyanophenyl | H | H | F | H | H | H | Br | H | nonafluoro-2-butyl | H | CF3 |
| 6-1673 | 4-cyanophenyl | H | H | F | H | H | H | Br | H | nonafluoro-2-butyl | H | CF3 |
| 6-1674 | 2,6-difluorophenyl | H | H | F | H | H | H | Br | H | nonafluoro-2-butyl | H | CF3 |
| 6-1675 | 3,4-dichlorophenyl | H | H | F | H | H | H | Br | H | nonafluoro-2-butyl | H | CF3 |
| 6-1676 | 2,4-dichlorophenyl | H | H | F | H | H | H | Br | H | nonafluoro-2-butyl | H | CF3 |
| 6-1677 | 2-chloro-4-fluorophenyl | H | H | F | H | H | H | Br | H | nonafluoro-2-butyl | H | CF3 |
| 6-1678 | 2-chloro-4,5-difluorophenyl | H | H | F | H | H | H | Br | H | nonafluoro-2-butyl | H | CF3 |
| 6-1679 | 4-bromo-2-chlorophenyl | H | H | F | H | H | H | Br | H | nonafluoro-2-butyl | H | CF3 |
| 6-1680 | 2-bromo-4-chlorophenyl | H | H | F | H | H | H | Br | H | nonafluoro-2-butyl | H | CF3 |
| 6-1681 | 2-bromo-4-fluorophenyl | H | H | F | H | H | H | Br | H | nonafluoro-2-butyl | H | CF3 |
| 6-1682 | 2-chloro-4-nitrophenyl | H | H | F | H | H | H | Br | H | nonafluoro-2-butyl | H | CF3 |
| 6-1683 | 3,5-dicyanophenyl | H | H | F | H | H | H | Br | H | nonafluoro-2-butyl | H | CF3 |
| 6-1684 | 4-cyano-2-fluorophenyl | H | H | F | H | H | H | Br | H | nonafluoro-2-butyl | H | CF3 |
| 6-1685 | 2-chloro-4-cyanophenyl | H | H | F | H | H | H | Br | H | nonafluoro-2-butyl | H | CF3 |
| 6-1686 | pyridin-3-yl | H | H | F | H | H | H | Br | H | nonafluoro-2-butyl | H | CF3 |
| 6-1687 | 2-fluoropyridin-3-yl | H | H | F | H | H | H | Br | H | nonafluoro-2-butyl | H | CF3 |
| 6-1688 | 2-chloropyridin-3-yl | H | H | F | H | H | H | Br | H | nonafluoro-2-butyl | H | CF3 |
| 6-1689 | 2-bromopyridin-3-yl | H | H | F | H | H | H | Br | H | nonafluoro-2-butyl | H | CF3 |
| 6-1690 | 2-iodopyridin-3-yl | H | H | F | H | H | H | Br | H | nonafluoro-2-butyl | H | CF3 |
| 6-1691 | 2-(trifluoromethyl)pyridin-3-yl | H | H | F | H | H | H | Br | H | nonafluoro-2-butyl | H | CF3 |
| 6-1692 | 2-nitropyridin-3-yl | H | H | F | H | H | H | Br | H | nonafluoro-2-butyl | H | CF3 |
| 6-1693 | 2-cyanopyridin-3-yl | H | H | F | H | H | H | Br | H | nonafluoro-2-butyl | H | CF3 |
| 6-1694 | 6-fluoropyridin-3-yl | H | H | F | H | H | H | Br | H | nonafluoro-2-butyl | H | CF3 |
| 6-1695 | 6-chloropyridin-3-yl | H | H | F | H | H | H | Br | H | nonafluoro-2-butyl | H | CF3 |
| 6-1696 | 6-bromopyridin-3-yl | H | H | F | H | H | H | Br | H | nonafluoro-2-butyl | H | CF3 |
| 6-1697 | 6-iodopyridin-3-yl | H | H | F | H | H | H | Br | H | nonafluoro-2-butyl | H | CF3 |
| 6-1698 | 6-(trifluoromethyl)pyridin-3-yl | H | H | F | H | H | H | Br | H | nonafluoro-2-butyl | H | CF3 |
| 6-1699 | 6-nitropyridin-3-yl | H | H | F | H | H | H | Br | H | nonafluoro-2-butyl | H | CF3 |
| 6-1700 | 6-cyanopyridin-3-yl | H | H | F | H | H | H | Br | H | nonafluoro-2-butyl | H | CF3 |
| 6-1701 | 5-fluoropyridin-3-yl | H | H | F | H | H | H | Br | H | nonafluoro-2-butyl | H | CF3 |
| 6-1702 | 5-chloropyridin-3-yl | H | H | F | H | H | H | Br | H | nonafluoro-2-butyl | H | CF3 |
| 6-1703 | 5-bromopyridin-3-yl | H | H | F | H | H | H | Br | H | nonafluoro-2-butyl | H | CF3 |
| 6-1704 | 5-iodopyridin-3-yl | H | H | F | H | H | H | Br | H | nonafluoro-2-butyl | H | CF3 |
| 6-1705 | 5-(trifluoromethyl)pyridin-3-yl | H | H | F | H | H | H | Br | H | nonafluoro-2-butyl | H | CF3 |
| 6-1706 | 5-nitropyridin-3-yl | H | H | F | H | H | H | Br | H | nonafluoro-2-butyl | H | CF3 |
| 6-1707 | 5-cyanopyridin-3-yl | H | H | F | H | H | H | Br | H | nonafluoro-2-butyl | H | CF3 |
| 6-1708 | 4-fluoropyridin-3-yl | H | H | F | H | H | H | Br | H | nonafluoro-2-butyl | H | CF3 |
| 6-1709 | 4-chloropyridin-3-yl | H | H | F | H | H | H | Br | H | nonafluoro-2-butyl | H | CF3 |

**Table 6(7)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 6-1710 | 4-bromopyridin-3-yl | H | H | F | H | H | H | Br | H | nonafluoro-2-butyl | H | CF3 |
| 6-1711 | 4-iodopyridin-3-yl | H | H | F | H | H | H | Br | H | nonafluoro-2-butyl | H | CF3 |
| 6-1712 | 4-(trifluoromethyl)pyridin-3-yl | H | H | F | H | H | H | Br | H | nonafluoro-2-butyl | H | CF3 |
| 6-1713 | 4-nitropyridin-3-yl | H | H | F | H | H | H | Br | H | nonafluoro-2-butyl | H | CF3 |
| 6-1714 | 4-cyanopyridin-3-yl | H | H | F | H | H | H | Br | H | nonafluoro-2-butyl | H | CF3 |
| 6-1715 | 2,6-dichloropyridin-3-yl | H | H | F | H | H | H | Br | H | nonafluoro-2-butyl | H | CF3 |
| 6-1716 | pyridin-3-yl N-oxide | H | H | F | H | H | H | Br | H | nonafluoro-2-butyl | H | CF3 |
| 6-1717 | pyridin-4-yl | H | H | F | H | H | H | Br | H | nonafluoro-2-butyl | H | CF3 |
| 6-1718 | 2-chloropyridin-4-yl | H | H | F | H | H | H | Br | H | nonafluoro-2-butyl | H | CF3 |
| 6-1719 | 3-bromopyridin-4-yl | H | H | F | H | H | H | Br | H | nonafluoro-2-butyl | H | CF3 |
| 6-1720 | 3,5-dichloropyridin-4-yl | H | H | F | H | H | H | Br | H | nonafluoro-2-butyl | H | CF3 |
| 6-1721 | 3-(trifluoromethyl)pyridin-4-yl | H | H | F | H | H | H | Br | H | nonafluoro-2-butyl | H | CF3 |
| 6-1722 | 2,6-dicyanopyridin-4-yl | H | H | F | H | H | H | Br | H | nonafluoro-2-butyl | H | CF3 |
| 6-1723 | pyridin-4-yl N-oxide | H | H | F | H | H | H | Br | H | nonafluoro-2-butyl | H | CF3 |
| 6-1724 | pyridin-2-yl | H | H | F | H | H | H | Br | H | nonafluoro-2-butyl | H | CF3 |
| 6-1725 | 3-chloropyridin-2-yl | H | H | F | H | H | H | Br | H | nonafluoro-2-butyl | H | CF3 |
| 6-1726 | 4-bromopyridin-2-yl | H | H | F | H | H | H | Br | H | nonafluoro-2-butyl | H | CF3 |
| 6-1727 | 5-iodopyridin-2-yl | H | H | F | H | H | H | Br | H | nonafluoro-2-butyl | H | CF3 |
| 6-1728 | 6-chloropyridin-2-yl | H | H | F | H | H | H | Br | H | nonafluoro-2-butyl | H | CF3 |
| 6-1729 | 4-cyanopyridin-2-yl | H | H | F | H | H | H | Br | H | nonafluoro-2-butyl | H | CF3 |
| 6-1730 | phenyl | H | H | F | H | H | H | I | H | heptafluoroisopropyl | H | CF3 |
| 6-1731 | 2-fluorophenyl | H | H | F | H | H | H | I | H | heptafluoroisopropyl | H | CF3 |
| 6-1732 | 3-fluorophenyl | H | H | F | H | H | H | I | H | heptafluoroisopropyl | H | CF3 |
| 6-1733 | 4-fluorophenyl | H | H | F | H | H | H | I | H | heptafluoroisopropyl | H | CF3 |
| 6-1734 | 2-chlorophenyl | H | H | F | H | H | H | I | H | heptafluoroisopropyl | H | CF3 |
| 6-1735 | 3-chlorophenyl | H | H | F | H | H | H | I | H | heptafluoroisopropyl | H | CF3 |
| 6-1736 | 4-chlorophenyl | H | H | F | H | H | H | I | H | heptafluoroisopropyl | H | CF3 |
| 6-1737 | 2-bromophenyl | H | H | F | H | H | H | I | H | heptafluoroisopropyl | H | CF3 |
| 6-1738 | 3-bromophenyl | H | H | F | H | H | H | I | H | heptafluoroisopropyl | H | CF3 |
| 6-1739 | 4-bromophenyl | H | H | F | H | H | H | I | H | heptafluoroisopropyl | H | CF3 |
| 6-1740 | 2-iodophenyl | H | H | F | H | H | H | I | H | heptafluoroisopropyl | H | CF3 |
| 6-1741 | 3-iodophenyl | H | H | F | H | H | H | I | H | heptafluoroisopropyl | H | CF3 |
| 6-1742 | 4-iodophenyl | H | H | F | H | H | H | I | H | heptafluoroisopropyl | H | CF3 |
| 6-1743 | (2-trifluoromethyl)phenyl | H | H | F | H | H | H | I | H | heptafluoroisopropyl | H | CF3 |
| 6-1744 | (3-trifluoromethyl)phenyl | H | H | F | H | H | H | I | H | heptafluoroisopropyl | H | CF3 |
| 6-1745 | (4-trifluoromethyl)phenyl | H | H | F | H | H | H | I | H | heptafluoroisopropyl | H | CF3 |
| 6-1746 | 2-nitrophenyl | H | H | F | H | H | H | I | H | heptafluoroisopropyl | H | CF3 |
| 6-1747 | 3-nitrophenyl | H | H | F | H | H | H | I | H | heptafluoroisopropyl | H | CF3 |
| 6-1748 | 4-nitrophenyl | H | H | F | H | H | H | I | H | heptafluoroisopropyl | H | CF3 |
| 6-1749 | 2-cyanophenyl | H | H | F | H | H | H | I | H | heptafluoroisopropyl | H | CF3 |
| 6-1750 | 3-cyanophenyl | H | H | F | H | H | H | I | H | heptafluoroisopropyl | H | CF3 |
| 6-1751 | 4-cyanophenyl | H | H | F | H | H | H | I | H | heptafluoroisopropyl | H | CF3 |
| 6-1752 | 2,6-difluorophenyl | H | H | F | H | H | H | I | H | heptafluoroisopropyl | H | CF3 |
| 6-1753 | 3,4-dichlorophenyl | H | H | F | H | H | H | I | H | heptafluoroisopropyl | H | CF3 |
| 6-1754 | 2,4-dichlorophenyl | H | H | F | H | H | H | I | H | heptafluoroisopropyl | H | CF3 |
| 6-1755 | 2-chloro-4-fluorophenyl | H | H | F | H | H | H | I | H | heptafluoroisopropyl | H | CF3 |
| 6-1756 | 2-chloro-4,5-difluorophenyl | H | H | F | H | H | H | I | H | heptafluoroisopropyl | H | CF3 |
| 6-1757 | 4-bromo-2-chlorophenyl | H | H | F | H | H | H | I | H | heptafluoroisopropyl | H | CF3 |
| 6-1758 | 2-bromo-4-chlorophenyl | H | H | F | H | H | H | I | H | heptafluoroisopropyl | H | CF3 |
| 6-1759 | 2-bromo-4-fluorophenyl | H | H | F | H | H | H | I | H | heptafluoroisopropyl | H | CF3 |
| 6-1760 | 2-chloro-4-nitrophenyl | H | H | F | H | H | H | I | H | heptafluoroisopropyl | H | CF3 |
| 6-1761 | 3,5-dicyanophenyl | H | H | F | H | H | H | I | H | heptafluoroisopropyl | H | CF3 |
| 6-1762 | 4-cyano-2-fluorophenyl | H | H | F | H | H | H | I | H | heptafluoroisopropyl | H | CF3 |
| 6-1763 | 2-chloro-4-cyanophenyl | H | H | F | H | H | H | I | H | heptafluoroisopropyl | H | CF3 |
| 6-1764 | pyridin-3-yl | H | H | F | H | H | H | I | H | heptafluoroisopropyl | H | CF3 |
| 6-1765 | 2-fluoropyridin-3-yl | H | H | F | H | H | H | I | H | heptafluoroisopropyl | H | CF3 |
| 6-1766 | 2-chloropyridin-3-yl | H | H | F | H | H | H | I | H | heptafluoroisopropyl | H | CF3 |
| 6-1767 | 2-bromopyridin-3-yl | H | H | F | H | H | H | I | H | heptafluoroisopropyl | H | CF3 |
| 6-1768 | 2-iodopyridin-3-yl | H | H | F | H | H | H | I | H | heptafluoroisopropyl | H | CF3 |
| 6-1769 | 2-(trifluoromethyl)pyridin-3-yl | H | H | F | H | H | H | I | H | heptafluoroisopropyl | H | CF3 |
| 6-1770 | 2-nitropyridin-3-yl | H | H | F | H | H | H | I | H | heptafluoroisopropyl | H | CF3 |
| 6-1771 | 2-cyanopyridin-3-yl | H | H | F | H | H | H | I | H | heptafluoroisopropyl | H | CF3 |
| 6-1772 | 6-fluoropyridin-3-yl | H | H | F | H | H | H | I | H | heptafluoroisopropyl | H | CF3 |
| 6-1773 | 6-chloropyridin-3-yl | H | H | F | H | H | H | I | H | heptafluoroisopropyl | H | CF3 |
| 6-1774 | 6-bromopyridin-3-yl | H | H | F | H | H | H | I | H | heptafluoroisopropyl | H | CF3 |

**Table 6(8)**

| compound number | Q₁ | R₁ | R₂ | X₁ | X₂ | X₃ | ×₄ | Y₁ | Y₂ | Y₃ | Y₄ | Y₅ |
|---|---|---|---|---|---|---|---|---|---|---|---|---|
| 6-1775 | 6-iodopyridin-3-yl | H | H | F | H | H | H | I | H | heptafluoroisopropyl | H | CF3 |
| 6-1776 | 6-(trifluoromethyl)pyridin-3-yl | H | H | F | H | H | H | I | H | heptafluoroisopropyl | H | CF3 |
| 6-1777 | 6-nitropyridin-3-yl | H | H | F | H | H | H | I | H | heptafluoroisopropyl | H | CF3 |
| 6-1778 | 6-cyanopyridin-3-yl | H | H | F | H | H | H | I | H | heptafluoroisopropyl | H | CF3 |
| 6-1779 | 5-fluoropyridin-3-yl | H | H | F | H | H | H | I | H | heptafluoroisopropyl | H | CF3 |
| 6-1780 | 5-chloropyridin-3-yl | H | H | F | H | H | H | I | H | heptafluoroisopropyl | H | CF3 |
| 6-1781 | 5-bromopyridin-3-yl | H | H | F | H | H | H | I | H | heptafluoroisopropyl | H | CF3 |
| 6-1782 | 5-iodopyridin-3-yl | H | H | F | H | H | H | I | H | heptafluoroisopropyl | H | CF3 |
| 6-1783 | 5-(trifluoromethyl)pyridin-3-yl | H | H | F | H | H | H | I | H | heptafluoroisopropyl | H | CF3 |
| 6-1784 | 5-nitropyridin-3-yl | H | H | F | H | H | H | I | H | heptafluoroisopropyl | H | CF3 |
| 6-1785 | 5-cyanopyridin-3-yl | H | H | F | H | H | H | I | H | heptafluoroisopropyl | H | CF3 |
| 6-1786 | 4-fluoropyridin-3-yl | H | H | F | H | H | H | I | H | heptafluoroisopropyl | H | CF3 |
| 6-1787 | 4-chloropyridin-3-yl | H | H | F | H | H | H | I | H | heptafluoroisopropyl | H | CF3 |
| 6-1788 | 4-bromopyridin-3-yl | H | H | F | H | H | H | I | H | heptafluoroisopropyl | H | CF3 |
| 6-1789 | 4-iodopyridin-3-yl | H | H | F | H | H | H | I | H | heptafluoroisopropyl | H | CF3 |
| 6-1790 | 4-(trifluoromethyl)pyridin-3-yl | H | H | F | H | H | H | I | H | heptafluoroisopropyl | H | CF3 |
| 6-1791 | 4-nitropyridin-3-yl | H | H | F | H | H | H | I | H | heptafluoroisopropyl | H | CF3 |
| 6-1792 | 4-cyanopyridin-3-yl | H | H | F | H | H | H | I | H | heptafluoroisopropyl | H | CF3 |
| 6-1793 | 2,6-diehloropyridin-3-yl | H | H | F | H | H | H | I | H | heptafluoroisopropyl | H | CF3 |
| 6-1794 | pyridin-3-yl N-oxide | H | H | F | H | H | H | I | H | heptafluoroisopropyl | H | CF3 |
| 6-1795 | pyridin-4-yl | H | H | F | H | H | H | I | H | heptafluoroisopropyl | H | CF3 |
| 6-1796 | 2-chloropyridin-4-yl | H | H | F | H | H | H | I | H | heptafluoroisopropyl | H | CF3 |
| 6-1797 | 3-bromopyridin-4-yl | H | H | F | H | H | H | I | H | heptafluoroisopropyl | H | CF3 |
| 6-1798 | 3,5-dichloropyridin-4-yl | H | H | F | H | H | H | I | H | heptafluoroisopropyl | H | CF3 |
| 6-1799 | 3-(trifluoromethyl)pyridin-4-yl | H | H | F | H | H | H | I | H | heptafluoroisopropyl | H | CF3 |
| 6-1800 | 2,6-dicyanopyridin-4-yl | H | H | F | H | H | H | I | H | heptafluoroisopropyl | H | CF3 |
| 6-1801 | pyridin-4-yl N-oxide | H | H | F | H | H | H | I | H | heptafluoroisopropyl | H | CF3 |
| 6-1802 | pyridin-2-yl | H | H | F | H | H | H | I | H | heptafluoroisopropyl | H | CF3 |
| 6-1803 | 3-chloropyridin-2-yl | H | H | F | H | H | H | I | H | heptafluoroisopropyl | H | CF3 |
| 6-1804 | 4-bromopyridin-2-yl | H | H | F | H | H | H | I | H | heptafluoroisopropyl | H | CF3 |
| 6-1805 | 5-iodopyridin-2-yl | H | H | F | H | H | H | I | H | heptafluoroisopropyl | H | CF3 |
| 6-1806 | 6-chloropyridin-2-yl | H | H | F | H | H | H | I | H | heptafluoroisopropyl | H | CF3 |
| 6-1807 | 4-cyanopyridin-2-yl | H | H | F | H | H | H | I | H | heptafluoroisopropyl | H | CF3 |
| 6-1808 | phenyl | H | H | F | H | H | H | I | H | nonafluoro-2-butyl | H | CF3 |
| 6-1809 | 2-fluorophenyl | H | H | F | H | H | H | I | H | nonafluoro-2-butyl | H | CF3 |
| 6-1810 | 3-fluorophenyl | H | H | F | H | H | H | I | H | nonafluoro-2-butyl | H | CF3 |
| 6-1811 | 4-fluorophenyl | H | H | F | H | H | H | I | H | nonafluoro-2-butyl | H | CF3 |
| 6-1812 | 2-chlorophenyl | H | H | F | H | H | H | I | H | nonafluoro-2-butyl | H | CF3 |
| 6-1813 | 3-chlorophenyl | H | H | F | H | H | H | I | H | nonafluoro-2-butyl | H | CF3 |
| 6-1814 | 4-chlorophenyl | H | H | F | H | H | H | I | H | nonafluoro-2-butyl | H | CF3 |
| 6-1815 | 2-bromophenyl | H | H | F | H | H | H | I | H | nonafluoro-2-butyl | H | CF3 |
| 6-1816 | 3-bromophenyl | H | H | F | H | H | H | I | H | nonafluoro-2-butyl | H | CF3 |
| 6-1817 | 4-bromophenyl | H | H | F | H | H | H | I | H | nonafluoro-2-butyl | H | CF3 |
| 6-1818 | 2-iodophenyl | H | H | F | H | H | H | I | H | nonafluoro-2-butyl | H | CF3 |
| 6-1819 | 3-iodophenyl | H | H | F | H | H | H | I | H | nonafluoro-2-butyl | H | CF3 |
| 6-1820 | 4-iodophenyl | H | H | F | H | H | H | I | H | nonafluoro-2-butyl | H | CF3 |
| 6-1821 | (2-trifluoromethyl)phenyl | H | H | F | H | H | H | I | H | nonafluoro-2-butyl | H | CF3 |
| 6-1822 | (3-trifluoromethyl)phenyl | H | H | F | H | H | H | I | H | nonafluoro-2-butyl | H | CF3 |
| 6-1823 | (4-trifluoromethyl)phenyl | H | H | F | H | H | H | I | H | nonafluoro-2-butyl | H | CF3 |
| 6-1824 | 2-nitrophenyl | H | H | F | H | H | H | I | H | nonafluoro-2-butyl | H | CF3 |
| 6-1825 | 3-nitrophenyl | H | H | F | H | H | H | I | H | nonafluoro-2-butyl | H | CF3 |
| 6-1826 | 4-nitrophenyl | H | H | F | H | H | H | I | H | nonafluoro-2-butyl | H | CF3 |
| 6-1827 | 2-cyanophenyl | H | H | F | H | H | H | I | H | nonafluoro-2-butyl | H | CF3 |
| 6-1828 | 3-cyanophenyl | H | H | F | H | H | H | I | H | nonafluoro-2-butyl | H | CF3 |
| 6-1829 | 4-cyanophenyl | H | H | F | H | H | H | I | H | nonafluoro-2-butyl | H | CF3 |
| 6-1830 | 2,6-difluorophenyl | H | H | F | H | H | H | I | H | nonafluoro-2-butyl | H | CF3 |
| 6-1831 | 3,4-dichlorophenyl | H | H | F | H | H | H | I | H | nonafluoro-2-butyl | H | CF3 |
| 6-1832 | 2,4-dichlorophenyl | H | H | F | H | H | H | I | H | nonafluoro-2-butyl | H | CF3 |
| 6-1833 | 2-chloro-4-fluorophenyl | H | H | F | H | H | H | I | H | nonafluoro-2-butyl | H | CF3 |
| 6-1834 | 2-chloro-4,5-difluorophenyl | H | H | F | H | H | H | I | H | nonafluoro-2-butyl | H | CF3 |
| 6-1835 | 4-bromo-2-chlorophenyl | H | H | F | H | H | H | I | H | nonafluoro-2-butyl | H | CF3 |
| 6-1836 | 2-bromo-4-chlorophenyl | H | H | F | H | H | H | I | H | nonafluoro-2-butyl | H | CF3 |
| 6-1837 | 2-bromo-4-fluorophenyl | H | H | F | H | H | H | I | H | nonafluoro-2-butyl | H | CF3 |
| 6-1838 | 2-chloro-4-nitrophenyl | H | H | F | H | H | H | I | H | nonafluoro-2-butyl | H | CF3 |

**Table 6(9)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 6-1839 | 3,5-dicyanophenyl | H | H | F | H | H | H | I | H | nonafluoro-2-butyl | H | CF3 |
| 6-1840 | 4-cyano-2-fluorophenyl | H | H | F | H | H | H | I | H | nonafluoro-2-butyl | H | CF3 |
| 6-1841 | 2-chloro-4-cyanophenyl | H | H | F | H | H | H | I | H | nonafluoro-2-butyl | H | CF3 |
| 6-1842 | pyridin-3-yl | H | H | F | H | H | H | I | H | nonafluoro-2-butyl | H | CF3 |
| 6-1843 | 2-fluoropyridin-3-yl | H | H | F | H | H | H | I | H | nonafluoro-2-butyl | H | CF3 |
| 6-1844 | 2-chloropyridin-3-yl | H | H | F | H | H | H | I | H | nonafluoro-2-butyl | H | CF3 |
| 6-1845 | 2-bromopyridin-3-yl | H | H | F | H | H | H | I | H | nonafluoro-2-butyl | H | CF3 |
| 6-1846 | 2-iodopyridin-3-yl | H | H | F | H | H | H | I | H | nonafluoro-2-butyl | H | CF3 |
| 6-1847 | 2-(trifluoromethyl)pyridin-3-yl | H | H | F | H | H | H | I | H | nonafluoro-2-butyl | H | CF3 |
| 6-1848 | 2-nitropyridin-3-yl | H | H | F | H | H | H | I | H | nonafluoro-2-butyl | H | CF3 |
| 6-1849 | 2-cyanopyridin-3-yl | H | H | F | H | H | H | I | H | nonafluoro-2-butyl | H | CF3 |
| 6-1850 | 6-fluoropyridin-3-yl | H | H | F | H | H | H | I | H | nonafluoro-2-butyl | H | CF3 |
| 6-1851 | 6-chloropyridin-3-yl | H | H | F | H | H | H | I | H | nonafluoro-2-butyl | H | CF3 |
| 6-1852 | 6-bromopyridin-3-yl | H | H | F | H | H | H | I | H | nonafluoro-2-butyl | H | CF3 |
| 6-1853 | 6-iodopyridin-3-yl | H | H | F | H | H | H | I | H | nonafluoro-2-butyl | H | CF3 |
| 6-1854 | 6-(trifluoromethyl)pyridin-3-yl | H | H | F | H | H | H | I | H | nonafluoro-2-butyl | H | CF3 |
| 6-1855 | 6-nitropyridin-3-yl | H | H | F | H | H | H | I | H | nonafluoro-2-butyl | H | CF3 |
| 6-1856 | 6-cyanopyridin-3-yl | H | H | F | H | H | H | I | H | nonafluoro-2-butyl | H | CF3 |
| 6-1857 | 5-fluoropyridin-3-yl | H | H | F | H | H | H | I | H | nonafluoro-2-butyl | H | CF3 |
| 6-1858 | 5-chloropyridin-3-yl | H | H | F | H | H | H | I | H | nonafluoro-2-butyl | H | CF3 |
| 6-1859 | 5-bromopyridin-3-yl | H | H | F | H | H | H | I | H | nonafluoro-2-butyl | H | CF3 |
| 6-1860 | 5-iodopyridin-3-yl | H | H | F | H | H | H | I | H | nonafluoro-2-butyl | H | CF3 |
| 6-1861 | 5-(trifluoromethyl)pyridin-3-yl | H | H | F | H | H | H | I | H | nonafluoro-2-butyl | H | CF3 |
| 6-1862 | 5-nitropyridin-3-yl | H | H | F | H | H | H | I | H | nonafluoro-2-butyl | H | CF3 |
| 6-1863 | 5-cyanopyridin-3-yl | H | H | F | H | H | H | I | H | nonafluoro-2-butyl | H | CF3 |
| 6-1864 | 4-fluoropyridin-3-yl | H | H | F | H | H | H | I | H | nonafluoro-2-butyl | H | CF3 |
| 6-1865 | 4-chloropyridin-3-yl | H | H | F | H | H | H | I | H | nonafluoro-2-butyl | H | CF3 |
| 6-1866 | 4-bromopyridin-3-yl | H | H | F | H | H | H | I | H | nonafluoro-2-butyl | H | CF3 |
| 6-1867 | 4-iodopyridin-3-yl | H | H | F | H | H | H | I | H | nonafluoro-2-butyl | H | CF3 |
| 6-1868 | 4-(trifluoromethyl)pyridin-3-yl | H | H | F | H | H | H | I | H | nonafluoro-2-butyl | H | CF3 |
| 6-1869 | 4-nitropyridin-3-yl | H | H | F | H | H | H | I | H | nonafluoro-2-butyl | H | CF3 |
| 6-1870 | 4-cyanopyridin-3-yl | H | H | F | H | H | H | I | H | nonafluoro-2-butyl | H | CF3 |
| 6-1871 | 2,6-dichloropyridin-3-yl | H | H | F | H | H | H | I | H | nonafluoro-2-butyl | H | CF3 |
| 6-1872 | pyridin-3-yl N-oxide | H | H | F | H | H | H | I | H | nonafluoro-2-butyl | H | CF3 |
| 6-1873 | pyridin-4-yl | H | H | F | H | H | H | I | H | nonafluoro-2-butyl | H | CF3 |
| 6-1874 | 2-chloropyridin-4-yl | H | H | F | H | H | H | I | H | nonafluoro-2-butyl | H | CF3 |
| 6-1875 | 3-bromopyridin-4-yl | H | H | F | H | H | H | I | H | nonafluoro-2-butyl | H | CF3 |
| 6-1876 | 3,5-dichloropyridin-4-yl | H | H | F | H | H | H | I | H | nonafluoro-2-butyl | H | CF3 |
| 6-1877 | 3-(trifluoromethyl)pyridin-4-yl | H | H | F | H | H | H | I | H | nonafluoro-2-butyl | H | CF3 |
| 6-1878 | 2,6-dieyanopyridin-4-yl | H | H | F | H | H | H | I | H | nonafluoro-2-butyl | H | CF3 |
| 6-1879 | pyridin-4-yl N-oxide | H | H | F | H | H | H | I | H | nonafluoro-2-butyl | H | CF3 |
| 6-1880 | pyridin-2-yl | H | H | F | H | H | H | I | H | nonafluoro-2-butyl | H | CF3 |
| 6-1881 | 3-chloropyridin-2-yl | H | H | F | H | H | H | I | H | nonafluoro-2-butyl | H | CF3 |
| 6-1882 | 4-bromopyridin-2-yl | H | H | F | H | H | H | I | H | nonafluoro-2-butyl | H | CF3 |
| 6-1883 | 5-iodopyridin-2-yl | H | H | F | H | H | H | I | H | nonafluoro-2-butyl | H | CF3 |
| 6-1884 | 6-chloropyridin-2-yl | H | H | F | H | H | H | I | H | nonafluoro-2-butyl | H | CF3 |
| 6-1885 | 4-cyanopyridin-2-yl | H | H | F | H | H | H | I | H | nonafluoro-2-butyl | H | CF3 |
| 6-1886 | phenyl | H | H | F | H | H | H | OCF3 | H | heptafluoroisopropyl | H | OCF3 |
| 6-1901 | 3-cyanophenyl | H | H | F | H | H | H | OCF3 | H | heptafluoroisopropyl | H | OCF3 |
| 6-1902 | 4-cyanophenyl | H | H | F | H | H | H | OCF3 | H | heptafluoroisopropyl | H | OCF3 |
| 6-1917 | 2-chloropyridin-3-yl | H | H | F | H | H | H | OCF3 | H | heptafluoroisopropyl | H | OCF3 |
| 6-1942 | phenyl | H | H | F | H | H | H | OCF3 | H | nonafluoro-2-butyl | H | OCF3 |
| 6-1957 | 3-cyanophenyl | H | H | F | H | H | H | OCF3 | H | nonafluoro-2-butyl | H | OCF3 |
| 6-1958 | 4-cyanophenyl | H | H | F | H | H | H | OCF3 | H | nonafluoro-2-butyl | H | OCF3 |
| 6-1973 | 2-chloropyridin-3-yl | H | H | F | H | H | H | OCF3 | H | nonafluoro-2-butyl | H | OCF3 |
| 6-1998 | phenyl | H | H | F | H | H | H | CF3 | H | heptafluoroisopropyl | H | OCF3 |
| 6-2013 | 3-cyanophenyl | H | H | F | H | H | H | CF3 | H | heptafluoroisopropyl | H | OCF3 |
| 6-2014 | 4-cyanophenyl | H | H | F | H | H | H | CF3 | H | heptafluoroisopropyl | H | OCF3 |
| 6-2029 | 2-chloropyridin-3-yl | H | H | F | H | H | H | CF3 | H | heptafluoroisopropyl | H | OCF3 |
| 6-2054 | phenyl | H | H | F | H | H | H | CF3 | H | nonafluoro-2-butyl | H | OCF3 |
| 6-2069 | 3-cyanophenyl | H | H | F | H | H | H | CF3 | H | nonafluoro-2-butyl | H | OCF3 |
| 6-2070 | 4-cyanophenyl | H | H | F | H | H | H | CF3 | H | nonafluoro-2-butyl | H | OCF3 |
| 6-2085 | 2-chloropyridin-3-yl | H | H | F | H | H | H | CF3 | H | nonafluoro-2-butyl | H | OCF3 |
| 6-2110 | phenyl | H | H | F | H | H | H | CF3 | H | heptafluoroisopropyl | H | CF3 |

**Table 6(10)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 6-2130 | 3-cyanophenyl | H | H | F | H | H | H | CF3 | H | heptafluoroisopropyl | H | CF3 |
| 6-2131 | 4-cyanophenyl | H | H | F | H | H | H | CF3 | H | heptafluoroisopropyl | H | CF3 |
| 6-2146 | 2-chloropyridin-3-yl | H | H | F | H | H | H | CF3 | H | heptafluoroisopropyl | H | CF3 |
| 6-2178 | phenyl | H | H | F | H | H | H | CF3 | H | nonafluoro-2-butyl | H | CF3 |
| 6-2198 | 3-cyanophenyl | H | H | F | H | H | H | CF3 | H | nonafluoro-2-butyl | H | CF3 |
| 6-2199 | 4-cyanophenyl | H | H | F | H | H | H | CF3 | H | nonafluoro-2-butyl | H | CF3 |
| 6-2214 | 2-chloropyridin-3-yl | H | H | F | H | H | H | CF3 | H | nonafluoro-2-butyl | H | CF3 |
| 6-2246 | phenyl | H | H | H | F | H | H | F | H | heptafluoroisopropyl | H | F |
| 6-2247 | 3-cyanophenyl | H | H | H | F | H | H | F | H | heptafluoroisopropyl | H | F |
| 6-2248 | 4-cyanophenyl | H | H | H | F | H | H | F | H | heptafluoroisopropyl | H | F |
| 6-2249 | 2-chloropyridin-3-yl | H | H | H | F | H | H | F | H | heptafluoroisopropyl | H | F |
| 6-2250 | phenyl | H | H | H | F | H | H | F | H | nonafluoro-2-butyl | H | F |
| 6-2251 | 3-cyanophenyl | H | H | H | F | H | H | F | H | nonafluoro-2-butyl | H | F |
| 6-2252 | 4-cyanophenyl | H | H | H | F | H | H | F | H | nonafluoro-2-butyl | H | F |
| 6-2253 | 2-chloropyridin-3-yl | H | H | H | F | H | H | F | H | nonafluoro-2-butyl | H | F |
| 6-2254 | phenyl | H | H | H | F | H | H | Cl | H | heptafluoroisopropyl | H | Cl |
| 6-2274 | 3-cyanophenyl | H | H | H | F | H | H | Cl | H | heptafluoroisopropyl | H | Cl |
| 6-2275 | 4-cyanophenyl | H | H | H | F | H | H | Cl | H | heptafluoroisopropyl | H | Cl |
| 6-2290 | 2-chloropyridin-3-yl | H | H | H | F | H | H | Cl | H | heptafluoroisopropyl | H | Cl |
| 6-2332 | phenyl | H | H | H | F | H | H | Cl | H | nonafluoro-2-butyl | H | Cl |
| 6-2352 | 3-cyanophenyl | H | H | H | F | H | H | Cl | H | nonafluoro-2-butyl | H | Cl |
| 6-2353 | 4-cyanophenyl | H | H | H | F | H | H | Cl | H | nonafluoro-2-butyl | H | Cl |
| 6-2368 | 2-chloropyridin-3-yl | H | H | H | F | H | H | Cl | H | nonafluoro-2-butyl | H | Cl |
| 6-2410 | phenyl | H | H | H | F | H | H | Br | H | heptafluoroisopropyl | H | Br |
| 6-2430 | 3-cyanophenyl | H | H | H | F | H | H | Br | H | heptafluoroisopropyl | H | Br |
| 6-2431 | 4-cyanophenyl | H | H | H | F | H | H | Br | H | heptafluoroisopropyl | H | Br |
| 6-2446 | 2-chloropyridin-3-yl | H | H | H | F | H | H | Br | H | heptafluoroisopropyl | H | Br |
| 6-2488 | phenyl | H | H | H | F | H | H | Br | H | nonafluoro-2-butyl | H | Br |
| 6-2508 | 3-cyanophenyl | H | H | H | F | H | H | Br | H | nonafluoro-2-butyl | H | Br |
| 6-2509 | 4-cyanophenyl | H | H | H | F | H | H | Br | H | nonafluoro-2-butyl | H | Br |
| 6-2524 | 2-chloropyridin-3-yl | H | H | H | F | H | H | Br | H | nonafluoro-2-butyl | H | Br |
| 6-2566 | phenyl | H | H | H | F | H | H | I | H | heptafluoroisopropyl | H | I |
| 6-2586 | 3-cyanophenyl | H | H | H | F | H | H | I | H | heptafluoroisopropyl | H | I |
| 6-2587 | 4-cyanophenyl | H | H | H | F | H | H | I | H | heptafluoroisopropyl | H | I |
| 6-2602 | 2-chloropyridin-3-yl | H | H | H | F | H | H | I | H | heptafluoroisopropyl | H | I |
| 6-2644 | phenyl | H | H | H | F | H | H | I | H | nonafluoro-2-butyl | H | I |
| 6-2664 | 3-cyanophenyl | H | H | H | F | H | H | I | H | nonafluoro-2-butyl | H | I |
| 6-2665 | 4-cyanophenyl | H | H | H | F | H | H | I | H | nonafluoro-2-butyl | H | I |
| 6-2680 | 2-chloropyridin-3-yl | H | H | H | F | H | H | I | H | nonafluoro-2-butyl | H | I |
| 6-2722 | phenyl | H | H | H | F | H | H | Cl | H | heptafluoroisopropyl | H | OCF3 |
| 6-2742 | 3-cyanophenyl | H | H | H | F | H | H | Cl | H | heptafluoroisopropyl | H | OCF3 |
| 6-2743 | 4-cyanophenyl | H | H | H | F | H | H | Cl | H | heptafluoroisopropyl | H | OCF3 |
| 6-2758 | 2-chloropyridin-3-yl | H | H | H | F | H | H | Cl | H | heptafluoroisopropyl | H | OCF3 |
| 6-2800 | phenyl | H | H | H | F | H | H | Cl | H | nonafluoro-2-butyl | H | OCF3 |
| 6-2820 | 3-cyanophenyl | H | H | H | F | H | H | Cl | H | nonafluoro-2-butyl | H | OCF3 |
| 6-2821 | 4-cyanophenyl | H | H | H | F | H | H | Cl | H | nonafluoro-2-butyl | H | OCF3 |
| 6-2836 | 2-chloropyridin-3-yl | H | H | H | F | H | H | Cl | H | nonafluoro-2-butyl | H | OCF3 |
| 6-2878 | phenyl | H | H | H | F | H | H | Br | H | heptafluoroisopropyl | H | OCF3 |
| 6-2898 | 3-cyanophenyl | H | H | H | F | H | H | Br | H | heptafluoroisopropyl | H | OCF3 |
| 6-2899 | 4-cyanophenyl | H | H | H | F | H | H | Br | H | heptafluoroisopropyl | H | OCF3 |
| 6-2914 | 2-chloropyridin-3-yl | H | H | H | F | H | H | Br | H | heptafluoroisopropyl | H | OCF3 |
| 6-2956 | phenyl | H | H | H | F | H | H | Br | H | nonafluoro-2-butyl | H | OCF3 |
| 6-2976 | 3-cyanophenyl | H | H | H | F | H | H | Br | H | nonafluoro-2-butyl | H | OCF3 |
| 6-2977 | 4-cyanophenyl | H | H | H | F | H | H | Br | H | nonafluoro-2-butyl | H | OCF3 |
| 6-2992 | 2-chloropyridin-3-yl | H | H | H | F | H | H | Br | H | nonafluoro-2-butyl | H | OCF3 |
| 6-3034 | phenyl | H | H | H | F | H | H | I | H | heptafluoroisopropyl | H | OCF3 |
| 6-3054 | 3-cyanophenyl | H | H | H | F | H | H | I | H | heptafluoroisopropyl | H | OCF3 |
| 6-3055 | 4-cyanophenyl | H | H | H | F | H | H | I | H | heptafluoroisopropyl | H | OCF3 |
| 6-3070 | 2-chloropyridin-3-yl | H | H | H | F | H | H | I | H | heptafluoroisopropyl | H | OCF3 |
| 6-3112 | phenyl | H | H | H | F | H | H | I | H | nonafluoro-2-butyl | H | OCF3 |
| 6-3132 | 3-cyanophenyl | H | H | H | F | H | H | I | H | nonafluoro-2-butyl | H | OCF3 |
| 6-3133 | 4-cyanophenyl | H | H | H | F | H | H | I | H | nonafluoro-2-butyl | H | OCF3 |
| 6-3148 | 2-chloropyridin-3-yl | H | H | H | F | H | H | I | H | nonafluoro-2-butyl | H | OCF3 |
| 6-3190 | phenyl | H | H | H | F | H | H | F | H | heptafluoroisopropyl | H | CF3 |

**Table 6(11)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 6-3191 | phenyl | H | H | H | F | H | H | Cl | H | heptafluoroisopropyl | H | CF3 |
| 6-3211 | 3-cyanophenyl | H | H | H | F | H | H | Cl | H | heptafluoroisopropyl | H | CF3 |
| 6-3212 | 4-cyanophenyl | H | H | H | F | H | H | Cl | H | heptafluoroisopropyl | H | CF3 |
| 6-3227 | 2-chloropyridin-3-yl | H | H | H | F | H | H | Cl | H | heptafluoroisopropyl | H | CF3 |
| 6-3269 | phenyl | H | H | H | F | H | H | F | H | nonafluoro-2-butyl | H | CF3 |
| 6-3270 | phenyl | H | H | H | F | H | H | Cl | H | nonafluoro-2-butyl | H | CF3 |
| 6-3290 | 3-cyanophenyl | H | H | H | F | H | H | Cl | H | nonafluoro-2-butyl | H | CF3 |
| 6-3291 | 4-cyanophenyl | H | H | H | F | H | H | Cl | H | nonafluoro-2-butyl | H | CF3 |
| 6-3306 | 2-chloropyridin-3-yl | H | H | H | F | H | H | Cl | H | nonafluoro-2-butyl | H | CF3 |
| 6-3348 | phenyl | H | H | H | F | H | H | Br | H | heptafluoroisopropyl | H | CF3 |
| 6-3368 | 3-cyanophenyl | H | H | H | F | H | H | Br | H | heptafluoroisopropyl | H | CF3 |
| 6-3369 | 4-cyanophenyl | H | H | H | F | H | H | Br | H | heptafluoroisopropyl | H | CF3 |
| 6-3384 | 2-chloropyridin-3-yl | H | H | H | F | H | H | Br | H | heptafluoroisopropyl | H | CF3 |
| 6-3426 | phenyl | H | H | H | F | H | H | Br | H | nonafluoro-2-butyl | H | CF3 |
| 6-3446 | 3-cyanophenyl | H | H | H | F | H | H | Br | H | nonafluoro-2-butyl | H | CF3 |
| 6-3447 | 4-cyanophenyl | H | H | H | F | H | H | Br | H | nonafluoro-2-butyl | H | CF3 |
| 6-3462 | 2-chloropyridin-3-yl | H | H | H | F | H | H | Br | H | nonafluoro-2-butyl | H | CF3 |
| 6-3504 | phenyl | H | H | H | F | H | H | I | H | heptafluoroisopropyl | H | CF3 |
| 6-3524 | 3-cyanophenyl | H | H | H | F | H | H | I | H | heptafluoroisopropyl | H | CF3 |
| 6-3525 | 4-cyanophenyl | H | H | H | F | H | H | I | H | heptafluoroisopropyl | H | CF3 |
| 6-3540 | 2-chloropyridin-3-yl | H | H | H | F | H | H | I | H | heptafluoroisopropyl | H | CF3 |
| 6-3582 | phenyl | H | H | H | F | H | H | I | H | nonafluoro-2-butyl | H | CF3 |
| 6-3602 | 3-cyanophenyl | H | H | H | F | H | H | I | H | nonafluoro-2-butyl | H | CF3 |
| 6-3603 | 4-cyanophenyl | H | H | H | F | H | H | I | H | nonafluoro-2-butyl | H | CF3 |
| 6-3618 | 2-chloropyridin-3-yl | H | H | H | F | H | H | I | H | nonafluoro-2-butyl | H | CF3 |
| 6-3660 | phenyl | H | H | H | F | H | H | OCF3 | H | heptafluoroisopropyl | H | OCF3 |
| 6-3675 | 3-cyanophenyl | H | H | H | F | H | H | OCF3 | H | heptafluoroisopropyl | H | OCF3 |
| 6-3676 | 4-cyanophenyl | H | H | H | F | H | H | OCF3 | H | heptafluoroisopropyl | H | OCF3 |
| 6-3691 | 2-chloropyridin-3-yl | H | H | H | F | H | H | OCF3 | H | heptafluoroisopropyl | H | OCF3 |
| 6-3716 | phenyl | H | H | H | F | H | H | OCF3 | H | nonafluoro-2-butyl | H | OCF3 |
| 6-3731 | 3-cyanophenyl | H | H | H | F | H | H | OCF3 | H | nonafluoro-2-butyl | H | OCF3 |
| 6-3732 | 4-cyanophenyl | H | H | H | F | H | H | OCF3 | H | nonafluoro-2-butyl | H | OCF3 |
| 6-3747 | 2-chloropyridin-3-yl | H | H | H | F | H | H | OCF3 | H | nonafluoro-2-butyl | H | OCF3 |
| 6-3772 | phenyl | H | H | H | F | H | H | CF3 | H | heptafluoroisopropyl | H | OCF3 |
| 6-3787 | 3-cyanophenyl | H | H | H | F | H | H | CF3 | H | heptafluoroisopropyl | H | OCF3 |
| 6-3788 | 4-cyanophenyl | H | H | H | F | H | H | CF3 | H | heptafluoroisopropyl | H | OCF3 |
| 6-3803 | 2-chloropyridin-3-yl | H | H | H | F | H | H | CF3 | H | heptafluoroisopropyl | H | OCF3 |
| 6-3828 | phenyl | H | H | H | F | H | H | CF3 | H | nonafluoro-2-butyl | H | OCF3 |
| 6-3843 | 3-cyanophenyl | H | H | H | F | H | H | CF3 | H | nonafluoro-2-butyl | H | OCF3 |
| 6-3844 | 4-cyanophenyl | H | H | H | F | H | H | CF3 | H | nonafluoro-2-butyl | H | OCF3 |
| 6-3859 | 2-chloropyridin-3-yl | H | H | H | F | H | H | CF3 | H | nonafluoro-2-butyl | H | OCF3 |
| 6-3884 | phenyl | H | H | H | F | H | H | CF3 | H | heptafluoroisopropyl | H | CF3 |
| 6-3904 | 3-cyanophenyl | H | H | H | F | H | H | CF3 | H | heptafluoroisopropyl | H | CF3 |
| 6-3905 | 4-cyanophenyl | H | H | H | F | H | H | CF3 | H | heptafluoroisopropyl | H | CF3 |
| 6-3920 | 2-chloropyridin-3-yl | H | H | H | F | H | H | CF3 | H | heptafluoroisopropyl | H | CF3 |
| 6-3952 | phenyl | H | H | H | F | H | H | CF3 | H | nonafluoro-2-butyl | H | CF3 |
| 6-3972 | 3-cyanophenyl | H | H | H | F | H | H | CF3 | H | nonafluoro-2-butyl | H | CF3 |
| 6-3973 | 4-cyanophenyl | H | H | H | F | H | H | CF3 | H | nonafluoro-2-butyl | H | CF3 |
| 6-3988 | 2-chloropyridin-3-yl | H | H | H | F | H | H | CF3 | H | nonafluoro-2-butyl | H | CF3 |
| 6-4020 | phenyl | H | H | F | F | H | H | F | H | heptafluoroisopropyl | H | F |
| 6-4021 | 3-cyanophenyl | H | H | F | F | H | H | F | H | heptafluoroisopropyl | H | F |
| 6-4022 | 4-cyanophenyl | H | H | F | F | H | H | F | H | heptafluoroisopropyl | H | F |
| 6-4023 | 2-chloropyridin-3-yl | H | H | F | F | H | H | F | H | heptafluoroisopropyl | H | F |
| 6-4024 | phenyl | H | H | F | F | H | H | F | H | nonafluoro-2-butyl | H | F |
| 6-4025 | 3-cyanophenyl | H | H | F | F | H | H | F | H | nonafluoro-2-butyl | H | F |
| 6-4026 | 4-cyanophenyl | H | H | F | F | H | H | F | H | nonafluoro-2-butyl | H | F |
| 6-4027 | 2-chloropyridin-3-yl | H | H | F | F | H | H | F | H | nonafluoro-2-butyl | H | F |
| 6-4028 | phenyl | H | H | F | F | H | H | Cl | H | heptafluoroisopropyl | H | Cl |
| 6-4048 | 3-cyanophenyl | H | H | F | F | H | H | Cl | H | heptafluoroisopropyl | H | Cl |
| 6-4049 | 4-cyanophenyl | H | H | F | F | H | H | Cl | H | heptafluoroisopropyl | H | Cl |
| 6-4064 | 2-chloropyridin-3-yl | H | H | F | F | H | H | Cl | H | heptafluoroisopropyl | H | Cl |
| 6-4106 | phenyl | H | H | F | F | H | H | Cl | H | nonafluoro-2-butyl | H | Cl |
| 6-4126 | 3-cyanophenyl | H | H | F | F | H | H | Cl | H | nonafluoro-2-butyl | H | Cl |
| 6-4127 | 4-cyanophenyl | H | H | F | F | H | H | Cl | H | nonafluoro-2-butyl | H | Cl |

**Table 6(12)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 6-4142 | 2-chloropyridin-3-yl | H | H | F | F | H | H | Cl | H | nonafluoro-2-butyl | H | Cl |
| 6-4184 | phenyl | H | H | F | F | H | H | Br | H | heptafluoroisopropyl | H | Br |
| 6-4204 | 3-cyanophenyl | H | H | F | F | H | H | Br | H | heptafluoroisopropyl | H | Br |
| 6-4205 | 4-cyanophenyl | H | H | F | F | H | H | Br | H | heptafluoroisopropyl | H | Br |
| 6-4220 | 2-chloropyridin-3-yl | H | H | F | F | H | H | Br | H | heptafluoroisopropyl | H | Br |
| 6-4262 | phenyl | H | H | F | F | H | H | Br | H | nonafluoro-2-butyl | H | Br |
| 6-4282 | 3-cyanophenyl | H | H | F | F | H | H | Br | H | nonafluoro-2-butyl | H | Br |
| 6-4283 | 4-cyanophenyl | H | H | F | F | H | H | Br | H | nonafluoro-2-butyl | H | Br |
| 6-4298 | 2-chloropyridin-3-yl | H | H | F | F | H | H | Br | H | nonafluoro-2-butyl | H | Br |
| 6-4340 | phenyl | H | H | F | F | H | H | I | H | heptafluoroisopropyl | H | I |
| 6-4360 | 3-cyanophenyl | H | H | F | F | H | H | I | H | heptafluoroisopropyl | H | I |
| 6-4361 | 4-cyanophenyl | H | H | F | F | H | H | I | H | heptafluoroisopropyl | H | I |
| 6-4376 | 2-chloropyridin-3-yl | H | H | F | F | H | H | I | H | heptafluoroisopropyl | H | I |
| 6-4418 | phenyl | H | H | F | F | H | H | I | H | nonafluoro-2-butyl | H | I |
| 6-4438 | 3-cyanophenyl | H | H | F | F | H | H | I | H | nonafluoro-2-butyl | H | I |
| 6-4439 | 4-cyanophenyl | H | H | F | F | H | H | I | H | nonafluoro-2-butyl | H | I |
| 6-4454 | 2-chloropyridin-3-yl | H | H | F | F | H | H | I | H | nonafluoro-2-butyl | H | I |
| 6-4496 | phenyl | H | H | F | F | H | H | Cl | H | heptafluoroisopropyl | H | OCF3 |
| 6-4516 | 3-cyanophenyl | H | H | F | F | H | H | Cl | H | heptafluoroisopropyl | H | OCF3 |
| 6-4517 | 4-cyanophenyl | H | H | F | F | H | H | Cl | H | heptafluoroisopropyl | H | OCF3 |
| 6-4532 | 2-chloropyridin-3-yl | H | H | F | F | H | H | Cl | H | heptafluoroisopropyl | H | OCF3 |
| 6-4574 | phenyl | H | H | F | F | H | H | Cl | H | nonafluoro-2-butyl | H | OCF3 |
| 6-4594 | 3-cyanophenyl | H | H | F | F | H | H | Cl | H | nonafluoro-2-butyl | H | OCF3 |
| 6-4595 | 4-cyanophenyl | H | H | F | F | H | H | Cl | H | nonafluoro-2-butyl | H | OCF3 |
| 6-4610 | 2-chloropyridin-3-yl | H | H | F | F | H | H | Cl | H | nonafluoro-2-butyl | H | OCF3 |
| 6-4652 | phenyl | H | H | F | F | H | H | Br | H | heptafluoroisopropyl | H | OCF3 |
| 6-4672 | 3-cyanophenyl | H | H | F | F | H | H | Br | H | heptafluoroisopropyl | H | OCF3 |
| 6-4673 | 4-cyanophenyl | H | H | F | F | H | H | Br | H | heptafluoroisopropyl | H | OCF3 |
| 6-4688 | 2-chloropyridin-3-yl | H | H | F | F | H | H | Br | H | heptafluoroisopropyl | H | OCF3 |
| 6-4730 | phenyl | H | H | F | F | H | H | Br | H | nonafluoro-2-butyl | H | OCF3 |
| 6-4750 | 3-cyanophenyl | H | H | F | F | H | H | Br | H | nonafluoro-2-butyl | H | OCF3 |
| 6-4751 | 4-cyanophenyl | H | H | F | F | H | H | Br | H | nonafluoro-2-butyl | H | OCF3 |
| 6-4766 | 2-chloropyridin-3-yl | H | H | F | F | H | H | Br | H | nonafluoro-2-butyl | H | OCF3 |
| 6-4808 | phenyl | H | H | F | F | H | H | I | H | heptafluoroisopropyl | H | OCF3 |
| 6-4828 | 3-cyanophenyl | H | H | F | F | H | H | I | H | heptafluoroisopropyl | H | OCF3 |
| 6-4829 | 4-cyanophenyl | H | H | F | F | H | H | I | H | heptafluoroisopropyl | H | OCF3 |
| 6-4844 | 2-chloropyridin-3-yl | H | H | F | F | H | H | I | H | heptafluoroisopropyl | H | OCF3 |
| 6-4886 | phenyl | H | H | F | F | H | H | I | H | nonafluoro-2-butyl | H | OCF3 |
| 6-4906 | 3-cyanophenyl | H | H | F | F | H | H | I | H | nonafluoro-2-butyl | H | OCF3 |
| 6-4907 | 4-cyanophenyl | H | H | F | F | H | H | I | H | nonafluoro-2-butyl | H | OCF3 |
| 6-4922 | 2-chloropyridin-3-yl | H | H | F | F | H | H | I | H | nonafluoro-2-butyl | H | OCF3 |
| 6-4964 | phenyl | H | H | F | F | H | H | F | H | heptafluoroisopropyl | H | CF3 |
| 6-4965 | phenyl | H | H | F | F | H | H | Cl | H | heptafluoroisopropyl | H | CF3 |
| 6-4985 | 3-cyanophenyl | H | H | F | F | H | H | Cl | H | heptafluoroisopropyl | H | CF3 |
| 6-4986 | 4-cyanophenyl | H | H | F | F | H | H | Cl | H | heptafluoroisopropyl | H | CF3 |
| 6-5001 | 2-chloropyridin-3-yl | H | H | F | F | H | H | Cl | H | heptafluoroisopropyl | H | CF3 |
| 6-5043 | phenyl | H | H | F | F | H | H | F | H | nonafluoro-2-butyl | H | CF3 |
| 6-5044 | phenyl | H | H | F | F | H | H | Cl | H | nonafluoro-2-butyl | H | CF3 |
| 6-5064 | 3-cyanophenyl | H | H | F | F | H | H | Cl | H | nonafluoro-2-butyl | H | CF3 |
| 6-5065 | 4-cyanophenyl | H | H | F | F | H | H | Cl | H | nonafluoro-2-butyl | H | CF3 |
| 6-5080 | 2-chloropyridin-3-yl | H | H | F | F | H | H | Cl | H | nonafluoro-2-butyl | H | CF3 |
| 6-5122 | phenyl | H | H | F | F | H | H | Br | H | heptafluoroisopropyl | H | CF3 |
| 6-5142 | 3-cyanophenyl | H | H | F | F | H | H | Br | H | heptafluoroisopropyl | H | CF3 |
| 6-5143 | 4-cyanophenyl | H | H | F | F | H | H | Br | H | heptafluoroisopropyl | H | CF3 |
| 6-5158 | 2-chloropyridin-3-yl | H | H | F | F | H | H | Br | H | heptafluoroisopropyl | H | CF3 |
| 6-5200 | phenyl | H | H | F | F | H | H | Br | H | nonafluoro-2-butyl | H | CF3 |
| 6-5220 | 3-cyanophenyl | H | H | F | F | H | H | Br | H | nonafluoro-2-butyl | H | CF3 |
| 6-5221 | 4-cyanophenyl | H | H | F | F | H | H | Br | H | nonafluoro-2-butyl | H | CF3 |
| 6-5236 | 2-chloropyridin-3-yl | H | H | F | F | H | H | Br | H | nonafluoro-2-butyl | H | CF3 |
| 6-5278 | phenyl | H | H | F | F | H | H | I | H | heptafluoroisopropyl | H | CF3 |
| 6-5298 | 3-cyanophenyl | H | H | F | F | H | H | I | H | heptafluoroisopropyl | H | CF3 |
| 6-5299 | 4-cyanophenyl | H | H | F | F | H | H | I | H | heptafluoroisopropyl | H | CF3 |
| 6-5314 | 2-chloropyridin-3-yl | H | H | F | F | H | H | I | H | heptafluoroisopropyl | H | CF3 |
| 6-5356 | phenyl | H | H | F | F | H | H | I | H | nonafluoro-2-butyl | H | CF3 |

**Table 6(13)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 6-5376 | 3-cyanophenyl | H | H | F | F | H | H | I | H | nonafluoro-2-butyl | H | CF3 |
| 6-5377 | 4-cyanophenyl | H | H | F | F | H | H | I | H | nonafluoro-2-butyl | H | CF3 |
| 6-5392 | 2-chloropyridin-3-yl | H | H | F | F | H | H | I | H | nonafluoro-2-butyl | H | CF3 |
| 6-5434 | phenyl | H | H | F | F | H | H | OCF3 | H | heptafluoroisopropyl | H | OCF3 |
| 6-5449 | 3-cyanophenyl | H | H | F | F | H | H | OCF3 | H | heptafluoroisopropyl | H | OCF3 |
| 6-5450 | 4-cyanophenyl | H | H | F | F | H | H | OCF3 | H | heptafluoroisopropyl | H | OCF3 |
| 6-5465 | 2-chloropyridin-3-yl | H | H | F | F | H | H | OCF3 | H | heptafluoroisopropyl | H | OCF3 |
| 6-5490 | phenyl | H | H | F | F | H | H | OCF3 | H | nonafluoro-2-butyl | H | OCF3 |
| 6-5505 | 3-cyanophenyl | H | H | F | F | H | H | OCF3 | H | nonafluoro-2-butyl | H | OCF3 |
| 6-5506 | 4-cyanophenyl | H | H | F | F | H | H | OCF3 | H | nonafluoro-2-butyl | H | OCF3 |
| 6-5521 | 2-chloropyridin-3-yl | H | H | F | F | H | H | OCF3 | H | nonafluoro-2-butyl | H | OCF3 |
| 6-5546 | phenyl | H | H | F | F | H | H | CF3 | H | heptafluoroisopropyl | H | OCF3 |
| 6-5561 | 3-cyanophenyl | H | H | F | F | H | H | CF3 | H | heptafluoroisopropyl | H | OCF3 |
| 6-5562 | 4-cyanophenyl | H | H | F | F | H | H | CF3 | H | heptafluoroisopropyl | H | OCF3 |
| 6-5577 | 2-chloropyridin-3-yl | H | H | F | F | H | H | CF3 | H | heptafluoroisopropyl | H | OCF3 |
| 6-5602 | phenyl | H | H | F | F | H | H | CF3 | H | nonafluoro-2-butyl | H | OCF3 |
| 6-5617 | 3-cyanophenyl | H | H | F | F | H | H | CF3 | H | nonafluoro-2-butyl | H | OCF3 |
| 6-5633 | 2-chloropyridin-3-yl | H | H | F | F | H | H | CF3 | H | nonafluoro-2-butyl | H | OCF3 |
| 6-5658 | phenyl | H | H | F | F | H | H | CF3 | H | heptafluoroisopropyl | H | CF3 |
| 6-5678 | 3-cyanophenyl | H | H | F | F | H | H | CF3 | H | heptafluoroisopropyl | H | CF3 |
| 6-5694 | 2-chloropyridin-3-yl | H | H | F | F | H | H | CF3 | H | heptafluoroisopropyl | H | CF3 |
| 6-5726 | phenyl | H | H | F | F | H | H | CF3 | H | nonafluoro-2-butyl | H | CF3 |
| 6-5746 | 3-cyanophenyl | H | H | F | F | H | H | CF3 | H | nonafluoro-2-butyl | H | CF3 |
| 6-5747 | 4-cyanophenyl | H | H | F | F | H | H | CF3 | H | nonafluoro-2-butyl | H | CF3 |
| 6-5762 | 2-chloropyridin-3-yl | H | H | F | F | H | H | CF3 | H | nonafluoro-2-butyl | H | CF3 |
| 6-5794 | phenyl | H | H | F | F | F | H | Cl | H | pentafluoroethyl | H | Cl |
| 6-5795 | phenyl | H | H | F | F | F | H | Br | H | heptafluoroisopropyl | H | Br |
| 6-5796 | phenyl | H | H | F | F | F | H | Br | H | heptafluoroisopropyl | H | OCF3 |
| 6-5797 | phenyl | H | H | F | F | F | H | I | H | heptafluoroisopropyl | H | CF3 |
| 6-5798 | phenyl | H | H | F | F | F | H | CF3 | H | heptafluoroisopropyl | H | CF3 |
| 6-5799 | phenyl | H | H | F | F | F | H | I | H | nonafluoro-2-butyl | H | I |
| 6-5800 | phenyl | H | H | F | F | F | H | Cl | H | nonafluoro-2-butyl | H | OCF3 |
| 6-5801 | phenyl | H | H | F | F | F | H | Br | H | nonafluoro-2-butyl | H | CF3 |
| 6-5802 | phenyl | H | H | F | F | F | H | CF3 | H | nonafluoro-2-butyl | H | CF3 |
| 6-5803 | phenyl | H | H | H | H | F | H | Cl | H | pentafluoroethyl | H | Cl |
| 6-5804 | phenyl | H | H | H | H | F | H | Br | H | heptafluoroisopropyl | H | Br |
| 6-5805 | phenyl | H | H | H | H | F | H | Br | H | heptafluoroisopropyl | H | OCF3 |
| 6-5806 | phenyl | H | H | H | H | F | H | I | H | heptafluoroisopropyl | H | CF3 |
| 6-5807 | phenyl | H | H | H | H | F | H | CF3 | H | heptafluoroisopropyl | H | CF3 |
| 6-5808 | phenyl | H | H | H | H | F | H | I | H | nonafluoro-2-butyl | H | I |
| 6-5809 | phenyl | H | H | H | H | F | H | Cl | H | nonafluoro-2-butyl | H | OCF3 |
| 6-5810 | phenyl | H | H | H | H | F | H | Br | H | nonafluoro-2-butyl | H | CF3 |
| 6-5811 | phenyl | H | H | H | H | F | H | CF3 | H | nonafluoro-2-butyl | H | CF3 |
| 6-5812 | phenyl | H | H | H | H | H | F | Cl | H | pentafluoroethyl | H | Cl |
| 6-5813 | phenyl | H | H | H | H | H | F | Br | H | heptafluoroisopropyl | H | Br |
| 6-5814 | phenyl | H | H | H | H | H | F | Br | H | heptafluoroisopropyl | H | OCF3 |
| 6-5815 | phenyl | H | H | H | H | H | F | I | H | heptafluoroisopropyl | H | CF3 |
| 6-5816 | phenyl | H | H | H | H | H | F | CF3 | H | heptafluoroisopropyl | H | CF3 |
| 6-5817 | phenyl | H | H | H | H | H | F | I | H | nonafluoro-2-butyl | H | I |
| 6-5818 | phenyl | H | H | H | H | H | F | Cl | H | nonafluoro-2-butyl | H | OCF3 |
| 6-5819 | phenyl | H | H | H | H | H | F | Br | H | nonafluoro-2-butyl | H | CF3 |
| 6-5820 | phenyl | H | H | H | H | H | F | CF3 | H | nonafluoro-2-butyl | H | CF3 |
| 6-5821 | phenyl | H | H | F | H | F | H | Cl | H | pentafluoroethyl | H | Cl |
| 6-5822 | phenyl | H | H | F | H | F | H | Br | H | heptafluoroisopropyl | H | Br |
| 6-5823 | phenyl | H | H | F | H | F | H | Br | H | heptafluoroisopropyl | H | OCF3 |
| 6-5824 | phenyl | H | H | F | H | F | H | I | H | heptafluoroisopropyl | H | CF3 |
| 6-5825 | phenyl | H | H | F | H | F | H | CF3 | H | heptafluoroisopropyl | H | CF3 |
| 6-5826 | phenyl | H | H | F | H | F | H | I | H | nonafluoro-2-butyl | H | I |
| 6-5827 | phenyl | H | H | F | H | F | H | Cl | H | nonafluoro-2-butyl | H | OCF3 |
| 6-5828 | phenyl | H | H | F | H | F | H | Br | H | nonafluoro-2-butyl | H | CF3 |
| 6-5829 | phenyl | H | H | F | H | F | H | CF3 | H | nonafluoro-2-butyl | H | CF3 |
| 6-5830 | phenyl | H | H | F | H | H | F | Cl | H | pentafluoroethyl | H | Cl |
| 6-5831 | phenyl | H | H | F | H | H | F | Br | H | heptafluoroisopropyl | H | Br |
| 6-5832 | phenyl | H | H | F | H | H | F | Br | H | heptafluoroisopropyl | H | OCF3 |

**Table 6(14)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 6-5833 | phenyl | H | H | F | H | H | F | I | H | heptafluoroisopropyl | H | CF3 |
| 6-5834 | phenyl | H | H | F | H | H | F | CF3 | H | heptafluoroisopropyl | H | CF3 |
| 6-5835 | phenyl | H | H | F | H | H | F | I | H | nonafluoro-2-butyl | H | I |
| 6-5836 | phenyl | H | H | F | H | H | F | Cl | H | nonafluoro-2-butyl | H | OCF3 |
| 6-5837 | phenyl | H | H | F | H | H | F | Br | H | nonafluoro-2-butyl | H | CF3 |
| 6-5838 | phenyl | H | H | F | H | H | F | CF3 | H | nonafluoro-2-butyl | H | CF3 |
| 6-5839 | phenyl | H | H | H | F | F | H | Cl | H | pentafluoroethyl | H | Cl |
| 6-5840 | phenyl | H | H | H | F | F | H | Br | H | heptafluoroisopropyl | H | Br |
| 6-5841 | phenyl | H | H | H | F | F | H | Br | H | heptafluoroisopropyl | H | OCF3 |
| 6-5842 | phenyl | H | H | H | F | F | H | I | H | heptafluoroisopropyl | H | CF3 |
| 6-5843 | phenyl | H | H | H | F | F | H | CF3 | H | heptafluoroisopropyl | H | CF3 |
| 6-5844 | phenyl | H | H | H | F | F | H | I | H | nonafluoro-2-butyl | H | I |
| 6-5845 | phenyl | H | H | H | F | F | H | Cl | H | nonafluoro-2-butyl | H | OCF3 |
| 6-5846 | phenyl | H | H | H | F | F | H | Br | H | nonafluoro-2-butyl | H | CF3 |
| 6-5847 | phenyl | H | H | H | F | F | H | CF3 | H | nonafluoro-2-butyl | H | CF3 |
| 6-5848 | phenyl | H | H | H | F | H | F | Cl | H | pentafluoroethyl | H | Cl |
| 6-5849 | phenyl | H | H | H | F | H | F | Br | H | heptafluoroisopropyl | H | Br |
| 6-5850 | phenyl | H | H | H | F | H | F | Br | H | heptafluoroisopropyl | H | OCF3 |
| 6-5851 | phenyl | H | H | H | F | H | F | I | H | heptafluoroisopropyl | H | CF3 |
| 6-5852 | phenyl | H | H | H | F | H | F | CF3 | H | heptafluoroisopropyl | H | CF3 |
| 6-5853 | phenyl | H | H | H | F | H | F | I | H | nonafluoro-2-butyl | H | I |
| 6-5854 | phenyl | H | H | H | F | H | F | Cl | H | nonafluoro-2-butyl | H | OCF3 |
| 6-5855 | phenyl | H | H | H | F | H | F | Br | H | nonafluoro-2-butyl | H | CF3 |
| 6-5856 | phenyl | H | H | H | F | H | F | CF3 | H | nonafluoro-2-butyl | H | CF3 |
| 6-5857 | phenyl | H | H | H | H | F | F | Cl | H | pentafluoroethyl | H | Cl |
| 6-5858 | phenyl | H | H | H | H | F | F | Br | H | heptafluoroisopropyl | H | Br |
| 6-5859 | phenyl | H | H | H | H | F | F | Br | H | heptafluoroisopropyl | H | OCF3 |
| 6-5860 | phenyl | H | H | H | H | F | F | I | H | heptafluoroisopropyl | H | CF3 |
| 6-5861 | phenyl | H | H | H | H | F | F | CF3 | H | heptafluoroisopropyl | H | CF3 |
| 6-5862 | phenyl | H | H | H | H | F | F | I | H | nonafluoro-2-butyl | H | I |
| 6-5863 | phenyl | H | H | H | H | F | F | Cl | H | nonafluoro-2-butyl | H | OCF3 |
| 6-5864 | phenyl | H | H | H | H | F | F | Br | H | nonafluoro-2-butyl | H | CF3 |
| 6-5865 | phenyl | H | H | H | H | F | F | CF3 | H | nonafluoro-2-butyl | H | CF3 |
| 6-5866 | phenyl | H | H | F | F | H | F | Cl | H | pentafluoroethyl | H | Cl |
| 6-5867 | phenyl | H | H | F | F | H | F | Br | H | heptafluoroisopropyl | H | Br |
| 6-5868 | phenyl | H | H | F | F | H | F | Br | H | heptafluoroisopropyl | H | OCF3 |
| 6-5869 | phenyl | H | H | F | F | H | F | I | H | heptafluoroisopropyl | H | CF3 |
| 6-5870 | phenyl | H | H | F | F | H | F | CF3 | H | heptafluoroisopropyl | H | CF3 |
| 6-5871 | phenyl | H | H | F | F | H | F | I | H | nonafluoro-2-butyl | H | I |
| 6-5872 | phenyl | H | H | F | F | H | F | Cl | H | nonafluoro-2-butyl | H | OCF3 |
| 6-5873 | phenyl | H | H | F | F | H | F | Br | H | nonafluoro-2-butyl | H | CF3 |
| 6-5874 | phenyl | H | H | F | F | H | F | CF3 | H | nonafluoro-2-butyl | H | CF3 |
| 6-5875 | phenyl | H | H | F | H | F | F | Cl | H | pentafluoroethyl | H | Cl |
| 6-5876 | phenyl | H | H | F | H | F | F | Br | H | heptafluoroisopropyl | H | Br |
| 6-5877 | phenyl | H | H | F | H | F | F | Br | H | heptafluoroisopropyl | H | OCF3 |
| 6-5878 | phenyl | H | H | F | H | F | F | I | H | heptafluoroisopropyl | H | CF3 |
| 6-5879 | phenyl | H | H | F | H | F | F | CF3 | H | heptafluoroisopropyl | H | CF3 |
| 6-5880 | phenyl | H | H | F | H | F | F | I | H | nonafluoro-2-butyl | H | I |
| 6-5881 | phenyl | H | H | F | H | F | F | Cl | H | nonafluoro-2-butyl | H | OCF3 |
| 6-5882 | phenyl | H | H | F | H | F | F | Br | H | nonafluoro-2-butyl | H | CF3 |
| 6-5883 | phenyl | H | H | F | H | F | F | CF3 | H | nonafluoro-2-butyl | H | CF3 |
| 6-5884 | phenyl | H | H | H | F | F | F | Cl | H | pentafluoroethyl | H | Cl |
| 6-5885 | phenyl | H | H | H | F | F | F | Br | H | heptafluoroisopropyl | H | Br |
| 6-5886 | phenyl | H | H | H | F | F | F | Br | H | heptafluoroisopropyl | H | OCF3 |
| 6-5887 | phenyl | H | H | H | F | F | F | I | H | heptafluoroisopropyl | H | CF3 |
| 6-5888 | phenyl | H | H | H | F | F | F | CF3 | H | heptafluoroisopropyl | H | CF3 |
| 6-5889 | phenyl | H | H | H | F | F | F | I | H | nonafluoro-2-butyl | H | I |
| 6-5890 | phenyl | H | H | H | F | F | F | Cl | H | nonafluoro-2-butyl | H | OCF3 |
| 6-5891 | phenyl | H | H | H | F | F | F | Br | H | nonafluoro-2-butyl | H | CF3 |
| 6-5892 | phenyl | H | H | H | F | F | F | CF3 | H | nonafluoro-2-butyl | H | CF3 |
| 6-5893 | phenyl | H | H | F | F | F | F | Cl | H | pentafluoroethyl | H | Cl |
| 6-5894 | phenyl | H | H | F | F | F | F | Br | H | heptafluoroisopropyl | H | Br |
| 6-5895 | phenyl | H | H | F | F | F | F | Br | H | heptafluoroisopropyl | H | OCF3 |
| 6-5896 | phenyl | H | H | F | F | F | F | I | H | heptafluoroisopropyl | H | CF3 |

**Table 6(15)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 6-5897 | phenyl | H | H | F | F | F | F | CF3 | H | heptafluoroisopropyl | H | CF3 |
| 6-5898 | phenyl | H | H | F | F | F | F | I | H | nonafluoro-2-butyl | H | I |
| 6-5899 | phenyl | H | H | F | F | F | F | Cl | H | nonafluoro-2-butyl | H | OCF3 |
| 6-5900 | phenyl | H | H | F | F | F | F | Br | H | nonafluoro-2-butyl | H | CF3 |
| 6-5901 | phenyl | H | H | F | F | F | F | CF3 | H | nonafluoro-2-butyl | H | CF3 |
| 6-5902 | phenyl | H | H | F | H | H | H | Br | H | heptafluoroisopropyl | H | Cl |
| 6-5903 | 3-fluorophenyl | H | H | H | H | H | H | I | H | nonafluoro-2-butyl | H | I |
| 6-5904 | 4-fluorophenyl | H | H | H | H | H | H | I | H | nonafluoro-2-butyl | H | I |
| 6-5905 | 3-fluorophenyl | H | H | H | H | H | H | Br | H | nonafluoro-2-butyl | H | Br |
| 6-5906 | 4-fluorophenyl | H | H | H | H | H | H | Br | H | nonafluoro-2-butyl | H | Br |
| 6-5907 | phenyl | H | H | F | H | H | H | Br | H | heptafluoroisopropyl | H | OCF3 |
| 6-5908 | 2-chloropyridin-3-yl | H | H | F | H | H | H | Br | H | heptafluoroisopropyl | H | OCF3 |
| 6-5909 | 4-cyanophenyl | H | H | F | H | H | H | Br | H | heptafluoroisopropyl | H | OCF3 |
| 6-5910 | phenyl | H | H | F | H | H | H | Br | H | heptafluoroisopropyl | H | C2F5 |
| 6-5911 | 4-cyanophenyl | H | H | F | H | H | H | Br | H | heptafluoroisopropyl | H | C2F5 |
| 6-5912 | 2-chloro-4-(methylsulfonyl)phenyl | H | H | H | H | H | H | I | H | heptafluoroisopropyl | H | CF3 |
| 6-5913 | phenyl | H | H | H | H | H | H | Br | H | pentafluoroethyl | H | CF3 |
| 6-5914 | 2-chloropyridin-3-yl | H | H | H | H | H | H | Br | H | pentafluoroethyl | H | CF3 |

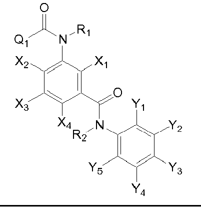

**Table 7(1)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 7-1 | phenyl | Me | H | H | H | H | H | F | H | nonafluoro-2-butyl | H | F |
| 7-2 | 3-cyanophenyl | Me | H | H | H | H | H | F | H | nonafluoro-2-butyl | H | F |
| 7-3 | 4-cyanophenyl | Me | H | H | H | H | H | F | H | nonafluoro-2-butyl | H | F |
| 7-4 | 2-chloropyridin-3-yl | Me | H | H | H | H | H | F | H | nonafluoro-2-butyl | H | F |
| 7-5 | phenyl | Me | H | H | H | H | H | Cl | H | nonafluoro-2-butyl | H | Cl |
| 7-12 | 3-cyanophenyl | Me | H | H | H | H | H | Cl | H | nonafluoro-2-butyl | H | Cl |
| 7-13 | 4-cyanophenyl | Me | H | H | H | H | H | Cl | H | nonafluoro-2-butyl | H | Cl |
| 7-24 | 2-chloropyridin-3-yl | Me | H | H | H | H | H | Cl | H | nonafluoro-2-butyl | H | Cl |
| 7-38 | phenyl | Me | H | H | H | H | H | Br | H | nonafluoro-2-butyl | H | Br |
| 7-39 | 2-fluorophenyl | Me | H | H | H | H | H | Br | H | nonafluoro-2-butyl | H | Br |
| 7-45 | 3-cyanophenyl | Me | H | H | H | H | H | Br | H | nonafluoro-2-butyl | H | Br |
| 7-46 | 4-cyanophenyl | Me | H | H | H | H | H | Br | H | nonafluoro-2-butyl | H | Br |
| 7-47 | 2,6-difluorophenyl | Me | H | H | H | H | H | Br | H | nonafluoro-2-butyl | H | Br |
| 7-57 | 2-chloropyridin-3-yl | Me | H | H | H | H | H | Br | H | nonafluoro-2-butyl | H | Br |
| 7-60 | 6-chloropyridin-3-yl | Me | H | H | H | H | H | Br | H | nonafluoro-2-butyl | H | Br |
| 7-71 | phenyl | Me | H | H | H | H | H | I | H | nonafluoro-2-butyl | H | I |
| 7-72 | 2-fluorophenyl | Me | H | H | H | H | H | I | H | nonafluoro-2-butyl | H | I |
| 7-78 | 3-cyanophenyl | Me | H | H | H | H | H | I | H | nonafluoro-2-butyl | H | I |
| 7-79 | 4-cyanophenyl | Me | H | H | H | H | H | I | H | nonafluoro-2-butyl | H | I |
| 7-80 | 2,6-difluorophenyl | Me | H | H | H | H | H | I | H | nonafluoro-2-butyl | H | I |
| 7-90 | 2-chloropyridin-3-yl | Me | H | H | H | H | H | I | H | nonafluoro-2-butyl | H | I |
| 7-93 | 6-chloropyridin-3-yl | Me | H | H | H | H | H | I | H | nonafluoro-2-butyl | H | I |
| 7-104 | phenyl | Me | H | H | H | H | H | F | H | nonafluoro-2-butyl | H | Cl |
| 7-105 | phenyl | Me | H | H | H | H | H | F | H | nonafluoro-2-butyl | H | Br |
| 7-106 | phenyl | Me | H | H | H | H | H | F | H | nonafluoro-2-butyl | H | I |
| 7-107 | phenyl | Me | H | H | H | H | H | Cl | H | nonafluoro-2-butyl | H | Br |
| 7-108 | phenyl | Me | H | H | H | H | H | Cl | H | nonafluoro-2-butyl | H | I |
| 7-109 | phenyl | Me | H | H | H | H | H | Br | H | nonafluoro-2-butyl | H | I |
| 7-110 | phenyl | Me | H | H | H | H | H | F | H | heptafluoroisopropyl | H | CF3 |
| 7-111 | phenyl | Me | H | H | H | H | H | Cl | H | heptafluoroisopropyl | H | CF3 |
| 7-131 | 3-cyanophenyl | Me | H | H | H | H | H | Cl | H | heptafluoroisopropyl | H | CF3 |
| 7-132 | 4-cyanophenyl | Me | H | H | H | H | H | Cl | H | heptafluoroisopropyl | H | CF3 |
| 7-147 | 2-chloropyridin-3-yl | Me | H | H | H | H | H | Cl | H | heptafluoroisopropyl | H | CF3 |
| 7-189 | phenyl | Me | H | H | H | H | H | F | H | nonafluoro-2-butyl | H | CF3 |
| 7-190 | phenyl | Me | H | H | H | H | H | Cl | H | nonafluoro-2-butyl | H | CF3 |
| 7-210 | 3-cyanophenyl | Me | H | H | H | H | H | Cl | H | nonafluoro-2-butyl | H | CF3 |
| 7-211 | 4-cyanophenyl | Me | H | H | H | H | H | Cl | H | nonafluoro-2-butyl | H | CF3 |
| 7-226 | 2-chloropyridin-3-yl | Me | H | H | H | H | H | Cl | H | nonafluoro-2-butyl | H | CF3 |
| 7-268 | phenyl | Me | H | H | H | H | H | Br | H | heptafluoroisopropyl | H | CF3 |
| 7-269 | 2-fluorophenyl | Me | H | H | H | H | H | Br | H | heptafluoroisopropyl | H | CF3 |
| 7-270 | 3-fluorophenyl | Me | H | H | H | H | H | Br | H | heptafluoroisopropyl | H | CF3 |
| 7-271 | 4-fluorophenyl | Me | H | H | H | H | H | Br | H | heptafluoroisopropyl | H | CF3 |
| 7-285 | 3-nitrophenyl | Me | H | H | H | H | H | Br | H | heptafluoroisopropyl | H | CF3 |
| 7-286 | 4-nitrophenyl | Me | H | H | H | H | H | Br | H | heptafluoroisopropyl | H | CF3 |
| 7-288 | 3-cyanophenyl | Me | H | H | H | H | H | Br | H | heptafluoroisopropyl | H | CF3 |
| 7-289 | 4-cyanophenyl | Me | H | H | H | H | H | Br | H | heptafluoroisopropyl | H | CF3 |
| 7-290 | 2,6-difluorophenyl | Me | H | H | H | H | H | Br | H | heptafluoroisopropyl | H | CF3 |
| 7-299 | 3,5-dicyanophenyl | Me | H | H | H | H | H | Br | H | heptafluoroisopropyl | H | CF3 |
| 7-300 | 4-cyano-2-fluorophenyl | Me | H | H | H | H | H | Br | H | heptafluoroisopropyl | H | CF3 |
| 7-301 | 2-chloro-4-cyanophenyl | Me | H | H | H | H | H | Br | H | heptafluoroisopropyl | H | CF3 |
| 7-303 | 2-fluoropyridin-3-yl | Me | H | H | H | H | H | Br | H | heptafluoroisopropyl | H | CF3 |
| 7-304 | 2-chloropyridin-3-yl | Me | H | H | H | H | H | Br | H | heptafluoroisopropyl | H | CF3 |
| 7-305 | 2-bromopyridin-3-yl | Me | H | H | H | H | H | Br | H | heptafluoroisopropyl | H | CF3 |
| 7-311 | 6-chloropyridin-3-yl | Me | H | H | H | H | H | Br | H | heptafluoroisopropyl | H | CF3 |
| 7-316 | 6-cyanopyridin-3-yl | Me | H | H | H | H | H | Br | H | heptafluoroisopropyl | H | CF3 |

**Table 7(2)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 7-346 | phenyl | Me | H | H | H | H | H | Br | H | nonafluoro-2-butyl | H | CF3 |
| 7-347 | 2-fluorophenyl | Me | H | H | H | H | H | Br | H | nonafluoro-2-butyl | H | CF3 |
| 7-348 | 3-fluorophenyl | Me | H | H | H | H | H | Br | H | nonafluoro-2-butyl | H | CF3 |
| 7-349 | 4-fluorophenyl | Me | H | H | H | H | H | Br | H | nonafluoro-2-butyl | H | CF3 |
| 7-366 | 3-cyanophenyl | Me | H | H | H | H | H | Br | H | nonafluoro-2-butyl | H | CF3 |
| 7-367 | 4-cyanophenyl | Me | H | H | H | H | H | Br | H | nonafluoro-2-butyl | H | CF3 |
| 7-368 | 2,6-difluorophenyl | Me | H | H | H | H | H | Br | H | nonafluoro-2-butyl | H | CF3 |
| 7-382 | 2-chloropyridin-3-yl | Me | H | H | H | H | H | Br | H | nonafluoro-2-butyl | H | CF3 |
| 7-389 | 6-chloropyridin-3-yl | Me | H | H | H | H | H | Br | H | nonafluoro-2-butyl | H | CF3 |
| 7-424 | phenyl | Me | H | H | H | H | H | I | H | heptafluoroisopropyl | H | CF3 |
| 7-425 | 2-fluorophenyl | Me | H | H | H | H | H | I | H | heptafluoroisopropyl | H | CF3 |
| 7-426 | 3-fluorophenyl | Me | H | H | H | H | H | I | H | heptafluoroisopropyl | H | CF3 |
| 7-427 | 4-fluorophenyl | Me | H | H | H | H | H | I | H | heptafluoroisopropyl | H | CF3 |
| 7-428 | 2-chlorophenyl | Me | H | H | H | H | H | I | H | heptafluoroisopropyl | H | CF3 |
| 7-444 | 3-cyanophenyl | Me | H | H | H | H | H | I | H | heptafluoroisopropyl | H | CF3 |
| 7-445 | 4-cyanophenyl | Me | H | H | H | H | H | I | H | heptafluoroisopropyl | H | CF3 |
| 7-446 | 2,6-difluorophenyl | Me | H | H | H | H | H | I | H | heptafluoroisopropyl | H | CF3 |
| 7-449 | 2-chloro-4-fluorophenyl | Me | H | H | H | H | H | I | H | heptafluoroisopropyl | H | CF3 |
| 7-460 | 2-chloropyridin-3-yl | Me | H | H | H | H | H | I | H | heptafluoroisopropyl | H | CF3 |
| 7-467 | 6-chloropyridin-3-yl | Me | H | H | H | H | H | I | H | heptafluoroisopropyl | H | CF3 |
| 7-502 | phenyl | Me | H | H | H | H | H | I | H | nonafluoro-2-butyl | H | CF3 |
| 7-504 | 3-fluorophenyl | Me | H | H | H | H | H | I | H | nonafluoro-2-butyl | H | CF3 |
| 7-505 | 4-fluorophenyl | Me | H | H | H | H | H | I | H | nonafluoro-2-butyl | H | CF3 |
| 7-522 | 3-cyanophenyl | Me | H | H | H | H | H | I | H | nonafluoro-2-butyl | H | CF3 |
| 7-523 | 4-cyanophenyl | Me | H | H | H | H | H | I | H | nonafluoro-2-butyl | H | CF3 |
| 7-538 | 2-chloropyridin-3-yl | Me | H | H | H | H | H | I | H | nonafluoro-2-butyl | H | CF3 |
| 7-580 | phenyl | Me | H | H | H | H | H | OCF3 | H | heptafluoroisopropyl | H | OCF3 |
| 7-611 | 2-chloropyridin-3-yl | Me | H | H | H | H | H | OCF3 | H | heptafluoroisopropyl | H | OCF3 |
| 7-636 | phenyl | Me | H | H | H | H | H | OCF3 | H | nonafluoro-2-butyl | H | OCF3 |
| 7-667 | 2-chloropyridin-3-yl | Me | H | H | H | H | H | OCF3 | H | nonafluoro-2-butyl | H | OCF3 |
| 7-692 | phenyl | Me | H | H | H | H | H | CF3 | H | heptafluoroisopropyl | H | OCF3 |
| 7-723 | 2-chloropyridin-3-yl | Me | H | H | H | H | H | CF3 | H | heptafluoroisopropyl | H | OCF3 |
| 7-748 | phenyl | Me | H | H | H | H | H | CF3 | H | nonafluoro-2-butyl | H | OCF3 |
| 7-779 | 2-chloropyridin-3-yl | Me | H | H | H | H | H | CF3 | H | nonafluoro-2-butyl | H | OCF3 |
| 7-804 | phenyl | Me | H | H | H | H | H | CF3 | H | heptafluoroisopropyl | H | CF3 |
| 7-824 | 3-cyanophenyl | Me | H | H | H | H | H | CF3 | H | heptafluoroisopropyl | H | CF3 |
| 7-825 | 4-cyanophenyl | Me | H | H | H | H | H | CF3 | H | heptafluoroisopropyl | H | CF3 |
| 7-840 | 2-chloropyridin-3-yl | Me | H | H | H | H | H | CF3 | H | heptafluoroisopropyl | H | CF3 |
| 7-841 | 2-bromopyridin-3-yl | Me | H | H | H | H | H | CF3 | H | heptafluoroisopropyl | H | CF3 |
| 7-872 | phenyl | Me | H | H | H | H | H | CF3 | H | nonafluoro-2-butyl | H | CF3 |
| 7-892 | 3-cyanophenyl | Me | H | H | H | H | H | CF3 | H | nonafluoro-2-butyl | H | CF3 |
| 7-893 | 4-cyanophenyl | Me | H | H | H | H | H | CF3 | H | nonafluoro-2-butyl | H | CF3 |
| 7-908 | 2-chloropyridin-3-yl | Me | H | H | H | H | H | CF3 | H | nonafluoro-2-butyl | H | CF3 |
| 7-940 | phenyl | Me | H | F | H | H | H | F | H | heptafluoroisopropyl | H | F |
| 7-941 | 3-cyanophenyl | Me | H | F | H | H | H | F | H | heptafluoroisopropyl | H | F |
| 7-942 | 4-cyanophenyl | Me | H | F | H | H | H | F | H | heptafluoroisopropyl | H | F |
| 7-943 | 2-chloropyridin-3-yl | Me | H | F | H | H | H | F | H | heptafluoroisopropyl | H | F |
| 7-944 | phenyl | Me | H | F | H | H | H | F | H | nonafluoro-2-butyl | H | F |
| 7-945 | 3-cyanophenyl | Me | H | F | H | H | H | F | H | nonafluoro-2-butyl | H | F |
| 7-946 | 4-cyanophenyl | Me | H | F | H | H | H | F | H | nonafluoro-2-butyl | H | F |
| 7-947 | 2-chloropyridin-3-yl | Me | H | F | H | H | H | F | H | nonafluoro-2-butyl | H | F |
| 7-948 | phenyl | Me | H | F | H | H | H | Cl | H | heptafluoroisopropyl | H | Cl |
| 7-968 | 3-cyanophenyl | Me | H | F | H | H | H | Cl | H | heptafluoroisopropyl | H | Cl |
| 7-969 | 4-cyanophenyl | Me | H | F | H | H | H | Cl | H | heptafluoroisopropyl | H | Cl |
| 7-984 | 2-chloropyridin-3-yl | Me | H | F | H | H | H | Cl | H | heptafluoroisopropyl | H | Cl |
| 7-1026 | phenyl | Me | H | F | H | H | H | Cl | H | nonafluoro-2-butyl | H | Cl |
| 7-1046 | 3-cyanophenyl | Me | H | F | H | H | H | Cl | H | nonafluoro-2-butyl | H | Cl |
| 7-1047 | 4-cyanophenyl | Me | H | F | H | H | H | Cl | H | nonafluoro-2-butyl | H | Cl |
| 7-1062 | 2-chloropyridin-3-yl | Me | H | F | H | H | H | Cl | H | nonafluoro-2-butyl | H | Cl |
| 7-1104 | phenyl | Me | H | F | H | H | H | Br | H | heptafluoroisopropyl | H | Br |
| 7-1105 | 2-fluorophenyl | Me | H | F | H | H | H | Br | H | heptafluoroisopropyl | H | Br |
| 7-1106 | 3-fluorophenyl | Me | H | F | H | H | H | Br | H | heptafluoroisopropyl | H | Br |
| 7-1107 | 4-fluorophenyl | Me | H | F | H | H | H | Br | H | heptafluoroisopropyl | H | Br |
| 7-1124 | 3-cyanophenyl | Me | H | F | H | H | H | Br | H | heptafluoroisopropyl | H | Br |

**Table 7(3)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 7-1125 | 4-cyanophenyl | Me | H | F | H | H | H | Br | H | heptafluoroisopropyl | H | Br |
| 7-1126 | 2,6-difluorophenyl | Me | H | F | H | H | H | Br | H | heptafluoroisopropyl | H | Br |
| 7-1140 | 2-chloropyridin-3-yl | Me | H | F | H | H | H | Br | H | heptafluoroisopropyl | H | Br |
| 7-1147 | 6-chloropyridin-3-yl | Me | H | F | H | H | H | Br | H | heptafluoroisopropyl | H | Br |
| 7-1182 | phenyl | Me | H | F | H | H | H | Br | H | nonafluoro-2-butyl | H | Br |
| 7-1183 | 2-fluorophenyl | Me | H | F | H | H | H | Br | H | nonafluoro-2-butyl | H | Br |
| 7-1184 | 3-fluorophenyl | Me | H | F | H | H | H | Br | H | nonafluoro-2-butyl | H | Br |
| 7-1185 | 4-fluorophenyl | Me | H | F | H | H | H | Br | H | nonafluoro-2-butyl | H | Br |
| 7-1202 | 3-cyanophenyl | Me | H | F | H | H | H | Br | H | nonafluoro-2-butyl | H | Br |
| 7-1203 | 4-cyanophenyl | Me | H | F | H | H | H | Br | H | nonafluoro-2-butyl | H | Br |
| 7-1204 | 2,6-difluorophenyl | Me | H | F | H | H | H | Br | H | nonafluoro-2-butyl | H | Br |
| 7-1218 | 2-chloropyridin-3-yl | Me | H | F | H | H | H | Br | H | nonafluoro-2-butyl | H | Br |
| 7-1225 | 6-chloropyridin-3-yl | Me | H | F | H | H | H | Br | H | nonafluoro-2-butyl | H | Br |
| 7-1260 | phenyl | Me | H | F | H | H | H | I | H | heptafluoroisopropyl | H | I |
| 7-1261 | 2-fluorophenyl | Me | H | F | H | H | H | I | H | heptafluoroisopropyl | H | I |
| 7-1262 | 3-fluorophenyl | Me | H | F | H | H | H | I | H | heptafluoroisopropyl | H | I |
| 7-1263 | 4-fluorophenyl | Me | H | F | H | H | H | I | H | heptafluoroisopropyl | H | I |
| 7-1280 | 3-cyanophenyl | Me | H | F | H | H | H | I | H | heptafluoroisopropyl | H | I |
| 7-1281 | 4-cyanophenyl | Me | H | F | H | H | H | I | H | heptafluoroisopropyl | H | I |
| 7-1282 | 2,6-difluorophenyl | Me | H | F | H | H | H | I | H | heptafluoroisopropyl | H | I |
| 7-1296 | 2-chloropyridin-3-yl | Me | H | F | H | H | H | I | H | heptafluoroisopropyl | H | I |
| 7-1303 | 6-chloropyridin-3-yl | Me | H | F | H | H | H | I | H | heptafluoroisopropyl | H | I |
| 7-1338 | phenyl | Me | H | F | H | H | H | I | H | nonafluoro-2-butyl | H | I |
| 7-1339 | 2-fluorophenyl | Me | H | F | H | H | H | I | H | nonafluoro-2-butyl | H | I |
| 7-1340 | 3-fluorophenyl | Me | H | F | H | H | H | I | H | nonafluoro-2-butyl | H | I |
| 7-1341 | 4-fluorophenyl | Me | H | F | H | H | H | I | H | nonafluoro-2-butyl | H | I |
| 7-1358 | 3-cyanophenyl | Me | H | F | H | H | H | I | H | nonafluoro-2-butyl | H | I |
| 7-1359 | 4-cyanophenyl | Me | H | F | H | H | H | I | H | nonafluoro-2-butyl | H | I |
| 7-1360 | 2,6-difluorophenyl | Me | H | F | H | H | H | I | H | nonafluoro-2-butyl | H | I |
| 7-1374 | 2-chloropyridin-3-yl | Me | H | F | H | H | H | I | H | nonafluoro-2-butyl | H | I |
| 7-1381 | 6-chloropyridin-3-yl | Me | H | F | H | H | H | I | H | nonafluoro-2-butyl | H | I |
| 7-1416 | phenyl | Me | H | F | H | H | H | F | H | heptafluoroisopropyl | H | CF3 |
| 7-1417 | phenyl | Me | H | F | H | H | H | Cl | H | heptafluoroisopropyl | H | CF3 |
| 7-1428 | 3-iodophenyl | Me | H | F | H | H | H | Cl | H | pentafluoroethyl | H | CF3 |
| 7-1429 | 4-iodophenyl | Me | H | F | H | H | H | Cl | H | pentafluoroethyl | H | CF3 |
| 7-1431 | (3-trifluoromethyl) phenyl | Et | H | F | H | H | H | Cl | H | heptafluoroisopropyl | H | CF3 |
| 7-1436 | 2-cyanophenyl | Me | H | F | H | H | H | Cl | H | pentafluoroethyl | H | C2F5 |
| 7-1437 | 3-cyanophenyl | Me | H | F | H | H | H | Cl | H | heptafluoroisopropyl | H | CF3 |
| 7-1438 | 4-cyanophenyl | Me | H | F | H | H | H | Cl | H | heptafluoroisopropyl | H | CF3 |
| 7-1441 | 2,4-dichlorophenyl | Me | H | F | H | H | H | Cl | H | heptafluoroisopropyl | H | C2F5 |
| 7-1442 | 2-chloro-4-fluorophenyl | n-Pr | H | F | H | H | H | Cl | H | heptafluoroisopropyl | H | CF3 |
| 7-1445 | 2-bromo-4-chlorophenyl | Me | H | F | H | H | H | Cl | H | heptafluoroisopropyl | H | n-C3F7 |
| 7-1452 | 2-fluoropyridin-3-yl | Me | H | F | H | H | H | Cl | H | pentafluoroethyl | H | CF3 |
| 7-1453 | 2-chloropyridin-3-yl | Me | H | F | H | H | H | Cl | H | heptafluoroisopropyl | H | CF3 |
| 7-1459 | 6-fluoropyridin-3-yl | i-Pr | H | F | H | H | H | Cl | H | heptafluoroisopropyl | H | CF3 |
| 7-1466 | 5-fluoropyridin-3-yl | Me | H | F | H | H | H | Cl | H | heptafluoroisopropyl | H | C2F5 |
| 7-1468 | 5-bromopyridin-3-yl | Me | H | F | H | H | H | Cl | H | heptafluoroisopropyl | H | n-C3F7 |
| 7-1469 | 5-iodopyridin-3-yl | Me | H | F | H | H | H | Cl | H | heptafluoroisopropyl | H | i-C3F7 |
| 7-1471 | 5-nitropyridin-3-yl | Me | H | F | H | H | H | Cl | H | pentafluoroethyl | H | CF3 |
| 7-1473 | 4-fluoropyridin-3-yl | Me | H | F | H | H | H | Cl | H | pentafluoroethyl | H | CF3 |
| 7-1475 | 4-bromopyridin-3-yl | CH2CH=CH2 | H | F | H | H | H | Cl | H | heptafluoroisopropyl | H | n-C3F7 |
| 7-1476 | 4-iodopyridin-3-yl | Me | H | F | H | H | H | Cl | H | heptafluoroisopropyl | H | i-C3F7 |
| 7-1482 | pyridin-4-yl | CN | H | F | H | H | H | Cl | H | heptafluoroisopropyl | H | CF3 |
| 7-1494 | 4-cyanopyridin-2-yl | Me | H | F | H | H | H | Cl | H | pentafluoroethyl | H | CF3 |
| 7-1495 | phenyl | Me | H | F | H | H | H | F | H | nonafluoro-2-butyl | H | CF3 |
| 7-1496 | phenyl | Me | H | F | H | H | H | Cl | H | nonafluoro-2-butyl | H | CF3 |
| 7-1504 | 3-bromophenyl | CH2C≡CH | H | F | H | H | H | Cl | H | nonafluoro-2-butyl | H | CF3 |
| 7-1509 | (2-trifluoromethyl) phenyl | Me | H | F | H | H | H | Cl | H | nonafluoro-2-butyl | H | C2F5 |
| 7-1511 | (4-trifluoromethyl) phenyl | NH2 | H | F | H | H | H | Cl | H | nonafluoro-2-butyl | H | CF3 |
| 7-1516 | 3-cyanophenyl | Me | H | F | H | H | H | Cl | H | nonafluoro-2-butyl | H | CF3 |
| 7-1517 | 4-cyanophenyl | Me | H | F | H | H | H | Cl | H | nonafluoro-2-butyl | H | CF3 |
| 7-1524 | 2-bromo-4-chlorophenyl | C(O)OMe | H | F | H | H | H | Cl | H | nonafluoro-2-butyl | H | CF3 |
| 7-1526 | 2-chloro-4-nitrophenyl | Me | H | F | H | H | H | Cl | H | nonafluoro-2-butyl | H | i-C3F7 |
| 7-1532 | 2-chloropyridin-3-yl | Me | H | F | H | H | H | Cl | H | nonafluoro-2-butyl | H | CF3 |

**Table 7(4)**

| compound | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 7-1545 | 5-fluoropyridin-3-yl | C(O)OEt | H | F | H | H | H | Cl | H | nonafluoro-2-butyl | H | C2F5 |
| 7-1547 | 5-bromopyridin-3-yl | Me | H | F | H | H | H | Cl | H | nonafluoro-2-butyl | H | n-C3F7 |
| 7-1548 | 5-iodopyridin-3-yl | Me | H | F | H | H | H | Cl | H | nonafluoro-2-butyl | H | i-C3F7 |
| 7-1552 | 4-fluoropyridin-3-yl | Me | H | F | H | H | H | Cl | H | nonafluoro-2-butyl | H | C2F5 |
| 7-1554 | 4-bromopyridin-3-yl | C(O)C(O)Me | H | F | H | H | H | Cl | H | nonafluoro-2-butyl | H | CF3 |
| 7-1556 | 4-(trifluoromethyl)pyridin-3-yl | Me | H | F | H | H | H | Cl | H | nonafluoro-2-butyl | H | n-C3F7 |
| 7-1557 | 4-nitropyridin-3-yl | C(O)C(O)Et | H | F | H | H | H | Cl | H | nonafluoro-2-butyl | H | i-C3F7 |
| 7-1571 | 5-iodopyridin-2-yl | C(O)Me | H | F | H | H | H | Cl | H | nonafluoro-2-butyl | H | CF3 |
| 7-1573 | 4-cyanopyridin-2-yl | C(O)Et | H | F | H | H | H | Cl | H | nonafluoro-2-butyl | H | C2F5 |
| 7-1574 | phenyl | Me | H | F | H | H | H | Br | H | heptafluoroisopropyl | H | CF3 |
| 7-1575 | 2-fluorophenyl | Me | H | F | H | H | H | Br | H | heptafluoroisopropyl | H | CF3 |
| 7-1576 | 3-fluorophenyl | Me | H | F | H | H | H | Br | H | heptafluoroisopropyl | H | CF3 |
| 7-1577 | 4-fluorophenyl | Me | H | F | H | H | H | Br | H | heptafluoroisopropyl | H | CF3 |
| 7-1578 | 2-chlorophenyl | Me | H | F | H | H | H | Br | H | heptafluoroisopropyl | H | CF3 |
| 7-1579 | 3-chlorophenyl | Me | H | F | H | H | H | Br | H | heptafluoroisopropyl | H | CF3 |
| 7-1580 | 4-chlorophenyl | Me | H | F | H | H | H | Br | H | heptafluoroisopropyl | H | CF3 |
| 7-1581 | 2-bromophenyl | Me | H | F | H | H | H | Br | H | heptafluoroisopropyl | H | CF3 |
| 7-1582 | 3-bromophenyl | Me | H | F | H | H | H | Br | H | heptafluoroisopropyl | H | CF3 |
| 7-1583 | 4-bromophenyl | Me | H | F | H | H | H | Br | H | heptafluoroisopropyl | H | CF3 |
| 7-1584 | 2-iodophenyl | Me | H | F | H | H | H | Br | H | heptafluoroisopropyl | H | CF3 |
| 7-1585 | 3-iodophenyl | Me | H | F | H | H | H | Br | H | heptafluoroisopropyl | H | CF3 |
| 7-1586 | 4-iodophenyl | Me | H | F | H | H | H | Br | H | heptafluoroisopropyl | H | CF3 |
| 7-1587 | (2-trifluoromethyl) phenyl | Me | H | F | H | H | H | Br | H | heptafluoroisopropyl | H | CF3 |
| 7-1588 | (3-trifluoromethyl) phenyl | S(O)2Me | H | F | H | H | H | Br | H | heptafluoroisopropyl | H | CF3 |
| 7-1589 | (4-trifluoromethyl) phenyl | Me | H | F | H | H | H | Br | H | heptafluoroisopropyl | H | CF3 |
| 7-1590 | 2-nitrophenyl | Me | H | F | H | H | H | Br | H | heptafluoroisopropyl | H | CF3 |
| 7-1591 | 3-nitrophenyl | Me | H | F | H | H | H | Br | H | heptafluoroisopropyl | H | CF3 |
| 7-1592 | 4-nitrophenyl | Me | H | F | H | H | H | Br | H | heptafluoroisopropyl | H | CF3 |
| 7-1593 | 2-cyanophenyl | Me | H | F | H | H | H | Br | H | heptafluoroisopropyl | H | CF3 |
| 7-1594 | 3-cyanophenyl | Me | H | F | H | H | H | Br | H | heptafluoroisopropyl | H | CF3 |
| 7-1595 | 4-cyanophenyl | Me | H | F | H | H | H | Br | H | heptafluoroisopropyl | H | CF3 |
| 7-1596 | 2,6-difluorophenyl | Me | H | F | H | H | H | Br | H | heptafluoroisopropyl | H | CF3 |
| 7-1597 | 3,4-dichlorophenyl | Me | H | F | H | H | H | Br | H | heptafluoroisopropyl | H | CF3 |
| 7-1598 | 2,4-dichlorophenyl | Me | H | F | H | H | H | Br | H | heptafluoroisopropyl | H | CF3 |
| 7-1599 | 2-chloro-4-fluorophenyl | Me | H | F | H | H | H | Br | H | heptafluoroisopropyl | H | CF3 |
| 7-1600 | 2-chloro-4,5-difluorophenyl | Me | H | F | H | H | H | Br | H | heptafluoroisopropyl | H | CF3 |
| 7-1601 | 4-bromo-2-chlorophenyl | Me | H | F | H | H | H | Br | H | heptafluoroisopropyl | H | CF3 |
| 7-1602 | 2-bromo-4-chlorophenyl | Me | H | F | H | H | H | Br | H | heptafluoroisopropyl | H | CF3 |
| 7-1603 | 2-bromo-4-fluorophenyl | Me | H | F | H | H | H | Br | H | heptafluoroisopropyl | H | CF3 |
| 7-1604 | 2-chloro-4-nitrophenyl | S(O)2Et | H | F | H | H | H | Br | H | heptafluoroisopropyl | H | CF3 |
| 7-1605 | 3,5-dicyanophenyl | Me | H | F | H | H | H | Br | H | heptafluoroisopropyl | H | CF3 |
| 7-1606 | 4-cyano-2-fluorophenyl | Me | H | F | H | H | H | Br | H | heptafluoroisopropyl | H | CF3 |
| 7-1607 | 2-chloro-4-cyanophenyl | Me | H | F | H | H | H | Br | H | heptafluoroisopropyl | H | CF3 |
| 7-1608 | pyridin-3-yl | Me | H | F | H | H | H | Br | H | heptafluoroisopropyl | H | CF3 |
| 7-1609 | 2-fluoropyridin-3-yl | Me | H | F | H | H | H | Br | H | heptafluoroisopropyl | H | CF3 |
| 7-1610 | 2-chloropyridin-3-yl | Me | H | F | H | H | H | Br | H | heptafluoroisopropyl | H | CF3 |
| 7-1611 | 2-bromopyridin-3-yl | Me | H | F | H | H | H | Br | H | heptafluoroisopropyl | H | CF3 |
| 7-1612 | 2-iodopyridin-3-yl | Me | H | F | H | H | H | Br | H | heptafluoroisopropyl | H | CF3 |
| 7-1613 | 2-(trifluoromethyl) pyridin-3-yl | Me | H | F | H | H | H | Br | H | heptafluoroisopropyl | H | CF3 |
| 7-1614 | 2-nitropyridin-3-yl | Me | H | F | H | H | H | Br | H | heptafluoroisopropyl | H | CF3 |
| 7-1615 | 2-cyanopyridin-3-yl | Me | H | F | H | H | H | Br | H | heptafluoroisopropyl | H | CF3 |
| 7-1616 | 6-fluoropyridin-3-yl | Me | H | F | H | H | H | Br | H | heptafluoroisopropyl | H | CF3 |
| 7-1617 | 6-chloropyridin-3-yl | Me | H | F | H | H | H | Br | H | heptafluoroisopropyl | H | CF3 |
| 7-1618 | 6-bromopyridin-3-yl | Me | H | F | H | H | H | Br | H | heptafluoroisopropyl | H | CF3 |
| 7-1619 | 6-iodopyridin-3-yl | Me | H | F | H | H | H | Br | H | heptafluoroisopropyl | H | CF3 |
| 7-1620 | 6-(trifluoromethyl) pyridin-3-yl | Me | H | F | H | H | H | Br | H | heptafluoroisopropyl | H | CF3 |
| 7-1621 | 6-nitropyridin-3-yl | CH2Ph | H | F | H | H | H | Br | H | heptafluoroisopropyl | H | CF3 |
| 7-1622 | 6-cyanopyridin-3-yl | Me | H | F | H | H | H | Br | H | heptafluoroisopropyl | H | CF3 |
| 7-1623 | 5-fluoropyridin-3-yl | Me | H | F | H | H | H | Br | H | heptafluoroisopropyl | H | CF3 |
| 7-1624 | 5-chloropyridin-3-yl | Me | H | F | H | H | H | Br | H | heptafluoroisopropyl | H | CF3 |
| 7-1625 | 5-bromopyridin-3-yl | Me | H | F | H | H | H | Br | H | heptafluoroisopropyl | H | CF3 |
| 7-1626 | 5-iodopyridin-3-yl | Me | H | F | H | H | H | Br | H | heptafluoroisopropyl | H | CF3 |
| 7-1627 | 5-(trifluoromethyl) pyridin-3-yl | Me | H | F | H | H | H | Br | H | heptafluoroisopropyl | H | CF3 |
| 7-1628 | 5-nitropyridin-3-yl | Me | H | F | H | H | H | Br | H | heptafluoroisopropyl | H | CF3 |
| 7-1629 | 5-cyanopyridin-3-yl | Me | H | F | H | H | H | Br | H | heptafluoroisopropyl | H | CF3 |

**Table 7(5)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 7-1630 | 4-fluoropyridin-3-yl | CH2(3-Py) | H | F | H | H | H | Br | H | heptafluoroisopropyl | H | CF3 |
| 7-1631 | 4-chloropyridin-3-yl | Me | H | F | H | H | H | Br | H | heptafluoroisopropyl | H | CF3 |
| 7-1632 | 4-bromopyridin-3-yl | Me | H | F | H | H | H | Br | H | heptafluoroisopropyl | H | CF3 |
| 7-1633 | 4-iodopyridin-3-yl | Me | H | F | H | H | H | Br | H | heptafluoroisopropyl | H | CF3 |
| 7-1634 | 4-(trifluoromethyl)pyridin-3-yl | Me | H | F | H | H | H | Br | H | heptafluoroisopropyl | H | CF3 |
| 7-1635 | 4-nitropyridin-3-yl | Me | H | F | H | H | H | Br | H | heptafluoroisopropyl | H | CF3 |
| 7-1636 | 4-cyanopyridin-3-yl | Me | H | F | H | H | H | Br | H | heptafluoroisopropyl | H | CF3 |
| 7-1637 | 2,6-dichloropyridin-3-yl | Me | H | F | H | H | H | Br | H | heptafluoroisopropyl | H | CF3 |
| 7-1638 | pyridin-3-ylN-oxide | Me | H | F | H | H | H | Br | H | heptafluoroisopropyl | H | CF3 |
| 7-1639 | pyridin-4-yl | Me | H | F | H | H | H | Br | H | heptafluoroisopropyl | H | CF3 |
| 7-1640 | 2-chloropyridin-4-yl | Me | H | F | H | H | H | Br | H | heptafluoroisopropyl | H | CF3 |
| 7-1641 | 3-bromopyridin-4-yl | Me | H | F | H | H | H | Br | H | heptafluoroisopropyl | H | CF3 |
| 7-1642 | 3,5-dichloropyridin-4-yl | Me | H | F | H | H | H | Br | H | heptafluoroisopropyl | H | CF3 |
| 7-1643 | 3-(trifluoromethyl)pyridin-4-yl | Me | H | F | H | H | H | Br | H | heptafluoroisopropyl | H | CF3 |
| 7-1644 | 2,6-dicyanopyridin-4-yl | Me | H | F | H | H | H | Br | H | heptafluoroisopropyl | H | CF3 |
| 7-1645 | pyridin-4-ylN-oxide | Me | H | F | H | H | H | Br | H | heptafluoroisopropyl | H | CF3 |
| 7-1646 | pyridin-2-yl | Me | H | F | H | H | H | Br | H | heptafluoroisopropyl | H | CF3 |
| 7-1647 | 3-chloropyridin-2-yl | Me | H | F | H | H | H | Br | H | heptafluoroisopropyl | H | CF3 |
| 7-1648 | 4-bromopyridin-2-yl | Me | H | F | H | H | H | Br | H | heptafluoroisopropyl | H | CF3 |
| 7-1649 | 5-iodopyridin-2-yl | Me | H | F | H | H | H | Br | H | heptafluoroisopropyl | H | CF3 |
| 7-1650 | 6-chloropyridin-2-yl | Me | H | F | H | H | H | Br | H | heptafluoroisopropyl | H | CF3 |
| 7-1651 | 4-cyanopyridin-2-yl | CH2(3-Py-N-oxide) | H | F | H | H | H | Br | H | heptafluoroisopropyl | H | CF3 |
| 7-1652 | phenyl | Me | H | F | H | H | H | Br | H | nonafluoro-2-butyl | H | CF3 |
| 7-1653 | 2-fluorophenyl | Me | H | F | H | H | H | Br | H | nonafluoro-2-butyl | H | CF3 |
| 7-1654 | 3-fluorophenyl | Me | H | F | H | H | H | Br | H | nonafluoro-2-butyl | H | CF3 |
| 7-1655 | 4-fluorophenyl | Me | H | F | H | H | H | Br | H | nonafluoro-2-butyl | H | CF3 |
| 7-1656 | 2-chlorophenyl | Me | H | F | H | H | H | Br | H | nonafluoro-2-butyl | H | CF3 |
| 7-1657 | 3-chlorophenyl | Me | H | F | H | H | H | Br | H | nonafluoro-2-butyl | H | CF3 |
| 7-1658 | 4-chlorophenyl | Me | H | F | H | H | H | Br | H | nonafluoro-2-butyl | H | CF3 |
| 7-1659 | 2-bromophenyl | Me | H | F | H | H | H | Br | H | nonafluoro-2-butyl | H | CF3 |
| 7-1660 | 3-bromophenyl | Me | H | F | H | H | H | Br | H | nonafluoro-2-butyl | H | CF3 |
| 7-1661 | 4-bromophenyl | Me | H | F | H | H | H | Br | H | nonafluoro-2-butyl | H | CF3 |
| 7-1662 | 2-iodophenyl | Me | H | F | H | H | H | Br | H | nonafluoro-2-butyl | H | CF3 |
| 7-1663 | 3-iodophenyl | Me | H | F | H | H | H | Br | H | nonafluoro-2-butyl | H | CF3 |
| 7-1664 | 4-iodophenyl | Me | H | F | H | H | H | Br | H | nonafluoro-2-butyl | H | CF3 |
| 7-1665 | (2-trifluoromethyl)phenyl | Me | H | F | H | H | H | Br | H | nonafluoro-2-butyl | H | CF3 |
| 7-1666 | (3-trifluoromethyl)phenyl | Me | H | F | H | H | H | Br | H | nonafluoro-2-butyl | H | CF3 |
| 7-1667 | (4-trifluoromethyl)phenyl | Me | H | F | H | H | H | Br | H | nonafluoro-2-butyl | H | CF3 |
| 7-1668 | 2-nitrophenyl | Me | H | F | H | H | H | Br | H | nonafluoro-2-butyl | H | CF3 |
| 7-1669 | 3-nitrophenyl | Me | H | F | H | H | H | Br | H | nonafluoro-2-butyl | H | CF3 |
| 7-1670 | 4-nitrophenyl | Me | H | F | H | H | H | Br | H | nonafluoro-2-butyl | H | CF3 |
| 7-1671 | 2-cyanophenyl | Me | H | F | H | H | H | Br | H | nonafluoro-2-butyl | H | CF3 |
| 7-1672 | 3-cyanophenyl | Me | H | F | H | H | H | Br | H | nonafluoro-2-butyl | H | CF3 |
| 7-1673 | 4-cyanophenyl | Me | H | F | H | H | H | Br | H | nonafluoro-2-butyl | H | CF3 |
| 7-1674 | 2,6-difluorophenyl | Me | H | F | H | H | H | Br | H | nonafluoro-2-butyl | H | CF3 |
| 7-1675 | 3,4-dichlorophenyl | Me | H | F | H | H | H | Br | H | nonafluoro-2-butyl | H | CF3 |
| 7-1676 | 2,4-dichlorophenyl | Me | H | F | H | H | H | Br | H | nonafluoro-2-butyl | H | CF3 |
| 7-1677 | 2-chloro-4-fluorophenyl | Me | H | F | H | H | H | Br | H | nonafluoro-2-butyl | H | CF3 |
| 7-1678 | 2-chloro-4,5-difluorophenyl | Me | H | F | H | H | H | Br | H | nonafluoro-2-butyl | H | CF3 |
| 7-1679 | 4-bromo-2-chlorophenyl | Et | H | F | H | H | H | Br | H | nonafluoro-2-butyl | H | CF3 |
| 7-1680 | 2-bromo-4-chlorophenyl | Me | H | F | H | H | H | Br | H | nonafluoro-2-butyl | H | CF3 |
| 7-1681 | 2-bromo-4-fluorophenyl | Me | H | F | H | H | H | Br | H | nonafluoro-2-butyl | H | CF3 |
| 7-1682 | 2-chloro-4-nitrophenyl | Me | H | F | H | H | H | Br | H | nonafluoro-2-butyl | H | CF3 |
| 7-1683 | 3,5-dicyanophenyl | Me | H | F | H | H | H | Br | H | nonafluoro-2-butyl | H | CF3 |
| 7-1684 | 4-cyano-2-fluorophenyl | Me | H | F | H | H | H | Br | H | nonafluoro-2-butyl | H | CF3 |
| 7-1685 | 2-chloro-4-cyanophenyl | Me | H | F | H | H | H | Br | H | nonafluoro-2-butyl | H | CF3 |
| 7-1686 | pyridin-3-yl | Me | H | F | H | H | H | Br | H | nonafluoro-2-butyl | H | CF3 |
| 7-1687 | 2-fluoropyridin-3-yl | Me | H | F | H | H | H | Br | H | nonafluoro-2-butyl | H | CF3 |
| 7-1688 | 2-chloropyridin-3-yl | Me | H | F | H | H | H | Br | H | nonafluoro-2-butyl | H | CF3 |
| 7-1689 | 2-bromopyridin-3-yl | Me | H | F | H | H | H | Br | H | nonafluoro-2-butyl | H | CF3 |
| 7-1690 | 2-iodopyridin-3-yl | Me | H | F | H | H | H | Br | H | nonafluoro-2-butyl | H | CF3 |
| 7-1691 | 2-(trifluoromethyl)pyridin-3-yl | Me | H | F | H | H | H | Br | H | nonafluoro-2-butyl | H | CF3 |
| 7-1692 | 2-nitropyridin-3-yl | Me | H | F | H | H | H | Br | H | nonafluoro-2-butyl | H | CF3 |
| 7-1693 | 2-cyanopyridin-3-yl | Me | H | F | H | H | H | Br | H | nonafluoro-2-butyl | H | CF3 |

**Table 7(6)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 7-1694 | 6-fluoropyridin-3-yl | Me | H | F | H | H | H | Br | H | nonafluoro-2-butyl | H | CF3 |
| 7-1695 | 6-chloropyridin-3-yl | Me | H | F | H | H | H | Br | H | nonafluoro-2-butyl | H | CF3 |
| 7-1696 | 6-bromopyridin-3-yl | Me | H | F | H | H | H | Br | H | nonafluoro-2-butyl | H | CF3 |
| 7-1697 | 6-iodopyridin-3-yl | Me | H | F | H | H | H | Br | H | nonafluoro-2-butyl | H | CF3 |
| 7-1698 | 6-(trifluoromethyl)pyridin-3-yl | Me | H | F | H | H | H | Br | H | nonafluoro-2-butyl | H | CF3 |
| 7-1699 | 6-nitropyridin-3-yl | Me | H | F | H | H | H | Br | H | nonafluoro-2-butyl | H | CF3 |
| 7-1700 | 6-cyanopyridin-3-yl | Me | H | F | H | H | H | Br | H | nonafluoro-2-butyl | H | CF3 |
| 7-1701 | 5-fluoropyridin-3-yl | Me | H | F | H | H | H | Br | H | nonafluoro-2-butyl | H | CF3 |
| 7-1702 | 5-chloropyridin-3-yl | Me | H | F | H | H | H | Br | H | nonafluoro-2-butyl | H | CF3 |
| 7-1703 | 5-bromopyridin-3-yl | Me | H | F | H | H | H | Br | H | nonafluoro-2-butyl | H | CF3 |
| 7-1704 | 5-iodopyridin-3-yl | Me | H | F | H | H | H | Br | H | nonafluoro-2-butyl | H | CF3 |
| 7-1705 | 5-(trifluoromethyl)pyridin-3-yl | CN | H | F | H | H | H | Br | H | nonafluoro-2-butyl | H | CF3 |
| 7-1706 | 5-nitropyridin-3-yl | Me | H | F | H | H | H | Br | H | nonafluoro-2-butyl | H | CF3 |
| 7-1707 | 5-cyanopyridin-3-yl | Me | H | F | H | H | H | Br | H | nonafluoro-2-butyl | H | CF3 |
| 7-1708 | 4-fluoropyridin-3-yl | Me | H | F | H | H | H | Br | H | nonafluoro-2-butyl | H | CF3 |
| 7-1709 | 4-chloropyridin-3-yl | Me | H | F | H | H | H | Br | H | nonafluoro-2-butyl | H | CF3 |
| 7-1710 | 4-bromopyridin-3-yl | Me | H | F | H | H | H | Br | H | nonafluoro-2-butyl | H | CF3 |
| 7-1711 | 4-iodopyridin-3-yl | Me | H | F | H | H | H | Br | H | nonafluoro-2-butyl | H | CF3 |
| 7-1712 | 4-(trifluoromethyl)pyridin-3-yl | Me | H | F | H | H | H | Br | H | nonafluoro-2-butyl | H | CF3 |
| 7-1713 | 4-nitropyridin-3-yl | Me | H | F | H | H | H | Br | H | nonafluoro-2-butyl | H | CF3 |
| 7-1714 | 4-cyanopyridin-3-yl | Me | H | F | H | H | H | Br | H | nonafluoro-2-butyl | H | CF3 |
| 7-1715 | 2,6-dichloropyridin-3-yl | Me | H | F | H | H | H | Br | H | nonafluoro-2-butyl | H | CF3 |
| 7-1716 | pyridin-3-ylN-oxide | Me | H | F | H | H | H | Br | H | nonafluoro-2-butyl | H | CF3 |
| 7-1717 | pyridin-4-yl | Me | H | F | H | H | H | Br | H | nonafluoro-2-butyl | H | CF3 |
| 7-1718 | 2-chloropyridin-4-yl | Me | H | F | H | H | H | Br | H | nonafluoro-2-butyl | H | CF3 |
| 7-1719 | 3-bromopyridin-4-yl | Me | H | F | H | H | H | Br | H | nonafluoro-2-butyl | H | CF3 |
| 7-1720 | 3,5-dichloropyridin-4-yl | Me | H | F | H | H | H | Br | H | nonafluoro-2-butyl | H | CF3 |
| 7-1721 | 3-(trifluoromethyl)pyridin-4-yl | Me | H | F | H | H | H | Br | H | nonafluoro-2-butyl | H | CF3 |
| 7-1722 | 2,6-dicyanopyridin-4-yl | Me | H | F | H | H | H | Br | H | nonafluoro-2-butyl | H | CF3 |
| 7-1723 | pyridin-4-ylN-oxide | Me | H | F | H | H | H | Br | H | nonafluoro-2-butyl | H | CF3 |
| 7-1724 | pyridin-2-yl | Me | H | F | H | H | H | Br | H | nonafluoro-2-butyl | H | CF3 |
| 7-1725 | 3-chloropyridin-2-yl | Me | H | F | H | H | H | Br | H | nonafluoro-2-butyl | H | CF3 |
| 7-1726 | 4-bromopyridin-2-yl | Me | H | F | H | H | H | Br | H | nonafluoro-2-butyl | H | CF3 |
| 7-1727 | 5-iodopyridin-2-yl | Me | H | F | H | H | H | Br | H | nonafluoro-2-butyl | H | CF3 |
| 7-1728 | 6-chloropyridin-2-yl | Me | H | F | H | H | H | Br | H | nonafluoro-2-butyl | H | CF3 |
| 7-1729 | 4-cyanopyridin-2-yl | Me | H | F | H | H | H | Br | H | nonafluoro-2-butyl | H | CF3 |
| 7-1730 | phenyl | Me | H | F | H | H | H | I | H | heptafluoroisopropyl | H | CF3 |
| 7-1731 | 2-fluorophenyl | Me | H | F | H | H | H | I | H | heptafluoroisopropyl | H | CF3 |
| 7-1732 | 3-fluorophenyl | Me | H | F | H | H | H | I | H | heptafluoroisopropyl | H | CF3 |
| 7-1733 | 4-fluorophenyl | Me | H | F | H | H | H | I | H | heptafluoroisopropyl | H | CF3 |
| 7-1734 | 2-chlorophenyl | Me | H | F | H | H | H | I | H | heptafluoroisopropyl | H | CF3 |
| 7-1735 | 3-chlorophenyl | Me | H | F | H | H | H | I | H | heptafluoroisopropyl | H | CF3 |
| 7-1736 | 4-chlorophenyl | Me | H | F | H | H | H | I | H | heptafluoroisopropyl | H | CF3 |
| 7-1737 | 2-bromophenyl | Me | H | F | H | H | H | I | H | heptafluoroisopropyl | H | CF3 |
| 7-1738 | 3-bromophenyl | Me | H | F | H | H | H | I | H | heptafluoroisopropyl | H | CF3 |
| 7-1739 | 4-bromophenyl | Me | H | F | H | H | H | I | H | heptafluoroisopropyl | H | CF3 |
| 7-1740 | 2-iodophenyl | Me | H | F | H | H | H | I | H | heptafluoroisopropyl | H | CF3 |
| 7-1741 | 3-iodophenyl | Me | H | F | H | H | H | I | H | heptafluoroisopropyl | H | CF3 |
| 7-1742 | 4-iodophenyl | Me | H | F | H | H | H | I | H | heptafluoroisopropyl | H | CF3 |
| 7-1743 | (2-trifluoromethyl)phenyl | Me | H | F | H | H | H | I | H | heptafluoroisopropyl | H | CF3 |
| 7-1744 | (3-trifluoromethyl)phenyl | Me | H | F | H | H | H | I | H | heptafluoroisopropyl | H | CF3 |
| 7-1745 | (4-trifluoromethyl)phenyl | Me | H | F | H | H | H | I | H | heptafluoroisopropyl | H | CF3 |
| 7-1746 | 2-nitrophenyl | Me | H | F | H | H | H | I | H | heptafluoroisopropyl | H | CF3 |
| 7-1747 | 3-nitrophenyl | Me | H | F | H | H | H | I | H | heptafluoroisopropyl | H | CF3 |
| 7-1748 | 4-nitrophenyl | Me | H | F | H | H | H | I | H | heptafluoroisopropyl | H | CF3 |
| 7-1749 | 2-cyanophenyl | Me | H | F | H | H | H | I | H | heptafluoroisopropyl | H | CF3 |
| 7-1750 | 3-cyanophenyl | Me | H | F | H | H | H | I | H | heptafluoroisopropyl | H | CF3 |
| 7-1751 | 4-cyanophenyl | Me | H | F | H | H | H | I | H | heptafluoroisopropyl | H | CF3 |
| 7-1752 | 2,6-difluorophenyl | Me | H | F | H | H | H | I | H | heptafluoroisopropyl | H | CF3 |
| 7-1753 | 3,4-dichlorophenyl | Me | H | F | H | H | H | I | H | heptafluoroisopropyl | H | CF3 |
| 7-1754 | 2,4-dichlorophenyl | Me | H | F | H | H | H | I | H | heptafluoroisopropyl | H | CF3 |
| 7-1755 | 2-chloro-4-fluorophenyl | Me | H | F | H | H | H | I | H | heptafluoroisopropyl | H | CF3 |
| 7-1756 | 2-chloro-4,5-difluorophenyl | Me | H | F | H | H | H | I | H | heptafluoroisopropyl | H | CF3 |
| 7-1757 | 4-bromo-2-chlorophenyl | Me | H | F | H | H | H | I | H | heptafluoroisopropyl | H | CF3 |

**Table 7(7)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 7-1758 | 2-bromo-4-chlorophenyl | Me | H | F | H | H | H | I | H | heptafluoroisopropyl | H | CF3 |
| 7-1759 | 2-bromo-4-fluorophenyl | Me | H | F | H | H | H | I | H | heptafluoroisopropyl | H | CF3 |
| 7-1760 | 2-chloro-4-nitrophenyl | Me | H | F | H | H | H | I | H | heptafluoroisopropyl | H | CF3 |
| 7-1761 | 3,5-dicyanophenyl | Me | H | F | H | H | H | I | H | heptafluoroisopropyl | H | CF3 |
| 7-1762 | 4-cyano-2-fluorophenyl | Me | H | F | H | H | H | I | H | heptafluoroisopropyl | H | CF3 |
| 7-1763 | 2-chloro-4-cyanophenyl | Me | H | F | H | H | H | I | H | heptafluoroisopropyl | H | CF3 |
| 7-1764 | pyridin-3-yl | Me | H | F | H | H | H | I | H | heptafluoroisopropyl | H | CF3 |
| 7-1765 | 2-fluoropyridin-3-yl | Me | H | F | H | H | H | I | H | heptafluoroisopropyl | H | CF3 |
| 7-1766 | 2-chloropyridin-3-yl | Me | H | F | H | H | H | I | H | heptafluoroisopropyl | H | CF3 |
| 7-1767 | 2-bromopyridin-3-yl | Me | H | F | H | H | H | I | H | heptafluoroisopropyl | H | CF3 |
| 7-1768 | 2-iodopyridin-3-yl | Me | H | F | H | H | H | I | H | heptafluoroisopropyl | H | CF3 |
| 7-1769 | 2-(trifluoromethyl)pyridin-3-yl | Me | H | F | H | H | H | I | H | heptafluoroisopropyl | H | CF3 |
| 7-1770 | 2-nitropyridin-3-yl | Me | H | F | H | H | H | I | H | heptafluoroisopropyl | H | CF3 |
| 7-1771 | 2-cyanopyridin-3-yl | Me | H | F | H | H | H | I | H | heptafluoroisopropyl | H | CF3 |
| 7-1772 | 6-fluoropyridin-3-yl | Me | H | F | H | H | H | I | H | heptafluoroisopropyl | H | CF3 |
| 7-1773 | 6-chloropyridin-3-yl | Me | H | F | H | H | H | I | H | heptafluoroisopropyl | H | CF3 |
| 7-1774 | 6-bromopyridin-3-yl | Me | H | F | H | H | H | I | H | heptafluoroisopropyl | H | CF3 |
| 7-1775 | 6-iodopyridin-3-yl | Me | H | F | H | H | H | I | H | heptafluoroisopropyl | H | CF3 |
| 7-1776 | 6-(trifluoromethyl)pyridin-3-yl | Me | H | F | H | H | H | I | H | heptafluoroisopropyl | H | CF3 |
| 7-1777 | 6-nitropyridin-3-yl | Me | H | F | H | H | H | I | H | heptafluoroisopropyl | H | CF3 |
| 7-1778 | 6-cyanopyridin-3-yl | Me | H | F | H | H | H | I | H | heptafluoroisopropyl | H | CF3 |
| 7-1779 | 5-fluoropyridin-3-yl | C(O)Me | H | F | H | H | H | I | H | heptafluoroisopropyl | H | CF3 |
| 7-1780 | 5-chloropyridin-3-yl | Me | H | F | H | H | H | I | H | heptafluoroisopropyl | H | CF3 |
| 7-1781 | 5-bromopyridin-3-yl | Me | H | F | H | H | H | I | H | heptafluoroisopropyl | H | CF3 |
| 7-1782 | 5-iodopyridin-3-yl | Me | H | F | H | H | H | I | H | heptafluoroisopropyl | H | CF3 |
| 7-1783 | 5-(trifluoromethyl)pyridin-3-yl | Me | H | F | H | H | H | I | H | heptafluoroisopropyl | H | CF3 |
| 7-1784 | 5-nitropyridin-3-yl | Me | H | F | H | H | H | I | H | heptafluoroisopropyl | H | CF3 |
| 7-1785 | 5-cyanopyridin-3-yl | Me | H | F | H | H | H | I | H | heptafluoroisopropyl | H | CF3 |
| 7-1786 | 4-fluoropyridin-3-yl | Me | H | F | H | H | H | I | H | heptafluoroisopropyl | H | CF3 |
| 7-1787 | 4-chloropyridin-3-yl | Me | H | F | H | H | H | I | H | heptafluoroisopropyl | H | CF3 |
| 7-1788 | 4-bromopyridin-3-yl | Me | H | F | H | H | H | I | H | heptafluoroisopropyl | H | CF3 |
| 7-1789 | 4-iodopyridin-3-yl | Me | H | F | H | H | H | I | H | heptafluoroisopropyl | H | CF3 |
| 7-1790 | 4-(trifluoromethyl)pyridin-3-yl | Me | H | F | H | H | H | I | H | heptafluoroisopropyl | H | CF3 |
| 7-1791 | 4-nitropyridin-3-yl | Me | H | F | H | H | H | I | H | heptafluoroisopropyl | H | CF3 |
| 7-1792 | 4-cyanopyridin-3-yl | Me | H | F | H | H | H | I | H | heptafluoroisopropyl | H | CF3 |
| 7-1793 | 2,6-dichloropyridin-3-yl | Me | H | F | H | H | H | I | H | heptafluoroisopropyl | H | CF3 |
| 7-1794 | pyridin-3-yl N-oxide | Me | H | F | H | H | H | I | H | heptafluoroisopropyl | H | CF3 |
| 7-1795 | pyridin-4-yl | Me | H | F | H | H | H | I | H | heptafluoroisopropyl | H | CF3 |
| 7-1796 | 2-chloropyridin-4-yl | Me | H | F | H | H | H | I | H | heptafluoroisopropyl | H | CF3 |
| 7-1797 | 3-bromopyridin-4-yl | Me | H | F | H | H | H | I | H | heptafluoroisopropyl | H | CF3 |
| 7-1798 | 3,5-dichloropyridin-4-yl | Me | H | F | H | H | H | I | H | heptafluoroisopropyl | H | CF3 |
| 7-1799 | 3-(trifluoromethyl)pyridin-4-yl | Me | H | F | H | H | H | I | H | heptafluoroisopropyl | H | CF3 |
| 7-1800 | 2,6-dicyanopyridin-4-yl | Me | H | F | H | H | H | I | H | heptafluoroisopropyl | H | CF3 |
| 7-1801 | pyridin-4-yl N-oxide | Me | H | F | H | H | H | I | H | heptafluoroisopropyl | H | CF3 |
| 7-1802 | pyridin-2-yl | Me | H | F | H | H | H | I | H | heptafluoroisopropyl | H | CF3 |
| 7-1803 | 3-chloropyridin-2-yl | Me | H | F | H | H | H | I | H | heptafluoroisopropyl | H | CF3 |
| 7-1804 | 4-bromopyridin-2-yl | Me | H | F | H | H | H | I | H | heptafluoroisopropyl | H | CF3 |
| 7-1805 | 5-iodopyridin-2-yl | Me | H | F | H | H | H | I | H | heptafluoroisopropyl | H | CF3 |
| 7-1806 | 6-chloropyridin-2-yl | Me | H | F | H | H | H | I | H | heptafluoroisopropyl | H | CF3 |
| 7-1807 | 4-cyanopyridin-2-yl | Me | H | F | H | H | H | I | H | heptafluoroisopropyl | H | CF3 |
| 7-1808 | phenyl | Me | H | F | H | H | H | I | H | nonafluoro-2-butyl | H | CF3 |
| 7-1809 | 2-fluorophenyl | Me | H | F | H | H | H | I | H | nonafluoro-2-butyl | H | CF3 |
| 7-1810 | 3-fluorophenyl | Me | H | F | H | H | H | I | H | nonafluoro-2-butyl | H | CF3 |
| 7-1811 | 4-fluorophenyl | Me | H | F | H | H | H | I | H | nonafluoro-2-butyl | H | CF3 |
| 7-1812 | 2-chlorophenyl | Me | H | F | H | H | H | I | H | nonafluoro-2-butyl | H | CF3 |
| 7-1813 | 3-chlorophenyl | Me | H | F | H | H | H | I | H | nonafluoro-2-butyl | H | CF3 |
| 7-1814 | 4-chlorophenyl | Me | H | F | H | H | H | I | H | nonafluoro-2-butyl | H | CF3 |
| 7-1815 | 2-bromophenyl | Me | H | F | H | H | H | I | H | nonafluoro-2-butyl | H | CF3 |
| 7-1816 | 3-bromophenyl | Me | H | F | H | H | H | I | H | nonafluoro-2-butyl | H | CF3 |
| 7-1817 | 4-bromophenyl | Me | H | F | H | H | H | I | H | nonafluoro-2-butyl | H | CF3 |
| 7-1818 | 2-iodophenyl | Me | H | F | H | H | H | I | H | nonafluoro-2-butyl | H | CF3 |
| 7-1819 | 3-iodophenyl | Me | H | F | H | H | H | I | H | nonafluoro-2-butyl | H | CF3 |
| 7-1820 | 4-iodophenyl | Me | H | F | H | H | H | I | H | nonafluoro-2-butyl | H | CF3 |
| 7-1821 | (2-trifluoromethyl) phenyl | Me | H | F | H | H | H | I | H | nonafluoro-2-butyl | H | CF3 |

**Table 7(8)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 7-1822 | (3-trifluoromethyl) phenyl | Me | H | F | H | H | H | I | H | nonafluoro-2-butyl | H | CF3 |
| 7-1823 | (4-trifluoromethyl) phenyl | Me | H | F | H | H | H | I | H | nonafluoro-2-butyl | H | CF3 |
| 7-1824 | 2-nitrophenyl | Me | H | F | H | H | H | I | H | nonafluoro-2-butyl | H | CF3 |
| 7-1825 | 3-nitrophenyl | Me | H | F | H | H | H | I | H | nonafluoro-2-butyl | H | CF3 |
| 7-1826 | 4-nitrophenyl | Me | H | F | H | H | H | I | H | nonafluoro-2-butyl | H | CF3 |
| 7-1827 | 2-cyanophenyl | Me | H | F | H | H | H | I | H | nonafluoro-2-butyl | H | CF3 |
| 7-1828 | 3-cyanophenyl | Me | H | F | H | H | H | I | H | nonafluoro-2-butyl | H | CF3 |
| 7-1829 | 4-cyanophenyl | Me | H | F | H | H | H | I | H | nonafluoro-2-butyl | H | CF3 |
| 7-1830 | 2,6-difluorophenyl | Me | H | F | H | H | H | I | H | nonafluoro-2-butyl | H | CF3 |
| 7-1831 | 3,4-dichlorophenyl | Me | H | F | H | H | H | I | H | nonafluoro-2-butyl | H | CF3 |
| 7-1832 | 2,4-dichlorophenyl | Me | H | F | H | H | H | I | H | nonafluoro-2-butyl | H | CF3 |
| 7-1833 | 2-chloro-4-fluorophenyl | Me | H | F | H | H | H | I | H | nonafluoro-2-butyl | H | CF3 |
| 7-1834 | 2-chloro-4,5-difluorophenyl | Me | H | F | H | H | H | I | H | nonafluoro-2-butyl | H | CF3 |
| 7-1835 | 4-bromo-2-chlorophenyl | Me | H | F | H | H | H | I | H | nonafluoro-2-butyl | H | CF3 |
| 7-1836 | 2-bromo-4-chlorophenyl | Me | H | F | H | H | H | I | H | nonafluoro-2-butyl | H | CF3 |
| 7-1837 | 2-bromo-4-fluorophenyl | Me | H | F | H | H | H | I | H | nonafluoro-2-butyl | H | CF3 |
| 7-1838 | 2-chloro-4-nitrophenyl | Me | H | F | H | H | H | I | H | nonafluoro-2-butyl | H | CF3 |
| 7-1839 | 3,5-dicyanophenyl | Me | H | F | H | H | H | I | H | nonafluoro-2-butyl | H | CF3 |
| 7-1840 | 4-cyano-2-fluorophenyl | Me | H | F | H | H | H | I | H | nonafluoro-2-butyl | H | CF3 |
| 7-1841 | 2-chloro-4-cyanophenyl | Me | H | F | H | H | H | I | H | nonafluoro-2-butyl | H | CF3 |
| 7-1842 | pyridin-3-yl | Me | H | F | H | H | H | I | H | nonafluoro-2-butyl | H | CF3 |
| 7-1843 | 2-fluoropyridin-3-yl | Me | H | F | H | H | H | I | H | nonafluoro-2-butyl | H | CF3 |
| 7-1844 | 2-chloropyridin-3-yl | Me | H | F | H | H | H | I | H | nonafluoro-2-butyl | H | CF3 |
| 7-1845 | 2-bromopyridin-3-yl | Me | H | F | H | H | H | I | H | nonafluoro-2-butyl | H | CF3 |
| 7-1846 | 2-iodopyridin-3-yl | Me | H | F | H | H | H | I | H | nonafluoro-2-butyl | H | CF3 |
| 7-1847 | 2-(trifluoromethyl) pyridin-3-yl | Me | H | F | H | H | H | I | H | nonafluoro-2-butyl | H | CF3 |
| 7-1848 | 2-nitropyridin-3-yl | Me | H | F | H | H | H | I | H | nonafluoro-2-butyl | H | CF3 |
| 7-1849 | 2-cyanopyridin-3-yl | Me | H | F | H | H | H | I | H | nonafluoro-2-butyl | H | CF3 |
| 7-1850 | 6-fluoropyridin-3-yl | Me | H | F | H | H | H | I | H | nonafluoro-2-butyl | H | CF3 |
| 7-1851 | 6-chloropyridin-3-yl | Me | H | F | H | H | H | I | H | nonafluoro-2-butyl | H | CF3 |
| 7-1852 | 6-bromopyridin-3-yl | Me | H | F | H | H | H | I | H | nonafluoro-2-butyl | H | CF3 |
| 7-1853 | 6-iodopyridin-3-yl | Me | H | F | H | H | H | I | H | nonafluoro-2-butyl | H | CF3 |
| 7-1854 | 6-(trifluoromethyl) pyridin-3-yl | Me | H | F | H | H | H | I | H | nonafluoro-2-butyl | H | CF3 |
| 7-1855 | 6-nitropyridin-3-yl | Me | H | F | H | H | H | I | H | nonafluoro-2-butyl | H | CF3 |
| 7-1856 | 6-cyanopyridin-3-yl | Me | H | F | H | H | H | I | H | nonafluoro-2-butyl | H | CF3 |
| 7-1857 | 5-fluoropyridin-3-yl | Me | H | F | H | H | H | I | H | nonafluoro-2-butyl | H | CF3 |
| 7-1858 | 5-chloropyridin-3-yl | Me | H | F | H | H | H | I | H | nonafluoro-2-butyl | H | CF3 |
| 7-1859 | 5-bromopyridin-3-yl | Me | H | F | H | H | H | I | H | nonafluoro-2-butyl | H | CF3 |
| 7-1860 | 5-iodopyridin-3-yl | Me | H | F | H | H | H | I | H | nonafluoro-2-butyl | H | CF3 |
| 7-1861 | 5-(trifluoromethyl) pyridin-3-yl | Me | H | F | H | H | H | I | H | nonafluoro-2-butyl | H | CF3 |
| 7-1862 | 5-nitropyridin-3-yl | Me | H | F | H | H | H | I | H | nonafluoro-2-butyl | H | CF3 |
| 7-1863 | 5-cyanopyridin-3-yl | Me | H | F | H | H | H | I | H | nonafluoro-2-butyl | H | CF3 |
| 7-1864 | 4-fluoropyridin-3-yl | Me | H | F | H | H | H | I | H | nonafluoro-2-butyl | H | CF3 |
| 7-1865 | 4-chloropyridin-3-yl | Me | H | F | H | H | H | I | H | nonafluoro-2-butyl | H | CF3 |
| 7-1866 | 4-bromopyridin-3-yl | Me | H | F | H | H | H | I | H | nonafluoro-2-butyl | H | CF3 |
| 7-1867 | 4-iodopyridin-3-yl | Me | H | F | H | H | H | I | H | nonafluoro-2-butyl | H | CF3 |
| 7-1868 | 4-(trifluoromethyl) pyridin-3-yl | Me | H | F | H | H | H | I | H | nonafluoro-2-butyl | H | CF3 |
| 7-1869 | 4-nitropyridin-3-yl | Me | H | F | H | H | H | I | H | nonafluoro-2-butyl | H | CF3 |
| 7-1870 | 4-cyanopyridin-3-yl | Me | H | F | H | H | H | I | H | nonafluoro-2-butyl | H | CF3 |
| 7-1871 | 2,6-dichloropyridin-3-yl | Me | H | F | H | H | H | I | H | nonafluoro-2-butyl | H | CF3 |
| 7-1872 | pyridin-3-ylN-oxide | Me | H | F | H | H | H | I | H | nonafluoro-2-butyl | H | CF3 |
| 7-1873 | pyridin-4-yl | Me | H | F | H | H | H | I | H | nonafluoro-2-butyl | H | CF3 |
| 7-1874 | 2-chloropyridin-4-yl | Me | H | F | H | H | H | I | H | nonafluoro-2-butyl | H | CF3 |
| 7-1875 | 3-bromopyridin-4-yl | Me | H | F | H | H | H | I | H | nonafluoro-2-butyl | H | CF3 |
| 7-1876 | 3,5-dichloropyridin-4-yl | Me | H | F | H | H | H | I | H | nonafluoro-2-butyl | H | CF3 |
| 7-1877 | 3-(trifluoromethyl)pyridin-4-yl | Me | H | F | H | H | H | I | H | nonafluoro-2-butyl | H | CF3 |
| 7-1878 | 2,6-dicyanopyridin-4-yl | Me | H | F | H | H | H | I | H | nonafluoro-2-butyl | H | CF3 |
| 7-1879 | pyridin-4-ylN-oxide | Me | H | F | H | H | H | I | H | nonafluoro-2-butyl | H | CF3 |
| 7-1880 | pyridin-2-yl | Me | H | F | H | H | H | I | H | nonafluoro-2-butyl | H | CF3 |
| 7-1881 | 3-chloropyridin-2-yl | Me | H | F | H | H | H | I | H | nonafluoro-2-butyl | H | CF3 |
| 7-1882 | 4-bromopyridin-2-yl | Me | H | F | H | H | H | I | H | nonafluoro-2-butyl | H | CF3 |
| 7-1883 | 5-iodopyridin-2-yl | Me | H | F | H | H | H | I | H | nonafluoro-2-butyl | H | CF3 |
| 7-1884 | 6-chloropyridin-2-yl | Me | H | F | H | H | H | I | H | nonafluoro-2-butyl | H | CF3 |
| 7-1885 | 4-cyanopyridin-2-yl | Me | H | F | H | H | H | I | H | nonafluoro-2-butyl | H | CF3 |

**Table 7(9)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 7-1886 | phenyl | Me | H | F | H | H | H | OCF3 | H | heptafluoroisopropyl | H | OCF3 |
| 7-1901 | 3-cyanophenyl | Me | H | F | H | H | H | OCF3 | H | heptafluoroisopropyl | H | OCF3 |
| 7-1902 | 4-cyanophenyl | Me | H | F | H | H | H | OCF3 | H | heptafluoroisopropyl | H | OCF3 |
| 7-1917 | 2-chloropyridin-3-yl | Me | H | F | H | H | H | OCF3 | H | heptafluoroisopropyl | H | OCF3 |
| 7-1942 | phenyl | Me | H | F | H | H | H | OCF3 | H | nonafluoro-2-butyl | H | OCF3 |
| 7-1957 | 3-cyanophenyl | Me | H | F | H | H | H | OCF3 | H | nonafluoro-2-butyl | H | OCF3 |
| 7-1958 | 4-cyanophenyl | Me | H | F | H | H | H | OCF3 | H | nonafluoro-2-butyl | H | OCF3 |
| 7-1973 | 2-chloropyridin-3-yl | Me | H | F | H | H | H | OCF3 | H | nonafluoro-2-butyl | H | OCF3 |
| 7-1998 | phenyl | Me | H | F | H | H | H | CF3 | H | heptafluoroisopropyl | H | OCF3 |
| 7-2013 | 3-cyanophenyl | Me | H | F | H | H | H | CF3 | H | heptafluoroisopropyl | H | OCF3 |
| 7-2014 | 4-cyanophenyl | Me | H | F | H | H | H | CF3 | H | heptafluoroisopropyl | H | OCF3 |
| 7-2029 | 2-chloropyridin-3-yl | Me | H | F | H | H | H | CF3 | H | heptafluoroisopropyl | H | OCF3 |
| 7-2054 | phenyl | Me | H | F | H | H | H | CF3 | H | nonafluoro-2-butyl | H | OCF3 |
| 7-2069 | 3-cyanophenyl | Me | H | F | H | H | H | CF3 | H | nonafluoro-2-butyl | H | OCF3 |
| 7-2070 | 4-cyanophenyl | Me | H | F | H | H | H | CF3 | H | nonafluoro-2-butyl | H | OCF3 |
| 7-2085 | 2-chloropyridin-3-yl | Me | H | F | H | H | H | CF3 | H | nonafluoro-2-butyl | H | OCF3 |
| 7-2110 | phenyl | Me | H | F | H | H | H | CF3 | H | heptafluoroisopropyl | H | CF3 |
| 7-2130 | 3-cyanophenyl | Me | H | F | H | H | H | CF3 | H | heptafluoroisopropyl | H | CF3 |
| 7-2131 | 4-cyanophenyl | Me | H | F | H | H | H | CF3 | H | heptafluoroisopropyl | H | CF3 |
| 7-2146 | 2-chloropyridin-3-yl | Me | H | F | H | H | H | CF3 | H | heptafluoroisopropyl | H | CF3 |
| 7-2178 | phenyl | Me | H | F | H | H | H | CF3 | H | nonafluoro-2-butyl | H | CF3 |
| 7-2198 | 3-cyanophenyl | Me | H | F | H | H | H | CF3 | H | nonafluoro-2-butyl | H | CF3 |
| 7-2199 | 4-cyanophenyl | Me | H | F | H | H | H | CF3 | H | nonafluoro-2-butyl | H | CF3 |
| 7-2214 | 2-chloropyridin-3-yl | Me | H | F | H | H | H | CF3 | H | nonafluoro-2-butyl | H | CF3 |
| 7-2246 | phenyl | Me | H | H | F | H | H | F | H | heptafluoroisopropyl | H | F |
| 7-2247 | 3-cyanophenyl | Me | H | H | F | H | H | F | H | heptafluoroisopropyl | H | F |
| 7-2248 | 4-cyanophenyl | Me | H | H | F | H | H | F | H | heptafluoroisopropyl | H | F |
| 7-2249 | 2-chloropyridin-3-yl | Me | H | H | F | H | H | F | H | heptafluoroisopropyl | H | F |
| 7-2250 | phenyl | Me | H | H | F | H | H | F | H | nonafluoro-2-butyl | H | F |
| 7-2251 | 3-cyanophenyl | Me | H | H | F | H | H | F | H | nonafluoro-2-butyl | H | F |
| 7-2252 | 4-cyanophenyl | Me | H | H | F | H | H | F | H | nonafluoro-2-butyl | H | F |
| 7-2253 | 2-chloropyridin-3-yl | Me | H | H | F | H | H | F | H | nonafluoro-2-butyl | H | F |
| 7-2254 | phenyl | Me | H | H | F | H | H | Cl | H | heptafluoroisopropyl | H | Cl |
| 7-2274 | 3-cyanophenyl | Me | H | H | F | H | H | Cl | H | heptafluoroisopropyl | H | Cl |
| 7-2275 | 4-cyanophenyl | Me | H | H | F | H | H | Cl | H | heptafluoroisopropyl | H | Cl |
| 7-2290 | 2-chloropyridin-3-yl | Me | H | H | F | H | H | Cl | H | heptafluoroisopropyl | H | Cl |
| 7-2332 | phenyl | Me | H | H | F | H | H | Cl | H | nonafluoro-2-butyl | H | Cl |
| 7-2352 | 3-cyanophenyl | Me | H | H | F | H | H | Cl | H | nonafluoro-2-butyl | H | Cl |
| 7-2353 | 4-cyanophenyl | Me | H | H | F | H | H | Cl | H | nonafluoro-2-butyl | H | Cl |
| 7-2368 | 2-chloropyridin-3-yl | Me | H | H | F | H | H | Cl | H | nonafluoro-2-butyl | H | Cl |
| 7-2410 | phenyl | Me | H | H | F | H | H | Br | H | heptafluoroisopropyl | H | Br |
| 7-2430 | 3-cyanophenyl | Me | H | H | F | H | H | Br | H | heptafluoroisopropyl | H | Br |
| 7-2431 | 4-cyanophenyl | Me | H | H | F | H | H | Br | H | heptafluoroisopropyl | H | Br |
| 7-2446 | 2-chloropyridin-3-yl | Me | H | H | F | H | H | Br | H | heptafluoroisopropyl | H | Br |
| 7-2488 | phenyl | Me | H | H | F | H | H | Br | H | nonafluoro-2-butyl | H | Br |
| 7-2508 | 3-cyanophenyl | Me | H | H | F | H | H | Br | H | nonafluoro-2-butyl | H | Br |
| 7-2509 | 4-cyanophenyl | Me | H | H | F | H | H | Br | H | nonafluoro-2-butyl | H | Br |
| 7-2524 | 2-chloropyridin-3-yl | Me | H | H | F | H | H | Br | H | nonafluoro-2-butyl | H | Br |
| 7-2566 | phenyl | Me | H | H | F | H | H | I | H | heptafluoroisopropyl | H | I |
| 7-2586 | 3-cyanophenyl | Me | H | H | F | H | H | I | H | heptafluoroisopropyl | H | I |
| 7-2587 | 4-cyanophenyl | Me | H | H | F | H | H | I | H | heptafluoroisopropyl | H | I |
| 7-2602 | 2-chloropyridin-3-yl | Me | H | H | F | H | H | I | H | heptafluoroisopropyl | H | I |
| 7-2644 | phenyl | Me | H | H | F | H | H | I | H | nonafluoro-2-butyl | H | I |
| 7-2664 | 3-cyanophenyl | Me | H | H | F | H | H | I | H | nonafluoro-2-butyl | H | I |
| 7-2665 | 4-cyanophenyl | Me | H | H | F | H | H | I | H | nonafluoro-2-butyl | H | I |
| 7-2680 | 2-chloropyridin-3-yl | Me | H | H | F | H | H | I | H | nonafluoro-2-butyl | H | I |
| 7-2722 | phenyl | Me | H | H | F | H | H | Cl | H | heptafluoroisopropyl | H | OCF3 |
| 7-2742 | 3-cyanophenyl | Me | H | H | F | H | H | Cl | H | heptafluoroisopropyl | H | OCF3 |
| 7-2743 | 4-cyanophenyl | Me | H | H | F | H | H | Cl | H | heptafluoroisopropyl | H | OCF3 |
| 7-2758 | 2-chloropyridin-3-yl | Me | H | H | F | H | H | Cl | H | heptafluoroisopropyl | H | OCF3 |
| 7-2800 | phenyl | Me | H | H | F | H | H | Cl | H | nonafluoro-2-butyl | H | OCF3 |
| 7-2820 | 3-cyanophenyl | Me | H | H | F | H | H | Cl | H | nonafluoro-2-butyl | H | OCF3 |
| 7-2821 | 4-cyanophenyl | Me | H | H | F | H | H | Cl | H | nonafluoro-2-butyl | H | OCF3 |
| 7-2836 | 2-chloropyridin-3-yl | Me | H | H | F | H | H | Cl | H | nonafluoro-2-butyl | H | OCF3 |

**Table 7(10)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 7-2878 | phenyl | Me | H | H | F | H | H | Br | H | heptafluoroisopropyl | H | OCF3 |
| 7-2898 | 3-cyanophenyl | Me | H | H | F | H | H | Br | H | heptafluoroisopropyl | H | OCF3 |
| 7-2899 | 4-cyanophenyl | Me | H | H | F | H | H | Br | H | heptafluoroisopropyl | H | OCF3 |
| 7-2914 | 2-chloropyridin-3-yl | Me | H | H | F | H | H | Br | H | heptafluoroisopropyl | H | OCF3 |
| 7-2956 | phenyl | Me | H | H | F | H | H | Br | H | nonafluoro-2-butyl | H | OCF3 |
| 7-2976 | 3-cyanophenyl | Me | H | H | F | H | H | Br | H | nonafluoro-2-butyl | H | OCF3 |
| 7-2977 | 4-cyanophenyl | Me | H | H | F | H | H | Br | H | nonafluoro-2-butyl | H | OCF3 |
| 7-2992 | 2-chloropyridin-3-yl | Me | H | H | F | H | H | Br | H | nonafluoro-2-butyl | H | OCF3 |
| 7-3034 | phenyl | Me | H | H | F | H | H | I | H | heptafluoroisopropyl | H | OCF3 |
| 7-3054 | 3-cyanophenyl | Me | H | H | F | H | H | I | H | heptafluoroisopropyl | H | OCF3 |
| 7-3055 | 4-cyanophenyl | Me | H | H | F | H | H | I | H | heptafluoroisopropyl | H | OCF3 |
| 7-3070 | 2-chloropyridin-3-yl | Me | H | H | F | H | H | I | H | heptafluoroisopropyl | H | OCF3 |
| 7-3112 | phenyl | Me | H | H | F | H | H | I | H | nonafluoro-2-butyl | H | OCF3 |
| 7-3132 | 3-cyanophenyl | Me | H | H | F | H | H | I | H | nonafluoro-2-butyl | H | OCF3 |
| 7-3133 | 4-cyanophenyl | Me | H | H | F | H | H | I | H | nonafluoro-2-butyl | H | OCF3 |
| 7-3148 | 2-chloropyridin-3-yl | Me | H | H | F | H | H | I | H | nonafluoro-2-butyl | H | OCF3 |
| 7-3190 | phenyl | Me | H | H | F | H | H | F | H | heptafluoroisopropyl | H | CF3 |
| 7-3191 | phenyl | Me | H | H | F | H | H | Cl | H | heptafluoroisopropyl | H | CF3 |
| 7-3211 | 3-cyanophenyl | Me | H | H | F | H | H | Cl | H | heptafluoroisopropyl | H | CF3 |
| 7-3212 | 4-cyanophenyl | Me | H | H | F | H | H | Cl | H | heptafluoroisopropyl | H | CF3 |
| 7-3227 | 2-chloropyridin-3-yl | Me | H | H | F | H | H | Cl | H | heptafluoroisopropyl | H | CF3 |
| 7-3269 | phenyl | Me | H | H | F | H | H | F | H | nonafluoro-2-butyl | H | CF3 |
| 7-3270 | phenyl | Me | H | H | F | H | H | Cl | H | nonafluoro-2-butyl | H | CF3 |
| 7-3290 | 3-cyanophenyl | Me | H | H | F | H | H | Cl | H | nonafluoro-2-butyl | H | CF3 |
| 7-3291 | 4-cyanophenyl | Me | H | H | F | H | H | Cl | H | nonafluoro-2-butyl | H | CF3 |
| 7-3306 | 2-chloropyridin-3-yl | Me | H | H | F | H | H | Cl | H | nonafluoro-2-butyl | H | CF3 |
| 7-3348 | phenyl | Me | H | H | F | H | H | Br | H | heptafluoroisopropyl | H | CF3 |
| 7-3368 | 3-cyanophenyl | Me | H | H | F | H | H | Br | H | heptafluoroisopropyl | H | CF3 |
| 7-3369 | 4-cyanophenyl | Me | H | H | F | H | H | Br | H | heptafluoroisopropyl | H | CF3 |
| 7-3384 | 2-chloropyridin-3-yl | Me | H | H | F | H | H | Br | H | heptafluoroisopropyl | H | CF3 |
| 7-3426 | phenyl | Me | H | H | F | H | H | Br | H | nonafluoro-2-butyl | H | CF3 |
| 7-3446 | 3-cyanophenyl | Me | H | H | F | H | H | Br | H | nonafluoro-2-butyl | H | CF3 |
| 7-3447 | 4-cyanophenyl | Me | H | H | F | H | H | Br | H | nonafluoro-2-butyl | H | CF3 |
| 7-3462 | 2-chloropyridin-3-yl | Me | H | H | F | H | H | Br | H | nonafluoro-2-butyl | H | CF3 |
| 7-3504 | phenyl | Me | H | H | F | H | H | I | H | heptafluoroisopropyl | H | CF3 |
| 7-3524 | 3-cyanophenyl | Me | H | H | F | H | H | I | H | heptafluoroisopropyl | H | CF3 |
| 7-3525 | 4-cyanophenyl | Me | H | H | F | H | H | I | H | heptafluoroisopropyl | H | CF3 |
| 7-3582 | phenyl | Me | H | H | F | H | H | I | H | nonafluoro-2-butyl | H | CF3 |
| 7-3602 | 3-cyanophenyl | Me | H | H | F | H | H | I | H | nonafluoro-2-butyl | H | CF3 |
| 7-3603 | 4-cyanophenyl | Me | H | H | F | H | H | I | H | nonafluoro-2-butyl | H | CF3 |
| 7-3618 | 2-chloropyridin-3-yl | Me | H | H | F | H | H | I | H | nonafluoro-2-butyl | H | CF3 |
| 7-3660 | phenyl | Me | H | H | F | H | H | OCF3 | H | heptafluoroisopropyl | H | OCF3 |
| 7-3691 | 2-chloropyridin-3-yl | Me | H | H | F | H | H | OCF3 | H | heptafluoroisopropyl | H | OCF3 |
| 7-3716 | phenyl | Me | H | H | F | H | H | OCF3 | H | nonafluoro-2-butyl | H | OCF3 |
| 7-3747 | 2-chloropyridin-3-yl | Me | H | H | F | H | H | OCF3 | H | nonafluoro-2-butyl | H | OCF3 |
| 7-3772 | phenyl | Me | H | H | F | H | H | CF3 | H | heptafluoroisopropyl | H | OCF3 |
| 7-3803 | 2-chloropyridin-3-yl | Me | H | H | F | H | H | CF3 | H | heptafluoroisopropyl | H | OCF3 |
| 7-3828 | phenyl | Me | H | H | F | H | H | CF3 | H | nonafluoro-2-butyl | H | OCF3 |
| 7-3859 | 2-chloropyridin-3-yl | Me | H | H | F | H | H | CF3 | H | nonafluoro-2-butyl | H | OCF3 |
| 7-3884 | phenyl | Me | H | H | F | H | H | CF3 | H | heptafluoroisopropyl | H | CF3 |
| 7-3904 | 3-cyanophenyl | Me | H | H | F | H | H | CF3 | H | heptafluoroisopropyl | H | CF3 |
| 7-3905 | 4-cyanophenyl | Me | H | H | F | H | H | CF3 | H | heptafluoroisopropyl | H | CF3 |
| 7-3920 | 2-chloropyridin-3-yl | Me | H | H | F | H | H | CF3 | H | heptafluoroisopropyl | H | CF3 |
| 7-3952 | phenyl | Me | H | H | F | H | H | CF3 | H | nonafluoro-2-butyl | H | CF3 |
| 7-3972 | 3-cyanophenyl | Me | H | H | F | H | H | CF3 | H | nonafluoro-2-butyl | H | CF3 |
| 7-3973 | 4-cyanophenyl | Me | H | H | F | H | H | CF3 | H | nonafluoro-2-butyl | H | CF3 |
| 7-3988 | 2-chloropyridin-3-yl | Me | H | H | F | H | H | CF3 | H | nonafluoro-2-butyl | H | CF3 |
| 7-4020 | phenyl | Me | H | F | F | H | H | F | H | heptafluoroisopropyl | H | F |
| 7-4021 | 3-cyanophenyl | Me | H | F | F | H | H | F | H | heptafluoroisopropyl | H | F |
| 7-4022 | 4-cyanophenyl | Me | H | F | F | H | H | F | H | heptafluoroisopropyl | H | F |
| 7-4023 | 2-chloropyridin-3-yl | Me | H | F | F | H | H | F | H | heptafluoroisopropyl | H | F |
| 7-4024 | phenyl | Me | H | F | F | H | H | F | H | nonafluoro-2-butyl | H | F |
| 7-4025 | 3-cyanophenyl | Me | H | F | F | H | H | F | H | nonafluoro-2-butyl | H | F |
| 7-4026 | 4-cyanophenyl | Me | H | F | F | H | H | F | H | nonafluoro-2-butyl | H | F |

**Table 7(11)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 7-4027 | 2-chloropyridin-3-yl | Me | H | F | F | H | H | F | H | nonafluoro-2-butyl | H | F |
| 7-4028 | phenyl | Me | H | F | F | H | H | Cl | H | heptafluoroisopropyl | H | Cl |
| 7-4048 | 3-cyanophenyl | Me | H | F | F | H | H | Cl | H | heptafluoroisopropyl | H | Cl |
| 7-4049 | 4-cyanophenyl | Me | H | F | F | H | H | Cl | H | heptafluoroisopropyl | H | Cl |
| 7-4064 | 2-chloropyridin-3-yl | Me | H | F | F | H | H | Cl | H | heptafluoroisopropyl | H | Cl |
| 7-4106 | phenyl | Me | H | F | F | H | H | Cl | H | nonafluoro-2-butyl | H | Cl |
| 7-4126 | 3-cyanophenyl | Me | H | F | F | H | H | Cl | H | nonafluoro-2-butyl | H | Cl |
| 7-4127 | 4-cyanophenyl | Me | H | F | F | H | H | Cl | H | nonafluoro-2-butyl | H | Cl |
| 7-4142 | 2-chloropyridin-3-yl | Me | H | F | F | H | H | Cl | H | nonafluoro-2-butyl | H | Cl |
| 7-4184 | phenyl | Me | H | F | F | H | H | Br | H | heptafluoroisopropyl | H | Br |
| 7-4204 | 3-cyanophenyl | Me | H | F | F | H | H | Br | H | heptafluoroisopropyl | H | Br |
| 7-4205 | 4-cyanophenyl | Me | H | F | F | H | H | Br | H | heptafluoroisopropyl | H | Br |
| 7-4220 | 2-chloropyridin-3-yl | Me | H | F | F | H | H | Br | H | heptafluoroisopropyl | H | Br |
| 7-4262 | phenyl | Me | H | F | F | H | H | Br | H | nonafluoro-2-butyl | H | Br |
| 7-4282 | 3-cyanophenyl | Me | H | F | F | H | H | Br | H | nonafluoro-2-butyl | H | Br |
| 7-4283 | 4-cyanophenyl | Me | H | F | F | H | H | Br | H | nonafluoro-2-butyl | H | Br |
| 7-4298 | 2-chloropyridin-3-yl | Me | H | F | F | H | H | Br | H | nonafluoro-2-butyl | H | Br |
| 7-4340 | phenyl | Me | H | F | F | H | H | I | H | heptafluoroisopropyl | H | I |
| 7-4360 | 3-cyanophenyl | Me | H | F | F | H | H | I | H | heptafluoroisopropyl | H | I |
| 7-4361 | 4-cyanophenyl | Me | H | F | F | H | H | I | H | heptafluoroisopropyl | H | I |
| 7-4376 | 2-chloropyridin-3-yl | Me | H | F | F | H | H | I | H | heptafluoroisopropyl | H | I |
| 7-4418 | phenyl | Me | H | F | F | H | H | I | H | nonafluoro-2-butyl | H | I |
| 7-4438 | 3-cyanophenyl | Me | H | F | F | H | H | I | H | nonafluoro-2-butyl | H | I |
| 7-4439 | 4-cyanophenyl | Me | H | F | F | H | H | I | H | nonafluoro-2-butyl | H | I |
| 7-4454 | 2-chloropyridin-3-yl | Me | H | F | F | H | H | I | H | nonafluoro-2-butyl | H | I |
| 7-4496 | phenyl | Me | H | F | F | H | H | Cl | H | heptafluoroisopropyl | H | OCF3 |
| 7-4516 | 3-cyanophenyl | Me | H | F | F | H | H | Cl | H | heptafluoroisopropyl | H | OCF3 |
| 7-4517 | 4-cyanophenyl | Me | H | F | F | H | H | Cl | H | heptafluoroisopropyl | H | OCF3 |
| 7-4532 | 2-chloropyridin-3-yl | Me | H | F | F | H | H | Cl | H | heptafluoroisopropyl | H | OCF3 |
| 7-4574 | phenyl | Me | H | F | F | H | H | Cl | H | nonafluoro-2-butyl | H | OCF3 |
| 7-4594 | 3-cyanophenyl | Me | H | F | F | H | H | Cl | H | nonafluoro-2-butyl | H | OCF3 |
| 7-4595 | 4-cyanophenyl | Me | H | F | F | H | H | Cl | H | nonafluoro-2-butyl | H | OCF3 |
| 7-4610 | 2-chloropyridin-3-yl | Me | H | F | F | H | H | Cl | H | nonafluoro-2-butyl | H | OCF3 |
| 7-4652 | phenyl | Me | H | F | F | H | H | Br | H | heptafluoroisopropyl | H | OCF3 |
| 7-4672 | 3-cyanophenyl | Me | H | F | F | H | H | Br | H | heptafluoroisopropyl | H | OCF3 |
| 7-4673 | 4-cyanophenyl | Me | H | F | F | H | H | Br | H | heptafluoroisopropyl | H | OCF3 |
| 7-4688 | 2-chloropyridin-3-yl | Me | H | F | F | H | H | Br | H | heptafluoroisopropyl | H | OCF3 |
| 7-4730 | phenyl | Me | H | F | F | H | H | Br | H | nonafluoro-2-butyl | H | OCF3 |
| 7-4750 | 3-cyanophenyl | Me | H | F | F | H | H | Br | H | nonafluoro-2-butyl | H | OCF3 |
| 7-4751 | 4-cyanophenyl | Me | H | F | F | H | H | Br | H | nonafluoro-2-butyl | H | OCF3 |
| 7-4766 | 2-chloropyridin-3-yl | Me | H | F | F | H | H | Br | H | nonafluoro-2-butyl | H | OCF3 |
| 7-4808 | phenyl | Me | H | F | F | H | H | I | H | heptafluoroisopropyl | H | OCF3 |
| 7-4828 | 3-cyanophenyl | Me | H | F | F | H | H | I | H | heptafluoroisopropyl | H | OCF3 |
| 7-4829 | 4-cyanophenyl | Me | H | F | F | H | H | I | H | heptafluoroisopropyl | H | OCF3 |
| 7-4844 | 2-chloropyridin-3-yl | Me | H | F | F | H | H | I | H | heptafluoroisopropyl | H | OCF3 |
| 7-4886 | phenyl | Me | H | F | F | H | H | I | H | nonafluoro-2-butyl | H | OCF3 |
| 7-4906 | 3-cyanophenyl | Me | H | F | F | H | H | I | H | nonafluoro-2-butyl | H | OCF3 |
| 7-4907 | 4-cyanophenyl | Me | H | F | F | H | H | I | H | nonafluoro-2-butyl | H | OCF3 |
| 7-4922 | 2-chloropyridin-3-yl | Me | H | F | F | H | H | I | H | nonafluoro-2-butyl | H | OCF3 |
| 7-4964 | phenyl | Me | H | F | F | H | H | F | H | heptafluoroisopropyl | H | CF3 |
| 7-4965 | phenyl | Me | H | F | F | H | H | Cl | H | heptafluoroisopropyl | H | CF3 |
| 7-4985 | 3-cyanophenyl | Me | H | F | F | H | H | Cl | H | heptafluoroisopropyl | H | CF3 |
| 7-4986 | 4-cyanophenyl | Me | H | F | F | H | H | Cl | H | heptafluoroisopropyl | H | CF3 |
| 7-5001 | 2-chloropyridin-3-yl | Me | H | F | F | H | H | Cl | H | heptafluoroisopropyl | H | CF3 |
| 7-5043 | phenyl | Me | H | F | F | H | H | F | H | nonafluoro-2-butyl | H | CF3 |
| 7-5044 | phenyl | Me | H | F | F | H | H | Cl | H | nonafluoro-2-butyl | H | CF3 |
| 7-5064 | 3-cyanophenyl | Me | H | F | F | H | H | Cl | H | nonafluoro-2-butyl | H | CF3 |
| 7-5065 | 4-cyanophenyl | Me | H | F | F | H | H | Cl | H | nonafluoro-2-butyl | H | CF3 |
| 7-5080 | 2-chloropyridin-3-yl | Me | H | F | F | H | H | Cl | H | nonafluoro-2-butyl | H | CF3 |
| 7-5122 | phenyl | Me | H | F | F | H | H | Br | H | heptafluoroisopropyl | H | CF3 |
| 7-5142 | 3-cyanophenyl | Me | H | F | F | H | H | Br | H | heptafluoroisopropyl | H | CF3 |
| 7-5143 | 4-cyanophenyl | Me | H | F | F | H | H | Br | H | heptafluoroisopropyl | H | CF3 |
| 7-5158 | 2-chloropyridin-3-yl | Me | H | F | F | H | H | Br | H | heptafluoroisopropyl | H | CF3 |
| 7-5200 | phenyl | Me | H | F | F | H | H | Br | H | nonafluoro-2-butyl | H | CF3 |

**Table 7(12)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 7-5220 | 3-cyanophenyl | Me | H | F | F | H | H | Br | H | nonafluoro-2-butyl | H | CF3 |
| 7-5221 | 4-cyanophenyl | Me | H | F | F | H | H | Br | H | nonafluoro-2-butyl | H | CF3 |
| 7-5236 | 2-chloropyridin-3-yl | Me | H | F | F | H | H | Br | H | nonafluoro-2-butyl | H | CF3 |
| 7-5278 | phenyl | Me | H | F | F | H | H | I | H | heptafluoroisopropyl | H | CF3 |
| 7-5298 | 3-cyanophenyl | Me | H | F | F | H | H | I | H | heptafluoroisopropyl | H | CF3 |
| 7-5299 | 4-cyanophenyl | Me | H | F | F | H | H | I | H | heptafluoroisopropyl | H | CF3 |
| 7-5314 | 2-chloropyridin-3-yl | Me | H | F | F | H | H | I | H | heptafluoroisopropyl | H | CF3 |
| 7-5356 | phenyl | Me | H | F | F | H | H | I | H | nonafluoro-2-butyl | H | CF3 |
| 7-5376 | 3-cyanophenyl | Me | H | F | F | H | H | I | H | nonafluoro-2-butyl | H | CF3 |
| 7-5377 | 4-cyanophenyl | Me | H | F | F | H | H | I | H | nonafluoro-2-butyl | H | CF3 |
| 7-5392 | 2-chloropyridin-3-yl | Me | H | F | F | H | H | I | H | nonafluoro-2-butyl | H | CF3 |
| 7-5434 | phenyl | Me | H | F | F | H | H | OCF3 | H | heptafluoroisopropyl | H | OCF3 |
| 7-5465 | 2-chloropyridin-3-yl | Me | H | F | F | H | H | OCF3 | H | heptafluoroisopropyl | H | OCF3 |
| 7-5490 | phenyl | Me | H | F | F | H | H | OCF3 | H | nonafluoro-2-butyl | H | OCF3 |
| 7-5521 | 2-chloropyridin-3-yl | Me | H | F | F | H | H | OCF3 | H | nonafluoro-2-butyl | H | OCF3 |
| 7-5546 | phenyl | Me | H | F | F | H | H | CF3 | H | heptafluoroisopropyl | H | OCF3 |
| 7-5577 | 2-chloropyridin-3-yl | Me | H | F | F | H | H | CF3 | H | heptafluoroisopropyl | H | OCF3 |
| 7-5602 | phenyl | Me | H | F | F | H | H | CF3 | H | nonafluoro-2-butyl | H | OCF3 |
| 7-5633 | 2-chloropyridin-3-yl | Me | H | F | F | H | H | CF3 | H | nonafluoro-2-butyl | H | OCF3 |
| 7-5658 | phenyl | Me | H | F | F | H | H | CF3 | H | heptafluoroisopropyl | H | CF3 |
| 7-5678 | 3-cyanophenyl | Me | H | F | F | H | H | CF3 | H | heptafluoroisopropyl | H | CF3 |
| 7-5679 | 4-cyanophenyl | Me | H | F | F | H | H | CF3 | H | heptafluoroisopropyl | H | CF3 |
| 7-5694 | 2-chloropyridin-3-yl | Me | H | F | F | H | H | CF3 | H | heptafluoroisopropyl | H | CF3 |
| 7-5726 | phenyl | Me | H | F | F | H | H | CF3 | H | nonafluoro-2-butyl | H | CF3 |
| 7-5746 | 3-cyanophenyl | Me | H | F | F | H | H | CF3 | H | nonafluoro-2-butyl | H | CF3 |
| 7-5747 | 4-cyanophenyl | Me | H | F | F | H | H | CF3 | H | nonafluoro-2-butyl | H | CF3 |
| 7-5762 | 2-chloropyridin-3-yl | Me | H | F | F | H | H | CF3 | H | nonafluoro-2-butyl | H | CF3 |
| 7-5794 | phenyl | Me | H | F | F | F | H | Cl | H | pentafluoroethyl | H | Cl |
| 7-5795 | phenyl | Me | H | F | F | F | H | Br | H | heptafluoroisopropyl | H | Br |
| 7-5796 | phenyl | Me | H | F | F | F | H | Br | H | heptafluoroisopropyl | H | OCF3 |
| 7-5797 | phenyl | Me | H | F | F | F | H | I | H | heptafluoroisopropyl | H | CF3 |
| 7-5798 | phenyl | Me | H | F | F | F | H | CF3 | H | heptafluoroisopropyl | H | CF3 |
| 7-5799 | phenyl | Me | H | F | F | F | H | I | H | nonafluoro-2-butyl | H | I |
| 7-5800 | phenyl | Me | H | F | F | F | H | Cl | H | nonafluoro-2-butyl | H | OCF3 |
| 7-5801 | phenyl | Me | H | F | F | F | H | Br | H | nonafluoro-2-butyl | H | CF3 |
| 7-5802 | phenyl | Me | H | F | F | F | H | CF3 | H | nonafluoro-2-butyl | H | CF3 |
| 7-5803 | phenyl | Me | H | H | H | F | H | Cl | H | pentafluoroethyl | H | Cl |
| 7-5804 | phenyl | Me | H | H | H | F | H | Br | H | heptafluoroisopropyl | H | Br |
| 7-5805 | phenyl | Me | H | H | H | F | H | Br | H | heptafluoroisopropyl | H | OCF3 |
| 7-5806 | phenyl | Me | H | H | H | F | H | I | H | heptafluoroisopropyl | H | CF3 |
| 7-5807 | phenyl | Me | H | H | H | F | H | CF3 | H | heptafluoroisopropyl | H | CF3 |
| 7-5808 | phenyl | Me | H | H | H | F | H | I | H | nonafluoro-2-butyl | H | I |
| 7-5809 | phenyl | Me | H | H | H | F | H | Cl | H | nonafluoro-2-butyl | H | OCF3 |
| 7-5810 | phenyl | Me | H | H | H | F | H | Br | H | nonafluoro-2-butyl | H | CF3 |
| 7-5811 | phenyl | Me | H | H | H | F | H | CF3 | H | nonafluoro-2-butyl | H | CF3 |
| 7-5812 | phenyl | Me | H | H | H | H | F | Cl | H | pentafluoroethyl | H | Cl |
| 7-5813 | phenyl | Me | H | H | H | H | F | Br | H | heptafluoroisopropyl | H | Br |
| 7-5814 | phenyl | Me | H | H | H | H | F | Br | H | heptafluoroisopropyl | H | OCF3 |
| 7-5815 | phenyl | Me | H | H | H | H | F | I | H | heptafluoroisopropyl | H | CF3 |
| 7-5816 | phenyl | Me | H | H | H | H | F | CF3 | H | heptafluoroisopropyl | H | CF3 |
| 7-5817 | phenyl | Me | H | H | H | H | F | I | H | nonafluoro-2-butyl | H | I |
| 7-5818 | phenyl | Me | H | H | H | H | F | Cl | H | nonafluoro-2-butyl | H | OCF3 |
| 7-5819 | phenyl | Me | H | H | H | H | F | Br | H | nonafluoro-2-butyl | H | CF3 |
| 7-5820 | phenyl | Me | H | H | H | H | F | CF3 | H | nonafluoro-2-butyl | H | CF3 |
| 7-5821 | phenyl | Me | H | F | H | F | H | Cl | H | pentafluoroethyl | H | Cl |
| 7-5822 | phenyl | Me | H | F | H | F | H | Br | H | heptafluoroisopropyl | H | Br |
| 7-5823 | phenyl | Me | H | F | H | F | H | Br | H | heptafluoroisopropyl | H | OCF3 |
| 7-5824 | phenyl | Me | H | F | H | F | H | I | H | heptafluoroisopropyl | H | CF3 |
| 7-5825 | phenyl | Me | H | F | H | F | H | CF3 | H | heptafluoroisopropyl | H | CF3 |
| 7-5826 | phenyl | Me | H | F | H | F | H | I | H | nonafluoro-2-butyl | H | I |
| 7-5827 | phenyl | Me | H | F | H | F | H | Cl | H | nonafluoro-2-butyl | H | OCF3 |
| 7-5828 | phenyl | Me | H | F | H | F | H | Br | H | nonafluoro-2-butyl | H | CF3 |
| 7-5829 | phenyl | Me | H | F | H | F | H | CF3 | H | nonafluoro-2-butyl | H | CF3 |
| 7-5830 | phenyl | Me | H | F | H | H | F | Cl | H | pentafluoroethyl | H | Cl |

**Table 7(13)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 7-5831 | phenyl | Me | H | F | H | H | F | Br | H | heptafluoroisopropyl | H | Br |
| 7-5832 | phenyl | Me | H | F | H | H | F | Br | H | heptafluoroisopropyl | H | OCF3 |
| 7-5833 | phenyl | Me | H | F | H | H | F | I | H | heptafluoroisopropyl | H | CF3 |
| 7-5834 | phenyl | Me | H | F | H | H | F | CF3 | H | heptafluoroisopropyl | H | CF3 |
| 7-5835 | phenyl | Me | H | F | H | H | F | I | H | nonafluoro-2-butyl | H | I |
| 7-5836 | phenyl | Me | H | F | H | H | F | Cl | H | nonafluoro-2-butyl | H | OCF3 |
| 7-5837 | phenyl | Me | H | F | H | H | F | Br | H | nonafluoro-2-butyl | H | CF3 |
| 7-5838 | phenyl | Me | H | F | H | H | F | CF3 | H | nonafluoro-2-butyl | H | CF3 |
| 7-5839 | phenyl | Me | H | H | F | F | H | Cl | H | pentafluoroethyl | H | Cl |
| 7-5840 | phenyl | Me | H | H | F | F | H | Br | H | heptafluoroisopropyl | H | Br |
| 7-5841 | phenyl | Me | H | H | F | F | H | Br | H | heptafluoroisopropyl | H | OCF3 |
| 7-5842 | phenyl | Me | H | H | F | F | H | I | H | heptafluoroisopropyl | H | CF3 |
| 7-5843 | phenyl | Me | H | H | F | F | H | CF3 | H | heptafluoroisopropyl | H | CF3 |
| 7-5844 | phenyl | Me | H | H | F | F | H | I | H | nonafluoro-2-butyl | H | I |
| 7-5845 | phenyl | Me | H | H | F | F | H | Cl | H | nonafluoro-2-butyl | H | OCF3 |
| 7-5846 | phenyl | Me | H | H | F | F | H | Br | H | nonafluoro-2-butyl | H | CF3 |
| 7-5847 | phenyl | Me | H | H | F | F | H | CF3 | H | nonafluoro-2-butyl | H | CF3 |
| 7-5848 | phenyl | Me | H | H | F | H | F | Cl | H | pentafluoroethyl | H | Cl |
| 7-5849 | phenyl | Me | H | H | F | H | F | Br | H | heptafluoroisopropyl | H | Br |
| 7-5850 | phenyl | Me | H | H | F | H | F | Br | H | heptafluoroisopropyl | H | OCF3 |
| 7-5851 | phenyl | Me | H | H | F | H | F | I | H | heptafluoroisopropyl | H | CF3 |
| 7-5852 | phenyl | Me | H | H | F | H | F | CF3 | H | heptafluoroisopropyl | H | CF3 |
| 7-5853 | phenyl | Me | H | H | F | H | F | I | H | nonafluoro-2-butyl | H | I |
| 7-5854 | phenyl | Me | H | H | F | H | F | Cl | H | nonafluoro-2-butyl | H | OCF3 |
| 7-5855 | phenyl | Me | H | H | F | H | F | Br | H | nonafluoro-2-butyl | H | CF3 |
| 7-5856 | phenyl | Me | H | H | F | H | F | CF3 | H | nonafluoro-2-butyl | H | CF3 |
| 7-5857 | phenyl | Me | H | H | H | F | F | Cl | H | pentafluoroethyl | H | Cl |
| 7-5858 | phenyl | Me | H | H | H | F | F | Br | H | heptafluoroisopropyl | H | Br |
| 7-5859 | phenyl | Me | H | H | H | F | F | Br | H | heptafluoroisopropyl | H | OCF3 |
| 7-5860 | phenyl | Me | H | H | H | F | F | I | H | heptafluoroisopropyl | H | CF3 |
| 7-5861 | phenyl | Me | H | H | H | F | F | CF3 | H | heptafluoroisopropyl | H | CF3 |
| 7-5862 | phenyl | Me | H | H | H | F | F | I | H | nonafluoro-2-butyl | H | I |
| 7-5863 | phenyl | Me | H | H | H | F | F | Cl | H | nonafluoro-2-butyl | H | OCF3 |
| 7-5864 | phenyl | Me | H | H | H | F | F | Br | H | nonafluoro-2-butyl | H | CF3 |
| 7-5865 | phenyl | Me | H | H | H | F | F | CF3 | H | nonafluoro-2-butyl | H | CF3 |
| 7-5866 | phenyl | Me | H | F | F | H | F | Cl | H | pentafluoroethyl | H | Cl |
| 7-5867 | phenyl | Me | H | F | F | H | F | Br | H | heptafluoroisopropyl | H | Br |
| 7-5868 | phenyl | Me | H | F | F | H | F | Br | H | heptafluoroisopropyl | H | OCF3 |
| 7-5869 | phenyl | Me | H | F | F | H | F | I | H | heptafluoroisopropyl | H | CF3 |
| 7-5870 | phenyl | Me | H | F | F | H | F | CF3 | H | heptafluoroisopropyl | H | CF3 |
| 7-5871 | phenyl | Me | H | F | F | H | F | I | H | nonafluoro-2-butyl | H | I |
| 7-5872 | phenyl | Me | H | F | F | H | F | Cl | H | nonafluoro-2-butyl | H | OCF3 |
| 7-5873 | phenyl | Me | H | F | F | H | F | Br | H | nonafluoro-2-butyl | H | CF3 |
| 7-5874 | phenyl | Me | H | F | F | H | F | CF3 | H | nonafluoro-2-butyl | H | CF3 |
| 7-5875 | phenyl | Me | H | F | H | F | F | Cl | H | pentafluoroethyl | H | Cl |
| 7-5876 | phenyl | Me | H | F | H | F | F | Br | H | heptafluoroisopropyl | H | Br |
| 7-5877 | phenyl | Me | H | F | H | F | F | Br | H | heptafluoroisopropyl | H | OCF3 |
| 7-5878 | phenyl | Me | H | F | H | F | F | I | H | heptafluoroisopropyl | H | CF3 |
| 7-5879 | phenyl | Me | H | F | H | F | F | CF3 | H | heptafluoroisopropyl | H | CF3 |
| 7-5880 | phenyl | Me | H | F | H | F | F | I | H | nonafluoro-2-butyl | H | I |
| 7-5881 | phenyl | Me | H | F | H | F | F | Cl | H | nonafluoro-2-butyl | H | OCF3 |
| 7-5882 | phenyl | Me | H | F | H | F | F | Br | H | nonafluoro-2-butyl | H | CF3 |
| 7-5883 | phenyl | Me | H | F | H | F | F | CF3 | H | nonafluoro-2-butyl | H | CF3 |
| 7-5884 | phenyl | Me | H | H | F | F | F | Cl | H | pentafluoroethyl | H | Cl |
| 7-5885 | phenyl | Me | H | H | F | F | F | Br | H | heptafluoroisopropyl | H | Br |
| 7-5886 | phenyl | Me | H | H | F | F | F | Br | H | heptafluoroisopropyl | H | OCF3 |
| 7-5887 | phenyl | Me | H | H | F | F | F | I | H | heptafluoroisopropyl | H | CF3 |
| 7-5888 | phenyl | Me | H | H | F | F | F | CF3 | H | heptafluoroisopropyl | H | CF3 |
| 7-5889 | phenyl | Me | H | H | F | F | F | I | H | nonafluoro-2-butyl | H | I |
| 7-5890 | phenyl | Me | H | H | F | F | F | Cl | H | nonafluoro-2-butyl | H | OCF3 |
| 7-5891 | phenyl | Me | H | H | F | F | F | Br | H | nonafluoro-2-butyl | H | CF3 |
| 7-5892 | phenyl | Me | H | H | F | F | F | CF3 | H | nonafluoro-2-butyl | H | CF3 |
| 7-5893 | phenyl | Me | H | F | F | F | F | Cl | H | pentafluoroethyl | H | Cl |

**Table 7(14)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 7-5894 | phenyl | Me | H | F | F | F | F | Br | H | heptafluoroisopropyl | H | Br |
| 7-5895 | phenyl | Me | H | F | F | F | F | Br | H | heptafluoroisopropyl | H | OCF3 |
| 7-5896 | phenyl | Me | H | F | F | F | F | I | H | heptafluoroisopropyl | H | CF3 |
| 7-5897 | phenyl | Me | H | F | F | F | F | CF3 | H | heptafluoroisopropyl | H | CF3 |
| 7-5898 | phenyl | Me | H | F | F | F | F | I | H | nonafluoro-2-butyl | H | I |
| 7-5899 | phenyl | Me | H | F | F | F | F | Cl | H | nonafluoro-2-butyl | H | OCF3 |
| 7-5900 | phenyl | Me | H | F | F | F | F | Br | H | nonafluoro-2-butyl | H | CF3 |
| 7-5901 | phenyl | Me | H | F | F | F | F | CF3 | H | nonafluoro-2-butyl | H | CF3 |
| 7-5902 | phenyl | Me | H | F | H | H | H | I | H | heptafluoroisopropyl | H | Br |
| 7-5903 | 2-chloropyridine-3-yl | Me | H | F | H | H | H | I | H | heptafluoroisopropyl | H | Br |
| 7-5904 | 3-fluorophenyl | Me | H | H | H | H | H | I | H | nonafluoro-2-butyl | H | I |
| 7-5905 | 4-fluorophenyl | Me | H | H | H | H | H | I | H | nonafluoro-2-butyl | H | I |
| 7-5906 | 3-fluorophenyl | Me | H | H | H | H | H | Br | H | nonafluoro-2-butyl | H | Br |
| 7-5907 | 4-fluorophenyl | Me | H | H | H | H | H | Br | H | nonafluoro-2-butyl | H | Br |
| 7-5908 | 4-(methylsulfonyl)phenyl | Me | H | H | H | H | H | Br | H | heptafluoroisopropyl | H | CF3 |
| 7-5909 | pyrimidine-5-yl | Me | H | H | H | H | H | I | H | heptafluoroisopropyl | H | CF3 |
| 7-5910 | 3-cyano-5-(methoxycarbonyl)phenyl | Me | H | F | H | H | H | Br | H | heptafluoroisopropyl | H | CF3 |
| 7-5911 | phenyl | Me | H | H | H | H | H | Br | H | pentafluoroethyl | H | CF3 |
| 7-5912 | 2-chloropyridine-3-yl | Me | H | H | H | H | H | Br | H | pentafluoroethyl | H | CF3 |

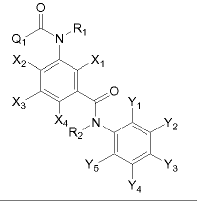

**Table 8(1)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 8-1 | phenyl | H | Me | H | H | H | H | F | H | nonafluoro-2-butyl | H | F |
| 8-2 | 3-cyanophenyl | H | Me | H | H | H | H | F | H | nonafluoro-2-butyl | H | F |
| 8-3 | 4-cyanophenyl | H | Me | H | H | H | H | F | H | nonafluoro-2-butyl | H | F |
| 8-4 | 2-chloropyridin-3-yl | H | Me | H | H | H | H | F | H | nonafluoro-2-butyl | H | F |
| 8-5 | phenyl | H | Me | H | H | H | H | Cl | H | nonafluoro-2-butyl | H | Cl |
| 8-12 | 3-cyanophenyl | H | Me | H | H | H | H | Cl | H | nonafluoro-2-butyl | H | Cl |
| 8-13 | 4-cyanophenyl | H | Me | H | H | H | H | Cl | H | nonafluoro-2-butyl | H | Cl |
| 8-24 | 2-chloropyridin-3-yl | H | Me | H | H | H | H | Cl | H | nonafluoro-2-butyl | H | Cl |
| 8-38 | phenyl | H | Me | H | H | H | H | Br | H | nonafluoro-2-butyl | H | Br |
| 8-45 | 3-cyanophenyl | H | Me | H | H | H | H | Br | H | nonafluoro-2-butyl | H | Br |
| 8-46 | 4-cyanophenyl | H | Me | H | H | H | H | Br | H | nonafluoro-2-butyl | H | Br |
| 8-57 | 2-chloropyridin-3-yl | H | Me | H | H | H | H | Br | H | nonafluoro-2-butyl | H | Br |
| 8-71 | phenyl | H | Me | H | H | H | H | I | H | nonafluoro-2-butyl | H | I |
| 8-78 | 3-cyanophenyl | H | Me | H | H | H | H | I | H | nonafluoro-2-butyl | H | I |
| 8-79 | 4-cyanophenyl | H | Me | H | H | H | H | I | H | nonafluoro-2-butyl | H | I |
| 8-90 | 2-chloropyridin-3-yl | H | Me | H | H | H | H | I | H | nonafluoro-2-butyl | H | I |
| 8-104 | phenyl | H | Me | H | H | H | H | F | H | nonafluoro-2-butyl | H | Cl |
| 8-105 | phenyl | H | Me | H | H | H | H | F | H | nonafluoro-2-butyl | H | Br |
| 8-106 | phenyl | H | Me | H | H | H | H | F | H | nonafluoro-2-butyl | H | I |
| 8-107 | phenyl | H | Me | H | H | H | H | Cl | H | nonafluoro-2-butyl | H | Br |
| 8-108 | phenyl | H | Me | H | H | H | H | Cl | H | nonafluoro-2-butyl | H | I |
| 8-109 | phenyl | H | Me | H | H | H | H | Br | H | nonafluoro-2-butyl | H | I |
| 8-110 | phenyl | H | Me | H | H | H | H | F | H | heptafluoroisopropyl | H | CF3 |
| 8-111 | phenyl | H | Me | H | H | H | H | Cl | H | heptafluoroisopropyl | H | CF3 |
| 8-131 | 3-cyanophenyl | H | Me | H | H | H | H | Cl | H | heptafluoroisopropyl | H | CF3 |
| 8-132 | 4-cyanophenyl | H | Me | H | H | H | H | Cl | H | heptafluoroisopropyl | H | CF3 |
| 8-147 | 2-chloropyridin-3-yl | H | Me | H | H | H | H | Cl | H | heptafluoroisopropyl | H | CF3 |
| 8-189 | phenyl | H | Me | H | H | H | H | F | H | nonafluoro-2-butyl | H | CF3 |
| 8-190 | phenyl | H | Me | H | H | H | H | Cl | H | nonafluoro-2-butyl | H | CF3 |
| 8-210 | 3-cyanophenyl | H | Me | H | H | H | H | Cl | H | nonafluoro-2-butyl | H | CF3 |
| 8-211 | 4-cyanophenyl | H | Me | H | H | H | H | Cl | H | nonafluoro-2-butyl | H | CF3 |
| 8-226 | 2-chloropyridin-3-yl | H | Me | H | H | H | H | Cl | H | nonafluoro-2-butyl | H | CF3 |
| 8-268 | phenyl | H | Me | H | H | H | H | Br | H | heptafluoroisopropyl | H | CF3 |
| 8-288 | 3-cyanophenyl | H | Me | H | H | H | H | Br | H | heptafluoroisopropyl | H | CF3 |
| 8-289 | 4-cyanophenyl | H | Me | H | H | H | H | Br | H | heptafluoroisopropyl | H | CF3 |
| 8-304 | 2-chloropyridin-3-yl | H | Me | H | H | H | H | Br | H | heptafluoroisopropyl | H | CF3 |
| 8-305 | 2-bromopyridin-3-yl | H | Me | H | H | H | H | Br | H | heptafluoroisopropyl | H | CF3 |
| 8-346 | phenyl | H | Me | H | H | H | H | Br | H | nonafluoro-2-butyl | H | CF3 |
| 8-366 | 3-cyanophenyl | H | Me | H | H | H | H | Br | H | nonafluoro-2-butyl | H | CF3 |
| 8-367 | 4-cyanophenyl | H | Me | H | H | H | H | Br | H | nonafluoro-2-butyl | H | CF3 |
| 8-382 | 2-chloropyridin-3-yl | H | Me | H | H | H | H | Br | H | nonafluoro-2-butyl | H | CF3 |
| 8-424 | phenyl | H | Me | H | H | H | H | I | H | heptafluoroisopropyl | H | CF3 |
| 8-444 | 3-cyanophenyl | H | Me | H | H | H | H | I | H | heptafluoroisopropyl | H | CF3 |
| 8-445 | 4-cyanophenyl | H | Me | H | H | H | H | I | H | heptafluoroisopropyl | H | CF3 |
| 8-460 | 2-chloropyridin-3-yl | H | Me | H | H | H | H | I | H | heptafluoroisopropyl | H | CF3 |
| 8-502 | phenyl | H | Me | H | H | H | H | I | H | nonafluoro-2-butyl | H | CF3 |
| 8-522 | 3-cyanophenyl | H | Me | H | H | H | H | I | H | nonafluoro-2-butyl | H | CF3 |
| 8-523 | 4-cyanophenyl | H | Me | H | H | H | H | I | H | nonafluoro-2-butyl | H | CF3 |
| 8-538 | 2-chloropyridin-3-yl | H | Me | H | H | H | H | I | H | nonafluoro-2-butyl | H | CF3 |
| 8-580 | phenyl | H | Me | H | H | H | H | OCF3 | H | heptafluoroisopropyl | H | OCF3 |
| 8-611 | 2-chloropyridin-3-yl | H | Me | H | H | H | H | OCF3 | H | heptafluoroisopropyl | H | OCF3 |
| 8-636 | phenyl | H | Me | H | H | H | H | OCF3 | H | nonafluoro-2-butyl | H | OCF3 |
| 8-667 | 2-chloropyridin-3-yl | H | Me | H | H | H | H | OCF3 | H | nonafluoro-2-butyl | H | OCF3 |
| 8-692 | phenyl | H | Me | H | H | H | H | CF3 | H | heptafluoroisopropyl | H | OCF3 |
| 8-723 | 2-chloropyridin-3-yl | H | Me | H | H | H | H | CF3 | H | heptafluoroisopropyl | H | OCF3 |

**Table 8(2)**

| compound number | Q₁ | R₁ | R₂ | X₁ | X₂ | X₃ | X₄ | Y₁ | Y₂ | Y₃ | Y₄ | Y₄ |
|---|---|---|---|---|---|---|---|---|---|---|---|---|
| 8-748 | phenyl | H | Me | H | H | H | H | CF3 | H | nonafluoro-2-butyl | H | OCF3 |
| 8-779 | 2-chloropyridin-3-yl | H | Me | H | H | H | H | CF3 | H | nonafluoro-2-butyl | H | OCF3 |
| 8-804 | phenyl | H | Me | H | H | H | H | CF3 | H | heptafluoroisopropyl | H | CF3 |
| 8-824 | 3-cyanophenyl | H | Me | H | H | H | H | CF3 | H | heptafluoroisopropyl | H | CF3 |
| 8-825 | 4-cyanophenyl | H | Me | H | H | H | H | CF3 | H | heptafluoroisopropyl | H | CF3 |
| 8-840 | 2-chloropyridin-3-yl | H | Me | H | H | H | H | CF3 | H | heptafluoroisopropyl | H | CF3 |
| 8-841 | 2-bromopyridin-3-yl | H | Me | H | H | H | H | CF3 | H | heptafluoroisopropyl | H | CF3 |
| 8-872 | phenyl | H | Me | H | H | H | H | CF3 | H | nonafluoro-2-butyl | H | CF3 |
| 8-892 | 3-cyanophenyl | H | Me | H | H | H | H | CF3 | H | nonafluoro-2-butyl | H | CF3 |
| 8-893 | 4-cyanophenyl | H | Me | H | H | H | H | CF3 | H | nonafluoro-2-butyl | H | CF3 |
| 8-908 | 2-chloropyridin-3-yl | H | Me | H | H | H | H | CF3 | H | nonafluoro-2-butyl | H | CF3 |
| 8-940 | phenyl | H | Me | F | H | H | H | F | H | heptafluoroisopropyl | H | F |
| 8-941 | 3-cyanophenyl | H | Me | F | H | H | H | F | H | heptafluoroisopropyl | H | F |
| 8-942 | 4-cyanophenyl | H | Me | F | H | H | H | F | H | heptafluoroisopropyl | H | F |
| 8-943 | 2-chloropyridin-3-yl | H | Me | F | H | H | H | F | H | heptafluoroisopropyl | H | F |
| 8-944 | phenyl | H | Me | F | H | H | H | F | H | nonafluoro-2-butyl | H | F |
| 8-945 | 3-cyanophenyl | H | Me | F | H | H | H | F | H | nonafluoro-2-butyl | H | F |
| 8-946 | 4-cyanophenyl | H | Me | F | H | H | H | F | H | nonafluoro-2-butyl | H | F |
| 8-947 | 2-chloropyridin-3-yl | H | Me | F | H | H | H | F | H | nonafluoro-2-butyl | H | F |
| 8-948 | phenyl | H | Me | F | H | H | H | Cl | H | heptafluoroisopropyl | H | Cl |
| 8-952 | 2-chlorophenyl | H | Me | F | H | H | H | F | H | heptafluoroisopropyl | H | Cl |
| 8-956 | 3-bromophenyl | H | Me | F | H | H | H | F | H | heptafluoroisopropyl | H | Br |
| 8-961 | (2⁻trifluoromethyl)phenyl | H | i-Pr | F | H | H | H | F | H | heptafluoroisopropyl | H | I |
| 8-964 | 2-nitrophenyl | H | Me | F | H | H | H | Cl | H | heptafluoroisopropyl | H | Br |
| 8-968 | 3-cyanophenyl | H | Me | F | H | H | H | Cl | H | heptafluoroisopropyl | H | Cl |
| 8-969 | 4-cyanophenyl | H | Me | F | H | H | H | Cl | H | heptafluoroisopropyl | H | Cl |
| 8-970 | 2,6-difluorophenyl | H | Me | F | H | H | H | Cl | H | heptafluoroisopropyl | H | I |
| 8-982 | pyridin-3-yl | H | Me | F | H | H | H | Br | H | heptafluoroisopropyl | H | I |
| 8-984 | 2-chloropyridin-3-yl | H | Me | F | H | H | H | Cl | H | heptafluoroisopropyl | H | Cl |
| 8-1026 | phenyl | H | Me | F | H | H | H | Cl | H | nonafluoro-2-butyl | H | Cl |
| 8-1046 | 3-cyanophenyl | H | Me | F | H | H | H | Cl | H | nonafluoro-2-butyl | H | Cl |
| 8-1047 | 4-cyanophenyl | H | Me | F | H | H | H | Cl | H | nonafluoro-2-butyl | H | Cl |
| 8-1062 | 2-chloropyridin-3-yl | H | Me | F | H | H | H | Cl | H | nonafluoro-2-butyl | H | Cl |
| 8-1104 | phenyl | H | Me | F | H | H | H | Br | H | heptafluoroisopropyl | H | Br |
| 8-1124 | 3-cyanophenyl | H | Me | F | H | H | H | Br | H | heptafluoroisopropyl | H | Br |
| 8-1125 | 4-cyanophenyl | H | Me | F | H | H | H | Br | H | heptafluoroisopropyl | H | Br |
| 8-1140 | 2-chloropyridin-3-yl | H | Me | F | H | H | H | Br | H | heptafluoroisopropyl | H | Br |
| 8-1182 | phenyl | H | Me | F | H | H | H | Br | H | nonafluoro-2-butyl | H | Br |
| 8-1202 | 3-cyanophenyl | H | Me | F | H | H | H | Br | H | nonafluoro-2-butyl | H | Br |
| 8-1203 | 4-cyanophenyl | H | Me | F | H | H | H | Br | H | nonafluoro-2-butyl | H | Br |
| 8-1218 | 2-chloropyridin-3-yl | H | Me | F | H | H | H | Br | H | nonafluoro-2-butyl | H | Br |
| 8-1260 | phenyl | H | Me | F | H | H | H | I | H | heptafluoroisopropyl | H | I |
| 8-1280 | 3-cyanophenyl | H | Me | F | H | H | H | I | H | heptafluoroisopropyl | H | I |
| 8-1281 | 4-cyanophenyl | H | Me | F | H | H | H | I | H | heptafluoroisopropyl | H | I |
| 8-1296 | 2-chloropyridin-3-yl | H | Me | F | H | H | H | I | H | heptafluoroisopropyl | H | I |
| 8-1338 | phenyl | H | Me | F | H | H | H | I | H | nonafluoro-2-butyl | H | I |
| 8-1358 | 3-cyanophenyl | H | Me | F | H | H | H | I | H | nonafluoro-2-butyl | H | I |
| 8-1359 | 4-cyanophenyl | H | Me | F | H | H | H | I | H | nonafluoro-2-butyl | H | I |
| 8-1374 | 2-chloropyridin-3-yl | H | Me | F | H | H | H | I | H | nonafluoro-2-butyl | H | I |
| 8-1416 | phenyl | H | Me | F | H | H | H | F | H | heptafluoroisopropyl | H | CF3 |
| 8-1417 | phenyl | H | Me | F | H | H | H | Cl | H | heptafluoroisopropyl | H | CF3 |
| 8-1431 | (3-trifluoromethyl)phenyl | H | Et | F | H | H | H | Cl | H | heptafluoroisopropyl | H | CF3 |
| 8-1437 | 3-cyanophenyl | H | Me | F | H | H | H | Cl | H | heptafluoroisopropyl | H | CF3 |
| 8-1438 | 4-cyanophenyl | H | Me | F | H | H | H | Cl | H | heptafluoroisopropyl | H | CF3 |
| 8-1442 | 2-chloro-4-fluorophenyl | H | n-Pr | F | H | H | H | Cl | H | heptafluoroisopropyl | H | CF3 |
| 8-1453 | 2-chloropyridin-3-yl | H | Me | F | H | H | H | Cl | H | heptafluoroisopropyl | H | CF3 |
| 8-1459 | 6-fluoropyridin-3-yl | H | i-Pr | F | H | H | H | Cl | H | heptafluoroisopropyl | H | CF3 |
| 8-1475 | 4-bromopyridin-3-yl | H | CH2CH=CH2 | F | H | H | H | Cl | H | heptafluoroisopropyl | H | n-C3F7 |
| 8-1482 | pyridin-4-yl | H | CN | F | H | H | H | Cl | H | heptafluoroisopropyl | H | CF3 |
| 8-1495 | phenyl | H | Me | F | H | H | H | F | H | nonafluoro-2-butyl | H | CF3 |
| 8-1496 | phenyl | H | Me | F | H | H | H | Cl | H | nonafluoro-2-butyl | H | CF3 |
| 8-1504 | 3-bromophenyl | H | CH2C ≡ CH | F | H | H | H | Cl | H | nonafluoro-2-butyl | H | CF3 |
| 8-1511 | (4-trifluoromethyl)phenyl | H | NH2 | F | H | H | H | Cl | H | nonafluoro-2-butyl | H | CF3 |
| 8-1516 | 3-cyanophenyl | H | Me | F | H | H | H | Cl | H | nonafluoro-2-butyl | H | CF3 |

**Table 8(3)**

| compound number | Q, | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 8-1517 | 4-cyanophenyl | H | Me | F | H | H | H | Cl | H | nonafluoro-2-butyl | H | CF3 |
| 8-1524 | 2-bromo-4-chlorophenyl | H | C(O)OMe | F | H | H | H | Cl | H | nonafluoro-2-butyl | H | CF3 |
| 8-1532 | 2-chloropyridin-3-yl | H | Me | F | H | H | H | Cl | H | nonafluoro-2-butyl | H | CF3 |
| 8-1545 | 5-fluoropyridin-3-yl | H | C(O)OEt | F | H | H | H | Cl | H | nonafluoro-2-butyl | H | C2F5 |
| 8-1554 | 4-bromopyridin-3-yl | H | C(O)C(O)Me | F | H | H | H | Cl | H | nonafluoro-2-butyl | H | CF3 |
| 8-1557 | 4-nitropyridin-3-yl | H | C(O)C(O)Et | F | H | H | H | Cl | H | nonafluoro-2-butyl | H | i-C3F7 |
| 8-1571 | 5-iodopyridin-2-yl | H | C(O)Me | F | H | H | H | Cl | H | nonafluoro-2-butyl | H | CF3 |
| 8-1573 | 4-cyanopyridin-2-yl | H | C(O)Et | F | H | H | H | Cl | H | nonafluoro-2-butyl | H | C2F5 |
| 8-1574 | phenyl | H | Me | F | H | H | H | Br | H | heptafluoroisopropyl | H | CF3 |
| 8-1588 | (3-trifluoromethyl) phenyl | H | S(O)2Me | F | H | H | H | Br | H | heptafluoroisopropyl | H | CF3 |
| 8-1594 | 3-cyanophenyl | H | Me | F | H | H | H | Br | H | heptafluoroisopropyl | H | CF3 |
| 8-1595 | 4-cyanophenyl | H | Me | F | H | H | H | Br | H | heptafluoroisopropyl | H | CF3 |
| 8-1606 | 4-cyano-2⁻fluorophenyl | H | S(O)2Et | F | H | H | H | Br | H | heptafluoroisopropyl | H | CF3 |
| 8-1610 | 2-chloropyridin-3-yl | H | Me | F | H | H | H | Br | H | heptafluoroisopropyl | H | CF3 |
| 8-1621 | 6-nitropyridin-3-yl | H | CH2Ph | F | H | H | H | Br | H | heptafluoroisopropyl | H | CF3 |
| 8-1630 | 4-fluoropyridin-3-yl | H | CH2(3-Py) | F | H | H | H | Br | H | heptafluoroisopropyl | H | CF3 |
| 8-1651 | 4-cyanopyridin-2-yl | H | CH2(3-Py-N-oxide) | F | H | H | H | Br | H | heptafluoroisopropyl | H | CF3 |
| 8-1652 | phenyl | H | Me | F | H | H | H | Br | H | nonafluoro-2-butyl | H | CF3 |
| 8-1672 | 3-cyanophenyl | H | Me | F | H | H | H | Br | H | nonafluoro-2-butyl | H | CF3 |
| 8-1673 | 4-cyanophenyl | H | Me | F | H | H | H | Br | H | nonafluoro-2-butyl | H | CF3 |
| 8-1679 | 4-bromo-2-chlorophenyl | H | Et | F | H | H | H | Br | H | nonafluoro-2-butyl | H | CF3 |
| 8-1688 | 2-chloropyridin-3-yl | H | Me | F | H | H | H | Br | H | nonafluoro-2-butyl | H | CF3 |
| 8-1705 | 5-(trifluoromethyl)pyridin-3-yl | H | CN | F | H | H | H | Br | H | nonafluoro-2-butyl | H | CF3 |
| 8-1730 | phenyl | H | Me | F | H | H | H | I | H | heptafluoroisopropyl | H | CF3 |
| 8-1750 | 3-cyanophenyl | H | Me | F | H | H | H | I | H | heptafluoroisopropyl | H | CF3 |
| 8-1751 | 4-cyanophenyl | H | Me | F | H | H | H | I | H | heptafluoroisopropyl | H | CF3 |
| 8-1766 | 2-chloropyridin-3-yl | H | Me | F | H | H | H | I | H | heptafluoroisopropyl | H | CF3 |
| 8-1779 | 5-fluoropyridin-3-yl | H | C(O)Me | F | H | H | H | I | H | heptafluoroisopropyl | H | CF3 |
| 8-1808 | phenyl | H | Me | F | H | H | H | I | H | nonafluoro-2-butyl | H | CF3 |
| 8-1828 | 3-cyanophenyl | H | Me | F | H | H | H | I | H | nonafluoro-2-butyl | H | CF3 |
| 8-1829 | 4-cyanophenyl | H | Me | F | H | H | H | I | H | nonafluoro-2-butyl | H | CF3 |
| 8-1844 | 2-chloropyridin-3-yl | H | Me | F | H | H | H | I | H | nonafluoro-2-butyl | H | CF3 |
| 8-1886 | phenyl | H | Me | F | H | H | H | OCF3 | H | heptafluoroisopropyl | H | OCF3 |
| 8-1901 | 3-cyanophenyl | H | Me | F | H | H | H | OCF3 | H | heptafluoroisopropyl | H | OCF3 |
| 8-1902 | 4-cyanophenyl | H | Me | F | H | H | H | OCF3 | H | heptafluoroisopropyl | H | OCF3 |
| 8-1917 | 2-chloropyridin-3-yl | H | Me | F | H | H | H | OCF3 | H | heptafluoroisopropyl | H | OCF3 |
| 8-1942 | phenyl | H | Me | F | H | H | H | OCF3 | H | nonafluoro-2-butyl | H | OCF3 |
| 8-1957 | 3-cyanophenyl | H | Me | F | H | H | H | OCF3 | H | nonafluoro-2-butyl | H | OCF3 |
| 8-1958 | 4-cyanophenyl | H | Me | F | H | H | H | OCF3 | H | nonafluoro-2-butyl | H | OCF3 |
| 8-1973 | 2-chloropyridin-3-yl | H | Me | F | H | H | H | OCF3 | H | nonafluoro-2-butyl | H | OCF3 |
| 8-1998 | phenyl | H | Me | F | H | H | H | CF3 | H | heptafluoroisopropyl | H | OCF3 |
| 8-2013 | 3-cyanophenyl | H | Me | F | H | H | H | CF3 | H | heptafluoroisopropyl | H | OCF3 |
| 8-2014 | 4-cyanophenyl | H | Me | F | H | H | H | CF3 | H | heptafluoroisopropyl | H | OCF3 |
| 8-2029 | 2-chloropyridin-3-yl | H | Me | F | H | H | H | CF3 | H | heptafluoroisopropyl | H | OCF3 |
| 8-2054 | phenyl | H | Me | F | H | H | H | CF3 | H | nonafluoro-2-butyl | H | OCF3 |
| 8-2069 | 3-cyanophenyl | H | Me | F | H | H | H | CF3 | H | nonafluoro-2-butyl | H | OCF3 |
| 8-2070 | 4-cyanophenyl | H | Me | F | H | H | H | CF3 | H | nonafluoro-2-butyl | H | OCF3 |
| 8-2085 | 2-chloropyridin-3-yl | H | Me | F | H | H | H | CF3 | H | nonafluoro-2-butyl | H | OCF3 |
| 8-2110 | phenyl | H | Me | F | H | H | H | CF3 | H | heptafluoroisopropyl | H | CF3 |
| 8-2130 | 3-cyanophenyl | H | Me | F | H | H | H | CF3 | H | heptafluoroisopropyl | H | CF3 |
| 8-2131 | 4-cyanophenyl | H | Me | F | H | H | H | CF3 | H | heptafluoroisopropyl | H | CF3 |
| 8-2146 | 2-chloropyridin-3-yl | H | Me | F | H | H | H | CF3 | H | heptafluoroisopropyl | H | CF3 |
| 8-2178 | phenyl | H | Me | F | H | H | H | CF3 | H | nonafluoro-2-butyl | H | CF3 |
| 8-2198 | 3-cyanophenyl | H | Me | F | H | H | H | CF3 | H | nonafluoro-2-butyl | H | CF3 |
| 8-2199 | 4-cyanophenyl | H | Me | F | H | H | H | CF3 | H | nonafluoro-2-butyl | H | CF3 |
| 8-2214 | 2-chloropyridin-3-yl | H | Me | F | H | H | H | CF3 | H | nonafluoro-2-butyl | H | CF3 |
| 8-2246 | phenyl | H | Me | H | F | H | H | F | H | heptafluoroisopropyl | H | F |
| 8-2247 | 3-cyanophenyl | H | Me | H | F | H | H | F | H | heptafluoroisopropyl | H | F |
| 8-2248 | 4-cyanophenyl | H | Me | H | F | H | H | F | H | heptafluoroisopropyl | H | F |
| 8-2249 | 2-chloropyridin-3-yl | H | Me | H | F | H | H | F | H | heptafluoroisopropyl | H | F |
| 8-2250 | phenyl | H | Me | H | F | H | H | F | H | nonafluoro-2-butyl | H | F |
| 8-2251 | 3-cyanophenyl | H | Me | H | F | H | H | F | H | nonafluoro-2-butyl | H | F |
| 8-2252 | 4-cyanophenyl | H | Me | H | F | H | H | F | H | nonafluoro-2-butyl | H | F |
| 8-2253 | 2-chloropyridin-3-yl | H | Me | H | F | H | H | F | H | nonafluoro-2-butyl | H | F |

**Table 8(4)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 8-2254 | phenyl | H | Me | H | F | H | H | Cl | H | heptafluoroisopropyl | H | Cl |
| 8-2274 | 3-cyanophenyl | H | Me | H | F | H | H | Cl | H | heptafluoroisopropyl | H | Cl |
| 8-2275 | 4-cyanophenyl | H | Me | H | F | H | H | Cl | H | heptafluoroisopropyl | H | Cl |
| 8-2290 | 2-chloropyridin-3-yl | H | Me | H | F | H | H | Cl | H | heptafluoroisopropyl | H | Cl |
| 8-2332 | phenyl | H | Me | H | F | H | H | Cl | H | nonafluoro-2-butyl | H | Cl |
| 8-2352 | 3-cyanophenyl | H | Me | H | F | H | H | Cl | H | nonafluoro-2-butyl | H | Cl |
| 8-2353 | 4-cyanophenyl | H | Me | H | F | H | H | Cl | H | nonafluoro-2-butyl | H | Cl |
| 8-2368 | 2-chloropyridin-3-yl | H | Me | H | F | H | H | Cl | H | nonafluoro-2-butyl | H | Cl |
| 8-2410 | phenyl | H | Me | H | F | H | H | Br | H | heptafluoroisopropyl | H | Br |
| 8-2430 | 3-cyanophenyl | H | Me | H | F | H | H | Br | H | heptafluoroisopropyl | H | Br |
| 8-2431 | 4-cyanophenyl | H | Me | H | F | H | H | Br | H | heptafluoroisopropyl | H | Br |
| 8-2446 | 2-chloropyridin-3-yl | H | Me | H | F | H | H | Br | H | heptafluoroisopropyl | H | Br |
| 8-2488 | phenyl | H | Me | H | F | H | H | Br | H | nonafluoro-2-butyl | H | Br |
| 8-2508 | 3-cyanophenyl | H | Me | H | F | H | H | Br | H | nonafluoro-2-butyl | H | Br |
| 8-2509 | 4-cyanophenyl | H | Me | H | F | H | H | Br | H | nonafluoro-2-butyl | H | Br |
| 8-2524 | 2-chloropyridin-3-yl | H | Me | H | F | H | H | Br | H | nonafluoro-2-butyl | H | Br |
| 8-2566 | phenyl | H | Me | H | F | H | H | I | H | heptafluoroisopropyl | H | I |
| 8-2586 | 3-cyanophenyl | H | Me | H | F | H | H | I | H | heptafluoroisopropyl | H | I |
| 8-2587 | 4-cyanophenyl | H | Me | H | F | H | H | I | H | heptafluoroisopropyl | H | I |
| 8-2602 | 2-chloropyridin-3-yl | H | Me | H | F | H | H | I | H | heptafluoroisopropyl | H | I |
| 8-2644 | phenyl | H | Me | H | F | H | H | I | H | nonafluoro-2-butyl | H | I |
| 8-2664 | 3-cyanophenyl | H | Me | H | F | H | H | I | H | nonafluoro-2-butyl | H | I |
| 8-2665 | 4-cyanophenyl | H | Me | H | F | H | H | I | H | nonafluoro-2-butyl | H | I |
| 8-2680 | 2-chloropyridin-3-yl | H | Me | H | F | H | H | I | H | nonafluoro-2-butyl | H | I |
| 8-2722 | phenyl | H | Me | H | F | H | H | Cl | H | heptafluoroisopropyl | H | OCF3 |
| 8-2742 | 3-cyanophenyl | H | Me | H | F | H | H | Cl | H | heptafluoroisopropyl | H | OCF3 |
| 8-2743 | 4-cyanophenyl | H | Me | H | F | H | H | Cl | H | heptafluoroisopropyl | H | OCF3 |
| 8-2758 | 2-chloropyridin-3-yl | H | Me | H | F | H | H | Cl | H | heptafluoroisopropyl | H | OCF3 |
| 8-2800 | phenyl | H | Me | H | F | H | H | Cl | H | nonafluoro-2-butyl | H | OCF3 |
| 8-2820 | 3-cyanophenyl | H | Me | H | F | H | H | Cl | H | nonafluoro-2-butyl | H | OCF3 |
| 8-2821 | 4-cyanophenyl | H | Me | H | F | H | H | Cl | H | nonafluoro-2-butyl | H | OCF3 |
| 8-2836 | 2-chloropyridin-3-yl | H | Me | H | F | H | H | Cl | H | nonafluoro-2-butyl | H | OCF3 |
| 8-2878 | phenyl | H | Me | H | F | H | H | Br | H | heptafluoroisopropyl | H | OCF3 |
| 8-2898 | 3-cyanophenyl | H | Me | H | F | H | H | Br | H | heptafluoroisopropyl | H | OCF3 |
| 8-2899 | 4-cyanophenyl | H | Me | H | F | H | H | Br | H | heptafluoroisopropyl | H | OCF3 |
| 8-2914 | 2-chloropyridin-3-yl | H | Me | H | F | H | H | Br | H | heptafluoroisopropyl | H | OCF3 |
| 8-2956 | phenyl | H | Me | H | F | H | H | Br | H | nonafluoro-2-butyl | H | OCF3 |
| 8-2976 | 3-cyanophenyl | H | Me | H | F | H | H | Br | H | nonafluoro-2-butyl | H | OCF3 |
| 8-2977 | 4-cyanophenyl | H | Me | H | F | H | H | Br | H | nonafluoro-2-butyl | H | OCF3 |
| 8-2992 | 2-chloropyridin-3-yl | H | Me | H | F | H | H | Br | H | nonafluoro-2-butyl | H | OCF3 |
| 8-3034 | phenyl | H | Me | H | F | H | H | I | H | heptafluoroisopropyl | H | OCF3 |
| 8-3054 | 3-cyanophenyl | H | Me | H | F | H | H | I | H | heptafluoroisopropyl | H | OCF3 |
| 8-3055 | 4-cyanophenyl | H | Me | H | F | H | H | I | H | heptafluoroisopropyl | H | OCF3 |
| 8-3070 | 2-chloropyridin-3-yl | H | Me | H | F | H | H | I | H | heptafluoroisopropyl | H | OCF3 |
| 8-3112 | phenyl | H | Me | H | F | H | H | I | H | nonafluoro-2-butyl | H | OCF3 |
| 8-3132 | 3-cyanophenyl | H | Me | H | F | H | H | I | H | nonafluoro-2-butyl | H | OCF3 |
| 8-3133 | 4-cyanophenyl | H | Me | H | F | H | H | I | H | nonafluoro-2-butyl | H | OCF3 |
| 8-3148 | 2-chloropyridin-3-yl | H | Me | H | F | H | H | I | H | nonafluoro-2-butyl | H | OCF3 |
| 8-3190 | phenyl | H | Me | H | F | H | H | F | H | heptafluoroisopropyl | H | CF3 |
| 8-3191 | phenyl | H | Me | H | F | H | H | Cl | H | heptafluoroisopropyl | H | CF3 |
| 8-3211 | 3-cyanophenyl | H | Me | H | F | H | H | Cl | H | heptafluoroisopropyl | H | CF3 |
| 8-3212 | 4-cyanophenyl | H | Me | H | F | H | H | Cl | H | heptafluoroisopropyl | H | CF3 |
| 8-3227 | 2-chloropyridin-3-yl | H | Me | H | F | H | H | Cl | H | heptafluoroisopropyl | H | CF3 |
| 8-3269 | phenyl | H | Me | H | F | H | H | F | H | nonafluoro-2-butyl | H | CF3 |
| 8-3270 | phenyl | H | Me | H | F | H | H | Cl | H | nonafluoro-2-butyl | H | CF3 |
| 8-3290 | 3-cyanophenyl | H | Me | H | F | H | H | Cl | H | nonafluoro-2-butyl | H | CF3 |
| 8-3291 | 4-cyanophenyl | H | Me | H | F | H | H | Cl | H | nonafluoro-2-butyl | H | CF3 |
| 8-3306 | 2-chloropyridin-3-yl | H | Me | H | F | H | H | Cl | H | nonafluoro-2-butyl | H | CF3 |
| 8-3348 | phenyl | H | Me | H | F | H | H | Br | H | heptafluoroisopropyl | H | CF3 |
| 8-3368 | 3-cyanophenyl | H | Me | H | F | H | H | Br | H | heptafluoroisopropyl | H | CF3 |
| 8-3369 | 4-cyanophenyl | H | Me | H | F | H | H | Br | H | heptafluoroisopropyl | H | CF3 |
| 8-3384 | 2-chloropyridin-3-yl | H | Me | H | F | H | H | Br | H | heptafluoroisopropyl | H | CF3 |
| 8-3426 | phenyl | H | Me | H | F | H | H | Br | H | nonafluoro-2-butyl | H | CF3 |
| 8-3446 | 3-cyanophenyl | H | Me | H | F | H | H | Br | H | nonafluoro-2-butyl | H | CF3 |
| 8-3447 | 4-cyanophenyl | H | Me | H | F | H | H | Br | H | nonafluoro-2-butyl | H | CF3 |

**Table 8(5)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 8-3462 | 2-chloropyridin-3-yl | H | Me | H | F | H | H | Br | H | nonafluoro-2-butyl | H | CF3 |
| 8-3504 | phenyl | H | Me | H | F | H | H | I | H | heptafluoroisopropyl | H | CF3 |
| 8-3524 | 3-cyanophenyl | H | Me | H | F | H | H | I | H | heptafluoroisopropyl | H | CF3 |
| 8-3525 | 4-cyanophenyl | H | Me | H | F | H | H | I | H | heptafluoroisopropyl | H | CF3 |
| 8-3540 | 2-chloropiridin-3-yl | H | Me | H | F | H | H | I | H | heptafluoroisopropyl | H | CF3 |
| 8-3582 | phenyl | H | Me | H | F | H | H | I | H | nonafluoro-2-butyl | H | CF3 |
| 8-3602 | 3-cyanophenyl | H | Me | H | F | H | H | I | H | nonafluoro-2-butyl | H | CF3 |
| 8-3603 | 4-cyanophenyl | H | Me | H | F | H | H | I | H | nonafluoro-2-butyl | H | CF3 |
| 8-3618 | 2-chloropyridin-3-yl | H | Me | H | F | H | H | I | H | nonafluoro-2-butyl | H | CF3 |
| 8-3660 | phenyl | H | Me | H | F | H | H | OCF3 | H | heptafluoroisopropyl | H | OCF3 |
| 8-3691 | 2-chloropyridin-3-yl | H | Me | H | F | H | H | OCF3 | H | heptafluoroisopropyl | H | OCF3 |
| 8-3716 | phenyl | H | Me | H | F | H | H | OCF3 | H | nonafluoro-2-butyl | H | OCF3 |
| 8-3747 | 2-chloropyridin-3-yl | H | Me | H | F | H | H | OCF3 | H | nonafluoro-2-butyl | H | OCF3 |
| 8-3772 | phenyl | H | Me | H | F | H | H | CF3 | H | heptafluoroisopropyl | H | OCF3 |
| 8-3803 | 2-chloropyridin-3-yl | H | Me | H | F | H | H | CF3 | H | heptafluoroisopropyl | H | OCF3 |
| 8-3828 | phenyl | H | Me | H | F | H | H | CF3 | H | nonafluoro-2-butyl | H | OCF3 |
| 8-3859 | 2-chloropyridin-3-yl | H | Me | H | F | H | H | CF3 | H | nonafluoro-2-butyl | H | OCF3 |
| 8-3884 | phenyl | H | Me | H | F | H | H | CF3 | H | heptafluoroisopropyl | H | CF3 |
| 8-3904 | 3-cyanophenyl | H | Me | H | F | H | H | CF3 | H | heptafluoroisopropyl | H | CF3 |
| 8-3905 | 4-cyanophenyl | H | Me | H | F | H | H | CF3 | H | heptafluoroisopropyl | H | CF3 |
| 8-3920 | 2-chloropyridin-3-yl | H | Me | H | F | H | H | CF3 | H | heptafluoroisopropyl | H | CF3 |
| 8-3952 | phenyl | H | Me | H | F | H | H | CF3 | H | nonafluoro-2-butyl | H | CF3 |
| 8-3972 | 3-cyanophenyl | H | Me | H | F | H | H | CF3 | H | nonafluoro-2-butyl | H | CF3 |
| 8-3973 | 4-cyanophenyl | H | Me | H | F | H | H | CF3 | H | nonafluoro-2-butyl | H | CF3 |
| 8-3988 | 2-chloropyridin-3-yl | H | Me | H | F | H | H | CF3 | H | nonafluoro-2-butyl | H | CF3 |
| 8-4020 | phenyl | H | Me | F | F | H | H | F | H | heptafluoroisopropyl | H | F |
| 8-4021 | 3-cyanophenyl | H | Me | F | F | H | H | F | H | heptafluoroisopropyl | H | F |
| 8-4022 | 4-cyanophenyl | H | Me | F | F | H | H | F | H | heptafluoroisopropyl | H | F |
| 8-4023 | 2-chloropyridin-3-yl | H | Me | F | F | H | H | F | H | heptafluoroisopropyl | H | F |
| 8-4024 | phenyl | H | Me | F | F | H | H | F | H | nonafluoro-2-butyl | H | F |
| 8-4025 | 3-cyanophenyl | H | Me | F | F | H | H | F | H | nonafluoro-2-butyl | H | F |
| 8-4026 | 4-cyanophenyl | H | Me | F | F | H | H | F | H | nonafluoro-2-butyl | H | F |
| 8-4027 | 2-chloropyridin-3-yl | H | Me | F | F | H | H | F | H | nonafluoro-2-butyl | H | F |
| 8-4028 | phenyl | H | Me | F | F | H | H | Cl | H | heptafluoroisopropyl | H | Cl |
| 8-4048 | 3-cyanophenyl | H | Me | F | F | H | H | Cl | H | heptafluoroisopropyl | H | Cl |
| 8-4049 | 4-cyanophenyl | H | Me | F | F | H | H | Cl | H | heptafluoroisopropyl | H | Cl |
| 8-4064 | 2-chloropyridin-3-yl | H | Me | F | F | H | H | Cl | H | heptafluoroisopropyl | H | Cl |
| 8-4106 | phenyl | H | Me | F | F | H | H | Cl | H | nonafluoro-2-butyl | H | Cl |
| 8-4126 | 3-cyanophenyl | H | Me | F | F | H | H | Cl | H | nonafluoro-2-butyl | H | Cl |
| 8-4127 | 4-cyanophenyl | H | Me | F | F | H | H | Cl | H | nonafluoro-2-butyl | H | Cl |
| 8-4142 | 2-chloropyridin-3-yl | H | Me | F | F | H | H | Cl | H | nonafluoro-2-butyl | H | Cl |
| 8-4184 | phenyl | H | Me | F | F | H | H | Br | H | heptafluoroisopropyl | H | Br |
| 8-4204 | 3-cyanophenyl | H | Me | F | F | H | H | Br | H | heptafluoroisopropyl | H | Br |
| 8-4205 | 4-cyanophenyl | H | Me | F | F | H | H | Br | H | heptafluoroisopropyl | H | Br |
| 8-4220 | 2-chloropyridin-3-yl | H | Me | F | F | H | H | Br | H | heptafluoroisopropyl | H | Br |
| 8-4262 | phenyl | H | Me | F | F | H | H | Br | H | nonafluoro-2-butyl | H | Br |
| 8-4282 | 3-cyanophenyl | H | Me | F | F | H | H | Br | H | nonafluoro-2-butyl | H | Br |
| 8-4283 | 4-cyanophenyl | H | Me | F | F | H | H | Br | H | nonafluoro-2-butyl | H | Br |
| 8-4298 | 2-chloropyridin-3-yl | H | Me | F | F | H | H | Br | H | nonafluoro-2-butyl | H | Br |
| 8-4340 | phenyl | H | Me | F | F | H | H | I | H | heptafluoroisopropyl | H | I |
| 8-4360 | 3-cyanophenyl | H | Me | F | F | H | H | I | H | heptafluoroisopropyl | H | I |
| 8-4361 | 4-cyanophenyl | H | Me | F | F | H | H | I | H | heptafluoroisopropyl | H | I |
| 8-4376 | 2-chloropyridin-3-yl | H | Me | F | F | H | H | I | H | heptafluoroisopropyl | H | I |
| 8-4418 | phenyl | H | Me | F | F | H | H | I | H | nonafluoro-2-butyl | H | I |
| 8-4438 | 3-cyanophenyl | H | Me | F | F | H | H | I | H | nonafluoro-2-butyl | H | I |
| 8-4439 | 4-cyanophenyl | H | Me | F | F | H | H | I | H | nonafluoro-2-butyl | H | I |
| 8-4454 | 2-chloropyridin-3-yl | H | Me | F | F | H | H | I | H | nonafluoro-2-butyl | H | I |
| 8-4496 | phenyl | H | Me | F | F | H | H | Cl | H | heptafluoroisopropyl | H | OCF3 |
| 8-4516 | 3-cyanophenyl | H | Me | F | F | H | H | Cl | H | heptafluoroisopropyl | H | OCF3 |
| 8-4517 | 4-cyanophenyl | H | Me | F | F | H | H | Cl | H | heptafluoroisopropyl | H | OCF3 |
| 8-4532 | 2-chloropyridin-3-yl | H | Me | F | F | H | H | Cl | H | heptafluoroisopropyl | H | OCF3 |
| 8-4574 | phenyl | H | Me | F | F | H | H | Cl | H | nonafluoro-2-butyl | H | OCF3 |
| 8-4594 | 3-cyanophenyl | H | Me | F | F | H | H | Cl | H | nonafluoro-2-butyl | H | OCF3 |
| 8-4595 | 4-cyanophenyl | H | Me | F | F | H | H | Cl | H | nonafluoro-2-butyl | H | OCF3 |

**Table 8(6)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 8-4610 | 2-chloropyridin-3-yl | H | Me | F | F | H | H | Cl | H | nonafluoro-2-butyl | H | OCF3 |
| 8-4652 | phenyl | H | Me | F | F | H | H | Br | H | heptafluoroisopropyl | H | OCF3 |
| 8-4672 | 3-cyanophenyl | H | Me | F | F | H | H | Br | H | heptafluoroisopropyl | H | OCF3 |
| 8-4673 | 4-cyanophenyl | H | Me | F | F | H | H | Br | H | heptafluoroisopropyl | H | OCF3 |
| 8-4688 | 2-chloropyridin-3-yl | H | Me | F | F | H | H | Br | H | heptafluoroisopropyl | H | OCF3 |
| 8-4730 | phenyl | H | Me | F | F | H | H | Br | H | nonafluoro-2-butyl | H | OCF3 |
| 8-4750 | 3-cyanophenyl | H | Me | F | F | H | H | Br | H | nonafluoro-2-butyl | H | OCF3 |
| 8-4751 | 4-cyanophenyl | H | Me | F | F | H | H | Br | H | nonafluoro-2-butyl | H | OCF3 |
| 8-4808 | phenyl | H | Me | F | F | H | H | I | H | heptafluoroisopropyl | H | OCF3 |
| 8-4828 | 3-cyanophenyl | H | Me | F | F | H | H | I | H | heptafluoroisopropyl | H | OCF3 |
| 8-4829 | 4-cyanophenyl | H | Me | F | F | H | H | I | H | heptafluoroisopropyl | H | OCF3 |
| 8-4844 | 2-chloropyridin-3-yl | H | Me | F | F | H | H | I | H | heptafluoroisopropyl | H | OCF3 |
| 8-4886 | phenyl | H | Me | F | F | H | H | I | H | nonafluoro-2-butyl | H | OCF3 |
| 8-4906 | 3-cyanophenyl | H | Me | F | F | H | H | I | H | nonafluoro-2-butyl | H | OCF3 |
| 8-4907 | 4-cyanophenyl | H | Me | F | F | H | H | I | H | nonafluoro-2-butyl | H | OCF3 |
| 8-4922 | 2-chloropyridin-3-yl | H | Me | F | F | H | H | I | H | nonafluoro-2-butyl | H | OCF3 |
| 8-4964 | phenyl | H | Me | F | F | H | H | F | H | heptafluoroisopropyl | H | CF3 |
| 8-4965 | phenyl | H | Me | F | F | H | H | Cl | H | heptafluoroisopropyl | H | CF3 |
| 8-4985 | 3-cyanophenyl | H | Me | F | F | H | H | Cl | H | heptafluoroisopropyl | H | CF3 |
| 8-4986 | 4-cyanophenyl | H | Me | F | F | H | H | Cl | H | heptafluoroisopropyl | H | CF3 |
| 8-5001 | 2-chloropyridin-3-yl | H | Me | F | F | H | H | Cl | H | heptafluoroisopropyl | H | CF3 |
| 8-5043 | phenyl | H | Me | F | F | H | H | F | H | nonafluoro-2-butyl | H | CF3 |
| 8-5044 | phenyl | H | Me | F | F | H | H | Cl | H | nonafluoro-2-butyl | H | CF3 |
| 8-5064 | 3-cyanophenyl | H | Me | F | F | H | H | Cl | H | nonafluoro-2-butyl | H | CF3 |
| 8-5065 | 4-cyanophenyl | H | Me | F | F | H | H | Cl | H | nonafluoro-2-butyl | H | CF3 |
| 8-5080 | 2-chloropyridin-3-yl | H | Me | F | F | H | H | Cl | H | nonafluoro-2-butyl | H | CF3 |
| 8-5122 | phenyl | H | Me | F | F | H | H | Br | H | heptafluoroisopropyl | H | CF3 |
| 8-5142 | 3-cyanophenyl | H | Me | F | F | H | H | Br | H | heptafluoroisopropyl | H | CF3 |
| 8-5143 | 4-cyanophenyl | H | Me | F | F | H | H | Br | H | heptafluoroisopropyl | H | CF3 |
| 8-5158 | 2-chloropyridin-3-yl | H | Me | F | F | H | H | Br | H | heptafluoroisopropyl | H | CF3 |
| 8-5200 | phenyl | H | Me | F | F | H | H | Br | H | nonafluoro-2-butyl | H | CF3 |
| 8-5220 | 3-cyanophenyl | H | Me | F | F | H | H | Br | H | nonafluoro-2-butyl | H | CF3 |
| 8-5221 | 4-cyanophenyl | H | Me | F | F | H | H | Br | H | nonafluoro-2-butyl | H | CF3 |
| 8-5236 | 2-chloropyridin-3-yl | H | Me | F | F | H | H | Br | H | nonafluoro-2-butyl | H | CF3 |
| 8-5278 | phenyl | H | Me | F | F | H | H | I | H | heptafluoroisopropyl | H | CF3 |
| 8-5298 | 3-cyanophenyl | H | Me | F | F | H | H | I | H | heptafluoroisopropyl | H | CF3 |
| 8-5299 | 4-cyanophenyl | H | Me | F | F | H | H | I | H | heptafluoroisopropyl | H | CF3 |
| 8-5314 | 2-chloropyridin-3-yl | H | Me | F | F | H | H | I | H | heptafluoroisopropyl | H | CF3 |
| 8-5356 | phenyl | H | Me | F | F | H | H | I | H | nonafluoro-2-butyl | H | CF3 |
| 8-5376 | 3-cyanophenyl | H | Me | F | F | H | H | I | H | nonafluoro-2-butyl | H | CF3 |
| 8-5377 | 4-cyanophenyl | H | Me | F | F | H | H | I | H | nonafluoro-2-butyl | H | CF3 |
| 8-5392 | 2-chloropyridin-3-yl | H | Me | F | F | H | H | I | H | nonafluoro-2-butyl | H | CF3 |
| 8-5434 | phenyl | H | Me | F | F | H | H | OCF3 | H | heptafluoroisopropyl | H | OCF3 |
| 8-5465 | 2-chloropyridin-3-yl | H | Me | F | F | H | H | OCF3 | H | heptafluoroisopropyl | H | OCF3 |
| 8-5490 | phenyl | H | Me | F | F | H | H | OCF3 | H | nonafluoro-2-butyl | H | OCF3 |
| 8-5521 | 2-chloropyridin-3-yl | H | Me | F | F | H | H | OCF3 | H | nonafluoro-2-butyl | H | OCF3 |
| 8-5546 | phenyl | H | Me | F | F | H | H | CF3 | H | heptafluoroisopropyl | H | OCF3 |
| 8-5577 | 2-chloropyridin-3-yl | H | Me | F | F | H | H | CF3 | H | heptafluoroisopropyl | H | OCF3 |
| 8-5602 | phenyl | H | Me | F | F | H | H | CF3 | H | nonafluoro-2-butyl | H | OCF3 |
| 8-5633 | 2-chloropyridin-3-yl | H | Me | F | F | H | H | CF3 | H | nonafluoro-2-butyl | H | OCF3 |
| 8-5678 | 3-cyanophenyl | H | Me | F | F | H | H | CF3 | H | heptafluoroisopropyl | H | CF3 |
| 8-5679 | 4-cyanophenyl | H | Me | F | F | H | H | CF3 | H | heptafluoroisopropyl | H | CF3 |
| 8-5694 | 2-chloropyridin-3-yl | H | Me | F | F | H | H | CF3 | H | heptafluoroisopropyl | H | CF3 |
| 8-5726 | phenyl | H | Me | F | F | H | H | CF3 | H | nonafluoro-2-butyl | H | CF3 |
| 8-5746 | 3-cyanophenyl | H | Me | F | F | H | H | CF3 | H | nonafluoro-2-butyl | H | CF3 |
| 8-5747 | 4-cyanophenyl | H | Me | F | F | H | H | CF3 | H | nonafluoro-2-butyl | H | CF3 |
| 8-5762 | 2-chloropyridin-3-yl | H | Me | F | F | H | H | CF3 | H | nonafluoro-2-butyl | H | CF3 |
| 8-5794 | phenyl | H | Me | F | F | F | H | Cl | H | pentafluoroethyl | H | Cl |
| 8-5795 | phenyl | H | Me | F | F | F | H | Br | H | heptafluoroisopropyl | H | Br |
| 8-5796 | phenyl | H | Me | F | F | F | H | Br | H | heptafluoroisopropyl | H | OCF3 |
| 8-5797 | phenyl | H | Me | F | F | F | H | I | H | heptafluoroisopropyl | H | CF3 |
| 8-5798 | phenyl | H | Me | F | F | F | H | CF3 | H | heptafluoroisopropyl | H | CF3 |
| 8-5799 | phenyl | H | Me | F | F | F | H | I | H | nonafluoro-2-butyl | H | I |
| 8-5800 | phenyl | H | Me | F | F | F | H | Cl | H | nonafluoro-2-butyl | H | OCF3 |

**Table 8(7)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 8-5801 | phenyl | H | Me | F | F | F | H | Br | H | nonafluoro-2-butyl | H | CF3 |
| 8-5802 | phenyl | H | Me | F | F | F | H | CF3 | H | nonafluoro-2-butyl | H | CF3 |
| 8-5803 | phenyl | H | Me | H | H | F | H | Cl | H | pentafluoroethyl | H | Cl |
| 8-5804 | phenyl | H | Me | H | H | F | H | Br | H | heptafluoroisopropyl | H | Br |
| 8-5805 | phenyl | H | Me | H | H | F | H | Br | H | heptafluoroisopropyl | H | OCF3 |
| 8-5806 | phenyl | H | Me | H | H | F | H | I | H | heptafluoroisopropyl | H | CF3 |
| 8-5807 | phenyl | H | Me | H | H | F | H | CF3 | H | heptafluoroisopropyl | H | CF3 |
| 8-5808 | phenyl | H | Me | H | H | F | H | I | H | nonafluoro-2-butyl | H | I |
| 8-5809 | phenyl | H | Me | H | H | F | H | Cl | H | nonafluoro-2-butyl | H | OCF3 |
| 8-5810 | phenyl | H | Me | H | H | F | H | Br | H | nonafluoro-2-butyl | H | CF3 |
| 8-5811 | phenyl | H | Me | H | H | F | H | CF3 | H | nonafluoro-2-butyl | H | CF3 |
| 8-5812 | phenyl | H | Me | H | H | H | F | Cl | H | pentafluoroethyl | H | Cl |
| 8-5813 | phenyl | H | Me | H | H | H | F | Br | H | heptafluoroisopropyl | H | Br |
| 8-5814 | phenyl | H | Me | H | H | H | F | Br | H | heptafluoroisopropyl | H | OCF3 |
| 8-5815 | phenyl | H | Me | H | H | H | F | I | H | heptafluoroisopropyl | H | CF3 |
| 8-5816 | phenyl | H | Me | H | H | H | F | CF3 | H | heptafluoroisopropyl | H | CF3 |
| 8-5817 | phenyl | H | Me | H | H | H | F | I | H | nonafluoro-2-butyl | H | I |
| 8-5818 | phenyl | H | Me | H | H | H | F | Cl | H | nonafluoro-2-butyl | H | OCF3 |
| 8-5819 | phenyl | H | Me | H | H | H | F | Br | H | nonafluoro-2-butyl | H | CF3 |
| 8-5820 | phenyl | H | Me | H | H | H | F | CF3 | H | nonafluoro-2-butyl | H | CF3 |
| 8-5821 | phenyl | H | Me | F | H | F | H | Cl | H | pentafluoroethyl | H | Cl |
| 8-5822 | phenyl | H | Me | F | H | F | H | Br | H | heptafluoroisopropyl | H | Br |
| 8-5823 | phenyl | H | Me | F | H | F | H | Br | H | heptafluoroisopropyl | H | OCF3 |
| 8-5824 | phenyl | H | Me | F | H | F | H | I | H | heptafluoroisopropyl | H | CF3 |
| 8-5825 | phenyl | H | Me | F | H | F | H | CF3 | H | heptafluoroisopropyl | H | CF3 |
| 8-5826 | phenyl | H | Me | F | H | F | H | I | H | nonafluoro-2-butyl | H | I |
| 8-5827 | phenyl | H | Me | F | H | F | H | Cl | H | nonafluoro-2-butyl | H | OCF3 |
| 8-5828 | phenyl | H | Me | F | H | F | H | Br | H | nonafluoro-2-butyl | H | CF3 |
| 8-5829 | phenyl | H | Me | F | H | F | H | CF3 | H | nonafluoro-2-butyl | H | CF3 |
| 8-5830 | phenyl | H | Me | F | H | H | F | Cl | H | pentafluoroethyl | H | Cl |
| 8-5831 | phenyl | H | Me | F | H | H | F | Br | H | heptafluoroisopropyl | H | Br |
| 8-5832 | phenyl | H | Me | F | H | H | F | Br | H | heptafluoroisopropyl | H | OCF3 |
| 8-5833 | phenyl | H | Me | F | H | H | F | I | H | heptafluoroisopropyl | H | CF3 |
| 8-5834 | phenyl | H | Me | F | H | H | F | CF3 | H | heptafluoroisopropyl | H | CF3 |
| 8-5835 | phenyl | H | Me | F | H | H | F | I | H | nonafluoro-2-butyl | H | I |
| 8-5836 | phenyl | H | Me | F | H | H | F | Cl | H | nonafluoro-2-butyl | H | OCF3 |
| 8-5837 | phenyl | H | Me | F | H | H | F | Br | H | nonafluoro-2-butyl | H | CF3 |
| 8-5838 | phenyl | H | Me | F | H | H | F | CF3 | H | nonafluoro-2-butyl | H | CF3 |
| 8-5839 | phenyl | H | Me | H | F | F | H | Cl | H | pentafluoroethyl | H | Cl |
| 8-5840 | phenyl | H | Me | H | F | F | H | Br | H | heptafluoroisopropyl | H | Br |
| 8-5841 | phenyl | H | Me | H | F | F | H | Br | H | heptafluoroisopropyl | H | OCF3 |
| 8-5842 | phenyl | H | Me | H | F | F | H | I | H | heptafluoroisopropyl | H | CF3 |
| 8-5843 | phenyl | H | Me | H | F | F | H | CF3 | H | heptafluoroisopropyl | H | CF3 |
| 8-5844 | phenyl | H | Me | H | F | F | H | I | H | nonafluoro-2-butyl | H | I |
| 8-5845 | phenyl | H | Me | H | F | F | H | Cl | H | nonafluoro-2-butyl | H | OCF3 |
| 8-5846 | phenyl | H | Me | H | F | F | H | Br | H | nonafluoro-2-butyl | H | CF3 |
| 8-5847 | phenyl | H | Me | H | F | F | H | CF3 | H | nonafluoro-2-butyl | H | CF3 |
| 8-5848 | phenyl | H | Me | H | F | H | F | Cl | H | pentafluoroethyl | H | Cl |
| 8-5849 | phenyl | H | Me | H | F | H | F | Br | H | heptafluoroisopropyl | H | Br |
| 8-5850 | phenyl | H | Me | H | F | H | F | Br | H | heptafluoroisopropyl | H | OCF3 |
| 8-5851 | phenyl | H | Me | H | F | H | F | I | H | heptafluoroisopropyl | H | CF3 |
| 8-5852 | phenyl | H | Me | H | F | H | F | CF3 | H | heptafluoroisopropyl | H | CF3 |
| 8-5853 | phenyl | H | Me | H | F | H | F | I | H | nonafluoro-2-butyl | H | I |
| 8-5854 | phenyl | H | Me | H | F | H | F | Cl | H | nonafluoro-2-butyl | H | OCF3 |
| 8-5855 | phenyl | H | Me | H | F | H | F | Br | H | nonafluoro-2-butyl | H | CF3 |
| 8-5856 | phenyl | H | Me | H | F | H | F | CF3 | H | nonafluoro-2-butyl | H | CF3 |
| 8-5857 | phenyl | H | Me | H | H | F | F | Cl | H | pentafluoroethyl | H | Cl |
| 8-5858 | phenyl | H | Me | H | H | F | F | Br | H | heptafluoroisopropyl | H | Br |
| 8-5859 | phenyl | H | Me | H | H | F | F | Br | H | heptafluoroisopropyl | H | OCF3 |
| 8-5860 | phenyl | H | Me | H | H | F | F | I | H | heptafluoroisopropyl | H | CF3 |
| 8-5861 | phenyl | H | Me | H | H | F | F | CF3 | H | heptafluoroisopropyl | H | CF3 |
| 8-5862 | phenyl | H | Me | H | H | F | F | I | H | nonafluoro-2-butyl | H | I |
| 8-5863 | phenyl | H | Me | H | H | F | F | Cl | H | nonafluoro-2-butyl | H | OCF3 |
| 8-5864 | phenyl | H | Me | H | H | F | F | Br | H | nonafluoro-2-butyl | H | CF3 |

**Table 8(8)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 8-5865 | phenyl | H | Me | H | H | F | F | CF3 | H | nonafluoro-2-butyl | H | CF3 |
| 8-5866 | phenyl | H | Me | F | F | H | F | Cl | H | pentafluoroethyl | H | Cl |
| 8-5867 | phenyl | H | Me | F | F | H | F | Br | H | heptafluoroisopropyl | H | Br |
| 8-5868 | phenyl | H | Me | F | F | H | F | Br | H | heptafluoroisopropyl | H | OCF3 |
| 8-5869 | phenyl | H | Me | F | F | H | F | I | H | heptafluoroisopropyl | H | CF3 |
| 8-5870 | phenyl | H | Me | F | F | H | F | CF3 | H | heptafluoroisopropyl | H | CF3 |
| 8-5871 | phenyl | H | Me | F | F | H | F | I | H | nonafluoro-2-butyl | H | I |
| 8-5872 | phenyl | H | Me | F | F | H | F | Cl | H | nonafluoro-2-butyl | H | OCF3 |
| 8-5873 | phenyl | H | Me | F | F | H | F | Br | H | nonafluoro-2-butyl | H | CF3 |
| 8-5874 | phenyl | H | Me | F | F | H | F | CF3 | H | nonafluoro-2-butyl | H | CF3 |
| 8-5875 | phenyl | H | Me | F | H | F | F | Cl | H | pentafluoroethyl | H | Cl |
| 8-5876 | phenyl | H | Me | F | H | F | F | Br | H | heptafluoroisopropyl | H | Br |
| 8-5877 | phenyl | H | Me | F | H | F | F | Br | H | heptafluoroisopropyl | H | OCF3 |
| 8-5878 | phenyl | H | Me | F | H | F | F | I | H | heptafluoroisopropyl | H | CF3 |
| 8-5879 | phenyl | H | Me | F | H | F | F | CF3 | H | heptafluoroisopropyl | H | CF3 |
| 8-5880 | phenyl | H | Me | F | H | F | F | I | H | nonafluoro-2-butyl | H | I |
| 8-5881 | phenyl | H | Me | F | H | F | F | Cl | H | nonafluoro-2-butyl | H | OCF3 |
| 8-5882 | phenyl | H | Me | F | H | F | F | Br | H | nonafluoro-2-butyl | H | CF3 |
| 8-5883 | phenyl | H | Me | F | H | F | F | CF3 | H | nonafluoro-2-butyl | H | CF3 |
| 8-5884 | phenyl | H | Me | H | F | F | F | Cl | H | pentafluoroethyl | H | Cl |
| 8-5885 | phenyl | H | Me | H | F | F | F | Br | H | heptafluoroisopropyl | H | Br |
| 8-5886 | phenyl | H | Me | H | F | F | F | Br | H | heptafluoroisopropyl | H | OCF3 |
| 8-5887 | phenyl | H | Me | H | F | F | F | I | H | heptafluoroisopropyl | H | CF3 |
| 8-5888 | phenyl | H | Me | H | F | F | F | CF3 | H | heptafluoroisopropyl | H | CF3 |
| 8-5889 | phenyl | H | Me | H | F | F | F | I | H | nonafluoro-2-butyl | H | I |
| 8-5890 | phenyl | H | Me | H | F | F | F | Cl | H | nonafluoro-2-butyl | H | OCF3 |
| 8-5891 | phenyl | H | Me | H | F | F | F | Br | H | nonafluoro-2-butyl | H | CF3 |
| 8-5892 | phenyl | H | Me | H | F | F | F | CF3 | H | nonafluoro-2-butyl | H | CF3 |
| 8-5893 | phenyl | H | Me | F | F | F | F | Cl | H | pentafluoroethyl | H | Cl |
| 8-5894 | phenyl | H | Me | F | F | F | F | Br | H | heptafluoroisopropyl | H | Br |
| 8-5895 | phenyl | H | Me | F | F | F | F | Br | H | heptafluoroisopropyl | H | OCF3 |
| 8-5896 | phenyl | H | Me | F | F | F | F | I | H | heptafluoroisopropyl | H | CF3 |
| 8-5897 | phenyl | H | Me | F | F | F | F | CF3 | H | heptafluoroisopropyl | H | CF3 |
| 8-5898 | phenyl | H | Me | F | F | F | F | I | H | nonafluoro-2-butyl | H | I |
| 8-5899 | phenyl | H | Me | F | F | F | F | Cl | H | nonafluoro-2-butyl | H | OCF3 |
| 8-5900 | phenyl | H | Me | F | F | F | F | Br | H | nonafluoro-2-butyl | H | CF3 |
| 8-5901 | phenyl | H | Me | F | F | F | F | CF3 | H | nonafluoro-2-butyl | H | CF3 |

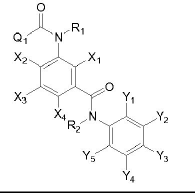

**Table 9(1)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 9-1 | phenyl | Me | Me | H | H | H | H | F | H | nonafluoro-2-butyl | H | F |
| 9-2 | 3-cyanophenyl | Me | Me | H | H | H | H | F | H | nonafluoro-2-butyl | H | F |
| 9-3 | 4-cyanophenyl | Me | Me | H | H | H | H | F | H | nonafluoro-2-butyl | H | F |
| 9-4 | 2-chloropyridin-3-yl | Me | Me | H | H | H | H | F | H | nonafluoro-2-butyl | H | F |
| 9-5 | phenyl | Me | Me | H | H | H | H | Cl | H | nonafluoro-2-butyl | H | Cl |
| 9-12 | 3-cyanophenyl | Me | Me | H | H | H | H | Cl | H | nonafluoro-2-butyl | H | Cl |
| 9-13 | 4-cyanophenyl | Me | Me | H | H | H | H | Cl | H | nonafluoro-2-butyl | H | Cl |
| 9-24 | 2-chloropyridin-3-yl | Me | Me | H | H | H | H | Cl | H | nonafluoro-2-butyl | H | Cl |
| 9-38 | phenyl | Me | Me | H | H | H | H | Br | H | nonafluoro-2-butyl | H | Br |
| 9-45 | 3-cyanophenyl | Me | Me | H | H | H | H | Br | H | nonafluoro-2-butyl | H | Br |
| 9-46 | 4-cyanophenyl | Me | Me | H | H | H | H | Br | H | nonafluoro-2-butyl | H | Br |
| 9-57 | 2-chloropyridin-3-yl | Me | Me | H | H | H | H | Br | H | nonafluoro-2-butyl | H | Br |
| 9-71 | phenyl | Me | Me | H | H | H | H | I | H | nonafluoro-2-butyl | H | I |
| 9-78 | 3-cyanophenyl | Me | Me | H | H | H | H | I | H | nonafluoro-2-butyl | H | I |
| 9-79 | 4-cyanophenyl | Me | Me | H | H | H | H | I | H | nonafluoro-2-butyl | H | I |
| 9-90 | 2-chloropyridin-3-yl | Me | Me | H | H | H | H | I | H | nonafluoro-2-butyl | H | I |
| 9-109 | phenyl | Me | Me | H | H | H | H | Cl | H | nonafluoro-2-butyl | H | Br |
| 9-112 | phenyl | Me | Me | H | H | H | H | F | H | heptafluoroisopropyl | H | CF3 |
| 9-113 | phenyl | Me | Me | H | H | H | H | Cl | H | heptafluoroisopropyl | H | CF3 |
| 9-133 | 3-cyanophenyl | Me | Me | H | H | H | H | Cl | H | heptafluoroisopropyl | H | CF3 |
| 9-134 | 4-cyanophenyl | Me | Me | H | H | H | H | Cl | H | heptafluoroisopropyl | H | CF3 |
| 9-149 | 2-chloropyridin-3-yl | Me | Me | H | H | H | H | Cl | H | heptafluoroisopropyl | H | CF3 |
| 9-191 | phenyl | Me | Me | H | H | H | H | F | H | nonafluoro-2-butyl | H | CF3 |
| 9-192 | phenyl | Me | Me | H | H | H | H | Cl | H | nonafluoro-2-butyl | H | CF3 |
| 9-212 | 3-cyanophenyl | Me | Me | H | H | H | H | Cl | H | nonafluoro-2-butyl | H | CF3 |
| 9-213 | 4-cyanophenyl | Me | Me | H | H | H | H | Cl | H | nonafluoro-2-butyl | H | CF3 |
| 9-228 | 2-chloropyridin-3-yl | Me | Me | H | H | H | H | Cl | H | nonafluoro-2-butyl | H | CF3 |
| 9-270 | phenyl | Me | Me | H | H | H | H | Br | H | heptafluoroisopropyl | H | CF3 |
| 9-290 | 3-cyanophenyl | Me | Me | H | H | H | H | Br | H | heptafluoroisopropyl | H | CF3 |
| 9-291 | 4-cyanophenyl | Me | Me | H | H | H | H | Br | H | heptafluoroisopropyl | H | CF3 |
| 9-306 | 2-chloropyridin-3-yl | Me | Me | H | H | H | H | Br | H | heptafluoroisopropyl | H | CF3 |
| 9-348 | phenyl | Me | Me | H | H | H | H | Br | H | nonafluoro-2-butyl | H | CF3 |
| 9-368 | 3-cyanophenyl | Me | Me | H | H | H | H | Br | H | nonafluoro-2-butyl | H | CF3 |
| 9-369 | 4-cyanophenyl | Me | Me | H | H | H | H | Br | H | nonafluoro-2-butyl | H | CF3 |
| 9-384 | 2-chloropyridin-3-yl | Me | Me | H | H | H | H | Br | H | nonafluoro-2-butyl | H | CF3 |
| 9-426 | phenyl | Me | Me | H | H | H | H | I | H | heptafluoroisopropyl | H | CF3 |
| 9-446 | 3-cyanophenyl | Me | Me | H | H | H | H | I | H | heptafluoroisopropyl | H | CF3 |
| 9-447 | 4-cyanophenyl | Me | Me | H | H | H | H | I | H | heptafluoroisopropyl | H | CF3 |
| 9-462 | 2-chloropyridin-3-yl | Me | Me | H | H | H | H | I | H | heptafluoroisopropyl | H | CF3 |
| 9-504 | phenyl | Me | Me | H | H | H | H | I | H | nonafluoro-2-butyl | H | CF3 |
| 9-524 | 3-cyanophenyl | Me | Me | H | H | H | H | I | H | nonafluoro-2-butyl | H | CF3 |
| 9-525 | 4-cyanophenyl | Me | Me | H | H | H | H | I | H | nonafluoro-2-butyl | H | CF3 |
| 9-540 | 2-chloropyridin-3-yl | Me | Me | H | H | H | H | I | H | nonafluoro-2-butyl | H | CF3 |
| 9-778 | phenyl | Me | Me | H | H | H | H | OCF3 | H | heptafluoroisopropyl | H | OCF3 |
| 9-809 | 2-chloropyridin-3-yl | Me | Me | H | H | H | H | OCF3 | H | heptafluoroisopropyl | H | OCF3 |
| 9-834 | phenyl | Me | Me | H | H | H | H | OCF3 | H | nonafluoro-2-butyl | H | OCF3 |
| 9-865 | 2-chloropyridin-3-yl | Me | Me | H | H | H | H | OCF3 | H | nonafluoro-2-butyl | H | OCF3 |
| 9-890 | phenyl | Me | Me | H | H | H | H | CF3 | H | heptafluoroisopropyl | H | OCF3 |
| 9-921 | 2-chloropyridin-3-yl | Me | Me | H | H | H | H | CF3 | H | heptafluoroisopropyl | H | OCF3 |
| 9-946 | phenyl | Me | Me | H | H | H | H | CF3 | H | nonafluoro-2-butyl | H | OCF3 |
| 9-977 | 2-chloropyridin-3-yl | Me | Me | H | H | H | H | CF3 | H | nonafluoro-2-butyl | H | OCF3 |
| 9-1002 | phenyl | Me | Me | H | H | H | H | CF3 | H | heptafluoroisopropyl | H | CF3 |
| 9-1022 | 3-cyanophenyl | Me | Me | H | H | H | H | CF3 | H | heptafluoroisopropyl | H | CF3 |
| 9-1023 | 4-cyanophenyl | Me | Me | H | H | H | H | CF3 | H | heptafluoroisopropyl | H | CF3 |
| 9-1038 | 2-chloropyridin-3-yl | Me | Me | H | H | H | H | CF3 | H | heptafluoroisopropyl | H | CF3 |

**Table 9(2)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 9-1070 | phenyl | Me | Me | H | H | H | H | CF3 | H | nonafluoro-2-butyl | H | CF3 |
| 9-1090 | 3-cyanophenyl | Me | Me | H | H | H | H | CF3 | H | nonafluoro-2-butyl | H | CF3 |
| 9-1091 | 4-cyanophenyl | Me | Me | H | H | H | H | CF3 | H | nonafluoro-2-butyl | H | CF3 |
| 9-1106 | 2-chloropyridin-3-yl | Me | Me | H | H | H | H | CF3 | H | nonafluoro-2-butyl | H | CF3 |
| 9-1334 | phenyl | Me | Me | F | H | H | H | F | H | heptafluoroisopropyl | H | F |
| 9-1335 | 3-cyanophenyl | Me | Me | F | H | H | H | F | H | heptafluoroisopropyl | H | F |
| 9-1336 | 4-cyanophenyl | Me | Me | F | H | H | H | F | H | heptafluoroisopropyl | H | F |
| 9-1337 | 2-chloropyridin-3-yl | Me | Me | F | H | H | H | F | H | heptafluoroisopropyl | H | F |
| 9-1338 | phenyl | Me | Me | F | H | H | H | F | H | nonafluoro-2-butyl | H | F |
| 9-1339 | 3-cyanophenyl | Me | Me | F | H | H | H | F | H | nonafluoro-2-butyl | H | F |
| 9-1340 | 4-cyanophenyl | Me | Me | F | H | H | H | F | H | nonafluoro-2-butyl | H | F |
| 9-1341 | 2-chloropyridin-3-yl | Me | Me | F | H | H | H | F | H | nonafluoro-2-butyl | H | F |
| 9-1342 | phenyl | Me | Me | F | H | H | H | Cl | H | heptafluoroisopropyl | H | Cl |
| 9-1362 | 3-cyanophenyl | Me | Me | F | H | H | H | Cl | H | heptafluoroisopropyl | H | Cl |
| 9-1363 | 4-cyanophenyl | Me | Me | F | H | H | H | Cl | H | heptafluoroisopropyl | H | Cl |
| 9-1378 | 2-chloropyridin-3-yl | Me | Me | F | H | H | H | Cl | H | heptafluoroisopropyl | H | Cl |
| 9-1420 | phenyl | Me | Me | F | H | H | H | Cl | H | nonafluoro-2-butyl | H | Cl |
| 9-1440 | 3-cyanophenyl | Me | Me | F | H | H | H | Cl | H | nonafluoro-2-butyl | H | Cl |
| 9-1441 | 4-cyanophenyl | Me | Me | F | H | H | H | Cl | H | nonafluoro-2-butyl | H | Cl |
| 9-1456 | 2-chloropyridin-3-yl | Me | Me | F | H | H | H | Cl | H | nonafluoro-2-butyl | H | Cl |
| 9-1498 | phenyl | Me | Me | F | H | H | H | Br | H | heptafluoroisopropyl | H | Br |
| 9-1518 | 3-cyanophenyl | Me | Me | F | H | H | H | Br | H | heptafluoroisopropyl | H | Br |
| 9-1519 | 4-cyanophenyl | Me | Me | F | H | H | H | Br | H | heptafluoroisopropyl | H | Br |
| 9-1534 | 2-chloropyridin-3-yl | Me | Me | F | H | H | H | Br | H | heptafluoroisopropyl | H | Br |
| 9-1576 | phenyl | Me | Me | F | H | H | H | Br | H | nonafluoro-2-butyl | H | Br |
| 9-1596 | 3-cyanophenyl | Me | Me | F | H | H | H | Br | H | nonafluoro-2-butyl | H | Br |
| 9-1597 | 4-cyanophenyl | Me | Me | F | H | H | H | Br | H | nonafluoro-2-butyl | H | Br |
| 9-1612 | 2-chloropyridin-3-yl | Me | Me | F | H | H | H | Br | H | nonafluoro-2-butyl | H | Br |
| 9-1654 | phenyl | Me | Me | F | H | H | H | I | H | heptafluoroisopropyl | H | I |
| 9-1674 | 3-cyanophenyl | Me | Me | F | H | H | H | I | H | heptafluoroisopropyl | H | I |
| 9-1675 | 4-cyanophenyl | Me | Me | F | H | H | H | I | H | heptafluoroisopropyl | H | I |
| 9-1690 | 2-chloropyridin-3-yl | Me | Me | F | H | H | H | I | H | heptafluoroisopropyl | H | I |
| 9-1732 | phenyl | Me | Me | F | H | H | H | I | H | nonafluoro-2-butyl | H | I |
| 9-1752 | 3-cyanophenyl | Me | Me | F | H | H | H | I | H | nonafluoro-2-butyl | H | I |
| 9-1753 | 4-cyanophenyl | Me | Me | F | H | H | H | I | H | nonafluoro-2-butyl | H | I |
| 9-1768 | 2-chloropyridin-3-yl | Me | Me | F | H | H | H | I | H | nonafluoro-2-butyl | H | I |
| 9-2006 | phenyl | Me | Me | F | H | H | H | F | H | heptafluoroisopropyl | H | CF3 |
| 9-2007 | phenyl | Me | Me | F | H | H | H | Cl | H | heptafluoroisopropyl | H | CF3 |
| 9-2019 | 4-iodophenyl | Me | Et | F | H | H | H | Cl | H | pentafluoroethyl | H | CF3 |
| 9-2021 | (3-trifluoromethyl) phenyl | Et | Me | F | H | H | H | Cl | H | heptafluoroisopropyl | H | C2F5 |
| 9-2027 | 3-cyanophenyl | Me | Me | F | H | H | H | Cl | H | heptafluoroisopropyl | H | CF3 |
| 9-2028 | 4-cyanophenyl | Me | Me | F | H | H | H | Cl | H | heptafluoroisopropyl | H | CF3 |
| 9-2030 | 34-dichlorophenyl | Me | n-Pr | F | H | H | H | Cl | H | heptafluoroisopropyl | H | CF3 |
| 9-2032 | 2-chloro-4-fluorophenyl | n-Pr | Me | F | H | H | H | Cl | H | heptafluoroisopropyl | H | CF3 |
| 9-2043 | 2-chloropyridin-3-yl | Me | Me | F | H | H | H | Cl | H | heptafluoroisopropyl | H | CF3 |
| 9-2047 | 2-nitropyridin-3-yl | Me | i-Pr | F | H | H | H | Cl | H | heptafluoroisopropyl | H | CF3 |
| 9-2049 | 6-fluoropyridin-3-yl | i-Pr | Me | F | H | H | H | Cl | H | heptafluoroisopropyl | H | CF3 |
| 9-2063 | 4-fluoropyridin-3-yl | Me | CH2CH=CH2 | F | H | H | H | Cl | H | pentafluoroethyl | H | CF3 |
| 9-2065 | 4-bromopyridin-3-yl | CH2CH=CH2 | Me | F | H | H | H | Cl | H | heptafluoroisopropyl | H | n-C3F7 |
| 9-2070 | 2.6-dichloropyridin-3-yl | Me | CN | F | H | H | H | Cl | H | heptafluoroisopropyl | H | CF3 |
| 9-2072 | pyridin-4-yl | CN | Me | F | H | H | H | Cl | H | heptafluoroisopropyl | H | CF3 |
| 9-2085 | phenyl | Me | Me | F | H | H | H | F | H | nonafluoro-2-butyl | H | CF3 |
| 9-2086 | phenyl | Me | Me | F | H | H | H | Cl | H | nonafluoro-2-butyl | H | CF3 |
| 9-2092 | 4-chlorophenyl | Me | CH2C≡CH | F | H | H | H | Cl | H | nonafluoro-2-butyl | H | CF3 |
| 9-2094 | 3-bromophenyl | CH2C≡CH | Me | F | H | H | H | Cl | H | nonafluoro-2-butyl | H | CF3 |
| 9-2099 | (2-trifluoromethyl) phenyl | Me | NH2 | F | H | H | H | Cl | H | nonafluoro-2-butyl | H | C2F5 |
| 9-2101 | (4-trifluoromethyl) phenyl | NH2 | Me | F | H | H | H | Cl | H | nonafluoro-2-butyl | H | CF3 |
| 9-2106 | 3-cyanophenyl | Me | Me | F | H | H | H | Cl | H | nonafluoro-2-butyl | H | CF3 |
| 9-2107 | 4-cyanophenyl | Me | Me | F | H | H | H | Cl | H | nonafluoro-2-butyl | H | CF3 |
| 9-2112 | 2-chloro-4,5-difluorophenyl | Me | C(O)OMe | F | H | H | H | Cl | H | nonafluoro-2-butyl | H | CF3 |
| 9-2114 | 2-bromo-4-chlorophenyl | C(O)OMe | Me | F | H | H | H | Cl | H | nonafluoro-2-butyl | H | CF3 |
| 9-2122 | 2-chloropyridin-3-yl | Me | Me | F | H | H | H | Cl | H | nonafluoro-2-butvl | H | CF3 |
| 9-2133 | 6-nitropyridin-3-yl | Me | C(O)OEt | F | H | H | H | Cl | H | nonafluoro-2-butyl | H | CF3 |
| 9-2135 | 5-fluoropyridin-3-yl | C(O)OEt | Me | F | H | H | H | Cl | H | nonafluoro-2-butyl | H | C2F5 |

**Table 9 (3)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 9-2142 | 4-fluoropyridin-3-yl | Me | C(O)C(O)Me | F | H | H | H | Cl | H | nonafluoro-2-butyl | H | C2F5 |
| 9-2144 | 4-bromopyridin-3-yl | C(O)C(O)Me | Me | F | H | H | H | Cl | H | nonafluoro-2-butyl | H | CF3 |
| 9-2145 | 4-iodopyridin-3-yl | Me | C(O)C(O)Et | F | H | H | H | Cl | H | nonafluoro-2-butyl | H | CF3 |
| 9-2147 | 4-nitropyridin-3-yl | C(O)C(O)Et | Me | F | H | H | H | Cl | H | nonafluoro-2-butyl | H | i-C3F7 |
| 9-2156 | 2,6-dicyanopyridin-4-yl | Me | C(O)Et | F | H | H | H | Cl | H | nonafluoro-2-butyl | H | CF3 |
| 9-2159 | 3-chloropyridin-2-yl | Me | C(O)Me | F | H | H | H | Cl | H | nonafluoro-2-butyl | H | CF3 |
| 9-2161 | 5-iodopyridin-2-yl | C(O)Me | Me | F | H | H | H | Cl | H | nonafluoro-2-butyl | H | CF3 |
| 9-2163 | 4-cyanopyridin-2-yl | C(O)Et | Me | F | H | H | H | Cl | H | nonafluoro-2-butyl | H | C2F5 |
| 9-2164 | phenyl | Me | Me | F | H | H | H | Br | H | heptafluoroisopropyl | H | CF3 |
| 9-2176 | 4-iodophenyl | Me | S(O)2Me | F | H | H | H | Br | H | heptafluoroisopropyl | H | CF3 |
| 9-2178 | (3-trifluoromethyl) phenyl | S(O)2Me | Me | F | H | H | H | Br | H | heptafluoroisopropyl | H | CF3 |
| 9-2184 | 3-cyanophenyl | Me | Me | F | H | H | H | Br | H | heptafluoroisopropyl | H | CF3 |
| 9-2185 | 4-cyanophenyl | Me | Me | F | H | H | H | Br | H | heptafluoroisopropyl | H | CF3 |
| 9-2194 | 2-chloro-4-nitrophenyl | Me | S(O)2Et | F | H | H | H | Br | H | heptafluoroisopropyl | H | CF3 |
| 9-2196 | 4-cyano-2-fluorophenyl | S(O)2Et | Me | F | H | H | H | Br | H | heptafluoroisopropyl | H | CF3 |
| 9-2200 | 2-chloropyridin-3-yl | Me | Me | F | H | H | H | Br | H | heptafluoroisopropyl | H | CF3 |
| 9-2209 | 6-iodopyridin-3-yl | Me | CH2Ph | F | H | H | H | Br | H | heptafluoroisopropyl | H | CF3 |
| 9-2211 | 6-nitropyridin-3-yl | CH2Ph | Me | F | H | H | H | Br | H | heptafluoroisopropyl | H | CF3 |
| 9-2218 | 5-nitropyridin-3-yl | Me | CH2(3-Py) | F | H | H | H | Br | H | heptafluoroisopropyl | H | CF3 |
| 9-2220 | 4-fluoropyridin-3-yl | CH2(3-Py) | Me | F | H | H | H | Br | H | heptafluoroisopropyl | H | CF3 |
| 9-2239 | 5-iodopyridin-2-yl | Me | CH2(3-Py-N-oxide) | F | H | H | H | Br | H | heptafluoroisopropyl | H | CF3 |
| 9-2241 | 4-cyanopyridin-2-yl | CH2(3-Py-N-oxide) | Me | F | H | H | H | Br | H | heptafluoroisopropyl | H | CF3 |
| 9-2242 | phenyl | Me | Me | F | H | H | H | Br | H | nonafluoro-2-butyl | H | CF3 |
| 9-2262 | 3-cyanophenyl | Me | Me | F | H | H | H | Br | H | nonafluoro-2-butyl | H | CF3 |
| 9-2263 | 4-cyanophenyl | Me | Me | F | H | H | H | Br | H | nonafluoro-2-butyl | H | CF3 |
| 9-2267 | 2-chloro-4-fluorophenyl | Me | Et | F | H | H | H | Br | H | nonafluoro-2-butyl | H | CF3 |
| 9-2269 | 4-bromo-2-chlorophenyl | Et | Me | F | H | H | H | Br | H | nonafluoro-2-butyl | H | CF3 |
| 9-2278 | 2-chloropyridin-3-yl | Me | Me | F | H | H | H | Br | H | nonafluoro-2-butyl | H | CF3 |
| 9-2293 | 5-bromopyridin-3-yl | Me | CN | F | H | H | H | Br | H | nonafluoro-2-butyl | H | CF3 |
| 9-2295 | 5-(trifluoromethyl)pyridin-3-yl | CN | Me | F | H | H | H | Br | H | nonafluoro-2-butyl | H | CF3 |
| 9-2320 | phenyl | Me | Me | F | H | H | H | I | H | heptafluoroisopropyl | H | CF3 |
| 9-2322 | 3-fluorophenyl | Me | Me | F | H | H | H | I | H | heptafluoroisopropyl | H | CF3 |
| 9-2340 | 3-cyanophenyl | Me | Me | F | H | H | H | I | H | heptafluoroisopropyl | H | CF3 |
| 9-2341 | 4-cyanophenyl | Me | Me | F | H | H | H | I | H | heptafluoroisopropyl | H | CF3 |
| 9-2356 | 2-chloropyridin-3-yl | Me | Me | F | H | H | H | I | H | heptafluoroisopropyl | H | CF3 |
| 9-2367 | 6-nitropyridin-3-yl | Me | C(O)Me | F | H | H | H | I | H | heptafluoroisopropyl | H | CF3 |
| 9-2369 | 5-fluoropyridin-3-yl | C(O)Me | Me | F | H | H | H | I | H | heptafluoroisopropyl | H | CF3 |
| 9-2398 | phenyl | Me | Me | F | H | H | H | I | H | nonafluoro-2-butyl | H | CF3 |
| 9-2418 | 3-cyanophenyl | Me | Me | F | H | H | H | I | H | nonafluoro-2-butyl | H | CF3 |
| 9-2419 | 4-cyanophenyl | Me | Me | F | H | H | H | I | H | nonafluoro-2-butyl | H | CF3 |
| 9-2434 | 2-chloropyridin-3-yl | Me | Me | F | H | H | H | I | H | nonafluoro-2-butyl | H | CF3 |
| 9-2476 | phenyl | Et | Et | F | H | H | H | I | H | nonafluoro-2-butyl | H | CF3 |
| 9-2477 | phenyl | Et | n-Pr | F | H | H | H | I | H | nonafluoro-2-butyl | H | CF3 |
| 9-2478 | phenyl | Et | i-Pr | F | H | H | H | I | H | nonafluoro-2-butyl | H | CF3 |
| 9-2479 | phenyl | Et | CH2CH=CH2 | F | H | H | H | I | H | nonafluoro-2-butyl | H | CF3 |
| 9-2480 | phenyl | Et | CN | F | H | H | H | I | H | nonafluoro-2-butyl | H | CF3 |
| 9-2481 | phenyl | Et | CH2C≡CH | F | H | H | H | I | H | nonafluoro-2-butyl | H | CF3 |
| 9-2482 | phenyl | Et | NH2 | F | H | H | H | I | H | nonafluoro-2-butyl | H | CF3 |
| 9-2483 | phenyl | Et | C(O)OMe | F | H | H | H | I | H | nonafluoro-2-butyl | H | CF3 |
| 9-2484 | phenyl | Et | C(O)C(O)OMe | F | H | H | H | I | H | nonafluoro-2-butyl | H | CF3 |
| 9-2485 | phenyl | Et | C(O)Me | F | H | H | H | I | H | nonafluoro-2-butyl | H | CF3 |
| 9-2486 | phenyl | Et | S(O)2Me | F | H | H | H | I | H | nonafluoro-2-butyl | H | CF3 |
| 9-2487 | phenyl | Et | CH2Ph | F | H | H | H | I | H | nonafluoro-2-butyl | H | CF3 |
| 9-2488 | phenyl | Et | CH2(3-Py) | F | H | H | H | I | H | nonafluoro-2-butyl | H | CF3 |
| 9-2489 | phenyl | Et | CH2(3-Py-N-oxide) | F | H | H | H | I | H | nonafluoro-2-butyl | H | CF3 |
| 9-2490 | phenyl | n-Pr | Et | F | H | H | H | I | H | nonafluoro-2-butyl | H | CF3 |
| 9-2491 | phenyl | n-Pr | n-Pr | F | H | H | H | I | H | nonafluoro-2-butyl | H | CF3 |
| 9-2492 | phenyl | n-Pr | i-Pr | F | H | H | H | I | H | nonafluoro-2-butyl | H | CF3 |
| 9-2493 | phenyl | n-Pr | CH2CH=CH2 | F | H | H | H | I | H | nonafluoro-2-butvl | H | CF3 |
| 9-2494 | phenyl | n-Pr | CN | F | H | H | H | I | H | nonafluoro-2-butyl | H | CF3 |
| 9-2495 | phenyl | n-Pr | CH2C≡CH | F | H | H | H | I | H | nonafluoro-2-butyl | H | CF3 |
| 9-2496 | phenyl | n-Pr | NH2 | F | H | H | H | I | H | nonafluoro-2-butyl | H | CF3 |
| 9-2497 | phenyl | n-Pr | C(O)OMe | F | H | H | H | I | H | nonafluoro-2-butyl | H | CF3 |
| 9-2498 | phenyl | n-Pr | C(C)C(C)CMe | F | H | H | H | I | H | nonafluoro-2-butyl | H | CF3 |

**Table 9(4)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 9-2499 | phenyl | n-Pr | C(O)Me | F | H | H | H | I | H | nonafluoro-2-butyl | H | CF3 |
| 9-2500 | phenyl | n-Pr | S(O)2Me | F | H | H | H | I | H | nonafluoro-2-butyl | H | CF3 |
| 9-2501 | phenyl | n-Pr | CH2Ph | F | H | H | H | I | H | nonafluoro-2-butyl | H | CF3 |
| 9-2502 | phenyl | n-Pr | CH2(3-Py) | F | H | H | H | I | H | nonafluoro-2-butyl | H | CF3 |
| 9-2503 | phenyl | n-Pr | CH2(3-Py-N-oxide) | F | H | H | H | I | H | nonafluoro-2-butyl | H | CF3 |
| 9-2504 | phenyl | i-Pr | Et | F | H | H | H | I | H | nonafluoro-2-butyl | H | CF3 |
| 9-2505 | phenyl | i-Pr | n-Pr | F | H | H | H | I | H | nonafluoro-2-butyl | H | CF3 |
| 9-2506 | phenyl | i-Pr | i-Pr | F | H | H | H | I | H | nonafluoro-2-butyl | H | CF3 |
| 9-2507 | phenyl | i-Pr | CH2CH=CH2 | F | H | H | H | I | H | nonafluoro-2-butyl | H | CF3 |
| 9-2508 | phenyl | i-Pr | CN | F | H | H | H | I | H | nonafluoro-2-butyl | H | CF3 |
| 9-2509 | phenyl | i-Pr | CH2C≡CH | F | H | H | H | I | H | nonafluoro-2-butyl | H | CF3 |
| 9-2510 | phenyl | i-Pr | NH2 | F | H | H | H | I | H | nonafluoro-2-butyl | H | CF3 |
| 9-2511 | phenyl | i-Pr | C(O)OMe | F | H | H | H | I | H | nonafluoro-2-butyl | H | CF3 |
| 9-2512 | phenyl | i-Pr | C(O)C(O)OMe | F | H | H | H | I | H | nonafluoro-2-butyl | H | CF3 |
| 9-2513 | phenyl | i-Pr | C(O)Me | F | H | H | H | I | H | nonafluoro-2-butyl | H | CF3 |
| 9-2514 | phenyl | i-Pr | S(O)2Me | F | H | H | H | I | H | nonafluoro-2-butyl | H | CF3 |
| 9-2515 | phenyl | i-Pr | CH2Ph | F | H | H | H | I | H | nonafluoro-2-butyl | H | CF3 |
| 9-2516 | phenyl | i-Pr | CH2(3-Py) | F | H | H | H | I | H | nonafluoro-2-butyl | H | CF3 |
| 9-2517 | phenyl | i-Pr | CH2(3-Py-N-oxide) | F | H | H | H | I | H | nonafluoro-2-butyl | H | CF3 |
| 9-2518 | phenyl | CH2CH=CH2 | Et | F | H | H | H | I | H | nonafluoro-2-butyl | H | CF3 |
| 9-2519 | phenyl | CH2CH=CH2 | n-Pr | F | H | H | H | I | H | nonafluoro-2-butyl | H | CF3 |
| 9-2520 | phenyl | CH2CH=CH2 | i-Pr | F | H | H | H | I | H | nonafluoro-2-butyl | H | CF3 |
| 9-2521 | phenyl | CH2CH=CH2 | CH2CH=CH2 | F | H | H | H | I | H | nonafluoro-2-butyl | H | CF3 |
| 9-2522 | phenyl | CH2CH=CH2 | CN | F | H | H | H | I | H | nonafluoro-2-butyl | H | CF3 |
| 9-2523 | phenyl | CH2CH=CH2 | CH2C≡CH | F | H | H | H | I | H | nonafluoro-2-butyl | H | CF3 |
| 9-2524 | phenyl | CH2CH=CH2 | NH2 | F | H | H | H | I | H | nonafluoro-2-butyl | H | CF3 |
| 9-2525 | phenyl | CH2CH=CH2 | C(O)OMe | F | H | H | H | I | H | nonafluoro-2-butyl | H | CF3 |
| 9-2526 | phenyl | CH2CH=CH2 | C(O)C(O)OMe | F | H | H | H | I | H | nonafluoro-2-butyl | H | CF3 |
| 9-2527 | phenyl | CH2CH=CH2 | C(O)Me | F | H | H | H | I | H | nonafluoro-2-butyl | H | CF3 |
| 9-2528 | phenyl | CH2CH=CH2 | S(O)2Me | F | H | H | H | I | H | nonafluoro-2-butyl | H | CF3 |
| 9-2529 | phenyl | CH2CH=CH2 | CH2Ph | F | H | H | H | I | H | nonafluoro-2-butyl | H | CF3 |
| 9-2530 | phenyl | CH2CH=CH2 | CH2(3-Py) | F | H | H | H | I | H | nonafluoro-2-butyl | H | CF3 |
| 9-2531 | phenyl | CH2CH=CH2 | CH2(3-Py-N-oxide) | F | H | H | H | I | H | nonafluoro-2-butyl | H | CF3 |
| 9-2532 | phenyl | CN | Et | F | H | H | H | I | H | nonafluoro-2-butyl | H | CF3 |
| 9-2533 | phenyl | CN | n-Pr | F | H | H | H | I | H | nonafluoro-2-butyl | H | CF3 |
| 9-2534 | phenyl | CN | i-Pr | F | H | H | H | I | H | nonafluoro-2-butyl | H | CF3 |
| 9-2535 | phenyl | CN | CH2CH=CH2 | F | H | H | H | I | H | nonafluoro-2-butyl | H | CF3 |
| 9-2536 | phenyl | CN | CN | F | H | H | H | I | H | nonafluoro-2-butyl | H | CF3 |
| 9-2537 | phenyl | CN | CH2C≡CH | F | H | H | H | I | H | nonafluoro-2-butyl | H | CF3 |
| 9-2538 | phenyl | CN | NH2 | F | H | H | H | I | H | nonafluoro-2-butyl | H | CF3 |
| 9-2539 | phenyl | CN | C(O)OMe | F | H | H | H | I | H | nonafluoro-2-butyl | H | CF3 |
| 9-2540 | phenyl | CN | C(O)C(O)OMe | F | H | H | H | I | H | nonafluoro-2-butyl | H | CF3 |
| 9-2541 | phenyl | CN | C(O)Me | F | H | H | H | I | H | nonafluoro-2-butyl | H | CF3 |
| 9-2542 | phenyl | CN | S(O)2Me | F | H | H | H | I | H | nonafluoro-2-butyl | H | CF3 |
| 9-2543 | phenyl | CN | CH2Ph | F | H | H | H | I | H | nonafluoro-2-butyl | H | CF3 |
| 9-2544 | phenyl | CN | CH2(3-Py) | F | H | H | H | I | H | nonafluoro-2-butyl | H | CF3 |
| 9-2545 | phenyl | CN | CH2(3-Py-N-oxide) | F | H | H | H | I | H | nonafluoro-2-butyl | H | CF3 |
| 9-2546 | phenyl | CH2C≡CH | Et | F | H | H | H | I | H | nonafluoro-2-butyl | H | CF3 |
| 9-2547 | phenyl | CH2C≡CH | n-Pr | F | H | H | H | I | H | nonafluoro-2-butyl | H | CF3 |
| 9-2548 | phenyl | CH2C≡CH | i-Pr | F | H | H | H | I | H | nonafluoro-2-butyl | H | CF3 |
| 9-2549 | phenyl | CH2C≡CH | CH2CH=CH2 | F | H | H | H | I | H | nonafluoro-2-butyl | H | CF3 |
| 9-2550 | phenyl | CH2C≡CH | CN | F | H | H | H | I | H | nonafluoro-2-butyl | H | CF3 |
| 9-2551 | phenyl | CH2C≡CH | CH2C≡CH | F | H | H | H | I | H | nonafluoro-2-butyl | H | CF3 |
| 9-2552 | phenyl | CH2C≡CH | NH2 | F | H | H | H | I | H | nonafluoro-2-butyl | H | CF3 |
| 9-2553 | phenyl | CH2C≡CH | C(O)OMe | F | H | H | H | I | H | nonafluoro-2-butyl | H | CF3 |
| 9-2554 | phenyl | CH2C≡CH | C(O)C(O)OMe | F | H | H | H | I | H | nonafluoro-2-butyl | H | CF3 |
| 9-2555 | phenyl | CH2C≡CH | C(O)Me | F | H | H | H | I | H | nonafluoro-2-butyl | H | CF3 |
| 9-2556 | phenyl | CH2C≡CH | S(O)2Me | F | H | H | H | I | H | nonafluoro-2-butvl | H | CF3 |
| 9-2557 | phenyl | CH2C≡CH | CH2Ph | F | H | H | H | I | H | nonafluoro-2-butyl | H | CF3 |
| 9-2558 | phenyl | CH2C≡CH | CH2(3-Py) | F | H | H | H | I | H | nonafluoro-2-butyl | H | CF3 |
| 9-2559 | phenyl | CH2C≡CH | CH2(3-Py-N-oxide) | F | H | H | H | I | H | nonafluoro-2-butyl | H | CF3 |
| 9-2560 | phenyl | NH2 | Et | F | H | H | H | I | H | nonafluoro-2-butyl | H | CF3 |
| 9-2561 | phenyl | NH2 | n-Pr | F | H | H | H | I | H | nonafluoro-2-butyl | H | CF3 |
| 9-2562 | phenyl | NH2 | i-Pr | F | H | H | H | I | H | nonafluoro-2-butyl | H | CF3 |
| 9-2563 | phenyl | NH2 | CH2CH=CH2 | F | H | H | H | I | H | nonafluoro-2-butyl | H | CF3 |

**Table 9(5)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 9-2564 | phenyl | NH2 | CN | F | H | H | H | I | H | nonafluoro-2-butyl | H | CF3 |
| 9-2565 | phenyl | NH2 | CH2C≡CH | F | H | H | H | I | H | nonafluoro-2-butyl | H | CF3 |
| 9-2566 | phenyl | NH2 | NH2 | F | H | H | H | I | H | nonafluoro-2-butyl | H | CF3 |
| 9-2567 | phenyl | NH2 | C(O)OMe | F | H | H | H | I | H | nonafluoro-2-butyl | H | CF3 |
| 9-2568 | phenyl | NH2 | C(O)C(O)OMe | F | H | H | H | I | H | nonafluoro-2-butyl | H | CF3 |
| 9-2569 | phenyl | NH2 | C(O)Me | F | H | H | H | I | H | nonafluoro-2-butyl | H | CF3 |
| 9-2570 | phenyl | NH2 | S(O)2Me | F | H | H | H | I | H | nonafluoro-2-butyl | H | CF3 |
| 9-2571 | phenyl | NH2 | CH2Ph | F | H | H | H | I | H | nonafluoro-2-butyl | H | CF3 |
| 9-2572 | phenyl | NH2 | CH2(3-Py) | F | H | H | H | I | H | nonafluoro-2-butyl | H | CF3 |
| 9-2573 | phenyl | NH2 | CH2(3-Py-N-oxide) | F | H | H | H | I | H | nonafluoro-2-butyl | H | CF3 |
| 9-2574 | phenyl | C(O)OMe | Et | F | H | H | H | I | H | nonafluoro-2-butyl | H | CF3 |
| 9-2575 | phenyl | C(O)OMe | n-Pr | F | H | H | H | I | H | nonafluoro-2-butyl | H | CF3 |
| 9-2576 | phenyl | C(O)OMe | i-Pr | F | H | H | H | I | H | nonafluoro-2-butyl | H | CF3 |
| 9-2577 | phenyl | C(O)OMe | CH2CH=CH2 | F | H | H | H | I | H | nonafluoro-2-butyl | H | CF3 |
| 9-2578 | phenyl | C(O)OMe | CN | F | H | H | H | I | H | nonafluoro-2-butyl | H | CF3 |
| 9-2579 | phenyl | C(O)OMe | CH2C≡CH | F | H | H | H | I | H | nonafluoro-2-butyl | H | CF3 |
| 9-2580 | phenyl | C(O)OMe | NH2 | F | H | H | H | I | H | nonafluoro-2-butyl | H | CF3 |
| 9-2581 | phenyl | C(O)OMe | C(O)OMe | F | H | H | H | I | H | nonafluoro-2-butyl | H | CF3 |
| 9-2582 | phenyl | C(O)OMe | C(O)C(O)OMe | F | H | H | H | I | H | nonafluoro-2-butyl | H | CF3 |
| 9-2583 | phenyl | C(O)OMe | C(O)Me | F | H | H | H | I | H | nonafluoro-2-butyl | H | CF3 |
| 9-2584 | phenyl | C(O)OMe | S(O)2Me | F | H | H | H | I | H | nonafluoro-2-butyl | H | CF3 |
| 9-2585 | phenyl | C(O)OMe | CH2Ph | F | H | H | H | I | H | nonafluoro-2-butyl | H | CF3 |
| 9-2586 | phenyl | C(O)OMe | CH2(3-Py) | F | H | H | H | I | H | nonafluoro-2-butyl | H | CF3 |
| 9-2587 | phenyl | C(O)OMe | CH2(3-Py-N-oxide) | F | H | H | H | I | H | nonafluoro-2-butyl | H | CF3 |
| 9-2588 | phenyl | C(O)C(O)OMe | Et | F | H | H | H | I | H | nonafluoro-2-butyl | H | CF3 |
| 9-2589 | phenyl | C(O)C(O)OMe | n-Pr | F | H | H | H | I | H | nonafluoro-2-butyl | H | CF3 |
| 9-2590 | phenyl | C(O)C(O)OMe | i-Pr | F | H | H | H | I | H | nonafluoro-2-butyl | H | CF3 |
| 9-2591 | phenyl | C(O)C(O)OMe | CH2CH=CH2 | F | H | H | H | I | H | nonafluoro-2-butyl | H | CF3 |
| 9-2592 | phenyl | C(O)C(O)OMe | CN | F | H | H | H | I | H | nonafluoro-2-butyl | H | CF3 |
| 9-2593 | phenyl | C(O)C(O)OMe | CH2C≡CH | F | H | H | H | I | H | nonafluoro-2-butyl | H | CF3 |
| 9-2594 | phenyl | C(O)C(O)OMe | NH2 | F | H | H | H | I | H | nonafluoro-2-butyl | H | CF3 |
| 9-2595 | phenyl | C(O)C(O)OMe | C(O)OMe | F | H | H | H | I | H | nonafluoro-2-butyl | H | CF3 |
| 9-2596 | phenyl | C(O)C(O)OMe | C(O)C(O)OMe | F | H | H | H | I | H | nonafluoro-2-butyl | H | CF3 |
| 9-2597 | phenyl | C(O)C(O)OMe | C(O)Me | F | H | H | H | I | H | nonafluoro-2-butyl | H | CF3 |
| 9-2598 | phenyl | C(O)C(O)OMe | S(O)2Me | F | H | H | H | I | H | nonafluoro-2-butyl | H | CF3 |
| 9-2599 | phenyl | C(O)C(O)OMe | CH2Ph | F | H | H | H | I | H | nonafluoro-2-butyl | H | CF3 |
| 9-2600 | phenyl | C(O)C(O)OMe | CH2(3-Py) | F | H | H | H | I | H | nonafluoro-2-butyl | H | CF3 |
| 9-2601 | phenyl | C(O)C(O)OMe | CH2(3-Py-N-oxide) | F | H | H | H | I | H | nonafluoro-2-butyl | H | CF3 |
| 9-2602 | phenyl | C(O)Me | Et | F | H | H | H | I | H | nonafluoro-2-butyl | H | CF3 |
| 9-2603 | phenyl | C(O)Me | n-Pr | F | H | H | H | I | H | nonafluoro-2-butyl | H | CF3 |
| 9-2604 | phenyl | C(O)Me | i-Pr | F | H | H | H | I | H | nonafluoro-2-butyl | H | CF3 |
| 9-2605 | phenyl | C(O)Me | CH2CH=CH2 | F | H | H | H | I | H | nonafluoro-2-butyl | H | CF3 |
| 9-2606 | phenyl | C(O)Me | CN | F | H | H | H | I | H | nonafluoro-2-butyl | H | CF3 |
| 9-2607 | phenyl | C(O)Me | CH2C≡CH | F | H | H | H | I | H | nonafluoro-2-butyl | H | CF3 |
| 9-2608 | phenyl | C(O)Me | NH2 | F | H | H | H | I | H | nonafluoro-2-butyl | H | CF3 |
| 9-2609 | phenyl | C(O)Me | C(O)OMe | F | H | H | H | I | H | nonafluoro-2-butyl | H | CF3 |
| 9-2610 | phenyl | C(O)Me | C(O)C(O)OMe | F | H | H | H | I | H | nonafluoro-2-butyl | H | CF3 |
| 9-2611 | phenyl | C(O)Me | C(O)Me | F | H | H | H | Br | H | heptafluoroisopropyl | H | CF3 |
| 9-2612 | phenyl | C(O)Me | S(O)2Me | F | H | H | H | I | H | nonafluoro-2-butyl | H | CF3 |
| 9-2613 | phenyl | C(O)Me | CH2Ph | F | H | H | H | I | H | nonafluoro-2-butyl | H | CF3 |
| 9-2614 | phenyl | C(O)Me | CH2(3-Py) | F | H | H | H | I | H | nonafluoro-2-butyl | H | CF3 |
| 9-2615 | phenyl | C(O)Me | CH2(3-Py-N-oxide) | F | H | H | H | I | H | nonafluoro-2-butyl | H | CF3 |
| 9-2616 | phenyl | S(O)2Me | Et | F | H | H | H | I | H | nonafluoro-2-butyl | H | CF3 |
| 9-2617 | phenyl | S(O)2Me | n-Pr | F | H | H | H | I | H | nonafluoro-2-butyl | H | CF3 |
| 9-2618 | phenyl | S(O)2Me | i-Pr | F | H | H | H | I | H | nonafluoro-2-butyl | H | CF3 |
| 9-2619 | phenyl | S(O)2Me | CH2CH=CH2 | F | H | H | H | I | H | nonafluoro-2-butyl | H | CF3 |
| 9-2620 | phenyl | S(O)2Me | CN | F | H | H | H | I | H | nonafluoro-2-butyl | H | CF3 |
| 9-2621 | phenyl | S(O)2Me | CH2C≡CH | F | H | H | H | I | H | nonafluoro-2-butvl | H | CF3 |
| 9-2622 | phenyl | S(O)2Me | NH2 | F | H | H | H | I | H | nonafluoro-2-butyl | H | CF3 |
| 9-2623 | phenyl | S(O)2Me | C(O)OMe | F | H | H | H | I | H | nonafluoro-2-butyl | H | CF3 |
| 9-2624 | phenyl | S(O)2Me | C(O)C(O)OMe | F | H | H | H | I | H | nonafluoro-2-butyl | H | CF3 |
| 9-2625 | phenyl | S(O)2Me | C(O)Me | F | H | H | H | I | H | nonafluoro-2-butyl | H | CF3 |
| 9-2626 | phenyl | S(O)2Me | S(O)2Me | F | H | H | H | I | H | nonafluoro-2-butyl | H | CF3 |
| 9-2627 | phenyl | S(O)2Me | CH2Ph | F | H | H | H | I | H | nonafluoro-2-butyl | H | CF3 |

**Table 9(6)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 9-2628 | phenyl | S(O)2Me | CH2(3-Py) | F | H | H | H | I | H | nonafluoro-2-butyl | H | CF3 |
| 9-2629 | phenyl | S(O)2Me | CH2(3-Py-N-oxide) | F | H | H | H | I | H | nonafluoro-2-butyl | H | CF3 |
| 9-2630 | phenyl | CH2Ph | Et | F | H | H | H | I | H | nonafluoro-2-butyl | H | CF3 |
| 9-2631 | phenyl | CH2Ph | n-Pr | F | H | H | H | I | H | nonafluoro-2-butyl | H | CF3 |
| 9-2632 | phenyl | CH2Ph | i-Pr | F | H | H | H | I | H | nonafluoro-2-butyl | H | CF3 |
| 9-2633 | phenyl | CH2Ph | CH2CH=CH2 | F | H | H | H | I | H | nonafluoro-2-butyl | H | CF3 |
| 9-2634 | phenyl | CH2Ph | CN | F | H | H | H | I | H | nonafluoro-2-butyl | H | CF3 |
| 9-2635 | phenyl | CH2Ph | CH2C≡CH | F | H | H | H | I | H | nonafluoro-2-butyl | H | CF3 |
| 9-2636 | phenyl | CH2Ph | NH2 | F | H | H | H | I | H | nonafluoro-2-butyl | H | CF3 |
| 9-2637 | phenyl | CH2Ph | C(O)OMe | F | H | H | H | I | H | nonafluoro-2-butyl | H | CF3 |
| 9-2638 | phenyl | CH2Ph | C(O)C(O)OMe | F | H | H | H | I | H | nonafluoro-2-butyl | H | CF3 |
| 9-2639 | phenyl | CH2Ph | C(O)Me | F | H | H | H | I | H | nonafluoro-2-butyl | H | CF3 |
| 9-2640 | phenyl | CH2Ph | S(O)2Me | F | H | H | H | I | H | nonafluoro-2-butyl | H | CF3 |
| 9-2641 | phenyl | CH2Ph | CH2Ph | F | H | H | H | I | H | nonafluoro-2-butyl | H | CF3 |
| 9-2642 | phenyl | CH2Ph | CH2(3-Py) | F | H | H | H | I | H | nonafluoro-2-butyl | H | CF3 |
| 9-2643 | phenyl | CH2Ph | CH2(3-Py-N-oxide) | F | H | H | H | I | H | nonafluoro-2-butyl | H | CF3 |
| 9-2644 | phenyl | CH2(3-Py) | Et | F | H | H | H | I | H | nonafluoro-2-butyl | H | CF3 |
| 9-2645 | phenyl | CH2(3-Py) | n-Pr | F | H | H | H | I | H | nonafluoro-2-butyl | H | CF3 |
| 9-2646 | phenyl | CH2(3-Py) | i-Pr | F | H | H | H | I | H | nonafluoro-2-butyl | H | CF3 |
| 9-2647 | phenyl | CH2(3-Py) | CH2CH=CH2 | F | H | H | H | I | H | nonafluoro-2-butyl | H | CF3 |
| 9-2648 | phenyl | CH2(3-Py) | CN | F | H | H | H | I | H | nonafluoro-2-butyl | H | CF3 |
| 9-2649 | phenyl | CH2(3-Py) | CH2C≡CH | F | H | H | H | I | H | nonafluoro-2-butyl | H | CF3 |
| 9-2650 | phenyl | CH2(3-Py) | NH2 | F | H | H | H | I | H | nonafluoro-2-butyl | H | CF3 |
| 9-2651 | phenyl | CH2(3-Py) | C(O)OMe | F | H | H | H | I | H | nonafluoro-2-butyl | H | CF3 |
| 9-2652 | phenyl | CH2(3-Py) | C(O)C(O)OMe | F | H | H | H | I | H | nonafluoro-2-butyl | H | CF3 |
| 9-2653 | phenyl | CH2(3-Py) | C(O)Me | F | H | H | H | I | H | nonafluoro-2-butyl | H | CF3 |
| 9-2654 | phenyl | CH2(3-Py) | S(O)2Me | F | H | H | H | I | H | nonafluoro-2-butyl | H | CF3 |
| 9-2655 | phenyl | CH2(3-Py) | CH2Ph | F | H | H | H | I | H | nonafluoro-2-butyl | H | CF3 |
| 9-2656 | phenyl | CH2(3-Py) | CH2(3-Py) | F | H | H | H | I | H | nonafluoro-2-butyl | H | CF3 |
| 9-2657 | phenyl | CH2(3-Py) | CH2(3-Py-N-oxide) | F | H | H | H | I | H | nonafluoro-2-butyl | H | CF3 |
| 9-2658 | phenyl | CH2(3-Py-N-oxide) | Et | F | H | H | H | I | H | nonafluoro-2-butyl | H | CF3 |
| 9-2659 | phenyl | CH2(3-Py-N-oxide) | n-Pr | F | H | H | H | I | H | nonafluoro-2-butyl | H | CF3 |
| 9-2660 | phenyl | CH2(3-Py-N-oxide) | i-Pr | F | H | H | H | I | H | nonafluoro-2-butyl | H | CF3 |
| 9-2661 | phenyl | CH2(3-Py-N-oxide) | CH2CH=CH2 | F | H | H | H | I | H | nonafluoro-2-butyl | H | CF3 |
| 9-2662 | phenyl | CH2(3-Py-N-oxide) | CN | F | H | H | H | I | H | nonafluoro-2-butyl | H | CF3 |
| 9-2663 | phenyl | CH2(3-Py-N-oxide) | CH2C≡CH | F | H | H | H | I | H | nonafluoro-2-butyl | H | CF3 |
| 9-2664 | phenyl | CH2(3-Py-N-oxide) | NH2 | F | H | H | H | I | H | nonafluoro-2-butyl | H | CF3 |
| 9-2665 | phenyl | CH2(3-Py-N-oxide) | C(O)OMe | F | H | H | H | I | H | nonafluoro-2-butyl | H | CF3 |
| 9-2666 | phenyl | CH2(3-Py-N-oxide) | C(O)C(O)OMe | F | H | H | H | I | H | nonafluoro-2-butyl | H | CF3 |
| 9-2667 | phenyl | CH2(3-Py-N-oxide) | C(O)Me | F | H | H | H | I | H | nonafluoro-2-butyl | H | CF3 |
| 9-2668 | phenyl | CH2(3-Py-N-oxide) | S(O)2Me | F | H | H | H | I | H | nonafluoro-2-butyl | H | CF3 |
| 9-2669 | phenyl | CH2(3-Py-N-oxide) | CH2Ph | F | H | H | H | I | H | nonafluoro-2-butyl | H | CF3 |
| 9-2670 | phenyl | CH2(3-Py-N-oxide) | CH2(3-Py) | F | H | H | H | I | H | nonafluoro-2-butyl | H | CF3 |
| 9-2671 | phenyl | CH2(3-Py-N-oxide) | CH2(3-Py-N-oxide) | F | H | H | H | I | H | nonafluoro-2-butyl | H | CF3 |
| 9-2672 | phenyl | Me | Me | F | H | H | H | OCF3 | H | heptafluoroisopropyl | H | OCF3 |
| 9-2687 | 3-cyanophenyl | Me | Me | F | H | H | H | OCF3 | H | heptafluoroisopropyl | H | OCF3 |
| 9-2688 | 4-cyanophenyl | Me | Me | F | H | H | H | OCF3 | H | heptafluoroisopropyl | H | OCF3 |
| 9-2703 | 2-chloropyridin-3-yl | Me | Me | F | H | H | H | OCF3 | H | heptafluoroisopropyl | H | OCF3 |
| 9-2728 | phenyl | Me | Me | F | H | H | H | OCF3 | H | nonafluoro-2-butyl | H | OCF3 |
| 9-2743 | 3-cyanophenyl | Me | Me | F | H | H | H | OCF3 | H | nonafluoro-2-butyl | H | OCF3 |
| 9-2744 | 4-cyanophenyl | Me | Me | F | H | H | H | OCF3 | H | nonafluoro-2-butyl | H | OCF3 |
| 9-2759 | 2-chloropyridin-3-yl | Me | Me | F | H | H | H | OCF3 | H | nonafluoro-2-butyl | H | OCF3 |
| 9-2784 | phenyl | Me | Me | F | H | H | H | CF3 | H | heptafluoroisopropyl | H | OCF3 |
| 9-2799 | 3-cyanophenyl | Me | Me | F | H | H | H | CF3 | H | heptafluoroisopropyl | H | OCF3 |
| 9-2800 | 4-cyanophenyl | Me | Me | F | H | H | H | CF3 | H | heptafluoroisopropyl | H | OCF3 |
| 9-2815 | 2-chloropyridin-3-yl | Me | Me | F | H | H | H | CF3 | H | heptafluoroisopropyl | H | OCF3 |
| 9-2840 | phenyl | Me | Me | F | H | H | H | CF3 | H | nonafluoro-2-butyl | H | OCF3 |
| 9-2855 | 3-cyanophenyl | Me | Me | F | H | H | H | CF3 | H | nonafluoro-2-butyl | H | OCF3 |
| 9-2856 | 4-cyanophenyl | Me | Me | F | H | H | H | CF3 | H | nonafluoro-2-butyl | H | OCF3 |

**Table 9(7)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 9-2871 | 2-chloropyridin-3-yl | Me | Me | F | H | H | H | CF3 | H | nonafluoro-2-butyl | H | OCF3 |
| 9-2896 | phenyl | Me | Me | F | H | H | H | CF3 | H | heptafluoroisopropyl | H | CF3 |
| 9-2916 | 3-cyanophenyl | Me | Me | F | H | H | H | CF3 | H | heptafluoroisopropyl | H | CF3 |
| 9-2917 | 4-cyanophenyl | Me | Me | F | H | H | H | CF3 | H | heptafluoroisopropyl | H | CF3 |
| 9-2932 | 2-chloropyridin-3-yl | Me | Me | F | H | H | H | CF3 | H | heptafluoroisopropyl | H | CF3 |
| 9-2964 | phenyl | Me | Me | F | H | H | H | CF3 | H | nonafluoro-2-butyl | H | CF3 |
| 9-2984 | 3-cyanophenyl | Me | Me | F | H | H | H | CF3 | H | nonafluoro-2-butyl | H | CF3 |
| 9-2985 | 4-cyanophenyl | Me | Me | F | H | H | H | CF3 | H | nonafluoro-2-butyl | H | CF3 |
| 9-3000 | 2-chloropyridin-3-yl | Me | Me | F | H | H | H | CF3 | H | nonafluoro-2-butyl | H | CF3 |
| 9-3228 | phenyl | Me | Me | H | F | H | H | F | H | heptafluoroisopropyl | H | F |
| 9-3229 | 3-cyanophenyl | Me | Me | H | F | H | H | F | H | heptafluoroisopropyl | H | F |
| 9-3230 | 4-cyanophenyl | Me | Me | H | F | H | H | F | H | heptafluoroisopropyl | H | F |
| 9-3231 | 2-chloropyridin-3-yl | Me | Me | H | F | H | H | F | H | heptafluoroisopropyl | H | F |
| 9-3232 | phenyl | Me | Me | H | F | H | H | F | H | nonafluoro-2-butyl | H | F |
| 9-3233 | 3-cyanophenyl | Me | Me | H | F | H | H | F | H | nonafluoro-2-butyl | H | F |
| 9-3234 | 4-cyanophenyl | Me | Me | H | F | H | H | F | H | nonafluoro-2-butyl | H | F |
| 9-3235 | 2-chloropyridin-3-yl | Me | Me | H | F | H | H | F | H | nonafluoro-2-butyl | H | F |
| 9-3236 | phenyl | Me | Me | H | F | H | H | Cl | H | heptafluoroisopropyl | H | Cl |
| 9-3256 | 3-cyanophenyl | Me | Me | H | F | H | H | Cl | H | heptafluoroisopropyl | H | Cl |
| 9-3257 | 4-cyanophenyl | Me | Me | H | F | H | H | Cl | H | heptafluoroisopropyl | H | Cl |
| 9-3272 | 2-chloropyridin-3-yl | Me | Me | H | F | H | H | Cl | H | heptafluoroisopropyl | H | Cl |
| 9-3314 | phenyl | Me | Me | H | F | H | H | Cl | H | nonafluoro-2-butyl | H | Cl |
| 9-3334 | 3-cyanophenyl | Me | Me | H | F | H | H | Cl | H | nonafluoro-2-butyl | H | Cl |
| 9-3335 | 4-cyanophenyl | Me | Me | H | F | H | H | Cl | H | nonafluoro-2-butyl | H | Cl |
| 9-3350 | 2-chloropyridin-3-yl | Me | Me | H | F | H | H | Cl | H | nonafluoro-2-butyl | H | Cl |
| 9-3392 | phenyl | Me | Me | H | F | H | H | Br | H | heptafluoroisopropyl | H | Br |
| 9-3412 | 3-cyanophenyl | Me | Me | H | F | H | H | Br | H | heptafluoroisopropyl | H | Br |
| 9-3413 | 4-cyanophenyl | Me | Me | H | F | H | H | Br | H | heptafluoroisopropyl | H | Br |
| 9-3428 | 2-chloropyridin-3-yl | Me | Me | H | F | H | H | Br | H | heptafluoroisopropyl | H | Br |
| 9-3470 | phenyl | Me | Me | H | F | H | H | Br | H | nonafluoro-2-butyl | H | Br |
| 9-3490 | 3-cyanophenyl | Me | Me | H | F | H | H | Br | H | nonafluoro-2-butyl | H | Br |
| 9-3491 | 4-cyanophenyl | Me | Me | H | F | H | H | Br | H | nonafluoro-2-butyl | H | Br |
| 9-3506 | 2-chloropyridin-3-yl | Me | Me | H | F | H | H | Br | H | nonafluoro-2-butyl | H | Br |
| 9-3548 | phenyl | Me | Me | H | F | H | H | I | H | heptafluoroisopropyl | H | I |
| 9-3568 | 3-cyanophenyl | Me | Me | H | F | H | H | I | H | heptafluoroisopropyl | H | I |
| 9-3569 | 4-cyanophenyl | Me | Me | H | F | H | H | I | H | heptafluoroisopropyl | H | I |
| 9-3584 | 2-chloropyridin-3-yl | Me | Me | H | F | H | H | I | H | heptafluoroisopropyl | H | I |
| 9-3626 | phenyl | Me | Me | H | F | H | H | I | H | nonafluoro-2-butyl | H | I |
| 9-3646 | 3-cyanophenyl | Me | Me | H | F | H | H | I | H | nonafluoro-2-butyl | H | I |
| 9-3647 | 4-cyanophenyl | Me | Me | H | F | H | H | I | H | nonafluoro-2-butyl | H | I |
| 9-3662 | 2-chloropyridin-3-yl | Me | Me | H | F | H | H | I | H | nonafluoro-2-butyl | H | I |
| 9-3900 | phenyl | Me | Me | H | F | H | H | Cl | H | heptafluoroisopropyl | H | OCF3 |
| 9-3920 | 3-cyanophenyl | Me | Me | H | F | H | H | Cl | H | heptafluoroisopropyl | H | OCF3 |
| 9-3921 | 4-cyanophenyl | Me | Me | H | F | H | H | Cl | H | heptafluoroisopropyl | H | OCF3 |
| 9-3936 | 2-chloropyridin-3-yl | Me | Me | H | F | H | H | Cl | H | heptafluoroisopropyl | H | OCF3 |
| 9-3978 | phenyl | Me | Me | H | F | H | H | Cl | H | nonafluoro-2-butyl | H | OCF3 |
| 9-3998 | 3-cyanophenyl | Me | Me | H | F | H | H | Cl | H | nonafluoro-2-butyl | H | OCF3 |
| 9-3999 | 4-cyanophenyl | Me | Me | H | F | H | H | Cl | H | nonafluoro-2-butyl | H | OCF3 |
| 9-4014 | 2-chloropyridin-3-yl | Me | Me | H | F | H | H | Cl | H | nonafluoro-2-butyl | H | OCF3 |
| 9-4056 | phenyl | Me | Me | H | F | H | H | Br | H | heptafluoroisopropyl | H | OCF3 |
| 9-4076 | 3-cyanophenyl | Me | Me | H | F | H | H | Br | H | heptafluoroisopropyl | H | OCF3 |
| 9-4077 | 4-cyanophenyl | Me | Me | H | F | H | H | Br | H | heptafluoroisopropyl | H | OCF3 |
| 9-4092 | 2-chloropyridin-3-yl | Me | Me | H | F | H | H | Br | H | heptafluoroisopropyl | H | OCF3 |
| 9-4134 | phenyl | Me | Me | H | F | H | H | Br | H | nonafluoro-2-butyl | H | OCF3 |
| 9-4154 | 3-cyanophenyl | Me | Me | H | F | H | H | Br | H | nonafluoro-2-butyl | H | OCF3 |
| 9-4155 | 4-cyanophenyl | Me | Me | H | F | H | H | Br | H | nonafluoro-2-butyl | H | OCF3 |
| 9-4170 | 2-chloropyridin-3-yl | Me | Me | H | F | H | H | Br | H | nonafluoro-2-butyl | H | OCF3 |
| 9-4212 | phenyl | Me | Me | H | F | H | H | I | H | heptafluoroisopropyl | H | OCF3 |
| 9-4232 | 3-cyanophenyl | Me | Me | H | F | H | H | I | H | heptafluoroisopropyl | H | OCF3 |
| 9-4233 | 4-cyanophenyl | Me | Me | H | F | H | H | I | H | heptafluoroisopropyl | H | OCF3 |
| 9-4248 | 2-chloropyridin-3-yl | Me | Me | H | F | H | H | I | H | heptafluoroisopropyl | H | OCF3 |
| 9-4290 | phenyl | Me | Me | H | F | H | H | I | H | nonafluoro-2-butvl | H | OCF3 |
| 9-4310 | 3-cyanophenyl | Me | Me | H | F | H | H | I | H | nonafluoro-2-butyl | H | OCF3 |
| 9-4311 | 4-cyanophenyl | Me | Me | H | F | H | H | I | H | nonafluoro-2-butyl | H | OCF3 |
| 9-4326 | 2-chloropyridin-3-yl | Me | Me | H | F | H | H | I | H | nonafluoro-2-butyl | H | OCF3 |
| 9-4564 | phenyl | Me | Me | H | F | H | H | F | H | heptafluoroisopropyl | H | CF3 |
| 9-4565 | phenyl | Me | Me | H | F | H | H | Cl | H | heptafluoroisopropyl | H | CF3 |
| 9-4585 | 3-cyanophenyl | Me | Me | H | F | H | H | Cl | H | heptafluoroisopropyl | H | CF3 |
| 9-4586 | 4-cyanophenyl | Me | Me | H | F | H | H | Cl | H | heptafluoroisopropyl | H | CF3 |

**Table 9(8)**

| compound u 6er | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 9-4601 | 2-chloropyridin-3-yl | Me | Me | H | F | H | H | Cl | H | heptafluoroisopropyl | H | CF3 |
| 9-4643 | phenyl | Me | Me | H | F | H | H | F | H | nonafluoro-2-butyl | H | CF3 |
| 9-4644 | phenyl | Me | Me | H | F | H | H | Cl | H | nonafluoro-2-butyl | H | CF3 |
| 9-4664 | 3-cyanophenyl | Me | Me | H | F | H | H | Cl | H | nonafluoro-2-butyl | H | CF3 |
| 9-4665 | 4-cyanophenyl | Me | Me | H | F | H | H | Cl | H | nonafluoro-2-butyl | H | CF3 |
| 9-4680 | 2-chloropyridin-3-yl | Me | Me | H | F | H | H | Cl | H | nonafluoro-2-butyl | H | CF3 |
| 9-4722 | phenyl | Me | Me | H | F | H | H | Br | H | heptafluoroisopropyl | H | CF3 |
| 9-4742 | 3-cyanophenyl | Me | Me | H | F | H | H | Br | H | heptafluoroisopropyl | H | CF3 |
| 9-4743 | 4-cyanophenyl | Me | Me | H | F | H | H | Br | H | heptafluoroisopropyl | H | CF3 |
| 9-4758 | 2-chloropyridin-3-yl | Me | Me | H | F | H | H | Br | H | heptafluoroisopropyl | H | CF3 |
| 9-4800 | phenyl | Me | Me | H | F | H | H | Br | H | nonafluoro-2-butyl | H | CF3 |
| 9-4820 | 3-cyanophenyl | Me | Me | H | F | H | H | Br | H | nonafluoro-2-butyl | H | CF3 |
| 9-4821 | 4-cyanophenyl | Me | Me | H | F | H | H | Br | H | nonafluoro-2-butyl | H | CF3 |
| 9-4836 | 2-chloropyridin-3-yl | Me | Me | H | F | H | H | Br | H | nonafluoro-2-butyl | H | CF3 |
| 9-4878 | phenyl | Me | Me | H | F | H | H | I | H | heptafluoroisopropyl | H | CF3 |
| 9-4898 | 3-cyanophenyl | Me | Me | H | F | H | H | I | H | heptafluoroisopropyl | H | CF3 |
| 9-4899 | 4-cyanophenyl | Me | Me | H | F | H | H | I | H | heptafluoroisopropyl | H | CF3 |
| 9-4914 | 2-chloropyridin-3-yl | Me | Me | H | F | H | H | I | H | heptafluoroisopropyl | H | CF3 |
| 9-4956 | phenyl | Me | Me | H | F | H | H | I | H | nonafluoro-2-butyl | H | CF3 |
| 9-4976 | 3-cyanophenyl | Me | Me | H | F | H | H | I | H | nonafluoro-2-butyl | H | CF3 |
| 9-4977 | 4-cyanophenyl | Me | Me | H | F | H | H | I | H | nonafluoro-2-butyl | H | CF3 |
| 9-4992 | 2-chloropyridin-3-yl | Me | Me | H | F | H | H | I | H | nonafluoro-2-butyl | H | CF3 |
| 9-5230 | phenyl | Me | Me | H | F | H | H | OCF3 | H | heptafluoroisopropyl | H | OCF3 |
| 9-5261 | 2-chloropyridin-3-yl | Me | Me | H | F | H | H | OCF3 | H | heptafluoroisopropyl | H | OCF3 |
| 9-5286 | phenyl | Me | Me | H | F | H | H | OCF3 | H | nonafluoro-2-butyl | H | OCF3 |
| 9-5317 | 2-chloropyridin-3-yl | Me | Me | H | F | H | H | OCF3 | H | nonafluoro-2-butyl | H | OCF3 |
| 9-5342 | phenyl | Me | Me | H | F | H | H | CF3 | H | heptafluoroisopropyl | H | OCF3 |
| 9-5373 | 2-chloropyridin-3-yl | Me | Me | H | F | H | H | CF3 | H | heptafluoroisopropyl | H | OCF3 |
| 9-5398 | phenyl | Me | Me | H | F | H | H | CF3 | H | nonafluoro-2-butyl | H | OCF3 |
| 9-5429 | 2-chloropyridin-3-yl | Me | Me | H | F | H | H | CF3 | H | nonafluoro-2-butyl | H | OCF3 |
| 9-5454 | phenyl | Me | Me | H | F | H | H | CF3 | H | heptafluoroisopropyl | H | CF3 |
| 9-5474 | 3-cyanophenyl | Me | Me | H | F | H | H | CF3 | H | heptafluoroisopropyl | H | CF3 |
| 9-5475 | 4-cyanophenyl | Me | Me | H | F | H | H | CF3 | H | heptafluoroisopropyl | H | CF3 |
| 9-5490 | 2-chloropyridin-3-yl | Me | Me | H | F | H | H | CF3 | H | heptafluoroisopropyl | H | CF3 |
| 9-5522 | phenyl | Me | Me | H | F | H | H | CF3 | H | nonafluoro-2-butyl | H | CF3 |
| 9-5542 | 3-cyanophenyl | Me | Me | H | F | H | H | CF3 | H | nonafluoro-2-butyl | H | CF3 |
| 9-5543 | 4-cyanophenyl | Me | Me | H | F | H | H | CF3 | H | nonafluoro-2-butyl | H | CF3 |
| 9-5558 | 2-chloropyridin-3-yl | Me | Me | H | F | H | H | CF3 | H | nonafluoro-2-butyl | H | CF3 |
| 9-5786 | phenyl | Me | Me | F | F | H | H | F | H | heptafluoroisopropyl | H | F |
| 9-5787 | 3-cyanophenyl | Me | Me | F | F | H | H | F | H | heptafluoroisopropyl | H | F |
| 9-5788 | 4-cyanophenyl | Me | Me | F | F | H | H | F | H | heptafluoroisopropyl | H | F |
| 9-5789 | 2-chloropyridin-3-yl | Me | Me | F | F | H | H | F | H | heptafluoroisopropyl | H | F |
| 9-5790 | phenyl | Me | Me | F | F | H | H | F | H | nonafluoro-2-butyl | H | F |
| 9-5791 | 3-cyanophenyl | Me | Me | F | F | H | H | F | H | nonafluoro-2-butyl | H | F |
| 9-5792 | 4-cyanophenyl | Me | Me | F | F | H | H | F | H | nonafluoro-2-butyl | H | F |
| 9-5793 | 2-chloropyridin-3-yl | Me | Me | F | F | H | H | F | H | nonafluoro-2-butyl | H | F |
| 9-5794 | phenyl | Me | Me | F | F | H | H | Cl | H | heptafluoroisopropyl | H | Cl |
| 9-5814 | 3-cyanophenyl | Me | Me | F | F | H | H | Cl | H | heptafluoroisopropyl | H | Cl |
| 9-5815 | 4-cyanophenyl | Me | Me | F | F | H | H | Cl | H | heptafluoroisopropyl | H | Cl |
| 9-5830 | 2-chloropyridin-3-yl | Me | Me | F | F | H | H | Cl | H | heptafluoroisopropyl | H | Cl |
| 9-5872 | phenyl | Me | Me | F | F | H | H | Cl | H | nonafluoro-2-butyl | H | Cl |
| 9-5892 | 3-cyanophenyl | Me | Me | F | F | H | H | Cl | H | nonafluoro-2-butyl | H | Cl |
| 9-5893 | 4-cyanophenyl | Me | Me | F | F | H | H | Cl | H | nonafluoro-2-butyl | H | Cl |
| 9-5908 | 2-chloropyridin-3-yl | Me | Me | F | F | H | H | Cl | H | nonafluoro-2-butyl | H | Cl |
| 9-5950 | phenyl | Me | Me | F | F | H | H | Br | H | heptafluoroisopropyl | H | Br |
| 9-5970 | 3-cyanophenyl | Me | Me | F | F | H | H | Br | H | heptafluoroisopropyl | H | Br |
| 9-5971 | 4-cyanophenyl | Me | Me | F | F | H | H | Br | H | heptafluoroisopropyl | H | Br |
| 9-5986 | 2-chloropyridin-3-yl | Me | Me | F | F | H | H | Br | H | heptafluoroisopropyl | H | Br |
| 9-6028 | phenyl | Me | Me | F | F | H | H | Br | H | nonafluoro-2-butyl | H | Br |
| 9-6048 | 3-cyanophenyl | Me | Me | F | F | H | H | Br | H | nonafluoro-2-butyl | H | Br |
| 9-6049 | 4-cyanophenyl | Me | Me | F | F | H | H | Br | H | nonafluoro-2-butyl | H | Br |
| 9-6064 | 2-chloropyridin-3-yl | Me | Me | F | F | H | H | Br | H | nonafluoro-2-butvl | H | Br |
| 9-6106 | phenyl | Me | Me | F | F | H | H | I | H | heptafluoroisopropyl | H | I |
| 9-6126 | 3-cyanophenyl | Me | Me | F | F | H | H | I | H | heptafluoroisopropyl | H | I |

**Table 9(9)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 9-6127 | 4-cyanophenyl | Me | Me | F | F | H | H | I | H | heptafluoroisopropyl | H | I |
| 9-6142 | 2-chloropyridin-3-yl | Me | Me | F | F | H | H | I | H | heptafluoroisopropyl | H | I |
| 9-6184 | phenyl | Me | Me | F | F | H | H | I | H | nonafluoro-2-butyl | H | I |
| 9-6204 | 3-cyanophenyl | Me | Me | F | F | H | H | I | H | nonafluoro-2-butyl | H | I |
| 9-6205 | 4-cyanophenyl | Me | Me | F | F | H | H | I | H | nonafluoro-2-butyl | H | I |
| 9-6220 | 2-chloropyridin-3-yl | Me | Me | F | F | H | H | I | H | nonafluoro-2-butyl | H | I |
| 9-6458 | phenyl | Me | Me | F | F | H | H | Cl | H | heptafluoroisopropyl | H | OCF3 |
| 9-6478 | 3-cyanophenyl | Me | Me | F | F | H | H | Cl | H | heptafluoroisopropyl | H | OCF3 |
| 9-6479 | 4-cyanophenyl | Me | Me | F | F | H | H | Cl | H | heptafluoroisopropyl | H | OCF3 |
| 9-6494 | 2-chloropyridin-3-yl | Me | Me | F | F | H | H | Cl | H | heptafluoroisopropyl | H | OCF3 |
| 9-6536 | phenyl | Me | Me | F | F | H | H | Cl | H | nonafluoro-2-butyl | H | OCF3 |
| 9-6556 | 3-cyanophenyl | Me | Me | F | F | H | H | Cl | H | nonafluoro-2-butyl | H | OCF3 |
| 9-6557 | 4-cyanophenyl | Me | Me | F | F | H | H | Cl | H | nonafluoro-2-butyl | H | OCF3 |
| 9-6572 | 2-chloropyridin-3-yl | Me | Me | F | F | H | H | Cl | H | nonafluoro-2-butyl | H | OCF3 |
| 9-6614 | phenyl | Me | Me | F | F | H | H | Br | H | heptafluoroisopropyl | H | OCF3 |
| 9-6634 | 3-cyanophenyl | Me | Me | F | F | H | H | Br | H | heptafluoroisopropyl | H | OCF3 |
| 9-6635 | 4-cyanophenyl | Me | Me | F | F | H | H | Br | H | heptafluoroisopropyl | H | OCF3 |
| 9-6650 | 2-chloropyridin-3-yl | Me | Me | F | F | H | H | Br | H | heptafluoroisopropyl | H | OCF3 |
| 9-6692 | phenyl | Me | Me | F | F | H | H | Br | H | nonafluoro-2-butyl | H | OCF3 |
| 9-6712 | 3-cyanophenyl | Me | Me | F | F | H | H | Br | H | nonafluoro-2-butyl | H | OCF3 |
| 9-6713 | 4-cyanophenyl | Me | Me | F | F | H | H | Br | H | nonafluoro-2-butyl | H | OCF3 |
| 9-6728 | 2-chloropyridin-3-yl | Me | Me | F | F | H | H | Br | H | nonafluoro-2-butyl | H | OCF3 |
| 9-6770 | phenyl | Me | Me | F | F | H | H | I | H | heptafluoroisopropyl | H | OCF3 |
| 9-6790 | 3-cyanophenyl | Me | Me | F | F | H | H | I | H | heptafluoroisopropyl | H | OCF3 |
| 9-6791 | 4-cyanophenyl | Me | Me | F | F | H | H | I | H | heptafluoroisopropyl | H | OCF3 |
| 9-6806 | 2-chloropyridin-3-yl | Me | Me | F | F | H | H | I | H | heptafluoroisopropyl | H | OCF3 |
| 9-6848 | phenyl | Me | Me | F | F | H | H | I | H | nonafluoro-2-butyl | H | OCF3 |
| 9-6868 | 3-cyanophenyl | Me | Me | F | F | H | H | I | H | nonafluoro-2-butyl | H | OCF3 |
| 9-6869 | 4-cyanophenyl | Me | Me | F | F | H | H | I | H | nonafluoro-2-butyl | H | OCF3 |
| 9-6884 | 2-chloropyridin-3-yl | Me | Me | F | F | H | H | I | H | nonafluoro-2-butyl | H | OCF3 |
| 9-7122 | phenyl | Me | Me | F | F | H | H | F | H | heptafluoroisopropyl | H | CF3 |
| 9-7123 | phenyl | Me | Me | F | F | H | H | Cl | H | heptafluoroisopropyl | H | CF3 |
| 9-7143 | 3-cyanophenyl | Me | Me | F | F | H | H | Cl | H | heptafluoroisopropyl | H | CF3 |
| 9-7144 | 4-cyanophenyl | Me | Me | F | F | H | H | Cl | H | heptafluoroisopropyl | H | CF3 |
| 9-7159 | 2-chloropyridin-3-yl | Me | Me | F | F | H | H | Cl | H | heptafluoroisopropyl | H | CF3 |
| 9-7201 | phenyl | Me | Me | F | F | H | H | F | H | nonafluoro-2-butyl | H | CF3 |
| 9-7202 | phenyl | Me | Me | F | F | H | H | Cl | H | nonafluoro-2-butyl | H | CF3 |
| 9-7222 | 3-cyanophenyl | Me | Me | F | F | H | H | Cl | H | nonafluoro-2-butyl | H | CF3 |
| 9-7223 | 4-cyanophenyl | Me | Me | F | F | H | H | Cl | H | nonafluoro-2-butyl | H | CF3 |
| 9-7238 | 2-chloropyridin-3-yl | Me | Me | F | F | H | H | Cl | H | nonafluoro-2-butyl | H | CF3 |
| 9-7280 | phenyl | Me | Me | F | F | H | H | Br | H | heptafluoroisopropyl | H | CF3 |
| 9-7300 | 3-cyanophenyl | Me | Me | F | F | H | H | Br | H | heptafluoroisopropyl | H | CF3 |
| 9-7301 | 4-cyanophenyl | Me | Me | F | F | H | H | Br | H | heptafluoroisopropyl | H | CF3 |
| 9-7316 | 2-chloropyridin-3-yl | Me | Me | F | F | H | H | Br | H | heptafluoroisopropyl | H | CF3 |
| 9-7358 | phenyl | Me | Me | F | F | H | H | Br | H | nonafluoro-2-butyl | H | CF3 |
| 9-7378 | 3-cyanophenyl | Me | Me | F | F | H | H | Br | H | nonafluoro-2-butyl | H | CF3 |
| 9-7379 | 4-cyanophenyl | Me | Me | F | F | H | H | Br | H | nonafluoro-2-butyl | H | CF3 |
| 9-7394 | 2-chloropyridin-3-yl | Me | Me | F | F | H | H | Br | H | nonafluoro-2-butyl | H | CF3 |
| 9-7436 | phenyl | Me | Me | F | F | H | H | I | H | heptafluoroisopropyl | H | CF3 |
| 9-7456 | 3-cyanophenyl | Me | Me | F | F | H | H | I | H | heptafluoroisopropyl | H | CF3 |
| 9-7457 | 4-cyanophenyl | Me | Me | F | F | H | H | I | H | heptafluoroisopropyl | H | CF3 |
| 9-7472 | 2-chloropyridin-3-yl | Me | Me | F | F | H | H | I | H | heptafluoroisopropyl | H | CF3 |
| 9-7514 | phenyl | Me | Me | F | F | H | H | I | H | nonafluoro-2-butyl | H | CF3 |
| 9-7534 | 3-cyanophenyl | Me | Me | F | F | H | H | I | H | nonafluoro-2-butyl | H | CF3 |
| 9-7535 | 4-cyanophenyl | Me | Me | F | F | H | H | I | H | nonafluoro-2-butyl | H | CF3 |
| 9-7550 | 2-chloropyridin-3-yl | Me | Me | F | F | H | H | I | H | nonafluoro-2-butyl | H | CF3 |
| 9-7788 | phenyl | Me | Me | F | F | H | H | OCF3 | H | heptafluoroisopropyl | H | OCF3 |
| 9-7819 | 2-chloropyridin-3-yl | Me | Me | F | F | H | H | OCF3 | H | heptafluoroisopropyl | H | OCF3 |
| 9-7844 | phenyl | Me | Me | F | F | H | H | OCF3 | H | nonafluoro-2-butyl | H | OCF3 |
| 9-7875 | 2-chloropyridin-3-yl | Me | Me | F | F | H | H | OCF3 | H | nonafluoro-2-butyl | H | OCF3 |
| 9-7900 | phenyl | Me | Me | F | F | H | H | CF3 | H | heptafluoroisopropyl | H | OCF3 |
| 9-7931 | 2-chloropyridin-3-yl | Me | Me | F | F | H | H | CF3 | H | heptafluoroisopropyl | H | OCF3 |
| 9-7956 | phenyl | Me | Me | F | F | H | H | CF3 | H | nonafluoro-2-butyl | H | OCF3 |
| 9-7987 | 2-chloropyridin-3-yl | Me | Me | F | F | H | H | CF3 | H | nonafluoro-2-butyl | H | OCF3 |

**Table 9(10)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 9-8012 | phenyl | Me | Me | F | F | H | H | CF3 | H | heptafluoroisopropyl | H | CF3 |
| 9-8032 | 3-cyanophenyl | Me | Me | F | F | H | H | CF3 | H | heptafluoroisopropyl | H | CF3 |
| 9-8033 | 4-cyanophenyl | Me | Me | F | F | H | H | CF3 | H | heptafluoroisopropyl | H | CF3 |
| 9-8048 | 2-chloropyridin-3-yl | Me | Me | F | F | H | H | CF3 | H | heptafluoroisopropyl | H | CF3 |
| 9-8080 | phenyl | Me | Me | F | F | H | H | CF3 | H | nonafluoro-2-butyl | H | CF3 |
| 9-8100 | 3-cyanophenyl | Me | Me | F | F | H | H | CF3 | H | nonafluoro-2-butyl | H | CF3 |
| 9-8101 | 4-cyanophenyl | Me | Me | F | F | H | H | CF3 | H | nonafluoro-2-butyl | H | CF3 |
| 9-8116 | 2-chloropyridin-3-yl | Me | Me | F | F | H | H | CF3 | H | nonafluoro-2-butyl | H | CF3 |
| 9-8344 | phenyl | Me | Me | F | F | F | H | Cl | H | pentafluoroethyl | H | Cl |
| 9-8345 | phenyl | Me | Me | F | F | F | H | Br | H | heptafluoroisopropyl | H | Br |
| 9-8346 | phenyl | Me | Me | F | F | F | H | Br | H | heptafluoroisopropyl | H | OCF3 |
| 9-8347 | phenyl | Me | Me | F | F | F | H | I | H | heptafluoroisopropyl | H | CF3 |
| 9-8348 | phenyl | Me | Me | F | F | F | H | CF3 | H | heptafluoroisopropyl | H | CF3 |
| 9-8349 | phenyl | Me | Me | F | F | F | H | I | H | nonafluoro-2-butyl | H | I |
| 9-8350 | phenyl | Me | Me | F | F | F | H | Cl | H | nonafluoro-2-butyl | H | OCF3 |
| 9-8351 | phenyl | Me | Me | F | F | F | H | Br | H | nonafluoro-2-butyl | H | CF3 |
| 9-8352 | phenyl | Me | Me | F | F | F | H | CF3 | H | nonafluoro-2-butyl | H | CF3 |
| 9-8353 | phenyl | Me | Me | H | H | F | H | Cl | H | pentafluoroethyl | H | Cl |
| 9-8354 | phenyl | Me | Me | H | H | F | H | Br | H | heptafluoroisopropyl | H | Br |
| 9-8355 | phenyl | Me | Me | H | H | F | H | Br | H | heptafluoroisopropyl | H | OCF3 |
| 9-8356 | phenyl | Me | Me | H | H | F | H | I | H | heptafluoroisopropyl | H | CF3 |
| 9-8357 | phenyl | Me | Me | H | H | F | H | CF3 | H | heptafluoroisopropyl | H | CF3 |
| 9-8358 | phenyl | Me | Me | H | H | F | H | I | H | nonafluoro-2-butyl | H | I |
| 9-8359 | phenyl | Me | Me | H | H | F | H | Cl | H | nonafluoro-2-butyl | H | OCF3 |
| 9-8360 | phenyl | Me | Me | H | H | F | H | Br | H | nonafluoro-2-butyl | H | CF3 |
| 9-8361 | phenyl | Me | Me | H | H | F | H | CF3 | H | nonafluoro-2-butyl | H | CF3 |
| 9-8362 | phenyl | Me | Me | H | H | H | F | Cl | H | pentafluoroethyl | H | Cl |
| 9-8363 | phenyl | Me | Me | H | H | H | F | Br | H | heptafluoroisopropyl | H | Br |
| 9-8364 | phenyl | Me | Me | H | H | H | F | Br | H | heptafluoroisopropyl | H | OCF3 |
| 9-8365 | phenyl | Me | Me | H | H | H | F | I | H | heptafluoroisopropyl | H | CF3 |
| 9-8366 | phenyl | Me | Me | H | H | H | F | CF3 | H | heptafluoroisopropyl | H | CF3 |
| 9-8367 | phenyl | Me | Me | H | H | H | F | I | H | nonafluoro-2-butyl | H | I |
| 9-8368 | phenyl | Me | Me | H | H | H | F | Cl | H | nonafluoro-2-butyl | H | OCF3 |
| 9-8369 | phenyl | Me | Me | H | H | H | F | Br | H | nonafluoro-2-butyl | H | CF3 |
| 9-8370 | phenyl | Me | Me | H | H | H | F | CF3 | H | nonafluoro-2-butyl | H | CF3 |
| 9-8371 | phenyl | Me | Me | F | H | F | H | Cl | H | pentafluoroethyl | H | Cl |
| 9-8372 | phenyl | Me | Me | F | H | F | H | Br | H | heptafluoroisopropyl | H | Br |
| 9-8373 | phenyl | Me | Me | F | H | F | H | Br | H | heptafluoroisopropyl | H | OCF3 |
| 9-8374 | phenyl | Me | Me | F | H | F | H | I | H | heptafluoroisopropyl | H | CF3 |
| 9-8375 | phenyl | Me | Me | F | H | F | H | CF3 | H | heptafluoroisopropyl | H | CF3 |
| 9-8376 | phenyl | Me | Me | F | H | F | H | I | H | nonafluoro-2-butyl | H | I |
| 9-8377 | phenyl | Me | Me | F | H | F | H | Cl | H | nonafluoro-2-butyl | H | OCF3 |
| 9-8378 | phenyl | Me | Me | F | H | F | H | Br | H | nonafluoro-2-butyl | H | CF3 |
| 9-8379 | phenyl | Me | Me | F | H | F | H | CF3 | H | nonafluoro-2-butyl | H | CF3 |
| 9-8380 | phenyl | Me | Me | F | H | H | F | Cl | H | pentafluoroethyl | H | Cl |
| 9-8381 | phenyl | Me | Me | F | H | H | F | Br | H | heptafluoroisopropyl | H | Br |
| 9-8382 | phenyl | Me | Me | F | H | H | F | Br | H | heptafluoroisopropyl | H | OCF3 |
| 9-8383 | phenyl | Me | Me | F | H | H | F | I | H | heptafluoroisopropyl | H | CF3 |
| 9-8384 | phenyl | Me | Me | F | H | H | F | CF3 | H | heptafluoroisopropyl | H | CF3 |
| 9-8385 | phenyl | Me | Me | F | H | H | F | I | H | nonafluoro-2-butyl | H | I |
| 9-8386 | phenyl | Me | Me | F | H | H | F | Cl | H | nonafluoro-2-butyl | H | OCF3 |
| 9-8387 | phenyl | Me | Me | F | H | H | F | Br | H | nonafluoro-2-butyl | H | CF3 |
| 9-8388 | phenyl | Me | Me | F | H | H | F | CF3 | H | nonafluoro-2-butyl | H | CF3 |
| 9-8389 | phenyl | Me | Me | H | F | F | H | Cl | H | pentafluoroethyl | H | Cl |
| 9-8390 | phenyl | Me | Me | H | F | F | H | Br | H | heptafluoroisopropyl | H | Br |
| 9-8391 | phenyl | Me | Me | H | F | F | H | Br | H | heptafluoroisopropyl | H | OCF3 |
| 9-8392 | phenyl | Me | Me | H | F | F | H | I | H | heptafluoroisopropyl | H | CF3 |
| 9-8393 | phenyl | Me | Me | H | F | F | H | CF3 | H | heptafluoroisopropyl | H | CF3 |
| 9-8394 | phenyl | Me | Me | H | F | F | H | I | H | nonafluoro-2-butyl | H | I |
| 9-8395 | phenyl | Me | Me | H | F | F | H | Cl | H | nonafluoro-2-butyl | H | OCF3 |
| 9-8396 | phenyl | Me | Me | H | F | F | H | Br | H | nonafluoro-2-butyl | H | CF3 |
| 9-8397 | phenyl | Me | Me | H | F | F | H | CF3 | H | nonafluoro-2-butyl | H | CF3 |
| 9-8398 | phenyl | Me | Me | H | F | H | F | Cl | H | pentafluoroethyl | H | Cl |
| 9-8399 | phenyl | Me | Me | H | F | H | F | Br | H | heptafluoroisopropyl | H | Br |

**Table 9(11)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 9-8400 | phenyl | Me | Me | H | F | H | F | Br | H | heptafluoroisopropyl | H | OCF3 |
| 9-8401 | phenyl | Me | Me | H | F | H | F | I | H | heptafluoroisopropyl | H | CF3 |
| 9-8402 | phenyl | Me | Me | H | F | H | F | CF3 | H | heptafluoroisopropyl | H | CF3 |
| 9-8403 | phenyl | Me | Me | H | F | H | F | I | H | nonafluoro-2-butyl | H | I |
| 9-8404 | phenyl | Me | Me | H | F | H | F | Cl | H | nonafluoro-2-butyl | H | OCF3 |
| 9-8405 | phenyl | Me | Me | H | F | H | F | Br | H | nonafluoro-2-butyl | H | CF3 |
| 9-8406 | phenyl | Me | Me | H | F | H | F | CF3 | H | nonafluoro-2-butyl | H | CF3 |
| 9-8407 | phenyl | Me | Me | H | H | F | F | Cl | H | pentafluoroethyl | H | Cl |
| 9-8408 | phenyl | Me | Me | H | H | F | F | Br | H | heptafluoroisopropyl | H | Br |
| 9-8409 | phenyl | Me | Me | H | H | F | F | Br | H | heptafluoroisopropyl | H | OCF3 |
| 9-8410 | phenyl | Me | Me | H | H | F | F | I | H | heptafluoroisopropyl | H | CF3 |
| 9-8411 | phenyl | Me | Me | H | H | F | F | CF3 | H | heptafluoroisopropyl | H | CF3 |
| 9-8412 | phenyl | Me | Me | H | H | F | F | I | H | nonafluoro-2-butyl | H | I |
| 9-8413 | phenyl | Me | Me | H | H | F | F | Cl | H | nonafluoro-2-butyl | H | OCF3 |
| 9-8414 | phenyl | Me | Me | H | H | F | F | Br | H | nonafluoro-2-butyl | H | CF3 |
| 9-8415 | phenyl | Me | Me | H | H | F | F | CF3 | H | nonafluoro-2-butyl | H | CF3 |
| 9-8416 | phenyl | Me | Me | F | F | H | F | Cl | H | pentafluoroethyl | H | Cl |
| 9-8417 | phenyl | Me | Me | F | F | H | F | Br | H | heptafluoroisopropyl | H | Br |
| 9-8418 | phenyl | Me | Me | F | F | H | F | Br | H | heptafluoroisopropyl | H | OCF3 |
| 9-8419 | phenyl | Me | Me | F | F | H | F | I | H | heptafluoroisopropyl | H | CF3 |
| 9-8420 | phenyl | Me | Me | F | F | H | F | CF3 | H | heptafluoroisopropyl | H | CF3 |
| 9-8421 | phenyl | Me | Me | F | F | H | F | I | H | nonafluoro-2-butyl | H | I |
| 9-8422 | phenyl | Me | Me | F | F | H | F | Cl | H | nonafluoro-2-butyl | H | OCF3 |
| 9-8423 | phenyl | Me | Me | F | F | H | F | Br | H | nonafluoro-2-butyl | H | CF3 |
| 9-8424 | phenyl | Me | Me | F | F | H | F | CF3 | H | nonafluoro-2-butyl | H | CF3 |
| 9-8425 | phenyl | Me | Me | F | H | F | F | Cl | H | pentafluoroethyl | H | Cl |
| 9-8426 | phenyl | Me | Me | F | H | F | F | Br | H | heptafluoroisopropyl | H | Br |
| 9-8427 | phenyl | Me | Me | F | H | F | F | Br | H | heptafluoroisopropyl | H | OCF3 |
| 9-8428 | phenyl | Me | Me | F | H | F | F | I | H | heptafluoroisopropyl | H | CF3 |
| 9-8429 | phenyl | Me | Me | F | H | F | F | CF3 | H | heptafluoroisopropyl | H | CF3 |
| 9-8430 | phenyl | Me | Me | F | H | F | F | I | H | nonafluoro-2-butyl | H | I |
| 9-8431 | phenyl | Me | Me | F | H | F | F | Cl | H | nonafluoro-2-butyl | H | OCF3 |
| 9-8432 | phenyl | Me | Me | F | H | F | F | Br | H | nonafluoro-2-butyl | H | CF3 |
| 9-8433 | phenyl | Me | Me | F | H | F | F | CF3 | H | nonafluoro-2-butyl | H | CF3 |
| 9-8434 | phenyl | Me | Me | H | F | F | F | Cl | H | pentafluoroethyl | H | Cl |
| 9-8435 | phenyl | Me | Me | H | F | F | F | Br | H | heptafluoroisopropyl | H | Br |
| 9-8436 | phenyl | Me | Me | H | F | F | F | Br | H | heptafluoroisopropyl | H | OCF3 |
| 9-8437 | phenyl | Me | Me | H | F | F | F | I | H | heptafluoroisopropyl | H | CF3 |
| 9-8438 | phenyl | Me | Me | H | F | F | F | CF3 | H | heptafluoroisopropyl | H | CF3 |
| 9-8439 | phenyl | Me | Me | H | F | F | F | I | H | nonafluoro-2-butyl | H | I |
| 9-8440 | phenyl | Me | Me | H | F | F | F | Cl | H | nonafluoro-2-butyl | H | OCF3 |
| 9-8441 | phenyl | Me | Me | H | F | F | F | Br | H | nonafluoro-2-butyl | H | CF3 |
| 9-8442 | phenyl | Me | Me | H | F | F | F | CF3 | H | nonafluoro-2-butyl | H | CF3 |
| 9-8443 | phenyl | Me | Me | F | F | F | F | Cl | H | pentafluoroethyl | H | Cl |
| 9-8444 | phenyl | Me | Me | F | F | F | F | Br | H | heptafluoroisopropyl | H | Br |
| 9-8445 | phenyl | Me | Me | F | F | F | F | Br | H | heptafluoroisopropyl | H | OCF3 |
| 9-8446 | phenyl | Me | Me | F | F | F | F | I | H | heptafluoroisopropyl | H | CF3 |
| 9-8447 | phenyl | Me | Me | F | F | F | F | CF3 | H | heptafluoroisopropyl | H | CF3 |
| 9-8448 | phenyl | Me | Me | F | F | F | F | I | H | nonafluoro-2-butyl | H | I |
| 9-8449 | phenyl | Me | Me | F | F | F | F | Cl | H | nonafluoro-2-butyl | H | OCF3 |
| 9-8450 | phenyl | Me | Me | F | F | F | F | Br | H | nonafluoro-2-butyl | H | CF3 |
| 9-8451 | phenyl | Me | Me | F | F | F | F | CF3 | H | nonafluoro-2-butyl | H | CF3 |

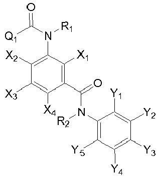

**Table 10(1)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 10-9 | 3-bromophenyl | H | Et | H | H | H | H | Cl | F | nonafluoro-2-butyl | F | Cl |
| 10-39 | 2-iodopyridin-3-yl | H | H | H | H | H | H | Cl | Me | nonafluoro-2-butyl | Me | I |
| 10-148 | 2-chlorophenyl | H | H | H | H | H | H | F | Br | heptafluoroisopropyl | H | CF3 |
| 10-153 | 4-bromophenyl | H | H | H | H | H | H | Cl | F | heptafluoroisopropyl | Cl | CF3 |
| 10-167 | 3,4-dichlomphanyl | H | H | H | H | H | H | Cl | I | nonafluoro-2-butyl | I | CF3 |
| 10-168 | 2,4-dichlomphanyl | H | H | H | H | H | H | Cl | Me | nonafluoro-2-butyl | H | CF3 |
| 10-169 | 2-chloro-4-fluorophenyl | H | H | H | H | H | H | Cl | H | nonafluoro-2-butyl | Me | CF3 |
| 10-170 | 2-chloro-4,5-difluorophenyl | i-Pr | H | H | H | H | H | Cl | Et | nonafluoro-2-butyl | H | CF3 |
| 10-171 | 4-bromo-2-chlorophenyl | H | H | H | H | H | H | Cl | H | nonafluoro-2-butyl | Et | CF3 |
| 10-182 | 2-iodopyridin-3-yl | H | H | H | H | H | H | Cl | Me | nonafluoro-2-butyl | Me | n-C3F7 |
| 10-183 | 2-(trifluoromethyl)pyridin-3-yl | H | H | H | H | H | H | Cl | Me | nonafluoro-2-butyl | Et | i-C3F7 |
| 10-189 | 6-iodopyridin-3-yl | H | H | H | H | H | H | Cl | Et | nonafluoro-2-butyl | Me | n-C3F7 |
| 10-190 | 6-(trifluoromethyl)pyridin-3-yl | H | H | H | H | H | H | Cl | Et | nonafluoro-2-butyl | Et | i-C3F7 |
| 10-196 | 5-bromopyridin-3-yl | H | H | H | H | H | H | Br | n-Pr | heptafluoroisopropyl | Me | CF3 |
| 10-197 | 5-iodopyridin-3-yl | H | H | H | H | H | H | Br | n-Pr | heptafluoroisopropyl | Et | CF3 |
| 10-203 | 4-bromopyridin-3-yl | Me | n-Pr | H | H | H | H | Br | i-Pr | heptafluoroisopropyl | Me | CF3 |
| 10-204 | 4-iodopyridin-3-yl | H | H | H | H | H | H | Br | i-Pr | heptafluoroisopropyl | Et | CF3 |
| 10-210 | pyridin-4-yl | H | H | H | H | H | H | Br | n-Bu | heptafluoroisopropyl | Me | CF3 |
| 10-211 | 2-chloropyridin-4-yl | H | H | H | H | H | H | Br | n-Bu | heptafluoroisopropyl | Et | CF3 |
| 10-217 | pyridin-2-yl | H | H | H | H | H | H | Br | i-Bu | nonafluoro-2-butyl | Me | CF3 |
| 10-218 | 3-chloropyridin-2-yl | H | H | H | H | H | H | Br | i-Bu | nonafluoro-2-butyl | Et | CF3 |
| 10-224 | 2-fluorophenyl | H | H | H | H | H | H | Br | s-Bu | nonafluoro-2-butyl | Me | CF3 |
| 10-225 | 3-fluorophenyl | H | H | H | H | H | H | Br | s-Bu | nonafluoro-2-butyl | Et | CF3 |
| 10-231 | 3-bromophenyl | H | H | H | H | H | H | Br | F | nonafluoro-2-butyl | Me | CF3 |
| 10-232 | 4-bromophenyl | H | H | H | H | H | H | Br | F | nonafluoro-2-butyl | Et | CF3 |
| 10-238 | (4-trifluoromethyl)phenyl | Et | i-Pr | H | H | H | H | Br | Cl | nonafluoro-2-butyl | Me | CF3 |
| 10-239 | 2-nitrophenyl | H | H | H | H | H | H | Br | Cl | nonafluoro-2-butyl | Et | CF3 |
| 10-245 | 4-cyanophenyl | H | H | H | H | H | H | I | Br | heptafluoroisopropyl | Me | CF3 |
| 10-246 | 2,6-difluorophenyl | H | H | H | H | H | H | I | Br | heptafluoroisopropyl | Et | CF3 |
| 10-252 | 2-bromo-4-chlorophenyl | n-Pr | Me | H | H | H | H | I | I | heptafluoroisopropyl | Me | CF3 |
| 10-253 | 2-bromo-4-fluorophenyl | H | H | H | H | H | H | I | I | heptafluoroisopropyl | Et | CF3 |
| 10-259 | 2-fluoropyridin-3-yl | H | H | H | H | H | H | I | Me | heptafluoroisopropyl | F | C2F5 |
| 10-260 | 2-chloropyridin-3-yl | H | H | H | H | H | H | I | Me | nonafluoro-2-butyl | Cl | CF3 |
| 10-263 | 2-(trifluoromethyl)pyridin-3-yl | H | H | H | H | H | H | I | Et | nonafluoro-2-butyl | F | CF3 |
| 10-264 | 2-nitropyridin-3-yl | H | H | H | H | H | H | I | Et | nonafluoro-2-butyl | Cl | CF3 |
| 10-267 | 6-chloropyridin-3-yl | H | H | H | H | H | H | I | n-Pr | nonafluoro-2-butyl | F | CF3 |
| 10-268 | 6-bromopyridin-3-yl | H | H | H | H | H | H | I | n-Pr | nonafluoro-2-butyl | Cl | CF3 |
| 10-271 | 6-nitropyridin-3-yl | H | H | H | H | H | H | I | i-Pr | nonafluoro-2-butyl | F | i-C3F7 |
| 10-272 | 6-cyanopyridin-3-yl | H | H | H | H | H | H | I | i-Pr | nonafluoro-2-butyl | Cl | CF3 |
| 10-275 | 5-bromopyridin-3-yl | H | H | H | H | H | H | I | n-Bu | nonafluoro-2-butyl | F | CF3 |
| 10-276 | 5-iodopyridin-3-yl | H | H | H | H | H | H | I | n-Bu | nonafluoro-2-butyl | Cl | CF3 |
| 10-279 | 5-cyanopyridin-3-yl | H | H | H | H | H | H | I | i-Bu | nonafluoro-2-butyl | F | CF3 |
| 10-280 | 4-fluoropyridin-3-yl | H | H | H | H | H | H | I | i-Bu | nonafluoro-2-butyl | Cl | CF3 |
| 10-283 | 4-iodopyridin-3-yl | i-Pr | n-Pr | H | H | H | H | I | s-Bu | nonafluoro-2-butyl | F | CF3 |
| 10-284 | 4-(trifluoromethyl)pyridin-3-yl | H | H | H | H | H | H | I | s-Bu | nonafluoro-2-butyl | Cl | CF3 |
| 10-295 | 3-bromophenyl | H | Et | H | H | H | H | OCF3 | F | heptafluoroisopropyl | F | OCF3 |
| 10-325 | 2-iodopyridin-3-yl | H | H | H | H | H | H | OCF3 | Me | nonafluoro-2-butyl | Me | OCF3 |
| 10-438 | 3-bromophenyl | H | Et | H | H | H | H | OCF3 | F | heptafluoroisopropyl | F | CF3 |
| 10-468 | 2-iodopyridin-3-yl | H | H | H | H | H | H | OCF3 | Me | nonafluoro-2-butyl | Me | CF3 |
| 10-581 | 3-bromophenyl | H | Et | H | H | H | H | CF3 | F | heptafluoroisopropyl | F | CF3 |
| 10-611 | 2-iodopyridin-3-yl | H | H | H | H | H | H | CF3 | Me | nonafluoro-2-butyl | Me | CF3 |
| 10-724 | 3-bromophenyl | H | Et | F | H | H | H | Cl | F | heptafluoroisopropyl | F | Cl |
| 10-754 | 2-iodopyridin-3-yl | H | H | F | H | H | H | Cl | Me | nonafluoro-2-butyl | Me | I |
| 10-859 | phenyl | H | H | F | H | H | H | Cl | F | heptafluoroisopropyl | H | CF3 |
| 10-860 | 2-fluorophenyl | Me | H | F | H | H | H | Cl | H | heptafluoroisopropyl | F | CF3 |

**Table 10(2)**

| compound number | Q₁ | R₁ | R₂ | X₁ | X₂ |
|---|---|---|---|---|---|
| 10-861 | 3-fluorophenyl | H | Me | F | H |
| 10-862 | 4-fluorophenyl | Me | Me | F | H |
| 10-863 | 2-chlorophenyl | H | H | F | H |
| 10-864 | 3-chlorophenyl | H | H | F | H |
| 10-865 | 4-chlorophenyl | Et | H | F | H |
| 10-866 | 2-bromophenyl | H | H | F | H |
| 10-867 | 3-bromophenyl | H | Et | F | H |
| 10-868 | 4-bromophenyl | H | H | F | H |
| 10-869 | 2-iodophenyl | H | H | F | H |
| 10-870 | 3-iodophenyl | Et | Et | F | H |
| 10-871 | 4-iodophenyl | H | H | F | H |
| 10-872 | (2-trifluoromethyl)phenyl | H | H | F | H |
| 10-873 | (3-trifluoromethyl)phenyl | n-Pr | H | F | H |
| 10-874 | (4-trifluoromethyl)phenyl | H | H | F | H |
| 10-875 | 2-nitrophenyl | H | n-Pr | F | H |
| 10-876 | 3-nitrophenyl | H | H | F | H |
| 10-877 | 4-nitrophenyl | H | H | F | H |
| 10-878 | 2-cyanophenyl | n-Pr | n-Pr | F | H |
| 10-879 | 3-cyanophenyl | H | H | F | H |
| 10-880 | 4-cyanophenyl | H | H | F | H |
| 10-881 | 2,6-difluorophenyl | H | H | F | H |
| 10-882 | 3,4-dichlorophenyl | H | H | F | H |
| 10-883 | 2,4-dichlorophenyl | H | H | F | H |
| 10-884 | 2-chloro-4-fluorophenyl | H | H | F | H |
| 10-885 | 2-chloro-4,5-difluorophenyl | i-Pr | H | F | H |
| 10-886 | 4-bromo-2-chlorophenyl | H | H | F | H |
| 10-887 | 2-bromo-4-chlorophenyl | H | H | F | H |
| 10-888 | 2-bromo-4-fluorophenyl | H | i-Pr | F | H |
| 10-889 | 2-chloro-4-nitrophenyl | i-Pr | i-Pr | F | H |
| 10-890 | 3,5-dicyanophenyl | H | H | F | H |
| 10-891 | 4-cyano-2-fluorophenyl | H | H | F | H |
| 10-892 | 2-chloro-4-cyanophenyl | H | H | F | H |
| 10-893 | pyridin-3-yl | H | H | F | H |
| 10-894 | 2-fluoropyridin-3-yl | H | H | F | H |
| 10-895 | 2-chloropyridin-3-yl | H | H | F | H |
| 10-896 | 2-bromopyridin-3-yl | H | H | F | H |
| 10-897 | 2-iodopyridin-3-yl | H | H | F | H |
| 10-898 | 2- (trifluoromethyl) pyridin-3-yl | H | H | F | H |
| 10-899 | 2-nitropyridin-3-yl | C(O)Me | H | F | H |
| 10-900 | 2-cyanopyridin-3-yl | H | H | F | H |
| 10-901 | 6-fluoropyridin-3-yl | H | H | F | H |
| 10-902 | 6-chloropyridin-3-yl | H | H | F | H |
| 10-903 | 6-bromopyridin-3-yl | H | C(O)Me | F | H |
| 10-904 | 6-iodopyridin-3-yl | H | H | F | H |
| 10-905 | 6-(trifluoromethyl)pyridin-3-yl | H | H | F | H |
| 10-906 | 6-nitropyridin-3-yl | C(O)Me | C(O)Me | F | H |
| 10-907 | 6-cyanopyridin-3-yl | H | H | F | H |
| 10-908 | phenyl | H | H | F | H |
| 10-909 | 5-fluoropyridin-3-yl | H | H | F | H |
| 10-910 | 5-chloropyridin-3-yl | H | H | F | H |
| 10-911 | 5-bromopyridin-3-yl | H | H | F | H |
| 10-912 | 5-iodopyridin-3-yl | H | H | F | H |
| 10-913 | 5-(trifluoromethyl)pyridin-3-yl | Me | Et | F | H |
| 10-914 | 5-nitropyridin-3-yl | H | H | F | H |
| 10-915 | 5-cyanopyridin-3-yl | H | H | F | H |
| 10-916 | 4-fluoropyridin-3-yl | H | H | F | H |
| 10-917 | 4-chloropyridin-3-yl | H | H | F | H |
| 10-918 | 4-bromopyridin-3-yl | Me | n-Pr | F | H |
| 10-919 | 4-iodopyridin-3-yl | H | H | F | H |
| 10-920 | 4-(trifluoromethyl)pyridin-3-yl | H | H | F | H |
| 10-921 | 4-nitropyridin-3-yl | H | H | F | H |
| 10-922 | 4-cyanopyridin-3-yl | H | H | F | H |
| 10-923 | 2,6-dichloropyridin-3-yl | H | H | F | H |
| 10-924 | pyridin-3-yl N-oxide | Me | i-Pr | F | H |

**Table 10(3)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 10-925 | pyridin-4-yl | H | H | F | H | H | H | Br | n-Bu | heptafluoroisopropyl | Me | CF3 |
| 10-926 | 2-chloropyridin-4-yl | H | H | F | H | H | H | Br | n-Bu | heptafluoroisopropyl | Et | CF3 |
| 10-927 | 3-bromopyridin-4-yl | H | H | F | H | H | H | Br | n-Bu | heptafluoroisopropyl | n-Pr | CF3 |
| 10-928 | 3,5-dichloropyridin-4-yl | H | H | F | H | H | H | Br | n-Bu | nonafluoro-2-butyl | i-Pr | CF3 |
| 10-929 | 3-(trifluoromothyl)pyridin-4-yl | H | H | F | H | H | H | Br | n-Bu | nonafluoro-2-butyl | n-Bu | CF3 |
| 10-930 | 2,6-dicyanopyridin-4-yl | H | H | F | H | H | H | Br | n-Bu | nonafluoro-2-butyl | i-Bu | CF3 |
| 10-931 | pyridin-4-yl N-oxide | Me | C(O)Me | F | H | H | H | Br | n-Bu | nonafluoro-2-butyl | s-Bu | CF3 |
| 10-932 | pyridin-2-yl | H | H | F | H | H | H | Br | i-Bu | nonafluoro-2-butyl | Me | CF3 |
| 10-933 | 3-chloropyridin-2-yl | H | H | F | H | H | H | Br | i-Bu | nonafluoro-2-butyl | Et | CF3 |
| 10-934 | 4-bromopyridin-2-yl | H | H | F | H | H | H | Br | i-Bu | nonafluoro-2-butyl | n-Pr | CF3 |
| 10-935 | 5-iodopyridin-2-yl | H | H | F | H | H | H | Br | i-Bu | nonafluoro-2-butyl | i-Pr | CF3 |
| 10-936 | 6-chloropyridin-2-yl | H | H | F | H | H | H | Br | i-Bu | nonafluoro-2-butyl | n-Bu | CF3 |
| 10-937 | 4-cyanopyridin-2-yl | H | H | F | H | H | H | Br | i-Bu | nonafluoro-2-butyl | i-Bu | CF3 |
| 10-938 | phenyl | H | H | F | H | H | H | Br | i-Bu | nonafluoro-2-butyl | s-Bu | C2F5 |
| 10-939 | 2-fluorophenyl | H | H | F | H | H | H | Br | s-Bu | nonafluoro-2-butyl | Me | CF3 |
| 10-940 | 3-fluorophenyl | H | H | F | H | H | H | Br | s-Bu | nonafluoro-2-butyl | Et | CF3 |
| 10-941 | 4-fluorophenyl | H | H | F | H | H | H | Br | s-Bu | nonafluoro-2-butyl | n-Pr | CF3 |
| 10-942 | 2-chlorophenyl | H | H | F | H | H | H | Br | s-Bu | nonafluoro-2-butyl | i-Pr | CF3 |
| 10-943 | 3-chlorophenyl | H | H | F | H | H | H | Br | s-Bu | nonafluoro-2-butyl | n-Bu | CF3 |
| 10-944 | 4-chlorophenyl | H | H | F | H | H | H | Br | s-Bu | nonafluoro-2-butyl | i-Bu | CF3 |
| 10-945 | 2-bromophenyl | Et | Me | F | H | H | H | Br | s-Bu | nonafluoro-2-butyl | s-Bu | CF3 |
| 10-946 | 3-bromophenyl | H | H | F | H | H | H | Br | F | nonafluoro-2-butyl | Me | CF3 |
| 10-947 | 4-bromophenyl | H | H | F | H | H | H | Br | F | nonafluoro-2-butyl | Et | CF3 |
| 10-948 | 2-iodophenyl | H | H | F | H | H | H | Br | F | nonafluoro-2-butyl | n-Pr | CF3 |
| 10-949 | 3-iodophenyl | H | H | F | H | H | H | Br | F | nonafluoro-2-butyl | i-Pr | CF3 |
| 10-950 | 4-iodophenyl | H | H | F | H | H | H | Br | F | nonafluoro-2-butyl | n-Bu | CF3 |
| 10-951 | (2-trifluoromethyl)phenyl | H | H | F | H | H | H | Br | F | nonafluoro-2-butyl | i-Bu | CF3 |
| 10-952 | (3-trifluoromethyl)phenyl | Et | n-Pr | F | H | H | H | Br | F | nonafluoro-2-butyl | s-Bu | CF3 |
| 10-953 | (4-trifluoromethyl)phenyl | Et | i-Pr | F | H | H | H | Br | Cl | nonafluoro-2-butyl | Me | CF3 |
| 10-954 | 2-nitrophenyl | H | H | F | H | H | H | Br | Cl | nonafluoro-2-butyl | Et | CF3 |
| 10-955 | phenyl | H | H | F | H | H | H | I | Cl | pentafluoroethyl | n-Pr | i-C3F7 |
| 10-956 | 3-nitrophenyl | H | H | F | H | H | H | I | Cl | heptafluoroisopropyl | i-Pr | CF3 |
| 10-957 | 4-nitrophenyl | H | H | F | H | H | H | I | Cl | heptafluoroisopropyl | n-Bu | CF3 |
| 10-958 | 2-cyanophenyl | Et | C(O)Me | F | H | H | H | I | Cl | heptafluoroisopropyl | i-Bu | CF3 |
| 10-959 | 3-cyanophenyl | H | H | F | H | H | H | I | Cl | heptafluoroisopropyl | s-Bu | CF3 |
| 10-960 | 4-cyanophenyl | H | H | F | H | H | H | I | Br | heptafluoroisopropyl | Me | CF3 |
| 10-961 | 2,6-difluorophenyl | H | H | F | H | H | H | I | Br | heptafluoroisopropyl | Et | CF3 |
| 10-962 | 3,4-dichlorophenyl | H | H | F | H | H | H | I | Br | pentafluoroethyl | n-Pr | CF3 |
| 10-963 | 2,4-dichlorophenyl | H | H | F | H | H | H | I | Br | heptafluoroisopropyl | i-Pr | CF3 |
| 10-964 | 2-chloro-4-fluorophenyl | H | H | F | H | H | H | I | Br | heptafluoroisopropyl | n-Bu | CF3 |
| 10-965 | 2-chloro-4,5-difluorophenyl | H | H | F | H | H | H | I | Br | heptafluoroisopropyl | i-Bu | CF3 |
| 10-966 | 4-bromo-2-chlorophenyl | H | H | F | H | H | H | I | Br | heptafluoroisopropyl | s-Bu | CF3 |
| 10-967 | 2-bromo-4-chlorophenyl | n-Pr | Me | F | H | H | H | I | I | heptafluoroisopropyl | Me | CF3 |
| 10-968 | 2-bromo-4-fluorophenyl | H | H | F | H | H | H | I | I | heptafluoroisopropyl | Et | CF3 |
| 10-969 | 2-chloro-4-nitrophenyl | n-Pr | Et | F | H | H | H | I | I | heptafluoroisopropyl | n-Pr | CF3 |
| 10-970 | 3,5-dicyanophenyl | H | H | F | H | H | H | I | I | heptafluoroisopropyl | i-Pr | CF3 |
| 10-971 | 4-cyano-2-fluorophenyl | H | H | F | H | H | H | I | I | heptafluoroisopropyl | n-Bu | CF3 |
| 10-972 | 2-chloro-4-cyanophenyl | H | H | F | H | H | H | I | I | heptafluoroisopropyl | i-Bu | CF3 |
| 10-973 | pyridin-3-yl | H | H | F | H | H | H | I | I | heptafluoroisopropyl | s-Bu | CF3 |
| 10-974 | 2-fluoropyridin-3-yl | H | H | F | H | H | H | I | Me | heptafluoroisopropyl | F | C2F5 |
| 10-975 | 2-chloropyridin-3-yl | H | H | F | H | H | H | I | Me | nonafluoro-2-butyl | Cl | CF3 |
| 10-976 | 2-bromopyridin-3-yl | H | H | F | H | H | H | I | Me | nonafluoro-2-butyl | Br | n-C3F7 |
| 10-977 | 2-iodopyridin-3-yl | H | H | F | H | H | H | I | Me | nonafluoro-2-butyl | I | i-C3F7 |
| 10-978 | 2-(trifluoromethyl)pyridin-3-yl | H | H | F | H | H | H | I | Et | nonafluoro-2-butyl | F | CF3 |
| 10-979 | 2-nitropyridin-3-yl | H | H | F | H | H | H | I | Et | nonafluoro-2-butyl | Cl | CF3 |
| 10-980 | 2-cyanopyridin-3-yl | H | H | F | H | H | H | I | Et | nonafluoro-2-butyl | Br | CF3 |
| 10-981 | 6-fluoropyridin-3-yl | n-Pr | i-Pr | F | H | H | H | I | Et | nonafluoro-2-butyl | I | C2F5 |
| 10-982 | 6-chloropyridin-3-yl | H | H | F | H | H | H | I | n-Pr | nonafluoro-2-butyl | F | CF3 |
| 10-983 | 6-bromopyridin-3-yl | H | H | F | H | H | H | I | n-Pr | nonafluoro-2-butyl | Cl | CF3 |
| 10-984 | 6-iodopyridin-3-yl | H | H | F | H | H | H | I | n-Pr | nonafluoro-2-butyl | Br | CF3 |
| 10-985 | 6-(trifluoromethyl)pyridin-3-yl | H | H | F | H | H | H | I | n-Pr | nonafluoro-2-butyl | I | n-C3F7 |
| 10-986 | 6-nitropyridin-3-yl | H | H | F | H | H | H | I | i-Pr | nonafluoro-2-butyl | F | i-C3F7 |
| 10-987 | 6-cyanopyridin-3-yl | H | H | F | H | H | H | I | i-Pr | nonafluoro-2-butyl | Cl | CF3 |
| 10-988 | 5-fluoropyridin-3-yl | n-Pr | C(O)Me | F | H | H | H | I | i-Pr | nonafluoro-2-butyl | Br | CF3 |

**Table 10(4)**

| compound number | Q₁ | R₁ | R₂ | X₁ | X₂ | X₃ | X₄ | Y₂ | Y₂ | Y₃ | Y₄ | Y₅ |
|---|---|---|---|---|---|---|---|---|---|---|---|---|
| 10-989 | 5-chloropyridin-3-yl | H | H | F | H | H | H | I | i-Pr | nonafluoro-2-butyl | I | CF3 |
| 10-990 | 5-bromopyridin-3-yl | H | H | F | H | H | H | I | n-Bu | nonafluoro-2-butyl | F | CF3 |
| 10-991 | 5-iodopyridin-3-yl | H | H | F | H | H | H | I | n-Bu | nonafluoro-2-butyl | Cl | CF3 |
| 10-992 | 5-(trifluoromethyl)pyridin-3-yl | H | H | F | H | H | H | I | n-Bu | nonafluoro-2-butyl | Br | CF3 |
| 10-993 | 5-nitropyridin-3-yl | i-Pr | Me | F | H | H | H | I | n-Bu | nonafluoro-2-butyl | I | CF3 |
| 10-994 | 5-cyanopyridin-3-yl | H | H | F | H | H | H | I | i-Bu | nonafluoro-2-butyl | F | CF3 |
| 10-995 | 4-fluoropyridin-3-yl | H | H | F | H | H | H | I | i-Bu | nonafluoro-2-butyl | Cl | CF3 |
| 10-996 | 4-chloropyridin-3-yl | H | H | F | H | H | H | I | i-Bu | nonafluoro-2-butyl | Br | CF3 |
| 10-997 | 4-bromopyridin-3-yl | i-Pr | Et | F | H | H | H | I | i-Bu | nonafluoro-2-butyl | I | CF3 |
| 10-998 | 4-iodopyridin-3-yl | i-Pr | n-Pr | F | H | H | H | I | s-Bu | nonafluoro-2-butyl | F | CF3 |
| 10-999 | 4-(trifluoromethyl)pyridin-3-yl | H | H | F | H | H | H | I | s-Bu | nonafluoro-2-butyl | Cl | CF3 |
| 10-1000 | 4-nitropyridin-3-yl | i-Pr | C(O)Me | F | H | H | H | I | s-Bu | nonafluoro-2-butyl | Br | CF3 |
| 10-1001 | 4-cyanopyridin-3-yl | H | H | F | H | H | H | I | s-Bu | nonafluoro-2-butyl | I | CF3 |
| 10-1010 | 3-bromophenyl | H | Et | F | H | H | H | OCF3 | F | heptafluoroisopropyl | F | OCF3 |
| 10-1040 | 2-iodopyridin-3-yl | H | H | F | H | H | H | OCF3 | Me | nonafluoro-2-butyl | Me | OCF3 |
| 10-1153 | 3-bromophenyl | H | Et | F | H | H | H | OCF3 | F | heptafluoroisopropyl | F | CF3 |
| 10-1183 | 2-iodopyridin-3-yl | H | H | F | H | H | H | OCF3 | Me | nonafluoro-2-butyl | Me | CF3 |
| 10-1296 | 3-bromophenyl | H | Et | F | H | H | H | CF3 | F | heptafluoroisopropyl | F | CF3 |
| 10-1326 | 2-iodopyridin-3-yl | H | H | F | H | H | H | CF3 | Me | nonafluoro-2-butyl | Me | CF3 |
| 10-1439 | 3-bromophenyl | H | Et | H | F | H | H | Cl | F | heptafluoroisopropyl | F | Cl |
| 10-1469 | 2-iodopyridin-3-yl | H | H | H | F | H | H | Cl | Me | nonafluoro-2-butyl | Me | I |
| 10-1582 | 3-bromophenyl | H | Et | H | F | H | H | Cl | F | heptafluoroisopropyl | F | OCF3 |
| 10-1612 | 2-iodopyridin-3-yl | H | H | H | F | H | H | Cl | Me | nonafluoro-2-butyl | Me | OCF3 |
| 10-1726 | 4-bromophenyl | H | H | H | F | H | H | Cl | F | heptafluoroisopropyl | Cl | CF3 |
| 10-1733 | 2-nitrophenyl | H | n-Pr | H | F | H | H | Cl | Br | heptafluoroisopropyl | F | CF3 |
| 10-1741 | 2,4-dichlorophenyl | H | H | H | F | H | H | Cl | Me | nonafluoro-2-butyl | H | CF3 |
| 10-1742 | 2-chloro-4-fluorophenyl | H | H | H | F | H | H | Cl | H | nonafluoro-2-butyl | Me | CF3 |
| 10-1743 | 2-chloro-4,5-difluorophenyl | i-Pr | H | H | F | H | H | Cl | Et | nonafluoro-2-butyl | H | CF3 |
| 10-1744 | 4-bromo-2-chlorophenyl | H | H | H | F | H | H | Cl | H | nonafluoro-2-butyl | Et | CF3 |
| 10-1755 | 2-iodopyridin-3-yl | H | H | H | F | H | H | Cl | Me | nonafluoro-2-butyl | Me | n-C3F7 |
| 10-1756 | 2-(trifluoromethyl)pyridin-3-yl | H | H | H | F | H | H | Cl | Me | nonafluoro-2-butyl | Et | i-C3F7 |
| 10-1762 | 6-iodopyridin-3-yl | H | H | H | F | H | H | Cl | Et | nonafluoro-2-butyl | Me | n-C3F7 |
| 10-1763 | 6-(trifluoromethyl)pyridin-3-yl | H | H | H | F | H | H | Cl | Et | nonafluoro-2-butyl | Et | i-C3F7 |
| 10-1769 | 5-bromopyridin-3-yl | H | H | H | F | H | H | Br | n-Pr | heptafluoroisopropyl | Me | CF3 |
| 10-1770 | 5-iodopyridin-3-yl | H | H | H | F | H | H | Br | n-Pr | heptafluoroisopropyl | Et | CF3 |
| 10-1776 | 4-bromopyridin-3-yl | Me | n-Pr | H | F | H | H | Br | i-Pr | heptafluoroisopropyl | Me | CF3 |
| 10-1777 | 4-iodopyridin-3-yl | H | H | H | F | H | H | Br | i-Pr | heptafluoroisopropyl | Et | CF3 |
| 10-1783 | pyridin-4-yl | H | H | H | F | H | H | Br | n-Bu | heptafluoroisopropyl | Me | CF3 |
| 10-1784 | 2-chloropyridin-4-yl | H | H | H | F | H | H | Br | n-Bu | heptafluoroisopropyl | Et | CF3 |
| 10-1790 | pyridin-2-yl | H | H | H | F | H | H | Br | i-Bu | nonafluoro-2-butyl | Me | CF3 |
| 10-1791 | 3-chloropyridin-2-yl | H | H | H | F | H | H | Br | i-Bu | nonafluoro-2-butyl | Et | CF3 |
| 10-1797 | 2-fluorophenyl | H | H | H | F | H | H | Br | s-Bu | nonafluoro-2-butyl | Me | CF3 |
| 10-1798 | 3-fluorophenyl | H | H | H | F | H | H | Br | s-Bu | nonafluoro-2-butyl | Et | CF3 |
| 10-1804 | 3-bromophenyl | H | H | H | F | H | H | Br | F | nonafluoro-2-butyl | Me | CF3 |
| 10-1805 | 4-bromophenyl | H | H | H | F | H | H | Br | F | nonafluoro-2-butyl | Et | CF3 |
| 10-1811 | (4-trifluoromethyl)phenyl | Et | i-Pr | H | F | H | H | Br | Cl | nonafluoro-2-butyl | Me | CF3 |
| 10-1812 | 2-nitrophenyl | H | H | H | F | H | H | Br | Cl | nonafluoro-2-butyl | Et | CF3 |
| 10-1818 | 4-cyanophenyl | H | H | H | F | H | H | I | Br | heptafluoroisopropyl | Me | CF3 |
| 10-1819 | 2,6-difluorophenyl | H | H | H | F | H | H | I | Br | heptafluoroisopropyl | Et | CF3 |
| 10-1825 | 2-bromo-4-chlorophenyl | n-Pr | Me | H | F | H | H | I | I | heptafluoroisopropyl | Me | CF3 |
| 10-1826 | 2-bromo-4-fluorophenyl | H | H | H | F | H | H | I | I | heptafluoroisopropyl | Et | CF3 |
| 10-1832 | 2-fluoropyridin-3-yl | H | H | H | F | H | H | I | Me | heptafluoroisopropyl | F | C2F5 |
| 10-1833 | 2-chloropyridin-3-yl | H | H | H | F | H | H | I | Me | nonafluoro-2-butyl | Cl | CF3 |
| 10-1834 | 2-bromopyridin-3-yl | H | H | H | F | H | H | I | Me | nonafluoro-2-butyl | Br | n-C3F7 |
| 10-1837 | 2-nitropyridin-3-yl | H | H | H | F | H | H | I | Et | nonafluoro-2-butyl | Cl | CF3 |
| 10-1838 | 2-cyanopyridin-3-yl | H | H | H | F | H | H | I | Et | nonafluoro-2-butyl | Br | CF3 |
| 10-1841 | 6-bromopyridin-3-yl | H | H | H | F | H | H | I | n-Pr | nonafluoro-2-butyl | Cl | CF3 |
| 10-1842 | 6-iodopyridin-3-yl | H | H | H | F | H | H | I | n-Pr | nonafluoro-2-butyl | Br | CF3 |
| 10-1845 | 6-cyanopyridin-3-yl | H | H | H | F | H | H | I | i-Pr | nonafluoro-2-butyl | Cl | CF3 |
| 10-1846 | 5-fluoropyridin-3-yl | n-Pr | C(O)Me | H | F | H | H | I | i-Pr | nonafluoro-2-butyl | Br | CF3 |
| 10-1849 | 5-iodopyridin-3-yl | H | H | H | F | H | H | I | n-Bu | nonafluoro-2-butyl | Cl | CF3 |
| 10-1850 | 5-(trifluoromethyl)pyridin-3-yl | H | H | H | F | H | H | I | n-Bu | nonafluoro-2-butyl | Br | CF3 |
| 10-1853 | 4-fluoropyridin-3-yl | H | H | H | F | H | H | I | i-Bu | nonafluoro-2-butyl | Cl | CF3 |
| 10-1854 | 4-chloropyridin-3-yl | H | H | H | F | H | H | I | i-Bu | nonafluoro-2-butyl | Br | CF3 |
| 10-1857 | 4-(trifluoromethyl)pyridin-3-yl | H | H | H | F | H | H | I | s-Bu | nonafluoro-2-butyl | Cl | CF3 |
| 10-1858 | 4-nitropyridin-3-yl | i-Pr | C(O)Me | H | F | H | H | I | s-Bu | nonafluoro-2-butyl | Br | CF3 |
| 10-1898 | 2-iodopyridin-3-yl | H | H | H | F | H | H | OCF3 | Me | nonafluoro-2-butyl | Me | OCF3 |
| 10-2011 | 3-bromophenyl | H | Et | H | F | H | H | OCF3 | F | heptafluoroisopropyl | F | CF3 |
| 10-2041 | 2-iodopyridin-3-yl | H | H | H | F | H | H | OCF3 | Me | nonafluoro-2-butyl | Me | CF3 |
| 10-2154 | 3-bromophenyl | H | Et | H | F | H | H | CF3 | F | heptafluoroisopropyl | F | CF3 |
| 10-2184 | 2-iodopyridin-3-yl | H | H | H | F | H | H | CF3 | Me | nonafluoro-2-butyl | Me | CF3 |
| 10-2297 | 3-bromophenyl | H | Et | F | F | H | H | Cl | F | heptafluoroisopropyl | F | Cl |
| 10-2327 | 2-iodopyridin-3-yl | H | H | F | F | H | H | Cl | Me | nonafluoro-2-butyl | Me | I |
| 10-2440 | 3-bromophenyl | H | Et | F | F | H | H | Cl | F | heptafluoroisopropyl | F | OCF3 |

**Table 10(5)**

| compound number | Q₁ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 10-2470 | 2-iodopyridin-3-yl | H | H | F | F | H | H | Cl | Me | nonafluoro-2-butyl | Me | OCF3 |
| 10-2584 | 4-bromophenyl | H | H | F | F | H | H | Cl | F | heptafluoroisopropyl | Cl | CF3 |
| 10-2594 | 2-cyanophenyl | n-Pr | n-Pr | F | F | H | H | Cl | Br | pentafluoroethyl | I | CF3 |
| 10-2599 | 2,4-dichlorophenyl | H | H | F | F | H | H | Cl | Me | nonafluoro-2-butyl | H | CF3 |
| 10-2600 | 2-chloro-4-fluorophenyl | H | H | F | F | H | H | Cl | H | nonafluoro-2-butyl | Me | CF3 |
| 10-2613 | 2-iodopyridin-3-yl | H | H | F | F | H | H | Cl | Me | nonafluoro-2-butyl | Me | n-C3F7 |
| 10-2614 | 2-(trifluoromethyl)pyridin-3-yl | H | H | F | F | H | H | Cl | Me | nonafluoro-2-butyl | Et | i-C3F7 |
| 10-2620 | 6-iodopyridin-3-yl | H | H | F | F | H | H | Cl | Et | nonafluoro-2-butyl | Me | n-C3F7 |
| 10-2621 | 6-(trifluoromethyl)pyridin-3-yl | H | H | F | F | H | H | Cl | Et | nonafluoro-2-butyl | Et | i-C3F7 |
| 10-2627 | 5-bromopyridin-3-yl | H | H | F | F | H | H | Br | n-Pr | heptafluoroisopropyl | Me | CF3 |
| 10-2628 | 5-iodopyridin-3-yl | H | H | F | F | H | H | Br | n-Pr | heptafluoroisopropyl | Et | CF3 |
| 10-2634 | 4-bromopyridin-3-yl | Me | n-Pr | F | F | H | H | Br | i-Pr | heptafluoroisopropyl | Me | CF3 |
| 10-2635 | 4-iodopyridin-3-yl | H | H | F | F | H | H | Br | i-Pr | heptafluoroisopropyl | Et | CF3 |
| 10-2641 | pyridin-4-yl | H | H | F | F | H | H | Br | n-Bu | heptafluoroisopropyl | Me | CF3 |
| 10-2642 | 2-chloropyridin-4-yl | H | H | F | F | H | H | Br | n-Bu | heptafluoroisopropyl | Et | CF3 |
| 10-2648 | pyridin-2-yl | H | H | F | F | H | H | Br | i-Bu | nonafluoro-2-butyl | Me | CF3 |
| 10-2649 | 3-chloropyridin-2-yl | H | H | F | F | H | H | Br | i-Bu | nonafluoro-2-butyl | Et | CF3 |
| 10-2655 | 2-fluorophenyl | H | H | F | F | H | H | Br | s-Bu | nonafluoro-2-butyl | Me | CF3 |
| 10-2656 | 3-fluorophenyl | H | H | F | F | H | H | Br | s-Bu | nonafluoro-2-butyl | Et | CF3 |
| 10-2662 | 3-bromophenyl | H | H | F | F | H | H | Br | F | nonafluoro-2-butyl | Me | CF3 |
| 10-2663 | 4-bromophenyl | H | H | F | F | H | H | Br | F | nonafluoro-2-butyl | Et | CF3 |
| 10-2669 | (4-trifluoromethyl)phenyl | Et | i-Pr | F | F | H | H | Br | Cl | nonafluoro-2-butyl | Me | CF3 |
| 10-2670 | 2-nitrophenyl | H | H | F | F | H | H | Br | Cl | nonafluoro-2-butyl | Et | CF3 |
| 10-2676 | 4-cyanophenyl | H | H | F | F | H | H | I | Br | heptafluoroisopropyl | Me | CF3 |
| 10-2677 | 2,6-difluorophenyl | H | H | F | F | H | H | I | Br | heptafluoroisopropyl | Et | CF3 |
| 10-2683 | 2-bromo-4-chlorophenyl | n-Pr | Me | F | F | H | H | I | I | heptafluoroisopropyl | Me | CF3 |
| 10-2684 | 2-bromo-4-fluorophenyl | H | H | F | F | H | H | I | I | heptafluoroisopropyl | Et | CF3 |
| 10-2690 | 2-fluoropyridin-3-yl | H | H | F | F | H | H | I | Me | heptafluoroisopropyl | F | C2F5 |
| 10-2691 | 2-chloropyridin-3-yl | H | H | F | F | H | H | I | Me | nonafluoro-2-butyl | Cl | CF3 |
| 10-2694 | 2-(trifluoromethyl)pyridin-3-yl | H | H | F | F | H | H | I | Et | nonafluoro-2-butyl | F | CF3 |
| 10-2695 | 2-nitropyridin-3-yl | H | H | F | F | H | H | I | Et | nonafluoro-2-butyl | Cl | CF3 |
| 10-2698 | 6-chloropyridin-3-yl | H | H | F | F | H | H | I | n-Pr | nonafluoro-2-butyl | F | CF3 |
| 10-2699 | 6-bromopyridin-3-yl | H | H | F | F | H | H | I | n-Pr | nonafluoro-2-butyl | Cl | CF3 |
| 10-2702 | 6-nitropyridin-3-yl | H | H | F | F | H | H | I | i-Pr | nonafluoro-2-butyl | F | i-C3F7 |
| 10-2703 | 6-cyanopyridin-3-yl | H | H | F | F | H | H | I | i-Pr | nonafluoro-2-butyl | Cl | CF3 |
| 10-2706 | 5-bromopyridin-3-yl | H | H | F | F | H | H | I | n-Bu | nonafluoro-2-butyl | F | CF3 |
| 10-2707 | 5-iodopyridin-3-yl | H | H | F | F | H | H | I | n-Bu | nonafluoro-2-butyl | Cl | CF3 |
| 10-2710 | 5-cyanopyridin-3-yl | H | H | F | F | H | H | I | i-Bu | nonafluoro-2-butyl | F | CF3 |
| 10-2711 | 4-fluoropyridin-3-yl | H | H | F | F | H | H | I | i-Bu | nonafluoro-2-butyl | Cl | CF3 |
| 10-2714 | 4-iodopyridin-3-yl | i-Pr | n-Pr | F | F | H | H | I | s-Bu | nonafluoro-2-butyl | F | CF3 |
| 10-2715 | 4-(trifluoromethyl)pyridin-3-yl | H | H | F | F | H | H | I | s-Bu | nonafluoro-2-butyl | Cl | CF3 |
| 10-2726 | 3-bromophenyl | H | Et | F | F | H | H | OCF3 | F | heptafluoroisopropyl | F | OCF3 |
| 10-2756 | 2-iodopyridin-3-yl | H | H | F | F | H | H | OCF3 | Me | nonafluoro-2-butyl | Me | OCF3 |
| 10-2869 | 3-bromophenyl | H | Et | F | F | H | H | OCF3 | F | heptafluoroisopropyl | F | CF3 |
| 10-2899 | 2-iodopyridin-3-yl | H | H | F | F | H | H | OCF3 | Me | nonafluoro-2-butyl | Me | CF3 |
| 10-3012 | 3-bromophenyl | H | Et | F | F | H | H | CF3 | F | heptafluoroisopropyl | F | CF3 |
| 10-3042 | 2-iodopyridin-3-yl | H | H | F | F | H | H | CF3 | Me | nonafluoro-2-butyl | Me | CF3 |
| 10-3147 | phenyl | H | H | F | F | F | H | Cl | F | pentafluoroethyl | H | Cl |
| 10-3168 | phenyl | H | H | H | H | F | H | Cl | I | pentafluoroethyl | Cl | Cl |
| 10-3189 | phenyl | H | H | H | H | H | F | Cl | Me | pentafluoroethyl | n-Bu | Cl |
| 10-3198 | phenyl | H | H | F | H | F | H | Cl | Et | pentafluoroethyl | s-Bu | Cl |
| 10-3218 | phenyl | H | H | F | H | H | F | Cl | n-Bu | pentafluoroethyl | i-Bu | Cl |
| 10-3227 | phenyl | H | H | H | F | F | H | Cl | s-Bu | pentafluoroethyl | Me | Cl |
| 10-3236 | phenyl | H | H | H | F | H | F | Cl | F | pentafluoroethyl | n-Pr | Cl |
| 10-3245 | phenyl | H | H | H | H | F | F | Cl | Cl | pentafluoroethyl | n-Bu | Cl |
| 10-3254 | phenyl | H | H | F | F | H | F | Cl | Br | pentafluoroethyl | s-Bu | Cl |
| 10-3263 | phenyl | H | H | F | H | F | F | Cl | Me | pentafluoroethyl | Cl | Cl |
| 10-3272 | phenyl | H | H | H | F | F | F | Cl | n-Pr | pentafluoroethyl | Br | Cl |
| 10-3281 | phenyl | i-Pr | Me | F | F | F | F | Cl | n-Bu | pentafluoroethyl | I | Cl |

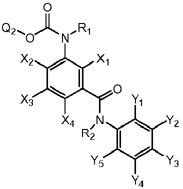

**Table 11(1)**

| compound number | Q₂ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 11-1 | 2,2,2-trichloroethyl | H | H | H | H | H | H | F | H | heptafluoroisopropyl | H | OCF3 |
| 11-2 | 2,2,2-trichloroethyl | H | H | H | H | H | H | F | H | nonafluoro-2-butyl | H | OCF3 |
| 11-13 | 2,2,2-trichloroethyl | H | H | H | H | H | H | Cl | H | heptafluoroisopropyl | H | OCF3 |
| 11-42 | 2,2,2-trichloroethyl | H | H | H | H | H | H | Cl | H | nonafluoro-2-butyl | H | OCF3 |
| 11-71 | 2,2,2-trichloroethyl | H | H | H | H | H | H | Br | H | heptafluoroisopropyl | H | OCF3 |
| 11-100 | 2,2,2-trichloroethyl | H | H | H | H | H | H | Br | H | nonafluoro-2-butyl | H | OCF3 |
| 11-129 | 2,2,2-trichloroethyl | H | H | H | H | H | H | I | H | heptafluoroisopropyl | H | OCF3 |
| 11-158 | 2,2,2-trichloroethyl | H | H | H | H | H | H | I | H | nonafluoro-2-butyl | H | OCF3 |
| 11-189 | 2,2,2-trichloroethyl | H | H | H | H | H | H | Cl | H | heptafluoroisopropyl | H | CF3 |
| 11-218 | 2,2,2-trichloroethyl | H | H | H | H | H | H | Cl | H | nonafluoro-2-butyl | H | CF3 |
| 11-247 | 2,2,2-trichloroethyl | H | H | H | H | H | H | Br | H | heptafluoroisopropyl | H | CF3 |
| 11-260 | 4,4,4-trifluoro-n-butyl | H | H | H | H | H | H | Br | H | pentafluoroethyl | H | CF3 |
| 11-276 | 2,2,2-trichloroethyl | H | H | H | H | H | H | Br | H | nonafluoro-2-butyl | H | CF3 |
| 11-289 | 4,4,4-trifluoro-n-butyl | H | H | H | H | H | H | Br | H | nonafluoro-2-butyl | H | CF3 |
| 11-305 | 2,2,2-trichloroethyl | H | H | H | H | H | H | I | H | heptafluoroisopropyl | H | CF3 |
| 11-318 | 4,4,4-trifluoro-n-butyl | H | H | H | H | H | H | I | H | heptafluoroisopropyl | H | C2F5 |
| 11-334 | 2,2,2-trichloroethyl | H | H | H | H | H | H | I | H | nonafluoro-2-butyl | H | CF3 |
| 11-347 | 4,4,4-trifluoro-n-butyl | H | H | H | H | H | H | I | H | nonafluoro-2-butyl | H | CF3 |
| 11-363 | 2,2,2-trichloroethyl | H | H | H | H | H | H | OCF3 | H | heptafluoroisopropyl | H | OCF3 |
| 11-392 | 2,2,2-trichloroethyl | H | H | H | H | H | H | OCF3 | H | nonafluoro-2-butyl | H | OCF3 |
| 11-421 | 2,2,2-trichloroethyl | H | H | H | H | H | H | CF3 | H | heptafluoroisopropyl | H | OCF3 |
| 11-450 | 2,2,2-trichloroethyl | H | H | H | H | H | H | CF3 | H | nonafluoro-2-butyl | H | OCF3 |
| 11-479 | 2,2,2-trichloroethyl | H | H | H | H | H | H | CF3 | H | heptafluoroisopropyl | H | CF3 |
| 11-508 | 2,2,2-trichloroethyl | H | H | H | H | H | H | CF3 | H | nonafluoro-2-butyl | H | CF3 |
| 11-527 | 2,2,2-trichloroethyl | H | H | F | H | H | H | F | H | heptafluoroisopropyl | H | F |
| 11-528 | 2,2,2-trichloroethyl | H | H | F | H | H | H | Cl | H | heptafluoroisopropyl | H | Cl |
| 11-529 | 2,2,2-trichloroethyl | H | H | F | H | H | H | Br | H | heptafluoroisopropyl | H | Br |
| 11-530 | 2,2,2-trichloroethyl | H | H | F | H | H | H | I | H | heptafluoroisopropyl | H | I |
| 11-531 | 2,2,2-trichloroethyl | H | H | F | H | H | H | F | H | heptafluoroisopropyl | H | OCF3 |
| 11-532 | 2,2,2-trichloroethyl | H | H | F | H | H | H | F | H | nonafluoro-2-butyl | H | OCF3 |
| 11-543 | 2,2,2-trichloroethyl | H | H | F | H | H | H | Cl | H | heptafluoroisopropyl | H | OCF3 |
| 11-556 | 4,4,4-trifluoro-n-butyl | H | H | F | H | H | H | Cl | H | heptafluoroisopropyl | H | OCF3 |
| 11-572 | 2,2,2-trichloroethyl | H | H | F | H | H | H | Cl | H | nonafluoro-2-butyl | H | OCF3 |
| 11-585 | 4,4,4-trifluoro-n-butyl | H | H | F | H | H | H | Cl | H | pentafluoroethyl | H | OCF3 |
| 11-591 | methyl | H | H | F | H | H | H | Br | H | heptafluoroisopropyl | H | OCF3 |
| 11-592 | ethyl | H | H | F | H | H | H | Br | H | heptafluoroisopropyl | H | OCF3 |
| 11-593 | 2-fluoroethyl | H | H | F | H | H | H | Br | H | heptafluoroisopropyl | H | OCF3 |
| 11-594 | 2-chloroethyl | H | H | F | H | H | H | Br | H | heptafluoroisopropyl | H | OCF3 |
| 11-595 | 2-bromoethyl | H | H | F | H | H | H | Br | H | heptafluoroisopropyl | H | OCF3 |
| 11-596 | 2-iodoethyl | H | H | F | H | H | H | Br | H | heptafluoroisopropyl | H | OC2F5 |
| 11-597 | 2-cyanoethyl | H | H | F | H | H | H | Br | H | heptafluoroisopropyl | H | OCF3 |
| 11-598 | 2,2-difluoroethyl | H | H | F | H | H | H | Br | H | heptafluoroisopropyl | H | OCF3 |
| 11-599 | 2,2-dichloroethyl | H | H | F | H | H | H | Br | H | heptafluoroisopropyl | H | OCF3 |
| 11-600 | 2,2,2-trifluoroethyl | H | H | F | H | H | H | Br | H | heptafluoroisopropyl | H | OCF3 |
| 11-601 | 2,2,2-trichloroethyl | H | H | F | H | H | H | Br | H | heptafluoroisopropyl | H | OCF3 |
| 11-602 | 2,2,2-tribromoethyl | H | H | F | H | H | H | Br | H | heptafluoroisopropyl | H | OCF3 |
| 11-603 | n-propyl | H | H | F | H | H | H | Br | H | heptafluoroisopropyl | H | OCF3 |
| 11-604 | 3-fluoro-n-propyl | H | H | F | H | H | H | Br | H | heptafluoroisopropyl | H | OCF3 |
| 11-605 | 3-chloro-n-propyl | H | H | F | H | H | H | Br | H | heptafluoroisopropyl | H | OCF3 |
| 11-606 | 3-bromo-n-propyl | H | H | F | H | H | H | Br | H | heptafluoroisopropyl | H | OCF3 |
| 11-607 | i-propyl | H | H | F | H | H | H | Br | H | heptafluoroisopropyl | H | OCF3 |
| 11-608 | 1,2-difluoro-2-propyl | H | H | F | H | H | H | Br | H | heptafluoroisopropyl | H | OCF3 |
| 11-609 | 1,3-difluoro-2-propyl | H | H | F | H | H | H | Br | H | heptafluoroisopropyl | H | OCF3 |
| 11-610 | 1,3-dichloro-2-propyl | H | H | F | H | H | H | Br | H | heptafluoroisopropyl | H | OCF3 |
| 11-611 | 1-chloro-3-fluoro-2-propyl | H | H | F | H | H | H | Br | H | heptafluoroisopropyl | H | OCF3 |

**Table 11 (2)**

| compound number | Q₂ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 11-612 | 3,3,3-trifluoro-n-propyl | H | H | F | H | H | H | Br | H | heptafluoroisopropyl | H | OCF3 |
| 11-613 | n-butyl | H | H | F | H | H | H | Br | H | heptafluoroisopropyl | H | OCF3 |
| 11-614 | 4,4,4-trifluoro-n-butyl | H | H | F | H | H | H | Br | H | heptafluoroisopropyl | H | OCF3 |
| 11-615 | i-butyl | H | H | F | H | H | H | Br | H | heptafluoroisopropyl | H | OCF3 |
| 11-616 | s-butyl | H | H | F | H | H | H | Br | H | heptafluoroisopropyl | H | OCF3 |
| 11-617 | vinyl | H | H | F | H | H | H | Br | H | heptafluoroisopropyl | H | OCF3 |
| 11-618 | benzyl | H | H | F | H | H | H | Br | H | heptafluoroisopropyl | H | OCF3 |
| 11-619 | phenyl | H | H | F | H | H | H | Br | H | heptafluoroisopropyl | H | OCF3 |
| 11-620 | methyl | H | H | F | H | H | H | Br | H | nonafluoro-2-butyl | H | OCF3 |
| 11-621 | ethyl | H | H | F | H | H | H | Br | H | nonafluoro-2-butyl | H | OCF3 |
| 11-622 | 2-fluoroethyl | H | H | F | H | H | H | Br | H | nonafluoro-2-butyl | H | OCF3 |
| 11-623 | 2-chloroethyl | H | H | F | H | H | H | Br | H | nonafluoro-2-butyl | H | OCF3 |
| 11-624 | 2-bromoethyl | H | H | F | H | H | H | Br | H | nonafluoro-2-butyl | H | OCF3 |
| 11-625 | 2-iodoethyl | H | H | F | H | H | H | Br | H | nonafluoro-2-butyl | H | OCF3 |
| 11-626 | 2-cyanoethyl | H | H | F | H | H | H | Br | H | nonafluoro-2-butyl | H | OCF3 |
| 11-627 | 2,2-difluoroethyl | H | H | F | H | H | H | Br | H | nonafluoro-2-butyl | H | OCF3 |
| 11-628 | 2,2-dichloroethyl | H | H | F | H | H | H | Br | H | nonafluoro-2-butyl | H | OCF3 |
| 11-629 | 2,2,2-trifluoroethyl | H | H | F | H | H | H | Br | H | nonafluoro-2-butyl | H | OCF3 |
| 11-630 | 2,2,2-trichloroethyl | H | H | F | H | H | H | Br | H | nonafluoro-2-butyl | H | OCF3 |
| 11-631 | 2,2,2-tribromoethyl | H | H | F | H | H | H | Br | H | nonafluoro-2-butyl | H | OCF3 |
| 11-632 | n-propyl | H | H | F | H | H | H | Br | H | nonafluoro-2-butyl | H | OC2F5 |
| 11-633 | 3-fluoro-n-propyl | H | H | F | H | H | H | Br | H | nonafluoro-2-butyl | H | OCF3 |
| 11-634 | 3-chloro-n-propyl | H | H | F | H | H | H | Br | H | nonafluoro-2-butyl | H | OCF3 |
| 11-635 | 3-bromo-n-propyl | H | H | F | H | H | H | Br | H | nonafluoro-2-butyl | H | OCF3 |
| 11-636 | i-propyl | H | H | F | H | H | H | Br | H | nonafluoro-2-butyl | H | OCF3 |
| 11-637 | 1,2-difluoro-2-propyl | H | H | F | H | H | H | Br | H | nonafluoro-2-butyl | H | OCF3 |
| 11-638 | 1,3-difluoro-2-propyl | H | H | F | H | H | H | Br | H | nonafluoro-2-butyl | H | OCF3 |
| 11-639 | 1,3-dichloro-2-propyl | H | H | F | H | H | H | Br | H | nonafluoro-2-butyl | H | OCF3 |
| 11-640 | 1-chloro-3-fluoro-2-propyl | H | H | F | H | H | H | Br | H | nonafluoro-2-butyl | H | OCF3 |
| 11-641 | 3,3,3-trifluoro-n-propyl | H | H | F | H | H | H | Br | H | nonafluoro-2-butyl | H | OCF3 |
| 11-642 | n-butyl | H | H | F | H | H | H | Br | H | nonafluoro-2-butyl | H | OCF3 |
| 11-643 | 4,4,4-trifluoro-n-butyl | H | H | F | H | H | H | Br | H | nonafluoro-2-butyl | H | OCF3 |
| 11-644 | i-butyl | H | H | F | H | H | H | Br | H | nonafluoro-2-butyl | H | OCF3 |
| 11-645 | s-butyl | H | H | F | H | H | H | Br | H | nonafluoro-2-butyl | H | OCF3 |
| 11-646 | vinyl | H | H | F | H | H | H | Br | H | nonafluoro-2-butyl | H | OCF3 |
| 11-647 | benzyl | H | H | F | H | H | H | Br | H | nonafluoro-2-butyl | H | OCF3 |
| 11-648 | phenyl | H | H | F | H | H | H | Br | H | nonafluoro-2-butyl | H | OCF3 |
| 11-649 | methyl | H | H | F | H | H | H | I | H | heptafluoroisopropyl | H | OCF3 |
| 11-650 | ethyl | H | H | F | H | H | H | I | H | heptafluoroisopropyl | H | OCF3 |
| 11-651 | 2-fluoroethyl | H | H | F | H | H | H | I | H | heptafluoroisopropyl | H | OCF3 |
| 11-652 | 2-chloroethyl | H | H | F | H | H | H | I | H | heptafluoroisopropyl | H | OCF3 |
| 11-653 | 2-bromoethyl | H | H | F | H | H | H | I | H | heptafluoroisopropyl | H | OCF3 |
| 11-654 | 2-iodoethyl | H | H | F | H | H | H | I | H | heptafluoroisopropyl | H | OCF3 |
| 11-655 | 2-cyanoethyl | H | H | F | H | H | H | I | H | heptafluoroisopropyl | H | OCF3 |
| 11-656 | 2,2-difluoroethyl | H | H | F | H | H | H | I | H | heptafluoroisopropyl | H | OCF3 |
| 11-657 | 2,2-dichloroethyl | H | H | F | H | H | H | I | H | heptafluoroisopropyl | H | OCF3 |
| 11-658 | 2,2,2-trifluoroethyl | H | H | F | H | H | H | I | H | heptafluoroisopropyl | H | OCF3 |
| 11-659 | 2,2,2-trichloroethyl | H | H | F | H | H | H | I | H | heptafluoroisopropyl | H | OCF3 |
| 11-660 | 2,2,2-tribromoethyl | H | H | F | H | H | H | I | H | heptafluoroisopropyl | H | OCF3 |
| 11-661 | n-propyl | H | H | F | H | H | H | I | H | heptafluoroisopropyl | H | OCF3 |
| 11-662 | 3-fluoro-n-propyl | H | H | F | H | H | H | I | H | heptafluoroisopropyl | H | OCF3 |
| 11-663 | 3-chloro-n-propyl | H | H | F | H | H | H | I | H | heptafluoroisopropyl | H | OC2F5 |
| 11-664 | 3-bromo-n-propyl | H | H | F | H | H | H | I | H | heptafluoroisopropyl | H | OCF3 |
| 11-665 | i-propyl | H | H | F | H | H | H | I | H | heptafluoroisopropyl | H | OCF3 |
| 11-666 | 1,2-difluoro-2-propyl | H | H | F | H | H | H | I | H | heptafluoroisopropyl | H | OCF3 |
| 11-667 | 1,3-difluoro-2-propyl | H | H | F | H | H | H | I | H | heptafluoroisopropyl | H | OCF3 |
| 11-668 | 1,3-dichloro-2-propyl | H | H | F | H | H | H | I | H | heptafluoroisopropyl | H | OCF3 |
| 11-669 | 1-chloro-3-fluoro-2-propyl | H | H | F | H | H | H | I | H | heptafluoroisopropyl | H | OCF3 |
| 11-670 | 3,3,3-trifluoro-n-propyl | H | H | F | H | H | H | I | H | heptafluoroisopropyl | H | OCF3 |
| 11-671 | n-butyl | H | H | F | H | H | H | I | H | heptafluoroisopropyl | H | OCF3 |
| 11-672 | 4,4,4-trifluoro-n-butyl | H | H | F | H | H | H | I | H | heptafluoroisopropyl | H | OCF3 |
| 11-673 | i-butyl | H | H | F | H | H | H | I | H | heptafluoroisopropyl | H | OCF3 |
| 11-674 | s-butyl | H | H | F | H | H | H | I | H | heptafluoroisopropyl | H | OCF3 |
| 11-675 | vinyl | H | H | F | H | H | H | I | H | heptafluoroisopropyl | H | OCF3 |

**Table 11 (3)**

| compound number | Q₂ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 11-676 | benzyl | H | H | F | H | H | H | I | H | heptafluoroisopropyl | H | OCF3 |
| 11-677 | phenyl | H | H | F | H | H | H | I | H | heptafluoroisopropyl | H | OCF3 |
| 11-678 | methyl | H | H | F | H | H | H | I | H | nonafluoro-2-butyl | H | OCF3 |
| 11-679 | ethyl | H | H | F | H | H | H | I | H | nonafluoro-2-butyl | H | OCF3 |
| 11-680 | 2-fluoroethyl | H | H | F | H | H | H | I | H | nonafluoro-2-butyl | H | OCF3 |
| 11-681 | 2-chloroethyl | H | H | F | H | H | H | I | H | nonafluoro-2-butyl | H | OCF3 |
| 11-682 | 2-bromoethyl | H | H | F | H | H | H | I | H | nonafluoro-2-butyl | H | OCF3 |
| 11-683 | 2-iodoethyl | H | H | F | H | H | H | I | H | nonafluoro-2-butyl | H | OCF3 |
| 11-684 | 2-cyanoethyl | H | H | F | H | H | H | I | H | nonafluoro-2-butyl | H | OCF3 |
| 11-685 | 2,2-difluoroethyl | H | H | F | H | H | H | I | H | nonafluoro-2-butyl | H | OCF3 |
| 11-686 | 2,2-dichloroethyl | H | H | F | H | H | H | I | H | nonafluoro-2-butyl | H | OCF3 |
| 11-687 | 2,2,2-trifluoroethyl | H | H | F | H | H | H | I | H | nonafluoro-2-butyl | H | OCF3 |
| 11-688 | 2,2,2-trichloroethyl | H | H | F | H | H | H | I | H | nonafluoro-2-butyl | H | OCF3 |
| 11-689 | 2,2,2-tribromoethyl | H | H | F | H | H | H | I | H | nonafluoro-2-butyl | H | OCF3 |
| 11-690 | n-propyl | H | H | F | H | H | H | I | H | nonafluoro-2-butyl | H | OCF3 |
| 11-691 | 3-fluoro-n-propyl | H | H | F | H | H | H | I | H | nonafluoro-2-butyl | H | OCF3 |
| 11-692 | 3-chloro-n-propyl | H | H | F | H | H | H | I | H | nonafluoro-2-butyl | H | OCF3 |
| 11-693 | 3-bromo-n-propyl | H | H | F | H | H | H | I | H | nonafluoro-2-butyl | H | OCF3 |
| 11-694 | i-propyl | H | H | F | H | H | H | I | H | nonafluoro-2-butyl | H | OCF3 |
| 11-695 | 1,2-difluoro-2-propyl | H | H | F | H | H | H | I | H | nonafluoro-2-butyl | H | OCF3 |
| 11-696 | 1,3-difluoro-2-propyl | H | H | F | H | H | H | I | H | nonafluoro-2-butyl | H | OCF3 |
| 11-697 | 1,3-dichloro-2-propyl | H | H | F | H | H | H | I | H | nonafluoro-2-butyl | H | OCF3 |
| 11-698 | 1-chloro-3-fluoro-2-propyl | H | H | F | H | H | H | I | H | nonafluoro-2-butyl | H | OCF3 |
| 11-699 | 3,3,3-trifluoro-n-propyl | H | H | F | H | H | H | I | H | nonafluoro-2-butyl | H | OCF3 |
| 11-700 | n-butyl | H | H | F | H | H | H | I | H | nonafluoro-2-butyl | H | OCF3 |
| 11-701 | 4,4,4-trifluoro-n-butyl | H | H | F | H | H | H | I | H | nonafluoro-2-butyl | H | OCF3 |
| 11-702 | i-butyl | H | H | F | H | H | H | I | H | nonafluoro-2-butyl | H | OCF3 |
| 11-703 | s-butyl | H | H | F | H | H | H | I | H | nonafluoro-2-butyl | H | OCF3 |
| 11-704 | vinyl | H | H | F | H | H | H | I | H | nonafluoro-2-butyl | H | OCF3 |
| 11-705 | benzyl | H | H | F | H | H | H | I | H | nonafluoro-2-butyl | H | OC2F5 |
| 11-706 | phenyl | H | H | F | H | H | H | I | H | nonafluoro-2-butyl | H | OCF3 |
| 11-707 | 2,2,2-trichloroethyl | H | H | F | H | H | H | F | H | heptafluoroisopropyl | H | CF3 |
| 11-708 | 2,2,2-trichloroethyl | H | H | F | H | H | H | F | H | nonafluoro-2-butyl | H | CF3 |
| 11-709 | methyl | H | H | F | H | H | H | Cl | H | heptafluoroisopropyl | H | CF3 |
| 11-710 | ethyl | H | H | F | H | H | H | Cl | H | heptafluoroisopropyl | H | CF3 |
| 11-711 | 2-fluoroethyl | H | H | F | H | H | H | Cl | H | heptafluoroisopropyl | H | CF3 |
| 11-712 | 2-chloroethyl | H | H | F | H | H | H | Cl | H | heptafluoroisopropyl | H | CF3 |
| 11-713 | 2-bromoethyl | H | H | F | H | H | H | Cl | H | heptafluoroisopropyl | H | CF3 |
| 11-714 | 2-iodoethyl | H | H | F | H | H | H | Cl | H | heptafluoroisopropyl | H | CF3 |
| 11-715 | 2-cyanoethyl | H | H | F | H | H | H | Cl | H | heptafluoroisopropyl | H | C2F5 |
| 11-716 | 2,2-difluoroethyl | H | H | F | H | H | H | Cl | H | heptafluoroisopropyl | H | CF3 |
| 11-717 | 2,2-dichloroethyl | H | H | F | H | H | H | Cl | H | heptafluoroisopropyl | H | CF3 |
| 11-718 | 2,2,2-trifluoroethyl | H | H | F | H | H | H | Cl | H | heptafluoroisopropyl | H | CF3 |
| 11-719 | 2,2,2-trichloroethyl | H | H | F | H | H | H | Cl | H | heptafluoroisopropyl | H | CF3 |
| 11-720 | 2,2,2-tribromoethyl | H | H | F | H | H | H | Cl | H | heptafluoroisopropyl | H | CF3 |
| 11-721 | n-propyl | H | H | F | H | H | H | Cl | H | pentafluoroethyl | H | CF3 |
| 11-722 | 3-fluoro-n-propyl | H | H | F | H | H | H | Cl | H | heptafluoroisopropyl | H | CF3 |
| 11-723 | 3-chloro-n-propyl | H | H | F | H | H | H | Cl | H | heptafluoroisopropyl | H | CF3 |
| 11-724 | 3-bromo-n-propyl | H | H | F | H | H | H | Cl | H | heptafluoroisopropyl | H | CF3 |
| 11-725 | i-propyl | H | H | F | H | H | H | Cl | H | heptafluoroisopropyl | H | n-C3F7 |
| 11-726 | 1,2-difluoro-2-propyl | H | H | F | H | H | H | Cl | H | heptafluoroisopropyl | H | CF3 |
| 11-727 | 1,3-difluoro-2-propyl | H | H | F | H | H | H | Cl | H | heptafluoroisopropyl | H | CF3 |
| 11-728 | 1,3-dichloro-2-propyl | H | H | F | H | H | H | Cl | H | pentafluoroethyl | H | CF3 |
| 11-729 | 1-chloro-3-fluoro-2-propyl | H | H | F | H | H | H | Cl | H | heptafluoroisopropyl | H | CF3 |
| 11-730 | 3,3,3-trifluoro-n-propyl | H | H | F | H | H | H | Cl | H | heptafluoroisopropyl | H | CF3 |
| 11-731 | n-butyl | H | H | F | H | H | H | Cl | H | heptafluoroisopropyl | H | CF3 |
| 11-732 | 4,4,4-trifluoro-n-butyl | H | H | F | H | H | H | Cl | H | heptafluoroisopropyl | H | CF3 |
| 11-733 | i-butyl | H | H | F | H | H | H | Cl | H | heptafluoroisopropyl | H | CF3 |
| 11-734 | s-butyl | H | H | F | H | H | H | Cl | H | heptafluoroisopropyl | H | CF3 |
| 11-735 | vinyl | H | H | F | H | H | H | Cl | H | heptafluoroisopropyl | H | CF3 |
| 11-736 | benzyl | H | H | F | H | H | H | Cl | H | heptafluoroisopropyl | H | CF3 |
| 11-737 | phenyl | H | H | F | H | H | H | Cl | H | heptafluoroisopropyl | H | CF3 |
| 11-738 | methyl | H | H | F | H | H | H | Cl | H | nonafluoro-2-butyl | H | CF3 |
| 11-739 | ethyl | H | H | F | H | H | H | Cl | H | nonafluoro-2-butyl | H | i-C3F7 |

**Table 11 (4)**

| compound number | Q₂ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 11-740 | 2-fluoroethyl | H | H | F | H | H | H | Cl | H | nonafluoro-2-butyl | H | CF3 |
| 11-741 | 2-chloroethyl | H | H | F | H | H | H | Cl | H | nonafluoro-2-butyl | H | CF3 |
| 11-742 | 2-bromoethyl | H | H | F | H | H | H | Cl | H | nonafluoro-2-butyl | H | CF3 |
| 11-743 | 2-iodoethyl | H | H | F | H | H | H | Cl | H | nonafluoro-2-butyl | H | CF3 |
| 11-744 | 2-cyanoethyl | H | H | F | H | H | H | Cl | H | nonafluoro-2-butyl | H | CF3 |
| 11-745 | 2,2-difluoroethyl | H | H | F | H | H | H | Cl | H | nonafluoro-2-butyl | H | CF3 |
| 11-746 | 2,2-dichloroethyl | H | H | F | H | H | H | Cl | H | nonafluoro-2-butyl | H | CF3 |
| 11-747 | 2,2,2-trifluoroethyl | H | H | F | H | H | H | Cl | H | nonafluoro-2-butyl | H | CF3 |
| 11-748 | 2,2,2-trichloroethyl | H | H | F | H | H | H | Cl | H | nonafluoro-2-butyl | H | CF3 |
| 11-749 | 2,2,2-tribromoethyl | H | H | F | H | H | H | Cl | H | nonafluoro-2-butyl | H | CF3 |
| 11-750 | n-propyl | H | H | F | H | H | H | Cl | H | nonafluoro-2-butyl | H | CF3 |
| 11-751 | 3-fluoro-n-propyl | H | H | F | H | H | H | Cl | H | nonafluoro-2-butyl | H | CF3 |
| 11-752 | 3-chloro-n-propyl | H | H | F | H | H | H | Cl | H | nonafluoro-2-butyl | H | CF3 |
| 11-753 | 3-bromo-n-propyl | H | H | F | H | H | H | Cl | H | nonafluoro-2-butyl | H | CF3 |
| 11-754 | i-propyl | H | H | F | H | H | H | Cl | H | nonafluoro-2-butyl | H | CF3 |
| 11-755 | 1,2-difluoro-2-propyl | H | H | F | H | H | H | Cl | H | nonafluoro-2-butyl | H | CF3 |
| 11-756 | 1,3-difluoro-2-propyl | H | H | F | H | H | H | Cl | H | nonafluoro-2-butyl | H | CF3 |
| 11-757 | 1,3-dichloro-2-propyl | H | H | F | H | H | H | Cl | H | nonafluoro-2-butyl | H | CF3 |
| 11-758 | 1-chloro-3-fluoro-2-propyl | H | H | F | H | H | H | Cl | H | nonafluoro-2-butyl | H | CF3 |
| 11-759 | 3,3,3-trifluoro-n-propyl | H | H | F | H | H | H | Cl | H | nonafluoro-2-butyl | H | CF3 |
| 11-760 | n-butyl | H | H | F | H | H | H | Cl | H | nonafluoro-2-butyl | H | CF3 |
| 11-761 | 4,4,4-trifluoro-n-butyl | H | H | F | H | H | H | Cl | H | nonafluoro-2-butyl | H | CF3 |
| 11-762 | i-butyl | H | H | F | H | H | H | Cl | H | nonafluoro-2-butyl | H | CF3 |
| 11-763 | s-butyl | H | H | F | H | H | H | Cl | H | nonafluoro-2-butyl | H | CF3 |
| 11-764 | vinyl | H | H | F | H | H | H | Cl | H | nonafluoro-2-butyl | H | CF3 |
| 11-765 | benzyl | H | H | F | H | H | H | Cl | H | nonafluoro-2-butyl | H | CF3 |
| 11-766 | phenyl | H | H | F | H | H | H | Cl | H | nonafluoro-2-butyl | H | CF3 |
| 11-767 | methyl | H | H | F | H | H | H | Br | H | heptafluoroisopropyl | H | CF3 |
| 11-768 | ethyl | H | H | F | H | H | H | Br | H | heptafluoroisopropyl | H | CF3 |
| 11-769 | 2-fluoroethyl | H | H | F | H | H | H | Br | H | heptafluoroisopropyl | H | CF3 |
| 11-770 | 2-chloroethyl | H | H | F | H | H | H | Br | H | heptafluoroisopropyl | H | CF3 |
| 11-771 | 2-bromoethyl | H | H | F | H | H | H | Br | H | heptafluoroisopropyl | H | CF3 |
| 11-772 | 2-iodoethyl | H | H | F | H | H | H | Br | H | heptafluoroisopropyl | H | CF3 |
| 11-773 | 2-cyanoethyl | H | H | F | H | H | H | Br | H | heptafluoroisopropyl | H | C2F5 |
| 11-774 | 2,2-difluoroethyl | H | H | F | H | H | H | Br | H | heptafluoroisopropyl | H | CF3 |
| 11-775 | 2,2-dichloroethyl | H | H | F | H | H | H | Br | H | heptafluoroisopropyl | H | CF3 |
| 11-776 | 2,2,2-trifluoroethyl | H | H | F | H | H | H | Br | H | heptafluoroisopropyl | H | CF3 |
| 11-777 | 2,2,2-trichloroethyl | H | H | F | H | H | H | Br | H | heptafluoroisopropyl | H | CF3 |
| 11-778 | 2,2,2-tribromoethyl | H | H | F | H | H | H | Br | H | heptafluoroisopropyl | H | CF3 |
| 11-779 | n-propyl | H | H | F | H | H | H | Br | H | heptafluoroisopropyl | H | CF3 |
| 11-780 | 3-fluoro-n-propyl | H | H | F | H | H | H | Br | H | heptafluoroisopropyl | H | CF3 |
| 11-781 | 3-chloro-n-propyl | H | H | F | H | H | H | Br | H | heptafluoroisopropyl | H | CF3 |
| 11-782 | 3-bromo-n-propyl | H | H | F | H | H | H | Br | H | heptafluoroisopropyl | H | CF3 |
| 11-783 | i-propyl | H | H | F | H | H | H | Br | H | heptafluoroisopropyl | H | CF3 |
| 11-784 | 1,2-difluoro-2-propyl | H | H | F | H | H | H | Br | H | heptafluoroisopropyl | H | n-C3F7 |
| 11-785 | 1,3-difluoro-2-propyl | H | H | F | H | H | H | Br | H | heptafluoroisopropyl | H | CF3 |
| 11-786 | 1,3-dichloro-2-propyl | H | H | F | H | H | H | Br | H | heptafluoroisopropyl | H | CF3 |
| 11-787 | 1-chloro-3-fluoro-2-propyl | H | H | F | H | H | H | Br | H | heptafluoroisopropyl | H | CF3 |
| 11-788 | 3,3,3-trifluoro-n-propyl | H | H | F | H | H | H | Br | H | heptafluoroisopropyl | H | CF3 |
| 11-789 | n-butyl | H | H | F | H | H | H | Br | H | heptafluoroisopropyl | H | CF3 |
| 11-790 | 4,4,4-trifluoro-n-butyl | H | H | F | H | H | H | Br | H | heptafluoroisopropyl | H | CF3 |
| 11-791 | i-butyl | H | H | F | H | H | H | Br | H | heptafluoroisopropyl | H | CF3 |
| 11-792 | s-butyl | H | H | F | H | H | H | Br | H | heptafluoroisopropyl | H | CF3 |
| 11-793 | vinyl | H | H | F | H | H | H | Br | H | heptafluoroisopropyl | H | CF3 |
| 11-794 | benzyl | H | H | F | H | H | H | Br | H | heptafluoroisopropyl | H | CF3 |
| 11-795 | phenyl | H | H | F | H | H | H | Br | H | heptafluoroisopropyl | H | CF3 |
| 11-796 | methyl | H | H | F | H | H | H | Br | H | nonafluoro-2-butyl | H | CF3 |
| 11-797 | ethyl | H | H | F | H | H | H | Br | H | nonafluoro-2-butyl | H | CF3 |
| 11-798 | 2-fluoroethyl | H | H | F | H | H | H | Br | H | nonafluoro-2-butyl | H | CF3 |
| 11-799 | 2-chloroethyl | H | H | F | H | H | H | Br | H | nonafluoro-2-butyl | H | CF3 |
| 11-800 | 2-bromoethyl | H | H | F | H | H | H | Br | H | nonafluoro-2-butyl | H | CF3 |
| 11-801 | 2-iodoethyl | H | H | F | H | H | H | Br | H | nonafluoro-2-butyl | H | CF3 |
| 11-802 | 2-cyanoethyl | H | H | F | H | H | H | Br | H | nonafluoro-2-butyl | H | CF3 |
| 11-803 | 2,2-difluoroethyl | H | H | F | H | H | H | Br | H | nonafluoro-2-butyl | H | CF3 |

**Table 11 (5)**

| compound number | Q₂ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 11-804 | 2,2-dichloroethyl | H | H | F | H | H | H | Br | H | nonafluoro-2-butyl | H | CF3 |
| 11-805 | 2,2,2-trifluoroethyl | H | H | F | H | H | H | Br | H | nonafluoro-2-butyl | H | CF3 |
| 11-806 | 2,2,2-trichloroethyl | H | H | F | H | H | H | Br | H | nonafluoro-2-butyl | H | CF3 |
| 11-807 | 2,2,2-tribromoethyl | H | H | F | H | H | H | Br | H | nonafluoro-2-butyl | H | CF3 |
| 11-808 | n-propyl | H | H | F | H | H | H | Br | H | nonafluoro-2-butyl | H | CF3 |
| 11-809 | 3-fluoro-n-propyl | H | H | F | H | H | H | Br | H | nonafluoro-2-butyl | H | CF3 |
| 11-810 | 3-chloro-n-propyl | H | H | F | H | H | H | Br | H | nonafluoro-2-butyl | H | CF3 |
| 11-811 | 3-bromo-n-propyl | H | H | F | H | H | H | Br | H | nonafluoro-2-butyl | H | CF3 |
| 11-812 | i-propyl | H | H | F | H | H | H | Br | H | nonafluoro-2-butyl | H | CF3 |
| 11-813 | 1,2-difluoro-2-propyl | H | H | F | H | H | H | Br | H | nonafluoro-2-butyl | H | CF3 |
| 11-814 | 1,3-difluoro-2-propyl | H | H | F | H | H | H | Br | H | nonafluoro-2-butyl | H | CF3 |
| 11-815 | 1,3-dichloro-2-propyl | H | H | F | H | H | H | Br | H | nonafluoro-2-butyl | H | CF3 |
| 11-816 | 1-chloro-3-fluoro-2-propyl | H | H | F | H | H | H | Br | H | nonafluoro-2-butyl | H | CF3 |
| 11-817 | 3,3,3-trifluoro-n-propyl | H | H | F | H | H | H | Br | H | nonafluoro-2-butyl | H | CF3 |
| 11-818 | n-butyl | H | H | F | H | H | H | Br | H | nonafluoro-2-butyl | H | CF3 |
| 11-819 | 4,4,4-trifluoro-n-butyl | H | H | F | H | H | H | Br | H | nonafluoro-2-butyl | H | CF3 |
| 11-820 | i-butyl | H | H | F | H | H | H | Br | H | nonafluoro-2-butyl | H | CF3 |
| 11-821 | s-butyl | H | H | F | H | H | H | Br | H | nonafluoro-2-butyl | H | CF3 |
| 11-822 | vinyl | H | H | F | H | H | H | Br | H | nonafluoro-2-butyl | H | CF3 |
| 11-823 | benzyl | H | H | F | H | H | H | Br | H | nonafluoro-2-butyl | H | CF3 |
| 11-824 | phenyl | H | H | F | H | H | H | Br | H | nonafluoro-2-butyl | H | i-C3F7 |
| 11-825 | methyl | H | H | F | H | H | H | I | H | heptafluoroisopropyl | H | CF3 |
| 11-826 | ethyl | H | H | F | H | H | H | I | H | heptafluoroisopropyl | H | CF3 |
| 11-827 | 2-fluoroethyl | H | H | F | H | H | H | I | H | heptafluoroisopropyl | H | CF3 |
| 11-828 | 2-chloroethyl | H | H | F | H | H | H | I | H | heptafluoroisopropyl | H | CF3 |
| 11-829 | 2-bromoethyl | H | H | F | H | H | H | I | H | heptafluoroisopropyl | H | CF3 |
| 11-830 | 2-iodoethyl | H | H | F | H | H | H | I | H | heptafluoroisopropyl | H | CF3 |
| 11-831 | 2-cyanoethyl | H | H | F | H | H | H | I | H | heptafluoroisopropyl | H | CF3 |
| 11-832 | 2,2-difluoroethyl | H | H | F | H | H | H | I | H | heptafluoroisopropyl | H | CF3 |
| 11-833 | 2,2-dichloroethyl | H | H | F | H | H | H | I | H | heptafluoroisopropyl | H | CF3 |
| 11-834 | 2,2,2-trifluoroethyl | H | H | F | H | H | H | I | H | heptafluoroisopropyl | H | CF3 |
| 11-835 | 2,2,2-trichloroethyl | H | H | F | H | H | H | I | H | heptafluoroisopropyl | H | CF3 |
| 11-836 | 2,2,2-tribromoethyl | H | H | F | H | H | H | I | H | heptafluoroisopropyl | H | CF3 |
| 11-837 | n-propyl | H | H | F | H | H | H | I | H | heptafluoroisopropyl | H | CF3 |
| 11-838 | 3-fluoro-n-propyl | H | H | F | H | H | H | I | H | heptafluoroisopropyl | H | CF3 |
| 11-839 | 3-chloro-n-propyl | H | H | F | H | H | H | I | H | heptafluoroisopropyl | H | CF3 |
| 11-840 | 3-bromo-n-propyl | H | H | F | H | H | H | I | H | heptafluoroisopropyl | H | C2F5 |
| 11-841 | i-propyl | H | H | F | H | H | H | I | H | heptafluoroisopropyl | H | CF3 |
| 11-842 | 1,2-difluoro-2-propyl | H | H | F | H | H | H | I | H | heptafluoroisopropyl | H | CF3 |
| 11-843 | 1,3-difluoro-2-propyl | H | H | F | H | H | H | I | H | heptafluoroisopropyl | H | CF3 |
| 11-844 | 1,3-dichloro-2-propyl | H | H | F | H | H | H | I | H | heptafluoroisopropyl | H | CF3 |
| 11-845 | 1-chloro-3-fluoro-2-propyl | H | H | F | H | H | H | I | H | heptafluoroisopropyl | H | CF3 |
| 11-846 | 3,3,3-trifluoro-n-propyl | H | H | F | H | H | H | I | H | heptafluoroisopropyl | H | CF3 |
| 11-847 | n-butyl | H | H | F | H | H | H | I | H | heptafluoroisopropyl | H | CF3 |
| 11-848 | 4,4,4-trifluoro-n-butyl | H | H | F | H | H | H | I | H | heptafluoroisopropyl | H | CF3 |
| 11-849 | i-butyl | H | H | F | H | H | H | I | H | heptafluoroisopropyl | H | CF3 |
| 11-850 | s-butyl | H | H | F | H | H | H | I | H | heptafluoroisopropyl | H | CF3 |
| 11-851 | vinyl | H | H | F | H | H | H | I | H | heptafluoroisopropyl | H | CF3 |
| 11-852 | benzyl | H | H | F | H | H | H | I | H | heptafluoroisopropyl | H | CF3 |
| 11-853 | phenyl | H | H | F | H | H | H | I | H | heptafluoroisopropyl | H | CF3 |
| 11-854 | methyl | H | H | F | H | H | H | I | H | nonafluoro-2-butyl | H | CF3 |
| 11-855 | ethyl | H | H | F | H | H | H | I | H | nonafluoro-2-butyl | H | CF3 |
| 11-856 | 2-fluoroethyl | H | H | F | H | H | H | I | H | nonafluoro-2-butyl | H | CF3 |
| 11-857 | 2-chloroethyl | H | H | F | H | H | H | I | H | nonafluoro-2-butyl | H | CF3 |
| 11-858 | 2-bromoethyl | H | H | F | H | H | H | I | H | nonafluoro-2-butyl | H | CF3 |
| 11-859 | 2-iodoethyl | H | H | F | H | H | H | I | H | nonafluoro-2-butyl | H | CF3 |
| 11-860 | 2-cyanoethyl | H | H | F | H | H | H | I | H | nonafluoro-2-butyl | H | CF3 |
| 11-861 | 2,2-difluoroethyl | H | H | F | H | H | H | I | H | nonafluoro-2-butyl | H | CF3 |
| 11-862 | 2,2-dichloroethyl | H | H | F | H | H | H | I | H | nonafluoro-2-butyl | H | CF3 |
| 11-863 | 2,2,2-trifluoroethyl | H | H | F | H | H | H | I | H | nonafluoro-2-butyl | H | CF3 |
| 11-864 | 2,2,2-trichloroethyl | H | H | F | H | H | H | I | H | nonafluoro-2-butyl | H | CF3 |
| 11-865 | 2,2,2-tribromoethyl | H | H | F | H | H | H | I | H | nonafluoro-2-butyl | H | CF3 |
| 11-866 | n-propyl | H | H | F | H | H | H | I | H | nonafluoro-2-butyl | H | n-C3F7 |
| 11-867 | 3-fluoro-n-propyl | H | H | F | H | H | H | I | H | nonafluoro-2-butyl | H | CF3 |

**Table 11(6)**

| compound number | Q₂ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 11-868 | 3-chloro-n-propyl | H | H | F | H | H | H | I | H | nonafluoro-2-butyl | H | CF3 |
| 11-869 | 3-bromo-n-propyl | H | H | F | H | H | H | I | H | nonafluoro-2-butyl | H | CF3 |
| 11-870 | i-propyl | H | H | F | H | H | H | I | H | nonafluoro-2-butyl | H | CF3 |
| 11-871 | 1,2-difluoro-2-propyl | H | H | F | H | H | H | I | H | nonafluoro-2-butyl | H | CF3 |
| 11-872 | 1,3-difluoro-2-propyl | H | H | F | H | H | H | I | H | nonafluoro-2-butyl | H | CF3 |
| 11-873 | 1,3-dichloro-2-propyl | H | H | F | H | H | H | I | H | nonafluoro-2-butyl | H | CF3 |
| 11-874 | 1-chloro-3-fluoro-2-propyl | H | H | F | H | H | H | I | H | nonafluoro-2-butyl | H | CF3 |
| 11-875 | 3,3,3-trifluoro-n-propyl | H | H | F | H | H | H | I | H | nonafluoro-2-butyl | H | CF3 |
| 11-876 | n-butyl | H | H | F | H | H | H | I | H | nonafluoro-2-butyl | H | CF3 |
| 11-877 | 4,4,4-trifluoro-n-butyl | H | H | F | H | H | H | I | H | nonafluoro-2-butyl | H | CF3 |
| 11-878 | i-butyl | H | H | F | H | H | H | I | H | nonafluoro-2-butyl | H | CF3 |
| 11-879 | s-butyl | H | H | F | H | H | H | I | H | nonafluoro-2-butyl | H | CF3 |
| 11-880 | vinyl | H | H | F | H | H | H | I | H | nonafluoro-2-butyl | H | i-C3F7 |
| 11-881 | benzyl | H | H | F | H | H | H | I | H | nonafluoro-2-butyl | H | CF3 |
| 11-882 | phenyl | H | H | F | H | H | H | I | H | nonafluoro-2-butyl | H | CF3 |
| 11-893 | 2,2,2-trichloroethyl | H | H | F | H | H | H | OCF3 | H | heptafluoroisopropyl | H | OCF3 |
| 11-922 | 2,2,2-trichloroethyl | H | H | F | H | H | H | OCF3 | H | nonafluoro-2-butyl | H | OCF3 |
| 11-951 | 2,2,2-trichloroethyl | H | H | F | H | H | H | CF3 | H | heptafluoroisopropyl | H | OCF3 |
| 11-980 | 2,2,2-trichloroethyl | H | H | F | H | H | H | CF3 | H | nonafluoro-2-butyl | H | OCF3 |
| 11-999 | methyl | H | H | F | H | H | H | CF3 | H | heptafluoroisopropyl | H | CF3 |
| 11-1000 | ethyl | H | H | F | H | H | H | CF3 | H | heptafluoroisopropyl | H | CF3 |
| 11-1001 | 2-fluoroethyl | H | H | F | H | H | H | CF3 | H | heptafluoroisopropyl | H | CF3 |
| 11-1002 | 2-chloroethyl | H | H | F | H | H | H | CF3 | H | heptafluoroisopropyl | H | CF3 |
| 11-1003 | 2-bromoethyl | H | H | F | H | H | H | CF3 | H | heptafluoroisopropyl | H | CF3 |
| 11-1004 | 2-iodoethyl | H | H | F | H | H | H | CF3 | H | heptafluoroisopropyl | H | CF3 |
| 11-1005 | 2-cyanoethyl | H | H | F | H | H | H | CF3 | H | heptafluoroisopropyl | H | CF3 |
| 11-1006 | 2,2-difluoroethyl | H | H | F | H | H | H | CF3 | H | heptafluoroisopropyl | H | CF3 |
| 11-1007 | 2,2-dichloroethyl | H | H | F | H | H | H | CF3 | H | heptafluoroisopropyl | H | CF3 |
| 11-1008 | 2,2,2-trifluoroethyl | H | H | F | H | H | H | CF3 | H | heptafluoroisopropyl | H | CF3 |
| 11-1009 | 2,2,2-trichloroethyl | H | H | F | H | H | H | CF3 | H | heptafluoroisopropyl | H | CF3 |
| 11-1010 | 2,2,2-tribromoethyl | H | H | F | H | H | H | CF3 | H | heptafluoroisopropyl | H | CF3 |
| 11-1011 | n-propyl | H | H | F | H | H | H | CF3 | H | heptafluoroisopropyl | H | CF3 |
| 11-1012 | 3-fluoro-n-propyl | H | H | F | H | H | H | C2F5 | H | heptafluoroisopropyl | H | CF3 |
| 11-1013 | 3-chloro-n-propyl | H | H | F | H | H | H | CF3 | H | heptafluoroisopropyl | H | CF3 |
| 11-1014 | 3-bromo-n-propyl | H | H | F | H | H | H | CF3 | H | heptafluoroisopropyl | H | CF3 |
| 11-1015 | i-propyl | H | H | F | H | H | H | i-C3F7 | H | heptafluoroisopropyl | H | CF3 |
| 11-1016 | 1,2-difluoro-2-propyl | H | H | F | H | H | H | CF3 | H | heptafluoroisopropyl | H | CF3 |
| 11-1017 | 1,3-difluoro-2-propyl | H | H | F | H | H | H | CF3 | H | pentafluoroethyl | H | CF3 |
| 11-1018 | 1,3-dichloro-2-propyl | H | H | F | H | H | H | CF3 | H | heptafluoroisopropyl | H | CF3 |
| 11-1019 | 1-chloro-3-fluoro-2-propyl | H | H | F | H | H | H | CF3 | H | heptafluoroisopropyl | H | CF3 |
| 11-1020 | 3,3,3-trifluoro-n-propyl | H | H | F | H | H | H | CF3 | H | heptafluoroisopropyl | H | CF3 |
| 11-1021 | n-butyl | H | H | F | H | H | H | C2F5 | H | heptafluoroisopropyl | H | C2F5 |
| 11-1022 | 4,4,4-trifluoro-n-butyl | H | H | F | H | H | H | CF3 | H | heptafluoroisopropyl | H | CF3 |
| 11-1023 | i-butyl | H | H | F | H | H | H | C2F5 | H | heptafluoroisopropyl | H | n-C3F7 |
| 11-1024 | s-butyl | H | H | F | H | H | H | CF3 | H | heptafluoroisopropyl | H | CF3 |
| 11-1025 | vinyl | H | H | F | H | H | H | n-C3F7 | H | heptafluoroisopropyl | H | CF3 |
| 11-1026 | benzyl | H | H | F | H | H | H | CF3 | H | heptafluoroisopropyl | H | CF3 |
| 11-1027 | phenyl | H | H | F | H | H | H | CF3 | H | heptafluoroisopropyl | H | CF3 |
| 11-1030 | 2-fluoroethyl | H | H | F | H | H | H | CF3 | H | nonafluoro-2-butyl | H | CF3 |
| 11-1031 | 2-chloroethyl | H | H | F | H | H | H | CF3 | H | nonafluoro-2-butyl | H | CF3 |
| 11-1032 | 2-bromoethyl | H | H | F | H | H | H | CF3 | H | nonafluoro-2-butyl | H | CF3 |
| 11-1033 | 2-iodoethyl | H | H | F | H | H | H | CF3 | H | nonafluoro-2-butyl | H | CF3 |
| 11-1035 | 2,2-difluoroethyl | H | H | F | H | H | H | CF3 | H | nonafluoro-2-butyl | H | CF3 |
| 11-1036 | 2,2-dichloroethyl | H | H | F | H | H | H | CF3 | H | nonafluoro-2-butyl | H | CF3 |
| 11-1037 | 2,2,2-trifluoroethyl | H | H | F | H | H | H | CF3 | H | nonafluoro-2-butyl | H | CF3 |
| 11-1038 | 2,2,2-trichloroethyl | H | H | F | H | H | H | CF3 | H | nonafluoro-2-butyl | H | CF3 |
| 11-1039 | 2,2,2-tribromoethyl | H | H | F | H | H | H | CF3 | H | nonafluoro-2-butyl | H | CF3 |
| 11-1041 | 3-fluoro-n-propyl | H | H | F | H | H | H | CF3 | H | nonafluoro-2-butyl | H | CF3 |
| 11-1042 | 3-chloro-n-propyl | H | H | F | H | H | H | CF3 | H | nonafluoro-2-butyl | H | CF3 |
| 11-1043 | 3-bromo-n-propyl | H | H | F | H | H | H | CF3 | H | nonafluoro-2-butyl | H | CF3 |
| 11-1045 | 1,2-difluoro-2-propyl | H | H | F | H | H | H | CF3 | H | nonafluoro-2-butyl | H | CF3 |
| 11-1046 | 1,3-difluoro-2-propyl | H | H | F | H | H | H | CF3 | H | nonafluoro-2-butyl | H | CF3 |
| 11-1047 | 1,3-dichloro-2-propyl | H | H | F | H | H | H | CF3 | H | nonafluoro-2-butyl | H | CF3 |
| 11-1048 | 1-chloro-3-fluoro-2-propyl | H | H | F | H | H | H | CF3 | H | nonafluoro-2-butyl | H | CF3 |

**Table 11 (7)**

| compound number | Q₂ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 11-1049 | 3,3,3-trifluoro-n-propyl | H | H | F | H | H | H | n-C3F7 | H | nonafluoro-2-butyl | H | i-C3F7 |
| 11-1051 | 4,4,4-trifluoro-n-butyl | H | H | F | H | H | H | CF3 | H | nonafluoro-2-butyl | H | CF3 |
| 11-1057 | 2,2,2-trichloroethyl | H | H | H | F | H | H | F | H | heptafluoroisopropyl | H | F |
| 11-1058 | 2,2,2-trichloroethyl | H | H | H | F | H | H | F | H | nonafluoro-2-butyl | H | F |
| 11-1069 | 2,2,2-trichloroethyl | H | H | H | F | H | H | Cl | H | heptafluoroisopropyl | H | Cl |
| 11-1098 | 2,2,2-trichloroethyl | H | H | H | F | H | H | Cl | H | nonafluoro-2-butyl | H | Cl |
| 11-1127 | 2,2,2-trichloroethyl | H | H | H | F | H | H | Br | H | heptafluoroisopropyl | H | Br |
| 11-1156 | 2,2,2-trichloroethyl | H | H | H | F | H | H | Br | H | nonafluoro-2-butyl | H | Br |
| 11-1185 | 2,2,2-trichloroethyl | H | H | H | F | H | H | I | H | heptafluoroisopropyl | H | I |
| 11-1214 | 2,2,2-trichloroethyl | H | H | H | F | H | H | I | H | nonafluoro-2-butyl | H | I |
| 11-1233 | 2,2,2-trichloroethyl | H | H | H | F | H | H | F | H | heptafluoroisopropyl | H | Cl |
| 11-1234 | 2,2,2-trichloroethyl | H | H | H | F | H | H | F | H | heptafluoroisopropyl | H | Br |
| 11-1235 | 2,2,2-trichloroethyl | H | H | H | F | H | H | F | H | heptafluoroisopropyl | H | I |
| 11-1236 | 2,2,2-trichloroethyl | H | H | H | F | H | H | Cl | H | heptafluoroisopropyl | H | Br |
| 11-1237 | 2,2,2-trichloroethyl | H | H | H | F | H | H | Cl | H | heptafluoroisopropyl | H | I |
| 11-1238 | 2,2,2-trichloroethyl | H | H | H | F | H | H | Br | H | heptafluoroisopropyl | H | I |
| 11-1239 | 2,2,2-trichloroethyl | H | H | H | F | H | H | F | H | nonafluoro-2-butyl | H | Cl |
| 11-1240 | 2,2,2-trichloroethyl | H | H | H | F | H | H | F | H | nonafluoro-2-butyl | H | Br |
| 11-1241 | 2,2,2-trichloroethyl | H | H | H | F | H | H | F | H | nonafluoro-2-butyl | H | I |
| 11-1242 | 2,2,2-trichloroethyl | H | H | H | F | H | H | Cl | H | nonafluoro-2-butyl | H | Br |
| 11-1243 | 2,2,2-trichloroethyl | H | H | H | F | H | H | Cl | H | nonafluoro-2-butyl | H | I |
| 11-1244 | 2,2,2-trichloroethyl | H | H | H | F | H | H | Br | H | nonafluoro-2-butyl | H | I |
| 11-1245 | 2,2,2-trichloroethyl | H | H | H | F | H | H | F | H | heptafluoroisopropyl | H | OCF3 |
| 11-1246 | 2,2,2-trichloroethyl | H | H | H | F | H | H | F | H | nonafluoro-2-butyl | H | OCF3 |
| 11-1257 | 2,2,2-trichloroethyl | H | H | H | F | H | H | Cl | H | heptafluoroisopropyl | H | OCF3 |
| 11-1286 | 2,2,2-trichloroethyl | H | H | H | F | H | H | Cl | H | nonafluoro-2-butyl | H | OCF3 |
| 11-1315 | 2,2,2-trichloroethyl | H | H | H | F | H | H | Br | H | heptafluoroisopropyl | H | OCF3 |
| 11-1344 | 2,2,2-trichloroethyl | H | H | H | F | H | H | Br | H | nonafluoro-2-butyl | H | OCF3 |
| 11-1373 | 2,2,2-trichloroethyl | H | H | H | F | H | H | I | H | heptafluoroisopropyl | H | OCF3 |
| 11-1402 | 2,2,2-trichloroethyl | H | H | H | F | H | H | I | H | nonafluoro-2-butyl | H | OCF3 |
| 11-1421 | 2,2,2-trichloroethyl | H | H | H | F | H | H | F | H | heptafluoroisopropyl | H | CF3 |
| 11-1422 | 2,2,2-trichloroethyl | H | H | H | F | H | H | F | H | nonafluoro-2-butyl | H | CF3 |
| 11-1433 | 2,2,2-trichloroethyl | H | H | H | F | H | H | Cl | H | heptafluoroisopropyl | H | CF3 |
| 11-1461 | 2,2,2-trifluoroethyl | H | H | H | F | H | H | Cl | H | nonafluoro-2-butyl | H | CF3 |
| 11-1491 | 2,2,2-trichloroethyl | H | H | H | F | H | H | Br | H | heptafluoroisopropyl | H | CF3 |
| 11-1504 | 4,4,4-trifluoro-n-butyl | H | H | H | F | H | H | Br | H | heptafluoroisopropyl | H | CF3 |
| 11-1520 | 2,2,2-trichloroethyl | H | H | H | F | H | H | Br | H | nonafluoro-2-butyl | H | CF3 |
| 11-1533 | 4,4,4-trifluoro-n-butyl | H | H | H | F | H | H | Br | H | nonafluoro-2-butyl | H | CF3 |
| 11-1548 | 2,2,2-trifluoroethyl | H | H | H | F | H | H | I | H | heptafluoroisopropyl | H | CF3 |
| 11-1562 | 4,4,4-trifluoro-n-butyl | H | H | H | F | H | H | I | H | heptafluoroisopropyl | H | CF3 |
| 11-1578 | 2,2,2-trichloroethyl | H | H | H | F | H | H | I | H | nonafluoro-2-butyl | H | CF3 |
| 11-1591 | 4,4,4-trifluoro-n-butyl | H | H | H | F | H | H | I | H | nonafluoro-2-butyl | H | CF3 |
| 11-1607 | 2,2,2-trichloroethyl | H | H | H | F | H | H | OCF3 | H | heptafluoroisopropyl | H | OCF3 |
| 11-1636 | 2,2,2-trichloroethyl | H | H | H | F | H | H | OCF3 | H | nonafluoro-2-butyl | H | OCF3 |
| 11-1665 | 2,2,2-trichloroethyl | H | H | H | F | H | H | CF3 | H | heptafluoroisopropyl | H | OCF3 |
| 11-1694 | 2,2,2-trichloroethyl | H | H | H | F | H | H | CF3 | H | nonafluoro-2-butyl | H | OCF3 |
| 11-1723 | 2,2,2-trichloroethyl | H | H | H | F | H | H | CF3 | H | heptafluoroisopropyl | H | CF3 |
| 11-1752 | 2,2,2-trichloroethyl | H | H | H | F | H | H | CF3 | H | nonafluoro-2-butyl | H | CF3 |
| 11-1771 | 2,2,2-trichloroethyl | H | H | F | F | H | H | F | H | heptafluoroisopropyl | H | F |
| 11-1772 | 2,2,2-trichloroethyl | H | H | F | F | H | H | F | H | nonafluoro-2-butyl | H | F |
| 11-1783 | 2,2,2-trichloroethyl | H | H | F | F | H | H | Cl | H | heptafluoroisopropyl | H | Cl |
| 11-1812 | 2,2,2-trichloroethyl | H | H | F | F | H | H | Cl | H | nonafluoro-2-butyl | H | Cl |
| 11-1841 | 2,2,2-trichloroethyl | H | H | F | F | H | H | Br | H | heptafluoroisopropyl | H | Br |
| 11-1870 | 2,2,2-trichloroethyl | H | H | F | F | H | H | Br | H | nonafluoro-2-butyl | H | Br |
| 11-1899 | 2,2,2-trichloroethyl | H | H | F | F | H | H | I | H | heptafluoroisopropyl | H | I |
| 11-1928 | 2,2,2-trichloroethyl | H | H | F | F | H | H | I | H | nonafluoro-2-butyl | H | I |
| 11-1947 | 2,2,2-trichloroethyl | H | H | F | F | H | H | F | H | heptafluoroisopropyl | H | Cl |
| 11-1948 | 2,2,2-trichloroethyl | H | H | F | F | H | H | F | H | heptafluoroisopropyl | H | Br |
| 11-1949 | 2,2,2-trichloroethyl | H | H | F | F | H | H | F | H | heptafluoroisopropyl | H | I |
| 11-1950 | 2,2,2-trichloroethyl | H | H | F | F | H | H | Cl | H | heptafluoroisopropyl | H | Br |
| 11-1951 | 2,2,2-trichloroethyl | H | H | F | F | H | H | Cl | H | heptafluoroisopropyl | H | I |
| 11-1952 | 2,2,2-trichloroethyl | H | H | F | F | H | H | Br | H | heptafluoroisopropyl | H | I |
| 11-1953 | 2,2,2-trichloroethyl | H | H | F | F | H | H | F | H | nonafluoro-2-butyl | H | Cl |
| 11-1954 | 2,2,2-trichloroethyl | H | H | F | F | H | H | F | H | nonafluoro-2-butyl | H | Br |

**Table 11 (8)**

| compound number | Q₂ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 11-1955 | 2,2,2-trichloroethyl | H | H | F | F | H | H | F | H | nonafluoro-2-butyl | H | I |
| 11-1956 | 2,2,2-trichloroethyl | H | H | F | F | H | H | Cl | H | nonafluoro-2-butyl | H | Br |
| 11-1957 | 2,2,2-trichloroethyl | H | H | F | F | H | H | Cl | H | nonafluoro-2-butyl | H | I |
| 11-1958 | 2,2,2-trichloroethyl | H | H | F | F | H | H | Br | H | nonafluoro-2-butyl | H | I |
| 11-1959 | 2,2,2-trichloroethyl | H | H | F | F | H | H | F | H | heptafluoroisopropyl | H | OCF3 |
| 11-1960 | 2,2,2-trichloroethyl | H | H | F | F | H | H | F | H | nonafluoro-2-butyl | H | OCF3 |
| 11-1971 | 2,2,2-trichloroethyl | H | H | F | F | H | H | Cl | H | heptafluoroisopropyl | H | OCF3 |
| 11-2000 | 2,2,2-trichloroethyl | H | H | F | F | H | H | Cl | H | nonafluoro-2-butyl | H | OCF3 |
| 11-2029 | 2,2,2-trichloroethyl | H | H | F | F | H | H | Br | H | heptafluoroisopropyl | H | OCF3 |
| 11-2058 | 2,2,2-trichloroethyl | H | H | F | F | H | H | Br | H | nonafluoro-2-butyl | H | OCF3 |
| 11-2087 | 2,2,2-trichloroethyl | H | H | F | F | H | H | I | H | heptafluoroisopropyl | H | OCF3 |
| 11-2116 | 2,2,2-trichloroethyl | H | H | F | F | H | H | I | H | nonafluoro-2-butyl | H | OCF3 |
| 11-2135 | 2,2,2-trichloroethyl | H | H | F | F | H | H | F | H | heptafluoroisopropyl | H | CF3 |
| 11-2136 | 2,2,2-trichloroethyl | H | H | F | F | H | H | F | H | nonafluoro-2-butyl | H | CF3 |
| 11-2147 | 2,2,2-trichloroethyl | H | H | F | F | H | H | Cl | H | heptafluoroisopropyl | H | CF3 |
| 11-2176 | 2,2,2-trichloroethyl | H | H | F | F | H | H | Cl | H | nonafluoro-2-butyl | H | CF3 |
| 11-2205 | 2,2,2-trichloroethyl | H | H | F | F | H | H | Br | H | heptafluoroisopropyl | H | CF3 |
| 11-2218 | 4,4,4-trifluoro-n-butyl | H | H | F | F | H | H | Br | H | heptafluoroisopropyl | H | CF3 |
| 11-2234 | 2,2,2-trichloroethyl | H | H | F | F | H | H | Br | H | nonafluoro-2-butyl | H | CF3 |
| 11-2247 | 4,4,4-trifluoro-n-butyl | H | H | F | F | H | H | Br | H | nonafluoro-2-butyl | H | CF3 |
| 11-2263 | 2,2,2-trichloroethyl | H | H | F | F | H | H | I | H | heptafluoroisopropyl | H | CF3 |
| 11-2276 | 4,4,4-trifluoro-n-butyl | H | H | F | F | H | H | I | H | heptafluoroisopropyl | H | CF3 |
| 11-2292 | 2,2,2-trichloroethyl | H | H | F | F | H | H | I | H | nonafluoro-2-butyl | H | CF3 |
| 11-2305 | 4,4,4-trifluoro-n-butyl | H | H | F | F | H | H | I | H | nonafluoro-2-butyl | H | CF3 |
| 11-2321 | 2,2,2-trichloroethyl | H | H | F | F | H | H | OCF3 | H | heptafluoroisopropyl | H | OCF3 |
| 11-2350 | 2,2,2-trichloroethyl | H | H | F | F | H | H | OCF3 | H | nonafluoro-2-butyl | H | OCF3 |
| 11-2379 | 2,2,2-trichloroethyl | H | H | F | F | H | H | CF3 | H | heptafluoroisopropyl | H | OCF3 |
| 11-2408 | 2,2,2-trichloroethyl | H | H | F | F | H | H | CF3 | H | nonafluoro-2-butyl | H | OCF3 |
| 11-2437 | 2,2,2-trichloroethyl | H | H | F | F | H | H | CF3 | H | heptafluoroisopropyl | H | CF3 |
| 11-2466 | 2,2,2-trichloroethyl | H | H | F | F | H | H | CF3 | H | nonafluoro-2-butyl | H | CF3 |
| 11-2485 | 2,2,2-trichloroethyl | H | H | F | F | F | H | Cl | H | pentafluoroethyl | H | Cl |
| 11-2486 | 2,2,2-trichloroethyl | H | H | F | F | F | H | Br | H | heptafluoroisopropyl | H | Br |
| 11-2487 | 2,2,2-trichloroethyl | H | H | F | F | F | H | Br | H | heptafluoroisopropyl | H | OCF3 |
| 11-2488 | 2,2,2-trichloroethyl | H | H | F | F | F | H | I | H | heptafluoroisopropyl | H | CF3 |
| 11-2489 | 2,2,2-trichloroethyl | H | H | F | F | F | H | CF3 | H | heptafluoroisopropyl | H | CF3 |
| 11-2490 | 2,2,2-trichloroethyl | H | H | F | F | F | H | I | H | nonafluoro-2-butyl | H | I |
| 11-2491 | 2,2,2-trichloroethyl | H | H | F | F | F | H | Cl | H | nonafluoro-2-butyl | H | OCF3 |
| 11-2492 | 2,2,2-trichloroethyl | H | H | F | F | F | H | Br | H | nonafluoro-2-butyl | H | CF3 |
| 11-2493 | 2,2,2-trichloroethyl | H | H | F | F | F | H | CF3 | H | nonafluoro-2-butyl | H | CF3 |
| 11-2494 | 2,2,2-trichloroethyl | H | H | H | H | F | H | Cl | H | pentafluoroethyl | H | Cl |
| 11-2495 | 2,2,2-trichloroethyl | H | H | H | H | F | H | Br | H | heptafluoroisopropyl | H | Br |
| 11-2496 | 2,2,2-trichloroethyl | H | H | H | H | F | H | Br | H | heptafluoroisopropyl | H | OCF3 |
| 11-2497 | 2,2,2-trichloroethyl | H | H | H | H | F | H | I | H | heptafluoroisopropyl | H | CF3 |
| 11-2498 | 2,2,2-trichloroethyl | H | H | H | H | F | H | CF3 | H | heptafluoroisopropyl | H | CF3 |
| 11-2499 | 2,2,2-trichloroethyl | H | H | H | H | F | H | I | H | nonafluoro-2-butyl | H | I |
| 11-2500 | 2,2,2-trichloroethyl | H | H | H | H | F | H | Cl | H | nonafluoro-2-butyl | H | OCF3 |
| 11-2501 | 2,2,2-trichloroethyl | H | H | H | H | F | H | Br | H | nonafluoro-2-butyl | H | CF3 |
| 11-2502 | 2,2,2-trichloroethyl | H | H | H | H | F | H | CF3 | H | nonafluoro-2-butyl | H | CF3 |
| 11-2503 | 2,2,2-trichloroethyl | H | H | H | H | H | F | Cl | H | pentafluoroethyl | H | Cl |
| 11-2504 | 2,2,2-trichloroethyl | H | H | H | H | H | F | Br | H | heptafluoroisopropyl | H | Br |
| 11-2505 | 2,2,2-trichloroethyl | H | H | H | H | H | F | Br | H | heptafluoroisopropyl | H | OCF3 |
| 11-2506 | 2,2,2-trichloroethyl | H | H | H | H | H | F | I | H | heptafluoroisopropyl | H | CF3 |
| 11-2507 | 2,2,2-trichloroethyl | H | H | H | H | H | F | CF3 | H | heptafluoroisopropyl | H | CF3 |
| 11-2508 | 2,2,2-trichloroethyl | H | H | H | H | H | F | I | H | nonafluoro-2-butyl | H | I |
| 11-2509 | 2,2,2-trichloroethyl | H | H | H | H | H | F | Cl | H | nonafluoro-2-butyl | H | OCF3 |
| 11-2510 | 2,2,2-trichloroethyl | H | H | H | H | H | F | Br | H | nonafluoro-2-butyl | H | CF3 |
| 11-2511 | 2,2,2-trichloroethyl | H | H | H | H | H | F | CF3 | H | nonafluoro-2-butyl | H | CF3 |
| 11-2512 | 2,2,2-trichloroethyl | H | H | F | H | F | H | Cl | H | pentafluoroethyl | H | Cl |
| 11-2513 | 2,2,2-trichloroethyl | H | H | F | H | F | H | Br | H | heptafluoroisopropyl | H | Br |
| 11-2514 | 2,2,2-trichloroethyl | H | H | F | H | F | H | Br | H | heptafluoroisopropyl | H | OCF3 |
| 11-2515 | 2,2,2-trichloroethyl | H | H | F | H | F | H | I | H | heptafluoroisopropyl | H | CF3 |
| 11-2516 | 2,2,2-trichloroethyl | H | H | F | H | F | H | CF3 | H | heptafluoroisopropyl | H | CF3 |
| 11-2517 | 2,2,2-trichloroethyl | H | H | F | H | F | H | I | H | nonafluoro-2-butyl | H | I |
| 11-2518 | 2,2,2-trichloroethyl | H | H | F | H | F | H | Cl | H | nonafluoro-2-butyl | H | OCF3 |

**Table 11 (9)**

| compound number | Q₂ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 11-2519 | 2,2,2-trichloroethyl | H | H | F | H | F | H | Br | H | nonafluoro-2-butyl | H | CF3 |
| 11-2520 | 2,2,2-trichloroethyl | H | H | F | H | F | H | CF3 | H | nonafluoro-2-butyl | H | CF3 |
| 11-2521 | 2,2,2-trichloroethyl | H | H | F | H | H | F | Cl | H | pentafluoroethyl | H | Cl |
| 11-2522 | 2,2,2-trichloroethyl | H | H | F | H | H | F | Br | H | heptafluoroisopropyl | H | Br |
| 11-2523 | 2,2,2-trichloroethyl | H | H | F | H | H | F | Br | H | heptafluoroisopropyl | H | OCF3 |
| 11-2524 | 2,2,2-trichloroethyl | H | H | F | H | H | F | I | H | heptafluoroisopropyl | H | CF3 |
| 11-2525 | 2,2,2-trichloroethyl | H | H | F | H | H | F | CF3 | H | heptafluoroisopropyl | H | CF3 |
| 11-2526 | 2,2,2-trichloroethyl | H | H | F | H | H | F | I | H | nonafluoro-2-butyl | H | I |
| 11-2527 | 2,2,2-trichloroethyl | H | H | F | H | H | F | Cl | H | nonafluoro-2-butyl | H | OCF3 |
| 11-2528 | 2,2,2-trichloroethyl | H | H | F | H | H | F | Br | H | nonafluoro-2-butyl | H | CF3 |
| 11-2529 | 2,2,2-trichloroethyl | H | H | F | H | H | F | CF3 | H | nonafluoro-2-butyl | H | CF3 |
| 11-2530 | 2,2,2-trichloroethyl | H | H | H | F | F | H | Cl | H | pentafluoroethyl | H | Cl |
| 11-2531 | 2,2,2-trichloroethyl | H | H | H | F | F | H | Br | H | heptafluoroisopropyl | H | Br |
| 11-2532 | 2,2,2-trichloroethyl | H | H | H | F | F | H | Br | H | heptafluoroisopropyl | H | OCF3 |
| 11-2533 | 2,2,2-trichloroethyl | H | H | H | F | F | H | I | H | heptafluoroisopropyl | H | CF3 |
| 11-2534 | 2,2,2-trichloroethyl | H | H | H | F | F | H | CF3 | H | heptafluoroisopropyl | H | CF3 |
| 11-2535 | 2,2,2-trichloroethyl | H | H | H | F | F | H | I | H | nonafluoro-2-butyl | H | I |
| 11-2536 | 2,2,2-trichloroethyl | H | H | H | F | F | H | Cl | H | nonafluoro-2-butyl | H | OCF3 |
| 11-2537 | 2,2,2-trichloroethyl | H | H | H | F | F | H | Br | H | nonafluoro-2-butyl | H | CF3 |
| 11-2538 | 2,2,2-trichloroethyl | H | H | H | F | F | H | CF3 | H | nonafluoro-2-butyl | H | CF3 |
| 11-2539 | 2,2,2-trichloroethyl | H | H | H | F | H | F | Cl | H | pentafluoroethyl | H | Cl |
| 11-2540 | 2,2,2-trichloroethyl | H | H | H | F | H | F | Br | H | heptafluoroisopropyl | H | Br |
| 11-2541 | 2,2,2-trichloroethyl | H | H | H | F | H | F | Br | H | heptafluoroisopropyl | H | OCF3 |
| 11-2542 | 2,2,2-trichloroethyl | H | H | H | F | H | F | I | H | heptafluoroisopropyl | H | CF3 |
| 11-2543 | 2,2,2-trichloroethyl | H | H | H | F | H | F | CF3 | H | heptafluoroisopropyl | H | CF3 |
| 11-2544 | 2,2,2-trichloroethyl | H | H | H | F | H | F | I | H | nonafluoro-2-butyl | H | I |
| 11-2545 | 2,2,2-trichloroethyl | H | H | H | F | H | F | Cl | H | nonafluoro-2-butyl | H | OCF3 |
| 11-2546 | 2,2,2-trichloroethyl | H | H | H | F | H | F | Br | H | nonafluoro-2-butyl | H | CF3 |
| 11-2547 | 2,2,2-trichloroethyl | H | H | H | F | H | F | CF3 | H | nonafluoro-2-butyl | H | CF3 |
| 11-2548 | 2,2,2-trichloroethyl | H | H | H | H | F | F | Cl | H | pentafluoroethyl | H | Cl |
| 11-2549 | 2,2,2-trichloroethyl | H | H | H | H | F | F | Br | H | heptafluoroisopropyl | H | Br |
| 11-2550 | 2,2,2-trichloroethyl | H | H | H | H | F | F | Br | H | heptafluoroisopropyl | H | OCF3 |
| 11-2551 | 2,2,2-trichloroethyl | H | H | H | H | F | F | I | H | heptafluoroisopropyl | H | CF3 |
| 11-2552 | 2,2,2-trichloroethyl | H | H | H | H | F | F | CF3 | H | heptafluoroisopropyl | H | CF3 |
| 11-2553 | 2,2,2-trichloroethyl | H | H | H | H | F | F | I | H | nonafluoro-2-butyl | H | I |
| 11-2554 | 2,2,2-trichloroethyl | H | H | H | H | F | F | Cl | H | nonafluoro-2-butyl | H | OCF3 |
| 11-2555 | 2,2,2-trichloroethyl | H | H | H | H | F | F | Br | H | nonafluoro-2-butyl | H | CF3 |
| 11-2556 | 2,2,2-trichloroethyl | H | H | H | H | F | F | CF3 | H | nonafluoro-2-butyl | H | CF3 |
| 11-2557 | 2,2,2-trichloroethyl | H | H | F | F | H | F | Cl | H | pentafluoroethyl | H | Cl |
| 11-2558 | 2,2,2-trichloroethyl | H | H | F | F | H | F | Br | H | heptafluoroisopropyl | H | Br |
| 11-2559 | 2,2,2-trichloroethyl | H | H | F | F | H | F | Br | H | heptafluoroisopropyl | H | OCF3 |
| 11-2560 | 2,2,2-trichloroethyl | H | H | F | F | H | F | I | H | heptafluoroisopropyl | H | CF3 |
| 11-2561 | 2,2,2-trichloroethyl | H | H | F | F | H | F | CF3 | H | heptafluoroisopropyl | H | CF3 |
| 11-2562 | 2,2,2-trichloroethyl | H | H | F | F | H | F | I | H | nonafluoro-2-butyl | H | I |
| 11-2563 | 2,2,2-trichloroethyl | H | H | F | F | H | F | Cl | H | nonafluoro-2-butyl | H | OCF3 |
| 11-2564 | 2,2,2-trichloroethyl | H | H | F | F | H | F | Br | H | nonafluoro-2-butyl | H | CF3 |
| 11-2565 | 2,2,2-trichloroethyl | H | H | F | F | H | F | CF3 | H | nonafluoro-2-butyl | H | CF3 |
| 11-2566 | 2,2,2-trichloroethyl | H | H | F | H | F | F | Cl | H | pentafluoroethyl | H | Cl |
| 11-2567 | 2,2,2-trichloroethyl | H | H | F | H | F | F | Br | H | heptafluoroisopropyl | H | Br |
| 11-2568 | 2,2,2-trichloroethyl | H | H | F | H | F | F | Br | H | heptafluoroisopropyl | H | OCF3 |
| 11-2569 | 2,2,2-trichloroethyl | H | H | F | H | F | F | I | H | heptafluoroisopropyl | H | CF3 |
| 11-2570 | 2,2,2-trichloroethyl | H | H | F | H | F | F | CF3 | H | heptafluoroisopropyl | H | CF3 |
| 11-2571 | 2,2,2-trichloroethyl | H | H | F | H | F | F | I | H | nonafluoro-2-butyl | H | I |
| 11-2572 | 2,2,2-trichloroethyl | H | H | F | H | F | F | Cl | H | nonafluoro-2-butyl | H | OCF3 |
| 11-2573 | 2,2,2-trichloroethyl | H | H | F | H | F | F | Br | H | nonafluoro-2-butyl | H | CF3 |
| 11-2574 | 2,2,2-trichloroethyl | H | H | F | H | F | F | CF3 | H | nonafluoro-2-butyl | H | CF3 |
| 11-2575 | 2,2,2-trichloroethyl | H | H | H | F | F | F | Cl | H | pentafluoroethyl | H | Cl |
| 11-2576 | 2,2,2-trichloroethyl | H | H | H | F | F | F | Br | H | heptafluoroisopropyl | H | Br |
| 11-2577 | 2,2,2-trichloroethyl | H | H | H | F | F | F | Br | H | heptafluoroisopropyl | H | OCF3 |
| 11-2578 | 2,2,2-trichloroethyl | H | H | H | F | F | F | I | H | heptafluoroisopropyl | H | CF3 |
| 11-2579 | 2,2,2-trichloroethyl | H | H | H | F | F | F | CF3 | H | heptafluoroisopropyl | H | CF3 |
| 11-2580 | 2,2,2-trichloroethyl | H | H | H | F | F | F | I | H | nonafluoro-2-butyl | H | I |
| 11-2581 | 2,2,2-trichloroethyl | H | H | H | F | F | F | Cl | H | nonafluoro-2-butyl | H | OCF3 |
| 11-2582 | 2,2,2-trichloroethyl | H | H | H | F | F | F | Br | H | nonafluoro-2-butyl | H | CF3 |

**Table 11 (10)**

| compound number | Q₂ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 11-2583 | 2,2,2-trichloroethyl | H | H | H | F | F | F | CF3 | H | nonafluoro-2-butyl | H | CF3 |
| 11-2584 | 2,2,2-trichloroethyl | H | H | F | F | F | F | Cl | H | pentafluoroethyl | H | Cl |
| 11-2585 | 2,2,2-trichloroethyl | H | H | F | F | F | F | Br | H | heptafluoroisopropyl | H | Br |
| 11-2586 | 2,2,2-trichloroethyl | H | H | F | F | F | F | Br | H | heptafluoroisopropyl | H | OCF3 |
| 11-2587 | 2,2,2-trichloroethyl | H | H | F | F | F | F | I | H | heptafluoroisopropyl | H | CF3 |
| 11-2588 | 2,2,2-trichloroethyl | H | H | F | F | F | F | CF3 | H | heptafluoroisopropyl | H | CF3 |
| 11-2589 | 2,2,2-trichloroethyl | H | H | F | F | F | F | I | H | nonafluoro-2-butyl | H | I |
| 11-2590 | 2,2,2-trichloroethyl | H | H | F | F | F | F | Cl | H | nonafluoro-2-butyl | H | OCF3 |
| 11-2591 | 2,2,2-trichloroethyl | H | H | F | F | F | F | Br | H | nonafluoro-2-butyl | H | CF3 |
| 11-2592 | 2,2,2-trichloroethyl | H | H | F | F | F | F | CF3 | H | nonafluoro-2-butyl | H | CF3 |
| 11-2593 | 2,2,2-trichloroethyl | H | H | H | CN | H | H | F | H | heptafluoroisopropyl | H | OCF3 |
| 11-2594 | 2,2,2-trichloroethyl | H | H | H | CN | H | H | F | H | nonafluoro-2-butyl | H | OCF3 |
| 11-2605 | 2,2,2-trichloroethyl | H | H | H | CN | H | H | Cl | H | heptafluoroisopropyl | H | OCF3 |
| 11-2634 | 2,2,2-trichloroethyl | H | H | H | CN | H | H | Cl | H | nonafluoro-2-butyl | H | OCF3 |
| 11-2663 | 2,2,2-trichloroethyl | H | H | H | CN | H | H | Br | H | heptafluoroisopropyl | H | OCF3 |
| 11-2692 | 2,2,2-trichloroethyl | H | H | H | CN | H | H | Br | H | nonafluoro-2-butyl | H | OCF3 |
| 11-2721 | 2,2,2-trichloroethyl | H | H | H | CN | H | H | I | H | heptafluoroisopropyl | H | OCF3 |
| 11-2750 | 2,2,2-trichloroethyl | H | H | H | CN | H | H | I | H | nonafluoro-2-butyl | H | OCF3 |
| 11-2769 | 2,2,2-trichloroethyl | H | H | H | CN | H | H | F | H | heptafluoroisopropyl | H | CF3 |
| 11-2770 | 2,2,2-trichloroethyl | H | H | H | CN | H | H | F | H | nonafluoro-2-butyl | H | CF3 |
| 11-2781 | 2,2,2-trichloroethyl | H | H | H | CN | H | H | Cl | H | heptafluoroisopropyl | H | CF3 |
| 11-2810 | 2,2,2-trichloroethyl | H | H | H | CN | H | H | Cl | H | nonafluoro-2-butyl | H | CF3 |
| 11-2839 | 2,2,2-trichloroethyl | H | H | H | CN | H | H | Br | H | heptafluoroisopropyl | H | CF3 |
| 11-2852 | 4,4,4-trifluoro-n-butyl | H | H | H | CN | H | H | Br | H | heptafluoroisopropyl | H | CF3 |
| 11-2868 | 2,2,2-trichloroethyl | H | H | H | CN | H | H | Br | H | nonafluoro-2-butyl | H | CF3 |
| 11-2881 | 4,4,4-trifluoro-n-butyl | H | H | H | CN | H | H | Br | H | nonafluoro-2-butyl | H | CF3 |
| 11-2897 | 2,2,2-trichloroethyl | H | H | H | CN | H | H | I | H | heptafluoroisopropyl | H | CF3 |
| 11-2910 | 4,4,4-trifluoro-n-butyl | H | H | H | CN | H | H | I | H | heptafluoroisopropyl | H | CF3 |
| 11-2926 | 2,2,2-trichloroethyl | H | H | H | CN | H | H | I | H | nonafluoro-2-butyl | H | CF3 |
| 11-2939 | 4,4,4-trifluoro-n-butyl | H | H | H | CN | H | H | I | H | nonafluoro-2-butyl | H | CF3 |
| 11-2955 | 2,2,2-trichloroethyl | H | H | H | CN | H | H | OCF3 | H | heptafluoroisopropyl | H | OCF3 |
| 11-2984 | 2,2,2-trichloroethyl | H | H | H | CN | H | H | OCF3 | H | nonafluoro-2-butyl | H | OCF3 |
| 11-3013 | 2,2,2-trichloroethyl | H | H | H | CN | H | H | CF3 | H | heptafluoroisopropyl | H | OCF3 |
| 11-3042 | 2,2,2-trichloroethyl | H | H | H | CN | H | H | CF3 | H | nonafluoro-2-butyl | H | OCF3 |
| 11-3071 | 2,2,2-trichloroethyl | H | H | H | CN | H | H | CF3 | H | heptafluoroisopropyl | H | CF3 |
| 11-3100 | 2,2,2-trichloroethyl | H | H | H | CN | H | H | CF3 | H | nonafluoro-2-butyl | H | CF3 |
| 11-3119 | 2,2,2-trichloroethyl | H | H | F | CN | H | H | F | H | heptafluoroisopropyl | H | F |
| 11-3120 | 2,2,2-trichloroethyl | H | H | F | CN | H | H | F | H | nonafluoro-2-butyl | H | F |
| 11-3131 | 2,2,2-trichloroethyl | H | H | F | CN | H | H | Cl | H | heptafluoroisopropyl | H | Cl |
| 11-3160 | 2,2,2-trichloroethyl | H | H | F | CN | H | H | Cl | H | nonafluoro-2-butyl | H | Cl |
| 11-3189 | 2,2,2-trichloroethyl | H | H | F | CN | H | H | Br | H | heptafluoroisopropyl | H | Br |
| 11-3218 | 2,2,2-trichloroethyl | H | H | F | CN | H | H | Br | H | nonafluoro-2-butyl | H | Br |
| 11-3247 | 2,2,2-trichloroethyl | H | H | F | CN | H | H | I | H | heptafluoroisopropyl | H | I |
| 11-3276 | 2,2,2-trichloroethyl | H | H | F | CN | H | H | I | H | nonafluoro-2-butyl | H | I |
| 11-3295 | 2,2,2-trichloroethyl | H | H | F | CN | H | H | F | H | heptafluoroisopropyl | H | Cl |
| 11-3296 | 2,2,2-trichloroethyl | H | H | F | CN | H | H | F | H | heptafluoroisopropyl | H | Br |
| 11-3297 | 2,2,2-trichloroethyl | H | H | F | CN | H | H | F | H | heptafluoroisopropyl | H | I |
| 11-3298 | 2,2,2-trichloroethyl | H | H | F | CN | H | H | Cl | H | heptafluoroisopropyl | H | Br |
| 11-3299 | 2,2,2-trichloroethyl | H | H | F | CN | H | H | Cl | H | heptafluoroisopropyl | H | I |
| 11-3300 | 2,2,2-trichloroethyl | H | H | F | CN | H | H | Br | H | heptafluoroisopropyl | H | I |
| 11-3301 | 2,2,2-trichloroethyl | H | H | F | CN | H | H | F | H | nonafluoro-2-butyl | H | Cl |
| 11-3302 | 2,2,2-trichloroethyl | H | H | F | CN | H | H | F | H | nonafluoro-2-butyl | H | Br |
| 11-3303 | 2,2,2-trichloroethyl | H | H | F | CN | H | H | F | H | nonafluoro-2-butyl | H | I |
| 11-3304 | 2,2,2-trichloroethyl | H | H | F | CN | H | H | Cl | H | nonafluoro-2-butyl | H | Br |
| 11-3305 | 2,2,2-trichloroethyl | H | H | F | CN | H | H | Cl | H | nonafluoro-2-butyl | H | I |
| 11-3306 | 2,2,2-trichloroethyl | H | H | F | CN | H | H | Br | H | nonafluoro-2-butyl | H | I |
| 11-3307 | 2,2,2-trichloroethyl | H | H | F | CN | H | H | F | H | heptafluoroisopropyl | H | OCF3 |
| 11-3308 | 2,2,2-trichloroethyl | H | H | F | CN | H | H | F | H | nonafluoro-2-butyl | H | OCF3 |
| 11-3319 | 2,2,2-trichloroethyl | H | H | F | CN | H | H | Cl | H | heptafluoroisopropyl | H | OCF3 |
| 11-3348 | 2,2,2-trichloroethyl | H | H | F | CN | H | H | Cl | H | nonafluoro-2-butyl | H | OCF3 |
| 11-3377 | 2,2,2-trichloroethyl | H | H | F | CN | H | H | Br | H | heptafluoroisopropyl | H | OCF3 |
| 11-3406 | 2,2,2-trichloroethyl | H | H | F | CN | H | H | Br | H | nonafluoro-2-butyl | H | OCF3 |
| 11-3435 | 2,2,2-trichloroethyl | H | H | F | CN | H | H | I | H | heptafluoroisopropyl | H | OCF3 |
| 11-3464 | 2,2,2-trichloroethyl | H | H | F | CN | H | H | I | H | nonafluoro-2-butyl | H | OCF3 |

**Table 11 (11)**

| compound number | Q₂ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 11-3483 | 2,2,2-trichloroethyl | H | H | F | CN | H | H | F | H | heptafluoroisopropyl | H | CF3 |
| 11-3484 | 2,2,2-trichloroethyl | H | H | F | CN | H | H | F | H | nonafluoro-2-butyl | H | CF3 |
| 11-3495 | 2,2,2-trichloroethyl | H | H | F | CN | H | H | Cl | H | heptafluoroisopropyl | H | CF3 |
| 11-3524 | 2,2,2-trichloroethyl | H | H | F | CN | H | H | Cl | H | nonafluoro-2-butyl | H | CF3 |
| 11-3553 | 2,2,2-trichloroethyl | H | H | F | CN | H | H | Br | H | heptafluoroisopropyl | H | CF3 |
| 11-3566 | 4,4,4-trifluoro-n-butyl | H | H | F | CN | H | H | Br | H | heptafluoroisopropyl | H | CF3 |
| 11-3582 | 2,2,2-trichloroethyl | H | H | F | CN | H | H | Br | H | nonafluoro-2-butyl | H | CF3 |
| 11-3595 | 4,4,4-trifluoro-n-butyl | H | H | F | CN | H | H | Br | H | nonafluoro-2-butyl | H | CF3 |
| 11-3611 | 2,2,2-trichloroethyl | H | H | F | CN | H | H | I | H | heptafluoroisopropyl | H | CF3 |
| 11-3624 | 4,4,4-trifluoro-n-butyl | H | H | F | CN | H | H | I | H | heptafluoroisopropyl | H | CF3 |
| 11-3640 | 2,2,2-trichloroethyl | H | H | F | CN | H | H | I | H | nonafluoro-2-butyl | H | CF3 |
| 11-3653 | 4,4,4-trifluoro-n-butyl | H | H | F | CN | H | H | I | H | nonafluoro-2-butyl | H | CF3 |
| 11-3669 | 2,2,2-trichloroethyl | H | H | F | CN | H | H | OCF3 | H | heptafluoroisopropyl | H | OCF3 |
| 11-3698 | 2,2,2-trichloroethyl | H | H | F | CN | H | H | OCF3 | H | nonafluoro-2-butyl | H | OCF3 |
| 11-3727 | 2,2,2-trichloroethyl | H | H | F | CN | H | H | CF3 | H | heptafluoroisopropyl | H | OCF3 |
| 11-3756 | 2,2,2-trichloroethyl | H | H | F | CN | H | H | CF3 | H | nonafluoro-2-butyl | H | OCF3 |
| 11-3785 | 2,2,2-trichloroethyl | H | H | F | CN | H | H | CF3 | H | heptafluoroisopropyl | H | CF3 |
| 11-3814 | 2,2,2-trichloroethyl | H | H | F | CN | H | H | CF3 | H | nonafluoro-2-butyl | H | CF3 |
| 11-3833 | 2,2,2-trichloroethyl | H | H | F | CN | F | H | F | H | heptafluoroisopropyl | H | F |
| 11-3834 | 2,2,2-trichloroethyl | H | H | F | CN | F | H | F | H | nonafluoro-2-butyl | H | F |
| 11-3845 | 2,2,2-trichloroethyl | H | H | F | CN | F | H | Cl | H | heptafluoroisopropyl | H | Cl |
| 11-3874 | 2,2,2-trichloroethyl | H | H | F | CN | F | H | Cl | H | nonafluoro-2-butyl | H | Cl |
| 11-3903 | 2,2,2-trichloroethyl | H | H | F | CN | F | H | Br | H | heptafluoroisopropyl | H | Br |
| 11-3932 | 2,2,2-trichloroethyl | H | H | F | CN | F | H | Br | H | nonafluoro-2-butyl | H | Br |
| 11-3961 | 2,2,2-trichloroethyl | H | H | F | CN | F | H | I | H | heptafluoroisopropyl | H | I |
| 11-3990 | 2,2,2-trichloroethyl | H | H | F | CN | F | H | I | H | nonafluoro-2-butyl | H | I |
| 11-4009 | 2,2,2-trichloroethyl | H | H | F | CN | F | H | F | H | heptafluoroisopropyl | H | Cl |
| 11-4010 | 2,2,2-trichloroethyl | H | H | F | CN | F | H | F | H | heptafluoroisopropyl | H | Br |
| 11-4011 | 2,2,2-trichloroethyl | H | H | F | CN | F | H | F | H | heptafluoroisopropyl | H | I |
| 11-4012 | 2,2,2-trichloroethyl | H | H | F | CN | F | H | Cl | H | heptafluoroisopropyl | H | Br |
| 11-4013 | 2,2,2-trichloroethyl | H | H | F | CN | F | H | Cl | H | heptafluoroisopropyl | H | I |
| 11-4014 | 2,2,2-trichloroethyl | H | H | F | CN | F | H | Br | H | heptafluoroisopropyl | H | I |
| 11-4015 | 2,2,2-trichloroethyl | H | H | F | CN | F | H | F | H | nonafluoro-2-butyl | H | Cl |
| 11-4016 | 2,2,2-trichloroethyl | H | H | F | CN | F | H | F | H | nonafluoro-2-butyl | H | Br |
| 11-4017 | 2,2,2-trichloroethyl | H | H | F | CN | F | H | F | H | nonafluoro-2-butyl | H | I |
| 11-4018 | 2,2,2-trichloroethyl | H | H | F | CN | F | H | Cl | H | nonafluoro-2-butyl | H | Br |
| 11-4019 | 2,2,2-trichloroethyl | H | H | F | CN | F | H | Cl | H | nonafluoro-2-butyl | H | I |
| 11-4020 | 2,2,2-trichloroethyl | H | H | F | CN | F | H | Br | H | nonafluoro-2-butyl | H | I |
| 11-4021 | 2,2,2-trichloroethyl | H | H | F | CN | F | H | F | H | heptafluoroisopropyl | H | OCF3 |
| 11-4022 | 2,2,2-trichloroethyl | H | H | F | CN | F | H | F | H | nonafluoro-2-butyl | H | OCF3 |
| 11-4033 | 2,2,2-trichloroethyl | H | H | F | CN | F | H | Cl | H | heptafluoroisopropyl | H | OCF3 |
| 11-4062 | 2,2,2-trichloroethyl | H | H | F | CN | F | H | Cl | H | nonafluoro-2-butyl | H | OCF3 |
| 11-4091 | 2,2,2-trichloroethyl | H | H | F | CN | F | H | Br | H | heptafluoroisopropyl | H | OCF3 |
| 11-4120 | 2,2,2-trichloroethyl | H | H | F | CN | F | H | Br | H | nonafluoro-2-butyl | H | OCF3 |
| 11-4149 | 2,2,2-trichloroethyl | H | H | F | CN | F | H | I | H | heptafluoroisopropyl | H | OCF3 |
| 11-4178 | 2,2,2-trichloroethyl | H | H | F | CN | F | H | I | H | nonafluoro-2-butyl | H | OCF3 |
| 11-4209 | 2,2,2-trichloroethyl | H | H | F | CN | F | H | Cl | H | heptafluoroisopropyl | H | CF3 |
| 11-4238 | 2,2,2-trichloroethyl | H | H | F | CN | F | H | Cl | H | nonafluoro-2-butyl | H | CF3 |
| 11-4267 | 2,2,2-trichloroethyl | H | H | F | CN | F | H | Br | H | heptafluoroisopropyl | H | CF3 |
| 11-4280 | 4,4,4-trifluoro-n-butyl | H | H | F | CN | F | H | Br | H | heptafluoroisopropyl | H | CF3 |
| 11-4296 | 2,2,2-trichloroethyl | H | H | F | CN | F | H | Br | H | nonafluoro-2-butyl | H | CF3 |
| 11-4309 | 4,4,4-trifluoro-n-butyl | H | H | F | CN | F | H | Br | H | nonafluoro-2-butyl | H | CF3 |
| 11-4325 | 2,2,2-trichloroethyl | H | H | F | CN | F | H | I | H | heptafluoroisopropyl | H | CF3 |
| 11-4338 | 4,4,4-trifluoro-n-butyl | H | H | F | CN | F | H | I | H | heptafluoroisopropyl | H | CF3 |
| 11-4354 | 2,2,2-trichloroethyl | H | H | F | CN | F | H | I | H | nonafluoro-2-butyl | H | CF3 |
| 11-4367 | 4,4,4-trifluoro-n-butyl | H | H | F | CN | F | H | I | H | nonafluoro-2-butyl | H | CF3 |
| 11-4383 | 2,2,2-trichloroethyl | H | H | F | CN | F | H | OCF3 | H | heptafluoroisopropyl | H | OCF3 |
| 11-4412 | 2,2,2-trichloroethyl | H | H | F | CN | F | H | OCF3 | H | nonafluoro-2-butyl | H | OCF3 |
| 11-4441 | 2,2,2-trichloroethyl | H | H | F | CN | F | H | CF3 | H | heptafluoroisopropyl | H | OCF3 |
| 11-4470 | 2,2,2-trichloroethyl | H | H | F | CN | F | H | CF3 | H | nonafluoro-2-butyl | H | OCF3 |
| 11-4499 | 2,2,2-trichloroethyl | H | H | F | CN | F | H | CF3 | H | heptafluoroisopropyl | H | CF3 |
| 11-4528 | 2,2,2-trichloroethyl | H | H | F | CN | F | H | CF3 | H | nonafluoro-2-butyl | H | CF3 |

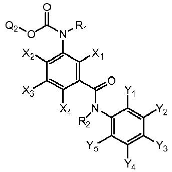

**Table 12(1)**

| compound number | Q₂ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 12-1 | 2,2,2-trichloroethyl | Me | H | H | H | H | H | F | H | heptafluoroisopropyl | H | OCF3 |
| 12-2 | 2,2,2-trichloroethyl | Me | H | H | H | H | H | F | H | nonafluoro-2-butyl | H | OCF3 |
| 12-13 | 2,2,2-trichloroethyl | Me | H | H | H | H | H | Cl | H | heptafluoroisopropyl | H | OCF3 |
| 12-42 | 2,2,2-trichloroethyl | Me | H | H | H | H | H | Cl | H | nonafluoro-2-butyl | H | OCF3 |
| 12-71 | 2,2,2-trichloroethyl | Me | H | H | H | H | H | Br | H | heptafluoroisopropyl | H | OCF3 |
| 12-100 | 2,2,2-trichloroethyl | Me | H | H | H | H | H | Br | H | nonafluoro-2-butyl | H | OCF3 |
| 12-129 | 2,2,2-trichloroethyl | Me | H | H | H | H | H | I | H | heptafluoroisopropyl | H | OCF3 |
| 12-158 | 2,2,2-trichloroethyl | Me | H | H | H | H | H | I | H | nonafluoro-2-butyl | H | OCF3 |
| 12-177 | 2,2,2-trichloroethyl | Me | H | H | H | H | H | F | H | heptafluoroisopropyl | H | CF3 |
| 12-178 | 2,2,2-trichloroethyl | Me | H | H | H | H | H | F | H | nonafluoro-2-butyl | H | CF3 |
| 12-189 | 2,2,2-trichloroethyl | Me | H | H | H | H | H | Cl | H | heptafluoroisopropyl | H | CF3 |
| 12-218 | 2,2,2-trichloroethyl | Me | H | H | H | H | H | Cl | H | nonafluoro-2-butyl | H | CF3 |
| 12-247 | 2,2,2-trichloroethyl | Me | H | H | H | H | H | Br | H | heptafluoroisopropyl | H | CF3 |
| 12-276 | 2,2,2-trichloroethyl | Me | H | H | H | H | H | Br | H | nonafluoro-2-butyl | H | CF3 |
| 12-305 | 2,2,2-trichloroethyl | Me | H | H | H | H | H | I | H | heptafluoroisopropyl | H | CF3 |
| 12-334 | 2,2,2-trichloroethyl | Me | H | H | H | H | H | I | H | nonafluoro-2-butyl | H | CF3 |
| 12-363 | 2,2,2-trichloroethyl | Me | H | H | H | H | H | OCF3 | H | heptafluoroisopropyl | H | OCF3 |
| 12-392 | 2,2,2-trichloroethyl | Me | H | H | H | H | H | OCF3 | H | nonafluoro-2-butyl | H | OCF3 |
| 12-421 | 2,2,2-trichloroethyl | Me | H | H | H | H | H | CF3 | H | heptafluoroisopropyl | H | OCF3 |
| 12-450 | 2,2,2-trichloroethyl | Me | H | H | H | H | H | CF3 | H | nonafluoro-2-butyl | H | OCF3 |
| 12-479 | 2,2,2-trichloroethyl | Me | H | H | H | H | H | CF3 | H | heptafluoroisopropyl | H | CF3 |
| 12-508 | 2,2,2-trichloroethyl | Me | H | H | H | H | H | CF3 | H | nonafluoro-2-butyl | H | CF3 |
| 12-527 | 2,2,2-trichloroethyl | Me | H | F | H | H | H | F | H | heptafluoroisopropyl | H | F |
| 12-528 | 2,2,2-trichloroethyl | Me | H | F | H | H | H | Cl | H | heptafluoroisopropyl | H | Cl |
| 12-529 | 2,2,2-trichloroethyl | Me | H | F | H | H | H | Br | H | heptafluoroisopropyl | H | Br |
| 12-530 | 2,2,2-trichloroethyl | Me | H | F | H | H | H | I | H | heptafluoroisopropyl | H | I |
| 12-531 | 2,2,2-trichloroethyl | Me | H | F | H | H | H | F | H | heptafluoroisopropyl | H | OCF3 |
| 12-532 | 2,2,2-trichloroethyl | Me | H | F | H | H | H | F | H | nonafluoro-2-butyl | H | OCF3 |
| 12-543 | 2,2,2-trichloroethyl | Me | H | F | H | H | H | Cl | H | heptafluoroisopropyl | H | OCF3 |
| 12-572 | 2,2,2-trichloroethyl | Me | H | F | H | H | H | Cl | H | nonafluoro-2-butyl | H | OCF3 |
| 12-601 | 2,2,2-trichloroethyl | Me | H | F | H | H | H | Br | H | heptafluoroisopropyl | H | OCF3 |
| 12-630 | 2,2,2-trichloroethyl | Me | H | F | H | H | H | Br | H | nonafluoro-2-butyl | H | OCF3 |
| 12-659 | 2,2,2-trichloroethyl | Me | H | F | H | H | H | I | H | heptafluoroisopropyl | H | OCF3 |
| 12-688 | 2,2,2-trichloroethyl | Me | H | F | H | H | H | I | H | nonafluoro-2-butyl | H | OCF3 |
| 12-707 | 2,2,2-trichloroethyl | Me | H | F | H | H | H | F | H | heptafluoroisopropyl | H | CF3 |
| 12-708 | 2,2,2-trichloroethyl | Me | H | F | H | H | H | F | H | nonafluoro-2-butyl | H | CF3 |
| 12-715 | 2-cyanoethyl | Et | H | F | H | H | H | Cl | H | heptafluoroisopropyl | H | C2F5 |
| 12-719 | 2,2,2-trichloroethyl | Me | H | F | H | H | H | Cl | H | heptafluoroisopropyl | H | CF3 |
| 12-721 | n-propyl | Me | H | F | H | H | H | Cl | H | pentafluoroethyl | H | CF3 |
| 12-725 | i-propyl | n-Pr | H | F | H | H | H | Cl | H | heptafluoroisopropyl | H | n-C3F7 |
| 12-728 | 1,3-dichloro-2-propyl | Me | H | F | H | H | H | Cl | H | pentafluoroethyl | H | CF3 |
| 12-729 | 1-chloro-3-fluoro-2-propyl | i-Pr | H | F | H | H | H | Cl | H | heptafluoroisopropyl | H | CF3 |
| 12-733 | i-butyl | CH2CH=CH2 | H | F | H | H | H | Cl | H | heptafluoroisopropyl | H | CF3 |
| 12-739 | ethyl | Me | H | F | H | H | H | Cl | H | nonafluoro-2-butyl | H | i-C3F7 |
| 12-744 | 2-cyanoethyl | CN | H | F | H | H | H | Cl | H | nonafluoro-2-butyl | H | CF3 |
| 12-748 | 2,2,2-trichloroethyl | Me | H | F | H | H | H | Cl | H | nonafluoro-2-butyl | H | CF3 |
| 12-750 | n-propyl | CH2C≡CH | H | F | H | H | H | Cl | H | nonafluoro-2-butyl | H | CF3 |
| 12-762 | i-butyl | NH2 | H | F | H | H | H | Cl | H | nonafluoro-2-butyl | H | CF3 |
| 12-767 | methyl | Me | H | F | H | H | H | Br | H | heptafluoroisopropyl | H | CF3 |
| 12-768 | ethyl | Me | H | F | H | H | H | Br | H | heptafluoroisopropyl | H | CF3 |
| 12-769 | 2-fluoroethyl | Me | H | F | H | H | H | Br | H | heptafluoroisopropyl | H | CF3 |
| 12-770 | 2-chloroethyl | Me | H | F | H | H | H | Br | H | heptafluoroisopropyl | H | CF3 |
| 12-771 | 2-bromoethyl | Me | H | F | H | H | H | Br | H | heptafluoroisopropyl | H | CF3 |
| 12-772 | 2-iodoethyl | Me | H | F | H | H | H | Br | H | heptafluoroisopropyl | H | CF3 |
| 12-773 | 2-cyanoethyl | Me | H | F | H | H | H | Br | H | heptafluoroisopropyl | H | C2F5 |

**Table 12(2)**

| compound number | Q₂ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 12-774 | 2,2-difluoroethyl | Me | H | F | H | H | H | Br | H | heptafluoroisopropyl | H | CF3 |
| 12-775 | 2,2-dichloroethyl | Me | H | F | H | H | H | Br | H | heptafluoroisopropyl | H | CF3 |
| 12-776 | 2,2,2-trifluoroethyl | Me | H | F | H | H | H | Br | H | heptafluoroisopropyl | H | CF3 |
| 12-777 | 2,2,2-trichloroethyl | Me | H | F | H | H | H | Br | H | heptafluoroisopropyl | H | CF3 |
| 12-778 | 2,2,2-tribromoethyl | Me | H | F | H | H | H | Br | H | heptafluoroisopropyl | H | CF3 |
| 12-779 | n-propyl | C(O)OMe | H | F | H | H | H | Br | H | heptafluoroisopropyl | H | CF3 |
| 12-780 | 3-fluoro-n-propyl | Me | H | F | H | H | H | Br | H | heptafluoroisopropyl | H | CF3 |
| 12-781 | 3-chloro-n-propyl | Me | H | F | H | H | H | Br | H | heptafluoroisopropyl | H | CF3 |
| 12-782 | 3-bromo-n-propyl | Me | H | F | H | H | H | Br | H | heptafluoroisopropyl | H | CF3 |
| 12-783 | i-propyl | Me | H | F | H | H | H | Br | H | heptafluoroisopropyl | H | CF3 |
| 12-784 | 1,2-difluoro-2-propyl | Me | H | F | H | H | H | Br | H | heptafluoroisopropyl | H | n-C3F7 |
| 12-785 | 1,3-difluoro-2-propyl | Me | H | F | H | H | H | Br | H | heptafluoroisopropyl | H | CF3 |
| 12-786 | 1,3-dichloro-2-propyl | Me | H | F | H | H | H | Br | H | heptafluoroisopropyl | H | CF3 |
| 12-787 | 1-chloro-3-fluoro-2-propyl | Me | H | F | H | H | H | Br | H | heptafluoroisopropyl | H | CF3 |
| 12-788 | 3,3,3-trifluoro-n-propyl | Me | H | F | H | H | H | Br | H | heptafluoroisopropyl | H | CF3 |
| 12-789 | n-butyl | Me | H | F | H | H | H | Br | H | heptafluoroisopropyl | H | CF3 |
| 12-790 | 4,4,4-trifluoro-n-butyl | Me | H | F | H | H | H | Br | H | heptafluoroisopropyl | H | CF3 |
| 12-791 | i-butyl | Me | H | F | H | H | H | Br | H | heptafluoroisopropyl | H | CF3 |
| 12-792 | s-butyl | Me | H | F | H | H | H | Br | H | heptafluoroisopropyl | H | CF3 |
| 12-793 | vinyl | C(O)C(O)OMe | H | F | H | H | H | Br | H | heptafluoroisopropyl | H | CF3 |
| 12-794 | benzyl | Me | H | F | H | H | H | Br | H | heptafluoroisopropyl | H | CF3 |
| 12-795 | phenyl | Me | H | F | H | H | H | Br | H | heptafluoroisopropyl | H | CF3 |
| 12-796 | methyl | Me | H | F | H | H | H | Br | H | nonafluoro-2-butyl | H | CF3 |
| 12-797 | ethyl | Me | H | F | H | H | H | Br | H | nonafluoro-2-butyl | H | CF3 |
| 12-798 | 2-fluoroethyl | Me | H | F | H | H | H | Br | H | nonafluoro-2-butyl | H | CF3 |
| 12-799 | 2-chloroethyl | Me | H | F | H | H | H | Br | H | nonafluoro-2-butyl | H | CF3 |
| 12-800 | 2-bromoethyl | Me | H | F | H | H | H | Br | H | nonafluoro-2-butyl | H | CF3 |
| 12-801 | 2-iodoethyl | Me | H | F | H | H | H | Br | H | nonafluoro-2-butyl | H | CF3 |
| 12-802 | 2-cyanoethyl | Me | H | F | H | H | H | Br | H | nonafluoro-2-butyl | H | CF3 |
| 12-803 | 2,2-difluoroethyl | Me | H | F | H | H | H | Br | H | nonafluoro-2-butyl | H | CF3 |
| 12-804 | 2,2-dichloroethyl | Me | H | F | H | H | H | Br | H | nonafluoro-2-butyl | H | CF3 |
| 12-805 | 2,2,2-trifluoroethyl | Me | H | F | H | H | H | Br | H | nonafluoro-2-butyl | H | CF3 |
| 12-806 | 2,2,2-trichloroethyl | Me | H | F | H | H | H | Br | H | nonafluoro-2-butyl | H | CF3 |
| 12-807 | 2,2,2-tribromoethyl | Me | H | F | H | H | H | Br | H | nonafluoro-2-butyl | H | CF3 |
| 12-808 | n-propyl | C(O)OEt | H | F | H | H | H | Br | H | nonafluoro-2-butyl | H | CF3 |
| 12-809 | 3-fluoro-n-propyl | Me | H | F | H | H | H | Br | H | nonafluoro-2-butyl | H | CF3 |
| 12-810 | 3-chloro-n-propyl | Me | H | F | H | H | H | Br | H | nonafluoro-2-butyl | H | CF3 |
| 12-811 | 3-bromo-n-propyl | Me | H | F | H | H | H | Br | H | nonafluoro-2-butyl | H | CF3 |
| 12-812 | i-propyl | C(O)Me | H | F | H | H | H | Br | H | nonafluoro-2-butyl | H | CF3 |
| 12-813 | 1,2-difluoro-2-propyl | Me | H | F | H | H | H | Br | H | nonafluoro-2-butyl | H | CF3 |
| 12-814 | 1,3-difluoro-2-propyl | Me | H | F | H | H | H | Br | H | nonafluoro-2-butyl | H | CF3 |
| 12-815 | 1,3-dichloro-2-propyl | Me | H | F | H | H | H | Br | H | nonafluoro-2-butyl | H | CF3 |
| 12-816 | 1-chloro-3-fluoro-2-propyl | Me | H | F | H | H | H | Br | H | nonafluoro-2-butyl | H | CF3 |
| 12-817 | 3,3,3-trifluoro-n-propyl | Me | H | F | H | H | H | Br | H | nonafluoro-2-butyl | H | CF3 |
| 12-818 | n-butyl | Me | H | F | H | H | H | Br | H | nonafluoro-2-butyl | H | CF3 |
| 12-819 | 4,4,4-trifluoro-n-butyl | Me | H | F | H | H | H | Br | H | nonafluoro-2-butyl | H | CF3 |
| 12-820 | i-butyl | Me | H | F | H | H | H | Br | H | nonafluoro-2-butyl | H | CF3 |
| 12-821 | s-butyl | Me | H | F | H | H | H | Br | H | nonafluoro-2-butyl | H | CF3 |
| 12-822 | vinyl | Me | H | F | H | H | H | Br | H | nonafluoro-2-butyl | H | CF3 |
| 12-823 | benzyl | Me | H | F | H | H | H | Br | H | nonafluoro-2-butyl | H | CF3 |
| 12-824 | phenyl | C(O)C(O)OEt | H | F | H | H | H | Br | H | nonafluoro-2-butyl | H | i-C3F7 |
| 12-825 | methyl | Me | H | F | H | H | H | I | H | heptafluoroisopropyl | H | CF3 |
| 12-826 | ethyl | Me | H | F | H | H | H | I | H | heptafluoroisopropyl | H | CF3 |
| 12-827 | 2-fluoroethyl | Me | H | F | H | H | H | I | H | heptafluoroisopropyl | H | CF3 |
| 12-828 | 2-chloroethyl | Me | H | F | H | H | H | I | H | heptafluoroisopropyl | H | CF3 |
| 12-829 | 2-bromoethyl | Me | H | F | H | H | H | I | H | heptafluoroisopropyl | H | CF3 |
| 12-830 | 2-iodoethyl | Me | H | F | H | H | H | I | H | heptafluoroisopropyl | H | CF3 |
| 12-831 | 2-cyanoethyl | Me | H | F | H | H | H | I | H | heptafluoroisopropyl | H | CF3 |
| 12-832 | 2,2-difluoroethyl | Me | H | F | H | H | H | I | H | heptafluoroisopropyl | H | CF3 |
| 12-833 | 2,2-dichloroethyl | Me | H | F | H | H | H | I | H | heptafluoroisopropyl | H | CF3 |
| 12-834 | 2,2,2-trifluoroethyl | Me | H | F | H | H | H | I | H | heptafluoroisopropyl | H | CF3 |
| 12-835 | 2,2,2-trichloroethyl | Me | H | F | H | H | H | I | H | heptafluoroisopropyl | H | CF3 |
| 12-836 | 2,2,2-tribromoethyl | Me | H | F | H | H | H | I | H | heptafluoroisopropyl | H | CF3 |
| 12-837 | n-propyl | Me | H | F | H | H | H | I | H | heptafluoroisopropyl | H | CF3 |

**Table 12(3)**

| compound number | Q₂ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 12-838 | 3-fluoro-n-propyl | Me | H | F | H | H | H | I | H | heptafluoroisopropyl | H | CF3 |
| 12-839 | 3-chloro-n-propyl | Me | H | F | H | H | H | I | H | heptafluoroisopropyl | H | CF3 |
| 12-840 | 3-bromo-n-propyl | C(O)Et | H | F | H | H | H | I | H | heptafluoroisopropyl | H | C2F5 |
| 12-841 | i-propyl | Me | H | F | H | H | H | I | H | heptafluoroisopropyl | H | CF3 |
| 12-842 | 12-difluoro-2-propyl | Me | H | F | H | H | H | I | H | heptafluoroisopropyl | H | CF3 |
| 12-843 | 1,3-difluoro-2-propyl | Me | H | F | H | H | H | I | H | heptafluoroisopropyl | H | CF3 |
| 12-844 | 1,3-dichloro-2-propyl | Me | H | F | H | H | H | I | H | heptafluoroisopropyl | H | CF3 |
| 12-845 | 1-chloro-3-fluoro-2-propyl | Me | H | F | H | H | H | I | H | heptafluoroisopropyl | H | CF3 |
| 12-846 | 3,3,3-trifluoro-n-propyl | Me | H | F | H | H | H | I | H | heptafluoroisopropyl | H | CF3 |
| 12-847 | n-butyl | Me | H | F | H | H | H | I | H | heptafluoroisopropyl | H | CF3 |
| 12-848 | 4,4,4-trifluoro-n-butyl | Me | H | F | H | H | H | I | H | heptafluoroisopropyl | H | CF3 |
| 12-849 | i-butyl | Me | H | F | H | H | H | I | H | heptafluoroisopropyl | H | CF3 |
| 12-850 | s-butyl | Me | H | F | H | H | H | I | H | heptafluoroisopropyl | H | CF3 |
| 12-851 | vinyl | Me | H | F | H | H | H | I | H | heptafluoroisopropyl | H | CF3 |
| 12-852 | benzyl | S(O)2Me | H | F | H | H | H | I | H | heptafluoroisopropyl | H | CF3 |
| 12-853 | phenyl | Me | H | F | H | H | H | I | H | heptafluoroisopropyl | H | CF3 |
| 12-854 | methyl | Me | H | F | H | H | H | I | H | nonafluoro-2-butyl | H | CF3 |
| 12-855 | ethyl | Me | H | F | H | H | H | I | H | nonafluoro-2-butyl | H | CF3 |
| 12-856 | 2-fluoroethyl | Me | H | F | H | H | H | I | H | nonafluoro-2-butyl | H | CF3 |
| 12-857 | 2-chloroethyl | Me | H | F | H | H | H | I | H | nonafluoro-2-butyl | H | CF3 |
| 12-858 | 2-bromoethyl | Me | H | F | H | H | H | I | H | nonafluoro-2-butyl | H | CF3 |
| 12-859 | 2-iodoethyl | Me | H | F | H | H | H | I | H | nonafluoro-2-butyl | H | CF3 |
| 12-860 | 2-cyanoethyl | S(O)2Et | H | F | H | H | H | I | H | nonafluoro-2-butyl | H | CF3 |
| 12-861 | 2,2-difluoroethyl | Me | H | F | H | H | H | I | H | nonafluoro-2-butyl | H | CF3 |
| 12-862 | 2,2-dichloroethyl | Me | H | F | H | H | H | I | H | nonafluoro-2-butyl | H | CF3 |
| 12-863 | 2,2,2-trifluoroethyl | Me | H | F | H | H | H | I | H | nonafluoro-2-butyl | H | CF3 |
| 12-864 | 2,2,2-trichloroethyl | Me | H | F | H | H | H | I | H | nonafluoro-2-butyl | H | CF3 |
| 12-865 | 2,2,2-tribromoethyl | Me | H | F | H | H | H | I | H | nonafluoro-2-butyl | H | CF3 |
| 12-866 | n-propyl | CH2Ph | H | F | H | H | H | I | H | nonafluoro-2-butyl | H | n-C3F7 |
| 12-867 | 3-fluoro-n-propyl | Me | H | F | H | H | H | I | H | nonafluoro-2-butyl | H | CF3 |
| 12-868 | 3-chloro-n-propyl | Me | H | F | H | H | H | I | H | nonafluoro-2-butyl | H | CF3 |
| 12-869 | 3-bromo-n-propyl | Me | H | F | H | H | H | I | H | nonafluoro-2-butyl | H | CF3 |
| 12-870 | i-propyl | CH2(3-Py) | H | F | H | H | H | I | H | nonafluoro-2-butyl | H | CF3 |
| 12-871 | 1,2-difluoro-2-propyl | Me | H | F | H | H | H | I | H | nonafluoro-2-butyl | H | CF3 |
| 12-872 | 1,3-difluoro-2-propyl | Me | H | F | H | H | H | I | H | nonafluoro-2-butyl | H | CF3 |
| 12-873 | 1,3-dichloro-2-propyl | Me | H | F | H | H | H | I | H | nonafluoro-2-butyl | H | CF3 |
| 12-874 | 1-chloro-3-fluoro-2-propyl | Me | H | F | H | H | H | I | H | nonafluoro-2-butyl | H | CF3 |
| 12-875 | 3,3,3-trifluoro-n-propyl | Me | H | F | H | H | H | I | H | nonafluoro-2-butyl | H | CF3 |
| 12-876 | n-butyl | Me | H | F | H | H | H | I | H | nonafluoro-2-butyl | H | CF3 |
| 12-877 | 4,4,4-trifluoro-n-butyl | Me | H | F | H | H | H | I | H | nonafluoro-2-butyl | H | CF3 |
| 12-878 | i-butyl | Me | H | F | H | H | H | I | H | nonafluoro-2-butyl | H | CF3 |
| 12-879 | s-butyl | CH2(3-Py-N-oxide) | H | F | H | H | H | I | H | nonafluoro-2-butyl | H | CF3 |
| 12-880 | vinyl | Me | H | F | H | H | H | I | H | nonafluoro-2-butyl | H | i-C3F7 |
| 12-881 | benzyl | Me | H | F | H | H | H | I | H | nonafluoro-2-butyl | H | CF3 |
| 12-882 | phenyl | Me | H | F | H | H | H | I | H | nonafluoro-2-butyl | H | CF3 |
| 12-893 | 2,2,2-trichloroethyl | Me | H | F | H | H | H | OCF3 | H | heptafluoroisopropyl | H | OCF3 |
| 12-922 | 2,2,2-trichloroethyl | Me | H | F | H | H | H | OCF3 | H | nonafluoro-2-butyl | H | OCF3 |
| 12-951 | 2,2,2-trichloroethyl | Me | H | F | H | H | H | CF3 | H | heptafluoroisopropyl | H | OCF3 |
| 12-980 | 2,2,2-trichloroethyl | Me | H | F | H | H | H | CF3 | H | nonafluoro-2-butyl | H | OCF3 |
| 12-1009 | 2,2,2-trichloroethyl | Me | H | F | H | H | H | CF3 | H | heptafluoroisopropyl | H | CF3 |
| 12-1038 | 2,2,2-trichloroethyl | Me | H | F | H | H | H | CF3 | H | nonafluoro-2-butyl | H | CF3 |
| 12-1057 | 2,2,2-trichloroethyl | Me | H | H | F | H | H | F | H | heptafluoroisopropyl | H | F |
| 12-1058 | 2,2,2-trichloroethyl | Me | H | H | F | H | H | F | H | nonafluoro-2-butyl | H | F |
| 12-1069 | 2,2,2-trichloroethyl | Me | H | H | F | H | H | Cl | H | heptafluoroisopropyl | H | Cl |
| 12-1098 | 2,2,2-trichloroethyl | Me | H | H | F | H | H | Cl | H | nonafluoro-2-butyl | H | Cl |
| 12-1127 | 2,2,2-trichloroethyl | Me | H | H | F | H | H | Br | H | heptafluoroisopropyl | H | Br |
| 12-1156 | 2,2,2-trichloroethyl | Me | H | H | F | H | H | Br | H | nonafluoro-2-butyl | H | Br |
| 12-1185 | 2,2,2-trichloroethyl | Me | H | H | F | H | H | I | H | heptafluoroisopropyl | H | I |
| 12-1214 | 2,2,2-trichloroethyl | Me | H | H | F | H | H | I | H | nonafluoro-2-butyl | H | I |
| 12-1236 | 2,2,2-trichloroethyl | Me | H | H | F | H | H | Cl | H | heptafluoroisopropyl | H | Br |
| 12-1245 | 2,2,2-trichloroethyl | Me | H | H | F | H | H | F | H | heptafluoroisopropyl | H | OCF3 |
| 12-1246 | 2,2,2-trichloroethyl | Me | H | H | F | H | H | F | H | nonafluoro-2-butyl | H | OCF3 |
| 12-1257 | 2,2,2-trichloroethyl | Me | H | H | F | H | H | Cl | H | heptafluoroisopropyl | H | OCF3 |
| 12-1286 | 2,2,2-trichloroethyl | Me | H | H | F | H | H | Cl | H | nonafluoro-2-butyl | H | OCF3 |

**Table 12(4)**

| compound number | Q₂ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 12-1315 | 2,2,2-trichloroethyl | Me | H | H | F | H | H | Br | H | heptafluoroisopropyl | H | OCF3 |
| 12-1344 | 2,2,2-trichloroethyl | Me | H | H | F | H | H | Br | H | nonafluoro-2-butyl | H | OCF3 |
| 12-1373 | 2,2,2-trichloroethyl | Me | H | H | F | H | H | I | H | heptafluoroisopropyl | H | OCF3 |
| 12-1402 | 2,2,2-trichloroethyl | Me | H | H | F | H | H | I | H | nonafluoro-2-butyl | H | OCF3 |
| 12-1421 | 2,2,2-trichloroethyl | Me | H | H | F | H | H | F | H | heptafluoroisopropyl | H | CF3 |
| 12-1422 | 2,2,2-trichloroethyl | Me | H | H | F | H | H | F | H | nonafluoro-2-butyl | H | CF3 |
| 12-1433 | 2,2,2-trichloroethyl | Me | H | H | F | H | H | Cl | H | heptafluoroisopropyl | H | CF3 |
| 12-1462 | 2,2,2-trichloroethyl | Me | H | H | F | H | H | Cl | H | nonafluoro-2-butyl | H | CF3 |
| 12-1491 | 2,2,2-trichloroethyl | Me | H | H | F | H | H | Br | H | heptafluoroisopropyl | H | CF3 |
| 12-1520 | 2,2,2-trichloroethyl | Me | H | H | F | H | H | Br | H | nonafluoro-2-butyl | H | CF3 |
| 12-1549 | 2,2,2-trichloroethyl | Me | H | H | F | H | H | I | H | heptafluoroisopropyl | H | CF3 |
| 12-1578 | 2,2,2-trichloroethyl | Me | H | H | F | H | H | I | H | nonafluoro-2-butyl | H | CF3 |
| 12-1607 | 2,2,2-trichloroethyl | Me | H | H | F | H | H | OCF3 | H | heptafluoroisopropyl | H | OCF3 |
| 12-1636 | 2,2,2-trichloroethyl | Me | H | H | F | H | H | OCF3 | H | nonafluoro-2-butyl | H | OCF3 |
| 12-1665 | 2,2,2-trichloroethyl | Me | H | H | F | H | H | CF3 | H | heptafluoroisopropyl | H | OCF3 |
| 12-1694 | 2,2,2-trichloroethyl | Me | H | H | F | H | H | CF3 | H | nonafluoro-2-butyl | H | OCF3 |
| 12-1723 | 2,2,2-trichloroethyl | Me | H | H | F | H | H | CF3 | H | heptafluoroisopropyl | H | CF3 |
| 12-1752 | 2,2,2-trichloroethyl | Me | H | H | F | H | H | CF3 | H | nonafluoro-2-butyl | H | CF3 |
| 12-1771 | 2,2,2-trichloroethyl | Me | H | F | F | H | H | F | H | heptafluoroisopropyl | H | F |
| 12-1772 | 2,2,2-trichloroethyl | Me | H | F | F | H | H | F | H | nonafluoro-2-butyl | H | F |
| 12-1783 | 2,2,2-trichloroethyl | Me | H | F | F | H | H | Cl | H | heptafluoroisopropyl | H | Cl |
| 12-1812 | 2,2,2-trichloroethyl | Me | H | F | F | H | H | Cl | H | nonafluoro-2-butyl | H | Cl |
| 12-1841 | 2,2,2-trichloroethyl | Me | H | F | F | H | H | Br | H | heptafluoroisopropyl | H | Br |
| 12-1870 | 2,2,2-trichloroethyl | Me | H | F | F | H | H | Br | H | nonafluoro-2-butyl | H | Br |
| 12-1899 | 2,2,2-trichloroethyl | Me | H | F | F | H | H | I | H | heptafluoroisopropyl | H | I |
| 12-1928 | 2,2,2-trichloroethyl | Me | H | F | F | H | H | I | H | nonafluoro-2-butyl | H | I |
| 12-1956 | 2,2,2-trichloroethyl | Me | H | F | F | H | H | Cl | H | nonafluoro-2-butyl | H | Br |
| 12-1959 | 2,2,2-trichloroethyl | Me | H | F | F | H | H | F | H | heptafluoroisopropyl | H | OCF3 |
| 12-1960 | 2,2,2-trichloroethyl | Me | H | F | F | H | H | F | H | nonafluoro-2-butyl | H | OCF3 |
| 12-1971 | 2,2,2-trichloroethyl | Me | H | F | F | H | H | Cl | H | heptafluoroisopropyl | H | OCF3 |
| 12-2000 | 2,2,2-trichloroethyl | Me | H | F | F | H | H | Cl | H | nonafluoro-2-butyl | H | OCF3 |
| 12-2029 | 2,2,2-trichloroethyl | Me | H | F | F | H | H | Br | H | heptafluoroisopropyl | H | OCF3 |
| 12-2058 | 2,2,2-trichloroethyl | Me | H | F | F | H | H | Br | H | nonafluoro-2-butyl | H | OCF3 |
| 12-2087 | 2,2,2-trichloroethyl | Me | H | F | F | H | H | I | H | heptafluoroisopropyl | H | OCF3 |
| 12-2116 | 2,2,2-trichloroethyl | Me | H | F | F | H | H | I | H | nonafluoro-2-butyl | H | OCF3 |
| 12-2135 | 2,2,2-trichloroethyl | Me | H | F | F | H | H | F | H | heptafluoroisopropyl | H | CF3 |
| 12-2136 | 2,2,2-trichloroethyl | Me | H | F | F | H | H | F | H | nonafluoro-2-butyl | H | CF3 |
| 12-2147 | 2,2,2-trichloroethyl | Me | H | F | F | H | H | Cl | H | heptafluoroisopropyl | H | CF3 |
| 12-2176 | 2,2,2-trichloroethyl | Me | H | F | F | H | H | Cl | H | nonafluoro-2-butyl | H | CF3 |
| 12-2205 | 2,2,2-trichloroethyl | Me | H | F | F | H | H | Br | H | heptafluoroisopropyl | H | CF3 |
| 12-2234 | 2,2,2-trichloroethyl | Me | H | F | F | H | H | Br | H | nonafluoro-2-butyl | H | CF3 |
| 12-2263 | 2,2,2-trichloroethyl | Me | H | F | F | H | H | I | H | heptafluoroisopropyl | H | CF3 |
| 12-2292 | 2,2,2-trichloroethyl | Me | H | F | F | H | H | I | H | nonafluoro-2-butyl | H | CF3 |
| 12-2321 | 2,2,2-trichloroethyl | Me | H | F | F | H | H | OCF3 | H | heptafluoroisopropyl | H | OCF3 |
| 12-2350 | 2,2,2-trichloroethyl | Me | H | F | F | H | H | OCF3 | H | nonafluoro-2-butyl | H | OCF3 |
| 12-2379 | 2,2,2-trichloroethyl | Me | H | F | F | H | H | CF3 | H | heptafluoroisopropyl | H | OCF3 |
| 12-2408 | 2,2,2-trichloroethyl | Me | H | F | F | H | H | CF3 | H | nonafluoro-2-butyl | H | OCF3 |
| 12-2437 | 2,2,2-trichloroethyl | Me | H | F | F | H | H | CF3 | H | heptafluoroisopropyl | H | CF3 |
| 12-2466 | 2,2,2-trichloroethyl | Me | H | F | F | H | H | CF3 | H | nonafluoro-2-butyl | H | CF3 |
| 12-2485 | 2,2,2-trichloroethyl | Me | H | F | F | F | H | Cl | H | pentafluoroethyl | H | Cl |
| 12-2486 | 2,2,2-trichloroethyl | Me | H | F | F | F | H | Br | H | heptafluoroisopropyl | H | Br |
| 12-2487 | 2,2,2-trichloroethyl | Me | H | F | F | F | H | Br | H | heptafluoroisopropyl | H | OCF3 |
| 12-2488 | 2,2,2-trichloroethyl | Me | H | F | F | F | H | I | H | heptafluoroisopropyl | H | CF3 |
| 12-2489 | 2,2,2-trichloroethyl | Me | H | F | F | F | H | CF3 | H | heptafluoroisopropyl | H | CF3 |
| 12-2490 | 2,2,2-trichloroethyl | Me | H | F | F | F | H | I | H | nonafluoro-2-butyl | H | I |
| 12-2491 | 2,2,2-trichloroethyl | Me | H | F | F | F | H | Cl | H | nonafluoro-2-butyl | H | OCF3 |
| 12-2492 | 2,2,2-trichloroethyl | Me | H | F | F | F | H | Br | H | nonafluoro-2-butyl | H | CF3 |
| 12-2493 | 2,2,2-trichloroethyl | Me | H | F | F | F | H | CF3 | H | nonafluoro-2-butyl | H | CF3 |
| 12-2494 | 2,2,2-trichloroethyl | Me | H | H | H | F | H | Cl | H | pentafluoroethyl | H | Cl |
| 12-2495 | 2,2,2-trichloroethyl | Me | H | H | H | F | H | Br | H | heptafluoroisopropyl | H | Br |
| 12-2496 | 2,2,2-trichloroethyl | Me | H | H | H | F | H | Br | H | heptafluoroisopropyl | H | OCF3 |
| 12-2497 | 2,2,2-trichloroethyl | Me | H | H | H | F | H | I | H | heptafluoroisopropyl | H | CF3 |
| 12-2498 | 2,2,2-trichloroethyl | Me | H | H | H | F | H | CF3 | H | heptafluoroisopropyl | H | CF3 |
| 12-2499 | 2,2,2-trichloroethyl | Me | H | H | H | F | H | I | H | nonafluoro-2-butyl | H | I |

**Table 12(5)**

| compound number | Q₂ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 12-2500 | 2,2,2-trichloroethyl | Me | H | H | H | F | H | Cl | H | nonafluoro-2-butyl | H | OCF3 |
| 12-2501 | 2.2.2-trichloroethyl | Me | H | H | H | F | H | Br | H | nonafluoro-2-butyl | H | CF3 |
| 12-2502 | 2.2.2-trichloroethyl | Me | H | H | H | F | H | CF3 | H | nonafluoro-2-butyl | H | CF3 |
| 12-2503 | 2,2,2-trichloroethyl | Me | H | H | H | H | F | Cl | H | pentafluoroethyl | H | Cl |
| 12-2504 | 2.2.2-trichloroethyl | Me | H | H | H | H | F | Br | H | heptafluoroisopropyl | H | Br |
| 12-2505 | 2,2,2-trichloroethyl | Me | H | H | H | H | F | Br | H | heptafluoroisopropyl | H | OCF3 |
| 12-2506 | 2,2,2-trichloroethyl | Me | H | H | H | H | F | I | H | heptafluoroisopropyl | H | CF3 |
| 12-2507 | 2,2,2-trichloroethyl | Me | H | H | H | H | F | CF3 | H | heptafluoroisopropyl | H | CF3 |
| 12-2508 | 2,2,2-trichloroethyl | Me | H | H | H | H | F | I | H | nonafluoro-2-butyl | H | I |
| 12-2509 | 2,2,2-trichloroethyl | Me | H | H | H | H | F | Cl | H | nonafluoro-2-butyl | H | OCF3 |
| 12-2510 | 2,2,2-trichloroethyl | Me | H | H | H | H | F | Br | H | nonafluoro-2-butyl | H | CF3 |
| 12-2511 | 2,2,2-trichloroethyl | Me | H | H | H | H | F | CF3 | H | nonafluoro-2-butyl | H | CF3 |
| 12-2512 | 2,2,2-trichloroethyl | Me | H | F | H | F | H | Cl | H | pentafluoroethyl | H | Cl |
| 12-2513 | 2.2.2-trichloroethyl | Me | H | F | H | F | H | Br | H | heptafluoroisopropyl | H | Br |
| 12-2514 | 2.2.2-trichloroethyl | Me | H | F | H | F | H | Br | H | heptafluoroisopropyl | H | OCF3 |
| 12-2515 | 2.2.2-trichloroethyl | Me | H | F | H | F | H | I | H | heptafluoroisopropyl | H | CF3 |
| 12-2516 | 2,2,2-trichloroethyl | Me | H | F | H | F | H | CF3 | H | heptafluoroisopropyl | H | CF3 |
| 12-2517 | 2.2.2-trichloroethyl | Me | H | F | H | F | H | I | H | nonafluoro-2-butyl | H | I |
| 12-2518 | 2,2,2-trichloroethyl | Me | H | F | H | F | H | Cl | H | nonafluoro-2-butyl | H | OCF3 |
| 12-2519 | 2.2.2-trichloroethyl | Me | H | F | H | F | H | Br | H | nonafluoro-2-butyl | H | CF3 |
| 12-2520 | 2,2,2-trichloroethyl | Me | H | F | H | F | H | CF3 | H | nonafluoro-2-butyl | H | CF3 |
| 12-2521 | 2,2,2-trichloroethyl | Me | H | F | H | H | F | Cl | H | pentafluoroethyl | H | Cl |
| 12-2522 | 2.2.2-trichloroethyl | Me | H | F | H | H | F | Br | H | heptafluoroisopropyl | H | Br |
| 12-2523 | 2.2.2-trichloroethyl | Me | H | F | H | H | F | Br | H | heptafluoroisopropyl | H | OCF3 |
| 12-2524 | 2.2.2-trichloroethyl | Me | H | F | H | H | F | I | H | heptafluoroisopropyl | H | CF3 |
| 12-2525 | 2.2.2-trichloroethyl | Me | H | F | H | H | F | CF3 | H | heptafluoroisopropyl | H | CF3 |
| 12-2526 | 2.2.2-trichloroethyl | Me | H | F | H | H | F | I | H | nonafluoro-2-butyl | H | I |
| 12-2527 | 2,2,2-trichloroethyl | Me | H | F | H | H | F | Cl | H | nonafluoro-2-butyl | H | OCF3 |
| 12-2528 | 2.2.2-trichloroethyl | Me | H | F | H | H | F | Br | H | nonafluoro-2-butyl | H | CF3 |
| 12-2529 | 2.2.2-trichloroethyl | Me | H | F | H | H | F | CF3 | H | nonafluoro-2-butyl | H | CF3 |
| 12-2530 | 2.2.2-trichloroethyl | Me | H | H | F | F | H | Cl | H | pentafluoroethyl | H | Cl |
| 12-2531 | 2.2.2-trichloroethyl | Me | H | H | F | F | H | Br | H | heptafluoroisopropyl | H | Br |
| 12-2532 | 2.2.2-trichloroethyl | Me | H | H | F | F | H | Br | H | heptafluoroisopropyl | H | OCF3 |
| 12-2533 | 2.2.2-trichloroethyl | Me | H | H | F | F | H | I | H | heptafluoroisopropyl | H | CF3 |
| 12-2534 | 2.2.2-trichloroethyl | Me | H | H | F | F | H | CF3 | H | heptafluoroisopropyl | H | CF3 |
| 12-2535 | 2.2.2-trichloroethyl | Me | H | H | F | F | H | I | H | nonafluoro-2-butyl | H | I |
| 12-2536 | 2,2,2-trichloroethyl | Me | H | H | F | F | H | Cl | H | nonafluoro-2-butyl | H | OCF3 |
| 12-2537 | 2.2.2-trichloroethyl | Me | H | H | F | F | H | Br | H | nonafluoro-2-butyl | H | CF3 |
| 12-2538 | 2.2.2-trichloroethyl | Me | H | H | F | F | H | CF3 | H | nonafluoro-2-butyl | H | CF3 |
| 12-2539 | 2,2,2-trichloroethyl | Me | H | H | F | H | F | Cl | H | pentafluoroethyl | H | Cl |
| 12-2540 | 2.2.2-trichloroethyl | Me | H | H | F | H | F | Br | H | heptafluoroisopropyl | H | Br |
| 12-2541 | 2.2.2-trichloroethyl | Me | H | H | F | H | F | Br | H | heptafluoroisopropyl | H | OCF3 |
| 12-2542 | 2.2.2-trichloroethyl | Me | H | H | F | H | F | I | H | heptafluoroisopropyl | H | CF3 |
| 12-2543 | 2.2.2-trichloroethyl | Me | H | H | F | H | F | CF3 | H | heptafluoroisopropyl | H | CF3 |
| 12-2544 | 2.2.2-trichloroethyl | Me | H | H | F | H | F | I | H | nonafluoro-2-butyl | H | I |
| 12-2545 | 2,2,2-trichloroethyl | Me | H | H | F | H | F | Cl | H | nonafluoro-2-butyl | H | OCF3 |
| 12-2546 | 2.2.2-trichloroethyl | Me | H | H | F | H | F | Br | H | nonafluoro-2-butyl | H | CF3 |
| 12-2547 | 2.2.2-trichloroethyl | Me | H | H | F | H | F | CF3 | H | nonafluoro-2-butyl | H | CF3 |
| 12-2548 | 2,2,2-trichloroethyl | Me | H | H | H | F | F | Cl | H | pentafluoroethyl | H | Cl |
| 12-2549 | 2.2.2-trichloroethyl | Me | H | H | H | F | F | Br | H | heptafluoroisopropyl | H | Br |
| 12-2550 | 2.2.2-trichloroethyl | Me | H | H | H | F | F | Br | H | heptafluoroisopropyl | H | OCF3 |
| 12-2551 | 2.2.2-trichloroethyl | Me | H | H | H | F | F | I | H | heptafluoroisopropyl | H | CF3 |
| 12-2552 | 2.2.2-trichloroethyl | Me | H | H | H | F | F | CF3 | H | heptafluoroisopropyl | H | CF3 |
| 12-2553 | 2.2.2-trichloroethyl | Me | H | H | H | F | F | I | H | nonafluoro-2-butyl | H | I |
| 12-2554 | 2,2,2-trichloroethyl | Me | H | H | H | F | F | Cl | H | nonafluoro-2-butyl | H | OCF3 |
| 12-2555 | 2.2.2-trichloroethyl | Me | H | H | H | F | F | Br | H | nonafluoro-2-butyl | H | CF3 |
| 12-2556 | 2,2,2-trichloroethyl | Me | H | H | H | F | F | CF3 | H | nonafluoro-2-butyl | H | CF3 |
| 12-2557 | 2,2,2-trichloroethyl | Me | H | F | F | H | F | Cl | H | pentafluoroethyl | H | Cl |
| 12-2558 | 2.2.2-trichloroethyl | Me | H | F | F | H | F | Br | H | heptafluoroisopropyl | H | Br |
| 12-2559 | 2.2.2-trichloroethyl | Me | H | F | F | H | F | Br | H | heptafluoroisopropyl | H | OCF3 |
| 12-2560 | 2.2.2-trichloroethyl | Me | H | F | F | H | F | I | H | heptafluoroisopropyl | H | CF3 |
| 12-2561 | 2.2.2-trichloroethyl | Me | H | F | F | H | F | CF3 | H | heptafluoroisopropyl | H | CF3 |
| 12-2562 | 2.2.2-trichloroethyl | Me | H | F | F | H | F | I | H | nonafluoro-2-butyl | H | I |
| 12-2563 | 2,2,2-trichloroethyl | Me | H | F | F | H | F | Cl | H | nonafluoro-2-butyl | H | OCF3 |

**Table 12(6)**

| compound number | Q₂ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 12-2564 | 2,2,2-trichloroethyl | Me | H | F | F | H | F | Br | H | nonafluoro-2-butyl | H | CF3 |
| 12-2565 | 2.2.2-trichloroethyl | Me | H | F | F | H | F | CF3 | H | nonafluoro-2-butyl | H | CF3 |
| 12-2566 | 2,2,2-trichloroethyl | Me | H | F | H | F | F | Cl | H | pentafluoroethyl | H | Cl |
| 12-2567 | 2.2.2-trichloroethyl | Me | H | F | H | F | F | Br | H | heptafluoroisopropyl | H | Br |
| 12-2568 | 2,2,2-trichloroethyl | Me | H | F | H | F | F | Br | H | heptafluoroisopropyl | H | OCF3 |
| 12-2569 | 2.2.2-trichloroethyl | Me | H | F | H | F | F | I | H | heptafluoroisopropyl | H | CF3 |
| 12-2570 | 2.2.2-trichloroethyl | Me | H | F | H | F | F | CF3 | H | heptafluoroisopropyl | H | CF3 |
| 12-2571 | 2.2.2-trichloroethyl | Me | H | F | H | F | F | I | H | nonafluoro-2-butyl | H | I |
| 12-2572 | 2,2,2-trichloroethyl | Me | H | F | H | F | F | Cl | H | nonafluoro-2-butyl | H | OCF3 |
| 12-2573 | 2.2.2-trichloroethyl | Me | H | F | H | F | F | Br | H | nonafluoro-2-butyl | H | CF3 |
| 12-2574 | 2.2.2-trichloroethyl | Me | H | F | H | F | F | CF3 | H | nonafluoro-2-butyl | H | CF3 |
| 12-2575 | 2,2,2-trichloroethyl | Me | H | H | F | F | F | Cl | H | pentafluoroethyl | H | Cl |
| 12-2576 | 2,2,2-trichloroethyl | Me | H | H | F | F | F | Br | H | heptafluoroisopropyl | H | Br |
| 12-2577 | 2.2.2-trichloroethyl | Me | H | H | F | F | F | Br | H | heptafluoroisopropyl | H | OCF3 |
| 12-2578 | 2.2.2-trichloroethyl | Me | H | H | F | F | F | I | H | heptafluoroisopropyl | H | CF3 |
| 12-2579 | 2.2.2-trichloroethyl | Me | H | H | F | F | F | CF3 | H | heptafluoroisopropyl | H | CF3 |
| 12-2580 | 2,2,2-trichloroethyl | Me | H | H | F | F | F | I | H | nonafluoro-2-butyl | H | I |
| 12-2581 | 2,2,2-trichloroethyl | Me | H | H | F | F | F | Cl | H | nonafluoro-2-butyl | H | OCF3 |
| 12-2582 | 2.2.2-trichloroethyl | Me | H | H | F | F | F | Br | H | nonafluoro-2-butyl | H | CF3 |
| 12-2583 | 2.2.2-trichloroethyl | Me | H | H | F | F | F | CF3 | H | nonafluoro-2-butyl | H | CF3 |
| 12-2584 | 2,2,2-trichloroethyl | Me | H | F | F | F | F | Cl | H | pentafluoroethyl | H | Cl |
| 12-2585 | 2.2.2-trichloroethyl | Me | H | F | F | F | F | Br | H | heptafluoroisopropyl | H | Br |
| 12-2586 | 2.2.2-trichloroethyl | Me | H | F | F | F | F | Br | H | heptafluoroisopropyl | H | OCF3 |
| 12-2587 | 2.2.2-trichloroethyl | Me | H | F | F | F | F | I | H | heptafluoroisopropyl | H | CF3 |
| 12-2588 | 2.2.2-trichloroethyl | Me | H | F | F | F | F | CF3 | H | heptafluoroisopropyl | H | CF3 |
| 12-2589 | 2.2.2-trichloroethyl | Me | H | F | F | F | F | I | H | nonafluoro-2-butyl | H | I |
| 12-2590 | 2,2,2-trichloroethyl | Me | H | F | F | F | F | Cl | H | nonafluoro-2-butyl | H | OCF3 |
| 12-2591 | 2.2.2-trichloroethyl | Me | H | F | F | F | F | Br | H | nonafluoro-2-butyl | H | CF3 |
| 12-2592 | 2.2.2-trichloroethyl | Me | H | F | F | F | F | CF3 | H | nonafluoro-2-butyl | H | CF3 |
| 12-2593 | 2.2.2-trichloroethyl | Me | H | H | CN | H | H | F | H | heptafluoroisopropyl | H | OCF3 |
| 12-2594 | 2,2,2-trichloroethyl | Me | H | H | CN | H | H | F | H | nonafluoro-2-butyl | H | OCF3 |
| 12-2605 | 2.2.2-trichloroethyl | Me | H | H | CN | H | H | Cl | H | heptafluoroisopropyl | H | OCF3 |
| 12-2634 | 2.2.2-trichloroethyl | Me | H | H | CN | H | H | Cl | H | nonafluoro-2-butyl | H | OCF3 |
| 12-2663 | 2.2.2-trichloroethyl | Me | H | H | CN | H | H | Br | H | heptafluoroisopropyl | H | OCF3 |
| 12-2692 | 2.2.2-trichloroethyl | Me | H | H | CN | H | H | Br | H | nonafluoro-2-butyl | H | OCF3 |
| 12-2721 | 2.2.2-trichloroethyl | Me | H | H | CN | H | H | I | H | heptafluoroisopropyl | H | OCF3 |
| 12-2750 | 2.2.2-trichloroethyl | Me | H | H | CN | H | H | I | H | nonafluoro-2-butyl | H | OCF3 |
| 12-2769 | 2.2.2-trichloroethyl | Me | H | H | CN | H | H | F | H | heptafluoroisopropyl | H | CF3 |
| 12-2770 | 2.2.2-trichloroethyl | Me | H | H | CN | H | H | F | H | nonafluoro-2-butyl | H | CF3 |
| 12-2781 | 2.2.2-trichloroethyl | Me | H | H | CN | H | H | Cl | H | heptafluoroisopropyl | H | CF3 |
| 12-2810 | 2.2.2-trichloroethyl | Me | H | H | CN | H | H | Cl | H | nonafluoro-2-butyl | H | CF3 |
| 12-2839 | 2.2.2-trichloroethyl | Me | H | H | CN | H | H | Br | H | heptafluoroisopropyl | H | CF3 |
| 12-2868 | 2.2.2-trichloroethyl | Me | H | H | CN | H | H | Br | H | nonafluoro-2-butyl | H | CF3 |
| 12-2897 | 2.2.2-trichloroethyl | Me | H | H | CN | H | H | I | H | heptafluoroisopropyl | H | CF3 |
| 12-2926 | 2.2.2-trichloroethyl | Me | H | H | CN | H | H | I | H | nonafluoro-2-butyl | H | CF3 |
| 12-2955 | 2.2.2-trichloroethyl | Me | H | H | CN | H | H | OCF3 | H | heptafluoroisopropyl | H | OCF3 |
| 12-2984 | 2.2.2-trichloroethyl | Me | H | H | CN | H | H | OCF3 | H | nonafluoro-2-butyl | H | OCF3 |
| 12-3013 | 2.2.2-trichloroethyl | Me | H | H | CN | H | H | CF3 | H | heptafluoroisopropyl | H | OCF3 |
| 12-3042 | 2.2.2-trichloroethyl | Me | H | H | CN | H | H | CF3 | H | nonafluoro-2-butyl | H | OCF3 |
| 12-3071 | 2.2.2-trichloroethyl | Me | H | H | CN | H | H | CF3 | H | heptafluoroisopropyl | H | CF3 |
| 12-3100 | 2.2.2-trichloroethyl | Me | H | H | CN | H | H | CF3 | H | nonafluoro-2-butyl | H | CF3 |
| 12-3119 | 2.2.2-trichloroethyl | Me | H | F | CN | H | H | F | H | heptafluoroisopropyl | H | F |
| 12-3120 | 2.2.2-trichloroethyl | Me | H | F | CN | H | H | F | H | nonafluoro-2-butyl | H | F |
| 12-3131 | 2.2.2-trichloroethyl | Me | H | F | CN | H | H | Cl | H | heptafluoroisopropyl | H | Cl |
| 12-3160 | 2.2.2-trichloroethyl | Me | H | F | CN | H | H | Cl | H | nonafluoro-2-butyl | H | Cl |
| 12-3189 | 2.2.2-trichloroethyl | Me | H | F | CN | H | H | Br | H | heptafluoroisopropyl | H | Br |
| 12-3218 | 2,2,2-trichloroethyl | Me | H | F | CN | H | H | Br | H | nonafluoro-2-butyl | H | Br |
| 12-3247 | 2.2.2-trichloroethyl | Me | H | F | CN | H | H | I | H | heptafluoroisopropyl | H | I |
| 12-3276 | 2.2.2-trichloroethyl | Me | H | F | CN | H | H | I | H | nonafluoro-2-butyl | H | I |
| 12-3298 | 2.2.2-trichloroethyl | Me | H | F | CN | H | H | Cl | H | heptafluoroisopropyl | H | Br |
| 12-3307 | 2.2.2-trichloroethyl | Me | H | F | CN | H | H | F | H | heptafluoroisopropyl | H | OCF3 |
| 12-3308 | 2.2.2-trichloroethyl | Me | H | F | CN | H | H | F | H | nonafluoro-2-butyl | H | OCF3 |
| 12-3319 | 2.2.2-trichloroethyl | Me | H | F | CN | H | H | Cl | H | heptafluoroisopropyl | H | OCF3 |
| 12-3348 | 2.2.2-trichloroethyl | Me | H | F | CN | H | H | Cl | H | nonafluoro-2-butyl | H | OCF3 |

**Table 12(7)**

| compound number | Q₂ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 12-3377 | 2.2.2-trichloroethyl | Me | H | F | CN | H | H | Br | H | heptafluoroisopropyl | H | OCF3 |
| 12-3406 | 2,2,2-trichloroethyl | Me | H | F | CN | H | H | Br | H | nonafluoro-2-butyl | H | OCF3 |
| 12-3435 | 2.2.2-trichloroethyl | Me | H | F | CN | H | H | I | H | heptafluoroisopropyl | H | OCF3 |
| 12-3464 | 2.2.2-trichloroethyl | Me | H | F | CN | H | H | I | H | nonafluoro-2-butyl | H | OCF3 |
| 12-3483 | 2,2,2-trichloroethyl | Me | H | F | CN | H | H | F | H | heptafluoroisopropyl | H | CF3 |
| 12-3484 | 2,2,2-trichloroethyl | Me | H | F | CN | H | H | F | H | nonafluoro-2-butyl | H | CF3 |
| 12-3495 | 2.2.2-trichloroethyl | Me | H | F | CN | H | H | Cl | H | heptafluoroisopropyl | H | CF3 |
| 12-3524 | 2.2.2-trichloroethyl | Me | H | F | CN | H | H | Cl | H | nonafluoro-2-butyl | H | CF3 |
| 12-3553 | 2.2.2-trichloroethyl | Me | H | F | CN | H | H | Br | H | heptafluoroisopropyl | H | CF3 |
| 12-3582 | 2.2.2-trichloroethyl | Me | H | F | CN | H | H | Br | H | nonafluoro-2-butyl | H | CF3 |
| 12-3611 | 2.2.2-trichloroethyl | Me | H | F | CN | H | H | I | H | heptafluoroisopropyl | H | CF3 |
| 12-3640 | 2.2.2-trichloroethyl | Me | H | F | CN | H | H | I | H | nonafluoro-2-butyl | H | CF3 |
| 12-3669 | 2.2.2-trichloroethyl | Me | H | F | CN | H | H | OCF3 | H | heptafluoroisopropyl | H | OCF3 |
| 12-3698 | 2.2.2-trichloroethyl | Me | H | F | CN | H | H | OCF3 | H | nonafluoro-2-butyl | H | OCF3 |
| 12-3727 | 2.2.2-trichloroethyl | Me | H | F | CN | H | H | CF3 | H | heptafluoroisopropyl | H | OCF3 |
| 12-3756 | 2.2.2-trichloroethyl | Me | H | F | CN | H | H | CF3 | H | nonafluoro-2-butyl | H | OCF3 |
| 12-3785 | 2.2.2-trichloroethyl | Me | H | F | CN | H | H | CF3 | H | heptafluoroisopropyl | H | CF3 |
| 12-3814 | 2.2.2-trichloroethyl | Me | H | F | CN | H | H | CF3 | H | nonafluoro-2-butyl | H | CF3 |
| 12-3833 | 2.2.2-trichloroethyl | Me | H | F | CN | F | H | F | H | heptafluoroisopropyl | H | F |
| 12-3834 | 2.2.2-trichloroethyl | Me | H | F | CN | F | H | F | H | nonafluoro-2-butyl | H | F |
| 12-3845 | 2.2.2-trichloroethyl | Me | H | F | CN | F | H | Cl | H | heptafluoroisopropyl | H | Cl |
| 12-3874 | 2.2.2-trichloroethyl | Me | H | F | CN | F | H | Cl | H | nonafluoro-2-butyl | H | Cl |
| 12-3903 | 2.2.2-trichloroethyl | Me | H | F | CN | F | H | Br | H | heptafluoroisopropyl | H | Br |
| 12-3932 | 2.2.2-trichloroethyl | Me | H | F | CN | F | H | Br | H | nonafluoro-2-butyl | H | Br |
| 12-3961 | 2.2.2-trichloroethyl | Me | H | F | CN | F | H | I | H | heptafluoroisopropyl | H | I |
| 12-3990 | 2.2.2-trichloroethyl | Me | H | F | CN | F | H | I | H | nonafluoro-2-butyl | H | I |
| 12-4018 | 2.2.2-trichloroethyl | Me | H | F | CN | F | H | Cl | H | nonafluoro-2-butyl | H | Br |
| 12-4021 | 2.2.2-trichloroethyl | Me | H | F | CN | F | H | F | H | heptafluoroisopropyl | H | OCF3 |
| 12-4022 | 2.2.2-trichloroethyl | Me | H | F | CN | F | H | F | H | nonafluoro-2-butyl | H | OCF3 |
| 12-4033 | 2.2.2-trichloroethyl | Me | H | F | CN | F | H | Cl | H | heptafluoroisopropyl | H | OCF3 |
| 12-4062 | 2,2,2-trichloroethyl | Me | H | F | CN | F | H | Cl | H | nonafluoro-2-butyl | H | OCF3 |
| 12-4091 | 2.2.2-trichloroethyl | Me | H | F | CN | F | H | Br | H | heptafluoroisopropyl | H | OCF3 |
| 12-4120 | 2.2.2-trichloroethyl | Me | H | F | CN | F | H | Br | H | nonafluoro-2-butyl | H | OCF3 |
| 12-4149 | 2.2.2-trichloroethyl | Me | H | F | CN | F | H | I | H | heptafluoroisopropyl | H | OCF3 |
| 12-4178 | 2.2.2-trichloroethyl | Me | H | F | CN | F | H | I | H | nonafluoro-2-butyl | H | OCF3 |
| 12-4197 | 2.2.2-trichloroethyl | Me | H | F | CN | F | H | F | H | heptafluoroisopropyl | H | CF3 |
| 12-4198 | 2.2.2-trichloroethyl | Me | H | F | CN | F | H | F | H | nonafluoro-2-butyl | H | CF3 |
| 12-4209 | 2.2.2-trichloroethyl | Me | H | F | CN | F | H | Cl | H | heptafluoroisopropyl | H | CF3 |
| 12-4238 | 2.2.2-trichloroethyl | Me | H | F | CN | F | H | Cl | H | nonafluoro-2-butyl | H | CF3 |
| 12-4267 | 2.2.2-trichloroethyl | Me | H | F | CN | F | H | Br | H | heptafluoroisopropyl | H | CF3 |
| 12-4296 | 2.2.2-trichloroethyl | Me | H | F | CN | F | H | Br | H | nonafluoro-2-butyl | H | CF3 |
| 12-4325 | 2.2.2-trichloroethyl | Me | H | F | CN | F | H | I | H | heptafluoroisopropyl | H | CF3 |
| 12-4354 | 2.2.2-trichloroethyl | Me | H | F | CN | F | H | I | H | nonafluoro-2-butyl | H | CF3 |
| 12-4383 | 2.2.2-trichloroethyl | Me | H | F | CN | F | H | OCF3 | H | heptafluoroisopropyl | H | OCF3 |
| 12-4412 | 2.2.2-trichloroethyl | Me | H | F | CN | F | H | OCF3 | H | nonafluoro-2-butyl | H | OCF3 |
| 12-4441 | 2.2.2-trichloroethyl | Me | H | F | CN | F | H | CF3 | H | heptafluoroisopropyl | H | OCF3 |
| 12-4470 | 2.2.2-trichloroethyl | Me | H | F | CN | F | H | CF3 | H | nonafluoro-2-butyl | H | OCF3 |
| 12-4499 | 2.2.2-trichloroethyl | Me | H | F | CN | F | H | CF3 | H | heptafluoroisopropyl | H | CF3 |
| 12-4528 | 2.2.2-trichloroethyl | Me | H | F | CN | F | H | CF3 | H | nonafluoro-2-butyl | H | CF3 |

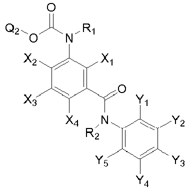

**Table 13(1)**

| compound number | Q₂ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 13-1 | 2,2,2-trichloroethyl | H | Me | H | H | H | H | F | H | heptafluoroisopropyl | H | OCF3 |
| 13-2 | 2.2.2-trichloroethyl | H | Me | H | H | H | H | F | H | nonafluoro-2-butyl | H | OCF3 |
| 13-13 | 2,2,2-trichloroethyl | H | Me | H | H | H | H | Cl | H | heptafluoroisopropyl | H | OCF3 |
| 13-42 | 2,2,2-trichloroethyl | H | Me | H | H | H | H | Cl | H | nonafluoro-2-butyl | H | OCF3 |
| 13-71 | 2,2,2-trichloroethyl | H | Me | H | H | H | H | Br | H | heptafluoroisopropyl | H | OCF3 |
| 13-100 | 2.2.2-trichloroethyl | H | Me | H | H | H | H | Br | H | nonafluoro-2-butyl | H | OCF3 |
| 13-129 | 2.2.2-trichloroethyl | H | Me | H | H | H | H | I | H | heptafluoroisopropyl | H | OCF3 |
| 13-158 | 2.2.2-trichloroethyl | H | Me | H | H | H | H | I | H | nonafluoro-2-butyl | H | OCF3 |
| 13-177 | 2,2,2-trichloroethyl | H | Me | H | H | H | H | F | H | heptafluoroisopropyl | H | CF3 |
| 13-178 | 2.2.2-trichloroethyl | H | Me | H | H | H | H | F | H | nonafluoro-2-butyl | H | CF3 |
| 13-189 | 2.2.2-trichloroethyl | H | Me | H | H | H | H | Cl | H | heptafluoroisopropyl | H | CF3 |
| 13-218 | 2.2.2-trichloroethyl | H | Me | H | H | H | H | Cl | H | nonafluoro-2-butyl | H | CF3 |
| 13-247 | 2,2,2-trichloroethyl | H | Me | H | H | H | H | Br | H | heptafluoroisopropyl | H | CF3 |
| 13-276 | 2.2.2-trichloroethyl | H | Me | H | H | H | H | Br | H | nonafluoro-2-butyl | H | CF3 |
| 13-305 | 2.2.2-trichloroethyl | H | Me | H | H | H | H | I | H | heptafluoroisopropyl | H | CF3 |
| 13-334 | 2.2.2-trichloroethyl | H | Me | H | H | H | H | I | H | nonafluoro-2-butyl | H | CF3 |
| 13-363 | 2,2,2-trichloroethyl | H | Me | H | H | H | H | OCF3 | H | heptafluoroisopropyl | H | OCF3 |
| 13-392 | 2.2.2-trichloroethyl | H | Me | H | H | H | H | OCF3 | H | nonafluoro-2-butyl | H | OCF3 |
| 13-421 | 2.2.2-trichloroethyl | H | Me | H | H | H | H | CF3 | H | heptafluoroisopropyl | H | OCF3 |
| 13-450 | 2.2.2-trichloroethyl | H | Me | H | H | H | H | CF3 | H | nonafluoro-2-butyl | H | OCF3 |
| 13-479 | 2,2,2-trichloroethyl | H | Me | H | H | H | H | CF3 | H | heptafluoroisopropyl | H | CF3 |
| 13-508 | 2.2.2-trichloroethyl | H | Me | H | H | H | H | CF3 | H | nonafluoro-2-butyl | H | CF3 |
| 13-527 | 2.2.2-trichloroethyl | H | Me | F | H | H | H | F | H | heptafluoroisopropyl | H | F |
| 13-528 | 2,2,2-trichloroethyl | H | Me | F | H | H | H | Cl | H | heptafluoroisopropyl | H | Cl |
| 13-529 | 2,2,2-trichloroethyl | H | Me | F | H | H | H | Br | H | heptafluoroisopropyl | H | Br |
| 13-530 | 2.2.2-trichloroethyl | H | Me | F | H | H | H | I | H | heptafluoroisopropyl | H | I |
| 13-531 | 2.2.2-trichloroethyl | H | Me | F | H | H | H | F | H | heptafluoroisopropyl | H | OCF3 |
| 13-532 | 2.2.2-trichloroethyl | H | Me | F | H | H | H | F | H | nonafluoro-2-butyl | H | OCF3 |
| 13-543 | 2,2,2-trichloroethyl | H | Me | F | H | H | H | Cl | H | heptafluoroisopropyl | H | OCF3 |
| 13-572 | 2,2,2-trichloroethyl | H | Me | F | H | H | H | Cl | H | nonafluoro-2-butyl | H | OCF3 |
| 13-601 | 2.2.2-trichloroethyl | H | Me | F | H | H | H | Br | H | heptafluoroisopropyl | H | OCF3 |
| 13-630 | 2.2.2-trichloroethyl | H | Me | F | H | H | H | Br | H | nonafluoro-2-butyl | H | OCF3 |
| 13-659 | 2.2.2-trichloroethyl | H | Me | F | H | H | H | I | H | heptafluoroisopropyl | H | OCF3 |
| 13-688 | 2.2.2-trichloroethyl | H | Me | F | H | H | H | I | H | nonafluoro-2-butyl | H | OCF3 |
| 13-707 | 2.2.2-trichloroethyl | H | Me | F | H | H | H | F | H | heptafluoroisopropyl | H | CF3 |
| 13-708 | 2.2.2-trichloroethyl | H | Me | F | H | H | H | F | H | nonafluoro-2-butyl | H | CF3 |
| 13-715 | 2-cyanoethyl | H | Et | F | H | H | H | Cl | H | heptafluoroisopropyl | H | C2F5 |
| 13-719 | 2,2,2-trichloroethyl | H | Me | F | H | H | H | Cl | H | heptafluoroisopropyl | H | CF3 |
| 13-721 | n-propyl | H | Me | F | H | H | H | Cl | H | pentafluoroethyl | H | CF3 |
| 13-725 | i-propyl | H | n-Pr | F | H | H | H | Cl | H | heptafluoroisopropyl | H | n-C3F7 |
| 13-728 | 1,3-dichloro-2-propyl | H | Me | F | H | H | H | Cl | H | pentafluoroethyl | H | CF3 |
| 13-729 | 1-chloro-3-fluoro-2-propyl | H | i-Pr | F | H | H | H | Cl | H | heptafluoroisopropyl | H | CF3 |
| 13-733 | i-butyl | H | CH2CH=CH2 | F | H | H | H | Cl | H | heptafluoroisopropyl | H | CF3 |
| 13-739 | ethyl | H | Me | F | H | H | H | Cl | H | nonafluoro-2-butyl | H | i-C3F7 |
| 13-744 | 2-cyanoethyl | H | CN | F | H | H | H | Cl | H | nonafluoro-2-butyl | H | CF3 |
| 13-748 | 2,2,2⁻trichloroethyl | H | Me | F | H | H | H | Cl | H | nonafluoro-2-butyl | H | CF3 |
| 13-750 | n-propyl | H | CH2C≡CH | F | H | H | H | Cl | H | nonafluoro-2-butyl | H | CF3 |
| 13-762 | i-butyl | H | NH2 | F | H | H | H | Cl | H | nonafluoro-2-butyl | H | CF3 |
| 13-773 | 2-cyanoethyl | H | Me | F | H | H | H | Br | H | heptafluoroisopropyl | H | C2F5 |
| 13-777 | 2.2.2-trichloroethyl | H | Me | F | H | H | H | Br | H | heptafluoroisopropyl | H | CF3 |
| 13-779 | n-propyl | H | C(O)OMe | F | H | H | H | Br | H | heptafluoroisopropyl | H | CF3 |
| 13-784 | 1,2-difluoro-2-propyl | H | Me | F | H | H | H | Br | H | heptafluoroisopropyl | H | n-C3F7 |
| 13-790 | 4,4,4-trifluoro-n-butyl | H | Me | F | H | H | H | Br | H | heptafluoroisopropyl | H | CF3 |
| 13-793 | vinyl | H | C(O)C(O)OMe | F | H | H | H | Br | H | heptafluoroisopropyl | H | CF3 |
| 13-806 | 2.2.2-trichloroethyl | H | Me | F | H | H | H | Br | H | nonafluoro-2-butyl | H | CF3 |

**Table 13(2)**

| compound number | Q₂ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 13-808 | n-propyl | H | C(O)OEt | F | H | H | H | Br | H | nonafluoro-2-butyl | H | CF3 |
| 13-812 | i-propyl | H | C(O)Me | F | H | H | H | Br | H | nonafluoro-2-butyl | H | CF3 |
| 13-819 | 4,4,4-trifluoro-n-butyl | H | Me | F | H | H | H | Br | H | nonafluoro-2-butyl | H | CF3 |
| 13-824 | phenyl | H | C(O)C(O)OEt | F | H | H | H | Br | H | nonafluoro-2-butyl | H | i-C3F7 |
| 13-835 | 2,2,2-trichloroethyl | H | Me | F | H | H | H | I | H | heptafluoroisopropyl | H | CF3 |
| 13-840 | 3-bromo-n-propyl | H | C(O)Et | F | H | H | H | I | H | heptafluoroisopropyl | H | C2F5 |
| 13-848 | 4,4,4-trifluoro-n-butyl | H | Me | F | H | H | H | I | H | heptafluoroisopropyl | H | CF3 |
| 13-852 | benzyl | H | S(O)2Me | F | H | H | H | I | H | heptafluoroisopropyl | H | CF3 |
| 13-860 | 2-cyanoethyl | H | S(O)2Et | F | H | H | H | I | H | nonafluoro-2-butyl | H | CF3 |
| 13-864 | 2,2,2-trichloroethyl | H | Me | F | H | H | H | I | H | nonafluoro-2-butyl | H | CF3 |
| 13-866 | n-propyl | H | CH2Ph | F | H | H | H | I | H | nonafluoro-2-butyl | H | n-C3F7 |
| 13-870 | i-propyl | H | CH2(3-Py) | F | H | H | H | I | H | nonafluoro-2-butyl | H | CF3 |
| 13-877 | 4,4,4-trifluoro-n-butyl | H | Me | F | H | H | H | I | H | nonafluoro-2-butyl | H | CF3 |
| 13-879 | s-butyl | H | CH2(3-Py-N-oxide) | F | H | H | H | I | H | nonafluoro-2-butyl | H | CF3 |
| 13-880 | vinyl | H | Me | F | H | H | H | I | H | nonafluoro-2-butyl | H | i-C3F7 |
| 13-893 | 2.2.2-trichloroethyl | H | Me | F | H | H | H | OCF3 | H | heptafluoroisopropyl | H | OCF3 |
| 13-922 | 2.2.2-trichloroethyl | H | Me | F | H | H | H | OCF3 | H | nonafluoro-2-butyl | H | OCF3 |
| 13-951 | 2.2.2-trichloroethyl | H | Me | F | H | H | H | CF3 | H | heptafluoroisopropyl | H | OCF3 |
| 13-980 | 2.2.2-trichloroethyl | H | Me | F | H | H | H | CF3 | H | nonafluoro-2-butyl | H | OCF3 |
| 13-1009 | 2.2.2-trichloroethyl | H | Me | F | H | H | H | CF3 | H | heptafluoroisopropyl | H | CF3 |
| 13-1038 | 2.2.2-trichloroethyl | H | Me | F | H | H | H | CF3 | H | nonafluoro-2-butyl | H | CF3 |
| 13-1057 | 2.2.2-trichloroethyl | H | Me | H | F | H | H | F | H | heptafluoroisopropyl | H | F |
| 13-1058 | 2.2.2-trichloroethyl | H | Me | H | F | H | H | F | H | nonafluoro-2-butyl | H | F |
| 13-1069 | 2,2,2-trichloroethyl | H | Me | H | F | H | H | Cl | H | heptafluoroisopropyl | H | Cl |
| 13-1098 | 2,2,2-trichloroethyl | H | Me | H | F | H | H | Cl | H | nonafluoro-2-butyl | H | Cl |
| 13-1127 | 2.2.2-trichloroethyl | H | Me | H | F | H | H | Br | H | heptafluoroisopropyl | H | Br |
| 13-1156 | 2.2.2-trichloroethyl | H | Me | H | F | H | H | Br | H | nonafluoro-2-butyl | H | Br |
| 13-1185 | 2.2.2-trichloroethyl | H | Me | H | F | H | H | I | H | heptafluoroisopropyl | H | I |
| 13-1214 | 2.2.2-trichloroethyl | H | Me | H | F | H | H | I | H | nonafluoro-2-butyl | H | I |
| 13-1236 | 2,2,2-trichloroethyl | H | Me | H | F | H | H | Cl | H | heptafluoroisopropyl | H | Br |
| 13-1245 | 2.2.2-trichloroethyl | H | Me | H | F | H | H | F | H | heptafluoroisopropyl | H | OCF3 |
| 13-1246 | 2.2.2-trichloroethyl | H | Me | H | F | H | H | F | H | nonafluoro-2-butyl | H | OCF3 |
| 13-1257 | 2,2,2-trichloroethyl | H | Me | H | F | H | H | Cl | H | heptafluoroisopropyl | H | OCF3 |
| 13-1286 | 2,2,2-trichloroethyl | H | Me | H | F | H | H | Cl | H | nonafluoro-2-butyl | H | OCF3 |
| 13-1315 | 2.2.2-trichloroethyl | H | Me | H | F | H | H | Br | H | heptafluoroisopropyl | H | OCF3 |
| 13-1344 | 2.2.2-trichloroethyl | H | Me | H | F | H | H | Br | H | nonafluoro-2-butyl | H | OCF3 |
| 13-1373 | 2.2.2-trichloroethyl | H | Me | H | F | H | H | I | H | heptafluoroisopropyl | H | OCF3 |
| 13-1402 | 2.2.2-trichloroethyl | H | Me | H | F | H | H | I | H | nonafluoro-2-butyl | H | OCF3 |
| 13-1421 | 2.2.2-trichloroethyl | H | Me | H | F | H | H | F | H | heptafluoroisopropyl | H | CF3 |
| 13-1422 | 2.2.2-trichloroethyl | H | Me | H | F | H | H | F | H | nonafluoro-2-butyl | H | CF3 |
| 13-1433 | 2,2,2-trichloroethyl | H | Me | H | F | H | H | Cl | H | heptafluoroisopropyl | H | CF3 |
| 13-1462 | 2,2,2-trichloroethyl | H | Me | H | F | H | H | Cl | H | nonafluoro-2-butyl | H | CF3 |
| 13-1491 | 2.2.2-trichloroethyl | H | Me | H | F | H | H | Br | H | heptafluoroisopropyl | H | CF3 |
| 13-1520 | 2.2.2-trichloroethyl | H | Me | H | F | H | H | Br | H | nonafluoro-2-butyl | H | CF3 |
| 13-1549 | 2.2.2-trichloroethyl | H | Me | H | F | H | H | I | H | heptafluoroisopropyl | H | CF3 |
| 13-1578 | 2.2.2-trichloroethyl | H | Me | H | F | H | H | I | H | nonafluoro-2-butyl | H | CF3 |
| 13-1607 | 2.2.2-trichloroethyl | H | Me | H | F | H | H | OCF3 | H | heptafluoroisopropyl | H | OCF3 |
| 13-1636 | 2,2,2-trichloroethyl | H | Me | H | F | H | H | OCF3 | H | nonafluoro-2-butyl | H | OCF3 |
| 13-1665 | 2,2,2-trichloroethyl | H | Me | H | F | H | H | CF3 | H | heptafluoroisopropyl | H | OCF3 |
| 13-1694 | 2,2,2-trichloroethyl | H | Me | H | F | H | H | CF3 | H | nonafluoro-2-butyl | H | OCF3 |
| 13-1723 | 2.2.2-trichloroethyl | H | Me | H | F | H | H | CF3 | H | heptafluoroisopropyl | H | CF3 |
| 13-1752 | 2,2,2-trichloroethyl | H | Me | H | F | H | H | CF3 | H | nonafluoro-2-butyl | H | CF3 |
| 13-1771 | 2,2.2-trichloroethyl | H | Me | F | F | H | H | F | H | heptafluoroisopropyl | H | F |
| 13-1772 | 2,2,2-trichloroethyl | H | Me | F | F | H | H | F | H | nonafluoro-2-butyl | H | F |
| 13-1783 | 2,2,2-trichloroethyl | H | Me | F | F | H | H | Cl | H | heptafluoroisopropyl | H | Cl |
| 13-1812 | 2,2,2-trichloroethyl | H | Me | F | F | H | H | Cl | H | nonafluoro-2-butyl | H | Cl |
| 13-1841 | 2.2.2-trichloroethyl | H | Me | F | F | H | H | Br | H | heptafluoroisopropyl | H | Br |
| 13-1870 | 2.2.2-trichloroethyl | H | Me | F | F | H | H | Br | H | nonafluoro-2-butyl | H | Br |
| 13-1899 | 2.2.2-trichloroethyl | H | Me | F | F | H | H | I | H | heptafluoroisopropyl | H | I |
| 13-1928 | 2.2.2-trichloroethyl | H | Me | F | F | H | H | I | H | nonafluoro-2-butyl | H | I |
| 13-1956 | 2,2,2-trichloroethyl | H | Me | F | F | H | H | Cl | H | nonafluoro-2-butyl | H | Br |
| 13-1959 | 2.2.2-trichloroethyl | H | Me | F | F | H | H | F | H | heptafluoroisopropyl | H | OCF3 |
| 13-1960 | 2.2.2-trichloroethyl | H | Me | F | F | H | H | F | H | nonafluoro-2-butyl | H | OCF3 |
| 13-1971 | 2,2,2-trichloroethyl | H | Me | F | F | H | H | Cl | H | heptafluoroisopropyl | H | OCF3 |

**Table 13(3)**

| compound number | Q₂ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 13-2000 | 2,2,2-trichloroethyl | H | Me | F | F | H | H | Cl | H | nonafluoro-2-butyl | H | OCF3 |
| 13-2029 | 2.2.2-trichloroethyl | H | Me | F | F | H | H | Br | H | heptafluoroisopropyl | H | OCF3 |
| 13-2058 | 2.2.2-trichloroethyl | H | Me | F | F | H | H | Br | H | nonafluoro-2-butyl | H | OCF3 |
| 13-2087 | 2.2.2-trichloroethyl | H | Me | F | F | H | H | I | H | heptafluoroisopropyl | H | OCF3 |
| 13-2116 | 2,2,2-trichloroethyl | H | Me | F | F | H | H | I | H | nonafluoro-2-butyl | H | OCF3 |
| 13-2135 | 2.2.2-trichloroethyl | H | Me | F | F | H | H | F | H | heptafluoroisopropyl | H | CF3 |
| 13-2136 | 2.2.2-trichloroethyl | H | Me | F | F | H | H | F | H | nonafluoro-2-butyl | H | CF3 |
| 13-2147 | 2,2,2-trichloroethyl | H | Me | F | F | H | H | Cl | H | heptafluoroisopropyl | H | CF3 |
| 13-2176 | 2,2,2-trichloroethyl | H | Me | F | F | H | H | Cl | H | nonafluoro-2-butyl | H | CF3 |
| 13-2205 | 2.2.2-trichloroethyl | H | Me | F | F | H | H | Br | H | heptafluoroisopropyl | H | CF3 |
| 13-2234 | 2.2.2-trichloroethyl | H | Me | F | F | H | H | Br | H | nonafluoro-2-butyl | H | CF3 |
| 13-2263 | 2.2.2-trichloroethyl | H | Me | F | F | H | H | I | H | heptafluoroisopropyl | H | CF3 |
| 13-2292 | 2,2,2-trichloroethyl | H | Me | F | F | H | H | I | H | nonafluoro-2-butyl | H | CF3 |
| 13-2321 | 2.2.2-trichloroethyl | H | Me | F | F | H | H | OCF3 | H | heptafluoroisopropyl | H | OCF3 |
| 13-2350 | 2.2.2-trichloroethyl | H | Me | F | F | H | H | OCF3 | H | nonafluoro-2-butyl | H | OCF3 |
| 13-2379 | 2.2.2-trichloroethyl | H | Me | F | F | H | H | CF3 | H | heptafluoroisopropyl | H | OCF3 |
| 13-2408 | 2,2,2-trichloroethyl | H | Me | F | F | H | H | CF3 | H | nonafluoro-2-butyl | H | OCF3 |
| 13-2437 | 2.2.2-trichloroethyl | H | Me | F | F | H | H | CF3 | H | heptafluoroisopropyl | H | CF3 |
| 13-2439 | n-propyl | H | S(O)2Me | F | F | H | H | CF3 | H | heptafluoroisopropyl | H | CF3 |
| 13-2466 | 2.2.2-trichloroethyl | H | Me | F | F | H | H | CF3 | H | nonafluoro-2-butyl | H | CF3 |
| 13-2485 | 2,2,2-trichloroethyl | H | Me | F | F | F | H | Cl | H | pentafluoroethyl | H | Cl |
| 13-2486 | 2.2.2-trichloroethyl | H | Me | F | F | F | H | Br | H | heptafluoroisopropyl | H | Br |
| 13-2487 | 2.2.2-trichloroethyl | H | Me | F | F | F | H | Br | H | heptafluoroisopropyl | H | OCF3 |
| 13-2488 | 2.2.2-trichloroethyl | H | Me | F | F | F | H | I | H | heptafluoroisopropyl | H | CF3 |
| 13-2489 | 2.2.2-trichloroethyl | H | Me | F | F | F | H | CF3 | H | heptafluoroisopropyl | H | CF3 |
| 13-2490 | 2.2.2-trichloroethyl | H | Me | F | F | F | H | I | H | nonafluoro-2-butyl | H | I |
| 13-2491 | 2,2,2-trichloroethyl | H | Me | F | F | F | H | Cl | H | nonafluoro-2-butyl | H | OCF3 |
| 13-2492 | 2.2.2-trichloroethyl | H | Me | F | F | F | H | Br | H | nonafluoro-2-butyl | H | CF3 |
| 13-2493 | 2.2.2-trichloroethyl | H | Me | F | F | F | H | CF3 | H | nonafluoro-2-butyl | H | CF3 |
| 13-2494 | 2,2,2-trichloroethyl | H | Me | H | H | F | H | Cl | H | pentafluoroethyl | H | Cl |
| 13-2495 | 2,2,2-trichloroethyl | H | Me | H | H | F | H | Br | H | heptafluoroisopropyl | H | Br |
| 13-2496 | 2.2.2-trichloroethyl | H | Me | H | H | F | H | Br | H | heptafluoroisopropyl | H | OCF3 |
| 13-2497 | 2.2.2-trichloroethyl | H | Me | H | H | F | H | I | H | heptafluoroisopropyl | H | CF3 |
| 13-2498 | 2.2.2-trichloroethyl | H | Me | H | H | F | H | CF3 | H | heptafluoroisopropyl | H | CF3 |
| 13-2499 | 2.2.2-trichloroethyl | H | Me | H | H | F | H | I | H | nonafluoro-2-butyl | H | I |
| 13-2500 | 2,2,2-trichloroethyl | H | Me | H | H | F | H | Cl | H | nonafluoro-2-butyl | H | OCF3 |
| 13-2501 | 2.2.2-trichloroethyl | H | Me | H | H | F | H | Br | H | nonafluoro-2-butyl | H | CF3 |
| 13-2502 | 2.2.2-trichloroethyl | H | Me | H | H | F | H | CF3 | H | nonafluoro-2-butyl | H | CF3 |
| 13-2503 | 2.2.2-trichloroethyl | H | Me | H | H | H | F | Cl | H | pentafluoroethyl | H | Cl |
| 13-2504 | 2.2.2-trichloroethyl | H | Me | H | H | H | F | Br | H | heptafluoroisopropyl | H | Br |
| 13-2505 | 2.2.2-trichloroethyl | H | Me | H | H | H | F | Br | H | heptafluoroisopropyl | H | OCF3 |
| 13-2506 | 2.2.2-trichloroethyl | H | Me | H | H | H | F | I | H | heptafluoroisopropyl | H | CF3 |
| 13-2507 | 2.2.2-trichloroethyl | H | Me | H | H | H | F | CF3 | H | heptafluoroisopropyl | H | CF3 |
| 13-2508 | 2.2.2-trichloroethyl | H | Me | H | H | H | F | I | H | nonafluoro-2-butyl | H | I |
| 13-2509 | 2.2.2-trichloroethyl | H | Me | H | H | H | F | Cl | H | nonafluoro-2-butyl | H | OCF3 |
| 13-2510 | 2.2.2-trichloroethyl | H | Me | H | H | H | F | Br | H | nonafluoro-2-butyl | H | CF3 |
| 13-2511 | 2.2.2-trichloroethyl | H | Me | H | H | H | F | CF3 | H | nonafluoro-2-butyl | H | CF3 |
| 13-2512 | 2,2,2-trichloroethyl | H | Me | F | H | F | H | Cl | H | pentafluoroethyl | H | Cl |
| 13-2513 | 2.2.2-trichloroethyl | H | Me | F | H | F | H | Br | H | heptafluoroisopropyl | H | Br |
| 13-2514 | 2.2.2-trichloroethyl | H | Me | F | H | F | H | Br | H | heptafluoroisopropyl | H | OCF3 |
| 13-2515 | 2.2.2-trichloroethyl | H | Me | F | H | F | H | I | H | heptafluoroisopropyl | H | CF3 |
| 13-2516 | 2.2.2-trichloroethyl | H | Me | F | H | F | H | CF3 | H | heptafluoroisopropyl | H | CF3 |
| 13-2517 | 2.2.2-trichloroethyl | H | Me | F | H | F | H | I | H | nonafluoro-2-butyl | H | I |
| 13-2518 | 2,2,2-trichloroethyl | H | Me | F | H | F | H | Cl | H | nonafluoro-2-butyl | H | OCF3 |
| 13-2519 | 2.2.2-trichloroethyl | H | Me | F | H | F | H | Br | H | nonafluoro-2-butyl | H | CF3 |
| 13-2520 | 2.2.2-trichloroethyl | H | Me | F | H | F | H | CF3 | H | nonafluoro-2-butyl | H | CF3 |
| 13-2521 | 2,2,2-trichloroethyl | H | Me | F | H | H | F | Cl | H | pentafluoroethyl | H | Cl |
| 13-2522 | 2.2.2-trichloroethyl | H | Me | F | H | H | F | Br | H | heptafluoroisopropyl | H | Br |
| 13-2523 | 2.2.2-trichloroethyl | H | Me | F | H | H | F | Br | H | heptafluoroisopropyl | H | OCF3 |
| 13-2524 | 2.2.2-trichloroethyl | H | Me | F | H | H | F | I | H | heptafluoroisopropyl | H | CF3 |
| 13-2525 | 2.2.2-trichloroethyl | H | Me | F | H | H | F | CF3 | H | heptafluoroisopropyl | H | CF3 |
| 13-2526 | 2.2.2-trichloroethyl | H | Me | F | H | H | F | I | H | nonafluoro-2-butyl | H | I |
| 13-2527 | 2,2,2-trichloroethyl | H | Me | F | H | H | F | Cl | H | nonafluoro-2-butyl | H | OCF3 |
| 13-2528 | 2.2.2-trichloroethyl | H | Me | F | H | H | F | Br | H | nonafluoro-2-butyl | H | CF3 |

**Table 13 (4)**

| compound number | Q₂ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 13-2529 | 2,2,2-trichloroethyl | H | Me | F | H | H | F | CF3 | H | nonafluoro-2-butyl | H | CF3 |
| 13-2530 | 2,2,2-trichloroethyl | H | Me | H | F | F | H | Cl | H | pentafluoroethyl | H | Cl |
| 13-2531 | 2.2.2-trichloroethyl | H | Me | H | F | F | H | Br | H | heptafluoroisopropyl | H | Br |
| 13-2532 | 2.2.2-trichloroethyl | H | Me | H | F | F | H | Br | H | heptafluoroisopropyl | H | OCF3 |
| 13-2533 | 2,2,2-trichloroethyl | H | Me | H | F | F | H | I | H | heptafluoroisopropyl | H | CF3 |
| 13-2534 | 2.2.2-trichloroethyl | H | Me | H | F | F | H | CF3 | H | heptafluoroisopropyl | H | CF3 |
| 13-2535 | 2.2.2-trichloroethyl | H | Me | H | F | F | H | I | H | nonafluoro-2-butyl | H | I |
| 13-2536 | 2,2,2-trichloroethyl | H | Me | H | F | F | H | Cl | H | nonafluoro-2-butyl | H | OCF3 |
| 13-2537 | 2,2,2-trichloroethyl | H | Me | H | F | F | H | Br | H | nonafluoro-2-butyl | H | CF3 |
| 13-2538 | 2.2.2-trichloroethyl | H | Me | H | F | F | H | CF3 | H | nonafluoro-2-butyl | H | CF3 |
| 13-2539 | 2,2,2-trichloroethyl | H | Me | H | F | H | F | Cl | H | pentafluoroethyl | H | Cl |
| 13-2540 | 2.2.2-trichloroethyl | H | Me | H | F | H | F | Br | H | heptafluoroisopropyl | H | Br |
| 13-2541 | 2,2,2-trichloroethyl | H | Me | H | F | H | F | Br | H | heptafluoroisopropyl | H | OCF3 |
| 13-2542 | 2.2.2-trichloroethyl | H | Me | H | F | H | F | I | H | heptafluoroisopropyl | H | CF3 |
| 13-2543 | 2.2.2-trichloroethyl | H | Me | H | F | H | F | CF3 | H | heptafluoroisopropyl | H | CF3 |
| 13-2544 | 2.2.2-trichloroethyl | H | Me | H | F | H | F | I | H | nonafluoro-2-butyl | H | I |
| 13-2545 | 2,2,2-trichloroethyl | H | Me | H | F | H | F | Cl | H | nonafluoro-2-butyl | H | OCF3 |
| 13-2546 | 2.2.2-trichloroethyl | H | Me | H | F | H | F | Br | H | nonafluoro-2-butyl | H | CF3 |
| 13-2547 | 2.2.2-trichloroethyl | H | Me | H | F | H | F | CF3 | H | nonafluoro-2-butyl | H | CF3 |
| 13-2548 | 2,2,2-trichloroethyl | H | Me | H | H | F | F | Cl | H | pentafluoroethyl | H | Cl |
| 13-2549 | 2,2,2-trichloroethyl | H | Me | H | H | F | F | Br | H | heptafluoroisopropyl | H | Br |
| 13-2550 | 2.2.2-trichloroethyl | H | Me | H | H | F | F | Br | H | heptafluoroisopropyl | H | OCF3 |
| 13-2551 | 2.2.2-trichloroethyl | H | Me | H | H | F | F | I | H | heptafluoroisopropyl | H | CF3 |
| 13-2552 | 2.2.2-trichloroethyl | H | Me | H | H | F | F | CF3 | H | heptafluoroisopropyl | H | CF3 |
| 13-2553 | 2.2.2-trichloroethyl | H | Me | H | H | F | F | I | H | nonafluoro-2-butyl | H | I |
| 13-2554 | 2,2,2-trichloroethyl | H | Me | H | H | F | F | Cl | H | nonafluoro-2-butyl | H | OCF3 |
| 13-2555 | 2.2.2-trichloroethyl | H | Me | H | H | F | F | Br | H | nonafluoro-2-butyl | H | CF3 |
| 13-2556 | 2.2.2-trichloroethyl | H | Me | H | H | F | F | CF3 | H | nonafluoro-2-butyl | H | CF3 |
| 13-2557 | 2,2,2-trichloroethyl | H | Me | F | F | H | F | Cl | H | pentafluoroethyl | H | Cl |
| 13-2558 | 2.2.2-trichloroethyl | H | Me | F | F | H | F | Br | H | heptafluoroisopropyl | H | Br |
| 13-2559 | 2,2,2-trichloroethyl | H | Me | F | F | H | F | Br | H | heptafluoroisopropyl | H | OCF3 |
| 13-2560 | 2.2.2-trichloroethyl | H | Me | F | F | H | F | I | H | heptafluoroisopropyl | H | CF3 |
| 13-2561 | 2.2.2-trichloroethyl | H | Me | F | F | H | F | CF3 | H | heptafluoroisopropyl | H | CF3 |
| 13-2562 | 2.2.2-trichloroethyl | H | Me | F | F | H | F | I | H | nonafluoro-2-butyl | H | I |
| 13-2563 | 2,2,2-trichloroethyl | H | Me | F | F | H | F | Cl | H | nonafluoro-2-butyl | H | OCF3 |
| 13-2564 | 2.2.2-trichloroethyl | H | Me | F | F | H | F | Br | H | nonafluoro-2-butyl | H | CF3 |
| 13-2565 | 2.2.2-trichloroethyl | H | Me | F | F | H | F | CF3 | H | nonafluoro-2-butyl | H | CF3 |
| 13-2566 | 2,2,2-trichloroethyl | H | Me | F | H | F | F | Cl | H | pentafluoroethyl | H | Cl |
| 13-2567 | 2.2.2-trichloroethyl | H | Me | F | H | F | F | Br | H | heptafluoroisopropyl | H | Br |
| 13-2568 | 2.2.2-trichloroethyl | H | Me | F | H | F | F | Br | H | heptafluoroisopropyl | H | OCF3 |
| 13-2569 | 2.2.2-trichloroethyl | H | Me | F | H | F | F | I | H | heptafluoroisopropyl | H | CF3 |
| 13-2570 | 2.2.2-trichloroethyl | H | Me | F | H | F | F | CF3 | H | heptafluoroisopropyl | H | CF3 |
| 13-2571 | 2.2.2-trichloroethyl | H | Me | F | H | F | F | I | H | nonafluoro-2-butyl | H | I |
| 13-2572 | 2,2,2-trichloroethyl | H | Me | F | H | F | F | Cl | H | nonafluoro-2-butyl | H | OCF3 |
| 13-2573 | 2.2.2-trichloroethyl | H | Me | F | H | F | F | Br | H | nonafluoro-2-butyl | H | CF3 |
| 13-2574 | 2.2.2-trichloroethyl | H | Me | F | H | F | F | CF3 | H | nonafluoro-2-butyl | H | CF3 |
| 13-2575 | 2,2,2-trichloroethyl | H | Me | H | F | F | F | Cl | H | pentafluoroethyl | H | Cl |
| 13-2576 | 2.2.2-trichloroethyl | H | Me | H | F | F | F | Br | H | heptafluoroisopropyl | H | Br |
| 13-2577 | 2.2.2-trichloroethyl | H | Me | H | F | F | F | Br | H | heptafluoroisopropyl | H | OCF3 |
| 13-2578 | 2.2.2-trichloroethyl | H | Me | H | F | F | F | I | H | heptafluoroisopropyl | H | CF3 |
| 13-2579 | 2.2.2-trichloroethyl | H | Me | H | F | F | F | CF3 | H | heptafluoroisopropyl | H | CF3 |
| 13-2580 | 2.2.2-trichloroethyl | H | Me | H | F | F | F | I | H | nonafluoro-2-butyl | H | I |
| 13-2581 | 2,2,2-trichloroethyl | H | Me | H | F | F | F | Cl | H | nonafluoro-2-butyl | H | OCF3 |
| 13-2582 | 2.2.2-trichloroethyl | H | Me | H | F | F | F | Br | H | nonafluoro-2-butyl | H | CF3 |
| 13-2583 | 2.2.2-trichloroethyl | H | Me | H | F | F | F | CF3 | H | nonafluoro-2-butyl | H | CF3 |
| 13-2584 | 2,2,2-trichloroethyl | H | Me | F | F | F | F | Cl | H | pentafluoroethyl | H | Cl |
| 13-2585 | 222-trichloroethyl | H | Me | F | F | F | F | Br | H | heptafluoroisopropyl | H | Br |
| 13-2586 | 2.2.2-trichloroethyl | H | Me | F | F | F | F | Br | H | heptafluoroisopropyl | H | OCF3 |
| 13-2587 | 2.2.2-trichloroethyl | H | Me | F | F | F | F | I | H | heptafluoroisopropyl | H | CF3 |
| 13-2588 | 2.2.2-trichloroethyl | H | Me | F | F | F | F | CF3 | H | heptafluoroisopropyl | H | CF3 |
| 13-2589 | 2.2.2-trichloroethyl | H | Me | F | F | F | F | I | H | nonafluoro-2-butyl | H | I |
| 13-2590 | 2,2,2-trichloroethyl | H | Me | F | F | F | F | Cl | H | nonafluoro-2-butyl | H | OCF3 |
| 13-2591 | 2.2.2-trichloroethyl | H | Me | F | F | F | F | Br | H | nonafluoro-2-butyl | H | CF3 |
| 13-2592 | 2.2.2-trichloroethyl | H | Me | F | F | F | F | CF3 | H | nonafluoro-2-butyl | H | CF3 |

**Table 13(5)**

| compound number | Q₂ | R₁ | R₂ | X₁ | X₁ | X₃ | X₄ | Y₁ | Y₂ | Y₃ | Y₄ | Y₅ |
|---|---|---|---|---|---|---|---|---|---|---|---|---|
| 13-2593 | 2,2,2-trichloroethyl | H | Me | H | CN | H | H | F | H | heptafluoroisopropyl | H | OCF3 |
| 13-2594 | 2.2.2-trichloroethyl | H | Me | H | CN | H | H | F | H | nonafluoro-2-butyl | H | OCF3 |
| 13-2605 | 2.2.2-trichloroethyl | H | Me | H | CN | H | H | Cl | H | heptafluoroisopropyl | H | OCF3 |
| 13-2634 | 2.2.2-trichloroethyl | H | Me | H | CN | H | H | Cl | H | nonafluoro-2-butyl | H | OCF3 |
| 13-2663 | 2,2,2-trichloroethvl | H | Me | H | CN | H | H | Br | H | heDtafluoroisoDroDyl | H | OCF3 |
| 13-2692 | 2.2.2-trichloroethyl | H | Me | H | CN | H | H | Br | H | nonafluoro-2-butyl | H | OCF3 |
| 13-2721 | 2.2.2-trichloroethyl | H | Me | H | CN | H | H | I | H | heptafluoroisopropyl | H | OCF3 |
| 13-2750 | 2.2.2-trichloroethyl | H | Me | H | CN | H | H | I | H | nonafluoro-2-butyl | H | OCF3 |
| 13-2769 | 2,2,2-trichloroethyl | H | Me | H | CN | H | H | F | H | heptafluoroisopropyl | H | CF3 |
| 13-2770 | 2.2.2-trichloroethyl | H | Me | H | CN | H | H | F | H | nonafluoro-2-butyl | H | CF3 |
| 13-2781 | 2.2.2-trichloroethyl | H | Me | H | CN | H | H | Cl | H | heptafluoroisopropyl | H | CF3 |
| 13-2810 | 2.2.2-trichloroethyl | H | Me | H | CN | H | H | Cl | H | nonafluoro-2-butyl | H | CF3 |
| 13-2839 | 2,2,2-trichloroethyl | H | Me | H | CN | H | H | Br | H | heptafluoroisopropyl | H | CF3 |
| 13-2868 | 2.2.2-trichloroethyl | H | Me | H | CN | H | H | Br | H | nonafluoro-2-butyl | H | CF3 |
| 13-2897 | 2.2.2-trichloroethyl | H | Me | H | CN | H | H | I | H | heptafluoroisopropyl | H | CF3 |
| 13-2926 | 2.2.2-trichloroethyl | H | Me | H | CN | H | H | I | H | nonafluoro-2-butyl | H | CF3 |
| 13-2955 | 2,2,2-trichloroethyl | H | Me | H | CN | H | H | OCF3 | H | heptafluoroisopropyl | H | OCF3 |
| 13-2984 | 2.2.2-trichloroethyl | H | Me | H | CN | H | H | OCF3 | H | nonafluoro-2-butyl | H | OCF3 |
| 13-3013 | 2.2.2-trichloroethyl | H | Me | H | CN | H | H | CF3 | H | heptafluoroisopropyl | H | OCF3 |
| 13-3042 | 2.2.2-trichloroethyl | H | Me | H | CN | H | H | CF3 | H | nonafluoro-2-butyl | H | OCF3 |
| 13-3071 | 2,2,2-trichloroethyl | H | Me | H | CN | H | H | CF3 | H | heptafluoroisopropyl | H | CF3 |
| 13-3100 | 2.2.2-trichloroethyl | H | Me | H | CN | H | H | CF3 | H | nonafluoro-2-butyl | H | CF3 |
| 13-3119 | 2.2.2-trichloroethyl | H | Me | F | CN | H | H | F | H | heptafluoroisopropyl | H | F |
| 13-3120 | 2.2.2-trichloroethyl | H | Me | F | CN | H | H | F | H | nonafluoro-2-butyl | H | F |
| 13-3131 | 2.2.2-trichloroethyl | H | Me | F | CN | H | H | Cl | H | heptafluoroisopropyl | H | Cl |
| 13-3160 | 2.2.2-trichloroethyl | H | Me | F | CN | H | H | Cl | H | nonafluoro-2-butyl | H | Cl |
| 13-3189 | 2.2.2-trichloroethyl | H | Me | F | CN | H | H | Br | H | heptafluoroisopropyl | H | Br |
| 13-3218 | 2.2.2-trichloroethyl | H | Me | F | CN | H | H | Br | H | nonafluoro-2-butyl | H | Br |
| 13-3247 | 2.2.2-trichloroethyl | H | Me | F | CN | H | H | I | H | heptafluoroisopropyl | H | I |
| 13-3276 | 2.2.2-trichloroethyl | H | Me | F | CN | H | H | I | H | nonafluoro-2-butyl | H | I |
| 13-3298 | 2.2.2-trichloroethvl | H | Me | F | CN | H | H | Cl | H | heptafluoroisopropyl | H | Br |
| 13-3307 | 2.2.2-trichloroethyl | H | Me | F | CN | H | H | F | H | heptafluoroisopropyl | H | OCF3 |
| 13-3308 | 2.2.2-trichloroethyl | H | Me | F | CN | H | H | F | H | nonafluoro-2-butyl | H | OCF3 |
| 13-3319 | 2.2.2-trichloroethyl | H | Me | F | CN | H | H | Cl | H | heptafluoroisopropyl | H | OCF3 |
| 13-3348 | 2.2.2-trichloroethyl | H | Me | F | CN | H | H | Cl | H | nonafluoro-2-butyl | H | OCF3 |
| 13-3377 | 2.2.2-trichloroethyl | H | Me | F | CN | H | H | Br | H | heptafluoroisopropyl | H | OCF3 |
| 13-3406 | 2.2.2-trichloroethyl | H | Me | F | CN | H | H | Br | H | nonafluoro-2-butyl | H | OCF3 |
| 13-3435 | 2.2.2-trichloroethyl | H | Me | F | CN | H | H | I | H | heptafluoroisopropyl | H | OCF3 |
| 13-3464 | 2.2.2-trichloroethyl | H | Me | F | CN | H | H | I | H | nonafluoro-2-butyl | H | OCF3 |
| 13-3483 | 2.2.2-trichloroethyl | H | Me | F | CN | H | H | F | H | heptafluoroisopropyl | H | CF3 |
| 13-3484 | 2.2.2-trichloroethyl | H | Me | F | CN | H | H | F | H | nonafluoro-2-butyl | H | CF3 |
| 13-3495 | 2.2.2-trichloroethyl | H | Me | F | CN | H | H | Cl | H | heptafluoroisopropyl | H | CF3 |
| 13-3524 | 2.2.2-trichloroethyl | H | Me | F | CN | H | H | Cl | H | nonafluoro-2-butyl | H | CF3 |
| 13-3553 | 2.2.2-trichloroethyl | H | Me | F | CN | H | H | Br | H | heptafluoroisopropyl | H | CF3 |
| 13-3582 | 2.2.2-trichloroethyl | H | Me | F | CN | H | H | Br | H | nonafluoro-2-butyl | H | CF3 |
| 13-3611 | 2.2.2-trichloroethyl | H | Me | F | CN | H | H | I | H | heptafluoroisopropyl | H | CF3 |
| 13-3640 | 2.2.2-trichloroethyl | H | Me | F | CN | H | H | I | H | nonafluoro-2-butyl | H | CF3 |
| 13-3669 | 2.2.2-trichloroethyl | H | Me | F | CN | H | H | OCF3 | H | heptafluoroisopropyl | H | OCF3 |
| 13-3698 | 2.2.2-trichloroethyl | H | Me | F | CN | H | H | OCF3 | H | nonafluoro-2-butyl | H | OCF3 |
| 13-3727 | 2.2.2-trichloroethyl | H | Me | F | CN | H | H | CF3 | H | heptafluoroisopropyl | H | OCF3 |
| 13-3756 | 2.2.2-trichloroethyl | H | Me | F | CN | H | H | CF3 | H | nonafluoro-2-butyl | H | OCF3 |
| 13-3785 | 2.2.2-trichloroethyl | H | Me | F | CN | H | H | CF3 | H | heptafluoroisopropyl | H | CF3 |
| 13-3814 | 2.2.2-trichloroethyl | H | Me | F | CN | H | H | CF3 | H | nonafluoro-2-butyl | H | CF3 |
| 13-3833 | 2.2.2-trichloroethyl | H | Me | F | CN | F | H | F | H | heptafluoroisopropyl | H | F |
| 13-3834 | 2.2.2-trichloroethyl | H | Me | F | CN | F | H | F | H | nonafluoro-2-butyl | H | F |
| 13-3845 | 2.2.2-trichloroethyl | H | Me | F | CN | F | H | Cl | H | heptafluoroisopropyl | H | Cl |
| 13-3874 | 2.2.2-trichloroethyl | H | Me | F | CN | F | H | Cl | H | nonafluoro-2-butyl | H | Cl |
| 13-3903 | 2.2.2-trichloroethyl | H | Me | F | CN | F | H | Br | H | heptafluoroisopropyl | H | Br |
| 13-3932 | 2.2.2-trichloroethyl | H | Me | F | CN | F | H | Br | H | nonafluoro-2-butyl | H | Br |
| 13-3961 | 2.2.2-trichloroethyl | H | Me | F | CN | F | H | I | H | heptafluoroisopropyl | H | I |
| 13-3990 | 2.2.2-trichloroethyl | H | Me | F | CN | F | H | I | H | nonafluoro-2-butyl | H | I |
| 13-4012 | 2.2.2-trichloroethyl | H | Me | F | CN | F | H | Cl | H | heptafluoroisopropyl | H | Br |
| 13-4021 | 2.2.2-trichloroethyl | H | Me | F | CN | F | H | F | H | heptafluoroisopropyl | H | OCF3 |
| 13-4022 | 2.2.2-trichloroethyl | H | Me | F | CN | F | H | F | H | nonafluoro-2-butyl | H | OCF3 |

**Table 13(6)**

| compound number | Q₂ | R₁ | R₂ | X₁ | X₁ | X₃ | X₄ | Y₁ | Y₂ | Y₃ | Y₄ | Y₅ |
|---|---|---|---|---|---|---|---|---|---|---|---|---|
| 13-4033 | 2,2,2-trichlomethyl | H | Me | F | CN | F | H | Cl | H | heptafluoroisopropyl | H | OCF3 |
| 13-4062 | 2,2,2-trichloroethyl | H | Me | F | CN | F | H | Cl | H | nonafluoro-2-butyl | H | OCF3 |
| 13-4091 | 2,2,2-trichloroethyl | H | Me | F | CN | F | H | Br | H | heptafluoroisopropyl | H | OCF3 |
| 13-4120 | 2,2,2-trichloroethyl | H | Me | F | CN | F | H | Br | H | nonafluoro-2-butyl | H | OCF3 |
| 13-4149 | 2.2.2-trichlomethyl | H | Me | F | CN | F | H | I | H | heptafluoroisopropyl | H | OCF3 |
| 13-4178 | 2,2,2-trichloroethyl | H | Me | F | CN | F | H | I | H | nonafluoro-2-butyl | H | OCF3 |
| 13-4197 | 2,2,2-trichlomethyl | H | Me | F | CN | F | H | F | H | heptafluoroisopropyl | H | CF3 |
| 13-4198 | 2,2,2-trichlomethyl | H | Me | F | CN | F | H | F | H | nonafluoro-2-butyl | H | CF3 |
| 13-4209 | 2,2,2-trichlomethyl | H | Me | F | CN | F | H | Cl | H | heptafluoroisopropyl | H | CF3 |
| 13-4238 | 2,2,2-trichloroethyl | H | Me | F | CN | F | H | Cl | H | nonafluoro-2-butyl | H | CF3 |
| 13-4267 | 2,2,2-trichloroethyl | H | Me | F | CN | F | H | Br | H | heptafluoroisopropyl | H | CF3 |
| 13-4296 | 2,2,2-trichloroethyl | H | Me | F | CN | F | H | Br | H | nonafluoro-2-butyl | H | CF3 |
| 13-4325 | 2,2,2-trichlomethyl | H | Me | F | CN | F | H | I | H | heptafluoroisopropyl | H | CF3 |
| 13-4354 | 2,2,2-trichlomethyl | H | Me | F | CN | F | H | I | H | nonafluoro-2-butyl | H | CF3 |
| 13-4383 | 2,2,2-trichlomethyl | H | Me | F | CN | F | H | OCF3 | H | heptafluoroisopropyl | H | OCF3 |
| 13-4412 | 2,2,2-trichlomethyl | H | Me | F | CN | F | H | OCF3 | H | nonafluoro-2-butyl | H | OCF3 |
| 13-4441 | 2,2,2-trichlomethyl | H | Me | F | CN | F | H | CF3 | H | heptafluoroisopropyl | H | OCF3 |
| 13-4470 | 2,2,2-trichlomethyl | H | Me | F | CN | F | H | CF3 | H | nonafluoro-2-butyl | H | OCF3 |
| 13-4499 | 2,2,2-trichlomethyl | H | Me | F | CN | F | H | CF3 | H | heptafluoroisopropyl | H | CF3 |
| 13-4528 | 2,2,2-trichlomethyl | H | Me | F | CN | F | H | CF3 | H | nonafluoro-2-butyl | H | CF3 |

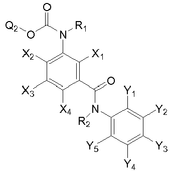

**Table 14(1)**

| compound number | Q₂ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 14-1 | 2,2,2-trichloroethyl | Me | Me | H | H | H | H | F | H | heptafluoroisopropyl | H | OCF3 |
| 14-2 | 2,2,2-trichloroethyl | Me | Me | H | H | H | H | F | H | nonafluoro-2-butyl | H | OCF3 |
| 14-13 | 2,2,2-trichloroethyl | Me | Me | H | H | H | H | Cl | H | heptafluoroisopropyl | H | OCF3 |
| 14-42 | 2,2,2-trichloroethyl | Me | Me | H | H | H | H | Cl | H | nonafluoro-2-butyl | H | OCF3 |
| 14-71 | 2,2,2-trichloroethyl | Me | Me | H | H | H | H | Br | H | heptafluoroisopropyl | H | OCF3 |
| 14-100 | 2,2,2-trichloroethyl | Me | Me | H | H | H | H | Br | H | nonafluoro-2-butyl | H | OCF3 |
| 14-129 | 2,2,2-trichloroethyl | Me | Me | H | H | H | H | I | H | heptafluoroisopropyl | H | OCF3 |
| 14-158 | 2,2,2-trichloroethyl | Me | Me | H | H | H | H | I | H | nonafluoro-2-butyl | H | OCF3 |
| 14-373 | 2,2,2-trichloroethyl | Me | Me | H | H | H | H | F | H | heptafluoroisopropyl | H | CF3 |
| 14-374 | 2,2,2-trichloroethyl | Me | Me | H | H | H | H | F | H | nonafluoro-2-butyl | H | CF3 |
| 14-385 | 2,2,2-trichloroethyl | Me | Me | H | H | H | H | Cl | H | heptafluoroisopropyl | H | CF3 |
| 14-414 | 2,2,2-trichloroethyl | Me | Me | H | H | H | H | Cl | H | nonafluoro-2-butyl | H | CF3 |
| 14-443 | 2,2,2-trichloroethyl | Me | Me | H | H | H | H | Br | H | heptafluoroisopropyl | H | CF3 |
| 14-472 | 2,2,2-trichloroethyl | Me | Me | H | H | H | H | Br | H | nonafluoro-2-butyl | H | CF3 |
| 14-501 | 2,2,2-trichloroethyl | Me | Me | H | H | H | H | I | H | heptafluoroisopropyl | H | CF3 |
| 14-530 | 2,2,2-trichloroethyl | Me | Me | H | H | H | H | I | H | nonafluoro-2-butyl | H | CF3 |
| 14-755 | 2,2,2-trichloroethyl | Me | Me | H | H | H | H | OCF3 | H | heptafluoroisopropyl | H | OCF3 |
| 14-784 | 2,2,2-trichloroethyl | Me | Me | H | H | H | H | OCF3 | H | nonafluoro-2-butyl | H | OCF3 |
| 14-813 | 2,2,2-trichloroethyl | Me | Me | H | H | H | H | CF3 | H | heptafluoroisopropyl | H | OCF3 |
| 14-842 | 2,2,2-trichloroethyl | Me | Me | H | H | H | H | CF3 | H | nonafluoro-2-butyl | H | OCF3 |
| 14-871 | 2,2,2-trichloroethyl | Me | Me | H | H | H | H | CF3 | H | heptafluoroisopropyl | H | CF3 |
| 14-900 | 2,2,2-trichloroethyl | Me | Me | H | H | H | H | CF3 | H | nonafluoro-2-butyl | H | CF3 |
| 14-1115 | 2,2,2-trichloroethyl | Me | Me | F | H | H | H | F | H | heptafluoroisopropyl | H | F |
| 14-1116 | 2,2,2-trichloroethyl | Me | Me | F | H | H | H | Cl | H | heptafluoroisopropyl | H | Cl |
| 14-1117 | 2,2,2-trichloroethyl | Me | Me | F | H | H | H | Br | H | heptafluoroisopropyl | H | Br |
| 14-1118 | 2,2,2-trichloroethyl | Me | Me | F | H | H | H | I | H | heptafluoroisopropyl | H | I |
| 14-1119 | 2,2,2-trichloroethyl | Me | Me | F | H | H | H | F | H | heptafluoroisopropyl | H | OCF3 |
| 14-1120 | 2,2,2-trichloroethyl | Me | Me | F | H | H | H | F | H | nonafluoro-2-butyl | H | OCF3 |
| 14-1131 | 2,2,2-trichloroethyl | Me | Me | F | H | H | H | Cl | H | heptafluoroisopropyl | H | OCF3 |
| 14-1160 | 2,2,2-trichloroethyl | Me | Me | F | H | H | H | Cl | H | nonafluoro-2-butyl | H | OCF3 |
| 14-1189 | 2,2,2-trichloroethyl | Me | Me | F | H | H | H | Br | H | heptafluoroisopropyl | H | OCF3 |
| 14-1218 | 2,2,2-trichloroethyl | Me | Me | F | H | H | H | Br | H | nonafluoro-2-butyl | H | OCF3 |
| 14-1247 | 2,2,2-trichloroethyl | Me | Me | F | H | H | H | I | H | heptafluoroisopropyl | H | OCF3 |
| 14-1276 | 2,2,2-trichloroethyl | Me | Me | F | H | H | H | I | H | nonafluoro-2-butyl | H | OCF3 |
| 14-1491 | 2,2,2-trichloroethyl | Me | Me | F | H | H | H | F | H | heptafluoroisopropyl | H | CF3 |
| 14-1492 | 2,2,2-trichloroethyl | Me | Me | F | H | H | H | F | H | nonafluoro-2-butyl | H | CF3 |
| 14-1498 | 2-iodoethyl | Me | Et | F | H | H | H | Cl | H | heptafluoroisopropyl | H | CF3 |
| 14-1499 | 2-cyanoethyl | Et | Me | F | H | H | H | Cl | H | heptafluoroisopropyl | H | C2F5 |
| 14-1503 | 2,2,2-trichloroethyl | Me | Me | F | H | H | H | Cl | H | heptafluoroisopropyl | H | CF3 |
| 14-1505 | n-propyl | Me | n-Pr | F | H | H | H | Cl | H | pentafluoroethyl | H | CF3 |
| 14-1509 | i-propyl | n-Pr | Me | F | H | H | H | Cl | H | heptafluoroisopropyl | H | n-C3F7 |
| 14-1512 | 1,3-dichloro-2-propyl | Me | Me | F | H | H | H | Cl | H | pentafluoroethyl | H | CF3 |
| 14-1513 | 1-chloro-3-fluoro-2-propyl | i-Pr | Me | F | H | H | H | Cl | H | heptafluoroisopropyl | H | CF3 |
| 14-1515 | n-butyl | Me | i-Pr | F | H | H | H | Cl | H | heptafluoroisopropyl | H | CF3 |
| 14-1517 | i-butyl | CH2CH=CH2 | Me | F | H | H | H | Cl | H | heptafluoroisopropyl | H | CF3 |
| 14-1518 | s-butyl | Me | CH2CH=CH2 | F | H | H | H | Cl | H | heptafluoroisopropyl | H | CF3 |
| 14-1519 | vinyl | Me | CN | F | H | H | H | Cl | H | heptafluoroisopropyl | H | CF3 |
| 14-1523 | ethyl | Me | Me | F | H | H | H | Cl | H | nonafluoro-2-butyl | H | i-C3F7 |
| 14-1528 | 2-cyanoethyl | CN | Me | F | H | H | H | Cl | H | nonafluoro-2-butyl | H | CF3 |
| 14-1532 | 2,2,2-trichloroethyl | Me | Me | F | H | H | H | Cl | H | nonafluoro-2-butyl | H | CF3 |
| 14-1533 | 2,2,2-tribromoethyl | Me | CH2C≡CH | F | H | H | H | Cl | H | nonafluoro-2-butyl | H | CF3 |
| 14-1534 | n-propyl | CH2CE≡CH | Me | F | H | H | H | Cl | H | nonafluoro-2-butyl | H | CF3 |
| 14-1544 | n-butyl | Me | NH2 | F | H | H | H | Cl | H | nonafluoro-2-butyl | H | CF3 |
| 14-1546 | i-butyl | NH2 | Me | F | H | H | H | Cl | H | nonafluoro-2-butyl | H | CF3 |
| 14-1557 | 2-cyanoethyl | Me | C(O)OMe | F | H | H | H | Br | H | heptafluoroisopropyl | H | C2F5 |

**Table 14(2)**

| compound number | Q₂ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 14-1561 | 2,2,2-trichloroethyl | Me | Me | F | H | H | H | Br | H | heptafluoroisopropyl | H | CF3 |
| 14-1563 | n-propyl | C(O)OMe | Me | F | H | H | H | Br | H | heptafluoroisopropyl | H | CF3 |
| 14-1568 | 1,2-difluoro-2-propyl | Me | Me | F | H | H | H | Br | H | heDtafluoroisoDroDyl | H | n-C3F7 |
| 14-1574 | 4,4,4-trifluoro-n-butyl | Me | Me | F | H | H | H | Br | H | heDtafluoroisoDroDyl | H | CF3 |
| 14-1575 | i-butyl | Me | C(C)C(O)OMe | F | H | H | H | Br | H | heDtafluoroisoDroDyl | H | CF3 |
| 14-1577 | vinyl | C(O)C(O)OMe | Me | F | H | H | H | Br | H | heptafluoroisopropyl | H | CF3 |
| 14-1586 | 2-cyanoethyl | Me | C(O)OEt | F | H | H | H | Br | H | nonafluoro-2-butyl | H | CF3 |
| 14-1590 | 2,2,2-trichloroethyl | Me | Me | F | H | H | H | Br | H | nonafluoro-2-butyl | H | CF3 |
| 14-1592 | n-propyl | C(O)OEt | Me | F | H | H | H | Br | H | nonafluoro-2-butyl | H | CF3 |
| 14-1595 | 3-bromo-n-propyl | Me | C(O)Me | F | H | H | H | Br | H | nonafluoro-2-butyl | H | CF3 |
| 14-1596 | i-propyl | C(O)Me | Me | F | H | H | H | Br | H | nonafluoro-2-butyl | H | CF3 |
| 14-1603 | 4,4,4-trifluoro-n-butyl | Me | Me | F | H | H | H | Br | H | nonafluoro-2-butyl | H | CF3 |
| 14-1604 | i-butyl | Me | C(O)C(O)OEt | F | H | H | H | Br | H | nonafluoro-2-butyl | H | CF3 |
| 14-1608 | phenyl | C(O)C(O)OEt | Me | F | H | H | H | Br | H | nonafluoro-2-butyl | H | i-C3F7 |
| 14-1619 | 2,2,2-trichloroethyl | Me | Me | F | H | H | H | I | H | heDtafluoroisoDroDyl | H | CF3 |
| 14-1621 | n-propyl | Me | C(O)Et | F | H | H | H | I | H | heDtafluoroisoDroDyl | H | CF3 |
| 14-1624 | 3-bromo-n-propyl | C(O)Et | Me | F | H | H | H | I | H | heDtafluoroisoDroDyl | H | C2F5 |
| 14-1632 | 4,4,4-trifluoro-n-butyl | Me | Me | F | H | H | H | I | H | heptafluoroisopropyl | H | CF3 |
| 14-1633 | i-butyl | Me | S(O)2Me | F | H | H | H | I | H | heptafluoroisopropyl | H | CF3 |
| 14-1636 | benzyl | S(O)2Me | Me | F | H | H | H | I | H | heptafluoroisopropyl | H | CF3 |
| 14-1643 | 2-iodoethyl | Me | S(O)2Et | F | H | H | H | I | H | nonafluoro-2-butyl | H | CF3 |
| 14-1644 | 2-cyanoethyl | S(O)2Et | Me | F | H | H | H | I | H | nonafluoro-2-butyl | H | CF3 |
| 14-1648 | 2,2,2-trichloroethyl | Me | Me | F | H | H | H | I | H | nonafluoro-2-butyl | H | CF3 |
| 14-1649 | 2,2,2-tribromoethyl | Me | CH2Ph | F | H | H | H | I | H | nonafluoro-2-butyl | H | CF3 |
| 14-1650 | n-propyl | CH2Ph | Me | F | H | H | H | I | H | nonafluoro-2-butyl | H | n-C3F7 |
| 14-1654 | i-propyl | CH2(3-Py) | Me | F | H | H | H | I | H | nonafluoro-2-butyl | H | CF3 |
| 14-1660 | n-butyl | Me | CH2(3-Py) | F | H | H | H | I | H | nonafluoro-2-butyl | H | CF3 |
| 14-1661 | 4,4,4-trifluoro-n-butyl | Me | Me | F | H | H | H | I | H | nonafluoro-2-butyl | H | CF3 |
| 14-1663 | s-butyl | CH2(3-Py-N-oxide) | Me | F | H | H | H | I | H | nonafluoro-2-butyl | H | CF3 |
| 14-1664 | vinyl | Me | Me | F | H | H | H | I | H | nonafluoro-2-butyl | H | i-C3F7 |
| 14-1666 | phenyl | Me | CH2(3-Py-N-oxide) | F | H | H | H | I | H | nonafluoro-2-butyl | H | CF3 |
| 14-1667 | 2,2,2-trichloroethyl | Et | Et | F | H | H | H | I | H | nonafluoro-2-butyl | H | CF3 |
| 14-1668 | 2,2,2-trichloroethyl | Et | n-Pr | F | H | H | H | I | H | nonafluoro-2-butyl | H | CF3 |
| 14-1669 | 2,2,2-trichloroethyl | Et | i-Pr | F | H | H | H | I | H | nonafluoro-2-butyl | H | CF3 |
| 14-1670 | 2,2,2-trichloroethyl | Et | CH2CH=CH2 | F | H | H | H | I | H | nonafluoro-2-butyl | H | CF3 |
| 14-1671 | 2,2,2-trichloroethyl | Et | CN | F | H | H | H | I | H | nonafluoro-2-butyl | H | CF3 |
| 14-1672 | 2,2,2-trichloroethyl | Et | CH2C≡CH | F | H | H | H | I | H | nonafluoro-2-butyl | H | CF3 |
| 14-1673 | 2,2,2-trichloroethyl | Et | NH2 | F | H | H | H | I | H | nonafluoro-2-butyl | H | CF3 |
| 14-1674 | 2,2,2-trichloroethyl | Et | C(O)OMe | F | H | H | H | I | H | nonafluoro-2-butyl | H | CF3 |
| 14-1675 | 2,2,2-trichloroethyl | Et | C(O)C(O)OMe | F | H | H | H | I | H | nonafluoro-2-butyl | H | CF3 |
| 14-1676 | 2,2,2-trichloroethyl | Et | C(O)Me | F | H | H | H | I | H | nonafluoro-2-butyl | H | CF3 |
| 14-1677 | 2,2,2-trichloroethyl | Et | S(O)2Me | F | H | H | H | I | H | nonafluoro-2-butyl | H | CF3 |
| 14-1678 | 2,2,2-trichloroethyl | Et | CH2Ph | F | H | H | H | I | H | nonafluoro-2-butyl | H | CF3 |
| 14-1679 | 2,2,2-trichloroethyl | Et | CH2(3-Py) | F | H | H | H | I | H | nonafluoro-2-butyl | H | CF3 |
| 14-1680 | 2,2,2-trichloroethyl | Et | CH2(3-Py-N-oxide) | F | H | H | H | I | H | nonafluoro-2-butyl | H | CF3 |
| 14-1681 | 2,2,2-trichloroethyl | n-Pr | Et | F | H | H | H | I | H | nonafluoro-2-butyl | H | CF3 |
| 14-1682 | 2,2,2-trichloroethyl | n-Pr | n-Pr | F | H | H | H | I | H | nonafluoro-2-butyl | H | CF3 |
| 14-1683 | 2,2,2-trichloroethyl | n-Pr | i-Pr | F | H | H | H | I | H | nonafluoro-2-butyl | H | CF3 |
| 14-1684 | 2,2,2-trichloroethyl | n-Pr | CH2CH=CH2 | F | H | H | H | I | H | nonafluoro-2-butyl | H | CF3 |
| 14-1685 | 2,2,2-trichloroethyl | n-Pr | CN | F | H | H | H | I | H | nonafluoro-2-butyl | H | CF3 |
| 14-1686 | 2,2,2-trichloroethyl | n-Pr | CH2C≡CH | F | H | H | H | I | H | nonafluoro-2-butyl | H | CF3 |
| 14-1687 | 2,2,2-trichloroethyl | n-Pr | NH2 | F | H | H | H | I | H | nonafluoro-2-butyl | H | CF3 |
| 14-1688 | 2,2,2-trichloroethyl | n-Pr | C(O)OMe | F | H | H | H | I | H | nonafluoro-2-butyl | H | CF3 |
| 14-1689 | 2,2,2-trichloroethyl | n-Pr | C(O)C(O)OMe | F | H | H | H | I | H | nonafluoro-2-butyl | H | CF3 |
| 14-1690 | 2,2,2-trichloroethyl | n-Pr | C(O)Me | F | H | H | H | I | H | nonafluoro-2-butyl | H | CF3 |
| 14-1691 | 2,2,2-trichloroethyl | n-Pr | S(O)2Me | F | H | H | H | I | H | nonafluoro-2-butyl | H | CF3 |
| 14-1692 | 2,2,2-trichloroethyl | n-Pr | CH2Ph | F | H | H | H | I | H | nonafluoro-2-butyl | H | CF3 |
| 14-1693 | 2,2,2-trichloroethyl | n-Pr | CH2(3-Py) | F | H | H | H | I | H | nonafluoro-2-butyl | H | CF3 |
| 14-1694 | 2,2,2-trichloroethyl | n-Pr | CH2(3-Py-N-oxide) | F | H | H | H | I | H | nonafluoro-2-butyl | H | CF3 |
| 14-1695 | 2,2,2-trichloroethyl | i-Pr | Et | F | H | H | H | I | H | nonafluoro-2-butyl | H | CF3 |
| 14-1696 | 2,2,2-trichloroethyl | i-Pr | n-Pr | F | H | H | H | I | H | nonafluoro-2-butyl | H | CF3 |
| 14-1697 | 2,2,2-trichloroethyl | i-Pr | i-Pr | F | H | H | H | I | H | nonafluoro-2-butyl | H | CF3 |
| 14-1698 | 2,2,2-trichloroethyl | i-Pr | CH2CH=CH2 | F | H | H | H | I | H | nonafluoro-2-butyl | H | CF3 |
| 14-1699 | 2,2,2-trichloroethyl | i-Pr | CN | F | H | H | H | I | H | nonafluoro-2-butyl | H | CF3 |

**Table 14(3)**

| compound number | Q₂ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 14-1700 | 2,2,2-trichloroethyl | i-Pr | CH2CE≡CH | F | H | H | H | I | H | nonafluoro-2-butyl | H | CF3 |
| 14-1701 | 2,2,2-trichloroethyl | i-Pr | NH2 | F | H | H | H | I | H | nonafluoro-2-butyl | H | CF3 |
| 14-1702 | 2,2,2-trichloroethyl | i-Pr | C(O)OMe | F | H | H | H | I | H | nonafluoro-2-butyl | H | CF3 |
| 14-1703 | 2,2,2-trichloroethyl | i-Pr | C(O)C(O)OMe | F | H | H | H | I | H | nonafluoro-2-butyl | H | CF3 |
| 14-1704 | 2,2,2-trichloroethyl | i-Pr | C(O)Me | F | H | H | H | I | H | nonafluoro-2-butyl | H | CF3 |
| 14-1705 | 2,2,2-trichloroethyl | i-Pr | S(O)2Me | F | H | H | H | I | H | nonafluoro-2-butyl | H | CF3 |
| 14-1706 | 2,2,2-trichloroethyl | i-Pr | CH2Ph | F | H | H | H | I | H | nonafluoro-2-butyl | H | CF3 |
| 14-1707 | 2,2,2-trichloroethyl | i-Pr | CH2(3-Py) | F | H | H | H | I | H | nonafluoro-2-butyl | H | CF3 |
| 14-1708 | 2,2,2 trichloroethyl | i-Pr | CH2(3-Py-N-oxide) | F | H | H | H | I | H | nonafluoro-2-butyl | H | CF3 |
| 14-1709 | 2,2,2-trichloroethyl | CH2CH=CH2 | Et | F | H | H | H | I | H | nonafluoro-2-butyl | H | CF3 |
| 14-1710 | 2,2,2-trichloroethyl | CH2CH=CH2 | n-Pr | F | H | H | H | I | H | nonafluoro-2-butyl | H | CF3 |
| 14-1711 | 2,2,2-trichloroethyl | CH2CH=CH2 | i-Pr | F | H | H | H | I | H | nonafluoro-2-butyl | H | CF3 |
| 14-1712 | 2,2,2-trichloroethyl | CH2CH=CH2 | CH2CH=CH2 | F | H | H | H | I | H | nonafluoro-2-butyl | H | CF3 |
| 14-1713 | 2,2,2-trichloroethyl | CH2CH=CH2 | CN | F | H | H | H | I | H | nonafluoro-2-butyl | H | CF3 |
| 14-1714 | 2,2,2-trichloroethyl | CH2CH=CH2 | CH2C≡CH | F | H | H | H | I | H | nonafluoro-2-butyl | H | CF3 |
| 14-1715 | 2,2,2-trichloroethyl | CH2CH=CH2 | NH2 | F | H | H | H | I | H | nonafluoro-2-butyl | H | CF3 |
| 14-1716 | 2,2,2-trichloroethyl | CH2CH=CH2 | C(O)OMe | F | H | H | H | I | H | nonafluoro-2-butyl | H | CF3 |
| 14-1717 | 2,2,2-trichloroethyl | CH2CH=CH2 | C(C)C(O)OMe | F | H | H | H | I | H | nonafluoro-2-butyl | H | CF3 |
| 14-1718 | 2,2,2-trichloroethyl | CH2CH=CH2 | C(O)Me | F | H | H | H | I | H | nonafluoro-2-butyl | H | CF3 |
| 14-1719 | 2,2,2-trichloroethyl | CH2CH=CH2 | S(O)2Me | F | H | H | H | I | H | nonafluoro-2-butyl | H | CF3 |
| 14-1720 | 2,2,2-trichloroethyl | CH2CH=CH2 | CH2Ph | F | H | H | H | I | H | nonafluoro-2-butyl | H | CF3 |
| 14-1721 | 2,2,2-trichloroethyl | CH2CH=CH2 | CH2(3-Py) | F | H | H | H | I | H | nonafluoro-2-butyl | H | CF3 |
| 14-1722 | 2,2,2-trichloroethyl | CH2CH=CH2 | CH2(3-Py-N-oxide) | F | H | H | H | I | H | nonafluoro-2-butyl | H | CF3 |
| 14-1723 | 2,2,2-trichloroethyl | CN | Et | F | H | H | H | I | H | nonafluoro-2-butyl | H | CF3 |
| 14-1724 | 2,2,2-trichloroethyl | CN | n-Pr | F | H | H | H | I | H | nonafluoro-2-butyl | H | CF3 |
| 14-1725 | 2,2,2-trichloroethyl | CN | i-Pr | F | H | H | H | I | H | nonafluoro-2-butyl | H | CF3 |
| 14-1726 | 2,2,2-trichloroethyl | CN | CH2CH=CH2 | F | H | H | H | I | H | nonafluoro-2-butyl | H | CF3 |
| 14-1727 | 2,2,2-trichloroethyl | CN | CN | F | H | H | H | I | H | nonafluoro-2-butyl | H | CF3 |
| 14-1728 | 2,2,2-trichloroethyl | CN | CH2C≡CH | F | H | H | H | I | H | nonafluoro-2-butyl | H | CF3 |
| 14-1729 | 2,2,2-trichloroethyl | CN | NH2 | F | H | H | H | I | H | nonafluoro-2-butyl | H | CF3 |
| 14-1730 | 2,2,2-trichloroethyl | CN | C(O)OMe | F | H | H | H | I | H | nonafluoro-2-butyl | H | CF3 |
| 14-1731 | 2,2,2-trichloroethyl | CN | C(O)C(O)OMe | F | H | H | H | I | H | nonafluoro-2-butyl | H | CF3 |
| 14-1732 | 2,2,2-trichloroethyl | CN | C(O)Me | F | H | H | H | I | H | nonafluoro-2-butyl | H | CF3 |
| 14-1733 | 2,2,2-trichloroethyl | CN | S(O)2Me | F | H | H | H | I | H | nonafluoro-2-butyl | H | CF3 |
| 14-1734 | 2,2,2-trichloroethyl | CN | CH2Ph | F | H | H | H | I | H | nonafluoro-2-butyl | H | CF3 |
| 14-1735 | 2,2,2-trichloroethyl | CN | CH2(3-Py) | F | H | H | H | I | H | nonafluoro-2-butyl | H | CF3 |
| 14-1736 | 2,2,2-trichloroethyl | CN | CH2(3-Py-N-oxide) | F | H | H | H | I | H | nonafluoro-2-butyl | H | CF3 |
| 14-1737 | 2,2,2-trichloroethyl | CH2CE≡CH | Et | F | H | H | H | I | H | nonafluoro-2-butyl | H | CF3 |
| 14-1738 | 2,2,2-trichloroethyl | CH2CE≡CH | n-Pr | F | H | H | H | I | H | nonafluoro-2-butyl | H | CF3 |
| 14-1739 | 2,2,2-trichloroethyl | CH2CE≡CH | i-Pr | F | H | H | H | I | H | nonafluoro-2-butyl | H | CF3 |
| 14-1740 | 2,2,2-trichloroethyl | CH2CE≡CH | CH2CH=CH2 | F | H | H | H | I | H | nonafluoro-2-butyl | H | CF3 |
| 14-1741 | 2,2,2-trichloroethyl | CH2CE≡CH | CN | F | H | H | H | I | H | nonafluoro-2-butyl | H | CF3 |
| 14-1742 | 2,2,2-trichloroethyl | CH2CE≡CH | CH2C≡CH | F | H | H | H | I | H | nonafluoro-2-butyl | H | CF3 |
| 14-1743 | 2,2,2-trichloroethyl | CH2CE≡CH | NH2 | F | H | H | H | I | H | nonafluoro-2-butyl | H | CF3 |
| 14-1744 | 2,2,2-trichloroethyl | CH2CE≡CH | C(O)OMe | F | H | H | H | I | H | nonafluoro-2-butyl | H | CF3 |
| 14-1745 | 2,2,2-trichloroethyl | CH2CE≡CH | C(O)C(O)OMe | F | H | H | H | I | H | nonafluoro-2-butyl | H | CF3 |
| 14-1746 | 2,2,2-trichloroethyl | CH2CE≡CH | C(O)Me | F | H | H | H | I | H | nonafluoro-2-butyl | H | CF3 |
| 14-1747 | 2,2,2-trichloroethyl | CH2CE≡CH | S(O)2Me | F | H | H | H | I | H | nonafluoro-2-butyl | H | CF3 |
| 14-1748 | 2,2,2-trichloroethyl | CH2CE≡CH | CH2Ph | F | H | H | H | I | H | nonafluoro-2-butyl | H | CF3 |
| 14-1749 | 2,2,2-trichloroethyl | CH2CE≡CH | CH2(3-Py) | F | H | H | H | I | H | nonafluoro-2-butyl | H | CF3 |
| 14-1750 | 2,2,2-trichloroethyl | CH2CE≡CH | CH2(3-Py-N-oxide) | F | H | H | H | I | H | nonafluoro-2-butyl | H | CF3 |
| 14-1751 | 2,2,2-trichloroethyl | NH2 | Et | F | H | H | H | I | H | nonafluoro-2-butyl | H | CF3 |
| 14-1752 | 2,2,2-trichloroethyl | NH2 | n-Pr | F | H | H | H | I | H | nonafluoro-2-butyl | H | CF3 |
| 14-1753 | 2,2,2-trichloroethyl | NH2 | i-Pr | F | H | H | H | I | H | nonafluoro-2-butyl | H | CF3 |
| 14-1754 | 2,2,2-trichloroethyl | NH2 | CH2CH=CH2 | F | H | H | H | I | H | nonafluoro-2-butyl | H | CF3 |
| 14-1755 | 2,2,2-trichloroethyl | NH2 | CN | F | H | H | H | I | H | nonafluoro-2-butyl | H | CF3 |
| 14-1756 | 2,2,2-trichloroethyl | NH2 | CH2C≡CH | F | H | H | H | I | H | nonafluoro-2-butyl | H | CF3 |
| 14-1757 | 2,2,2-trichioroethyl | NH2 | NH2 | F | H | H | H | I | H | nonafluoro-2-butvl | H | CF3 |
| 14-1758 | 2,2,2-trichloroethyl | NH2 | C(O)OMe | F | H | H | H | I | H | nonafluoro-2-butyl | H | CF3 |
| 14-1759 | 2,2,2-trichloroethyl | NH2 | C(O)C(O)OMe | F | H | H | H | I | H | nonafluoro-2-butyl | H | CF3 |
| 14-1760 | 2,2,2-trichloroethyl | NH2 | C(O)Me | F | H | H | H | I | H | nonafluoro-2-butyl | H | CF3 |
| 14-1761 | 2,2,2-trichloroethyl | NH2 | S(O)2Me | F | H | H | H | I | H | nonafluoro-2-butyl | H | CF3 |
| 14-1762 | 2,2,2-trichloroethyl | NH2 | CH2Ph | F | H | H | H | I | H | nonafluoro-2-butyl | H | CF3 |
| 14-1763 | 2,2,2-trichloroethyl | NH2 | CH2(3-Py) | F | H | H | H | I | H | nonafluoro-2-butyl | H | CF3 |

**Table 14(4)**

| compound number | Q₂ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 14-1764 | 2,2,2 trichloroethyl | NH2 | CH2(3-Py-N-oxide) | F | H | H | H | I | H | nonafluoro-2-butyl | H | CF3 |
| 14-1765 | 2,2,2-trichloroethyl | C(O)OMe | Et | F | H | H | H | I | H | nonafluoro-2-butyl | H | CF3 |
| 14-1766 | 2,2,2-trichloroethyl | C(O)OMe | n-Pr | F | H | H | H | I | H | nonafluoro-2-butyl | H | CF3 |
| 14-1767 | 2,2,2-trichloroethyl | C(O)OMe | i-Pr | F | H | H | H | I | H | nonafluoro-2-butyl | H | CF3 |
| 14-1768 | 2,2,2-trichloroethyl | C(O)OMe | CH2CH=CH2 | F | H | H | H | I | H | nonafluoro-2-butyl | H | CF3 |
| 14-1769 | 2,2,2-trichloroethyl | C(O)OMe | CN | F | H | H | H | I | H | nonafluoro-2-butyl | H | CF3 |
| 14-1770 | 2,2,2-trichloroethyl | C(O)OMe | CH2CE≡CH | F | H | H | H | I | H | nonafluoro-2-butyl | H | CF3 |
| 14-1771 | 2,2,2-trichloroethyl | C(O)OMe | NH2 | F | H | H | H | I | H | nonafluoro-2-butyl | H | CF3 |
| 14-1772 | 2,2,2-trichloroethyl | C(O)OMe | C(O)OMe | F | H | H | H | I | H | nonafluoro-2-butyl | H | CF3 |
| 14-1773 | 2,2,2-trichloroethyl | C(O)OMe | C(O)C(O)OMe | F | H | H | H | I | H | nonafluoro-2-butyl | H | CF3 |
| 14-1774 | 2,2,2-trichloroethyl | C(O)OMe | C(O)Me | F | H | H | H | I | H | nonafluoro-2-butyl | H | CF3 |
| 14-1775 | 2,2,2-trichloroethyl | C(O)OMe | S(O)2Me | F | H | H | H | I | H | nonafluoro-2-butyl | H | CF3 |
| 14-1776 | 2,2,2-trichloroethyl | C(O)OMe | CH2Ph | F | H | H | H | I | H | nonafluoro-2-butyl | H | CF3 |
| 14-1777 | 2,2,2-trichloroethyl | C(O)OMe | CH2(3-Py) | F | H | H | H | I | H | nonafluoro-2-butyl | H | CF3 |
| 14-1778 | 2,2,2-trichloroethyl | C(O)OMe | CH2(3-Py-N-oxide) | F | H | H | H | I | H | nonafluoro-2-butyl | H | CF3 |
| 14-1779 | 2,2,2-trichloroethyl | C(O)C(O)OMe | Et | F | H | H | H | I | H | nonafluoro-2-butyl | H | CF3 |
| 14-1780 | 2,2,2-trichloroethyl | C(O)C(O)OMe | n-Pr | F | H | H | H | I | H | nonafluoro-2-butyl | H | CF3 |
| 14-1781 | 2,2,2-trichloroethyl | C(O)C(O)OMe | i-Pr | F | H | H | H | I | H | nonafluoro-2-butyl | H | CF3 |
| 14-1782 | 2,2,2-trichloroethyl | C(O)C(O)OMe | CH2CH=CH2 | F | H | H | H | I | H | nonafluoro-2-butyl | H | CF3 |
| 14-1783 | 2,2,2-trichloroethyl | C(O)C(O)OMe | CN | F | H | H | H | I | H | nonafluoro-2-butyl | H | CF3 |
| 14-1784 | 2,2,2-trichloroethyl | C(O)C(O)OMe | CH2CE≡CH | F | H | H | H | I | H | nonafluoro-2-butyl | H | CF3 |
| 14-1785 | 2,2,2-trichloroethyl | C(O)C(O)OMe | NH2 | F | H | H | H | I | H | nonafluoro-2-butyl | H | CF3 |
| 14-1786 | 2,2,2-trichloroethyl | C(O)C(O)OMe | C(O)OMe | F | H | H | H | I | H | nonafluoro-2-butyl | H | CF3 |
| 14-1787 | 2,2,2-trichloroethyl | C(O)C(O)OMe | C(O)C(O)OMe | F | H | H | H | I | H | nonafluoro-2-butyl | H | CF3 |
| 14-1788 | 2,2,2-trichloroethyl | C(O)C(O)OMe | C(O)Me | F | H | H | H | I | H | nonafluoro-2-butyl | H | CF3 |
| 14-1789 | 2,2,2-trichloroethyl | C(O)C(O)OMe | S(O)2Me | F | H | H | H | I | H | nonafluoro-2-butyl | H | CF3 |
| 14-1790 | 2,2,2-trichloroethyl | C(O)C(O)OMe | CH2Ph | F | H | H | H | I | H | nonafluoro-2-butyl | H | CF3 |
| 14-1791 | 2,2,2-trichloroethyl | C(O)C(O)OMe | CH2(3-Py) | F | H | H | H | I | H | nonafluoro-2-butyl | H | CF3 |
| 14-1792 | 2,2,2-trichloroethyl | C(O)C(O)OMe | CH2(3-Py-N-oxide) | F | H | H | H | I | H | nonafluoro-2-butyl | H | CF3 |
| 14-1793 | 2,2,2-trichloroethyl | C(O)Me | Et | F | H | H | H | I | H | nonafluoro-2-butyl | H | CF3 |
| 14-1794 | 2,2,2-trichloroethyl | C(O)Me | n-Pr | F | H | H | H | I | H | nonafluoro-2-butyl | H | CF3 |
| 14-1795 | 2,2,2-trichloroethyl | C(O)Me | i-Pr | F | H | H | H | I | H | nonafluoro-2-butyl | H | CF3 |
| 14-1796 | 2,2,2-trichloroethyl | C(O)Me | CH2CH=CH2 | F | H | H | H | I | H | nonafluoro-2-butyl | H | CF3 |
| 14-1797 | 2,2,2-trichloroethyl | C(O)Me | CN | F | H | H | H | I | H | nonafluoro-2-butyl | H | CF3 |
| 14-1798 | 2,2,2-trichloroethyl | C(O)Me | CH2C≡CH | F | H | H | H | I | H | nonafluoro-2-butyl | H | CF3 |
| 14-1799 | 2,2,2-trichloroethyl | C(O)Me | NH2 | F | H | H | H | I | H | nonafluoro-2-butyl | H | CF3 |
| 14-1800 | 2,2,2-trichloroethyl | C(O)Me | C(O)OMe | F | H | H | H | I | H | nonafluoro-2-butyl | H | CF3 |
| 14-1801 | 2,2,2-trichloroethyl | C(O)Me | C(O)C(O)OMe | F | H | H | H | I | H | nonafluoro-2-butyl | H | CF3 |
| 14-1802 | 2,2,2-trichloroethyl | C(O)Me | C(O)Me | F | H | H | H | I | H | nonafluoro-2-butyl | H | CF3 |
| 14-1803 | 2,2,2-trichloroethyl | C(O)Me | S(O)2Me | F | H | H | H | I | H | nonafluoro-2-butyl | H | CF3 |
| 14-1804 | 2,2,2-trichloroethyl | C(O)Me | CH2Ph | F | H | H | H | I | H | nonafluoro-2-butyl | H | CF3 |
| 14-1805 | 2,2,2-trichloroethyl | C(O)Me | CH2(3-Py) | F | H | H | H | I | H | nonafluoro-2-butyl | H | CF3 |
| 14-1806 | 2,2,2-trichloroethyl | C(O)Me | CH2(3-Py-N-oxide) | F | H | H | H | I | H | nonafluoro-2-butyl | H | CF3 |
| 14-1807 | 2,2,2-trichloroethyl | S(O)2Me | Et | F | H | H | H | I | H | nonafluoro-2-butyl | H | CF3 |
| 14-1808 | 2,2,2-trichloroethyl | S(O)2Me | n-Pr | F | H | H | H | I | H | nonafluoro-2-butyl | H | CF3 |
| 14-1809 | 2,2,2-trichloroethyl | S(O)2Me | i-Pr | F | H | H | H | I | H | nonafluoro-2-butyl | H | CF3 |
| 14-1810 | 2,2,2-trichloroethyl | S(O)2Me | CH2CH=CH2 | F | H | H | H | I | H | nonafluoro-2-butyl | H | CF3 |
| 14-1811 | 2,2,2-trichloroethyl | S(O)2Me | CN | F | H | H | H | I | H | nonafluoro-2-butyl | H | CF3 |
| 14-1812 | 2,2,2-trichloroethyl | S(O)2Me | CH2C≡CH | F | H | H | H | I | H | nonafluoro-2-butyl | H | CF3 |
| 14-1813 | 2,2,2-trichloroethyl | S(O)2Me | NH2 | F | H | H | H | I | H | nonafluoro-2-butyl | H | CF3 |
| 14-1814 | 2,2,2-trichloroethyl | S(O)2Me | C(O)OMe | F | H | H | H | I | H | nonafluoro-2-butyl | H | CF3 |
| 14-1815 | 2,2,2-trichloroethyl | S(O)2Me | C(O)C(O)OMe | F | H | H | H | I | H | nonafluoro-2-butyl | H | CF3 |
| 14-1816 | 2,2,2-trichloroethyl | S(O)2Me | C(O)Me | F | H | H | H | I | H | nonafluoro-2-butyl | H | CF3 |
| 14-1817 | 2,2,2-trichloroethyl | S(O)2Me | S(O)2Me | F | H | H | H | I | H | nonafluoro-2-butyl | H | CF3 |
| 14-1818 | 2,2,2-trichloroethyl | S(O)2Me | CH2Ph | F | H | H | H | I | H | nonafluoro-2-butyl | H | CF3 |
| 14-1819 | 2,2,2-trichloroethyl | S(O)2Me | CH2(3-Py) | F | H | H | H | I | H | nonafluoro-2-butyl | H | CF3 |
| 14-1820 | 2,2,2-trichloroethyl | S(O)2Me | CH2(3-Py-N-oxide) | F | H | H | H | I | H | nonafluoro-2-butyl | H | CF3 |
| 14-1821 | 2,2,2-trichloroethyl | CH2Ph | Et | F | H | H | H | I | H | nonafluoro-2-butyl | H | CF3 |
| 14-1822 | 2,2,2-trichloroethyl | CH2Ph | n-Pr | F | H | H | H | I | H | nonafluoro-2-butyl | H | CF3 |
| 14-1823 | 2,2,2-trichloroethyl | CH2Ph | i-Pr | F | H | H | H | I | H | nonafluoro-2-butyl | H | CF3 |
| 14-1824 | 2,2,2-trichloroethyl | CH2Ph | CH2CH=CH2 | F | H | H | H | I | H | nonafluoro-2-butyl | H | CF3 |
| 14-1825 | 2,2,2-trichloroethyl | CH2Ph | CN | F | H | H | H | I | H | nonafluoro-2-butyl | H | CF3 |
| 14-1826 | 2,2,2-trichloroethyl | CH2Ph | CH2CE≡CH | F | H | H | H | I | H | nonafluoro-2-butyl | H | CF3 |
| 14-1827 | 2,2,2-trichloroethyl | CH2Ph | NH2 | F | H | H | H | I | H | nonafluoro-2-butyl | H | CF3 |

**Table 14(5)**

| compound number | Q₂ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 14-1828 | 2,2,2-trichloroethyl | CH2Ph | C(O)OMe | F | H | H | H | I | H | nonafluoro-2-butyl | H | CF3 |
| 14-1829 | 2,2,2-trichloroethyl | CH2Ph | C(O)C(O)OMe | F | H | H | H | I | H | nonafluoro-2-butyl | H | CF3 |
| 14-1830 | 2,2,2-trichloroethyl | CH2Ph | C(O)Me | F | H | H | H | I | H | nonafluoro-2-butyl | H | CF3 |
| 14-1831 | 2,2,2-trichloroethyl | CH2Ph | S(O)2Me | F | H | H | H | I | H | nonafluoro-2-butyl | H | CF3 |
| 14-1832 | 2,2,2-trichloroethyl | CH2Ph | CH2Ph | F | H | H | H | I | H | nonafluoro-2-butyl | H | CF3 |
| 14-1833 | 2,2,2-trichloroethyl | CH2Ph | CH2(3-Py) | F | H | H | H | I | H | nonafluoro-2-butyl | H | CF3 |
| 14-1834 | 2,2,2-trichloroethyl | CH2Ph | CH2(3-Py-N-oxide) | F | H | H | H | I | H | nonafluoro-2-butyl | H | CF3 |
| 14-1835 | 2,2,2-trichloroethyl | CH2(3-Py) | Et | F | H | H | H | I | H | nonafluoro-2-butyl | H | CF3 |
| 14-1836 | 2,2,2-trichloroethyl | CH2(3-Py) | n-Pr | F | H | H | H | I | H | nonafluoro-2-butyl | H | CF3 |
| 14-1837 | 2,2,2-trichloroethyl | CH2(3-Py) | i-Pr | F | H | H | H | I | H | nonafluoro-2-butyl | H | CF3 |
| 14-1838 | 2,2,2-trichloroethyl | CH2(3-Py) | CH2CH=CH2 | F | H | H | H | I | H | nonafluoro-2-butyl | H | CF3 |
| 14-1839 | 2,2,2-trichloroethyl | CH2(3-Py) | CN | F | H | H | H | I | H | nonafluoro-2-butyl | H | CF3 |
| 14-1840 | 2,2,2-trichloroethyl | CH2(3-Py) | CH2C≡CH | F | H | H | H | I | H | nonafluoro-2-butyl | H | CF3 |
| 14-1841 | 2,2,2-trichloroethyl | CH2(3-Py) | NH2 | F | H | H | H | I | H | nonafluoro-2-butyl | H | CF3 |
| 14-1842 | 2,2,2-trichloroethyl | CH2(3-Py) | C(O)OMe | F | H | H | H | I | H | nonafluoro-2-butyl | H | CF3 |
| 14-1843 | 2,2,2-trichloroethyl | CH2(3-Py) | C(O)C(O)OMe | F | H | H | H | I | H | nonafluoro-2-butyl | H | CF3 |
| 14-1844 | 2,2,2-trichloroethyl | CH2(3-Py) | C(O)Me | F | H | H | H | I | H | nonafluoro-2-butyl | H | CF3 |
| 14-1845 | 2,2,2-trichloroethyl | CH2(3-Py) | S(O)2Me | F | H | H | H | I | H | nonafluoro-2-butyl | H | CF3 |
| 14-1846 | 2,2,2-trichloroethyl | CH2(3-Py) | CH2Ph | F | H | H | H | I | H | nonafluoro-2-butyl | H | CF3 |
| 14-1847 | 2,2,2-trichloroethyl | CH2(3-Py) | CH2(3-Py) | F | H | H | H | I | H | nonafluoro-2-butyl | H | CF3 |
| 14-1848 | 2,2,2-trichloroethyl | CH2(3-Py) | CH2(3-Py-N-oxide) | F | H | H | H | I | H | nonafluoro-2-butyl | H | CF3 |
| 14-1849 | 2,2,2-trichloroethyl | CH2(3-Py-N-oxide) | Et | F | H | H | H | I | H | nonafluoro-2-butyl | H | CF3 |
| 14-1850 | 2,2,2-trichloroethyl | CH2(3-Py-N-oxide) | n-Pr | F | H | H | H | I | H | nonafluoro-2-butyl | H | CF3 |
| 14-1851 | 2,2,2-trichloroethyl | CH2(3-Py-N-oxide) | i-Pr | F | H | H | H | I | H | nonafluoro-2-butyl | H | CF3 |
| 14-1852 | 2,2,2-trichloroethyl | CH2(3-Py-N-oxide) | CH2CH=CH2 | F | H | H | H | I | H | nonafluoro-2-butyl | H | CF3 |
| 14-1853 | 2,2,2-trichloroethyl | CH2(3-Py-N-oxide) | CN | F | H | H | H | I | H | nonafluoro-2-butyl | H | CF3 |
| 14-1854 | 2,2,2-trichloroethyl | CH2(3-Py-N-oxide) | CH2C≡CH | F | H | H | H | I | H | nonafluoro-2-butyl | H | CF3 |
| 14-1855 | 2,2,2-trichloroethyl | CH2(3-Py-N-oxide) | NH2 | F | H | H | H | I | H | nonafluoro-2-butyl | H | CF3 |
| 14-1856 | 2,2,2-trichloroethyl | CH2(3-Py-N-oxide) | C(O)OMe | F | H | H | H | I | H | nonafluoro-2-butyl | H | CF3 |
| 14-1857 | 2,2,2-trichloroethyl | CH2(3-Py-N-oxide) | C(O)C(O)OMe | F | H | H | H | I | H | nonafluoro-2-butyl | H | CF3 |
| 14-1858 | 2,2,2-trichloroethyl | CH2(3-Py-N-oxide) | C(O)Me | F | H | H | H | I | H | nonafluoro-2-butyl | H | CF3 |
| 14-1859 | 2,2,2-trichloroethyl | CH2(3-Py-N-oxide) | S(O)2Me | F | H | H | H | I | H | nonafluoro-2-butyl | H | CF3 |
| 14-1860 | 2,2,2-trichloroethyl | CH2(3-Py-N-oxide) | CH2Ph | F | H | H | H | I | H | nonafluoro-2-butyl | H | CF3 |
| 14-1861 | 2,2,2-trichloroethyl | CH2(3-Py-N-oxide) | CH2(3-Py) | F | H | H | H | I | H | nonafluoro-2-butyl | H | CF3 |
| 14-1862 | 2,2,2-trichloroethyl | CH2(3-Py-N-oxide) | CH2(3-Py-N-oxide) | F | H | H | H | I | H | nonafluoro-2-butyl | H | CF3 |
| 14-1873 | 2,2,2-trichloroethyl | Me | Me | F | H | H | H | OCF3 | H | heptafluoroisopropyl | H | OCF3 |
| 14-1902 | 2,2,2-trichloroethyl | Me | Me | F | H | H | H | OCF3 | H | nonafluoro-2-butyl | H | OCF3 |
| 14-1931 | 2,2,2-trichloroethyl | Me | Me | F | H | H | H | CF3 | H | heptafluoroisopropyl | H | OCF3 |
| 14-1960 | 2,2,2-trichloroethyl | Me | Me | F | H | H | H | CF3 | H | nonafluoro-2-butyl | H | OCF3 |
| 14-1989 | 2,2,2-trichloroethyl | Me | Me | F | H | H | H | CF3 | H | heptafluoroisopropyl | H | CF3 |
| 14-2019 | 2,2,2-tribromoethyl | Me | CH2C≡CH | F | H | H | H | CF3 | H | nonafluoro-2-butyl | H | CF3 |
| 14-2233 | 2,2,2-trichloroethyl | Me | Me | H | F | H | H | F | H | heptafluoroisopropyl | H | F |
| 14-2234 | 2,2,2-trichloroethyl | Me | Me | H | F | H | H | F | H | nonafluoro-2-butyl | H | F |
| 14-2245 | 2,2,2-trichloroethyl | Me | Me | H | F | H | H | Cl | H | heptafluoroisopropyl | H | Cl |
| 14-2274 | 2,2,2-trichloroethyl | Me | Me | H | F | H | H | Cl | H | nonafluoro-2-butyl | H | Cl |
| 14-2303 | 222-trichloroethyl | Me | Me | H | F | H | H | Br | H | heptafluoroisopropyl | H | Br |
| 14-2332 | 2,2,2-trichloroethyl | Me | Me | H | F | H | H | Br | H | nonafluoro-2-butyl | H | Br |
| 14-2361 | 2,2,2-trichloroethyl | Me | Me | H | F | H | H | I | H | heptafluoroisopropyl | H | I |
| 14-2390 | 2,2,2-trichloroethyl | Me | Me | H | F | H | H | I | H | nonafluoro-2-butyl | H | I |
| 13-1236 | 2,2,2-trichloroethyl | Me | Me | H | F | H | H | Cl | H | heptafluoroisopropyl | H | Br |
| 14-2605 | 2,2,2-trichloroethyl | Me | Me | H | F | H | H | F | H | heptafluoroisopropyl | H | OCF3 |
| 14-2606 | 2,2,2-trichloroethyl | Me | Me | H | F | H | H | F | H | nonafluoro-2-butyl | H | OCF3 |
| 14-2617 | 2,2,2-trichloroethyl | Me | Me | H | F | H | H | Cl | H | heptafluoroisopropyl | H | OCF3 |
| 14-2646 | 2,2,2-trichloroethyl | Me | Me | H | F | H | H | Cl | H | nonafluoro-2-butyl | H | OCF3 |
| 14-2675 | 2,2,2-trichloroethyl | Me | Me | H | F | H | H | Br | H | heptafluoroisopropyl | H | OCF3 |
| 14-2704 | 2,2,2-trichloroethyl | Me | Me | H | F | H | H | Br | H | nonafluoro-2-butyl | H | OCF3 |
| 14-2733 | 2,2,2-trichloroethyl | Me | Me | H | F | H | H | I | H | heptafluoroisopropyl | H | OCF3 |
| 14-2762 | 2,2,2-trichloroethyl | Me | Me | H | F | H | H | I | H | nonafluoro-2-butyl | H | OCF3 |
| 14-2977 | 2,2,2-trichloroethyl | Me | Me | H | F | H | H | F | H | heptafluoroisopropyl | H | CF3 |

**Table 14(6)**

| compound number | Q₂ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 14-2978 | 2,2,2-trichloroethyl | Me | Me | H | F | H | H | F | H | nonafluoro-2-butyl | H | CF3 |
| 14-2989 | 2,2,2-trichloroethyl | Me | Me | H | F | H | H | Cl | H | heptafluoroisopropyl | H | CF3 |
| 14-3018 | 2,2,2-trichloroethyl | Me | Me | H | F | H | H | Cl | H | nonafluoro-2-butyl | H | CF3 |
| 14-3047 | 2,2,2-trichloroethyl | Me | Me | H | F | H | H | Br | H | heptafluoroisopropyl | H | CF3 |
| 14-3076 | 2,2,2-trichloroethyl | Me | Me | H | F | H | H | Br | H | nonafluoro-2-butyl | H | CF3 |
| 14-3105 | 2,2,2-trichloroethyl | Me | Me | H | F | H | H | I | H | heptafluoroisopropyl | H | CF3 |
| 14-3134 | 2,2,2-trichloroethyl | Me | Me | H | F | H | H | I | H | nonafluoro-2-butyl | H | CF3 |
| 14-3359 | 2,2,2-trichloroethyl | Me | Me | H | F | H | H | OCF3 | H | heptafluoroisopropyl | H | OCF3 |
| 14-3388 | 2,2,2-trichloroethyl | Me | Me | H | F | H | H | OCF3 | H | nonafluoro-2-butyl | H | OCF3 |
| 14-3417 | 2,2,2-trichloroethyl | Me | Me | H | F | H | H | CF3 | H | heptafluoroisopropyl | H | OCF3 |
| 14-3446 | 2,2,2-trichloroethyl | Me | Me | H | F | H | H | CF3 | H | nonafluoro-2-butyl | H | OCF3 |
| 14-3475 | 2,2,2-trichloroethyl | Me | Me | H | F | H | H | CF3 | H | heptafluoroisopropyl | H | CF3 |
| 14-3504 | 2,2,2-trichloroethyl | Me | Me | H | F | H | H | CF3 | H | nonafluoro-2-butyl | H | CF3 |
| 14-3719 | 2,2,2-trichloroethyl | Me | Me | F | F | H | H | F | H | heptafluoroisopropyl | H | F |
| 14-3720 | 2,2,2-trichloroethyl | Me | Me | F | F | H | H | F | H | nonafluoro-2-butyl | H | F |
| 14-3731 | 2,2,2-trichloroethyl | Me | Me | F | F | H | H | Cl | H | heptafluoroisopropyl | H | Cl |
| 14-3760 | 2,2,2-trichloroethyl | Me | Me | F | F | H | H | Cl | H | nonafluoro-2-butyl | H | Cl |
| 14-3789 | 2,2,2-trichloroethyl | Me | Me | F | F | H | H | Br | H | heptafluoroisopropyl | H | Br |
| 14-3818 | 2,2,2-trichloroethyl | Me | Me | F | F | H | H | Br | H | nonafluoro-2-butyl | H | Br |
| 14-3847 | 2,2,2-trichloroethyl | Me | Me | F | F | H | H | I | H | heptafluoroisopropyl | H | I |
| 14-3876 | 2,2,2-trichloroethyl | Me | Me | F | F | H | H | I | H | nonafluoro-2-butyl | H | I |
| 13-1950 | 2,2,2-trichloroethyl | Me | Me | F | F | H | H | Cl | H | heptafluoroisopropyl | H | Br |
| 14-4091 | 2,2,2-trichloroethyl | Me | Me | F | F | H | H | F | H | heptafluoroisopropyl | H | OCF3 |
| 14-4092 | 2,2,2-trichloroethyl | Me | Me | F | F | H | H | F | H | nonafluoro-2-butyl | H | OCF3 |
| 14-4103 | 2,2,2-trichloroethyl | Me | Me | F | F | H | H | Cl | H | heptafluoroisopropyl | H | OCF3 |
| 14-4132 | 2,2,2-trichloroethyl | Me | Me | F | F | H | H | Cl | H | nonafluoro-2-butyl | H | OCF3 |
| 14-4161 | 2,2,2-trichloroethyl | Me | Me | F | F | H | H | Br | H | heptafluoroisopropyl | H | OCF3 |
| 14-4190 | 2,2,2-trichloroethyl | Me | Me | F | F | H | H | Br | H | nonafluoro-2-butyl | H | OCF3 |
| 14-4219 | 2,2,2-trichloroethyl | Me | Me | F | F | H | H | I | H | heptafluoroisopropyl | H | OCF3 |
| 14-4248 | 2,2,2-trichloroethyl | Me | Me | F | F | H | H | I | H | nonafluoro-2-butyl | H | OCF3 |
| 14-4463 | 2,2,2-trichloroethyl | Me | Me | F | F | H | H | F | H | heptafluoroisopropyl | H | CF3 |
| 14-4464 | 2,2,2-trichloroethyl | Me | Me | F | F | H | H | F | H | nonafluoro-2-butyl | H | CF3 |
| 14-4475 | 2,2,2-trichloroethyl | Me | Me | F | F | H | H | Cl | H | heptafluoroisopropyl | H | CF3 |
| 14-4504 | 2,2,2-trichloroethyl | Me | Me | F | F | H | H | Cl | H | nonafluoro-2-butyl | H | CF3 |
| 14-4533 | 2,2,2-trichloroethyl | Me | Me | F | F | H | H | Br | H | heptafluoroisopropyl | H | CF3 |
| 14-4562 | 2,2,2-trichloroethyl | Me | Me | F | F | H | H | Br | H | nonafluoro-2-butyl | H | CF3 |
| 14-4591 | 2,2,2-trichloroethyl | Me | Me | F | F | H | H | I | H | heptafluoroisopropyl | H | CF3 |
| 14-4620 | 2,2,2-trichloroethyl | Me | Me | F | F | H | H | I | H | nonafluoro-2-butyl | H | CF3 |
| 14-4845 | 2,2,2-trichloroethyl | Me | Me | F | F | H | H | OCF3 | H | heptafluoroisopropyl | H | OCF3 |
| 14-4874 | 2,2,2-trichloroethyl | Me | Me | F | F | H | H | OCF3 | H | nonafluoro-2-butyl | H | OCF3 |
| 14-4903 | 2,2,2-trichloroethyl | Me | Me | F | F | H | H | CF3 | H | heptafluoroisopropyl | H | OCF3 |
| 14-4932 | 2,2,2-trichloroethyl | Me | Me | F | F | H | H | CF3 | H | nonafluoro-2-butyl | H | OCF3 |
| 14-4961 | 2,2,2-trichloroethyl | Me | Me | F | F | H | H | CF3 | H | heptafluoroisopropyl | H | CF3 |
| 14-4990 | 2,2,2-trichloroethyl | Me | Me | F | F | H | H | CF3 | H | nonafluoro-2-butyl | H | CF3 |
| 14-5205 | 2,2,2-trichloroethyl | Me | Me | F | F | F | H | Cl | H | pentafluoroethyl | H | Cl |
| 14-5206 | 2,2,2-trichloroethyl | Me | Me | F | F | F | H | Br | H | heptafluoroisopropyl | H | Br |
| 14-5207 | 2,2,2-trichloroethyl | Me | Me | F | F | F | H | Br | H | heptafluoroisopropyl | H | OCF3 |
| 14-5208 | 2,2,2-trichloroethyl | Me | Me | F | F | F | H | I | H | heptafluoroisopropyl | H | CF3 |
| 14-5209 | 2,2,2-trichloroethyl | Me | Me | F | F | F | H | CF3 | H | heptafluoroisopropyl | H | CF3 |
| 14-5210 | 2,2,2-trichloroethyl | Me | Me | F | F | F | H | I | H | nonafluoro-2-butyl | H | I |
| 14-5211 | 2,2,2-trichloroethyl | Me | Me | F | F | F | H | Cl | H | nonafluoro-2-butyl | H | OCF3 |
| 14-5212 | 2,2,2-trichloroethyl | Me | Me | F | F | F | H | Br | H | nonafluoro-2-butyl | H | CF3 |
| 14-5213 | 2,2,2-trichloroethyl | Me | Me | F | F | F | H | CF3 | H | nonafluoro-2-butyl | H | CF3 |
| 14-5214 | 2,2,2-trichloroethyl | Me | Me | H | H | F | H | Cl | H | pentafluoroethyl | H | Cl |
| 14-5215 | 2,2,2-trichloroethyl | Me | Me | H | H | F | H | Br | H | heptafluoroisopropyl | H | Br |
| 14-5216 | 2,2,2-trichloroethyl | Me | Me | H | H | F | H | Br | H | heptafluoroisopropyl | H | OCF3 |
| 14-5217 | 2,2,2-trichloroethyl | Me | Me | H | H | F | H | I | H | heptafluoroisopropyl | H | CF3 |
| 14-5218 | 2,2,2-trichloroethyl | Me | Me | H | H | F | H | CF3 | H | heptafluoroisopropyl | H | CF3 |
| 14-5219 | 2,2,2-trichloroethyl | Me | Me | H | H | F | H | I | H | nonafluoro-2-butyl | H | I |
| 14-5220 | 2,2,2-trichloroethyl | Me | Me | H | H | F | H | Cl | H | nonafluoro-2-butyl | H | OCF3 |
| 14-5221 | 2,2,2-trichloroethyl | Me | Me | H | H | F | H | Br | H | nonafluoro-2-butyl | H | CF3 |
| 14-5222 | 2,2,2-trichloroethyl | Me | Me | H | H | F | H | CF3 | H | nonafluoro-2-butyl | H | CF3 |
| 14-5223 | 2,2,2-trichloroethyl | Me | Me | H | H | H | F | Cl | H | pentafluoroethyl | H | Cl |
| 14-5224 | 2,2,2-trichloroethyl | Me | Me | H | H | H | F | Br | H | heptafluoroisopropyl | H | Br |
| 14-5225 | 2,2,2-trichloroethyl | Me | Me | H | H | H | F | Br | H | heptafluoroisopropyl | H | OCF3 |
| 14-5226 | 2,2,2-trichloroethyl | Me | Me | H | H | H | F | I | H | heptafluoroisopropyl | H | CF3 |
| 14-5227 | 2,2,2-trichloroethyl | Me | Me | H | H | H | F | CF3 | H | heptafluoroisopropyl | H | CF3 |
| 14-5228 | 2,2,2-trichloroethyl | Me | Me | H | H | H | F | I | H | nonafluoro-2-butyl | H | I |
| 14-5229 | 2,2,2-trichloroethyl | Me | Me | H | H | H | F | Cl | H | nonafluoro-2-butyl | H | OCF3 |

**Table 14(7)**

| compound number | Q₂ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 14-5230 | 2,2,2-trichloroethyl | Me | Me | H | H | H | F | Br | H | nonafluoro-2-butyl | H | CF3 |
| 14-5231 | 2,2,2-trichloroethyl | Me | Me | H | H | H | F | CF3 | H | nonafluoro-2-butyl | H | CF3 |
| 14-5232 | 2,2,2-trichloroethyl | Me | Me | F | H | F | H | Cl | H | pentafluoroethyl | H | Cl |
| 14-5233 | 2,2,2-trichloroethyl | Me | Me | F | H | F | H | Br | H | heptafluoroisopropyl | H | Br |
| 14-5234 | 2,2,2-trichloroethyl | Me | Me | F | H | F | H | Br | H | heptafluoroisopropyl | H | OCF3 |
| 14-5235 | 2,2,2-trichloroethyl | Me | Me | F | H | F | H | I | H | heptafluoroisopropyl | H | CF3 |
| 14-5236 | 2,2,2-trichloroethyl | Me | Me | F | H | F | H | CF3 | H | heptafluoroisopropyl | H | CF3 |
| 14-5237 | 2,2,2-trichloroethyl | Me | Me | F | H | F | H | I | H | nonafluoro-2-butyl | H | I |
| 14-5238 | 2,2,2-trichloroethyl | Me | Me | F | H | F | H | Cl | H | nonafluoro-2-butyl | H | OCF3 |
| 14-5239 | 2,2,2-trichloroethyl | Me | Me | F | H | F | H | Br | H | nonafluoro-2-butyl | H | CF3 |
| 14-5240 | 2,2,2-trichloroethyl | Me | Me | F | H | F | H | CF3 | H | nonafluoro-2-butyl | H | CF3 |
| 14-5241 | 2,2,2-trichloroethyl | Me | Me | F | H | H | F | Cl | H | pentafluoroethyl | H | Cl |
| 14-5242 | 2,2,2-trichloroethyl | Me | Me | F | H | H | F | Br | H | heptafluoroisopropyl | H | Br |
| 14-5243 | 2,2,2-trichloroethyl | Me | Me | F | H | H | F | Br | H | heptafluoroisopropyl | H | OCF3 |
| 14-5244 | 2,2,2-trichloroethyl | Me | Me | F | H | H | F | I | H | heptafluoroisopropyl | H | CF3 |
| 14-5245 | 2,2,2-trichloroethyl | Me | Me | F | H | H | F | CF3 | H | heptafluoroisopropyl | H | CF3 |
| 14-5246 | 2,2,2-trichloroethyl | Me | Me | F | H | H | F | I | H | nonafluoro-2-butyl | H | I |
| 14-5247 | 2,2,2-trichloroethyl | Me | Me | F | H | H | F | Cl | H | nonafluoro-2-butyl | H | OCF3 |
| 14-5248 | 2,2,2-trichloroethyl | Me | Me | F | H | H | F | Br | H | nonafluoro-2-butyl | H | CF3 |
| 14-5249 | 2,2,2-trichloroethyl | Me | Me | F | H | H | F | CF3 | H | nonafluoro-2-butyl | H | CF3 |
| 14-5250 | 2,2,2-trichloroethyl | Me | Me | H | F | F | H | Cl | H | pentafluoroethyl | H | Cl |
| 14-5251 | 2,2,2-trichloroethyl | Me | Me | H | F | F | H | Br | H | heptafluoroisopropyl | H | Br |
| 14-5252 | 2,2,2-trichloroethyl | Me | Me | H | F | F | H | Br | H | heptafluoroisopropyl | H | OCF3 |
| 14-5253 | 2,2,2-trichloroethyl | Me | Me | H | F | F | H | I | H | heptafluoroisopropyl | H | CF3 |
| 14-5254 | 2,2,2-trichloroethyl | Me | Me | H | F | F | H | CF3 | H | heptafluoroisopropyl | H | CF3 |
| 14-5255 | 2,2,2-trichloroethyl | Me | Me | H | F | F | H | I | H | nonafluoro-2-butyl | H | I |
| 14-5256 | 2,2,2-trichloroethyl | Me | Me | H | F | F | H | Cl | H | nonafluoro-2-butyl | H | OCF3 |
| 14-5257 | 2,2,2-trichloroethyl | Me | Me | H | F | F | H | Br | H | nonafluoro-2-butyl | H | CF3 |
| 14-5258 | 2,2,2-trichloroethyl | Me | Me | H | F | F | H | CF3 | H | nonafluoro-2-butyl | H | CF3 |
| 14-5259 | 2,2,2-trichloroethyl | Me | Me | H | F | H | F | Cl | H | pentafluoroethyl | H | Cl |
| 14-5260 | 2,2,2-trichloroethyl | Me | Me | H | F | H | F | Br | H | heptafluoroisopropyl | H | Br |
| 14-5261 | 2,2,2-trichloroethyl | Me | Me | H | F | H | F | Br | H | heptafluoroisopropyl | H | OCF3 |
| 14-5262 | 2,2,2-trichloroethyl | Me | Me | H | F | H | F | I | H | heptafluoroisopropyl | H | CF3 |
| 14-5263 | 2,2,2-trichloroethyl | Me | Me | H | F | H | F | CF3 | H | heptafluoroisopropyl | H | CF3 |
| 14-5264 | 2,2,2-trichloroethyl | Me | Me | H | F | H | F | I | H | nonafluoro-2-butyl | H | I |
| 14-5265 | 2,2,2-trichloroethyl | Me | Me | H | F | H | F | Cl | H | nonafluoro-2-butyl | H | OCF3 |
| 14-5266 | 2,2,2-trichloroethyl | Me | Me | H | F | H | F | Br | H | nonafluoro-2-butyl | H | CF3 |
| 14-5267 | 2,2,2-trichloroethyl | Me | Me | H | F | H | F | CF3 | H | nonafluoro-2-butyl | H | CF3 |
| 14-5268 | 2,2,2-trichloroethyl | Me | Me | H | H | F | F | Cl | H | pentafluoroethyl | H | Cl |
| 14-5269 | 2,2,2-trichloroethyl | Me | Me | H | H | F | F | Br | H | heptafluoroisopropyl | H | Br |
| 14-5270 | 2,2,2-trichloroethyl | Me | Me | H | H | F | F | Br | H | heptafluoroisopropyl | H | OCF3 |
| 14-5271 | 2,2,2-trichloroethyl | Me | Me | H | H | F | F | I | H | heptafluoroisopropyl | H | CF3 |
| 14-5272 | 2,2,2-trichloroethyl | Me | Me | H | H | F | F | CF3 | H | heptafluoroisopropyl | H | CF3 |
| 14-5273 | 2,2,2-trichloroethyl | Me | Me | H | H | F | F | I | H | nonafluoro-2-butyl | H | I |
| 14-5274 | 2,2,2-trichloroethyl | Me | Me | H | H | F | F | Cl | H | nonafluoro-2-butyl | H | OCF3 |
| 14-5275 | 2,2,2-trichloroethyl | Me | Me | H | H | F | F | Br | H | nonafluoro-2-butyl | H | CF3 |
| 14-5276 | 2,2,2-trichloroethyl | Me | Me | H | H | F | F | CF3 | H | nonafluoro-2-butyl | H | CF3 |
| 14-5277 | 2,2,2-trichloroethyl | Me | Me | F | F | H | F | Cl | H | pentafluoroethyl | H | Cl |
| 14-5278 | 2,2,2-trichloroethyl | Me | Me | F | F | H | F | Br | H | heptafluoroisopropyl | H | Br |
| 14-5279 | 2,2,2-trichloroethyl | Me | Me | F | F | H | F | Br | H | heptafluoroisopropyl | H | OCF3 |
| 14-5280 | 2,2,2-trichloroethyl | Me | Me | F | F | H | F | I | H | heptafluoroisopropyl | H | CF3 |
| 14-5281 | 2,2,2-trichloroethyl | Me | Me | F | F | H | F | CF3 | H | heptafluoroisopropyl | H | CF3 |
| 14-5282 | 2,2,2-trichloroethyl | Me | Me | F | F | H | F | I | H | nonafluoro-2-butyl | H | I |
| 14-5283 | 2,2,2-trichloroethyl | Me | Me | F | F | H | F | Cl | H | nonafluoro-2-butyl | H | OCF3 |
| 14-5284 | 2,2,2-trichloroethyl | Me | Me | F | F | H | F | Br | H | nonafluoro-2-butyl | H | CF3 |
| 14-5285 | 2,2,2-trichloroethyl | Me | Me | F | F | H | F | CF3 | H | nonafluoro-2-butyl | H | CF3 |
| 14-5286 | 2,2,2-trichloroethyl | Me | Me | F | H | F | F | Cl | H | pentafluoroethyl | H | Cl |
| 14-5287 | 2,2,2-trichloroethyl | Me | Me | F | H | F | F | Br | H | heptafluoroisopropyl | H | Br |
| 14-5288 | 2,2,2-trichloroethyl | Me | Me | F | H | F | F | Br | H | heptafluoroisopropyl | H | OCF3 |
| 14-5289 | 2,2,2-trichloroethyl | Me | Me | F | H | F | F | I | H | heptafluoroisopropyl | H | CF3 |
| 14-5290 | 2,2,2-trichloroethyl | Me | Me | F | H | F | F | CF3 | H | heptafluoroisopropyl | H | CF3 |
| 14-5291 | 2,2,2-trichloroethyl | Me | Me | F | H | F | F | I | H | nonafluoro-2-butyl | H | I |
| 14-5292 | 2,2,2-trichloroethyl | Me | Me | F | H | F | F | Cl | H | nonafluoro-2-butyl | H | OCF3 |
| 14-5293 | 2,2,2-trichloroethyl | Me | Me | F | H | F | F | Br | H | nonafluoro-2-butyl | H | CF3 |

**Table 14(8)**

| compound number | Q₂ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 14-5294 | 2,2,2-trichloroethyl | Me | Me | F | H | F | F | CF3 | H | nonafluoro-2-butyl | H | CF3 |
| 14-5295 | 2,2,2-trichloroethyl | Me | Me | H | F | F | F | Cl | H | pentafluoroethyl | H | Cl |
| 14-5296 | 2,2,2-trichloroethyl | Me | Me | H | F | F | F | Br | H | heptafluoroisopropyl | H | Br |
| 14-5297 | 2,2,2-trichloroethyl | Me | Me | H | F | F | F | Br | H | heptafluoroisopropyl | H | OCF3 |
| 14-5298 | 2,2,2-trichloroethyl | Me | Me | H | F | F | F | I | H | heptafluoroisopropyl | H | CF3 |
| 14-5299 | 2,2,2-trichloroethyl | Me | Me | H | F | F | F | CF3 | H | heptafluoroisopropyl | H | CF3 |
| 14-5300 | 2,2,2-trichloroethyl | Me | Me | H | F | F | F | I | H | nonafluoro-2-butyl | H | I |
| 14-5301 | 2,2,2-trichloroethyl | Me | Me | H | F | F | F | Cl | H | nonafluoro-2-butyl | H | OCF3 |
| 14-5302 | 2,2,2-trichloroethyl | Me | Me | H | F | F | F | Br | H | nonafluoro-2-butyl | H | CF3 |
| 14-5303 | 2,2,2-trichloroethyl | Me | Me | H | F | F | F | CF3 | H | nonafluoro-2-butyl | H | CF3 |
| 14-5304 | 2,2,2-trichloroethyl | Me | Me | F | F | F | F | Cl | H | pentafluoroethyl | H | Cl |
| 14-5305 | 2,2,2-trichloroethyl | Me | Me | F | F | F | F | Br | H | heptafluoroisopropyl | H | Br |
| 14-5306 | 2,2,2-trichloroethyl | Me | Me | F | F | F | F | Br | H | heptafluoroisopropyl | H | OCF3 |
| 14-5307 | 2,2,2-trichloroethyl | Me | Me | F | F | F | F | I | H | heptafluoroisopropyl | H | CF3 |
| 14-5308 | 2,2,2-trichloroethyl | Me | Me | F | F | F | F | CF3 | H | heptafluoroisopropyl | H | CF3 |
| 14-5309 | 2,2,2-trichloroethyl | Me | Me | F | F | F | F | I | H | nonafluoro-2-butyl | H | I |
| 14-5310 | 2,2,2-trichloroethyl | Me | Me | F | F | F | F | Cl | H | nonafluoro-2-butyl | H | OCF3 |
| 14-5311 | 2,2,2-trichloroethyl | Me | Me | F | F | F | F | Br | H | nonafluoro-2-butyl | H | CF3 |
| 14-5312 | 2,2,2-trichloroethyl | Me | Me | F | F | F | F | CF3 | H | nonafluoro-2-butyl | H | CF3 |
| 14-5313 | 2,2,2-trichloroethyl | Me | Me | H | CN | H | H | F | H | heptafluoroisopropyl | H | OCF3 |
| 14-5314 | 2,2,2-trichloroethyl | Me | Me | H | CN | H | H | F | H | nonafluoro-2-butyl | H | OCF3 |
| 14-5325 | 2,2,2-trichloroethyl | Me | Me | H | CN | H | H | Cl | H | heptafluoroisopropyl | H | OCF3 |
| 14-5354 | 2,2,2-trichloroethyl | Me | Me | H | CN | H | H | Cl | H | nonafluoro-2-butyl | H | OCF3 |
| 14-5383 | 2,2,2-trichloroethyl | Me | Me | H | CN | H | H | Br | H | heptafluoroisopropyl | H | OCF3 |
| 14-5412 | 2,2,2-trichloroethyl | Me | Me | H | CN | H | H | Br | H | nonafluoro-2-butyl | H | OCF3 |
| 14-5441 | 2,2,2-trichloroethyl | Me | Me | H | CN | H | H | I | H | heptafluoroisopropyl | H | OCF3 |
| 14-5470 | 2,2,2-trichloroethyl | Me | Me | H | CN | H | H | I | H | nonafluoro-2-butyl | H | OCF3 |
| 14-5685 | 2,2,2-trichloroethyl | Me | Me | H | CN | H | H | F | H | heptafluoroisopropyl | H | CF3 |
| 14-5686 | 2,2,2-trichloroethyl | Me | Me | H | CN | H | H | F | H | nonafluoro-2-butyl | H | CF3 |
| 14-5697 | 2,2,2-trichloroethyl | Me | Me | H | CN | H | H | Cl | H | heptafluoroisopropyl | H | CF3 |
| 14-5726 | 2,2,2-trichloroethyl | Me | Me | H | CN | H | H | Cl | H | nonafluoro-2-butyl | H | CF3 |
| 14-5755 | 2,2,2-trichloroethyl | Me | Me | H | CN | H | H | Br | H | heptafluoroisopropyl | H | CF3 |
| 14-5784 | 2,2,2-trichloroethyl | Me | Me | H | CN | H | H | Br | H | nonafluoro-2-butyl | H | CF3 |
| 14-5813 | 2,2,2-trichloroethyl | Me | Me | H | CN | H | H | I | H | heptafluoroisopropyl | H | CF3 |
| 14-5842 | 2,2,2-trichloroethyl | Me | Me | H | CN | H | H | I | H | nonafluoro-2-butyl | H | CF3 |
| 14-6067 | 2,2,2-trichloroethyl | Me | Me | H | CN | H | H | OCF3 | H | heptafluoroisopropyl | H | OCF3 |
| 14-6096 | 2,2,2-trichloroethyl | Me | Me | H | CN | H | H | OCF3 | H | nonafluoro-2-butyl | H | OCF3 |
| 14-6125 | 2,2,2-trichloroethyl | Me | Me | H | CN | H | H | CF3 | H | heptafluoroisopropyl | H | OCF3 |
| 14-6154 | 2,2,2-trichloroethyl | Me | Me | H | CN | H | H | CF3 | H | nonafluoro-2-butyl | H | OCF3 |
| 14-6183 | 2,2,2-trichloroethyl | Me | Me | H | CN | H | H | CF3 | H | heptafluoroisopropyl | H | CF3 |
| 14-6212 | 2,2,2-trichloroethyl | Me | Me | H | CN | H | H | CF3 | H | nonafluoro-2-butyl | H | CF3 |
| 14-6427 | 2,2,2-trichloroethyl | Me | Me | F | CN | H | H | F | H | heptafluoroisopropyl | H | F |
| 14-6428 | 2,2,2-trichloroethyl | Me | Me | F | CN | H | H | F | H | nonafluoro-2-butyl | H | F |
| 14-6439 | 2,2,2-trichloroethyl | Me | Me | F | CN | H | H | Cl | H | heptafluoroisopropyl | H | Cl |
| 14-6468 | 2,2,2-trichloroethyl | Me | Me | F | CN | H | H | Cl | H | nonafluoro-2-butyl | H | Cl |
| 14-6497 | 2,2,2-trichloroethyl | Me | Me | F | CN | H | H | Br | H | heptafluoroisopropyl | H | Br |
| 14-6526 | 2,2,2-trichloroethyl | Me | Me | F | CN | H | H | Br | H | nonafluoro-2-butyl | H | Br |
| 14-6555 | 2,2,2-trichloroethyl | Me | Me | F | CN | H | H | I | H | heptafluoroisopropyl | H | I |
| 14-6584 | 2,2,2-trichloroethyl | Me | Me | F | CN | H | H | I | H | nonafluoro-2-butyl | H | I |
| 13-3306 | 2,2,2-trichloroethyl | Me | Me | F | CN | H | H | Br | H | nonafluoro-2-butyl | H | I |
| 14-6799 | 2,2,2-trichloroethyl | Me | Me | F | CN | H | H | F | H | heptafluoroisopropyl | H | OCF3 |
| 14-6800 | 2,2,2-trichloroethyl | Me | Me | F | CN | H | H | F | H | nonafluoro-2-butyl | H | OCF3 |
| 14-6811 | 2,2,2-trichloroethyl | Me | Me | F | CN | H | H | Cl | H | heptafluoroisopropyl | H | OCF3 |
| 14-6840 | 2,2,2-trichloroethyl | Me | Me | F | CN | H | H | Cl | H | nonafluoro-2-butyl | H | OCF3 |
| 14-6869 | 2,2,2-trichloroethyl | Me | Me | F | CN | H | H | Br | H | heptafluoroisopropyl | H | OCF3 |
| 14-6898 | 2,2,2-trichloroethyl | Me | Me | F | CN | H | H | Br | H | nonafluoro-2-butyl | H | OCF3 |
| 14-6927 | 2,2,2-trichloroethyl | Me | Me | F | CN | H | H | I | H | heptafluoroisopropyl | H | OCF3 |
| 14-6956 | 2,2,2-trichloroethyl | Me | Me | F | CN | H | H | I | H | nonafluoro-2-butyl | H | OCF3 |
| 14-7171 | 2,2,2-trichloroethyl | Me | Me | F | CN | H | H | F | H | heptafluoroisopropyl | H | CF3 |
| 14-7172 | 2,2,2-trichloroethyl | Me | Me | F | CN | H | H | F | H | nonafluoro-2-butyl | H | CF3 |
| 14-7183 | 2,2,2-trichloroethyl | Me | Me | F | CN | H | H | Cl | H | heptafluoroisopropyl | H | CF3 |
| 14-7212 | 2,2,2-trichloroethyl | Me | Me | F | CN | H | H | Cl | H | nonafluoro-2-butyl | H | CF3 |
| 14-7241 | 2,2,2-trichloroethyl | Me | Me | F | CN | H | H | Br | H | heptafluoroisopropyl | H | CF3 |
| 14-7270 | 2,2,2-trichloroethyl | Me | Me | F | CN | H | H | Br | H | nonafluoro-2-butyl | H | CF3 |

**Table 14(9)**

| compound number | Q₂ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 14-7299 | 2,2,2-trichloroethyl | Me | Me | F | CN | H | H | I | H | heptafluoroisopropyl | H | CF3 |
| 14-7328 | 2,2,2-trichloroethyl | Me | Me | F | CN | H | H | I | H | nonafluoro-2-butyl | H | CF3 |
| 14-7553 | 2,2,2-trichloroethyl | Me | Me | F | CN | H | H | OCF3 | H | heptafluoroisopropyl | H | OCF3 |
| 14-7582 | 2,2,2-trichloroethyl | Me | Me | F | CN | H | H | OCF3 | H | nonafluoro-2-butyl | H | OCF3 |
| 14-7611 | 2,2,2-trichloroethyl | Me | Me | F | CN | H | H | CF3 | H | heptafluoroisopropyl | H | OCF3 |
| 14-7640 | 2,2,2-trichloroethyl | Me | Me | F | CN | H | H | CF3 | H | nonafluoro-2-butyl | H | OCF3 |
| 14-7669 | 2,2,2-trichloroethyl | Me | Me | F | CN | H | H | CF3 | H | heptafluoroisopropyl | H | CF3 |
| 14-7698 | 2,2,2-trichloroethyl | Me | Me | F | CN | H | H | CF3 | H | nonafluoro-2-butyl | H | CF3 |
| 14-7913 | 2,2,2-trichloroethyl | Me | Me | F | CN | F | H | F | H | heptafluoroisopropyl | H | F |
| 14-7914 | 2,2,2-trichloroethyl | Me | Me | F | CN | F | H | F | H | nonafluoro-2-butyl | H | F |
| 14-7925 | 2,2,2-trichloroethyl | Me | Me | F | CN | F | H | Cl | H | heptafluoroisopropyl | H | Cl |
| 14-7954 | 2,2,2-trichloroethyl | Me | Me | F | CN | F | H | Cl | H | nonafluoro-2-butyl | H | Cl |
| 14-7983 | 2,2,2-trichloroethyl | Me | Me | F | CN | F | H | Br | H | heptafluoroisopropyl | H | Br |
| 14-8012 | 2,2,2-trichloroethyl | Me | Me | F | CN | F | H | Br | H | nonafluoro-2-butyl | H | Br |
| 14-8041 | 2,2,2-trichloroethyl | Me | Me | F | CN | F | H | I | H | heptafluoroisopropyl | H | I |
| 14-8070 | 2,2,2-trichloroethyl | Me | Me | F | CN | F | H | I | H | nonafluoro-2-butyl | H | I |
| 13-4012 | 2,2,2-trichloroethyl | Me | Me | F | CN | F | H | Cl | H | heptafluoroisopropyl | H | Br |
| 14-8285 | 2,2,2-trichloroethyl | Me | Me | F | CN | F | H | F | H | heptafluoroisopropyl | H | OCF3 |
| 14-8286 | 2,2,2-trichloroethyl | Me | Me | F | CN | F | H | F | H | nonafluoro-2-butyl | H | OCF3 |
| 14-8297 | 2,2,2-trichloroethyl | Me | Me | F | CN | F | H | Cl | H | heptafluoroisopropyl | H | OCF3 |
| 14-8326 | 2,2,2-trichloroethyl | Me | Me | F | CN | F | H | Cl | H | nonafluoro-2-butyl | H | OCF3 |
| 14-8355 | 2,2,2-trichloroethyl | Me | Me | F | CN | F | H | Br | H | heptafluoroisopropyl | H | OCF3 |
| 14-8384 | 2,2,2-trichloroethyl | Me | Me | F | CN | F | H | Br | H | nonafluoro-2-butyl | H | OCF3 |
| 14-8413 | 2,2,2-trichloroethyl | Me | Me | F | CN | F | H | I | H | heptafluoroisopropyl | H | OCF3 |
| 14-8442 | 2,2,2-trichloroethyl | Me | Me | F | CN | F | H | I | H | nonafluoro-2-butyl | H | OCF3 |
| 14-8657 | 2,2,2-trichloroethyl | Me | Me | F | CN | F | H | F | H | heptafluoroisopropyl | H | CF3 |
| 14-8658 | 2,2,2-trichloroethyl | Me | Me | F | CN | F | H | F | H | nonafluoro-2-butyl | H | CF3 |
| 14-8669 | 2,2,2-trichloroethyl | Me | Me | F | CN | F | H | Cl | H | heptafluoroisopropyl | H | CF3 |
| 14-8698 | 2,2,2-trichloroethyl | Me | Me | F | CN | F | H | Cl | H | nonafluoro-2-butyl | H | CF3 |
| 14-8727 | 2,2,2-trichloroethyl | Me | Me | F | CN | F | H | Br | H | heptafluoroisopropyl | H | CF3 |
| 14-8756 | 2,2,2-trichloroethyl | Me | Me | F | CN | F | H | Br | H | nonafluoro-2-butyl | H | CF3 |
| 14-8785 | 2,2,2-trichloroethyl | Me | Me | F | CN | F | H | I | H | heptafluoroisopropyl | H | CF3 |
| 14-8814 | 2,2,2-trichloroethyl | Me | Me | F | CN | F | H | I | H | nonafluoro-2-butyl | H | CF3 |
| 1 4-9039 | 2,2,2-trichloroethyl | Me | Me | F | CN | F | H | OCF3 | H | heptafluoroisopropyl | H | OCF3 |
| 14-9068 | 2,2,2-trichloroethyl | Me | Me | F | CN | F | H | OCF3 | H | nonafluoro-2-butyl | H | OCF3 |
| 14-9097 | 2,2,2-trichloroethyl | Me | Me | F | CN | F | H | CF3 | H | heptafluoroisopropyl | H | OCF3 |
| 14-9126 | 2,2,2-trichloroethyl | Me | Me | F | CN | F | H | CF3 | H | nonafluoro-2-butyl | H | OCF3 |
| 14-9155 | 2,2,2-trichloroethyl | Me | Me | F | CN | F | H | CF3 | H | heptafluoroisopropyl | H | CF3 |
| 14-9184 | 2,2,2-trichloroethyl | Me | Me | F | CN | F | H | CF3 | H | nonafluoro-2-butyl | H | CF3 |

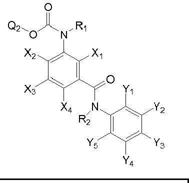

**Table 15(1)**

| compound number | Q₂ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 15-9 | 2,2-dichloroethyl | H | Et | H | H | H | H | Cl | F | heptafluoroisopropyl | F | OCF3 |
| 15-39 | 2,2,2-trifluoroethyl | H | H | H | H | H | H | Cl | Me | nonafluoro-2-butyl | Me | OCF3 |
| 15-144 | methyl | H | H | H | H | H | H | Cl | F | heptafluoroisopropyl | H | CF3 |
| 15-147 | 2-chloroethyl | Me | Me | H | H | H | H | Cl | H | heptafluoroisopropyl | Cl | CF3 |
| 15-148 | 2-bromoethyl | H | H | H | H | H | H | F | Br | heptafluoroisopropyl | H | CF3 |
| 15-153 | 2,2,2-trifluoroethyl | H | H | H | H | H | H | Cl | F | heptafluoroisopropyl | Cl | CF3 |
| 15-165 | 3,3,3-trifluoro-n-propyl | H | H | H | H | H | H | Cl | I | nonafluoro-2-butyl | Cl | CF3 |
| 15-168 | i-butyl | H | H | H | H | H | H | Cl | Me | nonafluoro-2-butyl | H | CF3 |
| 15-169 | s-butyl | H | H | H | H | H | H | Cl | H | nonafluoro-2-butyl | Me | CF3 |
| 15-170 | vinyl | i-Pr | H | H | H | H | H | Cl | Et | nonafluoro-2-butyl | H | CF3 |
| 15-171 | benzyl | H | H | H | H | H | H | Cl | H | nonafluoro-2-butyl | Et | CF3 |
| 15-182 | 2,2,2-trifluoroethyl | H | H | H | H | H | H | Cl | Me | nonafluoro-2-butyl | Me | n-C3F7 |
| 15-183 | 2,2,2-trichloroethyl | H | H | H | H | H | H | Cl | Me | nonafluoro-2-butyl | Et | i-C3F7 |
| 15-189 | i-propyl | H | H | H | H | H | H | Cl | Et | nonafluoro-2-butyl | Me | n-C3F7 |
| 15-190 | 1,2-difluoro-2-propyl | H | H | H | H | H | H | Cl | Et | nonafluoro-2-butyl | Et | i-C3F7 |
| 15-196 | 2-chloroethyl | H | H | H | H | H | H | Br | n-Pr | heptafluoroisopropyl | Me | CF3 |
| 15-197 | 2-bromoethyl | H | H | H | H | H | H | Br | n-Pr | heptafluoroisopropyl | Et | CF3 |
| 15-203 | 2,2,2-trichloroethyl | H | H | H | H | H | H | Br | i-Pr | heptafluoroisopropyl | Me | CF3 |
| 15-204 | 2,2,2-tribromoethyl | H | H | H | H | H | H | Br | i-Pr | heptafluoroisopropyl | Et | CF3 |
| 15-210 | 1,2-difluoro-2-propyl | H | H | H | H | H | H | Br | n-Bu | heptafluoroisopropyl | Me | CF3 |
| 15-211 | 1,3-difluoro-2-propyl | H | H | H | H | H | H | Br | n-Bu | heptafluoroisopropyl | Et | CF3 |
| 15-217 | i-butyl | H | H | H | H | H | H | Br | i-Bu | nonafluoro-2-butyl | Me | CF3 |
| 15-218 | s-butyl | H | H | H | H | H | H | Br | i-Bu | nonafluoro-2-butyl | Et | CF3 |
| 15-224 | 2-fluoroethyl | H | H | H | H | H | H | Br | s-Bu | nonafluoro-2-butyl | Me | CF3 |
| 15-225 | 2-chloroethyl | H | H | H | H | H | H | Br | s-Bu | nonafluoro-2-butyl | Et | CF3 |
| 15-231 | 2,2,2-trifluoroethyl | H | H | H | H | H | H | Br | F | nonafluoro-2-butyl | Me | CF3 |
| 15-232 | 2,2,2-trichloroethyl | H | H | H | H | H | H | Br | F | nonafluoro-2-butyl | Et | CF3 |
| 15-238 | i-propyl | Et | i-Pr | H | H | H | H | Br | Cl | nonafluoro-2-butyl | Me | CF3 |
| 15-239 | 1,2-difluoro-2-propyl | H | H | H | H | H | H | Br | Cl | nonafluoro-2-butyl | Et | CF3 |
| 15-245 | 2-iodoethyl | H | H | H | H | H | H | I | Br | heptafluoroisopropyl | Me | CF3 |
| 15-246 | 2-cyanoethyl | H | H | H | H | H | H | I | Br | heptafluoroisopropyl | Et | CF3 |
| 15-252 | n-propyl | n-Pr | Me | H | H | H | H | I | I | heptafluoroisopropyl | Me | CF3 |
| 15-253 | 3-fluoro-n-propyl | H | H | H | H | H | H | I | I | heptafluoroisopropyl | Et | CF3 |
| 15-259 | 1,3-dichloro-2-propyl | H | H | H | H | H | H | I | Me | heptafluoroisopropyl | F | C2F5 |
| 15-260 | 1-chloro-3-fluoro-2-propyl | H | H | H | H | H | H | I | Me | nonafluoro-2-butyl | Cl | CF3 |
| 15-263 | 4,4,4-trifluoro-n-butyl | H | H | H | H | H | H | I | Et | nonafluoro-2-butyl | F | CF3 |
| 15-264 | i-butyl | H | H | H | H | H | H | I | Et | nonafluoro-2-butyl | Cl | CF3 |
| 15-267 | benzyl | H | H | H | H | H | H | I | n-Pr | nonafluoro-2-butyl | F | CF3 |
| 15-268 | phenyl | H | H | H | H | H | H | I | n-Pr | nonafluoro-2-butyl | Cl | CF3 |
| 15-271 | 2-fluoroethyl | H | H | H | H | H | H | I | i-Pr | nonafluoro-2-butyl | F | i-C3F7 |
| 15-272 | 2-chloroethyl | H | H | H | H | H | H | I | i-Pr | nonafluoro-2-butyl | Cl | CF3 |
| 15-275 | 2-cyanoethyl | H | H | H | H | H | H | I | n-Bu | nonafluoro-2-butyl | F | CF3 |
| 15-276 | 2,2-difluoroethyl | H | H | H | H | H | H | I | n-Bu | nonafluoro-2-butyl | Cl | CF3 |
| 15-279 | 2,2,2-trichloroethyl | H | H | H | H | H | H | I | i-Bu | nonafluoro-2-butyl | F | CF3 |
| 15-280 | 2,2,2-tribromoethyl | H | H | H | H | H | H | I | i-Bu | nonafluoro-2-butyl | Cl | CF3 |
| 15-283 | 3-chloro-n-propyl | i-Pr | n-Pr | H | H | H | H | I | s-Bu | nonafluoro-2-butyl | F | CF3 |
| 15-284 | 3-bromo-n-propyl | H | H | H | H | H | H | I | s-Bu | nonafluoro-2-butyl | Cl | CF3 |
| 15-295 | 2,2-dichloroethyl | H | Et | H | H | H | H | OCF3 | F | heptafluoroisopropyl | F | OCF3 |
| 15-325 | 2,2,2-trifluoroethyl | H | H | H | H | H | H | OCF3 | Me | nonafluoro-2-butyl | Me | OCF3 |
| 15-438 | 2,2-dichloroethyl | H | Et | H | H | H | H | OCF3 | F | heptafluoroisopropyl | F | CF3 |
| 15-468 | 2,2,2-trifluoroethyl | H | H | H | H | H | H | OCF3 | Me | nonafluoro-2-butyl | Me | CF3 |
| 15-581 | 2,2-dichloroethyl | H | Et | H | H | H | H | CF3 | F | heptafluoroisopropyl | F | CF3 |
| 15-611 | 2,2,2-trifluoroethyl | H | H | H | H | H | H | CF3 | Me | nonafluoro-2-butyl | Me | CF3 |
| 15-734 | 2,2-dichloroethyl | H | Et | F | H | H | H | Cl | F | heptafluoroisopropyl | F | OCF3 |
| 15-764 | 2,2,2-trifluoroethyl | H | H | F | H | H | H | Cl | Me | nonafluoro-2-butyl | Me | OCF3 |

**Table 15(2)**

| compound number | Q₂ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 15-869 | methyl | H | H | F | H | H | H | Cl | F | heptafluoroisopropyl | H | CF3 |
| 15-870 | ethyl | Me | H | F | H | H | H | Cl | H | heptafluoroisopropyl | F | CF3 |
| 15-871 | 2-fluoroethyl | H | Me | F | H | H | H | Cl | Cl | heptafluoroisopropyl | H | CF3 |
| 15-872 | 2-chloroethyl | Me | Me | F | H | H | H | Cl | H | heptafluoroisopropyl | Cl | CF3 |
| 15-873 | 2-bromoethyl | H | H | F | H | H | H | F | Br | heptafluoroisopropyl | H | CF3 |
| 15-874 | 2-iodoethyl | H | H | F | H | H | H | Cl | H | heptafluoroisopropyl | Br | CF3 |
| 15-875 | 2-cyanoethyl | Et | H | F | H | H | H | Cl | I | heptafluoroisopropyl | H | C2F5 |
| 15-876 | 2,2-difluoroethyl | H | H | F | H | H | H | Cl | H | heptafluoroisopropyl | I | CF3 |
| 15-877 | 2,2-dichloroethyl | H | Et | F | H | H | H | Cl | F | heptafluoroisopropyl | F | CF3 |
| 15-878 | 2,2,2-trifluoroethyl | H | H | F | H | H | H | Cl | F | heptafluoroisopropyl | Cl | CF3 |
| 15-879 | 2,2,2-trichloroethyl | H | H | F | H | H | H | Cl | F | heptafluoroisopropyl | Br | CF3 |
| 15-880 | 2,2,2-tribromoethyl | Et | Et | F | H | H | H | Cl | F | pentafluoroethyl | I | C2F5 |
| 15-881 | n-propyl | H | H | F | H | H | H | Cl | Cl | pentafluoroethyl | F | CF3 |
| 15-882 | 3-fluoro-n-propyl | H | H | F | H | H | H | Cl | Cl | heptafluoroisopropyl | Cl | CF3 |
| 15-883 | 3-chloro-n-propyl | n-Pr | H | F | H | H | H | Cl | Cl | heptafluoroisopropyl | Br | CF3 |
| 15-884 | 3-bromo-n-propyl | H | H | F | H | H | H | Cl | Cl | heptafluoroisopropyl | I | n-C3F7 |
| 15-885 | i-propyl | H | n-Pr | F | H | H | H | Cl | Br | heptafluoroisopropyl | F | CF3 |
| 15-886 | 1,2-difluoro-2-propyl | H | H | F | H | H | H | Cl | Br | heptafluoroisopropyl | Cl | CF3 |
| 15-887 | 1,3-difluoro-2-propyl | H | H | F | H | H | H | Cl | Br | heptafluoroisopropyl | Br | CF3 |
| 15-888 | 1,3-dichloro-2-propyl | n-Pr | n-Pr | F | H | H | H | Cl | Br | pentafluoroethyl | I | CF3 |
| 15-889 | 1-chloro-3-fluoro-2-propyl | H | H | F | H | H | H | F | I | nonafluoro-2-butyl | F | CF3 |
| 15-890 | 3,3,3-trifluoro-n-propyl | H | H | F | H | H | H | Cl | I | nonafluoro-2-butyl | Cl | CF3 |
| 15-891 | n-butyl | H | H | F | H | H | H | Cl | I | nonafluoro-2-butyl | Br | CF3 |
| 15-892 | 4,4,4-trifluoro-n-butyl | H | H | F | H | H | H | Cl | I | nonafluoro-2-butyl | I | CF3 |
| 15-893 | i-butyl | H | H | F | H | H | H | Cl | Me | nonafluoro-2-butyl | H | CF3 |
| 15-894 | s-butyl | H | H | F | H | H | H | Cl | H | nonafluoro-2-butyl | Me | CF3 |
| 15-895 | vinyl | i-Pr | H | F | H | H | H | Cl | Et | nonafluoro-2-butyl | H | CF3 |
| 15-896 | benzyl | H | H | F | H | H | H | Cl | H | nonafluoro-2-butyl | Et | CF3 |
| 15-897 | phenyl | H | H | F | H | H | H | Cl | n-Pr | nonafluoro-2-butyl | H | CF3 |
| 15-898 | methyl | H | i-Pr | F | H | H | H | Cl | H | nonafluoro-2-butyl | n-Pr | CF3 |
| 15-899 | ethyl | i-Pr | i-Pr | F | H | H | H | Cl | i-Pr | nonafluoro-2-butyl | H | CF3 |
| 15-900 | 2-fluoroethyl | H | H | F | H | H | H | Cl | H | nonafluoro-2-butyl | i-Pr | CF3 |
| 15-901 | 2-chloroethyl | H | H | F | H | H | H | Cl | n-Bu | nonafluoro-2-butyl | H | CF3 |
| 15-902 | 2-bromoethyl | H | H | F | H | H | H | Cl | H | nonafluoro-2-butyl | n-Bu | CF3 |
| 15-903 | 2-iodoethyl | H | H | F | H | H | H | Cl | i-Bu | nonafluoro-2-butyl | H | CF3 |
| 15-904 | 2-cyanoethyl | H | H | F | H | H | H | Cl | H | nonafluoro-2-butyl | i-Bu | CF3 |
| 15-905 | 2,2-difluoroethyl | H | H | F | H | H | H | Cl | s-Bu | nonafluoro-2-butyl | H | C2F5 |
| 15-906 | 2,2-dichloroethyl | H | H | F | H | H | H | Cl | H | nonafluoro-2-butyl | s-Bu | CF3 |
| 15-907 | 2,2,2-trifluoroethyl | H | H | F | H | H | H | Cl | Me | nonafluoro-2-butyl | Me | n-C3F7 |
| 15-908 | 2,2,2-trichloroethyl | H | H | F | H | H | H | Cl | Me | nonafluoro-2-butyl | Et | i-C3F7 |
| 15-909 | 2,2,2-tribromoethyl | C(O)Me | H | F | H | H | H | Cl | Me | nonafluoro-2-butyl | n-Pr | CF3 |
| 15-910 | n-propyl | H | H | F | H | H | H | Cl | Me | nonafluoro-2-butyl | i-Pr | CF3 |
| 15-911 | 3-fluoro-n-propyl | H | H | F | H | H | H | Cl | Me | nonafluoro-2-butyl | n-Bu | CF3 |
| 15-912 | 3-chloro-n-propyl | H | H | F | H | H | H | Cl | Me | nonafluoro-2-butyl | i-Bu | CF3 |
| 15-913 | 3-bromo-n-propyl | H | C(O)Me | F | H | H | H | Cl | Me | nonafluoro-2-butyl | s-Bu | CF3 |
| 15-914 | i-propyl | H | H | F | H | H | H | Cl | Et | nonafluoro-2-butyl | Me | n-C3F7 |
| 15-915 | 1,2-difluoro-2-propyl | H | H | F | H | H | H | Cl | Et | nonafluoro-2-butyl | Et | i-C3F7 |
| 15-916 | 1,3-difluoro-2-propyl | C(O)Me | C(O)Me | F | H | H | H | Cl | Et | nonafluoro-2-butyl | n-Pr | CF3 |
| 15-917 | 1,3-dichloro-2-propyl | H | H | F | H | H | H | Cl | Et | nonafluoro-2-butyl | i-Pr | CF3 |
| 15-918 | methyl | H | H | F | H | H | H | Br | Et | heptafluoroisopropyl | n-Bu | CF3 |
| 15-919 | ethyl | H | H | F | H | H | H | Br | Et | heptafluoroisopropyl | i-Bu | CF3 |
| 15-920 | 2-fluoroethyl | H | H | F | H | H | H | Br | Et | heptafluoroisopropyl | s-Bu | CF3 |
| 15-921 | 2-chloroethyl | H | H | F | H | H | H | Br | n-Pr | heptafluoroisopropyl | Me | CF3 |
| 15-922 | 2-bromoethyl | H | H | F | H | H | H | Br | n-Pr | heptafluoroisopropyl | Et | CF3 |
| 15-923 | 2-iodoethyl | Me | Et | F | H | H | H | Br | n-Pr | pentafluoroethyl | n-Pr | CF3 |
| 15-924 | 2-cyanoethyl | H | H | F | H | H | H | Br | n-Pr | heptafluoroisopropyl | i-Pr | CF3 |
| 15-925 | 2,2-difluoroethyl | H | H | F | H | H | H | Br | n-Pr | pentafluoroethyl | n-Bu | CF3 |
| 15-926 | 2,2-dichloroethyl | H | H | F | H | H | H | Br | n-Pr | heptafluoroisopropyl | i-Bu | CF3 |
| 15-927 | 2,2,2-trifluoroethyl | H | H | F | H | H | H | Br | n-Pr | heptafluoroisopropyl | s-Bu | CF3 |
| 15-928 | 2,2,2-trichloroethyl | H | H | F | H | H | H | Br | i-Pr | heptafluoroisopropyl | Me | CF3 |
| 15-929 | 2,2,2-tribromoethyl | H | H | F | H | H | H | Br | i-Pr | heptafluoroisopropyl | Et | CF3 |
| 15-930 | n-propyl | Me | n-Pr | F | H | H | H | Br | i-Pr | heptafluoroisopropyl | n-Pr | CF3 |
| 15-931 | 3-fluoro-n-propyl | H | H | F | H | H | H | Br | i-Pr | heptafluoroisopropyl | i-Pr | CF3 |
| 15-932 | 3-chloro-n-propyl | H | H | F | H | H | H | Br | i-Pr | heptafluoroisopropyl | n-Bu | CF3 |

**Table 15(3)**

| compound number | Q₂ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 15-933 | 3-bromo-n-propyl | H | H | F | H | H | H | Br | i-Pr | heptafluoroisopropyl | i-Bu | CF3 |
| 15-934 | i-propyl | Me | i-Pr | F | H | H | H | Br | i-Pr | heptafluoroisopropyl | s-Bu | CF3 |
| 15-935 | 1.2-difluoro-2-propyl | H | H | F | H | H | H | Br | n-Bu | heptafluoroisopropyl | Me | CF3 |
| 15-936 | 1,3-difluoro-2-propyl | H | H | F | H | H | H | Br | n-Bu | heptafluoroisopropyl | Et | CF3 |
| 15-937 | 1,3-dichloro-2-propyl | H | H | F | H | H | H | Br | n-Bu | heptafluoroisopropyl | n-Pr | CF3 |
| 15-938 | 1-chloro-3-fluoro-2-propyl | H | H | F | H | H | H | Br | n-Bu | nonafluoro-2-butyl | i-Pr | CF3 |
| 15-939 | 3,3,3-trifluoro-n-propyl | H | H | F | H | H | H | Br | n-Bu | nonafluoro-2-butyl | n-Bu | CF3 |
| 15-940 | n-butyl | H | H | F | H | H | H | Br | n-Bu | nonafluoro-2-butyl | i-Bu | CF3 |
| 15-941 | 4,4,4-trifluoro-n-butyl | Me | C(O)Me | F | H | H | H | Br | n-Bu | nonafluoro-2-butyl | s-Bu | CF3 |
| 15-942 | i-butyl | H | H | F | H | H | H | Br | i-Bu | nonafluoro-2-butyl | Me | CF3 |
| 15-943 | s-butyl | H | H | F | H | H | H | Br | i-Bu | nonafluoro-2-butyl | Et | CF3 |
| 15-944 | vinyl | H | H | F | H | H | H | Br | i-Bu | nonafluoro-2-butyl | n-Pr | CF3 |
| 15-945 | benzyl | H | H | F | H | H | H | Br | i-Bu | nonafluoro-2-butyl | i-Pr | CF3 |
| 15-946 | phenyl | H | H | F | H | H | H | Br | i-Bu | nonafluoro-2-butyl | n-Bu | CF3 |
| 15-947 | methyl | H | H | F | H | H | H | Br | i-Bu | nonafluoro-2-butyl | i-Bu | CF3 |
| 15-948 | ethyl | H | H | F | H | H | H | Br | i-Bu | nonafluoro-2-butyl | s-Bu | C2F5 |
| 15-949 | 2-fluoroethyl | H | H | F | H | H | H | Br | s-Bu | nonafluoro-2-butyl | Me | CF3 |
| 15-950 | 2-chloroethyl | H | H | F | H | H | H | Br | s-Bu | nonafluoro-2-butyl | Et | CF3 |
| 15-951 | 2-bromoethyl | H | H | F | H | H | H | Br | s-Bu | nonafluoro-2-butyl | n-Pr | CF3 |
| 15-952 | 2-iodoethyl | H | H | F | H | H | H | Br | s-Bu | nonafluoro-2-butyl | i-Pr | CF3 |
| 15-953 | 2-cyanoethyl | H | H | F | H | H | H | Br | s-Bu | nonafluoro-2-butyl | n-Bu | CF3 |
| 15-954 | 2,2-difluoroethyl | H | H | F | H | H | H | Br | s-Bu | nonafluoro-2-butyl | i-Bu | CF3 |
| 15-955 | 2,2-dichloroethyl | Et | Me | F | H | H | H | Br | s-Bu | nonafluoro-2-butyl | s-Bu | CF3 |
| 15-956 | 2,2,2-trifluoroethyl | H | H | F | H | H | H | Br | F | nonafluoro-2-butyl | Me | CF3 |
| 15-957 | 2,2,2-trichloroethyl | H | H | F | H | H | H | Br | F | nonafluoro-2-butyl | Et | CF3 |
| 15-958 | 2,2,2-tribromoethyl | H | H | F | H | H | H | Br | F | nonafluoro-2-butyl | n-Pr | CF3 |
| 15-959 | n-propyl | H | H | F | H | H | H | Br | F | nonafluoro-2-butyl | i-Pr | CF3 |
| 15-960 | 3-fluoro-n-propyl | H | H | F | H | H | H | Br | F | nonafluoro-2-butyl | n-Bu | CF3 |
| 15-961 | 3-chloro-n-propyl | H | H | F | H | H | H | Br | F | nonafluoro-2-butyl | i-Bu | CF3 |
| 15-962 | 3-bromo-n-propyl | Et | n-Pr | F | H | H | H | Br | F | nonafluoro-2-butyl | s-Bu | CF3 |
| 15-963 | i-propyl | Et | i-Pr | F | H | H | H | Br | Cl | nonafluoro-2-butyl | Me | CF3 |
| 15-964 | 1,2-difluoro-2-propyl | H | H | F | H | H | H | Br | Cl | nonafluoro-2-butyl | Et | CF3 |
| 15-965 | methyl | H | H | F | H | H | H | I | Cl | pentafluoroethyl | n-Pr | i-C3F7 |
| 15-966 | ethyl | H | H | F | H | H | H | I | Cl | heptafluoroisopropyl | i-Pr | CF3 |
| 15-967 | 2-fluoroethyl | H | H | F | H | H | H | I | Cl | heptafluoroisopropyl | n-Bu | CF3 |
| 15-968 | 2-chloroethyl | Et | C(O)Me | F | H | H | H | I | Cl | heptafluoroisopropyl | i-Bu | CF3 |
| 15-969 | 2-bromoethyl | H | H | F | H | H | H | I | Cl | heptafluoroisopropyl | s-Bu | CF3 |
| 15-970 | 2-iodoethyl | H | H | F | H | H | H | I | Br | heptafluoroisopropyl | Me | CF3 |
| 15-971 | 2-cyanoethyl | H | H | F | H | H | H | I | Br | heptafluoroisopropyl | Et | CF3 |
| 15-972 | 2,2-difluoroethyl | H | H | F | H | H | H | I | Br | pentafluoroethyl | n-Pr | CF3 |
| 15-973 | 2,2-dichloroethyl | H | H | F | H | H | H | I | Br | heptafluoroisopropyl | i-Pr | CF3 |
| 15-974 | 2,2,2-trifluoroethyl | H | H | F | H | H | H | I | Br | heptafluoroisopropyl | n-Bu | CF3 |
| 15-975 | 2,2,2-trichloroethyl | H | H | F | H | H | H | I | Br | heptafluoroisopropyl | i-Bu | CF3 |
| 15-976 | 2,2,2-tribromoethyl | H | H | F | H | H | H | I | Br | heptafluoroisopropyl | s-Bu | CF3 |
| 15-977 | n-propyl | n-Pr | Me | F | H | H | H | I | I | heptafluoroisopropyl | Me | CF3 |
| 15-978 | 3-fluoro-n-propyl | H | H | F | H | H | H | I | I | heptafluoroisopropyl | Et | CF3 |
| 15-979 | 3-chloro-n-propyl | n-Pr | Et | F | H | H | H | I | I | heptafluoroisopropyl | n-Pr | CF3 |
| 15-980 | 3-bromo-n-propyl | H | H | F | H | H | H | I | I | heptafluoroisopropyl | i-Pr | CF3 |
| 15-981 | i-propyl | H | H | F | H | H | H | I | I | heptafluoroisopropyl | n-Bu | CF3 |
| 15-982 | 1.2-difluoro-2-propyl | H | H | F | H | H | H | I | I | heptafluoroisopropyl | i-Bu | CF3 |
| 15-983 | 1,3-difluoro-2-propyl | H | H | F | H | H | H | I | I | heptafluoroisopropyl | s-Bu | CF3 |
| 15-984 | 1,3-dichloro-2-propyl | H | H | F | H | H | H | I | Me | heptafluoroisopropyl | F | C2F5 |
| 15-985 | 1-chloro-3-fluoro-2-propyl | H | H | F | H | H | H | I | Me | nonafluoro-2-butyl | Cl | CF3 |
| 15-986 | 3,3,3-trifluoro-n-propyl | H | H | F | H | H | H | I | Me | nonafluoro-2-butyl | Br | n-C3F7 |
| 15-987 | n-butyl | H | H | F | H | H | H | I | Me | nonafluoro-2-butyl | I | i-C3F7 |
| 15-988 | 4,4,4-trifluo ro-n-butyl | H | H | F | H | H | H | I | Et | nonafluoro-2-butyl | F | CF3 |
| 15-989 | i-butyl | H | H | F | H | H | H | I | Et | nonafluoro-2-butyl | Cl | CF3 |
| 15-990 | s-butyl | H | H | F | H | H | H | I | Et | nonafluoro-2-butyl | Br | CF3 |
| 15-991 | vinyl | n-Pr | i-Pr | F | H | H | H | I | Et | nonafluoro-2-butyl | I | C2F5 |
| 15-992 | benzyl | H | H | F | H | H | H | I | n-Pr | nonafluoro-2-butyl | F | CF3 |
| 15-993 | phenyl | H | H | F | H | H | H | I | n-Pr | nonafluoro-2-butyl | Cl | CF3 |
| 15-994 | methyl | H | H | F | H | H | H | I | n-Pr | nonafluoro-2-butyl | Br | CF3 |
| 15-995 | ethyl | H | H | F | H | H | H | I | n-Pr | nonafluoro-2-butyl | I | n-C3F7 |
| 15-996 | 2-fluoroethyl | H | H | F | H | H | H | I | i-Pr | nonafluoro-2-butyl | F | i-C3F7 |

**Table 15(4)**

| compound number | Q₂ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 15-997 | 2-chloroethyl | H | H | F | H | H | H | I | i-Pr | nonafluoro-2-butyl | Cl | CF3 |
| 15-998 | 2-bromoethyl | n-Pr | C(O)Me | F | H | H | H | I | i-Pr | nonafluoro-2-butyl | Br | CF3 |
| 15-999 | 2-iodoethyl | H | H | F | H | H | H | I | i-Pr | nonafluoro-2-butyl | I | CF3 |
| 15-1000 | 2-cyanoethyl | H | H | F | H | H | H | I | n-Bu | nonafluoro-2-butyl | F | CF3 |
| 15-1001 | 2,2-difluoroethyl | H | H | F | H | H | H | I | n-Bu | nonafluoro-2-butyl | Cl | CF3 |
| 15-1002 | 2.2-dichloroethyl | H | H | F | H | H | H | I | n-Bu | nonafluoro-2-butyl | Br | CF3 |
| 15-1003 | 2,2,2-trifluoroethyl | i-Pr | Me | F | H | H | H | I | n-Bu | nonafluoro-2-butyl | I | CF3 |
| 15-1004 | 2,2,2-trichloroethyl | H | H | F | H | H | H | I | i-Bu | nonafluoro-2-butyl | F | CF3 |
| 15-1005 | 2,2,2-tribromoethyl | H | H | F | H | H | H | I | i-Bu | nonafluoro-2-butyl | Cl | CF3 |
| 15-1006 | n-propyl | H | H | F | H | H | H | I | i-Bu | nonafluoro-2-butyl | Br | CF3 |
| 15-1007 | 3-fluoro-n-propyl | i-Pr | Et | F | H | H | H | I | i-Bu | nonafluoro-2-butyl | I | CF3 |
| 15-1008 | 3-chloro-n-propyl | i-Pr | n-Pr | F | H | H | H | I | s-Bu | nonafluoro-2-butyl | F | CF3 |
| 15-1009 | 3-bromo-n-propyl | H | H | F | H | H | H | I | s-Bu | nonafluoro-2-butyl | Cl | CF3 |
| 15-1010 | i-propyl | i-Pr | C(O)Me | F | H | H | H | I | s-Bu | nonafluoro-2-butyl | Br | CF3 |
| 15-1011 | 1,2-difluoro-2-propyl | H | H | F | H | H | H | I | s-Bu | nonafluoro-2-butyl | I | CF3 |
| 15-1020 | 2,2-dichloroethyl | H | Et | F | H | H | H | OCF3 | F | heptafluoroisopropyl | F | OCF3 |
| 15-1050 | 2,2,2-trifluoroethyl | H | H | F | H | H | H | OCF3 | Me | nonafluoro-2-butyl | Me | OCF3 |
| 15-1163 | 2,2-dichloroethyl | H | Et | F | H | H | H | OCF3 | F | heptafluoroisopropyl | F | CF3 |
| 15-1193 | 2,2,2-trifluoroethyl | H | H | F | H | H | H | OCF3 | Me | nonafluoro-2-butyl | Me | CF3 |
| 15-1306 | 2,2-dichloroethyl | H | Et | F | H | H | H | CF3 | F | heptafluoroisopropyl | F | CF3 |
| 15-1336 | 2,2,2-trifluoroethyl | H | H | F | H | H | H | CF3 | Me | nonafluoro-2-butyl | Me | CF3 |
| 15-1449 | 2,2-dichloroethyl | H | Et | H | F | H | H | Cl | F | heptafluoroisopropyl | F | Cl |
| 15-1479 | 2,2,2-trifluoroethyl | H | H | H | F | H | H | Cl | Me | nonafluoro-2-butyl | Me | I |
| 15-1592 | 2,2-dichloroethyl | H | Et | H | F | H | H | Cl | F | heptafluoroisopropyl | F | OCF3 |
| 15-1622 | 2,2,2-trifluoroethyl | H | H | H | F | H | H | Cl | Me | nonafluoro-2-butyl | Me | OCF3 |
| 15-1737 | 2,2,2-trichloroethyl | H | H | H | F | H | H | Cl | F | heptafluoroisopropyl | Br | CF3 |
| 15-1749 | n-butyl | H | H | H | F | H | H | Cl | I | nonafluoro-2-butyl | Br | CF3 |
| 15-1766 | 2,2,2-trichloroethyl | H | H | H | F | H | H | Cl | Me | nonafluoro-2-butyl | Et | i-C3F7 |
| 15-1772 | i-propyl | H | H | H | F | H | H | Cl | Et | nonafluoro-2-butyl | Me | n-C3F7 |
| 15-1773 | 1,2-difluoro-2-propyl | H | H | H | F | H | H | Cl | Et | nonafluoro-2-butyl | Et | i-C3F7 |
| 15-1779 | 2-chloroethyl | H | H | H | F | H | H | Br | n-Pr | heptafluoroisopropyl | Me | CF3 |
| 15-1780 | 2-bromoethyl | H | H | H | F | H | H | Br | n-Pr | heptafluoroisopropyl | Et | CF3 |
| 15-1786 | 2,2,2-trichloroethyl | H | H | H | F | H | H | Br | i-Pr | heptafluoroisopropyl | Me | CF3 |
| 15-1787 | 2,2,2-tribromoethyl | H | H | H | F | H | H | Br | i-Pr | heptafluoroisopropyl | Et | CF3 |
| 15-1793 | 1,2-difluoro-2-propyl | H | H | H | F | H | H | Br | n-Bu | heptafluoroisopropyl | Me | CF3 |
| 15-1794 | 1,3-difluoro-2-propyl | H | H | H | F | H | H | Br | n-Bu | heptafluoroisopropyl | Et | CF3 |
| 15-1800 | i-butyl | H | H | H | F | H | H | Br | i-Bu | nonafluoro-2-butyl | Me | CF3 |
| 15-1801 | s-butyl | H | H | H | F | H | H | Br | i-Bu | nonafluoro-2-butyl | Et | CF3 |
| 15-1807 | 2-fluoroethyl | H | H | H | F | H | H | Br | s-Bu | nonafluoro-2-butyl | Me | CF3 |
| 15-1808 | 2-chloroethyl | H | H | H | F | H | H | Br | s-Bu | nonafluoro-2-butyl | Et | CF3 |
| 15-1814 | 2,2,2-trifluoroethyl | H | H | H | F | H | H | Br | F | nonafluoro-2-butyl | Me | CF3 |
| 15-1815 | 2,2,2-trichloroethyl | H | H | H | F | H | H | Br | F | nonafluoro-2-butyl | Et | CF3 |
| 15-1821 | i-propyl | Et | i-Pr | H | F | H | H | Br | Cl | nonafluoro-2-butyl | Me | CF3 |
| 15-1822 | 1,2-difluoro-2-propyl | H | H | H | F | H | H | Br | Cl | nonafluoro-2-butyl | Et | CF3 |
| 15-1828 | 2-iodoethyl | H | H | H | F | H | H | I | Br | heptafluoroisopropyl | Me | CF3 |
| 15-1829 | 2-cyanoethyl | H | H | H | F | H | H | I | Br | heptafluoroisopropyl | Et | CF3 |
| 15-1835 | n-propyl | n-Pr | Me | H | F | H | H | I | I | heptafluoroisopropyl | Me | CF3 |
| 15-1836 | 3-fluoro-n-propyl | H | H | H | F | H | H | I | I | heptafluoroisopropyl | Et | CF3 |
| 15-1842 | 1,3-dichloro-2-propyl | H | H | H | F | H | H | I | Me | heptafluoroisopropyl | F | C2F5 |
| 15-1843 | 1-chloro-3-fluoro-2-propyl | H | H | H | F | H | H | I | Me | nonafluoro-2-butyl | Cl | CF3 |
| 15-1846 | 4,4,4-trifluoro-n-butyl | H | H | H | F | H | H | I | Et | nonafluoro-2-butyl | F | CF3 |
| 15-1847 | i-butyl | H | H | H | F | H | H | I | Et | nonafluoro-2-butyl | Cl | CF3 |
| 15-1850 | benzyl | H | H | H | F | H | H | I | n-Pr | nonafluoro-2-butyl | F | CF3 |
| 15-1851 | phenyl | H | H | H | F | H | H | I | n-Pr | nonafluoro-2-butyl | Cl | CF3 |
| 15-1854 | 2-fluoroethyl | H | H | H | F | H | H | I | i-Pr | nonafluoro-2-butyl | F | i-C3F7 |
| 15-1855 | 2-chloroethyl | H | H | H | F | H | H | I | i-Pr | nonafluoro-2-butyl | Cl | CF3 |
| 15-1858 | 2-cyanoethyl | H | H | H | F | H | H | I | n-Bu | nonafluoro-2-butyl | F | CF3 |
| 15-1859 | 2,2-difluoroethyl | H | H | H | F | H | H | I | n-Bu | nonafluoro-2-butyl | Cl | CF3 |
| 15-1862 | 2,2,2-trichloroethyl | H | H | H | F | H | H | I | i-Bu | nonafluoro-2-butyl | F | CF3 |
| 15-1863 | 2,2,2-tribromoethyl | H | H | H | F | H | H | I | i-Bu | nonafluoro-2-butyl | Cl | CF3 |
| 15-1866 | 3-chloro-n-propyl | i-Pr | n-Pr | H | F | H | H | I | s-Bu | nonafluoro-2-butyl | F | CF3 |
| 15-1867 | 3-bromo-n-propyl | H | H | H | F | H | H | I | s-Bu | nonafluoro-2-butyl | Cl | CF3 |
| 15-1878 | 2,2-dichloroethyl | H | Et | H | F | H | H | OCF3 | F | heptafluoroisopropyl | F | OCF3 |
| 15-1908 | 2,2,2-trifluoroethyl | H | H | H | F | H | H | OCF3 | Me | nonafluoro-2-butyl | Me | OCF3 |

**Table 15(5)**

| compound number | Q₂ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 15-2021 | 2,2-dichloroethyl | H | Et | H | F | H | H | OCF3 | F | heptafluoroisopropyl | F | CF3 |
| 15-2051 | 2,2,2-trifluoroethyl | H | H | H | F | H | H | OCF3 | Me | nonafluoro-2-butyl | Me | CF3 |
| 15-2164 | 2,2-dichloroethyl | H | Et | H | F | H | H | CF3 | F | heptafluoroisopropyl | F | CF3 |
| 15-2194 | 2,2,2-trifluoroethyl | H | H | H | F | H | H | CF3 | Me | nonafluoro-2-butyl | Me | CF3 |
| 15-2307 | 2,2-dichloroethyl | H | Et | F | F | H | H | Cl | F | heptafluoroisopropyl | F | Cl |
| 15-2337 | 2,2,2-trifluoroethyl | H | H | F | F | H | H | Cl | Me | nonafluoro-2-butyl | Me | I |
| 15-2450 | 2,2-dichloroethyl | H | Et | F | F | H | H | Cl | F | heptafluoroisopropyl | F | OCF3 |
| 15-2480 | 2,2,2-trifluoroethyl | H | H | F | F | H | H | Cl | Me | nonafluoro-2-butyl | Me | OCF3 |
| 15-2585 | methyl | H | H | F | F | H | H | Cl | F | heptafluoroisopropyl | H | CF3 |
| 15-2591 | 2-cyanoethyl | Et | H | F | F | H | H | Cl | I | heptafluoroisopropyl | H | C2F5 |
| 15-2594 | 2,2,2-trifluoroethyl | H | H | F | F | H | H | Cl | F | heptafluoroisopropyl | Cl | CF3 |
| 15-2602 | 1,2-difluoro-2-propyl | H | H | F | F | H | H | Cl | Br | heptafluoroisopropyl | Cl | CF3 |
| 15-2609 | i-butyl | H | H | F | F | H | H | Cl | Me | nonafluoro-2-butyl | H | CF3 |
| 15-2610 | s-butyl | H | H | F | F | H | H | Cl | H | nonafluoro-2-butyl | Me | CF3 |
| 15-2611 | vinyl | i-Pr | H | F | F | H | H | Cl | Et | nonafluoro-2-butyl | H | CF3 |
| 15-2612 | benzyl | H | H | F | F | H | H | Cl | H | nonafluoro-2-butyl | Et | CF3 |
| 15-2623 | 2,2,2-trifluoroethyl | H | H | F | F | H | H | Cl | Me | nonafluoro-2-butyl | Me | n-C3F7 |
| 15-2624 | 2,2,2-trichloroethyl | H | H | F | F | H | H | Cl | Me | nonafluoro-2-butyl | Et | i-C3F7 |
| 15-2630 | i-propyl | H | H | F | F | H | H | Cl | Et | nonafluoro-2-butyl | Me | n-C3F7 |
| 15-2631 | 1,2-difluoro-2-propyl | H | H | F | F | H | H | Cl | Et | nonafluoro-2-butyl | Et | i-C3F7 |
| 15-2637 | 2-chloroethyl | H | H | F | F | H | H | Br | n-Pr | heptafluoroisopropyl | Me | CF3 |
| 15-2638 | 2-bromoethyl | H | H | F | F | H | H | Br | n-Pr | heptafluoroisopropyl | Et | CF3 |
| 15-2644 | 2,2,2-trichloroethyl | H | H | F | F | H | H | Br | i-Pr | heptafluoroisopropyl | Me | CF3 |
| 15-2645 | 2,2,2-tribromoethyl | H | H | F | F | H | H | Br | i-Pr | heptafluoroisopropyl | Et | CF3 |
| 15-2651 | 1.2-difluoro-2-propyl | H | H | F | F | H | H | Br | n-Bu | heptafluoroisopropyl | Me | CF3 |
| 15-2652 | 1,3-difluoro-2-propyl | H | H | F | F | H | H | Br | n-Bu | heptafluoroisopropyl | Et | CF3 |
| 15-2658 | i-butyl | H | H | F | F | H | H | Br | i-Bu | nonafluoro-2-butyl | Me | CF3 |
| 15-2659 | s-butyl | H | H | F | F | H | H | Br | i-Bu | nonafluoro-2-butyl | Et | CF3 |
| 15-2665 | 2-fluoroethyl | H | H | F | F | H | H | Br | s-Bu | nonafluoro-2-butyl | Me | CF3 |
| 15-2666 | 2-chloroethyl | H | H | F | F | H | H | Br | s-Bu | nonafluoro-2-butyl | Et | CF3 |
| 15-2672 | 2,2,2-trifluoroethyl | H | H | F | F | H | H | Br | F | nonafluoro-2-butyl | Me | CF3 |
| 15-2673 | 2,2,2-trichloroethyl | H | H | F | F | H | H | Br | F | nonafluoro-2-butyl | Et | CF3 |
| 15-2679 | i-propyl | Et | i-Pr | F | F | H | H | Br | Cl | nonafluoro-2-butyl | Me | CF3 |
| 15-2680 | 1.2-difluoro-2-propyl | H | H | F | F | H | H | Br | Cl | nonafluoro-2-butyl | Et | CF3 |
| 15-2686 | 2-iodoethyl | H | H | F | F | H | H | I | Br | heptafluoroisopropyl | Me | CF3 |
| 15-2687 | 2-cyanoethyl | H | H | F | F | H | H | I | Br | heptafluoroisopropyl | Et | CF3 |
| 15-2693 | n-propyl | n-Pr | Me | F | F | H | H | I | I | heptafluoroisopropyl | Me | CF3 |
| 15-2694 | 3-fluoro-n-propyl | H | H | F | F | H | H | I | I | heptafluoroisopropyl | Et | CF3 |
| 15-2700 | 1,3-dichloro-2-propyl | H | H | F | F | H | H | I | Me | heptafluoroisopropyl | F | C2F5 |
| 15-2701 | 1-chloro-3-fluoro-2-propyl | H | H | F | F | H | H | I | Me | nonafluoro-2-butyl | Cl | CF3 |
| 15-2704 | 4,4,4-trifluoro-n-butyl | H | H | F | F | H | H | I | Et | nonafluoro-2-butyl | F | CF3 |
| 15-2705 | i-butyl | H | H | F | F | H | H | I | Et | nonafluoro-2-butyl | Cl | CF3 |
| 15-2708 | benzyl | H | H | F | F | H | H | I | n-Pr | nonafluoro-2-butyl | F | CF3 |
| 15-2709 | phenyl | H | H | F | F | H | H | I | n-Pr | nonafluoro-2-butyl | Cl | CF3 |
| 15-2712 | 2-fluoroethyl | H | H | F | F | H | H | I | i-Pr | nonafluoro-2-butyl | F | i-C3F7 |
| 15-2713 | 2-chloroethyl | H | H | F | F | H | H | I | i-Pr | nonafluoro-2-butyl | Cl | CF3 |
| 15-2716 | 2-cyanoethyl | H | H | F | F | H | H | I | n-Bu | nonafluoro-2-butyl | F | CF3 |
| 15-2717 | 2,2-difluoroethyl | H | H | F | F | H | H | I | n-Bu | nonafluoro-2-butyl | Cl | CF3 |
| 15-2720 | 2,2,2-trichloroethyl | H | H | F | F | H | H | I | i-Bu | nonafluoro-2-butyl | F | CF3 |
| 15-2721 | 2,2,2-tribromoethyl | H | H | F | F | H | H | I | i-Bu | nonafluoro-2-butyl | Cl | CF3 |
| 15-2724 | 3-chloro-n-propyl | i-Pr | n-Pr | F | F | H | H | I | s-Bu | nonafluoro-2-butyl | F | CF3 |
| 15-2725 | 3-bromo-n-propyl | H | H | F | F | H | H | I | s-Bu | nonafluoro-2-butyl | Cl | CF3 |
| 15-2736 | 2,2-dichloroethyl | H | Et | F | F | H | H | OCF3 | F | heptafluoroisopropyl | F | OCF3 |
| 15-2766 | 2,2,2-trifluoroethyl | H | H | F | F | H | H | OCF3 | Me | nonafluoro-2-butyl | Me | OCF3 |
| 15-2879 | 2,2-dichloroethyl | H | Et | F | F | H | H | OCF3 | F | heptafluoroisopropyl | F | CF3 |
| 15-2909 | 2,2,2-trifluoroethyl | H | H | F | F | H | H | OCF3 | Me | nonafluoro-2-butyl | Me | CF3 |
| 15-3022 | 2,2-dichloroethyl | H | Et | F | F | H | H | CF3 | F | heptafluoroisopropyl | F | CF3 |
| 15-3052 | 2,2,2-trifluoroethyl | H | H | F | F | H | H | CF3 | Me | nonafluoro-2-butyl | Me | CF3 |
| 15-3157 | 2,2,2-trichloroethyl | H | H | F | F | F | H | Cl | F | pentafluoroethyl | H | Cl |
| 15-3158 | 2,2,2-trichloroethyl | Me | H | F | F | F | H | Cl | H | heptafluoroisopropyl | F | Cl |
| 15-3159 | 2,2,2-trichloroethyl | H | Me | F | F | F | H | Br | Cl | heptafluoroisopropyl | H | Br |
| 15-3160 | 2,2,2-trichloroethyl | Me | Me | F | F | F | H | I | H | heptafluoroisopropyl | Cl | I |
| 15-3161 | 2,2,2-trichloroethyl | H | H | F | F | F | H | Cl | Br | heptafluoroisopropyl | H | OCF3 |
| 15-3162 | 2,2,2-trichloroethyl | H | H | F | F | F | H | Br | H | heotafluoroisopropyl | Br | OCF3 |

**Table 15(6)**

| compound number | Q₂ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 15-3163 | 2,2,2-trichloroethyl | Et | H | F | F | F | H | I | I | heptafluoroisopropyl | H | OCF3 |
| 15-3164 | 2,2,2-trichloroethyl | H | H | F | F | F | H | Cl | H | heptafluoroisopropyl | I | CF3 |
| 15-3165 | 2,2,2-trichloroethyl | H | Et | F | F | F | H | Br | F | heptafluoroisopropyl | F | CF3 |
| 15-3166 | 2,2,2-trichloroethyl | H | H | F | F | F | H | I | F | heptafluoroisopropyl | Cl | CF3 |
| 15-3167 | 2,2,2-trichloroethyl | H | H | F | F | F | H | CF3 | F | heptafluoroisopropyl | Br | CF3 |
| 15-3168 | 2,2,2-trichloroethyl | Et | Et | F | F | F | H | Cl | F | nonafluoro-2-butyl | I | Cl |
| 15-3169 | 2,2,2-trichloroethyl | H | H | F | F | F | H | Br | Cl | nonafluoro-2-butyl | F | Br |
| 15-3170 | 2,2,2-trichloroethyl | H | H | F | F | F | H | I | Cl | nonafluoro-2-butyl | Cl | I |
| 15-3171 | 2,2,2-trichloroethyl | n-Pr | H | F | F | F | H | Cl | Cl | nonafluoro-2-butyl | Br | OCF3 |
| 15-3172 | 2,2,2-trichloroethyl | H | H | F | F | F | H | Br | Cl | nonafluoro-2-butyl | I | OCF3 |
| 15-3173 | 2,2,2-trichloroethyl | H | n-Pr | F | F | F | H | I | Br | nonafluoro-2-butyl | F | OCF3 |
| 15-3174 | 2,2,2-trichloroethyl | H | H | F | F | F | H | Cl | Br | nonafluoro-2-butyl | Cl | CF3 |
| 15-3175 | 2,2,2-trichloroethyl | H | H | F | F | F | H | Br | Br | nonafluoro-2-butyl | Br | CF3 |
| 15-3176 | 2,2,2-trichloroethyl | n-Pr | n-Pr | F | F | F | H | I | Br | nonafluoro-2-butyl | I | CF3 |
| 15-3177 | 2,2,2-trichloroethyl | H | H | F | F | F | H | CF3 | I | nonafluoro-2-butyl | F | CF3 |
| 15-3178 | 2,2,2-trichloroethyl | H | H | H | H | F | H | Cl | I | pentafluoroethyl | Cl | Cl |
| 15-3179 | 2,2,2-trichloroethyl | H | H | H | H | F | H | Cl | I | heptafluoroisopropyl | Br | Cl |
| 15-3180 | 2,2,2-trichloroethyl | H | H | H | H | F | H | Br | I | heptafluoroisopropyl | I | Br |
| 15-3181 | 2,2,2-trichloroethyl | H | H | H | H | F | H | I | Me | heptafluoroisopropyl | H | I |
| 15-3182 | 2,2,2-trichloroethyl | H | H | H | H | F | H | Cl | H | heptafluoroisopropyl | Me | OCF3 |
| 15-3183 | 2,2,2-trichloroethyl | i-Pr | H | H | H | F | H | Br | Et | heptafluoroisopropyl | H | OCF3 |
| 15-3184 | 2,2,2-trichloroethyl | H | H | H | H | F | H | I | H | heptafluoroisopropyl | Et | OCF3 |
| 15-3185 | 2,2,2-trichloroethyl | H | H | H | H | F | H | Cl | n-Pr | heptafluoroisopropyl | H | CF3 |
| 15-3186 | 2,2,2-trichloroethyl | H | i-Pr | H | H | F | H | Br | H | heptafluoroisopropyl | n-Pr | CF3 |
| 15-3187 | 2,2,2-trichloroethyl | i-Pr | i-Pr | H | H | F | H | I | i-Pr | heptafluoroisopropyl | H | CF3 |
| 15-3188 | 2,2,2-trichloroethyl | H | H | H | H | F | H | CF3 | H | heptafluoroisopropyl | i-Pr | CF3 |
| 15-3189 | 2,2,2-trichloroethyl | H | H | H | H | F | H | Cl | n-Bu | nonafluoro-2-butyl | H | Cl |
| 15-3190 | 2,2,2-trichloroethyl | H | H | H | H | F | H | Br | H | nonafluoro-2-butyl | n-Bu | Br |
| 15-3191 | 2,2,2-trichloroethyl | H | H | H | H | F | H | I | i-Bu | nonafluoro-2-butyl | H | I |
| 15-3192 | 2,2,2-trichloroethyl | H | H | H | H | F | H | Cl | H | nonafluoro-2-butyl | i-Bu | OCF3 |
| 15-3193 | 2,2,2-trichloroethyl | H | H | H | H | F | H | Br | s-Bu | nonafluoro-2-butyl | H | OCF3 |
| 15-3194 | 2,2,2-trichloroethyl | H | H | H | H | F | H | I | H | nonafluoro-2-butyl | s-Bu | OCF3 |
| 15-3195 | 2,2,2-trichloroethyl | H | H | H | H | F | H | Cl | Me | nonafluoro-2-butyl | Me | CF3 |
| 15-3196 | 2,2,2-trichloroethyl | H | H | H | H | F | H | Br | Me | nonafluoro-2-butyl | Et | CF3 |
| 15-3197 | 222-trichloroethyl | C(O)Me | H | H | H | F | H | I | Me | nonafluoro-2-butyl | n-Pr | CF3 |
| 15-3198 | 2,2,2-trichloroethyl | H | H | H | H | F | H | CF3 | Me | nonafluoro-2-butyl | i-Pr | CF3 |
| 15-3199 | 2,2,2-trichloroethyl | H | H | H | H | H | F | Cl | Me | pentafluoroethyl | n-Bu | Cl |
| 15-3200 | 2,2,2-trichloroethyl | H | H | H | H | H | F | Br | Me | heptafluoroisopropyl | i-Bu | Br |
| 15-3201 | 2,2,2-trichloroethyl | H | C(O)Me | H | H | H | F | Br | Me | heptafluoroisopropyl | s-Bu | OCF3 |
| 15-3202 | 2,2,2-trichloroethyl | H | H | H | H | H | F | I | Et | heptafluoroisopropyl | Me | CF3 |
| 15-3203 | 2,2,2-trichloroethyl | H | H | H | H | H | F | CF3 | Et | heptafluoroisopropyl | Et | CF3 |
| 15-3204 | 2,2,2-trichloroethyl | C(O)Me | C(O)Me | H | H | H | F | I | Et | nonafluoro-2-butyl | n-Pr | I |
| 15-3205 | 2,2,2-trichloroethyl | H | H | H | H | H | F | Cl | Et | nonafluoro-2-butyl | i-Pr | OCF3 |
| 15-3206 | 2,2,2-trichloroethyl | H | H | H | H | H | F | Br | Et | nonafluoro-2-butyl | n-Bu | CF3 |
| 15-3207 | 2,2,2-trichloroethyl | H | H | H | H | H | F | CF3 | Et | nonafluoro-2-butyl | i-Bu | CF3 |
| 15-3208 | 2,2,2-trichloroethyl | H | H | F | H | F | H | Cl | Et | pentafluoroethyl | s-Bu | Cl |
| 15-3209 | 2,2,2-trichloroethyl | H | H | F | H | F | H | Cl | n-Pr | heptafluoroisopropyl | Me | Cl |
| 15-3210 | 2,2,2-trichloroethyl | H | H | F | H | F | H | Br | n-Pr | heptafluoroisopropyl | Et | Br |
| 15-3211 | 2,2,2-trichloroethyl | Me | Et | F | H | F | H | I | n-Pr | heptafluoroisopropyl | n-Pr | I |
| 15-3212 | 2,2,2-trichloroethyl | H | H | F | H | F | H | Cl | n-Pr | heptafluoroisopropyl | i-Pr | OCF3 |
| 15-3213 | 2,2,2-trichloroethyl | H | H | F | H | F | H | Br | n-Pr | heptafluoroisopropyl | n-Bu | OCF3 |
| 15-3214 | 2,2,2-trichloroethyl | H | H | F | H | F | H | I | n-Pr | heptafluoroisopropyl | i-Bu | OCF3 |
| 15-3215 | 2,2,2-trichloroethyl | H | H | F | H | F | H | Cl | n-Pr | heptafluoroisopropyl | s-Bu | CF3 |
| 15-3216 | 2,2,2-trichloroethyl | Me | n-Pr | F | H | F | H | Br | i-Pr | heptafluoroisopropyl | Me | CF3 |
| 15-3217 | 2,2,2-trichloroethyl | H | H | F | H | F | H | I | i-Pr | heptafluoroisopropyl | Et | CF3 |
| 15-3218 | 2,2,2-trichloroethyl | H | H | F | H | F | H | CF3 | i-Pr | heptafluoroisopropyl | n-Pr | CF3 |
| 15-3219 | 2,2,2-trichloroethyl | H | H | F | H | F | H | Cl | i-Pr | nonafluoro-2-butyl | i-Pr | Cl |
| 15-3220 | 2,2,2-trichloroethyl | H | H | F | H | F | H | Br | i-Pr | nonafluoro-2-butyl | n-Bu | Br |
| 15-3221 | 2,2,2-trichloroethyl | H | H | F | H | F | H | I | i-Pr | nonafluoro-2-butyl | i-Bu | I |
| 15-3222 | 2,2,2-trichloroethyl | Me | i-Pr | F | H | F | H | Cl | i-Pr | nonafluoro-2-butyl | s-Bu | OCF3 |
| 15-3223 | 2,2,2-trichloroethyl | H | H | F | H | F | H | Br | n-Bu | nonafluoro-2-butyl | Me | OCF3 |
| 15-3224 | 2,2,2-trichloroethyl | H | H | F | H | F | H | I | n-Bu | nonafluoro-2-butyl | Et | OCF3 |
| 15-3225 | 2,2,2-trichloroethyl | H | H | F | H | F | H | Br | n-Bu | nonafluoro-2-butyl | n-Pr | CF3 |
| 15-3226 | 2,2,2-trichlomethyl | H | H | F | H | F | H | I | n-Bu | nonafluoro-2-butyl | i-Pr | CF3 |

**Table 15(7)**

| compound number | Q₂ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 15-3227 | 2,2,2-trichloroethyl | H | H | F | H | F | H | CF3 | n-Bu | nonafluoro-2-butyl | n-Bu | CF3 |
| 15-3228 | 2,2,2-trichloroethyl | H | H | F | H | H | F | Cl | n-Bu | pentafluoroethyl | i-Bu | Cl |
| 15-3229 | 2,2,2-trichloroethyl | Me | C(O)Me | F | H | H | F | Br | n-Bu | heptafluoroisopropyl | s-Bu | Br |
| 15-3230 | 2,2,2-trichloroethyl | H | H | F | H | H | F | Br | i-Bu | heptafluoroisopropyl | Me | OCF3 |
| 15-3231 | 2,2,2-trichloroethyl | H | H | F | H | H | F | I | i-Bu | heptafluoroisopropyl | Et | CF3 |
| 15-3232 | 2,2,2-trichloroethyl | H | H | F | H | H | F | CF3 | i-Bu | heptafluoroisopropyl | n-Pr | CF3 |
| 15-3233 | 2,2,2-trichloroethyl | H | H | F | H | H | F | I | i-Bu | nonafluoro-2-butyl | i-Pr | I |
| 15-3234 | 2,2,2-trichloroethyl | H | H | F | H | H | F | Cl | i-Bu | nonafluoro-2-butyl | n-Bu | OCF3 |
| 15-3235 | 2,2,2-trichloroethyl | H | H | F | H | H | F | Br | i-Bu | nonafluoro-2-butyl | i-Bu | CF3 |
| 15-3236 | 2,2,2-trichloroethyl | H | H | F | H | H | F | CF3 | i-Bu | nonafluoro-2-butyl | s-Bu | CF3 |
| 15-3237 | 2,2,2-trichloroethyl | H | H | H | F | F | H | Cl | s-Bu | pentafluoroethyl | Me | Cl |
| 15-3238 | 2,2,2-trichloroethyl | H | H | H | F | F | H | Br | s-Bu | heptafluoroisopropyl | Et | Br |
| 15-3239 | 2,2,2-trichloroethyl | H | H | H | F | F | H | Br | s-Bu | heptafluoroisopropyl | n-Pr | OCF3 |
| 15-3240 | 2,2,2-trichloroethyl | H | H | H | F | F | H | I | s-Bu | heptafluoroisopropyl | i-Pr | CF3 |
| 15-3241 | 2,2,2-trichloroethyl | H | H | H | F | F | H | CF3 | s-Bu | heptafluoroisopropyl | n-Bu | CF3 |
| 15-3242 | 2,2,2-trichloroethyl | H | H | H | F | F | H | I | s-Bu | nonafluoro-2-butyl | i-Bu | I |
| 15-3243 | 2,2,2-trichloroethyl | Et | Me | H | F | F | H | Cl | s-Bu | nonafluoro-2-butyl | s-Bu | OCF3 |
| 15-3244 | 2,2,2-trichloroethyl | H | H | H | F | F | H | Br | F | nonafluoro-2-butyl | Me | CF3 |
| 15-3245 | 2,2,2-trichloroethyl | H | H | H | F | F | H | CF3 | F | nonafluoro-2-butyl | Et | CF3 |
| 15-3246 | 2,2,2-trichloroethyl | H | H | H | F | H | F | Cl | F | pentafluoroethyl | n-Pr | Cl |
| 15-3247 | 2,2,2-trichloroethyl | H | H | H | F | H | F | Br | F | heptafluoroisopropyl | i-Pr | Br |
| 15-3248 | 2,2,2-trichloroethyl | H | H | H | F | H | F | Br | F | heptafluoroisopropyl | n-Bu | OCF3 |
| 15-3249 | 2,2,2-trichloroethyl | H | H | H | F | H | F | I | F | heptafluoroisopropyl | i-Bu | CF3 |
| 15-3250 | 2,2,2-trichloroethyl | Et | n-Pr | H | F | H | F | CF3 | F | heptafluoroisopropyl | s-Bu | CF3 |
| 15-3251 | 2,2,2-trichloroethyl | Et | i-Pr | H | F | H | F | I | Cl | nonafluoro-2-butyl | Me | I |
| 15-3252 | 2,2,2-trichloroethyl | H | H | H | F | H | F | Cl | Cl | nonafluoro-2-butyl | Et | OCF3 |
| 15-3253 | 2,2,2-trichloroethyl | H | H | H | F | H | F | Br | Cl | nonafluoro-2-butyl | n-Pr | CF3 |
| 15-3254 | 2,2,2-trichloroethyl | H | H | H | F | H | F | CF3 | Cl | nonafluoro-2-butyl | i-Pr | CF3 |
| 15-3255 | 2,2,2-trichloroethyl | H | H | H | H | F | F | Cl | Cl | pentafluoroethyl | n-Bu | Cl |
| 15-3256 | 2,2,2-trichloroethyl | Et | C(O)Me | H | H | F | F | Br | Cl | heptafluoroisopropyl | i-Bu | Br |
| 15-3257 | 2,2,2-trichloroethyl | H | H | H | H | F | F | Br | Cl | heptafluoroisopropyl | s-Bu | OCF3 |
| 15-3258 | 2,2,2-trichloroethyl | H | H | H | H | F | F | I | Br | heptafluoroisopropyl | Me | CF3 |
| 15-3259 | 2,2,2-trichloroethyl | H | H | H | H | F | F | CF3 | Br | heptafluoroisopropyl | Et | CF3 |
| 15-3260 | 2,2,2-trichloroethyl | H | H | H | H | F | F | I | Br | nonafluoro-2-butyl | n-Pr | I |
| 15-3261 | 222-trichloroethyl | H | H | H | H | F | F | Cl | Br | nonafluoro-2-butyl | i-Pr | OCF3 |
| 15-3262 | 2,2,2-trichloroethyl | H | H | H | H | F | F | Br | Br | nonafluoro-2-butyl | n-Bu | CF3 |
| 15-3263 | 2,2,2-trichloroethyl | H | H | H | H | F | F | CF3 | Br | nonafluoro-2-butyl | i-Bu | CF3 |
| 15-3264 | 2,2,2-trichloroethyl | H | H | F | F | H | F | Cl | Br | pentafluoroethyl | s-Bu | Cl |
| 15-3265 | 2,2,2-trichloroethyl | n-Pr | Me | F | F | H | F | Br | I | heptafluoroisopropyl | Me | Br |
| 15-3266 | 2,2,2-trichloroethyl | H | H | F | F | H | F | Br | I | heptafluoroisopropyl | Et | OCF3 |
| 15-3267 | 2,2,2-trichloroethyl | n-Pr | Et | F | F | H | F | I | I | heptafluoroisopropyl | n-Pr | CF3 |
| 15-3268 | 2,2,2-trichloroethyl | H | H | F | F | H | F | CF3 | I | heptafluoroisopropyl | i-Pr | CF3 |
| 15-3269 | 2,2,2-trichloroethyl | H | H | F | F | H | F | I | I | nonafluoro-2-butyl | n-Bu | I |
| 15-3270 | 2,2,2-trichloroethyl | H | H | F | F | H | F | Cl | I | nonafluoro-2-butyl | i-Bu | OCF3 |
| 15-3271 | 2,2,2-trichloroethyl | H | H | F | F | H | F | Br | I | nonafluoro-2-butyl | s-Bu | CF3 |
| 15-3272 | 2,2,2-trichloroethyl | H | H | F | F | H | F | CF3 | Me | nonafluoro-2-butyl | F | CF3 |
| 15-3273 | 2,2,2-trichloroethyl | H | H | F | H | F | F | Cl | Me | pentafluoroethyl | Cl | Cl |
| 15-3274 | 2,2,2-trichloroethyl | H | H | F | H | F | F | Br | Me | heptafluoroisopropyl | Br | Br |
| 15-3275 | 2,2,2-trichloroethyl | H | H | F | H | F | F | Br | Me | heptafluoroisopropyl | I | OCF3 |
| 15-3276 | 2,2,2-trichloroethyl | H | H | F | H | F | F | I | Et | heptafluoroisopropyl | F | CF3 |
| 15-3277 | 2,2,2-trichloroethyl | H | H | F | H | F | F | CF3 | Et | heptafluoroisopropyl | Cl | CF3 |
| 15-3278 | 2,2,2-trichloroethyl | H | H | F | H | F | F | I | Et | nonafluoro-2-butyl | Br | I |
| 15-3279 | 2,2,2-trichloroethyl | n-Pr | i-Pr | F | H | F | F | Cl | Et | nonafluoro-2-butyl | I | OCF3 |
| 15-3280 | 2,2,2-trichloroethyl | H | H | F | H | F | F | Br | n-Pr | nonafluoro-2-butyl | F | CF3 |
| 15-3281 | 2,2,2-trichloroethyl | H | H | F | H | F | F | CF3 | n-Pr | nonafluoro-2-butyl | Cl | CF3 |
| 15-3282 | 2,2,2-trichloroethyl | H | H | H | F | F | F | Cl | n-Pr | pentafluoroethyl | Br | Cl |
| 15-3283 | 2,2,2-trichloroethyl | H | H | H | F | F | F | Br | n-Pr | heptafluoroisopropyl | I | Br |
| 15-3284 | 2,2,2-trichloroethyl | H | H | H | F | F | F | Br | i-Pr | heptafluoroisopropyl | F | OCF3 |
| 15-3285 | 2,2,2-trichloroethyl | H | H | H | F | F | F | I | i-Pr | heptafluoroisopropyl | Cl | CF3 |
| 15-3286 | 2,2,2-trichloroethyl | n-Pr | C(O)Me | H | F | F | F | CF3 | i-Pr | heptafluoroisopropyl | Br | CF3 |
| 15-3287 | 2,2,2-trichloroethyl | H | H | H | F | F | F | I | i-Pr | nonafluoro-2-butyl | I | I |
| 15-3288 | 2,2,2-trichloroethyl | H | H | H | F | F | F | Cl | n-Bu | nonafluoro-2-butyl | F | OCF3 |
| 15-3289 | 2,2,2-trichloroethyl | H | H | H | F | F | F | Br | n-Bu | nonafluoro-2-butyl | Cl | CF3 |
| 15-3290 | 2,2,2-trichloroethyl | H | H | H | F | F | F | CF3 | n-Bu | nonafluoro-2-butyl | Br | CF3 |

**Table 15(8)**

| compound number | Q₂ | R₁ | R₂ | X₁ | X₂ | X₃ | X₄ | Y₁ | Y₂ | V₃ | Y₄ | Y₅ |
|---|---|---|---|---|---|---|---|---|---|---|---|---|
| 15-3291 | 2,2,2-trichloroethyl | i-Pr | Me | F | F | F | F | Cl | n-Bu | pentafluoroethyl | I | Cl |
| 15-3292 | 2,2,2-trichloroethyl | H | H | F | F | F | F | Br | i-Bu | heptafluoroisopropyl | F | Br |
| 15-3293 | 2,2,2-trichloroethyl | H | H | F | F | F | F | Br | i-Bu | heptafluoroisopropyl | Cl | OCF3 |
| 15-3294 | 2,2,2-trichloroethyl | H | H | F | F | F | F | I | i-Bu | heptafluoroisopropyl | Br | CF3 |
| 15-3295 | 2,2,2-trichloroethyl | i-Pr | Et | F | F | F | F | CF3 | i-Bu | heptafluoroisopropyl | I | CF3 |
| 15-3296 | 2,2,2-trichloroethyl | i-Pr | n-Pr | F | F | F | F | I | s-Bu | nonafluoro-2-butyl | F | I |
| 15-3297 | 2,2,2-trichloroethyl | H | H | F | F | F | F | Cl | s-Bu | nonafluoro-2-butyl | Cl | OCF3 |
| 15-3298 | 2,2,2-trichloroethyl | i-Pr | C(O)Me | F | F | F | F | Br | s-Bu | nonafluoro-2-butyl | Br | CF3 |
| 15-3299 | 2,2,2-trichloroethyl | H | H | F | F | F | F | CF3 | s-Bu | nonafluoro-2-butyl | I | CF3 |
| 15-3308 | 2,2-dichloroethyl | H | Et | H | CN | H | H | Cl | F | heptafluoroisopropyl | F | OCF3 |
| 15-3338 | 2,2,2-trifluoroethyl | H | H | H | CN | H | H | Cl | Me | nonafluoro-2-butyl | Me | OCF3 |
| 15-3447 | 2-bromoethyl | H | H | H | CN | H | H | F | Br | heptafluoroisopropyl | H | CF3 |
| 15-3457 | 3-chloro-n-propyl | n-Pr | H | H | CN | H | H | Cl | Cl | heptafluoroisopropyl | Br | CF3 |
| 15-3464 | 3,3,3-trifluoro-n-propyl | H | H | H | CN | H | H | Cl | I | nonafluoro-2-butyl | Cl | CF3 |
| 15-3467 | i-butyl | H | H | H | CN | H | H | Cl | Me | nonafluoro-2-butyl | H | CF3 |
| 15-3468 | s-butyl | H | H | H | CN | H | H | Cl | H | nonafluoro-2-butyl | Me | CF3 |
| 15-3469 | vinyl | i-Pr | H | H | CN | H | H | Cl | Et | nonafluoro-2-butyl | H | CF3 |
| 15-3470 | benzyl | H | H | H | CN | H | H | Cl | H | nonafluoro-2-butyl | Et | CF3 |
| 15-3481 | 2,2,2-trifluoroethyl | H | H | H | CN | H | H | Cl | Me | nonafluoro-2-butyl | Me | n-C3F7 |
| 15-3482 | 2,2,2-trichloroethyl | H | H | H | CN | H | H | Cl | Me | nonafluoro-2-butyl | Et | i-C3F7 |
| 15-3488 | i-propyl | H | H | H | CN | H | H | Cl | Et | nonafluoro-2-butyl | Me | n-C3F7 |
| 15-3489 | 1,2-difluoro-2-propyl | H | H | H | CN | H | H | Cl | Et | nonafluoro-2-butyl | Et | i-C3F7 |
| 15-3495 | 2-chloroethyl | H | H | H | CN | H | H | Br | n-Pr | heptafluoroisopropyl | Me | CF3 |
| 15-3496 | 2-bromoethyl | H | H | H | CN | H | H | Br | n-Pr | heptafluoroisopropyl | Et | CF3 |
| 15-3502 | 2,2,2-trichloroethyl | H | H | H | CN | H | H | Br | i-Pr | heptafluoroisopropyl | Me | CF3 |
| 15-3503 | 2,2,2-tribromoethyl | H | H | H | CN | H | H | Br | i-Pr | heptafluoroisopropyl | Et | CF3 |
| 15-3509 | 1,2-difluoro-2-propyl | H | H | H | CN | H | H | Br | n-Bu | heptafluoroisopropyl | Me | CF3 |
| 15-3510 | 1,3-difluoro-2-propyl | H | H | H | CN | H | H | Br | n-Bu | heptafluoroisopropyl | Et | CF3 |
| 15-3516 | i-butyl | H | H | H | CN | H | H | Br | i-Bu | nonafluoro-2-butyl | Me | CF3 |
| 15-3517 | s-butyl | H | H | H | CN | H | H | Br | i-Bu | nonafluoro-2-butyl | Et | CF3 |
| 15-3523 | 2-fluoroethyl | H | H | H | CN | H | H | Br | s-Bu | nonafluoro-2-butyl | Me | CF3 |
| 15-3524 | 2-chloroethyl | H | H | H | CN | H | H | Br | s-Bu | nonafluoro-2-butyl | Et | CF3 |
| 15-3530 | 2,2,2-trifluoroethyl | H | H | H | CN | H | H | Br | F | nonafluoro-2-butyl | Me | CF3 |
| 15-3531 | 2,2,2-trichloroethyl | H | H | H | CN | H | H | Br | F | nonafluoro-2-butyl | Et | CF3 |
| 15-3537 | i-propyl | Et | i-Pr | H | CN | H | H | Br | Cl | nonafluoro-2-butyl | Me | CF3 |
| 15-3538 | 1,2-difluoro-2-propyl | H | H | H | CN | H | H | Br | Cl | nonafluoro-2-butyl | Et | CF3 |
| 15-3544 | 2-iodoethyl | H | H | H | CN | H | H | I | Br | heptafluoroisopropyl | Me | CF3 |
| 15-3545 | 2-cyanoethyl | H | H | H | CN | H | H | I | Br | heptafluoroisopropyl | Et | CF3 |
| 15-3551 | n-propyl | n-Pr | Me | H | CN | H | H | I | I | heptafluoroisopropyl | Me | CF3 |
| 15-3552 | 3-fluoro-n-propyl | H | H | H | CN | H | H | I | I | heptafluoroisopropyl | Et | CF3 |
| 15-3558 | 1,3-dichloro-2-propyl | H | H | H | CN | H | H | I | Me | heptafluoroisopropyl | F | C2F5 |
| 15-3559 | 1-chloro-3-fluoro-2-propyl | H | H | H | CN | H | H | I | Me | nonafluoro-2-butyl | Cl | CF3 |
| 15-3562 | 4,4, 4-triflu oro-n-butyl | H | H | H | CN | H | H | I | Et | nonafluoro-2-butyl | F | CF3 |
| 15-3563 | i-butyl | H | H | H | CN | H | H | I | Et | nonafluoro-2-butyl | Cl | CF3 |
| 15-3566 | benzyl | H | H | H | CN | H | H | I | n-Pr | nonafluoro-2-butyl | F | CF3 |
| 15-3567 | phenyl | H | H | H | CN | H | H | I | n-Pr | nonafluoro-2-butyl | Cl | CF3 |
| 15-3570 | 2-fluoroethyl | H | H | H | CN | H | H | I | i-Pr | nonafluoro-2-butyl | F | i-C3F7 |
| 15-3571 | 2-chloroethyl | H | H | H | CN | H | H | I | i-Pr | nonafluoro-2-butyl | Cl | CF3 |
| 15-3574 | 2-cyanoethyl | H | H | H | CN | H | H | I | n-Bu | nonafluoro-2-butyl | F | CF3 |
| 15-3575 | 2,2-difluoroethyl | H | H | H | CN | H | H | I | n-Bu | nonafluoro-2-butyl | Cl | CF3 |
| 15-3578 | 2,2,2-trichloroethyl | H | H | H | CN | H | H | I | i-Bu | nonafluoro-2-butyl | F | CF3 |
| 15-3579 | 2,2,2-tribromoethyl | H | H | H | CN | H | H | I | i-Bu | nonafluoro-2-butyl | Cl | CF3 |
| 15-3582 | 3-chloro-n-propyl | i-Pr | n-Pr | H | CN | H | H | I | s-Bu | nonafluoro-2-butyl | F | CF3 |
| 15-3583 | 3-bromo-n-propyl | H | H | H | CN | H | H | I | s-Bu | nonafluoro-2-butyl | Cl | CF3 |
| 15-3594 | 2,2-dichloroethyl | H | Et | H | CN | H | H | OCF3 | F | heptafluoroisopropyl | F | OCF3 |
| 15-3624 | 2,2,2-trifluoroethyl | H | H | H | CN | H | H | OCF3 | Me | nonafluoro-2-butyl | Me | OCF3 |
| 15-3737 | 2,2-dichloroethyl | H | Et | H | CN | H | H | OCF3 | F | heptafluoroisopropyl | F | CF3 |
| 15-3767 | 2,2,2-trifluoroethyl | H | H | H | CN | H | H | OCF3 | Me | nonafluoro-2-butyl | Me | CF3 |
| 15-3880 | 2,2-dichloroethyl | H | Et | H | CN | H | H | CF3 | F | heptafluoroisopropyl | F | CF3 |
| 15-3910 | 2,2,2-trifluoroethyl | H | H | H | CN | H | H | CF3 | Me | nonafluoro-2-butyl | Me | CF3 |
| 15-4023 | 2,2-dichloroethyl | H | Et | F | CN | H | H | Cl | F | heptafluoroisopropyl | F | Cl |
| 15-4053 | 2,2,2-trifluoroethyl | H | H | F | CN | H | H | Cl | Me | nonafluoro-2-butyl | Me | I |
| 15-4166 | 2,2-dichloroethyl | H | Et | F | CN | H | H | Cl | F | heptafluoroisopropyl | F | OCF3 |
| 15-4196 | 2,2,2-trifluoroethyl | H | H | F | CN | H | H | Cl | Me | nonafluoro-2-butyl | Me | OCF3 |

**Table 15(9)**

| compound number | Q₂ | R₁ | R₂ | X₁ | X₂ | X₃ | X₄ | Y₁ | Y₂ | V₃ | Y₄ | Y₅ |
|---|---|---|---|---|---|---|---|---|---|---|---|---|
| 15-4304 | 2-chloroethyl | Me | Me | F | CN | H | H | Cl | H | heptafluoroisopropyl | CI | CF3 |
| 15-4311 | 2,2,2-trichloroethyl | H | H | F | CN | H | H | Cl | F | heptafluoroisopropyl | Br | CF3 |
| 15-4318 | 1,2-difluoro-2-propyl | H | H | F | CN | H | H | Cl | Br | heptafluoroisopropyl | Cl | CF3 |
| 15-4325 | i-butyl | H | H | F | CN | H | H | Cl | Me | nonafluoro-2-butyl | H | CF3 |
| 15-4326 | s-butyl | H | H | F | CN | H | H | Cl | H | nonafluoro-2-butyl | Me | CF3 |
| 15-4327 | vinyl | i-Pr | H | F | CN | H | H | Cl | Et | nonafluoro-2-butyl | H | CF3 |
| 15-4328 | benzyl | H | H | F | CN | H | H | Cl | H | nonafluoro-2-butyl | Et | CF3 |
| 15-4339 | 2,2,2-trifluoroethyl | H | H | F | CN | H | H | Cl | Me | nonafluoro-2-butyl | Me | n-C3F7 |
| 15-4340 | 2,2,2-trichloroethyl | H | H | F | CN | H | H | Cl | Me | nonafluoro-2-butyl | Et | i-C3F7 |
| 15-4346 | i-propyl | H | H | F | CN | H | H | Cl | Et | nonafluoro-2-butyl | Me | n-C3F7 |
| 15-4347 | 1,2-difluoro-2-propyl | H | H | F | CN | H | H | Cl | Et | nonafluoro-2-butyl | Et | i-C3F7 |
| 15-4353 | 2-chloroethyl | H | H | F | CN | H | H | Br | n-Pr | heptafluoroisopropyl | Me | CF3 |
| 15-4354 | 2-bromoethyl | H | H | F | CN | H | H | Br | n-Pr | heptafluoroisopropyl | Et | CF3 |
| 15-4360 | 2,2,2-trichloroethyl | H | H | F | CN | H | H | Br | i-Pr | heptafluoroisopropyl | Me | CF3 |
| 15-4361 | 2,2,2-tribromoethyl | H | H | F | CN | H | H | Br | i-Pr | heptafluoroisopropyl | Et | CF3 |
| 15-4367 | 1,2-difluoro-2-propyl | H | H | F | CN | H | H | Br | n-Bu | heptafluoroisopropyl | Me | CF3 |
| 15-4368 | 1,3-difluoro-2-propyl | H | H | F | CN | H | H | Br | n-Bu | heptafluoroisopropyl | Et | CF3 |
| 15-4374 | i-butyl | H | H | F | CN | H | H | Br | i-Bu | nonafluoro-2-butyl | Me | CF3 |
| 15-4375 | s-butyl | H | H | F | CN | H | H | Br | i-Bu | nonafluoro-2-butyl | Et | CF3 |
| 15-4381 | 2-fluoroethyl | H | H | F | CN | H | H | Br | s-Bu | nonafluoro-2-butyl | Me | CF3 |
| 15-4382 | 2-chloroethyl | H | H | F | CN | H | H | Br | s-Bu | nonafluoro-2-butyl | Et | CF3 |
| 15-4388 | 2,2,2-trifluoroethyl | H | H | F | CN | H | H | Br | F | nonafluoro-2-butyl | Me | CF3 |
| 15-4389 | 2,2,2-trichloroethyl | H | H | F | CN | H | H | Br | F | nonafluoro-2-butyl | Et | CF3 |
| 15-4395 | i-propyl | Et | i-Pr | F | CN | H | H | Br | Cl | nonafluoro-2-butyl | Me | CF3 |
| 15-4396 | 1,2-difluoro-2-propyl | H | H | F | CN | H | H | Br | Cl | nonafluoro-2-butyl | Et | CF3 |
| 15-4402 | 2-iodoethyl | H | H | F | CN | H | H | I | Br | heptafluoroisopropyl | Me | CF3 |
| 15-4403 | 2-cyanoethyl | H | H | F | CN | H | H | I | Br | heptafluoroisopropyl | Et | CF3 |
| 15-4409 | n-propyl | n-Pr | Me | F | CN | H | H | I | I | heptafluoroisopropyl | Me | CF3 |
| 15-4410 | 3-fluoro-n-propyl | H | H | F | CN | H | H | I | I | heptafluoroisopropyl | Et | CF3 |
| 15-4416 | 1,3-dichloro-2-propyl | H | H | F | CN | H | H | I | Me | heptafluoroisopropyl | F | C2F5 |
| 15-4417 | 1-chloro-3-fluoro-2-propyl | H | H | F | CN | H | H | I | Me | nonafluoro-2-butyl | Cl | CF3 |
| 15-4420 | 4,4,4-trifluoro-n-butyl | H | H | F | CN | H | H | I | Et | nonafluoro-2-butyl | F | CF3 |
| 15-4421 | i-butyl | H | H | F | CN | H | H | I | Et | nonafluoro-2-butyl | Cl | CF3 |
| 15-4424 | benzyl | H | H | F | CN | H | H | I | n-Pr | nonafluoro-2-butyl | F | CF3 |
| 15-4425 | phenyl | H | H | F | CN | H | H | I | n-Pr | nonafluoro-2-butyl | Cl | CF3 |
| 15-4428 | 2-fluoroethyl | H | H | F | CN | H | H | I | i-Pr | nonafluoro-2-butyl | F | i-C3F7 |
| 15-4429 | 2-chloroethyl | H | H | F | CN | H | H | I | i-Pr | nonafluoro-2-butyl | Cl | CF3 |
| 15-4432 | 2-cyanoethyl | H | H | F | CN | H | H | I | n-Bu | nonafluoro-2-butyl | F | CF3 |
| 15-4433 | 2,2-difluoroethyl | H | H | F | CN | H | H | I | n-Bu | nonafluoro-2-butyl | Cl | CF3 |
| 15-4436 | 2,2,2-trichloroethyl | H | H | F | CN | H | H | I | i-Bu | nonafluoro-2-butyl | F | CF3 |
| 15-4437 | 2,2,2-tribromoethyl | H | H | F | CN | H | H | I | i-Bu | nonafluoro-2-butyl | Cl | CF3 |
| 15-4440 | 3-chloro-n-propyl | i-Pr | n-Pr | F | CN | H | H | I | s-Bu | nonafluoro-2-butyl | F | CF3 |
| 15-4441 | 3-bromo-n-propyl | H | H | F | CN | H | H | I | s-Bu | nonafluoro-2-butyl | Cl | CF3 |
| 15-4452 | 2,2-dichloroethyl | H | Et | F | CN | H | H | OCF3 | F | heptafluoroisopropyl | F | OCF3 |
| 15-4482 | 2,2,2-trifluoroethyl | H | H | F | CN | H | H | OCF3 | Me | nonafluoro-2-butyl | Me | OCF3 |
| 15-4595 | 2,2-dichloroethyl | H | Et | F | CN | H | H | OCF3 | F | heptafluoroisopropyl | F | CF3 |
| 15-4625 | 2,2,2-trifluoroethyl | H | H | F | CN | H | H | OCF3 | Me | nonafluoro-2-butyl | Me | CF3 |
| 15-4738 | 2,2-dichloroethyl | H | Et | F | CN | H | H | CF3 | F | heptafluoroisopropyl | F | CF3 |
| 15-4768 | 2,2,2-trifluoroethyl | H | H | F | CN | H | H | CF3 | Me | nonafluoro-2-butyl | Me | CF3 |
| 15-4881 | 2,2-dichloroethyl | H | Et | F | CN | F | H | Cl | F | heptafluoroisopropyl | F | Cl |
| 15-4911 | 2,2,2-trifluoroethyl | H | H | F | CN | F | H | Cl | Me | nonafluoro-2-butyl | Me | I |
| 15-5024 | 2,2-dichloroethyl | H | Et | F | CN | F | H | Cl | F | heptafluoroisopropyl | F | OCF3 |
| 15-5054 | 2,2,2-trifluoroethyl | H | H | F | CN | F | H | Cl | Me | nonafluoro-2-butyl | Me | OCF3 |
| 15-5163 | 2-bromoethyl | H | H | F | CN | F | H | F | Br | heptafluoroisopropyl | H | CF3 |
| 15-5168 | 2,2,2-trifluoroethyl | H | H | F | CN | F | H | Cl | F | heptafluoroisopropyl | Cl | CF3 |
| 15-5180 | 3,3,3-trifluoro-n-propyl | H | H | F | CN | F | H | Cl | I | nonafluoro-2-butyl | Cl | CF3 |
| 15-5183 | i-butyl | H | H | F | CN | F | H | Cl | Me | nonafluoro-2-butyl | H | CF3 |
| 15-5184 | s-butyl | H | H | F | CN | F | H | Cl | H | nonafluoro-2-butyl | Me | CF3 |
| 15-5185 | vinyl | i-Pr | H | F | CN | F | H | Cl | Et | nonafluoro-2-butyl | H | CF3 |
| 15-5186 | benzyl | H | H | F | CN | F | H | Cl | H | nonafluoro-2-butyl | Et | CF3 |
| 15-5197 | 2,2,2-trifluoroethyl | H | H | F | CN | F | H | Cl | Me | nonafluoro-2-butyl | Me | n-C3F7 |
| 15-5198 | 2,2,2-trichloroethyl | H | H | F | CN | F | H | Cl | Me | nonafluoro-2-butyl | Et | i-C3F7 |
| 15-5204 | i-propyl | H | H | F | CN | F | H | Cl | Et | nonafluoro-2-butyl | Me | n-C3F7 |
| 15-5205 | 1.2-difluoro-2-propyl | H | H | F | CN | F | H | Cl | Et | nonafluoro-2-butyl | Et | i-C3F7 |

**Table 15(10)**

| compound number | Q₂ | R₁ | R₂ | X₁ | X₂ | X₃ | X₄ | Y₁ | Y₂ | Y₃ | Y₄ | Y₅ |
|---|---|---|---|---|---|---|---|---|---|---|---|---|
| 15-5211 | 2-chloroethyl | H | H | F | CN | F | H | Br | n-Pr | heptafluoroisopropyl | Me | CF3 |
| 15-5212 | 2-bromoethyl | H | H | F | CN | F | H | Br | n-Pr | heptafluoroisopropyl | Et | CF3 |
| 15-5218 | 2,2,2-trichloroethyl | H | H | F | CN | F | H | Br | i-Pr | heptafluoroisopropyl | Me | CF3 |
| 15-5219 | 2,2,2-tribromoethyl | H | H | F | CN | F | H | Br | i-Pr | heptafluoroisopropyl | Et | CF3 |
| 15-5225 | 1,2-difluoro-2-propyl | H | H | F | CN | F | H | Br | n-Bu | heptafluoroisopropyl | Me | CF3 |
| 15-5226 | 1,3-difluoro-2-propyl | H | H | F | CN | F | H | Br | n-Bu | heptafluoroisopropyl | Et | CF3 |
| 15-5232 | i-butyl | H | H | F | CN | F | H | Br | i-Bu | nonafluoro-2-butyl | Me | CF3 |
| 15-5233 | s-butyl | H | H | F | CN | F | H | Br | i-Bu | nonafluoro-2-butyl | Et | CF3 |
| 15-5239 | 2-fluoroethyl | H | H | F | CN | F | H | Br | s-Bu | nonafluoro-2-butyl | Me | CF3 |
| 15-5240 | 2-chloroethyl | H | H | F | CN | F | H | Br | s-Bu | nonafluoro-2-butyl | Et | CF3 |
| 15-5246 | 2,2,2-trifluoroethyl | H | H | F | CN | F | H | Br | F | nonafluoro-2-butyl | Me | CF3 |
| 15-5247 | 2,2,2-trichloroethyl | H | H | F | CN | F | H | Br | F | nonafluoro-2-butyl | Et | CF3 |
| 15-5253 | i-propyl | Et | i-Pr | F | CN | F | H | Br | Cl | nonafluoro-2-butyl | Me | CF3 |
| 15-5254 | 1,2-difluoro-2-propyl | H | H | F | CN | F | H | Br | Cl | nonafluoro-2-butyl | Et | CF3 |
| 15-5260 | 2-iodoethyl | H | H | F | CN | F | H | I | Br | heptafluoroisopropyl | Me | CF3 |
| 15-5261 | 2-cyanoethyl | H | H | F | CN | F | H | I | Br | heptafluoroisopropyl | Et | CF3 |
| 15-5267 | n-propyl | n-Pr | Me | F | CN | F | H | I | I | heptafluoroisopropyl | Me | CF3 |
| 15-5268 | 3-fluoro-n-propyl | H | H | F | CN | F | H | I | I | heptafluoroisopropyl | Et | CF3 |
| 15-5274 | 1,3-dichloro-2-propyl | H | H | F | CN | F | H | I | Me | heptafluoroisopropyl | F | C2F5 |
| 15-5275 | 1-chloro-3-fluoro-2-propyl | H | H | F | CN | F | H | I | Me | nonafluoro-2-butyl | Cl | CF3 |
| 15-5278 | 4,4, 4-trifluoro-n-butyl | H | H | F | CN | F | H | I | Et | nonafluoro-2-butyl | F | CF3 |
| 15-5279 | i-butyl | H | H | F | CN | F | H | I | Et | nonafluoro-2-butyl | Cl | CF3 |
| 15-5282 | benzyl | H | H | F | CN | F | H | I | n-Pr | nonafluoro-2-butyl | F | CF3 |
| 15-5283 | phenyl | H | H | F | CN | F | H | I | n-Pr | nonafluoro-2-butyl | Cl | CF3 |
| 15-5286 | 2-fluoroethyl | H | H | F | CN | F | H | I | i-Pr | nonafluoro-2-butyl | F | i-C3F7 |
| 15-5287 | 2-chloroethyl | H | H | F | CN | F | H | I | i-Pr | nonafluoro-2-butyl | Cl | CF3 |
| 15-5290 | 2-cyanoethyl | H | H | F | CN | F | H | I | n-Bu | nonafluoro-2-butyl | F | CF3 |
| 15-5291 | 2,2-difluoroethyl | H | H | F | CN | F | H | I | n-Bu | nonafluoro-2-butyl | Cl | CF3 |
| 15-5294 | 2,2,2-trichloroethyl | H | H | F | CN | F | H | I | i-Bu | nonafluoro-2-butyl | F | CF3 |
| 15-5295 | 2,2,2-tribromoethyl | H | H | F | CN | F | H | I | i-Bu | nonafluoro-2-butyl | Cl | CF3 |
| 15-5298 | 3-chloro-n-propyl | i-Pr | n-Pr | F | CN | F | H | I | s-Bu | nonafluoro-2-butyl | F | CF3 |
| 15-5299 | 3-bromo-n-propyl | H | H | F | CN | F | H | I | s-Bu | nonafluoro-2-butyl | Cl | CF3 |
| 15-5310 | 2,2-dichloroethyl | H | Et | F | CN | F | H | OCF3 | F | heptafluoroisopropyl | F | OCF3 |
| 15-5340 | 2,2,2-trifluoroethyl | H | H | F | CN | F | H | OCF3 | Me | nonafluoro-2-butyl | Me | OCF3 |
| 15-5453 | 2,2-dichloroethyl | H | Et | F | CN | F | H | OCF3 | F | heptafluoroisopropyl | F | CF3 |
| 15-5483 | 2,2,2-trifluoroethyl | H | H | F | CN | F | H | OCF3 | Me | nonafluoro-2-butyl | Me | CF3 |
| 15-5596 | 2,2-dichloroethyl | H | Et | F | CN | F | H | CF3 | F | heptafluoroisopropyl | F | CF3 |
| 15-5626 | 2,2,2-trifluoroethyl | H | H | F | CN | F | H | CF3 | Me | nonafluoro-2-butyl | Me | CF3 |

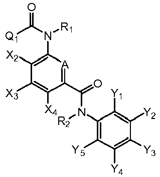

**Table 16(1)**

| compound number | Q₁ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 16-1 | phenyl | H | H | N | H | H | H | F | H | heptafluoroisopropyl | H | F |
| 16-2 | 3-cyanophenyl | H | H | N | H | H | H | F | H | heptafluoroisopropyl | H | F |
| 16-3 | 4-cyanophenyl | H | H | N | H | H | H | F | H | heptafluoroisopropyl | H | F |
| 16-4 | 2-chloropyridin-3-yl | H | H | N | H | H | H | F | H | heptafluoroisopropyl | H | F |
| 16-5 | phenyl | H | H | N | H | H | H | F | H | nonafluoro-2-butyl | H | F |
| 16-6 | 3-cyanophenyl | H | H | N | H | H | H | F | H | nonafluoro-2-butyl | H | F |
| 16-7 | 4-cyanophenyl | H | H | N | H | H | H | F | H | nonafluoro-2-butyl | H | F |
| 16-8 | 2-chloropyridin-3-yl | H | H | N | H | H | H | F | H | nonafluoro-2-butyl | H | F |
| 16-9 | phenyl | H | H | N | H | H | H | Cl | H | heptafluoroisopropyl | H | Cl |
| 16-13 | 2-chlorophenyl | H | H | N | H | H | H | F | H | heptafluoroisopropyl | H | Cl |
| 16-17 | 3-bromophenyl | H | H | N | H | H | H | F | H | heptafluoroisopropyl | H | Br |
| 16-20 | 3-iodophenyl | H | H | N | H | H | H | Cl | H | pentafluoroethyl | H | Cl |
| 16-22 | (2-trifluoromethyl) phenyl | H | H | N | H | H | H | F | H | heptafluoroisopropyl | H | I |
| 16-25 | 2-nitrophenyl | H | H | N | H | H | H | Cl | H | heptafluoroisopropyl | H | Br |
| 16-28 | 2-cyanophenyl | H | H | N | H | H | H | Cl | H | pentafluoroethyl | H | Cl |
| 16-29 | 3-cyanophenyl | H | H | N | H | H | H | Cl | H | heptafluoroisopropyl | H | Cl |
| 16-30 | 4-cyanophenyl | H | H | N | H | H | H | Cl | H | heptafluoroisopropyl | H | Cl |
| 16-31 | 2,6-difiuorophenyl | H | H | N | H | H | H | Cl | H | heptafluoroisopropyl | H | I |
| 16-43 | pyridin-3-yl | H | H | N | H | H | H | Br | H | heptafluoroisopropyl | H | I |
| 16-44 | 2-fluoropyridin-3-yl | H | H | N | H | H | H | Cl | H | pentafluoroethyl | H | Cl |
| 16-45 | 2-chloropyridin-3-yl | H | H | N | H | H | H | Cl | H | heptafluoroisopropyl | H | Cl |
| 16-63 | 5-nitropyridin-3-yl | H | H | N | H | H | H | Cl | H | pentafluoroethyl | H | Cl |
| 16-64 | 5-cyanopyridin-3-yl | H | H | N | H | H | H | Cl | H | heptafluoroisopropyl | H | Cl |
| 16-66 | 4-chloropyridin-3-yl | H | H | N | H | H | H | Cl | H | heptafluoroisopropyl | H | Cl |
| 16-86 | 4-cyanopyridin-2-yl | H | H | N | H | H | H | Cl | H | pentafluoroethyl | H | Cl |
| 16-87 | phenyl | H | H | N | H | H | H | Cl | H | nonafluoro-2-butyl | H | Cl |
| 16-107 | 3-cyanophenyl | H | H | N | H | H | H | Cl | H | nonafluoro-2-butyl | H | Cl |
| 16-108 | 4-cyanophenyl | H | H | N | H | H | H | Cl | H | nonafluoro-2-butyl | H | Cl |
| 16-123 | 2-chloropyridin-3-yl | H | H | N | H | H | H | Cl | H | nonafluoro-2-butyl | H | Cl |
| 16-165 | phenyl | H | H | N | H | H | H | Br | H | heptafluoroisopropyl | H | Br |
| 16-166 | 2-fluorophenyl | H | H | N | H | H | H | Br | H | heptafluoroisopropyl | H | Br |
| 16-177 | 4-iodophenyl | H | H | N | H | H | H | Br | H | pentafluoroethyl | H | Br |
| 16-185 | 3-cyanophenyl | H | H | N | H | H | H | Br | H | heptafluoroisopropyl | H | Br |
| 16-186 | 4-cyanophenyl | H | H | N | H | H | H | Br | H | heptafluoroisopropyl | H | Br |
| 16-200 | 2-fluoropyridin-3-yl | H | H | N | H | H | H | Br | H | pentafluoroethyl | H | Br |
| 16-201 | 2-chloropyridin-3-yl | H | H | N | H | H | H | Br | H | heptafluoroisopropyl | H | Br |
| 16-219 | 5-nitropyridin-3-yl | H | H | N | H | H | H | Br | H | pentafluoroethyl | H | Br |
| 16-220 | 5-cyanopyridin-3-yl | H | H | N | H | H | H | Br | H | heptafluoroisopropyl | H | Br |
| 16-221 | 4-fluoropyridin-3-yl | H | H | N | H | H | H | Br | H | pentafluoroethyl | H | Br |
| 16-242 | 4-cyanopyridin-2-yl | H | H | N | H | H | H | Br | H | pentafluoroethyl | H | Br |
| 16-243 | phenyl | H | H | N | H | H | H | Br | H | nonafluoro-2-butyl | H | Br |
| 16-244 | 2-fluorophenyl | H | H | N | H | H | H | Br | H | nonafluoro-2-butyl | H | Br |
| 16-263 | 3-cyanophenyl | H | H | N | H | H | H | Br | H | nonafluoro-2-butyl | H | Br |
| 16-264 | 4-cyanophenyl | H | H | N | H | H | H | Br | H | nonafluoro-2-butyl | H | Br |
| 16-279 | 2-chloropyridin-3-yl | H | H | N | H | H | H | Br | H | nonafluoro-2-butyl | H | Br |
| 16-321 | phenyl | H | H | N | H | H | H | I | H | heptafluoroisopropyl | H | I |
| 16-328 | 2-bromophenyl | H | H | N | H | H | H | I | H | pentafluoroethyl | H | I |
| 16-341 | 3-cyanophenyl | H | H | N | H | H | H | I | H | heptafluoroisopropyl | H | I |
| 16-342 | 4-cyanophenyl | H | H | N | H | H | H | I | H | heptafluoroisopropyl | H | I |
| 16-356 | 2-fluoropyridin-3-yl | H | H | N | H | H | H | I | H | pentafluoroethyl | H | I |
| 16-357 | 2-chloropyridin-3-yl | H | H | N | H | H | H | I | H | heptafluoroisopropyl | H | I |
| 16-375 | 5-nitropyridin-3-yl | H | H | N | H | H | H | I | H | pentafluoroethyl | H | I |
| 16-399 | phenyl | H | H | N | H | H | H | I | H | nonafluoro-2-butyl | H | I |
| 16-400 | 2-fluorophenyl | H | H | N | H | H | H | I | H | nonafluoro-2-butyl | H | I |
| 16-419 | 3-cyanophenyl | H | H | N | H | H | H | I | H | nonafluoro-2-butl | H | I |

**Table 16(2)**

| compound number | Q₁ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 16-420 | 4-cyanophenyl | H | H | N | H | H | H | I | H | nonafluoro-2-butyl | H | I |
| 16-435 | 2-chloropyridin-3-yl | H | H | N | H | H | H | I | H | nonafluoro-2-butyl | H | I |
| 16-477 | phenyl | H | H | N | H | H | H | Cl | H | heptafluoroisopropyl | H | OCF3 |
| 16-478 | 2-fluorophenyl | H | H | N | H | H | H | Cl | H | pentafluoroethyl | H | OCF3 |
| 16-492 | (4-trifluoromethyl) phenyl | H | H | N | H | H | H | Cl | H | heptafluoroisopropyl | H | OC2F5 |
| 16-493 | 2-nitrophenyl | H | H | N | H | H | H | F | H | heptafluoroisopropyl | H | OCF3 |
| 16-496 | 2-cyanophenyl | H | H | N | H | H | H | Cl | H | pentafluoroethyl | H | OC2F5 |
| 16-497 | 3-cyanophenyl | H | H | N | H | H | H | Cl | H | heptafluoroisopropyl | H | OCF3 |
| 16-498 | 4-cyanophenyl | H | H | N | H | H | H | Cl | H | heptafluoroisopropyl | H | OCF3 |
| 16-504 | 4-bromo-2-chlorophenyl | H | H | N | H | H | H | Cl | H | heptafluoroisopropyl | H | OC2F5 |
| 16-512 | 2-fluoropyridin-3-yl | H | H | N | H | H | H | Cl | H | pentafluoroethyl | H | OCF3 |
| 16-513 | 2-chloropyridin-3-yl | H | H | N | H | H | H | Cl | H | heptafluoroisopropyl | H | OCF3 |
| 16-555 | phenyl | H | H | N | H | H | H | Cl | H | nonafluoro-2-butyl | H | OCF3 |
| 16-565 | 2-iodophenyl | H | H | N | H | H | H | Cl | H | nonafluoro-2-butyl | H | OCF3 |
| 16-571 | 2-nitrophenyl | H | H | N | H | H | H | F | H | nonafluoro-2-butyl | H | OCF3 |
| 16-572 | 3-nitrophenyl | H | H | N | H | H | H | Cl | H | nonafluoro-2-butyl | H | OCF3 |
| 16-573 | 4-nitrophenyl | H | H | N | H | H | H | Cl | H | nonafluoro-2-butyl | H | OCF3 |
| 16-581 | 2-chloro-4,5-difluorophenyl | H | H | N | H | H | H | Cl | H | nonafluoro-2-butyl | H | OC2F5 |
| 16-591 | 2-chloropyridin-3-yl | H | H | N | H | H | H | Cl | H | nonafluoro-2-butyl | H | OCF3 |
| 16-597 | 6-fluoropyridin-3-yl | H | H | N | H | H | H | Cl | H | nonafluoro-2-butyl | H | OC2F5 |
| 16-633 | phenyl | H | H | N | H | H | H | Br | H | heptafluoroisopropyl | H | OCF3 |
| 16-646 | (2-trifluoromethyl) phenyl | H | H | N | H | H | H | Br | H | heptafluoroisopropyl | H | OCF3 |
| 16-653 | 3-cyanophenyl | H | H | N | H | H | H | Br | H | heptafluoroisopropyl | H | OCF3 |
| 16-654 | 4-cyanophenyl | H | H | N | H | H | H | Br | H | heptafluoroisopropyl | H | OCF3 |
| 16-669 | 2-chloropyridin-3-yl | H | H | N | H | H | H | Br | H | heptafluoroisopropyl | H | OCF3 |
| 16-676 | 6-chloropyridin-3-yl | H | H | N | H | H | H | Br | H | heptafluoroisopropyl | H | OCF3 |
| 16-711 | phenyl | H | H | N | H | H | H | Br | H | nonafluoro-2-butyl | H | OCF3 |
| 16-731 | 3-cyanophenyl | H | H | N | H | H | H | Br | H | nonafluoro-2-butyl | H | OCF3 |
| 16-732 | 4-cyanophenyl | H | H | N | H | H | H | Br | H | nonafluoro-2-butyl | H | OCF3 |
| 16-745 | pyridin-3-yl | H | H | N | H | H | H | Br | H | nonafluoro-2-butyl | H | OCF3 |
| 16-747 | 2-chloropyridin-3-yl | H | H | N | H | H | H | Br | H | nonafluoro-2-butyl | H | OCF3 |
| 16-789 | phenyl | H | H | N | H | H | H | I | H | heptafluoroisopropyl | H | OCF3 |
| 16-794 | 3-chlorophenyl | H | H | N | H | H | H | I | H | heptafluoroisopropyl | H | OCF3 |
| 16-809 | 3-cyanophenyl | H | H | N | H | H | H | I | H | heptafluoroisopropyl | H | OCF3 |
| 16-810 | 4-cyanophenyl | H | H | N | H | H | H | I | H | heptafluoroisopropyl | H | OCF3 |
| 16-825 | 2-chloropyridin-3-yl | H | H | N | H | H | H | I | H | heptafluoroisopropyl | H | OCF3 |
| 16-867 | phenyl | H | H | N | H | H | H | I | H | nonafluoro-2-butyl | H | OCF3 |
| 16-887 | 3-cyanophenyl | H | H | N | H | H | H | I | H | nonafluoro-2-butyl | H | OCF3 |
| 16-888 | 4-cyanophenyl | H | H | N | H | H | H | I | H | nonafluoro-2-butyl | H | OCF3 |
| 16-903 | 2-chloropyridin-3-yl | H | H | N | H | H | H | I | H | nonafluoro-2-butyl | H | OCF3 |
| 16-945 | phenyl | H | H | N | H | H | H | F | H | heptafluoroisopropyl | H | CF3 |
| 16-946 | phenyl | H | H | N | H | H | H | Cl | H | heptafluoroisopropyl | H | CF3 |
| 16-947 | 2-fluorophenyl | H | H | N | H | H | H | Cl | H | heptafluoroisopropyl | H | CF3 |
| 16-958 | 4-iodophenyl | H | H | N | H | H | H | Cl | H | pentafluoroethyl | H | CF3 |
| 16-965 | 2-cyanophenyl | H | H | N | H | H | H | Cl | H | pentafluoroethyl | H | C2F5 |
| 16-966 | 3-cyanophenyl | H | H | N | H | H | H | Cl | H | heptafluoroisopropyl | H | CF3 |
| 16-967 | 4-cyanophenyl | H | H | N | H | H | H | Cl | H | heptafluoroisopropyl | H | CF3 |
| 16-970 | 2,4-dichlorophenyl | H | H | N | H | H | H | Cl | H | heptafluoroisopropyl | H | C2F5 |
| 16-974 | 2-bromo-4-chlorophenyl | H | H | N | H | H | H | Cl | H | heptafluoroisopropyl | H | n-C3F7 |
| 16-981 | 2-fluoropyridin-3-yl | H | H | N | H | H | H | Cl | H | pentafluoroethyl | H | CF3 |
| 16-982 | 2-chloropyridin-3-yl | H | H | N | H | H | H | Cl | H | heptafluoroisopropyl | H | CF3 |
| 16-995 | 5-fluoropyridin-3-yl | H | H | N | H | H | H | Cl | H | heptafluoroisopropyl | H | C2F5 |
| 16-997 | 5-bromopyridin-3-yl | H | H | N | H | H | H | Cl | H | heptafluoroisopropyl | H | n-C3F7 |
| 16-998 | 5-iodopyridin-3-yl | H | H | N | H | H | H | Cl | H | heptafluoroisopropyl | H | i-C3F7 |
| 16-1000 | 5-nitropyridin-3-yl | H | H | N | H | H | H | Cl | H | pentafluoroethyl | H | CF3 |
| 16-1002 | 4-fluoropyridin-3-yl | H | H | N | H | H | H | Cl | H | pentafluoroethyl | H | CF3 |
| 16-1004 | 4-bromopyridin-3-yl | H | H | N | H | H | H | Cl | H | heptafluoroisopropyl | H | n-C3F7 |
| 16-1023 | 4-cyanopyridin-2-yl | H | H | N | H | H | H | Cl | H | pentafluoroethyl | H | CF3 |
| 16-1024 | phenyl | H | H | N | H | H | H | F | H | nonafluoro-2-butyl | H | CF3 |
| 16-1025 | phenyl | H | H | N | H | H | H | Cl | H | nonafluoro-2-butyl | H | CF3 |
| 16-1038 | (2-trifluoromethyl) phenyl | H | H | N | H | H | H | Cl | H | nonafluoro-2-butyl | H | C2F5 |
| 16-1045 | 3-cyanophenyl | H | H | N | H | H | H | Cl | H | nonafluoro-2-butyl | H | CF3 |
| 16-1046 | 4-cyanophenyl | H | H | N | H | H | H | Cl | H | nonafluoro-2-butyl | H | CF3 |
| 16-1055 | 2-chloro-4-nitrophenyl | H | H | N | H | H | H | Cl | H | nonafluoro-2-butyl | H | i-C3F7 |
| 16-1061 | 2-chloropyridin-3-yl | H | H | N | H | H | H | Cl | H | nonafluoro-2-butl | H | CF3 |

**Table 16(3)**

| compound number | Q₁ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 16-1074 | 5-fluoropyridin-3-yl | H | H | N | H | H | H | Cl | H | nonafluoro-2-butyl | H | C2F5 |
| 16-1076 | 5-bromopyridin-3-yl | H | H | N | H | H | H | Cl | H | nonafiuoro-2-butyl | H | n-C3F7 |
| 16-1077 | 5-iodopyridin-3-yl | H | H | N | H | H | H | Cl | H | nonafiuoro-2-butyl | H | i-C3F7 |
| 16-1081 | 4-fluoropyridin-3-yl | H | H | N | H | H | H | Cl | H | nonafluoro-2-butyl | H | C2F5 |
| 16-1085 | 4-(trifluoromethyl)pyridin-3-yl | H | H | N | H | H | H | Cl | H | nonafluoro-2-butyl | H | n-C3F7 |
| 16-1086 | 4-nitropyridin-3-yl | H | H | N | H | H | H | Cl | H | nonafluoro-2-butyl | H | i-C3F7 |
| 16-1102 | 4-cyanopyridin-2-yl | H | H | N | H | H | H | Cl | H | nonafluoro-2-butyl | H | C2F5 |
| 16-1103 | phenyl | H | H | N | H | H | H | Br | H | heptafluoroisopropyl | H | CF3 |
| 16-1104 | 2-fluorophenyl | H | H | N | H | H | H | Br | H | heptafluoroisopropyl | H | CF3 |
| 16-1105 | 3-fluorophenyl | H | H | N | H | H | H | Br | H | heptafluoroisopropyl | H | CF3 |
| 16-1106 | 4-fluorophenyl | H | H | N | H | H | H | Br | H | heptafluoroisopropyl | H | CF3 |
| 16-1107 | 2-chlorophenyl | H | H | N | H | H | H | Br | H | heptafluoroisopropyl | H | CF3 |
| 16-1108 | 3-chlorophenyl | H | H | N | H | H | H | Br | H | heptafluoroisopropyl | H | CF3 |
| 16-1109 | 4-chlorophenyl | H | H | N | H | H | H | Br | H | heptafluoroisopropyl | H | CF3 |
| 16-1110 | 2-bromophenyl | H | H | N | H | H | H | Br | H | heptafluoroisopropyl | H | CF3 |
| 16-1111 | 3-bromophenyl | H | H | N | H | H | H | Br | H | heptafluoroisopropyl | H | CF3 |
| 16-1112 | 4-bromophenyl | H | H | N | H | H | H | Br | H | heptafluoroisopropyl | H | CF3 |
| 16-1113 | 2-iodophenyl | H | H | N | H | H | H | Br | H | heptafluoroisopropyl | H | CF3 |
| 16-1114 | 3-iodophenyl | H | H | N | H | H | H | Br | H | heptafluoroisopropyl | H | CF3 |
| 16-1115 | 4-iodophenyl | H | H | N | H | H | H | Br | H | heptafluoroisopropyl | H | CF3 |
| 16-1116 | (2-trifluoromethyl) phenyl | H | H | N | H | H | H | Br | H | heptafluoroisopropyl | H | CF3 |
| 16-1117 | (3-trifluoromethyl) phenyl | H | H | N | H | H | H | Br | H | heptafluoroisopropyl | H | CF3 |
| 16-1118 | (4-trifluoromethyl) phenyl | H | H | N | H | H | H | Br | H | heptafluoroisopropyl | H | CF3 |
| 16-1119 | 2-nitrophenyl | H | H | N | H | H | H | Br | H | heptafluoroisopropyl | H | CF3 |
| 16-1120 | 3-nitrophenyl | H | H | N | H | H | H | Br | H | heptafluoroisopropyl | H | CF3 |
| 16-1121 | 4-nitrophenyl | H | H | N | H | H | H | Br | H | heptafluoroisopropyl | H | CF3 |
| 16-1122 | 2-cyanophenyl | H | H | N | H | H | H | Br | H | heptafluoroisopropyl | H | CF3 |
| 16-1123 | 3-cyanophenyl | H | H | N | H | H | H | Br | H | heptafluoroisopropyl | H | CF3 |
| 16-1124 | 4-cyanophenyl | H | H | N | H | H | H | Br | H | heptafluoroisopropyl | H | CF3 |
| 16-1125 | 2,6-difluorophenyl | H | H | N | H | H | H | Br | H | heptafluoroisopropyl | H | CF3 |
| 16-1126 | 3,4-dichlorophenyl | H | H | N | H | H | H | Br | H | heptafluoroisopropyl | H | CF3 |
| 16-1127 | 2,4-dichlorophenyl | H | H | N | H | H | H | Br | H | heptafluoroisopropyl | H | CF3 |
| 16-1128 | 2-chloro-4-fluorophenyl | H | H | N | H | H | H | Br | H | heptafluoroisopropyl | H | CF3 |
| 16-1129 | 2-chloro-4,5-difluorophenyl | H | H | N | H | H | H | Br | H | heptafluoroisopropyl | H | CF3 |
| 16-1130 | 4-bromo-2-chlorophenyl | H | H | N | H | H | H | Br | H | heptafluoroisopropyl | H | CF3 |
| 16-1131 | 2-bromo-4-chlorophenyl | H | H | N | H | H | H | Br | H | heptafluoroisopropyl | H | CF3 |
| 16-1132 | 2-bromo-4-fluorophenyl | H | H | N | H | H | H | Br | H | heptafluoroisopropyl | H | CF3 |
| 16-1133 | 2-chloro-4-nitrophenyl | H | H | N | H | H | H | Br | H | heptafluoroisopropyl | H | CF3 |
| 16-1134 | 3,5-dicyanophenyl | H | H | N | H | H | H | Br | H | heptafluoroisopropyl | H | CF3 |
| 16-1135 | 4-cyano-2-fluorophenyl | H | H | N | H | H | H | Br | H | heptafluoroisopropyl | H | CF3 |
| 16-1136 | 2-chloro-4-cyanophenyl | H | H | N | H | H | H | Br | H | heptafluoroisopropyl | H | CF3 |
| 16-1137 | pyridin-3-yl | H | H | N | H | H | H | Br | H | heptafluoroisopropyl | H | CF3 |
| 16-1138 | 2-fluoropyridin-3-yl | H | H | N | H | H | H | Br | H | heptafluoroisopropyl | H | CF3 |
| 16-1139 | 2-chloropyridin-3-yl | H | H | N | H | H | H | Br | H | heptafluoroisopropyl | H | CF3 |
| 16-1140 | 2-bromopyridin-3-yl | H | H | N | H | H | H | Br | H | heptafluoroisopropyl | H | CF3 |
| 16-1141 | 2-iodopyridin-3-yl | H | H | N | H | H | H | Br | H | heptafluoroisopropyl | H | CF3 |
| 16-1142 | 2-(trifluoromethyl)pyridin-3-yl | H | H | N | H | H | H | Br | H | heptafluoroisopropyl | H | CF3 |
| 16-1143 | 2-nitropyridin-3-yl | H | H | N | H | H | H | Br | H | heptafluoroisopropyl | H | CF3 |
| 16-1144 | 2-cyanopyridin-3-yl | H | H | N | H | H | H | Br | H | heptafluoroisopropyl | H | CF3 |
| 16-1145 | 6-fluoropyridin-3-yl | H | H | N | H | H | H | Br | H | heptafluoroisopropyl | H | CF3 |
| 16-1146 | 6-chloropyridin-3-yl | H | H | N | H | H | H | Br | H | heptafluoroisopropyl | H | CF3 |
| 16-1147 | 6-bromopyridin-3-yl | H | H | N | H | H | H | Br | H | heptafluoroisopropyl | H | CF3 |
| 16-1148 | 6-iodopyridin-3-yl | H | H | N | H | H | H | Br | H | heptafluoroisopropyl | H | CF3 |
| 16-1149 | 6-(trifluoromethyl)pyridin-3-yl | H | H | N | H | H | H | Br | H | heptafluoroisopropyl | H | CF3 |
| 16-1150 | 6-nitropyridin-3-yl | H | H | N | H | H | H | Br | H | heptafluoroisopropyl | H | CF3 |
| 16-1151 | 6-cyanopyridin-3-yl | H | H | N | H | H | H | Br | H | heptafluoroisopropyl | H | CF3 |
| 16-1152 | 5-fluoropyridin-3-yl | H | H | N | H | H | H | Br | H | heptafluoroisopropyl | H | CF3 |
| 16-1153 | 5-chloropyridin-3-yl | H | H | N | H | H | H | Br | H | heptafluoroisopropyl | H | CF3 |
| 16-1154 | 5-bromopyridin-3-yl | H | H | N | H | H | H | Br | H | heptafluoroisopropyl | H | CF3 |
| 16-1155 | 5-iodopyridin-3-yl | H | H | N | H | H | H | Br | H | heptafluoroisopropyl | H | CF3 |
| 16-1156 | 5-(trifluoromethyl)pyridin-3-yl | H | H | N | H | H | H | Br | H | heptafluoroisopropyl | H | CF3 |
| 16-1157 | 5-nitropyridin-3-yl | H | H | N | H | H | H | Br | H | heptafluoroisopropyl | H | CF3 |
| 16-1158 | 5-cyanopyridin-3-yl | H | H | N | H | H | H | Br | H | heptafluoroisopropyl | H | CF3 |

**Table 16(4)**

| compound number | Q₁ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 16-1159 | 4-fluoropyridin-3-yl | H | H | N | H | H | H | Br | H | heptafluoroisopropyl | H | CF3 |
| 16-1160 | 4-chloropyridin-3-yl | H | H | N | H | H | H | Br | H | heptafluoroisopropyl | H | CF3 |
| 16-1161 | 4-bromopyridin-3-yl | H | H | N | H | H | H | Br | H | heptafluoroisopropyl | H | CF3 |
| 16-1162 | 4-iodopyridin-3-yl | H | H | N | H | H | H | Br | H | heptafluoroisopropyl | H | CF3 |
| 16-1163 | 4-(trifluoromethyl)pyridin-3-yl | H | H | N | H | H | H | Br | H | heptafluoroisopropyl | H | CF3 |
| 16-1164 | 4-nitropyridin-3-yl | H | H | N | H | H | H | Br | H | heptafluoroisopropyl | H | CF3 |
| 16-1165 | 4-cyanopyridin-3-yl | H | H | N | H | H | H | Br | H | heptafluoroisopropyl | H | CF3 |
| 16-1166 | 2,6-dichloropyridin-3-yl | H | H | N | H | H | H | Br | H | heptafluoroisopropyl | H | CF3 |
| 16-1167 | pyridin-3-ylN-oxide | H | H | N | H | H | H | Br | H | heptafluoroisopropyl | H | CF3 |
| 16-1168 | pyridin-4-yl | H | H | N | H | H | H | Br | H | heptafluoroisopropyl | H | CF3 |
| 16-1169 | 2-chloropyridin-4-yl | H | H | N | H | H | H | Br | H | heptafluoroisopropyl | H | CF3 |
| 16-1170 | 3-bromopyridin-4-yl | H | H | N | H | H | H | Br | H | heptafluoroisopropyl | H | CF3 |
| 16-1171 | 3,5-dichloropyridin-4-yl | H | H | N | H | H | H | Br | H | heptafluoroisopropyl | H | CF3 |
| 16-1172 | 3-(trifluoromethyl)pyridin-4-yl | H | H | N | H | H | H | Br | H | heptafluoroisopropyl | H | CF3 |
| 16-1173 | 2,6-dicyanopyridin-4-yl | H | H | N | H | H | H | Br | H | heptafluoroisopropyl | H | CF3 |
| 16-1174 | pyridin-4-ylN-oxide | H | H | N | H | H | H | Br | H | heptafluoroisopropyl | H | CF3 |
| 16-1175 | pyridin-2-yl | H | H | N | H | H | H | Br | H | heptafluoroisopropyl | H | CF3 |
| 16-1176 | 3-chloropyridin-2-yl | H | H | N | H | H | H | Br | H | heptafluoroisopropyl | H | CF3 |
| 16-1177 | 4-bromopyridin-2-yl | H | H | N | H | H | H | Br | H | heptafluoroisopropyl | H | CF3 |
| 16-1178 | 5-iodopyridin-2-yl | H | H | N | H | H | H | Br | H | heptafluoroisopropyl | H | CF3 |
| 16-1179 | 6-chloropyridin-2-yl | H | H | N | H | H | H | Br | H | heptafluoroisopropyl | H | CF3 |
| 16-1180 | 4-cyanopyridin-2-yl | H | H | N | H | H | H | Br | H | heptafluoroisopropyl | H | CF3 |
| 16-1181 | phenyl | H | H | N | H | H | H | Br | H | nonafluoro-2-butyl | H | CF3 |
| 16-1182 | 2-fluorophenyl | H | H | N | H | H | H | Br | H | nonafluoro-2-butyl | H | CF3 |
| 16-1183 | 3-fluorophenyl | H | H | N | H | H | H | Br | H | nonafluoro-2-butyl | H | CF3 |
| 16-1184 | 4-fluorophenyl | H | H | N | H | H | H | Br | H | nonafluoro-2-butyl | H | CF3 |
| 16-1185 | 2-chlorophenyl | H | H | N | H | H | H | Br | H | nonafluoro-2-butyl | H | CF3 |
| 16-1186 | 3-chlorophenyl | H | H | N | H | H | H | Br | H | nonafluoro-2-butyl | H | CF3 |
| 16-1187 | 4-chlorophenyl | H | H | N | H | H | H | Br | H | nonafluoro-2-butyl | H | CF3 |
| 16-1188 | 2-bromophenyl | H | H | N | H | H | H | Br | H | nonafluoro-2-butyl | H | CF3 |
| 16-1189 | 3-bromophenyl | H | H | N | H | H | H | Br | H | nonafluoro-2-butyl | H | CF3 |
| 16-1190 | 4-bromophenyl | H | H | N | H | H | H | Br | H | nonafluoro-2-butyl | H | CF3 |
| 16-1191 | 2-iodophenyl | H | H | N | H | H | H | Br | H | nonafluoro-2-butyl | H | CF3 |
| 16-1192 | 3-iodophenyl | H | H | N | H | H | H | Br | H | nonafluoro-2-butyl | H | CF3 |
| 16-1193 | 4-iodophenyl | H | H | N | H | H | H | Br | H | nonafluoro-2-butyl | H | CF3 |
| 16-1194 | (2-trifluoromethyl)phenyl | H | H | N | H | H | H | Br | H | nonafluoro-2-butyl | H | CF3 |
| 16-1195 | (3-trifluoromethyl)phenyl | H | H | N | H | H | H | Br | H | nonafluoro-2-butyl | H | CF3 |
| 16-1196 | (4-trifluoromethyl)phenyl | H | H | N | H | H | H | Br | H | nonafluoro-2-butyl | H | CF3 |
| 16-1197 | 2-nitrophenyl | H | H | N | H | H | H | Br | H | nonafluoro-2-butyl | H | CF3 |
| 16-1198 | 3-nitrophenyl | H | H | N | H | H | H | Br | H | nonafluoro-2-butyl | H | CF3 |
| 16-1199 | 4-nitrophenyl | H | H | N | H | H | H | Br | H | nonafluoro-2-butyl | H | CF3 |
| 16-1200 | 2-cyanophenyl | H | H | N | H | H | H | Br | H | nonafluoro-2-butyl | H | CF3 |
| 16-1201 | 3-cyanophenyl | H | H | N | H | H | H | Br | H | nonafluoro-2-butyl | H | CF3 |
| 16-1202 | 4-cyanophenyl | H | H | N | H | H | H | Br | H | nonafluoro-2-butyl | H | CF3 |
| 16-1203 | 2,6-difluorophenyl | H | H | N | H | H | H | Br | H | nonafluoro-2-butyl | H | CF3 |
| 16-1204 | 3,4-dichlorophenyl | H | H | N | H | H | H | Br | H | nonafluoro-2-butyl | H | CF3 |
| 16-1205 | 2,4-dichlorophenyl | H | H | N | H | H | H | Br | H | nonafluoro-2-butyl | H | CF3 |
| 16-1206 | 2-chloro-4-fluorophenyl | H | H | N | H | H | H | Br | H | nonafluoro-2-butyl | H | CF3 |
| 16-1207 | 2-chloro-4,5-difluorophenyl | H | H | N | H | H | H | Br | H | nonafluoro-2-butyl | H | CF3 |
| 16-1208 | 4-bromo-2-chlorophenyl | H | H | N | H | H | H | Br | H | nonafluoro-2-butyl | H | CF3 |
| 16-1209 | 2-bromo-4-chlorophenyl | H | H | N | H | H | H | Br | H | nonafluoro-2-butyl | H | CF3 |
| 16-1210 | 2-bromo-4-fluorophenyl | H | H | N | H | H | H | Br | H | nonafluoro-2-butyl | H | CF3 |
| 16-1211 | 2-chloro-4-nitrophenyl | H | H | N | H | H | H | Br | H | nonafluoro-2-butyl | H | CF3 |
| 16-1212 | 3,5-dicyanophenyl | H | H | N | H | H | H | Br | H | nonafluoro-2-butyl | H | CF3 |
| 16-1213 | 4-cyano-2-fluorophenyl | H | H | N | H | H | H | Br | H | nonafluoro-2-butyl | H | CF3 |
| 16-1214 | 2-chloro-4-cyanophenyl | H | H | N | H | H | H | Br | H | nonafluoro-2-butyl | H | CF3 |
| 16-1215 | pyridin-3-yl | H | H | N | H | H | H | Br | H | nonafluoro-2-butyl | H | CF3 |
| 16-1216 | 2-fluoropyridin-3-yl | H | H | N | H | H | H | Br | H | nonafluoro-2-butyl | H | CF3 |
| 16-1217 | 2-chloropyridin-3-yl | H | H | N | H | H | H | Br | H | nonafluoro-2-butyl | H | CF3 |
| 16-1218 | 2-bromopyridin-3-yl | H | H | N | H | H | H | Br | H | nonafluoro-2-butyl | H | CF3 |
| 16-1219 | 2-iodopyridin-3-yl | H | H | N | H | H | H | Br | H | nonafluoro-2-butyl | H | CF3 |
| 16-1220 | 2-(trifluoromethyl)pyridin-3-yl | H | H | N | H | H | H | Br | H | nonafluoro-2-butyl | H | CF3 |
| 16-1221 | 2-nitropyridin-3-yl | H | H | N | H | H | H | Br | H | nonafluoro-2-butyl | H | CF3 |
| 16-1222 | 2-cyanopyridin-3-yl | H | H | N | H | H | H | Br | H | nonafluoro-2-butyl | H | CF3 |

**Table 16(5)**

| compound number | Q₁ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 16-1223 | 6-fluoropyridin-3-yl | H | H | N | H | H | H | Br | H | nonafluoro-2-butyl | H | CF3 |
| 16-1224 | 6-chloropyridin-3-yl | H | H | N | H | H | H | Br | H | nonafiuoro-2-butyl | H | CF3 |
| 16-1225 | 6-bromopyridin-3-yl | H | H | N | H | H | H | Br | H | nonafluoro-2-butyl | H | CF3 |
| 16-1226 | 6-iodopyridin-3-yl | H | H | N | H | H | H | Br | H | nonafluoro-2-butyl | H | CF3 |
| 16-1227 | 6-(trifluoromethyl)pyridin-3-yl | H | H | N | H | H | H | Br | H | nonafluoro-2-butyl | H | CF3 |
| 16-1228 | 6-nitropyridin-3-yl | H | H | N | H | H | H | Br | H | nonafluoro-2-butyl | H | CF3 |
| 16-1229 | 6-cyanopyridin-3-yl | H | H | N | H | H | H | Br | H | nonafluoro-2-butyl | H | CF3 |
| 16-1230 | 5-fluoropyridin-3-yl | H | H | N | H | H | H | Br | H | nonafluoro-2-butyl | H | CF3 |
| 16-1231 | 5-chloropyridin-3-yl | H | H | N | H | H | H | Br | H | nonafluoro-2-butyl | H | CF3 |
| 16-1232 | 5-bromopyridin-3-yl | H | H | N | H | H | H | Br | H | nonafluoro-2-butyl | H | CF3 |
| 16-1233 | 5-iodopyridin-3-yl | H | H | N | H | H | H | Br | H | nonafluoro-2-butyl | H | CF3 |
| 16-1234 | 5-(trifluoromethyl)pyridin-3-yl | H | H | N | H | H | H | Br | H | nonafluoro-2-butyl | H | CF3 |
| 16-1235 | 5-nitropyridin-3-yl | H | H | N | H | H | H | Br | H | nonafluoro-2-butyl | H | CF3 |
| 16-1236 | 5-cyanopyridin-3-yl | H | H | N | H | H | H | Br | H | nonafluoro-2-butyl | H | CF3 |
| 16-1237 | 4-fluoropyridin-3-yl | H | H | N | H | H | H | Br | H | nonafluoro-2-butyl | H | CF3 |
| 16-1238 | 4-chloropyridin-3-yl | H | H | N | H | H | H | Br | H | nonafluoro-2-butyl | H | CF3 |
| 16-1239 | 4-bromopyridin-3-yl | H | H | N | H | H | H | Br | H | nonafluoro-2-butyl | H | CF3 |
| 16-1240 | 4-iodopyridin-3-yl | H | H | N | H | H | H | Br | H | nonafluoro-2-butyl | H | CF3 |
| 16-1241 | 4-(trifluoromethyl)pyridin-3-yl | H | H | N | H | H | H | Br | H | nonafluoro-2-butyl | H | CF3 |
| 16-1242 | 4-nitropyridin-3-yl | H | H | N | H | H | H | Br | H | nonafluoro-2-butyl | H | CF3 |
| 16-1243 | 4-cyanopyridin-3-yl | H | H | N | H | H | H | Br | H | nonafluoro-2-butyl | H | CF3 |
| 16-1244 | 2,6-dichloropyridin-3-yl | H | H | N | H | H | H | Br | H | nonafluoro-2-butyl | H | CF3 |
| 16-1245 | pyridin-3-yl N-oxide | H | H | N | H | H | H | Br | H | nonafluoro-2-butyl | H | CF3 |
| 16-1246 | pyridin-4-yl | H | H | N | H | H | H | Br | H | nonafluoro-2-butyl | H | CF3 |
| 16-1247 | 2-chloropyridin-4-yl | H | H | N | H | H | H | Br | H | nonafluoro-2-butyl | H | CF3 |
| 16-1248 | 3-bromopyridin-4-yl | H | H | N | H | H | H | Br | H | nonafluoro-2-butyl | H | CF3 |
| 16-1249 | 3,5-dichloropyridin-4-yl | H | H | N | H | H | H | Br | H | nonafluoro-2-butyl | H | CF3 |
| 16-1250 | 3-(trifluoromethyl)pyridin-4-yl | H | H | N | H | H | H | Br | H | nonafluoro-2-butyl | H | CF3 |
| 16-1251 | 2,6-dicyanopyridin-4-yl | H | H | N | H | H | H | Br | H | nonafluoro-2-butyl | H | CF3 |
| 16-1252 | pyridin-4-yl N-oxide | H | H | N | H | H | H | Br | H | nonafluoro-2-butyl | H | CF3 |
| 16-1253 | pyridin-2-yl | H | H | N | H | H | H | Br | H | nonafluoro-2-butyl | H | CF3 |
| 16-1254 | 3-chloropyridin-2-yl | H | H | N | H | H | H | Br | H | nonafluoro-2-butyl | H | CF3 |
| 16-1255 | 4-bromopyridin-2-yl | H | H | N | H | H | H | Br | H | nonafluoro-2-butyl | H | CF3 |
| 16-1256 | 5-iodopyridin-2-yl | H | H | N | H | H | H | Br | H | nonafluoro-2-butyl | H | CF3 |
| 16-1257 | 6-chloropyridin-2-yl | H | H | N | H | H | H | Br | H | nonafluoro-2-butyl | H | CF3 |
| 16-1258 | 4-cyanopyridin-2-yl | H | H | N | H | H | H | Br | H | nonafluoro-2-butyl | H | CF3 |
| 16-1259 | phenyl | H | H | N | H | H | H | I | H | heptafluoroisopropyl | H | CF3 |
| 16-1260 | 2-fluorophenyl | H | H | N | H | H | H | I | H | heptafluoroisopropyl | H | CF3 |
| 16-1261 | 3-fluorophenyl | H | H | N | H | H | H | I | H | heptafluoroisopropyl | H | CF3 |
| 16-1262 | 4-fluorophenyl | H | H | N | H | H | H | I | H | heptafluoroisopropyl | H | CF3 |
| 16-1263 | 2-chlorophenyl | H | H | N | H | H | H | I | H | heptafluoroisopropyl | H | CF3 |
| 16-1264 | 3-chlorophenyl | H | H | N | H | H | H | I | H | heptafluoroisopropyl | H | CF3 |
| 16-1265 | 4-chlorophenyl | H | H | N | H | H | H | I | H | heptafluoroisopropyl | H | CF3 |
| 16-1266 | 2-bromophenyl | H | H | N | H | H | H | I | H | heptafluoroisopropyl | H | CF3 |
| 16-1267 | 3-bromophenyl | H | H | N | H | H | H | I | H | heptafluoroisopropyl | H | CF3 |
| 16-1268 | 4-bromophenyl | H | H | N | H | H | H | I | H | heptafluoroisopropyl | H | CF3 |
| 16-1269 | 2-iodophenyl | H | H | N | H | H | H | I | H | heptafluoroisopropyl | H | CF3 |
| 16-1270 | 3-iodophenyl | H | H | N | H | H | H | I | H | heptafluoroisopropyl | H | CF3 |
| 16-1271 | 4-iodophenyl | H | H | N | H | H | H | I | H | heptafluoroisopropyl | H | CF3 |
| 16-1272 | (2-trifluoromethyl)phenyl | H | H | N | H | H | H | I | H | heptafluoroisopropyl | H | CF3 |
| 16-1273 | (3-trifluoromethyl)phenyl | H | H | N | H | H | H | I | H | heptafluoroisopropyl | H | CF3 |
| 16-1274 | (4-trifluoromethyl)phenyl | H | H | N | H | H | H | I | H | heptafluoroisopropyl | H | CF3 |
| 16-1275 | 2-nitrophenyl | H | H | N | H | H | H | I | H | heptafluoroisopropyl | H | CF3 |
| 16-1276 | 3-nitrophenyl | H | H | N | H | H | H | I | H | heptafluoroisopropyl | H | CF3 |
| 16-1277 | 4-nitrophenyl | H | H | N | H | H | H | I | H | heptafluoroisopropyl | H | CF3 |
| 16-1278 | 2-cyanophenyl | H | H | N | H | H | H | I | H | heptafluoroisopropyl | H | CF3 |
| 16-1279 | 3-cyanophenyl | H | H | N | H | H | H | I | H | heptafluoroisopropyl | H | CF3 |
| 16-1280 | 4-cyanophenyl | H | H | N | H | H | H | I | H | heptafluoroisopropyl | H | CF3 |
| 16-1281 | 2,6-difluorophenyl | H | H | N | H | H | H | I | H | heptafluoroisopropyl | H | CF3 |
| 16-1282 | 3,4-dichlorophenyl | H | H | N | H | H | H | I | H | heptafluoroisopropyl | H | CF3 |
| 16-1283 | 2,4-dichlorophenyl | H | H | N | H | H | H | I | H | heptafluoroisopropyl | H | CF3 |
| 16-1284 | 2-chloro-4-fluorophenyl | H | H | N | H | H | H | I | H | heptafluoroisopropyl | H | CF3 |
| 16-1285 | 2-chloro-4,5-difluorophenyl | H | H | N | H | H | H | I | H | heptafluoroisopropyl | H | CF3 |
| 16-1286 | 4-bromo-2-chlorophenyl | H | H | N | H | H | H | I | H | heptafluoroisopropyl | H | CF3 |

**Table 16(6)**

| compound number | Q₁ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 16-1287 | 2-bromo-4-chlorophenyl | H | H | N | H | H | H | I | H | heptafluoroisopropyl | H | CF3 |
| 16-1288 | 2-bromo-4-fluorophenyl | H | H | N | H | H | H | I | H | heptafluoroisopropyl | H | CF3 |
| 16-1289 | 2-chloro-4-nitrophenyl | H | H | N | H | H | H | I | H | heptafluoroisopropyl | H | CF3 |
| 16-1290 | 3,5-dicyanophenyl | H | H | N | H | H | H | I | H | heptafluoroisopropyl | H | CF3 |
| 16-1291 | 4-cyano-2-fluorophenyl | H | H | N | H | H | H | I | H | heptafluoroisopropyl | H | CF3 |
| 16-1292 | 2-chloro-4-cyanophenyl | H | H | N | H | H | H | I | H | heptafluoroisopropyl | H | CF3 |
| 16-1293 | pyridin-3-yl | H | H | N | H | H | H | I | H | heptafluoroisopropyl | H | CF3 |
| 16-1294 | 2-fluoropyridin-3-yl | H | H | N | H | H | H | I | H | heptafluoroisopropyl | H | CF3 |
| 16-1295 | 2-chloropyridin-3-yl | H | H | N | H | H | H | I | H | heptafluoroisopropyl | H | CF3 |
| 16-1296 | 2-bromopyridin-3-yl | H | H | N | H | H | H | I | H | heptafluoroisopropyl | H | CF3 |
| 16-1297 | 2-iodopyridin-3-yl | H | H | N | H | H | H | I | H | heptafluoroisopropyl | H | CF3 |
| 16-1298 | 2-(trifluoromethyl)pyridin-3-yl | H | H | N | H | H | H | I | H | heptafluoroisopropyl | H | CF3 |
| 16-1299 | 2-nitropyridin-3-yl | H | H | N | H | H | H | I | H | heptafluoroisopropyl | H | CF3 |
| 16-1300 | 2-cyanopyridin-3-yl | H | H | N | H | H | H | I | H | heptafluoroisopropyl | H | CF3 |
| 16-1301 | 6-fluoropyridin-3-yl | H | H | N | H | H | H | I | H | heptafluoroisopropyl | H | CF3 |
| 16-1302 | 6-chloropyridin-3-yl | H | H | N | H | H | H | I | H | heptafluoroisopropyl | H | CF3 |
| 16-1303 | 6-bromopyridin-3-yl | H | H | N | H | H | H | I | H | heptafluoroisopropyl | H | CF3 |
| 16-1304 | 6-iodopyridin-3-yl | H | H | N | H | H | H | I | H | heptafluoroisopropyl | H | CF3 |
| 16-1305 | 6-(trifluoromethyl)pyridin-3-yl | H | H | N | H | H | H | I | H | heptafluoroisopropyl | H | CF3 |
| 16-1306 | 6-nitropyridin-3-yl | H | H | N | H | H | H | I | H | heptafluoroisopropyl | H | CF3 |
| 16-1307 | 6-cyanopyridin-3-yl | H | H | N | H | H | H | I | H | heptafluoroisopropyl | H | CF3 |
| 16-1308 | 5-fluoropyridin-3-yl | H | H | N | H | H | H | I | H | heptafluoroisopropyl | H | CF3 |
| 16-1309 | 5-chloropyridin-3-yl | H | H | N | H | H | H | I | H | heptafluoroisopropyl | H | CF3 |
| 16-1310 | 5-bromopyridin-3-yl | H | H | N | H | H | H | I | H | heptafluoroisopropyl | H | CF3 |
| 16-1311 | 5-iodopyridin-3-yl | H | H | N | H | H | H | I | H | heptafluoroisopropyl | H | CF3 |
| 16-1312 | 5-(trifluoromethyl)pyridin-3-yl | H | H | N | H | H | H | I | H | heptafluoroisopropyl | H | CF3 |
| 16-1313 | 5-nitropyridin-3-yl | H | H | N | H | H | H | 1 | H | heptafluoroisopropyl | H | CF3 |
| 16-1314 | 5-cyanopyridin-3-yl | H | H | N | H | H | H | I | H | heptafluoroisopropyl | H | CF3 |
| 16-1315 | 4-fluoropyridin-3-yl | H | H | N | H | H | H | I | H | heptafluoroisopropyl | H | CF3 |
| 16-1316 | 4-chloropyridin-3-yl | H | H | N | H | H | H | I | H | heptafluoroisopropyl | H | CF3 |
| 16-1317 | 4-bromopyridin-3-yl | H | H | N | H | H | H | I | H | heptafluoroisopropyl | H | CF3 |
| 16-1318 | 4-iodopyridin-3-yl | H | H | N | H | H | H | I | H | heptafluoroisopropyl | H | CF3 |
| 16-1319 | 4-(trifluoromethyl)pyridin-3-yl | H | H | N | H | H | H | I | H | heptafluoroisopropyl | H | CF3 |
| 16-1320 | 4-nitropyridin-3-yl | H | H | N | H | H | H | I | H | heptafluoroisopropyl | H | CF3 |
| 16-1321 | 4-cyanopyridin-3-yl | H | H | N | H | H | H | I | H | heptafluoroisopropyl | H | CF3 |
| 16-1322 | 2,6-dichloropyridin-3-yl | H | H | N | H | H | H | I | H | heptafluoroisopropyl | H | CF3 |
| 16-1323 | pyridin-3-yl N-oxide | H | H | N | H | H | H | I | H | heptafluoroisopropyl | H | CF3 |
| 16-1324 | pyridin-4-yl | H | H | N | H | H | H | I | H | heptafluoroisopropyl | H | CF3 |
| 16-1325 | 2-chloropyridin-4-yl | H | H | N | H | H | H | I | H | heptafluoroisopropyl | H | CF3 |
| 16-1326 | 3-bromopyridin-4-yl | H | H | N | H | H | H | I | H | heptafluoroisopropyl | H | CF3 |
| 16-1327 | 3,5-dichloropyridin-4-yl | H | H | N | H | H | H | I | H | heptafluoroisopropyl | H | CF3 |
| 16-1328 | 3-(trifluoromethyl)pyridin-4-yl | H | H | N | H | H | H | I | H | heptafluoroisopropyl | H | CF3 |
| 16-1329 | 2,6-dicyanopyridin-4-yl | H | H | N | H | H | H | I | H | heptafluoroisopropyl | H | CF3 |
| 16-1330 | pyridin-4-yl N-oxide | H | H | N | H | H | H | I | H | heptafluoroisopropyl | H | CF3 |
| 16-1331 | pyridin-2-yl | H | H | N | H | H | H | I | H | heptafluoroisopropyl | H | CF3 |
| 16-1332 | 3-chloropyridin-2-yl | H | H | N | H | H | H | I | H | heptafluoroisopropyl | H | CF3 |
| 16-1333 | 4-bromopyridin-2-yl | H | H | N | H | H | H | I | H | heptafluoroisopropyl | H | CF3 |
| 16-1334 | 5-iodopyridin-2-yl | H | H | N | H | H | H | I | H | heptafluoroisopropyl | H | CF3 |
| 16-1335 | 6-chloropyridin-2-yl | H | H | N | H | H | H | I | H | heptafluoroisopropyl | H | CF3 |
| 16-1336 | 4-cyanopyridin-2-yl | H | H | N | H | H | H | I | H | heptafluoroisopropyl | H | CF3 |
| 16-1337 | phenyl | H | H | N | H | H | H | I | H | nonafluoro-2-butyl | H | CF3 |
| 16-1338 | 2-fluorophenyl | H | H | N | H | H | H | I | H | nonafluoro-2-butyl | H | CF3 |
| 16-1339 | 3-fluorophenyl | H | H | N | H | H | H | I | H | nonafluoro-2-butyl | H | CF3 |
| 16-1340 | 4-fluorophenyl | H | H | N | H | H | H | I | H | nonafluoro-2-butyl | H | CF3 |
| 16-1341 | 2-chlorophenyl | H | H | N | H | H | H | I | H | nonafluoro-2-butyl | H | CF3 |
| 16-1342 | 3-chlorophenyl | H | H | N | H | H | H | I | H | nonafluoro-2-butyl | H | CF3 |
| 16-1343 | 4-chlorophenyl | H | H | N | H | H | H | I | H | nonafluoro-2-butyl | H | CF3 |
| 16-1344 | 2-bromophenyl | H | H | N | H | H | H | I | H | nonafluoro-2-butyl | H | CF3 |
| 16-1345 | 3-bromophenyl | H | H | N | H | H | H | I | H | nonafluoro-2-butyl | H | CF3 |
| 16-1346 | 4-bromophenyl | H | H | N | H | H | H | I | H | nonafluoro-2-butyl | H | CF3 |
| 16-1347 | 2-iodophenyl | H | H | N | H | H | H | I | H | nonafluoro-2-butyl | H | CF3 |
| 16-1348 | 3-iodophenyl | H | H | N | H | H | H | I | H | nonafluoro-2-butyl | H | CF3 |
| 16-1349 | 4-iodophenyl | H | H | N | H | H | H | I | H | nonafluoro-2-butyl | H | CF3 |
| 16-1350 | (2-trifluoromethyl)phenyl | H | H | N | H | H | H | I | H | nonafluoro-2-butyl | H | CF3 |

**Table 16(7)**

| compound number | Q₁ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 16-1351 | (3-trifluoromethyl) phenyl | H | H | N | H | H | H | I | H | nonafluoro-2-butyl | H | CF3 |
| 16-1352 | (4-trifluoromethyl) phenyl | H | H | N | H | H | H | I | H | nonafiuoro-2-butyl | H | CF3 |
| 16-1353 | 2-nitrophenyl | H | H | N | H | H | H | I | H | nonafluoro-2-butyl | H | CF3 |
| 16-1354 | 3-nitrophenyl | H | H | N | H | H | H | I | H | nonafluoro-2-butyl | H | CF3 |
| 16-1355 | 4-nitrophenyl | H | H | N | H | H | H | I | H | nonafluoro-2-butyl | H | CF3 |
| 16-1356 | 2-cyanophenyl | H | H | N | H | H | H | I | H | nonafluoro-2-butyl | H | CF3 |
| 16-1357 | 3-cyanophenyl | H | H | N | H | H | H | I | H | nonafluoro-2-butyl | H | CF3 |
| 16-1358 | 4-cyanophenyl | H | H | N | H | H | H | I | H | nonafluoro-2-butyl | H | CF3 |
| 16-1359 | 2,6-difluorophenyl | H | H | N | H | H | H | I | H | nonafluoro-2-butyl | H | CF3 |
| 16-1360 | 3,4-dichlorophenyl | H | H | N | H | H | H | I | H | nonafluoro-2-butyl | H | CF3 |
| 16-1361 | 2,4-dichlorophenyl | H | H | N | H | H | H | I | H | nonafluoro-2-butyl | H | CF3 |
| 16-1362 | 2-chloro-4-fluorophenyl | H | H | N | H | H | H | I | H | nonafluoro-2-butyl | H | CF3 |
| 16-1363 | 2-chloro-4,5-difluorophenyl | H | H | N | H | H | H | I | H | nonafluoro-2-butyl | H | CF3 |
| 16-1364 | 4-bromo-2-chlorophenyl | H | H | N | H | H | H | I | H | nonafluoro-2-butyl | H | CF3 |
| 16-1365 | 2-bromo-4-chlorophenyl | H | H | N | H | H | H | I | H | nonafluoro-2-butyl | H | CF3 |
| 16-1366 | 2-bromo-4-fluorophenyl | H | H | N | H | H | H | I | H | nonafluoro-2-butyl | H | CF3 |
| 16-1367 | 2-chloro-4-nitrophenyl | H | H | N | H | H | H | I | H | nonafluoro-2-butyl | H | CF3 |
| 16-1368 | 3,5-dicyanophenyl | H | H | N | H | H | H | I | H | nonafluoro-2-butyl | H | CF3 |
| 16-1369 | 4-cyano-2-fluorophenyl | H | H | N | H | H | H | I | H | nonafluoro-2-butyl | H | CF3 |
| 16-1370 | 2-chloro-4-cyanophenyl | H | H | N | H | H | H | I | H | nonafluoro-2-butyl | H | CF3 |
| 16-1371 | pyridin-3-yl | H | H | N | H | H | H | I | H | nonafluoro-2-butyl | H | CF3 |
| 16-1372 | 2-fluoropyridin-3-yl | H | H | N | H | H | H | I | H | nonafluoro-2-butyl | H | CF3 |
| 16-1373 | 2-chloropyridin-3-yl | H | H | N | H | H | H | I | H | nonafluoro-2-butyl | H | CF3 |
| 16-1374 | 2-bromopyridin-3-yl | H | H | N | H | H | H | I | H | nonafluoro-2-butyl | H | CF3 |
| 16-1375 | 2-iodopyridin-3-yl | H | H | N | H | H | H | I | H | nonafluoro-2-butyl | H | CF3 |
| 16-1376 | 2-(trifluoromethyl)pyridin-3-yl | H | H | N | H | H | H | I | H | nonafluoro-2-butyl | H | CF3 |
| 16-1377 | 2-nitropyridin-3-yl | H | H | N | H | H | H | I | H | nonafluoro-2-butyl | H | CF3 |
| 16-1378 | 2-cyanopyridin-3-yl | H | H | N | H | H | H | I | H | nonafluoro-2-butyl | H | CF3 |
| 16-1379 | 6-fluoropyridin-3-yl | H | H | N | H | H | H | I | H | nonafluoro-2-butyl | H | CF3 |
| 16-1380 | 6-chloropyridin-3-yl | H | H | N | H | H | H | I | H | nonafluoro-2-butyl | H | CF3 |
| 16-1381 | 6-bromopyridin-3-yl | H | H | N | H | H | H | I | H | nonafluoro-2-butyl | H | CF3 |
| 16-1382 | 6-iodopyridin-3-yl | H | H | N | H | H | H | I | H | nonafluoro-2-butyl | H | CF3 |
| 16-1383 | 6-(trifluoromethyl)pyridin-3-yl | H | H | N | H | H | H | I | H | nonafluoro-2-butyl | H | CF3 |
| 16-1384 | 6-nitropyridin-3-yl | H | H | N | H | H | H | I | H | nonafluoro-2-butyl | H | CF3 |
| 16-1385 | 6-cyanopyridin-3-yl | H | H | N | H | H | H | I | H | nonafluoro-2-butyl | H | CF3 |
| 16-1386 | 5-fluoropyridin-3-yl | H | H | N | H | H | H | I | H | nonafluoro-2-butyl | H | CF3 |
| 16-1387 | 5-chloropyridin-3-yl | H | H | N | H | H | H | I | H | nonafluoro-2-butyl | H | CF3 |
| 16-1388 | 5-bromopyridin-3-yl | H | H | N | H | H | H | I | H | nonafluoro-2-butyl | H | CF3 |
| 16-1389 | 5-iodopyridin-3-yl | H | H | N | H | H | H | I | H | nonafluoro-2-butyl | H | CF3 |
| 16-1390 | 5-(trifluoromethyl)pyridin-3-yl | H | H | N | H | H | H | I | H | nonafluoro-2-butyl | H | CF3 |
| 16-1391 | 5-nitropyridin-3-yl | H | H | N | H | H | H | I | H | nonafluoro-2-butyl | H | CF3 |
| 16-1392 | 5-cyanopyridin-3-yl | H | H | N | H | H | H | I | H | nonafluoro-2-butyl | H | CF3 |
| 16-1393 | 4-fluoropyridin-3-yl | H | H | N | H | H | H | I | H | nonafluoro-2-butyl | H | CF3 |
| 16-1394 | 4-chloropyridin-3-yl | H | H | N | H | H | H | I | H | nonafluoro-2-butyl | H | CF3 |
| 16-1395 | 4-bromopyridin-3-yl | H | H | N | H | H | H | I | H | nonafluoro-2-butyl | H | CF3 |
| 16-1396 | 4-iodopyridin-3-yl | H | H | N | H | H | H | I | H | nonafluoro-2-butyl | H | CF3 |
| 16-1397 | 4-(trifluoromethyl)pyridin-3-yl | H | H | N | H | H | H | I | H | nonafluoro-2-butyl | H | CF3 |
| 16-1398 | 4-nitropyridin-3-yl | H | H | N | H | H | H | I | H | nonafluoro-2-butyl | H | CF3 |
| 16-1399 | 4-cyanopyridin-3-yl | H | H | N | H | H | H | I | H | nonafluoro-2-butyl | H | CF3 |
| 16-1400 | 2,6-dichloropyridin-3-yl | H | H | N | H | H | H | I | H | nonafluoro-2-butyl | H | CF3 |
| 16-1401 | pyridin-3-yl N-oxide | H | H | N | H | H | H | I | H | nonafluoro-2-butyl | H | CF3 |
| 16-1402 | pyridin-4-yl | H | H | N | H | H | H | I | H | nonafluoro-2-butyl | H | CF3 |
| 16-1403 | 2-chloropyridin-4-yl | H | H | N | H | H | H | I | H | nonafluoro-2-butyl | H | CF3 |
| 16-1404 | 3-bromopyridin-4-yl | H | H | N | H | H | H | I | H | nonafluoro-2-butyl | H | CF3 |
| 16-1405 | 3,5-dichloropyridin-4-yl | H | H | N | H | H | H | I | H | nonafluoro-2-butyl | H | CF3 |
| 16-1406 | 3-(trifluoromethyl)pyridin-4-yl | H | H | N | H | H | H | I | H | nonafluoro-2-butyl | H | CF3 |
| 16-1407 | 2,6-dicyanopyridin-4-yl | H | H | N | H | H | H | I | H | nonafluoro-2-butyl | H | CF3 |
| 16-1408 | pyridin-4-yl N-oxide | H | H | N | H | H | H | I | H | nonafluoro-2-butyl | H | CF3 |
| 16-1409 | pyridin-2-yl | H | H | N | H | H | H | I | H | nonafluoro-2-butyl | H | CF3 |
| 16-1410 | 3-chloropyridin-2-yl | H | H | N | H | H | H | I | H | nonafluoro-2-butyl | H | CF3 |
| 16-1411 | 4-bromopyridin-2-yl | H | H | N | H | H | H | I | H | nonafluoro-2-butyl | H | CF3 |
| 16-1412 | 5-iodopyridin-2-yl | H | H | N | H | H | H | I | H | nonafluoro-2-butyl | H | CF3 |
| 16-1413 | 6-chloropyridin-2-yl | H | H | N | H | H | H | I | H | nonafluoro-2-butyl | H | CF3 |

**Table 16(8)**

| compound number | Q₁ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 16-1414 | 4-cyanopyridin-2-yl | H | H | N | H | H | H | I | H | nonafluoro-2-butyl | H | CF3 |
| 16-1415 | phenyl | H | H | N | H | H | H | OCF3 | H | heptafluoroisopropyl | H | OCF3 |
| 16-1422 | 2-bromophenyl | H | H | N | H | H | H | OCF3 | H | heptafluoroisopropyl | H | OC2F5 |
| 16-1427 | 4-iodophenyl | H | H | N | H | H | H | OCF3 | H | pentafluoroethyl | H | OCF3 |
| 16-1430 | 3-cyanophenyl | H | H | N | H | H | H | OCF3 | H | heptafluoroisopropyl | H | OCF3 |
| 16-1431 | 4-cyanophenyl | H | H | N | H | H | H | OCF3 | H | heptafluoroisopropyl | H | OCF3 |
| 16-1436 | 2-chloro-4,5-difluorophenyl | H | H | N | H | H | H | OC2F5 | H | heptafluoroisopropyl | H | OC2F5 |
| 16-1439 | 2-bromo-4-fluorophenyl | H | H | N | H | H | H | OC2F5 | H | heptafluoroisopropyl | H | OCF3 |
| 16-1445 | 2-fluoropyridin-3-yl | H | H | N | H | H | H | OCF3 | H | pentafluoroethyl | H | OCF3 |
| 16-1446 | 2-chloropyridin-3-yl | H | H | N | H | H | H | OCF3 | H | heptafluoroisopropyl | H | OCF3 |
| 16-1471 | phenyl | H | H | N | H | H | H | OCF3 | H | nonafluoro-2-butyl | H | OCF3 |
| 16-1478 | 2-bromophenyl | H | H | N | H | H | H | OC2F5 | H | nonafluoro-2-butyl | H | OCF3 |
| 16-1484 | (2-trifluoromethyl) phenyl | H | H | N | H | H | H | OCF3 | H | nonafluoro-2-butyl | H | OC2F5 |
| 16-1486 | 3-cyanophenyl | H | H | N | H | H | H | OCF3 | H | nonafluoro-2-butyl | H | OCF3 |
| 16-1487 | 4-cyanophenyl | H | H | N | H | H | H | OCF3 | H | nonafluoro-2-butyl | H | OCF3 |
| 16-1493 | 4-bromo-2-chlorophenyl | H | H | N | H | H | H | OC2F5 | H | nonafluoro-2-butyl | H | OC2F5 |
| 16-1502 | 2-chloropyridin-3-yl | H | H | N | H | H | H | OCF3 | H | nonafluoro-2-butyl | H | OCF3 |
| 16-1527 | phenyl | H | H | N | H | H | H | CF3 | H | heptafluoroisopropyl | H | OCF3 |
| 16-1534 | 2-bromophenyl | H | H | N | H | H | H | CF3 | H | heptafluoroisopropyl | H | OC2F5 |
| 16-1539 | 4-iodophenyl | H | H | N | H | H | H | CF3 | H | pentafluoroethyl | H | OCF3 |
| 16-1540 | (2-trifluoromethyl) phenyl | H | H | N | H | H | H | C2F5 | H | heptafluoroisopropyl | H | OC2F5 |
| 16-1542 | 3-cyanophenyl | H | H | N | H | H | H | CF3 | H | heptafluoroisopropyl | H | OCF3 |
| 16-1543 | 4-cyanophenyl | H | H | N | H | H | H | CF3 | H | heptafluoroisopropyl | H | OCF3 |
| 16-1550 | 2-bromo-4-chlorophenyl | H | H | N | H | H | H | n-C3F7 | H | heptafluoroisopropyl | H | OCF3 |
| 16-1554 | 4-cyano-2-fluorophenyl | H | H | N | H | H | H | i-C3F7 | H | heptafluoroisopropyl | H | OCF3 |
| 16-1558 | 2-chloropyridin-3-yl | H | H | N | H | H | H | CF3 | H | heptafluoroisopropyl | H | OCF3 |
| 16-1564 | 6-chloropyridin-3-yl | H | H | N | H | H | H | n-C3F7 | H | heptafluoroisopropyl | H | OC2F5 |
| 16-1567 | 5-cyanopyridin-3-yl | H | H | N | H | H | H | i-C3F7 | H | heptafluoroisopropyl | H | OC2F5 |
| 16-1568 | 4-chloropyridin-3-yl | H | H | N | H | H | H | CF3 | H | heptafluoroisopropyl | H | OCF3 |
| 16-1583 | phenyl | H | H | N | H | H | H | CF3 | H | nonafluoro-2-butyl | H | OCF3 |
| 16-1594 | 3-iodophenyl | H | H | N | H | H | H | CF3 | H | nonafluoro-2-butyl | H | OC2F5 |
| 16-1597 | 3-nitrophenyl | H | H | N | H | H | H | C2F5 | H | nonafluoro-2-butyl | H | OCF3 |
| 16-1598 | 3-cyanophenyl | H | H | N | H | H | H | CF3 | H | nonafluoro-2-butyl | H | OCF3 |
| 16-1599 | 4-cyanophenyl | H | H | N | H | H | H | CF3 | H | nonafluoro-2-butyl | H | OCF3 |
| 16-1605 | 4-bromo-2-chlorophenyl | H | H | N | H | H | H | i-C3F7 | H | nonafluoro-2-butyl | H | OCF3 |
| 16-1608 | 2-chloro-4-nitrophenyl | H | H | N | H | H | H | n-C3F7 | H | nonafluoro-2-butyl | H | OCF3 |
| 16-1614 | 2-chloropyridin-3-yl | H | H | N | H | H | H | CF3 | H | nonafluoro-2-butyl | H | OCF3 |
| 16-1615 | 2-bromopyridin-3-yl | H | H | N | H | H | H | CF3 | H | nonafluoro-2-butyl | H | OCF3 |
| 16-1616 | 2-iodopyridin-3-yl | H | H | N | H | H | H | CF3 | H | nonafluoro-2-butyl | H | OCF3 |
| 16-1617 | 2-(trifluoromethyl)pyridin-3-yl | H | H | N | H | H | H | CF3 | H | nonafluoro-2-butyl | H | OCF3 |
| 16-1618 | 2-nitropyridin-3-yl | H | H | N | H | H | H | CF3 | H | nonafluoro-2-butyl | H | OCF3 |
| 16-1619 | 2-cyanopyridin-3-yl | H | H | N | H | H | H | CF3 | H | nonafluoro-2-butyl | H | OCF3 |
| 16-1620 | 6-chloropyridin-3-yl | H | H | N | H | H | H | C2F5 | H | nonafluoro-2-butyl | H | OC2F5 |
| 16-1625 | 4-cyanopyridin-3-yl | H | H | N | H | H | H | n-C3F7 | H | nonafluoro-2-butyl | H | OC2F5 |
| 16-1636 | 5-iodopyridin-2-yl | H | H | N | H | H | H | i-C3F7 | H | nonafluoro-2-butyl | H | OC2F5 |
| 16-1639 | phenyl | H | H | N | H | H | H | CF3 | H | heptafluoroisopropyl | H | CF3 |
| 16-1640 | 2-fluorophenyl | H | H | N | H | H | H | CF3 | H | heptafluoroisopropyl | H | CF3 |
| 16-1651 | 4-iodophenyl | H | H | N | H | H | H | CF3 | H | pentafluoroethyl | H | CF3 |
| 16-1653 | (3-trifluoromethyl) phenyl | H | H | N | H | H | H | C2F5 | H | heptafluoroisopropyl | H | CF3 |
| 16-1658 | 2-cyanophenyl | H | H | N | H | H | H | CF3 | H | pentafluoroethyl | H | CF3 |
| 16-1659 | 3-cyanophenyl | H | H | N | H | H | H | CF3 | H | heptafluoroisopropyl | H | CF3 |
| 16-1660 | 4-cyanophenyl | H | H | N | H | H | H | CF3 | H | heptafluoroisopropyl | H | CF3 |
| 16-1666 | 4-bromo-2-chlorophenyl | H | H | N | H | H | H | n-C3F7 | H | heptafluoroisopropyl | H | CF3 |
| 16-1671 | 4-cyano-2-fluorophenyl | H | H | N | H | H | H | i-C3F7 | H | heptafluoroisopropyl | H | CF3 |
| 16-1675 | 2-chloropyridin-3-yl | H | H | N | H | H | H | CF3 | H | heptafluoroisopropyl | H | CF3 |
| 16-1676 | 2-bromopyridin-3-yl | H | H | N | H | H | H | C2F5 | H | heptafluoroisopropyl | H | C2F5 |
| 16-1677 | 2-iodopyridin-3-yl | H | H | N | H | H | H | CF3 | H | heptafluoroisopropyl | H | CF3 |
| 16-1678 | 2-(trifluoromethyl)pyridin-3-yl | H | H | N | H | H | H | CF3 | H | heptafluoroisopropyl | H | CF3 |
| 16-1679 | 2-nitropyridin-3-yl | H | H | N | H | H | H | CF3 | H | heptafluoroisopropyl | H | CF3 |
| 16-1680 | 2-cyanopyridin-3-yl | H | H | N | H | H | H | CF3 | H | heptafluoroisopropyl | H | CF3 |
| 16-1681 | 6-fluoropyridin-3-yl | H | H | N | H | H | H | CF3 | H | heptafluoroisopropyl | H | CF3 |
| 16-1682 | 6-chloropyridin-3-yl | H | H | N | H | H | H | C2F5 | H | heptafluoroisopropyl | H | n-C3F7 |
| 16-1691 | 4-cyanopyridin-3-yl | H | H | N | H | H | H | C2F5 | H | heptafluoroisopropyl | H | i-C3F7 |
| 16-1707 | phenyl | H | H | N | H | H | H | CF3 | H | nonafluoro-2-butyl | H | CF3 |
| 16-1708 | 2-fluorophenyl | H | H | N | H | H | H | CF3 | H | nonafluoro-2-butyl | H | CF3 |

**Table 16(9)**

| compound number | Q₁ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 16-1719 | 4-iodophenyl | H | H | N | H | H | H | C2F5 | H | nonafluoro-2-butyl | H | CF3 |
| 16-1727 | 3-cyanophenyl | H | H | N | H | H | H | CF3 | H | nonafluoro-2-butyl | H | CF3 |
| 16-1728 | 4-cyanophenyl | H | H | N | H | H | H | CF3 | H | nonafluoro-2-butyl | H | CF3 |
| 16-1734 | 4-bromo-2-chlorophenyl | H | H | N | H | H | H | n-C3F7 | H | nonafluoro-2-butyl | H | CF3 |
| 16-1739 | 4-cyano-2-fluorophenyl | H | H | N | H | H | H | i-C3F7 | H | nonafluoro-2-butyl | H | CF3 |
| 16-1743 | 2-chloropyridin-3-yl | H | H | N | H | H | H | CF3 | H | nonafluoro-2-butyl | H | CF3 |
| 16-1744 | 2-bromopyridin-3-yl | H | H | N | H | H | H | C2F5 | H | nonafluoro-2-butyl | H | C2F5 |
| 16-1755 | 6-cyanopyridin-3-yl | H | H | N | H | H | H | C2F5 | H | nonafluoro-2-butyl | H | n-C3F7 |
| 16-1759 | 4-cyanopyridin-3-yl | H | H | N | H | H | H | C2F5 | H | nonafluoro-2-butyl | H | i-C3F7 |
| 16-1775 | phenyl | H | H | N-oxide | H | H | H | F | H | heptafluoroisopropyl | H | F |
| 16-1776 | 3-cyanophenyl | H | H | N-oxide | H | H | H | F | H | heptafluoroisopropyl | H | F |
| 16-1777 | 4-cyanophenyl | H | H | N-oxide | H | H | H | F | H | heptafluoroisopropyl | H | F |
| 16-1778 | 2-chloropyridin-3-yl | H | H | N-oxide | H | H | H | F | H | heptafluoroisopropyl | H | F |
| 16-1779 | phenyl | H | H | N-oxide | H | H | H | F | H | nonafluoro-2-butyl | H | F |
| 16-1780 | 3-cyanophenyl | H | H | N-oxide | H | H | H | F | H | nonafluoro-2-butyl | H | F |
| 16-1781 | 4-cyanophenyl | H | H | N-oxide | H | H | H | F | H | nonafluoro-2-butyl | H | F |
| 16-1782 | 2-chloropyridin-3-yl | H | H | N-oxide | H | H | H | F | H | nonafluoro-2-butyl | H | F |
| 16-1783 | phenyl | H | H | N-oxide | H | H | H | Cl | H | heptafluoroisopropyl | H | Cl |
| 16-1787 | 2-chlorophenyl | H | H | N-oxide | H | H | H | F | H | heptafluoroisopropyl | H | Cl |
| 16-1791 | 3-bromophenyl | H | H | N-oxide | H | H | H | F | H | heptafluoroisopropyl | H | Br |
| 16-1795 | 4-iodophenyl | H | H | N-oxide | H | H | H | Cl | H | pentafluoroethyl | H | Cl |
| 16-1796 | (2-trifluoromethyl)phenyl | H | H | N-oxide | H | H | H | F | H | heptafluoroisopropyl | H | I |
| 16-1799 | 2-nitrophenyl | H | H | N-oxide | H | H | H | Cl | H | heptafluoroisopropyl | H | Br |
| 16-1803 | 3-cyanophenyl | H | H | N-oxide | H | H | H | Cl | H | heptafluoroisopropyl | H | Cl |
| 16-1804 | 4-cyanophenyl | H | H | N-oxide | H | H | H | Cl | H | heptafluoroisopropyl | H | Cl |
| 16-1805 | 2,6-difluorophenyl | H | H | N-oxide | H | H | H | Cl | H | heptafluoroisopropyl | H | I |
| 16-1817 | pyridin-3-yl | H | H | N-oxide | H | H | H | Br | H | heptafluoroisopropyl | H | I |
| 16-1818 | 2-fluoropyridin-3-yl | H | H | N-oxide | H | H | H | Cl | H | pentafluoroethyl | H | Cl |
| 16-1819 | 2-chloropyridin-3-yl | H | H | N-oxide | H | H | H | Cl | H | heptafluoroisopropyl | H | Cl |
| 16-1861 | phenyl | H | H | N-oxide | H | H | H | Cl | H | nonafluoro-2-butyl | H | Cl |
| 16-1881 | 3-cyanophenyl | H | H | N-oxide | H | H | H | Cl | H | nonafluoro-2-butyl | H | Cl |
| 16-1882 | 4-cyanophenyl | H | H | N-oxide | H | H | H | Cl | H | nonafluoro-2-butyl | H | Cl |
| 16-1897 | 2-chloropyridin-3-yl | H | H | N-oxide | H | H | H | Cl | H | nonafluoro-2-butyl | H | Cl |
| 16-1939 | phenyl | H | H | N-oxide | H | H | H | Br | H | heptafluoroisopropyl | H | Br |
| 16-1951 | 4-iodophenyl | H | H | N-oxide | H | H | H | Br | H | pentafluoroethyl | H | Br |
| 16-1959 | 3-cyanophenyl | H | H | N-oxide | H | H | H | Br | H | heptafluoroisopropyl | H | Br |
| 16-1960 | 4-cyanophenyl | H | H | N-oxide | H | H | H | Br | H | heptafluoroisopropyl | H | Br |
| 16-1973 | pyridin-3-yl | H | H | N-oxide | H | H | H | Br | H | heptafluoroisopropyl | H | Br |
| 16-1975 | 2-chloropyridin-3-yl | H | H | N-oxide | H | H | H | Br | H | heptafluoroisopropyl | H | Br |
| 16-1982 | 6-chloropyridin-3-yl | H | H | N-oxide | H | H | H | Br | H | heptafluoroisopropyl | H | Br |
| 16-2017 | phenyl | H | H | N-oxide | H | H | H | Br | H | nonafluoro-2-butyl | H | Br |
| 16-2037 | 3-cyanophenyl | H | H | N-oxide | H | H | H | Br | H | nonafluoro-2-butyl | H | Br |
| 16-2038 | 4-cyanophenyl | H | H | N-oxide | H | H | H | Br | H | nonafluoro-2-butyl | H | Br |
| 16-2053 | 2-chloropyridin-3-yl | H | H | N-oxide | H | H | H | Br | H | nonafluoro-2-butyl | H | Br |
| 16-2095 | phenyl | H | H | N-oxide | H | H | H | I | H | heptafluoroisopropyl | H | I |
| 16-2107 | 4-iodophenyl | H | H | N-oxide | H | H | H | I | H | pentafluoroethyl | H | I |
| 16-2115 | 3-cyanophenyl | H | H | N-oxide | H | H | H | I | H | heptafluoroisopropyl | H | I |
| 16-2116 | 4-cyanophenyl | H | H | N-oxide | H | H | H | I | H | heptafluoroisopropyl | H | I |
| 16-2131 | 2-chloropyridin-3-yl | H | H | N-oxide | H | H | H | I | H | heptafluoroisopropyl | H | I |
| 16-2160 | pyridin-4-yl | H | H | N-oxide | H | H | H | I | H | heptafluoroisopropyl | H | I |
| 16-2173 | phenyl | H | H | N-oxide | H | H | H | I | H | nonafluoro-2-butyl | H | I |
| 16-2193 | 3-cyanophenyl | H | H | N-oxide | H | H | H | I | H | nonafluoro-2-butyl | H | I |
| 16-2194 | 4-cyanophenyl | H | H | N-oxide | H | H | H | I | H | nonafluoro-2-butyl | H | I |
| 16-2209 | 2-chloropyridin-3-yl | H | H | N-oxide | H | H | H | I | H | nonafluoro-2-butyl | H | I |
| 16-2251 | phenyl | H | H | N-oxide | H | H | H | Cl | H | heptafluoroisopropyl | H | OCF3 |
| 16-2263 | 4-iodophenyl | H | H | N-oxide | H | H | H | Cl | H | pentafluoroethyl | H | OCF3 |
| 16-2266 | (4-trifluoromethyl)phenyl | H | H | N-oxide | H | H | H | Cl | H | heptafluoroisopropyl | H | OC2F5 |
| 16-2267 | 2-nitrophenyl | H | H | N-oxide | H | H | H | F | H | heptafluoroisopropyl | H | OCF3 |
| 16-2270 | 2-cyanophenyl | H | H | N-oxide | H | H | H | Cl | H | pentafluoroethyl | H | OC2F5 |
| 16-2271 | 3-cyanophenyl | H | H | N-oxide | H | H | H | Cl | H | heptafluoroisopropyl | H | OCF3 |
| 16-2272 | 4-cyanophenyl | H | H | N-oxide | H | H | H | Cl | H | heptafluoroisopropyl | H | OCF3 |
| 16-2287 | 2-chloropyridin-3-yl | H | H | N-oxide | H | H | H | Cl | H | heptafluoroisopropyl | H | OCF3 |
| 16-2329 | phenyl | H | H | N-oxide | H | H | H | Cl | H | nonafluoro-2-butyl | H | OCF3 |
| 16-2345 | 2-nitrophenyl | H | H | N-oxide | H | H | H | F | H | nonafluoro-2-butyl | H | OCF3 |

**Table 16(10)**

| compound number | Q₁ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 16-2349 | 3-cyanophenyl | H | H | N-oxide | H | H | H | Cl | H | nonafluoro-2-butyl | H | OCF3 |
| 16-2350 | 4-cyanophenyl | H | H | N-oxide | H | H | H | Cl | H | nonafluoro-2-butyl | H | OCF3 |
| 16-2365 | 2-chloropyridin-3-yl | H | H | N-oxide | H | H | H | Cl | H | nonafluoro-2-butyl | H | OCF3 |
| 16-2407 | phenyl | H | H | N-oxide | H | H | H | Br | H | heptafluoroisopropyl | H | OCF3 |
| 16-2427 | 3-cyanophenyl | H | H | N-oxide | H | H | H | Br | H | heptafluoroisopropyl | H | OCF3 |
| 16-2428 | 4-cyanophenyl | H | H | N-oxide | H | H | H | Br | H | heptafluoroisopropyl | H | OCF3 |
| 16-2444 | 2-bromopyridin-3-yl | H | H | N-oxide | H | H | H | Br | H | heptafluoroisopropyl | H | OCF3 |
| 16-2485 | phenyl | H | H | N-oxide | H | H | H | Br | H | nonafluoro-2-butyl | H | OCF3 |
| 16-2505 | 3-cyanophenyl | H | H | N-oxide | H | H | H | Br | H | nonafluoro-2-butyl | H | OCF3 |
| 16-2506 | 4-cyanophenyl | H | H | N-oxide | H | H | H | Br | H | nonafluoro-2-butyl | H | OCF3 |
| 16-2521 | 2-chloropyridin-3-yl | H | H | N-oxide | H | H | H | Br | H | nonafluoro-2-butyl | H | OCF3 |
| 16-2563 | phenyl | H | H | N-oxide | H | H | H | I | H | heptafluoroisopropyl | H | OCF3 |
| 16-2583 | 3-cyanophenyl | H | H | N-oxide | H | H | H | I | H | heptafluoroisopropyl | H | OCF3 |
| 16-2584 | 4-cyanophenyl | H | H | N-oxide | H | H | H | I | H | heptafluoroisopropyl | H | OCF3 |
| 16-2599 | 2-chloropyridin-3-yl | H | H | N-oxide | H | H | H | I | H | heptafluoroisopropyl | H | OCF3 |
| 16-2641 | phenyl | H | H | N-oxide | H | H | H | I | H | nonafluoro-2-butyl | H | OCF3 |
| 16-2661 | 3-cyanophenyl | H | H | N-oxide | H | H | H | I | H | nonafluoro-2-butyl | H | OCF3 |
| 16-2662 | 4-cyanophenyl | H | H | N-oxide | H | H | H | I | H | nonafluoro-2-butyl | H | OCF3 |
| 16-2677 | 2-chloropyridin-3-yl | H | H | N-oxide | H | H | H | I | H | nonafluoro-2-butyl | H | OCF3 |
| 16-2719 | phenyl | H | H | N-oxide | H | H | H | F | H | heptafluoroisopropyl | H | CF3 |
| 16-2720 | phenyl | H | H | N-oxide | H | H | H | Cl | H | heptafluoroisopropyl | H | CF3 |
| 16-2721 | 2-fluorophenyl | H | H | N-oxide | H | H | H | Cl | H | heptafluoroisopropyl | H | CF3 |
| 16-2732 | 4-iodophenyl | H | H | N-oxide | H | H | H | Cl | H | pentafluoroethyl | H | CF3 |
| 16-2739 | 2-cyanophenyl | H | H | N-oxide | H | H | H | Cl | H | pentafluoroethyl | H | C2F5 |
| 16-2740 | 3-cyanophenyl | H | H | N-oxide | H | H | H | Cl | H | heptafluoroisopropyl | H | CF3 |
| 16-2741 | 4-cyanophenyl | H | H | N-oxide | H | H | H | Cl | H | heptafluoroisopropyl | H | CF3 |
| 16-2744 | 2,4-dichlorophenyl | H | H | N-oxide | H | H | H | Cl | H | heptafluoroisopropyl | H | C2F5 |
| 16-2745 | 2-chloro-4-fluorophenyl | H | H | N-oxide | H | H | H | Cl | H | heptafluoroisopropyl | H | CF3 |
| 16-2748 | 2-bromo-4-chlorophenyl | H | H | N-oxide | H | H | H | Cl | H | heptafluoroisopropyl | H | n-C3F7 |
| 16-2756 | 2-chloropyridin-3-yl | H | H | N-oxide | H | H | H | Cl | H | heptafluoroisopropyl | H | CF3 |
| 16-2769 | 5-fluoropyridin-3-yl | H | H | N-oxide | H | H | H | Cl | H | heptafluoroisopropyl | H | C2F5 |
| 16-2771 | 5-bromopyridin-3-yl | H | H | N-oxide | H | H | H | Cl | H | heptafluoroisopropyl | H | n-C3F7 |
| 16-2772 | 5-iodopyridin-3-yl | H | H | N-oxide | H | H | H | Cl | H | heptafluoroisopropyl | H | i-C3F7 |
| 16-2774 | 5-nitropyridin-3-yl | H | H | N-oxide | H | H | H | Cl | H | pentafluoroethyl | H | CF3 |
| 16-2776 | 4-fluoropyridin-3-yl | H | H | N-oxide | H | H | H | Cl | H | pentafluoroethyl | H | CF3 |
| 16-2778 | 4-bromopyridin-3-yl | H | H | N-oxide | H | H | H | Cl | H | heptafluoroisopropyl | H | n-C3F7 |
| 16-2779 | 4-iodopyridin-3-yl | H | H | N-oxide | H | H | H | Cl | H | heptafluoroisopropyl | H | i-C3F7 |
| 16-2796 | 6-chloropyridin-2-yl | H | H | N-oxide | H | H | H | Cl | H | pentafluoroethyl | H | CF3 |
| 16-2798 | phenyl | H | H | N-oxide | H | H | H | F | H | nonafluoro-2-butyl | H | CF3 |
| 16-2799 | phenyl | H | H | N-oxide | H | H | H | Cl | H | nonafluoro-2-butyl | H | CF3 |
| 16-2811 | 4-iodophenyl | H | H | N-oxide | H | H | H | Cl | H | nonafluoro-2-butyl | H | CF3 |
| 16-2812 | (2-trifluoromethyl)phenyl | H | H | N-oxide | H | H | H | Cl | H | nonafluoro-2-butyl | H | C2F5 |
| 16-2819 | 3-cyanophenyl | H | H | N-oxide | H | H | H | Cl | H | nonafluoro-2-butyl | H | CF3 |
| 16-2820 | 4-cyanophenyl | H | H | N-oxide | H | H | H | Cl | H | nonafluoro-2-butyl | H | CF3 |
| 16-2829 | 2-chloro-4-nitrophenyl | H | H | N-oxide | H | H | H | Cl | H | nonafluoro-2-butyl | H | i-C3F7 |
| 16-2835 | 2-chloropyridin-3-yl | H | H | N-oxide | H | H | H | Cl | H | nonafluoro-2-butyl | H | CF3 |
| 16-2848 | 5-fluoropyridin-3-yl | H | H | N-oxide | H | H | H | Cl | H | nonafluoro-2-butyl | H | C2F5 |
| 16-2850 | 5-bromopyridin-3-yl | H | H | N-oxide | H | H | H | Cl | H | nonafluoro-2-butyl | H | n-C3F7 |
| 16-2851 | 5-iodopyridin-3-yl | H | H | N-oxide | H | H | H | Cl | H | nonafluoro-2-butyl | H | i-C3F7 |
| 16-2855 | 4-fluoropyridin-3-yl | H | H | N-oxide | H | H | H | Cl | H | nonafluoro-2-butyl | H | C2F5 |
| 16-2859 | 4-(trifluoromethyl)pyridin-3-yl | H | H | N-oxide | H | H | H | Cl | H | nonafluoro-2-butyl | H | n-C3F7 |
| 16-2860 | 4-nitropyridin-3-yl | H | H | N-oxide | H | H | H | Cl | H | nonafluoro-2-butyl | H | i-C3F7 |
| 16-2877 | phenyl | H | H | N-oxide | H | H | H | Br | H | heptafluoroisopropyl | H | CF3 |
| 16-2878 | 2-fluorophenyl | H | H | N-oxide | H | H | H | Br | H | heptafluoroisopropyl | H | CF3 |
| 16-2897 | 3-cyanophenyl | H | H | N-oxide | H | H | H | Br | H | heptafluoroisopropyl | H | CF3 |
| 16-2898 | 4-cyanophenyl | H | H | N-oxide | H | H | H | Br | H | heptafluoroisopropyl | H | CF3 |
| 16-2913 | 2-chloropyridin-3-yl | H | H | N-oxide | H | H | H | Br | H | heptafluoroisopropyl | H | CF3 |
| 16-2920 | 6-chloropyridin-3-yl | H | H | N-oxide | H | H | H | Br | H | heptafluoroisopropyl | H | CF3 |
| 16-2942 | pyridin-4-yl | H | H | N-oxide | H | H | H | Br | H | heptafluoroisopropyl | H | CF3 |
| 16-2955 | phenyl | H | H | N-oxide | H | H | H | Br | H | nonafluoro-2-butyl | H | CF3 |
| 16-2963 | 3-bromophenyl | H | H | N-oxide | H | H | H | Br | H | nonafluoro-2-butyl | H | CF3 |
| 16-2969 | (3-trifluoromethyl) phenyl | H | H | N-oxide | H | H | H | Br | H | nonafluoro-2-butyl | H | CF3 |
| 16-2975 | 3-cyanophenyl | H | H | N-oxide | H | H | H | Br | H | nonafluoro-2-butyl | H | CF3 |
| 16-2976 | 4-cyanophenyl | H | H | N-oxide | H | H | H | Br | H | nonafluoro-2-butyl | H | CF3 |

**Table 16(11)**

| compound number | Q₁ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 16-2991 | 2-chloropyridin-3-yl | H | H | N-oxide | H | H | H | Br | H | nonafluoro-2-butyl | H | CF3 |
| 16-2998 | 6-chloropyridin-3-yl | H | H | N-oxide | H | H | H | Br | H | nonafluoro-2-butyl | H | CF3 |
| 16-3005 | 5-chloropyridin-3-yl | H | H | N-oxide | H | H | H | Br | H | nonafluoro-2-butyl | H | CF3 |
| 16-3020 | pyridin-4-yl | H | H | N-oxide | H | H | H | Br | H | nonafluoro-2-butyl | H | CF3 |
| 16-3033 | phenyl | H | H | N-oxide | H | H | H | I | H | heptafluoroisopropyl | H | CF3 |
| 16-3034 | 2-fluorophenyl | H | H | N-oxide | H | H | H | I | H | heptafluoroisopropyl | H | CF3 |
| 16-3035 | 3-fluorophenyl | H | H | N-oxide | H | H | H | I | H | heptafluoroisopropyl | H | CF3 |
| 16-3036 | 4-fluorophenyl | H | H | N-oxide | H | H | H | I | H | heptafluoroisopropyl | H | CF3 |
| 16-3037 | 2-chlorophenyl | H | H | N-oxide | H | H | H | I | H | heptafluoroisopropyl | H | CF3 |
| 16-3038 | 3-chlorophenyl | H | H | N-oxide | H | H | H | I | H | heptafluoroisopropyl | H | CF3 |
| 16-3039 | 4-chlorophenyl | H | H | N-oxide | H | H | H | I | H | heptafluoroisopropyl | H | CF3 |
| 16-3040 | 2-bromophenyl | H | H | N-oxide | H | H | H | I | H | heptafluoroisopropyl | H | CF3 |
| 16-3041 | 3-bromophenyl | H | H | N-oxide | H | H | H | I | H | heptafluoroisopropyl | H | CF3 |
| 16-3042 | 4-bromophenyl | H | H | N-oxide | H | H | H | I | H | heptafluoroisopropyl | H | CF3 |
| 16-3043 | 2-iodophenyl | H | H | N-oxide | H | H | H | I | H | heptafluoroisopropyl | H | CF3 |
| 16-3044 | 3-iodophenyl | H | H | N-oxide | H | H | H | I | H | heptafluoroisopropyl | H | CF3 |
| 16-3045 | 4-iodophenyl | H | H | N-oxide | H | H | H | I | H | heptafluoroisopropyl | H | CF3 |
| 16-3046 | (2-trifluoromethyl)phenyl | H | H | N-oxide | H | H | H | I | H | heptafluoroisopropyl | H | CF3 |
| 16-3047 | (3-trifluoromethyl) phenyl | H | H | N-oxide | H | H | H | I | H | heptafluoroisopropyl | H | CF3 |
| 16-3048 | (4-trifluoromethyl) phenyl | H | H | N-oxide | H | H | H | I | H | heptafluoroisopropyl | H | CF3 |
| 16-3049 | 2-nitrophenyl | H | H | N-oxide | H | H | H | I | H | heptafluoroisopropyl | H | CF3 |
| 16-3050 | 3-nitrophenyl | H | H | N-oxide | H | H | H | I | H | heptafluoroisopropyl | H | CF3 |
| 16-3051 | 4-nitrophenyl | H | H | N-oxide | H | H | H | I | H | heptafluoroisopropyl | H | CF3 |
| 16-3052 | 2-cyanophenyl | H | H | N-oxide | H | H | H | I | H | heptafluoroisopropyl | H | CF3 |
| 16-3053 | 3-cyanophenyl | H | H | N-oxide | H | H | H | I | H | heptafluoroisopropyl | H | CF3 |
| 16-3054 | 4-cyanophenyl | H | H | N-oxide | H | H | H | I | H | heptafluoroisopropyl | H | CF3 |
| 16-3055 | 2,6-difluorophenyl | H | H | N-oxide | H | H | H | I | H | heptafluoroisopropyl | H | CF3 |
| 16-3056 | 3,4-dichlorophenyl | H | H | N-oxide | H | H | H | I | H | heptafluoroisopropyl | H | CF3 |
| 16-3057 | 2,4-dichlorophenyl | H | H | N-oxide | H | H | H | I | H | heptafluoroisopropyl | H | CF3 |
| 16-3058 | 2-chloro-4-fluorophenyl | H | H | N-oxide | H | H | H | I | H | heptafluoroisopropyl | H | CF3 |
| 16-3059 | 2-chloro-4,5-difluorophenyl | H | H | N-oxide | H | H | H | I | H | heptafluoroisopropyl | H | CF3 |
| 16-3060 | 4-bromo-2-chlorophenyl | H | H | N-oxide | H | H | H | I | H | heptafluoroisopropyl | H | CF3 |
| 16-3061 | 2-bromo-4-chlorophenyl | H | H | N-oxide | H | H | H | I | H | heptafluoroisopropyl | H | CF3 |
| 16-3062 | 2-bromo-4-fluorophenyl | H | H | N-oxide | H | H | H | I | H | heptafluoroisopropyl | H | CF3 |
| 16-3063 | 2-chloro-4-nitrophenyl | H | H | N-oxide | H | H | H | I | H | heptafluoroisopropyl | H | CF3 |
| 16-3064 | 3,5-dicyanophenyl | H | H | N-oxide | H | H | H | I | H | heptafluoroisopropyl | H | CF3 |
| 16-3065 | 4-cyano-2-fluorophenyl | H | H | N-oxide | H | H | H | I | H | heptafluoroisopropyl | H | CF3 |
| 16-3066 | 2-chloro-4-cyanophenyl | H | H | N-oxide | H | H | H | I | H | heptafluoroisopropyl | H | CF3 |
| 16-3067 | pyridin-3-yl | H | H | N-oxide | H | H | H | I | H | heptafluoroisopropyl | H | CF3 |
| 16-3068 | 2-fluoropyridin-3-yl | H | H | N-oxide | H | H | H | I | H | heptafluoroisopropyl | H | CF3 |
| 16-3069 | 2-chloropyridin-3-yl | H | H | N-oxide | H | H | H | I | H | heptafluoroisopropyl | H | CF3 |
| 16-3070 | 2-bromopyridin-3-yl | H | H | N-oxide | H | H | H | I | H | heptafluoroisopropyl | H | CF3 |
| 16-3071 | 2-iodopyridin-3-yl | H | H | N-oxide | H | H | H | I | H | heptafluoroisopropyl | H | CF3 |
| 16-3072 | 2-(trifluoromethyl)pyridin-3-yl | H | H | N-oxide | H | H | H | I | H | heptafluoroisopropyl | H | CF3 |
| 16-3073 | 2-nitropyridin-3-yl | H | H | N-oxide | H | H | H | I | H | heptafluoroisopropyl | H | CF3 |
| 16-3074 | 2-cyanopyridin-3-yl | H | H | N-oxide | H | H | H | I | H | heptafluoroisopropyl | H | CF3 |
| 16-3075 | 6-fluoropyridin-3-yl | H | H | N-oxide | H | H | H | I | H | heptafluoroisopropyl | H | CF3 |
| 16-3076 | 6-chloropyridin-3-yl | H | H | N-oxide | H | H | H | I | H | heptafluoroisopropyl | H | CF3 |
| 16-3077 | 6-bromopyridin-3-yl | H | H | N-oxide | H | H | H | I | H | heptafluoroisopropyl | H | CF3 |
| 16-3078 | 6-iodopyridin-3-yl | H | H | N-oxide | H | H | H | I | H | heptafluoroisopropyl | H | CF3 |
| 16-3079 | 6-(trifluoromethyl)pyridin-3-yl | H | H | N-oxide | H | H | H | I | H | heptafluoroisopropyl | H | CF3 |
| 16-3080 | 6-nitropyridin-3-yl | H | H | N-oxide | H | H | H | I | H | heptafluoroisopropyl | H | CF3 |
| 16-3081 | 6-cyanopyridin-3-yl | H | H | N-oxide | H | H | H | I | H | heptafluoroisopropyl | H | CF3 |
| 16-3082 | 5-fluoropyridin-3-yl | H | H | N-oxide | H | H | H | I | H | heptafluoroisopropyl | H | CF3 |
| 16-3083 | 5-chloropyridin-3-yl | H | H | N-oxide | H | H | H | I | H | heptafluoroisopropyl | H | CF3 |
| 16-3084 | 5-bromopyridin-3-yl | H | H | N-oxide | H | H | H | I | H | heptafluoroisopropyl | H | CF3 |
| 16-3085 | 5-iodopyridin-3-yl | H | H | N-oxide | H | H | H | I | H | heptafluoroisopropyl | H | CF3 |
| 16-3086 | 5-(trifluoromethyl)pyridin-3-yl | H | H | N-oxide | H | H | H | I | H | heptafluoroisopropyl | H | CF3 |
| 16-3087 | 5-nitropyridin-3-yl | H | H | N-oxide | H | H | H | I | H | heptafluoroisopropyl | H | CF3 |
| 16-3088 | 5-cyanopyridin-3-yl | H | H | N-oxide | H | H | H | I | H | heptafluoroisopropyl | H | CF3 |
| 16-3089 | 4-fluoropyridin-3-yl | H | H | N-oxide | H | H | H | I | H | heptafluoroisopropyl | H | CF3 |
| 16-3090 | 4-chloropyridin-3-yl | H | H | N-oxide | H | H | H | I | H | heptafluoroisopropyl | H | CF3 |
| 16-3091 | 4-bromopyridin-3-yl | H | H | N-oxide | H | H | H | I | H | heptafluoroisopropyl | H | CF3 |
| 16-3092 | 4-iodopyridin-3-yl | H | H | N-oxide | H | H | H | I | H | heptafluoroisopropyl | H | CF3 |

**Table 16(12)**

| compound number | Q₁ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 16-3093 | 4-(trifluoromethyl)pyridin-3-yl | H | H | N-oxide | H | H | H | I | H | heptafluoroisopropyl | H | CF3 |
| 16-3094 | 4-nitropyridin-3-yl | H | H | N-oxide | H | H | H | I | H | heptafluoroisopropyl | H | CF3 |
| 16-3095 | 4-cyanopyridin-3-yl | H | H | N-oxide | H | H | H | I | H | heptafluoroisopropyl | H | CF3 |
| 16-3096 | 2,6-dichloropyridin-3-yl | H | H | N-oxide | H | H | H | I | H | heptafluoroisopropyl | H | CF3 |
| 16-3097 | pyridin-3-yl N-oxide | H | H | N-oxide | H | H | H | I | H | heptafluoroisopropyl | H | CF3 |
| 16-3098 | pyridin-4-yl | H | H | N-oxide | H | H | H | I | H | heptafluoroisopropyl | H | CF3 |
| 16-3099 | 2-chloropyridin-4-yl | H | H | N-oxide | H | H | H | I | H | heptafluoroisopropyl | H | CF3 |
| 16-3100 | 3-bromopyridin-4-yl | H | H | N-oxide | H | H | H | I | H | heptafluoroisopropyl | H | CF3 |
| 16-3101 | 3,5-dichloropyridin-4-yl | H | H | N-oxide | H | H | H | I | H | heptafluoroisopropyl | H | CF3 |
| 16-3102 | 3-(trifluoromethyl)pyridin-4-yl | H | H | N-oxide | H | H | H | I | H | heptafluoroisopropyl | H | CF3 |
| 16-3103 | 2,6-dicyanopyridin-4-yl | H | H | N-oxide | H | H | H | I | H | heptafluoroisopropyl | H | CF3 |
| 16-3104 | pyridin-4-yl N-oxide | H | H | N-oxide | H | H | H | I | H | heptafluoroisopropyl | H | CF3 |
| 16-3105 | pyridin-2-yl | H | H | N-oxide | H | H | H | I | H | heptafluoroisopropyl | H | CF3 |
| 16-3106 | 3-chloropyridin-2-yl | H | H | N-oxide | H | H | H | I | H | heptafluoroisopropyl | H | CF3 |
| 16-3107 | 4-bromopyridin-2-yl | H | H | N-oxide | H | H | H | I | H | heptafluoroisopropyl | H | CF3 |
| 16-3108 | 5-iodopyridin-2-yl | H | H | N-oxide | H | H | H | I | H | heptafluoroisopropyl | H | CF3 |
| 16-3109 | 6-chloropyridin-2-yl | H | H | N-oxide | H | H | H | I | H | heptafluoroisopropyl | H | CF3 |
| 16-3110 | 4-cyanopyridin-2-yl | H | H | N-oxide | H | H | H | I | H | heptafluoroisopropyl | H | CF3 |
| 16-3111 | phenyl | H | H | N-oxide | H | H | H | I | H | nonafluoro-2-butyl | H | CF3 |
| 16-3112 | 2-fluorophenyl | H | H | N-oxide | H | H | H | I | H | nonafluoro-2-butyl | H | CF3 |
| 16-3113 | 3-fluorophenyl | H | H | N-oxide | H | H | H | I | H | nonafluoro-2-butyl | H | CF3 |
| 16-3114 | 4-fluorophenyl | H | H | N-oxide | H | H | H | I | H | nonafluoro-2-butyl | H | CF3 |
| 16-3115 | 2-chlorophenyl | H | H | N-oxide | H | H | H | I | H | nonafluoro-2-butyl | H | CF3 |
| 16-3116 | 3-chlorophenyl | H | H | N-oxide | H | H | H | I | H | nonafluoro-2-butyl | H | CF3 |
| 16-3117 | 4-chlorophenyl | H | H | N-oxide | H | H | H | I | H | nonafluoro-2-butyl | H | CF3 |
| 16-3118 | 2-bromophenyl | H | H | N-oxide | H | H | H | I | H | nonafluoro-2-butyl | H | CF3 |
| 16-3119 | 3-bromophenyl | H | H | N-oxide | H | H | H | I | H | nonafluoro-2-butyl | H | CF3 |
| 16-3120 | 4-bromophenyl | H | H | N-oxide | H | H | H | I | H | nonafluoro-2-butyl | H | CF3 |
| 16-3121 | 2-iodophenyl | H | H | N-oxide | H | H | H | I | H | nonafluoro-2-butyl | H | CF3 |
| 16-3122 | 3-iodophenyl | H | H | N-oxide | H | H | H | I | H | nonafluoro-2-butyl | H | CF3 |
| 16-3123 | 4-iodophenyl | H | H | N-oxide | H | H | H | I | H | nonafluoro-2-butyl | H | CF3 |
| 16-3124 | (2-trifluoromethyl)phenyl | H | H | N-oxide | H | H | H | I | H | nonafluoro-2-butyl | H | CF3 |
| 16-3125 | (3-trifluoromethyl)phenyl | H | H | N-oxide | H | H | H | I | H | nonafluoro-2-butyl | H | CF3 |
| 16-3126 | (4-trifluoromethyl)phenyl | H | H | N-oxide | H | H | H | I | H | nonafluoro-2-butyl | H | CF3 |
| 16-3127 | 2-nitrophenyl | H | H | N-oxide | H | H | H | I | H | nonafluoro-2-butyl | H | CF3 |
| 16-3128 | 3-nitrophenyl | H | H | N-oxide | H | H | H | I | H | nonafluoro-2-butyl | H | CF3 |
| 16-3129 | 4-nitrophenyl | H | H | N-oxide | H | H | H | I | H | nonafluoro-2-butyl | H | CF3 |
| 16-3130 | 2-cyanophenyl | H | H | N-oxide | H | H | H | I | H | nonafluoro-2-butyl | H | CF3 |
| 16-3131 | 3-cyanophenyl | H | H | N-oxide | H | H | H | I | H | nonafluoro-2-butyl | H | CF3 |
| 16-3132 | 4-cyanophenyl | H | H | N-oxide | H | H | H | I | H | nonafluoro-2-butyl | H | CF3 |
| 16-3133 | 2,6-difluorophenyl | H | H | N-oxide | H | H | H | I | H | nonafluoro-2-butyl | H | CF3 |
| 16-3134 | 3,4-dichlorophenyl | H | H | N-oxide | H | H | H | I | H | nonafluoro-2-butyl | H | CF3 |
| 16-3135 | 2,4-dichlorophenyl | H | H | N-oxide | H | H | H | I | H | nonafluoro-2-butyl | H | CF3 |
| 16-3136 | 2-chloro-4-fluorophenyl | H | H | N-oxide | H | H | H | I | H | nonafluoro-2-butyl | H | CF3 |
| 16-3137 | 2-chloro-4,5-difluorophenyl | H | H | N-oxide | H | H | H | I | H | nonafluoro-2-butyl | H | CF3 |
| 16-3138 | 4-bromo-2-chlorophenyl | H | H | N-oxide | H | H | H | I | H | nonafluoro-2-butyl | H | CF3 |
| 16-3139 | 2-bromo-4-chlorophenyl | H | H | N-oxide | H | H | H | I | H | nonafluoro-2-butyl | H | CF3 |
| 16-3140 | 2-bromo-4-fluorophenyl | H | H | N-oxide | H | H | H | I | H | nonafluoro-2-butyl | H | CF3 |
| 16-3141 | 2-chloro-4-nitrophenyl | H | H | N-oxide | H | H | H | I | H | nonafluoro-2-butyl | H | CF3 |
| 16-3142 | 3,5-dicyanophenyl | H | H | N-oxide | H | H | H | I | H | nonafluoro-2-butyl | H | CF3 |
| 16-3143 | 4-cyano-2-fluorophenyl | H | H | N-oxide | H | H | H | I | H | nonafluoro-2-butyl | H | CF3 |
| 16-3144 | 2-chloro-4-cyanophenyl | H | H | N-oxide | H | H | H | I | H | nonafluoro-2-butyl | H | CF3 |
| 16-3145 | pyridin-3-yl | H | H | N-oxide | H | H | H | I | H | nonafluoro-2-butyl | H | CF3 |
| 16-3146 | 2-fluoropyridin-3-yl | H | H | N-oxide | H | H | H | I | H | nonafluoro-2-butyl | H | CF3 |
| 16-3147 | 2-chloropyridin-3-yl | H | H | N-oxide | H | H | H | I | H | nonafluoro-2-butyl | H | CF3 |
| 16-3148 | 2-bromopyridin-3-yl | H | H | N-oxide | H | H | H | I | H | nonafluoro-2-butyl | H | CF3 |
| 16-3149 | 2-iodopyridin-3-yl | H | H | N-oxide | H | H | H | I | H | nonafluoro-2-butyl | H | CF3 |
| 16-3150 | 2-(trifluoromethyl)pyridin-3-yl | H | H | N-oxide | H | H | H | I | H | nonafluoro-2-butyl | H | CF3 |
| 16-3151 | 2-nitropyridin-3-yl | H | H | N-oxide | H | H | H | I | H | nonafluoro-2-butyl | H | CF3 |
| 16-3152 | 2-cyanopyridin-3-yl | H | H | N-oxide | H | H | H | I | H | nonafluoro-2-butyl | H | CF3 |
| 16-3153 | 6-fluoropyridin-3-yl | H | H | N-oxide | H | H | H | I | H | nonafluoro-2-butyl | H | CF3 |
| 16-3154 | 6-chloropyridin-3-yl | H | H | N-oxide | H | H | H | I | H | nonafluoro-2-butyl | H | CF3 |
| 16-3155 | 6-bromopyridin-3-yl | H | H | N-oxide | H | H | H | I | H | nonafluoro-2-butyl | H | CF3 |
| 16-3156 | 6-iodopyridin-3-yl | H | H | N-oxide | H | H | H | I | H | nonafluoro-2-butyl | H | CF3 |

**Table 16(13)**

| compound number | Q₁ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 16-3157 | 6-(trifluoromethyl)pyridin-3-yl | H | H | N-oxide | H | H | H | I | H | nonafluoro-2-butyl | H | CF3 |
| 16-3158 | 6-nitropyridin-3-yl | H | H | N-oxide | H | H | H | I | H | nonafluaro-2-butyl | H | CF3 |
| 16-3159 | 6-cyanopyridin-3-yl | H | H | N-oxide | H | H | H | I | H | nonafluoro-2-butyl | H | CF3 |
| 16-3160 | 5-fluoropyridin-3-yl | H | H | N-oxide | H | H | H | I | H | nonafluoro-2-butyl | H | CF3 |
| 16-3161 | 5-chloropyridin-3-yl | H | H | N-oxide | H | H | H | I | H | nonafluoro-2-butyl | H | CF3 |
| 16-3162 | 5-bromopyridin-3-yl | H | H | N-oxide | H | H | H | I | H | nonafluoro-2-butyl | H | CF3 |
| 16-3163 | 5-iodopyridin-3-yl | H | H | N-oxide | H | H | H | I | H | nonafluoro-2-butyl | H | CF3 |
| 16-3164 | 5-(trifluoromethyl)pyridin-3-yl | H | H | N-oxide | H | H | H | I | H | nonafluoro-2-butyl | H | CF3 |
| 16-3165 | 5-nitropyridin-3-yl | H | H | N-oxide | H | H | H | I | H | nonafluoro-2-butyl | H | CF3 |
| 16-3166 | 5-cyanopyridin-3-yl | H | H | N-oxide | H | H | H | I | H | nonafluoro-2-butyl | H | CF3 |
| 16-3167 | 4-fluoropyridin-3-yl | H | H | N-oxide | H | H | H | I | H | nonafluoro-2-butyl | H | CF3 |
| 16-3168 | 4-chloropyridin-3-yl | H | H | N-oxide | H | H | H | I | H | nonafluoro-2-butyl | H | CF3 |
| 16-3169 | 4-bromopyridin-3-yl | H | H | N-oxide | H | H | H | I | H | nonafluoro-2-butyl | H | CF3 |
| 16-3170 | 4-iodopyridin-3-yl | H | H | N-oxide | H | H | H | I | H | nonafluoro-2-butyl | H | CF3 |
| 16-3171 | 4-(trifluoromethyl)pyridin-3-yl | H | H | N-oxide | H | H | H | I | H | nonafluoro-2-butyl | H | CF3 |
| 16-3172 | 4-nitropyridin-3-yl | H | H | N-oxide | H | H | H | I | H | nonafluoro-2-butyl | H | CF3 |
| 16-3173 | 4-cyanopyridin-3-yl | H | H | N-oxide | H | H | H | I | H | nonafluoro-2-butyl | H | CF3 |
| 16-3174 | 2,6-dichloropyridin-3-yl | H | H | N-oxide | H | H | H | I | H | nonafluoro-2-butyl | H | CF3 |
| 16-3175 | pyridin-3-yl N-oxide | H | H | N-oxide | H | H | H | I | H | nonafluoro-2-butyl | H | CF3 |
| 16-3176 | pyridin-4-yl | H | H | N-oxide | H | H | H | I | H | nonafluoro-2-butyl | H | CF3 |
| 16-3177 | 2-chloropyridin-4-yl | H | H | N-oxide | H | H | H | I | H | nonafluoro-2-butyl | H | CF3 |
| 16-3178 | 3-bromopyridin-4-yl | H | H | N-oxide | H | H | H | I | H | nonafluoro-2-butyl | H | CF3 |
| 16-3179 | 3,5-dichloropyridin-4-yl | H | H | N-oxide | H | H | H | I | H | nonafluoro-2-butyl | H | CF3 |
| 16-3180 | 3-(trifluoromethyl)pyridin-4-yl | H | H | N-oxide | H | H | H | I | H | nonafluoro-2-butyl | H | CF3 |
| 16-3181 | 2,6-dicyanopyridin-4-yl | H | H | N-oxide | H | H | H | I | H | nonafluoro-2-butyl | H | CF3 |
| 16-3182 | pyridin-4-yl N-oxide | H | H | N-oxide | H | H | H | I | H | nonafluoro-2-butyl | H | CF3 |
| 16-3183 | pyridin-2-yl | H | H | N-oxide | H | H | H | I | H | nonafluoro-2-butyl | H | CF3 |
| 16-3184 | 3-chloropyridin-2-yl | H | H | N-oxide | H | H | H | I | H | nonafluoro-2-butyl | H | CF3 |
| 16-3185 | 4-bromopyridin-2-yl | H | H | N-oxide | H | H | H | I | H | nonafluoro-2-butyl | H | CF3 |
| 16-3186 | 5-iodopyridin-2-yl | H | H | N-oxide | H | H | H | I | H | nonafluoro-2-butyl | H | CF3 |
| 16-3187 | 6-chloropyridin-2-yl | H | H | N-oxide | H | H | H | I | H | nonafluoro-2-butyl | H | CF3 |
| 16-3188 | 4-cyanopyridin-2-yl | H | H | N-oxide | H | H | H | I | H | nonafluoro-2-butyl | H | CF3 |
| 16-3189 | phenyl | H | H | N-oxide | H | H | H | OCF3 | H | heptafluoroisopropyl | H | OCF3 |
| 16-3196 | 2-bromophenyl | H | H | N-oxide | H | H | H | OCF3 | H | heptafluoroisopropyl | H | OC2F5 |
| 16-3201 | 4-iodophenyl | H | H | N-oxide | H | H | H | OCF3 | H | pentafluoroethyl | H | OCF3 |
| 16-3204 | 3-cyanophenyl | H | H | N-oxide | H | H | H | OCF3 | H | heptafluoroisopropyl | H | OCF3 |
| 16-3205 | 4-cyanophenyl | H | H | N-oxide | H | H | H | OCF3 | H | heptafluoroisopropyl | H | OCF3 |
| 16-3213 | 2-bromo-4-fluorophenyl | H | H | N-oxide | H | H | H | OC2F5 | H | heptafluoroisopropyl | H | OCF3 |
| 16-3220 | 2-chloropyridin-3-yl | H | H | N-oxide | H | H | H | OCF3 | H | heptafluoroisopropyl | H | OCF3 |
| 16-3229 | 5-cyanopyridin-3-yl | H | H | N-oxide | H | H | H | OC2F5 | H | heptafluoroisopropyl | H | OC2F5 |
| 16-3231 | 4-cyanopyridin-3-yl | H | H | N-oxide | H | H | H | OCF3 | H | heptafluoroisopropyl | H | OC2F5 |
| 16-3245 | phenyl | H | H | N-oxide | H | H | H | OCF3 | H | nonafluoro-2-butyl | H | OCF3 |
| 16-3252 | 2-bromophenyl | H | H | N-oxide | H | H | H | OC2F5 | H | nonafluoro-2-butyl | H | OCF3 |
| 16-3258 | (2-trifluoromethyl) phenyl | H | H | N-oxide | H | H | H | OCF3 | H | nonafluoro-2-butyl | H | OC2F5 |
| 16-3260 | 3-cyanophenyl | H | H | N-oxide | H | H | H | OCF3 | H | nonafluoro-2-butyl | H | OCF3 |
| 16-3261 | 4-cyanophenyl | H | H | N-oxide | H | H | H | OCF3 | H | nonafluoro-2-butyl | H | OCF3 |
| 16-3276 | 2-chloropyridin-3-yl | H | H | N-oxide | H | H | H | OCF3 | H | nonafluoro-2-butyl | H | OCF3 |
| 16-3283 | 6-cyanopyridin-3-yl | H | H | N-oxide | H | H | H | OC2F5 | H | nonafluoro-2-butyl | H | OCF3 |
| 16-3284 | 5-chloropyridin-3-yl | H | H | N-oxide | H | H | H | OCF3 | H | nonafluoro-2-butyl | H | OC2F5 |
| 16-3301 | phenyl | H | H | N-oxide | H | H | H | CF3 | H | heptafluoroisopropyl | H | OCF3 |
| 16-3308 | 2-bromophenyl | H | H | N-oxide | H | H | H | CF3 | H | heptafluoroisopropyl | H | OC2F5 |
| 16-3313 | 4-iodophenyl | H | H | N-oxide | H | H | H | CF3 | H | pentafluoroethyl | H | OCF3 |
| 16-3314 | (2-trifluoromethyl) phenyl | H | H | N-oxide | H | H | H | C2F5 | H | heptafluoroisopropyl | H | OC2F5 |
| 16-3315 | 3-nitrophenyl | H | H | N-oxide | H | H | H | C2F5 | H | heptafluoroisopropyl | H | OCF3 |
| 16-3316 | 3-cyanophenyl | H | H | N-oxide | H | H | H | CF3 | H | heptafluoroisopropyl | H | OCF3 |
| 16-3317 | 4-cyanophenyl | H | H | N-oxide | H | H | H | CF3 | H | heptafluoroisopropyl | H | OCF3 |
| 16-3324 | 2-bromo-4-chlorophenyl | H | H | N-oxide | H | H | H | n-C3F7 | H | heptafluoroisopropyl | H | OCF3 |
| 16-3328 | 4-cyano-2-fluorophenyl | H | H | N-oxide | H | H | H | i-C3F7 | H | heptafluoroisopropyl | H | OCF3 |
| 16-3332 | 2-chloropyridin-3-yl | H | H | N-oxide | H | H | H | CF3 | H | heptafluoroisopropyl | H | OCF3 |
| 16-3338 | 6-chloropyridin-3-yl | H | H | N-oxide | H | H | H | n-C3F7 | H | heptafluoroisopropyl | H | OC2F5 |
| 16-3341 | 5-cyanopyridin-3-yl | H | H | N-oxide | H | H | H | i-C3F7 | H | heptafluoroisopropyl | H | OC2F5 |
| 16-3357 | phenyl | H | H | N-oxide | H | H | H | CF3 | H | nonafluoro-2-butyl | H | OCF3 |
| 16-3368 | 3-iodophenyl | H | H | N-oxide | H | H | H | CF3 | H | nonafluoro-2-butyl | H | OC2F5 |
| 16-3371 | 3-nitrophenyl | H | H | N-oxide | H | H | H | C2F5 | H | nonafluoro-2-butyl | H | OCF3 |

**Table 16(14)**

| compound number | Q₁ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 16-3372 | 3-cyanophenyl | H | H | N-oxide | H | H | H | CF3 | H | nonafluoro-2-butyl | H | OCF3 |
| 16-3373 | 4-cyanophenyl | H | H | N-oxide | H | H | H | CF3 | H | nonafluoro-2-butyl | H | OCF3 |
| 16-3379 | 4-bromo-2-chlorophenyl | H | H | N-oxide | H | H | H | i-C3F7 | H | nonafluoro-2-butyl | H | OCF3 |
| 16-3382 | 2-chloro-4-nitrophenyl | H | H | N-oxide | H | H | H | n-C3F7 | H | nonafluoro-2-butyl | H | OCF3 |
| 16-3388 | 2-chloropyridin-3-yl | H | H | N-oxide | H | H | H | CF3 | H | nonafluoro-2-butyl | H | OCF3 |
| 16-3397 | 5-cyanopyridin-3-yl | H | H | N-oxide | H | H | H | C2F5 | H | nonafluoro-2-butyl | H | OC2F5 |
| 16-3399 | 4-cyanopyridin-3-yl | H | H | N-oxide | H | H | H | n-C3F7 | H | nonafluoro-2-butyl | H | OC2F5 |
| 16-3410 | 5-iodopyridin-2-yl | H | H | N-oxide | H | H | H | i-C3F7 | H | nonafluoro-2-butyl | H | OC2F5 |
| 16-3413 | phenyl | H | H | N-oxide | H | H | H | CF3 | H | heptafluoroisopropyl | H | CF3 |
| 16-3425 | 4-iodophenyl | H | H | N-oxide | H | H | H | CF3 | H | pentafluoroethyl | H | CF3 |
| 16-3427 | (3-trifluoromethyl) phenyl | H | H | N-oxide | H | H | H | C2F5 | H | heptafluoroisopropyl | H | CF3 |
| 16-3433 | 3-cyanophenyl | H | H | N-oxide | H | H | H | CF3 | H | heptafluoroisopropyl | H | CF3 |
| 16-3434 | 4-cyanophenyl | H | H | N-oxide | H | H | H | CF3 | H | heptafluoroisopropyl | H | CF3 |
| 16-3440 | 4-bromo-2-chlorophenyl | H | H | N-oxide | H | H | H | n-C3F7 | H | heptafluoroisopropyl | H | CF3 |
| 16-3445 | 4-cyano-2-fluorophenyl | H | H | N-oxide | H | H | H | i-C3F7 | H | heptafluoroisopropyl | H | CF3 |
| 16-3447 | pyridin-3-yl | H | H | N-oxide | H | H | H | CF3 | H | heptafluoroisopropyl | H | CF3 |
| 16-3449 | 2-chloropyridin-3-yl | H | H | N-oxide | H | H | H | CF3 | H | heptafluoroisopropyl | H | CF3 |
| 16-3450 | 2-bromopyridin-3-yl | H | H | N-oxide | H | H | H | C2F5 | H | heptafluoroisopropyl | H | C2F5 |
| 16-3461 | 6-cyanopyridin-3-yl | H | H | N-oxide | H | H | H | C2F5 | H | heptafluoroisopropyl | H | n-C3F7 |
| 16-3465 | 4-cyanopyridin-3-yl | H | H | N-oxide | H | H | H | C2F5 | H | heptafluoroisopropyl | H | i-C3F7 |
| 16-3481 | phenyl | H | H | N-oxide | H | H | H | CF3 | H | nonafluoro-2-butyl | H | CF3 |
| 16-3493 | 4-iodophenyl | H | H | N-oxide | H | H | H | C2F5 | H | nonafluoro-2-butyl | H | CF3 |
| 16-3501 | 3-cyanophenyl | H | H | N-oxide | H | H | H | CF3 | H | nonafluoro-2-butyl | H | CF3 |
| 16-3502 | 4-cyanophenyl | H | H | N-oxide | H | H | H | CF3 | H | nonafluoro-2-butyl | H | CF3 |
| 16-3508 | 4-bromo-2-chlorophenyl | H | H | N-oxide | H | H | H | n-C3F7 | H | nonafluoro-2-butyl | H | CF3 |
| 16-3513 | 4-cyano-2-fluorophenyl | H | H | N-oxide | H | H | H | i-C3F7 | H | nonafluoro-2-butyl | H | CF3 |
| 16-3517 | 2-chloropyridin-3-yl | H | H | N-oxide | H | H | H | CF3 | H | nonafluoro-2-butyl | H | CF3 |
| 16-3518 | 2-bromopyridin-3-yl | H | H | N-oxide | H | H | H | C2F5 | H | nonafluoro-2-butyl | H | C2F5 |
| 16-3529 | 6-cyanopyridin-3-yl | H | H | N-oxide | H | H | H | C2F5 | H | nonafluoro-2-butyl | H | n-C3F7 |
| 16-3536 | pyridin-4-yl | H | H | N-oxide | H | H | H | C2F5 | H | nonafluoro-2-butyl | H | i-C3F7 |

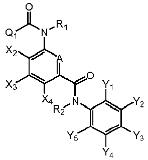

**Table 17(1)**

| compound number | Q₁ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 17-1 | phenyl | Me | H | N | H | H | H | F | H | heptafluoroisopropyl | H | F |
| 17-2 | 3-cyanophenyl | Me | H | N | H | H | H | F | H | heptafluoroisopropyl | H | F |
| 17-3 | 4-cyanophenyl | Me | H | N | H | H | H | F | H | heptafluoroisopropyl | H | F |
| 17-4 | 2-chloropyridin-3-yl | Me | H | N | H | H | H | F | H | heptafluoroisopropyl | H | F |
| 17-5 | phenyl | Me | H | N | H | H | H | F | H | nonafluoro-2-butyl | H | F |
| 17-6 | 3-cyanophenyl | Me | H | N | H | H | H | F | H | nonafluoro-2-butyl | H | F |
| 17-7 | 4-cyanophenyl | Me | H | N | H | H | H | F | H | nonafluoro-2-butyl | H | F |
| 17-8 | 2-chloropyridin-3-yl | Me | H | N | H | H | H | F | H | nonafluoro-2-butyl | H | F |
| 17-9 | phenyl | Me | H | N | H | H | H | Cl | H | heptafluoroisopropyl | H | Cl |
| 17-29 | 3-cyanophenyl | Me | H | N | H | H | H | Cl | H | heptafluoroisopropyl | H | Cl |
| 17-30 | 4-cyanophenyl | Me | H | N | H | H | H | Cl | H | heptafluoroisopropyl | H | Cl |
| 17-45 | 2-chloropyridin-3-yl | Me | H | N | H | H | H | Cl | H | heptafluoroisopropyl | H | Cl |
| 17-87 | phenyl | Me | H | N | H | H | H | Cl | H | nonafluoro-2-butyl | H | Cl |
| 17-107 | 3-cyanophenyl | Me | H | N | H | H | H | Cl | H | nonafluoro-2-butyl | H | Cl |
| 17-108 | 4-cyanophenyl | Me | H | N | H | H | H | Cl | H | nonafluoro-2-butyl | H | Cl |
| 17-123 | 2-chloropyridin-3-yl | Me | H | N | H | H | H | Cl | H | nonafluoro-2-butyl | H | Cl |
| 17-165 | phenyl | Me | H | N | H | H | H | Br | H | heptafluoroisopropyl | H | Br |
| 17-185 | 3-cyanophenyl | Me | H | N | H | H | H | Br | H | heptafluoroisopropyl | H | Br |
| 17-186 | 4-cyanophenyl | Me | H | N | H | H | H | Br | H | heptafluoroisopropyl | H | Br |
| 17-201 | 2-chloropyridin-3-yl | Me | H | N | H | H | H | Br | H | heptafluoroisopropyl | H | Br |
| 17-243 | phenyl | Me | H | N | H | H | H | Br | H | nonafluoro-2-butyl | H | Br |
| 17-263 | 3-cyanophenyl | Me | H | N | H | H | H | Br | H | nonafluoro-2-butyl | H | Br |
| 17-264 | 4-cyanophenyl | Me | H | N | H | H | H | Br | H | nonafluoro-2-butyl | H | Br |
| 17-279 | 2-chloropyridin-3-yl | Me | H | N | H | H | H | Br | H | nonafluoro-2-butyl | H | Br |
| 17-321 | phenyl | Me | H | N | H | H | H | I | H | heptafluoroisopropyl | H | I |
| 17-341 | 3-cyanophenyl | Me | H | N | H | H | H | I | H | heptafluoroisopropyl | H | I |
| 17-342 | 4-cyanophenyl | Me | H | N | H | H | H | I | H | heptafluoroisopropyl | H | I |
| 17-357 | 2-chloropyridin-3-yl | Me | H | N | H | H | H | I | H | heptafluoroisopropyl | H | I |
| 17-399 | phenyl | Me | H | N | H | H | H | I | H | nonafluoro-2-butyl | H | I |
| 17-400 | 2-fluorophenyl | Me | H | N | H | H | H | I | H | nonafluoro-2-butyl | H | I |
| 17-419 | 3-cyanophenyl | Me | H | N | H | H | H | I | H | nonafluoro-2-butyl | H | I |
| 17-420 | 4-cyanophenyl | Me | H | N | H | H | H | I | H | nonafluoro-2-butyl | H | I |
| 17-435 | 2-chloropyridin-3-yl | Me | H | N | H | H | H | I | H | nonafluoro-2-butyl | H | I |
| 17-477 | phenyl | Me | H | N | H | H | H | Cl | H | heptafluoroisopropyl | H | OCF3 |
| 17-497 | 3-cyanophenyl | Me | H | N | H | H | H | Cl | H | heptafluoroisopropyl | H | OCF3 |
| 17-498 | 4-cyanophenyl | Me | H | N | H | H | H | Cl | H | heptafluoroisopropyl | H | OCF3 |
| 17-513 | 2-chloropyridin-3-yl | Me | H | N | H | H | H | Cl | H | heptafluoroisopropyl | H | OCF3 |
| 17-555 | phenyl | Me | H | N | H | H | H | Cl | H | nonafluoro-2-butyl | H | OCF3 |
| 17-575 | 3-cyanophenyl | Me | H | N | H | H | H | Cl | H | nonafluoro-2-butyl | H | OCF3 |
| 17-576 | 4-cyanophenyl | Me | H | N | H | H | H | Cl | H | nonafluoro-2-butyl | H | OCF3 |
| 17-591 | 2-chloropyridin-3-yl | Me | H | N | H | H | H | Cl | H | nonafluoro-2-butyl | H | OCF3 |
| 17-633 | phenyl | Me | H | N | H | H | H | Br | H | heptafluoroisopropyl | H | OCF3 |
| 17-653 | 3-cyanophenyl | Me | H | N | H | H | H | Br | H | heptafluoroisopropyl | H | OCF3 |
| 17-654 | 4-cyanophenyl | Me | H | N | H | H | H | Br | H | heptafluoroisopropyl | H | OCF3 |
| 17-669 | 2-chloropyridin-3-yl | Me | H | N | H | H | H | Br | H | heptafluoroisopropyl | H | OCF3 |
| 17-711 | phenyl | Me | H | N | H | H | H | Br | H | nonafluoro-2-butyl | H | OCF3 |
| 17-731 | 3-cyanophenyl | Me | H | N | H | H | H | Br | H | nonafluoro-2-butyl | H | OCF3 |
| 17-732 | 4-cyanophenyl | Me | H | N | H | H | H | Br | H | nonafluoro-2-butyl | H | OCF3 |
| 17-747 | 2-chloropyridin-3-yl | Me | H | N | H | H | H | Br | H | nonafluoro-2-butyl | H | OCF3 |
| 17-789 | phenyl | Me | H | N | H | H | H | I | H | heptafluoroisopropyl | H | OCF3 |
| 17-809 | 3-cyanophenyl | Me | H | N | H | H | H | I | H | heptafluoroisopropyl | H | OCF3 |
| 17-810 | 4-cyanophenyl | Me | H | N | H | H | H | I | H | heptafluoroisopropyl | H | OCF3 |
| 17-825 | 2-chloropyridin-3-yl | Me | H | N | H | H | H | I | H | heptafluoroisopropyl | H | OCF3 |
| 17-867 | phenyl | Me | H | N | H | H | H | I | H | nonafluoro-2-butyl | H | OCF3 |
| 17-887 | 3-cyanophenyl | Me | H | N | H | H | H | I | H | nonafluoro-2-butyl | H | OCF3 |

**Table 17(2)**

| compound number | Q₁ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 17-888 | 4-cyanophenyl | Me | H | N | H | H | H | I | H | nonafluoro-2-butyl | H | OCF3 |
| 17-903 | 2-chloropyridin-3-yl | Me | H | N | H | H | H | I | H | nonafluoro-2-butyl | H | OCF3 |
| 17-945 | phenyl | Me | H | N | H | H | H | F | H | heptafluoroisopropyl | H | CF3 |
| 17-946 | phenyl | Me | H | N | H | H | H | Cl | H | heptafluoroisopropyl | H | CF3 |
| 17-957 | 3-iodophenyl | Me | H | N | H | H | H | Cl | H | pentafluoroethyl | H | CF3 |
| 17-958 | 4-iodophenyl | Me | H | N | H | H | H | Cl | H | pentafluoroethyl | H | CF3 |
| 17-965 | 2-cyanophenyl | Me | H | N | H | H | H | Cl | H | pentafluoroethyl | H | C2F5 |
| 17-966 | 3-cyanophenyl | Me | H | N | H | H | H | Cl | H | heptafluoroisopropyl | H | CF3 |
| 17-967 | 4-cyanophenyl | Me | H | N | H | H | H | Cl | H | heptafluoroisopropyl | H | CF3 |
| 17-970 | 2,4-dichlorophonyl | Me | H | N | H | H | H | Cl | H | heptafluoroisopropyl | H | C2F5 |
| 17-971 | 2-chloro-4-fluorophenyl | n-Pr | H | N | H | H | H | Cl | H | heptafluoroisopropyl | H | CF3 |
| 17-974 | 2-bromo-4-chlorophenyl | Me | H | N | H | H | H | Cl | H | heptafluoroisopropyl | H | n-C3F7 |
| 17-981 | 2-fluoropyridin-3-yl | Me | H | N | H | H | H | Cl | H | pentafluoroethyl | H | CF3 |
| 17-982 | 2-chloropyridin-3-yl | Me | H | N | H | H | H | Cl | H | heptafluoroisopropyl | H | CF3 |
| 17-988 | 6-fluoropyridin-3-yl | i-Pr | H | N | H | H | H | Cl | H | heptafluoroisopropyl | H | CF3 |
| 17-995 | 5-fluoropyridin-3-yl | Me | H | N | H | H | H | Cl | H | heptafluoroisopropyl | H | C2F5 |
| 17-997 | 5-bromopyridin-3-yl | Me | H | N | H | H | H | Cl | H | heptafluoroisopropyl | H | n-C3F7 |
| 17-998 | 5-iodopyridin-3-yl | Me | H | N | H | H | H | Cl | H | heptafluoroisopropyl | H | i-C3F7 |
| 17-1000 | 5-nitropyridin-3-yl | Me | H | N | H | H | H | Cl | H | pentafluoroethyl | H | CF3 |
| 17-1002 | 4-fluoropyridin-3-yl | Me | H | N | H | H | H | Cl | H | pentafluoroethyl | H | CF3 |
| 17-1004 | 4-bromopyridin-3-yl | CH2CH=CH2 | H | N | H | H | H | Cl | H | heptafluoroisopropyl | H | n-C3F7 |
| 17-1005 | 4-iodopyridin-3-yl | Me | H | N | H | H | H | Cl | H | heptafluoroisopropyl | H | i-C3F7 |
| 17-1011 | pyridin-4-yl | CN | H | N | H | H | H | Cl | H | heptafluoroisopropyl | H | CF3 |
| 17-1023 | 4-cyanopyridin-2-yl | Me | H | N | H | H | H | Cl | H | pentafluoroethyl | H | CF3 |
| 17-1024 | phenyl | Me | H | N | H | H | H | F | H | nonafluoro-2-butyl | H | CF3 |
| 17-1025 | phenyl | Me | H | N | H | H | H | Cl | H | nonafluoro-2-butyl | H | CF3 |
| 17-1033 | 3-bromophenyl | CH2C ≡ CH | H | N | H | H | H | Cl | H | nonafluoro-2-butyl | H | CF3 |
| 17-1038 | (2-trifluoromethyl)phenyl | Me | H | N | H | H | H | Cl | H | nonafluoro-2-butyl | H | C2F5 |
| 17-1040 | (4-trifluoromethyl) phenyl | NH2 | H | N | H | H | H | Cl | H | nonafluoro-2-butyl | H | CF3 |
| 17-1045 | 3-cyanophenyl | Me | H | N | H | H | H | Cl | H | nonafluoro-2-butyl | H | CF3 |
| 17-1046 | 4-cyanophenyl | Me | H | N | H | H | H | Cl | H | nonafluoro-2-butyl | H | CF3 |
| 17-1053 | 2-bromo-4-chlorophenyl | C(O)OMe | H | N | H | H | H | Cl | H | nonafluoro-2-butyl | H | CF3 |
| 17-1055 | 2-chloro-4-nitrophenyl | Me | H | N | H | H | H | Cl | H | nonafluoro-2-butyl | H | i-C3F7 |
| 17-1061 | 2-chloropyridin-3-yl | Me | H | N | H | H | H | Cl | H | nonafluoro-2-butyl | H | CF3 |
| 17-1074 | 5-fluoropyridin-3-yl | C(O)OEt | H | N | H | H | H | Cl | H | nonafluoro-2-butyl | H | C2F5 |
| 17-1076 | 5-bromopyridin-3-yl | Me | H | N | H | H | H | Cl | H | nonafluoro-2-butyl | H | n-C3F7 |
| 17-1077 | 5-iodopyridin-3-yl | Me | H | N | H | H | H | Cl | H | nonafluoro-2-butyl | H | i-C3F7 |
| 17-1081 | 4-fluoropyridin-3-yl | Me | H | N | H | H | H | Cl | H | nonafluoro-2-butyl | H | C2F5 |
| 17-1083 | 4-bromopyridin-3-yl | C(O)C(O)Me | H | N | H | H | H | Cl | H | nonafluoro-2-butyl | H | CF3 |
| 17-1085 | 4-(trifluoromethyl)pyridin-3-yl | Me | H | N | H | H | H | Cl | H | nonafluoro-2-butyl | H | n-C3F7 |
| 17-1086 | 4-nitropyridin-3-yl | C(O)C(O)Et | H | N | H | H | H | Cl | H | nonafluoro-2-butyl | H | i-C3F7 |
| 17-1100 | 5-iodopyridin-2-yl | C(O)Me | H | N | H | H | H | Cl | H | nonafluoro-2-butyl | H | CF3 |
| 17-1102 | 4-cyanopyridin-2-yl | C(O)Et | H | N | H | H | H | Cl | H | nonafluoro-2-butyl | H | C2F5 |
| 17-1103 | phenyl | Me | H | N | H | H | H | Br | H | heptafluoroisopropyl | H | CF3 |
| 17-1104 | 2-fluorophenyl | Me | H | N | H | H | H | Br | H | heptafluoroisopropyl | H | CF3 |
| 17-1117 | (3-trifluoromethyl)phenyl | S(O)2Me | H | N | H | H | H | Br | H | heptafluoroisopropyl | H | CF3 |
| 17-1135 | 4-cyano-2-fluorophenyl | S(O)2Et | H | N | H | H | H | Br | H | heptafluoroisopropyl | H | CF3 |
| 17-1139 | 2-chloropyridin-3-yl | Me | H | N | H | H | H | Br | H | heptafluoroisopropyl | H | CF3 |
| 17-1150 | 6-nitropyridin-3-yl | CH2Ph | H | N | H | H | H | Br | H | heptafluoroisopropyl | H | CF3 |
| 17-1159 | 4-fluoropyridin-3-yl | CH2(3-Py) | H | N | H | H | H | Br | H | heptafluoroisopropyl | H | CF3 |
| 17-1180 | 4-cyanopyridin-2-yl | CH2(3-Py-N-oxide) | H | N | H | H | H | Br | H | heptafluoroisopropyl | H | CF3 |
| 17-1181 | phenyl | Me | H | N | H | H | H | Br | H | nonafluoro-2-butyl | H | CF3 |
| 17-1182 | 2-fluorophenyl | Me | H | N | H | H | H | Br | H | nonafluoro-2-butyl | H | CF3 |
| 17-1201 | 3-cyanophenyl | Me | H | N | H | H | H | Br | H | nonafluoro-2-butyl | H | CF3 |
| 17-1202 | 4-cyanophenyl | Me | H | N | H | H | H | Br | H | nonafluoro-2-butyl | H | CF3 |
| 17-1208 | 4-bromo-2-chlorophenyl | Et | H | N | H | H | H | Br | H | nonafluoro-2-butyl | H | CF3 |
| 17-1217 | 2-chloropyridin-3-yl | Me | H | N | H | H | H | Br | H | nonafluoro-2-butyl | H | CF3 |
| 17-1234 | 5-(trifluoromethyl)pyridin-3-yl | CN | H | N | H | H | H | Br | H | nonafluoro-2-butyl | H | CF3 |
| 17-1259 | phenyl | Me | H | N | H | H | H | I | H | heptafluoroisopropyl | H | CF3 |
| 17-1260 | 2-fluorophenyl | Me | H | N | H | H | H | I | H | heptafluoroisopropyl | H | CF3 |
| 17-1279 | 3-cyanophenyl | Me | H | N | H | H | H | I | H | heptafluoroisopropyl | H | CF3 |
| 17-1280 | 4-cyanophenyl | Me | H | N | H | H | H | I | H | heptafluoroisopropyl | H | CF3 |
| 17-1295 | 2-chloropyridin-3-yl | Me | H | N | H | H | H | I | H | heptafluoroisopropyl | H | CF3 |
| 17-1308 | 5-fluoropyridin-3-yl | C(O)Me | H | N | H | H | H | I | H | heptafluoroisopropyl | H | CF3 |
| 17-1337 | phenyl | Me | H | N | H | H | H | I | H | nonafluoro-2-butyl | H | CF3 |

**Table 17(3)**

| compound number | Q₁ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 17-1338 | 2-fluorophenyl | Me | H | N | H | H | H | I | H | nonafluoro-2-butyl | H | CF3 |
| 17-1357 | 3-cyanophenyl | Me | H | N | H | H | H | I | H | nonafluoro-2-butyl | H | CF3 |
| 17-1358 | 4-cyanophenyl | Me | H | N | H | H | H | I | H | nonafluoro-2-butyl | H | CF3 |
| 17-1373 | 2-chloropyridin-3-yl | Me | H | N | H | H | H | I | H | nonafluoro-2-butyl | H | CF3 |
| 17-1415 | phenyl | Me | H | N | H | H | H | OCF3 | H | heptafluoroisopropyl | H | OCF3 |
| 17-1446 | 2-chloropyridin-3-yl | Me | H | N | H | H | H | OCF3 | H | heptafluoroisopropyl | H | OCF3 |
| 17-1471 | phenyl | Me | H | N | H | H | H | OCF3 | H | nonafluoro-2-butyl | H | OCF3 |
| 17-1502 | 2-chloropyridin-3-yl | Me | H | N | H | H | H | OCF3 | H | nonafluoro-2-butyl | H | OCF3 |
| 17-1527 | phenyl | Me | H | N | H | H | H | CF3 | H | heptafluoroisopropyl | H | OCF3 |
| 17-1558 | 2-chloropyridin-3-yl | Me | H | N | H | H | H | CF3 | H | heptafluoroisopropyl | H | OCF3 |
| 17-1583 | phenyl | Me | H | N | H | H | H | CF3 | H | nonafluoro-2-butyl | H | OCF3 |
| 17-1614 | 2-chloropyridin-3-yl | Me | H | N | H | H | H | CF3 | H | nonafluoro-2-butyl | H | OCF3 |
| 17-1639 | phenyl | Me | H | N | H | H | H | CF3 | H | heptafluoroisopropyl | H | CF3 |
| 17-1659 | 3-cyanophenyl | Me | H | N | H | H | H | CF3 | H | heptafluoroisopropyl | H | CF3 |
| 17-1660 | 4-cyanophenyl | Me | H | N | H | H | H | CF3 | H | heptafluoroisopropyl | H | CF3 |
| 17-1675 | 2-chloropyridin-3-yl | Me | H | N | H | H | H | CF3 | H | heptafluoroisopropyl | H | CF3 |
| 17-1707 | phenyl | Me | H | N | H | H | H | CF3 | H | nonafluoro-2-butyl | H | CF3 |
| 17-1727 | 3-cyanophenyl | Me | H | N | H | H | H | CF3 | H | nonafluoro-2-butyl | H | CF3 |
| 17-1728 | 4-cyanophenyl | Me | H | N | H | H | H | CF3 | H | nonafluoro-2-butyl | H | CF3 |
| 17-1743 | 2-chloropyridin-3-yl | Me | H | N | H | H | H | CF3 | H | nonafluoro-2-butyl | H | CF3 |
| 17-1775 | phenyl | Me | H | N-oxide | H | H | H | F | H | heptafluoroisopropyl | H | F |
| 17-1776 | 3-cyanophenyl | Me | H | N-oxide | H | H | H | F | H | heptafluoroisopropyl | H | F |
| 17-1777 | 4-cyanophenyl | Me | H | N-oxide | H | H | H | F | H | heptafluoroisopropyl | H | F |
| 17-1778 | 2-chloropyridin-3-yl | Me | H | N-oxide | H | H | H | F | H | heptafluoroisopropyl | H | F |
| 17-1779 | phenyl | Me | H | N-oxide | H | H | H | F | H | nonafluoro-2-butyl | H | F |
| 17-1780 | 3-cyanophenyl | Me | H | N-oxide | H | H | H | F | H | nonafluoro-2-butyl | H | F |
| 17-1781 | 4-cyanophenyl | Me | H | N-oxide | H | H | H | F | H | nonafluoro-2-butyl | H | F |
| 17-1782 | 2-chloropyridin-3-yl | Me | H | N-oxide | H | H | H | F | H | nonafluoro-2-butyl | H | F |
| 17-1783 | phenyl | Me | H | N-oxide | H | H | H | Cl | H | heptafluoroisopropyl | H | Cl |
| 17-1803 | 3-cyanophenyl | Me | H | N-oxide | H | H | H | Cl | H | heptafluoroisopropyl | H | Cl |
| 17-1804 | 4-cyanophenyl | Me | H | N-oxide | H | H | H | Cl | H | heptafluoroisopropyl | H | Cl |
| 17-1819 | 2-chloropyridin-3-yl | Me | H | N-oxide | H | H | H | Cl | H | heptafluoroisopropyl | H | Cl |
| 17-1861 | phenyl | Me | H | N-oxide | H | H | H | Cl | H | nonafluoro-2-butyl | H | Cl |
| 17-1881 | 3-cyanophenyl | Me | H | N-oxide | H | H | H | Cl | H | nonafluoro-2-butyl | H | Cl |
| 17-1882 | 4-cyanophenyl | Me | H | N-oxide | H | H | H | Cl | H | nonafluoro-2-butyl | H | Cl |
| 17-1897 | 2-chloropyridin-3-yl | Me | H | N-oxide | H | H | H | Cl | H | nonafluoro-2-butyl | H | Cl |
| 17-1939 | phenyl | Me | H | N-oxide | H | H | H | Br | H | heptafluoroisopropyl | H | Br |
| 17-1959 | 3-cyanophenyl | Me | H | N-oxide | H | H | H | Br | H | heptafluoroisopropyl | H | Br |
| 17-1960 | 4-cyanophenyl | Me | H | N-oxide | H | H | H | Br | H | heptafluoroisopropyl | H | Br |
| 17-1975 | 2-chloropyridin-3-yl | Me | H | N-oxide | H | H | H | Br | H | heptafluoroisopropyl | H | Br |
| 17-2017 | phenyl | Me | H | N-oxide | H | H | H | Br | H | nonafluoro-2-butyl | H | Br |
| 17-2037 | 3-cyanophenyl | Me | H | N-oxide | H | H | H | Br | H | nonafluoro-2-butyl | H | Br |
| 17-2038 | 4-cyanophenyl | Me | H | N-oxide | H | H | H | Br | H | nonafluoro-2-butyl | H | Br |
| 17-2053 | 2-chloropyridin-3-yl | Me | H | N-oxide | H | H | H | Br | H | nonafluoro-2-butyl | H | Br |
| 17-2095 | phenyl | Me | H | N-oxide | H | H | H | I | H | heptafluoroisopropyl | H | I |
| 17-2114 | 2-cyanophenyl | Me | H | N-oxide | H | H | H | I | H | pentafluoroethyl | H | I |
| 17-2115 | 3-cyanophenyl | Me | H | N-oxide | H | H | H | I | H | heptafluoroisopropyl | H | I |
| 17-2131 | 2-chloropyridin-3-yl | Me | H | N-oxide | H | H | H | I | H | heptafluoroisopropyl | H | I |
| 17-2173 | phenyl | Me | H | N-oxide | H | H | H | I | H | nonafluoro-2-butyl | H | I |
| 17-2193 | 3-cyanophenyl | Me | H | N-oxide | H | H | H | I | H | nonafluoro-2-butyl | H | I |
| 17-2194 | 4-cyanophenyl | Me | H | N-oxide | H | H | H | I | H | nonafluoro-2-butyl | H | I |
| 17-2209 | 2-chloropyridin-3-yl | Me | H | N-oxide | H | H | H | I | H | nonafluoro-2-butyl | H | I |
| 17-2251 | phenyl | Me | H | N-oxide | H | H | H | Cl | H | heptafluoroisopropyl | H | OCF3 |
| 17-2271 | 3-cyanophenyl | Me | H | N-oxide | H | H | H | Cl | H | heptafluoroisopropyl | H | OCF3 |
| 17-2272 | 4-cyanophenyl | Me | H | N-oxide | H | H | H | Cl | H | heptafluoroisopropyl | H | OCF3 |
| 17-2287 | 2-chloropyridin-3-yl | Me | H | N-oxide | H | H | H | Cl | H | heptafluoroisopropyl | H | OCF3 |
| 17-2329 | phenyl | Me | H | N-oxide | H | H | H | Cl | H | nonafluoro-2-butyl | H | OCF3 |
| 17-2349 | 3-cyanophenyl | Me | H | N-oxide | H | H | H | Cl | H | nonafluoro-2-butyl | H | OCF3 |
| 17-2350 | 4-cyanophenyl | Me | H | N-oxide | H | H | H | Cl | H | nonafluoro-2-butyl | H | OCF3 |
| 17-2365 | 2-chloropyridin-3-yl | Me | H | N-oxide | H | H | H | Cl | H | nonafluoro-2-butyl | H | OCF3 |
| 17-2407 | phenyl | Me | H | N-oxide | H | H | H | Br | H | heptafluoroisopropyl | H | OCF3 |
| 17-2427 | 3-cyanophenyl | Me | H | N-oxide | H | H | H | Br | H | heptafluoroisopropyl | H | OCF3 |
| 17-2428 | 4-cyanophenyl | Me | H | N-oxide | H | H | H | Br | H | heptafluoroisopropyl | H | OCF3 |

**Table 17(4)**

| compound number | Q₁ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 17-2443 | 2-chloropyridin-3-yl | Me | H | N-oxide | H | H | H | Br | H | heptafluoroisopropyl | H | OCF3 |
| 17-2485 | phenyl | Me | H | N-oxide | H | H | H | Br | H | nonafluoro-2-butyl | H | OCF3 |
| 17-2505 | 3-cyanophenyl | Me | H | N-oxide | H | H | H | Br | H | nonafluoro-2-butyl | H | OCF3 |
| 17-2506 | 4-cyanophenyl | Me | H | N-oxide | H | H | H | Br | H | nonafluoro-2-butyl | H | OCF3 |
| 17-2563 | phenyl | Me | H | N-oxide | H | H | H | I | H | heptafluoroisopropyl | H | OCF3 |
| 17-2583 | 3-cyanophenyl | Me | H | N-oxide | H | H | H | I | H | heptafluoroisopropyl | H | OCF3 |
| 17-2584 | 4-cyanophenyl | Me | H | N-oxide | H | H | H | I | H | heptafluoroisopropyl | H | OCF3 |
| 17-2599 | 2-chloropyridin-3-yl | Me | H | N-oxide | H | H | H | I | H | heptafluoroisopropyl | H | OCF3 |
| 17-2641 | phenyl | Me | H | N-oxide | H | H | H | I | H | nonafluoro-2-butyl | H | OCF3 |
| 17-2661 | 3-cyanophenyl | Me | H | N-oxide | H | H | H | I | H | nonafluoro-2-butyl | H | OCF3 |
| 17-2662 | 4-cyanophenyl | Me | H | N-oxide | H | H | H | I | H | nonafluoro-2-butyl | H | OCF3 |
| 17-2677 | 2-chloropyridin-3-yl | Me | H | N-oxide | H | H | H | I | H | nonafluoro-2-butyl | H | OCF3 |
| 17-2719 | phenyl | Me | H | N-oxide | H | H | H | F | H | heptafluoroisopropyl | H | CF3 |
| 17-2720 | phenyl | Me | H | N-oxide | H | H | H | Cl | H | heptafluoroisopropyl | H | CF3 |
| 17-2740 | 3-cyanophenyl | Me | H | N-oxide | H | H | H | Cl | H | heptafluoroisopropyl | H | CF3 |
| 17-2741 | 4-cyanophenyl | Me | H | N-oxide | H | H | H | Cl | H | heptafluoroisopropyl | H | CF3 |
| 17-2756 | 2-chloropyridin-3-yl | Me | H | N-oxide | H | H | H | Cl | H | heptafluoroisopropyl | H | CF3 |
| 17-2798 | phenyl | Me | H | N-oxide | H | H | H | F | H | nonafluoro-2-butyl | H | CF3 |
| 17-2799 | phenyl | Me | H | N-oxide | H | H | H | Cl | H | nonafluoro-2-butyl | H | CF3 |
| 17-2819 | 3-cyanophenyl | Me | H | N-oxide | H | H | H | Cl | H | nonafluoro-2-butyl | H | CF3 |
| 17-2820 | 4-cyanophenyl | Me | H | N-oxide | H | H | H | Cl | H | nonafluoro-2-butyl | H | CF3 |
| 17-2835 | 2-chloropyridin-3-yl | Me | H | N-oxide | H | H | H | Cl | H | nonafluoro-2-butyl | H | CF3 |
| 17-2877 | phenyl | Me | H | N-oxide | H | H | H | Br | H | heptafluoroisopropyl | H | CF3 |
| 17-2897 | 3-cyanophenyl | Me | H | N-oxide | H | H | H | Br | H | heptafluoroisopropyl | H | CF3 |
| 17-2898 | 4-cyanophenyl | Me | H | N-oxide | H | H | H | Br | H | heptafluoroisopropyl | H | CF3 |
| 17-2913 | 2-chloropyridin-3-yl | Me | H | N-oxide | H | H | H | Br | H | heptafluoroisopropyl | H | CF3 |
| 17-2914 | 2-bromopyridin-3-yl | Me | H | N-oxide | H | H | H | Br | H | heptafluoroisopropyl | H | CF3 |
| 17-2955 | phenyl | Me | H | N-oxide | H | H | H | Br | H | nonafluoro-2-butyl | H | CF3 |
| 17-2975 | 3-cyanophenyl | Me | H | N-oxide | H | H | H | Br | H | nonafluoro-2-butyl | H | CF3 |
| 17-2976 | 4-cyanophenyl | Me | H | N-oxide | H | H | H | Br | H | nonafluoro-2-butyl | H | CF3 |
| 17-2991 | 2-chloropyridin-3-yl | Me | H | N-oxide | H | H | H | Br | H | nonafluoro-2-butyl | H | CF3 |
| 17-3033 | phenyl | Me | H | N-oxide | H | H | H | I | H | heptafluoroisopropyl | H | CF3 |
| 17-3053 | 3-cyanophenyl | Me | H | N-oxide | H | H | H | I | H | heptafluoroisopropyl | H | CF3 |
| 17-3054 | 4-cyanophenyl | Me | H | N-oxide | H | H | H | I | H | heptafluoroisopropyl | H | CF3 |
| 17-3069 | 2-chloropyridin-3-yl | Me | H | N-oxide | H | H | H | I | H | heptafluoroisopropyl | H | CF3 |
| 17-3111 | phenyl | Me | H | N-oxide | H | H | H | I | H | nonafluoro-2-butyl | H | CF3 |
| 17-3131 | 3-cyanophenyl | Me | H | N-oxide | H | H | H | I | H | nonafluoro-2-butyl | H | CF3 |
| 17-3132 | 4-cyanophenyl | Me | H | N-oxide | H | H | H | I | H | nonafluoro-2-butyl | H | CF3 |
| 17-3147 | 2-chloropyridin-3-yl | Me | H | N-oxide | H | H | H | I | H | nonafluoro-2-butyl | H | CF3 |
| 17-3189 | phenyl | Me | H | N-oxide | H | H | H | OCF3 | H | heptafluoroisopropyl | H | OCF3 |
| 17-3220 | 2-chloropyridin-3-yl | Me | H | N-oxide | H | H | H | OCF3 | H | heptafluoroisopropyl | H | OCF3 |
| 17-3245 | phenyl | Me | H | N-oxide | H | H | H | OCF3 | H | nonafluoro-2-butyl | H | OCF3 |
| 17-3276 | 2-chloropyridin-3-yl | Me | H | N-oxide | H | H | H | OCF3 | H | nonafluoro-2-butyl | H | OCF3 |
| 17-3301 | phenyl | Me | H | N-oxide | H | H | H | CF3 | H | heptafluoroisopropyl | H | OCF3 |
| 17-3332 | 2-chloropyridin-3-yl | Me | H | N-oxide | H | H | H | CF3 | H | heptafluoroisopropyl | H | OCF3 |
| 17-3357 | phenyl | Me | H | N-oxide | H | H | H | CF3 | H | nonafluoro-2-butyl | H | OCF3 |
| 17-3388 | 2-chloropyridin-3-yl | Me | H | N-oxide | H | H | H | CF3 | H | nonafluoro-2-butyl | H | OCF3 |
| 17-3413 | phenyl | Me | H | N-oxide | H | H | H | CF3 | H | heptafluoroisopropyl | H | CF3 |
| 17-3433 | 3-cyanophenyl | Me | H | N-oxide | H | H | H | CF3 | H | heptafluoroisopropyl | H | CF3 |
| 17-3434 | 4-cyanophenyl | Me | H | N-oxide | H | H | H | CF3 | H | heptafluoroisopropyl | H | CF3 |
| 17-3449 | 2-chloropyridin-3-yl | Me | H | N-oxide | H | H | H | CF3 | H | heptafluoroisopropyl | H | CF3 |
| 17-3481 | phenyl | Me | H | N-oxide | H | H | H | CF3 | H | nonafluoro-2-butyl | H | CF3 |
| 17-3501 | 3-cyanophenyl | Me | H | N-oxide | H | H | H | CF3 | H | nonafluoro-2-butyl | H | CF3 |
| 17-3502 | 4-cyanophenyl | Me | H | N-oxide | H | H | H | CF3 | H | nonafluoro-2-butyl | H | CF3 |
| 17-3517 | 2-chloropyridin-3-yl | Me | H | N-oxide | H | H | H | CF3 | H | nonafluoro-2-butyl | H | CF3 |

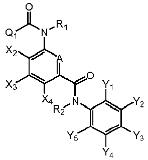

**Table 18(1)**

| compound number | Q₁ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 18-1 | phenyl | H | Me | N | H | H | H | F | H | heptafluoroisopropyl | H | F |
| 18-2 | 3-cyanophenyl | H | Me | N | H | H | H | F | H | heptafluoroisopropyl | H | F |
| 18-3 | 4-cyanophenyl | H | Me | N | H | H | H | F | H | heptafluoroisopropyl | H | F |
| 18-4 | 2-chloropyridin-3-yl | H | Me | N | H | H | H | F | H | heptafluoroisopropyl | H | F |
| 18-5 | phenyl | H | Me | N | H | H | H | F | H | nonafluoro-2-butyl | H | F |
| 18-6 | 3-cyanophenyl | H | Me | N | H | H | H | F | H | nonafluoro-2-butyl | H | F |
| 18-7 | 4-cyanophenyl | H | Me | N | H | H | H | F | H | nonafluoro-2-butyl | H | F |
| 18-8 | 2-chloropyridin-3-yl | H | Me | N | H | H | H | F | H | nonafluoro-2-butyl | H | F |
| 18-9 | phenyl | H | Me | N | H | H | H | Cl | H | heptafluoroisopropyl | H | Cl |
| 18-29 | 3-cyanophenyl | H | Me | N | H | H | H | Cl | H | heptafluoroisopropyl | H | Cl |
| 18-30 | 4-cyanophenyl | H | Me | N | H | H | H | Cl | H | heptafluoroisopropyl | H | Cl |
| 18-45 | 2-chloropyridin-3-yl | H | Me | N | H | H | H | Cl | H | heptafluoroisopropyl | H | Cl |
| 18-87 | phenyl | H | Me | N | H | H | H | Cl | H | nonafluoro-2-butyl | H | Cl |
| 18-107 | 3-cyanophenyl | H | Me | N | H | H | H | Cl | H | nonafluoro-2-butyl | H | Cl |
| 18-108 | 4-cyanophenyl | H | Me | N | H | H | H | Cl | H | nonafluoro-2-butyl | H | Cl |
| 18-123 | 2-chloropyridin-3-yl | H | Me | N | H | H | H | Cl | H | nonafluoro-2-butyl | H | Cl |
| 18-165 | phenyl | H | Me | N | H | H | H | Br | H | heptafluoroisopropyl | H | Br |
| 18-176 | 3-iodophenyl | H | Me | N | H | H | H | Br | H | pentafluoroethyl | H | Br |
| 18-177 | 4-iodophenyl | H | Me | N | H | H | H | Br | H | pentafluoroethyl | H | Br |
| 18-185 | 3-cyanophenyl | H | Me | N | H | H | H | Br | H | heptafluoroisopropyl | H | Br |
| 18-186 | 4-cyanophenyl | H | Me | N | H | H | H | Br | H | heptafluoroisopropyl | H | Br |
| 18-201 | 2-chloropyridin-3-yl | H | Me | N | H | H | H | Br | H | heptafluoroisopropyl | H | Br |
| 18-243 | phenyl | H | Me | N | H | H | H | Br | H | nonafluoro-2-butyl | H | Br |
| 18-263 | 3-cyanophenyl | H | Me | N | H | H | H | Br | H | nonafluoro-2-butyl | H | Br |
| 18-264 | 4-cyanophenyl | H | Me | N | H | H | H | Br | H | nonafluoro-2-butyl | H | Br |
| 18-279 | 2-chloropyridin-3-yl | H | Me | N | H | H | H | Br | H | nonafluoro-2-butyl | H | Br |
| 18-321 | phenyl | H | Me | N | H | H | H | I | H | heptafluoroisopropyl | H | I |
| 18-341 | 3-cyanophenyl | H | Me | N | H | H | H | I | H | heptafluoroisopropyl | H | I |
| 18-342 | 4-cyanophenyl | H | Me | N | H | H | H | I | H | heptafluoroisopropyl | H | I |
| 18-357 | 2-chloropyridin-3-yl | H | Me | N | H | H | H | I | H | heptafluoroisopropyl | H | I |
| 18-399 | phenyl | H | Me | N | H | H | H | I | H | nonafluoro-2-butyl | H | I |
| 18-419 | 3-cyanophenyl | H | Me | N | H | H | H | I | H | nonafluoro-2-butyl | H | I |
| 18-420 | 4-cyanophenyl | H | Me | N | H | H | H | I | H | nonafluoro-2-butyl | H | I |
| 18-435 | 2-chloropyridin-3-yl | H | Me | N | H | H | H | I | H | nonafluoro-2-butyl | H | I |
| 18-477 | phenyl | H | Me | N | H | H | H | Cl | H | heptafluoroisopropyl | H | OCF3 |
| 18-497 | 3-cyanophenyl | H | Me | N | H | H | H | Cl | H | heptafluoroisopropyl | H | OCF3 |
| 18-498 | 4-cyanophenyl | H | Me | N | H | H | H | Cl | H | heptafluoroisopropyl | H | OCF3 |
| 18-513 | 2-chloropyridin-3-yl | H | Me | N | H | H | H | Cl | H | heptafluoroisopropyl | H | OCF3 |
| 18-555 | phenyl | H | Me | N | H | H | H | Cl | H | nonafluoro-2-butyl | H | OCF3 |
| 18-575 | 3-cyanophenyl | H | Me | N | H | H | H | Cl | H | nonafluoro-2-butyl | H | OCF3 |
| 18-576 | 4-cyanophenyl | H | Me | N | H | H | H | Cl | H | nonafluoro-2-butyl | H | OCF3 |
| 18-591 | 2-chloropyridin-3-yl | H | Me | N | H | H | H | Cl | H | nonafluoro-2-butyl | H | OCF3 |
| 18-633 | phenyl | H | Me | N | H | H | H | Br | H | heptafluoroisopropyl | H | OCF3 |
| 18-653 | 3-cyanophenyl | H | Me | N | H | H | H | Br | H | heptafluoroisopropyl | H | OCF3 |
| 18-654 | 4-cyanophenyl | H | Me | N | H | H | H | Br | H | heptafluoroisopropyl | H | OCF3 |
| 18-669 | 2-chloropyridin-3-yl | H | Me | N | H | H | H | Br | H | heptafluoroisopropyl | H | OCF3 |
| 18-711 | phenyl | H | Me | N | H | H | H | Br | H | nonafluoro-2-butyl | H | OCF3 |
| 18-731 | 3-cyanophenyl | H | Me | N | H | H | H | Br | H | nonafluoro-2-butyl | H | OCF3 |
| 18-732 | 4-cyanophenyl | H | Me | N | H | H | H | Br | H | nonafluoro-2-butyl | H | OCF3 |
| 18-747 | 2-chloropyridin-3-yl | H | Me | N | H | H | H | Br | H | nonafluoro-2-butyl | H | OCF3 |
| 18-789 | phenyl | H | Me | N | H | H | H | I | H | heptafluoroisopropyl | H | OCF3 |
| 18-809 | 3-cyanophenyl | H | Me | N | H | H | H | I | H | heptafluoroisopropyl | H | OCF3 |
| 18-810 | 4-cyanophenyl | H | Me | N | H | H | H | I | H | heptafluoroisopropyl | H | OCF3 |
| 18-825 | 2-chloropyridin-3-yl | H | Me | N | H | H | H | I | H | heptafluoroisopropyl | H | OCF3 |
| 18-867 | phenyl | H | Me | N | H | H | H | I | H | nonafluoro-2-butyl | H | OCF3 |

**Table 18(2)**

| compound number | Q₁ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 18-887 | 3-cyanophenyl | H | Me | N | H | H | H | I | H | nonafluoro-2-butyl | H | OCF3 |
| 18-888 | 4-cyanophenyl | H | Me | N | H | H | H | I | H | nonafluoro-2-butyl | H | OCF3 |
| 18-903 | 2-chloropyridin-3-yl | H | Me | N | H | H | H | I | H | nonafluoro-2-butyl | H | OCF3 |
| 18-945 | phenyl | H | Me | N | H | H | H | F | H | heptafluoroisopropyl | H | CF3 |
| 18-946 | phenyl | H | Me | N | H | H | H | Cl | H | heptafluoroisopropyl | H | CF3 |
| 18-957 | 3-iodophenyl | H | Me | N | H | H | H | Cl | H | pentafluoroethyl | H | CF3 |
| 18-958 | 4-iodophenyl | H | Me | N | H | H | H | Cl | H | pentafluoroethyl | H | CF3 |
| 18-960 | (3-trifluoromethyl)phenyl | H | Et | N | H | H | H | Cl | H | heptafluoroisopropyl | H | CF3 |
| 18-965 | 2-cyanophenyl | H | Me | N | H | H | H | Cl | H | pentafluoroethyl | H | C2F5 |
| 18-966 | 3-cyanophenyl | H | Me | N | H | H | H | Cl | H | heptafluoroisopropyl | H | CF3 |
| 18-967 | 4-cyanophenyl | H | Me | N | H | H | H | Cl | H | heptafluoroisopropyl | H | CF3 |
| 18-970 | 2,4-dichlorophenyl | H | Me | N | H | H | H | Cl | H | heptafluoroisopropyl | H | C2F5 |
| 18-971 | 2-chloro-4-fluorophenyl | H | n-Pr | N | H | H | H | Cl | H | heptafluoroisopropyl | H | CF3 |
| 18-974 | 2-bromo-4-chlorophenyl | H | Me | N | H | H | H | Cl | H | heptafluoroisopropyl | H | n-C3F7 |
| 18-981 | 2-fluoropyridin-3-yl | H | Me | N | H | H | H | Cl | H | pentafluoroethyl | H | CF3 |
| 18-982 | 2-chloropyridin-3-yl | H | Me | N | H | H | H | Cl | H | heptafluoroisopropyl | H | CF3 |
| 18-988 | 6-fluoropyridin-3-yl | H | i-Pr | N | H | H | H | Cl | H | heptafluoroisopropyl | H | CF3 |
| 18-995 | 5-fluoropyridin-3-yl | H | Me | N | H | H | H | Cl | H | heptafluoroisopropyl | H | C2F5 |
| 18-997 | 5-bromopyridin-3-yl | H | Me | N | H | H | H | Cl | H | heptafluoroisopropyl | H | n-C3F7 |
| 18-998 | 5-iodopyridin-3-yl | H | Me | N | H | H | H | Cl | H | heptafluoroisopropyl | H | i-C3F7 |
| 18-1000 | 5-nitropyridin-3-yl | H | Me | N | H | H | H | Cl | H | pentafluoroethyl | H | CF3 |
| 18-1002 | 4-fluoropyridin-3-yl | H | Me | N | H | H | H | Cl | H | pentafluoroethyl | H | CF3 |
| 18-1004 | 4-bromopyridin-3-yl | H | CH2CH=CH2 | N | H | H | H | Cl | H | heptafluoroisopropyl | H | n-C3F7 |
| 18-1005 | 4-iodopyridin-3-yl | H | Me | N | H | H | H | Cl | H | heptafluoroisopropyl | H | i-C3F7 |
| 18-1011 | pyridin-4-yl | H | CN | N | H | H | H | Cl | H | heptafluoroisopropyl | H | CF3 |
| 18-1023 | 4-cyanopyridin-2-yl | H | Me | N | H | H | H | Cl | H | pentafluoroethyl | H | CF3 |
| 18-1024 | phenyl | H | Me | N | H | H | H | F | H | nonafluoro-2-butyl | H | CF3 |
| 18-1025 | phenyl | H | Me | N | H | H | H | Cl | H | nonafluoro-2-butyl | H | CF3 |
| 18-1033 | 3-bromophenyl | H | CH2C≡CH | N | H | H | H | Cl | H | nonafluoro-2-butyl | H | CF3 |
| 18-1038 | (2-trifluoromethyl)phenyl | H | Me | N | H | H | H | Cl | H | nonafluoro-2-butyl | H | C2F5 |
| 18-1040 | (4-trifluoromethyl) phenyl | H | NH2 | N | H | H | H | Cl | H | nonafluoro-2-butyl | H | CF3 |
| 18-1045 | 3-cyanophenyl | H | Me | N | H | H | H | Cl | H | nonafluoro-2-butyl | H | CF3 |
| 18-1046 | 4-cyanophenyl | H | Me | N | H | H | H | Cl | H | nonafluoro-2-butyl | H | CF3 |
| 18-1053 | 2-bromo-4-chlorophenyl | H | C(O)OMe | N | H | H | H | Cl | H | nonafluoro-2-butyl | H | CF3 |
| 18-1055 | 2-chloro-4-nitrophenyl | H | Me | N | H | H | H | Cl | H | nonafluoro-2-butyl | H | i-C3F7 |
| 18-1061 | 2-chloropyridin-3-yl | H | Me | N | H | H | H | Cl | H | nonafluoro-2-butyl | H | CF3 |
| 18-1074 | 5-fluoropyridin-3-yl | H | C(O)OEt | N | H | H | H | Cl | H | nonafluoro-2-butyl | H | C2F5 |
| 18-1076 | 5-bromopyridin-3-yl | H | Me | N | H | H | H | Cl | H | nonafluoro-2-butyl | H | n-C3F7 |
| 18-1077 | 5-iodopyridin-3-yl | H | Me | N | H | H | H | Cl | H | nonafluoro-2-butyl | H | i-C3F7 |
| 18-1081 | 4-fluoropyridin-3-yl | H | Me | N | H | H | H | Cl | H | nonafluoro-2-butyl | H | C2F5 |
| 18-1083 | 4-bromopyridin-3-yl | H | C(O)C(O)Me | N | H | H | H | Cl | H | nonafluoro-2-butyl | H | CF3 |
| 18-1085 | 4-(trifluoromethyl)pyridin-3-yl | H | Me | N | H | H | H | Cl | H | nonafluoro-2-butyl | H | n-C3F7 |
| 18-1086 | 4-nitropyridin-3-yl | H | C(O)C(O)Et | N | H | H | H | Cl | H | nonafluoro-2-butyl | H | i-C3F7 |
| 18-1100 | 5-iodopyridin-2-yl | H | C(O)Me | N | H | H | H | Cl | H | nonafluoro-2-butyl | H | CF3 |
| 18-1102 | 4-cyanopyridin-2-yl | H | C(O)Et | N | H | H | H | Cl | H | nonafluoro-2-butyl | H | C2F5 |
| 18-1103 | phenyl | H | Me | N | H | H | H | Br | H | heptafluoroisopropyl | H | CF3 |
| 18-1104 | 2-fluorophenyl | H | Me | N | H | H | H | Br | H | heptafluoroisopropyl | H | CF3 |
| 18-1117 | (3-trifluoromethyl)phenyl | H | S(O)2Me | N | H | H | H | Br | H | heptafluoroisopropyl | H | CF3 |
| 18-1123 | 3-cyanophenyl | H | Me | N | H | H | H | Br | H | heptafluoroisopropyl | H | CF3 |
| 18-1124 | 4-cyanophenyl | H | Me | N | H | H | H | Br | H | heptafluoroisopropyl | H | CF3 |
| 18-1135 | 4-cyano-2-fluorophenyl | H | S(O)2Et | N | H | H | H | Br | H | heptafluoroisopropyl | H | CF3 |
| 18-1139 | 2-chloropyridin-3-yl | H | Me | N | H | H | H | Br | H | heptafluoroisopropyl | H | CF3 |
| 18-1150 | 6-nitropyridin-3-yl | H | CH2Ph | N | H | H | H | Br | H | heptafluoroisopropyl | H | CF3 |
| 18-1159 | 4-fluoropyridin-3-yl | H | CH2(3-Py) | N | H | H | H | Br | H | heptafluoroisopropyl | H | CF3 |
| 18-1180 | 4-cyanopyridin-2-yl | H | CH2(3-Py-N-oxide) | N | H | H | H | Br | H | heptafluoroisopropyl | H | CF3 |
| 18-1181 | phenyl | H | Me | N | H | H | H | Br | H | nonafluoro-2-butyl | H | CF3 |
| 18-1182 | 2-fluorophenyl | H | Me | N | H | H | H | Br | H | nonafluoro-2-butyl | H | CF3 |
| 18-1201 | 3-cyanophenyl | H | Me | N | H | H | H | Br | H | nonafluoro-2-butyl | H | CF3 |
| 18-1202 | 4-cyanophenyl | H | Me | N | H | H | H | Br | H | nonafluoro-2-butyl | H | CF3 |
| 18-1208 | 4-bromo-2-chlorophenyl | H | Et | N | H | H | H | Br | H | nonafluoro-2-butyl | H | CF3 |
| 18-1217 | 2-chloropyridin-3-yl | H | Me | N | H | H | H | Br | H | nonafluoro-2-butyl | H | CF3 |
| 18-1234 | 5-(trifluoromethyl)pyridin-3-yl | H | CN | N | H | H | H | Br | H | nonafluoro-2-butyl | H | CF3 |
| 18-1259 | phenyl | H | Me | N | H | H | H | I | H | heptafluoroisopropyl | H | CF3 |
| 18-1260 | 2-fluorophenyl | H | Me | N | H | H | H | I | H | heptafluoroisopropyl | H | CF3 |
| 18-1279 | 3-cyanophenyl | H | Me | N | H | H | H | I | H | heptafluoroisopropyl | H | CF3 |

**Table 18(3)**

| compound number | Q₁ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 18-1280 | 4-cyanophenyl | H | Me | N | H | H | H | I | H | heptafluoroisopropyl | H | CF3 |
| 18-1295 | 2-chloropyridin-3-yl | H | Me | N | H | H | H | I | H | heptafluoroisopropyl | H | CF3 |
| 18-1308 | 5-fluoropyridin-3-yl | H | C(O)Me | N | H | H | H | I | H | heptafluoroisopropyl | H | CF3 |
| 18-1337 | phenyl | H | Me | N | H | H | H | I | H | nonafluoro-2-butyl | H | CF3 |
| 18-1338 | 2-fluorophenyl | H | Me | N | H | H | H | I | H | nonafluoro-2-butyl | H | CF3 |
| 18-1357 | 3-cyanophenyl | H | Me | N | H | H | H | I | H | nonafluoro-2-butyl | H | CF3 |
| 18-1358 | 4-cyanophenyl | H | Me | N | H | H | H | I | H | nonafluoro-2-butyl | H | CF3 |
| 18-1373 | 2-chloropyridin-3-yl | H | Me | N | H | H | H | I | H | nonafluoro-2-butyl | H | CF3 |
| 18-1415 | phenyl | H | Me | N | H | H | H | OCF3 | H | heptafluoroisopropyl | H | OCF3 |
| 18-1446 | 2-chloropyridin-3-yl | H | Me | N | H | H | H | OCF3 | H | heptafluoroisopropyl | H | OCF3 |
| 18-1471 | phenyl | H | Me | N | H | H | H | OCF3 | H | nonafluoro-2-butyl | H | OCF3 |
| 18-1502 | 2-chloropyridin-3-yl | H | Me | N | H | H | H | OCF3 | H | nonafluoro-2-butyl | H | OCF3 |
| 18-1527 | phenyl | H | Me | N | H | H | H | CF3 | H | heptafluoroisopropyl | H | OCF3 |
| 18-1558 | 2-chloropyridin-3-yl | H | Me | N | H | H | H | CF3 | H | heptafluoroisopropyl | H | OCF3 |
| 18-1583 | phenyl | H | Me | N | H | H | H | CF3 | H | nonafluoro-2-butyl | H | OCF3 |
| 18-1614 | 2-chloropyridin-3-yl | H | Me | N | H | H | H | CF3 | H | nonafluoro-2-butyl | H | OCF3 |
| 18-1639 | phenyl | H | Me | N | H | H | H | CF3 | H | heptafluoroisopropyl | H | CF3 |
| 18-1659 | 3-cyanophenyl | H | Me | N | H | H | H | CF3 | H | heptafluoroisopropyl | H | CF3 |
| 18-1660 | 4-cyanophenyl | H | Me | N | H | H | H | CF3 | H | heptafluoroisopropyl | H | CF3 |
| 18-1675 | 2-chloropyridin-3-yl | H | Me | N | H | H | H | CF3 | H | heptafluoroisopropyl | H | CF3 |
| 18-1707 | phenyl | H | Me | N | H | H | H | CF3 | H | nonafluoro-2-butyl | H | CF3 |
| 18-1727 | 3-cyanophenyl | H | Me | N | H | H | H | CF3 | H | nonafluoro-2-butyl | H | CF3 |
| 18-1728 | 4-cyanophenyl | H | Me | N | H | H | H | CF3 | H | nonafluoro-2-butyl | H | CF3 |
| 18-1743 | 2-chloropyridin-3-yl | H | Me | N | H | H | H | CF3 | H | nonafluoro-2-butyl | H | CF3 |
| 18-1775 | phenyl | H | Me | N-oxide | H | H | H | F | H | heptafluoroisopropyl | H | F |
| 18-1776 | 3-cyanophenyl | H | Me | N-oxide | H | H | H | F | H | heptafluoroisopropyl | H | F |
| 18-1777 | 4-cyanophenyl | H | Me | N-oxide | H | H | H | F | H | heptafluoroisopropyl | H | F |
| 18-1778 | 2-chloropyridin-3-yl | H | Me | N-oxide | H | H | H | F | H | heptafluoroisopropyl | H | F |
| 18-1779 | phenyl | H | Me | N-oxide | H | H | H | F | H | nonafluoro-2-butyl | H | F |
| 18-1780 | 3-cyanophenyl | H | Me | N-oxide | H | H | H | F | H | nonafluoro-2-butyl | H | F |
| 18-1781 | 4-cyanophenyl | H | Me | N-oxide | H | H | H | F | H | nonafluoro-2-butyl | H | F |
| 18-1782 | 2-chloropyridin-3-yl | H | Me | N-oxide | H | H | H | F | H | nonafluoro-2-butyl | H | F |
| 18-1783 | phenyl | H | Me | N-oxide | H | H | H | Cl | H | heptafluoroisopropyl | H | Cl |
| 18-1803 | 3-cyanophenyl | H | Me | N-oxide | H | H | H | Cl | H | heptafluoroisopropyl | H | Cl |
| 18-1804 | 4-cyanophenyl | H | Me | N-oxide | H | H | H | Cl | H | heptafluoroisopropyl | H | Cl |
| 18-1819 | 2-chloropyridin-3-yl | H | Me | N-oxide | H | H | H | Cl | H | heptafluoroisopropyl | H | Cl |
| 18-1861 | phenyl | H | Me | N-oxide | H | H | H | Cl | H | nonafluoro-2-butyl | H | Cl |
| 18-1881 | 3-cyanophenyl | H | Me | N-oxide | H | H | H | Cl | H | nonafluoro-2-butyl | H | Cl |
| 18-1882 | 4-cyanophenyl | H | Me | N-oxide | H | H | H | Cl | H | nonafluoro-2-butyl | H | Cl |
| 18-1897 | 2-chloropyridin-3-yl | H | Me | N-oxide | H | H | H | Cl | H | nonafluoro-2-butyl | H | Cl |
| 18-1939 | phenyl | H | Me | N-oxide | H | H | H | Br | H | heptafluoroisopropyl | H | Br |
| 18-1959 | 3-cyanophenyl | H | Me | N-oxide | H | H | H | Br | H | heptafluoroisopropyl | H | Br |
| 18-1960 | 4-cyanophenyl | H | Me | N-oxide | H | H | H | Br | H | heptafluoroisopropyl | H | Br |
| 18-1975 | 2-chloropyridin-3-yl | H | Me | N-oxide | H | H | H | Br | H | heptafluoroisopropyl | H | Br |
| 18-2017 | phenyl | H | Me | N-oxide | H | H | H | Br | H | nonafluoro-2-butyl | H | Br |
| 18-2037 | 3-cyanophenyl | H | Me | N-oxide | H | H | H | Br | H | nonafluoro-2-butyl | H | Br |
| 18-2038 | 4-cyanophenyl | H | Me | N-oxide | H | H | H | Br | H | nonafluoro-2-butyl | H | Br |
| 18-2053 | 2-chloropyridin-3-yl | H | Me | N-oxide | H | H | H | Br | H | nonafluoro-2-butyl | H | Br |
| 18-2095 | phenyl | H | Me | N-oxide | H | H | H | I | H | heptafluoroisopropyl | H | I |
| 18-2115 | 3-cyanophenyl | H | Me | N-oxide | H | H | H | I | H | heptafluoroisopropyl | H | I |
| 18-2116 | 4-cyanophenyl | H | Me | N-oxide | H | H | H | I | H | heptafluoroisopropyl | H | I |
| 18-2131 | 2-chloropyridin-3-yl | H | Me | N-oxide | H | H | H | I | H | heptafluoroisopropyl | H | I |
| 18-2173 | phenyl | H | Me | N-oxide | H | H | H | I | H | nonafluoro-2-butyl | H | I |
| 18-2193 | 3-cyanophenyl | H | Me | N-oxide | H | H | H | I | H | nonafluoro-2-butyl | H | I |
| 18-2194 | 4-cyanophenyl | H | Me | N-oxide | H | H | H | I | H | nonafluoro-2-butyl | H | I |
| 18-2209 | 2-chloropyridin-3-yl | H | Me | N-oxide | H | H | H | I | H | nonafluoro-2-butyl | H | I |
| 18-2251 | phenyl | H | Me | N-oxide | H | H | H | Cl | H | heptafluoroisopropyl | H | OCF3 |
| 18-2271 | 3-cyanophenyl | H | Me | N-oxide | H | H | H | Cl | H | heptafluoroisopropyl | H | OCF3 |
| 18-2272 | 4-cyanophenyl | H | Me | N-oxide | H | H | H | Cl | H | heptafluoroisopropyl | H | OCF3 |
| 18-2287 | 2-chloropyridin-3-yl | H | Me | N-oxide | H | H | H | Cl | H | heptafluoroisopropyl | H | OCF3 |
| 18-2291 | 2-nitropyridin-3-yl | H | Me | N-oxide | H | H | H | Cl | H | heptafluoroisopropyl | H | OC2F5 |
| 18-2329 | phenyl | H | Me | N-oxide | H | H | H | Cl | H | nonafluoro-2-butyl | H | OCF3 |
| 18-2349 | 3-cyanophenyl | H | Me | N-oxide | H | H | H | Cl | H | nonafluoro-2-butyl | H | OCF3 |

**Table 18(4)**

| compound number | Q₁ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 18-2350 | 4-cyanophenyl | H | Me | N-oxide | H | H | H | Cl | H | nonafluoro-2-butyl | H | OCF3 |
| 18-2365 | 2-chloropyridin-3-yl | H | Me | N-oxide | H | H | H | Cl | H | nonafluoro-2-butyl | H | OCF3 |
| 18-2407 | phenyl | H | Me | N-oxide | H | H | H | Br | H | heptafluoroisopropyl | H | OCF3 |
| 18-2427 | 3-cyanophenyl | H | Me | N-oxide | H | H | H | Br | H | heptafluoroisopropyl | H | OCF3 |
| 18-2428 | 4-cyanophenyl | H | Me | N-oxide | H | H | H | Br | H | heptafluoroisopropyl | H | OCF3 |
| 18-2443 | 2-chloropyridin-3-yl | H | Me | N-oxide | H | H | H | Br | H | heptafluoroisopropyl | H | OCF3 |
| 18-2485 | phenyl | H | Me | N-oxide | H | H | H | Br | H | nonafluoro-2-butyl | H | OCF3 |
| 18-2505 | 3-cyanophenyl | H | Me | N-oxide | H | H | H | Br | H | nonafluoro-2-butyl | H | OCF3 |
| 18-2506 | 4-cyanophenyl | H | Me | N-oxide | H | H | H | Br | H | nonafluoro-2-butyl | H | OCF3 |
| 18-2521 | 2-chloropyridin-3-yl | H | Me | N-oxide | H | H | H | Br | H | nonafluoro-2-butyl | H | OCF3 |
| 18-2563 | phenyl | H | Me | N-oxide | H | H | H | I | H | heptafluoroisopropyl | H | OCF3 |
| 18-2583 | 3-cyanophenyl | H | Me | N-oxide | H | H | H | I | H | heptafluoroisopropyl | H | OCF3 |
| 18-2584 | 4-cyanophenyl | H | Me | N-oxide | H | H | H | I | H | heptafluoroisopropyl | H | OCF3 |
| 18-2599 | 2-chloropyridin-3-yl | H | Me | N-oxide | H | H | H | I | H | heptafluoroisopropyl | H | OCF3 |
| 18-2641 | phenyl | H | Me | N-oxide | H | H | H | I | H | nonafluoro-2-butyl | H | OCF3 |
| 18-2661 | 3-cyanophenyl | H | Me | N-oxide | H | H | H | I | H | nonafluoro-2-butyl | H | OCF3 |
| 18-2662 | 4-cyanophenyl | H | Me | N-oxide | H | H | H | I | H | nonafluoro-2-butyl | H | OCF3 |
| 18-2677 | 2-chloropyridin-3-yl | H | Me | N-oxide | H | H | H | I | H | nonafluoro-2-butyl | H | OCF3 |
| 18-2719 | phenyl | H | Me | N-oxide | H | H | H | F | H | heptafluoroisopropyl | H | CF3 |
| 18-2720 | phenyl | H | Me | N-oxide | H | H | H | Cl | H | heptafluoroisopropyl | H | CF3 |
| 18-2740 | 3-cyanophenyl | H | Me | N-oxide | H | H | H | Cl | H | heptafluoroisopropyl | H | CF3 |
| 18-2741 | 4-cyanophenyl | H | Me | N-oxide | H | H | H | Cl | H | heptafluoroisopropyl | H | CF3 |
| 18-2756 | 2-chloropyridin-3-yl | H | Me | N-oxide | H | H | H | Cl | H | heptafluoroisopropyl | H | CF3 |
| 18-2798 | phenyl | H | Me | N-oxide | H | H | H | F | H | nonafluoro-2-butyl | H | CF3 |
| 18-2799 | phenyl | H | Me | N-oxide | H | H | H | Cl | H | nonafluoro-2-butyl | H | CF3 |
| 18-2819 | 3-cyanophenyl | H | Me | N-oxide | H | H | H | Cl | H | nonafluoro-2-butyl | H | CF3 |
| 18-2820 | 4-cyanophenyl | H | Me | N-oxide | H | H | H | Cl | H | nonafluoro-2-butyl | H | CF3 |
| 18-2835 | 2-chloropyridin-3-yl | H | Me | N-oxide | H | H | H | Cl | H | nonafluoro-2-butyl | H | CF3 |
| 18-2877 | phenyl | H | Me | N-oxide | H | H | H | Br | H | heptafluoroisopropyl | H | CF3 |
| 18-2897 | 3-cyanophenyl | H | Me | N-oxide | H | H | H | Br | H | heptafluoroisopropyl | H | CF3 |
| 18-2898 | 4-cyanophenyl | H | Me | N-oxide | H | H | H | Br | H | heptafluoroisopropyl | H | CF3 |
| 18-2913 | 2-chloropyridin-3-yl | H | Me | N-oxide | H | H | H | Br | H | heptafluoroisopropyl | H | CF3 |
| 18-2955 | phenyl | H | Me | N-oxide | H | H | H | Br | H | nonafluoro-2-butyl | H | CF3 |
| 18-2975 | 3-cyanophenyl | H | Me | N-oxide | H | H | H | Br | H | nonafluoro-2-butyl | H | CF3 |
| 18-2976 | 4-cyanophenyl | H | Me | N-oxide | H | H | H | Br | H | nonafluoro-2-butyl | H | CF3 |
| 18-2991 | 2-chloropyridin-3-yl | H | Me | N-oxide | H | H | H | Br | H | nonafluoro-2-butyl | H | CF3 |
| 18-3033 | phenyl | H | Me | N-oxide | H | H | H | I | H | heptafluoroisopropyl | H | CF3 |
| 18-3053 | 3-cyanophenyl | H | Me | N-oxide | H | H | H | I | H | heptafluoroisopropyl | H | CF3 |
| 18-3054 | 4-cyanophenyl | H | Me | N-oxide | H | H | H | I | H | heptafluoroisopropyl | H | CF3 |
| 18-3069 | 2-chloropyridin-3-yl | H | Me | N-oxide | H | H | H | I | H | heptafluoroisopropyl | H | CF3 |
| 18-3111 | phenyl | H | Me | N-oxide | H | H | H | I | H | nonafluoro-2-butyl | H | CF3 |
| 18-3131 | 3-cyanophenyl | H | Me | N-oxide | H | H | H | I | H | nonafluoro-2-butyl | H | CF3 |
| 18-3132 | 4-cyanophenyl | H | Me | N-oxide | H | H | H | I | H | nonafluoro-2-butyl | H | CF3 |
| 18-3147 | 2-chloropyridin-3-yl | H | Me | N-oxide | H | H | H | I | H | nonafluoro-2-butyl | H | CF3 |
| 18-3189 | phenyl | H | Me | N-oxide | H | H | H | OCF3 | H | heptafluoroisopropyl | H | OCF3 |
| 18-3220 | 2-chloropyridin-3-yl | H | Me | N-oxide | H | H | H | OCF3 | H | heptafluoroisopropyl | H | OCF3 |
| 18-3245 | phenyl | H | Me | N-oxide | H | H | H | OCF3 | H | nonafluoro-2-butyl | H | OCF3 |
| 18-3276 | 2-chloropyridin-3-yl | H | Me | N-oxide | H | H | H | OCF3 | H | nonafluoro-2-butyl | H | OCF3 |
| 18-3301 | phenyl | H | Me | N-oxide | H | H | H | CF3 | H | heptafluoroisopropyl | H | OCF3 |
| 18-3332 | 2-chloropyridin-3-yl | H | Me | N-oxide | H | H | H | CF3 | H | heptafluoroisopropyl | H | OCF3 |
| 18-3357 | phenyl | H | Me | N-oxide | H | H | H | CF3 | H | nonafluoro-2-butyl | H | OCF3 |
| 18-3388 | 2-chloropyridin-3-yl | H | Me | N-oxide | H | H | H | CF3 | H | nonafluoro-2-butyl | H | OCF3 |
| 18-3413 | phenyl | H | Me | N-oxide | H | H | H | CF3 | H | heptafluoroisopropyl | H | CF3 |
| 18-3433 | 3-cyanophenyl | H | Me | N-oxide | H | H | H | CF3 | H | heptafluoroisopropyl | H | CF3 |
| 18-3434 | 4-cyanophenyl | H | Me | N-oxide | H | H | H | CF3 | H | heptafluoroisopropyl | H | CF3 |
| 18-3449 | 2-chloropyridin-3-yl | H | Me | N-oxide | H | H | H | CF3 | H | heptafluoroisopropyl | H | CF3 |
| 18-3481 | phenyl | H | Me | N-oxide | H | H | H | CF3 | H | nonafluoro-2-butyl | H | CF3 |
| 18-3501 | 3-cyanophenyl | H | Me | N-oxide | H | H | H | CF3 | H | nonafluoro-2-butyl | H | CF3 |
| 18-3502 | 4-cyanophenyl | H | Me | N-oxide | H | H | H | CF3 | H | nonafluoro-2-butyl | H | CF3 |
| 18-3517 | 2-chloropyridin-3-yl | H | Me | N-oxide | H | H | H | CF3 | H | nonafluoro-2-butyl | H | CF3 |

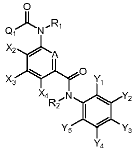

**Table 19(1)**

| compound number | Q₁ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 19-1 | phenyl | Me | Me | N | H | H | H | F | H | heptafluoroisopropyl | H | F |
| 19-2 | 3-cyanophenyl | Me | Me | N | H | H | H | F | H | heptafluoroisopropyl | H | F |
| 19-3 | 4-cyanophenyl | Me | Me | N | H | H | H | F | H | heptafluoroisopropyl | H | F |
| 19-4 | 2-chloropyridin-3-yl | Me | Me | N | H | H | H | F | H | heptafluoroisopropyl | H | F |
| 19-5 | phenyl | Me | Me | N | H | H | H | F | H | nonafluoro-2-butyl | H | F |
| 19-6 | 3-cyanophenyl | Me | Me | N | H | H | H | F | H | nonafluoro-2-butyl | H | F |
| 19-7 | 4-cyanophenyl | Me | Me | N | H | H | H | F | H | nonafluoro-2-butyl | H | F |
| 19-8 | 2-chloropyridin-3-yl | Me | Me | N | H | H | H | F | H | nonafluoro-2-butyl | H | F |
| 19-9 | phenyl | Me | Me | N | H | H | H | Cl | H | heptafluoroisopropyl | H | Cl |
| 19-11 | 3-fluorophenyl | Et | Me | N | H | H | H | Cl | H | heptafluoroisopropyl | H | Cl |
| 19-12 | 4-fluorophenyl | Me | Et | N | H | H | H | Cl | H | heptafluoroisopropyl | H | Cl |
| 19-29 | 3-cyanophenyl | Me | Me | N | H | H | H | Cl | H | heptafluoroisopropyl | H | Cl |
| 19-30 | 4-cyanophenyl | Me | Me | N | H | H | H | Cl | H | heptafluoroisopropyl | H | Cl |
| 19-45 | 2-chloropyridin-3-yl | Me | Me | N | H | H | H | Cl | H | heptafluoroisopropyl | H | Cl |
| 19-87 | phenyl | Me | Me | N | H | H | H | Cl | H | nonafluoro-2-butyl | H | Cl |
| 19-107 | 3-cyanophenyl | Me | Me | N | H | H | H | Cl | H | nonafluoro-2-butyl | H | Cl |
| 19-108 | 4-cyanophenyl | Me | Me | N | H | H | H | Cl | H | nonafluoro-2-butyl | H | Cl |
| 19-123 | 2-chloropyridin-3-yl | Me | Me | N | H | H | H | Cl | H | nonafluoro-2-butyl | H | Cl |
| 19-165 | phenyl | Me | Me | N | H | H | H | Br | H | heptafluoroisopropyl | H | Br |
| 19-185 | 3-cyanophenyl | Me | Me | N | H | H | H | Br | H | heptafluoroisopropyl | H | Br |
| 19-186 | 4-cyanophenyl | Me | Me | N | H | H | H | Br | H | heptafluoroisopropyl | H | Br |
| 19-201 | 2-chloropyridin-3-yl | Me | Me | N | H | H | H | Br | H | heptafluoroisopropyl | H | Br |
| 19-243 | phenyl | Me | Me | N | H | H | H | Br | H | nonafluoro-2-butyl | H | Br |
| 19-263 | 3-cyanophenyl | Me | Me | N | H | H | H | Br | H | nonafluoro-2-butyl | H | Br |
| 19-264 | 4-cyanophenyl | Me | Me | N | H | H | H | Br | H | nonafluoro-2-butyl | H | Br |
| 19-279 | 2-chloropyridin-3-yl | Me | Me | N | H | H | H | Br | H | nonafluoro-2-butyl | H | Br |
| 19-321 | phenyl | Me | Me | N | H | H | H | I | H | heptafluoroisopropyl | H | I |
| 19-341 | 3-cyanophenyl | Me | Me | N | H | H | H | I | H | heptafluoroisopropyl | H | I |
| 19-342 | 4-cyanophenyl | Me | Me | N | H | H | H | I | H | heptafluoroisopropyl | H | I |
| 19-357 | 2-chloropyridin-3-yl | Me | Me | N | H | H | H | I | H | heptafluoroisopropyl | H | I |
| 19-399 | phenyl | Me | Me | N | H | H | H | I | H | nonafluoro-2-butyl | H | I |
| 19-419 | 3-cyanophenyl | Me | Me | N | H | H | H | I | H | nonafluoro-2-butyl | H | I |
| 19-420 | 4-cyanophenyl | Me | Me | N | H | H | H | I | H | nonafluoro-2-butyl | H | I |
| 19-435 | 2-chloropyridin-3-yl | Me | Me | N | H | H | H | I | H | nonafluoro-2-butyl | H | I |
| 19-673 | phenyl | Me | Me | N | H | H | H | Cl | H | heptafluoroisopropyl | H | OCF3 |
| 19-693 | 3-cyanophenyl | Me | Me | N | H | H | H | Cl | H | heptafluoroisopropyl | H | OCF3 |
| 19-694 | 4-cyanophenyl | Me | Me | N | H | H | H | Cl | H | heptafluoroisopropyl | H | OCF3 |
| 19-709 | 2-chloropyridin-3-yl | Me | Me | N | H | H | H | Cl | H | heptafluoroisopropyl | H | OCF3 |
| 19-751 | phenyl | Me | Me | N | H | H | H | Cl | H | nonafluoro-2-butyl | H | OCF3 |
| 19-771 | 3-cyanophenyl | Me | Me | N | H | H | H | Cl | H | nonafluoro-2-butyl | H | OCF3 |
| 19-772 | 4-cyanophenyl | Me | Me | N | H | H | H | Cl | H | nonafluoro-2-butyl | H | OCF3 |
| 19-787 | 2-chloropyridin-3-yl | Me | Me | N | H | H | H | Cl | H | nonafluoro-2-butyl | H | OCF3 |
| 19-829 | phenyl | Me | Me | N | H | H | H | Br | H | heptafluoroisopropyl | H | OCF3 |
| 19-849 | 3-cyanophenyl | Me | Me | N | H | H | H | Br | H | heptafluoroisopropyl | H | OCF3 |
| 19-850 | 4-cyanophenyl | Me | Me | N | H | H | H | Br | H | heptafluoroisopropyl | H | OCF3 |
| 19-865 | 2-chloropyridin-3-yl | Me | Me | N | H | H | H | Br | H | heptafluoroisopropyl | H | OCF3 |
| 19-907 | phenyl | Me | Me | N | H | H | H | Br | H | nonafluoro-2-butyl | H | OCF3 |
| 19-927 | 3-cyanophenyl | Me | Me | N | H | H | H | Br | H | nonafluoro-2-butyl | H | OCF3 |
| 19-928 | 4-cyanophenyl | Me | Me | N | H | H | H | Br | H | nonafluoro-2-butyl | H | OCF3 |
| 19-943 | 2-chloropyridin-3-yl | Me | Me | N | H | H | H | Br | H | nonafluoro-2-butyl | H | OCF3 |
| 19-985 | phenyl | Me | Me | N | H | H | H | I | H | heptafluoroisopropyl | H | OCF3 |
| 19-1005 | 3-cyanophenyl | Me | Me | N | H | H | H | I | H | heptafluoroisopropyl | H | OCF3 |
| 19-1006 | 4-cyanophenyl | Me | Me | N | H | H | H | I | H | heptafluoroisopropyl | H | OCF3 |
| 19-1021 | 2-choropyridin3-yl | Me | Me | N | H | H | H | I | H | heptafluoroisopropyl | H | OCF3 |
| 19-1063 | phenyl | Me | Me | N | H | H | H | I | H | nonafluoro-2-butyl | H | OCF3 |

**Table 19(2)**

| compound number | Q₁ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 19-1083 | 3-cyanophenyl | Me | Me | N | H | H | H | I | H | nonafluoro-2-butyl | H | OCF3 |
| 19-1084 | 4-cyanophenyl | Me | Me | N | H | H | H | I | H | nonafluoro-2-butyl | H | OCF3 |
| 19-1099 | 2-chloropyridin-3-yl | Me | Me | N | H | H | H | I | H | nonafluoro-2-butyl | H | OCF3 |
| 19-1337 | phenyl | Me | Me | N | H | H | H | F | H | heptafluoroisopropyl | H | CF3 |
| 19-1338 | phenyl | Me | Me | N | H | H | H | Cl | H | heptafluoroisopropyl | H | CF3 |
| 19-1349 | 3-iodophenyl | Me | Me | N | H | H | H | Cl | H | pentafluoroethyl | H | CF3 |
| 19-1350 | 4-iodophenyl | Me | Me | N | H | H | H | Cl | H | pentafluoroethyl | H | CF3 |
| 19-1352 | (3-trifluoromethyl)phenyl | Et | Me | N | H | H | H | Cl | H | heptafluoroisopropyl | H | CF3 |
| 19-1353 | (4-trifluoromethyl)phenyl | Me | Et | N | H | H | H | Cl | H | heptafluoroisopropyl | H | CF3 |
| 19-1357 | 2-cyanophenyl | Me | Me | N | H | H | H | Cl | H | pentafluoroethyl | H | C2F5 |
| 19-1358 | 3-cyanophenyl | Me | Me | N | H | H | H | Cl | H | heptafluoroisopropyl | H | CF3 |
| 19-1359 | 4-cyanophenyl | Me | Me | N | H | H | H | Cl | H | heptafluoroisopropyl | H | CF3 |
| 19-1362 | 24-dichorophenyl | Me | Me | N | H | H | H | Cl | H | heptafluoroisopropyl | H | C2F5 |
| 19-1363 | 2-chloro-4-fluorophenyl | n-Pr | Me | N | H | H | H | Cl | H | heptafluoroisopropyl | H | CF3 |
| 19-1365 | 4-bromo-2-chlorophenyl | Me | n-Pr | N | H | H | H | Cl | H | heptafluoroisopropyl | H | CF3 |
| 19-1366 | 2-bromo-4-chlorophenyl | Me | Me | N | H | H | H | Cl | H | heptafluoroisopropyl | H | n-C3F7 |
| 19-1373 | 2-fluoropyridin-3-yl | Me | Me | N | H | H | H | Cl | H | pentafluoroethyl | H | CF3 |
| 19-1374 | 2-chloropyridin-3-yl | Me | Me | N | H | H | H | Cl | H | heptafluoroisopropyl | H | CF3 |
| 19-1378 | 2-nitropyridin-3-yl | Me | i-Pr | N | H | H | H | Cl | H | heptafluoroisopropyl | H | CF3 |
| 19-1380 | 6-fluoropyridin-3-yl | i-Pr | Me | N | H | H | H | Cl | H | heptafluoroisopropyl | H | CF3 |
| 19-1387 | 5-fluoropyridin-3-yl | Me | Me | N | H | H | H | Cl | H | heptafluoroisopropyl | H | C2F5 |
| 19-1389 | 5-bromopyridin-3-yl | Me | Me | N | H | H | H | Cl | H | heptafluoroisopropyl | H | n-C3F7 |
| 19-1390 | 5-iodopyridin-3-yl | Me | Me | N | H | H | H | Cl | H | heptafluoroisopropyl | H | i-C3F7 |
| 19-1392 | 5-nitropyridin-3-yl | Me | Me | N | H | H | H | Cl | H | pentafluoroethyl | H | CF3 |
| 19-1393 | 5-cyanopyridin-3-yl | Me | CH2CH=CH2 | N | H | H | H | Cl | H | heptafluoroisopropyl | H | CF3 |
| 19-1394 | 4-fluoropyridin-3-yl | Me | Me | N | H | H | H | Cl | H | pentafluoroethyl | H | CF3 |
| 19-1396 | 4-bromopyridin-3-yl | CH2CH=CH2 | Me | N | H | H | H | Cl | H | heptafluoroisopropyl | H | n-C3F7 |
| 19-1397 | 4-iodopyridin-3-yl | Me | Me | N | H | H | H | Cl | H | heptafluoroisopropyl | H | i-C3F7 |
| 19-1400 | 4-cyanopyridin-3-yl | Me | CN | N | H | H | H | Cl | H | heptafluoroisopropyl | H | CF3 |
| 19-1403 | pyridin-4-yl | CN | Me | N | H | H | H | Cl | H | heptafluoroisopropyl | H | CF3 |
| 19-1415 | 4-cyanopyridin-2-yl | Me | Me | N | H | H | H | Cl | H | pentafluoroethyl | H | CF3 |
| 19-1416 | phenyl | Me | Me | N | H | H | H | F | H | nonafluoro-2-butyl | H | CF3 |
| 19-1417 | phenyl | Me | Me | N | H | H | H | Cl | H | nonafluoro-2-butyl | H | CF3 |
| 19-1425 | 3-bromophenyl | CH2C≡CH | Me | N | H | H | H | Cl | H | nonafluoro-2-butyl | H | CF3 |
| 19-1429 | 4-iodophenyl | Me | CH2C≡CH | N | H | H | H | Cl | H | nonafluoro-2-butyl | H | CF3 |
| 19-1430 | (2-trifluoromethyl)phenyl | Me | Me | N | H | H | H | Cl | H | nonafluoro-2-butyl | H | C2F5 |
| 19-1432 | (4-trifluoromethyl)phenyl | NH2 | Me | N | H | H | H | Cl | H | nonafluoro-2-butyl | H | CF3 |
| 19-1436 | 2-cyanophenyl | Me | NH2 | N | H | H | H | Cl | H | nonafluoro-2-butyl | H | CF3 |
| 19-1437 | 3-cyanophenyl | Me | Me | N | H | H | H | Cl | H | nonafluoro-2-butyl | H | CF3 |
| 19-1438 | 4-cyanophenyl | Me | Me | N | H | H | H | Cl | H | nonafluoro-2-butyl | H | CF3 |
| 19-1442 | 2-chloro-4-fluorophenyl | Me | C(O)OMe | N | H | H | H | Cl | H | nonafluoro-2-butyl | H | CF3 |
| 19-1445 | 2-bromo-4-chlorophenyl | C(O)OMe | Me | N | H | H | H | Cl | H | nonafluoro-2-butyl | H | CF3 |
| 19-1447 | 2-chloro-4-nitrophenyl | Me | Me | N | H | H | H | Cl | H | nonafluoro-2-butyl | H | i-C3F7 |
| 19-1453 | 2-chloropyridin-3-yl | Me | Me | N | H | H | H | Cl | H | nonafluoro-2-butyl | H | CF3 |
| 19-1464 | 6-nitropyridin-3-yl | Me | C(O)OEt | N | H | H | H | Cl | H | nonafluoro-2-butyl | H | CF3 |
| 19-1466 | 5-fluoropyridin-3-yl | C(O)OEt | Me | N | H | H | H | Cl | H | nonafluoro-2-butyl | H | C2F5 |
| 19-1468 | 5-bromopyridin-3-yl | Me | Me | N | H | H | H | Cl | H | nonafluoro-2-butyl | H | n-C3F7 |
| 19-1469 | 5-iodopyridin-3-yl | Me | Me | N | H | H | H | Cl | H | nonafluoro-2-butyl | H | i-C3F7 |
| 19-1472 | 5-cyanopyridin-3-yl | Me | C(O)C(O)Me | N | H | H | H | Cl | H | nonafluoro-2-butyl | H | CF3 |
| 19-1473 | 4-fluoropyridin-3-yl | Me | Me | N | H | H | H | Cl | H | nonafluoro-2-butyl | H | C2F5 |
| 19-1475 | 4-bromopyridin-3-yl | C(O)C(O)Me | Me | N | H | H | H | Cl | H | nonafluoro-2-butyl | H | CF3 |
| 19-1477 | 4-(trifluoromethyl)pyridin-3-yl | Me | Me | N | H | H | H | Cl | H | nonafluoro-2-butyl | H | n-C3F7 |
| 19-1478 | 4-nitropyridin-3-yl | C(O)C(O)Et | Me | N | H | H | H | Cl | H | nonafluoro-2-butyl | H | i-C3F7 |
| 19-1479 | 4-cyanopyridin-3-yl | Me | C(O)C(O)Et | N | H | H | H | Cl | H | nonafluoro-2-butyl | H | CF3 |
| 19-1484 | 3-bromopyridin-4-yl | Me | C(O)ET | N | H | H | H | Cl | H | nonafluoro-2-butyl | H | CF3 |
| 19-1491 | 4-bromopyridin-2-yl | Me | C(O)Me | N | H | H | H | Cl | H | nonafluoro-2-butyl | H | CF3 |
| 19-1492 | 5-iodopyridin-2-yl | C(O)Me | Me | N | H | H | H | Cl | H | nonafluoro-2-butyl | H | CF3 |
| 19-1494 | 4-cyanopyridin-2-yl | C(O)Et | Me | N | H | H | H | Cl | H | nonafluoro-2-butyl | H | C2F5 |
| 19-1495 | phenyl | Me | Me | N | H | H | H | Br | H | heptafluoroisopropyl | H | CF3 |
| 19-1496 | 2-fluorophenyl | Me | Me | N | H | H | H | Br | H | heptafluoroisopropyl | H | CF3 |
| 19-1509 | (3-trifluoromethyl)phenyl | S(O)2Me | Me | N | H | H | H | Br | H | heptafluoroisopropyl | H | CF3 |
| 19-1514 | 2-cyanophenyl | Me | S(O)2Me | N | H | H | H | Br | H | heptafluoroisopropyl | H | CF3 |
| 19-1515 | 3-cyanophenyl | Me | Me | N | H | H | H | Br | H | heptafluoroisopropyl | H | CF3 |
| 19-1516 | 4-cyanophenyl | Me | Me | N | H | H | H | Br | H | heptafluoroisopropyl | H | CF3 |

**Table 19(3)**

| compound number | Q₁ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 19-1523 | 2-bromo-4-chlorophenyl | Me | S(O)2Et | N | H | H | H | Br | H | heptafluoroisopropyl | H | CF3 |
| | 4-cyano-2-fluorophenyl | S(O)2Et | Me | N | H | H | H | Br | H | heptafluoroisopropyl | H | CF3 |
| 19-1527 19-1531 | 2-chloropyridin-3-yl | Me | Me | N | H | H | H | Br | H | heptafluoroisopropyl | H | CF3 |
| 19-1542 | 6-nitropyridin-3-yl | CH2Ph | Me | N | H | H | H | Br | H | heptafluoroisopropyl | H | CF3 |
| 19-1546 | 5-bromopyridin-3-yl | Me | CH2Ph | N | H | H | H | Br | H | heptafluoroisopropyl | H | CF3 |
| 19-1551 | 4-fluoropyridin-3-yl | CH2(3-Py) | Me | N | H | H | H | Br | H | heptafluoroisopropyl | H | CF3 |
| 19-1556 | 4-nitropyridin-3-yl | Me | CH2(3-Py) | N | H | H | H | Br | H | heptafluoroisopropyl | H | CF3 |
| 19-1570 | 5-iodopyridin-2-yl | Me | CH2(3-Py-N-oxide) | N | H | H | H | Br | H | heptafluoroisopropyl | H | CF3 |
| 19-1572 | 4-cyanopyridin-2-yl | CH2(3-Py-N-oxide) | Me | N | H | H | H | Br | H | heptafluoroisopropyl | H | CF3 |
| 19-1573 | phenyl | Me | Me | N | H | H | H | Br | H | nonafluoro-2-butyl | H | CF3 |
| 19-1574 | 2-fluorophenyl | Me | Me | N | H | H | H | Br | H | nonafluoro-2-butyl | H | CF3 |
| 19-1593 | 3-cyanophenyl | Me | Me | N | H | H | H | Br | H | nonafluoro-2-butyl | H | CF3 |
| 19-1594 | 4-cyanophenyl | Me | Me | N | H | H | H | Br | H | nonafluoro-2-butyl | H | CF3 |
| 19-1600 | 4-bromo-2-chlorophenyl | Et | Me | N | H | H | H | Br | H | nonafluoro-2-butyl | H | CF3 |
| 19-1603 | 2-chloro-4-nitrophenyl | Me | Et | N | H | H | H | Br | H | nonafluoro-2-butyl | H | CF3 |
| 19-1609 | 2-choropyridin-3-yl | Me | Me | N | H | H | H | Br | H | nonafluoro-2-butyl | H | CF3 |
| 19-1626 | 5-(trifluoromethyl)pyridin-3-yl | CN | Me | N | H | H | H | Br | H | nonafluoro-2-butyl | H | CF3 |
| 19-1627 | 5-nitropyridin-3-yl | Me | CN | N | H | H | H | Br | H | nonafluoro-2-butyl | H | CF3 |
| 19-1651 | phenyl | Me | Me | N | H | H | H | I | H | heptafluoroisopropyl | H | CF3 |
| 19-1652 | 2-fluorophenyl | Me | Me | N | H | H | H | I | H | heptafluoroisopropyl | H | CF3 |
| 19-1671 | 3-cyanophenyl | Me | Me | N | H | H | H | I | H | heptafluoroisopropyl | H | CF3 |
| 19-1672 | 4-cyanophenyl | Me | Me | N | H | H | H | I | H | heptafluoroisopropyl | H | CF3 |
| 19-1687 | 2-chloropyridin-3-yl | Me | Me | N | H | H | H | I | H | heptafluoroisopropyl | H | CF3 |
| 19-1700 | 5-fluoropyridin-3-yl | C(O)Me | Me | N | H | H | H | I | H | heptafluoroisopropyl | H | CF3 |
| 19-1705 | 5-nitropyridin-3-yl | Me | C(O)ME | N | H | H | H | I | H | heptafluoroisopropyl | H | CF3 |
| 19-1729 | phenyl | Me | Me | N | H | H | H | I | H | nonafluoro-2-butyl | H | CF3 |
| 19-1730 | 2-fluorophenyl | Me | Me | N | H | H | H | I | H | nonafluoro-2-butyl | H | CF3 |
| 19-1749 | 3-cyanophenyl | Me | Me | N | H | H | H | I | H | nonafluoro-2-butyl | H | CF3 |
| 19-1750 | 4-cyanophenyl | Me | Me | N | H | H | H | I | H | nonafluoro-2-butyl | H | CF3 |
| 19-1765 | 2-chloropyridin-3-yl | Me | Me | N | H | H | H | I | H | nonafluoro-2-butyl | H | CF3 |
| 19-2003 | phenyl | Me | Me | N | H | H | H | OCF3 | H | heptafluoroisopropyl | H | OCF3 |
| 19-2034 | 2-chloropyridin-3-yl | Me | Me | N | H | H | H | OCF3 | H | heptafluoroisopropyl | H | OCF3 |
| 19-2059 | phenyl | Me | Me | N | H | H | H | OCF3 | H | nonafluoro-2-butyl | H | OCF3 |
| 19-2090 | 2-chloropyridin-3-yl | Me | Me | N | H | H | H | OCF3 | H | nonafluoro-2-butyl | H | OCF3 |
| 19-2115 | phenyl | Me | Me | N | H | H | H | CF3 | H | heptafluoroisopropyl | H | OCF3 |
| 19-2146 | 2-chloropyridin-3-yl | Me | Me | N | H | H | H | CF3 | H | heptafluoroisopropyl | H | OCF3 |
| 19-2171 | phenyl | Me | Me | N | H | H | H | CF3 | H | nonafluoro-2-butyl | H | OCF3 |
| 19-2202 | 2-chloropyridin-3-yl | Me | Me | N | H | H | H | CF3 | H | nonafluoro-2-butyl | H | OCF3 |
| 19-2227 | phenyl | Me | Me | N | H | H | H | CF3 | H | heptafluoroisopropyl | H | CF3 |
| 19-2247 | 3-cyanophenyl | Me | Me | N | H | H | H | CF3 | H | heptafluoroisopropyl | H | CF3 |
| 19-2248 | 4-cyanophenyl | Me | Me | N | H | H | H | CF3 | H | heptafluoroisopropyl | H | CF3 |
| 19-2263 | 2-chloropyridin-3-yl | Me | Me | N | H | H | H | CF3 | H | heptafluoroisopropyl | H | CF3 |
| 19-2295 | phenyl | Me | Me | N | H | H | H | CF3 | H | nonafluoro-2-butyl | H | CF3 |
| 19-2315 | 3-cyanophenyl | Me | Me | N | H | H | H | CF3 | H | nonafluoro-2-butyl | H | CF3 |
| 19-2316 | 4-cyanophenyl | Me | Me | N | H | H | H | CF3 | H | nonafluoro-2-butyl | H | CF3 |
| 19-2331 | 2-chloropyridin-3-yl | Me | Me | N | H | H | H | CF3 | H | nonafluoro-2-butyl | H | CF3 |
| 19-2559 | phenyl | Me | Me | N-oxide | H | H | H | F | H | heptafluoroisopropyl | H | F |
| 19-2560 | 3-cyanophenyl | Me | Me | N-oxide | H | H | H | F | H | heptafluoroisopropyl | H | F |
| 19-2561 | 4-cyanophenyl | Me | Me | N-oxide | H | H | H | F | H | heptafluoroisopropyl | H | F |
| 19-2562 | 2-chloropyridin-3-yl | Me | Me | N-oxide | H | H | H | F | H | heptafluoroisopropyl | H | F |
| 19-2563 | phenyl | Me | Me | N-oxide | H | H | H | F | H | nonafluoro-2-butyl | H | F |
| 19-2564 | 3-cyanophenyl | Me | Me | N-oxide | H | H | H | F | H | nonafluoro-2-butyl | H | F |
| 19-2565 | 4-cyanophenyl | Me | Me | N-oxide | H | H | H | F | H | nonafluoro-2-butyl | H | F |
| 19-2566 | 2-chloropyridin-3-yl | Me | Me | N-oxide | H | H | H | F | H | nonafluoro-2-butyl | H | F |
| 19-2567 | phenyl | Me | Me | N-oxide | H | H | H | Cl | H | heptafluoroisopropyl | H | Cl |
| 19-2587 | 3-cyanophenyl | Me | Me | N-oxide | H | H | H | Cl | H | heptafluoroisopropyl | H | Cl |
| 19-2588 | 4-cyanophenyl | Me | Me | N-oxide | H | H | H | Cl | H | heptafluoroisopropyl | H | Cl |
| 19-2603 | 2-chloropyridin-3-yl | Me | Me | N-oxide | H | H | H | Cl | H | heptafluoroisopropyl | H | Cl |
| 19-2645 | phenyl | Me | Me | N-oxide | H | H | H | Cl | H | nonafluoro-2-butyl | H | Cl |
| 19-2665 | 3-cyanophenyl | Me | Me | N-oxide | H | H | H | Cl | H | nonafluoro-2-butyl | H | Cl |
| 19-2666 | 4-cyanophenyl | Me | Me | N-oxide | H | H | H | Cl | H | nonafluoro-2-butyl | H | Cl |
| 19-2681 | 2-chloropyridin-3-yl | Me | Me | N-oxide | H | H | H | Cl | H | nonafluoro-2-butyl | H | Cl |
| 19-2723 | phenyl | Me | Me | N-oxide | H | H | H | Br | H | heptafluoroisopropyl | H | Br |
| 19-2743 | 3-cyanophenyl | Me | Me | N-oxide | H | H | H | Br | H | heptafluoroisopropyl | H | Br |

**Table 19(4)**

| compound number | Q₁ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 19-2744 | 4-cyanophenyl | Me | Me | N-oxide | H | H | H | Br | H | heptafluoroisopropyl | H | Br |
| 19-2759 | 2-choropyridin-3-yl | Me | Me | N-oxide | H | H | H | Br | H | heptafluoroisopropyl | H | Br |
| 19-2801 | phenyl | Me | Me | N-oxide | H | H | H | Br | H | nonafluoro-2-butyl | H | Br |
| 19-2821 | 3-cyanophenyl | Me | Me | N-oxide | H | H | H | Br | H | nonafluoro-2-butyl | H | Br |
| 19-2822 | 4-cyanophenyl | Me | Me | N-oxide | H | H | H | Br | H | nonafluoro-2-butyl | H | Br |
| 19-2837 | 2-chloropyridin-3-yl | Me | Me | N-oxide | H | H | H | Br | H | nonafluoro-2-butyl | H | Br |
| 19-2879 | phenyl | Me | Me | N-oxide | H | H | H | I | H | heptafluoroisopropyl | H | I |
| 19-2899 | 3-cyanophenyl | Me | Me | N-oxide | H | H | H | I | H | heptafluoroisopropyl | H | I |
| 19-2900 | 4-cyanophenyl | Me | Me | N-oxide | H | H | H | I | H | heptafluoroisopropyl | H | I |
| 19-2915 | 2-chloropyridin-3-yl | Me | Me | N-oxide | H | H | H | I | H | heptafluoroisopropyl | H | I |
| 19-2957 | phenyl | Me | Me | N-oxide | H | H | H | I | H | nonafluoro-2-butyl | H | I |
| 19-2977 | 3-cyanophenyl | Me | Me | N-oxide | H | H | H | I | H | nonafluoro-2-butyl | H | I |
| 19-2978 | 4-cyanophenyl | Me | Me | N-oxide | H | H | H | I | H | nonafluoro-2-butyl | H | I |
| 19-2993 | 2-chloropyridin-3-yl | Me | Me | N-oxide | H | H | H | I | H | nonafluoro-2-butyl | H | I |
| 19-3231 | phenyl | Me | Me | N-oxide | H | H | H | Cl | H | heptafluoroisopropyl | H | OCF3 |
| 19-3251 | 3-cyanophenyl | Me | Me | N-oxide | H | H | H | Cl | H | heptafluoroisopropyl | H | OCF3 |
| 19-3252 | 4-cyanophenyl | Me | Me | N-oxide | H | H | H | Cl | H | heptafluoroisopropyl | H | OCF3 |
| 19-3267 | 2-chloropyridin-3-yl | Me | Me | N-oxide | H | H | H | Cl | H | heptafluoroisopropyl | H | OCF3 |
| 19-3309 | phenyl | Me | Me | N-oxide | H | H | H | Cl | H | nonafluoro-2-butyl | H | OCF3 |
| 19-3329 | 3-cyanophenyl | Me | Me | N-oxide | H | H | H | Cl | H | nonafluoro-2-butyl | H | OCF3 |
| 19-3330 | 4-cyanophenyl | Me | Me | N-oxide | H | H | H | Cl | H | nonafluoro-2-butyl | H | OCF3 |
| 19-3345 | 2-chloropyridin-3-yl | Me | Me | N-oxide | H | H | H | Cl | H | nonafluoro-2-butyl | H | OCF3 |
| 19-3387 | phenyl | Me | Me | N-oxide | H | H | H | Br | H | heptafluoroisopropyl | H | OCF3 |
| 19-3407 | 3-cyanophenyl | Me | Me | N-oxide | H | H | H | Br | H | heptafluoroisopropyl | H | OCF3 |
| 19-3408 | 4-cyanophenyl | Me | Me | N-oxide | H | H | H | Br | H | heptafluoroisopropyl | H | OCF3 |
| 19-3423 | 2-chloropyridin-3-yl | Me | Me | N-oxide | H | H | H | Br | H | heptafluoroisopropyl | H | OCF3 |
| 19-3465 | phenyl | Me | Me | N-oxide | H | H | H | Br | H | nonafluoro-2-butyl | H | OCF3 |
| 19-3485 | 3-cyanophenyl | Me | Me | N-oxide | H | H | H | Br | H | nonafluoro-2-butyl | H | OCF3 |
| 19-3486 | 4-cyanophenyl | Me | Me | N-oxide | H | H | H | Br | H | nonafluoro-2-butyl | H | OCF3 |
| 19-3501 | 2-chloropyridin-3-yl | Me | Me | N-oxide | H | H | H | Br | H | nonafluoro-2-butyl | H | OCF3 |
| 19-3543 | phenyl | Me | Me | N-oxide | H | H | H | I | H | heptafluoroisopropyl | H | OCF3 |
| 19-3563 | 3-cyanophenyl | Me | Me | N-oxide | H | H | H | I | H | heptafluoroisopropyl | H | OCF3 |
| 19-3564 | 4-cyanophenyl | Me | Me | N-oxide | H | H | H | I | H | heptafluoroisopropyl | H | OCF3 |
| 19-3579 | 2-chloropyridin-3-yl | Me | Me | N-oxide | H | H | H | I | H | heptafluoroisopropyl | H | OCF3 |
| 19-3621 | phenyl | Me | Me | N-oxide | H | H | H | I | H | nonafluoro-2-butyl | H | OCF3 |
| 19-3641 | 3-cyanophenyl | Me | Me | N-oxide | H | H | H | I | H | nonafluoro-2-butyl | H | OCF3 |
| 19-3642 | 4-cyanophenyl | Me | Me | N-oxide | H | H | H | I | H | nonafluoro-2-butyl | H | OCF3 |
| 19-3657 | 2-chloropyridin-3-yl | Me | Me | N-oxide | H | H | H | I | H | nonafluoro-2-butyl | H | OCF3 |
| 19-3895 | phenyl | Me | Me | N-oxide | H | H | H | F | H | heptafluoroisopropyl | H | CF3 |
| 19-3896 | phenyl | Me | Me | N-oxide | H | H | H | Cl | H | heptafluoroisopropyl | H | CF3 |
| 19-3916 | 3-cyanophenyl | Me | Me | N-oxide | H | H | H | Cl | H | heptafluoroisopropyl | H | CF3 |
| 19-3917 | 4-cyanophenyl | Me | Me | N-oxide | H | H | H | Cl | H | heptafluoroisopropyl | H | CF3 |
| 19-3932 | 2-chloropyridin-3-yl | Me | Me | N-oxide | H | H | H | Cl | H | heptafluoroisopropyl | H | CF3 |
| 19-3974 | phenyl | Me | Me | N-oxide | H | H | H | F | H | nonafluoro-2-butyl | H | CF3 |
| 19-3975 | phenyl | Me | Me | N-oxide | H | H | H | Cl | H | nonafluoro-2-butyl | H | CF3 |
| 19-3995 | 3-cyanophenyl | Me | Me | N-oxide | H | H | H | Cl | H | nonafluoro-2-butyl | H | CF3 |
| 19-3996 | 4-cyanophenyl | Me | Me | N-oxide | H | H | H | Cl | H | nonafluoro-2-butyl | H | CF3 |
| 19-4011 | 2-chloropyridin-3-yl | Me | Me | N-oxide | H | H | H | Cl | H | nonafluoro-2-butyl | H | CF3 |
| 19-4053 | phenyl | Me | Me | N-oxide | H | H | H | Br | H | heptafluoroisopropyl | H | CF3 |
| 19-4073 | 3-cyanophenyl | Me | Me | N-oxide | H | H | H | Br | H | heptafluoroisopropyl | H | CF3 |
| 19-4074 | 4-cyanophenyl | Me | Me | N-oxide | H | H | H | Br | H | heptafluoroisopropyl | H | CF3 |
| 19-4089 | 2-chloropyridin-3-yl | Me | Me | N-oxide | H | H | H | Br | H | heptafluoroisopropyl | H | CF3 |
| 19-4131 | phenyl | Me | Me | N-oxide | H | H | H | Br | H | nonafluoro-2-butyl | H | CF3 |
| 19-4151 | 3-cyanophenyl | Me | Me | N-oxide | H | H | H | Br | H | nonafluoro-2-butyl | H | CF3 |
| 19-4152 | 4-cyanophenyl | Me | Me | N-oxide | H | H | H | Br | H | nonafluoro-2-butyl | H | CF3 |
| 19-4167 | 2-chloropyridin-3-yl | Me | Me | N-oxide | H | H | H | Br | H | nonafluoro-2-butyl | H | CF3 |
| 19-4209 | phenyl | Me | Me | N-oxide | H | H | H | I | H | heptafluoroisopropyl | H | CF3 |
| 19-4229 | 3-cyanophenyl | Me | Me | N-oxide | H | H | H | I | H | heptafluoroisopropyl | H | CF3 |
| 19-4230 | 4-cyanophenyl | Me | Me | N-oxide | H | H | H | I | H | heptafluoroisopropyl | H | CF3 |
| 19-4245 | 2-chloropyridin-3-yl | Me | Me | N-oxide | H | H | H | I | H | heptafluoroisopropyl | H | CF3 |
| 19-4287 | phenyl | Me | Me | N-oxide | H | H | H | I | H | nonafluoro-2-butyl | H | CF3 |
| 19-4307 | 3-cyanophenyl | Me | Me | N-oxide | H | H | H | I | H | nonafluoro-2-butyl | H | CF3 |
| 19-4308 | 4-cyanophenyl | Me | Me | N-oxide | H | H | H | I | H | nonafluoro-2-butyl | H | CF3 |
| 19-4323 | 2-chloropyridin-3-yl | Me | Me | N-oxide | H | H | H | I | H | nonafluoro-2-butyl | H | CF3 |

**Table 19(5)**

| compound number | Q₁ | R₁ | R₂ | A | X₂ | X₄ | X₄ | Y₁ | Y₁ | Y₃ | Y₄ | Y₅ |
|---|---|---|---|---|---|---|---|---|---|---|---|---|
| 19-4561 | phenyl | Me | Me | N-oxide | H | H | H | OCF3 | H | heptafluoroisopropyl | H | OCF3 |
| 19-4592 | 2-chloropyridin-3-yl | Me | Me | N-oxide | H | H | H | OCF3 | H | heDtafluoroisoDroDyl | H | OCF3 |
| 19-4617 | phenyl | Me | Me | N-oxide | H | H | H | OCF3 | H | nonafluoro-2-butyl | H | OCF3 |
| 19-4648 | 2-chloropyridin-3-yl | Me | Me | N-oxide | H | H | H | OCF3 | H | nonafluoro-2-butyl | H | OCF3 |
| 19-4673 | phenyl | Me | Me | N-oxide | H | H | H | CF3 | H | heptafluoroisopropyl | H | OCF3 |
| 19-4704 | 2-chloropyridin-3-yl | Me | Me | N-oxide | H | H | H | CF3 | H | heDtafluoroisoDroDyl | H | OCF3 |
| 19-4729 | phenyl | Me | Me | N-oxide | H | H | H | CF3 | H | nonafluoro-2-butyl | H | OCF3 |
| 19-4760 | 2-chloropyridin-3-yl | Me | Me | N-oxide | H | H | H | CF3 | H | nonafluoro-2-butyl | H | OCF3 |
| 19-4785 | phenyl | Me | Me | N-oxide | H | H | H | CF3 | H | heptafluoroisopropyl | H | CF3 |
| 19-4805 | 3-cyanophenyl | Me | Me | N-oxide | H | H | H | CF3 | H | heDtafluoroisoDroDyl | H | CF3 |
| 19-4806 | 4-cyanophenyl | Me | Me | N-oxide | H | H | H | CF3 | H | heDtafluoroisoDroDyl | H | CF3 |
| 19-4821 | 2-chloropyridin-3-yl | Me | Me | N-oxide | H | H | H | CF3 | H | heptafluoroisopropyl | H | CF3 |
| 19-4853 | phenyl | Me | Me | N-oxide | H | H | H | CF3 | H | nonafluoro-2-butyl | H | CF3 |
| 19-4873 | 3-cyanophenyl | Me | Me | N-oxide | H | H | H | CF3 | H | nonafluoro-2-butyl | H | CF3 |
| 19-4874 | 4-cyanophenyl | Me | Me | N-oxide | H | H | H | CF3 | H | nonafluoro-2-butyl | H | CF3 |
| 19-4889 | 2-chloropyridin-3-yl | Me | Me | N-oxide | H | H | H | CF3 | H | nonafluoro-2-butyl | H | CF3 |

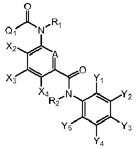

**Table 20(1)**

| compound number | Q₁ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 20-9 | 3-bromophenyl | H | Et | N | H | H | H | Cl | F | heptafluoroisopropyl | F | Cl |
| 20-39 | 2-iodopyridin-3-yl | H | H | N | H | H | H | Cl | Me | nonafluoro-2-butyl | Me | I |
| 20-152 | 3-bromophenyl | H | Et | N | H | H | H | Cl | F | heptafluoroisopropyl | F | OCF3 |
| 20-182 | 2-iodopyridin-3-yl | H | H | N | H | H | H | Cl | Me | nonafluoro-2-butyl | Me | OCF3 |
| 20-287 | phenyl | H | H | N | H | H | H | Cl | F | heptafluoroisopropyl | H | CF3 |
| 20-288 | 2-fluorophenyl | Me | H | N | H | H | H | Cl | H | heptafluoroisopropyl | F | CF3 |
| 20-289 | 3-fluorophenyl | H | Me | N | H | H | H | Cl | Cl | heptafluoroisopropyl | H | CF3 |
| 20-290 | 4-fluorophenyl | Me | Me | N | H | H | H | Cl | H | heptafluoroisopropyl | Cl | CF3 |
| 20-291 | 2-chlorophenyl | H | H | N | H | H | H | F | Br | heptafluoroisopropyl | H | CF3 |
| 20-292 | 3-chlorophenyl | H | H | N | H | H | H | Cl | H | heptafluoroisopropyl | Br | CF3 |
| 20-293 | 4-chlorophenyl | Et | H | N | H | H | H | Cl | I | heptafluoroisopropyl | H | C2F5 |
| 20-294 | 2-bromophenyl | H | H | N | H | H | H | Cl | H | heptafluoroisopropyl | I | CF3 |
| 20-295 | 3-bromophenyl | H | Et | N | H | H | H | Cl | F | heptafluoroisopropyl | F | CF3 |
| 20-296 | 4-bromophenyl | H | H | N | H | H | H | Cl | F | heptafluoroisopropyl | Cl | CF3 |
| 20-297 | 2-iodophenyl | H | H | N | H | H | H | Cl | F | heptafluoroisopropyl | Br | CF3 |
| 20-298 | 3-iodophenyl | Et | Et | N | H | H | H | Cl | F | pentafluoroethyl | I | C2F5 |
| 20-299 | 4-iodophenyl | H | H | N | H | H | H | Cl | Cl | pentafluoroethyl | F | CF3 |
| 20-300 | (2-trifluoromethyl)phenyl | H | H | N | H | H | H | Cl | Cl | heptafluoroisopropyl | Cl | CF3 |
| 20-301 | (3-trifluoromethyl)phenyl | n-Pr | H | N | H | H | H | Cl | Cl | heptafluoroisopropyl | Br | CF3 |
| 20-302 | (4-trifluoromethyl)phenyl | H | H | N | H | H | H | Cl | Cl | heptafluoroisopropyl | I | n-C3F7 |
| 20-303 | 2-nitrophenyl | H | n-Pr | N | H | H | H | Cl | Br | heptafluoroisopropyl | F | CF3 |
| 20-304 | 3-nitrophenyl | H | H | N | H | H | H | Cl | Br | heptafluoroisopropyl | Cl | CF3 |
| 20-305 | 4-nitrophenyl | H | H | N | H | H | H | Cl | Br | heptafluoroisopropyl | Br | CF3 |
| 20-306 | 2-cyanophenyl | n-Pr | n-Pr | N | H | H | H | Cl | Br | pentafluoroethyl | I | CF3 |
| 20-307 | 3-cyanophenyl | H | H | N | H | H | H | F | I | nonafluoro-2-butyl | F | CF3 |
| 20-308 | 4-cyanophenyl | H | H | N | H | H | H | Cl | I | nonafluoro-2-butyl | Cl | CF3 |
| 20-309 | 2,6-difluorophenyl | H | H | N | H | H | H | Cl | I | nonafluoro-2-butyl | Br | CF3 |
| 20-310 | 3,4-dichlorophenyl | H | H | N | H | H | H | Cl | I | nonafluoro-2-butyl | I | CF3 |
| 20-311 | 2,4-dichlorophenyl | H | H | N | H | H | H | Cl | Me | nonafluoro-2-butyl | H | CF3 |
| 20-312 | 2-chloro-4-fluorophenyl | H | H | N | H | H | H | Cl | H | nonafluoro-2-butyl | Me | CF3 |
| 20-313 | 2-chloro-4,5-difluorophenyl | i-Pr | H | N | H | H | H | Cl | Et | nonafluoro-2-butyl | H | CF3 |
| 20-314 | 4-bromo-2-chlorophenyl | H | H | N | H | H | H | Cl | H | nonafluoro-2-butyl | Et | CF3 |
| 20-315 | 2-bromo-4-chlorophenyl | H | H | N | H | H | H | Cl | n-Pr | nonafluoro-2-butyl | H | CF3 |
| 20-316 | 2-bromo-4-fluorophenyl | H | i-Pr | N | H | H | H | Cl | H | nonafluoro-2-butyl | n-Pr | CF3 |
| 20-317 | 2-chloro-4-nitrophenyl | i-Pr | i-Pr | N | H | H | H | Cl | i-Pr | nonafluoro-2-butyl | H | CF3 |
| 20-318 | 3,5-dicyanophenyl | H | H | N | H | H | H | Cl | H | nonafluoro-2-butyl | i-Pr | CF3 |
| 20-319 | 4-cyano-2-fluorophenyl | H | H | N | H | H | H | Cl | n-Bu | nonafluoro-2-butyl | H | CF3 |
| 20-320 | 2-chloro-4-cyanophenyl | H | H | N | H | H | H | Cl | H | nonafluoro-2-butyl | n-Bu | CF3 |
| 20-321 | pyridin-3-yl | H | H | N | H | H | H | Cl | i-Bu | nonafluoro-2-butyl | H | CF3 |
| 20-322 | 2-fluoropyridin-3-yl | H | H | N | H | H | H | Cl | H | nonafluoro-2-butyl | i-Bu | CF3 |
| 20-323 | 2-chloropyridin-3-yl | H | H | N | H | H | H | Cl | s-Bu | nonafluoro-2-butyl | H | C2F5 |
| 20-324 | 2-bromopyridin-3-yl | H | H | N | H | H | H | Cl | H | nonafluoro-2-butyl | s-Bu | CF3 |
| 20-325 | 2-iodopyridin-3-yl | H | H | N | H | H | H | Cl | Me | nonafluoro-2-butyl | Me | n-C3F7 |
| 20-326 | 2-(trifluoromethyl)pyridin-3-yl | H | H | N | H | H | H | Cl | Me | nonafluoro-2-butyl | Et | i-C3F7 |
| 20-327 | 2-nitropyridin-3-yl | C(O)Me | H | N | H | H | H | Cl | Me | nonafluoro-2-butyl | n-Pr | CF3 |
| 20-328 | 2-cyanopyridin-3-yl | H | H | N | H | H | H | Cl | Me | nonafluoro-2-butyl | i-Pr | CF3 |
| 20-329 | 6-fluoropyridin-3-yl | H | H | N | H | H | H | Cl | Me | nonafluoro-2-butyl | n-Bu | CF3 |
| 20-330 | 6-chloropyridin-3-yl | H | H | N | H | H | H | Cl | Me | nonafluoro-2-butyl | i-Bu | CF3 |
| 20-331 | 6-bromopyridin-3-yl | H | C(O)Me | N | H | H | H | Cl | Me | nonafluoro-2-butyl | s-Bu | CF3 |
| 20-332 | 6-iodopyridin-3-yl | H | H | N | H | H | H | Cl | Et | nonafluoro-2-butyl | Me | n-C3F7 |
| 20-333 | 6-(trifluoromethyl)pyridin-3-yl | H | H | N | H | H | H | Cl | Et | nonafluoro-2-butyl | Et | i-C3F7 |
| 20-334 | 6-nitropyridin-3-yl | C(O)Me | C(O)Me | N | H | H | H | Cl | Et | nonafluoro-2-butyl | n-Pr | CF3 |
| 20-335 | 6-cyanopyridin-3-yl | H | H | N | H | H | H | Cl | Et | nonafluoro-2-butyl | i-Pr | CF3 |
| 20-336 | phenyl | H | H | N | H | H | H | Br | Et | heptafluoroisopropyl | n-Bu | CF3 |
| 20-337 | 5-fluoropyridin-3-yl | H | H | N | H | H | H | Br | Et | heptafluoroisopropyl | i-Bu | CF3 |

**Table 20(2)**

| compound number | Q₁ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 20-338 | 5-chloropyridin-3-yl | H | H | N | H | H | H | Br | Et | heptafluoroisopropyl | s-Bu | CF3 |
| 20-339 | 5-bromopyridin-3-yl | H | H | N | H | H | H | Br | n-Pr | heptafluoroisopropyl | Me | CF3 |
| 20-340 | 5-iodopyridin-3-yl | H | H | N | H | H | H | Br | n-Pr | heptafluoroisopropyl | Et | CF3 |
| 20-341 | 5-(trifluoromethyl)pyridin-3-yl | Me | Et | N | H | H | H | Br | n-Pr | pentafluoroethyl | n-Pr | CF3 |
| 20-342 | 5-nitropyridin-3-yl | H | H | N | H | H | H | Br | n-Pr | heptafluoroisopropyl | i-Pr | CF3 |
| 20-343 | 5-cyanopyridin-3-yl | H | H | N | H | H | H | Br | n-Pr | pentafluoroethyl | n-Bu | CF3 |
| 20-344 | 4-fluoropyridin-3-yl | H | H | N | H | H | H | Br | n-Pr | heptafluoroisopropyl | i-Bu | CF3 |
| 20-345 | 4-chloropyridin-3-yl | H | H | N | H | H | H | Br | n-Pr | heptafluoroisopropyl | s-Bu | CF3 |
| 20-346 | 4-bromopyridin-3-yl | Me | n-Pr | N | H | H | H | Br | i-Pr | heptafluoroisopropyl | Me | CF3 |
| 20-347 | 4-iodopyridin-3-yl | H | H | N | H | H | H | Br | i-Pr | heptafluoroisopropyl | Et | CF3 |
| 20-348 | 4-(trifluoromethyl)pyridin-3-yl | H | H | N | H | H | H | Br | i-Pr | heptafluoroisopropyl | n-Pr | CF3 |
| 20-349 | 4-nitropyridin-3-yl | H | H | N | H | H | H | Br | i-Pr | heptafluoroisopropyl | i-Pr | CF3 |
| 20-350 | 4-cyanopyridin-3-yl | H | H | N | H | H | H | Br | i-Pr | heptafluoroisopropyl | n-Bu | CF3 |
| 20-351 | 2.6-dichloropyridin-3-yl | H | H | N | H | H | H | Br | i-Pr | heptafluoroisopropyl | i-Bu | CF3 |
| 20-352 | pyridin-3-yl-N-oxide | Me | i-Pr | N | H | H | H | Br | i-Pr | heptafluoroisopropyl | s-Bu | CF3 |
| 20-353 | pyridin-4-yl | H | H | N | H | H | H | Br | n-Bu | heptafluoroisopropyl | Me | CF3 |
| 20-354 | 2-chloropyridin-4-yl | H | H | N | H | H | H | Br | n-Bu | heptafluoroisopropyl | Et | CF3 |
| 20-355 | 3-bromopyridin-4-yl | H | H | N | H | H | H | Br | n-Bu | heptafluoroisopropyl | n-Pr | CF3 |
| 20-356 | 3,5-dichloropyridin-4-yl | H | H | N | H | H | H | Br | n-Bu | nonafluoro-2-butyl | i-Pr | CF3 |
| 20-357 | 3-(trifluoromethyl)pyridin-4-yl | H | H | N | H | H | H | Br | n-Bu | nonafluoro-2-butyl | n-Bu | CF3 |
| 20-358 | 2,6-dicyanopyridin-4-yl | H | H | N | H | H | H | Br | n-Bu | nonafluoro-2-butyl | i-Bu | CF3 |
| 20-359 | pyridin-4-yl N-oxide | Me | C(O)Me | N | H | H | H | Br | n-Bu | nonafluoro-2-butyl | s-Bu | CF3 |
| 20-360 | pyridin-2-yl | H | H | N | H | H | H | Br | i-Bu | nonafluoro-2-butyl | Me | CF3 |
| 20-361 | 3-chloropyridin-2-yl | H | H | N | H | H | H | Br | i-Bu | nonafluoro-2-butyl | Et | CF3 |
| 20-362 | 4-bromopyridin-2-yl | H | H | N | H | H | H | Br | i-Bu | nonafluoro-2-butyl | n-Pr | CF3 |
| 20-363 | 5-iodopyridin-2-yl | H | H | N | H | H | H | Br | i-Bu | nonafluoro-2-butyl | i-Pr | CF3 |
| 20-364 | 6-chloropyridin-2-yl | H | H | N | H | H | H | Br | i-Bu | nonafluoro-2-butyl | n-Bu | CF3 |
| 20-365 | 4-cyanopyridin-2-yl | H | H | N | H | H | H | Br | i-Bu | nonafluoro-2-butyl | i-Bu | CF3 |
| 20-366 | phenyl | H | H | N | H | H | H | Br | i-Bu | nonafluoro-2-butyl | s-Bu | C2F5 |
| 20-367 | 2-fluorophenyl | H | H | N | H | H | H | Br | s-Bu | nonafluoro-2-butyl | Me | CF3 |
| 20-368 | 3-fluorophenyl | H | H | N | H | H | H | Br | s-Bu | nonafluoro-2-butyl | Et | CF3 |
| 20-369 | 4-fluorophenyl | H | H | N | H | H | H | Br | s-Bu | nonafluoro-2-butyl | n-Pr | CF3 |
| 20-370 | 2-chlorophenyl | H | H | N | H | H | H | Br | s-Bu | nonafluoro-2-butyl | i-Pr | CF3 |
| 20-371 | 3-chlorophenyl | H | H | N | H | H | H | Br | s-Bu | nonafluoro-2-butyl | n-Bu | CF3 |
| 20-372 | 4-chlorophenyl | H | H | N | H | H | H | Br | s-Bu | nonafluoro-2-butyl | i-Bu | CF3 |
| 20-373 | 2-bromophenyl | Et | Me | N | H | H | H | Br | s-Bu | nonafluoro-2-butyl | s-Bu | CF3 |
| 20-374 | 3-bromophenyl | H | H | N | H | H | H | Br | F | nonafluoro-2-butyl | Me | CF3 |
| 20-375 | 4-bromophenyl | H | H | N | H | H | H | Br | F | nonafluoro-2-butyl | Et | CF3 |
| 20-376 | 2-iodophenyl | H | H | N | H | H | H | Br | F | nonafluoro-2-butyl | n-Pr | CF3 |
| 20-377 | 3-iodophenyl | H | H | N | H | H | H | Br | F | nonafluoro-2-butyl | i-Pr | CF3 |
| 20-378 | 4-iodophenyl | H | H | N | H | H | H | Br | F | nonafluoro-2-butyl | n-Bu | CF3 |
| 20-379 | (2-trifluoromethyl)phenyl | H | H | N | H | H | H | Br | F | nonafluoro-2-butyl | i-Bu | CF3 |
| 20-380 | (3-trifluoromethyl)phenyl | Et | n-Pr | N | H | H | H | Br | F | nonafluoro-2-butyl | s-Bu | CF3 |
| 20-381 | (4-trifluoromethyl)phenyl | Et | i-Pr | N | H | H | H | Br | Cl | nonafluoro-2-butyl | Me | CF3 |
| 20-382 | 2-nitrophenyl | H | H | N | H | H | H | Br | Cl | nonafluoro-2-butyl | Et | CF3 |
| 20-383 | phenyl | H | H | N | H | H | H | I | Cl | pentafluoroethyl | n-Pr | i-C3F7 |
| 20-384 | 3-nitrophenyl | H | H | N | H | H | H | I | Cl | heptafluoroisopropyl | i-Pr | CF3 |
| 20-385 | 4-nitrophenyl | H | H | N | H | H | H | I | Cl | heptafluoroisopropyl | n-Bu | CF3 |
| 20-386 | 2-cyanophenyl | Et | C(O)Me | N | H | H | H | I | Cl | heptafluoroisopropyl | i-Bu | CF3 |
| 20-387 | 3-cyanophenyl | H | H | N | H | H | H | I | Cl | heptafluoroisopropyl | s-Bu | CF3 |
| 20-388 | 4-cyanophenyl | H | H | N | H | H | H | I | Br | heptafluoroisopropyl | Me | CF3 |
| 20-389 | 2,6-difluorophenyl | H | H | N | H | H | H | I | Br | heptafluoroisopropyl | Et | CF3 |
| 20-390 | 3,4-dichlorophenyl | H | H | N | H | H | H | I | Br | pentafluoroethyl | n-Pr | CF3 |
| 20-391 | 2,4-dichlorophenyl | H | H | N | H | H | H | I | Br | heptafluoroisopropyl | i-Pr | CF3 |
| 20-392 | 2-chloro-4-fluorophenyl | H | H | N | H | H | H | I | Br | heptafluoroisopropyl | n-Bu | CF3 |
| 20-393 | 2-chloro-4,5-difluorophenyl | H | H | N | H | H | H | I | Br | heptafluoroisopropyl | i-Bu | CF3 |
| 20-394 | 4-bromo-2-chlorophenyl | H | H | N | H | H | H | I | Br | heptafluoroisopropyl | s-Bu | CF3 |
| 20-395 | 2-bromo-4-chlorophenyl | n-Pr | Me | N | H | H | H | I | I | heptafluoroisopropyl | Me | CF3 |
| 20-396 | 2-bromo-4-fluorophenyl | H | H | N | H | H | H | I | I | heptafluoroisopropyl | Et | CF3 |
| 20-397 | 2-chloro-4-nitrophenyl | n-Pr | Et | N | H | H | H | I | I | heptafluoroisopropyl | n-Pr | CF3 |
| 20-398 | 3,5-dicyanophenyl | H | H | N | H | H | H | I | I | heptafluoroisopropyl | i-Pr | CF3 |
| 20-399 | 4-cyano-2-fluorophenyl | H | H | N | H | H | H | I | I | heptafluoroisopropyl | n-Bu | CF3 |
| 20-400 | 2-chloro-4-cyanophenyl | H | H | N | H | H | H | I | I | heptafluoroisopropyl | i-Bu | CF3 |
| 20-401 | pyridin-3-yl | H | H | N | H | H | H | I | I | heptafluoroisopropyl | s-Bu | CF3 |

**Table 20(3)**

| compound number | Q₁ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 20-402 | 2-fluoropyridin-3-yl | H | H | N | H | H | H | I | Me | heptafluoroisopropyl | F | C2F5 |
| 20-403 | 2-chloropyridin-3-yl | H | H | N | H | H | H | I | Me | nonafluoro-2-butyl | Cl | CF3 |
| 20-404 | 2-bromopyridin-3-yl | H | H | N | H | H | H | I | Me | nonafluoro-2-butyl | Br | n-C3F7 |
| 20-405 | 2-iodopyridin-3-yl | H | H | N | H | H | H | I | Me | nonafluoro-2-butyl | I | i-C3F7 |
| 20-406 | 2- (trifluoromethyl) pyridin-3-yl | H | H | N | H | H | H | I | Et | nonafluoro-2-butyl | F | CF3 |
| 20-407 | 2-nitropyridin-3-yl | H | H | N | H | H | H | I | Et | nonafluoro-2-butyl | Cl | CF3 |
| 20-408 | 2-cyanopyridin-3-yl | H | H | N | H | H | H | I | Et | nonafluoro-2-butyl | Br | CF3 |
| 20-409 | 6-fluoropyridin-3-yl | n-Pr | i-Pr | N | H | H | H | I | Et | nonafluoro-2-butyl | I | C2F5 |
| 20-410 | 6-chloropyridin-3-yl | H | H | N | H | H | H | I | n-Pr | nonafluoro-2-butyl | F | CF3 |
| 20-411 | 6-bromopyridin-3-yl | H | H | N | H | H | H | I | n-Pr | nonafluoro-2-butyl | Cl | CF3 |
| 20-412 | 6-iodopyridin-3-yl | H | H | N | H | H | H | I | n-Pr | nonafluoro-2-butyl | Br | CF3 |
| 20-413 | 6- (trifluoromethyl) pyridin-3-yl | H | H | N | H | H | H | I | n-Pr | nonafluoro-2-butyl | I | n-C3F7 |
| 20-414 | 6-nitropyridin-3-yl | H | H | N | H | H | H | I | i-Pr | nonafluoro-2-butyl | F | i-C3F7 |
| 20-415 | 6-cyanopyridin-3-yl | H | H | N | H | H | H | I | i-Pr | nonafluoro-2-butyl | Cl | CF3 |
| 20-416 | 5-fluoropyridin-3-yl | n-Pr | CCO)Me | N | H | H | H | I | i-Pr | nonafluoro-2-butyl | Br | CF3 |
| 20-417 | 5-chloropyridin-3-yl | H | H | N | H | H | H | I | i-Pr | nonafluoro-2-butyl | I | CF3 |
| 20-418 | 5-bromopyridin-3-yl | H | H | N | H | H | H | I | n-Bu | nonafluoro-2-butyl | F | CF3 |
| 20-419 | 5-iodopyridin-3-yl | H | H | N | H | H | H | I | n-Bu | nonafluoro-2-butyl | Cl | CF3 |
| 20-420 | 5- (trifluoromethyl) pyridin-3-yl | H | H | N | H | H | H | I | n-Bu | nonafluoro-2-butyl | Br | CF3 |
| 20-421 | 5-nitropyridin-3-yl | i-Pr | Me | N | H | H | H | I | n-Bu | nonafluoro-2-butyl | I | CF3 |
| 20-422 | 5-cyanopyridin-3-yl | H | H | N | H | H | H | I | i-Bu | nonafluoro-2-butyl | F | CF3 |
| 20-423 | 4-fluoropyridin-3-yl | H | H | N | H | H | H | I | i-Bu | nonafluoro-2-butyl | Cl | CF3 |
| 20-424 | 4-chloropyridin-3-yl | H | H | N | H | H | H | I | i-Bu | nonafluoro-2-butyl | Br | CF3 |
| 20-425 | 4-bromopyridin-3-yl | i-Pr | Et | N | H | H | H | I | i-Bu | nonafluoro-2-butyl | I | CF3 |
| 20-426 | 4-iodopyridin-3-yl | i-Pr | n-Pr | N | H | H | H | I | s-Bu | nonafluoro-2-butyl | F | CF3 |
| 20-427 | 4- (trifluoromethyl) pyridin-3-yl | H | H | N | H | H | H | I | s-Bu | nonafluoro-2-butyl | Cl | CF3 |
| 20-428 | 4-nitropyridin-3-yl | i-Pr | C(O)Me | N | H | H | H | I | s-Bu | nonafluoro-2-butyl | Br | CF3 |
| 20-429 | 4-cyanopyridin-3-yl | H | H | N | H | H | H | I | s-Bu | nonafluoro-2-butyl | I | CF3 |
| 20-438 | 3-bromophenyl | H | Et | N | H | H | H | OCF3 | F | heptafluoroisopropyl | F | OCF3 |
| 20-468 | 2-iodopyridin-3-yl | H | H | N | H | H | H | OCF3 | Me | nonafluoro-2-butyl | Me | OCF3 |
| 20-581 | 3-bromophenyl | H | Et | N | H | H | H | OCF3 | F | heptafluoroisopropyl | F | CF3 |
| 20-611 | 2-iodopyridin-3-yl | H | H | N | H | H | H | OCF3 | Me | nonafluoro-2-butyl | Me | CF3 |
| 20-724 | 3-bromophenyl | H | Et | N | H | H | H | CF3 | F | heptafluoroisopropyl | F | CF3 |
| 20-754 | 2-iodopyridin-3-yl | H | H | N | H | H | H | CF3 | Me | nonafluoro-2-butyl | Me | CF3 |
| 20-867 | 3-bromophenyl | H | Et | N-oxide | H | H | H | Cl | F | heptafluoroisopropyl | F | Cl |
| 20-897 | 2-iodopyridin-3-yl | H | H | N-oxide | H | H | H | Cl | Me | nonafluoro-2-butyl | Me | I |
| 20-1010 | 3-bromophenyl | H | Et | N-oxide | H | H | H | Cl | F | heptafluoroisopropyl | F | OCF3 |
| 20-1040 | 2-iodopyridin-3-yl | H | H | N-oxide | H | H | H | Cl | Me | nonafluoro-2-butyl | Me | OCF3 |
| 20-1154 | 4-bromophenyl | H | H | N-oxide | H | H | H | Cl | F | heptafluoroisopropyl | Cl | CF3 |
| 20-1169 | 2,4-dichlorophenyl | H | H | N-oxide | H | H | H | Cl | Me | nonafluoro-2-butyl | H | CF3 |
| 20-1233 | 4-bromophenyl | H | H | N-oxide | H | H | H | Br | F | nonafluoro-2-butyl | Et | CF3 |
| 20-1261 | 2-chloropyridin-3-yl | H | H | N-oxide | H | H | H | I | Me | nonafluoro-2-butyl | Cl | CF3 |
| 20-1296 | 3-bromophenyl | H | Et | N-oxide | H | H | H | OCF3 | F | heptafluoroisopropyl | F | OCF3 |
| 20-1326 | 2-iodopyridin-3-yl | H | H | N-oxide | H | H | H | OCF3 | Me | nonafluoro-2-butyl | Me | OCF3 |
| 20-1439 | 3-bromopheyl | H | Et | N-oxide | H | H | H | OCF3 | F | heptafluoroisopropyl | F | CF3 |
| 20-1469 | 2-iodopyridin-3-yl | H | H | N-oxide | H | H | H | OCF3 | Me | nonafluoro-2-butyl | Me | CF3 |
| 20-1582 | 3-bromophenyl | H | Et | N-oxide | H | H | H | CF3 | F | heptafluoroisopropyl | F | CF3 |
| 20-1612 | 2-iodopyridin-3-yl | H | H | N-oxide | H | H | H | CF3 | Me | nonafluoro-2-butyl | Me | CF3 |

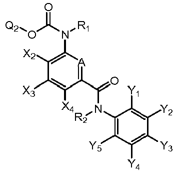

**Table 21 (1)**

| compound number | Q₂ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 21-1 | 2,2,2-trichloroethyl | H | H | N | H | H | H | F | H | heptafluoroisopropyl | H | F |
| 21-2 | 2,2,2-trichloroethyl | H | H | N | H | H | H | F | H | nonafluoro-2-butyl | H | F |
| 21-13 | 2,2,2-trichloroethyl | H | H | N | H | H | H | Cl | H | heptafluoroisopropyl | H | Cl |
| 21-14 | 2,2,2-tribromoethyl | H | H | N | H | H | H | Cl | H | pentafluoroethyl | H | Cl |
| 21-22 | 1,3-dichloro-2-propyl | H | H | N | H | H | H | Cl | H | pentafluoroethyl | H | Cl |
| 21-26 | 4,4,4-trifluoro-n-butyl | H | H | N | H | H | H | Cl | H | heptafluoroisopropyl | H | Cl |
| 21-42 | 2,2,2-trichloroethyl | H | H | N | H | H | H | Cl | H | nonafluoro-2-butyl | H | Cl |
| 21-55 | 4,4,4-trifluoro-n-butyl | H | H | N | H | H | H | Cl | H | nonafluoro-2-butyl | H | Cl |
| 21-71 | 2,2,2-trichloroethyl | H | H | N | H | H | H | Br | H | heptafluoroisopropyl | H | Br |
| 21-74 | 3-fluoro-n-propyl | H | H | N | H | H | H | Br | H | pentafluoroethyl | H | Br |
| 21-80 | 1,3-dichloro-2-propyl | H | H | N | H | H | H | Br | H | pentafluoroethyl | H | Br |
| 21-84 | 4,4,4-trifluoro-n-butyl | H | H | N | H | H | H | Br | H | heptafluoroisopropyl | H | Br |
| 21-100 | 2,2,2-trichloroethyl | H | H | N | H | H | H | Br | H | nonafluoro-2-butyl | H | Br |
| 21-113 | 4,4,4-trifluoro-n-butyl | H | H | N | H | H | H | Br | H | nonafluoro-2-butyl | H | Br |
| 21-129 | 2,2,2-trichloroethyl | H | H | N | H | H | H | I | H | heptafluoroisopropyl | H | I |
| 21-131 | n-propyl | H | H | N | H | H | H | I | H | pentafluoroethyl | H | I |
| 21-138 | 1,3-dichloro-2-propyl | H | H | N | H | H | H | I | H | pentafluoroethyl | H | I |
| 21-142 | 4,4,4-trifluoro-n-butyl | H | H | N | H | H | H | I | H | heptafluoroisopropyl | H | I |
| 21-158 | 2,2,2-trichloroethyl | H | H | N | H | H | H | I | H | nonafluoro-2-butyl | H | I |
| 21-171 | 4,4,4-trifluoro-n-butyl | H | H | N | H | H | H | I | H | nonafluoro-2-butyl | H | I |
| 21-177 | 2,2,2-trichloroethyl | H | H | N | H | H | H | F | H | heptafluoroisopropyl | H | Cl |
| 21-178 | 2,2,2-trichloroethyl | H | H | N | H | H | H | F | H | heptafluoroisopropyl | H | Br |
| 21-179 | 2,2,2-trichloroethyl | H | H | N | H | H | H | F | H | heptafluoroisopropyl | H | I |
| 21-180 | 2,2,2-trichloroethyl | H | H | N | H | H | H | Cl | H | heptafluoroisopropyl | H | Br |
| 21-181 | 2,2,2-trichloroethyl | H | H | N | H | H | H | Cl | H | heptafluoroisopropyl | H | I |
| 21-182 | 2,2,2-trichloroethyl | H | H | N | H | H | H | Br | H | heptafluoroisopropyl | H | I |
| 21-183 | 2,2,2-trichloroethyl | H | H | N | H | H | H | F | H | nonafluoro-2-butyl | H | Cl |
| 21-184 | 2,2,2-trichloroethyl | H | H | N | H | H | H | F | H | nonafluoro-2-butyl | H | Br |
| 21-185 | 2,2,2-trichloroethyl | H | H | N | H | H | H | F | H | nonafluoro-2-butyl | H | I |
| 21-186 | 2,2,2-trichloroethyl | H | H | N | H | H | H | Cl | H | nonafluoro-2-butyl | H | Br |
| 21-187 | 2,2,2-trichloroethyl | H | H | N | H | H | H | Cl | H | nonafluoro-2-butyl | H | I |
| 21-188 | 2,2,2-trichloroethyl | H | H | N | H | H | H | Br | H | nonafluoro-2-butyl | H | I |
| 21-189 | 2,2,2-trichloroethyl | H | H | N | H | H | H | F | H | heptafluoroisopropyl | H | OCF3 |
| 21-190 | 2,2,2-trichloroethyl | H | H | N | H | H | H | F | H | nonafluoro-2-butyl | H | OCF3 |
| 21-201 | 2,2,2-trichloroethyl | H | H | N | H | H | H | Cl | H | heptafluoroisopropyl | H | OCF3 |
| 21-202 | 2,2,2-tribromoethyl | H | H | N | H | H | H | Cl | H | pentafluoroethyl | H | OCF3 |
| 21-206 | 3-bromo-n-propyl | H | H | N | H | H | H | Cl | H | heptafluoroisopropyl | H | OC2F5 |
| 21-210 | 1,3-dichloro-2-propyl | H | H | N | H | H | H | Cl | H | pentafluoroethyl | H | OC2F5 |
| 21-214 | 4,4,4-trifluoro-n-butyl | H | H | N | H | H | H | Cl | H | heptafluoroisopropyl | H | OCF3 |
| 21-226 | 2-cyanoethyl | H | H | N | H | H | H | Cl | H | nonafluoro-2-butyl | H | OC2F5 |
| 21-230 | 2,2,2-trichloroethyl | H | H | N | H | H | H | Cl | H | nonafluoro-2-butyl | H | OCF3 |
| 21-243 | 4,4,4-trifluoro-n-butyl | H | H | N | H | H | H | Cl | H | nonafluoro-2-butyl | H | OCF3 |
| 21-245 | s-butyl | H | H | N | H | H | H | Cl | H | nonafluoro-2-butyl | H | OC2F5 |
| 21-254 | 2-iodoethyl | H | H | N | H | H | H | Br | H | heptafluoroisopropyl | H | OC2F5 |
| 21-259 | 2,2,2-trichloroethyl | H | H | N | H | H | H | Br | H | heptafluoroisopropyl | H | OCF3 |
| 21-260 | 2,2,2-tribromoethyl | H | H | N | H | H | H | Br | H | heptafluoroisopropyl | H | OCF3 |
| 21-272 | 4,4,4-trifluoro-n-butyl | H | H | N | H | H | H | Br | H | heptafluoroisopropyl | H | OCF3 |
| 21-288 | 2,2,2-trichloroethyl | H | H | N | H | H | H | Br | H | nonafluoro-2-butyl | H | OCF3 |
| 21-289 | 2,2,2-tribromoethyl | H | H | N | H | H | H | Br | H | nonafluoro-2-butyl | H | OC2F5 |
| 21-301 | 4,4,4-trifluoro-n-butyl | H | H | N | H | H | H | Br | H | nonafluoro-2-butyl | H | OCF3 |
| 21-317 | 2,2,2-trichloroethyl | H | H | N | H | H | H | I | H | heptafluoroisopropyl | H | OCF3 |
| 21-321 | 3-chloro-n-propyl | H | H | N | H | H | H | I | H | heptafluoroisopropyl | H | OC2F5 |
| 21-330 | 4,4,4-trifluoro-n-butyl | H | H | N | H | H | H | I | H | heptafluoroisopropyl | H | OCF3 |
| 21-346 | 2,2,2-trichloroethyl | H | H | N | H | H | H | I | H | nonafluoro-2-butyl | H | OCF3 |
| 21-359 | 4,4,4-trifluoro-n-butyl | H | H | N | H | H | H | I | H | nonafluoro-2-butyl | H | OCF3 |

**Table 21 (2)**

| compound number | Q₂ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 21-363 | benzyl | H | H | N | H | H | H | I | H | nonafluoro-2-butyl | H | OC2F5 |
| 21-365 | 2,2,2-trichloroethyl | H | H | N | H | H | H | F | H | heptafluoroisopropyl | H | CF3 |
| 21-366 | 2,2,2-trichloroethyl | H | H | N | H | H | H | F | H | nonafluoro-2-butyl | H | CF3 |
| 21-373 | 2-cyanoethyl | H | H | N | H | H | H | Cl | H | heptafluoroisopropyl | H | C2F5 |
| 21-377 | 2,2,2-trichloroethyl | H | H | N | H | H | H | Cl | H | heptafluoroisopropyl | H | CF3 |
| 21-378 | 2,2,2-tribromoethyl | H | H | N | H | H | H | Cl | H | pentafluoroethyl | H | CF3 |
| 21-383 | i-propyl | H | H | N | H | H | H | Cl | H | heptafluoroisopropyl | H | n-C3F7 |
| 21-390 | 4,4,4-trifluoro-n-butyl | H | H | N | H | H | H | Cl | H | heptafluoroisopropyl | H | CF3 |
| 21-397 | ethyl | H | H | N | H | H | H | Cl | H | nonafluoro-2-butyl | H | i-C3F7 |
| 21-406 | 2,2,2-trichloroethyl | H | H | N | H | H | H | Cl | H | nonafluoro-2-butyl | H | CF3 |
| 21-419 | 4,4,4-trifluoro-n-butyl | H | H | N | H | H | H | Cl | H | nonafluoro-2-butyl | H | CF3 |
| 21-425 | methyl | H | H | N | H | H | H | Br | H | heptafluoroisopropyl | H | CF3 |
| 21-426 | ethyl | H | H | N | H | H | H | Br | H | heptafluoroisopropyl | H | CF3 |
| 21-427 | 2-fluoroethyl | H | H | N | H | H | H | Br | H | heptafluoroisopropyl | H | CF3 |
| 21-428 | 2-chloroethyl | H | H | N | H | H | H | Br | H | heptafluoroisopropyl | H | CF3 |
| 21-429 | 2-bromoethyl | H | H | N | H | H | H | Br | H | heptafluoroisopropyl | H | CF3 |
| 21-430 | 2-iodoethyl | H | H | N | H | H | H | Br | H | heptafluoroisopropyl | H | CF3 |
| 21-431 | 2-cyanoethyl | H | H | N | H | H | H | Br | H | heptafluoroisopropyl | H | C2F5 |
| 21-432 | 2,2-difluoroethyl | H | H | N | H | H | H | Br | H | heptafluoroisopropyl | H | CF3 |
| 21-433 | 2,2-dichloroethyl | H | H | N | H | H | H | Br | H | heptafluoroisopropyl | H | CF3 |
| 21-434 | 2,2,2-trifluoroethyl | H | H | N | H | H | H | Br | H | heptafluoroisopropyl | H | CF3 |
| 21-435 | 2,2,2-trichloroethyl | H | H | N | H | H | H | Br | H | heptafluoroisopropyl | H | CF3 |
| 21-436 | 2,2,2-tribromoethyl | H | H | N | H | H | H | Br | H | heptafluoroisopropyl | H | CF3 |
| 21-437 | n-propyl | H | H | N | H | H | H | Br | H | heptafluoroisopropyl | H | CF3 |
| 21-438 | 3-fluoro-n-propyl | H | H | N | H | H | H | Br | H | heptafluoroisopropyl | H | CF3 |
| 21-439 | 3-chloro-n-propyl | H | H | N | H | H | H | Br | H | heptafluoroisopropyl | H | CF3 |
| 21-440 | 3-bromo-n-propyl | H | H | N | H | H | H | Br | H | heptafluoroisopropyl | H | CF3 |
| 21-441 | i-propyl | H | H | N | H | H | H | Br | H | heptafluoroisopropyl | H | CF3 |
| 21-442 | 1,2-difluoro-2-propyl | H | H | N | H | H | H | Br | H | heptafluoroisopropyl | H | n-C3F7 |
| 21-443 | 1,3-difluoro-2-propyl | H | H | N | H | H | H | Br | H | heptafluoroisopropyl | H | CF3 |
| 21-444 | 1,3-dichloro-2-propyl | H | H | N | H | H | H | Br | H | heptafluoroisopropyl | H | CF3 |
| 21-445 | 1-chloro-3-fluoro-2-propyl | H | H | N | H | H | H | Br | H | heptafluoroisopropyl | H | CF3 |
| 21-446 | 3,3,3-trifluoro-n-propyl | H | H | N | H | H | H | Br | H | heptafluoroisopropyl | H | CF3 |
| 21-447 | n-butyl | H | H | N | H | H | H | Br | H | heptafluoroisopropyl | H | CF3 |
| 21-448 | 4,4,4-trifluoro-n-butyl | H | H | N | H | H | H | Br | H | heptafluoroisopropyl | H | CF3 |
| 21-449 | i-butyl | H | H | N | H | H | H | Br | H | heptafluoroisopropyl | H | CF3 |
| 21-450 | s-butyl | H | H | N | H | H | H | Br | H | heptafluoroisopropyl | H | CF3 |
| 21-451 | vinyl | H | H | N | H | H | H | Br | H | heptafluoroisopropyl | H | CF3 |
| 21-452 | benzyl | H | H | N | H | H | H | Br | H | heptafluoroisopropyl | H | CF3 |
| 21-453 | phenyl | H | H | N | H | H | H | Br | H | heptafluoroisopropyl | H | CF3 |
| 21-454 | methyl | H | H | N | H | H | H | Br | H | nonafluoro-2-butyl | H | CF3 |
| 21-455 | ethyl | H | H | N | H | H | H | Br | H | nonafluoro-2-butyl | H | CF3 |
| 21-456 | 2-fluoroethyl | H | H | N | H | H | H | Br | H | nonafluoro-2-butyl | H | CF3 |
| 21-457 | 2-chloroethyl | H | H | N | H | H | H | Br | H | nonafluoro-2-butyl | H | CF3 |
| 21-458 | 2-bromoethyl | H | H | N | H | H | H | Br | H | nonafluoro-2-butyl | H | CF3 |
| 21-459 | 2-iodoethyl | H | H | N | H | H | H | Br | H | nonafluoro-2-butyl | H | CF3 |
| 21-460 | 2-cyanoethyl | H | H | N | H | H | H | Br | H | nonafluoro-2-butyl | H | CF3 |
| 21-461 | 2,2-difluoroethyl | H | H | N | H | H | H | Br | H | nonafluoro-2-butyl | H | CF3 |
| 21-462 | 2,2-dichloroethyl | H | H | N | H | H | H | Br | H | nonafluoro-2-butyl | H | CF3 |
| 21-463 | 2,2,2-trifluoroethyl | H | H | N | H | H | H | Br | H | nonafluoro-2-butyl | H | CF3 |
| 21-464 | 2,2,2-trichloroethyl | H | H | N | H | H | H | Br | H | nonafluoro-2-butyl | H | CF3 |
| 21-465 | 2,2,2-tribromoethyl | H | H | N | H | H | H | Br | H | nonafluoro-2-butyl | H | CF3 |
| 21-466 | n-propyl | H | H | N | H | H | H | Br | H | nonafluoro-2-butyl | H | CF3 |
| 21-467 | 3-fluoro-n-propyl | H | H | N | H | H | H | Br | H | nonafluoro-2-butyl | H | CF3 |
| 21-468 | 3-chloro-n-propyl | H | H | N | H | H | H | Br | H | nonafluoro-2-butyl | H | CF3 |
| 21-469 | 3-bromo-n-propyl | H | H | N | H | H | H | Br | H | nonafluoro-2-butyl | H | CF3 |
| 21-470 | i-propyl | H | H | N | H | H | H | Br | H | nonafluoro-2-butyl | H | CF3 |
| 21-471 | 1,2-difluoro-2-propyl | H | H | N | H | H | H | Br | H | nonafluoro-2-butyl | H | CF3 |
| 21-472 | 1,3-difluoro-2-propyl | H | H | N | H | H | H | Br | H | nonafluoro-2-butyl | H | CF3 |
| 21-473 | 1,3-dichloro-2-propyl | H | H | N | H | H | H | Br | H | nonafluoro-2-butyl | H | CF3 |
| 21-474 | 1-chloro-3-fluoro-2-propyl | H | H | N | H | H | H | Br | H | nonafluoro-2-butyl | H | CF3 |
| 21-475 | 3,3,3-trifluoro-n-propyl | H | H | N | H | H | H | Br | H | nonafluoro-2-butyl | H | CF3 |
| 21-476 | n-butyl | H | H | N | H | H | H | Br | H | nonafluoro-2-butyl | H | CF3 |
| 21-477 | 4,4,4-trifluoro-n-butyl | H | H | N | H | H | H | Br | H | nonafluoro-2-butyl | H | CF3 |

**Table 21 (3)**

| compound number | Q₂ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 21-478 | i-butyl | H | H | N | H | H | H | Br | H | nonafluoro-2-butyl | H | CF3 |
| 21-479 | s-butyl | H | H | N | H | H | H | Br | H | nonafluoro-2-butyl | H | CF3 |
| 21-480 | vinyl | H | H | N | H | H | H | Br | H | nonafluoro-2-butyl | H | CF3 |
| 21-481 | benzyl | H | H | N | H | H | H | Br | H | nonafluoro-2-butyl | H | CF3 |
| 21-482 | phenyl | H | H | N | H | H | H | Br | H | nonafluoro-2-butyl | H | i-C3F7 |
| 21-483 | methyl | H | H | N | H | H | H | I | H | heptafluoroisopropyl | H | CF3 |
| 21-484 | ethyl | H | H | N | H | H | H | I | H | heptafluoroisopropyl | H | CF3 |
| 21-485 | 2-fluoroethyl | H | H | N | H | H | H | I | H | heptafluoroisopropyl | H | CF3 |
| 21-486 | 2-chloroethyl | H | H | N | H | H | H | I | H | heptafluoroisopropyl | H | CF3 |
| 21-487 | 2-bromoethyl | H | H | N | H | H | H | I | H | heptafluoroisopropyl | H | CF3 |
| 21-488 | 2-iodoethyl | H | H | N | H | H | H | I | H | heptafluoroisopropyl | H | CF3 |
| 21-489 | 2-cyanoethyl | H | H | N | H | H | H | I | H | heptafluoroisopropyl | H | CF3 |
| 21-490 | 2,2-difluoroethyl | H | H | N | H | H | H | I | H | heptafluoroisopropyl | H | CF3 |
| 21-491 | 2,2-dichloroethyl | H | H | N | H | H | H | I | H | heptafluoroisopropyl | H | CF3 |
| 21-492 | 2,2,2-trifluoroethyl | H | H | N | H | H | H | I | H | heptafluoroisopropyl | H | CF3 |
| 21-493 | 2,2,2-trichloroethyl | H | H | N | H | H | H | I | H | heptafluoroisopropyl | H | CF3 |
| 21-494 | 2,2,2-tribromoethyl | H | H | N | H | H | H | I | H | heptafluoroisopropyl | H | CF3 |
| 21-495 | n-propyl | H | H | N | H | H | H | I | H | heptafluoroisopropyl | H | CF3 |
| 21-496 | 3-fluoro-n-propyl | H | H | N | H | H | H | I | H | heptafluoroisopropyl | H | CF3 |
| 21-497 | 3-chloro-n-propyl | H | H | N | H | H | H | I | H | heptafluoroisopropyl | H | CF3 |
| 21-498 | 3-bromo-n-propyl | H | H | N | H | H | H | I | H | heptafluoroisopropyl | H | C2F5 |
| 21-499 | i-propyl | H | H | N | H | H | H | I | H | heptafluoroisopropyl | H | CF3 |
| 21-500 | 1,2-difluoro-2-propyl | H | H | N | H | H | H | I | H | heptafluoroisopropyl | H | CF3 |
| 21-501 | 1,3-difluoro-2-propyl | H | H | N | H | H | H | I | H | heptafluoroisopropyl | H | CF3 |
| 21-502 | 1,3-dichloro-2-propyl | H | H | N | H | H | H | I | H | heptafluoroisopropyl | H | CF3 |
| 21-503 | 1-chloro-3-fluoro-2-propyl | H | H | N | H | H | H | I | H | heptafluoroisopropyl | H | CF3 |
| 21-504 | 3,3,3-trifluoro-n-propyl | H | H | N | H | H | H | I | H | heptafluoroisopropyl | H | CF3 |
| 21-505 | n-butyl | H | H | N | H | H | H | I | H | heptafluoroisopropyl | H | CF3 |
| 21-506 | 4,4,4-trifluoro-n-butyl | H | H | N | H | H | H | I | H | heptafluoroisopropyl | H | CF3 |
| 21-507 | i-butyl | H | H | N | H | H | H | I | H | heptafluoroisopropyl | H | CF3 |
| 21-508 | s-butyl | H | H | N | H | H | H | I | H | heptafluoroisopropyl | H | CF3 |
| 21-509 | vinyl | H | H | N | H | H | H | I | H | heptafluoroisopropyl | H | CF3 |
| 21-510 | benzyl | H | H | N | H | H | H | I | H | heptafluoroisopropyl | H | CF3 |
| 21-511 | phenyl | H | H | N | H | H | H | I | H | heptafluoroisopropyl | H | CF3 |
| 21-512 | methyl | H | H | N | H | H | H | I | H | nonafluoro-2-butyl | H | CF3 |
| 21-513 | ethyl | H | H | N | H | H | H | I | H | nonafluoro-2-butyl | H | CF3 |
| 21-514 | 2-fluoroethyl | H | H | N | H | H | H | I | H | nonafluoro-2-butyl | H | CF3 |
| 21-515 | 2-chloroethyl | H | H | N | H | H | H | I | H | nonafluoro-2-butyl | H | CF3 |
| 21-516 | 2-bromoethyl | H | H | N | H | H | H | I | H | nonafluoro-2-butyl | H | CF3 |
| 21-517 | 2-iodoethyl | H | H | N | H | H | H | I | H | nonafluoro-2-butyl | H | CF3 |
| 21-518 | 2-cyanoethyl | H | H | N | H | H | H | I | H | nonafluoro-2-butyl | H | CF3 |
| 21-519 | 2,2-difluoroethyl | H | H | N | H | H | H | I | H | nonafluoro-2-butyl | H | CF3 |
| 21-520 | 2,2-dichloroethyl | H | H | N | H | H | H | I | H | nonafluoro-2-butyl | H | CF3 |
| 21-521 | 2,2,2-trifluoroethyl | H | H | N | H | H | H | I | H | nonafluoro-2-butyl | H | CF3 |
| 21-522 | 2,2,2-trichloroethyl | H | H | N | H | H | H | I | H | nonafluoro-2-butyl | H | CF3 |
| 21-523 | 2,2,2-tribromoethyl | H | H | N | H | H | H | I | H | nonafluoro-2-butyl | H | CF3 |
| 21-524 | n-propyl | H | H | N | H | H | H | I | H | nonafluoro-2-butyl | H | n-C3F7 |
| 21-525 | 3-fluoro-n-propyl | H | H | N | H | H | H | I | H | nonafluoro-2-butyl | H | CF3 |
| 21-526 | 3-chloro-n-propyl | H | H | N | H | H | H | I | H | nonafluoro-2-butyl | H | CF3 |
| 21-527 | 3-bromo-n-propyl | H | H | N | H | H | H | I | H | nonafluoro-2-butyl | H | CF3 |
| 21-528 | i-propyl | H | H | N | H | H | H | I | H | nonafluoro-2-butyl | H | CF3 |
| 21-529 | 1,2-difluoro-2-propyl | H | H | N | H | H | H | I | H | nonafluoro-2-butyl | H | CF3 |
| 21-530 | 1,3-difluoro-2-propyl | H | H | N | H | H | H | I | H | nonafluoro-2-butyl | H | CF3 |
| 21-531 | 1,3-dichloro-2-propyl | H | H | N | H | H | H | I | H | nonafluoro-2-butyl | H | CF3 |
| 21-532 | 1-chloro-3-fluoro-2-propyl | H | H | N | H | H | H | I | H | nonafluoro-2-butyl | H | CF3 |
| 21-533 | 3,3,3-trifluoro-n-propyl | H | H | N | H | H | H | I | H | nonafluoro-2-butyl | H | CF3 |
| 21-534 | n-butyl | H | H | N | H | H | H | I | H | nonafluoro-2-butyl | H | CF3 |
| 21-535 | 4,4,4-trifluoro-n-butyl | H | H | N | H | H | H | I | H | nonafluoro-2-butyl | H | CF3 |
| 21-536 | i-butyl | H | H | N | H | H | H | I | H | nonafluoro-2-butyl | H | CF3 |
| 21-537 | s-butyl | H | H | N | H | H | H | I | H | nonafluoro-2-butyl | H | CF3 |
| 21-538 | vinyl | H | H | N | H | H | H | I | H | nonafluoro-2-butyl | H | i-C3F7 |
| 21-539 | benzyl | H | H | N | H | H | H | I | H | nonafluoro-2-butyl | H | CF3 |
| 21-540 | phenyl | H | H | N | H | H | H | I | H | nonafluoro-2-butyl | H | CF3 |
| 21-547 | 2-cyanoethyl | H | H | N | H | H | H | OCF3 | H | heptafluoroisopropyl | H | OC2F5 |

**Table 21 (4)**

| compound number | Q₂ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 21-551 | 2,2,2-trichloroethyl | H | H | N | H | H | H | OCF3 | H | heptafluoroisopropyl | H | OCF3 |
| 21-552 | 2,2,2-tribromoethyl | H | H | N | H | H | H | OCF3 | H | pentafluoroethyl | H | OCF3 |
| 21-561 | 1-chloro-3-fluoro-2-propyl | H | H | N | H | H | H | OC2F5 | H | heptafluoroisopropyl | H | OC2F5 |
| 21-564 | 4,4,4-trifluoro-n-butyl | H | H | N | H | H | H | OCF3 | H | heptafluoroisopropyl | H | OCF3 |
| 21-566 | *s*-butyl | H | H | N | H | H | H | OC2F5 | H | heptafluoroisopropyl | H | OCF3 |
| 21-576 | 2-cyanoethyl | H | H | N | H | H | H | OC2F5 | H | nonafluoro-2-butyl | H | OCF3 |
| 21-580 | 2,2,2-trichloroethyl | H | H | N | H | H | H | OCF3 | H | nonafluoro-2-butyl | H | OCF3 |
| 21-581 | 2,2,2-tribromoethyl | H | H | N | H | H | H | OCF3 | H | nonafluoro-2-butyl | H | OC2F5 |
| 21-592 | n-butyl | H | H | N | H | H | H | OC2F5 | H | nonafluoro-2-butyl | H | OC2F5 |
| 21-593 | 4,4,4-trifluoro-n-butyl | H | H | N | H | H | H | OCF3 | H | nonafluoro-2-butyl | H | OCF3 |
| 21-605 | 2-cyanoethyl | H | H | N | H | H | H | CF3 | H | heptafluoroisopropyl | H | OC2F5 |
| 21-609 | 2,2,2-trichloroethyl | H | H | N | H | H | H | CF3 | H | heptafluoroisopropyl | H | OCF3 |
| 21-610 | 2,2,2-tribromoethyl | H | H | N | H | H | H | C2F5 | H | heptafluoroisopropyl | H | OC2F5 |
| 21-621 | n-butyl | H | H | N | H | H | H | n-C3F7 | H | heptafluoroisopropyl | H | OCF3 |
| 21-622 | 4,4,4-trifluoro-n-butyl | H | H | N | H | H | H | CF3 | H | heptafluoroisopropyl | H | OCF3 |
| 21-625 | vinyl | H | H | N | H | H | H | i-C3F7 | H | heptafluoroisopropyl | H | OCF3 |
| 21-633 | 2-iodoethyl | H | H | N | H | H | H | C2F5 | H | nonafluoro-2-butyl | H | OCF3 |
| 21-638 | 2,2,2-trichloroethyl | H | H | N | H | H | H | CF3 | H | nonafluoro-2-butyl | H | OCF3 |
| 21-640 | n-propyl | H | H | N | H | H | H | n-C3F7 | H | nonafluoro-2-butyl | H | OCF3 |
| 21-644 | i-propyl | H | H | N | H | H | H | CF3 | H | nonafluoro-2-butyl | H | OC2F5 |
| 21-651 | 4,4,4-trifluoro-n-butyl | H | H | N | H | H | H | CF3 | H | nonafluoro-2-butyl | H | OCF3 |
| 21-652 | i-butyl | H | H | N | H | H | H | i-C3F7 | H | nonafluoro-2-butyl | H | OCF3 |
| 21-655 | benzyl | H | H | N | H | H | H | n-C3F7 | H | nonafluoro-2-butyl | H | OC2F5 |
| 21-656 | phenyl | H | H | N | H | H | H | i-C3F7 | H | nonafluoro-2-butyl | H | OC2F5 |
| 21-667 | 2,2,2-trichloroethyl | H | H | N | H | H | H | CF3 | H | heptafluoroisopropyl | H | CF3 |
| 21-670 | 3-fluoro-n-propyl | H | H | N | H | H | H | C2F5 | H | heptafluoroisopropyl | H | CF3 |
| 21-673 | i-propyl | H | H | N | H | H | H | i-C3F7 | H | heptafluoroisopropyl | H | CF3 |
| 21-675 | 1,3-difluoro-2-propyl | H | H | N | H | H | H | CF3 | H | pentafluoroethyl | H | CF3 |
| 21-679 | n-butyl | H | H | N | H | H | H | C2F5 | H | heptafluoroisopropyl | H | C2F5 |
| 21-680 | 4,4,4-trifluoro-n-butyl | H | H | N | H | H | H | CF3 | H | heptafluoroisopropyl | H | CF3 |
| 21-681 | i-butyl | H | H | N | H | H | H | C2F5 | H | heptafluoroisopropyl | H | n-C3F7 |
| 21-683 | vinyl | H | H | N | H | H | H | n-C3F7 | H | heptafluoroisopropyl | H | CF3 |
| 21-696 | 2,2,2-trichloroethyl | H | H | N | H | H | H | CF3 | H | nonafluoro-2-butyl | H | CF3 |
| 21-708 | n-butyl | H | H | N | H | H | H | C2F5 | H | nonafluoro-2-butyl | H | i-C3F7 |
| 21-709 | 4,4,4-trifluoro-n-butyl | H | H | N | H | H | H | CF3 | H | nonafluoro-2-butyl | H | CF3 |
| 21-712 | vinyl | H | H | N | H | H | H | n-C3F7 | H | nonafluoro-2-butyl | H | i-C3F7 |
| 21-715 | 2,2,2-trichloroethyl | H | H | N-oxide | H | H | H | F | H | heptafluoroisopropyl | H | F |
| 21-716 | 2,2,2-trichloroethyl | H | H | N-oxide | H | H | H | F | H | nonafluoro-2-butyl | H | F |
| 21-727 | 2,2,2-trichloroethyl | H | H | N-oxide | H | H | H | Cl | H | heptafluoroisopropyl | H | Cl |
| 21-756 | 2,2,2-trichloroethyl | H | H | N-oxide | H | H | H | Cl | H | nonafluoro-2-butyl | H | Cl |
| 21-785 | 2,2,2-trichloroethyl | H | H | N-oxide | H | H | H | Br | H | heptafluoroisopropyl | H | Br |
| 21-814 | 2,2,2-trichloroethyl | H | H | N-oxide | H | H | H | Br | H | nonafluoro-2-butyl | H | Br |
| 21-843 | 2,2,2-trichloroethyl | H | H | N-oxide | H | H | H | I | H | heptafluoroisopropyl | H | I |
| 21-872 | 2,2,2-trichloroethyl | H | H | N-oxide | H | H | H | I | H | nonafluoro-2-butyl | H | I |
| 21-894 | 2,2,2-trichloroethyl | H | H | N-oxide | H | H | H | Cl | H | heptafluoroisopropyl | H | Br |
| 21-903 | 2,2,2-trichloroethyl | H | H | N-oxide | H | H | H | F | H | heptafluoroisopropyl | H | OCF3 |
| 21-904 | 2,2,2-trichloroethyl | H | H | N-oxide | H | H | H | F | H | nonafluoro-2-butyl | H | OCF3 |
| 21-915 | 2,2,2-trichloroethyl | H | H | N-oxide | H | H | H | Cl | H | heptafluoroisopropyl | H | OCF3 |
| 21-944 | 2,2,2-trichloroethyl | H | H | N-oxide | H | H | H | Cl | H | nonafluoro-2-butyl | H | OCF3 |
| 21-973 | 2,2,2-trichloroethyl | H | H | N-oxide | H | H | H | Br | H | heptafluoroisopropyl | H | OCF3 |
| 21-1002 | 2,2,2-trichloroethyl | H | H | N-oxide | H | H | H | Br | H | nonafluoro-2-butyl | H | OCF3 |
| 21-1031 | 2,2,2-trichloroethyl | H | H | N-oxide | H | H | H | I | H | heptafluoroisopropyl | H | OCF3 |
| 21-1060 | 2,2,2-trichloroethyl | H | H | N-oxide | H | H | H | I | H | nonafluoro-2-butyl | H | OCF3 |
| 21-1079 | 2,2,2-trichloroethyl | H | H | N-oxide | H | H | H | F | H | heptafluoroisopropyl | H | CF3 |
| 21-1080 | 2,2,2-trichloroethyl | H | H | N-oxide | H | H | H | F | H | nonafluoro-2-butyl | H | CF3 |
| 21-1091 | 2,2,2-trichloroethyl | H | H | N-oxide | H | H | H | Cl | H | heptafluoroisopropyl | H | CF3 |
| 21-1120 | 2,2,2-trichloroethyl | H | H | N-oxide | H | H | H | Cl | H | nonafluoro-2-butyl | H | CF3 |
| 21-1149 | 2,2,2-trichloroethyl | H | H | N-oxide | H | H | H | Br | H | heptafluoroisopropyl | H | CF3 |
| 21-1178 | 2,2,2-trichloroethyl | H | H | N-oxide | H | H | H | Br | H | nonafluoro-2-butyl | H | CF3 |
| 21-1207 | 2,2,2-trichloroethyl | H | H | N-oxide | H | H | H | I | H | heptafluoroisopropyl | H | CF3 |
| 21-1236 | 2,2,2-trichloroethyl | H | H | N-oxide | H | H | H | I | H | nonafluoro-2-butyl | H | CF3 |
| 21-1265 | 2,2,2-trichloroethyl | H | H | N-oxide | H | H | H | OCF3 | H | heptafluoroisopropyl | H | OCF3 |
| 21-1294 | 2,2,2-trichloroethyl | H | H | N-oxide | H | H | H | OCF3 | H | nonafluoro-2-butyl | H | OCF3 |
| 21-1323 | 2,2,2-trichloroethyl | H | H | N-oxide | H | H | H | CF3 | H | heptafluoroisopropyl | H | OCF3 |
| 21-1352 | 2,2,2-trichloroethyl | H | H | N-oxide | H | H | H | CF3 | H | nonafluoro-2-butyl | H | OCF3 |
| 21-1381 | 2,2,2-trichloroethyl | H | H | N-oxide | H | H | H | CF3 | H | heptafluoroisopropyl | H | CF3 |
| 21-1410 | 2,2,2-trichloroethyl | H | H | N-oxide | H | H | H | CF3 | H | nonafluoro-2-butyl | H | CF3 |

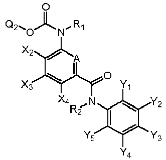

**Table 22(1)**

| compound number | Q₂ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 22-1 | 2,2,2-trichloroethyl | Me | H | N | H | H | H | F | H | heptafluoroisopropyl | H | F |
| 22-2 | 2,2,2-trichloroethyl | Me | H | N | H | H | H | F | H | nonafluoro-2-butyl | H | F |
| 22-13 | 2,2,2-trichloroethyl | Me | H | N | H | H | H | Cl | H | heptafluoroisopropyl | H | Cl |
| 22-42 | 2,2,2-trichloroethyl | Me | H | N | H | H | H | Cl | H | nonafluoro-2-butyl | H | Cl |
| 22-71 | 2,2,2-trichloroethyl | Me | H | N | H | H | H | Br | H | heptafluoroisopropyl | H | Br |
| 22-100 | 2,2,2-trichloroethyl | Me | H | N | H | H | H | Br | H | nonafluoro-2-butyl | H | Br |
| 22-129 | 2,2,2-trichloroethyl | Me | H | N | H | H | H | I | H | heptafluoroisopropyl | H | I |
| 22-158 | 2,2,2-trichloroethyl | Me | H | N | H | H | H | I | H | nonafluoro-2-butyl | H | I |
| 22-186 | 2,2,2-trichloroethyl | Me | H | N | H | H | H | Cl | H | nonafluoro-2-butyl | H | Br |
| 22-189 | 2,2,2-trichloroethyl | Me | H | N | H | H | H | F | H | heptafluoroisopropyl | H | OCF3 |
| 22-190 | 2,2,2-trichloroethyl | Me | H | N | H | H | H | F | H | nonafluoro-2-butyl | H | OCF3 |
| 22-201 | 2,2,2-trichloroethyl | Me | H | N | H | H | H | Cl | H | heptafluoroisopropyl | H | OCF3 |
| 22-230 | 2,2,2-trichloroethyl | Me | H | N | H | H | H | Cl | H | nonafluoro-2-butyl | H | OCF3 |
| 22-259 | 2,2,2-trichloroethyl | Me | H | N | H | H | H | Br | H | heptafluoroisopropyl | H | OCF3 |
| 22-288 | 2,2,2-trichloroethyl | Me | H | N | H | H | H | Br | H | nonafluoro-2-butyl | H | OCF3 |
| 22-317 | 2,2,2-trichloroethyl | Me | H | N | H | H | H | I | H | heptafluoroisopropyl | H | OCF3 |
| 22-346 | 2,2,2-trichloroethyl | Me | H | N | H | H | H | I | H | nonafluoro-2-butyl | H | OCF3 |
| 22-365 | 2,2,2-trichloroethyl | Me | H | N | H | H | H | F | H | heptafluoroisopropyl | H | CF3 |
| 22-366 | 2,2,2-trichloroethyl | Me | H | N | H | H | H | F | H | nonafluoro-2-butyl | H | CF3 |
| 22-373 | 2-cyanoethyl | Et | H | N | H | H | H | Cl | H | heptafluoroisopropyl | H | C2F5 |
| 22-377 | 2,2,2-trichloroethyl | Me | H | N | H | H | H | Cl | H | heptafluoroisopropyl | H | CF3 |
| 22-379 | n-propyl | Me | H | N | H | H | H | Cl | H | pentafluoroethyl | H | CF3 |
| 22-383 | i-propyl | n-Pr | H | N | H | H | H | Cl | H | heptafluoroisopropyl | H | n-C3F7 |
| 22-386 | 1,3-dichloro-2-propyl | Me | H | N | H | H | H | Cl | H | pentafluoroethyl | H | CF3 |
| 22-387 | 1-chloro-3-fluoro-2-propyl | i-Pr | H | N | H | H | H | Cl | H | heptafluoroisopropyl | H | CF3 |
| 22-390 | 4,4,4-trifluoro-n-butyl | Me | H | N | H | H | H | Cl | H | heptafluoroisopropyl | H | CF3 |
| 22-391 | i-butyl | CH2CH=CH2 | H | N | H | H | H | Cl | H | heptafluoroisopropyl | H | CF3 |
| 22-397 | ethyl | Me | H | N | H | H | H | Cl | H | nonafluoro-2-butyl | H | i-C3F7 |
| 22-402 | 2-cyanoethyl | CN | H | N | H | H | H | Cl | H | nonafluoro-2-butyl | H | CF3 |
| 22-406 | 2,2,2-trichloroethyl | Me | H | N | H | H | H | Cl | H | nonafluoro-2-butyl | H | CF3 |
| 22-408 | n-propyl | CH2C≡CH | H | N | H | H | H | Cl | H | nonafluoro-2-butyl | H | CF3 |
| 22-419 | 4,4,4-trifluoro-n-butyl | Me | H | N | H | H | H | Cl | H | nonafluoro-2-butyl | H | CF3 |
| 22-420 | i-butyl | NH2 | H | N | H | H | H | Cl | H | nonafluoro-2-butyl | H | CF3 |
| 22-431 | 2-cyanoethyl | Me | H | N | H | H | H | Br | H | heptafluoroisopropyl | H | C2F5 |
| 22-435 | 2,2,2-trichloroethyl | Me | H | N | H | H | H | Br | H | heptafluoroisopropyl | H | CF3 |
| 22-438 | 3-fluoro-n-propyl | C(O)OMe | H | N | H | H | H | Br | H | heptafluoroisopropyl | H | CF3 |
| 22-442 | 1,2-difluoro-2-propyl | Me | H | N | H | H | H | Br | H | heptafluoroisopropyl | H | n-C3F7 |
| 22-448 | 4,4,4-trifluoro-n-butyl | Me | H | N | H | H | H | Br | H | heptafluoroisopropyl | H | CF3 |
| 22-451 | vinyl | C(O)C(O)OMe | H | N | H | H | H | Br | H | heptafluoroisopropyl | H | CF3 |
| 22-464 | 2,2,2-trichloroethyl | Me | H | N | H | H | H | Br | H | nonafluoro-2-butyl | H | CF3 |
| 22-466 | n-propyl | C(O)OEt | H | N | H | H | H | Br | H | nonafluoro-2-butyl | H | CF3 |
| 22-470 | i-propyl | C(O)Me | H | N | H | H | H | Br | H | nonafluoro-2-butyl | H | CF3 |
| 22-477 | 4,4,4-trifluoro-n-butyl | Me | H | N | H | H | H | Br | H | nonafluoro-2-butyl | H | CF3 |
| 22-482 | phenyl | C(O)C(O)OEt | H | N | H | H | H | Br | H | nonafluoro-2-butyl | H | i-C3F7 |
| 22-493 | 2,2,2-trichloroethyl | Me | H | N | H | H | H | I | H | heptafluoroisopropyl | H | CF3 |
| 22-498 | 3-bromo-n-propyl | C(O)Et | H | N | H | H | H | I | H | heptafluoroisopropyl | H | C2F5 |
| 22-506 | 4,4,4-trifluoro-n-butyl | Me | H | N | H | H | H | I | H | heptafluoroisopropyl | H | CF3 |
| 22-510 | benzyl | S(O)2Me | H | N | H | H | H | I | H | heptafluoroisopropyl | H | CF3 |
| 22-518 | 2-cyanoethyl | S(O)2Et | H | N | H | H | H | I | H | nonafluoro-2-butyl | H | CF3 |
| 22-522 | 2,2,2-trichloroethyl | Me | H | N | H | H | H | I | H | nonafluoro-2-butyl | H | CF3 |
| 22-523 | 2,2,2-tribromoethyl | Me | H | N | H | H | H | I | H | nonafluoro-2-butyl | H | CF3 |
| 22-524 | n-propyl | CH2Ph | H | N | H | H | H | I | H | nonafluoro-2-butyl | H | n-C3F7 |
| 22-528 | i-propyl | CH2(3-Py) | H | N | H | H | H | I | H | nonafluoro-2-butyl | H | CF3 |
| 22-535 | 4,4,4-trifluoro-n-butyl | Me | H | N | H | H | H | I | H | nonafluoro-2-butyl | H | CF3 |
| 22-537 | s-butyl | CH2(3-Py-N-oxide) | H | N | H | H | H | I | H | nonafluoro-2-butyl | H | CF3 |

**Table 22(2)**

| compound number | Q₂ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 22-538 | vinyl | Me | H | N | H | H | H | I | H | nonafluoro-2-butyl | H | i-C3F7 |
| 22-539 | benzyl | Me | H | N | H | H | H | I | H | nonafluoro-2-butyl | H | CF3 |
| 22-540 | phenyl | Me | H | N | H | H | H | I | H | nonafluoro-2-butyl | H | CF3 |
| 22-551 | 2,2,2-trichloroethyl | Me | H | N | H | H | H | OCF3 | H | heptafluoroisopropyl | H | OCF3 |
| 22-580 | 2,2,2-trichloroethyl | Me | H | N | H | H | H | OCF3 | H | nonafluoro-2-butyl | H | OCF3 |
| 22-609 | 2,2,2-trichloroethyl | Me | H | N | H | H | H | CF3 | H | heptafluoroisopropyl | H | OCF3 |
| 22-638 | 2,2,2-trichloroethyl | Me | H | N | H | H | H | CF3 | H | nonafluoro-2-butyl | H | OCF3 |
| 22-667 | 2,2,2-trichloroethyl | Me | H | N | H | H | H | CF3 | H | heptafluoroisopropyl | H | CF3 |
| 22-696 | 2,2,2-trichloroethyl | Me | H | N | H | H | H | CF3 | H | nonafluoro-2-butyl | H | CF3 |
| 22-715 | 2,2,2-trichloroethyl | Me | H | N-oxide | H | H | H | F | H | heptafluoroisopropyl | H | F |
| 22-716 | 2,2,2-trichloroethyl | Me | H | N-oxide | H | H | H | F | H | nonafluoro-2-butyl | H | F |
| 22-727 | 2,2,2-trichloroethyl | Me | H | N-oxide | H | H | H | Cl | H | heptafluoroisopropyl | H | Cl |
| 22-756 | 2,2,2-trichloroethyl | Me | H | N-oxide | H | H | H | Cl | H | nonafluoro-2-butyl | H | Cl |
| 22-785 | 2,2,2-trichloroethyl | Me | H | N-oxide | H | H | H | Br | H | heptafluoroisopropyl | H | Br |
| 22-814 | 2,2,2-trichloroethyl | Me | H | N-oxide | H | H | H | Br | H | nonafluoro-2-butyl | H | Br |
| 22-843 | 2,2,2-trichloroethyl | Me | H | N-oxide | H | H | H | I | H | heptafluoroisopropyl | H | I |
| 22-872 | 2,2,2-trichloroethyl | Me | H | N-oxide | H | H | H | I | H | nonafluoro-2-butyl | H | I |
| 22-894 | 2,2,2-trichloroethyl | Me | H | N-oxide | H | H | H | Cl | H | heptafluoroisopropyl | H | Br |
| 22-903 | 2,2,2-trichloroethyl | Me | H | N-oxide | H | H | H | F | H | heptafluoroisopropyl | H | OCF3 |
| 22-904 | 2,2,2-trichloroethyl | Me | H | N-oxide | H | H | H | F | H | nonafluoro-2-butyl | H | OCF3 |
| 22-915 | 2,2,2-trichloroethyl | Me | H | N-oxide | H | H | H | Cl | H | heptafluoroisopropyl | H | OCF3 |
| 22-944 | 2,2,2-trichloroethyl | Me | H | N-oxide | H | H | H | Cl | H | nonafluoro-2-butyl | H | OCF3 |
| 22-973 | 2,2,2-trichloroethyl | Me | H | N-oxide | H | H | H | Br | H | heptafluoroisopropyl | H | OCF3 |
| 22-1002 | 2,2,2-trichloroethyl | Me | H | N-oxide | H | H | H | Br | H | nonafluoro-2-butyl | H | OCF3 |
| 22-1031 | 2,2,2-trichloroethyl | Me | H | N-oxide | H | H | H | I | H | heptafluoroisopropyl | H | OCF3 |
| 22-1060 | 2,2,2-trichloroethyl | Me | H | N-oxide | H | H | H | I | H | nonafluoro-2-butyl | H | OCF3 |
| 22-1079 | 2,2,2-trichloroethyl | Me | H | N-oxide | H | H | H | F | H | heptafluoroisopropyl | H | CF3 |
| 22-1080 | 2,2,2-trichloroethyl | Me | H | N-oxide | H | H | H | F | H | nonafluoro-2-butyl | H | CF3 |
| 22-1091 | 2,2,2-trichloroethyl | Me | H | N-oxide | H | H | H | Cl | H | heptafluoroisopropyl | H | CF3 |
| 22-1120 | 2,2,2-trichloroethyl | Me | H | N-oxide | H | H | H | Cl | H | nonafluoro-2-butyl | H | CF3 |
| 22-1149 | 2,2,2-trichloroethyl | Me | H | N-oxide | H | H | H | Br | H | heptafluoroisopropyl | H | CF3 |
| 22-1178 | 2,2,2-trichloroethyl | Me | H | N-oxide | H | H | H | Br | H | nonafluoro-2-butyl | H | CF3 |
| 22-1207 | 2,2,2-trichloroethyl | Me | H | N-oxide | H | H | H | I | H | heptafluoroisopropyl | H | CF3 |
| 22-1236 | 2,2,2-trichloroethyl | Me | H | N-oxide | H | H | H | I | H | nonafluoro-2-butyl | H | CF3 |
| 22-1265 | 2,2,2-trichloroethyl | Me | H | N-oxide | H | H | H | OCF3 | H | heptafluoroisopropyl | H | OCF3 |
| 22-1294 | 2,2,2-trichloroethyl | Me | H | N-oxide | H | H | H | OCF3 | H | nonafluoro-2-butyl | H | OCF3 |
| 22-1323 | 2,2,2-trichloroethyl | Me | H | N-oxide | H | H | H | CF3 | H | heptafluoroisopropyl | H | OCF3 |
| 22-1352 | 2,2,2-trichloroethyl | Me | H | N-oxide | H | H | H | CF3 | H | nonafluoro-2-butyl | H | OCF3 |
| 22-1381 | 2,2,2-trichloroethyl | Me | H | N-oxide | H | H | H | CF3 | H | heptafluoroisopropyl | H | CF3 |
| 22-1410 | 2,2,2-trichloroethyl | Me | H | N-oxide | H | H | H | CF3 | H | nonafluoro-2-butyl | H | CF3 |

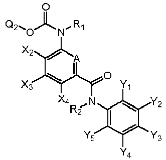

**Table 23(1)**

| compound number | Q₂ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 23-1 | 2,2,2-trichloroethyl | H | Me | N | H | H | H | F | H | heptafluoroisopropyl | H | F |
| 23-2 | 2,2,2-trichloroethyl | H | Me | N | H | H | H | F | H | nonafluoro-2-butyl | H | F |
| 23-13 | 2,2,2-trichloroethyl | H | Me | N | H | H | H | Cl | H | heptafluoroisopropyl | H | Cl |
| 23-42 | 2,2,2-trichloroethyl | H | Me | N | H | H | H | Cl | H | nonafluoro-2-butyl | H | Cl |
| 23-100 | 2,2,2-trichloroethyl | H | Me | N | H | H | H | Br | H | nonafluoro-2-butyl | H | Br |
| 23-129 | 2,2,2-trichloroethyl | H | Me | N | H | H | H | I | H | heptafluoroisopropyl | H | I |
| 23-158 | 2,2,2-trichloroethyl | H | Me | N | H | H | H | I | H | nonafluoro-2-butyl | H | I |
| 23-186 | 2,2,2-trichloroethyl | H | Me | N | H | H | H | Cl | H | nonafluoro-2-butyl | H | Br |
| 23-189 | 2,2,2-trichloroethyl | H | Me | N | H | H | H | F | H | heptafluoroisopropyl | H | OCF3 |
| 23-190 | 2,2,2-trichloroethyl | H | Me | N | H | H | H | F | H | nonafluoro-2-butyl | H | OCF3 |
| 23-201 | 2,2,2-trichloroethyl | H | Me | N | H | H | H | Cl | H | heptafluoroisopropyl | H | OCF3 |
| 23-230 | 2,2,2-trichloroethyl | H | Me | N | H | H | H | Cl | H | nonafluoro-2-butyl | H | OCF3 |
| 23-259 | 2,2,2-trichloroethyl | H | Me | N | H | H | H | Br | H | heptafluoroisopropyl | H | OCF3 |
| 23-288 | 2,2,2-trichloroethyl | H | Me | N | H | H | H | Br | H | nonafluoro-2-butyl | H | OCF3 |
| 23-317 | 2,2,2-trichloroethyl | H | Me | N | H | H | H | I | H | heptafluoroisopropyl | H | OCF3 |
| 23-346 | 2,2,2-trichloroethyl | H | Me | N | H | H | H | I | H | nonafluoro-2-butyl | H | OCF3 |
| 23-365 | 2,2,2-trichloroethyl | H | Me | N | H | H | H | F | H | heptafluoroisopropyl | H | CF3 |
| 23-366 | 2,2,2-trichloroethyl | H | Me | N | H | H | H | F | H | nonafluoro-2-butyl | H | CF3 |
| 23-373 | 2-cyanoethyl | H | Et | N | H | H | H | Cl | H | heptafluoroisopropyl | H | C2F5 |
| 23-377 | 2,2,2-trichloroethyl | H | Me | N | H | H | H | Cl | H | heptafluoroisopropyl | H | CF3 |
| 23-379 | n-propyl | H | Me | N | H | H | H | Cl | H | pentafluoroethyl | H | CF3 |
| 23-383 | i-propyl | H | n-Pr | N | H | H | H | Cl | H | heptafluoroisopropyl | H | n-C3F7 |
| 23-386 | 1,3-dichloro-2-propyl | H | Me | N | H | H | H | Cl | H | pentafluoroethyl | H | CF3 |
| 23-387 | 1-chloro-3-fluoro-2-propyl | H | i-Pr | N | H | H | H | Cl | H | heptafluoroisopropyl | H | CF3 |
| 23-391 | i-butyl | H | CH2CH=CH2 | N | H | H | H | Cl | H | heptafluoroisopropyl | H | CF3 |
| 23-397 | ethyl | H | Me | N | H | H | H | Cl | H | nonafluoro-2-butyl | H | i-C3F7 |
| 23-402 | 2-cyanoethyl | H | CN | N | H | H | H | Cl | H | nonafluoro-2-butyl | H | CF3 |
| 23-406 | 2,2,2-trichloroethyl | H | Me | N | H | H | H | Cl | H | nonafluoro-2-butyl | H | CF3 |
| 23-408 | n-propyl | H | CH2C≡CH | N | H | H | H | Cl | H | nonafluoro-2-butyl | H | CF3 |
| 23-420 | i-butyl | H | NH2 | N | H | H | H | Cl | H | nonafluoro-2-butyl | H | CF3 |
| 23-431 | 2-cyanoethyl | H | Me | N | H | H | H | Br | H | heptafluoroisopropyl | H | C2F5 |
| 23-435 | 2,2,2-trichloroethyl | H | Me | N | H | H | H | Br | H | heptafluoroisopropyl | H | CF3 |
| 23-437 | n-propyl | H | C(O)OMe | N | H | H | H | Br | H | heptafluoroisopropyl | H | CF3 |
| 23-442 | 1,2-difluoro-2-propyl | H | Me | N | H | H | H | Br | H | heptafluoroisopropyl | H | n-C3F7 |
| 23-448 | 4,4,4-trifluoro-n-butyl | H | Me | N | H | H | H | Br | H | heptafluoroisopropyl | H | CF3 |
| 23-451 | vinyl | H | C(O)C(O)OMe | N | H | H | H | Br | H | heptafluoroisopropyl | H | CF3 |
| 23-464 | 2,2,2-trichloroethyl | H | Me | N | H | H | H | Br | H | nonafluoro-2-butyl | H | CF3 |
| 23-466 | n-propyl | H | C(O)OEt | N | H | H | H | Br | H | nonafluoro-2-butyl | H | CF3 |
| 23-470 | i-propyl | H | C(O)Me | N | H | H | H | Br | H | nonafluoro-2-butyl | H | CF3 |
| 23-477 | 4,4,4-trifluoro-n-butyl | H | Me | N | H | H | H | Br | H | nonafluoro-2-butyl | H | CF3 |
| 23-482 | phenyl | H | C(O)C(O)OEt | N | H | H | H | Br | H | nonafluoro-2-butyl | H | i-C3F7 |
| 23-493 | 2,2,2-trichloroethyl | H | Me | N | H | H | H | I | H | heptafluoroisopropyl | H | CF3 |
| 23-498 | 3-bromo-n-propyl | H | C(O)Et | N | H | H | H | I | H | heptafluoroisopropyl | H | C2F5 |
| 23-506 | 4,4,4-trifluoro-n-butyl | H | Me | N | H | H | H | I | H | heptafluoroisopropyl | H | CF3 |
| 23-510 | benzyl | H | S(O)2Me | N | H | H | H | I | H | heptafluoroisopropyl | H | CF3 |
| 23-518 | 2-cyanoethyl | H | S(O)2Et | N | H | H | H | I | H | nonafluoro-2-butyl | H | CF3 |
| 23-522 | 2,2,2-trichloroethyl | H | Me | N | H | H | H | I | H | nonafluoro-2-butyl | H | CF3 |
| 23-524 | n-propyl | H | CH2Ph | N | H | H | H | I | H | nonafluoro-2-butyl | H | n-C3F7 |
| 23-528 | i-propyl | H | CH2(3-Py) | N | H | H | H | I | H | nonafluoro-2-butyl | H | CF3 |
| 23-535 | 4,4,4-trifluoro-n-butyl | H | Me | N | H | H | H | I | H | nonafluoro-2-butyl | H | CF3 |
| 23-537 | s-butyl | H | CH2(3-Py-N-oxide) | N | H | H | H | I | H | nonafluoro-2-butyl | H | CF3 |
| 23-538 | vinyl | H | Me | N | H | H | H | I | H | nonafluoro-2-butyl | H | i-C3F7 |
| 23-551 | 2,2,2-trichloroethyl | H | Me | N | H | H | H | OCF3 | H | heptafluoroisopropyl | H | OCF3 |
| 23-580 | 2,2,2-trichloroethyl | H | Me | N | H | H | H | OCF3 | H | nonafluoro-2-butyl | H | OCF3 |
| 23-609 | 2,2,2-trichloroethyl | H | Me | N | H | H | H | CF3 | H | heptafluoroisopropyl | H | OCF3 |

**Table 23(2)**

| compound number | Q₂ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 23-638 | 2,2,2-trichloroethyl | H | Me | N | H | H | H | CF3 | H | nonafluoro-2-butyl | H | OCF3 |
| 23-667 | 2,2,2-trichloroethyl | H | Me | N | H | H | H | CF3 | H | heptafluoroisopropyl | H | CF3 |
| 23-696 | 2,2,2-trichloroethyl | H | Me | N | H | H | H | CF3 | H | nonafluoro-2-butyl | H | CF3 |
| 23-715 | 2,2,2-trichloroethyl | H | Me | N-oxide | H | H | H | F | H | heptafluoroisopropyl | H | F |
| 23-716 | 2,2,2-trichloroethyl | H | Me | N-oxide | H | H | H | F | H | nonafluoro-2-butyl | H | F |
| 23-727 | 2,2,2-trichloroethyl | H | Me | N-oxide | H | H | H | Cl | H | heptafluoroisopropyl | H | Cl |
| 23-756 | 2,2,2-trichloroethyl | H | Me | N-oxide | H | H | H | Cl | H | nonafluoro-2-butyl | H | Cl |
| 23-785 | 2,2,2-trichloroethyl | H | Me | N-oxide | H | H | H | Br | H | heptafluoroisopropyl | H | Br |
| 23-814 | 2,2,2-trichloroethyl | H | Me | N-oxide | H | H | H | Br | H | nonafluoro-2-butyl | H | Br |
| 23-843 | 2,2,2-trichloroethyl | H | Me | N-oxide | H | H | H | I | H | heptafluoroisopropyl | H | I |
| 23-872 | 2,2,2-trichloroethyl | H | Me | N-oxide | H | H | H | I | H | nonafluoro-2-butyl | H | I |
| 23-895 | 2,2,2-trichloroethyl | H | Me | N-oxide | H | H | H | Cl | H | heptafluoroisopropyl | H | I |
| 23-903 | 2,2,2-trichloroethyl | H | Me | N-oxide | H | H | H | F | H | heptafluoroisopropyl | H | OCF3 |
| 23-904 | 2,2,2-trichloroethyl | H | Me | N-oxide | H | H | H | F | H | nonafluoro-2-butyl | H | OCF3 |
| 23-915 | 2,2,2-trichloroethyl | H | Me | N-oxide | H | H | H | Cl | H | heptafluoroisopropyl | H | OCF3 |
| 23-944 | 2,2,2-trichloroethyl | H | Me | N-oxide | H | H | H | Cl | H | nonafluoro-2-butyl | H | OCF3 |
| 23-973 | 2,2,2-trichloroethyl | H | Me | N-oxide | H | H | H | Br | H | heptafluoroisopropyl | H | OCF3 |
| 23-1002 | 2,2,2-trichloroethyl | H | Me | N-oxide | H | H | H | Br | H | nonafluoro-2-butyl | H | OCF3 |
| 23-1031 | 2,2,2-trichloroethyl | H | Me | N-oxide | H | H | H | I | H | heptafluoroisopropyl | H | OCF3 |
| 23-1060 | 2,2,2-trichloroethyl | H | Me | N-oxide | H | H | H | I | H | nonafluoro-2-butyl | H | OCF3 |
| 23-1079 | 2,2,2-trichloroethyl | H | Me | N-oxide | H | H | H | F | H | heptafluoroisopropyl | H | CF3 |
| 23-1080 | 2,2,2-trichloroethyl | H | Me | N-oxide | H | H | H | F | H | nonafluoro-2-butyl | H | CF3 |
| 23-1091 | 2,2,2-trichloroethyl | H | Me | N-oxide | H | H | H | Cl | H | heptafluoroisopropyl | H | CF3 |
| 23-1120 | 2,2,2-trichloroethyl | H | Me | N-oxide | H | H | H | Cl | H | nonafluoro-2-butyl | H | CF3 |
| 23-1149 | 2,2,2-trichloroethyl | H | Me | N-oxide | H | H | H | Br | H | heptafluoroisopropyl | H | CF3 |
| 23-1178 | 2,2,2-trichloroethyl | H | Me | N-oxide | H | H | H | Br | H | nonafluoro-2-butyl | H | CF3 |
| 23-1207 | 2,2,2-trichloroethyl | H | Me | N-oxide | H | H | H | I | H | heptafluoroisopropyl | H | CF3 |
| 23-1236 | 2,2,2-trichloroethyl | H | Me | N-oxide | H | H | H | I | H | nonafluoro-2-butyl | H | CF3 |
| 23-1265 | 2,2,2-trichloroethyl | H | Me | N-oxide | H | H | H | OCF3 | H | heptafluoroisopropyl | H | OCF3 |
| 23-1294 | 2,2,2-trichloroethyl | H | Me | N-oxide | H | H | H | OCF3 | H | nonafluoro-2-butyl | H | OCF3 |
| 23-1323 | 2,2,2-trichloroethyl | H | Me | N-oxide | H | H | H | CF3 | H | heptafluoroisopropyl | H | OCF3 |
| 23-1352 | 2,2,2-trichloroethyl | H | Me | N-oxide | H | H | H | CF3 | H | nonafluoro-2-butyl | H | OCF3 |
| 23-1381 | 2,2,2-trichloroethyl | H | Me | N-oxide | H | H | H | CF3 | H | heptafluoroisopropyl | H | CF3 |
| 23-1410 | 2,2,2-trichloroethyl | H | Me | N-oxide | H | H | H | CF3 | H | nonafluoro-2-butyl | H | CF3 |

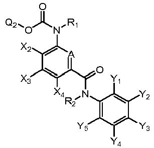

**Table 24(1)**

| compound number | Q₂ | R₁ | R₂ | A | X₂ | X₃ | X₄ | Y₁ | Y₁ | Y₃ | Y₄ | Y₅ |
|---|---|---|---|---|---|---|---|---|---|---|---|---|
| 24-1 | 2,2,2-trichloroethyl | Me | Me | N | H | H | H | F | H | heptafluoroisopropyl | H | F |
| 24-2 | 2,2,2-trichloroethyl | Me | Me | N | H | H | H | F | H | nonafluoro-2-butyl | H | F |
| 24-13 | 2,2,2-trichloroethyl | Me | Me | N | H | H | H | Cl | H | heptafluoroisopropyl | H | Cl |
| 24-42 | 2,2,2-trichloroethyl | Me | Me | N | H | H | H | Cl | H | nonafluoro-2-butyl | H | Cl |
| 24-71 | 2,2,2-trichloroethyl | Me | Me | N | H | H | H | Br | H | heptafluoroisopropyl | H | Br |
| 24-100 | 2,2,2-trichloroethyl | Me | Me | N | H | H | H | Br | H | nonafluoro-2-butyl | H | Br |
| 24-129 | 2,2,2-trichloroethyl | Me | Me | N | H | H | H | I | H | heptafluoroisopropyl | H | I |
| 24-158 | 2,2,2-trichloroethyl | Me | Me | N | H | H | H | I | H | nonafluoro-2-butyl | H | I |
| 24-376 | 2,2,2-trichloroethyl | Me | Me | N | H | H | H | Cl | H | heptafluoroisopropyl | H | Br |
| 24-385 | 2,2,2-trichloroethyl | Me | Me | N | H | H | H | F | H | heptafluoroisopropyl | H | OCF3 |
| 24-386 | 2,2,2-trichloroethyl | Me | Me | N | H | H | H | F | H | nonafluoro-2-butyl | H | OCF3 |
| 24-397 | 2,2,2-trichloroethyl | Me | Me | N | H | H | H | Cl | H | heptafluoroisopropyl | H | OCF3 |
| 24-426 | 2,2,2-trichloroethyl | Me | Me | N | H | H | H | Cl | H | nonafluoro-2-butyl | H | OCF3 |
| 24-455 | 2,2,2-trichloroethyl | Me | Me | N | H | H | H | Br | H | heptafluoroisopropyl | H | OCF3 |
| 24-484 | 2,2,2-trichloroethyl | Me | Me | N | H | H | H | Br | H | nonafluoro-2-butyl | H | OCF3 |
| 24-513 | 2,2,2-trichloroethyl | Me | Me | N | H | H | H | I | H | heptafluoroisopropyl | H | OCF3 |
| 24-542 | 2,2,2-trichloroethyl | Me | Me | N | H | H | H | I | H | nonafluoro-2-butyl | H | OCF3 |
| 24-757 | 2,2,2-trichloroethyl | Me | Me | N | H | H | H | F | H | heptafluoroisopropyl | H | CF3 |
| 24-758 | 2,2,2-trichloroethyl | Me | Me | N | H | H | H | F | H | nonafluoro-2-butyl | H | CF3 |
| 24-764 | 2-iodoethyl | Me | Et | N | H | H | H | Cl | H | heptafluoroisopropyl | H | CF3 |
| 24-765 | 2-cyanoethyl | Et | Me | N | H | H | H | Cl | H | heptafluoroisopropyl | H | C2F5 |
| 24-769 | 2,2,2-trichloroethyl | Me | Me | N | H | H | H | Cl | H | heptafluoroisopropyl | H | CF3 |
| 24-771 | n-propyl | Me | n-Pr | N | H | H | H | Cl | H | pentafluoroethyl | H | CF3 |
| 24-775 | i-propyl | n-Pr | Me | N | H | H | H | Cl | H | heptafluoroisopropyl | H | n-C3F7 |
| 24-778 | 1,3-dichloro-2-propyl | Me | Me | N | H | H | H | Cl | H | pentafluoroethyl | H | CF3 |
| 24-779 | 1-chloro-3-fluoro-2-propyl | i-Pr | Me | N | H | H | H | Cl | H | heptafluoroisopropyl | H | CF3 |
| 24-781 | n-butyl | Me | i-Pr | N | H | H | H | Cl | H | heptafluoroisopropyl | H | CF3 |
| 24-783 | i-butyl | CH2CH=CH2 | Me | N | H | H | H | Cl | H | heptafluoroisopropyl | H | CF3 |
| 24-784 | s-butyl | Me | CH2CH=CH2 | N | H | H | H | Cl | H | heptafluoroisopropyl | H | CF3 |
| 24-785 | vinyl | Me | CN | N | H | H | H | Cl | H | heptafluoroisopropyl | H | CF3 |
| 24-789 | ethyl | Me | Me | N | H | H | H | Cl | H | nonafluoro-2-butyl | H | i-C3F7 |
| 24-794 | 2-cyanoethyl | CN | Me | N | H | H | H | Cl | H | nonafluoro-2-butyl | H | CF3 |
| 24-798 | 2,2,2-trichloroethyl | Me | Me | N | H | H | H | Cl | H | nonafluoro-2-butyl | H | CF3 |
| 24-799 | 2,2,2-tribromoethyl | Me | CH2C≡CH | N | H | H | H | Cl | H | nonafluoro-2-butyl | H | CF3 |
| 24-800 | n-propyl | CH2C≡CH | Me | N | H | H | H | Cl | H | nonafluoro-2-butyl | H | CF3 |
| 24-810 | n-butyl | Me | NH2 | N | H | H | H | Cl | H | nonafluoro-2-butyl | H | CF3 |
| 24-812 | i-butyl | NH2 | Me | N | H | H | H | Cl | H | nonafluoro-2-butyl | H | CF3 |
| 24-823 | 2-cyanoethyl | Me | C(O)OMe | N | H | H | H | Br | H | heptafluoroisopropyl | H | C2F5 |
| 24-827 | 2,2,2-trichloroethyl | Me | Me | N | H | H | H | Br | H | heptafluoroisopropyl | H | CF3 |
| 24-829 | n-propyl | C(O)OMe | Me | N | H | H | H | Br | H | heptafluoroisopropyl | H | CF3 |
| 24-834 | 1,2-difluoro-2-propyl | Me | Me | N | H | H | H | Br | H | heptafluoroisopropyl | H | n-C3F7 |
| 24-840 | 4,4,4-trifluoro-n-butyl | Me | Me | N | H | H | H | Br | H | heptafluoroisopropyl | H | CF3 |
| 24-841 | i-butyl | Me | C(O)C(O)0Me | N | H | H | H | Br | H | heptafluoroisopropyl | H | CF3 |
| 24-842 | s-butyl | Me | Me | N | H | H | H | Br | H | heptafluoroisopropyl | H | CF3 |
| 24-843 | vinyl | C(O)C(O)OMe | Me | N | H | H | H | Br | H | heptafluoroisopropyl | H | CF3 |
| 24-852 | 2-cyanoethyl | Me | C(O)OEt | N | H | H | H | Br | H | nonafluoro-2-butyl | H | CF3 |
| 24-856 | 2,2,2-trichloroethyl | Me | Me | N | H | H | H | Br | H | nonafluoro-2-butyl | H | CF3 |
| 24-858 | n-propyl | C(O)OEt | Me | N | H | H | H | Br | H | nonafluoro-2-butyl | H | CF3 |
| 24-861 | 3-bromo-n-propyl | Me | C(O)Me | N | H | H | H | Br | H | nonafluoro-2-butyl | H | CF3 |
| 24-862 | i-propyl | C(O)Me | Me | N | H | H | H | Br | H | nonafluoro-2-butyl | H | CF3 |
| 24-869 | 4,4,4-trifluoro-n-butyl | Me | Me | N | H | H | H | Br | H | nonafluoro-2-butyl | H | CF3 |
| 24-870 | i-butyl | Me | C(O)C(O)OEt | N | H | H | H | Br | H | nonafluoro-2-butyl | H | CF3 |
| 24-874 | phenyl | C(O)C(O)OEt | Me | N | H | H | H | Br | H | nonafluoro-2-butyl | H | i-C3F7 |
| 24-885 | 2,2,2-trichloroethyl | Me | Me | N | H | H | H | I | H | heptafluoroisopropyl | H | CF3 |
| 24-887 | n-propyl | Me | C(O)Et | N | H | H | H | I | H | heptafluoroisopropyl | H | CF3 |

**Table 24(2)**

| compound | Q₂ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 24-890 | 3-bromo-n-propyl | C(O)Et | Me | N | H | H | H | I | H | heptafluoroisopropyl | H | C2F5 |
| 24-898 | 4,4,4-trifluoro-n-butyl | Me | Me | N | H | H | H | I | H | heptafluoroisopropyl | H | CF3 |
| 24-899 | i-butyl | Me | S(O)2Me | N | H | H | H | I | H | heptafluoroisopropyl | H | CF3 |
| 24-902 | benzyl | S(O)2Me | Me | N | H | H | H | I | H | heptafluoroisopropyl | H | CF3 |
| 24-909 | 2-iodoethyl | Me | S(O)2Et | N | H | H | H | I | H | nonafluoro-2-butyl | H | CF3 |
| 24-910 | 2-cyanoethyl | S(O)2Et | Me | N | H | H | H | I | H | nonafluoro-2-butyl | H | CF3 |
| 24-914 | 2,2,2-trichloroethyl | Me | Me | N | H | H | H | I | H | nonafluoro-2-butyl | H | CF3 |
| 24-915 | 2,2,2-tribromoethyl | Me | CH2Ph | N | H | H | H | I | H | nonafluoro-2-butyl | H | CF3 |
| 24-916 | n-propyl | CH2Ph | Me | N | H | H | H | I | H | nonafluoro-2-butyl | H | n-C3F7 |
| 24-920 | i-propyl | CH2(3-Py) | Me | N | H | H | H | I | H | nonafluoro-2-butyl | H | CF3 |
| 24-926 | n-butyl | Me | CH2(3-Py) | N | H | H | H | I | H | nonafluoro-2-butyl | H | CF3 |
| 24-927 | 4,4,4-trifluoro-n-butyl | Me | Me | N | H | H | H | I | H | nonafluoro-2-butyl | H | CF3 |
| 24-929 | s-butyl | CH2(3-Py-N-oxide) | Me | N | H | H | H | I | H | nonafluoro-2-butyl | H | CF3 |
| 24-930 | vinyl | Me | Me | N | H | H | H | I | H | nonafluoro-2-butyl | H | i-C3F7 |
| 24-932 | phenyl | Me | CH2(3-Py-N-oxide Me | N | H | H | H | I | H | nonafluoro-2-butyl | H | CF3 |
| 24-1139 | 2,2,2-trichloroethyl | Me | | N | H | H | H | OCF3 | H | heptafluoroisopropyl | H | OCF3 |
| 24-1168 | 2,2,2-trichloroethyl | Me | Me | N | H | H | H | OCF3 | H | nonafluoro-2-butyl | H | OCF3 |
| 24-1197 | 2,2,2-trichloroethyl | Me | Me | N | H | H | H | CF3 | H | heptafluoroisopropyl | H | OCF3 |
| 24-1226 | 2,2,2-trichloroethyl | Me | Me | N | H | H | H | CF3 | H | nonafluoro-2-butyl | H | OCF3 |
| 24-1255 | 2,2,2-trichloroethyl | Me | Me | N | H | H | H | CF3 | H | heptafluoroisopropyl | H | CF3 |
| 24-1284 | 2,2,2-trichloroethyl | Me | Me | N | H | H | H | CF3 | H | nonafluoro-2-butyl | H | CF3 |
| 24-1499 | 2,2,2-trichloroethyl | Me | Me | N-oxide | H | H | H | F | H | heptafluoroisopropyl | H | F |
| 24-1500 | 2,2,2-trichloroethyl | Me | Me | N-oxide | H | H | H | F | H | nonafluoro-2-butyl | H | F |
| 24-1511 | 2,2,2-trichloroethyl | Me | Me | N-oxide | H | H | H | Cl | H | heptafluoroisopropyl | H | Cl |
| 24-1540 | 2,2,2-trichloroethyl | Me | Me | N-oxide | H | H | H | Cl | H | nonafluoro-2-butyl | H | Cl |
| 24-1569 | 2,2,2-trichloroethyl | Me | Me | N-oxide | H | H | H | Br | H | heptafluoroisopropyl | H | Br |
| 24-1598 | 2,2,2-trichloroethyl | Me | Me | N-oxide | H | H | H | Br | H | nonafluoro-2-butyl | H | Br |
| 24-1627 | 2,2,2-trichloroethyl | Me | Me | N-oxide | H | H | H | I | H | heptafluoroisopropyl | H | I |
| 24-1656 | 2,2,2-trichloroethyl | Me | Me | N-oxide | H | H | H | I | H | nonafluoro-2-butyl | H | I |
| 24-1875 | 2,2,2-trichloroethyl | Me | Me | N-oxide | H | H | H | Cl | H | heptafluoroisopropyl | H | I |
| 24-1883 | 2,2,2-trichloroethyl | Me | Me | N-oxide | H | H | H | F | H | heptafluoroisopropyl | H | OCF3 |
| 24-1884 | 2,2,2-trichloroethyl | Me | Me | N-oxide | H | H | H | F | H | nonafluoro-2-butyl | H | OCF3 |
| 24-1895 | 2,2,2-trichloroethyl | Me | Me | N-oxide | H | H | H | Cl | H | heptafluoroisopropyl | H | OCF3 |
| 24-1924 | 2,2,2-trichloroethyl | Me | Me | N-oxide | H | H | H | Cl | H | nonafluoro-2-butyl | H | OCF3 |
| 24-1953 | 2,2,2-trichloroethyl | Me | Me | N-oxide | H | H | H | Br | H | heptafluoroisopropyl | H | OCF3 |
| 24-1982 | 2,2,2-trichloroethyl | Me | Me | N-oxide | H | H | H | Br | H | nonafluoro-2-butyl | H | OCF3 |
| 24-2011 | 2,2,2-trichloroethyl | Me | Me | N-oxide | H | H | H | I | H | heptafluoroisopropyl | H | OCF3 |
| 24-2040 | 2,2,2-trichloroethyl | Me | Me | N-oxide | H | H | H | I | H | nonafluoro-2-butyl | H | OCF3 |
| 24-2255 | 2,2,2-trichloroethyl | Me | Me | N-oxide | H | H | H | F | H | heptafluoroisopropyl | H | CF3 |
| 24-2256 | 2,2,2-trichloroethyl | Me | Me | N-oxide | H | H | H | F | H | nonafluoro-2-butyl | H | CF3 |
| 24-2267 | 2,2,2-trichloroethyl | Me | Me | N-oxide | H | H | H | Cl | H | heptafluoroisopropyl | H | CF3 |
| 24-2296 | 2,2,2-trichloroethyl | Me | Me | N-oxide | H | H | H | Cl | H | nonafluoro-2-butyl | H | CF3 |
| 24-2325 | 2,2,2-trichloroethyl | Me | Me | N-oxide | H | H | H | Br | H | heptafluoroisopropyl | H | CF3 |
| 24-2354 | 2,2,2-trichloroethyl | Me | Me | N-oxide | H | H | H | Br | H | nonafluoro-2-butyl | H | CF3 |
| 24-2383 | 2,2,2-trichloroethyl | Me | Me | N-oxide | H | H | H | I | H | heptafluoroisopropyl | H | CF3 |
| 24-2412 | 2,2,2-trichloroethyl | Me | Me | N-oxide | H | H | H | I | H | nonafluoro-2-butyl | H | CF3 |
| 24-2637 | 2,2,2-trichloroethyl | Me | Me | N-oxide | H | H | H | OCF3 | H | heptafluoroisopropyl | H | OCF3 |
| 24-2666 | 2,2,2-trichloroethyl | Me | Me | N-oxide | H | H | H | OCF3 | H | nonafluoro-2-butyl | H | OCF3 |
| 24-2695 | 2,2,2-trichloroethyl | Me | Me | N-oxide | H | H | H | CF3 | H | heptafluoroisopropyl | H | OCF3 |
| 24-2724 | 2,2,2-trichloroethyl | Me | Me | N-oxide | H | H | H | CF3 | H | nonafluoro-2-butyl | H | OCF3 |
| 24-2753 | 2,2,2-trichloroethyl | Me | Me | N-oxide | H | H | H | CF3 | H | heptafluoroisopropyl | H | CF3 |
| 24-2782 | 2,2,2-trichloroethyl | Me | Me | N-oxide | H | H | H | CF3 | H | nonafluoro-2-butyl | H | CF3 |

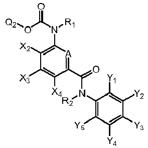

**Table 25(1)**

| compound number | Q₂ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 25-9 | 2,2-dichloroethyl | H | Et | N | H | H | H | Cl | F | heptafluoroisopropyl | F | Cl |
| 25-39 | 222-trifluoroethyl | H | H | N | H | H | H | Cl | Me | nonafluoro-2-butyl | Me | I |
| 25-152 | 2,2-dichloroethyl | H | Et | N | H | H | H | Cl | F | heptafluoroisopropyl | F | OCF3 |
| 25-182 | 2,2,2-trifluoroethyl | H | H | N | H | H | H | Cl | Me | nonafluoro-2-butyl | Me | OCF3 |
| 25-287 | methyl | H | H | N | H | H | H | Cl | F | heptafluoroisopropyl | H | CF3 |
| 25-288 | ethyl | Me | H | N | H | H | H | Cl | H | heptafluoroisopropyl | F | CF3 |
| 25-289 | 2-fluoroethyl | H | Me | N | H | H | H | Cl | Cl | heptafluoroisopropyl | H | CF3 |
| 25-290 | 2-chloroethyl | Me | Me | N | H | H | H | Cl | H | heptafluoroisopropyl | Cl | CF3 |
| 25-291 | 2-bromoethyl | H | H | N | H | H | H | F | Br | heptafluoroisopropyl | H | CF3 |
| 25-292 | 2-iodoethyl | H | H | N | H | H | H | Cl | H | heptafluoroisopropyl | Br | CF3 |
| 25-293 | 2-cyanoethyl | Et | H | N | H | H | H | Cl | I | heptafluoroisopropyl | H | C2F5 |
| 25-294 | 2,2-difluoroethyl | H | H | N | H | H | H | Cl | H | heptafluoroisopropyl | I | CF3 |
| 25-295 | 2,2-dichloroethyl | H | Et | N | H | H | H | Cl | F | heptafluoroisopropyl | F | CF3 |
| 25-296 | 222-trifluoroethyl | H | H | N | H | H | H | Cl | F | heptafluoroisopropyl | Cl | CF3 |
| 25-299 | n-propyl | H | H | N | H | H | H | Cl | Cl | pentafluoroethyl | F | CF3 |
| 25-300 | 3-fluoro-n-propyl | H | H | N | H | H | H | Cl | Cl | heptafluoroisopropyl | Cl | CF3 |
| 25-303 | i-propyl | H | n-Pr | N | H | H | H | Cl | Br | heptafluoroisopropyl | F | CF3 |
| 25-304 | 1,2-difluoro-2-propyl | H | H | N | H | H | H | Cl | Br | heptafluoroisopropyl | Cl | CF3 |
| 25-307 | 1-chloro-3-fluoro-2-propyl | H | H | N | H | H | H | F | I | nonafluoro-2-butyl | F | CF3 |
| 25-308 | 3,3,3-trifluoro-n-propyl | H | H | N | H | H | H | Cl | I | nonafluoro-2-butyl | Cl | CF3 |
| 25-311 | i-butyl | H | H | N | H | H | H | Cl | Me | nonafluoro-2-butyl | H | CF3 |
| 25-312 | s-butyl | H | H | N | H | H | H | Cl | H | nonafluoro-2-butyl | Me | CF3 |
| 25-313 | vinyl | i-Pr | H | N | H | H | H | Cl | Et | nonafluoro-2-butyl | H | CF3 |
| 25-314 | benzyl | H | H | N | H | H | H | Cl | H | nonafluoro-2-butyl | Et | CF3 |
| 25-325 | 2,2,2-trifluoroethyl | H | H | N | H | H | H | Cl | Me | nonafluoro-2-butyl | Me | n-C3F7 |
| 25-326 | 2,2,2-trichloroethyl | H | H | N | H | H | H | Cl | Me | nonafluoro-2-butyl | Et | i-C3F7 |
| 25-332 | i-propyl | H | H | N | H | H | H | Cl | Et | nonafluoro-2-butyl | Me | n-C3F7 |
| 25-333 | 1,2-difluoro-2-propyl | H | H | N | H | H | H | Cl | Et | nonafluoro-2-butyl | Et | i-C3F7 |
| 25-339 | 2-chloroethyl | H | H | N | H | H | H | Br | n-Pr | heptafluoroisopropyl | Me | CF3 |
| 25-340 | 2-bromoethyl | H | H | N | H | H | H | Br | n-Pr | heptafluoroisopropyl | Et | CF3 |
| 25-346 | 2,2,2-trichloroethyl | H | H | N | H | H | H | Br | i-Pr | heptafluoroisopropyl | Me | CF3 |
| 25-347 | 2,2,2-tribromoethyl | H | H | N | H | H | H | Br | i-Pr | heptafluoroisopropyl | Et | CF3 |
| 25-353 | 1,2-difluoro-2-propyl | H | H | N | H | H | H | Br | n-Bu | heptafluoroisopropyl | Me | CF3 |
| 25-354 | 1.3-difluoro-2-propyl | H | H | N | H | H | H | Br | n-Bu | heptafluoroisopropyl | Et | CF3 |
| 25-360 | i-butyl | H | H | N | H | H | H | Br | i-Bu | nonafluoro-2-butyl | Me | CF3 |
| 25-361 | s-butyl | H | H | N | H | H | H | Br | i-Bu | nonafluoro-2-butyl | Et | CF3 |
| 25-367 | 2-fluoroethyl | H | H | N | H | H | H | Br | s-Bu | nonafluoro-2-butyl | Me | CF3 |
| 25-368 | 2-chloroethyl | H | H | N | H | H | H | Br | s-Bu | nonafluoro-2-butyl | Et | CF3 |
| 25-374 | 2,2,2-trifluoroethyl | H | H | N | H | H | H | Br | F | nonafluoro-2-butyl | Me | CF3 |
| 25-375 | 2,2,2-trichloroethyl | H | H | N | H | H | H | Br | F | nonafluoro-2-butyl | Et | CF3 |
| 25-381 | i-propyl | Et | i-Pr | N | H | H | H | Br | Cl | nonafluoro-2-butyl | Me | CF3 |
| 25-382 | 1,2-difluoro-2-propyl | H | H | N | H | H | H | Br | Cl | nonafluoro-2-butyl | Et | CF3 |
| 25-388 | 2-iodoethyl | H | H | N | H | H | H | I | Br | heptafluoroisopropyl | Me | CF3 |
| 25-389 | 2-cyanoethyl | H | H | N | H | H | H | I | Br | heptafluoroisopropyl | Et | CF3 |
| 25-395 | n-propyl | n-Pr | Me | N | H | H | H | I | I | heptafluoroisopropyl | Me | CF3 |
| 25-396 | 3-fluoro-n-propyl | H | H | N | H | H | H | I | I | heptafluoroisopropyl | Et | CF3 |
| 25-402 | 1.3-dichloro-2-propyl | H | H | N | H | H | H | I | Me | heptafluoroisopropyl | F | C2F5 |
| 25-403 | 1-chloro-3-fluoro-2-propyl | H | H | N | H | H | H | I | Me | nonafluoro-2-butyl | Cl | CF3 |
| 25-406 | 4,4,4 trifluoro-n-butyl | H | H | N | H | H | H | I | Et | nonafluoro-2-butyl | F | CF3 |
| 25-407 | i-butyl | H | H | N | H | H | H | I | Et | nonafluoro-2-butyl | Cl | CF3 |
| 25-410 | benzyl | H | H | N | H | H | H | I | n-Pr | nonafluoro-2-butyl | F | CF3 |
| 25-413 | ethyl | H | H | N | H | H | H | I | n-Pr | nonafluoro-2-butyl | I | n-C3F7 |
| 25-414 | 2-fluoroethyl | H | H | N | H | H | H | I | i-Pr | nonafluoro-2-butyl | F | i-C3F7 |
| 25-415 | 2-chloroethyl | H | H | N | H | H | H | I | i-Pr | nonafluoro-2-butyl | Cl | CF3 |
| 25-418 | 2-cyanoethyl | H | H | N | H | H | H | I | n-Bu | nonafluoro-2-butyl | F | CF3 |

**Table 25(2)**

| compound number | Q₂ | R₁ | R₂ | A | X₂ | X₃ | X₄ | Y₁ | Y₂ | Y₃ | Y₄ | Y₅ |
|---|---|---|---|---|---|---|---|---|---|---|---|---|
| 25-419 | 2,2-difluoroethyl | H | H | N | H | H | H | I | n-Bu | nonafluoro-2-butyl | Cl | CF3 |
| 25-422 | 2,2,2-trichloroethyl | H | H | N | H | H | H | I | i-Bu | nonafluoro-2-butyl | F | CF3 |
| 25-423 | 2,2,2-tribromoethyl | H | H | N | H | H | H | I | i-Bu | nonafluoro-2-butyl | Cl | CF3 |
| 25-426 | 3-chloro-n-propyl | i-Pr | n-Pr | N | H | H | H | I | s-Bu | nonafluoro-2-butyl | F | CF3 |
| 25-427 | 3-bromo-n-propyl | H | H | N | H | H | H | I | s-Bu | nonafluoro-2-butyl | Cl | CF3 |
| 25-438 | 2,2-dichloroethyl | H | Et | N | H | H | H | OCF3 | F | heptafluoroisopropyl | F | OCF3 |
| 25-468 | 2,2,2-trifluoroethyl | H | H | N | H | H | H | OCF3 | Me | nonafluoro-2-butyl | Me | OCF3 |
| 25-581 | 2,2-dichloroethyl | H | Et | N | H | H | H | OCF3 | F | heptafluoroisopropyl | F | CF3 |
| 25-611 | 2,2,2-trifluoroethyl | H | H | N | H | H | H | OCF3 | Me | nonafluoro-2-butyl | Me | CF3 |
| 25-724 | 2,2-dichloroethyl | H | Et | N | H | H | H | CF3 | F | heptafluoroisopropyl | F | CF3 |
| 25-754 | 2,2,2-trifluoroethyl | H | H | N | H | H | H | CF3 | Me | nonafluoro-2-butyl | Me | CF3 |
| 25-867 | 2,2-dichloroethyl | H | Et | N-oxide | H | H | H | Cl | F | heptafluoroisopropyl | F | Cl |
| 25-897 | 2,2,2-trifluoroethyl | H | H | N-oxide | H | H | H | Cl | Me | nonafluoro-2-butyl | Me | I |
| 25-1010 | 2,2-dichloroethyl | H | Et | N-oxide | H | H | H | Cl | F | heptafluoroisopropyl | F | OCF3 |
| 25-1040 | 2,2,2-trifluoroethyl | H | H | N-oxide | H | H | H | Cl | Me | nonafluoro-2-butyl | Me | OCF3 |
| 25-1155 | 2,2,2-trichloroethyl | H | H | N-oxide | H | H | H | Cl | F | heptafluoroisopropyl | Br | CF3 |
| 25-1168 | 4,4,4-trifluoro-n-butyl | H | H | N-oxide | H | H | H | Cl | I | nonafluoro-2-butyl | I | CF3 |
| 25-1184 | 2,2,2-trichloroethyl | H | H | N-oxide | H | H | H | Cl | Me | nonafluoro-2-butyl | Et | i-C3F7 |
| 25-1233 | 2,2,2-trichloroethyl | H | H | N-oxide | H | H | H | Br | F | nonafluoro-2-butyl | Et | CF3 |
| 25-1264 | 4,4,4-trifluoro-n-butyl | H | H | N-oxide | H | H | H | I | Et | nonafluoro-2-butyl | F | CF3 |
| 25-1296 | 2,2-dichloroethyl | H | Et | N-oxide | H | H | H | OCF3 | F | heptafluoroisopropyl | F | OCF3 |
| 25-1326 | 2,2,2-trifluoroethyl | H | H | N-oxide | H | H | H | OCF3 | Me | nonafluoro-2-butyl | Me | OCF3 |
| 25-1439 | 2,2-dichloroethyl | H | Et | N-oxide | H | H | H | OCF3 | F | heptafluoroisopropyl | F | CF3 |
| 25-1469 | 2,2,2-trifluoroethyl | H | H | N-oxide | H | H | H | OCF3 | Me | nonafluoro-2-butyl | Me | CF3 |
| 25-1582 | 2,2-dichloroethyl | H | Et | N-oxide | H | H | H | CF3 | F | heptafluoroisopropyl | F | CF3 |
| 25-1612 | 2,2,2-trifluoroethyl | H | H | N-oxide | H | H | H | CF3 | Me | nonafluoro-2-butyl | Me | CF3 |

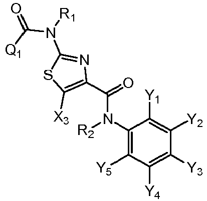

**Table 26(1)**

| compound number | Q₁ | R₁ | R₂ | X₃ | Y₁ | Y₂ | Y₃ | Y₄ | Y₅ |
|---|---|---|---|---|---|---|---|---|---|
| 26-1 | phenyl | H | H | H | Cl | H | heptafluoroisopropyl | H | OCF3 |
| 26-4 | 4-fluorophenyl | H | H | Cl | Cl | H | heptafluoroisopropyl | H | OCF3 |
| 26-12 | 3-iodophenyl | H | H | H | Cl | H | pentafluoroethyl | H | OCF3 |
| 26-13 | 4-iodophenyl | H | H | H | Cl | H | pentafluoroethyl | H | OCF3 |
| 26-16 | (4-trifluoromethyl) phenyl | H | H | H | Cl | H | heptafluoroisopropyl | H | OC2F5 |
| 26-17 | 2-nitrophenyl | H | H | H | F | H | heptafluoroisopropyl | H | OCF3 |
| 26-20 | 2-cyanophenyl | H | H | H | Cl | H | pentafluoroethyl | H | OC2F5 |
| 26-21 | 3-cyanophenyl | H | H | H | Cl | H | heptafluoroisopropyl | H | OCF3 |
| 26-22 | 4-cyanophenyl | H | H | H | Cl | H | heptafluoroisopropyl | H | OCF3 |
| 26-28 | 4-bromo-2-chlorophenyl | H | H | H | Cl | H | heptafluoroisopropyl | H | OC2F5 |
| 26-36 | 2-fluoropyridin-3-yl | H | H | H | Cl | H | pentafluoroethyl | H | OCF3 |
| 26-37 | 2-chloropyridin-3-yl | H | H | H | Cl | H | heptafluoroisopropyl | H | OCF3 |
| 26-41 | 2-nitropyridin-3-yl | H | H | H | Cl | H | heptafluoroisopropyl | H | OC2F5 |
| 26-50 | 5-fluoropyridin-3-yl | H | H | H | Cl | H | heptafluoroisopropyl | H | OC2F5 |
| 26-55 | 5-nitropyridin-3-yl | H | H | H | Cl | H | pentafluoroethyl | H | OCF3 |
| 26-59 | 4-bromopyridin-3-yl | H | H | H | Cl | H | heptafluoroisopropyl | H | OC2F5 |
| 26-60 | 4-iodopyridin-3-yl | H | H | H | Cl | H | heptafluoroisopropyl | H | OC2F5 |
| 26-78 | 4-cyanopyridin-2-yl | H | H | H | Cl | H | pentafluoroethyl | H | OCF3 |
| 26-79 | phenyl | H | H | H | Cl | H | nonafluoro-2-butyl | H | OCF3 |
| 26-95 | 2-nitrophenyl | H | H | H | F | H | nonafluoro-2-butyl | H | OCF3 |
| 26-99 | 3-cyanophenyl | H | H | H | Cl | H | nonafluoro-2-butyl | H | OCF3 |
| 26-100 | 4-cyanophenyl | H | H | H | Cl | H | nonafluoro-2-butyl | H | OCF3 |
| 26-105 | 2-chloro-4,5-difluorophenyl | H | H | H | Cl | H | nonafluoro-2-butyl | H | OC2F5 |
| 26-115 | 2-chloropyridin-3-yl | H | H | H | Cl | H | nonafluoro-2-butyl | H | OCF3 |
| 26-121 | 6-fluoropyridin-3-yl | H | H | H | Cl | H | nonafluoro-2-butyl | H | OC2F5 |
| 26-130 | 5-bromopyridin-3-yl | H | H | H | Cl | H | nonafluoro-2-butyl | H | OC2F5 |
| 26-131 | 5-iodopyridin-3-yl | H | H | H | Cl | H | nonafluoro-2-butyl | H | OC2F5 |
| 26-139 | 4-(trifluoromethyl)pyridin-3-yl | H | H | H | Cl | H | nonafluoro-2-butyl | H | OC2F5 |
| 26-140 | 4-nitropyridin-3-yl | H | H | H | Cl | H | nonafluoro-2-butyl | H | OC2F5 |
| 26-157 | phenyl | H | H | H | Br | H | heptafluoroisopropyl | H | OCF3 |
| 26-158 | 2-fluorophenyl | H | H | H | Br | H | heptafluoroisopropyl | H | OCF3 |
| 26-177 | 3-cyanophenyl | H | H | H | Br | H | heptafluoroisopropyl | H | OCF3 |
| 26-178 | 4-cyanophenyl | H | H | H | Br | H | heptafluoroisopropyl | H | OCF3 |
| 26-193 | 2-chloropyridin-3-yl | H | H | H | Br | H | heptafluoroisopropyl | H | OCF3 |
| 26-235 | phenyl | H | H | H | Br | H | nonafluoro-2-butyl | H | OCF3 |
| 26-236 | 2-fluorophenyl | H | H | H | Br | H | nonafluoro-2-butyl | H | OCF3 |
| 26-255 | 3-cyanophenyl | H | H | H | Br | H | nonafluoro-2-butyl | H | OCF3 |
| 26-256 | 4-cyanophenyl | H | H | H | Br | H | nonafluoro-2-butyl | H | OCF3 |
| 26-271 | 2-chloropyridin-3-yl | H | H | H | Br | H | nonafluoro-2-butyl | H | OCF3 |
| 26-313 | phenyl | H | H | H | I | H | heptafluoroisopropyl | H | OCF3 |
| 26-314 | 2-fluorophenyl | H | H | H | I | H | heptafluoroisopropyl | H | OCF3 |
| 26-333 | 3-cyanophenyl | H | H | H | I | H | heptafluoroisopropyl | H | OCF3 |
| 26-334 | 4-cyanophenyl | H | H | H | I | H | heptafluoroisopropyl | H | OCF3 |
| 26-349 | 2-chloropyridin-3-yl | H | H | H | I | H | heptafluoroisopropyl | H | OCF3 |
| 26-391 | phenyl | H | H | H | I | H | nonafluoro-2-butyl | H | OCF3 |
| 26-392 | 2-fluorophenyl | H | H | H | I | H | nonafluoro-2-butyl | H | OCF3 |
| 26-411 | 3-cyanophenyl | H | H | H | I | H | nonafluoro-2-butyl | H | OCF3 |
| 26-412 | 4-cyanophenyl | H | H | H | I | H | nonafluoro-2-butyl | H | OCF3 |
| 26-427 | 2-chloropyridin-3-yl | H | H | H | I | H | nonafluoro-2-butyl | H | OCF3 |
| 26-469 | phenyl | H | H | H | F | H | heptafluoroisopropyl | H | CF3 |

**Table 26(2)**

| compound number | Q₁ | R₁ | R₂ | X₃ | Y₁ | Y₂ | Y₃ | Y₄ | Y₅ |
|---|---|---|---|---|---|---|---|---|---|
| 26-470 | phenyl | H | H | H | Cl | H | heptafluoroisopropyl | H | CF3 |
| 26-471 | 2 -fluorophenyl | H | H | H | Cl | H | heptafluoroisopropyl | H | CF3 |
| 26-481 | 3-iodophenyl | H | H | H | Cl | H | pentafluoroethyl | H | CF3 |
| 26-482 | 4-iodophenyl | H | H | H | Cl | H | pentafluoroethyl | H | CF3 |
| 26-489 | 2-cyanophenyl | H | H | H | Cl | H | pentafluoroethyl | H | C2F5 |
| 26-490 | 3-cyanophenyl | H | H | H | Cl | H | heptafluoroisopropyl | H | CF3 |
| 26-491 | 4-cyanophenyl | H | H | H | Cl | H | heptafluoroisopropyl | H | CF3 |
| 26-494 | 2,4-dichlorophenyl | H | H | H | Cl | H | heptafluoroisopropyl | H | C2F5 |
| 26-498 | 2-bromo-4-chlorophenyl | H | H | H | Cl | H | heptafluoroisopropyl | H | n-C3F7 |
| 26-505 | 2-fluoropyridin-3-yl | H | H | H | Cl | H | pentafluoroethyl | H | CF3 |
| 26-506 | 2-chloropyridin-3-yl | H | H | H | Cl | H | heptafluoroisopropyl | H | CF3 |
| 26-510 | 2-nitropyridin-3-yl | H | H | Cl | Cl | H | heptafluoroisopropyl | H | CF3 |
| 26-519 | 5-fluoropyridin-3-yl | H | H | H | Cl | H | heptafluoroisopropyl | H | C2F5 |
| 26-521 | 5-bromopyridin-3-yl | H | H | H | Cl | H | heptafluoroisopropyl | H | n-C3F7 |
| 26-522 | 5-iodopyridin-3-yl | H | H | H | Cl | H | heptafluoroisopropyl | H | i-C3F7 |
| 26-524 | 5-nitropyridin-3-yl | H | H | H | Cl | H | pentafluoroethyl | H | CF3 |
| 26-526 | 4-fluoropyridin-3-yl | H | H | H | Cl | H | pentafluoroethyl | H | CF3 |
| 26-528 | 4-bromopyridin-3-yl | H | H | H | Cl | H | heptafluoroisopropyl | H | n-C3F7 |
| 26-529 | 4-iodopyridin-3-yl | H | H | H | Cl | H | heptafluoroisopropyl | H | i-C3F7 |
| 26-548 | phenyl | H | H | H | F | H | nonafluoro-2-butyl | H | CF3 |
| 26-549 | phenyl | H | H | H | Cl | H | nonafluoro-2-butyl | H | CF3 |
| 26-550 | 2 -fluorophenyl | H | H | H | Cl | H | nonafluoro-2-butyl | H | CF3 |
| 26-562 | (2-trifluoromethyl) phenyl | H | H | H | Cl | H | nonafluoro-2-butyl | H | C2F5 |
| 26-569 | 3-cyanophenyl | H | H | H | Cl | H | nonafluoro-2-butyl | H | CF3 |
| 26-570 | 4-cyanophenyl | H | H | H | Cl | H | nonafluoro-2-butyl | H | CF3 |
| 26-579 | 2-chloro-4-nitrophenyl | H | H | H | Cl | H | nonafluoro-2-butyl | H | i-C3F7 |
| 26-585 | 2-chloropyridin-3-yl | H | H | H | Cl | H | nonafluoro-2-butyl | H | CF3 |
| 26-598 | 5-fluoropyridin-3-yl | H | H | H | Cl | H | nonafluoro-2-butyl | H | C2F5 |
| 26-600 | 5-bromopyridin-3-yl | H | H | H | Cl | H | nonafluoro-2-butyl | H | n-C3F7 |
| 26-601 | 5-iodopyridin-3-yl | H | H | H | Cl | H | nonafluoro-2-butyl | H | i-C3F7 |
| 26-605 | 4-fluoropyridin-3-yl | H | H | H | Cl | H | nonafluoro-2-butyl | H | C2F5 |
| 26-609 | 4-(trifluoromethyl) pyridin-3-yl | H | H | H | Cl | H | nonafluoro-2-butyl | H | n-C3F7 |
| 26-610 | 4-nitropyridin-3-yl | H | H | H | Cl | H | nonafluoro-2-butyl | H | i-C3F7 |
| 26-626 | 4-cyanopyridin-2-yl | H | H | H | Cl | H | nonafluoro-2-butyl | H | C2F5 |
| 26-627 | phenyl | H | H | H | Br | H | heptafluoroisopropyl | H | CF3 |
| 26-628 | 2-fluorophenyl | H | H | H | Br | H | heptafluoroisopropyl | H | CF3 |
| 26-629 | 3-fluorophenyl | H | H | H | Br | H | heptafluoroisopropyl | H | CF3 |
| 26-630 | 4-fluorophenyl | H | H | H | Br | H | heptafluoroisopropyl | H | CF3 |
| 26-631 | 2-chlorophenyl | H | H | H | Br | H | heptafluoroisopropyl | H | CF3 |
| 26-632 | 3-chlorophenyl | H | H | H | Br | H | heptafluoroisopropyl | H | CF3 |
| 26-633 | 4-chlorophenyl | H | H | H | Br | H | heptafluoroisopropyl | H | CF3 |
| 26-634 | 2-bromophenyl | H | H | H | Br | H | heptafluoroisopropyl | H | CF3 |
| 26-635 | 3-bromophenyl | H | H | H | Br | H | heptafluoroisopropyl | H | CF3 |
| 26-636 | 4-bromophenyl | H | H | H | Br | H | heptafluoroisopropyl | H | CF3 |
| 26-637 | 2-iodophenyl | H | H | H | Br | H | heptafluoroisopropyl | H | CF3 |
| 26-638 | 3-iodophenyl | H | H | H | Br | H | heptafluoroisopropyl | H | CF3 |
| 26-639 | 4-iodophenyl | H | H | H | Br | H | heptafluoroisopropyl | H | CF3 |
| 26-640 | (2-trifluoromethyl) phenyl | H | H | H | Br | H | heptafluoroisopropyl | H | CF3 |
| 26-641 | (3-trifluoromethyl) phenyl | H | H | H | Br | H | heptafluoroisopropyl | H | CF3 |
| 26-642 | (4-trifluoromethyl) phenyl | H | H | H | Br | H | heptafluoroisopropyl | H | CF3 |
| 26-643 | 2-nitrophenyl | H | H | H | Br | H | heptafluoroisopropyl | H | CF3 |
| 26-644 | 3-nitrophenyl | H | H | H | Br | H | heptafluoroisopropyl | H | CF3 |
| 26-645 | 4-nitrophenyl | H | H | H | Br | H | heptafluoroisopropyl | H | CF3 |
| 26-646 | 2-cyanophenyl | H | H | H | Br | H | heptafluoroisopropyl | H | CF3 |
| 26-647 | 3-cyanophenyl | H | H | H | Br | H | heptafluoroisopropyl | H | CF3 |
| 26-648 | 4-cyanophenyl | H | H | H | Br | H | heptafluoroisopropyl | H | CF3 |
| 26-649 | 2,6-difluorophenyl | H | H | H | Br | H | heptafluoroisopropyl | H | CF3 |
| 26-650 | 3,4-dichlorophenyl | H | H | H | Br | H | heptafluoroisopropyl | H | CF3 |
| 26-651 | 2,4-dichlorophenyl | H | H | H | Br | H | heptafluoroisopropyl | H | CF3 |

**Table 26(3)**

| compound number | Q₁ | R₁ | R₂ | X₃ | Y₁ | Y₂ | Y₃ | Y₄ | Y₅ |
|---|---|---|---|---|---|---|---|---|---|
| 26-652 | 2-chloro-4-fluorophenyl | H | H | H | Br | H | heptafluoroisopropyl | H | CF3 |
| 26-653 | 2-chloro-4,5-difluorophenyl | H | H | H | Br | H | heptafluoroisopropyl | H | CF3 |
| 26-654 | 4-bromo-2-chlorophenyl | H | H | H | Br | H | heptafluoroisopropyl | H | CF3 |
| 26-655 | 2-bromo-4-chlorophenyl | H | H | H | Br | H | heptafluoroisopropyl | H | CF3 |
| 26-656 | 2-bromo-4-fluorophenyl | H | H | H | Br | H | heptafluoroisopropyl | H | CF3 |
| 26-657 | 2-chloro-4-nitrophenyl | H | H | H | Br | H | heptafluoroisopropyl | H | CF3 |
| 26-658 | 3,5-dicyanophenyl | H | H | H | Br | H | heptafluoroisopropyl | H | CF3 |
| 26-659 | 4-cyano-2-fluorophenyl | H | H | H | Br | H | heptafluoroisopropyl | H | CF3 |
| 26-660 | 2-chloro-4-cyanophenyl | H | H | H | Br | H | heptafluoroisopropyl | H | CF3 |
| 26-661 | pyridin-3-yl | H | H | H | Br | H | heptafluoroisopropyl | H | CF3 |
| 26-662 | 2-fluoropyridin-3-yl | H | H | H | Br | H | heptafluoroisopropyl | H | CF3 |
| 26-663 | 2-chloropyridin-3-yl | H | H | H | Br | H | heptafluoroisopropyl | H | CF3 |
| 26-664 | 2-bromopyridin-3-yl | H | H | H | Br | H | heptafluoroisopropyl | H | CF3 |
| 26-665 | 2-iodopyridin-3-yl | H | H | H | Br | H | heptafluoroisopropyl | H | CF3 |
| 26-666 | 2-(trifluoromethyl)pyridin-3-yl | H | H | H | Br | H | heptafluoroisopropyl | H | CF3 |
| 26-667 | 2-nitropyridin-3-yl | H | H | H | Br | H | heptafluoroisopropyl | H | CF3 |
| 26-668 | 2-cyanopyridin-3-yl | H | H | H | Br | H | heptafluoroisopropyl | H | CF3 |
| 26-669 | 6-fluoropyridin-3-yl | H | H | H | Br | H | heptafluoroisopropyl | H | CF3 |
| 26-670 | 6-chloropyridin-3-yl | H | H | H | Br | H | heptafluoroisopropyl | H | CF3 |
| 26-671 | 6-bromopyridin-3-yl | H | H | H | Br | H | heptafluoroisopropyl | H | CF3 |
| 26-672 | 6-iodopyridin-3-yl | H | H | H | Br | H | heptafluoroisopropyl | H | CF3 |
| 26-673 | 6-(trifluoromethyl)pyridin-3-yl | H | H | H | Br | H | heptafluoroisopropyl | H | CF3 |
| 26-674 | 6-nitropyridin-3-yl | H | H | H | Br | H | heptafluoroisopropyl | H | CF3 |
| 26-675 | 6-cyanopyridin-3-yl | H | H | H | Br | H | heptafluoroisopropyl | H | CF3 |
| 26-676 | 5-fluoropyridin-3-yl | H | H | H | Br | H | heptafluoroisopropyl | H | CF3 |
| 26-677 | 5-chloropyridin-3-yl | H | H | H | Br | H | heptafluoroisopropyl | H | CF3 |
| 26-678 | 5-bromopyridin-3-yl | H | H | H | Br | H | heptafluoroisopropyl | H | CF3 |
| 26-679 | 5-iodopyridin-3-yl | H | H | H | Br | H | heptafluoroisopropyl | H | CF3 |
| 26-680 | 5-(trifluoromethyl)pyridin-3-yl | H | H | H | Br | H | heptafluoroisopropyl | H | CF3 |
| 26-681 | 5-nitropyridin-3-yl | H | H | H | Br | H | heptafluoroisopropyl | H | CF3 |
| 26-682 | 5-cyanopyridin-3-yl | H | H | H | Br | H | heptafluoroisopropyl | H | CF3 |
| 26-683 | 4-fluoropyridin-3-yl | H | H | H | Br | H | heptafluoroisopropyl | H | CF3 |
| 26-684 | 4-chloropyridin-3-yl | H | H | H | Br | H | heptafluoroisopropyl | H | CF3 |
| 26-685 | 4-bromopyridin-3-yl | H | H | H | Br | H | heptafluoroisopropyl | H | CF3 |
| 26-686 | 4-iodopyridin-3-yl | H | H | H | Br | H | heptafluoroisopropyl | H | CF3 |
| 26-687 | 4-(trifluoromethyl)pyridin-3-yl | H | H | H | Br | H | heptafluoroisopropyl | H | CF3 |
| 26-688 | 4-nitropyridin-3-yl | H | H | H | Br | H | heptafluoroisopropyl | H | CF3 |
| 26-689 | 4-cyanopyridin-3-yl | H | H | H | Br | H | heptafluoroisopropyl | H | CF3 |
| 26-690 | 2,6-dichloropyridin-3-yl | H | H | H | Br | H | heptafluoroisopropyl | H | CF3 |
| 26-691 | pyridin-3-yl N-oxide | H | H | H | Br | H | heptafluoroisopropyl | H | CF3 |
| 26-692 | pyridin-4-yl | H | H | H | Br | H | heptafluoroisopropyl | H | CF3 |
| 26-693 | 2-chloropyridin-4-yl | H | H | H | Br | H | heptafluoroisopropyl | H | CF3 |
| 26-694 | 3-bromopyridin-4-yl | H | H | H | Br | H | heptafluoroisopropyl | H | CF3 |
| 26-695 | 3,5-dichloropyridin-4-yl | H | H | H | Br | H | heptafluoroisopropyl | H | CF3 |
| 26-696 | 3-(trifluoromethyl) pyridin-4-yl | H | H | H | Br | H | heptafluoroisopropyl | H | CF3 |
| 26-697 | 2,6-dicyanopyridin-4-yl | H | H | H | Br | H | heptafluoroisopropyl | H | CF3 |
| 26-698 | pyridin-4-yl N-oxide | H | H | H | Br | H | heptafluoroisopropyl | H | CF3 |
| 26-699 | pyridin-2-yl | H | H | H | Br | H | heptafluoroisopropyl | H | CF3 |
| 26-700 | 3-chloropyridin-2-yl | H | H | H | Br | H | heptafluoroisopropyl | H | CF3 |
| 26-701 | 4-bromopyridin-2-yl | H | H | H | Br | H | heptafluoroisopropyl | H | CF3 |
| 26-702 | 5-iodopyridin-2-yl | H | H | H | Br | H | heptafluoroisopropyl | H | CF3 |
| 26-703 | 6-chloropyridin-2-yl | H | H | H | Br | H | heptafluoroisopropyl | H | CF3 |
| 26-704 | 4-cyanopyridin-2-yl | H | H | H | Br | H | heptafluoroisopropyl | H | CF3 |
| 26-705 | phenyl | H | H | H | Br | H | nonafluoro-2-butyl | H | CF3 |
| 26-706 | 2-fluorophenyl | H | H | H | Br | H | nonafluoro-2-butyl | H | CF3 |
| 26-707 | 3-fluorophenyl | H | H | H | Br | H | nonafluoro-2-butyl | H | CF3 |
| 26-708 | 4-fluorophenyl | H | H | H | Br | H | nonafluoro-2-butyl | H | CF3 |
| 26-709 | 2-chlorophenyl | H | H | H | Br | H | nonafluoro-2-butyl | H | CF3 |
| 26-710 | 3-chlorophenyl | H | H | H | Br | H | nonafluoro-2-butyl | H | CF3 |

**Table 26(4)**

| compound number | Q₁ | R₁ | R₂ | X₃ | Y₁ | Y₂ | Y₃ | Y₄ | Y₅ |
|---|---|---|---|---|---|---|---|---|---|
| 26-711 | 4-chlorophenyl | H | H | H | Br | H | nonafluoro-2-butyl | H | CF3 |
| 26-712 | 2-bromophenyl | H | H | H | Br | H | nonafluoro-2-butyl | H | CF3 |
| 26-713 | 3-bromophenyl | H | H | H | Br | H | nonafluoro-2-butyl | H | CF3 |
| 26-714 | 4-bromophenyl | H | H | H | Br | H | nonafluoro-2-butyl | H | CF3 |
| 26-715 | 2-iodophenyl | H | H | H | Br | H | nonafluoro-2-butyl | H | CF3 |
| 26-716 | 3-iodophenyl | H | H | H | Br | H | nonafluoro-2-butyl | H | CF3 |
| 26-717 | 4-iodophenyl | H | H | H | Br | H | nonafluoro-2-butyl | H | CF3 |
| 26-718 | (2-trifluoromethyl) phenyl | H | H | H | Br | H | nonafluoro-2-butyl | H | CF3 |
| 26-719 | (3-trifluoromethyl) phenyl | H | H | H | Br | H | nonafluoro-2-butyl | H | CF3 |
| 26-720 | (4-trifluoromethyl) phenyl | H | H | H | Br | H | nonafluoro-2-butyl | H | CF3 |
| 26-721 | 2-nitrophenyl | H | H | H | Br | H | nonafluoro-2-butyl | H | CF3 |
| 26-722 | 3-nitrophenyl | H | H | H | Br | H | nonafluoro-2-butyl | H | CF3 |
| 26-723 | 4-nitrophenyl | H | H | H | Br | H | nonafluoro-2-butyl | H | CF3 |
| 26-724 | 2-cyanophenyl | H | H | H | Br | H | nonafluoro-2-butyl | H | CF3 |
| 26-725 | 3-cyanophenyl | H | H | H | Br | H | nonafluoro-2-butyl | H | CF3 |
| 26-726 | 4-cyanophenyl | H | H | H | Br | H | nonafluoro-2-butyl | H | CF3 |
| 26-727 | 2,6-difluorophenyl | H | H | H | Br | H | nonafluoro-2-butyl | H | CF3 |
| 26-728 | 3,4-dichlorophenyl | H | H | H | Br | H | nonafluoro-2-butyl | H | CF3 |
| 26-729 | 2,4-dichlorophenyl | H | H | H | Br | H | nonafluoro-2-butyl | H | CF3 |
| 26-730 | 2-chloro-4-fluorophenyl | H | H | H | Br | H | nonafluoro-2-butyl | H | CF3 |
| 26-731 | 2-chloro-4,5-difluorophenyl | H | H | H | Br | H | nonafluoro-2-butyl | H | CF3 |
| 26-732 | 4-bromo-2-chlorophenyl | H | H | H | Br | H | nonafluoro-2-butyl | H | CF3 |
| 26-733 | 2-bromo-4-chlorophenyl | H | H | H | Br | H | nonafluoro-2-butyl | H | CF3 |
| 26-734 | 2-bromo-4-fluorophenyl | H | H | H | Br | H | nonafluoro-2-butyl | H | CF3 |
| 26-735 | 2-chloro-4-nitrophenyl | H | H | H | Br | H | nonafluoro-2-butyl | H | CF3 |
| 26-736 | 3,5-dicyanophenyl | H | H | H | Br | H | nonafluoro-2-butyl | H | CF3 |
| 26-737 | 4-cyano-2-fluorophenyl | H | H | H | Br | H | nonafluoro-2-butyl | H | CF3 |
| 26-738 | 2-chloro-4-cyanophenyl | H | H | H | Br | H | nonafluoro-2-butyl | H | CF3 |
| 26-739 | pyridin-3-yl | H | H | H | Br | H | nonafluoro-2-butyl | H | CF3 |
| 26-740 | 2-fluoropyridin-3-yl | H | H | H | Br | H | nonafluoro-2-butyl | H | CF3 |
| 26-741 | 2-chloropyridin-3-yl | H | H | H | Br | H | nonafluoro-2-butyl | H | CF3 |
| 26-742 | 2-bromopyridin-3-yl | H | H | H | Br | H | nonafluoro-2-butyl | H | CF3 |
| 26-743 | 2-iodopyridin-3-yl | H | H | H | Br | H | nonafluoro-2-butyl | H | CF3 |
| 26-744 | 2-(trifluoromethyl) pyridin-3-yl | H | H | H | Br | H | nonafluoro-2-butyl | H | CF3 |
| 26-745 | 2-nitropyridin-3-yl | H | H | H | Br | H | nonafluoro-2-butyl | H | CF3 |
| 26-746 | 2-cyanopyridin-3-yl | H | H | H | Br | H | nonafluoro-2-butyl | H | CF3 |
| 26-747 | 6-fluoropyridin-3-yl | H | H | H | Br | H | nonafluoro-2-butyl | H | CF3 |
| 26-748 | 6-chloropyridin-3-yl | H | H | H | Br | H | nonafluoro-2-butyl | H | CF3 |
| 26-749 | 6-bromopyridin-3-yl | H | H | H | Br | H | nonafluoro-2-butyl | H | CF3 |
| 26-750 | 6-iodopyridin-3-yl | H | H | H | Br | H | nonafluoro-2-butyl | H | CF3 |
| 26-751 | 6-(trifluoromethyl) pyridin-3-yl | H | H | H | Br | H | nonafluoro-2-butyl | H | CF3 |
| 26-752 | 6-nitropyridin-3-yl | H | H | H | Br | H | nonafluoro-2-butyl | H | CF3 |
| 26-753 | 6-cyanopyridin-3-yl | H | H | H | Br | H | nonafluoro-2-butyl | H | CF3 |
| 26-754 | 5-fluoropyridin-3-yl | H | H | H | Br | H | nonafluoro-2-butyl | H | CF3 |
| 26-755 | 5-chloropyridin-3-yl | H | H | H | Br | H | nonafluoro-2-butyl | H | CF3 |
| 26-756 | 5-bromopyridin-3-yl | H | H | H | Br | H | nonafluoro-2-butyl | H | CF3 |
| 26-757 | 5-iodopyridin-3-yl | H | H | H | Br | H | nonafluoro-2-butyl | H | CF3 |
| 26-758 | 5-(trifluoromethyl)pyridin-3-yl | H | H | H | Br | H | nonafluoro-2-butyl | H | CF3 |
| 26-759 | 5-nitropyridin-3-yl | H | H | H | Br | H | nonafluoro-2-butyl | H | CF3 |
| 26-760 | 5-cyanopyridin-3-yl | H | H | H | Br | H | nonafluoro-2-butyl | H | CF3 |
| 26-761 | 4-fluoropyridin-3-yl | H | H | H | Br | H | nonafluoro-2-butyl | H | CF3 |
| 26-762 | 4-chloropyridin-3-yl | H | H | H | Br | H | nonafluoro-2-butyl | H | CF3 |
| 26-763 | 4-bromopyridin-3-yl | H | H | H | Br | H | nonafluoro-2-butyl | H | CF3 |
| 26-764 | 4-iodopyridin-3-yl | H | H | H | Br | H | nonafluoro-2-butyl | H | CF3 |
| 26-765 | 4-(trifluoromethyl)pyridin-3-yl | H | H | H | Br | H | nonafluoro-2-butyl | H | CF3 |
| 26-766 | 4-nitropyridin-3-yl | H | H | H | Br | H | nonafluoro-2-butyl | H | CF3 |
| 26-767 | 4-cyanopyridin-3-yl | H | H | H | Br | H | nonafluoro-2-butyl | H | CF3 |

**Table 26(5)**

| compound number | Q₁ | R₁ | R₂ | X₃ | Y₁ | Y₂ | Y₃ | Y₄ | Y₅ |
|---|---|---|---|---|---|---|---|---|---|
| 26-768 | 2,6-dichloropyridin-3-yl | H | H | H | Br | H | nonafluoro-2-butyl | H | CF3 |
| 26-769 | pyridin-3-yl N-oxide | H | H | H | Br | H | nonafluoro-2-butyl | H | CF3 |
| 26-770 | pyridin-4-yl | H | H | H | Br | H | nonafluoro-2-butyl | H | CF3 |
| 26-771 | 2-chloropyridin-4-yl | H | H | H | Br | H | nonafluoro-2-butyl | H | CF3 |
| 26-772 | 3-bromopyridin-4-yl | H | H | H | Br | H | nonafluoro-2-butyl | H | CF3 |
| 26-773 | 3,5-dichloropyridin-4-yl | H | H | H | Br | H | nonafluoro-2-butyl | H | CF3 |
| 26-774 | 3-(trifluoromethyl) pyridin-4-yl | H | H | H | Br | H | nonafluoro-2-butyl | H | CF3 |
| 26-775 | 2,6-dicyanopyridin-4-yl | H | H | H | Br | H | nonafluoro-2-butyl | H | CF3 |
| 26-776 | pyridin-4-yl N-oxide | H | H | H | Br | H | nonafluoro-2-butyl | H | CF3 |
| 26-777 | pyridin-2-yl | H | H | H | Br | H | nonafluoro-2-butyl | H | CF3 |
| 26-778 | 3-chloropyridin-2-yl | H | H | H | Br | H | nonafluoro-2-butyl | H | CF3 |
| 26-779 | 4-bromopyridin-2-yl | H | H | H | Br | H | nonafluoro-2-butyl | H | CF3 |
| 26-780 | 5-iodopyridin-2-yl | H | H | H | Br | H | nonafluoro-2-butyl | H | CF3 |
| 26-781 | 6-chloropyridin-2-yl | H | H | H | Br | H | nonafluoro-2-butyl | H | CF3 |
| 26-782 | 4-cyanopyridin-2-yl | H | H | H | Br | H | nonafluoro-2-butyl | H | CF3 |
| 26-783 | phenyl | H | H | H | I | H | heptafluoroisopropyl | H | CF3 |
| 26-784 | 2 -fluorophenyl | H | H | H | I | H | heptafluoroisopropyl | H | CF3 |
| 26-785 | 3-fluorophenyl | H | H | H | I | H | heptafluoroisopropyl | H | CF3 |
| 26-786 | 4-fluorophenyl | H | H | H | I | H | heptafluoroisopropyl | H | CF3 |
| 26-787 | 2-chlorophenyl | H | H | H | I | H | heptafluoroisopropyl | H | CF3 |
| 26-788 | 3-chlorophenyl | H | H | H | I | H | heptafluoroisopropyl | H | CF3 |
| 26-789 | 4-chlorophenyl | H | H | H | I | H | heptafluoroisopropyl | H | CF3 |
| 26-790 | 2-bromophenyl | H | H | H | I | H | heptafluoroisopropyl | H | CF3 |
| 26-791 | 3-bromophenyl | H | H | H | I | H | heptafluoroisopropyl | H | CF3 |
| 26-792 | 4-bromophenyl | H | H | H | I | H | heptafluoroisopropyl | H | CF3 |
| 26-793 | 2-iodophenyl | H | H | H | I | H | heptafluoroisopropyl | H | CF3 |
| 26-794 | 3-iodophenyl | H | H | H | I | H | heptafluoroisopropyl | H | CF3 |
| 26-795 | 4-iodophenyl | H | H | H | I | H | heptafluoroisopropyl | H | CF3 |
| 26-796 | (2-trifluoromethyl) phenyl | H | H | H | I | H | heptafluoroisopropyl | H | CF3 |
| 26-797 | (3-trifluoromethyl) phenyl | H | H | H | I | H | heptafluoroisopropyl | H | CF3 |
| 26-798 | (4-trifluoromethyl) phenyl | H | H | H | I | H | heptafluoroisopropyl | H | CF3 |
| 26-799 | 2-nitrophenyl | H | H | H | I | H | heptafluoroisopropyl | H | CF3 |
| 26-800 | 3-nitrophenyl | H | H | H | I | H | heptafluoroisopropyl | H | CF3 |
| 26-801 | 4-nitrophenyl | H | H | H | I | H | heptafluoroisopropyl | H | CF3 |
| 26-802 | 2-cyanophenyl | H | H | H | I | H | heptafluoroisopropyl | H | CF3 |
| 26-803 | 3-cyanophenyl | H | H | H | I | H | heptafluoroisopropyl | H | CF3 |
| 26-804 | 4-cyanophenyl | H | H | H | I | H | heptafluoroisopropyl | H | CF3 |
| 26-805 | 2,6-difluorophenyl | H | H | H | I | H | heptafluoroisopropyl | H | CF3 |
| 26-806 | 3,4-dichlorophenyl | H | H | H | I | H | heptafluoroisopropyl | H | CF3 |
| 26-807 | 2,4-dichlorophenyl | H | H | H | I | H | heptafluoroisopropyl | H | CF3 |
| 26-808 | 2-chloro-4-fluorophenyl | H | H | H | I | H | heptafluoroisopropyl | H | CF3 |
| 26-809 | 2-chloro-4,5-difluorophenyl | H | H | H | I | H | heptafluoroisopropyl | H | CF3 |
| 26-810 | 4-bromo-2-chlorophenyl | H | H | H | I | H | heptafluoroisopropyl | H | CF3 |
| 26-811 | 2-bromo-4-chlorophenyl | H | H | H | I | H | heptafluoroisopropyl | H | CF3 |
| 26-812 | 2-bromo-4-fluorophenyl | H | H | H | I | H | heptafluoroisopropyl | H | CF3 |
| 26-813 | 2-chloro-4-nitrophenyl | H | H | H | I | H | heptafluoroisopropyl | H | CF3 |
| 26-814 | 3,5-dicyanophenyl | H | H | H | I | H | heptafluoroisopropyl | H | CF3 |
| 26-815 | 4-cyano-2-fluorophenyl | H | H | H | I | H | heptafluoroisopropyl | H | CF3 |
| 26-816 | 2-chloro-4-cyanophenyl | H | H | H | I | H | heptafluoroisopropyl | H | CF3 |
| 26-817 | pyridin-3-yl | H | H | H | I | H | heptafluoroisopropyl | H | CF3 |
| 26-818 | 2-fluoropyridin-3-yl | H | H | H | I | H | heptafluoroisopropyl | H | CF3 |
| 26-819 | 2-chloropyridin-3-yl | H | H | H | I | H | heptafluoroisopropyl | H | CF3 |
| 26-820 | 2-bromopyridin-3-yl | H | H | H | I | H | heptafluoroisopropyl | H | CF3 |
| 26-821 | 2-iodopyridin-3-yl | H | H | H | I | H | heptafluoroisopropyl | H | CF3 |
| 26-822 | 2- (trifluoromethyl) pyridin-3-yl | H | H | H | I | H | heptafluoroisopropyl | H | CF3 |
| 26-823 | 2-nitropyridin-3-yl | H | H | H | I | H | heptafluoroisopropyl | H | CF3 |

**Table 26(6)**

| compound number | Q₁ | R₁ | R₂ | X₃ | Y₁ | Y₂ | Y₃ | Y₄ | Y₅ |
|---|---|---|---|---|---|---|---|---|---|
| 26-824 | 2-cyanopyridin-3-yl | H | H | H | I | H | heptafluoroisopropyl | H | CF3 |
| 26-825 | 6-fluoropyridin-3-yl | H | H | H | I | H | heptafluoroisopropyl | H | CF3 |
| 26-826 | 6-chloropyridin-3-yl | H | H | H | I | H | heptafluoroisopropyl | H | CF3 |
| 26-827 | 6-bromopyridin-3-yl | H | H | H | I | H | heptafluoroisopropyl | H | CF3 |
| 26-828 | 6-iodopyridin-3-yl | H | H | H | I | H | heptafluoroisopropyl | H | CF3 |
| 26-829 | 6- (trifluoromethyl) pyridin-3-yl | H | H | H | I | H | heptafluoroisopropyl | H | CF3 |
| 26-830 | 6-nitropyridin-3-yl | H | H | H | I | H | heptafluoroisopropyl | H | CF3 |
| 26-831 | 6-cyanopyridin-3-yl | H | H | H | I | H | heptafluoroisopropyl | H | CF3 |
| 26-832 | 5-fluoropyridin-3-yl | H | H | H | I | H | heptafluoroisopropyl | H | CF3 |
| 26-833 | 5-chloropyridin-3-yl | H | H | H | I | H | heptafluoroisopropyl | H | CF3 |
| 26-834 | 5-bromopyridin-3-yl | H | H | H | I | H | heptafluoroisopropyl | H | CF3 |
| 26-835 | 5-iodopyridin-3-yl | H | H | H | I | H | heptafluoroisopropyl | H | CF3 |
| 26-836 | 5-(trifluoromethyl)pyridin-3-yl | H | H | H | I | H | heptafluoroisopropyl | H | CF3 |
| 26-837 | 5-nitropyridin-3-yl | H | H | H | I | H | heptafluoroisopropyl | H | CF3 |
| 26-838 | 5-cyanopyridin-3-yl | H | H | H | I | H | heptafluoroisopropyl | H | CF3 |
| 26-839 | 4-fluoropyridin-3-yl | H | H | H | I | H | heptafluoroisopropyl | H | CF3 |
| 26-840 | 4-chloropyridin-3-yl | H | H | H | I | H | heptafluoroisopropyl | H | CF3 |
| 26-841 | 4-bromopyridin-3-yl | H | H | H | I | H | heptafluoroisopropyl | H | CF3 |
| 26-842 | 4-iodopyridin-3-yl | H | H | H | I | H | heptafluoroisopropyl | H | CF3 |
| 26-843 | 4-(trifluoromethyl) pyridin-3-yl | H | H | H | I | H | heptafluoroisopropyl | H | CF3 |
| 26-844 | 4-nitropyridin-3-yl | H | H | H | I | H | heptafluoroisopropyl | H | CF3 |
| 26-845 | 4-cyanopyridin-3-yl | H | H | H | I | H | heptafluoroisopropyl | H | CF3 |
| 26-846 | 2,6-dichloropyridin-3-yl | H | H | H | I | H | heptafluoroisopropyl | H | CF3 |
| 26-847 | pyridin-3-yl N-oxide | H | H | H | I | H | heptafluoroisopropyl | H | CF3 |
| 26-848 | pyridin-4-yl | H | H | H | I | H | heptafluoroisopropyl | H | CF3 |
| 26-849 | 2 -chloropyridin-4-yl | H | H | H | I | H | heptafluoroisopropyl | H | CF3 |
| 26-850 | 3-bromopyridin-4-yl | H | H | H | I | H | heptafluoroisopropyl | H | CF3 |
| 26-851 | 3,5-dichloropyridin-4-yl | H | H | H | I | H | heptafluoroisopropyl | H | CF3 |
| 26-852 | 3-(trifluoromethyl) pyridin-4-yl | H | H | H | I | H | heptafluoroisopropyl | H | CF3 |
| 26-853 | 2,6-dicyanopyridin-4-yl | H | H | H | I | H | heptafluoroisopropyl | H | CF3 |
| 26-854 | pyridin-4-yl N-oxide | H | H | H | I | H | heptafluoroisopropyl | H | CF3 |
| 26-855 | pyridin-2-yl | H | H | H | I | H | heptafluoroisopropyl | H | CF3 |
| 26-856 | 3-chloropyridin-2-yl | H | H | H | I | H | heptafluoroisopropyl | H | CF3 |
| 26-857 | 4-bromopyridin-2-yl | H | H | H | I | H | heptafluoroisopropyl | H | CF3 |
| 26-858 | 5-iodopyridin-2-yl | H | H | H | I | H | heptafluoroisopropyl | H | CF3 |
| 26-859 | 6-chloropyridin-2-yl | H | H | H | I | H | heptafluoroisopropyl | H | CF3 |
| 26-860 | 4-cyanopyridin-2-yl | H | H | H | I | H | heptafluoroisopropyl | H | CF3 |
| 26-861 | phenyl | H | H | H | I | H | nonafluoro-2-butyl | H | CF3 |
| 26-862 | 2-fluorophenyl | H | H | H | I | H | nonafluoro-2-butyl | H | CF3 |
| 26-863 | 3-fluorophenyl | H | H | H | I | H | nonafluoro-2-butyl | H | CF3 |
| 26-864 | 4-fluorophenyl | H | H | H | I | H | nonafluoro-2-butyl | H | CF3 |
| 26-865 | 2-chlorophenyl | H | H | H | I | H | nonafluoro-2-butyl | H | CF3 |
| 26-866 | 3-chlorophenyl | H | H | H | I | H | nonafluoro-2-butyl | H | CF3 |
| 26-867 | 4-chlorophenyl | H | H | H | I | H | nonafluoro-2-butyl | H | CF3 |
| 26-868 | 2-bromophenyl | H | H | H | I | H | nonafluoro-2-butyl | H | CF3 |
| 26-869 | 3-bromophenyl | H | H | H | I | H | nonafluoro-2-butyl | H | CF3 |
| 26-870 | 4-bromophenyl | H | H | H | I | H | nonafluoro-2-butyl | H | CF3 |
| 26-871 | 2-iodophenyl | H | H | H | I | H | nonafluoro-2-butyl | H | CF3 |
| 26-872 | 3-iodophenyl | H | H | H | I | H | nonafluoro-2-butyl | H | CF3 |
| 26-873 | 4-iodophenyl | H | H | H | I | H | nonafluoro-2-butyl | H | CF3 |
| 26-874 | (2-trifluoromethyl) phenyl | H | H | H | I | H | nonafluoro-2-butyl | H | CF3 |
| 26-875 | (3-trifluoromethyl) phenyl | H | H | H | I | H | nonafluoro-2-butyl | H | CF3 |
| 26-876 | (4-trifluoromethyl) phenyl | H | H | H | I | H | nonafluoro-2-butyl | H | CF3 |
| 26-877 | 2-nitrophenyl | H | H | H | I | H | nonafluoro-2-butyl | H | CF3 |
| 26-878 | 3-nitrophenyl | H | H | H | I | H | nonafluoro-2-butyl | H | CF3 |
| 26-879 | 4-nitrophenyl | H | H | H | I | H | nonafluoro-2-butyl | H | CF3 |

**Table 26(7)**

| compound number | Q₁ | R₁ | R₂ | X₃ | Y₁ | Y₂ | Y₃ | Y₄ | Y₅ |
|---|---|---|---|---|---|---|---|---|---|
| 26-880 | 2-cyanophenyl | H | H | H | I | H | nonafluoro-2-butyl | H | CF3 |
| 26-881 | 3-cyanophenyl | H | H | H | I | H | nonafluoro-2-butyl | H | CF3 |
| 26-882 | 4-cyanophenyl | H | H | H | I | H | nonafluoro-2-butyl | H | CF3 |
| 26-883 | 2,6-difluorophenyl | H | H | H | I | H | nonafluoro-2-butyl | H | CF3 |
| 26-884 | 3,4-dichlorophenyl | H | H | H | I | H | nonafluoro-2-butyl | H | CF3 |
| 26-885 | 2,4-dichlorophenyl | H | H | H | I | H | nonafluoro-2-butyl | H | CF3 |
| 26-886 | 2-chloro-4-fluorophenyl | H | H | H | I | H | nonafluoro-2-butyl | H | CF3 |
| 26-887 | 2-chloro-4,5-difluorophenyl | H | H | H | I | H | nonafluoro-2-butyl | H | CF3 |
| 26-888 | 4-bromo-2-chlorophenyl | H | H | H | I | H | nonafluoro-2-butyl | H | CF3 |
| 26-889 | 2-bromo-4-chlorophenyl | H | H | H | I | H | nonafluoro-2-butyl | H | CF3 |
| 26-890 | 2-bromo-4-fluorophenyl | H | H | H | I | H | nonafluoro-2-butyl | H | CF3 |
| 26-891 | 2-chloro-4-nitrophenyl | H | H | H | I | H | nonafluoro-2-butyl | H | CF3 |
| 26-892 | 3,5-dicyanophenyl | H | H | H | I | H | nonafluoro-2-butyl | H | CF3 |
| 26-893 | 4-cyano-2-fluorophenyl | H | H | H | I | H | nonafluoro-2-butyl | H | CF3 |
| 26-894 | 2-chloro-4-cyanophenyl | H | H | H | I | H | nonafluoro-2-butyl | H | CF3 |
| 26-895 | pyridin-3-yl | H | H | H | I | H | nonafluoro-2-butyl | H | CF3 |
| 26-896 | 2-fluoropyridin-3-yl | H | H | H | I | H | nonafluoro-2-butyl | H | CF3 |
| 26-897 | 2-chloropyridin-3-yl | H | H | H | I | H | nonafluoro-2-butyl | H | CF3 |
| 26-898 | 2-bromopyridin-3-yl | H | H | H | I | H | nonafluoro-2-butyl | H | CF3 |
| 26-899 | 2-iodopyridin-3-yl | H | H | H | I | H | nonafluoro-2-butyl | H | CF3 |
| 26-900 | 2-(trifluoromethyl) pyridin-3-yl | H | H | H | I | H | nonafluoro-2-butyl | H | CF3 |
| 26-901 | 2-nitropyridin-3-yl | H | H | H | I | H | nonafluoro-2-butyl | H | CF3 |
| 26-902 | 2-cyanopyridin-3-yl | H | H | H | I | H | nonafluoro-2-butyl | H | CF3 |
| 26-903 | 6-fluoropyridin-3-yl | H | H | H | I | H | nonafluoro-2-butyl | H | CF3 |
| 26-904 | 6-chloropyridin-3-yl | H | H | H | I | H | nonafluoro-2-butyl | H | CF3 |
| 26-905 | 6-bromopyridin-3-yl | H | H | H | I | H | nonafluoro-2-butyl | H | CF3 |
| 26-906 | 6-iodopyridin-3-yl | H | H | H | I | H | nonafluoro-2-butyl | H | CF3 |
| 26-907 | 6-(trifluoromethyl) pyridin-3-yl | H | H | H | I | H | nonafluoro-2-butyl | H | CF3 |
| 26-908 | 6-nitropyridin-3-yl | H | H | H | I | H | nonafluoro-2-butyl | H | CF3 |
| 26-909 | 6-cyanopyridin-3-yl | H | H | H | I | H | nonafluoro-2-butyl | H | CF3 |
| 26-910 | 5-fluoropyridin-3-yl | H | H | H | I | H | nonafluoro-2-butyl | H | CF3 |
| 26-911 | 5-chloropyridin-3-yl | H | H | H | I | H | nonafluoro-2-butyl | H | CF3 |
| 26-912 | 5-bromopyridin-3-yl | H | H | H | I | H | nonafluoro-2-butyl | H | CF3 |
| 26-913 | 5-iodopyridin-3-yl | H | H | H | I | H | nonafluoro-2-butyl | H | CF3 |
| 26-914 | 5-(trifluoromethyl)pyridin-3-yl | H | H | H | I | H | nonafluoro-2-butyl | H | CF3 |
| 26-915 | 5-nitropyridin-3-yl | H | H | H | I | H | nonafluoro-2-butyl | H | CF3 |
| 26-916 | 5-cyanopyridin-3-yl | H | H | H | I | H | nonafluoro-2-butyl | H | CF3 |
| 26-917 | 4-fluoropyridin-3-yl | H | H | H | I | H | nonafluoro-2-butyl | H | CF3 |
| 26-918 | 4-chloropyridin-3-yl | H | H | H | I | H | nonafluoro-2-butyl | H | CF3 |
| 26-919 | 4-bromopyridin-3-yl | H | H | H | I | H | nonafluoro-2-butyl | H | CF3 |
| 26-920 | 4-iodopyridin-3-yl | H | H | H | I | H | nonafluoro-2-butyl | H | CF3 |
| 26-921 | 4-(trifluoromethyl)pyridin-3-yl | H | H | H | I | H | nonafluoro-2-butyl | H | CF3 |
| 26-922 | 4-nitropyridin-3-yl | H | H | H | I | H | nonafluoro-2-butyl | H | CF3 |
| 26-923 | 4-cyanopyridin-3-yl | H | H | H | I | H | nonafluoro-2-butyl | H | CF3 |
| 26-924 | 2,6-dichloropyridin-3-yl | H | H | H | I | H | nonafluoro-2-butyl | H | CF3 |
| 26-925 | pyridin-3-yl N-oxide | H | H | H | I | H | nonafluoro-2-butyl | H | CF3 |
| 26-926 | pyridin-4-yl | H | H | H | I | H | nonafluoro-2-butyl | H | CF3 |
| 26-927 | 2-chloropyridin-4-yl | H | H | H | I | H | nonafluoro-2-butyl | H | CF3 |
| 26-928 | 3-bromopyridin-4-yl | H | H | H | I | H | nonafluoro-2-butyl | H | CF3 |
| 26-929 | 3,5-dichloropyridin-4-yl | H | H | H | I | H | nonafluoro-2-butyl | H | CF3 |
| 26-930 | 3-(trifluoromethyl) pyridin-4-yl | H | H | H | I | H | nonafluoro-2-butyl | H | CF3 |
| 26-931 | 2,6-dicyanopyridin-4-yl | H | H | H | I | H | nonafluoro-2-butyl | H | CF3 |
| 26-932 | pyridin-4-yl N-oxide | H | H | H | I | H | nonafluoro-2-butyl | H | CF3 |
| 26-933 | pyridin-2-yl | H | H | H | I | H | nonafluoro-2-butyl | H | CF3 |
| 26-934 | 3-chloropyridin-2-yl | H | H | H | I | H | nonafluoro-2-butyl | H | CF3 |
| 26-935 | 4-bromopyridin-2-yl | H | H | H | I | H | nonafluoro-2-butyl | H | CF3 |

**Table 26(8)**

| compound number | Q₁ | R₁ | R₂ | X₃ | Y₁ | Y₂ | Y₃ | Y₄ | Y₅ |
|---|---|---|---|---|---|---|---|---|---|
| 26-936 | 5-iodopyridin-2-yl | H | H | H | I | H | nonafluoro-2-butyl | H | CF3 |
| 26-937 | 6-chloropyridin-2-yl | H | H | H | I | H | nonafluoro-2-butyl | H | CF3 |
| 26-938 | 4-cyanopyridin-2-yl | H | H | H | I | H | nonafluoro-2-butyl | H | CF3 |
| 26-939 | phenyl | H | H | H | OCF3 | H | heptafluoroisopropyl | H | OCF3 |
| 26-946 | 2-bromophenyl | H | H | H | OCF3 | H | heptafluoroisopropyl | H | OC2F5 |
| 26-950 | 3-iodophenyl | H | H | H | OCF3 | H | pentafluoroethyl | H | OCF3 |
| 26-951 | 4-iodophenyl | H | H | H | OCF3 | H | pentafluoroethyl | H | OCF3 |
| 26-954 | 3-cyanophenyl | H | H | H | OCF3 | H | heptafluoroisopropyl | H | OCF3 |
| 26-955 | 4-cyanophenyl | H | H | H | OCF3 | H | heptafluoroisopropyl | H | OCF3 |
| 26-960 | 2-chloro-4,5-difluorophenyl | H | H | H | OC2F5 | H | heptafluoroisopropyl | H | OC2F5 |
| 26-963 | 2-bromo-4-fluorophenyl | H | H | H | OC2F5 | H | heptafluoroisopropyl | H | OCF3 |
| 26-964 | 2-chloro-4-nitrophenyl | H | H | Cl | OCF3 | H | heptafluoroisopropyl | H | OCF3 |
| 26-969 | 2-fluoropyridin-3-yl | H | H | H | OCF3 | H | pentafluoroethyl | H | OCF3 |
| 26-970 | 2-chloropyridin-3-yl | H | H | H | OCF3 | H | heptafluoroisopropyl | H | OCF3 |
| 26-979 | 5-cyanopyridin-3-yl | H | H | H | OC2F5 | H | heptafluoroisopropyl | H | OC2F5 |
| 26-981 | 4-cyanopyridin-3-yl | H | H | H | OCF3 | H | heptafluoroisopropyl | H | OC2F5 |
| 26-994 | 4-cyanopyridin-2-yl | H | H | H | OCF3 | H | pentafluoroethyl | H | OCF3 |
| 26-995 | phenyl | H | H | H | OCF3 | H | nonafluoro-2-butyl | H | OCF3 |
| 26-1002 | 2-bromophenyl | H | H | H | OC2F5 | H | nonafluoro-2-butyl | H | OCF3 |
| 26-1008 | (2-trifluoromethyl) phenyl | H | H | H | OCF3 | H | nonafluoro-2-butyl | H | OC2F5 |
| 26-1010 | 3-cyanophenyl | H | H | H | OCF3 | H | nonafluoro-2-butyl | H | OCF3 |
| 26-1011 | 4-cyanophenyl | H | H | H | OCF3 | H | nonafluoro-2-butyl | H | OCF3 |
| 26-1017 | 4-bromo-2-chlorophenyl | H | H | H | OC2F5 | H | nonafluoro-2-butyl | H | OC2F5 |
| 26-1026 | 2-chloropyridin-3-yl | H | H | H | OCF3 | H | nonafluoro-2-butyl | H | OCF3 |
| 26-1033 | 6-cyanopyridin-3-yl | H | H | H | OC2F5 | H | nonafluoro-2-butyl | H | OCF3 |
| 26-1035 | 5-cyanopyridin-3-yl | H | H | H | OCF3 | H | nonafluoro-2-butyl | H | OC2F5 |
| 26-1037 | 4-cyanopyridin-3-yl | H | H | H | OC2F5 | H | nonafluoro-2-butyl | H | OC2F5 |
| 26-1051 | phenyl | H | H | H | CF3 | H | heptafluoroisopropyl | H | OCF3 |
| 26-1058 | 2-bromophenyl | H | H | H | CF3 | H | heptafluoroisopropyl | H | OC2F5 |
| 26-1062 | 3-iodophenyl | H | H | H | CF3 | H | pentafluoroethyl | H | OCF3 |
| 26-1063 | 4-iodophenyl | H | H | H | CF3 | H | pentafluoroethyl | H | OCF3 |
| 26-1064 | (2-trifluoromethyl) phenyl | H | H | H | C2F5 | H | heptafluoroisopropyl | H | OC2F5 |
| 26-1065 | 3-nitrophenyl | H | H | H | C2F5 | H | heptafluoroisopropyl | H | OCF3 |
| 26-1066 | 3-cyanophenyl | H | H | H | CF3 | H | heptafluoroisopropyl | H | OCF3 |
| 26-1067 | 4-cyanophenyl | H | H | H | CF3 | H | heptafluoroisopropyl | H | OCF3 |
| 26-1074 | 2-bromo-4-chlorophenyl | H | H | H | n-C3F7 | H | heptafluoroisopropyl | H | OCF3 |
| 26-1078 | 4-cyano-2-fluorophenyl | H | H | H | i-C3F7 | H | heptafluoroisopropyl | H | OCF3 |
| 26-1081 | 2-fluoropyridin-3-yl | H | H | H | CF3 | H | pentafluoroethyl | H | OCF3 |
| 26-1082 | 2-chloropyridin-3-yl | H | H | H | CF3 | H | heptafluoroisopropyl | H | OCF3 |
| 26-1089 | 6-cyanopyridin-3-yl | H | H | H | n-C3F7 | H | heptafluoroisopropyl | H | OC2F5 |
| 26-1091 | 5-cyanopyridin-3-yl | H | H | H | i-C3F7 | H | heptafluoroisopropyl | H | OC2F5 |
| 26-1106 | 4-cyanopyridin-2-yl | H | H | H | CF3 | H | pentafluoroethyl | H | OCF3 |
| 26-1107 | phenyl | H | H | H | CF3 | H | nonafluoro-2-butyl | H | OCF3 |
| 26-1118 | 3-iodophenyl | H | H | H | CF3 | H | nonafluoro-2-butyl | H | OC2F5 |
| 26-1121 | 3-nitrophenyl | H | H | H | C2F5 | H | nonafluoro-2-butyl | H | OCF3 |
| 26-1122 | 3-cyanophenyl | H | H | H | CF3 | H | nonafluoro-2-butyl | H | OCF3 |
| 26-1123 | 4-cyanophenyl | H | H | H | CF3 | H | nonafluoro-2-butyl | H | OCF3 |
| 26-1129 | 4-bromo-2-chlorophenyl | H | H | H | i-C3F7 | H | nonafluoro-2-butyl | H | OCF3 |
| 26-1132 | 2-chloro-4-nitrophenyl | H | H | H | n-C3F7 | H | nonafluoro-2-butyl | H | OCF3 |
| 26-1138 | 2-chloropyridin-3-yl | H | H | H | CF3 | H | nonafluoro-2-butyl | H | OCF3 |
| 26-1147 | 5-cyanopyridin-3-yl | H | H | H | C2F5 | H | nonafluoro-2-butyl | H | OC2F5 |
| 26-1149 | 4-cyanopyridin-3-yl | H | H | H | n-C3F7 | H | nonafluoro-2-butyl | H | OC2F5 |
| 26-1160 | 5-iodopyridin-2-yl | H | H | H | i-C3F7 | H | nonafluoro-2-butyl | H | OC2F5 |
| 26-1163 | phenyl | H | H | H | CF3 | H | heptafluoroisopropyl | H | CF3 |
| 26-1174 | 3-iodophenyl | H | H | H | CF3 | H | pentafluoroethyl | H | CF3 |
| 26-1175 | 4-iodophenyl | H | H | H | CF3 | H | pentafluoroethyl | H | CF3 |

**Table 26(9)**

| compound number | Q₁ | R₁ | R₂ | X₃ | Y₁ | Y₂ | Y₃ | Y₄ | Y₅ |
|---|---|---|---|---|---|---|---|---|---|
| 26-1177 | (3-trifluoromethyl) phenyl | H | H | H | C2F5 | H | heptafluoroisopropyl | H | CF3 |
| 26-1183 | 3-cyanophenyl | H | H | H | CF3 | H | heptafluoroisopropyl | H | CF3 |
| 26-1184 | 4-cyanophenyl | H | H | H | CF3 | H | heptafluoroisopropyl | H | CF3 |
| 26-1190 | 4-bromo-2-chlorophenyl | H | H | H | n-C3F7 | H | heptafluoroisopropyl | H | CF3 |
| 26-1195 | 4-cyano-2-fluorophenyl | H | H | H | i-C3F7 | H | heptafluoroisopropyl | H | CF3 |
| 26-1198 | 2-fluoropyridin-3-yl | H | H | H | CF3 | H | pentafluoroethyl | H | CF3 |
| 26-1199 | 2-chloropyridin-3-yl | H | H | H | CF3 | H | heptafluoroisopropyl | H | CF3 |
| 26-1200 | 2-bromopyridin-3-yl | H | H | H | C2F5 | H | heptafluoroisopropyl | H | C2F5 |
| 26-1203 | 2-nitropyridin-3-yl | H | H | Cl | CF3 | H | heptafluoroisopropyl | H | CF3 |
| 26-1211 | 6-cyanopyridin-3-yl | H | H | H | C2F5 | H | heptafluoroisopropyl | H | n-C3F7 |
| 26-1215 | 4-cyanopyridin-3-yl | H | H | H | C2F5 | H | heptafluoroisopropyl | H | i-C3F7 |
| 26-1230 | 4-cyanopyridin-2-yl | H | H | H | CF3 | H | pentafluoroethyl | H | CF3 |
| 26-1231 | phenyl | H | H | H | CF3 | H | nonafluoro-2-butyl | H | CF3 |
| 26-1243 | 4-iodophenyl | H | H | H | C2F5 | H | nonafluoro-2-butyl | H | CF3 |
| 26-1251 | 3-cyanophenyl | H | H | H | CF3 | H | nonafluoro-2-butyl | H | CF3 |
| 26-1252 | 4-cyanophenyl | H | H | H | CF3 | H | nonafluoro-2-butyl | H | CF3 |
| 26-1258 | 4-bromo-2-chlorophenyl | H | H | H | n-C3F7 | H | nonafluoro-2-butyl | H | CF3 |
| 26-1263 | 4-cyano-2-fluorophenyl | H | H | H | i-C3F7 | H | nonafluoro-2-butyl | H | CF3 |
| 26-1267 | 2-chloropyridin-3-yl | H | H | H | CF3 | H | nonafluoro-2-butyl | H | CF3 |
| 26-1268 | 2-bromopyridin-3-yl | H | H | H | C2F5 | H | nonafluoro-2-butyl | H | C2F5 |
| 26-1279 | 6-cyanopyridin-3-yl | H | H | H | C2F5 | H | nonafluoro-2-butyl | H | n-C3F7 |
| 26-1283 | 4-cyanopyridin-3-yl | H | H | H | C2F5 | H | nonafluoro-2-butyl | H | i-C3F7 |

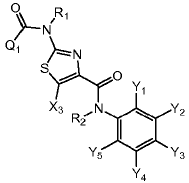

**Table27(1)**

| compound number | Q₁ | R₁ | R₂ | X₃ | Y₁ | Y₂ | Y₃ | Y₄ | Y₅ |
|---|---|---|---|---|---|---|---|---|---|
| 27-1 | phenyl | Me | H | H | Cl | H | heptafluoroisopropyl | H | OCF3 |
| 27-21 | 3-cyanophenyl | Me | H | H | Cl | H | heptafluoroisopropyl | H | OCF3 |
| 27-22 | 4-cyanophenyl | Me | H | H | Cl | H | heptafluoroisopropyl | H | OCF3 |
| 27-37 | 2-chloropyridin-3-yl | Me | H | H | Cl | H | heptafluoroisopropyl | H | OCF3 |
| 27-79 | phenyl | Me | H | H | Cl | H | nonafluoro-2-butyl | H | OCF3 |
| 27-99 | 3-cyanophenyl | Me | H | H | Cl | H | nonafluoro-2-butyl | H | OCF3 |
| 27-100 | 4-cyanophenyl | Me | H | H | Cl | H | nonafluoro-2-butyl | H | OCF3 |
| 27-115 | 2-chloropyridin-3-yl | Me | H | H | Cl | H | nonafluoro-2-butyl | H | OCF3 |
| 27-157 | phenyl | Me | H | H | Br | H | heptafluoroisopropyl | H | OCF3 |
| 27-177 | 3-cyanophenyl | Me | H | H | Br | H | heptafluoroisopropyl | H | OCF3 |
| 27-178 | 4-cyanophenyl | Me | H | H | Br | H | heptafluoroisopropyl | H | OCF3 |
| 27-193 | 2-chloropyridin-3-yl | Me | H | H | Br | H | heptafluoroisopropyl | H | OCF3 |
| 27-235 | phenyl | Me | H | H | Br | H | nonafluoro-2-butyl | H | OCF3 |
| 27-255 | 3-cyanophenyl | Me | H | H | Br | H | nonafluoro-2-butyl | H | OCF3 |
| 27-256 | 4-cyanophenyl | Me | H | H | Br | H | nonafluoro-2-butyl | H | OCF3 |
| 27-271 | 2-chloropyridin-3-yl | Me | H | H | Br | H | nonafluoro-2-butyl | H | OCF3 |
| 27-313 | phenyl | Me | H | H | I | H | heptafluoroisopropyl | H | OCF3 |
| 27-333 | 3-cyanophenyl | Me | H | H | I | H | heptafluoroisopropyl | H | OCF3 |
| 27-334 | 4-cyanophenyl | Me | H | H | I | H | heptafluoroisopropyl | H | OCF3 |
| 27-349 | 2-chloropyridin-3-yl | Me | H | H | I | H | heptafluoroisopropyl | H | OCF3 |
| 27-391 | phenyl | Me | H | H | I | H | nonafluoro-2-butyl | H | OCF3 |
| 27-411 | 3-cyanophenyl | Me | H | H | I | H | nonafluoro-2-butyl | H | OCF3 |
| 27-412 | 4-cyanophenyl | Me | H | H | I | H | nonafluoro-2-butyl | H | OCF3 |
| 27-427 | 2-chloropyridin-3-yl | Me | H | H | I | H | nonafluoro-2-butyl | H | OCF3 |
| 27-469 | phenyl | Me | H | H | F | H | heptafluoroisopropyl | H | CF3 |
| 27-470 | phenyl | Me | H | H | Cl | H | heptafluoroisopropyl | H | CF3 |
| 27-484 | (3-trifluoromethyl)phenyl | Et | H | H | Cl | H | heptafluoroisopropyl | H | CF3 |
| 27-489 | 2-cyanophenyl | Me | H | H | Cl | H | pentafluoroethyl | H | C2F5 |
| 27-490 | 3-cyanophenyl | Me | H | H | Cl | H | heptafluoroisopropyl | H | CF3 |
| 27-491 | 4-cyanophenyl | Me | H | H | Cl | H | heptafluoroisopropyl | H | CF3 |
| 27-494 | 2,4-dichlorophenyl | Me | H | H | Cl | H | heptafluoroisopropyl | H | C2F5 |
| 27-495 | 2-chloro-4-fluorophenyl | n-Pr | H | H | Cl | H | heptafluoroisopropyl | H | CF3 |
| 27-498 | 2-bromo-4-chlorophenyl | Me | H | H | Cl | H | heptafluoroisopropyl | H | n-C3F7 |
| 27-506 | 2-chloropyridin-3-yl | Me | H | H | Cl | H | heptafluoroisopropyl | H | CF3 |
| 27-510 | 2-nitropyridin-3-yl | Me | H | Cl | Cl | H | heptafluoroisopropyl | H | CF3 |
| 27-512 | 6-fluoropyridin-3-yl | i-Pr | H | H | Cl | H | heptafluoroisopropyl | H | CF3 |
| 27-519 | 5-fluoropyridin-3-yl | Me | H | H | Cl | H | heptafluoroisopropyl | H | C2F5 |
| 27-521 | 5-bromopyridin-3-yl | Me | H | H | Cl | H | heptafluoroisopropyl | H | n-C3F7 |
| 27-522 | 5-iodopyridin-3-yl | Me | H | H | Cl | H | heptafluoroisopropyl | H | i-C3F7 |
| 27-528 | 4-bromopyridin-3-yl | CH2CH=CH2 | H | H | Cl | H | heptafluoroisopropyl | H | n-C3F7 |
| 27-529 | 4-iodopyridin-3-yl | Me | H | H | Cl | H | heptafluoroisopropyl | H | i-C3F7 |
| 27-535 | pyridin-4-yl | CN | H | H | Cl | H | heptafluoroisopropyl | H | CF3 |
| 27-548 | phenyl | Me | H | H | F | H | nonafluoro-2-butyl | H | CF3 |
| 27-549 | phenyl | Me | H | H | Cl | H | nonafluoro-2-butyl | H | CF3 |
| 27-557 | 3-bromophenyl | CH2C≡CH | H | H | Cl | H | nonafluoro-2-butyl | H | CF3 |
| 27-562 | (2-trifluoromethyl)phenyl | Me | H | H | Cl | H | nonafluoro-2-butyl | H | C2F5 |
| 27-564 | (4-trifluoromethyl)phenyl | NH2 | H | H | Cl | H | nonafluoro-2-butyl | H | CF3 |
| 27-569 | 3-cyanophenyl | Me | H | H | Cl | H | nonafluoro-2-butyl | H | CF3 |
| 27-570 | 4-cyanophenyl | Me | H | H | Cl | H | nonafluoro-2-butyl | H | CF3 |
| 27-577 | 2-bromo-4-chlorophenyl | C(O)OMe | H | H | Cl | H | nonafluoro-2-butyl | H | CF3 |
| 27-579 | 2-chloro-4-nitrophenyl | Me | H | H | Cl | H | nonafluoro-2-butyl | H | i-C3F7 |
| 27-585 | 2-chloropyridin-3-yl | Me | H | H | Cl | H | nonafluoro-2-butyl | H | CF3 |
| 27-598 | 5-fluoropyridin-3-yl | C(O)OEt | H | H | Cl | H | nonafluoro-2-butyl | H | C2F5 |
| 27-600 | 5-bromopyridin-3-yl | Me | H | H | Cl | H | nonafluoro-2-butyl | H | n-C3F7 |
| 27-601 | 5-iodopyridin-3-yl | Me | H | H | Cl | H | nonafluoro-2-butyl | H | i-C3F7 |

**Table 27(2)**

| compound number | Q, | R₁ | R₂ | X₃ | Y₁ | Y₂ | Y₃ | Y₄ | Y₅ |
|---|---|---|---|---|---|---|---|---|---|
| 27-605 | 4-fluoropyridin-3-yl | Me | H | H | Cl | H | nonafluoro-2-butyl | H | C2F5 |
| 27-607 | 4-bromopyridin-3-yl | C(O)C(O)Me | H | H | Cl | H | nonafluoro-2-butyl | H | CF3 |
| 27-609 | 4-(trifluoromethyl)pyridin-3-yl | Me | H | H | Cl | H | nonafluoro-2-butyl | H | n-C3F7 |
| 27-610 | 4-nitropyridin-3-yl | C(O)C(O)Et | H | H | Cl | H | nonafluoro-2-butyl | H | i-C3F7 |
| 27-624 | 5-iodopyridin-2-yl | C(O)Me | H | H | Cl | H | nonafluoro-2-butyl | H | CF3 |
| 27-626 | 4-cyanopyridin-2-yl | C(O)Et | H | H | Cl | H | nonafluoro-2-butyl | H | C2F5 |
| 27-627 | phenyl | Me | H | H | Br | H | heptafluoroisopropyl | H | CF3 |
| 27-628 | 2-fluorophenyl | Me | H | H | Br | H | heptafluoroisopropyl | H | CF3 |
| 27-629 | 3-fluorophenyl | Me | H | H | Br | H | heptafluoroisopropyl | H | CF3 |
| 27-630 | 4-fluorophenyl | Me | H | H | Br | H | heptafluoroisopropyl | H | CF3 |
| 27-631 | 2-chlorophenyl | Me | H | H | Br | H | heptafluoroisopropyl | H | CF3 |
| 27-632 | 3-chlorophenyl | Me | H | H | Br | H | heptafluoroisopropyl | H | CF3 |
| 27-633 | 4-chlorophenyl | Me | H | H | Br | H | heptafluoroisopropyl | H | CF3 |
| 27-634 | 2-bromophenyl | Me | H | H | Br | H | heptafluoroisopropyl | H | CF3 |
| 27-635 | 3-bromophenyl | Me | H | H | Br | H | heptafluoroisopropyl | H | CF3 |
| 27-636 | 4-bromophenyl | Me | H | H | Br | H | heptafluoroisopropyl | H | CF3 |
| 27-637 | 2-iodophenyl | Me | H | H | Br | H | heptafluoroisopropyl | H | CF3 |
| 27-638 | 3-iodophenyl | Me | H | H | Br | H | heptafluoroisopropyl | H | CF3 |
| 27-639 | 4-iodophenyl | Me | H | H | Br | H | heptafluoroisopropyl | H | CF3 |
| 27-640 | (2-trifluoromethyl)phenyl | Me | H | H | Br | H | heptafluoroisopropyl | H | CF3 |
| 27-641 | (3-trifluoromethyl)phenyl | S(O)2Me | H | H | Br | H | heptafluoroisopropyl | H | CF3 |
| 27-642 | (4-trifluoromethyl)phenyl | Me | H | H | Br | H | heptafluoroisopropyl | H | CF3 |
| 27-643 | 2-nitrophenyl | Me | H | H | Br | H | heptafluoroisopropyl | H | CF3 |
| 27-644 | 3-nitrophenyl | Me | H | H | Br | H | heptafluoroisopropyl | H | CF3 |
| 27-645 | 4-nitrophenyl | Me | H | H | Br | H | heptafluoroisopropyl | H | CF3 |
| 27-646 | 2-cyanophenyl | Me | H | H | Br | H | heptafluoroisopropyl | H | CF3 |
| 27-647 | 3-cyanophenyl | Me | H | H | Br | H | heptafluoroisopropyl | H | CF3 |
| 27-648 | 4-cyanophenyl | Me | H | H | Br | H | heptafluoroisopropyl | H | CF3 |
| 27-649 | 2,6-difluorophenyl | Me | H | H | Br | H | heptafluoroisopropyl | H | CF3 |
| 27-650 | 3,4-dichlorophenyl | Me | H | H | Br | H | heptafluoroisopropyl | H | CF3 |
| 27-651 | 2,4-dichlorophenyl | Me | H | H | Br | H | heptafluoroisopropyl | H | CF3 |
| 27-652 | 2-chloro-4-fluorophenyl | Me | H | H | Br | H | heptafluoroisopropyl | H | CF3 |
| 27-653 | 2-chloro-4,5-difluorophenyl | Me | H | H | Br | H | heptafluoroisopropyl | H | CF3 |
| 27-654 | 4-bromo-2-chlorophenyl | Me | H | H | Br | H | heptafluoroisopropyl | H | CF3 |
| 27-655 | 2-bromo-4-chlorophenyl | Me | H | H | Br | H | heptafluoroisopropyl | H | CF3 |
| 27-656 | 2-bromo-4-fluorophenyl | Me | H | H | Br | H | heptafluoroisopropyl | H | CF3 |
| 27-657 | 2-chloro-4-nitrophenyl | Me | H | H | Br | H | heptafluoroisopropyl | H | CF3 |
| 27-658 | 3,5-dicyanophenyl | Me | H | H | Br | H | heptafluoroisopropyl | H | CF3 |
| 27-659 | 4-cyano-2-fluorophenyl | S(O)2Et | H | H | Br | H | heptafluoroisopropyl | H | CF3 |
| 27-660 | 2-chloro-4-cyanophenyl | Me | H | H | Br | H | heptafluoroisopropyl | H | CF3 |
| 27-661 | pyridin-3-yl | Me | H | H | Br | H | heptafluoroisopropyl | H | CF3 |
| 27-662 | 2-fluoropyridin-3-yl | Me | H | H | Br | H | heptafluoroisopropyl | H | CF3 |
| 27-663 | 2-chloropyridin-3-yl | Me | H | H | Br | H | heptafluoroisopropyl | H | CF3 |
| 27-664 | 2-bromopyridin-3-yl | Me | H | H | Br | H | heptafluoroisopropyl | H | CF3 |
| 27-665 | 2-iodopyridin-3-yl | Me | H | H | Br | H | heptafluoroisopropyl | H | CF3 |
| 27-666 | 2-(trifluoromethyl)pyridin-3-yl | Me | H | H | Br | H | heptafluoroisopropyl | H | CF3 |
| 27-667 | 2-nitropyridin-3-yl | Me | H | H | Br | H | heptafluoroisopropyl | H | CF3 |
| 27-668 | 2-cyanopyridin-3-yl | Me | H | H | Br | H | heptafluoroisopropyl | H | CF3 |
| 27-669 | 6-fluoropyridin-3-yl | Me | H | H | Br | H | heptafluoroisopropyl | H | CF3 |
| 27-670 | 6-chloropyridin-3-yl | Me | H | H | Br | H | heptafluoroisopropyl | H | CF3 |
| 27-671 | 6-bromopyridin-3-yl | Me | H | H | Br | H | heptafluoroisopropyl | H | CF3 |
| 27-672 | 6-iodopyridin-3-yl | Me | H | H | Br | H | heptafluoroisopropyl | H | CF3 |
| 27-673 | 6-(trifluoromethyl)pyridin-3-yl | Me | H | H | Br | H | heptafluoroisopropyl | H | CF3 |
| 27-674 | 6-nitropyridin-3-yl | CH2Ph | H | H | Br | H | heptafluoroisopropyl | H | CF3 |
| 27-675 | 6-cyanopyridin-3-yl | Me | H | H | Br | H | heptafluoroisopropyl | H | CF3 |
| 27-676 | 5-fluoropyridin-3-yl | Me | H | H | Br | H | heptafluoroisopropyl | H | CF3 |
| 27-677 | 5-chloropyridin-3-yl | Me | H | H | Br | H | heptafluoroisopropyl | H | CF3 |
| 27-678 | 5-bromopyridin-3-yl | Me | H | H | Br | H | heptafluoroisopropyl | H | CF3 |
| 27-679 | 5-iodopyridin-3-yl | Me | H | H | Br | H | heptafluoroisopropyl | H | CF3 |
| 27-680 | 5-(trifluoromethyl)pyridin-3-yl | Me | H | H | Br | H | heptafluoroisopropyl | H | CF3 |
| 27-681 | 5-nitropyridin-3-yl | Me | H | H | Br | H | heptafluoroisopropyl | H | CF3 |
| 27-682 | 5-cyanopyridin-3-yl | Me | H | H | Br | H | heptafluoroisopropyl | H | CF3 |
| 27-683 | 4-fluoropyridin-3-yl | CH2(3-Py) | H | H | Br | H | heptafluoroisopropyl | H | CF3 |
| 27-684 | 4-chloropyridin-3-yl | Me | H | H | Br | H | heptafluoroisopropyl | H | CF3 |

**Table 27(3)**

| compound number | Q₁ | R₁ | R₂ | X₃ | Y₁ | Y₂ | Y₃ | Y₄ | Y₅ |
|---|---|---|---|---|---|---|---|---|---|
| 27-685 | 4-bromopyridin-3-yl | Me | H | H | Br | H | heptafluoroisopropyl | H | CF3 |
| 27-686 | 4-iodopyridin-3-yl | Me | H | H | Br | H | heptafluoroisopropyl | H | CF3 |
| 27-687 | 4-(trifluoromethyl)pyridin-3-yl | Me | H | H | Br | H | heptafluoroisopropyl | H | CF3 |
| 27-688 | 4-nitropyridin-3-yl | Me | H | H | Br | H | heptafluoroisopropyl | H | CF3 |
| 27-689 | 4-cyanopyridin-3-yl | Me | H | H | Br | H | heptafluoroisopropyl | H | CF3 |
| 27-690 | 2,6-dichloropyridin-3-yl | Me | H | H | Br | H | heptafluoroisopropyl | H | CF3 |
| 27-691 | pyridin-3-yl N-oxide | Me | H | H | Br | H | heptafluoroisopropyl | H | CF3 |
| 27-692 | pyridin-4-yl | Me | H | H | Br | H | heptafluoroisopropyl | H | CF3 |
| 27-693 | 2-chloropyridin-4-yl | Me | H | H | Br | H | heptafluoroisopropyl | H | CF3 |
| 27-694 | 3-bromopyridin-4-yl | Me | H | H | Br | H | heptafluoroisopropyl | H | CF3 |
| 27-695 | 3,5-dichloropyridin-4-yl | Me | H | H | Br | H | heptafluoroisopropyl | H | CF3 |
| 27-696 | 3-(trifluoromethyl)pyridin-4-yl | Me | H | H | Br | H | heptafluoroisopropyl | H | CF3 |
| 27-697 | 2,6-dicyanopyridin-4-yl | Me | H | H | Br | H | heptafluoroisopropyl | H | CF3 |
| 27-698 | pyridin-4-yl N-oxide | Me | H | H | Br | H | heptafluoroisopropyl | H | CF3 |
| 27-699 | pyridin-2-yl | Me | H | H | Br | H | heptafluoroisopropyl | H | CF3 |
| 27-700 | 3-chloropyridin-2-yl | Me | H | H | Br | H | heptafluoroisopropyl | H | CF3 |
| 27-701 | 4-bromopyridin-2-yl | Me | H | H | Br | H | heptafluoroisopropyl | H | CF3 |
| 27-702 | 5-iodopyridin-2-yl | Me | H | H | Br | H | heptafluoroisopropyl | H | CF3 |
| 27-703 | 6-chloropyridin-2-yl | Me | H | H | Br | H | heptafluoroisopropyl | H | CF3 |
| 27-704 | 4-cyanopyridin-2-yl | CH2(3-Py-N-oxide) | H | H | Br | H | heptafluoroisopropyl | H | CF3 |
| 27-705 | phenyl | Me | H | H | Br | H | nonafluoro-2-butyl | H | CF3 |
| 27-706 | 2-fluorophenyl | Me | H | H | Br | H | nonafluoro-2-butyl | H | CF3 |
| 27-707 | 3-fluorophenyl | Me | H | H | Br | H | nonafluoro-2-butyl | H | CF3 |
| 27-708 | 4-fluorophenyl | Me | H | H | Br | H | nonafluoro-2-butyl | H | CF3 |
| 27-709 | 2-chlorophenyl | Me | H | H | Br | H | nonafluoro-2-butyl | H | CF3 |
| 27-710 | 3-chlorophenyl | Me | H | H | Br | H | nonafluoro-2-butyl | H | CF3 |
| 27-711 | 4-chlorophenyl | Me | H | H | Br | H | nonafluoro-2-butyl | H | CF3 |
| 27-712 | 2-bromophenyl | Me | H | H | Br | H | nonafluoro-2-butyl | H | CF3 |
| 27-713 | 3-bromophenyl | Me | H | H | Br | H | nonafluoro-2-butyl | H | CF3 |
| 27-714 | 4-bromophenyl | Me | H | H | Br | H | nonafluoro-2-butyl | H | CF3 |
| 27-715 | 2-iodophenyl | Me | H | H | Br | H | nonafluoro-2-butyl | H | CF3 |
| 27-716 | 3-iodophenyl | Me | H | H | Br | H | nonafluoro-2-butyl | H | CF3 |
| 27-717 | 4-iodophenyl | Me | H | H | Br | H | nonafluoro-2-butyl | H | CF3 |
| 27-718 | (2-trifluoromethyl)phenyl | Me | H | H | Br | H | nonafluoro-2-butyl | H | CF3 |
| 27-719 | (3-trifluoromethyl)phenyl | Me | H | H | Br | H | nonafluoro-2-butyl | H | CF3 |
| 27-720 | (4-trifluoromethyl)phenyl | Me | H | H | Br | H | nonafluoro-2-butyl | H | CF3 |
| 27-721 | 2-nitrophenyl | Me | H | H | Br | H | nonafluoro-2-butyl | H | CF3 |
| 27-722 | 3-nitrophenyl | Me | H | H | Br | H | nonafluoro-2-butyl | H | CF3 |
| 27-723 | 4-nitrophenyl | Me | H | H | Br | H | nonafluoro-2-butyl | H | CF3 |
| 27-724 | 2-cyanophenyl | Me | H | H | Br | H | nonafluoro-2-butyl | H | CF3 |
| 27-725 | 3-cyanophenyl | Me | H | H | Br | H | nonafluoro-2-butyl | H | CF3 |
| 27-726 | 4-cyanophenyl | Me | H | H | Br | H | nonafluoro-2-butyl | H | CF3 |
| 27-727 | 2,6-difluorophenyl | Me | H | H | Br | H | nonafluoro-2-butyl | H | CF3 |
| 27-728 | 3,4-dichlorophenyl | Me | H | H | Br | H | nonafluoro-2-butyl | H | CF3 |
| 27-729 | 2,4-dichlorophenyl | Me | H | H | Br | H | nonafluoro-2-butyl | H | CF3 |
| 27-730 | 2-chloro-4-fluorophenyl | Me | H | H | Br | H | nonafluoro-2-butyl | H | CF3 |
| 27-731 | 2-chloro-4,5-difluorophenyl | Me | H | H | Br | H | nonafluoro-2-butyl | H | CF3 |
| 27-732 | 4-bromo-2-chlorophenyl | Et | H | H | Br | H | nonafluoro-2-butyl | H | CF3 |
| 27-733 | 2-bromo-4-chlorophenyl | Me | H | H | Br | H | nonafluoro-2-butyl | H | CF3 |
| 27-734 | 2-bromo-4-fluorophenyl | Me | H | H | Br | H | nonafluoro-2-butyl | H | CF3 |
| 27-735 | 2-chloro-4-nitrophenyl | Me | H | H | Br | H | nonafluoro-2-butyl | H | CF3 |
| 27-736 | 3,5-dicyanophenyl | Me | H | H | Br | H | nonafluoro-2-butyl | H | CF3 |
| 27-737 | 4-cyano-2-fluorophenyl | Me | H | H | Br | H | nonafluoro-2-butyl | H | CF3 |
| 27-738 | 2-chloro-4-cyanophenyl | Me | H | H | Br | H | nonafluoro-2-butyl | H | CF3 |
| 27-739 | pyridin-3-yl | Me | H | H | Br | H | nonafluoro-2-butyl | H | CF3 |
| 27-740 | 2-fluoropyridin-3-yl | Me | H | H | Br | H | nonafluoro-2-butyl | H | CF3 |
| 27-741 | 2-chloropyridin-3-yl | Me | H | H | Br | H | nonafluoro-2-butyl | H | CF3 |
| 27-742 | 2-bromopyridin-3-yl | Me | H | H | Br | H | nonafluoro-2-butyl | H | CF3 |
| 27-743 | 2-iodopyridin-3-yl | Me | H | H | Br | H | nonafluoro-2-butyl | H | CF3 |
| 27-744 | 2-(trifluoromethyl)pyridin-3-yl | Me | H | H | Br | H | nonafluoro-2-butyl | H | CF3 |
| 27-745 | 2-nitropyridin-3-yl | Me | H | H | Br | H | nonafluoro-2-butyl | H | CF3 |
| 27-746 | 2-cyanopyridin-3-yl | Me | H | H | Br | H | nonafluoro-2-butyl | H | CF3 |
| 27-747 | 6-fluoropyridin-3-yl | Me | H | H | Br | H | nonafluoro-2-butyl | H | CF3 |
| 27-748 | 6-chloropyridin-3-yl | Me | H | H | Br | H | nonafluoro-2-butyl | H | CF3 |

**Table 27(4)**

| compound number | Q₁ | R₁ | R₂ | X₃ | Y₁ | Y₂ | Y₃ | Y₄ | Y₅ |
|---|---|---|---|---|---|---|---|---|---|
| 27-749 | 6-bromopyridin-3-yl | Me | H | H | Br | H | nonafluoro-2-butyl | H | CF3 |
| 27-750 | 6-iodopyridin-3-yl | Me | H | H | Br | H | nonafluoro-2-butyl | H | CF3 |
| 27-751 | 6-(trifluoromethyl)pyridin-3-yl | Me | H | H | Br | H | nonafluoro-2-butyl | H | CF3 |
| 27-752 | 6-nitropyridin-3-yl | Me | H | H | Br | H | nonafluoro-2-butyl | H | CF3 |
| 27-753 | 6-cyanopyridin-3-yl | Me | H | H | Br | H | nonafluoro-2-butyl | H | CF3 |
| 27-754 | 5-fluoropyridin-3-yl | Me | H | H | Br | H | nonafluoro-2-butyl | H | CF3 |
| 27-755 | 5-chloropyridin-3-yl | Me | H | H | Br | H | nonafluoro-2-butyl | H | CF3 |
| 27-756 | 5-bromopyridin-3-yl | Me | H | H | Br | H | nonafluoro-2-butyl | H | CF3 |
| 27-757 | 5-iodopyridin-3-yl | Me | H | H | Br | H | nonafluoro-2-butyl | H | CF3 |
| 27-758 | 5-(trifluoromethyl)pyridin-3-yl | CN | H | H | Br | H | nonafluoro-2-butyl | H | CF3 |
| 27-759 | 5-nitropyridin-3-yl | Me | H | H | Br | H | nonafluoro-2-butyl | H | CF3 |
| 27-760 | 5-cyanopyridin-3-yl | Me | H | H | Br | H | nonafluoro-2-butyl | H | CF3 |
| 27-761 | 4-fluoropyridin-3-yl | Me | H | H | Br | H | nonafluoro-2-butyl | H | CF3 |
| 27-762 | 4-chloropyridin-3-yl | Me | H | H | Br | H | nonafluoro-2-butyl | H | CF3 |
| 27-763 | 4-bromopyridin-3-yl | Me | H | H | Br | H | nonafluoro-2-butyl | H | CF3 |
| 27-764 | 4-iodopyridin-3-yl | Me | H | H | Br | H | nonafluoro-2-butyl | H | CF3 |
| 27-765 | 4-(trifluoromethyl)pyridin-3-yl | Me | H | H | Br | H | nonafluoro-2-butyl | H | CF3 |
| 27-766 | 4-nitropyridin-3-yl | Me | H | H | Br | H | nonafluoro-2-butyl | H | CF3 |
| 27-767 | 4-cyanopyridin-3-yl | Me | H | H | Br | H | nonafluoro-2-butyl | H | CF3 |
| 27-768 | 2,6-dichloropyridin-3-yl | Me | H | H | Br | H | nonafluoro-2-butyl | H | CF3 |
| 27-769 | pyridin-3-yl N-oxide | Me | H | H | Br | H | nonafluoro-2-butyl | H | CF3 |
| 27-770 | pyridin-4-yl | Me | H | H | Br | H | nonafluoro-2-butyl | H | CF3 |
| 27-771 | 2-chloropyridin-4-yl | Me | H | H | Br | H | nonafluoro-2-butyl | H | CF3 |
| 27-772 | 3-bromopyridin-4-yl | Me | H | H | Br | H | nonafluoro-2-butyl | H | CF3 |
| 27-773 | 3,5-dichloropyridin-4-yl | Me | H | H | Br | H | nonafluoro-2-butyl | H | CF3 |
| 27-774 | 3-(trifluoromethyl)pyridin-4-yl | Me | H | H | Br | H | nonafluoro-2-butyl | H | CF3 |
| 27-775 | 2,6-dicyanopyridin-4-yl | Me | H | H | Br | H | nonafluoro-2-butyl | H | CF3 |
| 27-776 | pyridin-4-yl N-oxide | Me | H | H | Br | H | nonafluoro-2-butyl | H | CF3 |
| 27-777 | pyridin-2-yl | Me | H | H | Br | H | nonafluoro-2-butyl | H | CF3 |
| 27-778 | 3-chloropyridin-2-yl | Me | H | H | Br | H | nonafluoro-2-butyl | H | CF3 |
| 27-779 | 4-bromopyridin-2-yl | Me | H | H | Br | H | nonafluoro-2-butyl | H | CF3 |
| 27-780 | 5-iodopyridin-2-yl | Me | H | H | Br | H | nonafluoro-2-butyl | H | CF3 |
| 27-781 | 6-chloropyridin-2-yl | Me | H | H | Br | H | nonafluoro-2-butyl | H | CF3 |
| 27-782 | 4-cyanopyridin-2-yl | Me | H | H | Br | H | nonafluoro-2-butyl | H | CF3 |
| 27-783 | phenyl | Me | H | H | I | H | heptafluoroisopropyl | H | CF3 |
| 27-784 | 2-fluorophenyl | Me | H | H | I | H | heptafluoroisopropyl | H | CF3 |
| 27-785 | 3-fluorophenyl | Me | H | H | I | H | heptafluoroisopropyl | H | CF3 |
| 27-786 | 4-fluorophenyl | Me | H | H | I | H | heptafluoroisopropyl | H | CF3 |
| 27-787 | 2-chlorophenyl | Me | H | H | I | H | heptafluoroisopropyl | H | CF3 |
| 27-788 | 3-chlorophenyl | Me | H | H | I | H | heptafluoroisopropyl | H | CF3 |
| 27-789 | 4-chlorophenyl | Me | H | H | I | H | heptafluoroisopropyl | H | CF3 |
| 27-790 | 2-bromophenyl | Me | H | H | I | H | heptafluoroisopropyl | H | CF3 |
| 27-791 | 3-bromophenyl | Me | H | H | I | H | heptafluoroisopropyl | H | CF3 |
| 27-792 | 4-bromophenyl | Me | H | H | I | H | heptafluoroisopropyl | H | CF3 |
| 27-793 | 2-iodophenyl | Me | H | H | I | H | heptafluoroisopropyl | H | CF3 |
| 27-794 | 3-iodophenyl | Me | H | H | I | H | heptafluoroisopropyl | H | CF3 |
| 27-795 | 4-iodophenyl | Me | H | H | I | H | heptafluoroisopropyl | H | CF3 |
| 27-796 | (2-trifluoromethyl)phenyl | Me | H | H | I | H | heptafluoroisopropyl | H | CF3 |
| 27-797 | (3-trifluoromethyl)phenyl | Me | H | H | I | H | heptafluoroisopropyl | H | CF3 |
| 27-798 | (4-trifluoromethyl)phenyl | Me | H | H | I | H | heptafluoroisopropyl | H | CF3 |
| 27-799 | 2-nitrophenyl | Me | H | H | I | H | heptafluoroisopropyl | H | CF3 |
| 27-800 | 3-nitrophenyl | Me | H | H | I | H | heptafluoroisopropyl | H | CF3 |
| 27-801 | 4-nitrophenyl | Me | H | H | I | H | heptafluoroisopropyl | H | CF3 |
| 27-802 | 2-cyanophenyl | Me | H | H | I | H | heptafluoroisopropyl | H | CF3 |
| 27-803 | 3-cyanophenyl | Me | H | H | I | H | heptafluoroisopropyl | H | CF3 |
| 27-804 | 4-cyanophenyl | Me | H | H | I | H | heptafluoroisopropyl | H | CF3 |
| 27-805 | 2,6-difluorophenyl | Me | H | H | I | H | heptafluoroisopropyl | H | CF3 |
| 27-806 | 3,4-dichlorophenyl | Me | H | H | I | H | heptafluoroisopropyl | H | CF3 |
| 27-807 | 2,4-dichlorophenyl | Me | H | H | I | H | heptafluoroisopropyl | H | CF3 |
| 27-808 | 2-chloro-4-fluorophenyl | Me | H | H | I | H | heptafluoroisopropyl | H | CF3 |
| 27-809 | 2-chloro-4,5-difluorophenyl | Me | H | H | I | H | heptafluoroisopropyl | H | CF3 |
| 27-810 | 4-bromo-2-chlorophenyl | Me | H | H | I | H | heptafluoroisopropyl | H | CF3 |
| 27-811 | 2-bromo-4-chlorophenyl | Me | H | H | I | H | heptafluoroisopropyl | H | CF3 |
| 27-812 | 2-bromo-4-fluorophenyl | Me | H | H | I | H | heptafluoroisopropyl | H | CF3 |

**Table 27(5)**

| compound number | Q₁ | R₁ | R₂ | X₃ | Y₁ | Y₂ | Y₃ | Y₄ | Y₅ |
|---|---|---|---|---|---|---|---|---|---|
| 27-813 | 2-chloro-4-nitrophenyl | Me | H | H | I | H | heptafluoroisopropyl | H | CF3 |
| 27-814 | 3,5-dicyanophenyl | Me | H | H | I | H | heptafluoroisopropyl | H | CF3 |
| 27-815 | 4-cyano-2-fluorophenyl | Me | H | H | I | H | heptafluoroisopropyl | H | CF3 |
| 27-816 | 2-chloro-4-cyanophenyl | Me | H | H | I | H | heptafluoroisopropyl | H | CF3 |
| 27-817 | pyridin-3-yl | Me | H | H | I | H | heptafluoroisopropyl | H | CF3 |
| 27-818 | 2-fluoropyridin-3-yl | Me | H | H | I | H | heptafluoroisopropyl | H | CF3 |
| 27-819 | 2-chloropyridin-3-yl | Me | H | H | I | H | heptafluoroisopropyl | H | CF3 |
| 27-820 | 2-bromopyridin-3-yl | Me | H | H | I | H | heptafluoroisopropyl | H | CF3 |
| 27-821 | 2-iodopyridin-3-yl | Me | H | H | I | H | heptafluoroisopropyl | H | CF3 |
| 27-822 | 2-(trifluoromethyl)pyridin-3-yl | Me | H | H | I | H | heptafluoroisopropyl | H | CF3 |
| 27-823 | 2-nitropyridin-3-yl | Me | H | H | I | H | heptafluoroisopropyl | H | CF3 |
| 27-824 | 2-cyanopyridin-3-yl | Me | H | H | I | H | heptafluoroisopropyl | H | CF3 |
| 27-825 | 6-fluoropyridin-3-yl | Me | H | H | I | H | heptafluoroisopropyl | H | CF3 |
| 27-826 | 6-chloropyridin-3-yl | Me | H | H | I | H | heptafluoroisopropyl | H | CF3 |
| 27-827 | 6-bromopyridin-3-yl | Me | H | H | I | H | heptafluoroisopropyl | H | CF3 |
| 27-828 | 6-iodopyridin-3-yl | Me | H | H | I | H | heptafluoroisopropyl | H | CF3 |
| 27-829 | 6-(trifluoromethyl)pyridin-3-yl | Me | H | H | I | H | heptafluoroisopropyl | H | CF3 |
| 27-830 | 6-nitropyridin-3-yl | Me | H | H | I | H | heptafluoroisopropyl | H | CF3 |
| 27-831 | 6-cyanopyridin-3-yl | Me | H | H | I | H | heptafluoroisopropyl | H | CF3 |
| 27-832 | 5-fluoropyridin-3-yl | C(O)Me | H | H | I | H | heptafluoroisopropyl | H | CF3 |
| 27-833 | 5-chloropyridin-3-yl | Me | H | H | I | H | heptafluoroisopropyl | H | CF3 |
| 27-834 | 5-bromopyridin-3-yl | Me | H | H | I | H | heptafluoroisopropyl | H | CF3 |
| 27-835 | 5-iodopyridin-3-yl | Me | H | H | I | H | heptafluoroisopropyl | H | CF3 |
| 27-836 | 5-(trifluoromethyl)pyridin-3-yl | Me | H | H | I | H | heptafluoroisopropyl | H | CF3 |
| 27-837 | 5-nitropyridin-3-yl | Me | H | H | I | H | heptafluoroisopropyl | H | CF3 |
| 27-838 | 5-cyanopyridin-3-yl | Me | H | H | I | H | heptafluoroisopropyl | H | CF3 |
| 27-839 | 4-fluoropyridin-3-yl | Me | H | H | I | H | heptafluoroisopropyl | H | CF3 |
| 27-840 | 4-chloropyridin-3-yl | Me | H | H | I | H | heptafluoroisopropyl | H | CF3 |
| 27-841 | 4-bromopyridin-3-yl | Me | H | H | I | H | heptafluoroisopropyl | H | CF3 |
| 27-842 | 4-iodopyridin-3-yl | Me | H | H | I | H | heptafluoroisopropyl | H | CF3 |
| 27-843 | 4-(trifluoromethyl)pyridin-3-yl | Me | H | H | I | H | heptafluoroisopropyl | H | CF3 |
| 27-844 | 4-nitropyridin-3-yl | Me | H | H | I | H | heptafluoroisopropyl | H | CF3 |
| 27-845 | 4-cyanopyridin-3-yl | Me | H | H | I | H | heptafluoroisopropyl | H | CF3 |
| 27-846 | 2,6-dichloropyridin-3-yl | Me | H | H | I | H | heptafluoroisopropyl | H | CF3 |
| 27-847 | pyridin-3-yl N-oxide | Me | H | H | I | H | heptafluoroisopropyl | H | CF3 |
| 27-848 | pyridin-4-yl | Me | H | H | I | H | heptafluoroisopropyl | H | CF3 |
| 27-849 | 2-chloropyridin-4-yl | Me | H | H | I | H | heptafluoroisopropyl | H | CF3 |
| 27-850 | 3-bromopyridin-4-yl | Me | H | H | I | H | heptafluoroisopropyl | H | CF3 |
| 27-851 | 3,5-dichloropyridin-4-yl | Me | H | H | I | H | heptafluoroisopropyl | H | CF3 |
| 27-852 | 3-(trifluoromethyl)pyridin-4-yl | Me | H | H | I | H | heptafluoroisopropyl | H | CF3 |
| 27-853 | 2,6-dicyanopyridin-4-yl | Me | H | H | I | H | heptafluoroisopropyl | H | CF3 |
| 27-854 | pyridin-4-yl N-oxide | Me | H | H | I | H | heptafluoroisopropyl | H | CF3 |
| 27-855 | pyridin-2-yl | Me | H | H | I | H | heptafluoroisopropyl | H | CF3 |
| 27-856 | 3-chloropyridin-2-yl | Me | H | H | I | H | heptafluoroisopropyl | H | CF3 |
| 27-857 | 4-bromopyridin-2-yl | Me | H | H | I | H | heptafluoroisopropyl | H | CF3 |
| 27-858 | 5-iodopyridin-2-yl | Me | H | H | I | H | heptafluoroisopropyl | H | CF3 |
| 27-859 | 6-chloropyridin-2-yl | Me | H | H | I | H | heptafluoroisopropyl | H | CF3 |
| 27-860 | 4-cyanopyridin-2-yl | Me | H | H | I | H | heptafluoroisopropyl | H | CF3 |
| 27-861 | phenyl | Me | H | H | I | H | nonafluoro-2-butyl | H | CF3 |
| 27-862 | 2-fluorophenyl | Me | H | H | I | H | nonafluoro-2-butyl | H | CF3 |
| 27-863 | 3-fluorophenyl | Me | H | H | I | H | nonafluoro-2-butyl | H | CF3 |
| 27-864 | 4-fluorophenyl | Me | H | H | I | H | nonafluoro-2-butyl | H | CF3 |
| 27-865 | 2-chlorophenyl | Me | H | H | I | H | nonafluoro-2-butyl | H | CF3 |
| 27-866 | 3-chlorophenyl | Me | H | H | I | H | nonafluoro-2-butyl | H | CF3 |
| 27-867 | 4-chlorophenyl | Me | H | H | I | H | nonafluoro-2-butyl | H | CF3 |
| 27-868 | 2-bromophenyl | Me | H | H | I | H | nonafluoro-2-butyl | H | CF3 |
| 27-869 | 3-bromophenyl | Me | H | H | I | H | nonafluoro-2-butyl | H | CF3 |
| 27-870 | 4-bromophenyl | Me | H | H | I | H | nonafluoro-2-butyl | H | CF3 |
| 27-871 | 2-iodophenyl | Me | H | H | I | H | nonafluoro-2-butyl | H | CF3 |
| 27-872 | 3-iodophenyl | Me | H | H | I | H | nonafluoro-2-butyl | H | CF3 |
| 27-873 | 4-iodophenyl | Me | H | H | I | H | nonafluoro-2-butyl | H | CF3 |
| 27-874 | (2-trifluoromethyl)phenyl | Me | H | H | I | H | nonafluoro-2-butyl | H | CF3 |
| 27-875 | (3-trifluoromethyl)phenyl | Me | H | H | I | H | nonafluoro-2-butyl | H | CF3 |
| 27-876 | (4-trifluoromethyl)phenyl | Me | H | H | I | H | nonafluoro-2-butyl | H | CF3 |

**Table 27(6)**

| compound number | Q₁ | R₁ | R₂ | X₃ | Y₁ | Y₂ | Y₃ | Y₄ | Y₅ |
|---|---|---|---|---|---|---|---|---|---|
| 27-877 | 2-nitrophenyl | Me | H | H | I | H | nonafluoro-2-butyl | H | CF3 |
| 27-878 | 3-nitrophenyl | Me | H | H | I | H | nonafluoro-2-butyl | H | CF3 |
| 27-879 | 4-nitrophenyl | Me | H | H | I | H | nonafluoro-2-butyl | H | CF3 |
| 27-880 | 2-cyanophenyl | Me | H | H | I | H | nonafluoro-2-butyl | H | CF3 |
| 27-881 | 3-cyanophenyl | Me | H | H | I | H | nonafluoro-2-butyl | H | CF3 |
| 27-882 | 4-cyanophenyl | Me | H | H | I | H | nonafluoro-2-butyl | H | CF3 |
| 27-883 | 2,6-difluorophenyl | Me | H | H | I | H | nonafluoro-2-butyl | H | CF3 |
| 27-884 | 3,4-dichlorophenyl | Me | H | H | I | H | nonafluoro-2-butyl | H | CF3 |
| 27-885 | 2,4-dichlorophenyl | Me | H | H | I | H | nonafluoro-2-butyl | H | CF3 |
| 27-886 | 2-chloro-4-fluorophenyl | Me | H | H | I | H | nonafluoro-2-butyl | H | CF3 |
| 27-887 | 2-chloro-4,5-difluorophenyl | Me | H | H | I | H | nonafluoro-2-butyl | H | CF3 |
| 27-888 | 4-bromo-2-chlorophenyl | Me | H | H | I | H | nonafluoro-2-butyl | H | CF3 |
| 27-889 | 2-bromo-4-chlorophenyl | Me | H | H | I | H | nonafluoro-2-butyl | H | CF3 |
| 27-890 | 2-bromo-4-fluorophenyl | Me | H | H | I | H | nonafluoro-2-butyl | H | CF3 |
| 27-891 | 2-chloro-4-nitrophenyl | Me | H | H | I | H | nonafluoro-2-butyl | H | CF3 |
| 27-892 | 3,5-dicyanophenyl | Me | H | H | I | H | nonafluoro-2-butyl | H | CF3 |
| 27-893 | 4-cyano-2-fluorophenyl | Me | H | H | I | H | nonafluoro-2-butyl | H | CF3 |
| 27-894 | 2-chloro-4-cyanophenyl | Me | H | H | I | H | nonafluoro-2-butyl | H | CF3 |
| 27-895 | pyridin-3-yl | Me | H | H | I | H | nonafluoro-2-butyl | H | CF3 |
| 27-896 | 2-fluoropyridin-3-yl | Me | H | H | I | H | nonafluoro-2-butyl | H | CF3 |
| 27-897 | 2-chloropyridin-3-yl | Me | H | H | I | H | nonafluoro-2-butyl | H | CF3 |
| 27-898 | 2-bromopyridin-3-yl | Me | H | H | I | H | nonafluoro-2-butyl | H | CF3 |
| 27-899 | 2-iodopyridin-3-yl | Me | H | H | I | H | nonafluoro-2-butyl | H | CF3 |
| 27-900 | 2-(trifluoromethyl)pyridin-3-yl | Me | H | H | I | H | nonafluoro-2-butyl | H | CF3 |
| 27-901 | 2-nitropyridin-3-yl | Me | H | H | I | H | nonafluoro-2-butyl | H | CF3 |
| 27-902 | 2-cyanopyridin-3-yl | Me | H | H | I | H | nonafluoro-2-butyl | H | CF3 |
| 27-903 | 6-fluoropyridin-3-yl | Me | H | H | I | H | nonafluoro-2-butyl | H | CF3 |
| 27-904 | 6-chloropyridin-3-yl | Me | H | H | I | H | nonafluoro-2-butyl | H | CF3 |
| 27-905 | 6-bromopyridin-3-yl | Me | H | H | I | H | nonafluoro-2-butyl | H | CF3 |
| 27-906 | 6-iodopyridin-3-yl | Me | H | H | I | H | nonafluoro-2-butyl | H | CF3 |
| 27-907 | 6-(trifluoromethyl)pyridin-3-yl | Me | H | H | I | H | nonafluoro-2-butyl | H | CF3 |
| 27-908 | 6-nitropyridin-3-yl | Me | H | H | I | H | nonafluoro-2-butyl | H | CF3 |
| 27-909 | 6-cyanopyridin-3-yl | Me | H | H | I | H | nonafluoro-2-butyl | H | CF3 |
| 27-910 | 5-fluoropyridin-3-yl | Me | H | H | I | H | nonafluoro-2-butyl | H | CF3 |
| 27-911 | 5-chloropyridin-3-yl | Me | H | H | I | H | nonafluoro-2-butyl | H | CF3 |
| 27-912 | 5-bromopyridin-3-yl | Me | H | H | I | H | nonafluoro-2-butyl | H | CF3 |
| 27-913 | 5-iodopyridin-3-yl | Me | H | H | I | H | nonafluoro-2-butyl | H | CF3 |
| 27-914 | 5-(trifluoromethyl)pyridin-3-yl | Me | H | H | I | H | nonafluoro-2-butyl | H | CF3 |
| 27-915 | 5-nitropyridin-3-yl | Me | H | H | I | H | nonafluoro-2-butyl | H | CF3 |
| 27-916 | 5-cyanopyridin-3-yl | Me | H | H | I | H | nonafluoro-2-butyl | H | CF3 |
| 27-917 | 4-fluoropyridin-3-yl | Me | H | H | I | H | nonafluoro-2-butyl | H | CF3 |
| 27-918 | 4-chloropyridin-3-yl | Me | H | H | I | H | nonafluoro-2-butyl | H | CF3 |
| 27-919 | 4-bromopyridin-3-yl | Me | H | H | I | H | nonafluoro-2-butyl | H | CF3 |
| 27-920 | 4-iodopyridin-3-yl | Me | H | H | I | H | nonafluoro-2-butyl | H | CF3 |
| 27-921 | 4-(trifluoromethyl)pyridin-3-yl | Me | H | H | I | H | nonafluoro-2-butyl | H | CF3 |
| 27-922 | 4-nitropyridin-3-yl | Me | H | H | I | H | nonafluoro-2-butyl | H | CF3 |
| 27-923 | 4-cyanopyridin-3-yl | Me | H | H | I | H | nonafluoro-2-butyl | H | CF3 |
| 27-924 | 2,6-dichloropyridin-3-yl | Me | H | H | I | H | nonafluoro-2-butyl | H | CF3 |
| 27-925 | pyridin-3-yl N-oxide | Me | H | H | I | H | nonafluoro-2-butyl | H | CF3 |
| 27-926 | pyridin-4-yl | Me | H | H | I | H | nonafluoro-2-butyl | H | CF3 |
| 27-927 | 2-chloropyridin-4-yl | Me | H | H | I | H | nonafluoro-2-butyl | H | CF3 |
| 27-928 | 3-bromopyridin-4-yl | Me | H | H | I | H | nonafluoro-2-butyl | H | CF3 |
| 27-929 | 3,5-dichloropyridin-4-yl | Me | H | H | I | H | nonafluoro-2-butyl | H | CF3 |
| 27-930 | 3-(trifluoromethyl)pyridin-4-yl | Me | H | H | I | H | nonafluoro-2-butyl | H | CF3 |
| 27-931 | 2,6-dicyanopyridin-4-yl | Me | H | H | I | H | nonafluoro-2-butyl | H | CF3 |
| 27-932 | pyridin-4-yl N-oxide | Me | H | H | I | H | nonafluoro-2-butyl | H | CF3 |
| 27-933 | pyridin-2-yl | Me | H | H | I | H | nonafluoro-2-butyl | H | CF3 |
| 27-934 | 3-chloropyridin-2-yl | Me | H | H | I | H | nonafluoro-2-butyl | H | CF3 |
| 27-935 | 4-bromopyridin-2-yl | Me | H | H | I | H | nonafluoro-2-butyl | H | CF3 |
| 27-936 | 5-iodopyridin-2-yl | Me | H | H | I | H | nonafluoro-2-butyl | H | CF3 |
| 27-937 | 6-chloropyridin-2-yl | Me | H | H | I | H | nonafluoro-2-butyl | H | CF3 |
| 27-938 | 4-cyanopyridin-2-yl | Me | H | H | I | H | nonafluoro-2-butyl | H | CF3 |
| 27-939 | phenyl | Me | H | H | OCF3 | H | heptafluoroisopropyl | H | OCF3 |
| 27-970 | 2-chloropyridin-3-yl | Me | H | H | OCF3 | H | heptafluoroisopropyl | H | OCF3 |

**Table 27(7)**

| compound number | Q, | R₁ | R₂ | X₃ | Y₁ | Y₂ | Y₃ | Y₄ | Y₅ |
|---|---|---|---|---|---|---|---|---|---|
| 27-995 | phenyl | Me | H | H | OCF3 | H | nonafluoro-2-butyl | H | OCF3 |
| 27-1026 | 2-chloropyridin-3-yl | Me | H | H | OCF3 | H | nonafluoro-2-butyl | H | OCF3 |
| 27-1051 | phenyl | Me | H | H | CF3 | H | heptafluoroisopropyl | H | OCF3 |
| 27-1082 | 2-chloropyridin-3-yl | Me | H | H | CF3 | H | heptafluoroisopropyl | H | OCF3 |
| 27-1107 | phenyl | Me | H | H | CF3 | H | nonafluoro-2-butyl | H | OCF3 |
| 27-1138 | 2-chloropyridin-3-yl | Me | H | H | CF3 | H | nonafluoro-2-butyl | H | OCF3 |
| 27-1163 | phenyl | Me | H | H | CF3 | H | heptafluoroisopropyl | H | CF3 |
| 27-1183 | 3-cyanophenyl | Me | H | H | CF3 | H | heptafluoroisopropyl | H | CF3 |
| 27-1184 | 4-cyanophenyl | Me | H | H | CF3 | H | heptafluoroisopropyl | H | CF3 |
| 27-1199 | 2-chloropyridin-3-yl | Me | H | H | CF3 | H | heptafluoroisopropyl | H | CF3 |
| 27-1231 | phenyl | Me | H | H | CF3 | H | nonafluoro-2-butyl | H | CF3 |
| 27-1251 | 3-cyanophenyl | Me | H | H | CF3 | H | nonafluoro-2-butyl | H | CF3 |
| 27-1252 | 4-cyanophenyl | Me | H | H | CF3 | H | nonafluoro-2-butyl | H | CF3 |
| 27-1267 | 2-chloropyridin-3-yl | Me | H | H | CF3 | H | nonafluoro-2-butyl | H | CF3 |

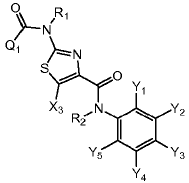

**Table 28(1)**

| compound number | Q₁ | R₁ | R₂ | X₃ | Y₁ | Y₂ | Y₃ | Y₄ | Y₅ |
|---|---|---|---|---|---|---|---|---|---|
| 28-1 | phenyl | H | Me | H | Cl | H | heptafluoroisopropyl | H | OCF3 |
| 28-21 | 3-cyanophenyl | H | Me | H | Cl | H | heptafluoroisopropyl | H | OCF3 |
| 28-22 | 4-cyanophenyl | H | Me | H | Cl | H | heptafluoroisopropyl | H | OCF3 |
| 28-37 | 2-chloropyridin-3-yl | H | Me | H | Cl | H | heptafluoroisopropyl | H | OCF3 |
| 28-79 | phenyl | H | Me | H | Cl | H | nonafluoro-2-butyl | H | OCF3 |
| 28-99 | 3-cyanophenyl | H | Me | H | Cl | H | nonafluoro-2-butyl | H | OCF3 |
| 28-100 | 4-cyanophenyl | H | Me | H | Cl | H | nonafluoro-2-butyl | H | OCF3 |
| 28-115 | 2-chloropyridin-3-yl | H | Me | H | Cl | H | nonafluoro-2-butyl | H | OCF3 |
| 28-157 | phenyl | H | Me | H | Br | H | heptafluoroisopropyl | H | OCF3 |
| 28-177 | 3-cyanophenyl | H | Me | H | Br | H | heptafluoroisopropyl | H | OCF3 |
| 28-178 | 4-cyanophenyl | H | Me | H | Br | H | heptafluoroisopropyl | H | OCF3 |
| 28-193 | 2-chloropyridin-3-yl | H | Me | H | Br | H | heptafluoroisopropyl | H | OCF3 |
| 28-235 | phenyl | H | Me | H | Br | H | nonafluoro-2-butyl | H | OCF3 |
| 28-255 | 3-cyanophenyl | H | Me | H | Br | H | nonafluoro-2-butyl | H | OCF3 |
| 28-256 | 4-cyanophenyl | H | Me | H | Br | H | nonafluoro-2-butyl | H | OCF3 |
| 28-271 | 2-chloropyridin-3-yl | H | Me | H | Br | H | nonafluoro-2-butyl | H | OCF3 |
| 28-313 | phenyl | H | Me | H | I | H | heptafluoroisopropyl | H | OCF3 |
| 28-333 | 3-cyanophenyl | H | Me | H | I | H | heptafluoroisopropyl | H | OCF3 |
| 28-334 | 4-cyanophenyl | H | Me | H | I | H | heptafluoroisopropyl | H | OCF3 |
| 28-349 | 2-chloropyridin-3-yl | H | Me | H | I | H | heptafluoroisopropyl | H | OCF3 |
| 28-391 | phenyl | H | Me | H | I | H | nonafluoro-2-butyl | H | OCF3 |
| 28-411 | 3-cyanophenyl | H | Me | H | I | H | nonafluoro-2-butyl | H | OCF3 |
| 28-412 | 4-cyanophenyl | H | Me | H | I | H | nonafluoro-2-butyl | H | OCF3 |
| 28-427 | 2-chloropyridin-3-yl | H | Me | H | I | H | nonafluoro-2-butyl | H | OCF3 |
| 28-469 | phenyl | H | Me | H | F | H | heptafluoroisopropyl | H | CF3 |
| 28-470 | phenyl | H | Me | H | Cl | H | heptafluoroisopropyl | H | CF3 |
| 28-481 | 3-iodophenyl | H | Me | H | Cl | H | pentafluoroethyl | H | CF3 |
| 28-482 | 4-iodophenyl | H | Me | H | Cl | H | pentafluoroethyl | H | CF3 |
| 28-484 | (3-trifluoromethyl)phenyl | H | Et | H | Cl | H | heptafluoroisopropyl | H | CF3 |
| 28-489 | 2-cyanophenyl | H | Me | H | Cl | H | pentafluoroethyl | H | C2F5 |
| 28-490 | 3-cyanophenyl | H | Me | H | Cl | H | heptafluoroisopropyl | H | CF3 |
| 28-491 | 4-cyanophenyl | H | Me | H | Cl | H | heptafluoroisopropyl | H | CF3 |
| 28-494 | 2,4-dichlorophenyl | H | Me | H | Cl | H | heptafluoroisopropyl | H | C2F5 |
| 28-495 | 2-chloro-4-fluorophenyl | H | n-Pr | H | Cl | H | heptafluoroisopropyl | H | CF3 |
| 28-498 | 2-bromo-4-chlorophenyl | H | Me | H | Cl | H | heptafluoroisopropyl | H | n-C3F7 |
| 28-505 | 2-fluoropyridin-3-yl | H | Me | H | Cl | H | pentafluoroethyl | H | CF3 |
| 28-506 | 2-chloropyridin-3-yl | H | Me | H | Cl | H | heptafluoroisopropyl | H | CF3 |
| 28-512 | 6-fluoropyridin-3-yl | H | i-Pr | H | Cl | H | heptafluoroisopropyl | H | CF3 |
| 28-519 | 5-fluoropyridin-3-yl | H | Me | H | Cl | H | heptafluoroisopropyl | H | C2F5 |
| 28-521 | 5-bromopyridin-3-yl | H | Me | H | Cl | H | heptafluoroisopropyl | H | n-C3F7 |
| 28-522 | 5-iodopyridin-3-yl | H | Me | H | Cl | H | heptafluoroisopropyl | H | i-C3F7 |
| 28-524 | 5-nitropyridin-3-yl | H | Me | H | Cl | H | pentafluoroethyl | H | CF3 |
| 28-526 | 4-fluoropyridin-3-yl | H | Me | H | Cl | H | pentafluoroethyl | H | CF3 |
| 28-528 | 4-bromopyridin-3-yl | H | CH2CH=CH2 | H | Cl | H | heptafluoroisopropyl | H | n-C3F7 |
| 28-529 | 4-iodopyridin-3-yl | H | Me | H | Cl | H | heptafluoroisopropyl | H | i-C3F7 |
| 28-531 | 4-nitropyridin-3-yl | H | Me | Cl | Cl | H | heptafluoroisopropyl | H | CF3 |
| 28-535 | pyridin-4-yl | H | CN | H | Cl | H | heptafluoroisopropyl | H | CF3 |
| 28-547 | 4-cyanopyridin-2-yl | H | Me | H | Cl | H | pentafluoroethyl | H | CF3 |
| 28-548 | phenyl | H | Me | H | F | H | nonafluoro-2-butyl | H | CF3 |
| 28-549 | phenyl | H | Me | H | Cl | H | nonafluoro-2-butyl | H | CF3 |
| 28-557 | 3-bromophenyl | H | CH2C=CH | H | Cl | H | nonafluoro-2-butyl | H | CF3 |
| 28-562 | (2-trifluoromethyl)phenyl | H | Me | H | Cl | H | nonafluoro-2-butyl | H | C2F5 |
| 28-564 | (4-trifluoromethyl)phenyl | H | NH2 | H | Cl | H | nonafluoro-2-butyl | H | CF3 |
| 28-569 | 3-cyanophenyl | H | Me | H | Cl | H | nonafluoro-2-butyl | H | CF3 |
| 28-570 | 4-cyanophenyl | H | Me | H | Cl | H | nonafluoro-2-butyl | H | CF3 |

**Table 28(2)**

| compound number | Q₁ | R₁ | R₂ | X₃ | Y₁ | Y₂ | Y₃ | Y₄ | Y₅ |
|---|---|---|---|---|---|---|---|---|---|
| 28-577 | 2-bromo-4-chlorophenyl | H | C(O)OMe | H | Cl | H | nonafluoro-2-butyl | H | CF3 |
| 28-579 | 2-chloro-4-nitrophenyl | H | Me | H | Cl | H | nonafluoro-2-butyl | H | i-C3F7 |
| 28-585 | 2-chloropyridin-3-yl | H | Me | H | Cl | H | nonafluoro-2-butyl | H | CF3 |
| 28-598 | 5-fluoropyridin-3-yl | H | C(O)OEt | H | Cl | H | nonafluoro-2-butyl | H | C2F5 |
| 28-600 | 5-bromopyridin-3-yl | H | Me | H | Cl | H | nonafluoro-2-butyl | H | n-C3F7 |
| 28-601 | 5-iodopyridin-3-yl | H | Me | H | Cl | H | nonafluoro-2-butyl | H | i-C3F7 |
| 28-605 | 4-fluoropyridin-3-yl | H | Me | H | Cl | H | nonafluoro-2-butyl | H | C2F5 |
| 28-607 | 4-bromopyridin-3-yl | H | C(O)C(O)Me | H | Cl | H | nonafluoro-2-butyl | H | CF3 |
| 28-609 | 4-(trifluoromethyl)pyridin-3-yl | H | Me | H | Cl | H | nonafluoro-2-butyl | H | n-C3F7 |
| 28-610 | 4-nitropyridin-3-yl | H | C(O)C(O)Et | H | Cl | H | nonafluoro-2-butyl | H | i-C3F7 |
| 28-624 | 5-iodopyridin-2-yl | H | C(O)Me | H | Cl | H | nonafluoro-2-butyl | H | CF3 |
| 28-626 | 4-cyanopyridin-2-yl | H | C(O)Et | H | Cl | H | nonafluoro-2-butyl | H | C2F5 |
| 28-627 | phenyl | H | Me | H | Br | H | heptafluoroisopropyl | H | CF3 |
| 28-628 | 2-fluorophenyl | H | Me | H | Br | H | heptafluoroisopropyl | H | CF3 |
| 28-641 | (3-trifluoromethyl)phenyl | H | S(O)2Me | H | Br | H | heptafluoroisopropyl | H | CF3 |
| 28-647 | 3-cyanophenyl | H | Me | H | Br | H | heptafluoroisopropyl | H | CF3 |
| 28-648 | 4-cyanophenyl | H | Me | H | Br | H | heptafluoroisopropyl | H | CF3 |
| 28-659 | 4-cyano-2-fluorophenyl | H | S(O)2Et | H | Br | H | heptafluoroisopropyl | H | CF3 |
| 28-663 | 2-chloropyridin-3-yl | H | Me | H | Br | H | heptafluoroisopropyl | H | CF3 |
| 28-674 | 6-nitropyridin-3-yl | H | CH2Ph | H | Br | H | heptafluoroisopropyl | H | CF3 |
| 28-683 | 4-fluoropyridin-3-yl | H | CH2(3-Py) | H | Br | H | heptafluoroisopropyl | H | CF3 |
| 28-704 | 4-cyanopyridin-2-yl | H | CH2(3-Py-N-oxide) | H | Br | H | heptafluoroisopropyl | H | CF3 |
| 28-705 | phenyl | H | Me | H | Br | H | nonafluoro-2-butyl | H | CF3 |
| 28-706 | 2-fluorophenyl | H | Me | H | Br | H | nonafluoro-2-butyl | H | CF3 |
| 28-725 | 3-cyanophenyl | H | Me | H | Br | H | nonafluoro-2-butyl | H | CF3 |
| 28-726 | 4-cyanophenyl | H | Me | H | Br | H | nonafluoro-2-butyl | H | CF3 |
| 28-732 | 4-bromo-2-chlorophenyl | H | Et | H | Br | H | nonafluoro-2-butyl | H | CF3 |
| 28-741 | 2-chloropyridin-3-yl | H | Me | H | Br | H | nonafluoro-2-butyl | H | CF3 |
| 28-758 | 5-(trifluoromethyl)pyridin-3-yl | H | CN | H | Br | H | nonafluoro-2-butyl | H | CF3 |
| 28-783 | phenyl | H | Me | H | I | H | heptafluoroisopropyl | H | CF3 |
| 28-784 | 2-fluorophenyl | H | Me | H | I | H | heptafluoroisopropyl | H | CF3 |
| 28-803 | 3-cyanophenyl | H | Me | H | I | H | heptafluoroisopropyl | H | CF3 |
| 28-804 | 4-cyanophenyl | H | Me | H | I | H | heptafluoroisopropyl | H | CF3 |
| 28-819 | 2-chloropyridin-3-yl | H | Me | H | I | H | heptafluoroisopropyl | H | CF3 |
| 28-832 | 5-fluoropyridin-3-yl | H | C(O)Me | H | I | H | heptafluoroisopropyl | H | CF3 |
| 28-861 | phenyl | H | Me | H | I | H | nonafluoro-2-butyl | H | CF3 |
| 28-862 | 2-fluorophenyl | H | Me | H | I | H | nonafluoro-2-butyl | H | CF3 |
| 28-881 | 3-cyanophenyl | H | Me | H | I | H | nonafluoro-2-butyl | H | CF3 |
| 28-882 | 4-cyanophenyl | H | Me | H | I | H | nonafluoro-2-butyl | H | CF3 |
| 28-897 | 2-chloropyridin-3-yl | H | Me | H | I | H | nonafluoro-2-butyl | H | CF3 |
| 28-939 | phenyl | H | Me | H | OCF3 | H | heptafluoroisopropyl | H | OCF3 |
| 28-970 | 2-chloropyridin-3-yl | H | Me | H | OCF3 | H | heptafluoroisopropyl | H | OCF3 |
| 28-995 | phenyl | H | Me | H | OCF3 | H | nonafluoro-2-butyl | H | OCF3 |
| 28-1026 | 2-chloropyridin-3-yl | H | Me | H | OCF3 | H | nonafluoro-2-butyl | H | OCF3 |
| 28-1051 | phenyl | H | Me | H | CF3 | H | heptafluoroisopropyl | H | OCF3 |
| 28-1082 | 2-chloropyridin-3-yl | H | Me | H | CF3 | H | heptafluoroisopropyl | H | OCF3 |
| 28-1107 | phenyl | H | Me | H | CF3 | H | nonafluoro-2-butyl | H | OCF3 |
| 28-1138 | 2-chloropyridin-3-yl | H | Me | H | CF3 | H | nonafluoro-2-butyl | H | OCF3 |
| 28-1163 | phenyl | H | Me | H | CF3 | H | heptafluoroisopropyl | H | CF3 |
| 28-1183 | 3-cyanophenyl | H | Me | H | CF3 | H | heptafluoroisopropyl | H | CF3 |
| 28-1184 | 4-cyanophenyl | H | Me | H | CF3 | H | heptafluoroisopropyl | H | CF3 |
| 28-1199 | 2-chloropyridin-3-yl | H | Me | H | CF3 | H | heptafluoroisopropyl | H | CF3 |
| 28-1231 | phenyl | H | Me | H | CF3 | H | nonafluoro-2-butyl | H | CF3 |
| 28-1251 | 3-cyanophenyl | H | Me | H | CF3 | H | nonafluoro-2-butyl | H | CF3 |
| 28-1252 | 4-cyanophenyl | H | Me | H | CF3 | H | nonafluoro-2-butyl | H | CF3 |
| 28-1267 | 2-chloropyridin-3-yl | H | Me | H | CF3 | H | nonafluoro-2-butyl | H | CF3 |

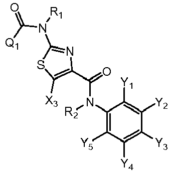

**Table 29(1)**

| compound number | Q₁ | R₁ | R₂ | X₃ | Y₁ | Y₂ | Y₃ | Y₄ | Y₅ |
|---|---|---|---|---|---|---|---|---|---|
| 29-1 | phenyl | Me | Me | H | Cl | H | heptafluoroisopropyl | H | OCF3 |
| 29-21 | 3-cyanophenyl | Me | Me | H | Cl | H | heptafluoroisopropyl | H | OCF3 |
| 29-22 | 4-cyanophenyl | Me | Me | H | Cl | H | heptafluoroisopropyl | H | OCF3 |
| 29-37 | 2-chloropyridin-3-yl | Me | Me | H | Cl | H | heptafluoroisopropyl | H | OCF3 |
| 29-79 | phenyl | Me | Me | H | Cl | H | nonafluoro-2-butyl | H | OCF3 |
| 29-99 | 3-cyanophenyl | Me | Me | H | Cl | H | nonafluoro-2-butyl | H | OCF3 |
| 29-100 | 4-cyanophenyl | Me | Me | H | Cl | H | nonafluoro-2-butyl | H | OCF3 |
| 29-115 | 2-chloropyridin-3-yl | Me | Me | H | Cl | H | nonafluoro-2-butyl | H | OCF3 |
| 29-157 | phenyl | Me | Me | H | Br | H | heptafluoroisopropyl | H | OCF3 |
| 29-177 | 3-cyanophenyl | Me | Me | H | Br | H | heptafluoroisopropyl | H | OCF3 |
| 29-178 | 4-cyanophenyl | Me | Me | H | Br | H | heptafluoroisopropyl | H | OCF3 |
| 29-193 | 2-chloropyridin-3-yl | Me | Me | H | Br | H | heptafluoroisopropyl | H | OCF3 |
| 29-235 | phenyl | Me | Me | H | Br | H | nonafluoro-2-butyl | H | OCF3 |
| 29-255 | 3-cyanophenyl | Me | Me | H | Br | H | nonafluoro-2-butyl | H | OCF3 |
| 29-256 | 4-cyanophenyl | Me | Me | H | Br | H | nonafluoro-2-butyl | H | OCF3 |
| 29-271 | 2-chloropyridin-3-yl | Me | Me | H | Br | H | nonafluoro-2-butyl | H | OCF3 |
| 29-313 | phenyl | Me | Me | H | I | H | heptafluoroisopropyl | H | OCF3 |
| 29-333 | 3-cyanophenyl | Me | Me | H | I | H | heptafluoroisopropyl | H | OCF3 |
| 29-334 | 4-cyanophenyl | Me | Me | H | I | H | heptafluoroisopropyl | H | OCF3 |
| 29-349 | 2-chloropyridin-3-yl | Me | Me | H | I | H | heptafluoroisopropyl | H | OCF3 |
| 29-391 | phenyl | Me | Me | H | I | H | nonafluoro-2-butyl | H | OCF3 |
| 29-411 | 3-cyanophenyl | Me | Me | H | I | H | nonafluoro-2-butyl | H | OCF3 |
| 29-412 | 4-cyanophenyl | Me | Me | H | I | H | nonafluoro-2-butyl | H | OCF3 |
| 29-427 | 2-chloropyridin-3-yl | Me | Me | H | I | H | nonafluoro-2-butyl | H | OCF3 |
| 29-665 | phenyl | Me | Me | H | F | H | heptafluoroisopropyl | H | CF3 |
| 29-666 | phenyl | Me | Me | H | Cl | H | heptafluoroisopropyl | H | CF3 |
| 29-677 | 3-iodophenyl | Me | Me | H | Cl | H | pentafluoroethyl | H | CF3 |
| 29-678 | 4-iodophenyl | Me | Et | H | Cl | H | pentafluoroethyl | H | CF3 |
| 29-680 | (3-trifluoromethyl) phenyl | Et | Me | H | Cl | H | heptafluoroisopropyl | H | CF3 |
| 29-682 | 2-nitrophenyl | Me | Me | Cl | Cl | H | heptafluoroisopropyl | H | CF3 |
| 29-685 | 2-cyanophenyl | Me | Me | H | Cl | H | pentafluoroethyl | H | C2F5 |
| 29-686 | 3-cyanophenyl | Me | Me | H | Cl | H | heptafluoroisopropyl | H | CF3 |
| 29-687 | 4-cyanophenyl | Me | Me | H | Cl | H | heptafluoroisopropyl | H | CF3 |
| 29-689 | 3,4-dichlorophenyl | Me | n-Pr | H | Cl | H | heptafluoroisopropyl | H | CF3 |
| 29-690 | 2,4-dichlorophenyl | Me | Me | H | Cl | H | heptafluoroisopropyl | H | C2F5 |
| 29-691 | 2-chloro-4-fluorophenyl | n-Pr | Me | H | Cl | H | heptafluoroisopropyl | H | CF3 |
| 29-694 | 2-bromo-4-chlorophenyl | Me | Me | H | Cl | H | heptafluoroisopropyl | H | n-C3F7 |
| 29-701 | 2-fluoropyridin-3-yl | Me | Me | H | Cl | H | pentafluoroethyl | H | CF3 |
| 29-702 | 2-chloropyridin-3-yl | Me | Me | H | Cl | H | heptafluoroisopropyl | H | CF3 |
| 29-706 | 2-nitropyridin-3-yl | Me | i-Pr | H | Cl | H | heptafluoroisopropyl | H | CF3 |
| 29-708 | 6-fluoropyridin-3-yl | i-Pr | Me | H | Cl | H | heptafluoroisopropyl | H | CF3 |
| 29-715 | 5-fluoropyridin-3-yl | Me | Me | H | Cl | H | heptafluoroisopropyl | H | C2F5 |
| 29-717 | 5-bromopyridin-3-yl | Me | Me | H | Cl | H | heptafluoroisopropyl | H | n-C3F7 |
| 29-718 | 5-iodopyridin-3-yl | Me | Me | H | Cl | H | heptafluoroisopropyl | H | i-C3F7 |
| 29-720 | 5-nitropyridin-3-yl | Me | Me | H | Cl | H | pentafluoroethyl | H | CF3 |
| 29-722 | 4-fluoropyridin-3-yl | Me | CH2CH=CH2 | H | Cl | H | pentafluoroethyl | H | CF3 |
| 29-724 | 4-bromopyridin-3-yl | CH2CH=CH2 | Me | H | Cl | H | heptafluoroisopropyl | H | n-C3F7 |
| 29-725 | 4-iodopyridin-3-yl | Me | Me | H | Cl | H | heptafluoroisopropyl | H | i-C3F7 |
| 29-729 | 2,6-dichloropyridin-3-yl | Me | CN | H | Cl | H | heptafluoroisopropyl | H | CF3 |
| 29-731 | pyridin-4-yl | CN | Me | H | Cl | H | heptafluoroisopropyl | H | CF3 |
| 29-743 | 4-cyanopyridin-2-yl | Me | Me | H | Cl | H | pentafluoroethyl | H | CF3 |
| 29-744 | phenyl | Me | Me | H | F | H | nonafluoro-2-butyl | H | CF3 |
| 29-745 | phenyl | Me | Me | H | Cl | H | nonafluoro-2-butyl | H | CF3 |
| 29-751 | 4-chlorophenyl | Me | CH2C≡CH | H | Cl | H | nonafluoro-2-butyl | H | CF3 |
| 29-753 | 3-bromophenyl | CH2C≡CH | Me | H | Cl | H | nonafluoro-2-butyl | H | CF3 |

**Table 29(2)**

| compound number | Q₁ | R₁ | R₂ | X₃ | Y₁ | Y₂ | Y₃ | Y₄ | Y₅ |
|---|---|---|---|---|---|---|---|---|---|
| 29-758 | (2-trifluoromethyl)phenyl | Me | NH2 | H | Cl | H | nonafluoro-2-butyl | H | C2F5 |
| 29-760 | (4-trifluoromethyl)phenyl | NH2 | Me | H | Cl | H | nonafluoro-2-butyl | H | CF3 |
| 29-765 | 3-cyanophenyl | Me | Me | H | Cl | H | nonafluoro-2-butyl | H | CF3 |
| 29-766 | 4-cyanophenyl | Me | Me | H | Cl | H | nonafluoro-2-butyl | H | CF3 |
| 29-771 | 2-chloro-4,5-difluorophenyl | Me | C(O)OMe | H | Cl | H | nonafluoro-2-butyl | H | CF3 |
| 29-773 | 2-bromo-4-chlorophenyl | C(O)OMe | Me | H | Cl | H | nonafluoro-2-butyl | H | CF3 |
| 29-775 | 2-chloro-4-nitrophenyl | Me | Me | H | Cl | H | nonafluoro-2-butyl | H | i-C3F7 |
| 29-781 | 2-chloropyridin-3-yl | Me | Me | H | Cl | H | nonafluoro-2-butyl | H | CF3 |
| 29-792 | 6-nitropyridin-3-yl | Me | C(O)OEt | H | Cl | H | nonafluoro-2-butyl | H | CF3 |
| 29-794 | 5-fluoropyridin-3-yl | C(O)OEt | Me | H | Cl | H | nonafluoro-2-butyl | H | C2F5 |
| 29-796 | 5-bromopyridin-3-yl | Me | Me | H | Cl | H | nonafluoro-2-butyl | H | n-C3F7 |
| 29-797 | 5-iodopyridin-3-yl | Me | Me | H | Cl | H | nonafluoro-2-butyl | H | i-C3F7 |
| 29-801 | 4-fluoropyridin-3-yl | Me | C(O)C(O)Me | H | Cl | H | nonafluoro-2-butyl | H | C2F5 |
| 29-803 | 4-bromopyridin-3-yl | C(O)C(O)Me | Me | H | Cl | H | nonafluoro-2-butyl | H | CF3 |
| 29-804 | 4-iodopyridin-3-yl | Me | C(O)C(O)Et | H | Cl | H | nonafluoro-2-butyl | H | CF3 |
| 29-805 | 4-(trifluoromethyl) pyridin-3-yl | Me | Me | H | Cl | H | nonafluoro-2-butyl | H | n-C3F7 |
| 29-806 | 4-nitropyridin-3-yl | C(O)C(O)Et | Me | H | Cl | H | nonafluoro-2-butyl | H | i-C3F7 |
| 29-818 | 3-chloropyridin-2-yl | Me | C(O)Me | H | Cl | H | nonafluoro-2-butyl | H | CF3 |
| 29-820 | 5-iodopyridin-2-yl | C(O)Me | Me | H | Cl | H | nonafluoro-2-butyl | H | CF3 |
| 29-821 | 6-chloropyridin-2-yl | Me | C(O)Et | H | Cl | H | nonafluoro-2-butyl | H | CF3 |
| 29-822 | 4-cyanopyridin-2-yl | C(O)Et | Me | H | Cl | H | nonafluoro-2-butyl | H | C2F5 |
| 29-823 | phenyl | Me | Me | H | Br | H | heptafluoroisopropyl | H | CF3 |
| 29-824 | 2-fluorophenyl | Me | Me | H | Br | H | heptafluoroisopropyl | H | CF3 |
| 29-835 | 4-iodophenyl | Me | S(O)2Me | H | Br | H | heptafluoroisopropyl | H | CF3 |
| 29-837 | (3-trifluoromethyl)phenyl | S(O)2Me | Me | H | Br | H | heptafluoroisopropyl | H | CF3 |
| 29-843 | 3-cyanophenyl | Me | Me | H | Br | H | heptafluoroisopropyl | H | CF3 |
| 29-844 | 4-cyanophenyl | Me | Me | H | Br | H | heptafluoroisopropyl | H | CF3 |
| 29-853 | 2-chloro-4-nitrophenyl | Me | S(O)2Et | H | Br | H | heptafluoroisopropyl | H | CF3 |
| 29-855 | 4-cyano-2-fluorophenyl | S(O)2Et | Me | H | Br | H | heptafluoroisopropyl | H | CF3 |
| 29-859 | 2-chloropyridin-3-yl | Me | Me | H | Br | H | heptafluoroisopropyl | H | CF3 |
| 29-868 | 6-iodopyridin-3-yl | Me | CH2Ph | H | Br | H | heptafluoroisopropyl | H | CF3 |
| 29-870 | 6-nitropyridin-3-yl | CH2Ph | Me | H | Br | H | heptafluoroisopropyl | H | CF3 |
| 29-877 | 5-nitropyridin-3-yl | Me | CH2(3-Py) | H | Br | H | heptafluoroisopropyl | H | CF3 |
| 29-879 | 4-fluoropyridin-3-yl | CH2(3-Py) | Me | H | Br | H | heptafluoroisopropyl | H | CF3 |
| 29-898 | 5-iodopyridin-2-yl | Me | CH2(3-Py-N-oxide) | H | Br | H | heptafluoroisopropyl | H | CF3 |
| 29-900 | 4-cyanopyridin-2-yl | CH2(3-Py-N-oxide) | Me | H | Br | H | heptafluoroisopropyl | H | CF3 |
| 29-901 | phenyl | Me | Me | H | Br | H | nonafluoro-2-butyl | H | CF3 |
| 29-902 | 2-fluorophenyl | Me | Me | H | Br | H | nonafluoro-2-butyl | H | CF3 |
| 29-921 | 3-cyanophenyl | Me | Me | H | Br | H | nonafluoro-2-butyl | H | CF3 |
| 29-922 | 4-cyanophenyl | Me | Me | H | Br | H | nonafluoro-2-butyl | H | CF3 |
| 29-926 | 2-chloro-4-fluorophenyl | Me | Et | H | Br | H | nonafluoro-2-butyl | H | CF3 |
| 29-928 | 4-bromo-2-chlorophenyl | Et | Me | H | Br | H | nonafluoro-2-butyl | H | CF3 |
| 29-937 | 2-chloropyridin-3-yl | Me | Me | H | Br | H | nonafluoro-2-butyl | H | CF3 |
| 29-952 | 5-bromopyridin-3-yl | Me | CN | H | Br | H | nonafluoro-2-butyl | H | CF3 |
| 29-954 | 5-(trifluoromethyl)pyridin-3-yl | CN | Me | H | Br | H | nonafluoro-2-butyl | H | CF3 |
| 29-979 | phenyl | Me | Me | H | I | H | heptafluoroisopropyl | H | CF3 |
| 29-980 | 2-fluorophenyl | Me | Me | H | I | H | heptafluoroisopropyl | H | CF3 |
| 29-999 | 3-cyanophenyl | Me | Me | H | I | H | heptafluoroisopropyl | H | CF3 |
| 29-1000 | 4-cyanophenyl | Me | Me | H | I | H | heptafluoroisopropyl | H | CF3 |
| 29-1015 | 2-chloropyridin-3-yl | Me | Me | H | I | H | heptafluoroisopropyl | H | CF3 |
| 29-1026 | 6-nitropyridin-3-yl | Me | C(O)Me | H | I | H | heptafluoroisopropyl | H | CF3 |
| 29-1028 | 5-fluoropyridin-3-yl | C(O)Me | Me | H | I | H | heptafluoroisopropyl | H | CF3 |
| 29-1057 | phenyl | Me | Me | H | I | H | nonafluoro-2-butyl | H | CF3 |
| 29-1058 | 2-fluorophenyl | Me | Me | H | I | H | nonafluoro-2-butyl | H | CF3 |
| 29-1077 | 3-cyanophenyl | Me | Me | H | I | H | nonafluoro-2-butyl | H | CF3 |
| 29-1078 | 4-cyanophenyl | Me | Me | H | I | H | nonafluoro-2-butyl | H | CF3 |
| 29-1093 | 2-chloropyridin-3-yl | Me | Me | H | I | H | nonafluoro-2-butyl | H | CF3 |
| 29-1331 | phenyl | Me | Me | H | OCF3 | H | heptafluoroisopropyl | H | OCF3 |
| 29-1362 | 2-chloropyridin-3-yl | Me | Me | H | OCF3 | H | heptafluoroisopropyl | H | OCF3 |
| 29-1387 | phenyl | Me | Me | H | OCF3 | H | nonafluoro-2-butyl | H | OCF3 |
| 29-1418 | 2-chloropyridin-3-yl | Me | Me | H | OCF3 | H | nonafluoro-2-butyl | H | OCF3 |
| 29-1443 | phenyl | Me | Me | H | CF3 | H | heptafluoroisopropyl | H | OCF3 |
| 29-1474 | 2-chloropyridin-3-yl | Me | Me | H | CF3 | H | heptafluoroisopropyl | H | OCF3 |
| 29-1499 | phenyl | Me | Me | H | CF3 | H | nonafluoro-2-butyl | H | OCF3 |

**Table 29(3)**

| compound number | Q₁ | R₁ | R₂ | X₃ | Y₁ | Y₂ | Y₃ | Y₄ | Y₅ |
|---|---|---|---|---|---|---|---|---|---|
| 29-1530 | 2-chloropyridin-3-yl | Me | Me | H | CF3 | H | nonafluoro-2-butyl | H | OCF3 |
| 29-1555 | phenyl | Me | Me | H | CF3 | H | heptafluoroisopropyl | H | CF3 |
| 29-1575 | 3-cyanophenyl | Me | Me | H | CF3 | H | heptafluoroisopropyl | H | CF3 |
| 29-1576 | 4-cyanophenyl | Me | Me | H | CF3 | H | heptafluoroisopropyl | H | CF3 |
| 29-1591 | 2-chloropyridin-3-yl | Me | Me | H | CF3 | H | heptafluoroisopropyl | H | CF3 |
| 29-1623 | phenyl | Me | Me | H | CF3 | H | nonafluoro-2-butyl | H | CF3 |
| 29-1643 | 3-cyanophenyl | Me | Me | H | CF3 | H | nonafluoro-2-butyl | H | CF3 |
| 29-1644 | 4-cyanophenyl | Me | Me | H | CF3 | H | nonafluoro-2-butyl | H | CF3 |
| 29-1659 | 2-chloropyridin-3-yl | Me | Me | H | CF3 | H | nonafluoro-2-butyl | H | CF3 |

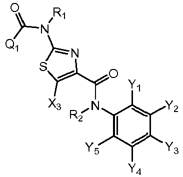

**Table 30(1)**

| compound number | Q₁ | R₁ | R₂ | X₃ | Y₁ | Y₂ | Y₃ | Y₄ | Y₅ |
|---|---|---|---|---|---|---|---|---|---|
| 30-9 | 3-bromophenyl | H | Et | H | Cl | F | heptafluoroisopropyl | F | OCF3 |
| 30-39 | 2-iodopyridin-3-yl | H | H | H | Cl | Me | nonafluoro-2-butyl | Me | OCF3 |
| 30-144 | phenyl | H | H | H | Cl | F | heptafluoroisopropyl | H | CF3 |
| 30-154 | 2-iodophenyl | H | H | H | Cl | F | heptafluoroisopropyl | Br | CF3 |
| 30-161 | 3-nitrophenyl | H | H | H | Cl | Br | heptafluoroisopropyl | Cl | CF3 |
| 30-168 | 2,4-dichlorophenyl | H | H | H | Cl | Me | nonafluoro-2-butyl | H | CF3 |
| 30-182 | 2-iodopyridin-3-yl | H | H | H | Cl | Me | nonafluoro-2-butyl | Me | n-C3F7 |
| 30-183 | 2-(trifluoromethyl)pyridin-3-yl | H | H | H | Cl | Me | nonafluoro-2-butyl | Et | i-C3F7 |
| 30-189 | 6-iodopyridin-3-yl | H | H | H | Cl | Et | nonafluoro-2-butyl | Me | n-C3F7 |
| 30-190 | 6-(trifluoromethyl)pyridin-3-yl | H | H | H | Cl | Et | nonafluoro-2-butyl | Et | i-C3F7 |
| 30-196 | 5-bromopyridin-3-yl | H | H | H | Br | n-Pr | heptafluoroisopropyl | Me | CF3 |
| 30-197 | 5-iodopyridin-3-yl | H | H | H | Br | n-Pr | heptafluoroisopropyl | Et | CF3 |
| 30-203 | 4-bromopyridin-3-yl | Me | n-Pr | H | Br | i-Pr | heptafluoroisopropyl | Me | CF3 |
| 30-204 | 4-iodopyridin-3-yl | H | H | H | Br | i-Pr | heptafluoroisopropyl | Et | CF3 |
| 30-210 | pyridin-4-yl | H | H | H | Br | n-Bu | heptafluoroisopropyl | Me | CF3 |
| 30-211 | 2-chloropyridin-4-yl | H | H | H | Br | n-Bu | heptafluoroisopropyl | Et | CF3 |
| 30-217 | pyridin-2-yl | H | H | H | Br | i-Bu | nonafluoro-2-butyl | Me | CF3 |
| 30-218 | 3-chloropyridin-2-yl | H | H | H | Br | i-Bu | nonafluoro-2-butyl | Et | CF3 |
| 30-224 | 2 -fluorophenyl | H | H | H | Br | s-Bu | nonafluoro-2-butyl | Me | CF3 |
| 30-225 | 3-fluorophenyl | H | H | H | Br | s-Bu | nonafluoro-2-butyl | Et | CF3 |
| 30-231 | 3-bromophenyl | H | H | H | Br | F | nonafluoro-2-butyl | Me | CF3 |
| 30-232 | 4-bromophenyl | H | H | H | Br | F | nonafluoro-2-butyl | Et | CF3 |
| 30-238 | (4-trifluoromethyl)phenyl | Et | i-Pr | H | Br | Cl | nonafluoro-2-butyl | Me | CF3 |
| 30-239 | 2-nitrophenyl | H | H | H | Br | Cl | nonafluoro-2-butyl | Et | CF3 |
| 30-245 | 4-cyanophenyl | H | H | H | I | Br | heptafluoroisopropyl | Me | CF3 |
| 30-246 | 2,6-difluorophenyl | H | H | H | I | Br | heptafluoroisopropyl | Et | CF3 |
| 30-252 | 2-bromo-4-chlorophenyl | n-Pr | Me | H | I | I | heptafluoroisopropyl | Me | CF3 |
| 30-253 | 2-bromo-4-fluorophenyl | H | H | H | I | I | heptafluoroisopropyl | Et | CF3 |
| 30-259 | 2-fluoropyridin-3-yl | H | H | H | I | Me | heptafluoroisopropyl | F | C2F5 |
| 30-260 | 2-chloropyridin-3-yl | H | H | H | I | Me | nonafluoro-2-butyl | Cl | CF3 |
| 30-263 | 2-(trifluoromethyl)pyridin-3-yl | H | H | H | I | Et | nonafluoro-2-butyl | F | CF3 |
| 30-264 | 2-nitropyridin-3-yl | H | H | H | I | Et | nonafluoro-2-butyl | Cl | CF3 |
| 30-267 | 6-chloropyridin-3-yl | H | H | H | I | n-Pr | nonafluoro-2-butyl | F | CF3 |
| 30-268 | 6-bromopyridin-3-yl | H | H | H | I | n-Pr | nonafluoro-2-butyl | Cl | CF3 |
| 30-271 | 6-nitropyridin-3-yl | H | H | H | I | i-Pr | nonafluoro-2-butyl | F | i-C3F7 |
| 30-272 | 6-cyanopyridin-3-yl | H | H | H | I | i-Pr | nonafluoro-2-butyl | Cl | CF3 |
| 30-275 | 5-bromopyridin-3-yl | H | H | H | I | n-Bu | nonafluoro-2-butyl | F | CF3 |
| 30-276 | 5-iodopyridin-3-yl | H | H | H | I | n-Bu | nonafluoro-2-butyl | Cl | CF3 |
| 30-279 | 5-cyanopyridin-3-yl | H | H | H | I | i-Bu | nonafluoro-2-butyl | F | CF3 |
| 30-280 | 4-fluoropyridin-3-yl | H | H | H | I | i-Bu | nonafluoro-2-butyl | Cl | CF3 |
| 30-283 | 4-iodopyridin-3-yl | i-Pr | n-Pr | H | I | s-Bu | nonafluoro-2-butyl | F | CF3 |
| 30-284 | 4-(trifluoromethyl)pyridin-3-yl | H | H | H | I | s-Bu | nonafluoro-2-butyl | Cl | CF3 |
| 30-295 | 3-bromophenyl | H | Et | H | OCF3 | F | heptafluoroisopropyl | F | OCF3 |
| 30-325 | 2-iodopyridin-3-yl | H | H | H | OCF3 | Me | nonafluoro-2-butyl | Me | OCF3 |
| 30-438 | 3-bromophenyl | H | Et | H | OCF3 | F | heptafluoroisopropyl | F | CF3 |
| 30-468 | 2-iodopyridin-3-yl | H | H | H | OCF3 | Me | nonafluoro-2-butyl | Me | CF3 |
| 30-581 | 3-bromophenyl | H | Et | H | CF3 | F | heptafluoroisopropyl | F | CF3 |
| 30-611 | 2-iodopyridin-3-yl | H | H | H | CF3 | Me | nonafluoro-2-butyl | Me | CF3 |

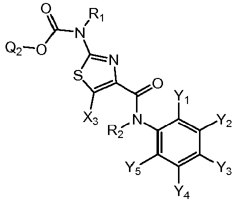

**Table 31 (1)**

| compound number | Q₂ | R₁ | R₂ | X₃ | Y₁ | Y₂ | Y₃ | Y₄ | Y₅ |
|---|---|---|---|---|---|---|---|---|---|
| 31-1 | 2,2,2-trichloroethyl | H | H | H | F | H | heptafluoroisopropyl | H | OCF3 |
| 31-2 | 2,2,2-trichloroethyl | H | H | H | F | H | nonafluoro-2-butyl | H | OCF3 |
| 31-9 | 2-chloroethyl | H | H | Cl | Cl | H | heptafluoroisopropyl | H | OCF3 |
| 31-13 | 2,2,2-trichloroethyl | H | H | H | Cl | H | heptafluoroisopropyl | H | OCF3 |
| 31-16 | 3-fluoro-n-propyl | H | H | H | Cl | H | pentafluoroethyl | H | OCF3 |
| 31-18 | 3-bromo-n-propyl | H | H | H | Cl | H | heptafluoroisopropyl | H | OC2F5 |
| 31-22 | 1,3-dichloro-2-propyl | H | H | H | Cl | H | pentafluoroethyl | H | OC2F5 |
| 31-26 | 4,4,4-trifl uoro-n-butyl | H | H | H | Cl | H | heptafluoroisopropyl | H | OCF3 |
| 31-36 | 2-bromoethyl | H | H | H | Cl | H | nonafluoro-2-butyl | H | OC2F5 |
| 31-42 | 2,2,2-trichloroethyl | H | H | H | Cl | H | nonafluoro-2-butyl | H | OCF3 |
| 31-55 | 4,4,4-trifl uoro-n-butyl | H | H | H | Cl | H | nonafluoro-2-butyl | H | OCF3 |
| 31-57 | s-butyl | H | H | H | Cl | H | nonafluoro-2-butyl | H | OC2F5 |
| 31-66 | 2-iodoethyl | H | H | H | Br | H | heptafluoroisopropyl | H | OC2F5 |
| 31-71 | 2,2,2-trichloroethyl | H | H | H | Br | H | heptafluoroisopropyl | H | OCF3 |
| 31-84 | 4,4,4-trifluoro-n-butyl | H | H | H | Br | H | heptafluoroisopropyl | H | OCF3 |
| 31-100 | 2,2,2-trichloroethyl | H | H | H | Br | H | nonafluoro-2-butyl | H | OCF3 |
| 31-102 | n-propyl | H | H | H | Br | H | nonafluoro-2-butyl | H | OC2F5 |
| 31-113 | 4,4,4-trifluoro-n-butyl | H | H | H | Br | H | nonafluoro-2-butyl | H | OCF3 |
| 31-129 | 2,2,2-trichloroethyl | H | H | H | I | H | heptafluoroisopropyl | H | OCF3 |
| 31-133 | 3-chloro-n-propyl | H | H | H | I | H | heptafluoroisopropyl | H | OC2F5 |
| 31-142 | 4,4,4-trifluoro-n-butyl | H | H | H | I | H | heptafluoroisopropyl | H | OCF3 |
| 31-158 | 2,2,2-trichloroethyl | H | H | H | I | H | nonafluoro-2-butyl | H | OCF3 |
| 31-171 | 4,4,4-trifl uoro-n-butyl | H | H | H | I | H | nonafluoro-2-butyl | H | OCF3 |
| 31-175 | benzyl | H | H | H | I | H | nonafluoro-2-butyl | H | OC2F5 |
| 31-177 | 2,2,2-trichloroethyl | H | H | H | F | H | heptafluoroisopropyl | H | CF3 |
| 31-178 | 2,2,2-trichloroethyl | H | H | H | F | H | nonafluoro-2-butyl | H | CF3 |
| 31-179 | methyl | H | H | H | Cl | H | heptafluoroisopropyl | H | CF3 |
| 31-180 | ethyl | H | H | H | Cl | H | heptafluoroisopropyl | H | CF3 |
| 31-181 | 2-fluoroethyl | H | H | H | Cl | H | heptafluoroisopropyl | H | CF3 |
| 31-182 | 2-chloroethyl | H | H | H | Cl | H | heptafluoroisopropyl | H | CF3 |
| 31-183 | 2-bromoethyl | H | H | H | Cl | H | heptafluoroisopropyl | H | CF3 |
| 31-184 | 2-iodoethyl | H | H | H | Cl | H | heptafluoroisopropyl | H | CF3 |
| 31-185 | 2-cyanoethyl | H | H | H | Cl | H | heptafluoroisopropyl | H | C2F5 |
| 31-186 | 2,2-difluoroethyl | H | H | H | Cl | H | heptafluoroisopropyl | H | CF3 |
| 31-187 | 2,2-dichloroethyl | H | H | H | Cl | H | heptafluoroisopropyl | H | CF3 |
| 31-188 | 2,2,2-trifluoroethyl | H | H | H | Cl | H | heptafluoroisopropyl | H | CF3 |
| 31-189 | 2,2,2-trichloroethyl | H | H | H | Cl | H | heptafluoroisopropyl | H | CF3 |
| 31-190 | 2,2,2-tribromoethyl | H | H | H | Cl | H | heptafluoroisopropyl | H | CF3 |
| 31-191 | n-propyl | H | H | H | Cl | H | pentafluoroethyl | H | CF3 |
| 31-192 | 3-fluoro-n-propyl | H | H | H | Cl | H | heptafluoroisopropyl | H | CF3 |
| 31-193 | 3-chloro-n-propyl | H | H | H | Cl | H | heptafluoroisopropyl | H | CF3 |
| 31-194 | 3-bromo-n-propyl | H | H | H | Cl | H | heptafluoroisopropyl | H | CF3 |
| 31-195 | i-propyl | H | H | H | Cl | H | heptafluoroisopropyl | H | n-C3F7 |
| 31-196 | 1,2-difluoro-2-propyl | H | H | H | Cl | H | heptafluoroisopropyl | H | CF3 |
| 31-197 | 1,3-difluoro-2-propyl | H | H | H | Cl | H | heptafluoroisopropyl | H | CF3 |
| 31-198 | 1,3-dichloro-2-propyl | H | H | H | Cl | H | pentafluoroethyl | H | CF3 |
| 31-199 | 1-chloro-3-fluoro-2-propyl | H | H | H | Cl | H | heptafluoroisopropyl | H | CF3 |
| 31-200 | 3,3,3-trifluoro-n-propyl | H | H | H | Cl | H | heptafluoroisopropyl | H | CF3 |
| 31-201 | n-butyl | H | H | H | Cl | H | heptafluoroisopropyl | H | CF3 |

**Table 31(2)**

| compound number | Q₂ | R₁ | R₂ | X₃ | Y₁ | Y₂ | Y₃ | Y₄ | Y₅ |
|---|---|---|---|---|---|---|---|---|---|
| 31-202 | 4,4,4-trifl uoro-n-butyl | H | H | H | Cl | H | heptafluoroisopropyl | H | CF3 |
| 31-203 | i-butyl | H | H | Cl | Cl | H | heptafluoroisopropyl | H | CF3 |
| 31-204 | s-butyl | H | H | H | Cl | H | heptafluoroisopropyl | H | CF3 |
| 31-205 | vinyl | H | H | H | Cl | H | heptafluoroisopropyl | H | CF3 |
| 31-206 | benzyl | H | H | H | Cl | H | heptafluoroisopropyl | H | CF3 |
| 31-207 | phenyl | H | H | H | Cl | H | heptafluoroisopropyl | H | CF3 |
| 31-208 | methyl | H | H | H | Cl | H | nonafluoro-2-butyl | H | CF3 |
| 31-209 | ethyl | H | H | H | Cl | H | nonafluoro-2-butyl | H | i-C3F7 |
| 31-210 | 2-fluoroethyl | H | H | H | Cl | H | nonafluoro-2-butyl | H | CF3 |
| 31-211 | 2-chloroethyl | H | H | H | Cl | H | nonafluoro-2-butyl | H | CF3 |
| 31-212 | 2-bromoethyl | H | H | H | Cl | H | nonafluoro-2-butyl | H | CF3 |
| 31-213 | 2-iodoethyl | H | H | H | Cl | H | nonafluoro-2-butyl | H | CF3 |
| 31-214 | 2-cyanoethyl | H | H | H | Cl | H | nonafluoro-2-butyl | H | CF3 |
| 31-215 | 2,2-difluoroethyl | H | H | H | Cl | H | nonafluoro-2-butyl | H | CF3 |
| 31-216 | 2,2-dichloroethyl | H | H | H | Cl | H | nonafluoro-2-butyl | H | CF3 |
| 31-217 | 2,2,2-trifluoroethyl | H | H | H | Cl | H | nonafluoro-2-butyl | H | CF3 |
| 31-218 | 2,2,2-trichloroethyl | H | H | H | Cl | H | nonafluoro-2-butyl | H | CF3 |
| 31-219 | 2,2,2-tribromoethyl | H | H | H | Cl | H | nonafluoro-2-butyl | H | CF3 |
| 31-220 | n-propyl | H | H | H | Cl | H | nonafluoro-2-butyl | H | CF3 |
| 31-221 | 3-fluoro-n-propyl | H | H | H | Cl | H | nonafluoro-2-butyl | H | CF3 |
| 31-222 | 3-chloro-n-propyl | H | H | H | Cl | H | nonafluoro-2-butyl | H | CF3 |
| 31-223 | 3-bromo-n-propyl | H | H | H | Cl | H | nonafluoro-2-butyl | H | CF3 |
| 31-224 | i-propyl | H | H | H | Cl | H | nonafluoro-2-butyl | H | CF3 |
| 31-225 | 1,2-difluoro-2-propyl | H | H | H | Cl | H | nonafluoro-2-butyl | H | CF3 |
| 31-226 | 1,3-difluoro-2-propyl | H | H | H | Cl | H | nonafluoro-2-butyl | H | CF3 |
| 31-227 | 1,3-dichloro-2-propyl | H | H | H | Cl | H | nonafluoro-2-butyl | H | CF3 |
| 31-228 | 1-chloro-3-fluoro-2-propyl | H | H | H | Cl | H | nonafluoro-2-butyl | H | CF3 |
| 31-229 | 3,3,3-trifluoro-n-propyl | H | H | H | Cl | H | nonafluoro-2-butyl | H | CF3 |
| 31-230 | n-butyl | H | H | H | Cl | H | nonafluoro-2-butyl | H | CF3 |
| 31-231 | 4,4,4-trifl uoro-n-butyl | H | H | H | Cl | H | nonafluoro-2-butyl | H | CF3 |
| 31-232 | i-butyl | H | H | H | Cl | H | nonafluoro-2-butyl | H | CF3 |
| 31-233 | s-butyl | H | H | H | Cl | H | nonafluoro-2-butyl | H | CF3 |
| 31-234 | vinyl | H | H | H | Cl | H | nonafluoro-2-butyl | H | CF3 |
| 31-235 | benzyl | H | H | H | Cl | H | nonafluoro-2-butyl | H | CF3 |
| 31-236 | phenyl | H | H | H | Cl | H | nonafluoro-2-butyl | H | CF3 |
| 31-237 | methyl | H | H | H | Br | H | heptafluoroisopropyl | H | CF3 |
| 31-238 | ethyl | H | H | H | Br | H | heptafluoroisopropyl | H | CF3 |
| 31-239 | 2-fluoroethyl | H | H | H | Br | H | heptafluoroisopropyl | H | CF3 |
| 31-240 | 2-chloroethyl | H | H | H | Br | H | heptafluoroisopropyl | H | CF3 |
| 31-241 | 2-bromoethyl | H | H | H | Br | H | heptafluoroisopropyl | H | CF3 |
| 31-242 | 2-iodoethyl | H | H | H | Br | H | heptafluoroisopropyl | H | CF3 |
| 31-243 | 2-cyanoethyl | H | H | H | Br | H | heptafluoroisopropyl | H | C2F5 |
| 31-244 | 2,2-difluoroethyl | H | H | H | Br | H | heptafluoroisopropyl | H | CF3 |
| 31-245 | 2,2-dichloroethyl | H | H | H | Br | H | heptafluoroisopropyl | H | CF3 |
| 31-246 | 2,2,2-trifluoroethyl | H | H | H | Br | H | heptafluoroisopropyl | H | CF3 |
| 31-247 | 2,2,2-trichloroethyl | H | H | H | Br | H | heptafluoroisopropyl | H | CF3 |
| 31-248 | 2,2,2-tribromoethyl | H | H | H | Br | H | heptafluoroisopropyl | H | CF3 |
| 31-249 | n-propyl | H | H | H | Br | H | heptafluoroisopropyl | H | CF3 |
| 31-250 | 3-fluoro-n-propyl | H | H | H | Br | H | heptafluoroisopropyl | H | CF3 |
| 31-251 | 3-chloro-n-propyl | H | H | H | Br | H | heptafluoroisopropyl | H | CF3 |
| 31-252 | 3-bromo-n-propyl | H | H | H | Br | H | heptafluoroisopropyl | H | CF3 |
| 31-253 | i-propyl | H | H | H | Br | H | heptafluoroisopropyl | H | CF3 |
| 31-254 | 1,2-difluoro-2-propyl | H | H | H | Br | H | heptafluoroisopropyl | H | n-C3F7 |
| 31-255 | 1,3-difluoro-2-propyl | H | H | H | Br | H | heptafluoroisopropyl | H | CF3 |
| 31-256 | 1,3-dichloro-2-propyl | H | H | H | Br | H | heptafluoroisopropyl | H | CF3 |
| 31-257 | 1-chloro-3-fluoro-2-propyl | H | H | H | Br | H | heptafluoroisopropyl | H | CF3 |
| 31-258 | 3,3,3-trifluoro-n-propyl | H | H | H | Br | H | heptafluoroisopropyl | H | CF3 |
| 31-259 | n-butyl | H | H | H | Br | H | heptafluoroisopropyl | H | CF3 |
| 31-260 | 4,4,4-trifluoro-n-butyl | H | H | H | Br | H | heptafluoroisopropyl | H | CF3 |

**Table 31(3)**

| compound number | Q₂ | R₁ | R₂ | X₃ | Y₁ | Y₂ | Y₃ | Y₄ | Y₅ |
|---|---|---|---|---|---|---|---|---|---|
| 31-261 | i-butyl | H | H | H | Br | H | heptafluoroisopropyl | H | CF3 |
| 31-262 | s-butyl | H | H | H | Br | H | heptafluoroisopropyl | H | CF3 |
| 31-263 | vinyl | H | H | H | Br | H | heptafluoroisopropyl | H | CF3 |
| 31-264 | benzyl | H | H | H | Br | H | heptafluoroisopropyl | H | CF3 |
| 31-265 | phenyl | H | H | H | Br | H | heptafluoroisopropyl | H | CF3 |
| 31-266 | methyl | H | H | H | Br | H | nonafluoro-2-butyl | H | CF3 |
| 31-267 | ethyl | H | H | H | Br | H | nonafluoro-2-butyl | H | CF3 |
| 31-268 | 2-fluoroethyl | H | H | H | Br | H | nonafluoro-2-butyl | H | CF3 |
| 31-269 | 2-chloroethyl | H | H | H | Br | H | nonafluoro-2-butyl | H | CF3 |
| 31-270 | 2-bromoethyl | H | H | H | Br | H | nonafluoro-2-butyl | H | CF3 |
| 31-271 | 2-iodoethyl | H | H | H | Br | H | nonafluoro-2-butyl | H | CF3 |
| 31-272 | 2-cyanoethyl | H | H | H | Br | H | nonafluoro-2-butyl | H | CF3 |
| 31-273 | 2,2-difluoroethyl | H | H | H | Br | H | nonafluoro-2-butyl | H | CF3 |
| 31-274 | 2,2-dichloroethyl | H | H | H | Br | H | nonafluoro-2-butyl | H | CF3 |
| 31-275 | 2,2,2-trifluoroethyl | H | H | H | Br | H | nonafluoro-2-butyl | H | CF3 |
| 31-276 | 2,2,2-trichloroethyl | H | H | H | Br | H | nonafluoro-2-butyl | H | CF3 |
| 31-277 | 2,2,2-tribromoethyl | H | H | H | Br | H | nonafluoro-2-butyl | H | CF3 |
| 31-278 | n-propyl | H | H | H | Br | H | nonafluoro-2-butyl | H | CF3 |
| 31-279 | 3-fluoro-n-propyl | H | H | H | Br | H | nonafluoro-2-butyl | H | CF3 |
| 31-280 | 3-chloro-n-propyl | H | H | H | Br | H | nonafluoro-2-butyl | H | CF3 |
| 31-281 | 3-bromo-n-propyl | H | H | H | Br | H | nonafluoro-2-butyl | H | CF3 |
| 31-282 | i-propyl | H | H | H | Br | H | nonafluoro-2-butyl | H | CF3 |
| 31-283 | 1,2-difluoro-2-propyl | H | H | H | Br | H | nonafluoro-2-butyl | H | CF3 |
| 31-284 | 1,3-difluoro-2-propyl | H | H | H | Br | H | nonafluoro-2-butyl | H | CF3 |
| 31-285 | 1,3-dichloro-2-propyl | H | H | H | Br | H | nonafluoro-2-butyl | H | CF3 |
| 31-286 | 1-chloro-3-fluoro-2-propyl | H | H | H | Br | H | nonafluoro-2-butyl | H | CF3 |
| 31-287 | 3,3,3-trifluoro-n-propyl | H | H | H | Br | H | nonafluoro-2-butyl | H | CF3 |
| 31-288 | n-butyl | H | H | H | Br | H | nonafluoro-2-butyl | H | CF3 |
| 31-289 | 4,4,4-trifluoro-n-butyl | H | H | H | Br | H | nonafluoro-2-butyl | H | CF3 |
| 31-290 | i-butyl | H | H | H | Br | H | nonafluoro-2-butyl | H | CF3 |
| 31-291 | s-butyl | H | H | H | Br | H | nonafluoro-2-butyl | H | CF3 |
| 31-292 | vinyl | H | H | H | Br | H | nonafluoro-2-butyl | H | CF3 |
| 31-293 | benzyl | H | H | H | Br | H | nonafluoro-2-butyl | H | CF3 |
| 31-294 | phenyl | H | H | H | Br | H | nonafluoro-2-butyl | H | i-C3F7 |
| 31-295 | methyl | H | H | H | I | H | heptafluoroisopropyl | H | CF3 |
| 31-296 | ethyl | H | H | H | I | H | heptafluoroisopropyl | H | CF3 |
| 31-297 | 2-fluoroethyl | H | H | H | I | H | heptafluoroisopropyl | H | CF3 |
| 31-298 | 2-chloroethyl | H | H | H | I | H | heptafluoroisopropyl | H | CF3 |
| 31-299 | 2-bromoethyl | H | H | H | I | H | heptafluoroisopropyl | H | CF3 |
| 31-300 | 2-iodoethyl | H | H | H | I | H | heptafluoroisopropyl | H | CF3 |
| 31-301 | 2-cyanoethyl | H | H | H | I | H | heptafluoroisopropyl | H | CF3 |
| 31-302 | 2,2-difluoroethyl | H | H | H | I | H | heptafluoroisopropyl | H | CF3 |
| 31-303 | 2,2-dichloroethyl | H | H | H | I | H | heptafluoroisopropyl | H | CF3 |
| 31-304 | 2,2,2-trifluoroethyl | H | H | H | I | H | heptafluoroisopropyl | H | CF3 |
| 31-305 | 2,2,2-trichloroethyl | H | H | H | I | H | heptafluoroisopropyl | H | CF3 |
| 31-306 | 2,2,2-tribromoethyl | H | H | H | I | H | heptafluoroisopropyl | H | CF3 |
| 31-307 | n-propyl | H | H | H | I | H | heptafluoroisopropyl | H | CF3 |
| 31-308 | 3-fluoro-n-propyl | H | H | H | I | H | heptafluoroisopropyl | H | CF3 |
| 31-309 | 3-chloro-n-propyl | H | H | H | I | H | heptafluoroisopropyl | H | CF3 |
| 31-310 | 3-bromo-n-propyl | H | H | H | I | H | heptafluoroisopropyl | H | C2F5 |
| 31-311 | i-propyl | H | H | H | I | H | heptafluoroisopropyl | H | CF3 |
| 31-312 | 1,2-difluoro-2-propyl | H | H | H | I | H | heptafluoroisopropyl | H | CF3 |
| 31-313 | 1,3-difluoro-2-propyl | H | H | H | I | H | heptafluoroisopropyl | H | CF3 |
| 31-314 | 1,3-dichloro-2-propyl | H | H | H | I | H | heptafluoroisopropyl | H | CF3 |
| 31-315 | 1-chloro-3-fluoro-2-propyl | H | H | H | I | H | heptafluoroisopropyl | H | CF3 |
| 31-316 | 3,3,3-trifluoro-n-propyl | H | H | H | I | H | heptafluoroisopropyl | H | CF3 |
| 31-317 | n-butyl | H | H | H | I | H | heptafluoroisopropyl | H | CF3 |
| 31-318 | 4,4,4-trifl uoro-n-butyl | H | H | H | I | H | heptafluoroisopropyl | H | CF3 |
| 31-319 | i-butyl | H | H | H | I | H | heptafluoroisopropyl | H | CF3 |
| 31-320 | s-butyl | H | H | H | I | H | heptafluoroisopropyl | H | CF3 |

**Table 31(4)**

| compound number | Q₂ | R₁ | R₂ | X₃ | Y₁ | Y₂ | Y₃ | Y₄ | Y₅ |
|---|---|---|---|---|---|---|---|---|---|
| 31-321 | vinyl | H | H | H | I | H | heptafluoroisopropyl | H | CF3 |
| 31-322 | benzyl | H | H | H | I | H | heptafluoroisopropyl | H | CF3 |
| 31-323 | phenyl | H | H | H | I | H | heptafluoroisopropyl | H | CF3 |
| 31-324 | methyl | H | H | H | I | H | nonafluoro-2-butyl | H | CF3 |
| 31-325 | ethyl | H | H | H | I | H | nonafluoro-2-butyl | H | CF3 |
| 31-326 | 2-fluoroethyl | H | H | H | I | H | nonafluoro-2-butyl | H | CF3 |
| 31-327 | 2-chloroethyl | H | H | H | I | H | nonafluoro-2-butyl | H | CF3 |
| 31-328 | 2-bromoethyl | H | H | H | I | H | nonafluoro-2-butyl | H | CF3 |
| 31-329 | 2-iodoethyl | H | H | H | I | H | nonafluoro-2-butyl | H | CF3 |
| 31-330 | 2-cyanoethyl | H | H | H | I | H | nonafluoro-2-butyl | H | CF3 |
| 31-331 | 2,2-difluoroethyl | H | H | H | I | H | nonafluoro-2-butyl | H | CF3 |
| 31-332 | 2,2-dichloroethyl | H | H | H | I | H | nonafluoro-2-butyl | H | CF3 |
| 31-333 | 2,2,2-trifluoroethyl | H | H | H | I | H | nonafluoro-2-butyl | H | CF3 |
| 31-334 | 2,2,2-trichloroethyl | H | H | H | I | H | nonafluoro-2-butyl | H | CF3 |
| 31-335 | 2,2,2-tribromoethyl | H | H | H | I | H | nonafluoro-2-butyl | H | CF3 |
| 31-336 | n-propyl | H | H | H | I | H | nonafluoro-2-butyl | H | n-C3F7 |
| 31-337 | 3-fluoro-n-propyl | H | H | H | I | H | nonafluoro-2-butyl | H | CF3 |
| 31-338 | 3-chloro-n-propyl | H | H | H | I | H | nonafluoro-2-butyl | H | CF3 |
| 31-339 | 3-bromo-n-propyl | H | H | H | I | H | nonafluoro-2-butyl | H | CF3 |
| 31-340 | i-propyl | H | H | H | I | H | nonafluoro-2-butyl | H | CF3 |
| 31-341 | 1,2-difluoro-2-propyl | H | H | H | I | H | nonafluoro-2-butyl | H | CF3 |
| 31-342 | 1,3-difluoro-2-propyl | H | H | H | I | H | nonafluoro-2-butyl | H | CF3 |
| 31-343 | 1,3-dichloro-2-propyl | H | H | H | I | H | nonafluoro-2-butyl | H | CF3 |
| 31-344 | 1-chloro-3-fluoro-2-propyl | H | H | H | I | H | nonafluoro-2-butyl | H | CF3 |
| 31-345 | 3,3,3-trifluoro-n-propyl | H | H | H | I | H | nonafluoro-2-butyl | H | CF3 |
| 31-346 | n-butyl | H | H | H | I | H | nonafluoro-2-butyl | H | CF3 |
| 31-347 | 4,4,4-trifl uoro-n-butyl | H | H | H | I | H | nonafluoro-2-butyl | H | CF3 |
| 31-348 | i-butyl | H | H | H | I | H | nonafluoro-2-butyl | H | CF3 |
| 31-349 | s-butyl | H | H | H | I | H | nonafluoro-2-butyl | H | CF3 |
| 31-350 | vinyl | H | H | H | I | H | nonafluoro-2-butyl | H | i-C3F7 |
| 31-351 | benzyl | H | H | H | I | H | nonafluoro-2-butyl | H | CF3 |
| 31-352 | phenyl | H | H | H | I | H | nonafluoro-2-butyl | H | CF3 |
| 31-359 | 2-cyanoethyl | H | H | H | OCF3 | H | heptafluoroisopropyl | H | OC2F5 |
| 31-363 | 2,2,2-trichloroethyl | H | H | H | OCF3 | H | heptafluoroisopropyl | H | OCF3 |
| 31-364 | 2,2,2-tribromoethyl | H | H | H | OCF3 | H | pentafluoroethyl | H | OCF3 |
| 31-373 | 1-chloro-3-fluoro-2-propyl | H | H | H | OC2F5 | H | heptafluoroisopropyl | H | OC2F5 |
| 31-376 | 4,4,4-trifluoro-n-butyl | H | H | H | OCF3 | H | heptafluoroisopropyl | H | OCF3 |
| 31-378 | s-butyl | H | H | H | OC2F5 | H | heptafluoroisopropyl | H | OCF3 |
| 31-391 | 2,2,2-trifluoroethyl | H | H | H | OC2F5 | H | nonafluoro-2-butyl | H | OCF3 |
| 31-392 | 2,2,2-trichloroethyl | H | H | H | OCF3 | H | nonafluoro-2-butyl | H | OCF3 |
| 31-394 | n-propyl | H | H | Cl | OCF3 | H | nonafluoro-2-butyl | H | OC2F5 |
| 31-403 | 3,3,3-trifluoro-n-propyl | H | H | H | OC2F5 | H | nonafluoro-2-butyl | H | OC2F5 |
| 31-405 | 4,4,4-trifl uoro-n-butyl | H | H | H | OCF3 | H | nonafluoro-2-butyl | H | OCF3 |
| 31-417 | 2-cyanoethyl | H | H | H | CF3 | H | heptafluoroisopropyl | H | OC2F5 |
| 31-421 | 2,2,2-trichloroethyl | H | H | H | CF3 | H | heptafluoroisopropyl | H | OCF3 |
| 31-423 | n-propyl | H | H | H | C2F5 | H | heptafluoroisopropyl | H | OC2F5 |
| 31-427 | i-propyl | H | H | H | CF3 | H | pentafluoroethyl | H | OCF3 |
| 31-432 | 3,3,3-trifluoro-n-propyl | H | H | Cl | n-C3F7 | H | heptafluoroisopropyl | H | OCF3 |
| 31-434 | 4,4,4-trifluoro-n-butyl | H | H | H | CF3 | H | heptafluoroisopropyl | H | OCF3 |
| 31-437 | vinyl | H | H | H | i-C3F7 | H | heptafluoroisopropyl | H | OCF3 |
| 31-445 | 2-iodoethyl | H | H | H | C2F5 | H | nonafluoro-2-butyl | H | OCF3 |
| 31-450 | 2,2,2-trichloroethyl | H | H | H | CF3 | H | nonafluoro-2-butyl | H | OCF3 |
| 31-452 | n-propyl | H | H | H | n-C3F7 | H | nonafluoro-2-butyl | H | OCF3 |
| 31-456 | i-propyl | H | H | H | CF3 | H | nonafluoro-2-butyl | H | OC2F5 |
| 31-463 | 4,4,4-trifl uoro-n-butyl | H | H | H | CF3 | H | nonafluoro-2-butyl | H | OCF3 |
| 31-464 | i-butyl | H | H | H | i-C3F7 | H | nonafluoro-2-butyl | H | OCF3 |
| 31-467 | benzyl | H | H | H | n-C3F7 | H | nonafluoro-2-butyl | H | OC2F5 |
| 31-468 | phenyl | H | H | H | i-C3F7 | H | nonafluoro-2-butyl | H | OC2F5 |

**Table 31(5)**

| compound number | Q₂ | R₁ | R₂ | X₃ | Y₁ | Y₂ | Y₃ | Y₄ | Y₅ |
|---|---|---|---|---|---|---|---|---|---|
| 31-479 | 2,2,2-trichloroethyl | H | H | H | CF3 | H | heptafluoroisopropyl | H | CF3 |
| 31-482 | 3-fluoro-n-propyl | H | H | H | C2F5 | H | heptafluoroisopropyl | H | CF3 |
| 31-485 | i-propyl | H | H | H | i-C3F7 | H | heptafluoroisopropyl | H | CF3 |
| 31-487 | 1,3-difluoro-2-propyl | H | H | H | CF3 | H | pentafluoroethyl | H | CF3 |
| 31-490 | 3,3,3-trifluoro-n-propyl | H | H | H | C2F5 | H | heptafluoroisopropyl | H | C2F5 |
| 31-492 | 4,4,4-trifluoro-n-butyl | H | H | H | CF3 | H | heptafluoroisopropyl | H | CF3 |
| 31-493 | i-butyl | H | H | H | C2F5 | H | heptafluoroisopropyl | H | n-C3F7 |
| 31-495 | vinyl | H | H | H | n-C3F7 | H | heptafluoroisopropyl | H | CF3 |
| 31-508 | 2,2,2-trichloroethyl | H | H | H | CF3 | H | nonafluoro-2-butyl | H | CF3 |
| 31-520 | n-butyl | H | H | H | C2F5 | H | nonafluoro-2-butyl | H | i-C3F7 |
| 31-521 | 4,4,4-trifluoro-n-butyl | H | H | H | CF3 | H | nonafluoro-2-butyl | H | CF3 |
| 31-522 | i-butyl | H | H | Cl | CF3 | H | nonafluoro-2-butyl | H | CF3 |
| 31-524 | vinyl | H | H | H | n-C3F7 | H | nonafluoro-2-butyl | H | i-C3F7 |

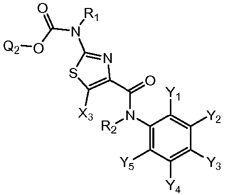

**Table 32(1)**

| compound number | Q₂ | R₁ | R₂ | X₃ | Y₁ | Y₂ | Y₃ | Y₄ | Y₅ |
|---|---|---|---|---|---|---|---|---|---|
| 32-1 | 2,2,2-trichloroethyl | Me | H | H | F | H | heptafluoroisopropyl | H | OCF3 |
| 32-2 | 2,2,2-trichloroethyl | Me | H | H | F | H | nonafluoro-2-butyl | H | OCF3 |
| 32-13 | 2,2,2-trichloroethyl | Me | H | H | Cl | H | heptafluoroisopropyl | H | OCF3 |
| 32-42 | 2,2,2-trichloroethyl | Me | H | H | Cl | H | nonafluoro-2-butyl | H | OCF3 |
| 32-71 | 2,2,2-trichloroethyl | Me | H | H | Br | H | heptafluoroisopropyl | H | OCF3 |
| 32-100 | 2,2,2-trichloroethyl | Me | H | H | Br | H | nonafluoro-2-butyl | H | OCF3 |
| 32-129 | 2,2,2-trichloroethyl | Me | H | H | I | H | heptafluoroisopropyl | H | OCF3 |
| 32-158 | 2,2,2-trichloroethyl | Me | H | H | I | H | nonafluoro-2-butyl | H | OCF3 |
| 32-177 | 2,2,2-trichloroethyl | Me | H | H | F | H | heptafluoroisopropyl | H | CF3 |
| 32-178 | 2,2,2-trichloroethyl | Me | H | H | F | H | nonafluoro-2-butyl | H | CF3 |
| 32-185 | 2-cyanoethyl | Et | H | H | Cl | H | heptafluoroisopropyl | H | C2F5 |
| 32-189 | 2,2,2-trichloroethyl | Me | H | H | Cl | H | heptafluoroisopropyl | H | CF3 |
| 32-191 | n-propyl | Me | H | H | Cl | H | pentafluoroethyl | H | CF3 |
| 32-195 | i-propyl | n-Pr | H | H | Cl | H | heptafluoroisopropyl | H | n-C3F7 |
| 32-198 | 1,3-dichloro-2-propyl | Me | H | H | Cl | H | pentafluoroethyl | H | CF3 |
| 32-199 | 1-chloro-3-fluoro-2-propyl | i-Pr | H | H | Cl | H | heptafluoroisopropyl | H | CF3 |
| 32-202 | 4,4,4-trifluoro-n-butyl | Me | H | H | Cl | H | heptafluoroisopropyl | H | CF3 |
| 32-203 | i-butyl | CH2CH=CH2 | H | H | Cl | H | heptafluoroisopropyl | H | CF3 |
| 32-207 | phenyl | Me | H | Cl | Cl | H | heptafluoroisopropyl | H | CF3 |
| 32-209 | ethyl | Me | H | H | Cl | H | nonafluoro-2-butyl | H | i-C3F7 |
| 32-214 | 2-cyanoethyl | CN | H | H | Cl | H | nonafluoro-2-butyl | H | CF3 |
| 32-218 | 2,2,2-trichloroethyl | Me | H | H | Cl | H | nonafluoro-2-butyl | H | CF3 |
| 32-220 | n-propyl | CH2C ≡ CH | H | H | Cl | H | nonafluoro-2-butyl | H | CF3 |
| 32-231 | 4,4,4-trifluoro-n-butyl | Me | H | H | Cl | H | nonafluoro-2-butyl | H | CF3 |
| 32-232 | i-butyl | NH2 | H | H | Cl | H | nonafluoro-2-butyl | H | CF3 |
| 32-243 | 2-cyanoethyl | Me | H | H | Br | H | heptafluoroisopropyl | H | C2F5 |
| 32-247 | 2,2,2-trichloroethyl | Me | H | H | Br | H | heptafluoroisopropyl | H | CF3 |
| 32-249 | n-propyl | C(O)OMe | H | H | Br | H | heptafluoroisopropyl | H | CF3 |
| 32-254 | 1,2-difluoro-2-propyl | Me | H | H | Br | H | heptafluoroisopropyl | H | n-C3F7 |
| 32-260 | 4,4,4-trifluoro-n-butyl | Me | H | H | Br | H | heptafluoroisopropyl | H | CF3 |
| 32-263 | vinyl | C(O)C(O)OMe | H | H | Br | H | heptafluoroisopropyl | H | CF3 |
| 32-276 | 2,2,2-trichloroethyl | Me | H | H | Br | H | nonafluoro-2-butyl | H | CF3 |
| 32-278 | n-propyl | C(O)OEt | H | H | Br | H | nonafluoro-2-butyl | H | CF3 |
| 32-282 | i-propyl | C(O)Me | H | H | Br | H | nonafluoro-2-butyl | H | CF3 |
| 32-289 | 4,4,4-trifluoro-n-butyl | Me | H | H | Br | H | nonafluoro-2-butyl | H | CF3 |
| 32-294 | phenyl | C(O)C(O)OEt | H | H | Br | H | nonafluoro-2-butyl | H | i-C3F7 |
| 32-305 | 2,2,2-trichloroethyl | Me | H | H | I | H | heptafluoroisopropyl | H | CF3 |
| 32-310 | 3-bromo-n-propyl | C(O)Et | H | H | I | H | heptafluoroisopropyl | H | C2F5 |
| 32-318 | 4,4,4-trifluoro-n-butyl | Me | H | H | I | H | heptafluoroisopropyl | H | CF3 |
| 32-322 | benzyl | S(O)2Me | H | H | I | H | heptafluoroisopropyl | H | CF3 |
| 32-330 | 2-cyanoethyl | S(O)2Et | H | H | I | H | nonafluoro-2-butyl | H | CF3 |
| 32-334 | 2,2,2-trichloroethyl | Me | H | H | I | H | nonafluoro-2-butyl | H | CF3 |
| 32-336 | n-propyl | CH2Ph | H | H | I | H | nonafluoro-2-butyl | H | n-C3F7 |
| 32-340 | i-propyl | CH2(3-Py) | H | H | I | H | nonafluoro-2-butyl | H | CF3 |
| 32-347 | 4,4,4-trifluoro-n-butyl | Me | H | H | I | H | nonafluoro-2-butyl | H | CF3 |
| 32-349 | s-butyl | CH2(3-Py-N-oxide) | H | H | I | H | nonafluoro-2-butyl | H | CF3 |
| 32-350 | vinyl | Me | H | H | I | H | nonafluoro-2-butyl | H | i-C3F7 |
| 32-363 | 2,2,2-trichloroethyl | Me | H | H | OCF3 | H | heptafluoroisopropyl | H | OCF3 |
| 32-392 | 2,2,2-trichloroethyl | Me | H | H | OCF3 | H | nonafluoro-2-butyl | H | OCF3 |
| 32-421 | 2,2,2-trichloroethyl | Me | H | H | CF3 | H | heptafluoroisopropyl | H | OCF3 |
| 32-450 | 2,2,2-trichloroethyl | Me | H | H | CF3 | H | nonafluoro-2-butyl | H | OCF3 |
| 32-479 | 2,2,2-trichloroethyl | Me | H | H | CF3 | H | heptafluoroisopropyl | H | CF3 |
| 32-508 | 2,2,2-trichloroethyl | Me | H | H | CF3 | H | nonafluoro-2-butyl | H | CF3 |

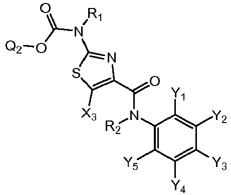

**Table 33(1)**

| compound number | Q₂ | R₁ | R₂ | X₃ | Y₁ | Y₂ | Y₃ | Y₄ | Y₅ |
|---|---|---|---|---|---|---|---|---|---|
| 33-1 | 2,2,2-trichloroethyl | H | Me | H | F | H | heptafluoroisopropyl | H | OCF3 |
| 33-2 | 2,2,2-trichloroethyl | H | Me | H | F | H | nonafluoro-2-butyl | H | OCF3 |
| 33-13 | 2,2,2-trichloroethyl | H | Me | H | Cl | H | heptafluoroisopropyl | H | OCF3 |
| 33-42 | 2,2,2-trichloroethyl | H | Me | H | Cl | H | nonafluoro-2-butyl | H | OCF3 |
| 33-71 | 2,2,2-trichloroethyl | H | Me | H | Br | H | heptafluoroisopropyl | H | OCF3 |
| 33-100 | 2,2,2-trichloroethyl | H | Me | H | Br | H | nonafluoro-2-butyl | H | OCF3 |
| 33-129 | 2,2,2-trichloroethyl | H | Me | H | I | H | heptafluoroisopropyl | H | OCF3 |
| 33-158 | 2,2,2-trichloroethyl | H | Me | H | I | H | nonafluoro-2-butyl | H | OCF3 |
| 33-177 | 2,2,2-trichloroethyl | H | Me | H | F | H | heptafluoroisopropyl | H | CF3 |
| 33-178 | 2,2,2-trichloroethyl | H | Me | H | F | H | nonafluoro-2-butyl | H | CF3 |
| 33-185 | 2-cyanoethyl | H | Et | H | Cl | H | heptafluoroisopropyl | H | C2F5 |
| 33-189 | 2,2,2-trichloroethyl | H | Me | H | Cl | H | heptafluoroisopropyl | H | CF3 |
| 33-191 | n-propyl | H | Me | H | Cl | H | pentafluoroethyl | H | CF3 |
| 33-195 | i-propyl | H | n-Pr | H | Cl | H | heptafluoroisopropyl | H | n-C3F7 |
| 33-198 | 1,3-dichloro-2-propyl | H | Me | H | Cl | H | pentafluoroethyl | H | CF3 |
| 33-199 | 1-chloro-3-fluoro-2-propyl | H | i-Pr | H | Cl | H | heptafluoroisopropyl | H | CF3 |
| 33-203 | i-butyl | H | CH2CH=CH2 | H | Cl | H | heptafluoroisopropyl | H | CF3 |
| 33-207 | phenyl | H | Me | Cl | Cl | H | heptafluoroisopropyl | H | CF3 |
| 33-209 | ethyl | H | Me | H | Cl | H | nonafluoro-2-butyl | H | i-C3F7 |
| 33-214 | 2-cyanoethyl | H | CN | H | Cl | H | nonafluoro-2-butyl | H | CF3 |
| 33-218 | 2,2,2-trichloroethyl | H | Me | H | Cl | H | nonafluoro-2-butyl | H | CF3 |
| 33-220 | n-propyl | H | CH2C≡CH | H | Cl | H | nonafluoro-2-butyl | H | CF3 |
| 33-232 | i-butyl | H | NH2 | H | Cl | H | nonafluoro-2-butyl | H | CF3 |
| 33-243 | 2-cyanoethyl | H | Me | H | Br | H | heptafluoroisopropyl | H | C2F5 |
| 33-247 | 2,2,2-trichloroethyl | H | Me | H | Br | H | heptafluoroisopropyl | H | CF3 |
| 33-249 | n-propyl | H | C(O)OMe | H | Br | H | heptafluoroisopropyl | H | CF3 |
| 33-254 | 1,2-difluoro-2-propyl | H | Me | H | Br | H | heptafluoroisopropyl | H | n-C3F7 |
| 33-260 | 4,4,4-trifluoro-n-butyl | H | Me | H | Br | H | heptafluoroisopropyl | H | CF3 |
| 33-263 | vinyl | H | C(O)C(O)OMe | H | Br | H | heptafluoroisopropyl | H | CF3 |
| 33-276 | 2,2,2-trichloroethyl | H | Me | H | Br | H | nonafluoro-2-butyl | H | CF3 |
| 33-278 | n-propyl | H | C(O)OEt | H | Br | H | nonafluoro-2-butyl | H | CF3 |
| 33-282 | i-propyl | H | C(O)Me | H | Br | H | nonafluoro-2-butyl | H | CF3 |
| 33-289 | 4,4,4-trifluoro-n-butyl | H | Me | H | Br | H | nonafluoro-2-butyl | H | CF3 |
| 33-294 | phenyl | H | C(O)C(O)OEt | H | Br | H | nonafluoro-2-butyl | H | i-C3F7 |
| 33-305 | 2,2,2-trichloroethyl | H | Me | H | I | H | heptafluoroisopropyl | H | CF3 |
| 33-310 | 3-bromo-n-propyl | H | C(O)Et | H | I | H | heptafluoroisopropyl | H | C2F5 |
| 33-318 | 4,4,4-trifluoro-n-butyl | H | Me | H | I | H | heptafluoroisopropyl | H | CF3 |
| 33-322 | benzyl | H | S(O)2Me | H | I | H | heptafluoroisopropyl | H | CF3 |
| 33-330 | 2-cyanoethyl | H | S(O)2Et | H | I | H | nonafluoro-2-butyl | H | CF3 |
| 33-334 | 2,2,2-trichloroethyl | H | Me | H | I | H | nonafluoro-2-butyl | H | CF3 |
| 33-336 | n-propyl | H | CH2Ph | H | I | H | nonafluoro-2-butyl | H | n-C3F7 |
| 33-340 | i-propyl | H | CH2(3-Py) | H | I | H | nonafluoro-2-butyl | H | CF3 |
| 33-347 | 4,4,4-trifluoro-n-butyl | H | Me | H | I | H | nonafluoro-2-butyl | H | CF3 |
| 33-349 | s-butyl | H | CH2(3-Py-N-oxide) | H | I | H | nonafluoro-2-butyl | H | CF3 |
| 33-350 | vinyl | H | Me | H | I | H | nonafluoro-2-butyl | H | i-C3F7 |
| 33-363 | 2,2,2-trichloroethyl | H | Me | H | OCF3 | H | heptafluoroisopropyl | H | OCF3 |
| 33-392 | 2,2,2-trichloroethyl | H | Me | H | OCF3 | H | nonafluoro-2-butyl | H | OCF3 |
| 33-421 | 2,2,2-trichloroethyl | H | Me | H | CF3 | H | heptafluoroisopropyl | H | OCF3 |
| 33-450 | 2,2,2-trichloroethyl | H | Me | H | CF3 | H | nonafluoro-2-butyl | H | OCF3 |
| 33-479 | 2,2,2-trichloroethyl | H | Me | H | CF3 | H | heptafluoroisopropyl | H | CF3 |
| 33-508 | 2,2,2-trichloroethyl | H | Me | H | CF3 | H | nonafluoro-2-butyl | H | CF3 |

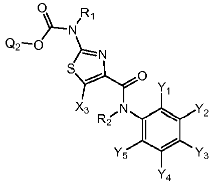

**Table 34(1)**

| compound number | Q₂ | R₁ | R₂ | X₃ | Y₁ | Y₂ | Y₃ | Y₄ | Y₅ |
|---|---|---|---|---|---|---|---|---|---|
| 34-1 | 2,2,2-trichloroethyl | Me | Me | H | F | H | heptafluoroisopropyl | H | OCF3 |
| 34-2 | 2,2,2-trichloroethyl | Me | Me | H | F | H | nonafluoro-2-butyl | H | OCF3 |
| 34-13 | 2,2,2-trichloroethyl | Me | Me | H | Cl | H | heptafluoroisopropyl | H | OCF3 |
| 34-42 | 2,2,2-trichloroethyl | Me | Me | H | Cl | H | nonafluoro-2-butyl | H | OCF3 |
| 34-71 | 2,2,2-trichloroethyl | Me | Me | H | Br | H | heptafluoroisopropyl | H | OCF3 |
| 34-100 | 2,2,2-trichloroethyl | Me | Me | H | Br | H | nonafluoro-2-butyl | H | OCF3 |
| 34-129 | 2,2,2-trichloroethyl | Me | Me | H | I | H | heptafluoroisopropyl | H | OCF3 |
| 34-158 | 2,2,2-trichloroethyl | Me | Me | H | I | H | nonafluoro-2-butyl | H | OCF3 |
| 34-373 | 2,2,2-trichloroethyl | Me | Me | H | F | H | heptafluoroisopropyl | H | CF3 |
| 34-374 | 2,2,2-trichloroethyl | Me | Me | H | F | H | nonafluoro-2-butyl | H | CF3 |
| 34-380 | 2-iodoethyl | Me | Et | H | Cl | H | heptafluoroisopropyl | H | CF3 |
| 34-381 | 2-cyanoethyl | Et | Me | H | Cl | H | heptafluoroisopropyl | H | C2F5 |
| 34-385 | 2,2,2-trichloroethyl | Me | Me | H | Cl | H | heptafluoroisopropyl | H | CF3 |
| 34-387 | n-propyl | Me | n-Pr | H | Cl | H | pentafluoroethyl | H | CF3 |
| 34-391 | i-propyl | n-Pr | Me | H | Cl | H | heptafluoroisopropyl | H | n-C3F7 |
| 34-394 | 1,3-dichloro-2-propyl | Me | Me | H | Cl | H | pentafluoroethyl | H | CF3 |
| 34-395 | 1-chloro-3-fluoro-2-propyl | i-Pr | Me | H | Cl | H | heptafluoroisopropyl | H | CF3 |
| 34-397 | n-butyl | Me | i-Pr | H | Cl | H | heptafluoroisopropyl | H | CF3 |
| 34-399 | i-butyl | CH2CH=CH2 | Me | H | Cl | H | heptafluoroisopropyl | H | CF3 |
| 34-400 | s-butyl | Me | CH2CH=CH2 | H | Cl | H | heptafluoroisopropyl | H | CF3 |
| 34-401 | vinyl | Me | CN | H | Cl | H | heptafluoroisopropyl | H | CF3 |
| 34-405 | ethyl | Me | Me | H | Cl | H | nonafluoro-2-butyl | H | i-C3F7 |
| 34-410 | 2-cyanoethyl | CN | Me | H | Cl | H | nonafluoro-2-butyl | H | CF3 |
| 34-414 | 2,2,2-trichloroethyl | Me | Me | H | Cl | H | nonafluoro-2-butyl | H | CF3 |
| 34-415 | 2,2,2-tribromoethyl | Me | CH2C≡CH | H | Cl | H | nonafluoro-2-butyl | H | CF3 |
| 34-416 | n-propyl | CH2C≡CH | Me | H | Cl | H | nonafluoro-2-butyl | H | CF3 |
| 34-420 | i-propyl | Me | Me | Cl | Cl | H | nonafluoro-2-butyl | H | CF3 |
| 34-426 | n-butyl | Me | NH2 | H | Cl | H | nonafluoro-2-butyl | H | CF3 |
| 34-428 | i-butyl | NH2 | Me | H | Cl | H | nonafluoro-2-butyl | H | CF3 |
| 34-439 | 2-cyanoethyl | Me | C(O)OMe | H | Br | H | heptafluoroisopropyl | H | C2F5 |
| 34-443 | 2,2,2-trichloroethyl | Me | Me | H | Br | H | heptafluoroisopropyl | H | CF3 |
| 34-445 | n-propyl | C(O)OMe | Me | H | Br | H | heptafluoroisopropyl | H | CF3 |
| 34-450 | 1,2-difluoro-2-propyl | Me | Me | H | Br | H | heptafluoroisopropyl | H | n-C3F7 |
| 34-456 | 4,4,4-trifluoro-n-butyl | Me | Me | H | Br | H | heptafluoroisopropyl | H | CF3 |
| 34-457 | i-butyl | Me | C(O)C(O)OMe | H | Br | H | heptafluoroisopropyl | H | CF3 |

**Table 34(2)**

| compound number | Q₂ | R₁ | R₂ | X₃ | Y₁ | Y₂ | Y₃ | Y₄ | Y₅ |
|---|---|---|---|---|---|---|---|---|---|
| 34-459 | vinyl | C(O)C(O)OMe | Me | H | Br | H | heptafluoroisopropyl | H | CF3 |
| 34-468 | 2-cyanoethyl | Me | C(O)OEt | H | Br | H | nonafluoro-2-butyl | H | CF3 |
| 34-472 | 2,2,2-trichloroethyl | Me | Me | H | Br | H | nonafluoro-2-butyl | H | CF3 |
| 34-474 | n-propyl | C(O)OEt | Me | H | Br | H | nonafluoro-2-butyl | H | CF3 |
| 34-477 | 3-bromo-n-propyl | Me | C(O)Me | H | Br | H | nonafluoro-2-butyl | H | CF3 |
| 34-478 | i-propyl | C(O)Me | Me | H | Br | H | nonafluoro-2-butyl | H | CF3 |
| 34-485 | 4,4,4-trifluoro-n-butyl | Me | Me | H | Br | H | nonafluoro-2-butyl | H | CF3 |
| 34-486 | i-butyl | Me | C(O)C(O)OEt | H | Br | H | nonafluoro-2-butyl | H | CF3 |
| 34-490 | phenyl | C(O)C(O)OEt | Me | H | Br | H | nonafluoro-2-butyl | H | i-C3F7 |
| 34-501 | 2,2,2-trichloroethyl | Me | Me | H | I | H | heptafluoroisopropyl | H | CF3 |
| 34-503 | n-propyl | Me | C(O)Et | H | I | H | heptafluoroisopropyl | H | CF3 |
| 34-506 | 3-bromo-n-propyl | C(O)Et | Me | H | I | H | heptafluoroisopropyl | H | C2F5 |
| 34-514 | 4,4,4-trifluoro-n-butyl | Me | Me | H | I | H | heptafluoroisopropyl | H | CF3 |
| 34-515 | i-butyl | Me | S(O)2Me | H | I | H | heptafluoroisopropyl | H | CF3 |
| 34-518 | benzyl | S(O)2Me | Me | H | I | H | heptafluoroisopropyl | H | CF3 |
| 34-525 | 2-iodoethyl | Me | S(O)2Et | H | I | H | nonafluoro-2-butyl | H | CF3 |
| 34-526 | 2-cyanoethyl | S(O)2Et | Me | H | I | H | nonafluoro-2-butyl | H | CF3 |
| 34-530 | 2,2,2-trichloroethyl | Me | Me | H | I | H | nonafluoro-2-butyl | H | CF3 |
| 34-531 | 2,2,2-tribromoethyl | Me | CH2Ph | H | I | H | nonafluoro-2-butyl | H | CF3 |
| 34-532 | n-propyl | CH2Ph | Me | H | I | H | nonafluoro-2-butyl | H | n-C3F7 |
| 34-536 | i-propyl | CH2(3-Py) | Me | H | I | H | nonafluoro-2-butyl | H | CF3 |
| 34-542 | n-butyl | Me | CH2(3-Py) | H | I | H | nonafluoro-2-butyl | H | CF3 |
| 34-543 | 4,4,4-trifluoro-n-butyl | Me | Me | H | I | H | nonafluoro-2-butyl | H | CF3 |
| 34-545 | s-butyl | CH2(3-Py-N-oxide) | Me | H | I | H | nonafluoro-2-butyl | H | CF3 |
| 34-546 | vinyl | Me | Me | H | I | H | nonafluoro-2-butyl | H | i-C3F7 |
| 34-548 | phenyl | Me | CH2(3-Py-N-oxide) | H | I | H | nonafluoro-2-butyl | H | CF3 |
| 34-755 | 2,2,2-trichloroethyl | Me | Me | H | OCF3 | H | heptafluoroisopropyl | H | OCF3 |
| 34-784 | 2,2,2-trichloroethyl | Me | Me | H | OCF3 | H | nonafluoro-2-butyl | H | OCF3 |
| 34-813 | 2,2,2-trichloroethyl | Me | Me | H | CF3 | H | heptafluoroisopropyl | H | OCF3 |
| 34-842 | 2,2,2-trichloroethyl | Me | Me | H | CF3 | H | nonafluoro-2-butyl | H | OCF3 |
| 34-871 | 2,2,2-trichloroethyl | Me | Me | H | CF3 | H | heptafluoroisopropyl | H | CF3 |
| 34-900 | 2,2,2-trichloroethyl | Me | Me | H | CF3 | H | nonafluoro-2-butyl | H | CF3 |

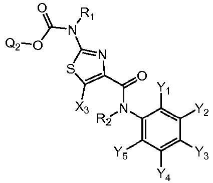

**Table 35(1)**

| compound number | Q₂ | R₁ | R₂ | X₃ | Y₁ | Y₂ | Y₃ | Y₄ | Y₅ |
|---|---|---|---|---|---|---|---|---|---|
| 35-9 | 2,2-dichloroethyl | H | Et | H | Cl | F | heptafluoroisopropyl | F | OCF3 |
| 35-39 | 2,2,2-trifluoroethyl | H | H | H | Cl | Me | nonafluoro-2-butyl | Me | OCF3 |
| 35-154 | 2,2,2-trichloroethyl | H | H | H | Cl | F | heptafluoroisopro pyl | Br | CF3 |
| 35-183 | 2,2,2-trichloroethyl | H | H | H | Cl | Me | nonafluoro-2-butyl | Et | i-C3F7 |
| 35-189 | i-propyl | H | H | Cl | Cl | Et | nonafluoro-2-butyl | Me | n-C3F7 |
| 35-190 | 1,2-difluoro-2-propyl | H | H | H | Cl | Et | nonafluoro-2-butyl | Et | i-C3F7 |
| 35-196 | 2-chloroethyl | H | H | H | Br | n-Pr | heptafluoroisopro pyl | Me | CF3 |
| 35-197 | 2-bromoethyl | H | H | H | Br | n-Pr | heptafluoroisopropyl | Et | CF3 |
| 35-203 | 2,2,2-trichloroethyl | H | H | H | Br | i-Pr | heptafluoroisopropyl | Me | CF3 |
| 35-204 | 2,2,2-tribromoethyl | H | H | H | Br | i-Pr | heptafluoroisopropyl | Et | CF3 |
| 35-210 | 1,2-difluoro-2-propyl | H | H | H | Br | n-Bu | heptafluoroisopropyl | Me | CF3 |
| 35-211 | 1,3-difluoro-2-propyl | H | H | H | Br | n-Bu | heptafluoroisopropyl | Et | CF3 |
| 35-217 | i-butyl | H | H | H | Br | i-Bu | nonafluoro-2-butyl | Me | CF3 |
| 35-218 | s-butyl | H | H | H | Br | i-Bu | nonafluoro-2-butyl | Et | CF3 |
| 35-224 | 2-fluoroethyl | H | H | H | Br | s-Bu | nonafluoro-2-butyl | Me | CF3 |
| 35-225 | 2-chloroethyl | H | H | H | Br | s-Bu | nonafluoro-2-butyl | Et | CF3 |
| 35-231 | 2,2,2-trifluoroethyl | H | H | H | Br | F | nonafluoro-2-butyl | Me | CF3 |
| 35-232 | 2,2,2-trichloroethyl | H | H | H | Br | F | nonafluoro-2-butyl | Et | CF3 |
| 35-238 | i-propyl | Et | i-Pr | H | Br | Cl | nonafluoro-2-butyl | Me | CF3 |
| 35-239 | 1,2-difluoro-2-propyl | H | H | H | Br | Cl | nonafluoro-2-butyl | Et | CF3 |
| 35-245 | 2-iodoethyl | H | H | H | I | Br | heptafluoroisopropyl | Me | CF3 |
| 35-246 | 2-cyanoethyl | H | H | H | I | Br | heptafluoroisopropyl | Et | CF3 |
| 35-252 | n-propyl | n-Pr | Me | H | I | I | heptafluoroisopropyl | Me | CF3 |
| 35-253 | 3-fluoro-n-propyl | H | H | H | I | I | heptafluoroisopropyl | Et | CF3 |
| 35-259 | 1,3-dichloro-2-propyl | H | H | H | I | Me | heptafluoroisopropyl | F | C2F5 |
| 35-260 | 1-chloro-3-fluoro-2-propyl | H | H | H | I | Me | nonafluoro-2-butyl | Cl | CF3 |
| 35-261 | 3,3,3-trifluoro-n-propyl | H | H | H | I | Me | nonafluoro-2-butyl | Br | n-C3F7 |
| 35-262 | n-butyl | H | H | H | I | Me | nonafluoro-2-butyl | I | i-C3F7 |
| 35-263 | 4,4,4-trifluoro-n-butyl | H | H | H | I | Et | nonafluoro-2-butyl | F | CF3 |
| 35-264 | i-butyl | H | H | H | I | Et | nonafluoro-2-butyl | Cl | CF3 |
| 35-265 | s-butyl | H | H | H | I | Et | nonafluoro-2-butyl | Br | CF3 |
| 35-266 | vinyl | n-Pr | i-Pr | H | I | Et | nonafluoro-2-butyl | I | C2F5 |
| 35-267 | benzyl | H | H | H | I | n-Pr | nonafluoro-2-butyl | F | CF3 |
| 35-268 | phenyl | H | H | H | I | n-Pr | nonafluoro-2-butyl | Cl | CF3 |
| 35-271 | 2-fluoroethyl | H | H | H | I | i-Pr | nonafluoro-2-butyl | F | i-C3F7 |
| 35-272 | 2-chloroethyl | H | H | H | I | i-Pr | nonafluoro-2-butyl | Cl | CF3 |
| 35-273 | 2-bromoethyl | n-Pr | C(O)Me | H | I | i-Pr | nonafluoro-2-butyl | Br | CF3 |
| 35-274 | 2-iodoethyl | H | H | H | I | i-Pr | nonafluoro-2-butyl | I | CF3 |
| 35-275 | 2-cyanoethyl | H | H | H | I | n-Bu | nonafluoro-2-butyl | F | CF3 |
| 35-276 | 2,2-difluoroethyl | H | H | H | I | n-Bu | nonafluoro-2-butyl | Cl | CF3 |
| 35-279 | 2,2,2-trichloroethyl | H | H | H | I | i-Bu | nonafluoro-2-butyl | F | CF3 |
| 35-280 | 2,2,2-tribromoethyl | H | H | H | I | i-Bu | nonafluoro-2-butyl | Cl | CF3 |
| 35-283 | 3-chloro-n-propyl | i-Pr | n-Pr | H | I | s-Bu | nonafluoro-2-butyl | F | CF3 |
| 35-284 | 3-bromo-n-propyl | H | H | H | I | s-Bu | nonafluoro-2-butyl | Cl | CF3 |
| 35-301 | 3-chloro-n-propyl | n-Pr | H | H | OCF3 | Cl | heptafluoroisopropyl | Br | OCF3 |
| 35-454 | i-butyl | H | H | H | OCF3 | Me | nonafluoro-2-butyl | H | CF3 |
| 35-582 | 2,2,2-trifluoroethyl | H | H | H | CF3 | F | heptafluoroisopropyl | Cl | CF3 |

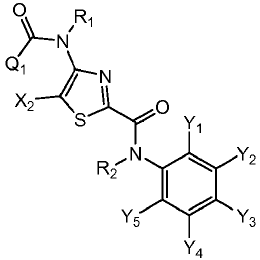

**Table 36(1)**

| compound number | Q₁ | R₁ | R₂ | X₂ | Y₁ | Y₂ | Y₃ | Y₄ | Y₅ |
|---|---|---|---|---|---|---|---|---|---|
| 36-1 | phenyl | H | H | H | Cl | H | heptafluoroisopropyl | H | OCF3 |
| 36-2 | phenyl | H | H | H | Br | H | heptafluoroisopropyl | H | CF3 |
| 36-3 | phenyl | H | H | Cl | Br | H | heptafluoroisopropyl | H | CF3 |
| 36-4 | phenyl | H | H | H | CF3 | H | heptafluoroisopropyl | H | CF3 |
| 36-5 | phenyl | H | H | H | I | H | nonafluoro-2-butyl | H | OCF3 |
| 36-6 | phenyl | H | H | H | Cl | H | nonafluoro-2-butyl | H | CF3 |
| 36-7 | phenyl | H | H | H | CF3 | H | nonafluoro-2-butyl | H | CF3 |
| 36-8 | phenyl | Me | H | H | Cl | H | heptafluoroisopropyl | H | OCF3 |
| 36-9 | phenyl | Me | H | H | Br | H | heptafluoroisopropyl | H | CF3 |
| 36-10 | phenyl | Me | H | H | CF3 | H | heptafluoroisopropyl | H | CF3 |
| 36-11 | phenyl | Me | H | H | I | H | nonafluoro-2-butyl | H | OCF3 |
| 36-12 | phenyl | Me | H | H | Cl | H | nonafluoro-2-butyl | H | CF3 |
| 36-13 | phenyl | Me | H | H | CF3 | H | nonafluoro-2-butyl | H | CF3 |
| 36-14 | phenyl | H | Me | H | Cl | H | heptafluoroisopropyl | H | OCF3 |
| 36-15 | phenyl | H | Me | H | Br | H | heptafluoroisopropyl | H | CF3 |
| 36-16 | phenyl | H | Me | H | CF3 | H | heptafluoroisopropyl | H | CF3 |
| 36-17 | phenyl | H | Me | H | I | H | nonafluoro-2-butyl | H | OCF3 |
| 36-18 | phenyl | H | Me | H | Cl | H | nonafluoro-2-butyl | H | CF3 |
| 36-19 | phenyl | H | Me | H | CF3 | H | nonafluoro-2-butyl | H | CF3 |
| 36-20 | phenyl | Me | Me | H | Cl | H | heptafluoroisopropyl | H | OCF3 |
| 36-21 | phenyl | Me | Me | H | Br | H | heptafluoroisopropyl | H | CF3 |
| 36-22 | phenyl | Me | Me | H | CF3 | H | heptafluoroisopropyl | H | CF3 |
| 36-23 | phenyl | Me | Me | H | I | H | nonafluoro-2-butyl | H | OCF3 |
| 36-24 | phenyl | Me | Me | H | Cl | H | nonafluoro-2-butyl | H | CF3 |
| 36-25 | phenyl | Me | Me | H | CF3 | H | nonafluoro-2-butyl | H | CF3 |
| 36-26 | phenyl | H | H | H | Cl | F | heptafluoroisopropyl | H | OCF3 |
| 36-27 | phenyl | Me | H | H | Br | H | heptafluoroisopropyl | F | CF3 |
| 36-28 | phenyl | H | Me | H | CF3 | Cl | heptafluoroisopropyl | H | CF3 |
| 36-29 | phenyl | H | H | H | I | H | nonafluoro-2-butyl | Cl | OCF3 |
| 36-30 | phenyl | H | H | H | Cl | Br | nonafluoro-2-butyl | H | CF3 |
| 36-31 | phenyl | H | H | H | Br | H | nonafluoro-2-butyl | I | CF3 |
| 36-32 | phenyl | H | H | H | I | Me | nonafluoro-2-butyl | H | CF3 |
| 36-33 | phenyl | H | H | H | CF3 | H | nonafluoro-2-butyl | Et | CF3 |

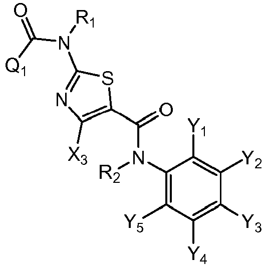

**Table 37(1)**

| compound number | Q₁ | R₁ | R₂ | X₃ | Y₁ | Y₂ | Y₃ | Y₄ | Y₅ |
|---|---|---|---|---|---|---|---|---|---|
| 37-1 | phenyl | H | H | H | Br | H | heptafluoroisopropyl | H | OCF3 |
| 37-2 | phenyl | H | H | H | I | H | heptafluoroisopropyl | H | CF3 |
| 37-3 | phenyl | H | H | Cl | I | H | heptafluoroisopropyl | H | CF3 |
| 37-4 | phenyl | H | H | H | CF3 | H | heptafluoroisopropyl | H | CF3 |
| 37-5 | phenyl | H | H | H | Cl | H | nonafluoro-2-butyl | H | OCF3 |
| 37-6 | phenyl | H | H | H | Br | H | nonafluoro-2-butyl | H | CF3 |
| 37-7 | phenyl | H | H | H | CF3 | H | nonafluoro-2-butyl | H | CF3 |
| 37-8 | phenyl | Me | H | H | Br | H | heptafluoroisopropyl | H | OCF3 |
| 37-9 | phenyl | Me | H | H | I | H | heptafluoroisopropyl | H | CF3 |
| 37-10 | phenyl | Me | H | H | CF3 | H | heptafluoroisopropyl | H | CF3 |
| 37-11 | phenyl | Me | H | H | Cl | H | nonafluoro-2-butyl | H | OCF3 |
| 37-12 | phenyl | Me | H | H | Br | H | nonafluoro-2-butyl | H | CF3 |
| 37-13 | phenyl | Me | H | H | CF3 | H | nonafluoro-2-butyl | H | CF3 |
| 37-14 | phenyl | H | Me | H | Br | H | heptafluoroisopropyl | H | OCF3 |
| 37-15 | phenyl | H | Me | H | I | H | heptafluoroisopropyl | H | CF3 |
| 37-16 | phenyl | H | Me | H | CF3 | H | heptafluoroisopropyl | H | CF3 |
| 37-17 | phenyl | H | Me | H | Cl | H | nonafluoro-2-butyl | H | OCF3 |
| 37-18 | phenyl | H | Me | H | Br | H | nonafluoro-2-butyl | H | CF3 |
| 37-19 | phenyl | H | Me | H | CF3 | H | nonafluoro-2-butyl | H | CF3 |
| 37-20 | phenyl | Me | Me | H | Br | H | heptafluoroisopropyl | H | OCF3 |
| 37-21 | phenyl | Me | Me | H | I | H | heptafluoroisopropyl | H | CF3 |
| 37-22 | phenyl | Me | Me | H | CF3 | H | heptafluoroisopropyl | H | CF3 |
| 37-23 | phenyl | Me | Me | H | Cl | H | nonafluoro-2-butyl | H | OCF3 |
| 37-24 | phenyl | Me | Me | H | Br | H | nonafluoro-2-butyl | H | CF3 |
| 37-25 | phenyl | Me | Me | H | CF3 | H | nonafluoro-2-butyl | H | CF3 |
| 37-26 | phenyl | Me | H | H | Br | F | heptafluoroisopropyl | H | OCF3 |
| 37-27 | phenyl | H | H | H | I | H | heptafluoroisopropyl | F | CF3 |
| 37-28 | phenyl | H | Me | H | CF3 | Cl | heptafluoroisopropyl | H | CF3 |
| 37-29 | phenyl | H | H | H | Cl | H | nonafluoro-2-butyl | Cl | OCF3 |
| 37-30 | phenyl | H | H | H | Br | Br | nonafluoro-2-butyl | H | CF3 |
| 37-31 | phenyl | H | H | H | I | H | nonafluoro-2-butyl | I | CF3 |
| 37-32 | phenyl | H | H | H | Cl | Me | nonafluoro-2-butyl | H | CF3 |
| 37-33 | phenyl | H | H | H | CF3 | H | nonafluoro-2-butyl | Et | CF3 |

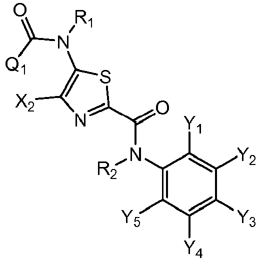

**Table 38(1)**

| compound number | Q₁ | R₁ | R₂ | X₂ | Y₁ | Y₂ | Y₃ | Y₄ | Y₅ |
|---|---|---|---|---|---|---|---|---|---|
| 38-1 | phenyl | H | H | H | I | H | heptafluoroisopropyl | H | OCF3 |
| 38-2 | phenyl | H | H | H | Cl | H | heptafluoroisopropyl | H | CF3 |
| 38-3 | phenyl | H | H | Cl | Cl | H | heptafluoroisopropyl | H | CF3 |
| 38-4 | phenyl | H | H | H | CF3 | H | heptafluoroisopropyl | H | CF3 |
| 38-5 | phenyl | H | H | H | Br | H | nonafluoro-2-butyl | H | OCF3 |
| 38-6 | phenyl | H | H | H | I | H | nonafluoro-2-butyl | H | CF3 |
| 38-7 | phenyl | H | H | H | CF3 | H | nonafluoro-2-butyl | H | CF3 |
| 38-8 | phenyl | Me | H | H | I | H | heptafluoroisopropyl | H | OCF3 |
| 38-9 | phenyl | Me | H | H | Cl | H | heptafluoroisopropyl | H | CF3 |
| 38-10 | phenyl | Me | H | H | CF3 | H | heptafluoroisopropyl | H | CF3 |
| 38-11 | phenyl | Me | H | H | Br | H | nonafluoro-2-butyl | H | OCF3 |
| 38-12 | phenyl | Me | H | H | I | H | nonafluoro-2-butyl | H | CF3 |
| 38-13 | phenyl | Me | H | H | CF3 | H | nonafluoro-2-butyl | H | CF3 |
| 38-14 | phenyl | H | Me | H | I | H | heptafluoroisopropyl | H | OCF3 |
| 38-15 | phenyl | H | Me | H | Cl | H | heptafluoroisopropyl | H | CF3 |
| 38-16 | phenyl | H | Me | H | CF3 | H | heptafluoroisopropyl | H | CF3 |
| 38-17 | phenyl | H | Me | H | Br | H | nonafluoro-2-butyl | H | OCF3 |
| 38-18 | phenyl | H | Me | H | I | H | nonafluoro-2-butyl | H | CF3 |
| 38-19 | phenyl | H | Me | H | CF3 | H | nonafluoro-2-butyl | H | CF3 |
| 38-20 | phenyl | Me | Me | H | I | H | heptafluoroisopropyl | H | OCF3 |
| 38-21 | phenyl | Me | Me | H | Cl | H | heptafluoroisopropyl | H | CF3 |
| 38-22 | phenyl | Me | Me | H | CF3 | H | heptafluoroisopropyl | H | CF3 |
| 38-23 | phenyl | Me | Me | H | Br | H | nonafluoro-2-butyl | H | OCF3 |
| 38-24 | phenyl | Me | Me | H | I | H | nonafluoro-2-butyl | H | CF3 |
| 38-25 | phenyl | Me | Me | H | CF3 | H | nonafluoro-2-butyl | H | CF3 |
| 38-26 | phenyl | H | H | H | I | F | heptafluoroisopropyl | H | OCF3 |
| 38-27 | phenyl | H | H | H | Cl | H | heptafluoroisopropyl | F | CF3 |
| 38-28 | phenyl | Me | H | H | CF3 | Cl | heptafluoroisopropyl | H | CF3 |
| 38-29 | phenyl | H | H | H | Br | H | nonafluoro-2-butyl | Cl | OCF3 |
| 38-30 | phenyl | H | Me | H | I | Br | nonafluoro-2-butyl | H | CF3 |
| 38-31 | phenyl | H | H | H | Cl | H | nonafluoro-2-butyl | I | CF3 |
| 38-32 | phenyl | H | H | H | Br | Me | nonafluoro-2-butyl | H | CF3 |
| 38-33 | phenyl | H | H | H | CF3 | H | nonafluoro-2-butyl | Et | CF3 |

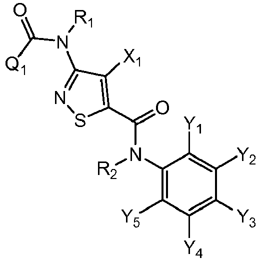

**Table 39(1)**

| compound number | Q₁ | R₁ | R₂ | X₁ | Y₁ | Y₂ | Y₃ | Y₄ | Y₅ |
|---|---|---|---|---|---|---|---|---|---|
| 39-1 | phenyl | H | H | H | Cl | H | heptafluoroisopropyl | H | OCF3 |
| 39-2 | phenyl | H | H | H | Br | H | heptafluoroisopropyl | H | CF3 |
| 39-3 | phenyl | H | H | Cl | Br | H | heptafluoroisopropyl | H | CF3 |
| 39-4 | phenyl | H | H | H | CF3 | H | heptafluoroisopropyl | H | CF3 |
| 39-5 | phenyl | H | H | H | I | H | nonafluoro-2-butyl | H | OCF3 |
| 39-6 | phenyl | H | H | H | Cl | H | nonafluoro-2-butyl | H | CF3 |
| 39-7 | phenyl | H | H | H | CF3 | H | nonafluoro-2-butyl | H | CF3 |
| 39-8 | phenyl | Me | H | H | Cl | H | heptafluoroisopropyl | H | OCF3 |
| 39-9 | phenyl | Me | H | H | Br | H | heptafluoroisopropyl | H | CF3 |
| 39-10 | phenyl | Me | H | H | CF3 | H | heptafluoroisopropyl | H | CF3 |
| 39-11 | phenyl | Me | H | H | I | H | nonafluoro-2-butyl | H | OCF3 |
| 39-12 | phenyl | Me | H | H | Cl | H | nonafluoro-2-butyl | H | CF3 |
| 39-13 | phenyl | Me | H | H | CF3 | H | nonafluoro-2-butyl | H | CF3 |
| 39-14 | phenyl | H | Me | H | Cl | H | heptafluoroisopropyl | H | OCF3 |
| 39-15 | phenyl | H | Me | H | Br | H | heptafluoroisopropyl | H | CF3 |
| 39-16 | phenyl | H | Me | H | CF3 | H | heptafluoroisopropyl | H | CF3 |
| 39-17 | phenyl | H | Me | H | I | H | nonafluoro-2-butyl | H | OCF3 |
| 39-18 | phenyl | H | Me | H | Cl | H | nonafluoro-2-butyl | H | CF3 |
| 39-19 | phenyl | H | Me | H | CF3 | H | nonafluoro-2-butyl | H | CF3 |
| 39-20 | phenyl | Me | Me | H | Cl | H | heptafluoroisopropyl | H | OCF3 |
| 39-21 | phenyl | Me | Me | H | Br | H | heptafluoroisopropyl | H | CF3 |
| 39-22 | phenyl | Me | Me | H | CF3 | H | heptafluoroisopropyl | H | CF3 |
| 39-23 | phenyl | Me | Me | H | I | H | nonafluoro-2-butyl | H | OCF3 |
| 39-24 | phenyl | Me | Me | H | Cl | H | nonafluoro-2-butyl | H | CF3 |
| 39-25 | phenyl | Me | Me | H | CF3 | H | nonafluoro-2-butyl | H | CF3 |
| 39-26 | phenyl | H | H | H | Cl | F | heptafluoroisopropyl | H | OCF3 |
| 39-27 | phenyl | Me | H | H | Br | H | heptafluoroisopropyl | F | CF3 |
| 39-28 | phenyl | H | Me | H | CF3 | Cl | heptafluoroisopropyl | H | CF3 |
| 39-29 | phenyl | H | H | H | I | H | nonafluoro-2-butyl | Cl | OCF3 |
| 39-30 | phenyl | H | H | H | Cl | Br | nonafluoro-2-butyl | H | CF3 |
| 39-31 | phenyl | H | H | H | Br | H | nonafluoro-2-butyl | I | CF3 |
| 39-32 | phenyl | H | H | H | I | Me | nonafluoro-2-butyl | H | CF3 |
| 39-33 | phenyl | H | H | H | CF3 | H | nonafluoro-2-butyl | Et | CF3 |

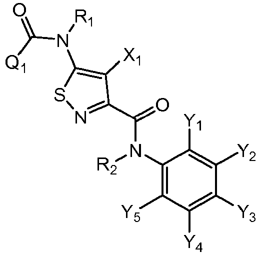

**Table 40(1)**

| compound number | Q₁ | R₁ | R₂ | X₁ | Y₁ | Y₂ | Y₃ | Y₄ | Y₅ |
|---|---|---|---|---|---|---|---|---|---|
| 40-1 | phenyl | H | H | H | Br | H | heptafluoroisopropyl | H | OCF3 |
| 40-2 | phenyl | H | H | H | I | H | heptafluoroisopropyl | H | CF3 |
| 40-3 | phenyl | H | H | Cl | I | H | heptafluoroisopropyl | H | CF3 |
| 40-4 | phenyl | H | H | H | CF3 | H | heptafluoroisopropyl | H | CF3 |
| 40-5 | phenyl | H | H | H | Cl | H | nonafluoro-2-butyl | H | OCF3 |
| 40-6 | phenyl | H | H | H | Br | H | nonafluoro-2-butyl | H | CF3 |
| 40-7 | phenyl | H | H | H | CF3 | H | nonafluoro-2-butyl | H | CF3 |
| 40-8 | phenyl | Me | H | H | Br | H | heptafluoroisopropyl | H | OCF3 |
| 40-9 | phenyl | Me | H | H | I | H | heptafluoroisopropyl | H | CF3 |
| 40-10 | phenyl | Me | H | H | CF3 | H | heptafluoroisopropyl | H | CF3 |
| 40-11 | phenyl | Me | H | H | Cl | H | nonafluoro-2-butyl | H | OCF3 |
| 40-12 | phenyl | Me | H | H | Br | H | nonafluoro-2-butyl | H | CF3 |
| 40-13 | phenyl | Me | H | H | CF3 | H | nonafluoro-2-butyl | H | CF3 |
| 40-14 | phenyl | H | Me | H | Br | H | heptafluoroisopropyl | H | OCF3 |
| 40-15 | phenyl | H | Me | H | I | H | heptafluoroisopropyl | H | CF3 |
| 40-16 | phenyl | H | Me | H | CF3 | H | heptafluoroisopropyl | H | CF3 |
| 40-17 | phenyl | H | Me | H | Cl | H | nonafluoro-2-butyl | H | OCF3 |
| 40-18 | phenyl | H | Me | H | Br | H | nonafluoro-2-butyl | H | CF3 |
| 40-19 | phenyl | H | Me | H | CF3 | H | nonafluoro-2-butyl | H | CF3 |
| 40-20 | phenyl | Me | Me | H | Br | H | heptafluoroisopropyl | H | OCF3 |
| 40-21 | phenyl | Me | Me | H | I | H | heptafluoroisopropyl | H | CF3 |
| 40-22 | phenyl | Me | Me | H | CF3 | H | heptafluoroisopropyl | H | CF3 |
| 40-23 | phenyl | Me | Me | H | Cl | H | nonafluoro-2-butyl | H | OCF3 |
| 40-24 | phenyl | Me | Me | H | Br | H | nonafluoro-2-butyl | H | CF3 |
| 40-25 | phenyl | Me | Me | H | CF3 | H | nonafluoro-2-butyl | H | CF3 |
| 40-26 | phenyl | H | H | H | Br | F | heptafluoroisopropyl | H | OCF3 |
| 40-27 | phenyl | H | H | H | I | H | heptafluoroisopropyl | F | CF3 |
| 40-28 | phenyl | Me | H | H | CF3 | Cl | heptafluoroisopropyl | H | CF3 |
| 40-29 | phenyl | H | H | H | Cl | H | nonafluoro-2-butyl | Cl | OCF3 |
| 40-30 | phenyl | H | H | H | Br | Br | nonafluoro-2-butyl | H | CF3 |
| 40-31 | phenyl | H | H | H | I | H | nonafluoro-2-butyl | I | CF3 |
| 40-32 | phenyl | H | H | H | Cl | Me | nonafluoro-2-butyl | H | CF3 |
| 40-33 | phenyl | H | Me | H | CF3 | H | nonafluoro-2-butyl | Et | CF3 |

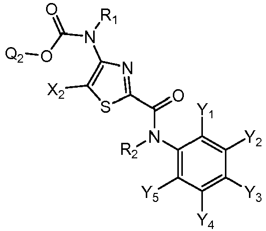

**Table 41(1)**

| compound number | Q₂ | R₁ | R₂ | X₂ | Y₁ | Y₂ | Y₃ | Y₄ | Y₅ |
|---|---|---|---|---|---|---|---|---|---|
| 41-1 | 2,2,2-trichloroethyl | H | H | H | Cl | H | heptafluoroisopropyl | H | OCF3 |
| 41-2 | 2,2,2-trichloroethyl | H | H | H | Br | H | heptafluoroisopropyl | H | CF3 |
| 41-3 | 2,2,2-trichloroethyl | H | H | H | CF3 | H | heptafluoroisopropyl | H | CF3 |
| 41-4 | 2,2,2-trichloroethyl | H | H | H | I | H | nonafluoro-2-butyl | H | OCF3 |
| 41-5 | 2,2,2-trichloroethyl | H | H | H | Cl | H | nonafluoro-2-butyl | H | CF3 |
| 41-6 | 2,2,2-trichloroethyl | H | H | H | CF3 | H | nonafluoro-2-butyl | H | CF3 |
| 41-7 | 2,2,2-trichloroethyl | Me | H | H | Br | H | heptafluoroisopropyl | H | OCF3 |
| 41-8 | 2,2,2-trichloroethyl | Me | H | H | I | H | heptafluoroisopropyl | H | CF3 |
| 41-9 | 2,2,2-trichloroethyl | Me | H | H | CF3 | H | heptafluoroisopropyl | H | CF3 |
| 41-10 | 2,2,2-trichloroethyl | Me | H | H | Cl | H | nonafluoro-2-butyl | H | OCF3 |
| 41-11 | 2,2,2-trichloroethyl | Me | H | H | Br | H | nonafluoro-2-butyl | H | CF3 |
| 41-12 | 2,2,2-trichloroethyl | Me | H | H | CF3 | H | nonafluoro-2-butyl | H | CF3 |
| 41-13 | 2,2,2-trichloroethyl | H | Me | H | I | H | heptafluoroisopropyl | H | OCF3 |
| 41-14 | 2,2,2-trichloroethyl | H | Me | H | Cl | H | heptafluoroisopropyl | H | CF3 |
| 41-15 | 2,2,2-trichloroethyl | H | Me | H | CF3 | H | heptafluoroisopropyl | H | CF3 |
| 41-16 | 2,2,2-trichloroethyl | H | Me | H | Br | H | nonafluoro-2-butyl | H | OCF3 |
| 41-17 | 2,2,2-trichloroethyl | H | Me | H | I | H | nonafluoro-2-butyl | H | CF3 |
| 41-18 | 2,2,2-trichloroethyl | H | Me | H | CF3 | H | nonafluoro-2-butyl | H | CF3 |
| 41-19 | 2,2,2-trichloroethyl | Me | Me | H | Cl | H | heptafluoroisopropyl | H | OCF3 |
| 41-20 | 2,2,2-trichloroethyl | Me | Me | H | Br | H | heptafluoroisopropyl | H | CF3 |
| 41-21 | 2,2,2-trichloroethyl | Me | Me | H | CF3 | H | heptafluoroisopropyl | H | CF3 |
| 41-22 | 2,2,2-trichloroethyl | Me | Me | H | I | H | nonafluoro-2-butyl | H | OCF3 |
| 41-23 | 2,2,2-trichloroethyl | Me | Me | H | Cl | H | nonafluoro-2-butyl | H | CF3 |
| 41-24 | 2,2,2-trichloroethyl | Me | Me | H | CF3 | H | nonafluoro-2-butyl | H | CF3 |
| 41-25 | 2,2,2-trichloroethyl | H | H | H | Br | F | heptafluoroisopropyl | H | OCF3 |
| 41-26 | 2,2,2-trichloroethyl | H | H | H | I | H | heptafluoroisopropyl | Cl | CF3 |
| 41-27 | 2,2,2-trichloroethyl | H | H | H | CF3 | Br | heptafluoroisopropyl | H | CF3 |
| 41-28 | 2,2,2-trichloroethyl | H | H | H | I | H | nonafluoro-2-butyl | I | OCF3 |
| 41-29 | 2,2,2-trichloroethyl | H | H | H | Br | Me | nonafluoro-2-butyl | H | CF3 |
| 41-30 | 2,2,2-trichloroethyl | H | H | H | CF3 | H | nonafluoro-2-butyl | Et | CF3 |
| 41-31 | 2,2,2-trichloroethyl | H | H | Cl | Br | H | heptafluoroisopropyl | H | CF3 |

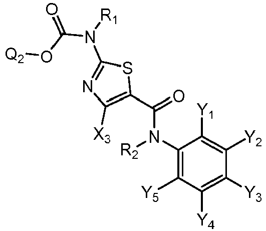

**Table 42(1)**

| compound number | Q₂ | R₁ | R₂ | X₃ | Y₁ | Y₂ | Y₃ | Y₄ | Y₅ |
|---|---|---|---|---|---|---|---|---|---|
| 42-1 | 2,2,2-trichloroethyl | H | H | H | Cl | H | heptafluoroisopropyl | H | OCF3 |
| 42-2 | 2,2,2-trichloroethyl | H | H | H | Br | H | heptafluoroisopropyl | H | CF3 |
| 42-3 | 2,2,2-trichloroethyl | H | H | H | CF3 | H | heptafluoroisopropyl | H | CF3 |
| 42-4 | 2,2,2-trichloroethyl | H | H | H | I | H | nonafluoro-2-butyl | H | OCF3 |
| 42-5 | 2,2,2-trichloroethyl | H | H | H | Cl | H | nonafluoro-2-butyl | H | CF3 |
| 42-6 | 2,2,2-trichloroethyl | H | H | H | CF3 | H | nonafluoro-2-butyl | H | CF3 |
| 42-7 | 2,2,2-trichloroethyl | Me | H | H | Br | H | heptafluoroisopropyl | H | OCF3 |
| 42-8 | 2,2,2-trichloroethyl | Me | H | H | I | H | heptafluoroisopropyl | H | CF3 |
| 42-9 | 2,2,2-trichloroethyl | Me | H | H | CF3 | H | heptafluoroisopropyl | H | CF3 |
| 42-10 | 2,2,2-trichloroethyl | Me | H | H | Cl | H | nonafluoro-2-butyl | H | OCF3 |
| 42-11 | 2,2,2-trichloroethyl | Me | H | H | Br | H | nonafluoro-2-butyl | H | CF3 |
| 42-12 | 2,2,2-trichloroethyl | Me | H | H | CF3 | H | nonafluoro-2-butyl | H | CF3 |
| 42-13 | 2,2,2-trichloroethyl | H | Me | H | I | H | heptafluoroisopropyl | H | OCF3 |
| 42-14 | 2,2,2-trichloroethyl | H | Me | H | Cl | H | heptafluoroisopropyl | H | CF3 |
| 42-15 | 2,2,2-trichloroethyl | H | Me | H | CF3 | H | heptafluoroisopropyl | H | CF3 |
| 42-16 | 2,2,2-trichloroethyl | H | Me | H | Br | H | nonafluoro-2-butyl | H | OCF3 |
| 42-17 | 2,2,2-trichloroethyl | H | Me | H | I | H | nonafluoro-2-butyl | H | CF3 |
| 42-18 | 2,2,2-trichloroethyl | H | Me | H | CF3 | H | nonafluoro-2-butyl | H | CF3 |
| 42-19 | 2,2,2-trichloroethyl | Me | Me | H | Cl | H | heptafluoroisopropyl | H | OCF3 |
| 42-20 | 2,2,2-trichloroethyl | Me | Me | H | Br | H | heptafluoroisopropyl | H | CF3 |
| 42-21 | 2,2,2-trichloroethyl | Me | Me | H | CF3 | H | heptafluoroisopropyl | H | CF3 |
| 42-22 | 2,2,2-trichloroethyl | Me | Me | H | I | H | nonafluoro-2-butyl | H | OCF3 |
| 42-23 | 2,2,2-trichloroethyl | Me | Me | H | Cl | H | nonafluoro-2-butyl | H | CF3 |
| 42-24 | 2,2,2-trichloroethyl | Me | Me | H | CF3 | H | nonafluoro-2-butyl | H | CF3 |
| 42-25 | 2,2,2-trichloroethyl | H | H | H | Br | F | heptafluoroisopropyl | H | OCF3 |
| 42-26 | 2,2,2-trichloroethyl | H | H | H | I | H | heptafluoroisopropyl | Cl | CF3 |
| 42-27 | 2,2,2-trichloroethyl | H | H | H | CF3 | Br | heptafluoroisopropyl | H | CF3 |
| 42-28 | 2,2,2-trichloroethyl | H | H | H | I | H | nonafluoro-2-butyl | I | OCF3 |
| 42-29 | 2,2,2-trichloroethyl | H | H | H | Br | Me | nonafluoro-2-butyl | H | CF3 |
| 42-30 | 2,2,2-trichloroethyl | H | H | H | CF3 | H | nonafluoro-2-butyl | Et | CF3 |
| 42-31 | 2,2,2-trichloroethyl | H | H | Cl | Br | H | heptafluoroisopropyl | H | CF3 |

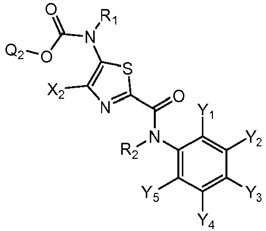

**Table 43(1)**

| compound number | Q₂ | R₁ | R₂ | X₂ | Y₁ | Y₂ | Y₃ | Y₄ | Y₅ |
|---|---|---|---|---|---|---|---|---|---|
| 43-1 | 2,2,2-trichloroethyl | H | H | H | Cl | H | heptafluoroisopropyl | H | OCF3 |
| 43-2 | 2,2,2-trichloroethyl | H | H | H | Br | H | heptafluoroisopropyl | H | CF3 |
| 43-3 | 2,2,2-trichloroethyl | H | H | H | CF3 | H | heptafluoroisopropyl | H | CF3 |
| 43-4 | 2,2,2-trichloroethyl | H | H | H | I | H | nonafluoro-2-butyl | H | OCF3 |
| 43-5 | 2,2,2-trichloroethyl | H | H | H | Cl | H | nonafluoro-2-butyl | H | CF3 |
| 43-6 | 2,2,2-trichloroethyl | H | H | H | CF3 | H | nonafluoro-2-butyl | H | CF3 |
| 43-7 | 2,2,2-trichloroethyl | Me | H | H | Br | H | heptafluoroisopropyl | H | OCF3 |
| 43-8 | 2,2,2-trichloroethyl | Me | H | H | I | H | heptafluoroisopropyl | H | CF3 |
| 43-9 | 2,2,2-trichloroethyl | Me | H | H | CF3 | H | heptafluoroisopropyl | H | CF3 |
| 43-10 | 2,2,2-trichloroethyl | Me | H | H | Cl | H | nonafluoro-2-butyl | H | OCF3 |
| 43-11 | 2,2,2-trichloroethyl | Me | H | H | Br | H | nonafluoro-2-butyl | H | CF3 |
| 43-12 | 2,2,2-trichloroethyl | Me | H | H | CF3 | H | nonafluoro-2-butyl | H | CF3 |
| 43-13 | 2,2,2-trichloroethyl | H | Me | H | I | H | heptafluoroisopropyl | H | OCF3 |
| 43-14 | 2,2,2-trichloroethyl | H | Me | H | Cl | H | heptafluoroisopropyl | H | CF3 |
| 43-15 | 2,2,2-trichloroethyl | H | Me | H | CF3 | H | heptafluoroisopropyl | H | CF3 |
| 43-16 | 2,2,2-trichloroethyl | H | Me | H | Br | H | nonafluoro-2-butyl | H | OCF3 |
| 43-17 | 2,2,2-trichloroethyl | H | Me | H | I | H | nonafluoro-2-butyl | H | CF3 |
| 43-18 | 2,2,2-trichloroethyl | H | Me | H | CF3 | H | nonafluoro-2-butyl | H | CF3 |
| 43-19 | 2,2,2-trichloroethyl | Me | Me | H | Cl | H | heptafluoroisopropyl | H | OCF3 |
| 43-20 | 2,2,2-trichloroethyl | Me | Me | H | Br | H | heptafluoroisopropyl | H | CF3 |
| 43-21 | 2,2,2-trichloroethyl | Me | Me | H | CF3 | H | heptafluoroisopropyl | H | CF3 |
| 43-22 | 2,2,2-trichloroethyl | Me | Me | H | I | H | nonafluoro-2-butyl | H | OCF3 |
| 43-23 | 2,2,2-trichloroethyl | Me | Me | H | Cl | H | nonafluoro-2-butyl | H | CF3 |
| 43-24 | 2,2,2-trichloroethyl | Me | Me | H | CF3 | H | nonafluoro-2-butyl | H | CF3 |
| 43-25 | 2,2,2-trichloroethyl | H | H | H | Br | F | heptafluoroisopropyl | H | OCF3 |
| 43-26 | 2,2,2-trichloroethyl | H | H | H | I | H | heptafluoroisopropyl | Cl | CF3 |
| 43-27 | 2,2,2-trichloroethyl | H | H | H | CF3 | Br | heptafluoroisopropyl | H | CF3 |
| 43-28 | 2,2,2-trichloroethyl | H | H | H | I | H | nonafluoro-2-butyl | I | OCF3 |
| 43-29 | 2,2,2-trichloroethyl | H | H | H | Br | Me | nonafluoro-2-butyl | H | CF3 |
| 43-30 | 2,2,2-trichloroethyl | H | H | H | CF3 | H | nonafluoro-2-butyl | Et | CF3 |
| 43-31 | 2,2,2-trichloroethyl | H | H | Cl | Br | H | heptafluoroisopropyl | H | CF3 |

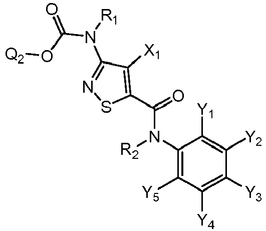

**Table 44(1)**

| compound number | Q₂ | R₁ | R₂ | X₁ | Y₁ | Y₂ | Y₃ | Y₄ | Y₅ |
|---|---|---|---|---|---|---|---|---|---|
| 44-1 | 2,2,2-trichloroethyl | H | H | H | Cl | H | heptafluoroisopropyl | H | OCF3 |
| 44-2 | 2,2,2-trichloroethyl | H | H | H | Br | H | heptafluoroisopropyl | H | CF3 |
| 44-3 | 2,2,2-trichloroethyl | H | H | H | CF3 | H | heptafluoroisopropyl | H | CF3 |
| 44-4 | 2,2,2-trichloroethyl | H | H | H | I | H | nonafluoro-2-butyl | H | OCF3 |
| 44-5 | 2,2,2-trichloroethyl | H | H | H | Cl | H | nonafluoro-2-butyl | H | CF3 |
| 44-6 | 2,2,2-trichloroethyl | H | H | H | CF3 | H | nonafluoro-2-butyl | H | CF3 |
| 44-7 | 2,2,2-trichloroethyl | Me | H | H | Br | H | heptafluoroisopropyl | H | OCF3 |
| 44-8 | 2,2,2-trichloroethyl | Me | H | H | I | H | heptafluoroisopropyl | H | CF3 |
| 44-9 | 2,2,2-trichloroethyl | Me | H | H | CF3 | H | heptafluoroisopropyl | H | CF3 |
| 44-10 | 2,2,2-trichloroethyl | Me | H | H | Cl | H | nonafluoro-2-butyl | H | OCF3 |
| 44-11 | 2,2,2-trichloroethyl | Me | H | H | Br | H | nonafluoro-2-butyl | H | CF3 |
| 44-12 | 2,2,2-trichloroethyl | Me | H | H | CF3 | H | nonafluoro-2-butyl | H | CF3 |
| 44-13 | 2,2,2-trichloroethyl | H | Me | H | I | H | heptafluoroisopropyl | H | OCF3 |
| 44-14 | 2,2,2-trichloroethyl | H | Me | H | Cl | H | heptafluoroisopropyl | H | CF3 |
| 44-15 | 2,2,2-trichloroethyl | H | Me | H | CF3 | H | heptafluoroisopropyl | H | CF3 |
| 44-16 | 2,2,2-trichloroethyl | H | Me | H | Br | H | nonafluoro-2-butyl | H | OCF3 |
| 44-17 | 2,2,2-trichloroethyl | H | Me | H | I | H | nonafluoro-2-butyl | H | CF3 |
| 44-18 | 2,2,2-trichloroethyl | H | Me | H | CF3 | H | nonafluoro-2-butyl | H | CF3 |
| 44-19 | 2,2,2-trichloroethyl | Me | Me | H | Cl | H | heptafluoroisopropyl | H | OCF3 |
| 44-20 | 2,2,2-trichloroethyl | Me | Me | H | Br | H | heptafluoroisopropyl | H | CF3 |
| 44-21 | 2,2,2-trichloroethyl | Me | Me | H | CF3 | H | heptafluoroisopropyl | H | CF3 |
| 44-22 | 2,2,2-trichloroethyl | Me | Me | H | I | H | nonafluoro-2-butyl | H | OCF3 |
| 44-23 | 2,2,2-trichloroethyl | Me | Me | H | Cl | H | nonafluoro-2-butyl | H | CF3 |
| 44-24 | 2,2,2-trichloroethyl | Me | Me | H | CF3 | H | nonafluoro-2-butyl | H | CF3 |
| 44-25 | 2,2,2-trichloroethyl | H | H | H | Br | F | heptafluoroisopropyl | H | OCF3 |
| 44-26 | 2,2,2-trichloroethyl | H | H | H | I | H | heptafluoroisopropyl | Cl | CF3 |
| 44-27 | 2,2,2-trichloroethyl | H | H | H | CF3 | Br | heptafluoroisopropyl | H | CF3 |
| 44-28 | 2,2,2-trichloroethyl | H | H | H | I | H | nonafluoro-2-butyl | I | OCF3 |
| 44-29 | 2,2,2-trichloroethyl | H | H | H | Br | Me | nonafluoro-2-butyl | H | CF3 |
| 44-30 | 2,2,2-trichloroethyl | H | H | H | CF3 | H | nonafluoro-2-butyl | Et | CF3 |
| 44-31 | 2,2,2-trichloroethyl | H | H | Cl | Br | H | heptafluoroisopropyl | H | CF3 |

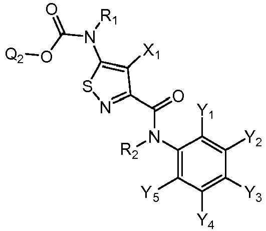

**Table 45(1)**

| compound number | Q₂ | R₁ | R₂ | X₁ | Y₁ | Y₂ | Y₃ | Y₄ | Y₅ |
|---|---|---|---|---|---|---|---|---|---|
| 45-1 | 2,2,2-trichloroethyl | H | H | H | Cl | H | heptafluoroisopropyl | H | OCF3 |
| 45-2 | 2,2,2-trichloroethyl | H | H | H | Br | H | heptafluoroisopropyl | H | CF3 |
| 45-3 | 2,2,2-trichloroethyl | H | H | H | CF3 | H | heptafluoroisopropyl | H | CF3 |
| 45-4 | 2,2,2-trichloroethyl | H | H | H | I | H | nonafluoro-2-butyl | H | OCF3 |
| 45-5 | 2,2,2-trichloroethyl | H | H | H | Cl | H | nonafluoro-2-butyl | H | CF3 |
| 45-6 | 2,2,2-trichloroethyl | H | H | H | CF3 | H | nonafluoro-2-butyl | H | CF3 |
| 45-7 | 2,2,2-trichloroethyl | Me | H | H | Br | H | heptafluoroisopropyl | H | OCF3 |
| 45-8 | 2,2,2-trichloroethyl | Me | H | H | I | H | heptafluoroisopropyl | H | CF3 |
| 45-9 | 2,2,2-trichloroethyl | Me | H | H | CF3 | H | heptafluoroisopropyl | H | CF3 |
| 45-10 | 2,2,2-trichloroethyl | Me | H | H | Cl | H | nonafluoro-2-butyl | H | OCF3 |
| 45-11 | 2,2,2-trichloroethyl | Me | H | H | Br | H | nonafluoro-2-butyl | H | CF3 |
| 45-12 | 2,2,2-trichloroethyl | Me | H | H | CF3 | H | nonafluoro-2-butyl | H | CF3 |
| 45-13 | 2,2,2-trichloroethyl | H | Me | H | I | H | heptafluoroisopropyl | H | OCF3 |
| 45-14 | 2,2,2-trichloroethyl | H | Me | H | Cl | H | heptafluoroisopropyl | H | CF3 |
| 45-15 | 2,2,2-trichloroethyl | H | Me | H | CF3 | H | heptafluoroisopropyl | H | CF3 |
| 45-16 | 2,2,2-trichloroethyl | H | Me | H | Br | H | nonafluoro-2-butyl | H | OCF3 |
| 45-17 | 2,2,2-trichloroethyl | H | Me | H | I | H | nonafluoro-2-butyl | H | CF3 |
| 45-18 | 2,2,2-trichloroethyl | H | Me | H | CF3 | H | nonafluoro-2-butyl | H | CF3 |
| 45-19 | 2,2,2-trichloroethyl | Me | Me | H | Cl | H | heptafluoroisopropyl | H | OCF3 |
| 45-20 | 2,2,2-trichloroethyl | Me | Me | H | Br | H | heptafluoroisopropyl | H | CF3 |
| 45-21 | 2,2,2-trichloroethyl | Me | Me | H | CF3 | H | heptafluoroisopropyl | H | CF3 |
| 45-22 | 2,2,2-trichloroethyl | Me | Me | H | I | H | nonafluoro-2-butyl | H | OCF3 |
| 45-23 | 2,2,2-trichloroethyl | Me | Me | H | Cl | H | nonafluoro-2-butyl | H | CF3 |
| 45-24 | 2,2,2-trichloroethyl | Me | Me | H | CF3 | H | nonafluoro-2-butyl | H | CF3 |
| 45-25 | 2,2,2-trichloroethyl | H | H | H | Br | F | heptafluoroisopropyl | H | OCF3 |
| 45-26 | 2,2,2-trichloroethyl | H | H | H | I | H | heptafluoroisopropyl | Cl | CF3 |
| 45-27 | 2,2,2-trichloroethyl | H | H | H | CF3 | Br | heptafluoroisopropyl | H | CF3 |
| 45-28 | 2,2,2-trichloroethyl | H | H | H | I | H | nonafluoro-2-butyl | I | OCF3 |
| 45-29 | 2,2,2-trichloroethyl | H | H | H | Br | Me | nonafluoro-2-butyl | H | CF3 |
| 45-30 | 2,2,2-trichloroethyl | H | H | H | CF3 | H | nonafluoro-2-butyl | Et | CF3 |
| 45-31 | 2,2,2-trichloroethyl | H | H | Cl | Br | H | heptafluoroisopropyl | H | CF3 |

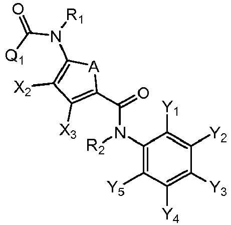

**Table 46(1)**

| compound number | Q₁ | R₁ | R₂ | A | X₂ | X₃ | Y₁ | Y₂ | Y₃ | Y₄ | Y₅ |
|---|---|---|---|---|---|---|---|---|---|---|---|
| 46-1 | phenyl | H | H | S | H | H | Cl | H | heptafluoroisopropyl | H | OCF3 |
| 46-2 | phenyl | H | H | S | H | H | Br | H | heptafluoroisopropyl | H | CF3 |
| 46-3 | phenyl | H | H | S | H | H | CF3 | H | heptafluoroisopropyl | H | CF3 |
| 46-4 | phenyl | H | H | S | H | H | I | H | nonafluoro-2-butyl | H | OCF3 |
| 46-5 | phenyl | H | H | S | H | H | Cl | H | nonafluoro-2-butyl | H | CF3 |
| 46-6 | phenyl | H | H | S | H | H | CF3 | H | nonafluoro-2-butyl | H | CF3 |
| 46-7 | phenyl | H | H | O | H | H | Br | H | heptafluoroisopropyl | H | OCF3 |
| 46-8 | phenyl | H | H | O | H | H | I | H | heptafluoroisopropyl | H | CF3 |
| 46-9 | phenyl | H | H | O | H | H | CF3 | H | heptafluoroisopropyl | H | CF3 |
| 46-10 | phenyl | H | H | O | H | H | Cl | H | nonafluoro-2-butyl | H | OCF3 |
| 46-11 | phenyl | H | H | O | H | H | Br | H | nonafluoro-2-butyl | H | CF3 |
| 46-12 | phenyl | H | H | O | H | H | CF3 | H | nonafluoro-2-butyl | H | CF3 |
| 46-13 | phenyl | H | H | NH | H | H | I | H | heptafluoroisopropyl | H | OCF3 |
| 46-14 | phenyl | H | H | NH | H | H | Cl | H | heptafluoroisopropyl | H | CF3 |
| 46-15 | phenyl | H | H | NH | H | H | CF3 | H | heptafluoroisopropyl | H | CF3 |
| 46-16 | phenyl | H | H | NH | H | H | Br | H | nonafluoro-2-butyl | H | OCF3 |
| 46-17 | phenyl | H | H | NH | H | H | I | H | nonafluoro-2-butyl | H | CF3 |
| 46-18 | phenyl | H | H | NH | H | H | CF3 | H | nonafluoro-2-butyl | H | CF3 |
| 46-19 | phenyl | Me | H | S | H | H | Cl | H | heptafluoroisopropyl | H | OCF3 |
| 46-20 | phenyl | Me | H | S | H | H | Br | H | heptafluoroisopropyl | H | CF3 |
| 46-21 | phenyl | Me | H | S | H | H | CF3 | H | heptafluoroisopropyl | H | CF3 |
| 46-22 | phenyl | Me | H | S | H | H | I | H | nonafluoro-2-butyl | H | OCF3 |
| 46-23 | phenyl | Me | H | S | H | H | Cl | H | nonafluoro-2-butyl | H | CF3 |
| 46-24 | phenyl | Me | H | S | H | H | CF3 | H | nonafluoro-2-butyl | H | CF3 |
| 46-25 | phenyl | Me | H | O | H | H | Br | H | heptafluoroisopropyl | H | OCF3 |
| 46-26 | phenyl | Me | H | O | H | H | I | H | heptafluoroisopropyl | H | CF3 |
| 46-27 | phenyl | Me | H | O | H | H | CF3 | H | heptafluoroisopropyl | H | CF3 |
| 46-28 | phenyl | Me | H | O | H | H | Cl | H | nonafluoro-2-butyl | H | OCF3 |
| 46-29 | phenyl | Me | H | O | H | H | Br | H | nonafluoro-2-butyl | H | CF3 |
| 46-30 | phenyl | Me | H | O | H | H | CF3 | H | nonafluoro-2-butyl | H | CF3 |
| 46-31 | phenyl | Me | H | NH | H | H | I | H | heptafluoroisopropyl | H | OCF3 |
| 46-32 | phenyl | Me | H | NH | H | H | Cl | H | heptafluoroisopropyl | H | CF3 |
| 46-33 | phenyl | Me | H | NH | H | H | CF3 | H | heptafluoroisopropyl | H | CF3 |
| 46-34 | phenyl | Me | H | NH | H | H | Br | H | nonafluoro-2-butyl | H | OCF3 |
| 46-35 | phenyl | Me | H | NH | H | H | I | H | nonafluoro-2-butyl | H | CF3 |
| 46-36 | phenyl | Me | H | NH | H | H | CF3 | H | nonafluoro-2-butyl | H | CF3 |
| 46-37 | phenyl | H | Me | S | H | H | Cl | H | heptafluoroisopropyl | H | OCF3 |
| 46-38 | phenyl | H | Me | S | H | H | Br | H | heptafluoroisopropyl | H | CF3 |
| 46-39 | phenyl | H | Me | S | H | H | CF3 | H | heptafluoroisopropyl | H | CF3 |
| 46-40 | phenyl | H | Me | S | H | H | I | H | nonafluoro-2-butyl | H | OCF3 |
| 46-41 | phenyl | H | Me | S | H | H | Cl | H | nonafluoro-2-butyl | H | CF3 |
| 46-42 | phenyl | H | Me | S | H | H | CF3 | H | nonafluoro-2-butyl | H | CF3 |
| 46-43 | phenyl | H | Me | O | H | H | Br | H | heptafluoroisopropyl | H | OCF3 |
| 46-44 | phenyl | H | Me | O | H | H | I | H | heptafluoroisopropyl | H | CF3 |
| 46-45 | phenyl | H | Me | O | H | H | CF3 | H | heptafluoroisopropyl | H | CF3 |
| 46-46 | phenyl | H | Me | O | H | H | Cl | H | nonafluoro-2-butyl | H | OCF3 |
| 46-47 | phenyl | H | Me | O | H | H | Br | H | nonafluoro-2-butyl | H | CF3 |
| 46-48 | phenyl | H | Me | O | H | H | CF3 | H | nonafluoro-2-butyl | H | CF3 |
| 46-49 | phenyl | H | Me | NH | H | H | I | H | heptafluoroisopropyl | H | OCF3 |
| 46-50 | phenyl | H | Me | NH | H | H | Cl | H | heptafluoroisopropyl | H | CF3 |
| 46-51 | phenyl | H | Me | NH | H | H | CF3 | H | heptafluoroisopropyl | H | CF3 |

**Table 46(2)**

| compound number | Q₁ | R₁ | R₂ | A | X₂ | X₃ | Y₁ | Y₂ | Y₃ | Y₄ | Y₅ |
|---|---|---|---|---|---|---|---|---|---|---|---|
| 46-52 | phenyl | H | Me | NH | H | H | Br | H | nonafluoro-2-butyl | H | OCF3 |
| 46-53 | phenyl | H | Me | NH | H | H | I | H | nonafluoro-2-butyl | H | CF3 |
| 46-54 | phenyl | H | Me | NH | H | H | CF3 | H | nonafluoro-2-butyl | H | CF3 |
| 46-55 | phenyl | Me | Me | S | H | H | Cl | H | heptafluoroisopropyl | H | OCF3 |
| 46-56 | phenyl | Me | Me | S | H | H | Br | H | heptafluoroisopropyl | H | CF3 |
| 46-57 | phenyl | Me | Me | S | H | H | CF3 | H | heptafluoroisopropyl | H | CF3 |
| 46-58 | phenyl | Me | Me | S | H | H | I | H | nonafluoro-2-butyl | H | OCF3 |
| 46-59 | phenyl | Me | Me | S | H | H | Cl | H | nonafluoro-2-butyl | H | CF3 |
| 46-60 | phenyl | Me | Me | S | H | H | CF3 | H | nonafluoro-2-butyl | H | CF3 |
| 46-61 | phenyl | Me | Me | O | H | H | Br | H | heptafluoroisopropyl | H | OCF3 |
| 46-62 | phenyl | Me | Me | O | H | H | I | H | heptafluoroisopropyl | H | CF3 |
| 46-63 | phenyl | Me | Me | O | H | H | CF3 | H | heptafluoroisopropyl | H | CF3 |
| 46-64 | phenyl | Me | Me | O | H | H | Cl | H | nonafluoro-2-butyl | H | OCF3 |
| 46-65 | phenyl | Me | Me | O | H | H | Br | H | nonafluoro-2-butyl | H | CF3 |
| 46-66 | phenyl | Me | Me | O | H | H | CF3 | H | nonafluoro-2-butyl | H | CF3 |
| 46-67 | phenyl | Me | Me | NH | H | H | I | H | heptafluoroisopropyl | H | OCF3 |
| 46-68 | phenyl | Me | Me | NH | H | H | Cl | H | heptafluoroisopropyl | H | CF3 |
| 46-69 | phenyl | Me | Me | NH | H | H | CF3 | H | heptafluoroisopropyl | H | CF3 |
| 46-70 | phenyl | Me | Me | NH | H | H | Br | H | nonafluoro-2-butyl | H | OCF3 |
| 46-71 | phenyl | Me | Me | NH | H | H | I | H | nonafluoro-2-butyl | H | CF3 |
| 46-72 | phenyl | Me | Me | NH | H | H | CF3 | H | nonafluoro-2-butyl | H | CF3 |
| 46-73 | phenyl | Me | H | S | H | H | Cl | F | heptafluoroisopropyl | H | OCF3 |
| 46-74 | phenyl | H | H | S | H | H | Br | H | heptafluoroisopropyl | F | CF3 |
| 46-75 | phenyl | H | H | S | H | H | CF3 | Cl | heptafluoroisopropyl | H | CF3 |
| 46-76 | phenyl | H | H | S | H | H | I | H | nonafluoro-2-butyl | Cl | OCF3 |
| 46-77 | phenyl | H | H | S | H | H | Cl | Br | nonafluoro-2-butyl | H | CF3 |
| 46-78 | phenyl | H | H | S | H | H | CF3 | H | nonafluoro-2-butyl | Br | CF3 |
| 46-79 | phenyl | H | H | O | H | H | Br | I | heptafluoroisopropyl | H | OCF3 |
| 46-80 | phenyl | Me | H | O | H | H | I | H | heptafluoroisopropyl | I | CF3 |
| 46-81 | phenyl | H | H | O | H | H | CF3 | Me | heptafluoroisopropyl | H | CF3 |
| 46-82 | phenyl | H | H | O | H | H | Cl | H | nonafluoro-2-butyl | Me | OCF3 |
| 46-83 | phenyl | H | H | O | H | H | Br | F | nonafluoro-2-butyl | F | CF3 |
| 46-84 | phenyl | H | H | O | H | H | CF3 | Cl | nonafluoro-2-butyl | Cl | CF3 |
| 46-85 | phenyl | H | H | NH | H | H | I | Br | heptafluoroisopropyl | Br | OCF3 |
| 46-86 | phenyl | H | H | NH | H | H | Cl | I | heptafluoroisopropyl | I | CF3 |
| 46-87 | phenyl | Me | H | NH | H | H | CF3 | F | heptafluoroisopropyl | Me | CF3 |
| 46-88 | phenyl | H | H | NH | H | H | Br | Cl | nonafluoro-2-butyl | Me | OCF3 |
| 46-89 | phenyl | H | H | NH | H | H | I | Br | nonafluoro-2-butyl | Me | CF3 |
| 46-90 | phenyl | H | H | NH | H | H | CF3 | I | nonafluoro-2-butyl | Me | CF3 |

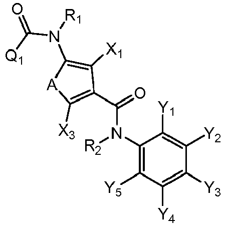

**Table 47(1)**

| compound number | Q₁ | R₁ | R₂ | A | X₁ | X₃ | Y₁ | Y₂ | Y₃ | Y₄ | Y₅ |
|---|---|---|---|---|---|---|---|---|---|---|---|
| 47-1 | phenyl | H | H | S | H | H | Cl | H | heptafluoroisopropyl | H | OCF3 |
| 47-2 | phenyl | H | H | S | H | H | Br | H | heptafluoroisopropyl | H | CF3 |
| 47-3 | phenyl | H | H | S | H | H | CF3 | H | heptafluoroisopropyl | H | CF3 |
| 47-4 | phenyl | H | H | S | H | H | I | H | nonafluoro-2-butyl | H | OCF3 |
| 47-5 | phenyl | H | H | S | H | H | Cl | H | nonafluoro-2-butyl | H | CF3 |
| 47-6 | phenyl | H | H | S | H | H | CF3 | H | nonafluoro-2-butyl | H | CF3 |
| 47-7 | phenyl | H | H | O | H | H | Br | H | heptafluoroisopropyl | H | OCF3 |
| 47-8 | phenyl | H | H | O | H | H | I | H | heptafluoroisopropyl | H | CF3 |
| 47-9 | phenyl | H | H | O | H | H | CF3 | H | heptafluoroisopropyl | H | CF3 |
| 47-10 | phenyl | H | H | O | H | H | Cl | H | nonafluoro-2-butyl | H | OCF3 |
| 47-11 | phenyl | H | H | O | H | H | Br | H | nonafluoro-2-butyl | H | CF3 |
| 47-12 | phenyl | H | H | O | H | H | CF3 | H | nonafluoro-2-butyl | H | CF3 |
| 47-13 | phenyl | H | H | NH | H | H | I | H | heptafluoroisopropyl | H | OCF3 |
| 47-14 | phenyl | H | H | NH | H | H | Cl | H | heptafluoroisopropyl | H | CF3 |
| 47-15 | phenyl | H | H | NH | H | H | CF3 | H | heptafluoroisopropyl | H | CF3 |
| 47-16 | phenyl | H | H | NH | H | H | Br | H | nonafluoro-2-butyl | H | OCF3 |
| 47-17 | phenyl | H | H | NH | H | H | I | H | nonafluoro-2-butyl | H | CF3 |
| 47-18 | phenyl | H | H | NH | H | H | CF3 | H | nonafluoro-2-butyl | H | CF3 |
| 47-19 | phenyl | Me | H | S | H | H | Cl | H | heptafluoroisopropyl | H | OCF3 |
| 47-20 | phenyl | Me | H | S | H | H | Br | H | heptafluoroisopropyl | H | CF3 |
| 47-21 | phenyl | Me | H | S | H | H | CF3 | H | heptafluoroisopropyl | H | CF3 |
| 47-22 | phenyl | Me | H | S | H | H | I | H | nonafluoro-2-butyl | H | OCF3 |
| 47-23 | phenyl | Me | H | S | H | H | Cl | H | nonafluoro-2-butyl | H | CF3 |
| 47-24 | phenyl | Me | H | S | H | H | CF3 | H | nonafluoro-2-butyl | H | CF3 |
| 47-25 | phenyl | Me | H | O | H | H | Br | H | heptafluoroisopropyl | H | OCF3 |
| 47-26 | phenyl | Me | H | O | H | H | I | H | heptafluoroisopropyl | H | CF3 |
| 47-27 | phenyl | Me | H | O | H | H | CF3 | H | heptafluoroisopropyl | H | CF3 |
| 47-28 | phenyl | Me | H | O | H | H | Cl | H | nonafluoro-2-butyl | H | OCF3 |
| 47-29 | phenyl | Me | H | O | H | H | Br | H | nonafluoro-2-butyl | H | CF3 |
| 47-30 | phenyl | Me | H | O | H | H | CF3 | H | nonafluoro-2-butyl | H | CF3 |
| 47-31 | phenyl | Me | H | NH | H | H | I | H | heptafluoroisopropyl | H | OCF3 |
| 47-32 | phenyl | Me | H | NH | H | H | Cl | H | heptafluoroisopropyl | H | CF3 |
| 47-33 | phenyl | Me | H | NH | H | H | CF3 | H | heptafluoroisopropyl | H | CF3 |
| 47-34 | phenyl | Me | H | NH | H | H | Br | H | nonafluoro-2-butyl | H | OCF3 |
| 47-35 | phenyl | Me | H | NH | H | H | I | H | nonafluoro-2-butyl | H | CF3 |
| 47-36 | phenyl | Me | H | NH | H | H | CF3 | H | nonafluoro-2-butyl | H | CF3 |
| 47-37 | phenyl | H | Me | S | H | H | Cl | H | heptafluoroisopropyl | H | OCF3 |
| 47-38 | phenyl | H | Me | S | H | H | Br | H | heptafluoroisopropyl | H | CF3 |
| 47-39 | phenyl | H | Me | S | H | H | CF3 | H | heptafluoroisopropyl | H | CF3 |
| 47-40 | phenyl | H | Me | S | H | H | I | H | nonafluoro-2-butyl | H | OCF3 |
| 47-41 | phenyl | H | Me | S | H | H | Cl | H | nonafluoro-2-butyl | H | CF3 |
| 47-42 | phenyl | H | Me | S | H | H | CF3 | H | nonafluoro-2-butyl | H | CF3 |
| 47-43 | phenyl | H | Me | O | H | H | Br | H | heptafluoroisopropyl | H | OCF3 |
| 47-44 | phenyl | H | Me | O | H | H | I | H | heptafluoroisopropyl | H | CF3 |
| 47-45 | phenyl | H | Me | O | H | H | CF3 | H | heptafluoroisopropyl | H | CF3 |
| 47-46 | phenyl | H | Me | O | H | H | Cl | H | nonafluoro-2-butyl | H | OCF3 |
| 47-47 | phenyl | H | Me | O | H | H | Br | H | nonafluoro-2-butyl | H | CF3 |
| 47-48 | phenyl | H | Me | O | H | H | CF3 | H | nonafluoro-2-butyl | H | CF3 |
| 47-49 | phenyl | H | Me | NH | H | H | I | H | heptafluoroisopropyl | H | OCF3 |
| 47-50 | phenyl | H | Me | NH | H | H | Cl | H | heptafluoroisopropyl | H | CF3 |
| 47-51 | phenyl | H | Me | NH | H | H | CF3 | H | heptafluoroisopropyl | H | CF3 |

**Table 47(2)**

| compound number | Q₁ | R₁ | R₂ | A | X₁ | X₃ | Y₁ | Y₂ | Y₃ | Y₄ | Y₅ |
|---|---|---|---|---|---|---|---|---|---|---|---|
| 47-52 | phenyl | H | Me | NH | H | H | Br | H | nonafluoro-2-butyl | H | OCF3 |
| 47-53 | phenyl | H | Me | NH | H | H | I | H | nonafluoro-2-butyl | H | CF3 |
| 47-54 | phenyl | H | Me | NH | H | H | CF3 | H | nonafluoro-2-butyl | H | CF3 |
| 47-55 | phenyl | Me | Me | S | H | H | Cl | H | heptafluoroisopropyl | H | OCF3 |
| 47-56 | phenyl | Me | Me | S | H | H | Br | H | heptafluoroisopropyl | H | CF3 |
| 47-57 | phenyl | Me | Me | S | H | H | CF3 | H | heptafluoroisopropyl | H | CF3 |
| 47-58 | phenyl | Me | Me | S | H | H | I | H | nonafluoro-2-butyl | H | OCF3 |
| 47-59 | phenyl | Me | Me | S | H | H | Cl | H | nonafluoro-2-butyl | H | CF3 |
| 47-60 | phenyl | Me | Me | S | H | H | CF3 | H | nonafluoro-2-butyl | H | CF3 |
| 47-61 | phenyl | Me | Me | O | H | H | Br | H | heptafluoroisopropyl | H | OCF3 |
| 47-62 | phenyl | Me | Me | O | H | H | I | H | heptafluoroisopropyl | H | CF3 |
| 47-63 | phenyl | Me | Me | O | H | H | CF3 | H | heptafluoroisopropyl | H | CF3 |
| 47-64 | phenyl | Me | Me | O | H | H | Cl | H | nonafluoro-2-butyl | H | OCF3 |
| 47-65 | phenyl | Me | Me | O | H | H | Br | H | nonafluoro-2-butyl | H | CF3 |
| 47-66 | phenyl | Me | Me | O | H | H | CF3 | H | nonafluoro-2-butyl | H | CF3 |
| 47-67 | phenyl | Me | Me | NH | H | H | I | H | heptafluoroisopropyl | H | OCF3 |
| 47-68 | phenyl | Me | Me | NH | H | H | Cl | H | heptafluoroisopropyl | H | CF3 |
| 47-69 | phenyl | Me | Me | NH | H | H | CF3 | H | heptafluoroisopropyl | H | CF3 |
| 47-70 | phenyl | Me | Me | NH | H | H | Br | H | nonafluoro-2-butyl | H | OCF3 |
| 47-71 | phenyl | Me | Me | NH | H | H | I | H | nonafluoro-2-butyl | H | CF3 |
| 47-72 | phenyl | Me | Me | NH | H | H | CF3 | H | nonafluoro-2-butyl | H | CF3 |
| 47-73 | phenyl | Me | H | S | H | H | Cl | F | heptafluoroisopropyl | H | OCF3 |
| 47-74 | phenyl | H | H | S | H | H | Br | H | heptafluoroisopropyl | F | CF3 |
| 47-75 | phenyl | H | H | S | H | H | CF3 | Cl | heptafluoroisopropyl | H | CF3 |
| 47-76 | phenyl | H | H | S | H | H | I | H | nonafluoro-2-butyl | Cl | OCF3 |
| 47-77 | phenyl | H | H | S | H | H | Cl | Br | nonafluoro-2-butyl | H | CF3 |
| 47-78 | phenyl | H | H | S | H | H | CF3 | H | nonafluoro-2-butyl | Br | CF3 |
| 47-79 | phenyl | H | H | O | H | H | Br | I | heptafluoroisopropyl | H | OCF3 |
| 47-80 | phenyl | Me | H | O | H | H | I | H | heptafluoroisopropyl | I | CF3 |
| 47-81 | phenyl | H | H | O | H | H | CF3 | Me | heptafluoroisopropyl | H | CF3 |
| 47-82 | phenyl | H | H | O | H | H | Cl | H | nonafluoro-2-butyl | Me | OCF3 |
| 47-83 | phenyl | H | H | O | H | H | Br | F | nonafluoro-2-butyl | F | CF3 |
| 47-84 | phenyl | H | H | O | H | H | CF3 | Cl | nonafluoro-2-butyl | Cl | CF3 |
| 47-85 | phenyl | H | H | NH | H | H | I | Br | heptafluoroisopropyl | Br | OCF3 |
| 47-86 | phenyl | H | H | NH | H | H | Cl | I | heptafluoroisopropyl | I | CF3 |
| 47-87 | phenyl | Me | H | NH | H | H | CF3 | F | heptafluoroisopropyl | Me | CF3 |
| 47-88 | phenyl | H | H | NH | H | H | Br | Cl | nonafluoro-2-butyl | Me | OCF3 |
| 47-89 | phenyl | H | H | NH | H | H | I | Br | nonafluoro-2-butyl | Me | CF3 |
| 47-90 | phenyl | H | H | NH | H | H | CF3 | I | nonafluoro-2-butyl | Me | CF3 |

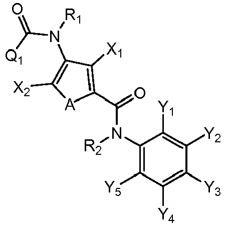

**Table 48(1)**

| compound number | Q₁ | R₁ | R₂ | A | X₁ | X₂ | Y₁ | Y₂ | Y₃ | Y₄ | Y₅ |
|---|---|---|---|---|---|---|---|---|---|---|---|
| 48-1 | phenyl | H | H | S | H | H | Cl | H | heptafluoroisopropyl | H | OCF3 |
| 48-2 | phenyl | H | H | S | H | H | Br | H | heptafluoroisopropyl | H | CF3 |
| 48-3 | phenyl | H | H | S | H | H | CF3 | H | heptafluoroisopropyl | H | CF3 |
| 48-4 | phenyl | H | H | S | H | H | I | H | nonafluoro-2-butyl | H | OCF3 |
| 48-5 | phenyl | H | H | S | H | H | Cl | H | nonafluoro-2-butyl | H | CF3 |
| 48-6 | phenyl | H | H | S | H | H | CF3 | H | nonafluoro-2-butyl | H | CF3 |
| 48-7 | phenyl | H | H | O | H | H | Br | H | heptafluoroisopropyl | H | OCF3 |
| 48-8 | phenyl | H | H | O | H | H | I | H | heptafluoroisopropyl | H | CF3 |
| 48-9 | phenyl | H | H | O | H | H | CF3 | H | heptafluoroisopropyl | H | CF3 |
| 48-10 | phenyl | H | H | O | H | H | Cl | H | nonafluoro-2-butyl | H | OCF3 |
| 48-11 | phenyl | H | H | O | H | H | Br | H | nonafluoro-2-butyl | H | CF3 |
| 48-12 | phenyl | H | H | O | H | H | CF3 | H | nonafluoro-2-butyl | H | CF3 |
| 48-13 | phenyl | H | H | NH | H | H | I | H | heptafluoroisopropyl | H | OCF3 |
| 48-14 | phenyl | H | H | NH | H | H | Cl | H | heptafluoroisopropyl | H | CF3 |
| 48-15 | phenyl | H | H | NH | H | H | CF3 | H | heptafluoroisopropyl | H | CF3 |
| 48-16 | phenyl | H | H | NH | H | H | Br | H | nonafluoro-2-butyl | H | OCF3 |
| 48-17 | phenyl | H | H | NH | H | H | I | H | nonafluoro-2-butyl | H | CF3 |
| 48-18 | phenyl | H | H | NH | H | H | CF3 | H | nonafluoro-2-butyl | H | CF3 |
| 48-19 | phenyl | Me | H | S | H | H | Cl | H | heptafluoroisopropyl | H | OCF3 |
| 48-20 | phenyl | Me | H | S | H | H | Br | H | heptafluoroisopropyl | H | CF3 |
| 48-21 | phenyl | Me | H | S | H | H | CF3 | H | heptafluoroisopropyl | H | CF3 |
| 48-22 | phenyl | Me | H | S | H | H | I | H | nonafluoro-2-butyl | H | OCF3 |
| 48-23 | phenyl | Me | H | S | H | H | Cl | H | nonafluoro-2-butyl | H | CF3 |
| 48-24 | phenyl | Me | H | S | H | H | CF3 | H | nonafluoro-2-butyl | H | CF3 |
| 48-25 | phenyl | Me | H | O | H | H | Br | H | heptafluoroisopropyl | H | OCF3 |
| 48-26 | phenyl | Me | H | O | H | H | I | H | heptafluoroisopropyl | H | CF3 |
| 48-27 | phenyl | Me | H | O | H | H | CF3 | H | heptafluoroisopropyl | H | CF3 |
| 48-28 | phenyl | Me | H | O | H | H | Cl | H | nonafluoro-2-butyl | H | OCF3 |
| 48-29 | phenyl | Me | H | O | H | H | Br | H | nonafluoro-2-butyl | H | CF3 |
| 48-30 | phenyl | Me | H | O | H | H | CF3 | H | nonafluoro-2-butyl | H | CF3 |
| 48-31 | phenyl | Me | H | NH | H | H | I | H | heptafluoroisopropyl | H | OCF3 |
| 48-32 | phenyl | Me | H | NH | H | H | Cl | H | heptafluoroisopropyl | H | CF3 |
| 48-33 | phenyl | Me | H | NH | H | H | CF3 | H | heptafluoroisopropyl | H | CF3 |
| 48-34 | phenyl | Me | H | NH | H | H | Br | H | nonafluoro-2-butyl | H | OCF3 |
| 48-35 | phenyl | Me | H | NH | H | H | I | H | nonafluoro-2-butyl | H | CF3 |
| 48-36 | phenyl | Me | H | NH | H | H | CF3 | H | nonafluoro-2-butyl | H | CF3 |
| 48-37 | phenyl | H | Me | S | H | H | Cl | H | heptafluoroisopropyl | H | OCF3 |
| 48-38 | phenyl | H | Me | S | H | H | Br | H | heptafluoroisopropyl | H | CF3 |
| 48-39 | phenyl | H | Me | S | H | H | CF3 | H | heptafluoroisopropyl | H | CF3 |
| 48-40 | phenyl | H | Me | S | H | H | I | H | nonafluoro-2-butyl | H | OCF3 |
| 48-41 | phenyl | H | Me | S | H | H | Cl | H | nonafluoro-2-butyl | H | CF3 |
| 48-42 | phenyl | H | Me | S | H | H | CF3 | H | nonafluoro-2-butyl | H | CF3 |
| 48-43 | phenyl | H | Me | O | H | H | Br | H | heptafluoroisopropyl | H | OCF3 |
| 48-44 | phenyl | H | Me | O | H | H | I | H | heptafluoroisopropyl | H | CF3 |
| 48-45 | phenyl | H | Me | O | H | H | CF3 | H | heptafluoroisopropyl | H | CF3 |
| 48-46 | phenyl | H | Me | O | H | H | Cl | H | nonafluoro-2-butyl | H | OCF3 |
| 48-47 | phenyl | H | Me | O | H | H | Br | H | nonafluoro-2-butyl | H | CF3 |
| 48-48 | phenyl | H | Me | O | H | H | CF3 | H | nonafluoro-2-butyl | H | CF3 |
| 48-49 | phenyl | H | Me | NH | H | H | I | H | heptafluoroisopropyl | H | OCF3 |
| 48-50 | phenyl | H | Me | NH | H | H | Cl | H | heptafluoroisopropyl | H | CF3 |
| 48-51 | phenyl | H | Me | NH | H | H | CF3 | H | heptafluoroisopropyl | H | CF3 |

**Table 48(2)**

| compound number | Q₁ | R₁ | R₂ | A | X₁ | X₂ | Y₁ | Y₂ | Y₃ | Y₄ | Y₅ |
|---|---|---|---|---|---|---|---|---|---|---|---|
| 48-52 | phenyl | H | Me | NH | H | H | Br | H | nonafluoro-2-butyl | H | OCF3 |
| 48-53 | phenyl | H | Me | NH | H | H | I | H | nonafluoro-2-butyl | H | CF3 |
| 48-54 | phenyl | H | Me | NH | H | H | CF3 | H | nonafluoro-2-butyl | H | CF3 |
| 48-55 | phenyl | Me | Me | S | H | H | Cl | H | heptafluoroisopropyl | H | OCF3 |
| 48-56 | phenyl | Me | Me | S | H | H | Br | H | heptafluoroisopropyl | H | CF3 |
| 48-57 | phenyl | Me | Me | S | H | H | CF3 | H | heptafluoroisopropyl | H | CF3 |
| 48-58 | phenyl | Me | Me | S | H | H | I | H | nonafluoro-2-butyl | H | OCF3 |
| 48-59 | phenyl | Me | Me | S | H | H | Cl | H | nonafluoro-2-butyl | H | CF3 |
| 48-60 | phenyl | Me | Me | S | H | H | CF3 | H | nonafluoro-2-butyl | H | CF3 |
| 48-61 | phenyl | Me | Me | O | H | H | Br | H | heptafluoroisopropyl | H | OCF3 |
| 48-62 | phenyl | Me | Me | O | H | H | I | H | heptafluoroisopropyl | H | CF3 |
| 48-63 | phenyl | Me | Me | O | H | H | CF3 | H | heptafluoroisopropyl | H | CF3 |
| 48-64 | phenyl | Me | Me | O | H | H | Cl | H | nonafluoro-2-butyl | H | OCF3 |
| 48-65 | phenyl | Me | Me | O | H | H | Br | H | nonafluoro-2-butyl | H | CF3 |
| 48-66 | phenyl | Me | Me | O | H | H | CF3 | H | nonafluoro-2-butyl | H | CF3 |
| 48-67 | phenyl | Me | Me | NH | H | H | I | H | heptafluoroisopropyl | H | OCF3 |
| 48-68 | phenyl | Me | Me | NH | H | H | Cl | H | heptafluoroisopropyl | H | CF3 |
| 48-69 | phenyl | Me | Me | NH | H | H | CF3 | H | heptafluoroisopropyl | H | CF3 |
| 48-70 | phenyl | Me | Me | NH | H | H | Br | H | nonafluoro-2-butyl | H | OCF3 |
| 48-71 | phenyl | Me | Me | NH | H | H | I | H | nonafluoro-2-butyl | H | CF3 |
| 48-72 | phenyl | Me | Me | NH | H | H | CF3 | H | nonafluoro-2-butyl | H | CF3 |
| 48-73 | phenyl | Me | H | S | H | H | Cl | F | heptafluoroisopropyl | H | OCF3 |
| 48-74 | phenyl | H | H | S | H | H | Br | H | heptafluoroisopropyl | F | CF3 |
| 48-75 | phenyl | H | H | S | H | H | CF3 | Cl | heptafluoroisopropyl | H | CF3 |
| 48-76 | phenyl | H | H | S | H | H | I | H | nonafluoro-2-butyl | Cl | OCF3 |
| 48-77 | phenyl | H | H | S | H | H | Cl | Br | nonafluoro-2-butyl | H | CF3 |
| 48-78 | phenyl | H | H | S | H | H | CF3 | H | nonafluoro-2-butyl | Br | CF3 |
| 48-79 | phenyl | H | H | O | H | H | Br | I | heptafluoroisopropyl | H | OCF3 |
| 48-80 | phenyl | Me | H | O | H | H | I | H | heptafluoroisopropyl | I | CF3 |
| 48-81 | phenyl | H | H | O | H | H | CF3 | Me | heptafluoroisopropyl | H | CF3 |
| 48-82 | phenyl | H | H | O | H | H | Cl | H | nonafluoro-2-butyl | Me | OCF3 |
| 48-83 | phenyl | H | H | O | H | H | Br | F | nonafluoro-2-butyl | F | CF3 |
| 48-84 | phenyl | H | H | O | H | H | CF3 | Cl | nonafluoro-2-butyl | Cl | CF3 |
| 48-85 | phenyl | H | H | NH | H | H | I | Br | heptafluoroisopropyl | Br | OCF3 |
| 48-86 | phenyl | H | H | NH | H | H | Cl | I | heptafluoroisopropyl | I | CF3 |
| 48-87 | phenyl | Me | H | NH | H | H | CF3 | F | heptafluoroisopropyl | Me | CF3 |
| 48-88 | phenyl | H | H | NH | H | H | Br | Cl | nonafluoro-2-butyl | Me | OCF3 |
| 48-89 | phenyl | H | H | NH | H | H | I | Br | nonafluoro-2-butyl | Me | CF3 |
| 48-90 | phenyl | H | H | NH | H | H | CF3 | I | nonafluoro-2-butyl | Me | CF3 |

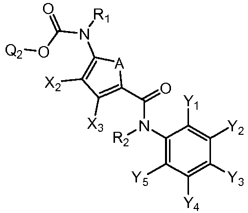

**Table 49(1)**

| compound number | Q₂ | R₁ | R₂ | A | X₂ | X₃ | Y₁ | Y₂ | Y₃ | Y₄ | Y₅ |
|---|---|---|---|---|---|---|---|---|---|---|---|
| 49-1 | 2,2,2-trichloroethyl | H | H | S | H | H | Cl | H | heptafluoroisopropyl | H | OCF3 |
| 49-2 | 2,2,2-trichloroethyl | H | H | S | H | H | Br | H | heptafluoroisopropyl | H | CF3 |
| 49-3 | 2,2,2-trichloroethyl | H | H | S | H | H | CF3 | H | heptafluoroisopropyl | H | CF3 |
| 49-4 | 2,2,2-trichloroethyl | H | H | S | H | H | I | H | nonafluoro-2-butyl | H | OCF3 |
| 49-5 | 2,2,2-trichloroethyl | H | H | S | H | H | Cl | H | nonafluoro-2-butyl | H | CF3 |
| 49-6 | 2,2,2-trichloroethyl | H | H | S | H | H | CF3 | H | nonafluoro-2-butyl | H | CF3 |
| 49-7 | 2,2,2-trichloroethyl | H | H | O | H | H | Br | H | heptafluoroisopropyl | H | OCF3 |
| 49-8 | 2,2,2-trichloroethyl | H | H | O | H | H | I | H | heptafluoroisopropyl | H | CF3 |
| 49-9 | 2,2,2-trichloroethyl | H | H | O | H | H | CF3 | H | heptafluoroisopropyl | H | CF3 |
| 49-10 | 2,2,2-trichloroethyl | H | H | O | H | H | Cl | H | nonafluoro-2-butyl | H | OCF3 |
| 49-11 | 2,2,2-trichloroethyl | H | H | O | H | H | Br | H | nonafluoro-2-butyl | H | CF3 |
| 49-12 | 2,2,2-trichloroethyl | H | H | O | H | H | CF3 | H | nonafluoro-2-butyl | H | CF3 |
| 49-13 | 2,2,2-trichloroethyl | H | H | NH | H | H | I | H | heptafluoroisopropyl | H | OCF3 |
| 49-14 | 2,2,2-trichloroethyl | H | H | NH | H | H | Cl | H | heptafluoroisopropyl | H | CF3 |
| 49-15 | 2,2,2-trichloroethyl | H | H | NH | H | H | CF3 | H | heptafluoroisopropyl | H | CF3 |
| 49-16 | 2,2,2-trichloroethyl | H | H | NH | H | H | Br | H | nonafluoro-2-butyl | H | OCF3 |
| 49-17 | 2,2,2-trichloroethyl | H | H | NH | H | H | I | H | nonafluoro-2-butyl | H | CF3 |
| 49-18 | 2,2,2-trichloroethyl | H | H | NH | H | H | CF3 | H | nonafluoro-2-butyl | H | CF3 |
| 49-19 | 2,2,2-trichloroethyl | Me | H | S | H | H | Cl | H | heptafluoroisopropyl | H | OCF3 |
| 49-20 | 2,2,2-trichloroethyl | Me | H | S | H | H | Br | H | heptafluoroisopropyl | H | CF3 |
| 49-21 | 2,2,2-trichloroethyl | Me | H | S | H | H | CF3 | H | heptafluoroisopropyl | H | CF3 |
| 49-22 | 2,2,2-trichloroethyl | Me | H | S | H | H | I | H | nonafluoro-2-butyl | H | OCF3 |
| 49-23 | 2,2,2-trichloroethyl | Me | H | S | H | H | Cl | H | nonafluoro-2-butyl | H | CF3 |
| 49-24 | 2,2,2-trichloroethyl | Me | H | S | H | H | CF3 | H | nonafluoro-2-butyl | H | CF3 |
| 49-25 | 2,2,2-trichloroethyl | Me | H | O | H | H | Br | H | heptafluoroisopropyl | H | OCF3 |
| 49-26 | 2,2,2-trichloroethyl | Me | H | O | H | H | I | H | heptafluoroisopropyl | H | CF3 |
| 49-27 | 2,2,2-trichloroethyl | Me | H | O | H | H | CF3 | H | heptafluoroisopropyl | H | CF3 |
| 49-28 | 2,2,2-trichloroethyl | Me | H | O | H | H | Cl | H | nonafluoro-2-butyl | H | OCF3 |
| 49-29 | 2,2,2-trichloroethyl | Me | H | O | H | H | Br | H | nonafluoro-2-butyl | H | CF3 |
| 49-30 | 2,2,2-trichloroethyl | Me | H | O | H | H | CF3 | H | nonafluoro-2-butyl | H | CF3 |
| 49-31 | 2,2,2-trichloroethyl | Me | H | NH | H | H | I | H | heptafluoroisopropyl | H | OCF3 |
| 49-32 | 2,2,2-trichloroethyl | Me | H | NH | H | H | Cl | H | heptafluoroisopropyl | H | CF3 |
| 49-33 | 2,2,2-trichloroethyl | Me | H | NH | H | H | CF3 | H | heptafluoroisopropyl | H | CF3 |
| 49-34 | 2,2,2-trichloroethyl | Me | H | NH | H | H | Br | H | nonafluoro-2-butyl | H | OCF3 |
| 49-35 | 2,2,2-trichloroethyl | Me | H | NH | H | H | I | H | nonafluoro-2-butyl | H | CF3 |
| 49-36 | 2,2,2-trichloroethyl | Me | H | NH | H | H | CF3 | H | nonafluoro-2-butyl | H | CF3 |
| 49-37 | 2,2,2-trichloroethyl | H | Me | S | H | H | Cl | H | heptafluoroisopropyl | H | OCF3 |
| 49-38 | 2,2,2-trichloroethyl | H | Me | S | H | H | Br | H | heptafluoroisopropyl | H | CF3 |
| 49-39 | 2,2,2-trichloroethyl | H | Me | S | H | H | CF3 | H | heptafluoroisopropyl | H | CF3 |
| 49-40 | 2,2,2-trichloroethyl | H | Me | S | H | H | I | H | nonafluoro-2-butyl | H | OCF3 |
| 49-41 | 2,2,2-trichloroethyl | H | Me | S | H | H | Cl | H | nonafluoro-2-butyl | H | CF3 |
| 49-42 | 2,2,2-trichloroethyl | H | Me | S | H | H | CF3 | H | nonafluoro-2-butyl | H | CF3 |
| 49-43 | 2,2,2-trichloroethyl | H | Me | O | H | H | Br | H | heptafluoroisopropyl | H | OCF3 |
| 49-44 | 2,2,2-trichloroethyl | H | Me | O | H | H | I | H | heptafluoroisopropyl | H | CF3 |
| 49-45 | 2,2,2-trichloroethyl | H | Me | O | H | H | CF3 | H | heptafluoroisopropyl | H | CF3 |
| 49-46 | 2,2,2-trichloroethyl | H | Me | O | H | H | Cl | H | nonafluoro-2-butyl | H | OCF3 |
| 49-47 | 2,2,2-trichloroethyl | H | Me | O | H | H | Br | H | nonafluoro-2-butyl | H | CF3 |
| 49-48 | 2,2,2-trichloroethyl | H | Me | O | H | H | CF3 | H | nonafluoro-2-butyl | H | CF3 |
| 49-49 | 2,2,2-trichloroethyl | H | Me | NH | H | H | I | H | heptafluoroisopropyl | H | OCF3 |
| 49-50 | 2,2,2-trichloroethyl | H | Me | NH | H | H | Cl | H | heptafluoroisopropyl | H | CF3 |
| 49-51 | 2,2,2-trichloroethyl | H | Me | NH | H | H | CF3 | H | heptafluoroisopropyl | H | CF3 |

**Table 49(2)**

| compound number | Q₂ | R₁ | R₂ | A | X₂ | X₃ | Y₁ | Y₂ | Y₃ | Y₄ | Y₅ |
|---|---|---|---|---|---|---|---|---|---|---|---|
| 49-52 | 2,2,2-trichloroethyl | H | Me | NH | H | H | Br | H | nonafluoro-2-butyl | H | OCF3 |
| 49-53 | 2,2,2-trichloroethyl | H | Me | NH | H | H | I | H | nonafluoro-2-butyl | H | CF3 |
| 49-54 | 2,2,2-trichloroethyl | H | Me | NH | H | H | CF3 | H | nonafluoro-2-butyl | H | CF3 |
| 49-55 | 2,2,2-trichloroethyl | Me | Me | S | H | H | Cl | H | heptafluoroisopropyl | H | OCF3 |
| 49-56 | 2,2,2-trichloroethyl | Me | Me | S | H | H | Br | H | heptafluoroisopropyl | H | CF3 |
| 49-57 | 2,2,2-trichloroethyl | Me | Me | S | H | H | CF3 | H | heptafluoroisopropyl | H | CF3 |
| 49-58 | 2,2,2-trichloroethyl | Me | Me | S | H | H | I | H | nonafluoro-2-butyl | H | OCF3 |
| 49-59 | 2,2,2-trichloroethyl | Me | Me | S | H | H | Cl | H | nonafluoro-2-butyl | H | CF3 |
| 49-60 | 2,2,2-trichloroethyl | Me | Me | S | H | H | CF3 | H | nonafluoro-2-butyl | H | CF3 |
| 49-61 | 2,2,2-trichloroethyl | Me | Me | O | H | H | Br | H | heptafluoroisopropyl | H | OCF3 |
| 49-62 | 2,2,2-trichloroethyl | Me | Me | O | H | H | I | H | heptafluoroisopropyl | H | CF3 |
| 49-63 | 2,2,2-trichloroethyl | Me | Me | O | H | H | CF3 | H | heptafluoroisopropyl | H | CF3 |
| 49-64 | 2,2,2-trichloroethyl | Me | Me | O | H | H | Cl | H | nonafluoro-2-butyl | H | OCF3 |
| 49-65 | 2,2,2-trichloroethyl | Me | Me | O | H | H | Br | H | nonafluoro-2-butyl | H | CF3 |
| 49-66 | 2,2,2-trichloroethyl | Me | Me | O | H | H | CF3 | H | nonafluoro-2-butyl | H | CF3 |
| 49-67 | 2,2,2-trichloroethyl | Me | Me | NH | H | H | I | H | heptafluoroisopropyl | H | OCF3 |
| 49-68 | 2,2,2-trichloroethyl | Me | Me | NH | H | H | Cl | H | heptafluoroisopropyl | H | CF3 |
| 49-69 | 2,2,2-trichloroethyl | Me | Me | NH | H | H | CF3 | H | heptafluoroisopropyl | H | CF3 |
| 49-70 | 2,2,2-trichloroethyl | Me | Me | NH | H | H | Br | H | nonafluoro-2-butyl | H | OCF3 |
| 49-71 | 2,2,2-trichloroethyl | Me | Me | NH | H | H | I | H | nonafluoro-2-butyl | H | CF3 |
| 49-72 | 2,2,2-trichloroethyl | Me | Me | NH | H | H | CF3 | H | nonafluoro-2-butyl | H | CF3 |
| 49-73 | 2,2,2-trichloroethyl | Me | H | S | H | H | Cl | F | heptafluoroisopropyl | H | OCF3 |
| 49-74 | 2,2,2-trichloroethyl | H | H | S | H | H | Br | H | heptafluoroisopropyl | F | CF3 |
| 49-75 | 2,2,2-trichloroethyl | H | H | S | H | H | CF3 | Cl | heptafluoroisopropyl | H | CF3 |
| 49-76 | 2,2,2-trichloroethyl | H | H | S | H | H | I | H | nonafluoro-2-butyl | Cl | OCF3 |
| 49-77 | 2,2,2-trichloroethyl | H | H | S | H | H | Cl | Br | nonafluoro-2-butyl | H | CF3 |
| 49-78 | 2,2,2-trichloroethyl | H | H | S | H | H | CF3 | H | nonafluoro-2-butyl | Br | CF3 |
| 49-79 | 2,2,2-trichloroethyl | H | H | O | H | H | Br | I | heptafluoroisopropyl | H | OCF3 |
| 49-80 | 2,2,2-trichloroethyl | Me | H | O | H | H | I | H | heptafluoroisopropyl | I | CF3 |
| 49-81 | 2,2,2-trichloroethyl | H | H | O | H | H | CF3 | Me | heptafluoroisopropyl | H | CF3 |
| 49-82 | 2,2,2-trichloroethyl | H | H | O | H | H | Cl | H | nonafluoro-2-butyl | Me | OCF3 |
| 49-83 | 2,2,2-trichloroethyl | H | H | O | H | H | Br | F | nonafluoro-2-butyl | F | CF3 |
| 49-84 | 2,2,2-trichloroethyl | H | H | O | H | H | CF3 | Cl | nonafluoro-2-butyl | Cl | CF3 |
| 49-85 | 2,2,2-trichloroethyl | H | H | NH | H | H | I | Br | heptafluoroisopropyl | Br | OCF3 |
| 49-86 | 2,2,2-trichloroethyl | H | H | NH | H | H | Cl | I | heptafluoroisopropyl | I | CF3 |
| 49-87 | 2,2,2-trichloroethyl | Me | H | NH | H | H | CF3 | F | heptafluoroisopropyl | Me | CF3 |
| 49-88 | 2,2,2-trichloroethyl | H | H | NH | H | H | Br | Cl | nonafluoro-2-butyl | Me | OCF3 |
| 49-89 | 2,2,2-trichloroethyl | H | H | NH | H | H | I | Br | nonafluoro-2-butyl | Me | CF3 |
| 49-90 | 2,2,2-trichloroethyl | H | H | NH | H | H | CF3 | I | nonafluoro-2-butyl | Me | CF3 |

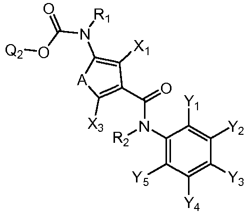

**Table 50(1)**

| compound number | Q₂ | R₁ | R₂ | A | X₁ | X₃ | Y₁ | Y₂ | Y₃ | Y₄ | Y₅ |
|---|---|---|---|---|---|---|---|---|---|---|---|
| 50-1 | 2,2,2-trichloroethyl | H | H | S | H | H | Cl | H | heptafluoroisopropyl | H | OCF3 |
| 50-2 | 2,2,2-trichloroethyl | H | H | S | H | H | Br | H | heptafluoroisopropyl | H | CF3 |
| 50-3 | 2,2,2-trichloroethyl | H | H | S | H | H | CF3 | H | heptafluoroisopropyl | H | CF3 |
| 50-4 | 2,2,2-trichloroethyl | H | H | S | H | H | I | H | nonafluoro-2-butyl | H | OCF3 |
| 50-5 | 2,2,2-trichloroethyl | H | H | S | H | H | Cl | H | nonafluoro-2-butyl | H | CF3 |
| 50-6 | 2,2,2-trichloroethyl | H | H | S | H | H | CF3 | H | nonafluoro-2-butyl | H | CF3 |
| 50-7 | 2,2,2-trichloroethyl | H | H | O | H | H | Br | H | heptafluoroisopropyl | H | OCF3 |
| 50-8 | 2,2,2-trichloroethyl | H | H | O | H | H | I | H | heptafluoroisopropyl | H | CF3 |
| 50-9 | 2,2,2-trichloroethyl | H | H | O | H | H | CF3 | H | heptafluoroisopropyl | H | CF3 |
| 50-10 | 2,2,2-trichloroethyl | H | H | O | H | H | Cl | H | nonafluoro-2-butyl | H | OCF3 |
| 50-11 | 2,2,2-trichloroethyl | H | H | O | H | H | Br | H | nonafluoro-2-butyl | H | CF3 |
| 50-12 | 2,2,2-trichloroethyl | H | H | O | H | H | CF3 | H | nonafluoro-2-butyl | H | CF3 |
| 50-13 | 2,2,2-trichloroethyl | H | H | NH | H | H | I | H | heptafluoroisopropyl | H | OCF3 |
| 50-14 | 2,2,2-trichloroethyl | H | H | NH | H | H | Cl | H | heptafluoroisopropyl | H | CF3 |
| 50-15 | 2,2,2-trichloroethyl | H | H | NH | H | H | CF3 | H | heptafluoroisopropyl | H | CF3 |
| 50-16 | 2,2,2-trichloroethyl | H | H | NH | H | H | Br | H | nonafluoro-2-butyl | H | OCF3 |
| 50-17 | 2,2,2-trichloroethyl | H | H | NH | H | H | I | H | nonafluoro-2-butyl | H | CF3 |
| 50-18 | 2,2,2-trichloroethyl | H | H | NH | H | H | CF3 | H | nonafluoro-2-butyl | H | CF3 |
| 50-19 | 2,2,2-trichloroethyl | Me | H | S | H | H | Cl | H | heptafluoroisopropyl | H | OCF3 |
| 50-20 | 2,2,2-trichloroethyl | Me | H | S | H | H | Br | H | heptafluoroisopropyl | H | CF3 |
| 50-21 | 2,2,2-trichloroethyl | Me | H | S | H | H | CF3 | H | heptafluoroisopropyl | H | CF3 |
| 50-22 | 2,2,2-trichloroethyl | Me | H | S | H | H | I | H | nonafluoro-2-butyl | H | OCF3 |
| 50-23 | 2,2,2-trichloroethyl | Me | H | S | H | H | Cl | H | nonafluoro-2-butyl | H | CF3 |
| 50-24 | 2,2,2-trichloroethyl | Me | H | S | H | H | CF3 | H | nonafluoro-2-butyl | H | CF3 |
| 50-25 | 2,2,2-trichloroethyl | Me | H | O | H | H | Br | H | heptafluoroisopropyl | H | OCF3 |
| 50-26 | 2,2,2-trichloroethyl | Me | H | O | H | H | I | H | heptafluoroisopropyl | H | CF3 |
| 50-27 | 2,2,2-trichloroethyl | Me | H | O | H | H | CF3 | H | heptafluoroisopropyl | H | CF3 |
| 50-28 | 2,2,2-trichloroethyl | Me | H | O | H | H | Cl | H | nonafluoro-2-butyl | H | OCF3 |
| 50-29 | 2,2,2-trichloroethyl | Me | H | O | H | H | Br | H | nonafluoro-2-butyl | H | CF3 |
| 50-30 | 2,2,2-trichloroethyl | Me | H | O | H | H | CF3 | H | nonafluoro-2-butyl | H | CF3 |
| 50-31 | 2,2,2-trichloroethyl | Me | H | NH | H | H | I | H | heptafluoroisopropyl | H | OCF3 |
| 50-32 | 2,2,2-trichloroethyl | Me | H | NH | H | H | Cl | H | heptafluoroisopropyl | H | CF3 |
| 50-33 | 2,2,2-trichloroethyl | Me | H | NH | H | H | CF3 | H | heptafluoroisopropyl | H | CF3 |
| 50-34 | 2,2,2-trichloroethyl | Me | H | NH | H | H | Br | H | nonafluoro-2-butyl | H | OCF3 |
| 50-35 | 2,2,2-trichloroethyl | Me | H | NH | H | H | I | H | nonafluoro-2-butyl | H | CF3 |
| 50-36 | 2,2,2-trichloroethyl | Me | H | NH | H | H | CF3 | H | nonafluoro-2-butyl | H | CF3 |
| 50-37 | 2,2,2-trichloroethyl | H | Me | S | H | H | Cl | H | heptafluoroisopropyl | H | OCF3 |
| 50-38 | 2,2,2-trichloroethyl | H | Me | S | H | H | Br | H | heptafluoroisopropyl | H | CF3 |
| 50-39 | 2,2,2-trichloroethyl | H | Me | S | H | H | CF3 | H | heptafluoroisopropyl | H | CF3 |
| 50-40 | 2,2,2-trichloroethyl | H | Me | S | H | H | I | H | nonafluoro-2-butyl | H | OCF3 |
| 50-41 | 2,2,2-trichloroethyl | H | Me | S | H | H | Cl | H | nonafluoro-2-butyl | H | CF3 |
| 50-42 | 2,2,2-trichloroethyl | H | Me | S | H | H | CF3 | H | nonafluoro-2-butyl | H | CF3 |
| 50-43 | 2,2,2-trichloroethyl | H | Me | O | H | H | Br | H | heptafluoroisopropyl | H | OCF3 |
| 50-44 | 2,2,2-trichloroethyl | H | Me | O | H | H | I | H | heptafluoroisopropyl | H | CF3 |
| 50-45 | 2,2,2-trichloroethyl | H | Me | O | H | H | CF3 | H | heptafluoroisopropyl | H | CF3 |
| 50-46 | 2,2,2-trichloroethyl | H | Me | O | H | H | Cl | H | nonafluoro-2-butyl | H | OCF3 |
| 50-47 | 2,2,2-trichloroethyl | H | Me | O | H | H | Br | H | nonafluoro-2-butyl | H | CF3 |
| 50-48 | 2,2,2-trichloroethyl | H | Me | O | H | H | CF3 | H | nonafluoro-2-butyl | H | CF3 |
| 50-49 | 2,2,2-trichloroethyl | H | Me | NH | H | H | I | H | heptafluoroisopropyl | H | OCF3 |
| 50-50 | 2,2,2-trichloroethyl | H | Me | NH | H | H | Cl | H | heptafluoroisopropyl | H | CF3 |
| 50-51 | 2,2,2-trichloroethyl | H | Me | NH | H | H | CF3 | H | heptafluoroisopropyl | H | CF3 |

**Table 50(2)**

| compound number | Q₂ | R₁ | R₂ | A | X₁ | X₃ | Y₁ | Y₂ | Y₃ | Y₄ | Y₅ |
|---|---|---|---|---|---|---|---|---|---|---|---|
| 50-52 | 2,2,2-trichloroethyl | H | Me | NH | H | H | Br | H | nonafluoro-2-butyl | H | OCF3 |
| 50-53 | 2,2,2-trichloroethyl | H | Me | NH | H | H | I | H | nonafluoro-2-butyl | H | CF3 |
| 50-54 | 2,2,2-trichloroethyl | H | Me | NH | H | H | CF3 | H | nonafluoro-2-butyl | H | CF3 |
| 50-55 | 2,2,2-trichloroethyl | Me | Me | S | H | H | Cl | H | heptafluoroisopropyl | H | OCF3 |
| 50-56 | 2,2,2-trichloroethyl | Me | Me | S | H | H | Br | H | heptafluoroisopropyl | H | CF3 |
| 50-57 | 2,2,2-trichloroethyl | Me | Me | S | H | H | CF3 | H | heptafluoroisopropyl | H | CF3 |
| 50-58 | 2,2,2-trichloroethyl | Me | Me | S | H | H | I | H | nonafluoro-2-butyl | H | OCF3 |
| 50-59 | 2,2,2-trichloroethyl | Me | Me | S | H | H | Cl | H | nonafluoro-2-butyl | H | CF3 |
| 50-60 | 2,2,2-trichloroethyl | Me | Me | S | H | H | CF3 | H | nonafluoro-2-butyl | H | CF3 |
| 50-61 | 2,2,2-trichloroethyl | Me | Me | O | H | H | Br | H | heptafluoroisopropyl | H | OCF3 |
| 50-62 | 2,2,2-trichloroethyl | Me | Me | O | H | H | I | H | heptafluoroisopropyl | H | CF3 |
| 50-63 | 2,2,2-trichloroethyl | Me | Me | O | H | H | CF3 | H | heptafluoroisopropyl | H | CF3 |
| 50-64 | 2,2,2-trichloroethyl | Me | Me | O | H | H | Cl | H | nonafluoro-2-butyl | H | OCF3 |
| 50-65 | 2,2,2-trichloroethyl | Me | Me | O | H | H | Br | H | nonafluoro-2-butyl | H | CF3 |
| 50-66 | 2,2,2-trichloroethyl | Me | Me | O | H | H | CF3 | H | nonafluoro-2-butyl | H | CF3 |
| 50-67 | 2,2,2-trichloroethyl | Me | Me | NH | H | H | I | H | heptafluoroisopropyl | H | OCF3 |
| 50-68 | 2,2,2-trichloroethyl | Me | Me | NH | H | H | Cl | H | heptafluoroisopropyl | H | CF3 |
| 50-69 | 2,2,2-trichloroethyl | Me | Me | NH | H | H | CF3 | H | heptafluoroisopropyl | H | CF3 |
| 50-70 | 2,2,2-trichloroethyl | Me | Me | NH | H | H | Br | H | nonafluoro-2-butyl | H | OCF3 |
| 50-71 | 2,2,2-trichloroethyl | Me | Me | NH | H | H | I | H | nonafluoro-2-butyl | H | CF3 |
| 50-72 | 2,2,2-trichloroethyl | Me | Me | NH | H | H | CF3 | H | nonafluoro-2-butyl | H | CF3 |
| 50-73 | 2,2,2-trichloroethyl | Me | H | S | H | H | Cl | F | heptafluoroisopropyl | H | OCF3 |
| 50-74 | 2,2,2-trichloroethyl | H | H | S | H | H | Br | H | heptafluoroisopropyl | F | CF3 |
| 50-75 | 2,2,2-trichloroethyl | H | H | S | H | H | CF3 | Cl | heptafluoroisopropyl | H | CF3 |
| 50-76 | 2,2,2-trichloroethyl | H | H | S | H | H | I | H | nonafluoro-2-butyl | Cl | OCF3 |
| 50-77 | 2,2,2-trichloroethyl | H | H | S | H | H | Cl | Br | nonafluoro-2-butyl | H | CF3 |
| 50-78 | 2,2,2-trichloroethyl | H | H | S | H | H | CF3 | H | nonafluoro-2-butyl | Br | CF3 |
| 50-79 | 2,2,2-trichloroethyl | H | H | O | H | H | Br | I | heptafluoroisopropyl | H | OCF3 |
| 50-80 | 2,2,2-trichloroethyl | Me | H | O | H | H | I | H | heptafluoroisopropyl | I | CF3 |
| 50-81 | 2,2,2-trichloroethyl | H | H | O | H | H | CF3 | Me | heptafluoroisopropyl | H | CF3 |
| 50-82 | 2,2,2-trichloroethyl | H | H | O | H | H | Cl | H | nonafluoro-2-butyl | Me | OCF3 |
| 50-83 | 2,2,2-trichloroethyl | H | H | O | H | H | Br | F | nonafluoro-2-butyl | F | CF3 |
| 50-84 | 2,2,2-trichloroethyl | H | H | O | H | H | CF3 | Cl | nonafluoro-2-butyl | Cl | CF3 |
| 50-85 | 2,2,2-trichloroethyl | H | H | NH | H | H | I | Br | heptafluoroisopropyl | Br | OCF3 |
| 50-86 | 2,2,2-trichloroethyl | H | H | NH | H | H | Cl | I | heptafluoroisopropyl | I | CF3 |
| 50-87 | 2,2,2-trichloroethyl | Me | H | NH | H | H | CF3 | F | heptafluoroisopropyl | Me | CF3 |
| 50-88 | 2,2,2-trichloroethyl | H | H | NH | H | H | Br | Cl | nonafluoro-2-butyl | Me | OCF3 |
| 50-89 | 2,2,2-trichloroethyl | H | H | NH | H | H | I | Br | nonafluoro-2-butyl | Me | CF3 |
| 50-90 | 2,2,2-trichloroethyl | H | H | NH | H | H | CF3 | I | nonafluoro-2-butyl | Me | CF3 |

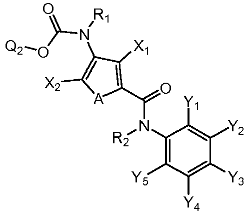

**Table 51(1)**

| compound number | Q₂ | R₁ | R₂ | A | X₁ | X₂ | Y₁ | Y₂ | Y₃ | Y₄ | Y₅ |
|---|---|---|---|---|---|---|---|---|---|---|---|
| 51-1 | 2,2,2-trichloroethyl | H | H | S | H | H | Cl | H | heptafluoroisopropyl | H | OCF3 |
| 51-2 | 2,2,2-trichloroethyl | H | H | S | H | H | Br | H | heptafluoroisopropyl | H | CF3 |
| 51-3 | 2,2,2-trichloroethyl | H | H | S | H | H | CF3 | H | heptafluoroisopropyl | H | CF3 |
| 51-4 | 2,2,2-trichloroethyl | H | H | S | H | H | I | H | nonafluoro-2-butyl | H | OCF3 |
| 51-5 | 2,2,2-trichloroethyl | H | H | S | H | H | Cl | H | nonafluoro-2-butyl | H | CF3 |
| 51-6 | 2,2,2-trichloroethyl | H | H | S | H | H | CF3 | H | nonafluoro-2-butyl | H | CF3 |
| 51-7 | 2,2,2-trichloroethyl | H | H | O | H | H | Br | H | heptafluoroisopropyl | H | OCF3 |
| 51-8 | 2,2,2-trichloroethyl | H | H | O | H | H | I | H | heptafluoroisopropyl | H | CF3 |
| 51-9 | 2,2,2-trichloroethyl | H | H | O | H | H | CF3 | H | heptafluoroisopropyl | H | CF3 |
| 51-10 | 2,2,2-trichloroethyl | H | H | O | H | H | Cl | H | nonafluoro-2-butyl | H | OCF3 |
| 51-11 | 2,2,2-trichloroethyl | H | H | O | H | H | Br | H | nonafluoro-2-butyl | H | CF3 |
| 51-12 | 2,2,2-trichloroethyl | H | H | O | H | H | CF3 | H | nonafluoro-2-butyl | H | CF3 |
| 51-13 | 2,2,2-trichloroethyl | H | H | NH | H | H | I | H | heptafluoroisopropyl | H | OCF3 |
| 51-14 | 2,2,2-trichloroethyl | H | H | NH | H | H | Cl | H | heptafluoroisopropyl | H | CF3 |
| 51-15 | 2,2,2-trichloroethyl | H | H | NH | H | H | CF3 | H | heptafluoroisopropyl | H | CF3 |
| 51-16 | 2,2,2-trichloroethyl | H | H | NH | H | H | Br | H | nonafluoro-2-butyl | H | OCF3 |
| 51-17 | 2,2,2-trichloroethyl | H | H | NH | H | H | I | H | nonafluoro-2-butyl | H | CF3 |
| 51-18 | 2,2,2-trichloroethyl | H | H | NH | H | H | CF3 | H | nonafluoro-2-butyl | H | CF3 |
| 51-19 | 2,2,2-trichloroethyl | Me | H | S | H | H | Cl | H | heptafluoroisopropyl | H | OCF3 |
| 51-20 | 2,2,2-trichloroethyl | Me | H | S | H | H | Br | H | heptafluoroisopropyl | H | CF3 |
| 51-21 | 2,2,2-trichloroethyl | Me | H | S | H | H | CF3 | H | heptafluoroisopropyl | H | CF3 |
| 51-22 | 2,2,2-trichloroethyl | Me | H | S | H | H | I | H | nonafluoro-2-butyl | H | OCF3 |
| 51-23 | 2,2,2-trichloroethyl | Me | H | S | H | H | Cl | H | nonafluoro-2-butyl | H | CF3 |
| 51-24 | 2,2,2-trichloroethyl | Me | H | S | H | H | CF3 | H | nonafluoro-2-butyl | H | CF3 |
| 51-25 | 2,2,2-trichloroethyl | Me | H | O | H | H | Br | H | heptafluoroisopropyl | H | OCF3 |
| 51-26 | 2,2,2-trichloroethyl | Me | H | O | H | H | I | H | heptafluoroisopropyl | H | CF3 |
| 51-27 | 2,2,2-trichloroethyl | Me | H | O | H | H | CF3 | H | heptafluoroisopropyl | H | CF3 |
| 51-28 | 2,2,2-trichloroethyl | Me | H | O | H | H | Cl | H | nonafluoro-2-butyl | H | OCF3 |
| 51-29 | 2,2,2-trichloroethyl | Me | H | O | H | H | Br | H | nonafluoro-2-butyl | H | CF3 |
| 51-30 | 2,2,2-trichloroethyl | Me | H | O | H | H | CF3 | H | nonafluoro-2-butyl | H | CF3 |
| 51-31 | 2,2,2-trichloroethyl | Me | H | NH | H | H | I | H | heptafluoroisopropyl | H | OCF3 |
| 51-32 | 2,2,2-trichloroethyl | Me | H | NH | H | H | Cl | H | heptafluoroisopropyl | H | CF3 |
| 51-33 | 2,2,2-trichloroethyl | Me | H | NH | H | H | CF3 | H | heptafluoroisopropyl | H | CF3 |
| 51-34 | 2,2,2-trichloroethyl | Me | H | NH | H | H | Br | H | nonafluoro-2-butyl | H | OCF3 |
| 51-35 | 2,2,2-trichloroethyl | Me | H | NH | H | H | I | H | nonafluoro-2-butyl | H | CF3 |
| 51-36 | 2,2,2-trichloroethyl | Me | H | NH | H | H | CF3 | H | nonafluoro-2-butyl | H | CF3 |
| 51-37 | 2,2,2-trichloroethyl | H | Me | S | H | H | Cl | H | heptafluoroisopropyl | H | OCF3 |
| 51-38 | 2,2,2-trichloroethyl | H | Me | S | H | H | Br | H | heptafluoroisopropyl | H | CF3 |
| 51-39 | 2,2,2-trichloroethyl | H | Me | S | H | H | CF3 | H | heptafluoroisopropyl | H | CF3 |
| 51-40 | 2,2,2-trichloroethyl | H | Me | S | H | H | I | H | nonafluoro-2-butyl | H | OCF3 |
| 51-41 | 2,2,2-trichloroethyl | H | Me | S | H | H | Cl | H | nonafluoro-2-butyl | H | CF3 |
| 51-42 | 2,2,2-trichloroethyl | H | Me | S | H | H | CF3 | H | nonafluoro-2-butyl | H | CF3 |
| 51-43 | 2,2,2-trichloroethyl | H | Me | O | H | H | Br | H | heptafluoroisopropyl | H | OCF3 |
| 51-44 | 2,2,2-trichloroethyl | H | Me | O | H | H | I | H | heptafluoroisopropyl | H | CF3 |
| 51-45 | 2,2,2-trichloroethyl | H | Me | O | H | H | CF3 | H | heptafluoroisopropyl | H | CF3 |
| 51-46 | 2,2,2-trichloroethyl | H | Me | O | H | H | Cl | H | nonafluoro-2-butyl | H | OCF3 |
| 51-47 | 2,2,2-trichloroethyl | H | Me | O | H | H | Br | H | nonafluoro-2-butyl | H | CF3 |
| 51-48 | 2,2,2-trichloroethyl | H | Me | O | H | H | CF3 | H | nonafluoro-2-butyl | H | CF3 |
| 51-49 | 2,2,2-trichloroethyl | H | Me | NH | H | H | I | H | heptafluoroisopropyl | H | OCF3 |
| 51-50 | 2,2,2-trichloroethyl | H | Me | NH | H | H | Cl | H | heptafluoroisopropyl | H | CF3 |
| 51-51 | 2,2,2-trichloroethyl | H | Me | NH | H | H | CF3 | H | heptafluoroisopropyl | H | CF3 |

**Table 51(2)**

| compound number | Q₂ | R₁ | R₂ | A | X₁ | X₂ | Y₁ | Y₂ | Y₃ | Y₄ | Y₅ |
|---|---|---|---|---|---|---|---|---|---|---|---|
| 51-52 | 2,2,2-trichloroethyl | H | Me | NH | H | H | Br | H | nonafluoro-2-butyl | H | OCF3 |
| 51-53 | 2,2,2-trichloroethyl | H | Me | NH | H | H | I | H | nonafluoro-2-butyl | H | CF3 |
| 51-54 | 2,2,2-trichloroethyl | H | Me | NH | H | H | CF3 | H | nonafluoro-2-butyl | H | CF3 |
| 51-55 | 2,2,2-trichloroethyl | Me | Me | S | H | H | Cl | H | heptafluoroisopropyl | H | OCF3 |
| 51-56 | 2,2,2-trichloroethyl | Me | Me | S | H | H | Br | H | heptafluoroisopropyl | H | CF3 |
| 51-57 | 2,2,2-trichloroethyl | Me | Me | S | H | H | CF3 | H | heptafluoroisopropyl | H | CF3 |
| 51-58 | 2,2,2-trichloroethyl | Me | Me | S | H | H | I | H | nonafluoro-2-butyl | H | OCF3 |
| 51-59 | 2,2,2-trichloroethyl | Me | Me | S | H | H | Cl | H | nonafluoro-2-butyl | H | CF3 |
| 51-60 | 2,2,2-trichloroethyl | Me | Me | S | H | H | CF3 | H | nonafluoro-2-butyl | H | CF3 |
| 51-61 | 2,2,2-trichloroethyl | Me | Me | O | H | H | Br | H | heptafluoroisopropyl | H | OCF3 |
| 51-62 | 2,2,2-trichloroethyl | Me | Me | O | H | H | I | H | heptafluoroisopropyl | H | CF3 |
| 51-63 | 2,2,2-trichloroethyl | Me | Me | O | H | H | CF3 | H | heptafluoroisopropyl | H | CF3 |
| 51-64 | 2,2,2-trichloroethyl | Me | Me | O | H | H | Cl | H | nonafluoro-2-butyl | H | OCF3 |
| 51-65 | 2,2,2-trichloroethyl | Me | Me | O | H | H | Br | H | nonafluoro-2-butyl | H | CF3 |
| 51-66 | 2,2,2-trichloroethyl | Me | Me | O | H | H | CF3 | H | nonafluoro-2-butyl | H | CF3 |
| 51-67 | 2,2,2-trichloroethyl | Me | Me | NH | H | H | I | H | heptafluoroisopropyl | H | OCF3 |
| 51-68 | 2,2,2-trichloroethyl | Me | Me | NH | H | H | Cl | H | heptafluoroisopropyl | H | CF3 |
| 51-69 | 2,2,2-trichloroethyl | Me | Me | NH | H | H | CF3 | H | heptafluoroisopropyl | H | CF3 |
| 51-70 | 2,2,2-trichloroethyl | Me | Me | NH | H | H | Br | H | nonafluoro-2-butyl | H | OCF3 |
| 51-71 | 2,2,2-trichloroethyl | Me | Me | NH | H | H | I | H | nonafluoro-2-butyl | H | CF3 |
| 51-72 | 2,2,2-trichloroethyl | Me | Me | NH | H | H | CF3 | H | nonafluoro-2-butyl | H | CF3 |
| 51-73 | 2,2,2-trichloroethyl | Me | H | S | H | H | Cl | F | heptafluoroisopropyl | H | OCF3 |
| 51-74 | 2,2,2-trichloroethyl | H | H | S | H | H | Br | H | heptafluoroisopropyl | F | CF3 |
| 51-75 | 2,2,2-trichloroethyl | H | H | S | H | H | CF3 | Cl | heptafluoroisopropyl | H | CF3 |
| 51-76 | 2,2,2-trichloroethyl | H | H | S | H | H | I | H | nonafluoro-2-butyl | Cl | OCF3 |
| 51-77 | 2,2,2-trichloroethyl | H | H | S | H | H | Cl | Br | nonafluoro-2-butyl | H | CF3 |
| 51-78 | 2,2,2-trichloroethyl | H | H | S | H | H | CF3 | H | nonafluoro-2-butyl | Br | CF3 |
| 51-79 | 2,2,2-trichloroethyl | H | H | O | H | H | Br | I | heptafluoroisopropyl | H | OCF3 |
| 51-80 | 2,2,2-trichloroethyl | Me | H | O | H | H | I | H | heptafluoroisopropyl | I | CF3 |
| 51-81 | 2,2,2-trichloroethyl | H | H | O | H | H | CF3 | Me | heptafluoroisopropyl | H | CF3 |
| 51-82 | 2,2,2-trichloroethyl | H | H | O | H | H | Cl | H | nonafluoro-2-butyl | Me | OCF3 |
| 51-83 | 2,2,2-trichloroethyl | H | H | O | H | H | Br | F | nonafluoro-2-butyl | F | CF3 |
| 51-84 | 2,2,2-trichloroethyl | H | H | O | H | H | CF3 | Cl | nonafluoro-2-butyl | Cl | CF3 |
| 51-85 | 2,2,2-trichloroethyl | H | H | NH | H | H | I | Br | heptafluoroisopropyl | Br | OCF3 |
| 51-86 | 2,2,2-trichloroethyl | H | H | NH | H | H | Cl | I | heptafluoroisopropyl | I | CF3 |
| 51-87 | 2,2,2-trichloroethyl | Me | H | NH | H | H | CF3 | F | heptafluoroisopropyl | Me | CF3 |
| 51-88 | 2,2,2-trichloroethyl | H | H | NH | H | H | Br | Cl | nonafluoro-2-butyl | Me | OCF3 |
| 51-89 | 2,2,2-trichloroethyl | H | H | NH | H | H | I | Br | nonafluoro-2-butyl | Me | CF3 |
| 51-90 | 2,2,2-trichloroethyl | H | H | NH | H | H | CF3 | I | nonafluoro-2-butyl | Me | CF3 |

Hereinbelow, Table 52 shows the physical properties of the representative compounds of the amide derivative of the present invention. The ¹H-NMR chemical shift values shown therein are based on tetramethylsilane as an internal standard substance unless specified otherwise.

**Table 52(1)**

| compound number | ¹H-NMR (CDCl₃,ppm) |
|---|---|
| 1-470 | *δ* 7.55-7.68 (3H, m), 7.80-7.99 (6H, m), 8.58 (1H, s), 8.61 (1H, s), 9.12 (1H, d, J =1.5Hz). |
| 1-491 | *δ* 7.34-7.91 (4H, m), 8.00 (1H, s), 8.08 (2H, dd, J =1.9,6.8Hz), 8.00(1 H, s), 8.49 (1H, s), 8.59 (1H, s), 9.04 (1H, s). |
| 1-506 | *δ* 7.48 (1H, dd, J =4.9,7.81 Hz), 7.83-7.90 (3H, m), 8.00 (1H, s), 8.31 (1H, dd, J =1.9,7.8Hz), 8.34 (1H, s), 8.61 (1H, dd, J =1.9,4.9Hz), 9.09 (1H, s), 9.10 (1H, s). |
| 1-513 | *δ* 7.54 (1H, d, J =7.8Hz), 7.83-7.90 (3H, m), 8.00 (1H, s), 8.21 (1H, dd, J =2.4,8.3Hz), 8.31 (1H, s), 8.51 (1H, s), 9.00 (1H, s), 9.01 (1H, d, J =7.8Hz). |
| 1-627 | *δ* 7.56-7.59(3H, m), 7.64-7.66(1 H, m), 7.80-7.87(2H, m), 7.94-7.97(2H, m), 8.16(1 H, s), 8.46(1 H, s), 8.57(1 H, s), 9.16(1 H, s). |
| 1-1925 | *δ* 7.52-7.59(2H, m), 7.65-7.71(2H, m), 7.93-7.95(2H, m), 8.00-8.02(2H, m), 8.12-8.16(2H, m), 8.52(1 H. d, J =11.2Hz). |
| 1-1968 | *δ* 7.54(1 H, d J =8.8Hz), 7.72(1 H, d J =8.8Hz), 7.93(1 H, s), 8.09(1 H, s), 8.17-8.18(2H, m), 8.27(1 H, dd, J= 2.4,8.8Hz), 8.38(1 H, d J =10.8Hz), 8.98(1 H, d, J =2.4Hz). |
| 2-491 | *δ* 3.81 (3H, broad-s), 7.52-7.84 (8H, m), 7.89 (1H, s), 8.00 (1H, s). |
| 2-513 | *δ* 3.58 (3H, s), 7.34-7.36 (1H, m), 7.81-8.00 (8H, m). |
| 2-518 | *δ* 3.59 (3H, s), 7.69 (1H, broad-s), 7.81-8.01 (7H, m), 8.50 (1H, broad-s). |
| 6-38 | *δ* 7.51-7.62 (4H, m), 7.75-8.00 (8H, m), 8.32(1H, s). |
| 6-39 | *δ* 7.22 (1H, dd, J =8.0, 12.4Hz), 7.32-7.36 (1H, m), 7.51-7.55 (2H, m), 7.78 (1H, d, J =7.6Hz), 7.86-7.90 (3H, m), 8.11(1H, s), 8.17-8.18 (1H, m), 8.35 (1H, s), 8.62-8.67(1H, m). |
| 6-45 | *δ* 7.57-7.59 (1H, m), 7.65-7.69 (1H, m), 7.77-7.79 (1H, m), 7.89-7.9 (5H, m), 8.14-8.29 (4H, m). |
| 6-46 | *δ* 7.55-7.59 (1H, m), 7.77-8.07 (10H, m), 8.27 (1H, s). |
| 6-47 | *δ* 7.01-7.05 (2H, m), 7.44-7.56 (2H, m), 7.78-7.86 (4H, m), 8.03 (1H, s), 8.11 (1H, s), 8.33 (1H, s). |
| 6-57 | *δ* 7.28-7.32 (1H, m), 7.45-7.67 (4H, m), 7.76-8.02 (5H, m), 8.30(1 H, s). |
| 6-60 | *δ* 7.50-7.60 (2H, m), 7.78-8.02 (6H, m), 8.19-8.27 (2H, m), 8.91(1 H, s). |
| 6-71 | *δ* 7.51-7.62(4H, m), 7.78-7.80(1 H, d, J = 8.0Hz), 7.90-7.92(3H, m), 8.06-8.09(4H, m), 8.34(1 H, s). |
| 6-72 | *δ* 7.22-7.25(1 H, m), 7.35(1 H, t, J =8.0Hz), 7.55-7.60(2H, m), 7.80(1 H, d, J =8.0Hz), 7.92(1 H, d, J =8.0Hz), 8.01(1 H, s), 8.10(2H, s), 8.20(1 H, t, J =8.0Hz), 8.37(1 H, s), 8.65(1 H, d, J =16.8Hz). |

**Table 52(2)**

| compound number | ¹H-NMR (CDCl₃,ppm) |
|---|---|
| 6-78 | *δ* 7.58(1 H, t, J =7.8Hz), 7.68(1 H, t, J =7.8), 7.80(1 H, d, J= 8.0Hz), 7.87(1 H, d, 8.0Hz), 7.95(1 H, d, J= 8.0Hz), 8.00(1 H, s), 8.09(2H, s), 8.15(1 H, d, J= 8.0Hz), 8.23(1 H, s), 8.26(1 H, s), 8.30(1 H, s). |
| 6-79 | *δ* 7.58(1 H, t, J =8.2Hz), 7.79-7.83(3H, m), 7.97(2H, s), 8.01(2H, d, J =8.0Hz), 8.09(2H, s), 8.18(1 H, s), 8.29(1 H, s). |
| 6-80 | *δ* 7.05(2H, t, J =8.2Hz), 7.48(1 H, t, J =6.6Hz), 7.56(1 H, t, J =7.8Hz), 7.81(1 H, d, J =8.0Hz), 7.89-7.91(2H, m), 7.98(1 H, s), 8.10(2H, s), 8.33(1 H, s). |
| 6-90 | *δ* 7.43-7.46(1 H, m), 7.59(1 H, t, J = 7.2Hz), 7.82(1 H, d, J = 7.2Hz), 7.93(2H, d, J = 9.6Hz), 8.10(2H, s), 8.24(1 H, d, J = 6.4Hz), 8.33(1 H, s), 8.44(1 H, s), 8.56(1 H,s). |
| 6-93 | *δ* 7.51(1 H, d, J =8.4Hz), 7.59(1 H, t, J =7.6Hz), 7.81(1H, d, J =7.6Hz), 7.94(2H, s), 8.10-8.11(3H, m), 8.20(1 H, d, J =8.0Hz), 8.28(1 H, s), 8.91(1 H, s). |
| 6-111 | (DMSO-d₆) *δ* 7.50-7.60(4H, m), 7.70-7.73(1 H, m), 7.93-8.00(3H, m), 8.04-8.08(1 H, m), 8.34-8.36(2H, m), 10.48(1 H, s), 10.60(1 H, s). |
| 6-147 | (DMSO-d₆) *δ* 7.55-7.61(2H, m), 7.74(1 H, d, J = 7.9Hz), 7.94-7.97(2H, m), 8.10(1 H, dd, J = 2.0,7.6Hz), 8.27(1 H, s), 8.34(1 H, s), 8.54(1 H, dd, J = 2.0,4.9Hz), 10.65(1 H, s), 10.89(1 H, s). |
| 6-268 | *δ* 7.50-7.61 (4H, m), 7.70 (1H, d, J =7.8Hz), 7.85-7.92 (4H, m), 7.96-8.01 (2H, m), 8.14 (1H, s), 8.24 (1H, t, J =2.0Hz). |
| 6-269 | *δ* 7.22-7.25(1 H, m), 7.36(1 H, t, J =8.0Hz), 7.56-7.57(2H, m), 7.75(1 H, d, J =8.0Hz), 7.90-8.00(2H, m), 8.07(1 H, s), 8.17-8.20(2H, m), 8.33(1 H, s), 8.65(1 H, d, J =16.4Hz). |
| 6-270 | *δ* 7.25(1 H, t, J =8.0Hz), 7.43-7.53(2H, m), 7.77-7.81 (4H, m), 7.85(1 H, d, J =8.0Hz), 7.91(1 H, s), 8.13(1 H, s), 8.19(1 H, d, J =8.0Hz), 8.24(1 H, s). |
| 6-271 | *δ* 7.09-7.22(2H, m), 7.55(1 H, t, J =7.8Hz), 7.71(1 H, d, J =8.0Hz), 7.89-7.93(4H, m), 8.03(2H, d, J =8.0Hz), 8.14(1 H, s), 8.23(1 H, s). |
| 6-272 | *δ* 7.40-7.50(4H, m), 7.56(1 H, t, J =8.0Hz), 7.80(2H, t, J =8.4Hz), 7.87(2H, s), 7.91(1 H, d, J =7.9Hz), 8.09(1 H, s), 8.32(1 H, s). |
| 6-288 | *δ* 7.54-7.55 (1H, m), 7.65-7.66 (1H, m), 7.72 (1H, d, J =7.8Hz), 7.85 (1H, d, J =7.8Hz), 7.91 (1H, s), 7.95-7.98 (2H, m), 8.13-8.23 (4H, m), 8.36 (1H, s). |
| 6-289 | *δ* 7.55-7.56 (1H, m), 7.71 (1H, d, J =7.8Hz), 7.80 (2H, d, J =8.3Hz), 7.91-7.96 (3H, m), 7.99 (2H, d, J =8.3Hz), 8.20 (1H, s), 8.21-8.22 (2H, m). |
| 6-290 | *δ* 7.01-7.05(2H, m), 7.42-7.44(1 H, m), 7.55(1 H, t, J =8.0Hz). 7.75(1 H, d, J =8.0Hz). 7.90-7.95(3H, m), 8.01(1 H, s), 8.14(1 H, s), 8.26(1 H, s). |
| 6-293 | *δ* 7.14(1 H, t, J =8.0Hz), 7.22-7.29(2H, m), 7.54(1 H, t, J =7.8Hz), 7.74(1 H, d, J =7.8Hz), 7.84-7.88(3H, m), 8.11(1 H, s), 8.15(1 H, s), 8.25(1 H, s). |
| 6-300 | *δ* 7.06-7.59 (2H, m), 7.62 (1H, dd, J =1.5, 7.8Hz), 7.81 (1H, d, J =7.8Hz), 7.92 (1H, s), 8.08-8.16 (4H, m), 9.35-9.40 (1H, m), 9.51 (1H, broad-s). |
| 6-304 | *δ* 7.44 (1H, dd, J =4.9,7.8Hz), 7.58 (1H, t, J =7.8Hz), 7.75 (1H, d, J =7.8Hz), 7.81 (1H, s), 7.92-7.96 (2H, m), 8.15 (1H, s), 8.22-8.26 (2H, m), 8.38 (1H, s), 8.55 (1H, dd, J =2.0,4.9Hz). |
| 6-306 | *δ* 7.42-7.45(1 H, m), 7.58(1 H, t, J = 7.8Hz), 7.75(1 H, d, J = 7.8Hz), 7.88(1 H, broad-s), 7.93-7.95(2H, m), 8.15(1 H, broad-s), 8.23(1 H, dd, J =2.0, 7.8Hz), 8.24(1 H, s), 8.39(1 H, broad-s), 8.55(1 H, dd, J =2.0, 4.8Hz). |

**Table 52(3)**

| compound number | ¹H-NMR (CDCl₃,ppm) |
|---|---|
| 6-311 | *δ* 7.45-7.52(1 H, m), 7.74(1 H, d, J =8.0Hz), 7.92-7.96(1 H, m), 8.00(1 H, s), 8.14(1 H, s), 8.22-8.23(3H, m), 8.29(1 H, d, J =8.0Hz), 8.91(1 H, s), 9.07(1 H, s). |
| 6-346 | *δ* 7.50-7.62(4H, m), 7.71-7.73(1 H, m), 7.89-7.93(4H, m), 8.02-8.04(2H, m), 8.13(1 H, s), 8.27(1 H, s). |
| 6-347 | *δ* 7.19-7.25 (1H, m), 7.33-7.37 (1H, m), 7.52-7.59 (2H, m), 7.73-7.75 (1H, m), 7.89-7.91 (2H, m), 8.09-8.21 (3H, m), 8.31 (1H, s), 8.65 (1H, s). |
| 6-348 | *δ* 7.30-7.31 (1H, m), 7.48-7.74 (5H, m), 7.91-7.92 (2H, m), 8.02 (1H, s), 8.07 (1H, s), 8.13 (1H, s), 8.25 (1H, s). |
| 6-349 | *δ* 7.17-7.21 (2H, m), 7.51-7.55 (1H, m), 7.70-7.72 (1H, m), 7.88-7.93 (4H, m), 8.04 (1H, s), 8.11-8.23 (2H, m), 8.23 (1H, s). |
| 6-366 | *δ* 7.53-7.55 (1H, m), 7.62-7.72 (2H, m), 7.84-7.94 (3H, m), 8.09-8.16 (3H, m), 8.22-8.24 (2H, m), 8.40 (1H, s). |
| 6-367 | *δ* 7.54-7.58 (1H, m), 7.72-7.74 (1H, m), 7.80-7.83 (2H, m), 7.91-7.94 (2H, m), 8.00-8.07 (2H, m), 8.13 (1H, s), 8.18 (1H, s), 8.23 (1H, s), 8.24 (1H, s). |
| 6-368 | *δ* 7.01-7.06 (2H, m), 7.44-7.57 (3H, m), 7.74-7.76 (1H, m), 7.86-7.91 (2H, m), 8.07 (1H, s), 8.13-8.14 (1H, m), 8.28 (1 H, s). |
| 6-382 | *δ* 7.43-7.46 (1H, m), 7.56-7.60 (1H, m), 7.76-7.78 (1H, m), 7.90-7.91 (2H, m), 8.02 (1H, s), 8.14 (1H, s), 8.25 (2H, s), 8.43 (1H, s), 8.54-8.56 (1H, m). |
| 6-389 | *δ* 7.30-7.60 (2H, m), 7.74-7.76 (1H, m), 7.91-7.98 (3H, m), 8.07 (1H, s), 8.14 (1H, s), 8.19-8.23 (2H, m), 8.90 (1H, s). |
| 6-424 | *δ* 7.51-7.62 (4H, m), 7.73 (1H, d, J =8.3Hz), 7.89-7.94 (4H, m), 8.01 (1H, s), 8.10 (1H, s), 8.27 (1H, t, J =1.5Hz), 8.35 (1H, s). |
| 6-425 | *δ* 7.20-7.25 (1H, m), 7.34-7.37 (1H, m), 7.54-7.58 (2H, m), 7.75 ( 1H, d, J =7.6Hz), 7.88-7.95 (2H, m), 8.18-8.22 (2H, m), 8.33-8.34 (2H, m), 8.66 (1H, d, J =16.4Hz). |
| 6-426 | *δ* 7.28-7.30 (1H, m), 7.46-7.66 (5H, m), 7.71-7.94 (2H, m), 8.11-8.24 (3H, m), 8.34 (1H, s). |
| 6-427 | *δ* 7.18-7.22 (2H, m), 7.52-7.57 (1H, m), 7.73 ( 1H, d, J =7.6Hz), 7.89-8.03 (5H, m), 8.22-8.25 (2H, m), 8.35 (1H, s). |
| 6-428 | *δ* 7.42-7.50(4H, m), 7.56(1 H, t, J =8.0Hz). 7.75(1 H, d, J =8.0Hz). 7.81(1 H, d, J =7.8Hz), 7.95-7.96(2H, m), 8.09(1 H, s), 8.28(1 H, s), 8.36(1 H, s). |
| 6-444 | *δ* 7.56 (1H, t, J =7.8Hz), 7.66 (1H, t, J =7.8Hz), 7.73 (1H, t. J =7.8Hz), 7.86 (1H, dd, J =1.5, 7.8Hz), 7.93-7.96 (2H, m), 8.07 (1H, s), 8.14-8.16 (1H, m), 8.22-8.26 (3H, m), 8.34 (1H, s). |
| 6-445 | *δ* 7.57 (1H, t, J =7.8Hz), 7.74 (1H, d, J =7.8Hz), 7.81-7.83 (2H, m), 7.95 (1H, s), 7.97-8.04 (4H, m), 8.12 (1H, broad-s), 8.23 (1H, t, J =2.0Hz), 8.35 (1H, broad-s). |
| 6-446 | *δ* 7.01-7.05 (2H, m), 7.43-7.57 (2H, m), 7.75-7.77 (1H, m), 7.88-7.94 (3H, m), 8.13 (1H, s), 8.27 (1H, s), 8.35 (1H, s). |
| 6-448 | *δ* 7.39-7.41 (1H, m), 7.51-7.58 (2H, m), 7.74-7.78 (2H, m), 7.90-7.95 (2H, m), 8.04 (1H, s), 8.15 (1H, s), 8.26 (1H, s), 8.35 (1H, s). |

**Table 52(4)**

| compound number | ¹H-NMR (CDCl₃,ppm) |
|---|---|
| 6-449 | *δ* 7.12-7.36 (3H, m), 7.52-7.58 (1H, m), 7.75-7.95 (4H, m), 8.21-8.35 (3H, m). |
| 6-450 | 6.82-6.86(1 H, m), 7.00-7.01(1 H, m), 7.15(1 H, t, J =7.8Hz), 7.56(1 H, t, J =8.2Hz), 7.74(1 H, d, J =8.1 Hz), 7.84(1 H, t, J =8.8Hz), 7.90-7.91 (1H, m), 7.95(1 H, s), 8.04(1 H, s), 8.42(1 H, s). |
| 6-451 | *δ* 7.23-7.69 (8H, m), 7.85 (1H, s), 8.04(1 H, s), 8.42 (1H, s). |
| 6-452 | *δ* 7.44 (1H, dd, J =2.0, 8.3 Hz), 7.59 (1H, t, J =8.3 Hz), 7.66 (1H, d, J =8.3 Hz), 7.70 (1H, d, J =2.0 Hz), 7.75 (1H, d, J =8.3 Hz), 7.86 (2H, broad-s), 7.95 (2H, broad-s), 8.24 (1H, broad-s), 8.37 (1H, broad-s). |
| 6-453 | *δ* 7.15 (1H, dt, J=2.4, 8.3 Hz), 7.40 (1H, dd, J=2.4, 8.3 Hz), 7.60 (1H, t, J =8.3 Hz), 7.68-7.74 (2H, m), 7.95-8.02 (4H, m), 8.24 (1H, s), 8.35 (1H, s). |
| 6-454 | *δ* 7.59 (1H, t, J =7.8 Hz), 7.75 (1H, d, J =7.8 Hz), 7.93 (1H, s), 7.95-7.99 (3H, m), 8.10 (1H, broad-s), 8.23-8.26 (2H, m), 8.36 (2H, d, J =1.9 Hz). |
| 6-460 | *δ* 7.43-7.46 (1H, m), 7.58 (1H, t, J =7.8Hz), 7.77 (1H, d, J =7.8Hz), 7.91-7.95 (2H, m), 8.01 (1H, s), 8.24 (1H, dd, J =2.0, 7.8Hz), 8.28 (1H, s), 8.36 (1H, s), 8.41 (1H, s), 8.54-8.56 (1H, m). |
| 6-461 | *δ* 7.45 (1H, dd, J =4.9, 7.3Hz), 7.59 (1H, t, J =7.8Hz), 7.77 (1H, d, J =7.8Hz), 7.92-7.99 (3H, m), 8.24-8.29 (2H, m), 8.36-8.39 (2H, m), 8.55 (1H, dd, J =2.0, 4.9Hz). |
| 6-467 | *δ* 7.45-7.60 (2H, m), 7.74 (1H, d, J =8.0Hz), 7.95-7.97 (2H, m), 8.03 (1H, s), 8.12 (1H, s), 8.19-8.35 (2H, m), 8.35 (1H, s), 8.90(1 H, s). |
| 6-502 | *δ* 7.51-7.62(4H, m), 7.73(1 H, d, J =7.6Hz), 7.89-7.95(4H, m), 8.01(2H, d, J =14.4Hz), 8.27(1 H, s), 8.34(1 H, s). |
| 6-503 | *δ* 7.20-7.23(1 H, m), 7.34(1 H, t, J =7.8Hz), 7.54-7.60(2H, m), 7.74(1 H, d, J =7.8Hz), 7.94(2H, s), 8.05(1 H, s), 8.19(1 H, t, J =7.8Hz), 8.33(2H, d, J =12.0Hz), 8.65(1 H, d, J =16.0Hz). |
| 6-504 | *δ* 7.47-7.69(5H, m), 7.73(1 H, d, J =7.8Hz), 7.93(2H, s), 8.05(2H, d, J =11.0Hz), 8.25(1 H, s), 8.34(1 H, s). |
| 6-505 | *δ* 7.20(2H, t, J =7.8Hz), 7.56(1 H, t, J =8.0Hz), 7.73(1 H, d, J =8.2Hz), 7.91-7.94(5H, m), 8.02(1 H, s), 8.24(1 H, s), 8.35(1 H, s). |
| 6-522 | *δ* 7.68-7.75(2H, m), 7.86(1 H, d, J =8.2Hz), 7.93-7.96(2H, m), 8.08(1 H, s), 8.14(1 H, d, J =8.0Hz), 8.24(2H, d, J =8.2Hz), 8.28(1 H, s), 8.33(1 H, s), 8.62(1 H, s). |
| 6-523 | *δ* 7.57(1 H, t, J =7.8Hz), 7.82(2H, d, J =8.0Hz), 7.93-8.02(6H, m), 8.14(1 H, s), 8.23(1 H, s), 8.34(1 H, s). |
| 6-524 | *δ* 7.05(2H, t, J =8.0Hz), 7.43-7.50(1 H, m), 7.56(1 H, t, J =8.0Hz), 7.75(1 H, d, J =7.8Hz), 7.86(1 H, s), 7.92(2H, d, J =8.2Hz), 8.00(1 H, s), 8.27(1 H, s), 8.34(1 H, s). |
| 6-538 | *δ* 7.45(1 H, t, J =7.6Hz), 7.59(2H, s), 7.70(1 H, d, J =4.0Hz), 7.93(2H, d, J =7.6Hz), 7.98(1 H, s), 8.28(1 H, s), 8.34(2H, d, J =7.6Hz), 8.41(1 H, s). |
| 6-545 | *δ* 7.56-7.60(1 H, m), 7.75(1 H, d, J =8.0Hz), 7.92-8.00(4H, m), 8.09(1 H, s), 8.19-8.23(2H, m), 8.35(1 H, s), 8.90(1 H, s). |
| 6-804 | *δ* 7.50-7.62 (4H, m), 7.68-7.70 (1H, m), 7.85-7.91 (3H, m), 8.10 (1H, s), 8.16-8.22 (4H, m). |

**Table 52(5)**

| compound number | ¹H-NMR (CDCl₃,ppm) |
|---|---|
| 6-824 | *δ* 7.55-7.59 (2H, m), 7.66-7.76 (2H, m), 7.86-7.92 (2H, m), 7.99-8.08 (2H, m), 8.13-8.22 (2H, m), 8.39-8.43 (2H, m). |
| 6-825 | *δ* 7.55-7.59 (2H, m), 7.66-7.70 (2H, m), 7.72-7.73 (2H, m), 7.86-7.92 (2H, m), 7.99-8.22 (3H, m), 8.40-8.42 (1H, m). |
| 6-840 | *δ* 7.42-7.45 (1H, m), 7.55-7.59 (1H, t, J =7.8Hz), 7.71-7.30 (1H, d, J =7.8Hz), 7.88-7.91 (1H, m), 8.00 (1H, s), 8.20-8.24 (4H, m), 8.41 (1H, s), 8.54-8.55 (1H, m). |
| 6-1104 | *δ* 7.40 (1H, t, J =7.8Hz), 7.53-7.64 (3H, m), 7.89 (2H, s), 7.90-7.95 (3H, m), 8.11-8.14 (2H, m), 8.69-8.70 (1H, m). |
| 6-1105 | *δ* 7.22-7.26 (1H, m), 7.35-7.41 (2H, m), 7.58 (1H, d, J =8.0Hz), 7.88-7.92 (3H, m), 8.15-8.25 (2H, m), 8.74-8.75 (2H, m). |
| 6-1106 | *δ* 7.35-7.34 (1H, m), 7.417.42 (1H, m), 7.53-7.54 (1H, m), 7.67-7.68 (2H, m), 7.89-7.90 (3H, m), 8.09-8.10 (2H, m), 8.66-8.68 (1H, m). |
| 6-1107 | *δ* 7.21-7.26 (2H, m), 7.39-7.41 (1H, m), 7.89-7.96 (4H, m), 8.05-8.13 (3H, m), 8.70-8.72 (1H, m). |
| 6-1124 | *δ* 7.42-7.43 (1H, m), 7.70-7.72 (1H, m), 7.89-7.92 (4H, m), 8.09-8.17 (3H, m), 8.22 (1H, s), 8.62-8.63 (1H, m). |
| 6-1125 | *δ* 7.42-7.44 (1H, m), 7.84-7.94 (5H, m), 8.02-8.11 (4H, m), 8.63-8.64 (1H, m). |
| 6-1126 | *δ* 7.05-7.09 (2H, m), 7.38-7.42 (1H, m), 7.49-7.50 (1H, m), 7.88-7.99 (4H, m), 8.09-8.12 (1H, m), 8.71-8.72 (1H, m). |
| 6-1140 | *δ* 7.40-7.49(2H, m), 7.89(2H, s), 7.94-7.96(1 H, m), 8.13(1 H, d, J = 12.7Hz), 8.32-8.34(1 H, m), 8.57-8.59(1 H, m), 8.67-8.71(1 H, m), 8.75(1 H, broad-s). |
| 6-1147 | *δ* 7.39-7.44 (1H, m), 7.52-7.55 (1H, m), 7.89 (1H, s), 7.96-7.98 (1H, m), 8.05-8.11 (3H, m), 8.21-8.23 (1H, m), 8.61-8.62 (1H, m), 8.93 (1H, s). |
| 6-1182 | *δ* 7.37-7.41 (1H, m), 7.53-7.64 (3H, m), 7.87-7.94 (5H, m), 8.11-8.14 (2H, m), 8.67-8.71 (1H, m). |
| 6-1183 | *δ* 7.35-7.41(2H, m), 7.58-7.61 (1H, m), 7.86-7.92(3H, m), 8.17-8.25(2H, m), 8.61(1H, s), 8.74(1H, t, J =7.8Hz), 8.90-8.93(1H, m). |
| 6-1184 | *δ* 7.33(1H, t, J =8.0Hz), 7.40(1H, t, J =7.8Hz), 7.50-7.56(1H, m), 7.64-7.69(2H, m), 7.88(2H, s), 7.92(1H, t, J =7.8Hz), 8.09-8.12(2H, m), 8.63(1H, t, J =8.2Hz). |
| 6-1185 | *δ* 7.22(2H, t, J =7.8Hz), 7.29-7.30(1 H, m), 7.40(1 H, t, J =8.0Hz), 7.88(2H, s), 7.90-7.97(2H, m), 8.05(1 H, s), 8.10-8.13(1 H, m), 8.64(1 H, t, J =7.8Hz). |
| 6-1202 | *δ* 7.42-7.44 (1H, m), 7.68-7.72 (1H, m), 7.88-7.94 (4H, m), 8.10-8.23 (4H, m), 8.60-8.65 (1H, m). |
| 6-1203 | *δ* 7.39-7.44 (1H, m), 7.84-7.88 (4H, m), 7.92-7.95 (1H, m), 8.02-8.13 (4H, m), 8.61-8.65 (1H, m). |
| 6-1204 | *δ* 7.05(2H, t, J =8.0Hz), 7.40(1 H, t, J =8.0Hz), 7.48-7.52(1 H, m), 7.87(2H, s), 7.92(1 H, t, J =8.0Hz), 8.01(1 H, s), 8.11-8.14(1 H, m), 8.68(1 H, t, J =7.4Hz). |

**Table 52(6)**

| compound number | ¹H-NMR (CDCl₃,ppm) |
|---|---|
| 6-1218 | *δ* 7.40-7.49(2H, m), 7.95(2H, s), 7.94(1 H, t, J =7.8Hz), 8.12-8.16(1 H, m), 8.34(1 H, d, J =8.0Hz), 8.58(1 H, s), 8.70(1 H, t, J =7.8Hz), 8.77(1 H, s). |
| 6-1225 | *δ* 7.42(1 H, t, J =8.2Hz), 7.53(1 H, d, J =8.0Hz), 7.88(2H, s), 7.94(1 H, t, J =7.0Hz), 8.06(1 H, s), 8.09-8.12(1 H, m), 8.20-8.23(1 H, m), 8.60(1 H, t, J =8.0Hz), 8.93(1 H, s). |
| 6-1260 | *δ* 7.39 (1H, t, J =7.8Hz), 7.52-7.57 (4H, m), 7.60-7.63 (2H, m), 7.93-7.94 (4H, m), 8.70 (1H, t, J =6.3Hz). |
| 6-1261 | *δ* 7.22-7.28 (1H, m), 7.35-7.42 (2H, m), 7.59-7.61 (1H, m), 7.94-7.95 (1H, m), 8.12 (2H, s), 8.15-8.25 (2H, m), 8.78 (1H, t, J =1.5Hz), 9.00 (1H, d, J =7.8Hz). |
| 6-1262 | *δ* 7.30-7.33(1 H, m), 7.40-7.44(1 H, m), 7.52-7.55(2H, m), 7.64-7.70(3H, m), 7.95-7.96(1 H, m), 8.09-8.13(2H, m), 8.67-8.68(1 H, m). |
| 6-1263 | *δ* 7.20-7.26 (3H, m), 7.38-7.42 (1H, m), 7.91-7.98 (3H, m), 8.07-8.12 (3H, m), 8.63-8.67 (1H, m). |
| 6-1280 | *δ* 7.41-7.45(1 H, m), 7.68-7.72(1 H, m), 7.90-7.99(2H, m), 8.08-8.25(5H, m), 8.22-8.24(1 H, m), 8.56-8.65(1 H, m). |
| 6-1281 | *δ* 7.41-7.50(1 H, m), 7.86-7.87(2H, m), 7.95-7.98(1 H, m), 8.03-8.13(6H, m), 8.65-8.67(1 H, m). |
| 6-1282 | *δ* 7.06 (2H, t, J =8.3Hz), 7.38-7.42 (1H, m), 7.47-7.52 (1H, m), 7.93-7.97 (1H, m), 8.01 (1H, s), 8.11-8.13 (3H, m), 8.68-8.72 (1H, m). |
| 6-1296 | *δ* 7.41-7.52(2H, m), 7.96-8.00(1 H, m), 8.12-8.14(3H, m), 8.33-8.35(1 H, m), 8.57-8.59(1 H, m), 8.68-8.76(2H, m). |
| 6-1303 | *δ* 7.41-7.45(1 H, m), 7.51-7.54(1 H, m), 7.96-7.99(1 H, m), 8.06-8.24(5H, m), 8.61(1 H, m), 8.94(1 H, m). |
| 6-1338 | *δ* 7.38-7.42 (1H, m), 7.52-7.64 (3H, m), 7.90-7.94 (3H, m), 8.11-8.15 (4H, m), 8.67-8.71 (1H, m). |
| 6-1339 | *δ* 7.35-7.42(2H, m), 7.60(1 H, d, J =8.0Hz), 7.94(1 H, t, J =8.0Hz), 8.1K2H, s), 8.17-8.25(2H, m), 8.62(1 H, s), 8.74(1 H, t, J =7.4Hz), 8.92(1 H, d, J =16.4Hz). |
| 6-1340 | *δ* 7.28-7.35(2H, m), 7.40(1 H, t, J =8.0Hz), 7.51-7.56(1 H, m), 7.64-7.70(2H, m), 7.94(1 H, t, J =8.0Hz), 8.10(2H, s), 8.14(1 H, s), 8.65(1 H, t, J =8.0Hz). |
| 6-1341 | *δ* 7.20-7.25(2H, m), 7.40(1 H. t, J =8.0Hz). 7.92-7.97(3H, m), 8.09(1 H. d, J =8.0Hz). 8.1K2H,s), 8.14(1 H. s), 8.64(1 H, t, J =8.0Hz). |
| 6-1358 | *δ* 7.41-7.45 (1H, m), 7.68-7.72 (1H, m), 7.89-7.98 (2H, m), 8.10-8.23 (6H, m), 8.60-8.64 (1H, m). |
| 6-1359 | *δ* 7.41-7.45 (1H, m), 7.83-7.89 (2H, m), 7.94-7.99 (1H, m), 8.03-8.14 (6H, m), 8.62-8.66 (1H, m). |
| 6-1360 | *δ* 7.05(1 H, t, J =8.8Hz), 7.41-7.46(1 H, m), 7.52(1 H, t, J =7.8Hz), 7.95(1 H, t, J =7.8Hz), 8.03(1 H, s), 8.10(2H, s), 8.15(1 H, s), 8.61(1H, s), 8.70(1 H, t, J =8.0Hz). |
| 6-1374 | *δ* 7.41-7.49(2H, m), 8.00(1 H, t, J =8.0Hz), 8.11(2H, s), 8.16(1 H, s), 8.34(1 H, d, J =7.2Hz), 8.58(1 H, d, J =2.0Hz), 8.70(1 H, t, J =7.8Hz), 8.78(1 H, s). |

**Table 52(7)**

| compound number | ¹H-NMR (CDCl₃,ppm) |
|---|---|
| 6-1381 | *δ* 7.43(1 H, t, J =8.0Hz), 7.53(1 H, d, J =8.0Hz), 7.97(1 H, t, J =7.8Hz), 8.11-8.12(3H, m), 8.14(1 H, s), 8.22(1 H, d, J =8.0Hz), 8.57(1 H, t, J =8.0Hz), 8.94(1 H, s). |
| 6-1574 | *δ* 7.37-7.41 (1H, m), 7.53-7.64(3H, m), 7.86-7.94(4H, m), 8.13-8.22(3H, m), 8.67-8.72(1 H, m). |
| 6-1575 | *δ* 7.22-7.27 (1H, m), 7.35-7.41 (2H, m), 7.57-7.60 (1H, m), 7.88-7.93 (2H, m), 8.16 (1H, s), 8.20-8.25 (2H, m), 8.74-8.75 (1H, m), 8.77-9.00 (1H, m). |
| 6-1576 | *δ* 7.27-7.37 (2H, m), 7.48-7.53 (1H, m), 7.72-7.80 (3H, m), 7.92 (1H, s), 8.15 (1H, s), 8.27-8.31 (1H, m), 9.16 (1H, s), 9.35 (1H, d, J =7.3Hz). |
| 6-1577 | *δ* 7.20-7.23 (3H, m), 7.24-7.26 (1H, m), 7.39-7.40 (3H, m), 7.86-7.97 (1H, m), 8.06 (1H, d, J =2.4Hz), 8.16-8.20 (1H, m), 8.62-8.67 (1H, m). |
| 6-1594 | *δ* 7.41-7.42 (1H, m), 7.70-7.71 (1H, m), 7.93-8.89 (3H, m), 8.12-8.24 (5H, m), 8.60-8.61 (1H, m). |
| 6-1595 | *δ* 7.41(1H, t, J = 7.8Hz), 7.84-7.87(2H, m), 7.90-7.94(2H, m), 8.03-8.05(2H, m), 8.12-8.18(3H, m), 8.62-8.66(1H, m). |
| 6-1596 | *δ* 7.00-7.09 (2H, m), 7.40-7.41 (1H, m), 7.46-7.53 (1H, m), 7.89-7.92 (2H, m), 8.00 (1H. s), 8.16-8.19 (2H, m), 8.71-8.72 (1H, m). |
| 6-1610 | *δ* 7.42-7.49(2H, m), 7.93-7.94(2H, m), 8.15-8.21(2H, m), 8.31-8.34(1 H, m), 8.57-8.59(1 H, m), 8.63-8.69(2H, m). |
| 6-1617 | *δ* 7.31-7.38 (1H, m), 7.49 (1H, d, J =8.3Hz), 7.82-7.86 (1H, m), 7.92 (1H, s), 8.15 (1H, s), 8.19-8.23 (1H, m), 8.30 (1H, dd, J =2.4, 8.3Hz), 9.04-9.09 (2H, m), 9.53 (1H, s). |
| 6-1652 | *δ* 7.40 (1H, t, J =7.8Hz), 7.53-7.64 (3H, m), 7.86-7.94 (4H, m), 8.13-8.20 (3H, m), 8.71-8.72 (1H, m). |
| 6-1653 | *δ* 7.22-7.25 (1H, m), 7.35-7.41 (2H, m), 7.57-7.60 (1H, m), 7.88-7.92 (2H, m), 8.15 (1H, s), 8.20-8.29(2H, m), 8.71-8.75 (1H, m), 8.89-8.93 (1H, m). |
| 6-1654 | *δ* 7.32-7.34 (1H, m), 7.38-7.42 (1H, m), 7.50-7.56 (1H, m), 7.63-7.70 (2H, m), 7.88-7.91 (2H, m), 8.09-8.20 (3H, m), 8.63-8.68 (1H, m). |
| 6-1655 | *δ* 7.21-7.30 (2H, m), 7.37-7.41 (1H, m), 7.86-7.97 (4H, m), 8.06-8.21 (3H, m), 8.63-8.67 (1H, m). |
| 6-1672 | *δ* 7.37-7.41 (1H, m), 7.67-7.71 (1H, m), 7.88-7.91 (3H, m), 8.15-8.18 (2H, m), 8.24-8.28 (3H, m), 8.52-8.56 (1H, m). |
| 6-1673 | *δ* 7.39-7.44 (1H, m), 7.85-7.93 (4H, m), 8.03-8.05 (2H, m), 8.13-8.18 (3H, m), 8.61-8.65 (1H, m). |
| 6-1674 | *δ* 7.04-7.09 (2H, m), 7.38-7.42 (1H, m), 7.47-7.52 (1H, m), 7.89-7.93 (2H, m), 8.00 (1H, s), 8.14-8.20 (2H, m), 8.69-8.73 (1H, m). |
| 6-1688 | *δ* 7.42 (1H, t, J =7.8Hz), 7.47 (1H, dd, J =4.9, 7.8Hz), 7.92-7.96 (2H, m), 8.14 (1H, s), 8.19 (1H, d, J =12.7Hz), 8.33 (1H, dd, J =2.0, 7.8Hz), 8.58 (1H, dd, J =2.0, 4.9Hz), 8.70-8.71 (1H, m), 8.74 (1H, s). |
| 6-1695 | *δ* 7.39-7.43 (1H, m), 7.52-7.54 (1H, m), 7.92-7.95 (2H, m), 8.08-8.09 (1H, m), 8.15 (1H, s), 8.17-8.23 (2H, m), 8.57-8.93 (1H, m), 8.94 (1H, s). |

**Table 52(8)**

| compound number | ¹H-NMR (CDCl₃,ppm) |
|---|---|
| 6-1730 | *δ* 7.40 (1H, t, J =7.8Hz), 7.53-7.64 (3H, m), 7.87-7.95 (4H, m), 8.14 (1H, s), 8.22 (1H, d, J =12.7Hz), 8.37 (1H, s), 8.71-8.72 (1H, m). |
| 6-1731 | *δ* 7.22-7.25(1 H, m), 7.35-7.37(2H, m), 7.58-7.60(1 H, m), 7.90(1 H, t, J =8.6Hz), 7.96(1 H, s), 8.22(1 H, t, J =8.8Hz), 8.29-8.32(1 H, d, J =12.4Hz), 8.37(1 H, s), 8.73(1 H, m), 8.94(1 H, s). |
| 6-1732 | *δ* 7.32-7.33(1 H, m), 7.37(1 H, t, J =8.0Hz), 7.52-7.54(1 H, m), 7.64-7.70(2H, m), 7.90(1 H, t, J =6.4Hz), 7.96(1 H, s), 8.10(1 H, s), 8.23(1 H, d, J =12.0Hz), 8.37(1 H, s), 8.65(1 H, t, J =8.0Hz). |
| 6-1733 | *δ* 7.19-7.25(2H, m), 7.35(1H, t, J =7.8Hz), 7.87-7.97(4H, m), 8.08(1H, s), 8.25(1H, d, J =12.0Hz), 8.37(1H, s), 8.65(1H, t, J =8.0Hz). |
| 6-1734 | *δ* 7.43-7.52(5H, m), 7.87-7.95(3H, m), 8.22(1 H, d, J =10.0Hz), 8.35(1 H, d, J =8.2Hz), 8.74(1 H, t, J =8.4Hz). |
| 6-1750 | *δ* 7.40(1 H, t, J =8.2Hz), 7.68(1 H, t, J =8.0Hz), 7.88-7.95(2H, m), 8.12(1 H, s), 8.16(1 H, d, J =8.0Hz), 8.23-8.25(2H, m), 8.30-8.33(1 H, m), 8.36(1 H, s), 8.53(1 H, t, J =7.4Hz). |
| 6-1751 | *δ* 7.44(1 H, t, J =8.0Hz), 7.86(2H, d, J =8.0Hz), 7.91-7.96(2H, m), 8.03(2H, d, J =7.6Hz), 8.13-8.22(2H, m), 8.37(1 H, s), 8.64(1 H, t, J =8.6Hz). |
| 6-1752 | *δ* 7.04-7.11 (2H, m), 7.38-7.53(2H, m), 7.90-7.95(2H, m), 801(1 H, s), 8.22(1 H, d, J =12.8Hz), 8.37(1 H, s), 8.69-8.73(1 H, m). |
| 6-1754 | *δ* 7.39-7.44(2H, m), 7.54(1 H, s), 7.85(1 H, d, J =8.0Hz), 7.90-7.95(2H, m), 8.22(1 H, d, J =12.0Hz), 8.68(1 H, s), 8.70(1 H, s), 8.69(1 H, t, J =7.8Hz). |
| 6-1755 | *δ* 7.16-7.19(1 H, m), 7.40(1 H, t, J =8.0Hz), 7.92-7.95(2H, m), 8.07(2H, t, J =4.8Hz), 8.24(1 H, d, J =12.8Hz), 8.37(1 H, s), 8.42(1 H, s), 8.70(1 H, t, J =8.0Hz). |
| 6-1766 | *δ* 7.41-7.49 (2H, m), 7.93-7.97 (2H, m), 8.22 (1H, d, J =13.2Hz), 8.33 (1H, dd, J =2.0, 7.3Hz), 8.37 (1H, s), 8.58 (1H, dd, J =2.0, 4.9Hz), 8.70-8.73 (2H, m). |
| 6-1773 | *δ* 7.40(1 H, t, J =8.2Hz), 7.52(1 H, d, J =8.0Hz), 7.91-7.96(2H, m), 8.12-8.28(3H, m), 8.37(1 H, s), 8.57(1 H, s), 8.94(1 H, s). |
| 6-1808 | *δ* 7.38-7.42 (1H, m), 7.53-7.64 (3H, m), 7.87-7.94 (4H, m), 8.14 (1H, s), 8.23-8.26 (1H, m), 8.36 (1H, s), 8.68-8.71 (1H, m). |
| 6-1809 | *δ* 7.22-7.25(2H, m), 7.38(1 H, t, J =8.0Hz), 7.58-7.61 (1H, m), 7.89-7.94(2H, m), 8.23(1 H, t, J =6.3Hz), 8.29(1 H, d, J =13.2Hz), 8.36(1 H, s), 8.74(1 H, t, J =6.3Hz), 8.93-8.94(1 H, m). |
| 6-1810 | *δ* 7.30-7.33(2H, m), 7.40(1 H. t, J =7.8Hz), 7.51-7.56(1 H, m), 7.64-7.70(2H, m), 7.88-7.94(1 H, m), 8.10(1 H. s), 8.23(1 H, d, J =12.2Hz), 8.36(1 H, s), 8.67(1 H, t, J =7.8Hz). |
| 6-1811 | *δ* 7.39(1 H, t, J =8.3Hz), 7.86-7.97(3H, m), 8.08(2H, s), 8.25(1 H, d, J =12.2Hz), 8.36(2H, s), 8.65(2H, t, J =6.3Hz). |
| 6-1828 | *δ* 7.30-7.33(2H, m), 7.41(1 H, t, J =7.6Hz), 7.68-7.73(1 H, m), 7.89-7.94(2H, m), 8.16(1 H, d, J =7.6Hz), 8.23-8.27(1 H, m), 8.41(1 H, s), 8.63-8.64(2H, m). |
| 6-1829 | *δ* 7.29-7.31(1 H, m), 7.42(1 H, t, J =8.0Hz), 7.90-7.94(2H, m), 8.04(2H, d, J =4.4Hz), 8.15(1 H, s), 8.23(1 H, d, J =12.0Hz), 8.36(1 H, s), 8.60-8.66(2H, m). |
| 6-1830 | *δ* 7.06(1 H, t, J =8.3Hz), 7.41(1 H, t, J =7.8Hz), 7.67-7.69(1 H, m), 7.90-7.93(2H, m), 8.03(1 H, s), 8.25(1 H, d, J =12.7Hz), 8.35(1 H, s), 861(1 H, s), 8.70(1 H, t, J =7.8Hz). |

**Table 52(9)**

| compound number | ¹H-NMR (CDCl₃,ppm) |
|---|---|
| 6-1851 | *δ* 7.42(1 H, t,J = 8.0Hz), 7.53(1 H, d, J =7.9Hz), 7.92-7.94(2H, m), 8.09(1 H, s), 8.21-8.24(2H, m), 8.36(1 H, s), 8.59(1 H, t, J =8.0Hz), 8.94(1 H, s). |
| 6-2110 | *δ* 7.36-7.40 (1H, m), 7.53-7.64 (3H, m), 7.84-7.97 (1H, m), 7.92-7.94 (2H, m), 8.04-8.07 (1H, m), 8.08-8.13 (1H, m), 8.20 (2H, s), 8.68-8.72 (1H, m). |
| 6-3348 | *δ* 7.29-7.34(1 H, m), 7.53-7.65(3H, m), 7.80-7.84(1 H, m), 7.90-7.92(3H, m), 8.14(1 H, broad-s), 8.20(1 H, d, J = 2.9Hz), 8.25(1 H, broad-s), 9.10(1 H, dd, J = 1.9,7.3Hz). |
| 6-3384 | *δ* 7.32-7.36(1 H, m), 7.45-7.48(1 H, m), 7.83-7.87(1 H, m), 7.93(1 H, broad-s), 8.10(1 H, broad-s), 8.15(1 H, broad-s), 8.30(1 H, dd, J = 1.5,7.3Hz), 8.57-8.79(1 H, m), 8.78(1 H, broad-s), 9.09(1 H, dd, J = 2.4,7.3Hz). |
| 6-5902 | (DMSO-d₆) *δ* 7.39(1 H, t, J = 7.8Hz), 7.52-7.64(4H, m), 7.81(1H, t, J = 6.8Hz), 7.95(1 H, s), 7.98-8.01(3H, m), 10.29(1 H, s), 10.68(1 H, s). |
| 6-5903 | *δ* 7.50-7.67(5H, m), 7.79(1 H, d, J =8.0Hz), 7.92(1 H, d, J =8.0Hz), 8.01(2H, d, J =8.0Hz), 8.10(2H, s), 8.31(1 H, s). |
| 6-5904 | *δ* 7.19-7.23(1 H, m), 7.28-7.31(1 H, m), 7.58(1 H, t, J =8.2Hz), 7.78(1 H, d, J =8.0Hz), 7.91-7.94(3H, m), 8.01(2H, d, J =8.0Hz), 8.30(2H, s), 8.61(1H, s). |
| 6-5905 | *δ* 7.26 (1H, s), 7.43-7.46 (1H, m), 7.56-7.60 (1H, m), 7.80-7.94 (5H, m), 8.22-8.56(4H, m). |
| 6-5906 | *δ* 7.18-7.12 (2H, m), 7.53-7.57 (1H, m), 7.76 (1H, d, J =7.6Hz), 7.86-8.28 (7H, m), 8.28 (1H, s). |
| 6-5907 | *δ* 7.39(1 H, t, J = 7.8Hz), 7.52-7.64(4H, m), 7.85-7.94(4H, m), 8.06(1 H, d, J = 12.2Hz), 8.14(1 H, broad-s), 8.67-8.71(1H, m). |
| 6-5908 | *δ* 7.39-7.49(2H, m), 7.59(1 H, s), 7.88-7.94(2H, m), 8.07(1 H, d, J = 12.2Hz), 8.31-8.33(1 H, m), 8.57-8.58(1 H, m), 8.60-8.70(1 H, m), 8.74(1 H, broad-s). |
| 6-5909 | *δ* 7.42(1 H, t, J = 7.8Hz), 7.59(1 H, s), 7.85-7.92(4H, m), 7.99(1 H, broad-s), 8.02-8.05(2H, m), 8.10(1 H, broad-s), 8.61-8.67(1 H, m). |
| 6-5910 | *δ* 7.39(1 H, t, J = 8.3Hz), 7.53-7.64(3H, m), 7.88-7.94(4H, m), 8.13(1 H, broad-s), 8.19(1 H, broad-s), 8.24(1 H, d, J = 13.2Hz), 8.70-8.72(1 H, m). |
| 6-5911 | *δ* 7.41(1 H, t, J = 8.3Hz), 7.84-7.87(3H, m), 7.91-7.95(1 H, m), 8.03-8.05(2H, m), 8.10(1 H, broad-s), 8.17-8.20(2H, m), 8.63-8.67(1 H, m). |
| 6-5912 | *δ* 3.10 (3H, s), 7.59 (1H, t, J =7.8 Hz), 7.76 (1H, d, J =7.8 hz), 7.77-8.00 (5H, m), 8.03 (1H, s), 8.19 (1H, s), 8.24 (1H, s), 8.36 (1H, s). |
| 6-5913 | *δ* 7.51-7.62 (4H, m), 7.72 (1H, dd, J =1.5, 7.8Hz), 7.89-8.00 (6H, m), 8.14 (1H, s), 8.27 (1H, t, J =2.0Hz). |
| 6-5914 | *δ* 7.44 (1H, dd, J =4.9, 7.8Hz), 7.56-7.60 (1H, m), 7.76 (1H, d, J =7.8Hz), 7.92 (3H, broad-s), 8.14 (1H, s), 8.22-8.27 (1H, m), 8.27 (1H, s), 8.41 (1H, s), 8.55 (1H, dd, J =2.0, 4.4Hz). |
| 7-38 | *δ* 3.57(3H, s), 7.21-7.24(1 H, m), 7.32-7.34(4H, m), 7.40-7.44(2H, m), 7.59(1 H, s), 7.71(1 H, d, J =8.0Hz), 7.84(3H, s). |
| 7-39 | *δ* 3.54(3H, s), 7.06-7.19(1 H, m), 7.28-7.34(2H, m), 7.43-7.48(2H, m), 7.57(1 H, s), 7.69(1 H, s), 7.75(1 H, d, J =8.0Hz), 7.90(1 H, d, J =8.0Hz), 8.06(2H, s). |

**Table 52(10)**

| compound number | ¹H-NMR (CDCl₃,ppm) |
|---|---|
| 7-45 | *δ* 3.57(3H, s), 7.29-7.30 (2H, m), 7.48(1 H, t, J =8.0Hz), 7.59(1 H, s), 7.68-7.71 (3H, m), 7.77(1 H, d, J =7.8Hz), 7.85(2H, s), 8.24(1 H, s). |
| 7-46 | *δ* 3.57(3H, s), 7.41-7.45(3H, m), 7.52(2H, s), 7.55(1 H, d, J =8.0Hz), 7.69(1 H, s), 7.74(1 H, d, J =7.8Hz), 7.86(2H, s), 8.62(1 H, s). |
| 7-47 | *δ* 3.56(3H, s), 6.97-7.05(3H, m), 7.17-7.19(1 H, m), 7.43-7.50(3H, m), 7.73(1 H, s), 7.85(2H, s). |
| 7-57 | *δ* 3.57(3H, s), 7.15(1 H, t, J =6.0Hz), 7.41(2H, s), 7.50(1 H, s), 7.52(1 H, s), 7.60(1 H, d, J =6.4Hz), 7.73(1 H, s), 7.85(2H, s), 8.27(1 H, s). |
| 7-60 | 3.57(3H, s), 7.30(1 H, d, J =8.0Hz), 7.47(1 H, t, J =8.0Hz), 7.61(1 H, s), 7.76(2H, s), 7.78-7.81 (1H, m), 7.85(2H, s), 8.11(1 H, s), 8.24(1 H, s). |
| 7-71 | *δ* 3.56(3H, s), 7.20-7.25(1 H, m), 7.33-7.38(3H, m), 7.43-7.47(3H, m), 7.56(1 H, s), 7.74(1 H, d, J =6.8Hz), 8.06-8.10(3H, m). |
| 7-72 | *δ* 3.55(3H, s), 7.06-7.10(1 H, m), 7.30-7.36(2H, m), 7.43-7.48(2H, m), 7.57(1 H, s), 7.68(1 H, s), 7.78(1 H, d, J =8.0Hz), 7.90(1 H, d, J =8.0Hz), 8.07(2H, s). |
| 7-78 | *δ* 3.57(3H, s), 7.29-7.64(2H, m), 7.70(1 H, s), 7.79(1 H, d, J =8.0Hz), 7.86(1 H, d, J =8.0Hz), 7.95(1 H, t, J =8.0Hz), 8.08(2H, s), 8.33(1 H, d, J =8.0Hz), 8.40(1 H, s), 8.71(1 H, s). |
| 7-79 | *δ* 3.58(3H, s), 7.42-7.53(3H, m), 7.60-7.66(1 H, m), 7.77(1 H, d, J =8.0Hz), 7.89(1 H, t, J =8.0Hz), 8.08(2H, s), 8.21(1 H, d, J =8.0Hz), 8.70(2H, s). |
| 7-80 | *δ* 3.56(3H, s), 7.02(1 H, t, J =8.0Hz), 7.42-7.44(1 H, m), 7.55(1 H, t, J =8.0Hz), 7.75(1 H, d, J =8.0Hz), 7.90-7.95(3H, m), 8.01(1 H, s), 8.14(1 H, s), 8.26(1 H, s). |
| 7-90 | *δ* 3.59(3H, s), 7.59(1 H, s), 7.60(1 H, d, J =6.0Hz), 7.80-7.83(1 H, m), 8.08(2H, s), 8.26-8.30(2H, m), 8.53(1 H, d, J =4.0Hz), 8.69(2H, s). |
| 7-93 | *δ* 3.58(3H, s), 7.34-7.52(3H, m), 7.63(1 H, d, J =16.0Hz), 7.69(1 H, d, J =8.0Hz), 7.80(1 H, d, J =8.0Hz), 7.85(1 H, t, J =8.0Hz), 8.25(1 H, s), 8.29(1 H, d, J =8.0Hz), 8.69(1 H, s). |
| 7-132 | *δ* 3.57 (3H, s), 7.26-7.28 (1H, m), 7.41-7.46 (3H, m), 7.52-7.54 (3H, m), 7.63 (1H, s), 7.68 (1H, d, J =7.8Hz), 7.87 (1H, s), 7.98 (1H, s). |
| 7-147 | *δ* 3.58 (3H, s), 7.14 (1H, dd, J = 4.9,7.8Hz), 7.41-7.42 (2H, m), 7.52 (1H, s), 7.59 (1H, dd, J = 1.5,7.8Hz), 7.67 (2H, broad-s), 7.87 (1H, s), 7.97 (1H, s), 8.26 (1H, dd, J =1.5,4.9Hz). |
| 7-268 | *δ* 3.49 (3H, s), 7.12-7.16 (2H, m), 7.21-7.28 (4H, m), 7.35 (1H, t, J =7.8Hz), 7.64 (1H, s), 7.70 (1H, dd, J =1.5,8.8Hz), 7.86 (1H, s), 8.07 (1H, d, J =1.5Hz), 8.29 (1H, s). |
| 7-269 | *δ* 3.54 (3H, s), 6.80-6.81 (1H, m), 7.70-7.08 (1H, m), 7.25-7.26 (1H, m), 7.39-7.41 (4H, m), 7.52-7.53 (1H, m), 7.70-7.71 (1H, m), 7.90 (1H, s), 8.12 (1H, s). |
| 7-270 | *δ* 3.55 (3H, s), 6.97-6.99 (1H, m), 7.04-7.06 (2H, m), 7.15-7.21 (1H, m), 7.32-7.34 (1H, m), 7.44 (1H, t, J =7.8Hz), 7.58-7.59 (2H, m), 7.70 (1H, d, J =7.8Hz), 7.90 (1H, s), 8.12 (1H, s). |
| 7-271 | *δ* 3.55 (3H, s), 6.88-6.92 (2H, m), 7.30-7.34 (3H, m), 7.43 (1H, t, J =7.8Hz), 7.57-7.58 (2H, m), 7.67-7.69 (1H, m), 7.90-7.91 (1H, m), 8.13-8.14 (1H, m). |
| 7-285 | *δ* 3.59 (3H, s), 7.34-7.52 (3H, m), 7.65-7.73 (4H, m), 7.90 (1H, s), 8.13-8.16 (3H, m). |

**Table 52(11)**

| compound number | ¹H-NMR (CDCl₃,ppm) |
|---|---|
| 7-286 | *δ* 3.59 (3H, s), 7.29-7.30 (1H, m), 7.42-7.52 (4H, m), 7.63 (1H, s), 7.69 (1H, d, J =7.8Hz), 7.91 (1H, s), 8.08-8.13 (3H, m). |
| 7-288 | *δ* 3.57 (3H, s), 7.15-7.37 (2H, m), 7.47-7.48 (1H, m), 7.52-7.62 (5H, m), 7.72 (1H, d, J =7.8Hz), 7.91 (1H, s), 8.14 (1H, s). |
| 7-289 | *δ* 3.57 (3H, s), 7.26-7.29 (1H, m), 7.40-7.46 (3H, m), 7.52 (2H, d, J =8.3Hz), 7.58-7.61 (2H, m), 7.70 (1H, d, J =7.8Hz), 7.92 (1H, s), 8.14(1 H, s). |
| 7-290 | *δ* 3.55 (3H, s), 6.70-6.74 (2H, m), 7.14-7.19 (1H, m), 7.43-7.44 (2H, m), 7.52 (1H, s), 7.67 (1H, s), 7.74-7.77 (1H, m), 7.91 (1H, s), 8.13 (1H, s). |
| 7-299 | *δ* 3.58 (3H, s), 7.33 (1H, d, J =6.8Hz), 7.52 (1H, t, J =7.8Hz), 7.64-7.65 (2H, m), 7.75-7.79 (3H, m), 7.83 (1H, s), 7.92 (1H, s), 8.14 (1H, s). |
| 7-300 | *δ* 3.56 (3H, broad-s), 7.14 (1H, broad-d, J =8.3Hz), 7.33-7.53 (4H, m), 7.59-7.60 (2H, m), 7.71-7.72 (1H, m), 7.92 (1H, s), 8.14 (1H, s). |
| 7-301 | *δ* 3.57 (3H, s), 7.33-7.59 (4H, m), 7.60 (1H, s), 7.64 (1H, s), 7.68-7.70 (2H, m), 7.92 (1H, s), 8.15 (1H, s). |
| 7-303 | *δ* 3.56 (3H, s), 7.17-7.12 (1H, m), 7.39-7.45 (2H, m), 7.55 (2H, s), 7.69 (1H, d, J =7.8Hz), 7.90 (2H, s), 8.13-8.14 (2H, m). |
| 7-304 | *δ* 3.54 (3H, s), 7.10 (1H, dd, J =4.9,7.8Hz), 7.35-7.40 (2H, m), 7.56 (1H, dd, J =1.5,7.8Hz), 7.70 (2H, s), 7.88-7.90 (2H, m), 8.12 (1H, s), 8.22 (1H, dd, J =1.5,4.9Hz). |
| 7-305 | *δ* 3.57 (3H, s), 7.13 (1H, dd, J =4.9,7.8Hz), 7.41-7.42 (2H, m), 7.57-7.62 (2H, m), 7.67-7.69 (2H, m), 7.91 (1H, s), 8.13 (1H, s), 8.24-8.26 (1H, m). |
| 7-311 | *δ* 3.57 (3H, s), 7.23-7.31 (2H, m), 7.47-7.48 (1H, m), 7.66-7.73 (4H, m), 7.92 (1H, s), 8.14 (1H, s), 8.23 (1H, d, J =1.9Hz). |
| 7-316 | *δ* 3.59 (3H, s), 7.27-7.30 (1H, m), 7.48-7.49 (1H, m), 7.60-7.67 (3H, m), 7.73 (1H, d, J =7.8Hz), 7.85 (1H, dd, J =1.9,7.8Hz), 7.92 (1H, s), 8.15 (1H, d, J =1.5Hz), 8.52 (1H, s). |
| 7-346 | *δ* 3.54(3H, s), 7.18-7.34(4H, m), 7.40(1 H, t, J =7.8Hz), 7.57(1 H, s), 7.67-7.69(1 H, m), 7.88(1 H, s), 8.10(1 H, s). |
| 7-347 | *δ* 3.53 (3H, s), 6.82 (1H, s), 7.07 (1H, s), 7.23-7.70 (7H, m), 7.89 (1H, s), 8.11 (1H, s). |
| 7-348 | *δ* 3.53 (3H, s), 6.96-6.99 (1H, m), 7.00-7.06 (2H, m), 7.15-7.19 (1H, m), 7.32-7.34 (1H, m), 7.42-7.46(1H, m), 7.60 (1H, s), 7.68-7.71 (2H, m), 7.89 (1H, s), 8.11 (1H, s). |
| 7-349 | *δ* 3.54 (3H, s), 6.88-6.92 (2H, m), 7.29-7.34 (3H, m), 7.41-7.44 (2H, m), 7.59 (1H, s), 7.68-7.70 (1H, m), 7.89 (1H, s), 8.11 (1H, s). |
| 7-366 | *δ* 3.64 (3H, s), 7.31-7.36 (2H, m), 7.44-7.48 (2H, m), 7.52-7.58 (1H, m), 7.61 (2H, s), 7.71-7.73 (2H, m), 7.89 (1H, s), 8.12 (1H, s). |
| 7-367 | *δ* 3.56 (3H, s), 7.22-7.31 (3H, m), 7.44-7.48 (1H, m), 7.66-7.77 (4H, m), 7.90 (1H, s), 8.12 (1H, s), 8.22 (1H, s). |
| 7-368 | *δ* 3.52 (3H, s), 6.67-6.72 (2H, m), 7.00 (1H, s), 7.13-7.17 (1H, m), 7.41-7.42 (2H, m), 7.72-7.89 (3H, m), 8.11 (1H, s). |

**Table 52(12)**

| compound number | ¹H-NMR (CDCl₃,ppm) |
|---|---|
| 7-382 | *δ* 3.58 (3H, s), 7.13-7.16 (1H, m), 7.43-7.61 (4H, m), 7.67(2H, s), 7.90 (1H, s), 8.13 (1H, s), 8.26-8.27 (1H, m). |
| 7-389 | *δ* 3.57 (3H, s), 7.29 (1H, s), 7.40-7.46 (3H, m), 7.52-7.54 (2H, m), 7.59-7.62 (1H, m), 7.69-7.70 (1H, m), 7.90 (1H, s), 8.13 (1H, s). |
| 7-424 | *δ* 3.56 (3H, s), 7.19-7.37 (6H, m), 7.44-7.45 (1H, m), 7.54 (1H, s), 7.60 (1H, broad-s), 7.68 (1H, d, J =7.8Hz), 7.92 (1H, s), 8.31 (1H, s). |
| 7-425 | *δ* 3.54 (3H, broad-s), 6.82 (1H, broad-s), 7.07 (1H, broad-s), 7.23-7.30 (2H, m), 7.31-7.51 (3H, m), 7.52-7.56 (1H, m), 7.71 (1H, broad-s), 7.92 (1H, s), 8.33 (1H, s). |
| 7-426 | *δ* 3.56 (3H, s), 6.96-7.00 (1H, m), 7.05-7.07 (2H, m), 7.15-7.21 (1H, m), 7.34-7.36 (1H, m), 7.46-7.47 (1H, m), 7.58-7.66 (2H, m), 7.71 (1H, d, J =7.3Hz), 7.93 (1H, s), 8.33 (1H, s). |
| 7-427 | *δ* 3.56 (3H, s), 6.88-6.92 (2H, m), 7.31-7.34 (2H, m), 7.45-7.46 (1H, m), 7.56 (1H, s), 7.62 (1H, s), 7.65 (1H, s), 7.70 (1H, d, J =7.3Hz), 7.93 (1H, s), 8.33 (1H, s). |
| 7-428 | *δ* 3.58(3H, s), 7.00-7.24(4H, m), 7.42-7.43(3H, m), 7.62(1 H, s), 7.66(1 H, s), 7.94(1 H, s), 8.34(1 H, s). |
| 7-444 | *δ* 3.57 (3H, s), 7.33-7.37 (2H, m), 7.48-7.49 (1H, m), 7.52-7.61 (4H, m), 7.69-7.74 (2H, m), 7.93 (1H, s), 8.34 (1H, s). |
| 7-445 | *δ* 3.58 (3H, s), 7.30 (1H, d, J =9.5Hz), 7.41-7.48 (3H, m), 7.51-7.53 (3H, m), 7.59 (1H, s), 7.70 (1H, d, J =7.9Hz), 7.94 (1H, s), 8.34 (1H, s). |
| 7-446 | *δ* 3.56 (3H, s), 6.69-6.73 (2H, m), 7.14-7.18 (1H, m), 7.44-7.45 (2H, m), 7.61 (1H, s), 7.69 (1H, s), 7.75-7.77 (1H, m), 7.93 (1H, s), 8.34 (1H, s). |
| 7-449 | *δ* 3.51(3H, s), 6.82-6.86(1 H, m), 6.94-6.98(1 H, m), 7.14-7.24(1 H, m), 7.28-7.29(2H, m), 7.41(1 H, s), 7.64(1 H, s), 7.69(1 H, d, J =5.9Hz), 7.94(1 H, s), 8.34(1 H, s). |
| 7-460 | *δ* 3.59 (3H, s), 7.13 (1H, dd, J =4.8, 7.1Hz), 7.43-7.44 (2H, m), 7.52 (1H, s), 7.58-7.60 (1H, m), 7.67 (2H, broad-s), 7.94 (1H, s), 8.26 (1H, dd, J =1.6, 4.8Hz), 8.34 (1H, s). |
| 7-467 | *δ* 3.58 (3H, s), 7.23 (1H, d, J =8.3Hz), 7.33 (1H, d, J =7.8Hz), 7.49-7.50 (1H, m), 7.62 (1H, s), 7.67-7.68 (2H, m), 7.73 (1H, d, J =7.8Hz), 7.93 (1H, s), 8.23 (1H, d, J =2.0Hz), 8.34 (1H, s). |
| 7-502 | *δ* 3.57(3H, s), 7.20-7.26(3H, m), 7.27-7.37(3H, m), 7.45(1 H, t, J =7.6Hz), 7.56(1 H, s), 7.68(1 H, s), 7.71(1 H, d, J =8.0Hz), 7.91(1 H, s), 8.31(1 H, s). |
| 7-504 | 3.56(3H, s), 6.96-7.01 (1H, m), 7.06(2H, d, J =7.2Hz), 7.18(1 H, t, J =6.8Hz), 7.35(1 H, d, J =8.0Hz), 7.45(1 H, t, J =8.0Hz), 7.56(2H, s), 7.71(1 H, d, J =8.0Hz), 7.91(1 H, s), 8.32(1 H, s). |
| 7-505 | *δ* 3.57(3H, s), 6.90(2H, t, J =8.8Hz), 7.31-7.35(3H, m), 7.45(1 H, t, J =8.0Hz), 7.54(2H, d, J =13.6Hz), 7.69(1 H, d, J =8.0Hz), 7.91(1 H, s), 8.32(1 H, s). |
| 7-522 | *δ* 3.57(3H, s), 7.32-7.37(2H, m), 7.46(1 H, t, J =8.0Hz), 7.55(1 H, t, J =7.6Hz), 7.59(2H, d, J =8.0Hz), 7.75(1 H, d, J =8.0Hz), 7.88(1 H, s), 7.92(2H, s), 8.33(1 H, s). |
| 7-523 | *δ* 3.56(3H, s), 7.41-7.47(4H, m), 7.53(2H, d, J =8.2Hz), 7.61(1 H, s), 7.71-7.72(2H, m), 7.92(1 H, s), 8.33(1 H, s). |
| 7-538 | *δ* 3.58(3H, s), 7.14(1 H, t, J =6.0Hz), 7.40-7.43(2H, m), 7.56-7.58(2H, m), 7.68-7.69(2H, m), 7.92(1 H, s), 8.26(1 H, s), 8.33(1 H, s). |

**Table 52(13)**

| compound number | ¹H-NMR (CDCl₃,ppm) |
|---|---|
| 7-804 | *δ* 3.55 (3H, s), 7.19-7.52 (9H, m), 7.62 (1H, d, J =7.8Hz), 8.16 (2H, s). |
| 7-824 | *δ* 3.57 (3H, s), 7.33-7.75 (7H, m), 7.98-7.99 (1H, m), 8.12 (2H, s), 8.18-8.43 (1H, m). |
| 7-825 | *δ* 3.57 (3H, s), 7.22-7.66 (7H, m), 7.85-7.88 (1H, m), 7.97-7.99 (2H, m), 8.17-8.27 (1H, m). |
| 7-840 | *δ* 3.57 (3H, s), 7.13-7.26 (1H, m), 7.40-7.44 (2H, m), 7.58-7.71 (3H, m), 8.18-8.23 (4H, m). |
| 7-948 | *δ* 3.49 (3H, s), 7.23-7.52 (8H, m), 7.66 (2H, s), 8.00 (1H, t, J =6.8Hz). |
| 7-969 | *δ* 3.51 (3H, s), 7.45-7.46 (3H, m), 7.53-7.55 (3H, m), 7.67 (2H, s), 7.92-7.93 (1H, m), 8.05-8.06 (1H, m). |
| 7-984 | *δ* 3.53 (3H, s), 7.13-7.16 (1H, m), 7.20-7.24 (1H, m), 7.52-7.53 (1H, m), 7.65-7.68 (3H, m), 7.99-8.03 (2H, m), 8.28-8.30 (1H, m). |
| 7-1104 | *δ* 3.51 (3H, s), 7.22-7.44 (7H, m), 7.86 (2H, s), 8.00-8.03 (2H, m). |
| 7-1105 | *δ* 3.52 (3H,s), 6.82 (1H, t, J =8.8Hz), 7.06-7.08 (1H, m), 7.18-7.24 (2H, m), 7.40-7.44 (2H, m), 7.87 (3H, s), 8.01-8.05 (1H, m). |
| 7-1106 | *δ* 3.50 (3H,s), 7.00-7.23 (4H, m), 7.29-7.31 (1H, m), 7.45 (1H, s), 7.87 (3H, s), 8.03-8.07 (1H, m). |
| 7-1107 | *δ* 3.49 (3H,s), 6.91-6.93 (2H, m), 7.28-7.44 (4H, m), 7.86 (2H, s), 8.00-8.10 (2H, m). |
| 7-1124 | *δ* 3.52 (3H, s), 7.30-7.34 (2H, m), 7.46-7.59 (3H, m), 7.71 (1H, broad-s), 7.87 (2H, s), 7.97-8.09 (2H, m). |
| 7-1125 | *δ* 3.52(3H, s), 7.29-7.32(1 H, m), 7.45-7.52(5H, m), 7.84(2H, s), 7.95-8.06(2H, m). |
| 7-1126 | *δ* 3.49 (3H,s), 6.72-6.76 (2H, m), 7.16-7.23 (2H, m), 7.43-7.50(1 H, m), 7.88 (2H, s), 8.02-8.06 (1H, m). 8.13-8.17(1 H, m). |
| 7-1140 | *δ* 3.54(3H, s), 7.13-7.16(1 H, m), 7.21-7.26(1 H, m), 7.43-7.46(1 H, m), 7.52(1 H, broad-s), 7.65-7.69(1 H, m), 7.88(2H, s), 8.00-8.04(1 H, m), 8.28-8.30(1 H, m). |
| 7-1147 | *δ* 3.52 (3H, s), 7.22-7.26 (1H, m), 7.34 (1H, t, J =7.8Hz), 7.46-7.52 (1H, m), 7.62-7.68 (1H, m), 7.87 (2H, s), 7.91-7.95 (1H, m), 8.07-8.11 (1H, m), 8.34 (1 H, broad-s). |
| 7-1182 | *δ* 3.51 (3H, s), 7.22-7.44 (7H, m), 7.85 (2H, s), 8.00-8.03 (2H, m). |
| 7-1183 | *δ* 3.51(3H, s), 6.79-6.83(1 H, m), 7.05(1 H, t, J =7.8Hz), 7.17-7.23(2H, m), 7.40-7.43(2H, m), 7.85(2H, s), 7.98-8.00(2H, m). |
| 7-1184 | *δ* 3.52(3H, s), 6.99-7.22(4H, m), 7.28-7.30(1 H, m), 7.44-7.45(1 H, m), 7.88(2H, s), 8.02-8.06(2H, m). |

**Table 52(14)**

| compound number | ¹H-NMR (CDCl₃,ppm) |
|---|---|
| 7-1185 | *δ* 3.49(3H, s), 6.91-7.00(2H, m), 7.26-7.44(4H, m), 7.85(2H, s), 8.01-8.10(2H, m). |
| 7-1202 | *δ* 3.52 (3H, s), 7.26-7.32 (1H, m), 7.45-7.52 (5H, m), 7.86 (2H, s), 7.84-7.95 (1H, m), 8.06-8.08 (1H, m). |
| 7-1203 | *δ* 3.52 (3H, s), 7.26-7.32 (1H, m), 7.45-7.52 (5H, m), 7.86 (2H, s), 7.84-7.95 (1H, m), 8.06-8.08 (1H, m). |
| 7-1204 | *δ* 3.51(3H, s), 7.02(1 H, t, J =8.8Hz), 7.39-7.44(1 H, m), 7.50(1 H, t, J =7.4Hz), 7.93(1 H, t, J =7.8Hz), 8.02(1 H, s), 8.10(2H, s), 8.13(1 H, s), 8.68(1 H, t, J =8.0Hz). |
| 7-1218 | *δ* 3.52(3H, s), 7.39-7.47(1 H, m), 7.96(1 H, t, J =8.0Hz), 8.11(2H, s), 8.16(1 H, s), 8.33(1 H, d, J =7.2Hz), 8.58-8.59(1 H, m), 8.68(1 H, t, J =7.8Hz), 8.78(1 H, s). |
| 7-1225 | *δ* 3.52 (3H, s), 7.22-7.24 (1H, m), 7.32-7.36 (1H, m), 7.46-7.50 (1H, m), 7.66-7.68 (1H, m), 7.85 (2H, s), 7.93-7.96 (1H, m), 8.07-8.11 (1H, m), 8.34 (1H, broad-s). |
| 7-1260 | *δ* 4.09 (3H, s), 7.21-7.49 (7H, m), 7.99-8.08 (4H, m). |
| 7-1261 | *δ* 3.53 (3H, s), 6.79-6.83 (1H, m), 7.03-7.07 (1H, m), 7.19-7.23 (2H, m), 7.42-7.43 (2H, m), 8.01-8.10 (4H, m). |
| 7-1262 | *δ* 3.51 (3H, s), 6.99-7.00 (1H, m), 7.08-7.14 (2H, m), 7.29-7.33 (2H, m), 7.46-7.47 (1H, m), 8.05-8.12 (4H, m). |
| 7-1263 | *δ* 3.52 (3H, s), 6.88-6.93 (2H, m), 7.29-7.37 (3H, m), 7.46-7.48 (1H, m), 8.04-8.09 (4H, m). |
| 7-1280 | *δ* 3.52 (3H, s), 7.31-7.47 (2H, m), 7.49 (1H, s), 7.57 (2H, m), 7.69 (1H, s), 8.07-8.10 (4H, m). |
| 7-1281 | *δ* 3.53 (3H, s), 7.23-7.52 (6H, m), 7.90-8.09 (4H, m). |
| 7-1282 | *δ* 3.55 (3H, s), 6.72-6.76 (2H, m), 7.17-7.24 (2H, m), 7.48-7.49 (1H, m), 8.04-8.16 (4H, m). |
| 7-1296 | *δ* 3.55 (3H, s), 7.12-7.15 (1H, m), 7.46-7.51 (1H, broad-s), 7.67-7.69 (1H, m), 8.03-8.12 (5H, m), 8.27-8.29 (1H, m). |
| 7-1303 | *δ* 3.54 (3H, s), 7.20-7.23 (1H, m), 7.35-7.39 (1H, m), 7.52-7.53 (1H, m), 7.65 (1H, s), 7.97 (1H, broad-s), 8.09-8.14 (3H, m), 8.34-8.35 (1H, m). |
| 7-1338 | *δ* 3.52 (3H, s), 7.10-7.35 (6H, m), 7.45-7.52 (1H, m), 8.02-8.07 (4H, m). |
| 7-1339 | *δ* 3.53(3H, s), 6.80(1 H, t, J =8.0Hz), 7.04(1 H, t, J =8.0Hz), 7.29-7.32(1 H, m), 7.43(1 H, t, J =8.0Hz), 7.68-7.71(1 H, m), 8.01-8.04(2H, m), 8.09(2H, s), 8.63(1 H, s). |
| 7-1340 | *δ* 3.56(3H, s), 7.04-7.08(1 H, m), 7.30-7.33(1 H, m), 7.43-7.48(2H, m), 7.57(1 H, s), 7.64(1 H, s), 7.77(1 H, d, J =8.0Hz), 7.90(1 H, d, J =8.0Hz), 8.07(2H, s). |
| 7-1341 | *δ* 3.52(3H, s), 6.91-6.92(2H, m), 7.30(1 H, t, J =8.0Hz), 7.36(2H, s), 7.47(1 H, t, J =8.0Hz), 8.00-8.06(2H, m), 8.08(2H, s). |

**Table 52(15)**

| compound number | ¹H-NMR (CDCl₃,ppm) |
|---|---|
| 7-1358 | *δ* 3.53 (3H, s), 7.31-7.40 (2H, m), 7.45-7.60 (3H, m), 7.69 (1H, s), 7.99-8.11 (4H, m). |
| 7-1359 | *δ* 3.53 (3H, s), 7.26-7.34 (1H, m), 7.40-7.60 (5H, m), 7.83-7.95 (1H, m), 8.01-8.06 (3H, m). |
| 7-1360 | *δ* 3.55(3H, s), 6.72-6.76(2H, m), 7.22-7.24(1 H, m), 7.42-7.44(1 H, m), 7.49(1 H, t, J =8.0Hz), 8.05(1 H, d, J =8.0Hz), 8.10(2H, s), 8.17(1 H, d, J =8.0Hz). |
| 7-1374 | *δ* 3.55(3H, s), 7.12-7.15(1 H, m), 7.52-7.53(2H, m), 7.68(1 H, d, J =8.0Hz), 8.02-8.06(2H, m), 8.11(2H, s), 8.28(1 H, s). |
| 7-1381 | *δ* 3.54(3H, s), 7.19-7.22(1 H, m), 7.37(1 H, t, J =8.0Hz), 7.54(1 H, t, J =6.0Hz), 7.66(1 H, d, J =6.8Hz), 7.93-7.96(1 H, m), 8.09-8.13(3H, m), 8.35(1 H, s). |
| 7-1417 | *δ* 3.49 (3H, s), 7.23-7.26 (3H, m), 7.27-7.33 (3H, m), 7.52-7.53 (1H, m), 7.85 (1H, s), 7.96-8.06 (3H, m). |
| 7-1574 | *δ* 3.50(3H, s), 6.99-7.33(6H, m), 7.43-7.45(1 H, m), 7.90(1 H, s), 7.97-8.06(2H, m), 8.13(1 H, s). |
| 7-1575 | *δ* 3.52 (3H, s), 6.82-6.83 (1H, m), 7.06-7.07 (1H, m), 7.19-7.26 (2H, m), 7.39-7.46 (2H, m), 7.91 (1H, s), 7.99-8.01 (1H, m), 8.07-8.14 (2H, m). |
| 7-1576 | *δ* 3.50 (3H, s), 7.01-7.17 (4H, m), 7.27-7.31 (1H, m), 7.46-7.52 (1H, m), 7.91 (1H, s), 8.01-8.05 (2H, m), 8.13 (1H, s). |
| 7-1577 | *δ* 3.50 (3H, s), 6.90-6.94 (2H, m), 7.26-7.35 (3H, m), 7.45-7.46 (1H, m), 7.90 (1H, s), 8.00-8.07 (2H, m), 8.13 (1H, s). |
| 7-1594 | *δ* 3.52(3H, s), 7.29-7.36(2H, m), 7.50(2H, broad-s), 7.60(1 H, broad-d, J = 6.8Hz), 7.70(1 H, broad-s), 7.91(1 H, broad-s), 8.04-8.07(2H, m), 8.14(1 H, s). |
| 7-1595 | *δ* 3.51(3H, s), 7.29-7.31(2H, m), 7.44-7.52(4H, m), 7.92(1 H, s), 8.03-8.06(2H, m), 8.14(1 H, broad-s). |
| 7-1596 | *δ* 3.54 (3H, s), 6.75 (2H, broad-s), 7.17-7.26 (2H, m), 7.50-7.51 (1H, m), 7.92 (1H, s), 8.01-8.05 (1H, m), 8.14-8.20 (2H, m). |
| 7-1605 | *δ* 3.53 (3H, s), 7.41 (1H, t, J =7.8Hz), 7.53-7.55 (1H, m), 7.82-7.86 (3H, m), 7.91 (1H, s), 7.99-8.01 (1H, m), 8.10-8.14 (2H, m). |
| 7-1606 | *δ* 2.53 (3H, s), 7.15-7.17 (1H, m), 7.24-7.28 (1H, m), 7.39-7.41 (1H, m), 7.41-7.44 (1H, m), 7.53-7.56 (1H, m), 7.93 (1H, s), 8.04-8.08 (2H, m), 8.15 (1H, s). |
| 7-1608 | *δ* 3.52 (3H, s), 7.22-7.23 (1H, m), 7.29-7.33 (1H, m), 7.48-7.52 (1H, m), 7.71-7.72 (1H, m), 7.90 (1H, s), 8.00-8.03 (2H, m), 8.13 (1H, s), 8.54-8.55 (2H, m). |
| 7-1610 | *δ* 3.54(3H, s), 7.14-7.17(2H, m), 7.45-7.70(2H, m), 7.96(1 H, s), 8.00-8.07(3H, m), 8.28-8.30(1 H, m). |
| 7-1616 | *δ* 3.52 (3H, s), 7.27-7.23 (1H, m), 7.35-7.36 (1H, m), 7.49-7.51 (1H, m), 7.68 (1H, d, J =6.8Hz), 7.91 (1H, s), 8.09-8.05 (2H, m), 8.13 (1H, s), 8.30 (1H, s). |
| 7-1617 | *δ* 3.52 (3H, s), 7.23-7.27 (1H, m), 7.33-7.37 (1H, m), 7.47-7.51 (1H, m), 7.67-7.68 (1H, m), 7.91 (1H, s), 8.05-8.09 (1H, m), 8.13 (2H, s), 8.30 (1H, s). |

**Table 52(16)**

| compound number | ¹H-NMR (CDCl₃,ppm) |
|---|---|
| 7-1638 | *δ* 3.50 (3H, s), 7.19-7.27 (2H, m), 7.32-7.33 (1H, m), 7.40-7.42 (1H, m), 7.91 (1H, s), 8.00-8.02(1 H, m), 7.09-8.11 (1H, m), 8.14 (1H, s), 8.18 (1H, s), 8.56 (1H, d, J =8.8Hz). |
| 7-1639 | *δ* 3.52 (3H, broad-s), 7.00-7.29 (3H, m), 7.44 (1H, broad-s), 7.52 (1H, s), 7.91-8.14 (3H, m), 8.51 (2H, broad-s). |
| 7-1645 | *δ* 3.50 (3H, s), 7.22-7.27 (2H, m), 7.33-7.37 (1H, m), 7.43-7.47 (1H, m), 7.92 (1H, s), 8.02-8.10 (3H, m), 8.15-8.19 (2H, m). |
| 7-1652 | *δ* 3.50 (3H, s), 7.23-7.26 (2H, m), 7.27-7.32 (4H, m), 7.44-7.45 (1H, m), 7.88 (1H, s), 7.98 (1H, t, J =6.8Hz), 8.04-8.08 (1H, m), 8.11 (1H, s). |
| 7-1672 | *δ* 3.51 (3H, s), 7.29-7.35 (2H, m), 7.49-7.50 (2H, m), 7.60 (1H, d, J =6.3Hz), 7.69 (1H, s), 7.89 (1H, s), 8.03-8.06 (2H, m), 8.12 (1H, s). |
| 7-1673 | *δ* 3.52 (3H, s), 7.29-7.32 (1H, m), 7.44-7.52 (5H, m), 7.89 (1H, s), 8.02-8.06 (2H, m), 8.12 (1H, s). |
| 7-1730 | *δ* 3.51 (3H, s), 7.21-7.23 (2H, m), 7.27-7.33 (4H, m), 7.44-7.46 (1H, m), 7.92 (1H, s), 8.00 (1H, t, J =6.3Hz), 8.08-8.09 (1H, m), 8.33 (1H, s). |
| 7-1731 | *δ* 3.51(3H, s), 6.79-6.83(1 H, m), 7.05(1 H, t, J =7.6Hz), 7.18-7.46(4H, m), 7.94-8.00(2H, m), 8.20(1 H, d, J =12.4Hz), 8.34(1 H, s). |
| 7-1732 | *δ* 3.50(3H, s), 7.00-7.18(4H, m), 7.27-7.31 (1H, m), 7.45-7.48(1 H, m), 7.93(1 H, s), 8.01-8.03(1 H, m), 8.12(1 H, broad-s), 8.34(1 H, s). |
| 7-1733 | *δ* 3.50(3H, s), 6.91(2H, s), 6.93-7.35(3H, m), 7.47(1 H, t, J =7.0Hz), 7.93(1 H, s), 8.01-8.10(1 H, m), 8.13(1 H, broad-s), 8.34(1 H, s). |
| 7-1750 | *δ* 3.52(3H, s), 7.28-7.71(6H, m), 7.93(1 H, s), 8.06(1 H, t, J =8.4Hz), 8.34(1 H, s), 8.60(1 H, s). |
| 7-1751 | *δ* 3.52 (3H, s), 7.32 (1H, t, J =8.3Hz), 7.44-7.52 (5H, m), 7.93 (1H, s), 8.04-8.07 (2H, m), 8.34 (1H, s). |
| 7-1752 | *δ* 3.54(3H, s), 6.74(2H, s), 7.16-7.24(2H, m), 7.50(1 H, t, J= 7.4Hz), 7.94(1 H, s), 8.01-8.05(1 H, m), 8.25(1 H, broad-s), 8.35(1 H, s). |
| 7-1764 | *δ* 3.53 (3H, s), 7.20-7.21 (1H, m), 7.32 (1H, t, J =7.8Hz), 7.50 (1H, t, J =7.8Hz), 7.70-7.71 (1H, m), 7.92 (1H, s), 8.01-8.04 (2H, m), 8.33 (1H, s), 8.53-8.54 (2H, m). |
| 7-1766 | *δ* 3.53(3H, s), 7.14-7.17(1 H, m), 7.23-7.25(1 H, m), 7.54-7.55(1 H, m), 7.68(1 H, d, J =8.0Hz). 7.95(1 H, s), 8.00(1 H, t, J =7.0Hz), 8.10-8.20(1 H, m), 8.28(1 H, d, J =4.8Hz), 8.36(1 H, s). |
| 7-1773 | *δ* 3.53(3H, s), 7.19-7.40(3H, m), 7.52-7.53(1 H, m), 7.67-7.68(1 H, m), 7.93(2H, s), 8.07-8.09(1 H, m), 8.32(1 H, d, J =14.4Hz), 8.35(1 H, s). |
| 7-1794 | *δ* 3.51 (3H, s), 7.18-7.23 (2H, m), 7.32 (1H, t, J =7.8Hz), 7.43-7.46 (1H, m), 7.92 (1H, s), 7.98-8.01 (1H, m), 8.08-8.09 (1H, m), 8.23-8.24 (1H, m), 8.32 (1H, s), 8.56-8.59 (1H, m). |
| 7-1808 | *δ* 3.51 (3H, s), 7.23-7.43 (6H, m), 7.44-7.60 (1H, m), 7.91 (1H, s), 7.98-8.01 (1H, m), 8.10 (1H, broad-s), 8.32 (1H, s). |
| 7-1809 | *δ* 3.52(3H, s), 6.80(1 H, t, J =8.8Hz), 7.06(1 H, t, J =3.6Hz), 7.17-7.23(2H, m), 7.39-7.44(1 H, m), 7.92-7.93(2H, m), 7.99(1 H, t, J =5.2Hz), 8.16(1 H, d, J =13.2Hz), 8.33(1 H, s). |

**Table 52(17)**

| compound number | ¹H-NMR (CDCl₃,ppm) |
|---|---|
| 7-1810 | *δ* 3.48(3H, s), 6.99-7.16(3H, m), 7.28-7.34(2H, m), 7.43-7.45(1 H, m), 7.72(1 H, t, J =8.0Hz), 7.92(1 H, s), 8.00(1 H, t, J =8.0Hz), 8.33(1 H, s). |
| 7-1811 | *δ* 3.53(3H, s), 6.90(2H, t, J =8.0Hz), 7.28-7.40(3H, m), 7.46(1 H, t, J =7.2Hz), 7.92(1 H, s), 8.02(1 H, t, J =8.0Hz), 8.13(1 H, d, J =12.4Hz), 8.33(1 H, s). |
| 7-1828 | *δ* 3.52(3H, s), 7.32-7.35(2H, m), 7.49-7.60(3H, m), 7.69(1 H, s), 7.92(1 H, s), 8.05-8.08(2H, m), 8.36(1 H, s). |
| 7-1829 | *δ* 3.53 (3H, s), 7.30-7.34 (1H, m), 7.45-7.52 (5H, m), 7.92 (1H, s), 8.04-8.07 (2H, m), 8.34 (1H, s). |
| 7-1844 | *δ* 3.54(3H, s), 7.15(1 H, t, J =5.2Hz), 7.52-7.54(1 H, m), 7.68-7.71 (2H, m), 7.94(1 H, s), 8.00(1 H, t, J =5.2Hz), 8.15-8.16(1 H, m), 8.28(1 H, d, J =4.8Hz), 8.34(1 H, s). |
| 7-1851 | *δ* 3.52(3H, s), 7.65(1 H, d, J =6.8Hz), 7.92(1 H, s), 8.05-8.08(2H, m), 8.11-8.18(3H, m), 8.26-8.31(1 H, m), 8.33(1 H, s). |
| 7-3348 | *δ* 3.48(3H, s), 7.18-7.30(6H, m), 7.45(1 H, broad-s), 7.52(1 H, broad-s), 7.74-7.76(1 H, m), 7.89(1 H, s), 8.11(1H, s). |
| 7-3369 | *δ* 3.49(3H, s), 7.18-7.22(1 H, m), 7.45-7.53(5H, m), 7.56(2H, broad-s), 7.92(1 H, s), 8.14(1 H, s). |
| 7-3384 | *δ* 3.52(3H, s), 7.15-7.20(2H, m), 7.45-7.85(4H, m), 7.92(1 H, s), 8.12-8.15(1 H, m), 8.27-8.29(1 H, m). |
| 7-5902 | *δ* 3.51 (3H, s), 7.00-7.52 (7H, m), 7.88 (1H, d, J =1.5Hz), 8.01-8.06 (3H, m). |
| 7-5903 | *δ* 3.55 (3H, s), 7.14 (1H, dd, J =4.8, 7.8Hz), 7.24-7.26 (1H, m), 7.40-7.60 (1H, m), 7.66-7.68 (1H, m), 7.91 (1H, s), 8.01-8.05 (2H, m), 8.08 (1H, d, J =1.5Hz), 8.28 (1H, dd, J =2.0, 4.8Hz). |
| 7-5904 | *δ* 3.56(3H, s), 7.05-7.09(1 H, m), 7.30-7.36(2H, m), 7.43-7.48(2H, m), 7.56(1 H, s), 7.65(1 H, s), 7.77(1 H, d, J =8.0Hz), 7.90(1 H, d, J =8.0Hz), 8.07(2H, s). |
| 7-5905 | *δ* 3.57(3H, s), 6.89(1 H, t, J =8.0Hz), 7.15(1 H, t, J =8.0Hz), 7.32-7.36(2H, m), 7.47(1 H, t, J =8.0Hz), 7.53(1 H, s), 7.61(1 H, s), 7.77(1 H, d, J =8.0Hz), 8.09(2H, s), 8.70(1 H, broad-s). |
| 7-5906 | *δ* 3.55(3H, s), 7.00(1 H, t, J =8.0Hz), 7.07(2H, d, J =7.8Hz), 7.16-7.22(1 H, m), 7.32(1 H, d, J =6.8Hz), 7.44(1 H, t, J =8.0Hz), 7.57(1 H, s), 7.65-7.67(1 H, m), 7.74(1 H, d, J =8.0Hz), 7.85(2H, s). |
| 7-5907 | *δ* 3.57(3H, s), 6.90(2H, t, J =7.8Hz), 7.29-7.34(3H, m), 7.44(1 H, t, J =7.8Hz), 7.49(1 H, s), 7.62(1 H, s), 7.73(1 H, d, J =7.8Hz), 7.85(2H, s). |
| 7-5908 | *δ* 2.99 (3H, s), 3.58 (3H, s), 7.27-7.30 (1H, m), 7.43-7.44 (1H, m), 7.51 (2H, d, J =8.3Hz), 7.65-7.70 (3H, m), 7.81 (2H, d, J =8.3Hz), 7.91 (1H, s), 8.14 (1H, s). |
| 7-5909 | *δ* 3.60 (3H, s), 7.33 (1H, d, J =7.9Hz), 7.50 (1H, t, J =7.9Hz), 7.63 (1H, s), 7.70 (1H, s), 7.75 (1H, d, J =7.9Hz), 7.94 (1H, s), 8.34 (1H, s), 8.65 (2H, s), 9.10 (1H, s). |
| 7-5910 | *δ* 3.53 (3H, s), 3.89 (3H, s), 7.32 (1H, t, J =7.8Hz), 7.48-7.49 (1H, m), 7.83-7.91 (2H, m), 8.01 (1H, t, J =7.3Hz), 8.10-8.14 (3H, m), 8.22-8.23 (1H, m). |
| 7-5911 | *δ* 3.57 (3H, s), 7.20-7.35 (6H, m), 7.41-7.45 (2H, m), 7.53 (1H, s), 7.67 (1H, d, J =7.8Hz), 7.89 (1H, s), 8.11 (1H, s). |

**Table 52(18)**

| compound number | ¹H-NMR (CDCl₃,ppm) |
|---|---|
| 7-5912 | *δ* 3.58 (3H, s), 7.14 (1H, dd, J =4.9, 7.8Hz), 7.42-7.43 (2H, m), 7.55 (1H, s), 7.59 (1H, dd, J =2.0, 7.8Hz), 7.67-7.68 (2H, m), 7.91 (1H, s), 8.13 (1H, s), 8.26 (1H, dd, J =2.0, 4.9Hz). |
| 8-268 | *δ* 3.28 (2/3∗3H, s), 3.39 (1/3∗3H, s), 7.13-7.14 (1/3∗1 H, m), 7.15-7.17 (1/3∗1 H, m), 7.34 (1/3∗1 H, s), 7.36 (1/3∗1 H, s), 7.46-7.57 (12/3∗1 H, m), 7.74-7.90 (13/3∗1 H, m), 7.94 (2/3∗1 H, s), 8.05 (1/3∗1 H, s), 8.15 (2/3*1 H, s), 8.24 (2/3*1 H, s). |
| 8-288 | (CDCl₃+DMSo-d₆) *δ* 3.30 13/4∗3H, s), 3.40 (1/4∗3H, s), 7.10-7.15 11/4∗1H, m), 7.20-7.25 11/4∗1H, m), 7.35 (1/4∗1 H, s), 7.37 (1/4∗1 H, s), 7.50 (3/4∗1 H, t, J =7.8Hz), 7.62-7.66 (6/4∗1 H, m), 7.82-7.84 (5/4∗1 H, m), 7.94 (1/4∗1 H, s), 7.95 (3/4*1 H, s), 8.00 (3/4*1 H, t, J =1.5Hz), 8.08-8.10 (4/4∗1 H, m), 8.17 (3/4*1 H, s), 8.18-8.19 (1/4∗1 H, m), 8.32-8.36 (4/4∗1 H, m), 8.48 (3/4*1 H, t, J =1.5Hz), 10.00 (1/4∗1 H, s), 10.29 (3/4∗1H, s) |
| 8-289 | *δ* 3.26 (2/3∗3H, s), 3.38 (1/3∗3H, s), 7.08-7.09 (1/3∗1 H, m), 7.12-7.14 (1/3∗1 H, m), 7.32 (2/3∗1 H, d, J =7.8Hz), 7.45-7.49 (3/3∗1 H, m), 7.72-7.76 (9/∗13H, m), 7.83 (1/3∗1 H, s), 7.85-7.89 (4/3∗1 H, m), 7.95 (2/3∗1 H, s), 7.98-8.00 (4/3∗1 H, m), 8.04 (2/3∗1 H, d, J =6.3Hz), 8.16 (2/3∗1 H, s), 8.57 (2/3∗1 H, s). |
| 8-304 | *δ* 3.29 (3/5∗3H, s), 3.38 (2/5∗3H, s), 7.14-7.18 (4/5∗1 H, m), 7.37-7.41 (8/5∗1 H, m), 7.50-7.52 (5/5∗1 H, m), 7.72 (2/5∗1 H, s), 7.83-7.86 (8/5∗1 H, m), 7.95 (3/5∗1 H, s), 8.05-8.07 (4/5∗1 H, m), 8.14-8.17 (6/5∗1 H, m), 8.30 (2/5∗1 H, s), 8.51-8.53 (5/5∗1 H, m), 8.57 (3/5∗1 H, s). |
| 8-804 | *δ* 3.31 (3H, s), 7.30-7.33 (1H, m), 7.48-7.52 (3H, m), 7.56-7.60 (1H, m), 7.72-7.74 (1H, m), 7.86-7.91 (3H, m), 8.06 (1H, s), 8.22 (2H, s). |
| 8-824 | (DMSO-d₆) *δ* 3.32 (12/13∗3H, s), 3.43 (1/13∗3H, s), 7.01-7.02 (1/13∗1 H, m), 7.20-7.21 (1/13∗1 H, m), 7.28 (12/13∗1 H, d, J =7.8Hz), 7.50 (12/13∗1 H, t, J =7.8Hz), 7.60-7.61 (1/13∗1 H, m), 7.65 (12/13∗1 H, t, J =7.8Hz), 7.83-7.84 (13/13∗1 H, m), 7.85 (1/13∗1 H, d, J =1.5Hz), 7.94 (12/13∗1 H, d, J =1.5Hz), 8.10-8.12 (12/13∗1 H, m), 8.14 (1/13∗2H, s), 8.21-8.22 (1/13∗1 H, m), 8.23 (12/13∗2H, s), 8.32-8.35 (13/13∗1 H, m), 8.47-8.48 (13/13∗1 H, m), 10.05 (1/13∗1 H, s), 10.37 (12/13∗1 H, s). |
| 8-840 | *δ* 3.30 (10/11∗3H, s), 3.43 (1/11∗3H, s), 7.17-7.18 (1/11∗1 H, m), 7.19-7.20 (1/11∗1 H, m), 7.33-7.42 (22/11∗1H, m), 7.52 (10/11∗1H, t, J =7.8Hz), 7.71 (1/11∗1H, s), 7.75 (10/11∗1H, s), 7.87 (10/11∗1H, d, J =8.3Hz), 8.12-8.13 (1/11∗1 H, m), 8.14 (1/11∗2H, s), 8.17 (10/11∗1H, dd, J =2.0,7.8Hz), 8.23 (10/11∗2H, s), 8.47-8.50 (11/11∗1H, m), 8.52-8.53 (11/11∗1 H, m). |
| 9-270 | *δ* 2.64 (3/4∗3H, s), 3.28 (1/4∗3H, s), 3.37 (1/4∗3H, s), 3.56 (3/4∗3H, s), 6.70-6.71 (1/4∗1H, m), 6.80-6.81 (1/4∗1 H, m), 7.02-7.03 (4/4∗1 H, m), 7.16-7.26 (16/4∗1 H, m), 7.34-7.36 (10/4∗1 H, m), 7.39-7.40 (1/4∗1 H, m), 7.46 (3/4*1 H, t, J =7.8Hz), 7.80 (1/4∗1 H, s), 7.91 (3/4*1 H, s), 8.01 (1/4∗1 H, s), 8.11 (3/4*1 H, s). |
| 9-290 | *δ* 2.88 (2/3∗3H, s), 3.33 (1/3∗3H, s), 3.37 (1/3∗3H, s), 3.56 (2/3∗3H, s), 6.70-6.71 (1/3∗1 H, m), 6.95-6.98 (2/3∗1 H, m), 7.10 (2/3∗1 H, s), 7.28-7.42 (9/3∗1 H, m), 7.47-7.60 (6/3∗1 H, m), 7.62-7.63 (4/3∗1 H, m), 7.86 (1/3∗1 H, s), 7.93 (2/3*1 H, s), 8.08 (1/3∗1 H, s), 8.14 (2/3*1 H, s). |
| 9-291 | *δ* 2.82 (2/3∗3H, s), 3.30 (1/3∗3H, s), 3.37 (1/3∗3H, s), 3.56 (2/3∗3H, s), 6.75-6.76 (1/3∗1 H, m), 6.95 (1/3∗1 H, t, J =7.8Hz), 7.00-7.01 (1/3∗1 H, m), 7.06 (2/3*1 H, s), 7.28-7.32 (4/3∗1 H, m), 7.39-7.54 (15/3∗1 H, m), 7.83 (1/3∗1 H, s), 7.93 (2/3*1 H, s), 8.04 (1/3∗1 H, s), 8.14 (2/3∗1 H, s). |
| 9-306 | *δ* 2.89 (2/3∗3H, s), 3.30 (1/3∗3H, s), 3.36 (1/3∗3H, s), 3.57 (2/3∗3H, s), 6.96-6.97 (1/3∗1 H, m), 7.04-7.05 (2/3∗1 H, m), 7.13-7.17 (2/3∗1 H, m), 7.27-7.41 (10/3∗1 H, m), 7.60-7.63 (3/3∗1 H, m), 7.81 (1/3∗1 H, s), 7.94 (2/3∗1 H, s), 8.02 (1/3∗1 H, s). 8.15 (2/3∗1 H, s), 8.24-8.25 (3/3∗1 H, m). |
| 9-2164 | *δ* 2.18-2.19 (3H, m), 3.48 (3H, s), 7.21-7.25 (4H, m), 7.32-7.40 (4H, m), 7.92 (1H, s), 8.13 (1H, s). |
| 9-2322 | *δ* 2.71-2.72 (3H, m), 3.47 (3H, s), 6.95-6.96 (1H, m), 7.11-7.16 (3H, m), 7.38-7.39 (2H, m), 7.95 (1H, s), 8.02 (1H, s), 8.34 (1H, s). |
| 11-777 | *δ* 4.88 (2H, s), 7.20-7.21 (1H, m), 7.36 (1H, t, J =7.8Hz), 7.84-7.87 (1H, m), 7.92 (1H, s), 8.14-8.17 (2H, m), 8.36 (1H, broad-s). |
| 11-835 | *δ* 4.88 (2H, s), 7.22-7.23 (1H, m), 7.36 (1H, t, J =7.8Hz), 7.85-7.88 (1H, m), 7.95 (1H, s), 8.19 (1H, d, J =13.1 Hz), 8.36-8.37 (2H, m). |

**Table 52(19)**

| compound number | ¹H-NMR (CDCl₃,ppm) |
|---|---|
| 12-777 | *δ* 3.39 (3H, s), 4.74 (2H, broad-s), 7.37 (1H, t, J =7.8Hz), 7.52-7.56 (1H, m), 7.92 (1H, s), 8.11-8.14 (2H, m), 8.25 (1H, d, J =14.1 Hz). |
| 12-835 | *δ* 3.40 (3H, s), 4.74 (2H, broad-s), 7.37 (1H, t, J =7.8Hz), 7.55-7.58 (1H, m), 7.95 (1H, s), 8.12-8.13 (1H, m), 8.28-8.31 (1H, m), 8.36 (1H, s). |
| 12-864 | *δ* 3.40 (3H, s), 4.74 (2H, broad-s), 7.37 (1H, t, J =7.8Hz), 7.52-7.58 (1H, m), 7.93 (1H, s), 8.12-8.15 (1H, m), 8.28-8.34 (2H, m). |
| 17-1103 | *δ* 3.65 (3H, s), 7.28-7.41 (6H, m), 7.77 (1H, t, J =7.8Hz), 7.91 (1H, s), 8.00-8.02 (1H, m), 8.14 (1H, s), 9.39 (1H, s). |
| 22-435 | *δ* 3.63 (3H, s), 4.90 (2H, s), 7.92-7.97 (2H, m), 8.08-8.15(3H, m), 9.72(1 H, s). |
| 27-627 | *δ* 3.72 (3H, s), 7.51-7.60 (5H, m), 7.90 (1H, s), 8.05 (1H, s), 8.14 (1H, s), 9.11 (1H, s). |
| 27-628 | *δ* 3.67 (3H, s), 6.99-7.24 (1H, m), 7.29-7.35 (1H, m), 7.52-7.59 (2H, m), 7.90 (1H, s), 8.06 (1H, s), 8.14 (1H, s), 9.08 (1H, s). |
| 27-663 | *δ* 3.63 (3H, s), 7.44-7.47 (1H, m), 7.80 (1H, dd, J =2.0,7.8Hz), 7.90 (1H, s), 8.10 (1H, s), 8.14 (1H, s), 8.58-8.60 (1H, m), 9.04 (1H, s). |
| 32-247 | *δ* 3.77-3.78 (3H, m), 4.96 (2H, s), 7.90 (1H, s), 8.00 (1H, s), 8.14 (1H, s), 9.02 (1H, s). |

The pest control agent including the compound as defined in claim 1 as an active ingredient can effectively control, at a low concentration thereof, any pests such as insects including various agricultural pests damaging agricultural/horticultural crops, trees, and the like, insanitary pests adversely affecting the living environment of humans such as houses and the like in various manners, stored grain pests damaging grain and the like stored in a warehouse, wood-eating pests damaging wood such as buildings and the like, and mites, crustaceans, molluscs, and nematodes which propagate and cause damage in a manner similar to that in the case of the insects.

Specific examples of the insects, the mites, the crustaceans, molluscs and nematodes which can be controlled using the compound as defined in claim 1 include lepidopteran insects such as *Adoxophyes honmai, Adoxophyes orana fasciata, Archips breviplicanus, Grapholita inopinata, Archips fuscocupreanus, Grapholita molesta, Choristoneura magnanima, Leguminivora glycinivorella, Olethreutes mori, Caloptilia zachrysa, Argyresthia conjugella, Spulerrina astaurota, Matsumuraeses phaseoli, Pandemis heparana, Bucculatrix pyrivorella, Lyonetia clerkella, Carposina niponensis, Lyonetia prunifoliella malinella, Caloptilia theivora, Phyllonorycter ringoniella, Phyllocnistis citrella, Acrolepiopsis sapporensis, Acrolepiopsis suzukiella, Plutella xylostella, Stathmopoda masinissa, Helcystogramma triannulella, Pectinophora gossypiella, Carposina sasakii, Chilo suppressalis, Cnaphalocrocis medinalis, Ephestia elutella, Conogethes punctiferalis, Diaphania indica, Etiella zinckenella, Glyphodes pyloalis, Scirpophaga incertulas, Hellula undalis, Ostrinia furnacalis, Ostrinia scapulalis, Parapediasia teterrella, Parnara guttata, Pieris brassicae, Pieris rapae crucivora, Papilio xuthus, Ascotis selenaria, Pseudoplusia includens, Euproctis pseudoconspersa, Lymantria dispar, Orgyia thyellina, Hyphantria cunea, Lemyra imparilis, Adris tyrannus, Aedia leucomelas, Agrotis ipsilon, Agrotis segetum, Autographa nigrisigna, Ctenoplusia agnata, Cydia pomonella, Helicoverpa armigera, Helicoverpa assulta, Helicoverpa zea, Heliothis virescens, Ostrinia nubilalis, Mamestra brassicae, Mythimna separata, Sesamia inferens, Naranga aenescens, Spodoptera eridania, Spodoptera exigua, Spodoptera frugiperda, Spodoptera littoralis, Spodoptera litura, Spodoptera depravata, Trichoplusia ni, Endopiza viteana, Manduca quinquemaculata, Manduca sexta,* and the like;
Hemipteran insects such as *Arboridia apicalis, Balclutha saltuella, Epiacanthus stramineus, Empoasca fabae, Empoasca nipponica, Empoasca onukii, Empoasca sakaii, Macrosteles striifrons, Nephotettix cincticeps, Pseudatomoscelis seriatus, Laodelphax striatella, Nilaparvata lugens, Sogatella furcifera, Diaphorina citri, Psylla pyrisuga, Aleurocanthus spiniferus, Bemisia argentifolii, Bemisia tabaci, Dialeurodes citri, Trialeurodes vaporariorum, Aleurolobus taonabae, Viteus vitifolii, Lipaphis erysimi, Aphis gossypii, Aphis spiraecola, Myzus persicae, Toxoptera aurantii, Drosicha corpulenta, Icerya purchasi, Phenacoccus solani, Pulvinaria aurantii, Planococcus citri, Pseudaonidia duplex, Planococcus kraunhiae, Pseudococcus comstocki, Comstockaspis perniciosa, Ceroplastes ceriferus, Ceroplastes rubens, Aonidiella aurantii, Fiorinia theae, Pseudaonidia paeoniae, Pseudaulacaspis pentagona, Pseudaulacaspis prunicola, Unaspis euonymi, Unaspis yanonensis, Cimex lectularius, Dolycoris baccarum, Eurydema rugosum, Eysarcoris aeneus, Eysarcoris lewisi, Eysarcoris ventralis, Glaucias subpunctatus, Halyomorpha halys, Nezara antennata, Nezara viridula, Piezodorus hybneri, Plautia crossota, Scotinophora lurida, Cletus punctiger, Leptocorisa chinensis, Riptortus clavatus, Rhopalus maculatus, Cavelerius saccharivorus, Togo hemipterus, Dysdercus cingulatus, Stephanitis pyrioides, Halticus insularis, Lygus lineolaris, Stenodema sibiricum, Stenotus rubrovittatus, Trigonotylus caelestialium,* and the like;
Coleopteran insects such as *Anomala cuprea, Anomala rufocuprea, Gametis jucunda, Heptophylla picea, Popillia japonica, Lepinotarsa decemlineata, Epilachna varivestis, Melanotus fortnumi, Melanotus tamsuyensis, Lasioderma serricorne, Lyctus brunneus, Tomicus piniperda, Rhizopertha dominica, Epuraea domina, Epilachna varivestis, Epilachna vigintioctopunctata, Tenebrio molitor, Tribolium castaneum, Anoplophora malasiaca, Monochamus alternatus, Psacothea hilaris, Xylotrechus pyrrhoderus, Callosobruchus chinensis, Aulacophora femoralis, Oulema oryzae, Chaetocnema concinna, Diabrotica undecimpunctata, Diabrotica virgifera, Diabrotica barberi, Phyllotreta striolata, Psylliodes angusticollis, Rhynchites heros, Cylas formicarius, Anthonomus grandis, Echinocnemus squameus, Euscepes postfasciatus, Hypera postica, Lissohoptrus oryzophilus, Otiorhynchus sulcatus, Sitophilus granarius, Sitophilus zeamais, Sphenophorus venatus vestitus, Paederus fuscipes,* and the like;
Thysanopteran insects such as *Frankliniella intonsa, Thrips flavus, Frankliniella occidentalis, Heliothrips haemorrhoidalis, Scirtothrips dorsalis, Thrips palmi, Thrips tabaci, Ponticulothrips diospyrosi,* and the like;
Dipterous insects such as *Asphondylia yushimai, Sitodiplosis mosellana, Bactrocera cucurbitae, Bactrocera dorsalis, Ceratitis capitata, Hydrellia griseola, Drosophila suzukii, Agromyza oryzae, Chromatomyia horticola, Liriomyza bryoniae, Liriomyza chinensis, Liriomyza sativae, Liriomyza trifolii, Delia platura, Delia antiqua, Pegomya cunicularia, Rhagoletis pomonella, Mayetiola destructor, Musca domestica, Stomoxys calcitrans, Melophagus ovinus, Hypoderma bovis, Hypoderma lineatum, Oestrus ovis, Glossina palpalis, Glossina morsitans, Prosimulium yezoensis, Tabanus trigonus, Telmatoscopus albipunctatus, Leptoconops nipponensis, Culex pipiens pallens, Aedes aegypti, Aedes albopictus, Anopheles hyracanus sinensis,* and the like;
Hymenopteran insects such as *Apethymus kuri, Athalia rosae, Arge pagana, Neodiprion sertifer, Dryocosmus kuriphilus, Eciton burchellii, Eciton schmitti, Camponotus japonicus, Vespa mandarinia, Myrmecia spp., Solenopsis spp., Monomorium pharaonis,* and the like;
Orthopteran insects such as *Teleogryllus emma, Gryllotalpa orientalis, Locusta migratoria, Oxya yezoensis, Schistocerca gregaria,* and the like;
Collembolan insects such as *Onychiurus folsomi, Onychiurus sibiricus, Bourletiella hortensis,* and the like;
Dictyopteran insects such as *Periplaneta fuliginosa, Periplaneta japonica, Blattella germanica, Periplaneta Americana,* and the like;
Isopterous insects such as *Coptotermes formosanus, Reticulitermes speratus, Odontotermes formosanus,* and the like; Siphonaptera insects such as *Ctenocephalides felis, Ctenocephalides canis, Echidnophaga gallinacea, Pulex irritans, Xenopsylla cheopis,* and the like;
Mallophaga insects such as *Menacanthus stramineus, Bovicola bovis,* and the like;
Anoplura insects such as *Haematopinus eurysternus, Haematopinus suis, Linognathus vituli, Solenopotes capillatus,* and the like;
Tarsonemidae such as *Phytonemus pallidus, Polyphagotarsonemus latus, Tarsonemus bilobatus,* and the like;
Eupodidae such as *Penthaleus erythrocephalus, Penthaleus major,* and the like;
Tetranychidae such as *Oligonychus shinkajii, Panonychus citri, Panonychus mori, Panonychus ulmi, Tetranychus kanzawai, Tetranychus urticae,* and the like;
Eriophyidae such as *Acaphylla theavagrans, Aceria tulipae, Aculops lycopersici, Aculops pelekassi, Aculus schlechtendali, Eriophyes chibaensis, Phyllocoptruta oleivora,* and the like;
Acaridae such as *Rhizoglyphus robini, Tyrophagus putrescentiae, Tyrophagus similis,* and the like;
Varroidae such as *Varroa jacobsoni,* and the like;
Ixodidae such as *Boophilus microplus, Rhipicephalus sanguineus, Haemaphysalis longicornis, Haemaphysalis flava, Haemaphysalis campanulata, Ixodes ovatus, Ixodes persulcatus, Amblyomma spp., Dermacentor spp.,* and the like;
Cheyletidae such as *Cheyletiella yasguri, Cheyletiella blakei,* and the like;
Demodicidae such as *Demodex canis, Demodex cati,* and the like;
Psoroptidae such as *Psoroptes ovis,* and the like;
Sarcoptidae such as *Sarcoptes scabiei, Notoedres cati, Knemidocoptes spp.,* and the like;
Crustacea such as *Armadillidium vulgare,* and the like;
Gastropoda such as *Pomacea canaliculata, Achatina fulica, Meghimatium bilineatum, Limax Valentiana, Acusta despecta sieboldiana, Euhadra peliomphala,* and the like; and
Nematoda such as *Pratylenchus coffeae, Pratylenchus penetrans, vulnus, Globodera rostochiensis, Heterodera glycines, Meloidogyne hapla, Meloidogyne incognita, Aphelenchoides besseyi, Bursaphelenchus xylophilus,* and the like, but the present invention is not limited thereto.

The pest control agent including the compound as defined in claim 1 as an active ingredient has a significant control effect against the above-described harmful crops which damage lowland crops, upland crops, fruit trees, vegetables, and other crops and ornamental flowers, and therefore, the effect as a pest control agent of the present invention can be obtained by treating the paddy field water, plant stems and leaves, or soil of the crops of lowland, upland, fruit trees, vegetables, other crops, ornamental flowers, and the like during the seasons in which the appearance of such pests is expected, or before or at the point when the pest appearance is observed.

The pest control agent including the compound as defined in claim 1 as an active ingredient has a significant control effect against stored grain pests and the like generated during storage of the harvest. That is, the pest control agent having the compound as defined in claim 1 as an active ingredient may be subjected to a treatment after the harvest (post-harvest) such as spray-spreading, coating, dipping, dressing, fumigation/smoking, pressurized injection, and the like with respect to the harvest or the place for storage of the harvest.

Further, the pest control agent including the compound as defined in claim 1 as an active ingredient can be applied to plant seeds to prevent the damage caused by pests generated in the plants after seeding. That is, the pest control agent having the compound as defined in claim 1 as an active ingredient may be subjected to a treatment such as spray-spreading, dipping, dressing, and the like on the plant seeds in an effective amount for controlling the pests as it is, as an adequate dilution with water or the like, or as a suspension to bring the compound as defined in claim 1 into contact with the plant seeds.

The plant seeds refer to those used for breeding in agriculture by storing the nutrients for seedling germination, and examples thereof include seeds such as corn, soybeans, red beans, cotton, rice, sugar beet, wheat, barley, sunflower, tomato, cucumber, eggplant, spinach, sting beans, squash, sugarcane, tobacco, pimento, canola, and the like, seed tubers such as taro, potato, sweet potato, konjac, and the like, bulbs such as edible lily, tulips, and the like, and seed balls such as rakkyo and the like.

The pest control agent having the compound as defined in claim 1 as an active ingredient has a significant control effect against insanitary pests such as Diptera pests (*Culex pipiens pallens, Culex pipiens, Chironomidae,* houseflies, sandflies, horsefly, and the like) Dictyoptera pests (*Blattella germanica, Periplaneta fuliginosa, Periplaneta americana,* and the like), and other pests.

The pest control agent having the compound as defined in claim 1 as an active ingredient has a significant control effect against wood-feeding pests such as termites, *Lyctus brunneus, Rhizopertha dominica, Anobiidae, Cerambycidae,* and the like, thus, the above-described wood-feeding pests can be controlled by treatment of wood, soil, buildings, and the like with the pest control agent.

Since the compound as defined in claim 1 exhibits a control effect against various pests and also exhibits an effect of protecting useful crops and an excellent control effect as a pesticide or a miticide at a low dose, it has an effect of contributing to reduction in an environmental impact. In addition, the compound as defined in claim 1 also exerts an excellent control effect when used in combination with other agricultural/horticultural pesticides, miticides, nematocides, fungicides, herbicides, plant growth regulators, biological agricultural chemicals, or the like.

For the use of the compound as defined in claim 1, the compound can be put to practical use as a preparation in any form such as a soluble concentrate, an emulsifiable concentrate, a wettable powder, a water-soluble powder, a water dispersible granule, a water-soluble granule, a suspension concentrate, a concentrated emulsion, a suspoemulsion, a microemulsion, a dustable powder, a granule, a tablet, and an emulsifiable gel, typically by mixing the compound with a suitable solid carrier or liquid carrier, further if desired by adding to the resultant mixture, a surfactant, a penetrant, a spreader, a thickener, an antifreezing agent, a binder, an anticaking agent, a disintegrant, a defoaming agent, an antiseptic or a stabilizer. In addition, from the labor-saving and safety-enhancing viewpoints, the compound can be put to use by encapsulating the above preparation in any form in a water-soluble packaging material such as a water-soluble capsule and a bag of water-soluble film.

The inert carrier which can be used in the present invention may be solids or liquids, and examples thereof include soybean powders, grain powders, wood powders, bark powders, sawdust powders, tobacco stem powders, walnut shell powders, brans, cellulose powders, residues from plant extraction, synthetic polymers such as pulverized synthetic resins, clays (for example, kaolin, bentonite, acidic white clay), talcs (for examples, talc, pyrophyllite, etc.), silica (for examples, diatomaceous earth, sand, mica, white carbon [hydrous silica powders, synthetic high dispersity silicates called hydrous silicate, there are also products containing calcium silicate as a main component]), activated carbon, sulfur powder, pumice, calcined diatomaceous powders, pulverized bricks, fly ash, sand, inorganic mineral powders such as calcium carbonate, calcium phosphate, and the like, chemical fertilizers such as ammonium sulfate, ammonium phosphate, ammonium nitrate, urea, ammonium chloride, and the like, a compost, and others, which are used alone or as a mixture of two or more kinds thereof.

Materials which can be used as the liquid inert carrier are selected from those having the function as solvent, as well as those capable of dispersing the active ingredient compound with the aid of an adjuvant even if the inert carrier does not have a function as a solvent. Representative examples thereof include the carriers listed below: water, alcohols (for example, methanol, ethanol, isopropanol, butanol, ethylene glycol, and the like), ketones (for example, acetone, methyl ethyl ketone, methyl isobutyl ketone, diisobutylketone, cyclohexanone, and the like), ethers (for example, diethyl ether, dioxane, cellosolve, diisopropyl ether, tetrahydrofuran, and the like), aliphatic hydrocarbons (for example, kerosene, mineral oil, and the like), aromatic hydrocarbons (for example, benzene, toluene, xylene, solvent naphtha, alkyl naphthalene, and the like), halogenated hydrocarbons (for example, dichloromethane, chloroform, tetrachlorocarbon, chlorobenzene, and the like), esters (for example, ethyl acetate, butyl acetate, ethyl propionate, diisobutyl phthalate, dibutyl phthalate, dioctyl phthalate, and the like), amides (for example, dimethyl formamide, diethyl formamide, dimethyl acetamide, and the like), and nitriles (for example, acetonitrile, and the like), which are used alone or as mixtures of two or more kinds thereof.

These solid and liquid carriers may be used alone or in a combination of two or more kinds thereof.

Examples of the surfactant include nonionic surfactants such as polyoxyethylene alkyl ethers, polyoxyethylene alkyl (mono- or di-)phenyl ethers, polyoxyethylene (mono-, di-, or tri-)styryl phenyl ethers, polyoxyethylene-polyoxypropylene block copolymers, polyoxyethylene aliphatic acid (mono- or di-) esters, sorbitan aliphatic acid esters, polyoxyethylene sorbitan aliphatic acid esters, castor oil ethylene-oxide adducts, acetylene glycol, acetylene alcohols, acetylene glycol ethylene-oxide adducts, acetylene alcohol ethylene-oxide adducts, alkylglucosides, and the like; anionic surfactants such as alkyl sulfate ester salts, alkylbenzene sulfonates, lignin sulfonates, alkyl sulfosuccinates, naphthalene sulfonates, alkylnaphthalene sulfonates, salts of naphthalene sulfonate formalin condensate, salts of alkylnaphthalene sulfonate formalin condensate, polyoxyethylenealkylether sulfate or phosphate esters salts, polyoxyethylene (mono- or di-) alkylphenyl ether sulfate or phosphate esters, polyoxyethylene (mono-, di-, or tri-) styrylphenyl ether sulfate or phosphate esters, polycarboxylic acid salts (such as polyacrylic acid salts, polymaleic acid salts, maleic acid-olefin copolymers, and the like), polystyrene sulfonates, and the like; cationic surfactants such as alkylamine salts, alkyl quaternary ammonium salts, and the like; amphoteric surfactants such as amino acid-type surfactants, betaine-type surfactants, and the like; silicone-based surfactants; and fluorinated surfactants.

The content of these surfactants is not particularly limited, but it is preferably in the range of usually 0.05 part by weight to 20 parts by weight, with respect to 100 parts by weight of the preparation of the present invention. In addition, these surfactants may be used alone or in combination of two or more kinds thereof.

In order to control various pests, an amount effective for blight control can be applied as it is or as an adequate dilution with water or the like, or as a suspension, to the crops on which appearance of the corresponding pests is expected or to places where such occurrence is not preferable. The amount of use depends on various factors such as, for example, the purpose, the pest to be controlled, the state of plant growth, trends in pest appearance, climate, environmental conditions, Formulation, method of use, place of use, timing of use, and the like, but it is preferable to use the active ingredient in the concentration of 0.0001 ppm to 5000 ppm, and preferably 0.01 ppm to 1000 ppm. The dose that can be used per 10 a is generally in the range of 1 g to 300 g of the active ingredient.

The amount of the active ingredient of the compound as defined in claim 1 is usually 0.1% by weight to 20% by weight for powders, 5% by weight to 50% by weight for emulsifiable concentrates, 3% by weight to 90% by weight for wettable powders, 0.1% by weight to 20% by weight for granules, 5% by weight to 90% by weight for flowable Formulations, or 3% by weight to 90% by weight for water dispersible granules. The amount of the carrier in each form is usually 60% by weight to 99.9% by weight for powders, 40% by weight to 95% by weight for emulsifiable concentrates, 10% by weight to 90% by weight for wettable powders, 80% by weight to 99.9% by weight for granules, 10% by weight to 95% by weight for flowable Formulations, or 10% by weight to 90% by weight for water dispersible granules. Further, the amount of the adjuvant is usually 0.1% by weight to 20% by weight for powders, 1% by weight to 20% by weight for emulsifiable concentrates, 0.1% by weight to 20% by weight for wettable powders, 0.1% by weight to 20% by weight for granules, 0.1% by weight to 20% by weight for flowable Formulations, and 0.1% by weight to 20% by weight for water dispersible granules.

When the compound as defined in claim 1 is used as an agricultural chemical, it may be used, if required, as a mixture with other herbicides, various pesticides, miticides, nematocides, fungicides, plant growth regulators, synergists, fertilizers, soil conditioners, or the like to be applied during the preparation or the dusting.

### Examples

Representative Examples of the present invention will be described with reference to the following Examples, but the present invention is not limited thereto. In the present Examples, DMF means N,N-dimethyl formamide, THF means tetrahydrofuran, IPE means isopropyl ether, DMI means 1,3-dimethyl-2-imidazolidinone, and DMSO means dimethylsulfoxide.

Furthermore, "%" is based on mass unless specified otherwise.

### <Example 1>

### Preparation of N-(2-bromo-4-(perfluoropropan-2-yl)-6-(trifluoromethyl)phenyl)-2-fluoro-3-(N-methylbenzamide)benzamide (Compound No. 7-1574).

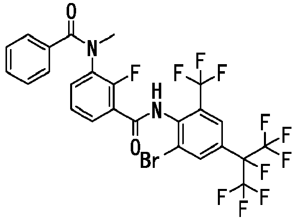

### <1-1>

### Preparation of 4-(perfluoropropan-2-yl)-2-(trifluoromethyl)aniline

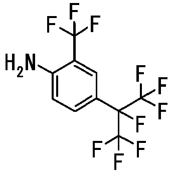

100 g (0.608 mol) of 2-(trifluoromethyl)aniline, 131 g (0.639 mol) of 85% sodium hydrosulfite, and 20.9 g (0.0608 mol) of tetrabutylammonium hydrogen sulfate were charged to a mixed solution of 1500 ml of ethyl acetate and 1500 ml of water, and 53.9 g (0.639 mol) of sodium hydrogen carbonate was added thereto. 198 g (0.669 mol) of heptafluoroisopropyl iodide was added dropwise thereto at room temperature, followed by stirring at room temperature for 6 hours. After the liquid separation, the solvent of the organic layer was evaporated under reduced pressure, and 500 ml of ethyl acetate was charged thereto. 160 g (0.608 mol) of a 4 M hydrogen chloride/ethyl acetate solution was added dropwise thereto, followed by stirring at room temperature for 30 minutes, and then stirring at 5°C for 1 hour. After the filtration, the filtrate was washed with water and a saturated aqueous sodium hydrogen carbonate in this order, and then dried over anhydrous magnesium sulfate, and the solvent was evaporated under reduced pressure. The obtained residue was purified by silica gel column chromatography (eluent solvent; hexane:ethyl acetate=10:1) to prepare 60.0 g (yield 30%) of a target compound.

¹H-NMR (CDCl₃, ppm) δ 4.49 (2H, broad-s), 6.81 (1H, d, J=8.3 Hz), 7.48 (1H, d, J=8.3 Hz), 7.64 (1H, s).

### <1-2>

### Preparation of 2-bromo-4-(perfluoropropan-2-yl)-6-(trifluoromethyl)aniline

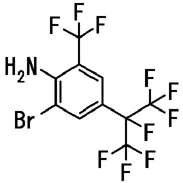

100 g (0.273 mol) of 4-(perfluoropropan-2-yl)-2-(trifluoromethyl)aniline was charged to 500 ml of DMF, and 52.1 g (0.287 mol) of N-bromosuccinimide was charged in separate portions thereto over 30 minutes. After stirring at 60°C for 2 hours, and then cooling to room temperature, the mixture was discharged to 2000 ml of water. The mixture was extracted with ethyl acetate, then washed with saturated brine, and dried over anhydrous magnesium sulfate. The solvent was evaporated under reduced pressure and the obtained residue was purified by silica gel column chromatography (eluent solvent; hexane:ethyl acetate=20:1) to prepare 89.0 g (yield 80%) of a target compound.

¹H-NMR (CDCl₃, ppm) δ 5.03 (2H, broad-s), 7.61 (1H, s), 7.79 (1H, s).

### <1-3>

### Preparation of N-(2-bromo-4-(perfluoropropan-2-yl)-6-(trifluoromethyl)phenyl)-2-chloro-3-nitrobenzamide

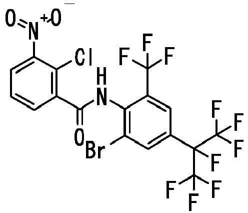

3.60 g (8.82 mmol) of 2-bromo-4-(perfluoropropan-2-yl)-6-(trifluoromethyl)aniline was charged to 20 ml of anhydrous THF, and cooled to -70°C under a nitrogen atmosphere. 4.85 ml (9.70 mmol) of a 2.0 M lithium diisopropyl amide hexane solution was added dropwise thereto, then 2.34 g (10.7 mmol) of acid chloride which was prepared from 2-chloro-3-nitrobenzoic acid and thionyl chloride was dissolved in 5 ml of anhydrous THF, and was added dropwise thereto, followed by stirring at -70°C for 30 minutes and then stirring at room temperature for 30 minutes. The mixture was discharged to an aqueous ammonium chloride solution, then extracted with ethyl acetate, and dried over anhydrous magnesium sulfate. The solvent was evaporated under reduced pressure and the obtained residue was purified by silica gel column chromatography (eluent solvent; hexane:ethyl acetate=10:1→8:2→3:1) to prepare 1.76 g (yield: 34%) of a target compound.

¹H-NMR (CDCl₃, ppm) δ 7.61 (1H, t, J=7.8 Hz), 7.67 (1H, broad-s), 7.93-7.97 (3H, m), 8.18 (1H, broad-s).

### <1-4>

### Preparation of N-(2-bromo-4-(perfluoropropan-2-yl)-6-(trifluoromethyl)phenyl)-2-fluoro-3-nitrobenzamide

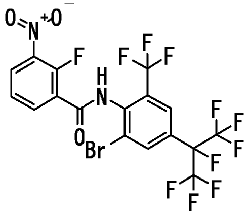

To a solution of N-(2-bromo-4-(perfluoropropan-2-yl)-6-(trifluoromethyl)phenyl)-2-chloro-3-nitrobenzamide 4.89 g (8.27 mmol) in 50 ml of anhydrous DMF was added 2.40 g (41.3 mmol) of potassium fluoride (spray-dried product) under a flow of nitrogen, followed by stirring at 130°C for 10 hours. Liquid separation was carried out by adding ethyl acetate, hexane, and water to the reaction mixture, and then the organic layer was washed with water and saturated brine, and dried over anhydrous magnesium sulfate. The solvent was evaporated under reduced pressure and the obtained residue was purified by silica gel column chromatography (eluent solvent; hexane:ethyl acetate=10:1) to prepare 0.940 g (yield 20%) of a target compound.

¹H-NMR (CDCl₃, ppm) δ 7.53 (1H, t, J=7.3 Hz), 7.93 (1H, broad-s), 8.17-8.18 (2H, m), 8.28-8.32 (1H, m), 8.44-8.48 (1H, m).

### <1-5>

### Preparation of 3-amino-N-(2-bromo-4-(perfluoropropan-2-yl)-6-(trifluoromethyl)phenyl)-2-fluorobenzamide

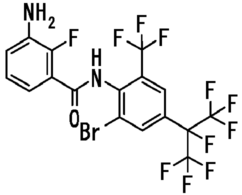

0.940 g (1.63 mmol) of N-(2-bromo-4-(perfluoropropan-2-yl)-6-(trifluoromethyl)phenyl)-2-fluoro-3-nitrobenzamide and 0.960 g (5.05 mmol) of stannous chloride (anhydrous) were added to 10 ml of ethanol, and 1.02 ml (9.78 mmol) of concentrated hydrochloric acid was added thereto, followed by stirring at 60°C for 4 hours. The reaction mixture was adjusted to pH 10 by the addition of an aqueous sodium hydroxide solution, and the precipitated insolubles were removed by filtration using Celite. The filtrate on Celite was washed with ethyl acetate. The filtrate was extracted with ethyl acetate, and the organic layer was washed with a 20% aqueous sodium hydroxide solution and saturated brine, and then dried over anhydrous magnesium sulfate. The solvent was evaporated under reduced pressure and the obtained residue was purified by silica gel column chromatography (eluent solvent; hexane:ethyl acetate=4:1) to prepare 0.930 g (yield 99%) of a target compound.

¹H-NMR (CDCl₃, ppm) δ 3.93 (2H, broad-s), 6.99-7.04 (1H, m), 7.11 (1H, t, J=7.8 Hz), 7.47-7.49 (1H, m), 7.91 (1H, s), 8.14 (1H, s), 8.28 (1H, d, J=14.6 Hz).

### <1-6>

### Preparation of N-(2-bromo-4-(perfluoropropan-2-yl)-6-(trifluoromethyl)phenyl)-2-fluoro-3-(methylamino)benzamide

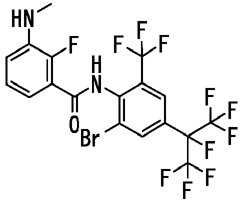

0.930 g (1. 71 mmol) of 3-amino-N-(2-bromo-4-(perfluoropropan-2-yl)-6-(trifluoromethyl)phenyl)-2-fluorobenzamide was added to 5 ml of concentrated sulfuric acid, and 10 ml of a 37% aqueous formaldehyde solution was charged dropwise thereto at 40°C. The reaction mixture was poured into ice-water, adjusted to pH 10 using an aqueous sodium hydroxide solution, and extracted with the addition of ethyl acetate. The organic layer was washed with a 20% aqueous sodium hydroxide solution and saturated brine, and then dried over anhydrous magnesium sulfate. The solvent was evaporated under reduced pressure and the obtained residue was purified by silica gel column chromatography (eluent solvent; hexane:ethyl acetate=8:1) to prepare 0.690 g (yield 72%) of a target compound.

¹H-NMR (CDCl₃, ppm) δ 2.94 (3H, s), 4.14 (1H, broad-s), 6.88-6.93 (1H, m), 7.18 (1H, t, J=7.8 Hz), 7.37-7.41 (1H, m), 7.90 (1H, s), 8.13 (1H, s), 8.27 (1H, d, J=14.6 Hz).

### [1-7] Preparation of N-(2-bromo-4-(perfluoropropan-2-yl)-6-(trifluoromethyl)phenyl)-2-fluoro-3-(N-methylbenzamide)benzamide (Compound No. 7-1574)

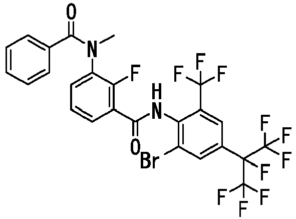

To a solution of 1.54 g (2.75 mmol) of N-(2-bromo-4-(perfluoropropan-2-yl)-6-(trifluoromethyl)phenyl)-2-fluoro-3-(methylamino)benzamide and 0.330 g (4.13 mmol) of pyridine in 5 ml of THF was added 0.460 g (3.30 mmol) of benzoyl chloride, followed by stirring at 60°C for 5 hours. To the reaction mixture were added water and ethyl acetate, and the organic layer was washed with 1 M hydrochloric acid, a saturated aqueous sodium hydrogen carbonate solution, and saturated brine, and then dried over anhydrous magnesium sulfate. The solvent was evaporated under reduced pressure and the obtained residue was purified by silica gel column chromatography (eluent solvent; hexane:ethyl acetate=8:1), and the obtained solid was washed with IPE to prepare 1.45 g (yield 80%) of a target compound.

¹H-NMR (CDCl₃, ppm) δ 3.50 (3H, s), 6.99-7.33 (6H, m), 7.43-7.45 (1H, m), 7.90 (1H, s), 7.97-8.06 (2H, m), 8.13 (1H, s).

### <Example 2>

### Preparation of 2-chloro-N-(3-(2,6-dibromo-4-(perfluoropropan-2-yl)phenylcarbamoyl)-2-fluorophenyl)nicotinamide (Compound No. 6-1140)

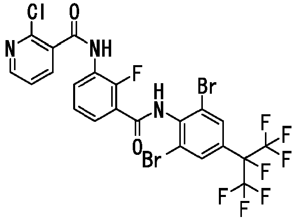

### <2-1>

### Preparation of 4-(perfluoropropan-2-yl)aniline

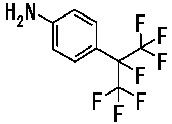

100 g (1.02 mol) of aniline, 230 g (1. 12 mol) of 85% sodium hydrosulfite, and 35.1 g (0.100 mol) of tetrabutylammonium hydrogen sulfate were charged to a mixed solution of 1500 ml of t-butyl methyl ether and 1500 ml of water, and 94.7 g (1.12 mol) of sodium hydrogen carbonate was added thereto. 350 g (1.12 mol) of heptafluoroisopropyl iodide was added dropwise thereto at room temperature, followed by stirring at room temperature for 6 hours. After the liquid separation, the organic layer was washed with 1 M hydrochloric acid, water, and a saturated aqueous sodium hydrogen carbonate solution, and then dried over anhydrous sodium sulfate. The solvent was evaporated under reduced pressure, and 500 ml of ethyl acetate was charged thereto. 255 g (1.02 mol) of a 4 M hydrogen chloride/ethyl acetate solution was added dropwise thereto, followed by stirring at room temperature for 30 minutes and at 5°C for 1 hour. The precipitated solid was separated by filtration, and the solid was charged to 1000 ml of ethyl acetate, adjusted to pH 8 to 9 by the addition of 1000 ml of a saturated aqueous sodium hydrogen carbonate solution at 20°C or lower, and subjected to liquid separation. The organic layer was dried over anhydrous sodium sulfate, and then the solvent was evaporated under reduced pressure to prepare 188 g (yield 71%) of a target compound.

¹H-NMR (CDCl₃, ppm) δ 3.92 (2H, broad-s), 6.69-6.74 (2H, m), 7.35 (2H, d, J=9.3 Hz).

### <2-2>

### Preparation of 2,6-dibromo-4-(perfluoropropan-2-yl)aniline

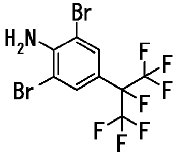

216 g (0.802 mol) of 4-(perfluoropropan-2-yl)aniline was charged to 863 ml of DMF, followed by cooling to 5°C. 285 g (1.60 mol) of N-bromosuccinimide was charged in separate portions thereto over 1 hour. The mixture was stirred at room temperature for 1 hour and then stirred at 37°C for 2 hours. The mixture was discharged to 2000 ml of water, extracted with 2000 ml of ethyl acetate, and washed with 1000 ml of saturated brine. After dried over anhydrous magnesium sulfate, the solvent was evaporated under reduced pressure. The obtained residue was purified by silica gel column chromatography (eluent solvent; hexane:ethyl acetate=20:1) to prepare 304 g (yield 90%) of a target compound.

¹H-NMR (CDCl₃, ppm) δ 4.88 (2H, broad-s), 7.59 (2H, s).

### <2-3>

### Preparation of 2-chloro-N-(2,6-dibromo-4-(perfluoropropan-2-yl)phenyl)-3-nitrobenzamide

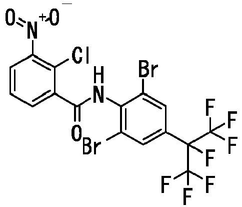

According to the method of 1-3 of Example 1, a target compound was prepared from 2,6-dibromo-4-(perfluoropropan-2-yl)aniline.

¹H-NMR (CDCl₃, ppm) δ 7.58 (1H, t, J=7.8 Hz), 7.66 (1H, broad-s), 7.90 (2H, s), 7.93 (1H, dd, J=1.5, 7.8 Hz), 7.98 (1H, d, J=7.8 Hz).

### <2-4>

### Preparation of N-(2,6-dibromo-4-(perfluoropropan-2-yl)phenyl)-2-fluoro-3-nitrobenzamide

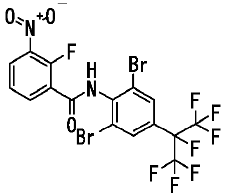

According to the method of 1-4 of Example 1, a target compound was prepared from 2-chloro-N-(2,6-dibromo-4-(perfluoropropan-2-yl)phenyl)-3-nitrobenzamide.

¹H-NMR (CDCl₃, ppm) δ 7.51-7.55 (1H, m), 7.90 (2H, s), 8.16 (1H, d, J=11.7 Hz), 8.27-8.31 (1H, m), 8.48 (1H, t, J=6.3 Hz).

### <2-5>

### Preparation of 3-amino-N-(2,6-dibromo-4-(perfluoropropan-2-yl)phenyl)-2-fluorobenzamide

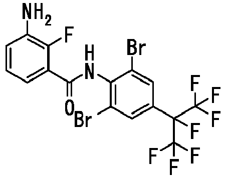

According to the method of 1-5 of Example 1, a target compound was prepared from N-(2,6-dibromo-4-(perfluoropropan-2-yl)phenyl)-2-fluoro-3-nitrobenzamide.

¹H-NMR (CDCl₃, ppm) δ 3.93 (2H, broad-s), 6.99-7.04 (1H, m), 7.11 (1H, t, J=7.8 Hz), 7.47-7.49 (1H, m), 7.91 (1H, s), 8.14 (1H, s), 8.28 (1H, d, J=14.6 Hz).

### <2-6>

### Preparation of 2-chloro-N-(3-(2,6-dibromo-4-(perfluoropropan-2-yl)phenylcarbamoyl)-2-fluorophenyl)nicotinamide (Compound No. 6-1140)

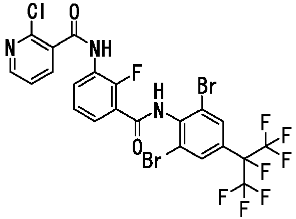

According to the method of 1-7 of Example 1, a target compound was prepared from 3-amino-N-(2,6-dibromo-4-(perfluoropropan-2-yl)phenyl)-2-fluorobenzamide and 2-chloronicotinic acid chloride.

¹H-NMR (CDCl₃, ppm) δ 7. 40-7. 49 (2H, m), 7.89 (2H, s), 7. 94-7. 96 (1H, m), 8. 13 (1H, d, J=12. 7 Hz), 8.32-8. 34 (1H, m), 8.57-8. 59 (1H, m), 8.67-8.71 (1H, m), 8.75 (1H, broad-s).

### <Example 3>

### Preparation of 3-benzamide-2-fluoro-N-(4-(perfluoropropan-2-yl)-2,6-bis(trifluoromethyl)phenyl)benzamide (Compound No. 6-2110)

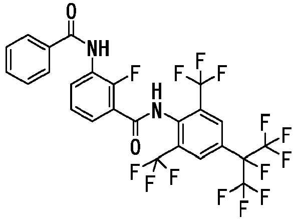

### <3-1>

### Preparation of 2,6-diiodo-4-(perfluoropropan-2-yl)aniline

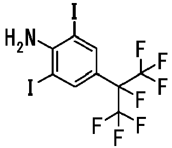

To a solution of 5.74 g (22.0 mmol) of 4-(perfluoropropan-2-yl)aniline obtained in 2-1 of Example 2 in 50 ml of ethanol was added 2.16 g (22.0 mmol) of concentrated sulfuric acid at 5°C. The reaction mixture was warmed to room temperature, and 10.0 g (44.0 mmol) of N-iodosuccinimide was added thereto, followed by stirring for 3 hours. The reaction mixture was neutralized with a saturated aqueous sodium hydrogen carbonate solution. The precipitated crystals were filtered, washed with water, and then dried to prepare 9.00 g (yield 80%) of a target compound.

¹H-NMR (CDCl₃, ppm) δ 4.95 (2H, broad-s), 7.79 (2H, s).

### <3-2>

### Preparation of 2-chloro-N-(2,6-diiodo-4-(perfluoropropan-2-yl)phenyl)-3-nitrobenzamide

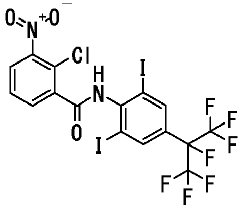

To a solution of 40.0 g (78.0 mmol) of 2,6-diiodo-4-(perfluoropropan-2-yl)aniline in 100 ml of DMI was added 20.6 g (94.0 mmol) of 2-chloro-3-nitrobenzoyl chloride, followed by stirring at 135°C for 3 hours. After cooling to room temperature, the reaction mixture was poured into 1000 ml of water. After extraction with the addition of 1000 ml of ethyl acetate, the organic layer was washed with water and then dried over anhydrous magnesium sulfate. The solvent was evaporated under reduced pressure and the obtained residue was washed with hexane to prepare 56.2 g (yield 99%) of a target compound.

¹H-NMR (CDCl₃, ppm) δ 7.58 (1H, t, J=8.3 Hz), 7.70 (1H, d, J=3.4 Hz), 7.93 (1H, dd, J=1.5, 6.3 Hz), 8.08-8.10 (1H, m), 8.13 (2H, s).

### <3-3>

### Preparation of N-(2,6-diiodo-4-(perfluoropropan-2-yl)phenyl)-2-fluoro-3-nitrobenzamide

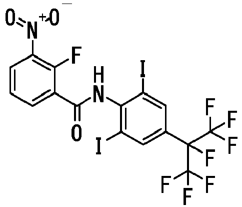

According to the method of 1-4 of Example 1, a target compound was prepared from 2-chloro-N-(2,6-diiodo-4-(perfluoropropan-2-yl)phenyl)-3-nitrobenzamide.

1H-NMR (CDCl₃, ppm) δ 7.52-7.55 (1H, m), 8.12-8.18 (3H, m), 8.29-8.32 (1H, m), 8.48-8.51 (1H, m).

### <3-4>

### Preparation of 3-amino-N-(2,6-diiodo-4-(perfluoropropan-2-yl)phenyl)-2-fluorobenzamide

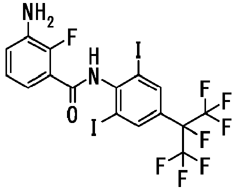

According to the method of 1-5 of Example 1, a target compound was prepared from N-(2,6-diiodo-4-(perfluoropropan-2-yl)phenyl)-2-fluoro-3-nitrobenzamide.

¹H-NMR (CDCl₃, ppm) δ 3.93 (2H, broad-s), 6.99-7.04 (1H, m), 7.08 (1H, t, J=7.8 Hz), 7.39-7.43 (1H, m), 8.10 (2H, s), 8.72 (1H, d, J=11.2 Hz).

### <3-5>

### Preparation of 3-benzamide-N-(2,6-diiodo-4-(perfluoropropan-2-yl)phenyl)-2-fluorobenzamide (Compound No. 6-1260)

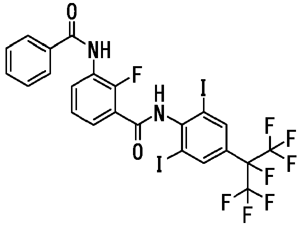

According to the method of 1-7 of Example 1, a target compound was prepared from 3-amino-N-(2,6-diiodo-4-(perfluoropropan-2-yl)phenyl)-2-fluorobenzamide.

¹H-NMR (CDCl₃, ppm) δ 7.39 (1H, t, J=7.8 Hz), 7.52-7.57(4H, m), 7.60-7.63 (2H, m), 7.93-7.94(4H, m), 8.70 (1H, t, J=6.3 Hz).

### <3-6>

### Preparation of 3-benzamide-2-fluoro-N-(4-(perfluoropropan-2-yl)-2,6-bis(trifluoromethyl)phenyl)benzamide (Compound No. 6-2110)

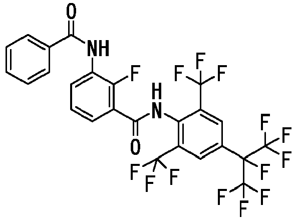

A solution of 1.95 g (2.59 mmol) of 3-benzamide-N-(2,6-diiodo-4-(perfluoropropan-2-yl)phenyl)-2-fluorobenzamide, 0.10 g (0.520 mmol) of copper iodide, and 1.24 g (6.48 mmol) of methyl fluorosulfonyl difluoroacetate in 50 ml of DMF was stirred at 100°C for 6 hours. The reaction mixture was cooled to room temperature, and then 10 ml of water and 100 ml of ethyl acetate were added thereto, followed by filtration through Celite. The organic layer of the liquid was washed with water and a saturated aqueous sodium hydrogen carbonate solution, and then dried over anhydrous magnesium sulfate. The solvent was evaporated under reduced pressure and the obtained residue was purified by silica gel column chromatography (eluent solvent; hexane:ethyl acetate=1:1). The obtained solid was washed with hexane to prepare 0.840 g (yield 51%) of a target compound.

¹H-NMR (CDCl₃, ppm) δ 7.36-7.40 (1H, m), 7.53-7.64 (3H, m), 7.84-7.97 (1H, m), 7.92-7.94 (2H, m), 8.04-8.07 (1H, m), 8.08-8.13 (1H, m), 8.20 (2H, s), 8.68-8.72 (1H, m).

### <Example 4>

### Preparation of N-(2,6-dibromo-4-(perfluorobutan-2-yl)phenyl)-2-fluoro-3-(N-methylbenzamide)benzamide (Compound No. 7-1182)

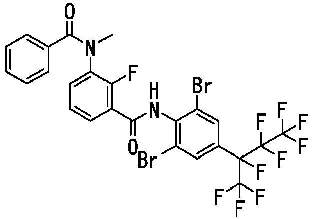

### <4-1>

### Preparation of 4-(perfluorobutan-2-yl)aniline

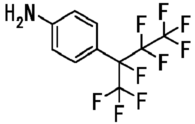

4.90 g (52.6 mmol) of aniline, 10.1 g (58.0 mmol) of 85% sodium hydrosulfite, and 1.90 g (5.77 mmol) of tetrabutylammonium hydrogen sulfate were charged to a mixed solution of 150 ml of t-butyl methyl ether and 150 ml of water using a light-shielded reaction vessel, and 4.84 g (57.6 mmol) of sodium hydrogen carbonate was added thereto. 20.0 g (57.8 mmol) of nonafluoro-s-butyliodide was added dropwise thereto at room temperature, followed by stirring at room temperature for 5 hours. The organic phase was collected by separation, washed with 2 mol/L of an aqueous hydrochloric acid solution twice, and then washed with saturated brine, an aqueous sodium hydrogen carbonate solution, and saturated brine. The organic layer was dried over anhydrous magnesium sulfate, and then the solvent was evaporated under reduced pressure to prepare 8.32 g (yield 51%) of a target compound.

¹H-NMR (CDCl₃, ppm) δ 3.92 (2H, broad-s), 6.72 (2H, d, J=8.8 Hz), 7.34 (2H, d, J=8.8 Hz).

### <4-2>

### Preparation of 2,6-dibromo-4-(perfluorobutan-2-yl)aniline

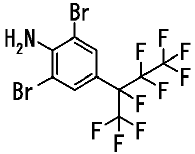

According to the method of 2-2 of Example 2, a target compound was prepared from 4-(perfluorobutan-2-yl)aniline.

¹H-NMR (CDCl₃, ppm) δ 4.89 (2H, broad-s), 7.57 (2H, s).

### <4-3>

### Preparation of 2-chloro-N-(2,6-dibromo-4-(perfluorobutan-2-yl)phenyl)-3-nitrobenzamide

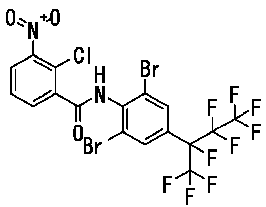

To 27 ml of DMI were added 9.90 g (21.1 mmol) of 2,6-dibromo-4-(perfluorobutan-2-yl)aniline and 4.60 g (20.9 mmol) of 2-chloro-3-nitrobenzoyl chloride, followed by stirring at 140°C for 4 hours. To the reaction solution were added water and ethyl acetate, and the organic phase was extracted, and washed with 1 mol/L of an aqueous sodium hydroxide solution, saturated brine, and dried over anhydrous magnesium sulfate. Then, the solvent was evaporated under reduced pressure. The obtained residue was purified by silica gel column chromatography (eluent solvent; hexane:ethyl acetate=20:1→ 10:1 →5:1 →3:1) to prepare 5.44 g (yield 40%) of a target compound.

¹H-NMR (CDCl₃, ppm) δ 7.52-7. 61 (2H, m), 7.89 (2H, s), 7. 94 (1H, dd, J=1.5, 8. 3 Hz), 7.99 (1H, d, J=7. 8 Hz).

### <4-4>

### Preparation of N-(2,6-dibromo-4-(perfluorobutan-2-yl)phenyl)-2-fluoro-3-nitrobenzamide

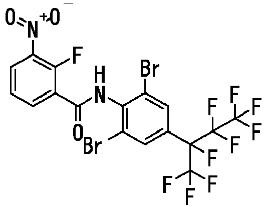

To 108 ml of DMSO were added 5.44 g (8.34 mmol) of 2-chloro-N-(2,6-dibromo-4-(perfluorobutan-2-yl)phenyl)-3-nitrobenzamide and 4.90 g (84.3 mmol) of potassium fluoride (spray-dried product), followed by stirring at 145°C for 2 hours. The reaction solution was poured into ice-water to precipitate the crystal, and the obtained crystals were filtered and washed with hexane. The obtained crystals were purified by silica gel column chromatography (eluent solvent; hexane:ethyl acetate=5:1) to prepare 2.42 g (yield 46%) of a target compound.

¹H-NMR (CDCl₃, ppm) δ 7.53-7.54 (1H, m), 7.89 (2H, s), 8.17 (1H, d, J=12.2 Hz), 8.29-8.30 (1H, m), 8.48-8.49 (1H, m).

### <4-5>

### Preparation of 3-amino-N-(2,6-dibromo-4-(perfluorobutan-2-yl)phenyl)-2-fluorobenzamide

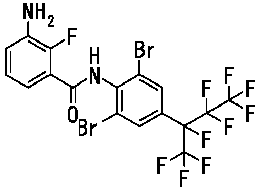

According to the method of 1-5 of Example 1, a target compound was prepared from N-(2,6-dibromo-4-(perfluorobutan-2-yl)phenyl)-2-fluoro-3-nitrobenzamide.

¹H-NMR (CDCl₃, ppm) δ 3.92 (2H, broad-s), 6.99-7.04 (1H, m), 7.11-7.12 (1H, m), 7.48-7.52 (1H, m), 7.86 (2H, s), 8.22 (1H, d, J=14.1 Hz).

### <4-6>

### Preparation of N-(2,6-dibromo-4-(perfluorobutan-2-yl)phenyl)-2-fluoro-3-(methylamino)benzamide

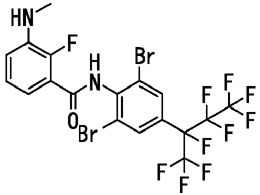

According to the method of 1-6 of Example 1, a target compound was prepared from 3-amino-N-(2,6-dibromo-4-(perfluorobutan-2-yl)phenyl)-2-fluorobenzamide.

¹H-NMR (CDCl₃, ppm) δ 2.95 (3H, s), 4.14 (1H, broad-s), 6.91-6.92 (1H, m), 7.17-7.21 (1H, m), 7.39-7.43 (1H, m), 7.85 (2H, s), 8.21 (1H, d, J=14.1 Hz).

### <4-7>

### Preparation of N-(2,6-dibromo-4-(perfluorobutan-2-yl)phenyl)-2-fluoro-3-(N-methylbenzamide)benzamide (Compound No. 7-1182)

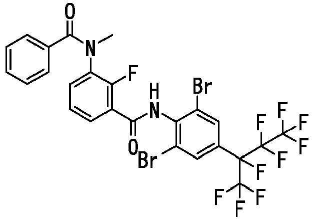

According to the method of 1-7 of Example 1, a target compound was prepared from N-(2,6-dibromo-4-(perfluorobutan-2-yl)phenyl)-2-fluoro-3-(methylamino)benzamide.

¹H-NMR (CDCl₃, ppm) δ 3.51 (3H, s), 7.22-7.44(7 H, m), 7.85 (2H, s), 8.00-8.03 (2H, m).

### <Example 5>

### Preparation of N-(2-bromo-6-(perfluoroethyl)-4-(perfluoropropan-2-yl)phenyl)-3-(4-cyanobenzamide)-2-fluorobenzamide (Compound No. 6-5911)

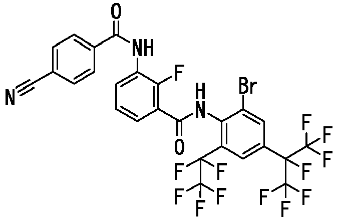

### <5-1>

### Preparation of 2-(perfluoroethyl)-4-(perfluoropropan-2-yl)aniline

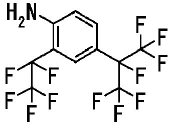

According to the method of 1-1 of Example 1, a target compound was prepared from 4-(perfluoropropan-2-yl)aniline obtained in Example 2-1 and 1,1,2,2,2-pentafluoroethyliodide.

¹H-NMR (CDCl₃, ppm) δ 4.56 (2H, broad-s), 6.79 (1H, d, J=8.8 Hz), 7.47 (1H, d, J=8.8 Hz), 7.53 (1H, s).

### <5-2>

### Preparation of 2-bromo-6-(perfluoroethyl)-4-(perfluoropropan-2-yl)aniline

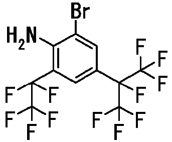

According to the method of 1-2 of Example 1, a target compound was prepared from 2-(perfluoroethyl)-4-(perfluoropropan-2-yl)aniline.

¹H-NMR (CDCl₃, ppm) δ 5.14 (2H, broad-s), 7.58 (1H, s), 7.81 (1H, s).

### <5-3>

### Preparation of N-(2-bromo-6-(perfluoroethyl)-4-(perfluoropropan-2-yl)phenyl)-2-chloro-3-nitrobenzamide

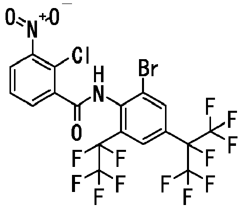

According to the method of 4-3 of Example 4, a target compound was prepared from 2-bromo-6-(perfluoroethyl)-4-(perfluoropropan-2-yl)aniline.

¹H-NMR (CDCl₃, ppm) δ 7.56-7.61 (1H, m), 7.73 (1H, s), 7.88 (1H, d, J=1.5 Hz), 7.92-7.98 (2H, m), 8.21 (1H, s).

### <5-4>

### Preparation of N-(2-bromo-6-(perfluoroethyl)-4-(perfluoropropan-2-yl)phenyl)-2-fluoro-3-nitrobenzamide

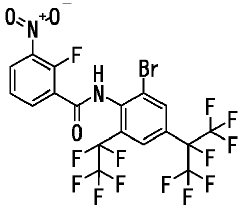

According to the method of 1-4 of Example 1, a target compound was prepared from N-(2-bromo-6-(perfluoroethyl)-4-(perfluoropropan-2-yl)phenyl)-2-chloro-3-nitrobenzamide.

APCI-MS m/z (M+1):626

### <5-5>

### Preparation of 3-amino-N-(2-bromo-6-(perfluoroethyl)-4-(perfluoropropan-2-yl)phenyl)-2-fluorobenzamide

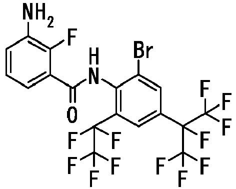

According to the method of 1-5 of Example 1, a target compound was prepared from N-(2-bromo-6-(perfluoroethyl)-4-(perfluoropropan-2-yl)phenyl)-2-fluoro-3-nitrobenzamide.

¹H-NMR (CDCl₃, ppm) δ 3.92 (2H, broad-s), 6.99-7.04 (1H, m), 7.05-7.18 (1H, m), 7.46-7.51 (1H, m), 7.85 (1H, broad-s), 8.17 (1H, broad-s), 8.34 (1H, d, J=15.1 Hz).

### <5-6>

### Preparation of N-(2-bromo-6-(perfluoroethyl)-4-(perfluoropropan-2-yl)phenyl)-3-(4-cyanobenzamide)-2-fluorobenzamide (Compound No. 6-5911)

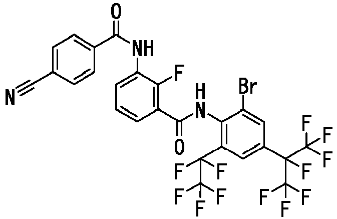

According to the method of 1-7 of Example 1, a target compound was prepared from 3-amino-N-(2-bromo-6-(perfluoroethyl)-4-(perfluoropropan-2-yl)phenyl)-2-fluorobenzamide and 4-cyanobenzoylchloride.

¹H-NMR (CDCl₃, ppm) δ 7.41 (1H, t, J=8.3 Hz), 7.84-7.87 (3H, m), 7.91-7.95 (1H, m), 8.03-8.05 (2H, m), 8.10 (1H, broad-s), 8.17-8.20 (2H, m), 8.63-8.67 (1H, m).

### <Example 6>

### Preparation of N-(2-bromo-4-(perfluoropropan-2-yl)-6-(trifluoromethyl)phenyl)-6-(N-methylbenzamide)picolinamide (Compound No. 17-1103)

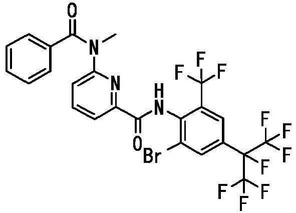

### <6-1>

### Preparation of N-(2-bromo-4-(perfluoropropan-2-yl)-6-(trifluoromethyl)phenyl)-6-chloropicoline amide

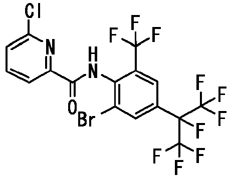

According to the method of 1-3 of Example 1, a target compound was prepared from 2-chloropyridine-6-carboxylic acid chloride prepared from 2-chloropyridine-6-carboxylic acid and thionyl chloride, and 2-bromo-4-(perfluoropropan-2-yl)-6-(trifluoromethyl)aniline obtained in 1-2 of Example 1.

¹H-NMR (CDCl₃, ppm) δ 7.59 (1H, d, J=7.3 Hz), 7.90-7.93 (2H, m), 8.14 (1H, s), 8.20-8.24 (1H, m), 9.60 (1H, s).

### <6-2>

### Preparation of N-(2-bromo-4-(perfluoropropan-2-yl)-6-(trifluoromethyl)phenyl)-6-(methylamino)picolinamide

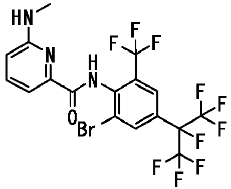

To a solution of 0.100 g (0.180 mmol) of N-(2-bromo-4-(perfluoropropan-2-yl)-6-(trifluoromethyl)phenyl)-6-chloropicoline amide in 5 ml of 1,4-dioxane were added 0.00600 g (0.0360 mmol) of copper sulfate and 0.140 g (1.80 mmol) of a 40% aqueous methylamine solution, followed by stirring at an oil bath temperature 80°C for 3 hours under an enclosed condition. The reaction liquid was returned to room temperature and opened, and water and ethyl acetate were added thereto. The organic layer was washed with water and saturated brine, and dried over anhydrous magnesium sulfate. The solvent was evaporated under reduced pressure and the obtained residue was purified by silica gel column chromatography (eluent solvent; hexane:ethyl acetate=2:1) to prepare 0.0700 g (yield 69%) of a target compound.

¹H-NMR (CDCl₃, ppm) δ 2.64 (3H, s), 3.79 (1H, broad-s), 7.56-7.60 (1H, m), 7.87-7.93 (2H, m), 8.14-8.15 (1H, m), 8.20-8.23 (1H, m), 9.60 (1H, s).

### <6-3>

### Preparation of N-(2-bromo-4-(perfluoropropan-2-yl)-6-(trifluoromethyl)phenyl)-6-(N-methylbenzamide)picolinamide (Compound No. 17-1103)

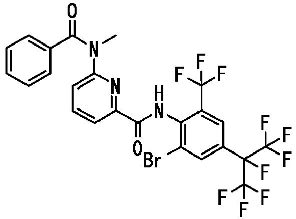

According to the method of 1-7 of Example 1, a target compound was prepared from N-(2-bromo-4-(perfluoropropan-2-yl)-6-(trifluoromethyl)phenyl)-6-(methylamino)picolinamide.

¹H-NMR (CDCl₃, ppm) δ 3.65 (3H, s), 7.28-7.41 (6H, m), 7.77 (1H, t, J=7.8 Hz), 7.91 (1H, s), 8.00-8.02 (1H, m), 8.14 (1H, s), 9.39 (1H, s).

### <Example 7>

### Preparation of N-(2-bromo-4-(perfluoropropan-2-yl)-6-(trifluoromethyl)phenyl)-2-(2-fluoro-N-methylbenzamide)thiazole-4-carboxamide (Compound No. 27-628)

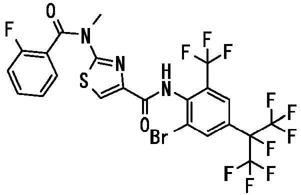

### <7-1>

### Preparation of 2-aminothiazole-4-carboxylic acid

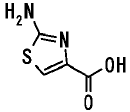

To 40 ml of an aqueous solution of 4.00 g (23.2 mmol) of ethyl 2-aminothiazole-4-carboxylate was added 1.86 g (46.5 mmol) of sodium hydroxide, followed by stirring at room temperature for 5 hours. The reaction liquid was adjusted to pH 1 by the addition of concentrated hydrochloric acid, and the precipitated crystals were collected by filtration to prepare 2.84 g (yield 85%) of a target compound.

¹H-NMR (CDCl₃, ppm) δ 7.18 (2H, broad-s), 7.38 (1H, s).

The proton of the carboxylic acid was not detected.

### <7-2>

### Preparation of 2-chlorothiazole-4-carboxylic acid

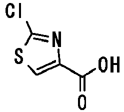

To a solution of 2.84 g (19.7 mmol) of 2-aminothiazole-4-carboxylic acid in 30 ml of 1,4-dioxane was added 50 ml of concentrated hydrochloric acid, followed by cooling to 0°C, and 10 ml of an aqueous solution of 2.04 g (29.6 mmol) of sodium nitrite was added charged dropwise thereto at 0°C to 5°C. The reaction liquid was stirred at 0°C for 2 hours, and then 2.93 g (29.6 mmol) of copper chloride was charged in separate portions thereto. The reaction liquid was returned to room temperature, followed by stirring for 8 hours. To the reaction liquid were added water and ethyl acetate, followed by extraction with ethyl acetate four times. The organic layer was washed with saturated brine, and then dried over anhydrous magnesium sulfate. The solvent was evaporated under reduced pressure to prepare 1.77 g (yield 55%) of a target compound.

¹H-NMR (DMSO-d₆, ppm) δ 8.41 (1H, s).

The proton of the carboxylic acid was not detected.

### <7-3>

### Preparation of N-(2-bromo-4-(perfluoropropan-2-yl)-6-(trifluoromethyl)phenyl)-2-chlorothiazole-4-carboxamide

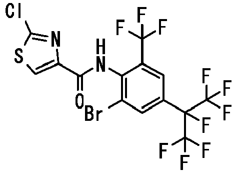

According to the method of 4-3 of Example 4, a target compound was prepared from 2-chlorothiazole-4-carbonylchloride prepared from 2-chlorothiazole-4-carboxylic acid and thionyl chloride, and 2-bromo-4-(perfluoropropan-2-yl)-6-(trifluoromethyl)aniline obtained in 1-2 of Example 1.

### ¹H-NMR (CDCl₃, ppm) δ 7.91 (1H, s), 8.13 (1H, s), 8.19 (1H, s), 8.82 (1H, s).

### <7-4>

### Preparation of N-(2-bromo-4-(perfluoropropan-2-yl)-6-(trifluoromethyl)phenyl)-2-(methylamino)thiazole-4-carboxamide

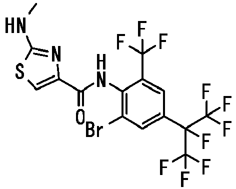

According to the method of 6-2 of Example 6, a target compound was prepared from N-(2-bromo-4-(perfluoropropan-2-yl)-6-(trifluoromethyl)phenyl)-2-chlorothiazole-4-carboxamide.

¹H-NMR (CDCl₃, ppm) δ 3.03 (3H, s), 5.11-5.12 (1H, m), 7.50 (1H, s), 7.88 (1H, s), 8.11 (1H, s), 8.99 (1H, s).

### <7-5>

### Preparation of N-(2-bromo-4-(perfluoropropan-2-yl)-6-(trifluoromethyl)phenyl)-2-(2-fluoro-N-methylbenzamide)thiazole-4-carboxamide (Compound No. 27-628)

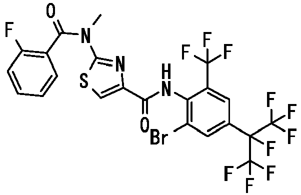

According to the method of 1-7 of Example 1, a target compound was prepared from N-(2-bromo-4-(perfluoropropan-2-yl)-6-(trifluoromethyl)phenyl)-2-(methylamino)thiazole-4-carboxamide and 2-fluorobenzoyl chloride.

¹H-NMR (CDCl₃, ppm) δ 3.67 (3H, s), 6.99-7.24 (1H, m), 7.29-7.35 (1H, m), 7.52-7.59 (2H, m), 7.90 (1H, s), 8.06 (1H, s), 8.14 (1H, s), 9.08 (1H, s).

### <Example 8>

### Preparation of 2,2,2-trichloroethyl 2-fluoro-3-(2-iodo-4-(perfluorobutan-2-yl)-6-(trifluoromethyl)phenylcarbamoyl)phenyl(methyl)carbamate (Compound No. 12-864)

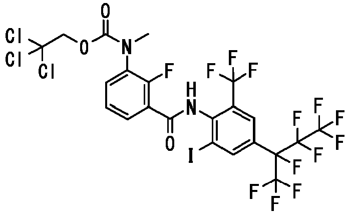

### <8-1>

### Preparation of 4-(perfluorobutan-2-yl)-2-(trifluoromethyl)aniline

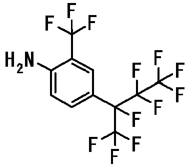

According to the method of 1-1 of Example 1, a target compound was prepared from 2-(trifluoromethyl)aniline and nonafluoro-s-butyliodide under the light-shielding reaction condition.

¹H-NMR (CDCl₃, ppm) δ 4.49 (2H, broad-s), 6.81 (1H, d, J=8.8 Hz), 7.47 (1H, d, J=8.8 Hz), 7.61 (1H, s).

### <8-2>

### Preparation of 2-iodo-4-(perfluorobutan-2-yl)-6-(trifluoromethyl)aniline

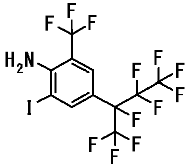

To 100 mL of ethanol was added 17.0 g (44.8 mmol) of 4-(perfluorobutan-2-yl)-2-(trifluoromethyl)aniline, and 5.28 g (53.8 mmol) of concentrated sulfuric acid and 12.6 g (55.8 mmol) of N-iodosuccinimide were added thereto under ice-cooling, followed by stirring at room temperature for 1 hour and 30 minutes and at 40°C for 4 hours. To the reaction solution was added a 4 M aqueous sodium hydroxide solution to neutralize the reaction solution, and then ethyl acetate was added thereto to extract the organic phase. The organic phase was washed with saturated brine, and dried over anhydrous magnesium sulfate, and then the solvent was evaporated under reduced pressure. The obtained residue was purified by silica gel column chromatography (eluent solvent; hexane:ethyl acetate=10:1) to prepare 14.6 g (yield 65%) of a target compound.

¹H-NMR (CDCl₃, ppm) δ 5.04 (2H, broad-s), 7.62 (1H, s), 7.97 (1H, s).

### <8-3>

### Preparation of 2-chloro-N-(2-iodo-4-(perfluorobutan-2-yl)-6-(trifluoromethyl)phenyl)-3-nitrobenzamide

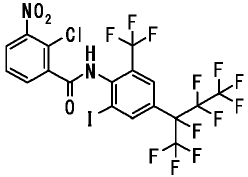

According to the method of 4-3 of Example 4, a target compound was prepared from 2-iodo-4-(perfluorobutan-2-yl)-6-(trifluoromethyl)aniline.

¹H-NMR (CDCl₃, ppm) δ 7.60-7.61 (1H, m), 7.77 (1H, s), 7.89-7.96 (2H, m), 8.03-8.04 (1H, m), 8.38 (1H, s).

### <8-4>

### Preparation of 2-fluoro-N-(2-iodo-4-(perfluorobutan-2-yl)-6-(trifluoromethyl)phenyl)-3-nitrobenzamide

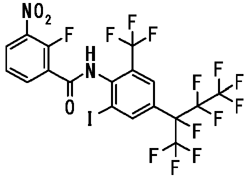

According to the method of 1-4 of Example 1, a target compound was prepared from 2-chloro-N-(2-iodo-4-(perfluorobutan-2-yl)-6-(trifluoromethyl)phenyl)-3-nitrobenzamide.

¹H-NMR (CDCl₃, ppm) δ 7.53-7.54 (1H, m), 7.95 (1H, s), 8.24-8.32 (2H, m), 8.36 (1H, s), 8.44-8.48 (1H, m).

### <8-5>

### Preparation of 3-amino-2-fluoro-N-(2-iodo-4-(perfluorobutan-2-yl)-6-(trifluoromethyl)phenyl)benzamide

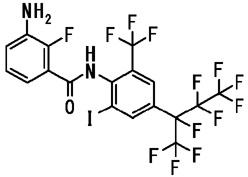

According to the method of 1-5 of Example 1, a target compound was prepared from 2-fluoro-N-(2-iodo-4-(perfluorobutan-2-yl)-6-(trifluoromethyl)phenyl)-3-nitrobenzamide.

¹H-NMR (CDCl₃, ppm) δ 3.93 (2H, broad-s), 7.02-7.03 (1H, m), 7.11-7.13 (1H, m), 7.47-7.51 (1H, m), 7.92 (1H, s), 8.31-8.34 (2H, m).

### <8-6>

### Preparation of 2-fluoro-N-(2-iodo-4-(perfluorobutan-2-yl)-6-(trifluoromethyl)phenyl)-3-(methylamino)benzamide

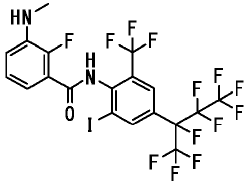

According to the method of 1-6 of Example 1, a target compound was prepared from 3-amino-2-fluoro-N-(2-iodo-4-(perfluorobutan-2-yl)-6-(trifluoromethyl)phenyl)benzamide.

¹H-NMR (CDCl₃, ppm) δ 2.95-2.96 (3H, m), 4.15 (1H, broad-s), 6.91-6.93 (1H, m), 7.19-7.20 (1H, m), 7.38-7.42 (1H, m), 7.92 (1H, s), 8.32 (1H, d, J=14.1 Hz), 8.34 (1H, s).

### <8-7>

### Preparation of 2,2,2-trichloroethyl 2-fluoro-3-(2-iodo-4-(perfluorobutan-2-yl)-6-(trifluoromethyl)phenylcarbamoyl)phenyl(methyl)carbamate (Compound No. 12-864)

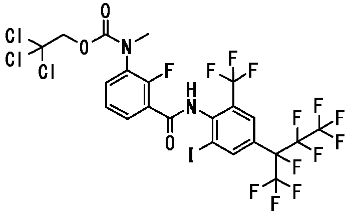

According to the method of 1-7 of Example 1, a target compound was prepared from 2-fluoro-N-(2-iodo-4-(perfluorobutan-2-yl)-6-(trifluoromethyl)phenyl)-3-(methylamino)benzamide and 2,2,2-trichloroethoxycarbonylchloride.

¹H-NMR (CDCl₃, ppm) δ 3.40 (3H, s), 4.74 (2H, broad-s), 7.37 (1H, t, J=7.8 Hz), 7.52-7.58 (1H, m), 7.93 (1H, s), 8.12-8.15 (1H, m), 8.28-8.34 (2H, m).

### <Example 9>

### Preparation of N-(2-chloro-4-(perfluoropropan-2-yl)-6-(trifluoromethyl)phenyl)-4-cyano-3-(4-cyano-N-methylbenzamide)benzamide (Compound No. 2-491)

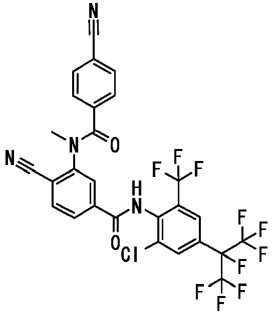

### <9-1>

### Preparation of 2-chloro-4-(perfluoropropan-2-yl)-6-(trifluoromethyl)aniline

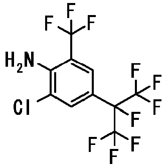

According to the method of 1-2 of Example 1, a target compound was prepared from 4-(perfluoropropan-2-yl)-2-(trifluoromethyl)aniline obtained in 1-1 of Example 1 and N-chlorosuccinimide.

¹H-NMR (CDCl₃, ppm) δ 4.97 (2H, broad-s), 7.57 (1H, s), 7.64 (1H, s).

### <9-2>

### Preparation of N-(2-chloro-4-(perfluoropropan-2-yl)-6-(trifluoromethyl)phenyl)-4-iodo-3-nitrobenzamide

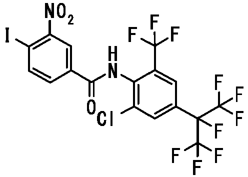

According to the method of 1-3 of Example 1, a target compound was prepared from 4-iodo-3-nitrobenzoyl chloride prepared from 4-iodo-3-nitrobenzoic acid and thionyl chloride, and 2-chloro-4-(perfluoropropan-2-yl)-6-(trifluoromethyl)aniline.

¹H-NMR (CDCl₃, ppm) δ 7.52-7.81 (2H, m), 7.89 (1H, s), 8.00 (1H, s), 8.25 (1H, d, J=8.3 Hz), 8.38 (1H, d, J=1.9 Hz).

### <9-3>

### Preparation of 3-amino-N-(2-chloro-4-(perfluoropropan-2-yl)-6-(trifluoromethyl)phenyl)-4-iodobenzamide

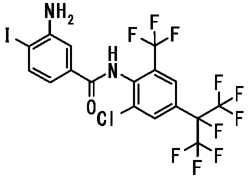

According to the method of 1-5 of Example 1, a target compound was prepared from N-(2-chloro-4-(perfluoropropan-2-yl)-6-(trifluoromethyl)phenyl)-4-iodo-3-nitrobenzamide.

¹H-NMR (CDCl₃, ppm) δ 4.35 (2H, s), 6.92 (1H, dd, J=1.9, 8.3 Hz), 7.29 (1H, d, J=1.9 Hz), 7.60 (1H, s), 7.79 (1H, d, J=8.3 Hz), 7.86 (1H, s), 7.97 (1H, s).

### <9-4>

### Preparation of N-(2-chloro-4-(perfluoropropan-2-yl)-6-(trifluoromethyl)phenyl)-4-iodo-3-(methylamino)benzamide

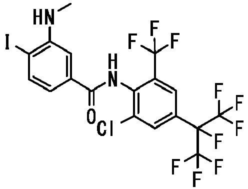

According to the method of 1-6 of Example 1, a target compound was prepared from 3-amino-N-(2-chloro-4-(perfluoropropan-2-yl)-6-(trifluoromethyl)phenyl)-4-iodobenzamide.

¹H-NMR (CDCl₃, ppm) δ 2.97 (3H, s), 4.46 (1H, broad-s), 6.89 (1H, dd, J=1.9, 8.3 Hz), 7.07 (1H, d, J=1.9 Hz), 7.65 (1H, s), 7.80 (1H, d, J=8.3 Hz), 7.86 (1H, s), 7.97 (1H, s).

### <9-5>

### Preparation of N-(2-chloro-4-(perfluoropropan-2-yl)-6-(trifluoromethyl)phenyl)-4-cyano-3-(methylamino)benzamide

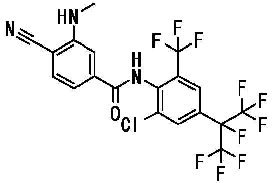

To 10 mL of DMF were added 0.350 g (0.560 mmol) of N-(2-chloro-4-(perfluoropropan-2-yl)-6-(trifluoromethyl)phenyl)-4-iodo-3-(methylamino)benzamide and 0.200 g (2.25 mmol) of copper (I) cyanide, followed by stirring at 140°C for 1 hour and 30 minutes. A saturated aqueous sodium thiosulfate solution was poured into the reaction solution to quench the reaction, and the organic layer was collected with ethyl acetate and washed with saturated brine. The organic layer was dried over anhydrous magnesium sulfate, and then the solvent was evaporated under reduced pressure. The obtained residue was purified by silica gel column chromatography (eluent solvent; hexane:ethyl acetate=5:1→3:1) to prepare 0.250 g (yield 86%) of a target compound.

¹H-NMR (CDCl₃, ppm) δ 3.01(1/2^{∗}3H, s), 3.03(1/2^{∗}3H, s), 4.89(1/2^{∗}1H, s), 4.90(1/2^{∗}1H, s), 7.80 (1H, dd, J=1.5, 8.3 Hz), 7.21-7.22 (1H, m), 7.54 (1H, d, J=8.3 Hz), 7.67 (1H, s), 7.88 (1H, s), 7.99 (1H, s).

### <9-6>

### Preparation of N-(2-chloro-4-(perfluoropropan-2-yl)-6-(trifluoromethyl)phenyl)-4-cyano-3-(4-cyano-N-methylbenzamide)benzamide (Compound No. 2-491)

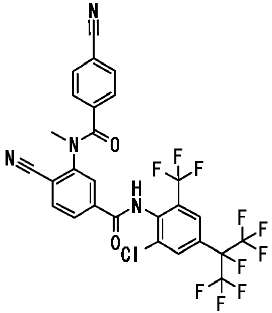

According to the method of 1-7 of Example 1, a target compound was prepared from N-(2-chloro-4-(perfluoropropan-2-yl)-6-(trifluoromethyl)phenyl)-4-cyano-3-(methylamino)benzamide and 4-cyanobenzoylchloride.

¹H-NMR (CDCl₃, ppm) δ 3.81 (3H, broad-s), 7.52-7.84 (8H, m), 7.89 (1H, s), 8.00 (1H, s).

### <Example 10>

### Preparation of N-(3-(2-bromo-4-(perfluoropropan-2-yl)-6-(trifluoromethyl)phenylcarbamoyl)-6-cyano-2-fluorophenyl)-6-chloronicotinamide (Compound No. 1-1968)

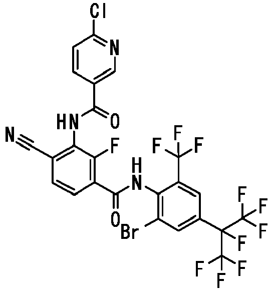

### <10-1>

### Preparation of N-(2-bromo-4-(perfluoropropan-2-yl)-6-(trifluoromethyl)phenyl)-4-cyano-2,3-difluorobenzamide

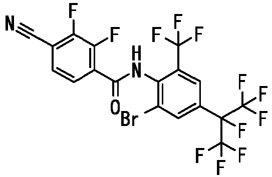

To a solution of 0.840 g (4.59 mmol) of 4-cyano-2,3-difluorobenzoic acid in 10 ml of dichloromethane were added one drop of DMF and 0.470 ml (5.51 mmol) of oxalyl chloride, followed by stirring at room temperature for 1 hour. The solvent was evaporated under reduced pressure and the obtained 4-cyano-2,3-difluorobenzoyl chloride was added to a solution of 1.56 g (3.83 mmol) of 2-bromo-4-(perfluoropropan-2-yl)-6-(trifluoromethyl)aniline obtained in 1-2 of Example 1 in 5 ml of DMI, followed by stirring at 130°C for 5 hours. To the reaction liquid were added water and ethyl acetate, and the organic layer was washed with a saturated aqueous sodium hydrogen carbonate solution and saturated brine, and then dried over anhydrous magnesium sulfate. The solvent was evaporated under reduced pressure and the obtained residue was purified by silica gel column chromatography (eluent solvent; hexane:ethyl acetate=1:0→10:1) to prepare 0.58 g (yield 27%) of a target compound.

¹H-NMR (CDCl₃, ppm) δ 7.52-7.62 (1H, m), 7.92-7.94 (1H, m), 8.02-8.06 (1H, m), 8.13-8.16 (2H, m).

### <10-2>

### Preparation of 3-amino-N-(2-bromo-4-(perfluoropropan-2-yl)-6-(trifluoromethyl)phenyl)-4-cyano-2-fluorobenzamide

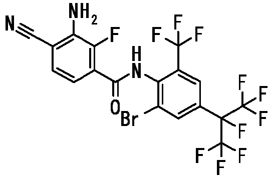

To a solution of N-(2-bromo-4-(perfluoropropan-2-yl)-6-(trifluoromethyl)phenyl)-4-cyano-2,3-difluorobenzamide in 5 ml of DMSO was added 49.0 mg of ammonium carbonate, followed by stirring 100°C for 5 hours. To the reaction liquid were added water and ethyl acetate, and the organic layer was washed with water, and then and dried over anhydrous magnesium sulfate. The solvent was evaporated under reduced pressure and the obtained residue was purified by silica gel column chromatography (eluent solvent; hexane:ethyl acetate=8:1→4:1) to prepare 0.30 g (yield 51%) of a target compound.

¹H-NMR (CDCl₃, ppm) δ 4.71 (2H, broad-s), 7.35-7.39 (1H, m), 7.40-7.44 (1H, m), 7.92 (1H, s), 8.12-8.15 (2H, m).

### <10-3>

### Preparation of N-(3-(2-bromo-4-(perfluoropropan-2-yl)-6-(trifluoromethyl)phenylcarbamoyl)-6-cyano-2-fluorophenyl)-6-chloronicotinamide (Compound No. 1-1968)

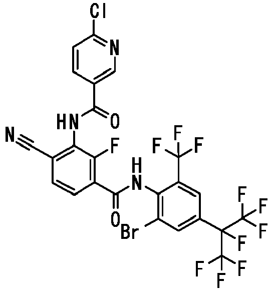

To a solution of 0.0500 g (0.0877 mol) of 3-amino-N-(2-bromo-4-(perfluoropropan-2-yl)-6-(trifluoromethyl)phenyl)-4-cyano-2-fluorobenzamide in 0.200 ml of DMI was added 0.0308 g (0.175 mmol) of 6-chloronicotinoylchloride, followed by stirring at 130°C for 6 hours. To the reaction liquid were added water and ethyl acetate, and the organic layer was washed with 1 M hydrochloric acid, a saturated aqueous sodium hydrogen carbonate solution, and saturated brine, and then dried over anhydrous magnesium sulfate. The solvent was evaporated under reduced pressure and the obtained residue was purified by silica gel column chromatography (eluent solvent; hexane:ethyl acetate=2:1) to prepare 0.0100 g (yield 16%) of a target compound.

¹H-NMR (CDCl₃, ppm) δ 7.54 (1H, d, J=8.8 Hz), 7.72 (1H, d, J=8.8 Hz), 7.93 (1H, s), 8.09 (1H, s), 8.17-8.18 (2H, m), 8.27 (1H, dd, J=2.4Hz 8.8 Hz), 8.38 (1H, d, J=10.8 Hz), 8.98 (1H, d, J=2.4 Hz).

### <Example 11>

### Preparation of 3-(4-cyanobenzamide)-N-(2,6-diiodo-4-(perfluorobutan-2-yl)phenyl)benzamide (Compound No. 6-79)

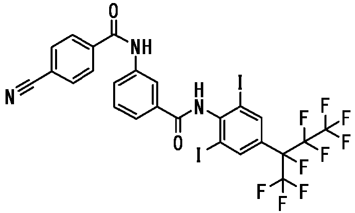

### <11-1>

### Preparation of 2,6-diiodo-4-(perfluorobutan-2-yl)aniline

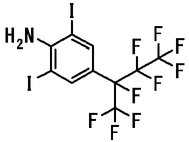

According to the method of 3-1 of Example 3, a target compound was prepared from 4-(perfluorobutan-2-yl)aniline obtained in 4-1 of Example 4.

¹H-NMR (CDCl₃, ppm) δ 4.95 (2H, broad-s), 7.78 (2H, s).

### <11-2>

### Preparation of N-(2,6-diiodo-4-(perfluorobutan-2-yl)phenyl)-3-nitrobenzamide

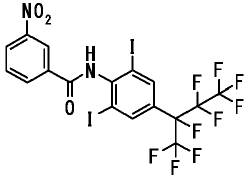

According to the method of 4-3 of Example 4, a target compound was prepared from 2,6-diiodo-4-(perfluorobutan-2-yl)aniline and 3-nitrobenzoyl chloride.

¹H-NMR (CDCl₃, ppm) δ 7.74 (1H, t, J=8.0 Hz), 8.11 (2H, s), 8.42 (1H, d, J=7.6 Hz), 8.46 (1H, d, J=8.4 Hz), 8.90 (1H, d, J=12.4 Hz), 8.92 (1H, s).

### <11-3>

### Preparation of 3-amino-N-(2,6-diiodo-4-(perfluorobutan-2-yl)phenyl)benzamide

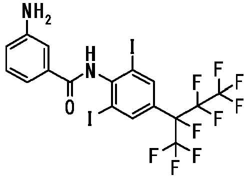

According to the method of 1-5 of Example 1, a target compound was prepared from N-(2,6-diiodo-4-(perfluorobutan-2-yl)phenyl)-3-nitrobenzamide.

¹H-NMR (CDCl₃, ppm) δ 5.39 (2H, broad-s), 6.89-6.93 (1H, m), 7.29-7.31 (3H, m), 7.68 (1H, s), 8.08 (2H, s).

### <11-4>

### Preparation of 3-(4-cyanobenzamide)-N-(2,6-diiodo-4-(perfluorobutan-2-yl)phenyl)benzamide (Compound No. 6-79)

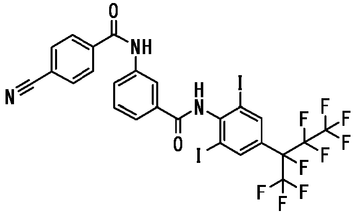

According to the method of 1-7 of Example 1, a target compound was prepared from 3-amino-N-(2,6-diiodo-4-(perfluorobutan-2-yl)phenyl)benzamide and 4-cyanobenzoylchloride.

¹H-NMR (CDCl₃, ppm) δ 7.58 (1H, t, J=8.2 Hz), 7.79-7.83 (3H, m), 7.97 (2H, s), 8.01 (2H, d, J=8.0 Hz), 8.09 (2H, s), 8.18 (1H, s), 8.29 (1H, s).

### <Example 12>

### Preparation of N-(3-(2-bromo-4-(perfluoropropan-2-yl)-6-(trifluoromethoxy)phenylcarbamoyl)-2-fluorophenyl)-2-chloronicotinamide (Compound No. 6-5908)

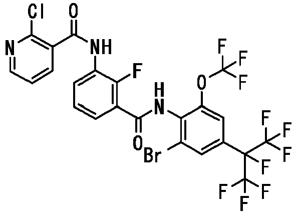

### <12-1>

### Preparation of 4-(perfluoropropan-2-yl)-2-(trifluoromethoxy)aniline

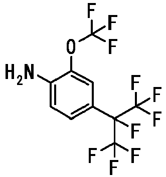

According to the method of 1-1 of Example 1, a target compound was prepared from 2-trifluoromethoxy aniline.

¹H-NMR (CDCl₃, ppm) δ 4.19 (2H, broad-s), 6.86 (1H, d, J=8.8 Hz), 7.30 (1H, d, J=8.8 Hz), 7.36 (1H, s).

### <12-2>

### Preparation of 2-bromo-4-(perfluoropropan-2-yl)-6-(trifluoromethoxy)aniline

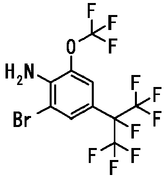

According to the method of 1-2 of Example 1, a target compound was prepared from 4-(perfluoropropan-2-yl)-2-(trifluoromethoxy)aniline.

¹H-NMR (CDCl₃, ppm) δ 4.65 (2H, broad-s), 7.33 (1H, s), 7.71 (1H, s).

### <12-3>

### Preparation of N-(2-bromo-4-(perfluoropropan-2-yl)-6-(trifluoromethoxy)phenyl)-2-chloro-3-nitrobenzamide

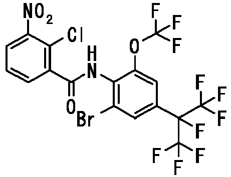

According to the method of 1-3 of Example 1, a target compound was prepared from 2-bromo-4-(perfluoropropan-2-yl)-6-(trifluoromethoxy)aniline.

¹H-NMR (CDCl₃, ppm) δ 7.49-7.61 (3H, m), 7.80-7.96 (3H, m).

### <12-4>

### Preparation of N-(2-bromo-4-(perfluoropropan-2-yl)-6-(trifluoromethoxy)phenyl)-2-fluoro-3-nitrobenzamide

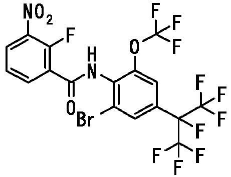

According to the method of 1-4 of Example 1, a target compound was prepared from N-(2-bromo-4-(perfluoropropan-2-yl)-6-(trifluoromethoxy)phenyl)-2-chloro-3-nitrobenzamide.

¹H-NMR (CDCl₃, ppm) δ 7.53 (1H, t, J=7.8 Hz), 7.60 (1H, broad-s), 7.89 (1H, d, J=1.5 Hz), 8.07 (1H, broad-d, J=12.7 Hz), 8.29-8.30 (1H, m), 8.43-8.47 (1H, m).

### <12-5>

### Preparation of 3-amino-N-(2-bromo-4-(perfluoropropan-2-yl)-6-(trifluoromethoxy)phenyl)-2-fluorobenzamide

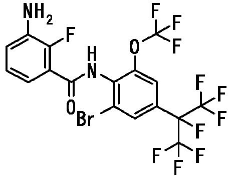

According to the method of 1-5 of Example 1, a target compound was prepared from N-(2-bromo-4-(perfluoropropan-2-yl)-6-(trifluoromethoxy)phenyl)-2-fluoro-3-nitrobenzamide.

¹H-NMR (CDCl₃, ppm) δ 3.92 (2H, broad-s), 6.99-7.04 (1H, m), 7.11 (1H, t, J=7.8 Hz), 7.45-7.49 (1H, m), 7.57 (1H, broad-s), 7.87 (1H, d, J=2.0 Hz), 8.14 (1H, d, J=14.2 Hz).

### <12-6>

### Preparation of N-(3-(2-bromo-4-(perfluoropropan-2-yl)-6-(trifluoromethoxy)phenylcarbamoyl)-2-fluorophenyl)-2-chloronicotinamide (Compound No. 6-5908)

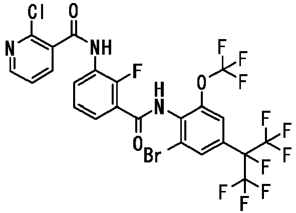

According to the method of 1-7 of Example 1, a target compound was prepared from 3-amino-N-(2-bromo-4-(perfluoropropan-2-yl)-6-(trifluoromethoxy)phenyl)-2-fluorobenzamide and 2-chloronicotinoylchloride.

¹H-NMR (CDCl₃, ppm) δ 7.39-7.49 (2H, m), 7.59 (1H, s), 7.88-7.94 (2H, m), 8.07 (1H, d, J=12.2 Hz), 8.31-8.33 (1H, m), 8.57-8.58 (1H, m), 8.60-8.70 (1H, m), 8.74 (1H, broad-s).

### <Example 13>

### Preparation of 3-benzamide-N-(2-bromo-4-(perfluoropropan-2-yl)-6-(trifluoromethyl)phenyl)-4-fluorobenzamide (Compound No. 6-3348)

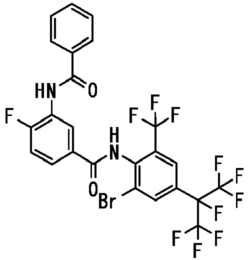

### <13-1>

### Preparation of N-(2-bromo-4-(perfluoropropan-2-yl)-6-(trifluoromethyl)phenyl)-4-fluoro-3 -nitrobenzamide

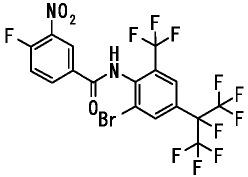

According to the method of 1-3 of Example 1, a target compound was prepared from 4-fluoro-3-nitrobenzoyl chloride prepared from 4-fluoro-3-nitrobenzoic acid and thionyl chloride, and 2-bromo-4-(perfluoropropan-2-yl)-6-(trifluoromethyl)aniline obtained in 1-2 of Example 1.

¹H-NMR (CDCl₃, ppm) δ 7.47-7.50 (1H, m), 7.92 (2H, d, J= 5.9 Hz), 8.16 (1H, s), 8.23-8.28 (1H, m), 8.65-8.67 (1H, m).

### <13-2>

### Preparation of 3-amino-N-(2-bromo-4-(perfluoropropan-2-yl)-6-(trifluoromethyl)phenyl)-4-fluorobenzamide

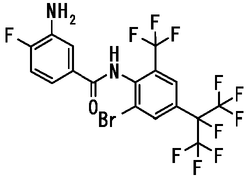

According to the method of 1-5 of Example 1, a target compound was prepared from N-(2-bromo-4-(perfluoropropan-2-yl)-6-(trifluoromethyl)phenyl)-4-fluoro-3-nitrobenzamide.

APCI-MS m/z (M+1):546

### <13-3>

### Preparation of 3-benzamide-N-(2-bromo-4-(perfluoropropan-2-yl)-6-(trifluoromethyl)phenyl)-4-fluorobenzamide (Compound No. 6-3348)

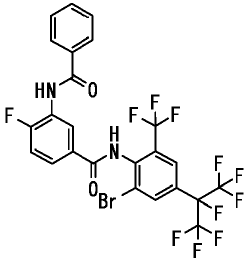

According to the method of 1-7 of Example 1, a target compound was prepared from 3-amino-N-(2-bromo-4-(perfluoropropan-2-yl)-6-(trifluoromethyl)phenyl)-4-fluorobenzamide.

¹H-NMR (CDCl₃, ppm) δ 7.29-7.34 (1H, m), 7.53-7.65 (3H, m), 7.80-7.84 (1H, m), 7.90-7.92 (3H, m), 8.14 (1H, broad-s), 8.20 (1H, d, J=2.9 Hz), 8.25 (1H, broad-s), 9.10 (1H, dd, J=1.9, 7.3 Hz).

### <Example 14>

### Preparation of 2-chloro-N-(3-(2-iodo-4-(perfluoropropan-2-yl)-6-(trifluoromethyl)phenylcarbamoyl)phenyl)nicotinamide (Compound No. 6-460)

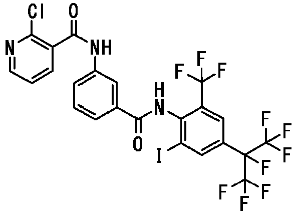

### <14-1>

### Preparation of 2-iodo-4-(perfluoropropan-2-yl)-6-(trifluoromethyl)aniline

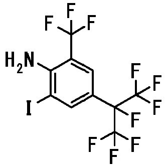

According to the method of 8-2 of Example 8, a target compound was prepared from 4-(perfluoropropan-2-yl)-2-(trifluoromethyl)aniline obtained in 1-1 of Example 1 and N-iodosuccinimide.

¹H-NMR (CDCl₃, ppm) δ 5.04 (2H, broad-s), 7.64 (1H, s), 7.99 (1H, s).

### <14-2>

### Preparation of N-(2-iodo-4-(perfluoropropan-2-yl)-6-(trifluoromethyl)phenyl)-3-nitrobenzamide

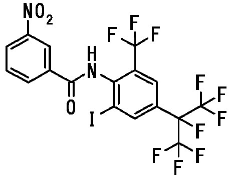

According to the method of 4-3 of Example 4, a target compound was prepared from 2-iodo-4-(perfluoropropan-2-yl)-6-(trifluoromethyl)aniline and 3-nitrobenzoyl chloride.

¹H-NMR (CDCl₃, ppm) δ 7.76-7.80 (2H, m), 7.97 (1H, s), 8.28-8.30 (1H, m), 8.37 (1H, s), 8.49-8.52 (1H, m), 8.78 (1H, s).

### <14-3>

### Preparation of 3-amino-N-(2-iodo-4-(perfluoropropan-2-yl)-6-(trifluoromethyl)phenyl)benzamide

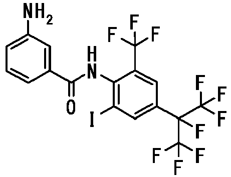

According to the method of 1-5 of Example 1, a target compound was prepared from N-(2-iodo-4-(perfluoropropan-2-yl)-6-(trifluoromethyl)phenyl)-3-nitrobenzamide.

¹H-NMR (CDCl₃, ppm) δ 3.89 (2H, broad-s), 6.89-6.92 (1H, m), 7.23-7.32 (3H, m), 7.68 (1H, s), 7.93 (1H, s), 8.34-8.36 (1H, m).

### <14-4>

### Preparation of 2-chloro-N-(3-(2-iodo-4-(perfluoropropan-2-yl)-6-(trifluoromethyl)phenylcarbamoyl)phenyl)nicotinamide (Compound No. 6-460)

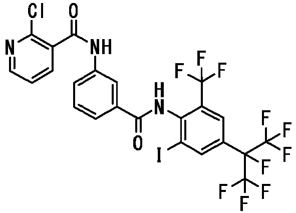

According to the method of 1-7 of Example 1, a target compound was prepared from 3-amino-N-(2-iodo-4-(perfluoropropan-2-yl)-6-(trifluoromethyl)phenyl)benzamide and 2-chloronicotinoylchloride.

¹H-NMR (CDCl₃, ppm) δ 7.43-7.46 (1H, m), 7.58 (1H, t, J=7.8 Hz), 7.77 (1H, d, J=7.8 Hz), 7.91-7.95 (2H, m), 8.01 (1H, s), 8.24 (1H, dd, J=2.0, 7.8 Hz), 8.28 (1H, s), 8.36 (1H, s), 8.41 (1H, s), 8.54-8.56 (1H, m).

### <Example 15>

### Preparation of 2-fluoro-3-(4-fluoro-N-methylbenzamide)-N-(2-iodo-4-(perfluoropropan-2-yl)-6-(trifluoromethyl)phenyl)benzamide (Compound No. 7-1733)

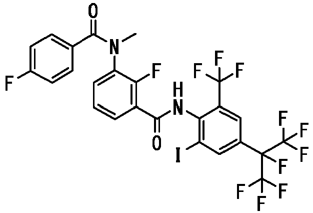

### <15-1>

### Preparation of 2-chloro-N-(2-iodo-4-(perfluoropropan-2-yl)-6-(trifluoromethyl)phenyl)-3-nitrobenzamide

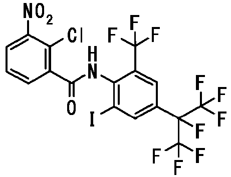

According to the method of 4-3 of Example 4, a target compound was prepared from 2-iodo-4-(perfluoropropan-2-yl)-6-(trifluoromethyl)aniline obtained in 14-1 of Example 14 and 2-chloro-3-nitrobenzoyl chloride.

¹H-NMR (CDCl₃, ppm) δ 7.60 (1H, t, J=7.8 Hz), 7.76 (1H, s), 7.94 (1H, dd, J=1.5, 7.8 Hz), 7.97 (1H, s), 8.03 (1H, dd, J=1.5, 7.8 Hz), 8.39 (1H, s).

### <15-2>

### Preparation of 2-fluoro-N-(2-iodo-4-(perfluoropropan-2-yl)-6-(trifluoromethyl)phenyl)-3-nitrobenzamide

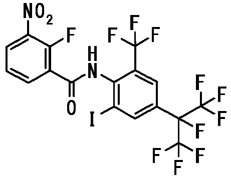

According to the method of 1-4 of Example 1, a target compound was prepared from 2-chloro-N-(2-iodo-4-(perfluoropropan-2-yl)-6-(trifluoromethyl)phenyl)-3-nitrobenzamide.

¹H-NMR (CDCl₃, ppm) δ 7.51-7.55 (1H, m), 7.97 (1H, s), 8.23 (1H, d, J=12.2 Hz), 8.28-8.32 (1H, m), 8.37 (1H, s), 8.44-8.48 (1H, m).

### <15-3>

### Preparation of 3-amino-2-fluoro-N-(2-iodo-4-(perfluoropropan-2-yl)-6-(trifluoromethyl)phenyl)benzamide

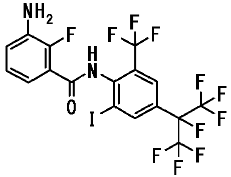

According to the method of 1-5 of Example 1, a target compound was prepared from 2-fluoro-N-(2-iodo-4-(perfluoropropan-2-yl)-6-(trifluoromethyl)phenyl)-3-nitrobenzamide.

¹H-NMR (CDCl₃, ppm) δ 3.92 (2H, broad-s), 7.02-7.04 (1H, m), 7.11 (1H, t, J=7.8 Hz), 7.47-7.52 (1H, m), 7.94 (1H, s), 8.30-8.35 (2H, m).

### <15-4>

### Preparation of 2-fluoro-N-(2-iodo-4-(perfluoropropan-2-yl)-6-(trifluoromethyl)phenyl)-3-(methylamino)benzamide

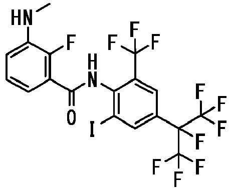

According to the method of 1-6 of Example 1, a target compound was prepared from 3-amino-2-fluoro-N-(2-iodo-4-(perfluoropropan-2-yl)-6-(trifluoromethyl)phenyl)benzamide.

¹H-NMR (CDCl₃, ppm) δ 2.95 (3H, s), 4.15 (1H, broad-s), 6.90 (1H, t, J=8.2 Hz), 7.19 (1H, t, J=7.8 Hz), 7.40 (1H, t, J=7.8 Hz), 7.92 (1H, s), 8.30 (1H, s), 8.34 (1H, s).

### <15-5>

### Preparation of 2-fluoro-3-(4-fluoro-N-methylbenzamide)-N-(2-iodo-4-(perfluoropropan-2-yl)-6-(trifluoromethyl)phenyl)benzamide (Compound No. 7-1733)

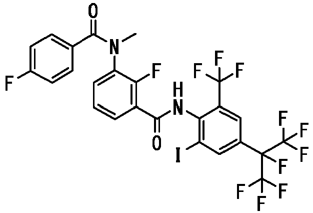

According to the method of 1-7 of Example 1, a target compound was prepared from 2-fluoro-N-(2-iodo-4-(perfluoropropan-2-yl)-6-(trifluoromethyl)phenyl)-3-(methylamino)benzamide and 4-fluorobenzoyl chloride.

¹H-NMR (CDCl₃, ppm) δ 3.50 (3H, s), 6.91 (2H, s), 6.93-7.35 (3H, m), 7.46 (1H, t, J=7.0 Hz), 7.93 (1H, s), 8.01-8.10 (1H, m), 8.13 (1H, broad-s), 8.34 (1H, s).

### <Example 16>

### Preparation of N-(2-bromo-4-(perfluoropropan-2-yl)-6-(trifluoromethyl)phenyl)-2-fluoro-N-methyl-3-(N-methylbenzamide)benzamide (Compound No. 9-2164)

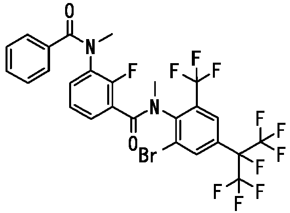

To a solution of 0.100 g (0.150 mmol) of N-(2-bromo-4-(perfluoropropan-2-yl)-6-(trifluoromethyl)phenyl)-2-fluoro-3-(N-methylbenzamide)benzamide obtained in 1-7 of Example 1 in 5 ml of DMF was added 0.00900 g (0.230 mmol) of sodium hydride (60% in oil), followed by stirring at room temperature for 40 minutes. To the reaction liquid was added 0.0300 g (0.180 mmol) of methyl iodide, followed by stirring at the same temperature for 6 hours. To the reaction liquid were added water and ethyl acetate, and the organic layer was washed with water and saturated brine, and dried over anhydrous magnesium sulfate. The solvent was evaporated under reduced pressure and the obtained residue was purified by silica gel column chromatography (eluent solvent; hexane:ethyl acetate=4:1) to prepare 0.106 g of a target compound quantitatively.

¹H-NMR (CDCl₃, ppm) δ 2.18-2.19 (3H, m), 3.48 (3H, s), 7.21-7.25(4H, m), 7.32-7.40(4H, m), 7.92 (1H, s), 8.13 (1H, s).

### <Example 17>

### Preparation of N-(2-bromo-4-(perfluoropropan-2-yl)-6-(trifluoromethyl)phenyl)-3-(4-cyanobenzamide)-N-methylbenzamide (Compound No. 8-289)

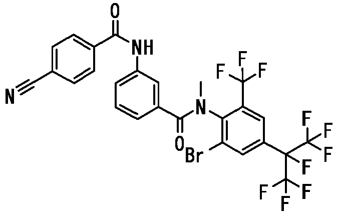

### <17-1>

### Preparation of N-(2-bromo-4-(perfluoropropan-2-yl)-6-(trifluoromethyl)phenyl)-3-nitrobenzamide

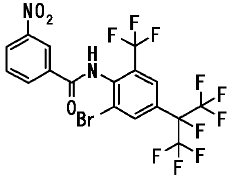

According to the method of 4-3 of Example 4, a target compound was prepared from 2-bromo-4-(perfluoropropan-2-yl)-6-(trifluoromethyl)aniline obtained in 1-2 of Example 1 and 3-nitrobenzoyl chloride.

¹H-NMR (CDCl₃, ppm) δ 7.75-7.79 (2H, m), 7.94 (1H, s), 8.17 (1H, d, J=1.0 Hz), 8.28 (1H, dd, J=1.5, 7.8 Hz), 8.48-8.51 (1H, m), 8.76-8.77 (1H, m).

### <17-2>

### Preparation of N-(2-bromo-4-(perfluoropropan-2-yl)-6-(trifluoromethyl)phenyl)-N-methyl-3-nitrobenzamide

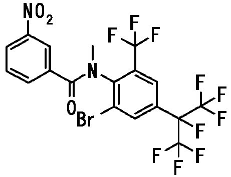

According to the method of Example 16, a target compound was prepared from N-(2-bromo-4-(perfluoropropan-2-yl)-6-(trifluoromethyl)phenyl)-3-nitrobenzamide.

¹H-NMR (CDCl₃, ppm) δ 3.28(1/2^{∗}3H, s), 3.44(1/2^{∗}3H, s), 7.41(1/2^{∗}1H, t, J=7.8 Hz), 7.71-7.76(2/2^{∗}1H, m), 7.84(1/2^{∗}1H, s), 7.93-7.95(1/2^{∗}1H, m), 7.98(1/2^{∗}1H, s), 8.07-8.09(2/2^{∗}1H, m), 8.14-8.16(1/2^{∗}1H, m), 8.19(1/2^{∗}1H, s), 8.39-8.41(1/2^{∗}1H, m), 8.45-8.46(1/2^{∗}1H, m).

### <17-3>

### Preparation of 3-amino-N-(2-bromo-4-(perfluoropropan-2-yl)-6-(trifluoromethyl)phenyl)-N-methylbenzamide

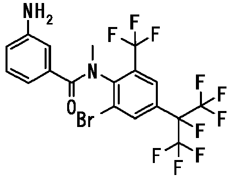

According to the method of 1-5 of Example 1, a target compound was prepared from N-(2-bromo-4-(perfluoropropan-2-yl)-6-(trifluoromethyl)phenyl)-N-methyl-3-nitrobenzamide.

¹H-NMR (CDCl₃, ppm) δ 3.24(3/4^{∗}3H, s), 3.37(1/4^{∗}3H, s), 3.80 (2H, broad-s), 6.47(1/4^{∗}1H, d, J=7.8 Hz), 6.54-6.57(1/4^{∗}1H, m), 6.78-6.84(5/4^{∗}1H, m), 6.86(3/4^{∗}1H, t, J=2.0 Hz), 6.96(3/4^{∗}1H, d, J=7.8 Hz), 7.23-7.27(3/4^{∗}1H, m), 7.79(1/4^{∗}1H, s), 7.94(3/4^{∗}1H, s), 8.00(1/4^{∗}1H, s), 8.15(3/4^{∗}1H, s).

### <17-4>

### Preparation of N-(2-bromo-4-(perfluoropropan-2-yl)-6-(trifluoromethyl)phenyl)-3-(4-cyanobenzamide)-N-methylbenzamide (Compound No. 8-289)

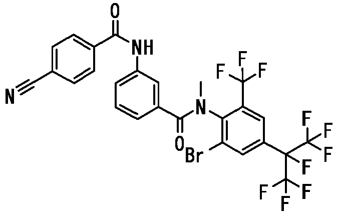

According to the method of 1-7 of Example 1, a target compound was prepared from 3-amino-N-(2-bromo-4-(perfluoropropan-2-yl)-6-(trifluoromethyl)phenyl)-N-methylbenzamide and 4-cyanobenzoylchloride.

¹H-NMR (CDCl₃, ppm) δ 3.26 (2/3^{∗}3H, s), 3.38 (1/3^{∗}3H, s), 7.08-7.09 (1/3^{∗}1H, m), 7.12-7.14 (1/3^{∗}1H, m), 7.32 (2/3^{∗}1H, d, J=7.8 Hz), 7.45-7.49 (3/3^{∗}1H, m), 7.72-7.76 (9/3^{∗}1H, m), 7.83 (1/3^{∗}1H, s), 7.85-7.89 (4/3^{∗}1H, m), 7.95 (2/3^{∗}1H, s), 7.98-8.00 (4/3^{∗}1H, m), 8.04 (2/3^{∗}1H, d, J=6.3 Hz), 8.16 (2/3^{∗}1H, s), 8.57 (2/3^{∗}1H, s).

### <Example 18>

### Preparation of 3-benzamide-N-(2-bromo-4-(perfluoroethyl)-6-(trifluoromethyl)phenyl)benzamide (Compound No. 6-5913)

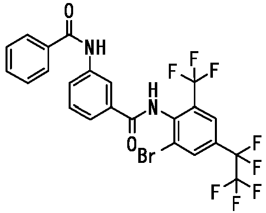

### <18-1>

### Preparation of 4-(perfluoroethyl)-2-(trifluoromethyl)aniline

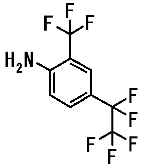

To 40 ml of an aqueous solution of 7.04 g (40.4 mmol) of 85% sodium hydrosulfite and 3.40 g (40.4 mmol) of sodium hydrogen carbonate were added 13.6 g (33.7 mmol) of 2-(trifluoromethyl)aniline and 40 ml of DMF. To this reaction liquid was added 50 ml of a solution of 11.2 g (45.5 mmol) of 1,1,2,2,2-pentafluoroethyliodide in DMF (DMF was cooled to -30°C, and 1,1,2,2,2-pentafluoroethyl iodide was dissolved therein), and placed in an autoclave, followed by stirring at 110°C for 9 hours. After being left to stand at room temperature overnight, water and ethyl acetate were added to the reaction liquid, followed by extraction with ethyl acetate. The organic layer was washed with water, a saturated aqueous sodium hydrogen carbonate solution, and saturated brine. The organic layer was dried over anhydrous sodium sulfate, then the solvent was evaporated under reduced pressure, and the obtained residue was purified by silica gel column chromatography (eluent solvent; hexane:ethyl acetate=10:1→ 5:1) to prepare 1.95 g (yield 21%) of a target compound.

¹H-NMR (CDCl₃, ppm) δ 4.53 (2H, broad-s), 6.81 (1H, d, J=8.3 Hz), 7.48 (1H, d, J=8.3 Hz), 7.63 (1H, broad-s).

### <18-2>

### Preparation of 2-bromo-4-(perfluoroethyl)-6-(trifluoromethyl)aniline

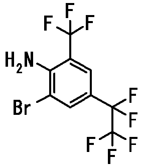

According to the method of 1-2 of Example 1, a target compound was prepared from 4-(perfluoroethyl)-2-(trifluoromethyl)aniline.

¹H-NMR (CDCl₃, ppm) δ 5.08 (2H, broad-s), 7.62 (1H, s), 7.80 (1H, s).

### <18-3>

### Preparation of N-(2-bromo-4-(perfluoroethyl)-6-(trifluoromethyl)phenyl)-3 - nitrobenzamide

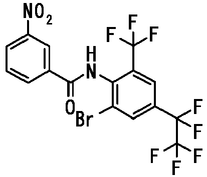

To a solution of 2.50 g (6.99 mmol) of 2-bromo-4-(perfluoroethyl)-6-(trifluoromethyl)aniline in 20 ml of pyridine was added 2.72 g (14.7 mmol) of 3-nitrobenzoyl chloride, followed by stirring at 100°C for 12 hours. To the reaction liquid were added water and ethyl acetate, followed by extraction with ethyl acetate. The organic layer was washed with 1 M hydrochloric acid, a saturated aqueous sodium hydrogen carbonate solution, and saturated brine. The organic layer was dried over anhydrous sodium sulfate, and then the solvent was evaporated under reduced pressure. To the obtained residue were added THF and an aqueous sodium hydroxide solution, followed by stirring at room temperature for 8 hours. The reaction liquid was extracted/dried in the same manner as described above, and the solvent was evaporated under reduced pressure. The obtained residue was purified by silica gel column chromatography (eluent solvent; hexane: ethyl acetate=7:1→5:1) to prepare 0.202 g (yield 6%) of a target compound.

¹H-NMR (CDCl₃, ppm) δ 7.75 (1H, s), 7.78 (1H, t, J=7.8 Hz), 7.94 (1H, s), 8.17 (1H, s), 8.29-8.30 (1H, m), 8.50-8.52 (1H, m), 8.78 (1H, t, J=2.0 Hz).

### <18-4>

### Preparation of 3-amino-N-(2-bromo-4-(perfluoroethyl)-6-(trifluoromethyl)phenyl)benzamide

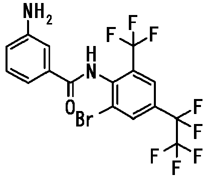

According to the method of 1-5 of Example 1, a target compound was prepared from N-(2-bromo-4-(perfluoroethyl)-6-(trifluoromethyl)phenyl)-3-nitrobenzamide.

¹H-NMR (CDCl₃, ppm) δ 3.89 (2H, broad-s), 6.90-6.92 (1H, m), 7.23-7.32 (3H, m), 7.64 (1H, s), 7.90 (1H, s), 8.13 (1H, s).

### <18-5>

### Preparation of 3-benzamide-N-(2-bromo-4-(perfluoroethyl)-6-(trifluoromethyl)phenyl)benzamide (Compound No. 6-5913)

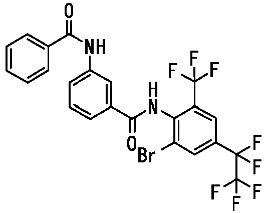

According to the method of 1-7 of Example 1, a target compound was prepared from 3-amino-N-(2-bromo-4-(perfluoroethyl)-6-(trifluoromethyl)phenyl)benzamide.

¹H-NMR (CDCl₃, ppm) δ 7.51-7.62(4H, m), 7.72 (1H, dd, J=1.5, 7.8 Hz), 7.89-8.00 (6H, m), 8.14 (1H, s), 8.27 (1H, t, J=2.0 Hz).

### <Example 19>

### Preparation of 3-benzamide-N-(2-bromo-4-(perfluoropropan-2-yl)-6-(trifluoromethyl)phenyl)-4-cyanobenzamide (Compound No. 1-627)

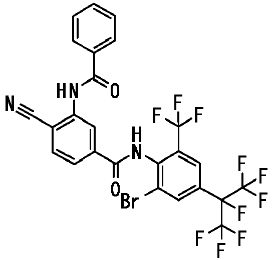

### <19-1>

### Preparation of N-(2-bromo-4-(perfluoropropan-2-yl)-6-(trifluoromethyl)phenyl)-4-fluoro-3 -nitrobenzamide

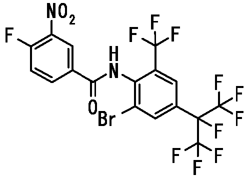

According to the method of 4-3 of Example 4, a target compound was prepared from 4-fluoro-3-nitrobenzoyl chloride prepared from 4-fluoro-3-nitrobenzoic acid and thionyl chloride, and 2-bromo-4-(perfluoropropan-2-yl)-6-(trifluoromethyl)aniline obtained in 1-2 of Example 1.

¹H-NMR (CDCl₃, ppm) δ 7.47-7.50 (1H, m), 7.92 (2H, d, J=5.9 Hz), 8.16 (1H, s), 8.23-8.28 (1H, m), 8.65-8.67 (1H, m).

### <19-2>

### Preparation of N-(2-bromo-4-(perfluoropropan-2-yl)-6-(trifluoromethyl)phenyl)-4-cyano-3 -nitrobenzamide

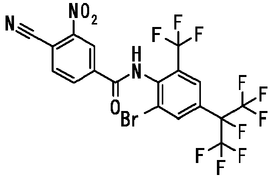

To a solution of 0.500 g (0.870 mmol) of N-(2-bromo-4-(perfluoropropan-2-yl)-6-(trifluoromethyl)phenyl)-4-fluoro-3-nitrobenzamide in 5 ml of DMF was added 0.0639 g (1.31 mmol) of sodium cyanide, followed by stirring at room temperature for 10 hours. To the reaction liquid were added water and ethyl acetate, followed by extraction with ethyl acetate. The organic layer was washed with a 10% aqueous sodium hydroxide solution and saturated brine. The organic layer was dried over anhydrous sodium sulfate, and then the solvent was evaporated under reduced pressure. The obtained residue was purified by silica gel column chromatography to prepare 0.0500 g (yield 10%) of a target compound.

¹H-NMR (CDCl₃, ppm) δ 7.80 (1H, s), 7.96 (1H, s), 8.12-8.14 (1H, m), 8.18 (1H, s), 8.36 (1H, dd, J=2.0, 8.3 Hz), 8.84 (1H, d, J=1.5 Hz).

### <19-3>

### Preparation of 3-amino-N-(2-bromo-4-(perfluoropropan-2-yl)-6-(trifluoromethyl)phenyl)-4-cyano benzamide

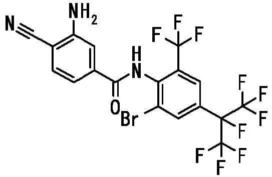

According to the method of 1-5 of Example 1, a target compound was prepared from N-(2-bromo-4-(perfluoropropan-2-yl)-6-(trifluoromethyl)phenyl)-4-cyano-3-nitrobenzamide.

¹H-NMR (CDCl₃, ppm) δ 4.68 (2H, broad-s), 7.18 (1H, dd, J=1.9, 8.3 Hz), 7.29 (1H, s), 7.52-7.55 (1H, m), 7.68 (1H, s), 7.92 (1H, s), 8.14 (1H, d, J=1.5 Hz).

### <19-4>

### Preparation of 3-benzamide-N-(2-bromo-4-(perfluoropropan-2-yl)-6-(trifluoromethyl)phenyl)-4-cyanobenzamide (Compound No. 1-627)

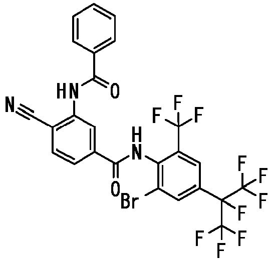

According to the method of 1-7 of Example 1, a target compound was prepared from 3-amino-N-(2-bromo-4-(perfluoropropan-2-yl)-6-(trifluoromethyl)phenyl)-4-cyanobenzamide.

¹H-NMR (CDCl₃, ppm) δ 7.56-7.59 (3H, m), 7.64-7.66 (1H, m), 7.80-7.87 (2H, m), 7.94-7.97 (2H, m), 8.16 (1H, s), 8.46 (1H, s), 8.57 (1H, s), 9.16 (1H, s).

Next, Preparation Examples in which the compound of the present invention is contained as an active ingredient will be shown, but the present invention is not limited thereto. Further, in Preparation Examples, parts represent parts by weight.

### <Preparation Example 1>

20 parts of the compound represented by Formula (1) of the present invention, 10 parts of polyoxyethylene styrylphenyl ether, and 70 parts of xylene were mixed uniformly to obtain an emulsion.

### <Preparation Example 2>

10 parts of the compound represented by Formula (1) of the present invention, 2 parts of sodium lauryl sulfate, 2 parts of dialkyl sulfosuccinate, 1 part of a β-naphthalene sulfonic acid formalin condensate sodium salt, and 85 parts of diatomaceous earth were stirred and mixed uniformly to obtain a wettable powder.

### <Preparation Example 3>

0.3 parts of the compound represented by Formula (1) of the present invention and 0.3 parts of white carbon were mixed uniformly, and 99.2 parts of clay and 0.2 parts of DRILESS A (manufactured by Sankyo Agro Co., Ltd.) were added thereto, followed by pulverizing and mixing uniformly, thereby obtaining powders.

### <Preparation Example 4>

3 parts of the compound represented by Formula (1) of the present invention, 1.5 parts of a polyoxyethylene/polyoxypropylene condensate, 3 parts of carboxymethyl cellulose, 64.8 parts of clay, and 27.7 parts of talc were pulverized and mixed uniformly, and water was added thereto, followed by kneading, granulating, and drying, thereby obtaining powders.

### <Preparation Example 5>

10 parts of the compound represented by Formula (1) of the present invention, 3 parts of a β-naphthalene sulfonic acid formalin condensate sodium salt, 1 part of tristyrylphenol, 5 parts of propylene glycol, 0.5 parts of a silicon-based defoaming agent, and 33.5 parts of water were sufficiently stirred and mixed, and then 0.3 parts of xanthan gum and 46.7 parts of water were mixed therewith, followed by stirring and mixing again, thereby obtaining a flowable Formulation.

### <Preparation Example 6>

20 parts of the compound represented by Formula (1) of the present invention, 6 parts of a naphthalene sulfonic acid formaldehyde condensate metal salt, 1 part of dialkylsulfosuccinate metal salt, and 73 parts of calcium carbonate were pulverized and mixed uniformly, and water was added thereto, followed by kneading, granulating, and drying, thereby obtaining a granule wettable powder.

For the use of Formulations obtained above, Formulation was diluted to 1-fold to 10000-fold with water or was not diluted, and then directly sprayed.

Next, the usefulness of the compound of the present invention as a pest control agent will be described specifically with reference to the following Test Examples, but the present invention is not limited thereto.

### <Test Example 1>

### Pesticidal Test against Spodoptera litura

A piece of a cabbage leaf was immersed for 30 seconds in a chemical solution in which a test compound had been prepared at a predetermined concentration, and air-dried, and then put into a 7 cm polyethylene cup having a filter paper laid on the bottom thereof, and 2-stage larvae of *Spodoptera litura* were released. They were left to stand in a thermostatic chamber at 25°C, and the numbers of the living pests and the dead pests were examined after 6 days. The test was carried out with five larvae per group in two replicates.

Further, the following compound (A) disclosed in the pamphlet of International Publication WO 2005/073165 was used as a comparative compound.

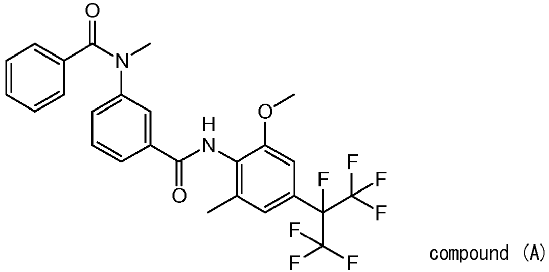

As the results of the above tests, the compounds of Compound No. 1-470, 1-491,1-513, 1-627, 1-1925, 2-491, 6-38, 6-39, 6-45, 6-46, 6-47, 6-57, 6-60, 6-71, 6-72, 6-78, 6-79, 6-80, 6-90, 6-93, 6-111, 6-147, 6-268, 6-269, 6-270, 6-271, 6-272, 6-288, 6-289, 6-290, 6-293, 6-300, 6-304, 6-306, 6-311, 6-346, 6-347, 6-348, 6-349, 6-366, 6-367, 6-368, 6-382, 6-389, 6-424, 6-425, 6-426, 6-427, 6-428, 6-444, 6-445, 6-446, 6-448, 6-449, 6-450, 6-452, 6-453, 6-460, 6-461, 6-467, 6-502, 6-503, 6-504, 6-505, 6-522, 6-523, 6-524, 6-538, 6-545, 6-804, 6-824, 6-825, 6-840, 6-1104, 6-1105, 6-1106, 6-1107, 6-1124, 6-1125, 6-1126, 6-1140, 6-1147, 6-1182, 6-1183, 6-1184, 6-1185, 6-1202, 6-1203, 6-1204, 6-1218, 6-1225, 6-1260, 6-1261, 6-1262, 6-1263, 6-1280, 6-1281, 6-1282, 6-1296, 6-1303, 6-1338, 6-1339, 6-1340, 6-1341, 6-1358, 6-1359, 6-1360, 6-1374, 6-1381, 6-1574, 6-1575, 6-1576, 6-1577, 6-1594, 6-1595, 6-1596, 6-1610, 6-1617, 6-1652, 6-1653, 6-1654, 6-1655, 6-1672, 6-1673, 6-1674, 6-1688, 6-1695, 6-1730, 6-1731, 6-1732, 6-1733, 6-1734, 6-1750, 6-1751, 6-1752, 6-1754, 6-1755, 6-1766, 6-1773, 6-1808, 6-1809, 6-1810, 6-1811, 6-1828, 6-1829, 6-1830, 6-1851, 6-2110, 6-3348, 6-3384, 6-5902, 6-5903, 6-5904, 6-5905, 6-5906, 6-5907, 6-5908, 6-5909, 6-5910, 6-5911, 6-5912, 7-38, 7-39, 7-45, 7-46, 7-47, 7-57, 7-60, 7-71, 7-72, 7-78, 7-79, 7-80, 7-90, 7-93, 7-132, 7-147, 7-268, 7-269, 7-270, 7-271, 7-285, 7-286, 7-288, 7-289, 7-290, 7-299, 7-300, 7-301, 7-303, 7-304, 7-305, 7-311, 7-346, 7-347, 7-348, 7-349, 7-366, 7-367, 7-368, 7-382, 7-389, 7-424, 7-425, 7-426, 7-427, 7-428, 7-444, 7-445, 7-446, 7-449, 7-460, 7-467, 7-502, 7-504, 7-505, 7-522, 7-523, 7-538, 7-804, 7-824, 7-825, 7-840, 7-948, 7-969, 7-984, 7-1104, 7-1105, 7-1106, 7-1107, 7-1124, 7-1125, 7-1126, 7-1140, 7-1147, 7-1182, 7-1183, 7-1184, 7-1185, 7-1202, 7-1203, 7-1204, 7-1218, 7-1225, 7-1260, 7-1261, 7-1262, 7-1263, 7-1280, 7-1281, 7-1282, 7-1296, 7-1303, 7-1338, 7-1339, 7-1340, 7-1341, 7-1358, 7-1359, 7-1360, 7-1374, 7-1381, 7-1417, 7-1574, 7-1575, 7-1576, 7-1577, 7-1594, 7-1595, 7-1596, 7-1605, 7-1606, 7-1608, 7-1610, 7-1616, 7-1617, 7-1638, 7-1639, 7-1645, 7-1652, 7-1672, 7-1673, 7-1730, 7-1731, 7-1732, 7-1733, 7-1750, 7-1751, 7-1752, 7-1764, 7-1766, 7-1773, 7-1794, 7-1808, 7-1809, 7-1810, 7-1811, 7-1828, 7-1829, 7-1844, 7-1851, 7-3348, 7-3369, 7-3384, 7-5902, 7-5903, 7-5904, 7-5905, 7-5906, 7-5907, 7-5910, 8-268, 8-288, 8-289, 8-304, 8-804, 8-824, 8-840, 9-270, 9-290, 9-291, 9-306, 9-2164, 9-2322, 11-777, 11-835, 12-777, 12-835, 12-864, 27-627, and 27-663 each showed a pesticidal rate of 70% or more at a concentration of 1 ppm.

Furthermore, the compounds of Compound No. 1-506, 1-1968, 2-513, 2-518, 6-454, 6-5913, 6-5914, 7-316, 7-5911, 7-5912, 7-5908, 7-5909, 17-1103, and 27-628 each showed a pesticidal rate of 70% or more at a concentration of 10 ppm.

Meanwhile, the compound (A) had a pesticidal rate of 50% or less at a concentration of 10 ppm.

### <Test Example 2>

### Pesticidal Test against Plutella xylostella

A piece of a cabbage leaf was immersed for 30 seconds in a chemical solution in which a test compound had been prepared at a predetermined concentration, and air-dried, and then put into a 7 cm polyethylene cup having a filter paper laid on the bottom thereof, and 2-stage larvae of *Plutella xylostella* were released. They were left to stand in a thermostatic chamber at 25°C, and the numbers of the living pests and the dead pests were examined after 6 days. The test was carried out with five larvae per group in two replicates.

Further, the above compound (A) was used as a comparative compound.

As the results of the above tests, the compounds of Compound No. 1-491,1-513, 1-627, 1-1925, 1-1968, 2-491,2-513, 6-38, 6-39, 6-45, 6-46, 6-47, 6-57, 6-71, 6-72, 6-78, 6-79, 6-80, 6-90, 6-111, 6-147, 6-268, 6-270, 6-271, 6-272, 6-288, 6-289, 6-290, 6-293, 6-300, 6-304, 6-306, 6-346, 6-347, 6-348, 6-349, 6-366, 6-367, 6-368, 6-382, 6-389, 6-424, 6-425, 6-426, 6-427, 6-428, 6-444, 6-445, 6-446, 6-449, 6-450, 6-453, 6-460, 6-461, 6-467, 6-502, 6-503, 6-504, 6-505, 6-522, 6-523, 6-524, 6-538, 6-545, 6-804, 6-824, 6-825, 6-840, 6-1104, 6-1105, 6-1106, 6-1107, 6-1124, 6-1125, 6-1126, 6-1140, 6-1147, 6-1182, 6-1183, 6-1184, 6-1185, 6-1202, 6-1203, 6-1204, 6-1218, 6-1225, 6-1260, 6-1261, 6-1262, 6-1263, 6-1280, 6-1281, 6-1282, 6-1296, 6-1303, 6-1338, 6-1339, 6-1340, 6-1341, 6-1358, 6-1359, 6-1360, 6-1374, 6-1381, 6-1574, 6-1575, 6-1576, 6-1577, 6-1594, 6-1595, 6-1596, 6-1610, 6-1617, 6-1652, 6-1653, 6-1654, 6-1655, 6-1672, 6-1673, 6-1674, 6-1688, 6-1695, 6-1730, 6-1731, 6-1732, 6-1733, 6-1734, 6-1750, 6-1751, 6-1752, 6-1754, 6-1755, 6-1766, 6-1773, 6-1808, 6-1809, 6-1810, 6-1811, 6-1828, 6-1829, 6-1830, 6-1851, 6-2110, 6-3348, 6-3384, 6-5903, 6-5904, 6-5905, 6-5906, 6-5907, 6-5908, 6-5909, 6-5910, 6-5911, 6-5912, 7-38, 7-39, 7-45, 7-46, 7-47, 7-57, 7-60, 7-71, 7-72, 7-78, 7-79, 7-80, 7-90, 7-93, 7-132, 7-147, 7-268, 7-271, 7-286, 7-288, 7-289, 7-290, 7-299, 7-300, 7-301, 7-303, 7-304, 7-305, 7-311, 7-346, 7-347, 7-348, 7-349, 7-366, 7-367, 7-368, 7-382, 7-389, 7-424, 7-425, 7-426, 7-427, 7-428, 7-444, 7-445, 7-446, 7-449, 7-460, 7-467, 7-502, 7-504, 7-505, 7-522, 7-523, 7-538, 7-824, 7-840, 7-948, 7-969, 7-984, 7-1104, 7-1105, 7-1106, 7-1107, 7-1124, 7-1125, 7-1126, 7-1140, 7-1147, 7-1182, 7-1183, 7-1184, 7-1185, 7-1202, 7-1203, 7-1204, 7-1218, 7-1225, 7-1260, 7-1261, 7-1262, 7-1263, 7-1280, 7-1281, 7-1282, 7-1296, 7-1303, 7-1338, 7-1339, 7-1340, 7-1341, 7-1358, 7-1359, 7-1360, 7-1374, 7-1381, 7-1417, 7-1574, 7-1575, 7-1576, 7-1577, 7-1594, 7-1595, 7-1596, 7-1605, 7-1606, 7-1608, 7-1610, 7-1616, 7-1617, 7-1638, 7-1639, 7-1645, 7-1652, 7-1672, 7-1673, 7-1730, 7-1731, 7-1732, 7-1733, 7-1750, 7-1751, 7-1752, 7-1764, 7-1766, 7-1773, 7-1794, 7-1808, 7-1809, 7-1810, 7-1811, 7-1828, 7-1829, 7-1844, 7-1851, 7-3348, 7-3369, 7-3384, 7-5902, 7-5903, 7-5904, 7-5905, 7-5906, 7-5907, 7-5909, 7-5910, 8-268, 8-288, 8-289, 8-304, 8-824, 9-270, 9-290, 9-291, 9-306, 9-2164, 9-2322, 11-777, 11-835, 12-777, 12-835, and 12-864 each showed a pesticidal rate of 70% or more at a concentration of 1 ppm.

Furthermore, the compounds of Compound No. 1-470, 1-506, 2-518, 6-60, 6-93, 6-269, 6-311, 6-448, 6-452, 6-454, 6-5913, 6-5914, 7-269, 7-270, 7-285, 7-316, 7-804, 7-825, 7-5908, 7-5911, 7-5912, 8-804, 8-840, 17-1103, 27-627, 27-628, and 27-663 each showed a pesticidal rate of 70% or more at a concentration of 10 ppm.

Meanwhile, the compound (A) had a pesticidal rate of 50% or less at a concentration of 10 ppm.

### <Test Example 3>

### Pesticidal Test against Adoxophyes honmai

A thinly sliced artificial feed was immersed for 30 seconds in a chemical solution in which a test compound had been prepared at a predetermined concentration, and air-dried, and then put into a 7 cm polyethylene cup having a filter paper laid on the bottom thereof, and 2-stage larvae of *Adoxophyes honmai* were released. They were left to stand in a thermostatic chamber at 25°C, and the numbers of the living pests and the dead pests were examined after 6 days. The test was carried out with five larvae per group in two replicates.

Further, the above compound (A) was used as a comparative compound.

As the results of the above tests, the compounds of Compound No. 6-72, 6-80, 6-90, 6-347, 6-348, 6-349, 6-366, 6-382, 6-427, 6-446, 6-449, 6-460, 6-503, 6-504, 6-505, 6-522, 6-524, 6-538, 6-1106, 6-1182, 6-1202, 6-1260, 6-1262, 6-1263, 6-1341, 6-1358, 6-1574, 6-1576, 6-1594, 6-1596, 6-1652, 6-1654, 6-1655, 6-1672, 6-1674, 6-1688, 6-1730, 6-1732, 6-1733, 6-1750, 6-1809, 6-1811, 6-1828, 6-1830, 7-348, 7-366, 7-382, 7-502, 7-505, 7-522, 7-538, 7-1106, 7-1202, 7-1262, 7-1280, 7-1303, 7-1338, 7-1358, 7-1574, 7-1576, 7-1594, 7-1605, 7-1652, 7-1730, 7-1732, 7-1733, 7-1750, 7-1764, 7-1808, 7-1809, 7-1810, and 7-1811 each showed a pesticidal rate of 70% or more at a concentration of 1 ppm.

Furthermore, the compounds of Compound No. 6-271, 6-367, 6-389, 6-426, 6-523, 6-1104, 6-1577, 6-1695, 6-1851, 6-2110, 7-424, 7-444, 7-523, 7-1104, 7-1124, 7-1260, 7-1263, 7-1577, 7-1616, 7-1731, 7-1773, and 7-1851 each showed a pesticidal rate of 70% or more at a concentration of 10 ppm.

Meanwhile, the compound (A) had a pesticidal rate of 50% or less at a concentration of 10 ppm.

### <Test Example 4>

### Pesticidal Test against Choristoneura magnanima

A thinly sliced artificial feed was immersed for 30 seconds in a chemical solution in which a test compound had been prepared at a predetermined concentration, and air-dried, and then put into a 7 cm polyethylene cup having a filter paper laid on the bottom thereof, and 2-stage larvae of *Choristoneura magnanima* were released. They were left to stand in a thermostatic chamber at 25°C, and the numbers of the living pests and the dead pests were examined after 6 days. The test was carried out with five larvae per group in two replicates.

Further, the above compound (A) was used as a comparative compound.

As the results of the above tests, the compounds of Compound No. 6-72, 6-80, 6-90, 6-271, 6-349, 6-366, 6-367, 6-426, 6-427, 6-1104, 6-1106, 6-1182, 6-1260, 6-1263, 6-1358, 6-1574, 6-1577, 6-1595, 6-1596, 6-1652, 6-1654, 6-1655, 6-1672, 6-1673, 6-1674, 6-1695, 6-1730, 6-1732, 6-1733, 6-1750, 6-1751, 6-1809, 6-1811, 6-1828, 6-1829, 6-1830, 6-1851, 7-90, 7-348, 7-366, 7-382, 7-389, 7-444, 7-502, 7-504, 7-505, 7-523, 7-1104, 7-1106, 7-1107, 7-1124, 7-1125, 7-1260, 7-1262, 7-1263, 7-1280, 7-1281, 7-1303, 7-1358, 7-1359, 7-1574, 7-1576, 7-1577, 7-1594, 7-1595, 7-1605, 7-1616, 7-1617, 7-1652, 7-1730, 7-1732, 7-1733, 7-1750, 7-1764, 7-1773, 7-1808, 7-1809, 7-1810, 7-1811, 7-1829, and 7-1851 each showed a pesticidal rate of 70% or more at a concentration of 1 ppm.

Furthermore, the compounds of Compound No. 6-382, 6-446, 6-460, 6-2110, 7-424, 7-538, and 7-1606 each showed a pesticidal rate of 70% or more at a concentration of 10 ppm.

Meanwhile, the compound (A) had a pesticidal rate of 50% or less at a concentration of 10 ppm.

### <Test Example 5>

### Pesticidal Test against Helicoverpa armigera

A cabbage leaf disk was immersed for 30 seconds in a chemical solution in which a test compound had been prepared at a predetermined concentration, and air-dried. The leaf disk was put into a 6-hole plastic cup having a filter paper laid on the bottom thereof, and 2-stage larvae of *Helicoverpa armigera* were released with one insect being released per hole. They were left to stand in a thermostatic chamber at 25°C, and the numbers of the living pests and the dead pests were examined after 3 days. The test was carried out with five larvae per group in two replicates.

Further, the above compound (A) was used as a comparative compound.

As the results of the above tests, the compounds of Compound No. 6-72, 6-80, 6-90, 6-271, 6-347, 6-348, 6-349, 6-366, 6-367, 6-382, 6-389, 6-426, 6-427, 6-446, 6-449, 6-460, 6-503, 6-504, 6-505, 6-522, 6-523, 6-524, 6-538, 6-1104, 6-1106, 6-1260, 6-1262, 6-1263, 6-1281, 6-1341, 6-1358, 6-1359, 6-1574, 6-1576, 6-1577, 6-1594, 6-1595, 6-1596, 6-1652, 6-1654, 6-1655, 6-1672, 6-1673, 6-1674, 6-1695, 6-1730, 6-1732, 6-1733, 6-1750, 6-1751, 6-1808, 6-1809, 6-1811, 6-1828, 6-1829, 6-1830, 6-1851, 6-2110, 7-90, 7-348, 7-366, 7-382, 7-389, 7-424, 7-444, 7-502, 7-505, 7-522, 7-523, 7-538, 7-1106, 7-1107, 7-1125, 7-1262, 7-1263, 7-1280, 7-1303, 7-1338, 7-1358, 7-1359, 7-1574, 7-1576, 7-1577, 7-1594, 7-1595, 7-1605, 7-1606, 7-1616, 7-1617, 7-1652, 7-1730, 7-1731, 7-1732, 7-1733, 7-1750, 7-1751, 7-1764, 7-1773, 7-1808, 7-1809, 7-1810, 7-1811, 7-1829, and 7-1851 each showed a pesticidal rate of 70% or more at a concentration of 1 ppm.

Furthermore, the compound of Compound No. 7-1104 showed a pesticidal rate of 70% or more at a concentration of 10 ppm.

Meanwhile, the compound (A) had a pesticidal rate of 50% or less at a concentration of 10 ppm.

### <Test Example 6>

### Pesticidal Test against Laodelphax striatellus

2.5 ml of an acetone solution in which a test compound had been prepared at a predetermined concentration was sprayed on rice seedlings, air-dried, and then put into a glass tube having a diameter of 3 cm and a height of 13 cm, having water therein, and 3-stage larvae of *Laodelphax striatellus* were released and the tube was capped. They were left to stand in a thermostatic chamber at 25°C, and the numbers of the living pests and the dead pests were examined after 6 days. The test was carried out with ten larvae per group in two replicates.

As the results of the above tests, the compounds of Compound No. 1-491, 1-627, 2-491, 6-39, 6-45, 6-47, 6-57, 6-71, 6-72, 6-79, 6-80, 6-90, 6-147, 6-268, 6-269, 6-270, 6-271, 6-290, 6-304, 6-306, 6-311, 6-346, 6-347, 6-368, 6-382, 6-425, 6-427, 6-444, 6-445, 6-446, 6-460, 6-461, 6-503, 6-1104, 6-1106, 6-1107, 6-1124, 6-1126, 6-1140, 6-1147, 6-1182, 6-1183, 6-1184, 6-1185, 6-1202, 6-1203, 6-1204, 6-1218, 6-1225, 6-1260, 6-1261, 6-1262, 6-1263, 6-1280, 6-1281, 6-1282, 6-1296, 6-1303, 6-1338, 6-1339, 6-1340, 6-1341, 6-1358, 6-1359, 6-1360, 6-1374, 6-1381, 6-1574, 6-1575, 6-1576, 6-1577, 6-1594, 6-1595, 6-1596, 6-1610, 6-1617, 6-1652, 6-1653, 6-1654, 6-1655, 6-1672, 6-1673, 6-1674, 6-1688, 6-1695, 6-1730, 6-1731, 6-1732, 6-1733, 6-1734, 6-1750, 6-1751, 6-1752, 6-1754, 6-1766, 6-1773, 6-1808, 6-1809, 6-1810, 6-1811, 6-1828, 6-1829, 6-1830, 6-1851, 6-2110, 6-3348, 6-3384, 6-5903, 6-5904, 6-5905, 6-5906, 6-5907, 6-5908, 6-5909, 6-5910, 6-5911, 6-5914, 7-47, 7-57, 7-90, 7-268, 7-285, 7-286, 7-288, 7-289, 7-301, 7-303, 7-304, 7-305, 7-311, 7-316, 7-346, 7-348, 7-368, 7-382, 7-389, 7-424, 7-426, 7-427, 7-444, 7-445, 7-446, 7-460, 7-467, 7-538, 7-825, 7-840, 7-948, 7-984, 7-1104, 7-1105, 7-1106, 7-1107, 7-1124, 7-1125, 7-1126, 7-1140, 7-1147, 7-1182, 7-1183, 7-1184, 7-1185, 7-1202, 7-1203, 7-1204, 7-1218, 7-1225, 7-1260, 7-1261, 7-1262, 7-1263, 7-1280, 7-1281, 7-1282, 7-1296, 7-1303, 7-1338, 7-1339, 7-1340, 7-1341, 7-1358, 7-1359, 7-1360, 7-1374, 7-1381, 7-1417, 7-1574, 7-1575, 7-1576, 7-1577, 7-1594, 7-1595, 7-1596, 7-1605, 7-1606, 7-1608, 7-1610, 7-1616, 7-1617, 7-1638, 7-1639, 7-1645, 7-1652, 7-1672, 7-1673, 7-1730, 7-1731, 7-1732, 7-1733, 7-1750, 7-1751, 7-1752, 7-1764, 7-1766, 7-1773, 7-1794, 7-1808, 7-1809, 7-1810, 7-1811, 7-1828, 7-1829, 7-1844, 7-1851, 7-3348, 7-3369, 7-5902, 7-5903, 7-5904, 7-5906, 7-5907, 7-5909, 7-5912, 8-289, 8-304, 8-840, 9-270, 9-290, 9-291, 9-306, 9-2164, 9-2322, 12-835, and 12-864 each showed a pesticidal rate of 70% or more at a concentration of 100 ppm.

### <Test Example 7>

### Pesticidal Test against Musca domestica

1 ml of an acetone solution in which a test compound had been prepared at a predetermined concentration was added dropwise to a petri dish having a diameter of 9 cm, and air-dried, and then the female adults of *Musca domestica* were released and the petri dish was capped. They were left to stand in a thermostatic chamber at 25°C, and the numbers of the living pests and the dead pests were examined after 1 day. The test was carried out with five larvae per group in two replicates.

As the results of the above tests, the compounds of Compound No. 1-1925, 1-1968, 6-39, 6-47, 6-72, 6-272, 6-293, 6-346, 6-347, 6-348, 6-366, 6-368, 6-382, 6-389, 6-426, 6-427, 6-428, 6-450, 6-452, 6-453, 6-460, 6-503, 6-504, 6-505, 6-522, 6-523, 6-524, 6-538, 6-1105, 6-1107, 6-1124, 6-1125, 6-1126, 6-1147, 6-1184, 6-1204, 6-1218, 6-1260, 6-1281, 6-1338, 6-1341, 6-1358, 6-1359, 6-1360, 6-1374, 6-1575, 6-1576, 6-1577, 6-1594, 6-1596, 6-1617, 6-1652, 6-1672, 6-1673, 6-1695, 6-1730, 6-1731, 6-1732, 6-1733, 6-1734, 6-1750, 6-1751, 6-1752, 6-1773, 6-1808, 6-1809, 6-1810, 6-1811, 6-1828, 6-1829, 6-1830, 6-1851, 6-2110, 6-5905, 7-39, 7-57, 7-90, 7-268, 7-346, 7-348, 7-349, 7-366, 7-367, 7-368, 7-382, 7-389, 7-424, 7-428, 7-444, 7-446, 7-449, 7-504, 7-522, 7-538, 7-1104, 7-1105, 7-1106, 7-1107, 7-1126, 7-1262, 7-1280, 7-1282, 7-1296, 7-1303, 7-1338, 7-1340, 7-1358, 7-1359, 7-1360, 7-1381, 7-1417, 7-1574, 7-1575, 7-1576, 7-1577, 7-1596, 7-1652, 7-1731, 7-1732, 7-1733, 7-1750, 7-1752, 7-1764, 7-1766, 7-1730, 7-1773, 7-1794, 7-1808, 7-1809, 7-1810, 7-1811, 7-1828, 7-1829, 7-1851, 9-2164, 9-2322, 11-777, 11-835, 12-777, 12-835, 12-864, 17-1103, and 27-627 each showed a pesticidal rate of 70% or more at a concentration of 100 ppm.

### <Test Example 8>

### Pesticidal Test against Blattella germanica

1 ml of an acetone solution in which a test compound had been prepared at a predetermined concentration was added dropwise to a petri dish having a diameter of 9 cm, and air-dried, and then the female adults of *Blattella germanica* were released and the petri dish was capped. They were left to stand in a thermostatic chamber at 25°C, and the numbers of the living pests and the dead pests were examined after 1 day. The test was carried out with five larvae per group in two replicates.

As the results of the above tests, the compounds of Compound No. 1-1925, 6-39, 6-47, 6-346, 6-347, 6-367, 6-368, 6-382, 6-389, 6-426, 6-427, 6-503, 6-538, 6-1105, 6-1106, 6-1107, 6-1124, 6-1125, 6-1126, 6-1147, 6-1184, 6-1204, 6-1218, 6-1280, 6-1296, 6-1338, 6-1358, 6-1359, 6-1575, 6-1576, 6-1577, 6-1594, 6-1595, 6-1596, 6-1610, 6-1617, 6-1730, 6-1731, 6-1732, 6-1733, 6-1750, 6-1751, 6-1752, 6-1773, 6-1811, 6-5905, 7-57, 7-268, 7-305, 7-347, 7-349, 7-367, 7-382, 7-389, 7-424, 7-444, 7-446, 7-502, 7-505, 7-538, 7-1104, 7-1105, 7-1106, 7-1107, 7-1125, 7-1126, 7-1261, 7-1262, 7-1280, 7-1282, 7-1296, 7-1303, 7-1381, 7-1574, 7-1575, 7-1576, 7-1577, 7-1594, 7-1595, 7-1596, 7-1617, 7-1731, 7-1732, 7-1733, 7-1750, 7-1752, 7-1766, 7-1773, and 7-1851 each showed a pesticidal rate of 70% or more at a concentration of 100 ppm.

### <Test Example 9>

### Pesticidal Test against Culex pipiens molestus

1 ml of an acetone solution in which a test compound had been prepared at a predetermined concentration was added dropwise to a petri dish having a diameter of 9 cm, and air-dried, and then the female adults of *Culex pipiens molestus* were released and the petri dish was capped. They were left to stand in a thermostatic chamber at 25°C, and the numbers of the living pests and the dead pests were examined after 1 day of treatment. The test was carried out with five larvae per group in two replicates.

As the results of the above tests, the compounds of Compound No.7-424, 7-1574, 7-1577, 7-1730, 7-1732, and 7-1733 each showed a pesticidal rate of 70% or more at a concentration of 1000 ppm.

### <Test Example 10>

### Pesticidal Test against Coptotermes formosanus

20 µl of an acetone solution in which a test compound had been prepared at a predetermined concentration was added dropwise to the filter paper having a diameter of 2.6 mm included in a polypropylene cup, and air-dried, and then 20 µl of water was added thereto. *Coptotermes formosanus* was released and the cup was capped. They were left to stand in a thermostatic chamber at 28°C, and the numbers of the living pests and the dead pests were examined after 5 days of treatment. The test was carried out with ten larvae per group in two replicates.

As the results of the above tests, the compounds of Compound No.6-460, 6-1260, 6-1652, 7-268, 7-424, 7-1104, 7-1574, 7-1577, 7-1730, 7-1732, and 7-1733 each showed a pesticidal rate of 70% or more at a concentration of 30 ppm.

### Industrial Applicability

According to the present invention, it becomes possible to provide a novel pest control agent comprising an amide derivative. The amide derivative shows a significant effect for a pest control activity, and has a high industrial availability.

## Claims

1. A pest control agent comprising at least one amide derivative selected from the group consisting of the following Formula (1), Formula (7), Formula (8) and Formula (9) as an active ingredient: 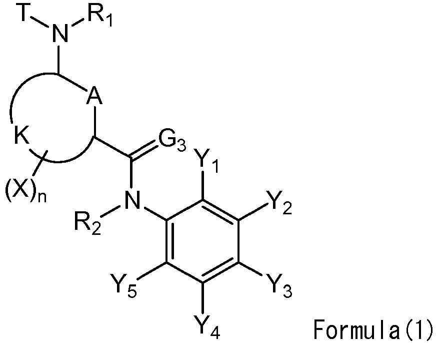 wherein, in Formula (1):
A represents a carbon atom, an oxygen atom, a nitrogen atom, an oxidized nitrogen atom, or a sulfur atom;
K represents a non-metal atom group necessary for forming a cyclic linking group derived from pyridine, pyridine-N-oxide, pyrrole, thiazole, furan, or thiophene in combination with A and two carbon atoms to which A bonds;
X represents a hydrogen atom, a halogen atom, a C1-C6 alkyl group, a C1-C6 haloalkyl group, a C3-C9 cycloalkyl group, a C3-C9 halocycloalkyl group, a C2-C6 alkenyl group, a C2-C6 haloalkenyl group, a C2-C6 alkynyl group, a C2-C6 haloalkynyl group, a C1-C6 alkoxy group, a C1-C6 haloalkoxy group, a C2-C6 alkenyloxy group, a C2-C6 haloalkenyloxy group, a C2-C6 alkynyloxy group, a C2-C6 haloalkynyloxy group, a C3-C9 cycloalkoxy group, a C3-C9 halocycloalkoxy group, a C1-C6 alkylthio group, a C1-C6 haloalkylthio group, a C1-C6 alkylsulfinyl group, a C1-C6 haloalkylsulfinyl group, a C1-C6 alkylsulfonyl group, a C1-C6 haloalkylsulfonyl group, a C1-C6 alkylsulfonyloxy group, a C1-C6 haloalkylsulfonyloxy group, a C2-C7 alkylcarbonyl group, a C2-C7 haloalkylcarbonyl group, a C3-C7 alkenylcarbonyl group, a C3-C7 haloalkenylcarbonyl group, a C3-C7 alkynylcarbonyl group, a C3-C7 haloalkynylcarbonyl group, a C4-C10 cycloalkylcarbonyl group, a C4-C10 halocycloalkylcarbonyl group, a C2-C7 alkylcarbonyloxy group, a C2-C7 haloalkylcarbonyloxy group, arylcarbonyloxy group, a C2-C7 alkoxycarbonyl group, a C2-C7 haloalkoxycarbonyl group, a C3-C7 alkenyloxycarbonyl group, a C3-C7 haloalkenyloxycarbonyl group, a C3-C7 alkynyloxycarbonyl group, a C3-C7 haloalkynyloxycarbonyl group, a C4-C10 cycloalkyloxycarbonyl group, a C4-C10 halocycloalkyloxycarbonyl group, a C2-C7 alkylcarbonylamino group, a C2-C7 haloalkylcarbonylamino group, a C2-C7 alkoxycarbonylamino group, a C2-C7 haloalkoxycarbonylamino group, a C2-C7 alkoxycarbonyloxy group, a C2-C7 haloalkoxycarbonyloxy group, an arylcarbonylamino group, an amino group, a carbamoyl group, a cyano group, a hydroxy group, pentafluorosulfanyl group, a C1-C6 alkylamino group, a C1-C6 haloalkylamino group, a C2-C6 alkenylamino group, a C2-C6 haloalkenylamino group, a C2-C6 alkynylamino group, a C2-C6 haloalkynylamino group, a C3-C9 cycloalkylamino group, a C3-C9 halocycloalkylamino group, a C2-C7 alkylaminocarbonyl group, a C2-C7 haloalkylaminocarbonyl group, a C3-C7 alkenylaminocarbonyl group, a C3-C7 haloalkenylaminocarbonyl group, a C3-C7 alkynylaminocarbonyl group, a C3-C7 haloalkynylaminocarbonyl group, a C4-C10 cycloalkylaminocarbonyl group, a C4-C10 halocycloalkylaminocarbonyl group, a phenyl group, or a heterocyclic group, and when there are plural X's, each X may be the same as or different from each other;
n represents an integer of from 0 to 4;
T represents -C(=G₁)-Q₁ or -C(=G₁)-G₂Q₂;
G₁ and G₂ each independently represent an oxygen atom or a sulfur atom;
Q₁ and Q₂ each represent:
a hydrogen atom, a C1-C6 alkyl group, a C1-C6 haloalkyl group, a C2-C6 alkenyl group, a C2-C6 haloalkenyl group, a C2-C6 alkynyl group, a C2-C6 haloalkynyl group, a C3-C9 cycloalkyl group, a C3-C9 halocycloalkyl group, a benzyl group, a phenyl group which may have a substituent, a naphthyl group, or a heterocyclic group which may have a substituent;
Y₁ and Y₅ represent a halogen atom or a C1-C3 haloalkyl group, and either Y₁ or Y₅ represents a C1-C3 haloalkyl group;
Y₂ and Y₄ represent a hydrogen atom;
Y₃ represents a C2-C4 perfluoroalkyl group;
wherein, in Qi and Q₂, the substituent of a phenyl group which may have a substituent and a heterocyclic group which may have a substituent represents one or more substituents selected from a group consisting of:
a halogen atom, a C1-C6 alkyl group, a C1-C6 haloalkyl group, a C3-C9 cycloalkyl group, a C3-C9 halocycloalkyl group, a C2-C6 alkenyl group, a C2-C6 haloalkenyl group, a C2-C6 alkynyl group, a C2-C6 haloalkynyl group, a C1-C6 alkoxy group, a C1-C6 haloalkoxy group, a C2-C6 alkenyloxy group, a C2-C6 haloalkenyloxy group, a C2-C6 alkynyloxy group, a C2-C6 haloalkynyloxy group, a C3-C9 cycloalkoxy group, a C3-C9 halocycloalkoxy group, a C1-C6 alkylthio group, a C1-C6 haloalkylthio group, a C1-C6 alkylsulfinyl group, a C1-C6 haloalkylsulfinyl group, a C1-C6 alkylsulfonyl group, a C1-C6 haloalkylsulfonyl group, a C2-C7 alkylcarbonyl group, a C2-C7 haloalkylcarbonyl group, a C3-C7 alkenylcarbonyl group, a C3-C7 haloalkenylcarbonyl group, a C3-C7 alkynylcarbonyl group, a C3-C7 haloalkynylcarbonyl group, a C4-C10 cycloalkylcarbonyl group, a C4-C10 halocycloalkylcarbonyl group, a C2-C7 alkylcarbonyloxy group, a C2-C7 haloalkylcarbonyloxy group, a C1-C6 alkylsulfonyloxy group, a C1-C6 haloalkylsulfonyloxy group, a C2-C7 alkoxycarbonyl group, a C2-C7 haloalkoxycarbonyl group, a C3-C7 alkenyloxycarbonyl group, a C3-C7 haloalkenyloxycarbonyl group, a C3-C7 alkynyloxycarbonyl group, a C3-C7 haloalkynyloxycarbonyl group, a C4-C10 cycloalkyloxycarbonyl group, a C4-C10 halocycloalkyloxycarbonyl group, a C2-C7 alkylcarbonylamino group, a C2-C7 haloalkylcarbonylamino group, a C2-C7 alkoxycarbonylamino group, a C2-C7 haloalkoxycarbonylamino group, a C1-C6 alkylamino group, a C1-C6 haloalkylamino group, a C2-C6 alkenylamino group, a C2-C6 haloalkenylamino group, a C2-C6 alkynylamino group, a C2-C6 haloalkynylamino group, a C3-C9 cycloalkylamino group, a C3-C9 halocycloalkylamino group, a C2-C7 alkylaminocarbonyl group, a C2-C7 haloalkylaminocarbonyl group, a C3-C7 alkenylaminocarbonyl group, a C3-C7 haloalkenylaminocarbonyl group, a C3-C7 alkynylaminocarbonyl group, a C3-C7 haloalkynylaminocarbonyl group, a C4-C10 cycloalkylaminocarbonyl group, a C4-C10 halocycloalkylaminocarbonyl group, an amino group, a carbamoyl group, a cyano group, a nitro group, a hydroxy group, a pentafluorosulfanyl group, a phenyl group which may have a substituent, and a heterocyclic group which may have a substituent, and when there are two or more substituents, the substituents may be the same as or different from each other;
wherein, the heterocyclic group in X, Q₁, and Q₂ represents a pyridyl group, a pyridine-N-oxide group, a pyrimidinyl group, a pyrazinyl group, a pyridazyl group, a furyl group, a thienyl group, an oxazolyl group, an isoxazolyl group, an oxadiazolyl group, a thiazolyl group, an isothiazolyl group, a thiadiazolyl group, a pyrrolyl group, an imidazolyl group, a triazolyl group, a pyrazolyl group, or a tetrazolyl group;
G₃ represents an oxygen atom or a sulfur atom; and
R₁ and R₂ each independently represents a hydrogen atom, an oxygen atom, a halogen atom, a hydroxy group, a nitro group, a nitroso group, a trimethylsilyl group, a t-butyldimethylsilyl group, a cyano group, an amino group, a C1-C6 alkyl group which may have a substituent, a C1-C6 haloalkyl group which may have a substituent, a C2-C7 alkylcarbonyl group, a C2-C7 haloalkylcarbonyl group, a C2-C6 alkenyl group which may have a substituent, a C2-C6 haloalkenyl group which may have a substituent, a C2-C6 alkynyl group which may have a substituent, a C2-C6 haloalkynyl group which may have a substituent, a C2-C7 alkoxycarbonyl group, a C2-C7 haloalkoxycarbonyl group, a C3-C7 alkenyloxycarbonyl group, a C3-C7 haloalkenyloxycarbonyl group, a C3-C7 alkynyloxycarbonyl group, a C3-C7 haloalkynyloxycarbonyl group, a phenoxycarbonyl group, a C2-C7 alkylaminocarbonyl group, a C2-C7 haloalkylaminocarbonyl group, a C2-C7 alkylcarbonyloxy group, a C2-C7 haloalkylcarbonyloxy group, a benzoyl group, a C1-C6 alkoxy group, a C1-C6 haloalkoxy group, a C1-C6 alkylthio group, a C1-C6 haloalkylthio group, a C1-C6 alkylsulfinyl group, a C1-C6 haloalkylsulfinyl group, a C1-C6 alkylsulfonyl group, a C1-C6 haloalkylsulfonyl group, a benzenesulfonyl group, a benzylsulfonyl group, a benzyl group, or -C(=O)C(=O)R₇, wherein R₇ represents a C1-C6 alkyl group, a C1-C6 haloalkyl group, a C1-C6 alkoxy group, or a C1-C6 haloalkoxy group; 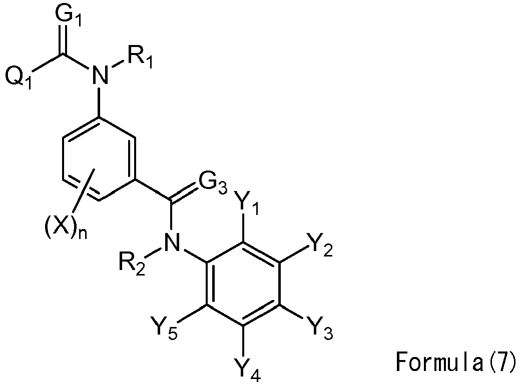
wherein in Formula (7), n represents 4;
X's each independently represent a hydrogen atom, a halogen atom, or cyano group;
Y₃ represents a C2-C4 perfluoroalkyl group;
Y₂ and Y₄ represent a hydrogen atom;
Y₁ and Y₅ represent a halogen atom or a C1-C3 haloalkyl group, and either Y₁ or Y₅ represents a C1-C3 haloalkyl group;
G₁ and G₃ each independently represent an oxygen atom or a sulfur atom;
Q₁ represents a hydrogen atom, a C1-C6 alkyl group, a C1-C6 haloalkyl group, a C2-C6 alkenyl group, a C2-C6 haloalkenyl group, a C2-C6 alkynyl group, a C2-C6 haloalkynyl group, a C3-C9 cycloalkyl group, a C3-C9 halocycloalkyl group, a benzyl group, a phenyl group which may have a substituent, a naphthyl group, or a heterocyclic group which may have a substituent;
wherein, in Q₁ the substituent of a phenyl group which may have a substituent and a substituents heterocyclic group which may have a substituent represents one or more substituents selected from a group consisting of:
a halogen atom, a C1-C6 alkyl group, a C1-C6 haloalkyl group, a C3-C9 cycloalkyl group, a C3-C9 halocycloalkyl group, a C2-C6 alkenyl group, a C2-C6 haloalkenyl group, a C2-C6 alkynyl group, a C2-C6 haloalkynyl group, a C1-C6 alkoxy group, a C1-C6 haloalkoxy group, a C2-C6 alkenyloxy group, a C2-C6 haloalkenyloxy group, a C2-C6 alkynyloxy group, a C2-C6 haloalkynyloxy group, a C3-C9 cycloalkoxy group, a C3-C9 halocycloalkoxy group, a C1-C6 alkylthio group, a C1-C6 haloalkylthio group, a C1-C6 alkylsulfinyl group, a C1-C6 haloalkylsulfinyl group, a C1-C6 alkylsulfonyl group, a C1-C6 haloalkylsulfonyl group, a C2-C7 alkylcarbonyl group, a C2-C7 haloalkylcarbonyl group, a C3-C7 alkenylcarbonyl group, a C3-C7 haloalkenylcarbonyl group, a C3-C7 alkynylcarbonyl group, a C3-C7 haloalkynylcarbonyl group, a C4-C10 cycloalkylcarbonyl group, a C4-C10 halocycloalkylcarbonyl group, a C2-C7 alkylcarbonyloxy group, a C2-C7 haloalkylcarbonyloxy group, a C1-C6 alkylsulfonyloxy group, a C1-C6 haloalkylsulfonyloxy group, a C2-C7 alkoxycarbonyl group, a C2-C7 haloalkoxycarbonyl group, a C3-C7 alkenyloxycarbonyl group, a C3-C7 haloalkenyloxycarbonyl group, a C3-C7 alkynyloxycarbonyl group, a C3-C7 haloalkynyloxycarbonyl group, a C4-C10 cycloalkyloxycarbonyl group, a C4-C10 halocycloalkyloxycarbonyl group, a C2-C7 alkylcarbonylamino group, a C2-C7 haloalkylcarbonylamino group, a C2-C7 alkoxycarbonylamino group, a C2-C7 haloalkoxycarbonylamino group, a C1-C6 alkylamino group, a C1-C6 haloalkylamino group, a C2-C6 alkenylamino group, a C2-C6 haloalkenylamino group, a C2-C6 alkynylamino group, a C2-C6 haloalkynylamino group, a C3-C9 cycloalkylamino group, a C3-C9 halocycloalkylamino group, a C2-C7 alkylaminocarbonyl group, a C2-C7 haloalkylaminocarbonyl group, a C3-C7 alkenylaminocarbonyl group, a C3-C7 haloalkenylaminocarbonyl group, a C3-C7 alkynylaminocarbonyl group, a C3-C7 haloalkynylaminocarbonyl group, a C4-C10 cycloalkylaminocarbonyl group, a C4-C10 halocycloalkylaminocarbonyl group, an amino group, a carbamoyl group, a cyano group, a nitro group, a hydroxy group, a pentafluorosulfanyl group, a phenyl group which may have a substituent, and a heterocyclic group which may have a substituent, and when there are two or more substituents, the substituents may be the same as or different from each other;
wherein, the heterocyclic group in Q₁ represents a pyridyl group, a pyridine-N-oxide group, a pyrimidinyl group, a pyrazinyl group, a pyridazyl group, a furyl group, a thienyl group, an oxazolyl group, an isoxazolyl group, an oxadiazolyl group, a thiazolyl group, an isothiazolyl group, a thiadiazolyl group, a pyrrolyl group, an imidazolyl group, a triazolyl group, a pyrazolyl group, or a tetrazolyl group;
R₁ and R₂ each independently represents a hydrogen atom, an oxygen atom, a halogen atom, a hydroxy group, a nitro group, a nitroso group, a trimethylsilyl group, a t-butyldimethylsilyl group, a cyano group, an amino group, a C1-C6 alkyl group which may have a substituent, a C1-C6 haloalkyl group which may have a substituent, a C2-C7 alkylcarbonyl group, a C2-C7 haloalkylcarbonyl group, a C2-C6 alkenyl group which may have a substituent, a C2-C6 haloalkenyl group which may have a substituent, a C2-C6 alkynyl group which may have a substituent, a C2-C6 haloalkynyl group which may have a substituent, a C2-C7 alkoxycarbonyl group, a C2-C7 haloalkoxycarbonyl group, a C3-C7 alkenyloxycarbonyl group, a C3-C7 haloalkenyloxycarbonyl group, a C3-C7 alkynyloxycarbonyl group, a C3-C7 haloalkynyloxycarbonyl group, a phenoxycarbonyl group, a C2-C7 alkylaminocarbonyl group, a C2-C7 haloalkylaminocarbonyl group, a C2-C7 alkylcarbonyloxy group, a C2-C7 haloalkylcarbonyloxy group, a benzoyl group, a C1-C6 alkoxy group, a C1-C6 haloalkoxy group, a C1-C6 alkylthio group, a C1-C6 haloalkylthio group, a C1-C6 alkylsulfinyl group, a C1-C6 haloalkylsulfinyl group, a C1-C6 alkylsulfonyl group, a C1-C6 haloalkylsulfonyl group, a benzenesulfonyl group, a benzylsulfonyl group, a benzyl group, or -C(=O)C(=O)R₇, wherein R₇ represents a C1-C6 alkyl group, a C1-C6 haloalkyl group, a C1-C6 alkoxy group, or a C1-C6 haloalkoxy group; 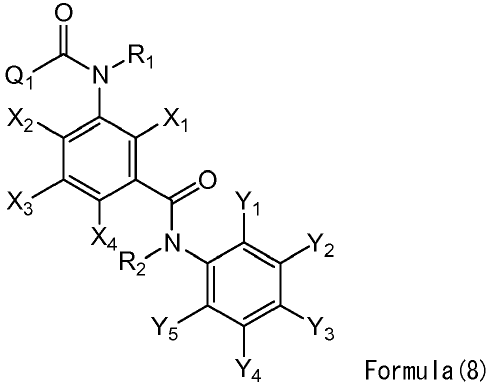
wherein in Formula (8), Q₁ represents a phenyl group or a pyridyl group which may have a substituent selected from a group consisting of: a halogen atom, C1 haloalkyl group, substituent nitro group, and a cyano group, in a case where Q₁ has the substituent the number of the substituents is 1 or 2;
X₁ and X₂ each independently represent a hydrogen atom or a fluorine atom, and X₃ and X₄ represent a hydrogen atom;
R₁ and R₂ each independently represent a hydrogen atom or a methyl group;
Y₁ and Y₅ each independently represent a chlorine atom, a bromine atom, an iodine atom, a trifluoromethoxy group, a trifluoromethyl group, or a pentafluoroethyl group;
Y₂ and Y₄ represent a hydrogen atom;
and Y₃ represents a C3-C4 perfluoroalkyl group;
in a case where Y₁ and Y₅ represent halogen atoms simultaneously, at least one of X₁ or X₂ represents a fluorine atom; and
in a case where Y₁ or Y₅ represents a trifluoromethoxy group, X₂ represents a fluorine atom; 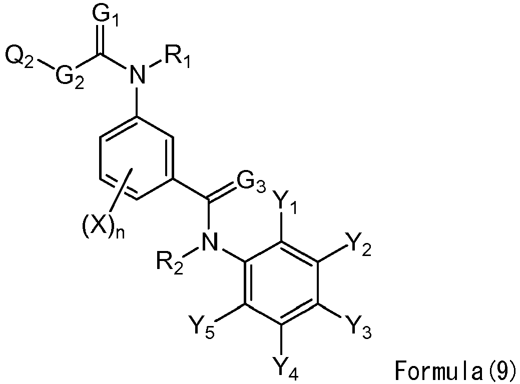
wherein in Formula (9), n represents 4;
four X's each independently represent a hydrogen atom, a halogen atom, or cyano group;
Y₃ represents a C2-C4 perfluoroalkyl group;
Y₂ and Y₄ represent a hydrogen atom;
Y₁ and Y₅ each independently represent a halogen atom or a C1-C3 haloalkyl group, and either Y₁ or Y₅ represents a C1-C3 haloalkyl group;
G₁, G₂ and G₃ each independently represent an oxygen atom or a sulfur atom;
Q₂ represents a hydrogen atom, a C1-C6 alkyl group, a C1-C6 haloalkyl group, a C2-C6 alkenyl group, a C2-C6 haloalkenyl group, a C2-C6 alkynyl group, a C2-C6 haloalkynyl group, a C3-C9 cycloalkyl group, a C3-C9 halocycloalkyl group, a benzyl group, a phenyl group which may have a substituent, a naphthyl group, or a heterocyclic group which may have a substituent;
wherein, in Q₂ the substituent of a phenyl group which may have a substituent and a heterocyclic group which may have a substituent represents one or more substituents selected from a group consisting of:
a halogen atom, a C1-C6 alkyl group, a C1-C6 haloalkyl group, a C3-C9 cycloalkyl group, a C3-C9 halocycloalkyl group, a C2-C6 alkenyl group, a C2-C6 haloalkenyl group, a C2-C6 alkynyl group, a C2-C6 haloalkynyl group, a C1-C6 alkoxy group, a C1-C6 haloalkoxy group, a C2-C6 alkenyloxy group, a C2-C6 haloalkenyloxy group, a C2-C6 alkynyloxy group, a C2-C6 haloalkynyloxy group, a C3-C9 cycloalkoxy group, a C3-C9 halocycloalkoxy group, a C1-C6 alkylthio group, a C1-C6 haloalkylthio group, a C1-C6 alkylsulfinyl group, a C1-C6 haloalkylsulfinyl group, a C1-C6 alkylsulfonyl group, a C1-C6 haloalkylsulfonyl group, a C2-C7 alkylcarbonyl group, a C2-C7 haloalkylcarbonyl group, a C3-C7 alkenylcarbonyl group, a C3-C7 haloalkenylcarbonyl group, a C3-C7 alkynylcarbonyl group, a C3-C7 haloalkynylcarbonyl group, a C4-C10 cycloalkylcarbonyl group, a C4-C10 halocycloalkylcarbonyl group, a C2-C7 alkylcarbonyloxy group, a C2-C7 haloalkylcarbonyloxy group, a C1-C6 alkylsulfonyloxy group, a C1-C6 haloalkylsulfonyloxy group, a C2-C7 alkoxycarbonyl group, a C2-C7 haloalkoxycarbonyl group, a C3-C7 alkenyloxycarbonyl group, a C3-C7 haloalkenyloxycarbonyl group, a C3-C7 alkynyloxycarbonyl group, a C3-C7 haloalkynyloxycarbonyl group, a C4-C10 cycloalkyloxycarbonyl group, a C4-C10 halocycloalkyloxycarbonyl group, a C2-C7 alkylcarbonylamino group, a C2-C7 haloalkylcarbonylamino group, a C2-C7 alkoxycarbonylamino group, a C2-C7 haloalkoxycarbonylamino group, a C1-C6 alkylamino group, a C1-C6 haloalkylamino group, a C2-C6 alkenylamino group, a C2-C6 haloalkenylamino group, a C2-C6 alkynylamino group, a C2-C6 haloalkynylamino group, a C3-C9 cycloalkylamino group, a C3-C9 halocycloalkylamino group, a C2-C7 alkylaminocarbonyl group, a C2-C7 haloalkylaminocarbonyl group, a C3-C7 alkenylaminocarbonyl group, a C3-C7 haloalkenylaminocarbonyl group, a C3-C7 alkynylaminocarbonyl group, a C3-C7 haloalkynylaminocarbonyl group, a C4-C10 cycloalkylaminocarbonyl group, a C4-C10 halocycloalkylaminocarbonyl group, an amino group, a carbamoyl group, a cyano group, a nitro group, a hydroxy group, a pentafluorosulfanyl group, a phenyl group which may have a substituent, and a heterocyclic group which may have a substituent, and when there are two or more substituents, the substituents may be the same as or different from each other;
wherein, the heterocyclic group in Q₂ represents a pyridyl group, a pyridine-N-oxide group, a pyrimidinyl group, a pyrazinyl group, a pyridazyl group, a furyl group, a thienyl group, an oxazolyl group, an isoxazolyl group, an oxadiazolyl group, a thiazolyl group, an isothiazolyl group, a thiadiazolyl group, a pyrrolyl group, an imidazolyl group, a triazolyl group, a pyrazolyl group, or a tetrazolyl group;
R₁ and R₂ each independently represent a hydrogen atom, an oxygen atom, a halogen atom, a hydroxy group, a nitro group, a nitroso group, a trimethylsilyl group, a t-butyldimethylsilyl group, a cyano group, an amino group, a C1-C6 alkyl group which may have a substituent, a C1-C6 haloalkyl group which may have a substituent, a C2-C7 alkylcarbonyl group, a C2-C7 haloalkylcarbonyl group, a C2-C6 alkenyl group which may have a substituent, a C2-C6 haloalkenyl group which may have a substituent, a C2-C6 alkynyl group which may have a substituent, a C2-C6 haloalkynyl group which may have a substituent, a C2-C7 alkoxycarbonyl group, a C2-C7 haloalkoxycarbonyl group, a C3-C7 alkenyloxycarbonyl group, a C3-C7 haloalkenyloxycarbonyl group, a C3-C7 alkynyloxycarbonyl group, a C3-C7 haloalkynyloxycarbonyl group, a phenoxycarbonyl group, a C2-C7 alkylaminocarbonyl group, a C2-C7 haloalkylaminocarbonyl group, a C2-C7 alkylcarbonyloxy group, a C2-C7 haloalkylcarbonyloxy group, a benzoyl group, a C1-C6 alkoxy group, a C1-C6 haloalkoxy group, a C1-C6 alkylthio group, a C1-C6 haloalkylthio group, a C1-C6 alkylsulfinyl group, a C1-C6 haloalkylsulfinyl group, a C1-C6 alkylsulfonyl group, a C1-C6 haloalkylsulfonyl group, a benzenesulfonyl group, a benzylsulfonyl group, a benzyl group, or -C(=O)C(=O)R₇, wherein R₇ represents a C1-C6 alkyl group, a C1-C6 haloalkyl group, a C1-C6 alkoxy group, or a C1-C6 haloalkoxy group, wherein the pest is selected from the group consisting of:
Siphonaptera insects such as *Ctenocephalides felis, Ctenocephalides canis, Echidnophaga gallinacea, Pulex irritans, and Xenopsylla cheopis;*
mallophaga insects such as *Menacanthus stramineus, and Bovicola bovis;*
anoplura insects such as *Haematopinus eurysternus, Haematopinus suis, Linognathus vituli, and Solenopotes capillatus;*
ixodidae such as *Boophilus microplus, Rhipicephalus sanguineus, Haemaphysalis longicornis, Haemaphysalis flava, Haemaphysalis campanulata, Ixodes ovatus, Ixodes persulcatus, Amblyomma spp., and Dermacentor spp.;*
cheyletidae such as *Cheyletiella yasguri, and Cheyletiella blakei;*
demodicidae such as *Demodex canis, Demodex cati;*
psoroptidae such as *Psoroptes ovis;* and
sarcoptidae such as *Sarcoptes scabiei, Notoedres cati, and Knemidocoptes spp.*

2. A composition comprising a pest control agent comprising at least one amide derivative according to Formula (1), Formula (7), Formula (8) or Formula (9), as defined in claim 1

3. A pest controlling method comprising applying the pest control agent according to claim 1, or the composition as defined in claim 2 to a pest as defined in claim 1, wherein the method is not a method of treatment.

4. A pest control agent comprising at least one amide derivative according to Formula (1), Formula (7), Formula (8) and
Formula (9), as defined in claim 1, or a composition as defined in claim 2, for use in a method of treatment of the human or animal body.

5. A pest control agent comprising at least one amide derivative according to Formula (1), Formula (7), Formula (8) and
Formula (9), as defined in claim 1, or a composition as defined in claim 2, for use in a method of treatment of the human or animal body,
wherein the method is for exterminating a pest selected from the group consisting of:
Siphonaptera insects such as *Ctenocephalides felis, Ctenocephalides canis, Echidnophaga gallinacea, Pulex irritans, and Xenopsylla cheopis;*
mallophaga insects such as *Menacanthus stramineus, and Bovicola bovis;*
anoplura insects such as *Haematopinus eurysternus, Haematopinus suis, Linognathus vituli, and Solenopotes capillatus;*
ixodidae such as *Boophilus microplus, Rhipicephalus sanguineus, Haemaphysalis longicornis, Haemaphysalis flava, Haemaphysalis campanulata, Ixodes ovatus, Ixodes persulcatus, Amblyomma spp., and Dermacentor spp.;*
cheyletidae such as *Cheyletiella yasguri, and Cheyletiella blakei;*
demodicidae such as *Demodex canis, Demodex cati;*
psoroptidae such as *Psoroptes ovis;* and
sarcoptidae such as *Sarcoptes scabiei, Notoedres cati, and Knemidocoptes spp.*

6. Use of a pest control agent comprising at least one amide derivative according to Formula (1), Formula (7), Formula (8) and
Formula (9), as defined in claim 1, or a composition as defined in claim 2, as a pest control agent, wherein the use is not a method of treatment of the human or animal body.

7. The use according to claim 6, wherein the use is for the control of a pest selected from the group consisting of:
Siphonaptera insects such as *Ctenocephalides felis, Ctenocephalides canis, Echidnophaga gallinacea, Pulex irritans, and Xenopsylla cheopis;*
mallophaga insects such as *Menacanthus stramineus, and Bovicola bovis;*
anoplura insects such as *Haematopinus eurysternus, Haematopinus suis, Linognathus vituli, and Solenopotes capillatus;*
ixodidae such as *Boophilus microplus, Rhipicephalus sanguineus, Haemaphysalis longicornis, Haemaphysalis flava, Haemaphysalis campanulata, Ixodes ovatus, Ixodes persulcatus, Amblyomma spp., and Dermacentor spp.;*
cheyletidae such as *Cheyletiella yasguri, and Cheyletiella blakei;*
demodicidae such as *Demodex canis, Demodex cati;*
psoroptidae such as *Psoroptes ovis;* and
sarcoptidae such as *Sarcoptes scabiei, Notoedres cati, and Knemidocoptes spp.*

## Patentansprüche

1. Schädlingsbekämpfungsmittel, das zumindest ein aus der aus folgender Formel (1), Formel (7), Formel (8) und Formel (9) bestehenden Gruppe ausgewähltes Amid-Derivat als Wirkbestandteil umfasst: 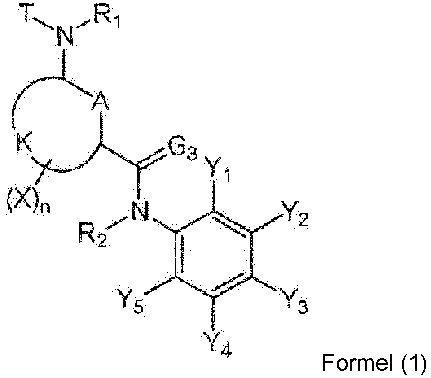 wobei in Formel (1):
A für ein Kohlenstoffatom, ein Sauerstoffatom, ein Stickstoffatom, ein oxidiertes Stickstoffatom oder ein Schwefelatom steht;
K für eine Nichtmetall-Atomgruppe steht, die für die Bildung einer von einer Pyridin-, Pyridin-N-oxid-, Pyrrol-, Thiazol-, Furan- oder Thiophengruppe abgeleiteten zyklischen Bindungsgruppe in Kombination mit A und zwei Kohlenstoffatomen, mit denen A verbunden ist, erforderlich ist;
X für ein Wasserstoffatom, ein Halogenatom, eine C1-C6-Alkylgruppe, , eine C1-C6-Halogenalkylgruppe, eine C3-C9-Cycloakylgruppe, eine C3-C9-Halogencycloalkylgruppe, eine C2-C6-Alkenylgruppe, eine C2-C6-Halogenalkenylgruppe, eine C2-C6-Alkinylgruppe, eine C2-C6-Halogenalkinylgruppe, eine C1-C6-Alkoxygruppe, eine C1-C6-Halogenalkoxygruppe, eine C2-C6-Alkenyloxygruppe, eine C2-C6-Halogenalkenyloxygruppe, eine C2-C6-Alkinyloxygruppe, eine C2-C6-Halogenalkinyloxygruppe, eine C3-C9-Cycloalkoxygruppe, eine C3-C9-Halogencycloalkoxygruppe, eine C1-C6-Alkylthiogruppe, eine C1-C6-Halogenalkylthiogruppe, eine C1-C6-Alkylsulfinylgruppe, eine C1-C6-Halogenalkylsulfinylgruppe, eine C1-C6-Alkylsulfonylgruppe, eine C1-C6-Halogenalkylsulfonylgruppe, eine C1-C6-Alkylsulfonyloxygruppe, eine C1-C6-Halogenalkylsulfonyloxygruppe, eine C2-C7-Alkylcarbonylgruppe, eine C2-C7-Halogenalkylcarbonylgruppe, eine C3-C7-Alkenylcarbonylgruppe, eine C3-C7-Halogenalkenylcarbonylgruppe, eine C3-C7-Alkinylcarbonylgruppe, eine C3-C7-Halogenalkinylcarbonylgruppe, eine C4-C10-Cycloalkylcarbonylgruppe, eine C4-C10-Halogencycloalkylcarbonylgruppe, eine C2-C7-Alkylcarbonyloxygruppe, eine C2-C7-Halogenalkylcarbonyloxygruppe, eine Arylcarbonyloxygruppe, eine C2-C7-Alkoxycarbonylgruppe, eine C2-C7-Halogenalkoxycarbonylgruppe, eine C3-C7-Alkenyloxycarbonylgruppe, eine C3-C7-Halogenalkenyloxycarbonylgruppe, eine C3-C7-Alkinyloxycarbonylgruppe, eine C3-C7-Halogenalkinyloxycarbonylgruppe, eine C4-C10-Cycloalkyloxycarbonylgruppe, eine C4-C10-Halogencycloalkyloxycarbonylgruppe, eine C2-C7-Alkylcarbonylaminogruppe, eine C2-C7-Halogenalkylcarbonylaminogruppe, eine C2-C7-Alkoxycarbonylaminogruppe, eine C2-C7-Halogenalkoxycarbonylaminogruppe, eine C2-C7-Alkoxycarbonyloxygruppe, eine C2-C7-Halogenalkoxycarbonyloxygruppe, eine Arylcarbonylaminogruppe, eine Aminogruppe, eine Carbamoylgruppe, eine Cyanogruppe, eine Hydroxygruppe, eine Pentafluorsulfanylgruppe, eine C1-C6-Alkylaminogruppe, eine C1-C6-Halogenalkylaminogruppe, eine C2-C6-Alkenylaminogruppe, eine C2-C6-Halogenalkenylaminogruppe, eine C2-C6-Alkinylaminogruppe, eine C2-C6-Halogenalkinylaminogruppe, eine C3-C9-Cycloalkylaminogruppe, eine C3-C9-Halogencycloalkylaminogruppe, eine C2-C7-Alkylaminocarbonylgruppe, eine C2-C7-Halogenalkylaminocarbonylgruppe, eine C3-C7-Alkenylaminocarbonylgruppe, eine C3-C7-Halogenalkenylaminocarbonylgruppe, eine C3-C7-Alkinylaminocarbonylgruppe, eine C3-C7-Halogenalkinylaminocarbonylgruppe, eine C4-C10-Cycloalkylaminocarbonylgruppe, eine C4-C10-Halogencycloalkylaminocarbonylgruppe, eine Phenylgruppe oder eine heterozyklische Gruppe steht und, wenn mehrere X vorhanden sind, die X gleich oder unterschiedlich sein können;
n für eine ganze Zahl von 0 bis 4 steht;
T für -C(=G₁)-Q₁ oder -C(=G₁)-G₂Q₂ steht; worin
G₁ und G₂ jeweils unabhängig voneinander für ein Sauerstoffatom oder ein Schwefelatom stehen; und
Q₁ und Q₂ jeweils für Folgendes stehen: ein Wasserstoffatom, eine C1-C6-Alkylgruppe, eine C1-C6-Halogenalkylgruppe, eine C2-C6-Alkenylgruppe, eine C2-C6-Halogenalkenylgruppe, eine C2-C6-Alkinylgruppe, eine C2-C6-Halogenalkinylgruppe, eine C3-C9-Cycloalkylgruppe, eine C3-C9-Halogencycloalkylgruppe, eine Benzylgruppe, eine Phenylgruppe, die gegebenenfalls einen Substituenten aufweist, eine Naphthylgruppe oder eine heterozyklische Gruppe, die gegebenenfalls einen Substituenten aufweist;
Y₁ und Y₅ für ein Halogenatom oder eine C1-C3-Halogenalkylgruppe stehen und entweder Y₁ oder Y₅ für eine C1-C3-Halogenalkylgruppe steht;
Y₂ und Y₄ für ein Wasserstoffatom stehen;
Y₃ für eine C2-C4-Perfluoralkylgruppe steht;
wobei in Q₁ und Q₂ der Substituent der Phenylgruppe, die gegebenenfalls einen Substituenten aufweist, und der heterozyklischen Gruppe, die gegebenenfalls einen Substituenten aufweist, für einen oder mehrere Substituenten steht, die aus der aus den folgenden bestehenden Gruppe ausgewählt sind:
einem Halogenatom, einer C1-C6-Alkylgruppe, einer C1-C6-Halogenalkylgruppe, einer C3-C9-Cycloalkylgruppe, einer C3-C9-Halogencycloalkylgruppe, einer C2-C6-Alkenylgruppe, einer C2-C6-Halogenalkenylgruppe, einer C2-C6-Alkinylgruppe, einer C2-C6-Halogenalkinylgruppe, einer C1-C6-Alkoxygruppe, einer C1-C6-Halogenalkoxygruppe, einer C2-C6-Alkenyloxygruppe, einer C2-C6-Halogenalkenyloxygruppe, einer C2-C6-Alkinyloxygruppe, einer C2-C6-Halogenalkinyloxygruppe, einer C3-C9-Cycloalkoxygruppe, einer C3-C9-Halogencycloalkoxygruppe, einer C1-C6-Alkylthiogruppe, einer C1-C6-Halogenalkylthiogruppe, einer C1-C6-Alkylsulfinylgruppe, einer C1-C6-Halogenalkylsulfinylgruppe, einer C1-C6-Alkylsulfonylgruppe, einer C1-C6-Halogenalkylsulfonylgruppe, einer C2-C7-Alkylcarbonylgruppe, einer C2-C7-Halogenalkylcarbonylgruppe, einer C3-C7-Alkenylcarbonylgruppe, einer C3-C7-Halogenalkenylcarbonylgruppe, einer C3-C7-Alkinylcarbonylgruppe, einer C3-C7-Halogenalkinylcarbonylgruppe, einer C4-C10-Cycloalkylcarbonylgruppe, einer C4-C10-Halogencycloalkylcarbonylgruppe, einer C2-C7-Alkylcarbonyloxygruppe, einer C2-C7-Halogenalkylcarbonyloxygruppe, einer C1-C6-Alkylsulfonyloxygruppe, einer C1-C6-Halogenalkylsulfonyloxygruppe, einer C2-C7-Alkoxycarbonylgruppe, einer C2-C7-Halogenalkoxycarbonylgruppe, einer C3-C7-Alkenyloxycarbonylgruppe, einer C3-C7-Halogenalkenyloxycarbonylgruppe, einer C3-C7-Alkinyloxycarbonylgruppe, einer C3-C7-Halogenalkinyloxycarbonylgruppe, einer C4-C10-Cycloalkyloxycarbonylgruppe, einer C4-C10-Halogencycloalkyloxycarbonylgruppe, einer C2-C7-Alkylcarbonylaminogruppe, einer C2-C7-Halogenalkylcarbonylaminogruppe, einer C2-C7-Alkoxycarbonylaminogruppe, einer C2-C7-Halogenalkoxycarbonylaminogruppe, einer C1-C6-Alkylaminogruppe, einer C1-C6-Halogenalkylaminogruppe, einer C2-C6-Alkenylaminogruppe, einer C2-C6-Halogenalkenylaminogruppe, einer C2-C6-Alkinylaminogruppe, einer C2-C6-Halogenalkinylaminogruppe, einer C3-C9-Cycloalkylaminogruppe, einer C3-C9-Halogencycloalkylaminogruppe, einer C2-C7-Alkylaminocarbonylgruppe, einer C2-C7-Halogenalkylaminocarbonylgruppe, einer C3-C7-Alkenylaminocarbonylgruppe, einer C3-C7-Halogenalkenylaminocarbonylgruppe, einer C3-C7-Alkinylaminocarbonylgruppe, einer C3-C7-Halogenalkinylaminocarbonylgruppe, einer C4-C10-Cycloalkylaminocarbonylgruppe, einer C4-C10-Halogencycloalkylaminocarbonylgruppe, einer Aminogruppe, einer Carbamoylgruppe, einer Cyanogruppe, einer Nitrogruppe, einer Hydroxygruppe, einer Pentafluorsulfanylgruppe, einer Phenylgruppe, die gegebenenfalls einen Substituenten aufweist, und einer heterozyklische Gruppe, die gegebenenfalls einen Substituenten aufweist, und wobei, wenn zwei oder mehr Substituenten vorhanden sind, die Substituenten gleich oder unterschiedlich sein können;
wobei die heterozyklische Gruppe in X, Q₁ und Q₂ für eine Pyridylgruppe, eine Pyridin-N-oxidgruppe, eine Pyrimidinylgruppe, eine Pyrazinylgruppe, eine Pyridazylgruppe, eine Furylgruppe, eine Thienylgruppe, eine Oxazolylgruppe, eine Isoxazolylgruppe, eine Oxadiazolylgruppe, eine Thiazolylgruppe, eine Isothiazolylgruppe, eine Thiadiazolylgruppe, eine Pyrrolylgruppe, eine Imidazolylgruppe, eine Triazolylgruppe, eine Pyrazolylgruppe oder eine Tetrazolylgruppe steht;
G₃ für ein Sauerstoffatom oder ein Schwefelatom steht; und
R₁ und R₂ jeweils unabhängig voneinander für ein Wasserstoffatom, ein Sauerstoffatom, ein Halogenatom, eine Hydroxygruppe, eine Nitrogruppe, eine Nitrosogruppe, eine Trimethylsilylgruppe, eine t-Butyldimethylsilylgruppe, eine Cyanogruppe, eine Aminogruppe, eine C1-C6-Alkylgruppe, die gegebenenfalls einen Substituenten aufweist, eine C1-C6-Halogenalkylgruppe, die gegebenenfalls einen Substituenten aufweist, eine C2-C7-Alkylcarbonylgruppe, eine C2-C7-Halogenalkylcarbonylgruppe, eine C2-C6-Alkenylgruppe, die gegebenenfalls einen Substituenten aufweist, eine C2-C6-Halogenalkenylgruppe, die gegebenenfalls einen Substituenten aufweist, eine C2-C6-Alkinylgruppe, die gegebenenfalls einen Substituenten aufweist, eine C2-C6-Halogenalkinylgruppe, die gegebenenfalls einen Substituenten aufweist, eine C2-C7-Alkoxycarbonylgruppe, eine C2-C7-Halogenalkoxycarbonylgruppe, eine C3-C7-Alkenyloxycarbonylgruppe, eine C3-C7-Halogenalkenyloxycarbonylgruppe, eine C3-C7-Alkinyloxycarbonylgruppe, eine C3-C7-Halogenalkinyloxycarbonylgruppe, eine Phenoxycarbonylgruppe, eine C2-C7-Alkylaminocarbonylgruppe, eine C2-C7-Halogenalkylaminocarbonylgruppe, eine C2-C7-Alkylcarbonyloxygruppe, eine C2-C7-Halogenalkylcarbonyloxygruppe, eine Benzoylgruppe, eine C1-C6-Alkoxygruppe, eine C1-C6-Halogenalkoxygruppe, eine C1-C6-Alkylthiogruppe, eine C1-C6-Halogenalkylthiogruppe, eine C1-C6-Alkylsulfinylgruppe, eine C1-C6-Halogenalkylsulfinylgruppe, eine C1-C6-Alkylsulfonylgruppe, eine C1-C6-Halogenalkylsulfonylgruppe, eine Benzolsulfonylgruppe, eine Benzylsulfonylgruppe, eine Benzylgruppe oder -C(=O)C(=O)R₇ steht, worin R₇ für eine C1-C6-Alkylgruppe, eine C1-C6-Halogenalkylgruppe, eine C1-C6-Alkoxygruppe oder eine C1-C6-Halogenalkoxygruppe stehen; 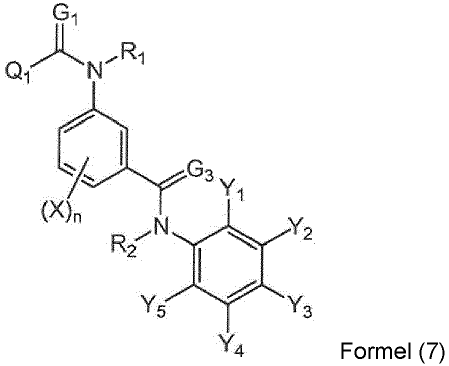
wobei in Formel (7):
n für 4 steht;
die X jeweils unabhängig voneinander für ein Wasserstoffatom, ein Halogenatom, oder eine Cyanogruppe stehen;
Y₃ für eine C2-C4-Perfluoralkylgruppe steht;
Y₂ und Y₄ für ein Wasserstoffatom stehen;
Y₁ und Y₅ für ein Halogenatom oder eine C1-C3-Halogenalkylgruppe stehen und entweder Y₁ oder Y₅ für eine C1-C3-Halogenalkylgruppe steht;
G₁ und G₃ jeweils unabhängig voneinander für ein Sauerstoffatom oder ein Schwefelatom stehen;
Q₁ für ein Wasserstoffatom, eine C1-C6-Alkylgruppe, eine C1-C6-Halogenalkylgruppe, eine C2-C6-Alkenylgruppe, eine C2-C6-Halogenalkenylgruppe, eine C2-C6-Alkinylgruppe, eine C2-C6-Halogenalkinylgruppe, eine C3-C9-Cycloalkylgruppe, eine C3-C9-Halogencycloalkylgruppe, eine Benzylgruppe, eine Phenylgruppe, die gegebenenfalls einen Substituenten aufweist, eine Naphthylgruppe oder eine heterozyklische Gruppe steht, die gegebenenfalls einen Substituenten aufweist;
wobei in Q₁ der Substituent der Phenylgruppe, die gegebenenfalls einen Substituenten aufweist, und der heterozyklischen Gruppe, die gegebenenfalls einen Substituenten aufweist, für einen oder mehrere Substituenten steht, die aus den aus den folgenden bestehenden Gruppe ausgewählt sind:
einem Halogenatom, einer C1-C6-Alkylgruppe, einer C1-C6-Halogenalkylgruppe, einer C3-C9-Cycloalkylgruppe, einer C3-C9-Halogencycloalkylgruppe, einer C2-C6-Alkenylgruppe, einer C2-C6-Halogenalkenylgruppe, einer C2-C6-Alkinylgruppe, einer C2-C6-Halogenalkinylgruppe, einer C1-C6-Alkoxygruppe, einer C1-C6-Halogenalkoxygruppe, einer C2-C6-Alkenyloxygruppe, einer C2-C6-Halogenalkenyloxygruppe, einer C2-C6-Alkinyloxygruppe, einer C2-C6-Halogenalkinyloxygruppe, einer C3-C9-Cycloalkoxygruppe, einer C3-C9-Halogencycloalkoxygruppe, einer C1-C6-Alkylthiogruppe, einer C1-C6-Halogenalkylthiogruppe, einer C1-C6-Alkylsulfinylgruppe, einer C1-C6-Halogenalkylsulfinylgruppe, einer C1-C6-Alkylsulfonylgruppe, einer C1-C6-Halogenalkylsulfonylgruppe, einer C2-C7-Alkylcarbonylgruppe, einer C2-C7-Halogenalkylcarbonylgruppe, einer C3-C7-Alkenylcarbonylgruppe, einer C3-C7-Halogenalkenylcarbonylgruppe, einer C3-C7-Alkinylcarbonylgruppe, einer C3-C7-Halogenalkinylcarbonylgruppe, einer C4-C10-Cycloalkylcarbonylgruppe, einer C4-C10-Halogencycloalkylcarbonylgruppe, einer C2-C7-Alkylcarbonyloxygruppe, einer C2-C7-Halogenalkylcarbonyloxygruppe, einer C1-C6-Alkylsulfonyloxygruppe, einer C1-C6-Halogenalkylsulfonyloxygruppe, einer C2-C7-Alkoxycarbonylgruppe, einer C2-C7-Halogenalkoxycarbonylgruppe, einer C3-C7-Alkenyloxycarbonylgruppe, einer C3-C7-Halogenalkenyloxycarbonylgruppe, einer C3-C7-Alkinyloxycarbonylgruppe, einer C3-C7-Halogenalkinyloxycarbonylgruppe, einer C4-C10-Cycloalkyloxycarbonylgruppe, einer C4-C10-Halogencycloalkyloxycarbonylgruppe, einer C2-C7-Alkylcarbonylaminogruppe, einer C2-C7-Halogenalkylcarbonylaminogruppe, einer C2-C7-Alkoxycarbonylaminogruppe, einer C2-C7-Halogenalkoxycarbonylaminogruppe, einer C1-C6-Alkylaminogruppe, einer C1-C6-Halogenalkylaminogruppe, einer C2-C6-Alkenylaminogruppe, einer C2-C6-Halogenalkenylaminogruppe, einer C2-C6-Alkinylaminogruppe, einer C2-C6-Halogenalkinylaminogruppe, einer C3-C9-Cycloalkylaminogruppe, einer C3-C9-Halogencycloalkylaminogruppe, einer C2-C7-Alkylaminocarbonylgruppe, einer C2-C7-Halogenalkylaminocarbonylgruppe, einer C3-C7-Alkenylaminocarbonylgruppe, einer C3-C7-Halogenalkenylaminocarbonylgruppe, einer C3-C7-Alkinylaminocarbonylgruppe, einer C3-C7-Halogenalkinylaminocarbonylgruppe, einer C4-C10-Cycloalkylaminocarbonylgruppe, einer C4-C10-Halogencycloalkylaminocarbonylgruppe, einer Aminogruppe, einer Carbamoylgruppe, einer Cyanogruppe, einer Nitrogruppe, einer Hydroxygruppe, einer Pentafluorsulfanylgruppe, einer Phenylgruppe, die gegebenenfalls einen Substituenten aufweist, und einer heterozyklische Gruppe, die gegebenenfalls einen Substituenten aufweist, und, wenn zwei oder mehr Substituenten vorhanden sind, die Substituenten gleich oder unterschiedlich sein können;
wobei die heterozyklische Gruppe in Q₁ für eine Pyridylgruppe, eine Pyridin-N-oxidgruppe, eine Pyrimidinylgruppe, eine Pyrazinylgruppe, eine Pyridazylgruppe, eine Furylgruppe, eine Thienylgruppe, eine Oxazolylgruppe, eine Isoxazolylgruppe, eine Oxadiazolylgruppe, eine Thiazolylgruppe, eine Isothiazolylgruppe, eine Thiadiazolylgruppe, eine Pyrrolylgruppe, eine Imidazolylgruppe, eine Triazolylgruppe, eine Pyrazolylgruppe oder eine Tetrazolylgruppe steht;
R₁ und R₂ jeweils unabhängig für ein Wasserstoffatom, eine Sauerstoffatom, ein Halogenatom, eine Hydroxygruppe, eine Nitrogruppe, eine Nitrosogruppe, eine Trimethylsilylgruppe, eine t-Butyldimethylsilylgruppe, eine Cyanogruppe, eine Aminogruppe, eine C1-C6-Alkylgruppe, die gegebenenfalls einen Substituenten aufweist, eine C1-C6-Halogenalkylgruppe, die gegebenenfalls einen Substituenten aufweist, eine C2-C7-Alkylcarbonylgruppe, eine C2-C7-Halogenalkylcarbonylgruppe, eine C2-C6-Alkenylgruppe, die gegebenenfalls einen Substituenten aufweist, eine C2-C6-Halogenalkenylgruppe, die gegebenenfalls einen Substituenten aufweist, eine C2-C6-Alkinylgruppe, die gegebenenfalls einen Substituenten aufweist, eine C2-C6-Halogenalkinylgruppe, die gegebenenfalls einen Substituenten aufweist, eine C2-C7-Alkoxycarbonylgruppe, eine C2-C7-Halogenalkoxycarbonylgruppe, eine C3-C7-Alkenyloxycarbonylgruppe, eine C3-C7-Halogenalkenyloxycarbonylgruppe, eine C3-C7-Alkinyloxycarbonylgruppe, eine C3-C7-Halogenalkinyloxycarbonylgruppe, eine Phenoxycarbonylgruppe, eine C2-C7-Alkylaminocarbonylgruppe, eine C2-C7-Halogenalkylaminocarbonylgruppe, eine C2-C7-Alkylcarbonyloxygruppe, eine C2-C7-Halogenalkylcarbonyloxygruppe, eine Benzoylgruppe, eine C1-C6-Alkoxygruppe, eine C1-C6-Halogenalkoxygruppe, eine C1-C6-Alkylthiogruppe, eine C1-C6-Halogenalkylthiogruppe, eine C1-C6-Alkylsulfinylgruppe, eine C1-C6-Halogenalkylsulfinylgruppe, eine C1-C6-Alkylsulfonylgruppe, eine C1-C6-Halogenalkylsulfonylgruppe, eine Benzolsulfonylgruppe, eine Benzylsulfonylgruppe, eine Benzylgruppe oder -C(=O)C(=O)R₇ stehen, worin R₇ für eine C1-C6-Alkylgruppe, eine C1-C6-Halogenalkylgruppe, eine C1-C6-Alkoxygruppe oder eine C1-C6-Halogenalkoxygruppe steht; 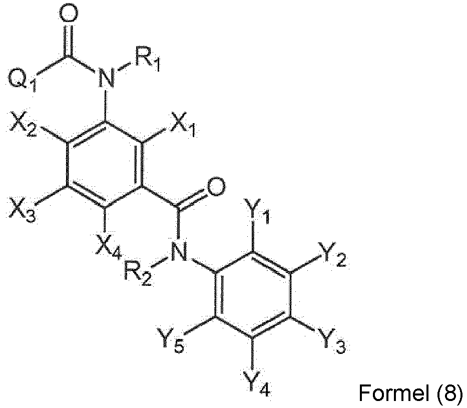
wobei in Formel (8):
Q₁ für eine Phenylgruppe oder eine Pyridylgruppe steht, die gegebenenfalls einen Substituenten aufweisen, der aus der aus den folgenden bestehenden Gruppe ausgewählt ist: einem Halogenatom, einer C1-Halogenalkylgruppe, einer Nitrogruppe und einer Cyanogruppe, und, wenn Q₁ den Substituenten aufweist, die Anzahl der Substituenten 1 oder 2 beträgt;
X₁ und X₂ jeweils unabhängig für ein Wasserstoffatom oder ein Fluoratom stehen und X₃ und X₄ für ein Wasserstoffatom stehen;
R₁ und R₂ jeweils unabhängig für ein Wasserstoffatom oder eine Methylgruppe stehen;
Y₁ und Y₅ jeweils unabhängig für ein Chloratom, ein Bromatom, ein lodatom, eine Trifluormethoxygruppe, eine Trifluormethylgruppe oder eine Pentafluorethylgruppe stehen;
Y₂ und Y₄ für ein Wasserstoffatom stehen; und
Y₃ für eine C3-C4-Perfluoralkylgruppe steht;
wobei, wenn Y₁ und Y₅ gleichzeitig für Halogenatome stehen, zumindest einer von X₁ und X₂ für ein Fluoratom steht; und
wobei, wenn Y₁ oder Y₅ für eine Trifluormethoxygruppe steht, X₂ für ein Fluoratom steht; 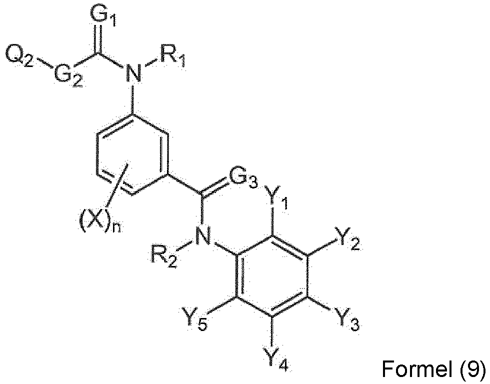
wobei in Formel (9):
n für 4 steht;
vier X jeweils unabhängig für ein Wasserstoffatom, ein Halogenatom oder eine Cyanogruppe stehen;
Y₃ für eine C2-C4-Perfluoralkylgruppe steht;
Y₂ und Y₄ für ein Wasserstoffatom stehen;
Y₁ und Y₅ jeweils unabhängig für ein Halogenatom oder eine C1-C3-Halogenalkylgruppe stehen und entweder Y₁ oder Y₅ für eine C1-C3-Halogenalkylgruppe steht;
G₁, G₂ und G₃ jeweils unabhängig für ein Sauerstoffatom oder ein Schwefelatom stehen;
Q₂ für ein Wasserstoffatom, eine C1-C6-Alkylgruppe, eine C1-C6-Halogenalkylgruppe, eine C2-C6-Alkenylgruppe, eine C2-C6-Halogenalkenylgruppe, eine C2-C6-Alkinylgruppe, eine C2-C6-Halogenalkinylgruppe, eine C3-C9-Cycloalkylgruppe, eine C3-C9-Halogencycloalkylgruppe, eine Benzylgruppe, eine Phenylgruppe, die gegebenenfalls einen Substituenten aufweist, eine Naphthylgruppe oder eine heterozyklische Gruppe steht, die gegebenenfalls einen Substituenten aufweist;
wobei in Q₂ der Substituent der Phenylgruppe, die gegebenenfalls einen Substituenten aufweist, und der heterozyklischen Gruppe, die gegebenenfalls einen Substituenten aufweist, für einen oder mehrere Substituenten steht, die aus der aus den folgenden bestehenden Gruppe ausgewählt sind:
einem Halogenatom, einer C1-C6-Alkylgruppe, einer C1-C6-Halogenalkylgruppe, einer C3-C9-Cycloalkylgruppe, einer C3-C9-Halogencycloalkylgruppe, einer C2-C6-Alkenylgruppe, einer C2-C6-Halogenalkenylgruppe, einer C2-C6-Alkinylgruppe, einer C2-C6-Halogenalkinylgruppe, einer C1-C6-Alkoxygruppe, einer C1-C6-Halogenalkoxygruppe, einer C2-C6-Alkenyloxygruppe, einer C2-C6-Halogenalkenyloxygruppe, einer C2-C6-Alkinyloxygruppe, einer C2-C6-Halogenalkinyloxygruppe, einer C3-C9-Cycloalkoxygruppe, einer C3-C9-Halogencycloalkoxygruppe, einer C1-C6-Alkylthiogruppe, einer C1-C6-Halogenalkylthiogruppe, einer C1-C6-Alkylsulfinylgruppe, einer C1-C6-Halogenalkylsulfinylgruppe, einer C1-C6-Alkylsulfonylgruppe, einer C1-C6-Halogenalkylsulfonylgruppe, einer C2-C7-Alkylcarbonylgruppe, einer C2-C7-Halogenalkylcarbonylgruppe, einer C3-C7-Alkenylcarbonylgruppe, einer C3-C7-Halogenalkenylcarbonylgruppe, einer C3-C7-Alkinylcarbonylgruppe, einer C3-C7-Halogenalkinylcarbonylgruppe, einer C4-C10-Cycloalkylcarbonylgruppe, einer C4-C10-Halogencycloalkylcarbonylgruppe, einer C2-C7-Alkylcarbonyloxygruppe, einer C2-C7-Halogenalkylcarbonyloxygruppe, einer C1-C6-Alkylsulfonyloxygruppe, einer C1-C6-Halogenalkylsulfonyloxygruppe, einer C2-C7-Alkoxycarbonylgruppe, einer C2-C7-Halogenalkoxycarbonylgruppe, einer C3-C7-Alkenyloxycarbonylgruppe, einer C3-C7-Halogenalkenyloxycarbonylgruppe, einer C3-C7-Alkinyloxycarbonylgruppe, einer C3-C7-Halogenalkinyloxycarbonylgruppe, einer C4-C10-Cycloalkyloxycarbonylgruppe, einer C4-C10-Halogencycloalkyloxycarbonylgruppe, einer C2-C7-Alkylcarbonylaminogruppe, einer C2-C7-Halogenalkylcarbonylaminogruppe, einer C2-C7-Alkoxycarbonylaminogruppe, einer C2-C7-Halogenalkoxycarbonylaminogruppe, einer C1-C6-Alkylaminogruppe, einer C1-C6-Halogenalkylaminogruppe, einer C2-C6-Alkenylaminogruppe, einer C2-C6-Halogenalkenylaminogruppe, einer C2-C6-Alkinylaminogruppe, einer C2-C6-Halogenalkinylaminogruppe, einer C3-C9-Cycloalkylaminogruppe, einer C3-C9-Halogencycloalkylaminogruppe, einer C2-C7-Alkylaminocarbonylgruppe, einer C2-C7-Halogenalkylaminocarbonylgruppe, einer C3-C7-Alkenylaminocarbonylgruppe, einer C3-C7-Halogenalkenylaminocarbonylgruppe, einer C3-C7-Alkinylaminocarbonylgruppe, einer C3-C7-Halogenalkinylaminocarbonylgruppe, einer C4-C10-Cycloalkylaminocarbonylgruppe, einer C4-C10-Halogencycloalkylaminocarbonylgruppe, einer Aminogruppe, einer Carbamoylgruppe, einer Cyanogruppe, einer Nitrogruppe, einer Hydroxygruppe, einer Pentafluorsulfanylgruppe, einer Phenylgruppe, die gegebenenfalls einen Substituenten aufweist, und einer heterozyklische Gruppe, die gegebenenfalls einen Substituenten aufweist, und, wenn zwei oder mehr Substituenten vorhanden sind, die Substituenten gleich oder unterschiedlich sein können;
wobei die heterozyklische Gruppe in Q₂ für eine Pyridylgruppe, eine Pyridin-N-oxidgruppe, eine Pyrimidinylgruppe, eine Pyrazinylgruppe, eine Pyridazylgruppe, eine Furylgruppe, eine Thienylgruppe, eine Oxazolylgruppe, eine Isoxazolylgruppe, eine Oxadiazolylgruppe, eine Thiazolylgruppe, eine Isothiazolylgruppe, eine Thiadiazolylgruppe, eine Pyrrolylgruppe, eine Imidazolylgruppe, eine Triazolylgruppe, eine Pyrazolylgruppe oder eine Tetrazolylgruppe steht;
R₁ und R₂ jeweils unabhängig voneinander für ein Wasserstoffatom, ein Sauerstoffatom, ein Halogenatom, eine Hydroxygruppe, eine Nitrogruppe, eine Nitrosogruppe, eine Trimethylsilylgruppe, eine t-Butyldimethylsilylgruppe, eine Cyanogruppe, eine Aminogruppe, eine C1-C6-Alkylgruppe, die gegebenenfalls einen Substituenten aufweist, eine C1-C6-Halogenalkylgruppe, die gegebenenfalls einen Substituenten aufweist, eine C2-C7-Alkylcarbonylgruppe, eine C2-C7-Halogenalkylcarbonylgruppe, eine C2-C6-Alkenylgruppe, die gegebenenfalls einen Substituenten aufweist, eine C2-C6-Halogenalkenylgruppe, die gegebenenfalls einen Substituenten aufweist, eine C2-C6-Alkinylgruppe, die gegebenenfalls einen Substituenten aufweist, eine C2-C6-Halogenalkinylgruppe, die gegebenenfalls einen Substituenten aufweist, eine C2-C7-Alkoxycarbonylgruppe, eine C2-C7-Halogenalkoxycarbonylgruppe, eine C3-C7-Alkenyloxycarbonylgruppe, eine C3-C7-Halogenalkenyloxycarbonylgruppe, eine C3-C7-Alkinyloxycarbonylgruppe, eine C3-C7-Halogenalkinyloxycarbonylgruppe, eine Phenoxycarbonylgruppe, eine C2-C7-Alkylaminocarbonylgruppe, eine C2-C7-Halogenalkylaminocarbonylgruppe, eine C2-C7-Alkylcarbonyloxygruppe, eine C2-C7-Halogenalkylcarbonyloxygruppe, eine Benzoylgruppe, eine C1-C6-Alkoxygruppe, eine C1-C6-Halogenalkoxygruppe,
eine C1-C6-Alkylthiogruppe, eine C1-C6-Halogenalkylthiogruppe, eine C1-C6-Alkylsulfinylgruppe, eine C1-C6-Halogenalkylsulfinylgruppe, eine C1-C6-Alkylsulfonylgruppe, eine C1-C6-Halogenalkylsulfonylgruppe, eine Benzolsulfonylgruppe, eine Benzylsulfonylgruppe, eine Benzylgruppe, oder -C(=O)C(=O)R₇ steht, worin R₇ für eine C1-C6-Alkylgruppe, eine C1-C6-Halogenalkylgruppe, eine C1-C6-Alkoxygruppe oder eine C1-C6-Halogenalkoxygruppe steht,
wobei der Schädling aus der aus den folgenden bestehenden Gruppe ausgewählt ist:
Siphonaptera-Insekten, wie z. B. *Ctenocephalides felis, Ctenocephalides canis, Echidnophaga gallinacea, Pulex irritans* und *Xenopsylla cheopis;*
Mallophaga-Insekten, wie z. B. *Menacanthus stramineus* und *Bovicola bovis;*
Anoplura-Insekten, wie z. B. *Haematopinus eurysternus, Haematopinus suis, Linognathus vituli* und *Solenopotes capillatus;*
Ixodidae, wie z. B. *Boophilus microplus, Rhipicephalus sanguineus, Haemaphysalis longicornis, Haemaphysalis flava, Haemaphysalis campanulata, Ixodes ovatus, Ixodes persulcatus, Amblyomma spp.* und *Dermacentor spp.;*
Cheyletidae, wie z. B. *Cheyletiella yasguri* und *Cheyletiella blakei*;
Demodicidae, wie z. B. *Demodex canis* und *Demodex cati*;
Psoroptidae, wie z. B. *Psoroptes ovis;* und
Sarcoptidae, wie z. B. *Sarcoptes scabiei, Notoedres cati* und *Knemidocoptes spp.*

2. Zusammensetzung, die ein Schädlingsbekämpfungsmittel umfasst, das zumindest ein Amid-Derivat der Formel (1), der Formel (7), der Formel (8) oder der Formel (9), wie in Anspruch 1 definiert, umfasst.

3. Schädlingsbekämpfungsverfahren, welches das Anwenden eines Schädlingsbekämpfungsmittels nach Anspruch 1 oder einer in Anspruch 2 definierten Zusammensetzung auf einen in Anspruch 1 definierten Schädling umfasst, wobei das Verfahren kein Behandlungsverfahren ist.

4. Schädlingsbekämpfungsmittel, das zumindest ein Amid-Derivat der Formel (1), der Formel (7), der Formel (8) oder der Formel (9), wie in Anspruch 1 definiert, umfasst, oder
Zusammensetzung, wie in Anspruch 2 definiert, zur Verwendung in einem Verfahren zur Behandlung des menschlichen oder eines tierischen Körpers.

5. Schädlingsbekämpfungsmittel, das zumindest ein Amid-Derivat der Formel (1), der Formel (7), der Formel (8) oder der Formel (9) umfasst, wie in Anspruch 1 definiert, oder Zusammensetzung, wie in Anspruch 2 definiert, zur Verwendung in einem Verfahren zur Behandlung des menschlichen oder eines tierischen Körpers,
wobei das Verfahren zur Vertilgung eines Schädlings dient, der aus der aus den folgenden bestehenden Gruppe ausgewählt ist:
Siphonaptera-Insekten, wie z. B. *Ctenocephalides felis, Ctenocephalides canis, Echidnophaga gallinacea, Pulex irritans* und *Xenopsylla cheopis;*
Mallophaga-Insekten, wie z. B. *Menacanthus stramineus* und *Bovicola bovis;*
Anoplura-Insekten, wie z. B. *Haematopinus eurysternus, Haematopinus suis, Linognathus vituli* und *Solenopotes capillatus;*
Ixodidae, wie z. B. *Boophilus microplus, Rhipicephalus sanguineus, Haemaphysalis longicornis, Haemaphysalis flava, Haemaphysalis campanulata, Ixodes ovatus, Ixodes persulcatus, Amblyomma spp.* und *Dermacentor spp.;*
Cheyletidae, wie z. B. *Cheyletiella yasguri* und *Cheyletiella blakei*;
Demodicidae, wie z. B. *Demodex canis* und *Demodex cati*;
Psoroptidae, wie z. B. *Psoroptes ovis;* und
Sarcoptidae, wie z. B. *Sarcoptes scabiei, Notoedres cati* und *Knemidocoptes spp.*

6. Verwendung eines Schädlingsbekämpfungsmittels, das zumindest ein Amid-Derviat der Formel (1), der Formel (7), der Formel (8) oder der Formel (9) umfasst, wie in Anspruch 1 definiert, oder einer in Anspruch 2 definierten Zusammensetzung als Schädlingsbekämpfungsmittel, wobei die Verwendung kein Verfahren zur Behandlung des menschlichen oder eines tierischen Körpers ist.

7. Verwendung nach Anspruch 6, wobei die Verwendung zur Bekämpfung eines Schädlings dient, der aus der aus den folgenden bestehenden Gruppe ausgewählt ist:
Siphonaptera-Insekten, wie z. B. *Ctenocephalides felis, Ctenocephalides canis, Echidnophaga gallinacea, Pulex irritans* und *Xenopsylla cheopis;*
Mallophaga-Insekten, wie z. B. *Menacanthus stramineus* und *Bovicola bovis;*
Anoplura-Insekten, wie z. B. *Haematopinus eurysternus, Haematopinus suis, Linognathus vituli* und *Solenopotes capillatus;*
Ixodidae, wie z. B. *Boophilus microplus, Rhipicephalus sanguineus, Haemaphysalis longicornis, Haemaphysalis flava, Haemaphysalis campanulata, Ixodes ovatus, Ixodes persulcatus, Amblyomma spp.* und *Dermacentor spp.;*
Cheyletidae, wie z. B. *Cheyletiella yasguri* und *Cheyletiella blakei*;
Demodicidae, wie z. B. *Demodex canis* und *Demodex cati*;
Psoroptidae, wie z. B. *Psoroptes ovis;* und
Sarcoptidae, wie z. B. *Sarcoptes scabiei, Notoedres cati* und *Knemidocoptes spp.*

## Revendications

1. Agent de lutte contre les nuisibles comprenant, en tant que principe actif, au moins un dérivé amide choisi dans l'ensemble constitué par la formule (1), la formule (7), la formule (8) et la formule (9) suivantes : 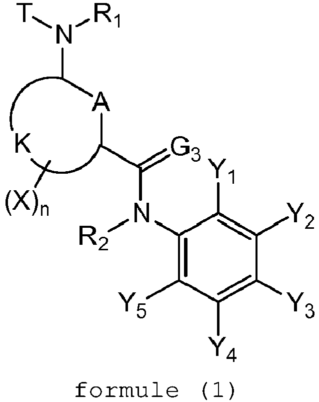 dans lequel, dans la formule (1) :
A représente un atome de carbone, un atome d'oxygène, un atome d'azote, un atome d'azote oxydé, ou un atome de soufre ;
K représente un groupe atomique non métallique nécessaire à la formation d'un groupe de liaison cyclique dérivé de pyridine, pyridine-N-oxyde, pyrrole, thiazole, furane ou thiophène en combinaison avec A et deux atomes de carbone auxquels A se lie ;
X représente un atome d'hydrogène, un atome d'halogène, un groupe alkyle en C₁ à C₆, un groupe halogénoalkyle en C₁ à C₅, un groupe cycloalkyle en C₃ à C₉, un groupe halogénocycloalkyle en C₃ à C₉, un groupe alcényle en C₂ à C₆, un groupe halogénoalcényle en C₂ à C₆, un groupe alcynyle en C₂ à C₆, un groupe halogénoalcynyle en C₂ à C₆, un groupe alcoxy en C₁ à C₆, un groupe halogénoalcoxy en C₁ à C₆, un groupe alcényloxy en C₂ à C₆, un groupe halogénoalcényloxy en C₂ à C₆, un groupe alcynyloxy en C₂ à C₆, un groupe halogénoalcynyloxy en C₂ à C₆, un groupe cycloalcoxy en C₃ à C₉, un groupe halogénocycloalcoxy en C₃ à C₉, un groupe alkylthio en C₁ à C₆, un groupe halogénoalkylthio en C₁ à C₆, un groupe alkylsulfinyle en C₁ à C₆, un groupe halogénoalkylsulfinyle en C₁ à C₆, un groupe alkylsulfonyle en C₁ à C₆, un groupe halogénoalkylsulfonyle en C₁ à C₆, un groupe alkylsulfonyloxy en C₁ à C₆, un groupe halogénoalkylsulfonyloxy en C₁ à C₆, un groupe alkylcarbonyle en C₂ à C₇, un groupe halogénoalkylcarbonyle en C₂ à C₇, un groupe alcénylcarbonyle en C₃ à C₇, un groupe halogénoalcénylcarbonyle en C₃ à C₇, un groupe alcynylcarbonyle en C₃ à C₇, un groupe halogénoalcynylcarbonyle en C₃ à C₇, un groupe cycloalkylcarbonyle en C₄ à C₁₀, un groupe halogénocycloalkylcarbonyle en C₄ à C₁₀, un groupe alkylcarbonyloxy en C₂ à C₇, un groupe halogénoalkylcarbonyloxy en C₂ à C₇, un groupe arylcarbonyloxy, un groupe alcoxycarbonyle en C₂ à C₇, un groupe halogénoalcoxycarbonyle en C₂ à C₇, un groupe alcényloxycarbonyle en C₃ à C₇, un groupe halogénoalcényloxycarbonyle en C₃ à C₇, un groupe alcynyloxycarbonyle en C₃ à C₇, un groupe halogénoalcynyloxycarbonyle en C₃ à C₇, un groupe cycloalkyloxycarbonyle en C₄ à C₁₀, un groupe halogénocycloalkyloxycarbonyle en C₄ à C₁₀, un groupe alkylcarbonylamino en C₂ à C₇, un groupe halogénoalkylcarbonylamino en C₂ à C₇, un groupe alcoxycarbonylamino en C₂ à C₇, un groupe halogénoalcoxycarbonylamino en C₂ à C₇, un groupe alcoxycarbonyloxy en C₂ à C₇, un groupe halogénoalcoxycarbonyloxy en C₂ à C₇, un groupe arylcarbonylamino, un groupe amino, un groupe carbamoyle, un groupe cyano, un groupe hydroxy, un groupe pentafluorosulfanyle, un groupe alkylamino en C₁ à C₆, un groupe halogénoalkylamino en C₁ à C₆, un groupe alcénylamino en C₂ à C₆, un groupe halogénoalcénylamino en C₂ à C₆, un groupe alcynylamino en C₂ à C₆, un groupe halogénoalcynylamino en C₂ à C₆, un groupe cycloalkylamino en C₃ à C₉, un groupe halogénocycloalkylamino en C₃ à C₉, un groupe alkylaminocarbonyle en C₂ à C₇, un groupe halogénoalkylaminocarbonyle en C₂ à C₇, un groupe alcénylaminocarbonyle en C₃ à C₇, un groupe halogénoalcénylaminocarbonyle en C₃ à C₇, un groupe alcynylaminocarbonyle en C₃ à C₇, un groupe halogénoalcynylaminocarbonyle en C₃ à C₇, un groupe cycloalkylaminocarbonyle en C₄ à C₁₀, un groupe halogénocycloalkylaminocarbonyle en C₄ à C₁₀, un groupe phényle, ou un groupe hétérocyclique, et quand il y a plusieurs X, les X peuvent être identiques ou différents ;
n représente un entier de 0 à 4 ;
T représente -C(=G₁)-Q₁ ou -C(=G₁)-G₂Q₂ ;
chacun de G₁ et G₂ représente indépendamment un atome d'oxygène ou un atome de soufre ;
chacun de Q₁ et Q₂ représente un atome d'hydrogène, un groupe alkyle en C₁ à C₆, un groupe halogénoalkyle en C₁ à C₆, un groupe alcényle en C₂ à C₆, un groupe halogénoalcényle en C₂ à C₆, un groupe alcynyle en C₂ à C₆, un groupe halogénoalcynyle en C₂ à C₆, un groupe cycloalkyle en C₃ à C₉, un groupe halogénocycloalkyle en C₃ à C₉, un groupe benzyle, un groupe phényle qui peut porter un substituant, un groupe naphtyle, ou un groupe hétérocyclique qui peut porter un substituant ;
Y₁ et Y₅ représentent un atome d'halogène ou un groupe halogénoalkyle en C₁ à C₃, et soit Y₁ soit Y₅ représente un groupe halogénoalkyle en C₁ à C₃ ;
Y₂ et Y₄ représentent un atome d'hydrogène ;
Y₃ représente un groupe perfluoroalkyle en C₂ à C₄ ;
où, dans Q₁ et Q₂, le substituant d'un groupe phényle qui peut porter un substituant et d'un groupe hétérocyclique qui peut porter un substituant est un ou plusieurs substituants choisis dans l'ensemble constitué par :
un atome d'halogène, un groupe alkyle en C₁ à C₆, un groupe halogénoalkyle en C₁ à C₆, un groupe cycloalkyle en C₃ à C₉, un groupe halogénocycloalkyle en C₃ à C₉, un groupe alcényle en C₂ à C₆, un groupe halogénoalcényle en C₂ à C₆, un groupe alcynyle en C₂ à C₆, un groupe halogénoalcynyle en C₂ à C₆, un groupe alcoxy en C₁ à C₆, un groupe halogénoalcoxy en C₁ à C₆, un groupe alcényloxy en C₂ à C₆, un groupe halogénoalcényloxy en C₂ à C₆, un groupe alcynyloxy en C₂ à C₆, un groupe halogénoalcynyloxy en C₂ à C₆, un groupe cycloalcoxy en C₃ à C₉, un groupe halogénocycloalcoxy en C₃ à C₉, un groupe alkylthio en C₁ à C₆, un groupe halogénoalkylthio en C₁ à C₆, un groupe alkylsulfinyle en C₁ à C₆, un groupe halogénoalkylsulfinyle en C₁ à C₆, un groupe alkylsulfonyle en C₁ à C₆, un groupe halogénoalkylsulfonyle en C₁ à C₆, un groupe alkylcarbonyle en C₂ à C₇, un groupe halogénoalkylcarbonyle en C₂ à C₇, un groupe alcénylcarbonyle en C₃ à C₇, un groupe halogénoalcénylcarbonyle en C₃ à C₇, un groupe alcynylcarbonyle en C₃ à C₇, un groupe halogénoalcynylcarbonyle en C₃ à C₇, un groupe cycloalkylcarbonyle en C₄ à C₁₀, un groupe halogénocycloalkylcarbonyle en C₄ à C₁₀, un groupe alkylcarbonyloxy en C₂ à C₇, un groupe halogénoalkylcarbonyloxy en C₂ à C₇, un groupe alkylsulfonyloxy en C₁ à C₆, un groupe halogénoalkylsulfonyloxy en C₁ à C₆, un groupe alcoxycarbonyle en C₂ à C₇, un groupe halogénoalcoxycarbonyle en C₂ à C₇, un groupe alcényloxycarbonyle en C₃ à C₇, un groupe halogénoalcényloxycarbonyle en C₃ à C₇, un groupe alcynyloxycarbonyle en C₃ à C₇, un groupe halogénoalcynyloxycarbonyle en C₃ à C₇, un groupe cycloalkyloxycarbonyle en C₄ à C₁₀, un groupe halogénocycloalkyloxycarbonyle en C₄ à C₁₀, un groupe alkylcarbonylamino en C₂ à C₇, un groupe halogénoalkylcarbonylamino en C₂ à C₇, un groupe alcoxycarbonylamino en C₂ à C₇, un groupe halogénoalcoxycarbonylamino en C₂ à C₇, un groupe alkylamino en C₁ à C₆, un groupe halogénoalkylamino en C₁ à C₆, un groupe alcénylamino en C₂ à C₆, un groupe halogénoalcénylamino en C₂ à C₆, un groupe alcynylamino en C₂ à C₆, un groupe halogénoalcynylamino en C₂ à C₆, un groupe cycloalkylamino en C₃ à C₉, un groupe halogénocycloalkylamino en C₃ à C₉, un groupe alkylaminocarbonyle en C₂ à C₇, un groupe halogénoalkylaminocarbonyle en C₂ à C₇, un groupe alcénylaminocarbonyle en C₃ à C₇, un groupe halogénoalcénylaminocarbonyle en C₃ à C₇, un groupe alcynylaminocarbonyle en C₃ à C₇, un groupe halogénoalcynylaminocarbonyle en C₃ à C₇, un groupe cycloalkylaminocarbonyle en C₄ à C₁₀, un groupe halogénocycloalkylaminocarbonyle en C₄ à C₁₀, un groupe amino, un groupe carbamoyle, un groupe cyano, un groupe nitro, un groupe hydroxy, un groupe pentafluorosulfanyle, un groupe phényle qui peut porter un substituant, et un groupe hétérocyclique qui peut porter un substituant, et quand il y a deux substituants ou plus, les substituants peuvent être identiques ou différents ;
où le groupe hétérocyclique dans X, Q₁ et Q₂ représente un groupe pyridyle, un groupe pyridine-N-oxyde, un groupe pyrimidinyle, un groupe pyrazinyle, un groupe pyridazyle, un groupe furyle, un groupe thiényle, un groupe oxazolyle, un groupe isoxazolyle, un groupe oxadiazolyle, un groupe thiazolyle, un groupe isothiazolyle, un groupe thiadiazolyle, un groupe pyrrolyle, un groupe imidazolyle, un groupe triazolyle, un groupe pyrazolyle, ou un groupe tétrazolyle ;
G₃ représente un atome d'oxygène ou un atome de soufre ; et
chacun de R₁ et R₂ représente indépendamment un atome d'hydrogène, un atome d'oxygène, un atome d'halogène, un groupe hydroxy, un groupe nitro, un groupe nitroso, un groupe triméthylsilyle, un groupe t-butyldiméthylsilyle, un groupe cyano, un groupe amino, un groupe alkyle en C₁ à C₆ qui peut porter un substituant, un groupe halogénoalkyle en C₁ à C₆ qui peut porter un substituant, un groupe alkylcarbonyle en C₂ à C₇, un groupe halogénoalkylcarbonyle en C₂ à C₇, un groupe alcényle en C₂ à C₆ qui peut porter un substituant, un groupe halogénoalcényle en C₂ à C₆ qui peut porter un substituant, un groupe alcynyle en C₂ à C₆ qui peut porter un substituant, un groupe halogénoalcynyle en C₂ à C₆ qui peut porter un substituant, un groupe alcoxycarbonyle en C₂ à C₇, un groupe halogénoalcoxycarbonyle en C₂ à C₇, un groupe alcényloxycarbonyle en C₃ à C₇, un groupe halogénoalcényloxycarbonyle en C₃ à C₇, un groupe alcynyloxycarbonyle en C₃ à C₇, un groupe halogénoalcynyloxycarbonyle en C₃ à C₇, un groupe phénoxycarbonyle, un groupe alkylaminocarbonyle en C₂ à C₇, un groupe halogénoalkylaminocarbonyle en C₂ à C₇, un groupe alkylcarbonyloxy en C₂ à C₇, un groupe halogénoalkylcarbonyloxy en C₂ à C₇, un groupe benzoyle, un groupe alcoxy en C₁ à C₆, un groupe halogénoalcoxy en C₁ à C₆, un groupe alkylthio en C₁ à C₆, un groupe halogénoalkylthio en C₁ à C₆, un groupe alkylsulfinyle en C₁ à C₆, un groupe halogénoalkylsulfinyle en C₁ à C₆, un groupe alkylsulfonyle en C₁ à C₆, un groupe halogénoalkylsulfonyle en C₁ à C₆, un groupe benzènesulfonyle, un groupe benzylsulfonyle, un groupe benzyle, ou -C(=O)C(=O)R₇, où R₇ représente un groupe alkyle en C₁ à C₆, un groupe halogénoalkyle en C₁ à C₆, un groupe alcoxy en C₁ à C₆, ou un groupe halogénoalcoxy en C₁ à C₆ ; 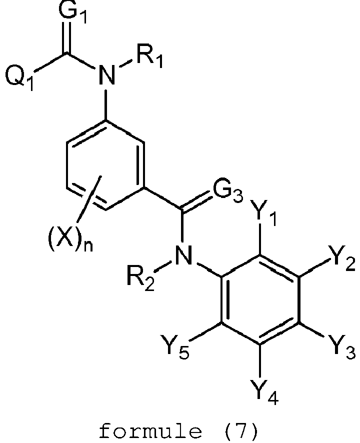
dans lequel, dans la formule (7) :
n vaut 4 ;
chaque X représente indépendamment un atome d'hydrogène, un atome d'halogène, ou un groupe cyano ;
Y₃ représente un groupe perfluoroalkyle en C₂ à C₄ ;
Y₂ et Y₄ représentent un atome d'hydrogène ;
Y₁ et Y₅ représentent un atome d'halogène ou un groupe halogénoalkyle en C₁ à C₃, et soit Y₁ soit Y₅ représente un groupe halogénoalkyle en C₁ à C₃ ;
chacun de G₁ et G₃ représente indépendamment un atome d'oxygène ou un atome de soufre ;
Q₁ représente un atome d'hydrogène, un groupe alkyle en C₁ à C₆, un groupe halogénoalkyle en C₁ à C₆, un groupe alcényle en C₂ à C₆, un groupe halogénoalcényle en C₂ à C₆, un groupe alcynyle en C₂ à C₆, un groupe halogénoalcynyle en C₂ à C₆, un groupe cycloalkyle en C₃ à C₉, un groupe halogénocycloalkyle en C₃ à C₉, un groupe benzyle, un groupe phényle qui peut porter un substituant, un groupe naphtyle, ou un groupe hétérocyclique qui peut porter un substituant ;
où, dans Q₁, le substituant d'un groupe phényle qui peut porter un substituant et d'un groupe hétérocyclique qui peut porter un substituant est un ou plusieurs substituants choisis dans l'ensemble constitué par :
un atome d'halogène, un groupe alkyle en C₁ à C₆, un groupe halogénoalkyle en C₁ à C₆, un groupe cycloalkyle en C₃ à C₉, un groupe halogénocycloalkyle en C₃ à C₉, un groupe alcényle en C₂ à C₆, un groupe halogénoalcényle en C₂ à C₆, un groupe alcynyle en C₂ à C₆, un groupe halogénoalcynyle en C₂ à C₆, un groupe alcoxy en C₁ à C₆, un groupe halogénoalcoxy en C₁ à C₆, un groupe alcényloxy en C₂ à C₆, un groupe halogénoalcényloxy en C₂ à C₆, un groupe alcynyloxy en C₂ à C₆, un groupe halogénoalcynyloxy en C₂ à C₆, un groupe cycloalcoxy en C₃ à C₉, un groupe halogénocycloalcoxy en C₃ à C₉, un groupe alkylthio en C₁ à C₆, un groupe halogénoalkylthio en C₁ à C₆, un groupe alkylsulfinyle en C₁ à C₆, un groupe halogénoalkylsulfinyle en C₁ à C₆, un groupe alkylsulfonyle en C₁ à C₆, un groupe halogénoalkylsulfonyle en C₁ à C₆, un groupe alkylcarbonyle en C₂ à C₇, un groupe halogénoalkylcarbonyle en C₂ à C₇, un groupe alcénylcarbonyle en C₃ à C₇, un groupe halogénoalcénylcarbonyle en C₃ à C₇, un groupe alcynylcarbonyle en C₃ à C₇, un groupe halogénoalcynylcarbonyle en C₃ à C₇, un groupe cycloalkylcarbonyle en C₄ à C₁₀, un groupe halogénocycloalkylcarbonyle en C₄ à C₁₀, un groupe alkylcarbonyloxy en C₂ à C₇, un groupe halogénoalkylcarbonyloxy en C₂ à C₇, un groupe alkylsulfonyloxy en C₁ à C₆, un groupe halogénoalkylsulfonyloxy en C₁ à C₆, un groupe alcoxycarbonyle en C₂ à C₇, un groupe halogénoalcoxycarbonyle en C₂ à C₇, un groupe alcényloxycarbonyle en C₃ à C₇, un groupe halogénoalcényloxycarbonyle en C₃ à C₇, un groupe alcynyloxycarbonyle en C₃ à C₇, un groupe halogénoalcynyloxycarbonyle en C₃ à C₇, un groupe cycloalkyloxycarbonyle en C₄ à C₁₀, un groupe halogénocycloalkyloxycarbonyle en C₄ à C₁₀, un groupe alkylcarbonylamino en C₂ à C₇, un groupe halogénoalkylcarbonylamino en C₂ à C₇, un groupe alcoxycarbonylamino en C₂ à C₇, un groupe halogénoalcoxycarbonylamino en C₂ à C₇, un groupe alkylamino en C₁ à C₆, un groupe halogénoalkylamino en C₁ à C₆, un groupe alcénylamino en C₂ à C₆, un groupe halogénoalcénylamino en C₂ à C₆, un groupe alcynylamino en C₂ à C₆, un groupe halogénoalcynylamino en C₂ à C₆, un groupe cycloalkylamino en C₃ à C₉, un groupe halogénocycloalkylamino en C₃ à C₉, un groupe alkylaminocarbonyle en C₂ à C₇, un groupe halogénoalkylaminocarbonyle en C₂ à C₇, un groupe alcénylaminocarbonyle en C₃ à C₇, un groupe halogénoalcénylaminocarbonyle en C₃ à C₇, un groupe alcynylaminocarbonyle en C₃ à C₇, un groupe halogénoalcynylaminocarbonyle en C₃ à C₇, un groupe cycloalkylaminocarbonyle en C₄ à C₁₀, un groupe halogénocycloalkylaminocarbonyle en C₄ à C₁₀, un groupe amino, un groupe carbamoyle, un groupe cyano, un groupe nitro, un groupe hydroxy, un groupe pentafluorosulfanyle, un groupe phényle qui peut porter un substituant, et un groupe hétérocyclique qui peut porter un substituant, et quand il y a deux substituants ou plus, les substituants peuvent être identiques ou différents ;
où le groupe hétérocyclique dans Q₁ représente un groupe pyridyle, un groupe pyridine-N-oxyde, un groupe pyrimidinyle, un groupe pyrazinyle, un groupe pyridazyle, un groupe furyle, un groupe thiényle, un groupe oxazolyle, un groupe isoxazolyle, un groupe oxadiazolyle, un groupe thiazolyle, un groupe isothiazolyle, un groupe thiadiazolyle, un groupe pyrrolyle, un groupe imidazolyle, un groupe triazolyle, un groupe pyrazolyle, ou un groupe tétrazolyle ;
chacun de R₁ et R₂ représente indépendamment un atome d'hydrogène, un atome d'oxygène, un atome d'halogène, un groupe hydroxy, un groupe nitro, un groupe nitroso, un groupe triméthylsilyle, un groupe t-butyldiméthylsilyle, un groupe cyano, un groupe amino, un groupe alkyle en C₁ à C₆ qui peut porter un substituant, un groupe halogénoalkyle en C₁ à C₆ qui peut porter un substituant, un groupe alkylcarbonyle en C₂ à C₇, un groupe halogénoalkylcarbonyle en C₂ à C₇, un groupe alcényle en C₂ à C₆ qui peut porter un substituant, un groupe halogénoalcényle en C₂ à C₆ qui peut porter un substituant, un groupe alcynyle en C₂ à C₆ qui peut porter un substituant, un groupe halogénoalcynyle en C₂ à C₆ qui peut porter un substituant, un groupe alcoxycarbonyle en C₂ à C₇, un groupe halogénoalcoxycarbonyle en C₂ à C₇, un groupe alcényloxycarbonyle en C₃ à C₇, un groupe halogénoalcényloxycarbonyle en C₃ à C₇, un groupe alcynyloxycarbonyle en C₃ à C₇, un groupe halogénoalcynyloxycarbonyle en C₃ à C₇, un groupe phénoxycarbonyle, un groupe alkylaminocarbonyle en C₂ à C₇, un groupe halogénoalkylaminocarbonyle en C₂ à C₇, un groupe alkylcarbonyloxy en C₂ à C₇, un groupe
halogénoalkylcarbonyloxy en C₂ à C₇, un groupe benzoyle, un groupe alcoxy en C₁ à C₆, un groupe halogénoalcoxy en C₁ à C₆, un groupe alkylthio en C₁ à C₆, un groupe halogénoalkylthio en C₁ à C₆, un groupe alkylsulfinyle en C₁ à C₆, un groupe halogénoalkylsulfinyle en C₁ à C₆, un groupe alkylsulfonyle en C₁ à C₆, un groupe halogénoalkylsulfonyle en C₁ à C₆, un groupe benzènesulfonyle, un groupe benzylsulfonyle, un groupe benzyle, ou -C(=O)C(=O)R₇ où R₇ représente un groupe alkyle en C₁ à C₆, un groupe halogénoalkyle en C₁ à C₆, un groupe alcoxy en C₁ à C₆, ou un groupe halogénoalcoxy en C₁ à C₆ ; 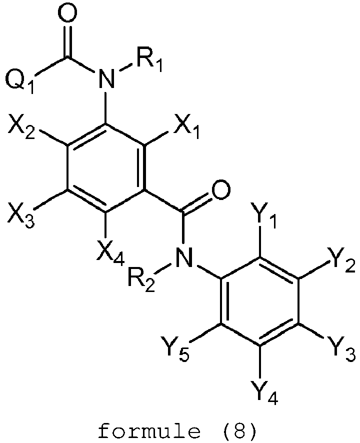
dans lequel, dans la formule (8) :
Q₁ représente un groupe phényle ou un groupe pyridyle qui peut porter un substituant choisi dans l'ensemble constitué par : un atome d'halogène, un groupe halogénoalkyle en C₁, un groupe nitro, et un groupe cyano, dans le cas où Q₁ porte des substituants et le nombre des substituants est de 1 ou 2 ;
chacun de X₁ et X₂ représente indépendamment un atome d'hydrogène ou un atome de fluor, et X₃ et X₄ représentent un atome d'hydrogène ;
chacun de R₁ et R₂ représente indépendamment un atome d'hydrogène ou un groupe méthyle ;
chacun de Y₁ et Y₅ représente indépendamment un atome de chlore, un atome de brome, un atome d'iode, un groupe trifluorométhoxy, un groupe trifluorométhyle, ou un groupe pentafluoroéthyle ;
Y₂ et Y₄ représentent un atome d'hydrogène ;
et Y₃ représente un groupe perfluoroalkyle en C₃ à C₄ ;
dans le cas où Y₁ et Y₅ représentent simultanément des atomes d'halogène, au moins l'un de X₁ et X₂ représente un atome de fluor ; et
dans le cas où Y₁ ou Y₅ représente un groupe trifluorométhoxy, X₂ représente un atome de fluor ; 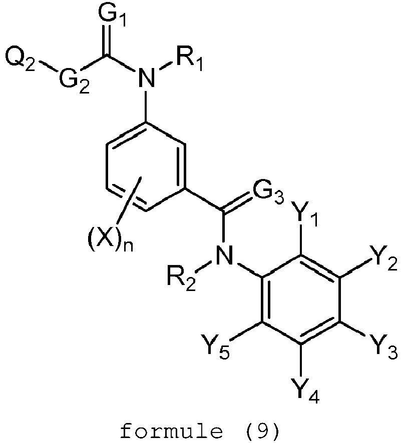
dans lequel, dans la formule (9) :
n vaut 4 ;
chacun des quatre X représente indépendamment un atome d'hydrogène, un atome d'halogène, ou un groupe cyano ;
Y₃ représente un groupe perfluoroalkyle en C₂ à C₄ ;
Y₂ et Y₄ représentent un atome d'hydrogène ;
chacun de Y₁ et Y₅ représente indépendamment un atome d'halogène ou un groupe halogénoalkyle en C₁ à C₃, et soit Y₁ soit Y₅ représente un groupe halogénoalkyle en C₁ à C₃ ;
chacun de G₁, G₂ et G₃ représente indépendamment un atome d'oxygène ou un atome de soufre ;
Q₂ représente un atome d'hydrogène, un groupe alkyle en C₁ à C₆, un groupe halogénoalkyle en C₁ à C₆, un groupe alcényle en C₂ à C₆, un groupe halogénoalcényle en C₂ à C₆, un groupe alcynyle en C₂ à C₆, un groupe halogénoalcynyle en C₂ à C₆, un groupe cycloalkyle en C₃ à C₉, un groupe halogénocycloalkyle en C₃ à C₉, un groupe benzyle, un groupe phényle qui peut porter un substituant, un groupe naphtyle, ou un groupe hétérocyclique qui peut porter un substituant ;
où, dans Q₂, le substituant d'un groupe phényle qui peut porter un substituant et d'un groupe hétérocyclique qui peut porter un substituant est un ou plusieurs substituants choisis dans l'ensemble constitué par :
un atome d'halogène, un groupe alkyle en C₁ à C₆, un groupe halogénoalkyle en C₁ à C₆, un groupe cycloalkyle en C₃ à C₉, un groupe halogénocycloalkyle en C₃ à C₉, un groupe alcényle en C₂ à C₆, un groupe halogénoalcényle en C₂ à C₆, un groupe alcynyle en C₂ à C₆, un groupe halogénoalcynyle en C₂ à C₆, un groupe alcoxy en C₁ à C₆, un groupe halogénoalcoxy en C₁ à C₆, un groupe alcényloxy en C₂ à C₆, un groupe halogénoalcényloxy en C₂ à C₆, un groupe alcynyloxy en C₂ à C₆, un groupe halogénoalcynyloxy en C₂ à C₆, un groupe cycloalcoxy en C₃ à C₉, un groupe halogénocycloalcoxy en C₃ à C₉, un groupe alkylthio en C₁ à C₆, un groupe halogénoalkylthio en C₁ à C₆, un groupe alkylsulfinyle en C₁ à C₆, un groupe halogénoalkylsulfinyle en C₁ à C₆, un groupe alkylsulfonyle en C₁ à C₆, un groupe halogénoalkylsulfonyle en C₁ à C₆, un groupe alkylcarbonyle en C₂ à C₇, un groupe halogénoalkylcarbonyle en C₂ à C₇, un groupe alcénylcarbonyle en C₃ à C₇, un groupe halogénoalcénylcarbonyle en C₃ à C₇, un groupe alcynylcarbonyle en C₃ à C₇, un groupe halogénoalcynylcarbonyle en C₃ à C₇, un groupe cycloalkylcarbonyle en C₄ à C₁₀, un groupe halogénocycloalkylcarbonyle en C₄ à C₁₀, un groupe alkylcarbonyloxy en C₂ à C₇, un groupe halogénoalkylcarbonyloxy en C₂ à C₇, un groupe alkylsulfonyloxy en C₁ à C₆, un groupe halogénoalkylsulfonyloxy en C₁ à C₆, un groupe alcoxycarbonyle en C₂ à C₇, un groupe halogénoalcoxycarbonyle en C₂ à C₇, un groupe alcényloxycarbonyle en C₃ à C₇, un groupe
halogénoalcényloxycarbonyle en C₃ à C₇, un groupe alcynyloxycarbonyle en C₃ à C₇, un groupe halogénoalcynyloxycarbonyle en C₃ à C₇, un groupe cycloalkyloxycarbonyle en C₄ à C₁₀, un groupe halogénocycloalkyloxycarbonyle en C₄ à C₁₀, un groupe alkylcarbonylamino en C₂ à C₇, un groupe halogénoalkylcarbonylamino en C₂ à C₇, un groupe alcoxycarbonylamino en C₂ à C₇, un groupe halogénoalcoxycarbonylamino en C₂ à C₇, un groupe alkylamino en C₁ à C₆, un groupe halogénoalkylamino en C₁ à C₆, un groupe alcénylamino en C₂ à C₆, un groupe halogénoalcénylamino en C₂ à C₆, un groupe alcynylamino en C₂ à C₆, un groupe halogénoalcynylamino en C₂ à C₆, un groupe cycloalkylamino en C₃ à C₉, un groupe halogénocycloalkylamino en C₃ à C₉, un groupe alkylaminocarbonyle en C₂ à C₇, un groupe halogénoalkylaminocarbonyle en C₂ à C₇, un groupe alcénylaminocarbonyle en C₃ à C₇, un groupe halogénoalcénylaminocarbonyle en C₃ à C₇, un groupe alcynylaminocarbonyle en C₃ à C₇, un groupe halogénoalcynylaminocarbonyle en C₃ à C₇, un groupe cycloalkylaminocarbonyle en C₄ à C₁₀, un groupe halogénocycloalkylaminocarbonyle en C₄ à C₁₀, un groupe amino, un groupe carbamoyle, un groupe cyano, un groupe nitro, un groupe hydroxy, un groupe pentafluorosulfanyle, un groupe phényle qui peut porter un substituant, et un groupe hétérocyclique qui peut porter un substituant, et quand il y a deux substituants ou plus, les substituants peuvent être identiques ou différents ;
où le groupe hétérocyclique dans Q₂ représente un groupe pyridyle, un groupe pyridine-N-oxyde, un groupe pyrimidinyle, un groupe pyrazinyle, un groupe pyridazyle, un groupe furyle, un groupe thiényle, un groupe oxazolyle, un groupe isoxazolyle, un groupe oxadiazolyle, un groupe thiazolyle, un groupe isothiazolyle, un groupe thiadiazolyle, un groupe pyrrolyle, un groupe imidazolyle, un groupe triazolyle, un groupe pyrazolyle, ou un groupe tétrazolyle ;
chacun de R₁ et R₂ représente indépendamment un atome d'hydrogène, un atome d'oxygène, un atome d'halogène, un groupe hydroxy, un groupe nitro, un groupe nitroso, un groupe triméthylsilyle, un groupe t-butyldiméthylsilyle, un groupe cyano, un groupe amino, un groupe alkyle en C₁ à C₆ qui peut porter un substituant, un groupe halogénoalkyle en C₁ à C₆ qui peut porter un substituant, un groupe alkylcarbonyle en C₂ à C₇, un groupe halogénoalkylcarbonyle en C₂ à C₇, un groupe alcényle en C₂ à C₆ qui peut porter un substituant, un groupe halogénoalcényle en C₂ à C₆ qui peut porter un substituant, un groupe alcynyle en C₂ à C₆ qui peut porter un substituant, un groupe halogénoalcynyle en C₂ à C₆ qui peut porter un substituant, un groupe alcoxycarbonyle en C₂ à C₇, un groupe halogénoalcoxycarbonyle en C₂ à C₇, un groupe alcényloxycarbonyle en C₃ à C₇, un groupe halogénoalcényloxycarbonyle en C₃ à C₇, un groupe alcynyloxycarbonyle en C₃ à C₇, un groupe halogénoalcynyloxycarbonyle en C₃ à C₇, un groupe phénoxycarbonyle, un groupe alkylaminocarbonyle en C₂ à C₇, un groupe halogénoalkylaminocarbonyle en C₂ à C₇, un groupe alkylcarbonyloxy en C₂ à C₇, un groupe halogénoalkylcarbonyloxy en C₂ à C₇, un groupe benzoyle, un groupe alcoxy en C₁ à C₆, un groupe halogénoalcoxy en C₁ à C₆, un groupe alkylthio en C₁ à C₆, un groupe halogénoalkylthio en C₁ à C₆, un groupe alkylsulfinyle en C₁ à C₆, un groupe halogénoalkylsulfinyle en C₁ à C₆, un groupe alkylsulfonyle en C₁ à C₆, un groupe halogénoalkylsulfonyle en C₁ à C₆, un groupe benzènesulfonyle, un groupe benzylsulfonyle, un groupe benzyle, ou -C(=O)C(=O)R₇, où R₇ représente un groupe alkyle en C₁ à C₆, un groupe halogénoalkyle en C₁ à C₆, un groupe alcoxy en C₁ à C₆, ou un groupe halogénoalcoxy en C₁ à C₆,
dans lequel le nuisible est choisi dans l'ensemble constitué par :
les insectes siphonaptères tels que *Ctenocephalides felis, Ctenocephalides canis, Echidnophaga gallinacea, Pulex irritans* et *Xenopsylla cheopis ;*
les insectes mallophages tels que *Menacanthus stramineus* et *Bovicola bovis ;*
les insectes anoploures tels que *Haematopinus eurysternus, Haematopinus suis, Linognathus vituli* et *Solenopotes capillatus ;*
les ixodidae tels que *Boophilus microplus, Rhipicephalus sanguineus, Haemaphysalis longicornis, Haemaphysalis flava, Haemaphysalis campanulata, Ixodes ovatus, Ixodes persulcatus, Amblyomma spp.* et *Dermacentor spp. ;*
les cheyletidae tels que *Cheyletiella yasguri* et *Cheyletiella blakei ;*
les demodicidae tels que *Demodex canis, Demodex cati* ; les psoroptidae tels que *Psoroptes ovis* ; et
les sarcoptidae tels que *Sarcoptes scabiei, Notoedres cati* et *Knemidocoptes* spp.

2. Composition comprenant un agent de lutte contre les nuisibles comprenant au moins un dérivé amide selon la formule (1), la formule (7), la formule (8) ou la formule (9), tel que défini dans la revendication 1.

3. Méthode de lutte contre les nuisibles comprenant l'application de l'agent de lutte contre les nuisibles de la revendication 1, ou de la composition telle que définie dans la revendication 2, sur un nuisible tel que défini dans la revendication 1, laquelle méthode n'est pas une méthode de traitement.

4. Agent de lutte contre les nuisibles comprenant, au moins un dérivé amide selon la formule (1), la formule (7), la formule (8) ou la formule (9), tel que défini selon la revendication 1, ou une composition telle que définie dans la revendication 2, pour une utilisation dans une méthode de traitement du corps humain ou animal.

5. Agent de lutte contre les nuisibles comprenant au moins un dérivé amide selon la formule (1), la formule (7), la formule (8) ou la formule (9), tel que défini selon la revendication 1, ou une composition telle que définie dans la revendication 2, pour une utilisation dans une méthode de traitement du corps humain ou animal,
dans lequel la méthode a pour but d'exterminer un nuisible choisi dans l'ensemble constitué par :
les insectes siphonaptères tels que *Ctenocephalides felis, Ctenocephalides canis, Echidnophaga gallinacea, Pulex irritans* et *Xenopsylla cheopis ;*
les insectes mallophages tels que *Menacanthus stramineus* et *Bovicola bovis ;*
les insectes anoploures tels que *Haematopinus eurysternus, Haematopinus suis, Linognathus vituli* et *Solenopotes capillatus ;*
les ixodidae tels que *Boophilus microplus, Rhipicephalus sanguineus, Haemaphysalis longicornis, Haemaphysalis flava, Haemaphysalis campanulata, Ixodes ovatus, Ixodes persulcatus, Amblyomma spp.* et *Dermacentor spp. ;*
les cheyletidae tels que *Cheyletiella yasguri* et *Cheyletiella blakei ;*
les demodicidae tels que *Demodex canis, Demodex cati* ;
les psoroptidae tels que *Psoroptes ovis* ; et
les sarcoptidae tels que *Sarcoptes scabiei, Notoedres cati* et *Knemidocoptes* spp.

6. Utilisation d'un agent de lutte contre les nuisibles comprenant au moins un dérivé amide selon la formule (1), la formule (7), la formule (8) ou la formule (9), tel que défini selon la revendication 1, ou une composition telle que définie dans la revendication 2, en tant qu'agent de lutte contre les nuisibles, laquelle utilisation n'est pas une méthode de traitement du corps humain ou animal.

7. Utilisation selon la revendication 6, laquelle utilisation a pour but de lutter contre un nuisible choisi dans l'ensemble constitué par :
les insectes siphonaptères tels que *Ctenocephalides felis, Ctenocephalides canis, Echidnophaga gallinacea, Pulex irritans* et *Xenopsylla cheopis ;*
les insectes mallophages tels que *Menacanthus stramineus* et *Bovicola bovis ;*
les insectes anoploures tels que *Haematopinus eurysternus, Haematopinus suis, Linognathus vituli* et *Solenopotes capillatus ;*
les ixodidae tels que *Boophilus microplus, Rhipicephalus sanguineus, Haemaphysalis longicornis, Haemaphysalis flava, Haemaphysalis campanulata, Ixodes ovatus, Ixodes persulcatus, Amblyomma spp.* et *Dermacentor spp. ;*
les cheyletidae tels que *Cheyletiella yasguri* et *Cheyletiella blakei ;*
les demodicidae tels que *Demodex canis, Demodex cati* ;
les psoroptidae tels que *Psoroptes ovis* ; et
les sarcoptidae tels que *Sarcoptes scabiei, Notoedres cati* et *Knemidocoptes* spp.
